# EUROPEAN PATENT SPECIFICATION

(11) **EP 4 126 855 B1**
(45) Date of publication and mention of the grant of the patent: **24.04.2024**
(21) Application number: 20714519.4
(22) Date of filing: 24.03.2020
(51) Int. Cl.: C07D 413/14, C07D 498/04, C07D 513/04, C07D 519/00, A61P 31/04, A61K 31/5383

(54) **COMPOUNDS AND THEIR USE IN THE TREATMENT OF BACTERIAL INFECTIONS**
VERBINDUNGEN UND DEREN VERWENDUNG BEI DER BEHANDLUNG VON BAKTERIELLEN INFEKTIONEN
COMPOSÉS ET LEUR UTILISATION DANS LE TRAITEMENT DES INFECTIONS BACTÉRIENNES

(43) Date of publication of application: 08.02.2023
(73) Proprietor: F. Hoffmann-La Roche AG, 4070 Basel (CH)
(72) Inventor: CUMMING, John G., 4070 Basel (CH); KRAMER, Christian, 4070 Basel (CH); KREIS, Lukas, 4070 Basel (CH); KUEHNE, Holger, 4070 Basel (CH); SCHNIDER, Patrick, 4070 Basel (CH)
(74) Representative: Jochnowitz, Evan
(86) International application number: PCT/EP2020/058045
(87) International publication number: WO 2021/190727

(56) References cited:
- US-A1- 2011 195 949
- US-A1- 2016 075 722
- DATABASE REGISTRY [Online] CHEMICAL ABSTRACTS SERVICE, COLUMBUS, OHIO, US; 11 July 2016 (2016-07-11), XP055696787, Database accession no. 1949607-17-3

## Description

The present invention provides compounds which exhibit broad spectrum antibacterial activity, their manufacture, pharmaceutical compositions comprising them and their use as medicaments for the treatment of microbial infections, in particular for the treatment of diseases and infections caused by *Pseudomonas aeruginosa, Klebsiella pneumoniae, Escherichia coli, Acinetobacter baumannii* and *Staphylococcus aureus,* more particulary caused by Gram negative bacterias, furthermore particularly to *Escherichia coli.*

Antibacterial compounds are known for instance from US 2011/195949 A1.

The present invention provides novel compounds of formula (I) 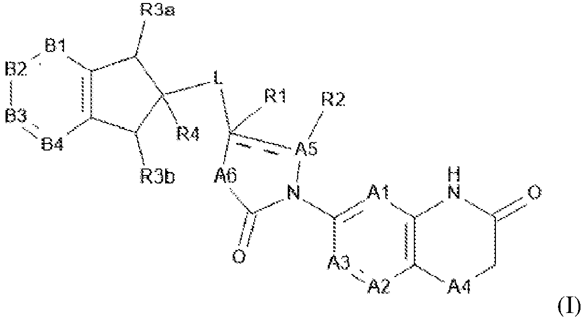 wherein
A¹ is selected from
   i) -N-, and
   ii) -CH-;
A² is selected from
   i) -N-, and
   ii) -CH-;
A³ is selected from
   i) -N-, and
   ii) -CH-;
A⁴ is selected from
   i) -O-, and
   ii) -S-;
A⁵ is selected from
   i) -N-, and
   ii) -CH-;
A⁶ is selected from
   i) -O-, and
   ii) -CH₂-;
-̅ -̅ represents a single or double bond;
B¹ is selected from
   i) -N-, and
   ii) -CR⁵-;
B² is selected from
   i) -N-, and
   ii) -CR⁶-;
wherein when B¹ is CR⁵ and B² is CR⁶, R⁵ and R⁶ may be bonded to each other to form a 4-to 6- membered ring optionally substituted with R^{a} and/or R^{b};
R^{a} and R^{b} are independently selected from
   i) COOR^{a1}, and
   ii) C₁₋₆-alkyl;
R^{a1} is selected from
   i) H, and
   ii) C₁₋₆-alkyl;
B³ is selected from
   i) -N-, and
   ii) -CR⁷-;
B⁴ is selected from
   i) -N-, and
   ii) -CR⁸-;
R¹ is selected from
   i) H,
   ii) NH₂,
   iii) OH, and
   iv) C₁₋₆ alkyl optionally substituted with R^{c},
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d};
R^{c} is selected from
   i) OH, and
   ii) NH₂;
R^{d} is selected from.
   i) (CH2)nNH₂, and
   ii) (CH2)nOH;
n represents an integer of 0 to 6;
R² is selected from
   i) H, and
   ii) C₁₋₆ alkyl optionally substituted with R^{c},
or R², L₂ and R^{w} may be bonded to each other to form a 4- to 6- membered ring optionally substituted with R^{d};
wherein
when -̅ -̅ represents a double bond, R₁ and R₂ are none;
R^{3a} is selected from
   i) H,
   ii) NH₂, and
   iii) OH;
R^{3b} is selected from
   i) H,
   ii) NH₂, and
   iii) OH;
wherein either one of R^{3a} and R^{3b} is H;
R⁴ is selected from
   i) H,
   ii) OH, and
   iii) NH₂;
R⁵ is selected from
   i) H,
   ii) halogen,
   iii) cyano,
   iv) C₁₋₆-alkyl
   v) 4- to 6 -membered heteroaryl optionally substituted with C₁₋₆-alkyl,
   vi) OR^{e},
   vii) SO₂R^{f},
   viii) SO₂NR^{e}R^{f}, and
   ix) NR^{e}R^{f};
R^{e} and R^{f} are independently selected from
   i) H, and
   ii) C₁₋₆-alkyl;
R⁶ is selected from
   i) H,
   ii) halogen,
   iii) C₁₋₆-alkyl,
   iv) OR^{g},
   v) NR^{h}Rⁱ, and
   vi) cyano;
R^{g} is selected from
   i) H,
   ii) -(CH₂)ₘ-R^{g1},
   iii) 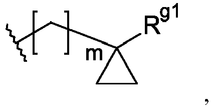
   iv) 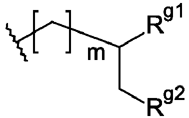
   v) 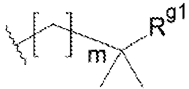 and
   vi) 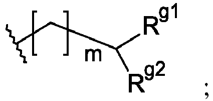
m represents an integer of 0 to 6;
R^{g1} and R^{g2} are independently selected from
   i) H,
   ii) OR^{j},
   iii) NR^{k}R^{l},
   iv) SO₂R^{m},
   v) CONRⁿR^{o},
   vi) NR^{p}COR^{q},
   vii) NR^{r}SO₂R^{s},
   viii) COOR^{t},
   ix) C₁₋₆-alkyl optionally substituted with C₁₋₆ alkoxy
   x) heterocycloalkyl optionally substituted with R^{u}
   xi) cyano, and
   xii) heteroaryl;
R^{j}, R^{k}, R^{l}, R^{m}, Rⁿ, R^{o}, R^{p}, R^{q}, R^{r}, R^{s}, R^{t}, and R^{u} are independently selected from
   i) H,
   ii) C₁₋₆-alkyl optionally substituted with C₁₋₆ alkoxy
   iii) C₃₋₈ cycloalkyl,
   iv) NH₂,
   v) heterocycloalkyl,
   vi) -S(O)₂-C₁₋₆-alkyl,
   vii) -C(O)-C₁₋₆-alkyl, and
   viii) -O-C₁₋₆-alkyl;
R^{h} and Rⁱ are independently selected from
   i) H, and
   ii) C₁₋₆-alkyl;
R⁷ is selected from
   i) H,
   ii) halogen,
   iii) C₁₋₆-alkyl, and
   vi) cyano;
R⁸ is selected from
   i) H,
   ii) halogen,
   iii) cyano
   iv) C₁₋₆-alkyl, and
   v) heteroaryl optionally substituted with C₁₋₆-alkyl;
L is selected from
   i) -L₁-NR⁹-L₂-, and
   ii) -L₁-heterocycloalkylene-L₂-;
L₁ is selected from
   i) (CH₂)x optionally substituted with R^{v},
   ii) heterocycloalkylene optionally substituted with R^{v}, and
   iii) C₃₋₈ cycloalkylene optionally substituted with R^{v};
x represents an integer of 0 to 6;
R⁹ and R¹⁰ are independently selected from
   i) H, and
   ii) C₁₋₆-alkyl optionally substituted with R^{v},
or R¹, R⁹ and L₂ may be bonded to each other to form a 4- to 6- membered ring optionally substituted with R^{d};
R^{v} is selected from
   i) OH,
   ii) NH₂, and
   iii) C₁₋₆-alkyl optionally substituted with hydroxy;
L₂ is selected from
   i) (CH₂)y optionally substituted with R^{w} and/or R^{x},
   ii) C₃₋₈-cycloalkylene optionally substituted with R^{w} and/or R^{x},
   iii) heterocycloalkylene optionally substituted with R^{w} and/or R^{x},
   iv) (CH₂)yNR¹⁰- optionally substituted with R^{w} and/or R^{x},
   v) C₃₋₈-cycloalkylene-NR¹⁰- optionally substituted with R^{w} and/or R^{x}, and
   vi) heterocycloalkylene- NR¹⁰- optionally substituted with R^{w} and/or R^{x}
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
y represents an integer of 0 to 6;
R^{w} and R^{x} are independently selected from
   i) OH,
   ii) NH₂, and
   iii) C₁₋₆-alkyl optionally substituted with hydroxy;
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
or pharmaceutically acceptable salts thereof.

Bacterial infections pose a continuing medical problem because anti-bacterial drugs eventually engender resistance in the bacteria on which they are used. Bacterial resistance against virtually all current antibiotic drugs is increasing. Many forms of antibiotic resistance can even cross international boundaries and spread with remarkable speed. Thus novel classes of antibacterial compounds are urgently needed.

Objects of the present invention are novel compounds of formula (I), their manufacture, medicaments based on a compound in accordance with the invention and their production as well as the use of compounds of formula (I), for the treatment or prophylaxis of bacterial infection, in particular for the treatment of diseases and infections caused by *Escherichia coli.*

The term "C₁₋₆-alkyl" denotes a monovalent linear or branched saturated hydrocarbon group of 1 to 6 carbon atoms. Examples of C₁₋₆-alkyl include methyl, ethyl, propyl, isopropyl, n-butyl, iso-butyl, sec-butyl, tert-butyl and pentyl. Particular C₁₋₆-alkyl groups are methyl, ethyl and n-butyl. More particular example is methyl.

The term "cyano" denotes a -C=N group.

The term "C₁₋₆-alkoxy" denotes a group of the formula -O-R', wherein R' is an C₁₋₆-alkyl group. Examples of C₁₋₆-alkoxy groups include methoxy, ethoxy, n-propoxy, isopropoxy, n-butoxy, isobutoxy and tert-butoxy. Particular example is methoxy.

The term "C₃₋₈-cycloalkyl" denotes a monovalent saturated monocyclic or bicyclic hydrocarbon group of 3 to 8 ring carbon atoms. Bicyclic means a ring system consisting of two saturated carbocycles having one or two carbon atoms in common. Examples of monocyclic C₃₋₈-cycloalkyl are cyclopropyl, cyclobutanyl, cyclopentyl, cyclohexyl or cycloheptyl. Particular monocyclic cycloalkyl groups are C₃₋₆ cycloalkyl, such as cyclopropyl, cyclobutanyl, cyclopentyl and cyclohexyl. More particular monocyclic cycloalkyl group is cyclopropyl. Example of bicyclic C₃₋₈-cycloalkyl is spiro[3.3]heptanyl.

The term "C₃₋₈-cycloalkylene" denotes a divalent saturated monocyclic or bicyclic hydrocarbon group of 3 to 8 ring carbon atoms. Bicyclic means a ring system consisting of two saturated carbocycles having one or two carbon atoms in common. Examples of monocyclic C₃₋₈-cycloalkylene are cyclopropylene, cyclobutanylene, cyclopentylene, cyclohexylene or cycloheptylene. Particular monocyclic cycloalkyl groups are C₃₋₆ cycloalkylene, such as cyclopropylene, cyclobutanylene, cyclopentylene and cyclohexylene. More particular monocyclic cycloalkyl group is cyclopropylene. Example of bicyclic C₃₋₈-cycloalkylene is spiro[3.3]heptanylene.

The term "halogen" denotes fluoro, chloro, bromo or iodo. Particular halogens are chloro and fluoro.

The term "heterocycloalkyl" denotes a monovalent saturated or partly unsaturated mono- or bicyclic ring system of 4 to 9 ring atoms, comprising 1, 2, or 3 ring heteroatoms selected from N, O and S, the remaining ring atoms being carbon which is optionally substituted with oxo. Bicyclic means consisting of two cycles having one or two ring atoms in common. The heterocycloalkyl is preferably a monovalent saturated or partly unsaturated monocyclic ring system of 4 to 6 ring atoms, comprising 1 or 2 ring heteroatoms selected from N, O and S (4- to 6- membered heterocycloalkyl). Examples for monocyclic saturated heterocycloalkyl are 4,5-dihydro-oxazolyl, oxetanyl, azetidinyl, pyrrolidinyl, 2-oxo-pyrrolidin-4-yl, 3-oxo- morpholin-6-yl, tetrahydrofuranyl, tetrahydrothiophenyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are oxabicyclo[2.2.1]heptanyl, oxaspiro[3.3]heptanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl or dihydropyranyl. Heterocyclyl is preferably azetidinyl, morpholinyl, pyrrolidinyl, piperazinyl, oxetanyl, 2-oxopyrrolidin-4-yl, or 3-oxo- morpholin-6-yl. Particular heterocycloalkyl are azetidinyl and pyrrolidinyl.

The term "heterocycloalkylene" denotes a divalent saturated or partly unsaturated mono- or bicyclic ring system of 4 to 9 ring atoms, comprising 1, 2, or 3 ring heteroatoms selected from N, O and S, the remaining ring atoms being carbon which is optionally substituted with oxo. Bicyclic means consisting of two cycles having one or two ring atoms in common. The heterocycloalkylene is preferably a divalent saturated or partly unsaturated monocyclic ring system of 4 to 6 ring atoms (4- to 6- membered heterocycloalkyl), comprising 1 or 2 ring heteroatoms selected from N, O and S. Particular heterocycloalkene is azetidinene.

The term "heteroaryl" denotes a monovalent aromatic mono- or bicyclic ring system of 4 to 9 ring atoms, comprising 1, 2, 3, or 4 ring heteroatoms selected from N, O and S, the remaining ring atoms being carbon which is optionally substituted with oxo. Bicyclic means consisting of two cycles having one or two ring atoms in common. The heteroaryl is preferably a monovalent aromatic ring system of 5 to 6 ring atoms, comprising 1 or 2 ring heteroatoms selected from N, O and S (5- to 6- membered heteroaryl). Example for heteroaryl are imidazole, pyrazole, oxazole, isoxazole, triazole, thiazole, tetrazole, pyridine, pyrimidine, pyrazine, or pyridazine. Particular heteroaryl is thiazole.

The term "4- to 6- membered ring" denotes a monocyclic saturated or unsaturated ring system of 4 to 6 ring atoms. "4- to 6- membered ring" can comprise 1, 2, or 3 ring heteroatoms selected from N, O and S, the remaining ring atoms being carbon which is optionally substituted with oxo. Example for 4- to 6- membered ring are cyclohexane, cyclopentane, cyclobutane, azetidine, pyrrolidine, and piperidine. Particular 4- to 6- membered ring is cyclohexane.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein, lactic acid and the like. In addition, these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are the hydrochloride salts, methanesulfonic acid salts, lactic acid salts and citric acid salts.

The compounds of the present invention may absorb moisture, have absorbed water or form hydrates when left in the air. Such hydrates are also included in the salts of the present invention.

Further, the compounds of the present invention may absorb certain other solvents to form solvates. Such solvates are also encompassed in the present invention as salts of the compounds of the formula (I).

"Pharmaceutically acceptable esters" means that compounds of general formula (I) may be derivatised at functional groups to provide derivatives which are capable of conversion back to the parent compounds in vivo. Examples of such compounds include physiologically acceptable and metabolically labile ester derivatives, such as methoxymethyl esters, methylthiomethyl esters and pivaloyloxymethyl esters. Additionally, any physiologically acceptable equivalents of the compounds of general formula (I), similar to the metabolically labile esters, which are capable of producing the parent compounds of general formula (I) in vivo, are within the scope of this invention.

The term "protecting group" (PG) denotes a group which selectively blocks a reactive site in a multifunctional compound such that a chemical reaction can be carried out selectively at another unprotected reactive site in the meaning conventionally associated with it in synthetic chemistry. Protecting groups can be removed at the appropriate point. Exemplary protecting groups are amino-protecting groups, carboxy-protecting groups or hydroxy-protecting groups. Particular protecting groups are the tert-butoxycarbonyl (Boc), benzyloxycarbonyl (Cbz), fluorenylmethoxycarbonyl (Fmoc) and benzyl (Bn) groups. Further particular protecting groups are the tert-butoxycarbonyl (Boc) and the fluorenylmethoxycarbonyl (Fmoc) groups. More particular protecting group is the tert-butoxycarbonyl (Boc) group.

The abbreviation uM means microMolar and is equivalent to the symbol µM.

The abbreviation uL means microliter and is equivalent to the symbol µL.

The abbreviation ug means microgram and is equivalent to the symbol µg.

The compounds of formula (I) can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention are compounds according to formula (I) as described herein and pharmaceutically acceptable salts or esters thereof, in particular compounds according to formula (I) as described herein and pharmaceutically acceptable salts thereof, more particularly compounds according to formula (I) as described herein.

A embodiment of the present invention are compounds according to formula (I) as described herein, wherein
A¹ is selected from
   i) -N-, and
   ii) -CH-;
A² is selected from
   i) -N-, and
   ii) -CH-;
A³ is selected from
   i) -N-, and
   ii) -CH-;
A⁴ is selected from
   i) -O-, and
   ii) -S-;
A⁵ is selected from
   i) -N-, and
   ii) -CH-;
A⁶ is selected from
   i) -O-, and
   ii) -CH₂-;
-̅ -̅ represents a single or double bond;
B¹ is selected from
   i) -N-, and
   ii) -CR⁵-;
B² is selected from
   i) -N-, and
   ii) -CR⁶-;
wherein when B¹ is CR⁵ and B² is CR⁶, R⁵ and R⁶ may be bonded to each other to form a 4-to 6- membered ring optionally substituted with R^{a} and/or R^{b};
R^{a} and R^{b} are independently selected from
   i) COOR^{a1}, and
   ii) C₁₋₆-alkyl;
R^{a1} is selected from
   i) H, and
   ii) C₁₋₆-alkyl;
B³ is selected from
   i) -N-, and
   ii) -CR⁷-;
B⁴ is selected from
   i) -N-, and
   ii) -CR⁸-;
R¹ is selected from
   i) H,
   ii) NH₂,
   iii) OH, and
   iv) C₁₋₆ alkyl optionally substituted with R^{c},
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d};
R^{c} is selected from
   i) OH, and
   ii) NH₂;
R^{d} is selected from.
   i) (CH2)nNH₂, and
   ii) (CH2)nOH;
n represents an integer of 0 to 6;
R² is selected from
   i) H, and
   ii) C₁₋₆ alkyl optionally substituted with R^{c},
or R², L₂ and R^{w} may be bonded to each other to form a 4- to 6- membered ring optionally substituted with R^{d};
wherein
when -̅ -̅ represents a double bond, R₁ and R₂ are none;
R^{3a} is selected from
   i) H,
   ii) NH₂, and
   iii) OH;
R^{3b} is selected from
   i) H,
   ii) NH₂, and
   iii) OH;
wherein either one of R^{3a} and R^{3b} is H;
R⁴ is selected from
   i) H,
   ii) OH, and
   iii) NH₂;
R⁵ is selected from
   i) H,
   ii) halogen,
   iii) cyano,
   iv) C₁₋₆-alkyl
   v) 4- to 6 -membered heteroaryl optionally substituted with C₁₋₆-alkyl,
   vi) OR^{e},
   vii) SO₂R^{f},
   viii) SO₂NR^{e}R^{f}, and
   ix) NR^{e}R^{f};
R^{e} and R^{f} are independently selected from
   i) H, and
   ii) C₁₋₆-alkyl;
R⁶ is selected from
   i) H,
   ii) halogen,
   iii) C₁₋₆-alkyl,
   iv) OR^{g},
   v) NR^{h}Rⁱ, and
   vi) cyano;
R^{g} is selected from
   i) H,
   ii) -(CH₂)ₘ-R^{g1},
   iii) 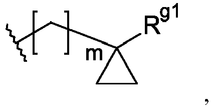
   iv) 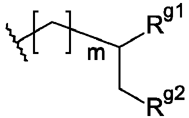
   v) 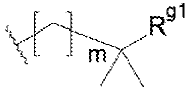 and
   vi) 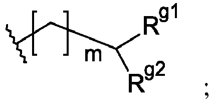
m represents an integer of 0 to 6;
R^{g1} and R^{g2} are independently selected from
   i) H,
   ii) OR^{j},
   iii) NR^{k}R^{l},
   iv) SO₂R^{m},
   v) CONRⁿR^{o},
   vi) NR^{p}COR^{q},
   vii) NR^{r}SO₂R^{s},
   viii) COOR¹,
   ix) C₁₋₆-alkyl optionally substituted with C₁₋₆ alkoxy
   x) heterocycloalkyl optionally substituted with R^{u}
   xi) cyano, and
   xii) heteroaryl;
R^{j}, R^{k}, R^{l}, R^{m}, Rⁿ, R^{o}, R^{p}, R^{q}, R^{r}, R^{s}, R^{t}, and R^{u} are independently selected from
   i) H,
   ii) C₁₋₆-alkyl optionally substituted with C₁₋₆ alkoxy
   iii) C₃₋₈ cycloalkyl,
   iv) NH₂, and
   v) heterocycloalkyl;
R^{h} and Rⁱ are independently selected from
   i) H, and
   ii) C₁₋₆-alkyl;
R⁷ is selected from
   i) H,
   ii) halogen,
   iii) C₁₋₆-alkyl, and
   vi) cyano;
R⁸ is selected from
   i) H,
   ii) halogen,
   iii) cyano
   iv) C₁₋₆-alkyl, and
   v) heteroaryl optionally substituted with C₁₋₆-alkyl;
L is selected from
   i) -L₁-NR⁹-L₂-, and
   ii) -L₁-heterocycloalkylene-L₂-;
L₁ is selected from
   i) (CH₂)x optionally substituted with R^{v},
   ii) heterocycloalkylene optionally substituted with R^{v}, and
   iii) C₃₋₈ cycloalkylene optionally substituted with R^{v};
x represents an integer of 0 to 6;
R⁹ and R¹⁰ are independently selected from
   i) H, and
   ii) C₁₋₆-alkyl optionally substituted with R^{v},
or R¹, R⁹ and L₂ may be bonded to each other to form a 4- to 6- membered ring optionally substituted with R^{d};
R^{v} is selected from
   i) OH, and
   ii) NH₂;
L₂ is selected from
   i) (CH₂)y optionally substituted with R^{w} and/or R^{x},
   ii) C₃₋₈-cycloalkylene optionally substituted with R^{w} and/or R^{x},
   iii) heterocycloalkylene optionally substituted with R^{w} and/or R^{x},
   iv) (CH₂)yNR¹⁰- optionally substituted with R^{w} and/or R^{x},
   v) C₃₋₈-cycloalkylene-NR¹⁰- optionally substituted with R^{w} and/or R^{x}, and
   vi) heterocycloalkylene-NR¹⁰- optionally substituted with R^{w} and/or R^{x}
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
y represents an integer of 0 to 6;
R^{w} and R^{x} are independently selected from
   i) OH,
   ii) NH₂, and
   iii) C₁₋₆-alkyl;
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
or pharmaceutically acceptable salts thereof.

Another embodiment of the present invention are compounds according to formula (I) as described herein, wherein
A¹ is selected from
   i) -N-, and
   ii) -CH-;
A² is selected from
   i) -N-, and
   ii) -CH-;
A³ is CH;
A⁴ is selected from
   i) -O-, and
   ii) -S-;
A⁵ is selected from
   i) -N-, and
   ii) -CH-;
A⁶ is selected from
   i) -O-, and
   ii) -CH₂-;
-̅ -̅ represents a single or double bond;
B¹ is selected from
   i) -N-, and
   ii) -CR⁵-;
B² is selected from
   i) -N-, and
   ii) -CR⁶-;
wherein when B¹ is CR⁵ and B² is CR⁶, R⁵ and R⁶ may be bonded to each other to form a 4-to 6- membered ring as below, 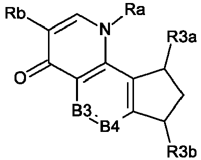
R^{a} and R^{b} are independently selected from
   i) COOR^{a1}, and
   ii) C₁₋₆-alkyl;
R^{a1} is selected from
   i) H, and
   ii) C₁₋₆-alkyl;
B³ is selected from
   i) -N-, and
   ii) -CR⁷-;
B⁴ is selected from
   i) -N-, and
   ii) -CR⁸-;
R¹ is selected from
   i) H, and
   ii) C₁₋₆ alkyl optionally substituted with R^{c},
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d};
R^{c} is NH₂;
R^{d} is (CH2)nNH₂;
n represents an integer of 0 to 3;
R² is H;
or R², L₂ and R^{w} may be bonded to each other to form a 4- to 6- membered ring optionally substituted with R^{d}
wherein
when -̅ -̅ represents a double bond, R₁ and R₂ are none;
R^{3a} is selected from
   i) H,
   ii) NH₂, and
   iii) OH;
R^{3b} is selected from
   i) H,
   ii) NH₂, and
   iii) OH;
wherein either one of R^{3a} and R^{3b} is H,
R⁴ is selected from
   i) H, and
   ii) NH₂;
R⁵ is selected from
   i) H,
   ii) halogen,
   iii) cyano,
   iv) C₁₋₆-alkyl, and,
   v) OR^{e};
R^{e} is C₁₋₆-alkyl;
R⁶ is selected from
   i) H,
   ii) C₁₋₆-alkyl, and
   iii) OR^{g};
R^{g} is selected from
   i) H,
   ii) -(CH₂)ₘ-R^{g1},
   iii) 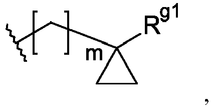
   iv) 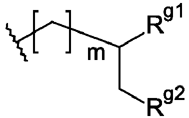
   v) 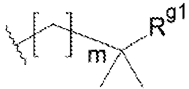 and
   vi) 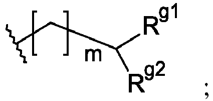
m represents an integer of 0 to 3;
R^{g1} and R^{g2} are independently selected from
   i) H,
   ii) OR^{j},
   iii) NR^{k}R^{l},
   iv) SO₂R^{m},
   v) CONRⁿR^{o},
   vi) NR^{p}COR^{q},
   vii) NR^{r}SO₂R^{s},
   viii) COOR¹,
   ix) C₁₋₆-alkyl optionally substituted with C₁₋₆ alkoxy
   x) heterocycloalkyl optionally substituted with R^{u}, and
   xi) cyano
R^{j}, R^{k}, R^{l}, R^{m}, Rⁿ, R^{o}, R^{p}, R^{q}, R^{r}, R^{s}, R^{t}, and R^{u} are independently selected from
   i) H,
   ii) C₁₋₆-alkyl optionally substituted with C₁₋₆ alkoxy, and,
   iii) NH₂,
R⁷ is selected from
   i) H, and
   ii) C₁₋₆-alkyl;
R⁸ is selected from
   i) H,
   ii) halogen,
   iii) cyano
   iv) C₁₋₆-alkyl, and
   v) heteroaryl optionally substituted with C₁₋₆-alkyl;
L is selected from
   i) -L₁-NR⁹-L₂-, and
   ii) -L₁- azetidinylene -L₂-
L₁ is selected from
   i) (CH₂)x optionally substituted with R^{v}, and,
   ii) heterocycloalkylene optionally substituted with R^{v};
x represents an integer of 0 to 3;
R⁹ and R¹⁰ are H;
or R¹, R⁹ and L₂ may be bonded to each other to form a 4- to 6- membered ring optionally substituted with R^{d};
R^{v} is NH₂;
L₂ is selected from
   i) (CH₂)y optionally substituted with R^{w} and/or R^{x},
   ii) C₃₋₈-cycloalkylene optionally substituted with R^{w} and/or R^{x},
   iii) heterocycloalkylene optionally substituted with R^{w} and/or R^{x},
   iv) (CH₂)yNR¹⁰- optionally substituted with R^{w} and/or R^{x},
   v) C₃₋₈-cycloalkylene-NR¹⁰- optionally substituted with R^{w} and/or R^{x}, and
   vi) heterocycloalkylene- NR¹⁰- optionally substituted with R^{w} and/or R^{x};
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
y represents an integer of 0 to 3;
R^{w} and R^{x} are OH;
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d}:
or pharmaceutically acceptable salts thereof.

Also a particular embodiment of the present invention provides compounds according to formula (I) as described herein, wherein
A¹ is selected from
   i) -N-, and
   ii) -CH-;
A² is selected from
   i) -N-, and
   ii) -CH-;
A³ is CH;
A⁴ is selected from
   i) -O-, and
   ii) -S-;
A⁵ is CH;
A⁶ is O;
-̅ -̅ is a single bond,
B¹ is selected from
   i) -N-, and
   ii) -CR⁵-;
B² is selected from
   i) -N-, and
   ii) -CR⁶-;
B³ is selected from
   i) -N-, and
   ii) -CR⁷-;
B⁴ is selected from
   i) -N-, and
   ii) -CR⁸-;
R¹ is H;
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d};
R^{d} is (CH2)nNH₂, and
n represents an integer of 0 to 3;
R² is H;
or R², L₂ and R^{w} may be bonded to each other to form a 4- to 6- membered ring optionally substituted with R^{d}
R^{3a} is selected from
   i) H,
   ii) NH₂, and
   iii) OH;
R^{3b} is selected from
   i) H,
   ii) NH₂, and
   iii) OH;
wherein either one of R^{3a} and R^{3b} is H,
R⁴ is H;
R⁵ is selected from
   i) H,
   ii) halogen,
   iii) cyano,
   iv) C₁₋₆-alkyl,
   v) OR^{e}, and
R^{e} is C₁₋₆-alkyl;
R⁶ is selected from
   i) H,
   ii) C₁₋₆-alkyl, and
   iii) OR^{g};
R^{g} is selected from
   i) H,
   ii) -(CH₂)ₘ-R^{g1},
   iii) 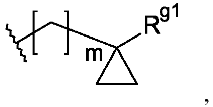
   iv) 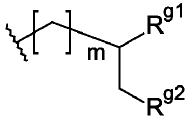
   v) 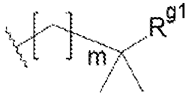 and
   vi) 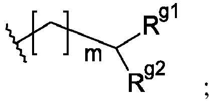
m represents an integer of 0 to 3;
R^{g1} and R^{g2} are independently selected from
   i) H,
   ii) OR^{j},
   iii) NR^{k}R^{l},
   iv) C₁₋₆-alkyl optionally substituted with C₁₋₆ alkoxy, and
   v) heterocycloalkyl optionally substituted with R^{u};
R^{j}, R^{k}, R^{l}, and R^{u} are independently selected from
   i) H,
   ii) C₁₋₆-alkyl optionally substituted with C₁₋₆ alkoxy, and,
   iii) NH₂,
R⁷ is selected from
   i) H, and
   ii) C₁₋₆-alkyl;
R⁸ is selected from
   i) H,
   ii) halogen,
   iii) cyano,
   iv) C₁₋₆-alkyl, and
   v) heteroaryl optionally substituted with C₁₋₆-alkyl;
L is -L₁-NR⁹-L₂-;
L₁ is (CH₂)x optionally substituted with R^{v};
x represents an integer of 0 to 3;
R⁹ and R¹⁰ are H
or R¹, R⁹ and L₂ may be bonded to each other to form a 4- to 6- membered ring optionally substituted with R^{d};
R^{v} is NH₂
L₂ is selected from
   i) (CH₂)y optionally substituted with R^{w} and/or R^{x},
   ii) C₃₋₈-cycloalkylene optionally substituted with R^{w} and/or R^{x},
   iii) heterocycloalkylene optionally substituted with R^{w} and/or R^{x},
   iv) (CH₂)yNR¹⁰- optionally substituted with R^{w} and/or R^{x},
   v) C₃₋₈-cycloalkylene-NR¹⁰- optionally substituted with R^{w} and/or R^{x}, and
   vi) heterocycloalkylene- NR¹⁰- optionally substituted with R^{w} and/or R^{x};
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
y represents an integer of 0 to 3;
R^{w} and R^{x} are OH
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d}:
or pharmaceutically acceptable salts thereof.

Also a particular embodiment of the present invention provides compounds according to formula (I) as described herein, wherein
A¹ is selected from
   i) -N-, and
   ii) -CH-;
A² is selected from
   i) -N-, and
   ii) -CH-;
A³ is CH;
A⁴ is selected from
   i) -O-, and
   ii) -S-;
A⁵ is CH;
A⁶ is O;
-̅ -̅ is a single bond;
B¹ is selected from
   i) -N-, and
   ii) -CR⁵-;
B² is selected from
   i) -N-, and
   ii) -CR⁶-;
B³ is selected from
   i) -N-, and
   ii) -CR⁷-;
B⁴ is selected from
   i) -N-, and
   ii) -CR⁸-;
R¹ is H;
or R¹, R⁹ and L₂ may be bonded to each other to form a 4- to 6- membered ring as below, 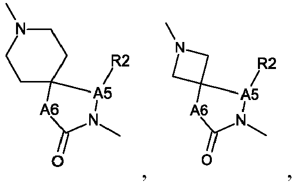
wherein the ring is optionally substituted with R^{d},
or R¹, L₂ and R^{w} may be bonded to each other to form a 4- to 6- membered ring as below, 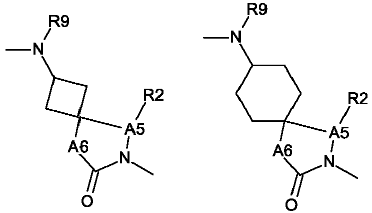
wherein the ring is optionally substituted with R^{d};
R^{d} is (CH2)nNH₂;
n represents an integer of 0 to 1;
R² is H;
or R², L₂ and R^{w} may be bonded to each other to form a 4- to 6- membered ring as below, 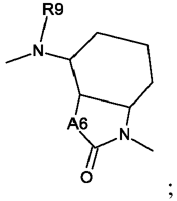
R^{3a} is selected from
   i) H,
   ii) NH₂, and
   iii) OH;
R^{3b} is selected from
   i) H,
   ii) NH₂, and
   iii) OH;
wherein either one of R^{3a} and R^{3b} is H,
R⁴ is H
R⁵ is selected from
   i) H,
   ii) halogen,
   iii) cyano,
   iv) C₁₋₆-alkyl, and,
   v) OR^{e}, and
R^{e} is C₁₋₆-alkyl;
R⁶ is selected from
   i) H,
   ii) C₁₋₆-alkyl, and
   iii) OR^{g};
R^{g} is selected from
   i) H,
   ii) -(CH₂)ₘ-R^{g1},
   iii) 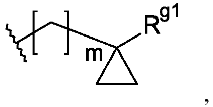
   iv) 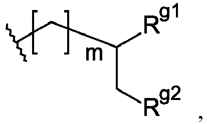
   v) 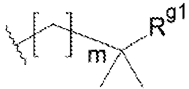 and
   vi) 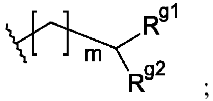
m represents an integer of 0 to 3;
R^{g1} and R^{g2} are independently selected from
   i) H,
   ii) OR^{j},
   iii) NR^{k}R^{l},
   iv) C₁₋₆-alkyl optionally substituted with C₁₋₆ alkoxy
   v) pyrrolidinyl optionally substituted with R^{u}
   vi) morpholinyl optionally substituted with R^{u}
   vii) oxetanyl optionally substituted with R^{u}
   viii) 3-oxo-morpholinyl optionally substituted with R^{u},
   ix) 2-oxo-pyrrolidinyl optionally substituted with R^{u}
   x) azetidinyl optionally substituted with R^{u}
   xi) piperazinyl optionally substituted with R^{u}, and
   xiI) 2-oxo-piperazinyl optionally substituted with R^{u};
R^{j}, R^{k}, R^{l}, and R^{u} are independently selected from
   i) H,
   ii) C₁₋₆-alkyl optionally substituted with C₁₋₆ alkoxy, and
   iii) NH₂
R⁷ is selected from
   i) H, and
   ii) C₁₋₆-alkyl;
R⁸ is selected from
   i) H,
   ii) halogen,
   iii) cyano
   iv) C₁₋₆-alkyl, and
   v) thiazole optionally substituted with C₁₋₆-alkyl;
L is -L₁-NR⁹-L₂-;
L₁ is (CH₂)x optionally substituted with R^{v};
x represents an integer of 0 to 3;
R⁹ and R¹⁰ are H;
or R¹, R⁹ and L₂ may be bonded to each other to form a 4- to 6- membered ring as below, 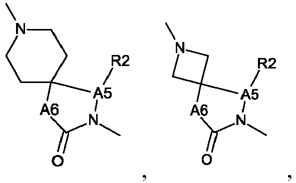
wherein the ring is optionally substituted with R^{d};
R^{v} is NH₂;
L₂ is selected from
   i) (CH₂)y optionally substituted with R^{w} and/or R^{x},
   ii) C₃₋₈-cycloalkylene optionally substituted with R^{w} and/or R^{x},
   iii) (CH₂)yNR¹⁰- optionally substituted with R^{w} and/or R^{x}, and
   iv) C₃₋₈-cycloalkylene-NR¹⁰- optionally substituted with R^{w} and/or R^{x}
or R¹, R⁹ and L₂ may be bonded to each other to form a 4- to 6- membered ring as below, 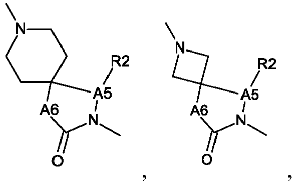
wherein the ring is optionally substituted with R^{d},
or R¹, L₂ and R^{w} may be bonded to each other to form a 4- to 6- membered ring as below, 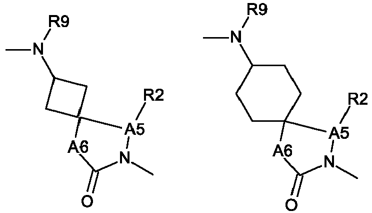
wherein the ring is optionally substituted with R^{d},
or R², L₂ and R^{w} may be bonded to each other to form a 4- to 6- membered ring as below, 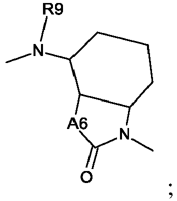
y represents an integer of 0 to 3;
R^{w} and R^{x} are OH
or R¹, L₂ and R^{w} may be bonded to each other to form a 4- to 6- membered ring as below, 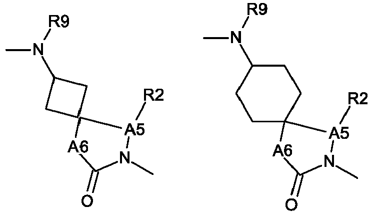
wherein the ring is optionally substituted with R^{d},
or R², L₂ and R^{w} may be bonded to each other to form a 4- to 6- membered ring as below, 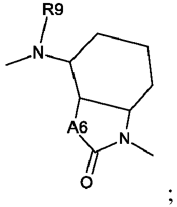
or pharmaceutically acceptable salts thereof.

A particular embodiment of the present invention provides compounds according to formula (I) as described herein, wherein A₁ is -N-.

A particular embodiment of the present invention provides compounds according to formula (I) as described herein, wherein A² is -CH-.

A particular embodiment of the present invention provides compounds according to formula (I) as described herein, wherein A³ is -CH-.

A particular embodiment of the present invention provides compounds according to formula (I) as described herein, wherein A⁴ is -O-.

A particular embodiment of the present invention provides compounds according to formula (I) as described herein, wherein A⁵ is -CH-.

A particular embodiment of the present invention provides compounds according to formula (I) as described herein, wherein A⁶ is -O-.

A particular embodiment of the present invention provides compounds according to formula (I) as described herein, wherein R^{3a} and R^{3b} are H.

A particular embodiment of the present invention provides compounds according to formula (I) as described herein, wherein R⁴ is H.

A particular embodiment of the present invention provides compounds according to formula (I) as described herein, wherein R¹ is H and R² is H.

A particular embodiment of the present invention provides compounds according to formula (I) as described herein, wherein -̅ -̅ is a single bond.

The above R⁵ is preferably selected from:
i) H,
ii) halogen,
iii) cyano,
iv) C₁₋₆-alkyl, and,
v) ORe;

The above R⁶ is preferably selected from,
i) H,
ii) C₁₋₆-alkyl, and
iii) OR^{g};

In case R⁵ and R⁶ may be bonded to each other to form a 4- to 6- membered ring, the ring is preferably 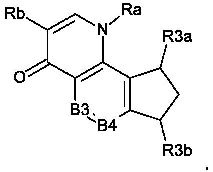
The above R⁷ is preferably selected from,
   i) H, and
   ii) C₁₋₆-alkyl;
The above R⁸ is preferably selected from,
   i) H,
   ii) halogen,
   iii) cyano
   iv) C₁₋₆-alkyl, and
   v) heteroaryl optionally substituted with C₁₋₆-alkyl;
More preferably, the above R⁸ is selected from,
   i) H,
   ii) halogen,
   iii) cyano
   iv) C₁₋₆-alkyl, and
   v) thiazole optionally substituted with C₁₋₆-alkyl;
L is preferably selected from
   i) -L₁-NR⁹-L₂-, and
   ii) -L₁- azetidinylene -L₂-
More preferably, L is -L₁-NR⁹-L₂-
The above L₁ is selected from
   i) (CH₂)x optionally substituted with R^{v}, and,
   ii) heterocycloalkylene optionally substituted with R^{v};
More preferably, L₁ is (CH₂)x optionally substituted with R^{v}
The above x preferably represents an integer of 0 to 3,
More preferably, x represents an integer of 0, 1, or 2.
The above L₂ is preferably selected from
   i) (CH₂)y optionally substituted with R^{w} and/or R^{x},
   ii) C₃₋₈-cycloalkylene optionally substituted with R^{w} and/or R^{x},
   iii) (CH₂)yNR¹⁰- optionally substituted with R^{w} and/or R^{x}, and
   iv) C₃₋₈-cycloalkylene-NR¹⁰- optionally substituted with R^{w} and/or R^{x}
More preferably, L² is selected from
   i) (CH₂)y optionally substituted with R^{w} and/or R^{x}, and
   ii) (CH₂)yNR¹⁰- optionally substituted with R^{w} and/or R^{x}.
The abaove y preferably represents an integer of 0, 1, or 2.
The above R⁹ is preferably H.
The above R¹⁰ is preferably H.
The above R^{d} is preferably (CH2)nNH₂.
The above n preferably represents an integer of 0,1, or 2.
The above R^{e} is preferably C₁₋₆-alkyl.
The above m preferably represents an integer of 0,1, or 2.
The above R^{g1} and R^{g2} are preferably independently selected from
   i) H,
   ii) OR^{j},
   iii) NR^{k}R^{l},
   iv) SO₂R^{m},
   v) CONRⁿR^{o},
   vi) NR^{p}COR^{q},
   vii) NR^{r}SO₂R^{s},
   viii) COOR^{t},
   ix) C₁₋₆-alkyl optionally substituted with C₁₋₆ alkoxy
   x) heterocycloalkyl optionally substituted with R^{u}, and
   xi) cyano
More preferably, R^{g1} and R^{g2} are independently selected from,
   i) H,
   ii) OR^{j},
   iii) NR^{k}R^{l},
   iv) C₁₋₆-alkyl optionally substituted with C₁₋₆ alkoxy
   v) pyrrolidinyl optionally substituted with R^{u}
   vi) morpholinyl optionally substituted with R^{u}
   vii) oxetanyl optionally substituted with R^{u}
   viii) 3-oxo-morpholinyl optionally substituted with R^{u},
   ix) 2-oxo-pyrrolidinyl optionally substituted with R^{u}
   x) azetidinyl optionally substituted with R^{u}
   xi) piperazinyl optionally substituted with R^{u}, and
   xiI) 2-oxo-piperazinyl optionally substituted with R^{u};
R^{j}, R^{k}, R^{l}, R^{m}, Rⁿ, R^{o}, R^{p}, R^{q}, R^{r}, R^{s}, R^{t}, and R^{u} are preferably independently selected from
   i) H,
   ii) C₁₋₆-alkyl optionally substituted with C₁₋₆ alkoxy, and,
   iii) NH₂;

In case R¹, R⁹ and L₂ may be bonded to each other to form a 4- to 6- membered ring, the ring is selected from, 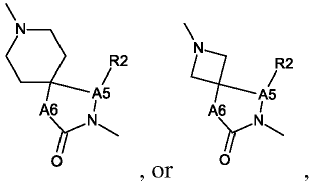 wherein the ring is optionally substituted with R^{d}.

In case R¹, L₂ and R^{w} may be bonded to each other to form a 4- to 6- membered ring, the ring is selected from, 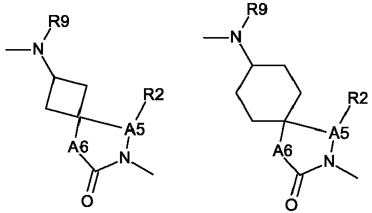 wherein the ring is optionally substituted with R^{d},

In case R², L₂ and R^{w} may be bonded to each other to form a 4- to 6- membered ring, the ring is selected from,

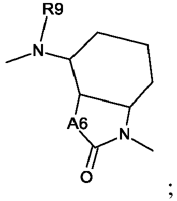

Processes for the manufacture of compounds of formula (I) as described herein are also an object of the invention.

The preparation of compounds of formula (I) of the present invention may be carried out in sequential or convergent synthetic routes. Syntheses of the invention are shown in the following general schemes. The skills required for carrying out the reactions and purifications of the resulting products are known to those skilled in the art. The substituents and indices used in the following description of the processes have the significance given herein before unless indicated to the contrary.

In more detail, the compounds of formula (I) can be manufactured by the methods given below, by the methods given in the examples or by analogous methods. Appropriate reaction conditions for the individual reaction steps are known to a person skilled in the art. The reaction sequence is not limited to the ones displayed in schemes 1 - 11, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The present compounds of formula (I), wherein L₁ is (CH₂)x and x is 1 to 6, and their pharmaceutically acceptable salts can be prepared by a process described below (Scheme 1).

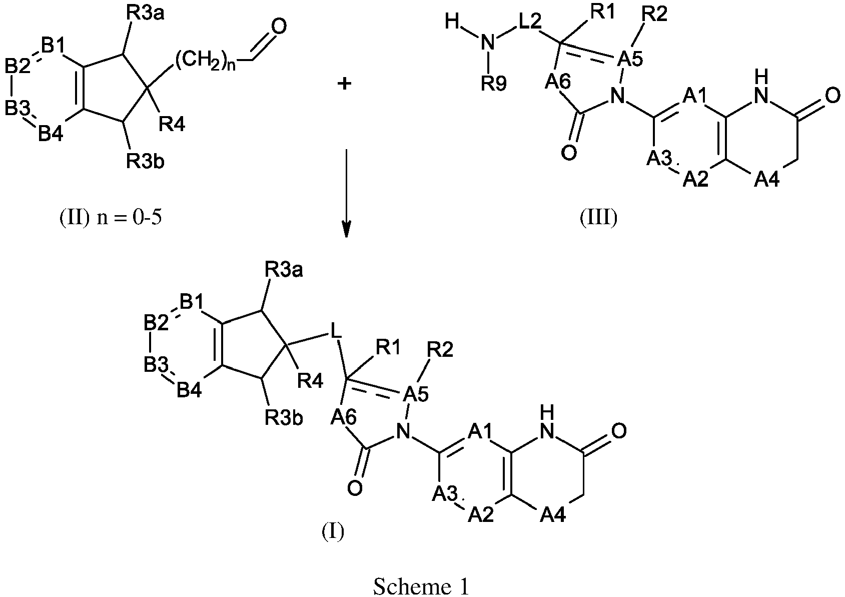

Reductive amination of aldehydes of formula (II) with amines of formula (III) affords compounds of formula (I) wherein L₁ is (CH₂)x and x is 1 to 6. Typical conditions include sodium triacetoxyborohydride or sodium cyanoborohydride, optionally in the presence of additives such as *N*,*N*-diisopropylethylamine or triethlyamine or acetic acid or powdered molecular sieves in a solvent such as methanol, THF or 1,2-dichloroethane at a temperature such as room temperature.

Alternatively, compounds of formula (I) may be prepared as illustrated in scheme 2.

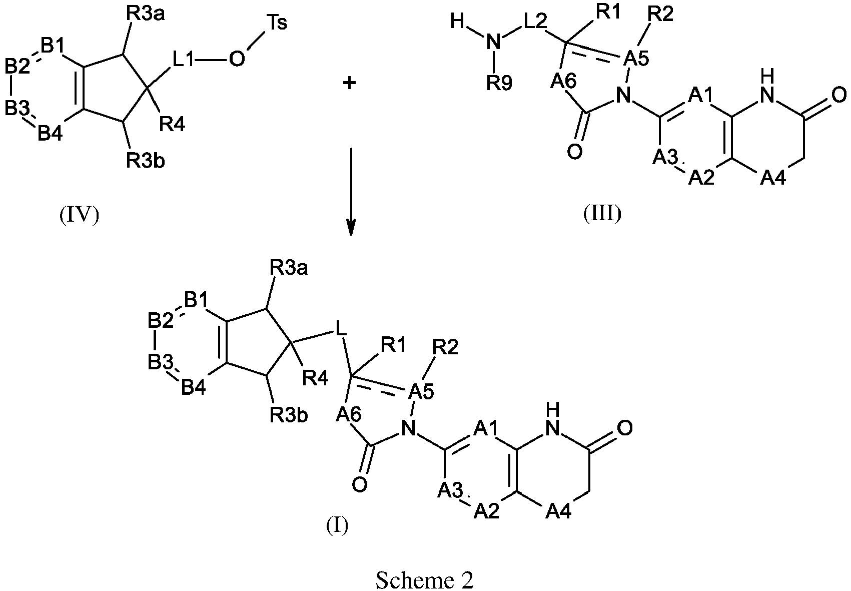

Reaction of tosylates of formula (IV) with amines of formula (III) affords compounds of formula (I). Typical conditions include a base such as potassium carbonate or caesium carbonate in a solvent such as DMF, DMA or NMP at an elevated temperature such as 80 °C.

Alternatively, compounds of formula (I), wherein R₄ is H, L₁ is (CH₂)x and x is 0, may be prepared as illustrated in scheme 3.

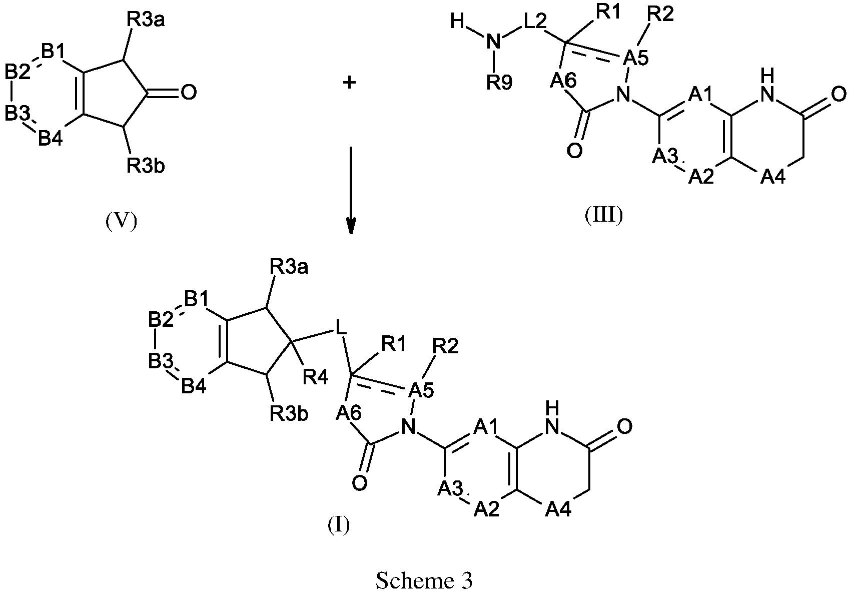

Reductive amination of ketones of formula (V) with amines of formula (III) affords compounds of formula (I), wherein R₄ is H, L₁ is (CH₂)x and x is 0. Typical conditions include sodium triacetoxyborohydride or sodium cyanoborohydride, optionally in the presence of additives such as *N*,*N*-diisopropylethylamine or triethlyamine or acetic acid or powdered molecular sieves in a solvent such as methanol, THF or 1,2-dichloroethane at a temperature such as room temperature.

Alternatively, compounds of formula (I), wherein L₂ is (CH₂)y and y is 1 to 6 may be prepared as illustrated in scheme 4.

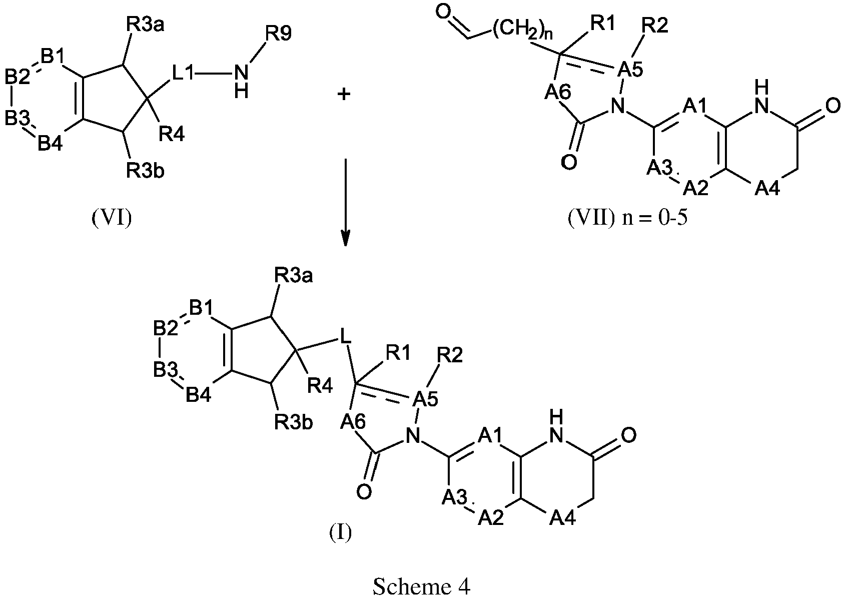

Reductive amination of aldehyes of formula (VII) with amines of formula (VI) affords compounds of formula (I), wherein wherein L₂ is (CH₂)y and y is 1 to 6. Typical conditions include sodium cyanoborohydride, optionally in the presence of additives such as acetic acid or N,N-diisopropylethylamine or triethlyamine and sodium sulfate or powdered molecular sieves in a solvent such as methanol or 1,2-dichloroethane, or a mixture of methanol and 1,2-dichloroethane at a temperature such as room temperature.

Intermediates of formula (II) may be prepared as illustrated in scheme 5.

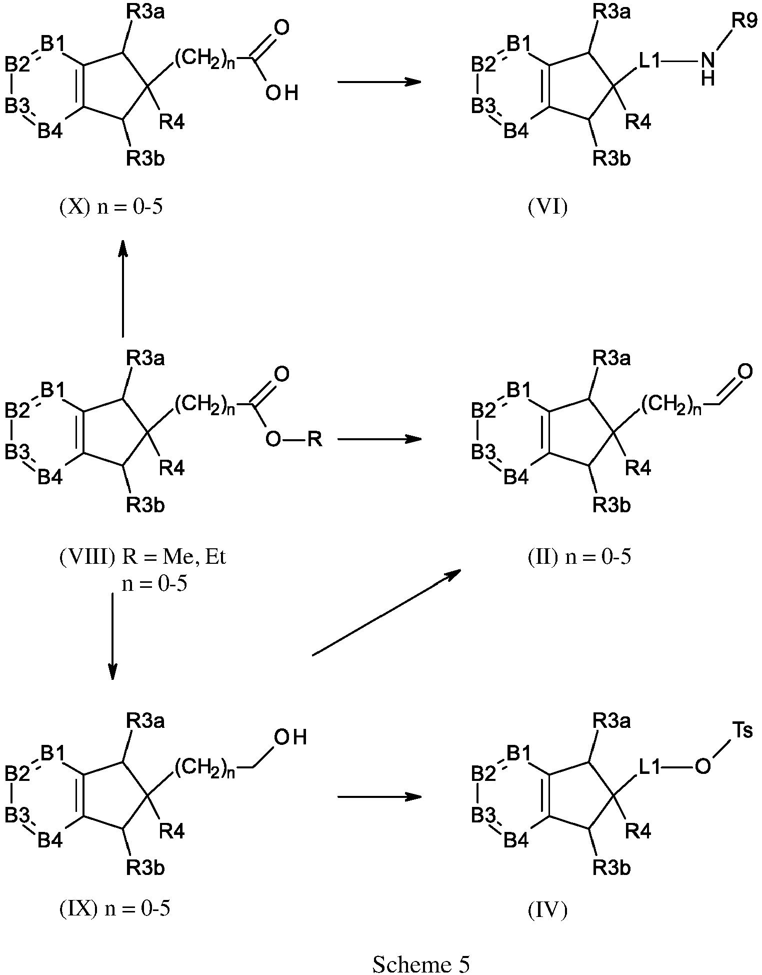

Reduction of esters of formula (VIII) affords alcohols of formula (IX). Typical conditions include a reducing agent such as lithium aluminium hydride in a solvent such as THF at a temperature such as between -78 °C and room temperature. Oxidation of alcohols of formula (IX) affords aldehydes of formula (II). Typical conditions include Dess-Martin periodiane in a solvent such as DCM at a temperature such as 0 to 25 °C. Alternatively, reduction of esters of formula (VIII) affords aldehydes of formula (II). Typical conditions include a reducing agent such as lithium aluminium hydride in a solvent such as THF at a reduced temperature such as between -78 °C and -20 °C.

Intermediates of formula (IV), wherein L₁ is (CH₂)x and x is 1 to 6, may be prepared as illustrated in scheme 5. Toslyation of alcohols of formula (IX) affords intermediates of formula (IV), wherein L₁ is (CH₂)x and x is 1 to 6. Typical conditions include p-toluenesulfonyl chloride, N,N-diisopropylethylamine and DMAP in a solvent such as DCM at a temperature such as room temperature.

Intermediates of formula (VI), wherein L₁ is (CH₂)x and x is 1 to 6, may be prepared as illustrated in scheme 5. Hydrolysis of esters of formula (VIII) affords acids of formula (X). Typical conditions include lithium hydroxide in water and a solvent such as THF at a temperature such as between 0 °C and 25 °C. Acids of formula (X) may be converted to intermediates of formula (VI), wherein L₁ is (CH₂)x and x is 1 to 6, in a two step procedure. Typical conditions for the first step include diphenylphosphoryl azide, a base such as triethylamine and an alcohol such as (4-methoxyphenyl)methanol or 2-(trimethylsilyl)ethanol in a solvent such as toluene at an elevated temperature such as 100 °C. Typical conditions for the second step include trifluoroacetic acid, optionally in a solvent such as DCM, at room temperature.

Esters of formula (VIII), wherein R₃ₐ, R_{3b} and R₄ are H, may be prepared as illustrated in scheme 6.

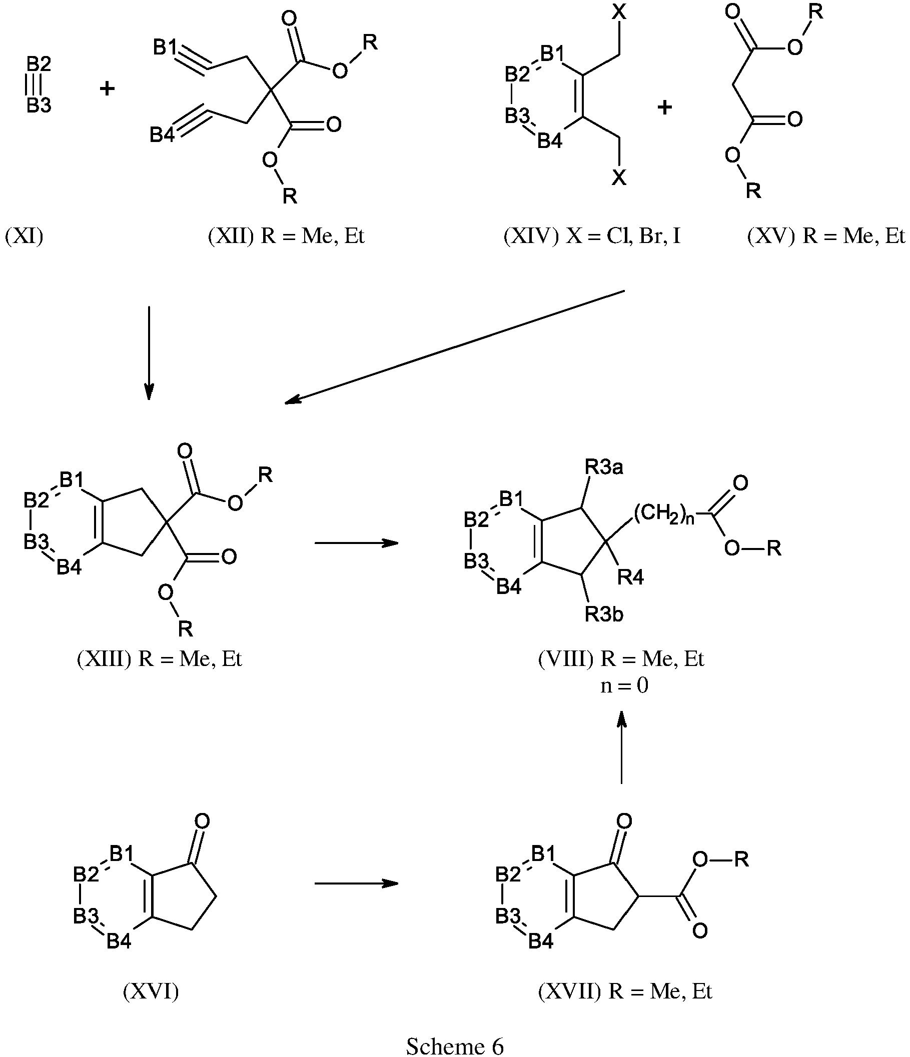

Cycloaddition of an alkyne or isocyanate of formula (XI) with a diyne or alkyne-nitrile of formula (XII) affords compounds of formula (XIII). Typical conditions include use of a catalyst mixture such as chloro(1,5-cyclooctadiene)iridium(I) dimer and 1,1'-bis(diphenylphosphino)ferrocene in a solvent such as benzene at an elevated temperature or a catalyst such as chloro(1,5-cyclooctadiene(pentamethylcyclopentadienyl)ruthenium(II) in a solvent such as 1,2-dichloroethane at an elevated temperature such as 90 °C.

Alternatively, reaction of a dihalo compound of formula (XIV) with a malonate diester of formula (XV) affords diesters of formula (XIII). Typical conditions include the use of a base such as sodium hydride in a solvent such as THF at a temperature such as 0 °C.

Diesters of formula (XIII) may be converted to esters of formula (VIII) wherein R₃ₐ, R_{3b} and R₄ are H. Typical conditions include use of a salt such as lithium chloride in DMSO and water at an elevated temperature such as 160 °C.

Alternatively, diesters of formula (XIII) may be treated with an acid such as HCl in water at a temperature such as 100 °C followed by reaction with an acid such as sulphuric acid in a solvent such as methanol or ethanol at a temperature such as 80 °C to afford esters of formula (VIII) wherein R₃ₐ, R_{3b} and R₄ are H.

Alternatively, ketones of formula (XVI) may be converted into keto-esters of formula (XVII). Typical conditions include reaction with diethyl carbonate or dimethyl carbonate in the presence of a base such as sodium hydride in a solvent such as toluene at a temperature such as 110 °C. Keto-esters of formula (XVII) may be reduced to esters of formula (VIII) wherein R₃ₐ, R_{3b} and R₄ are H. Typical conditions include triethylsilane and trifluoroacetic acid at a temperature such as between 0 °C and 25 °C.

Esters of formula (VIII), wherein R₃ₐ is protected NH₂ and R_{3b} and R₄ are H, may be prepared as illustrated in scheme 7.

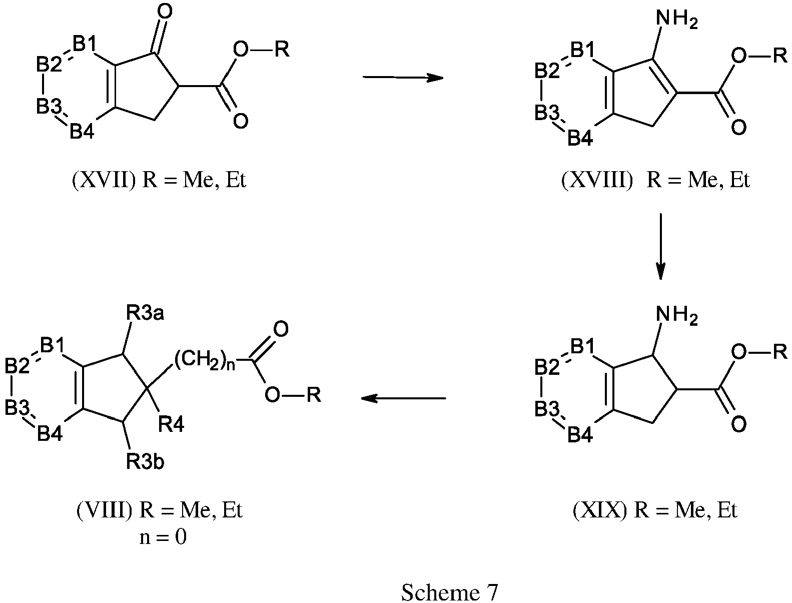

Keto-esters of formula (XVII) may be converted into compounds of formula (XVIII). Typical conditions include a reagent such as ammonium acetate in a solvent such as ethanol at a temperature such as 80 °C. Compounds of formula (XVIII) may be reduced to amino-esters of formula (XIX). Typical conditions include a reducing agent such as sodium borohydride in trifluoroacetic acid at a temperature such as 0 °C. Amino-esters of formula (XIX) may be converted to esters of formula (VIII), wherein R₃ₐ is protected NH₂ and R_{3b} and R₄ are H. Typical protecting groups include tert-butyloxycarbonyl. Typical conditions include a reagent such as di-tert-butyldicarbonate in a solvent such as dichloromethane at a temperature such as 25 °C.

Esters of formula (VIII), wherein R_{3b} is protected NH₂ and R₃ₐ and R₄ are H, may be prepared by an analogous method to that illustrated in scheme 7 starting from the appropriate regioisomer of the keto-esters of formula (XVII).

Esters of formula (VIII), wherein R₃ₐ is protected OH and R_{3b} and R₄ are H may be prepared as illustrated in scheme 8.

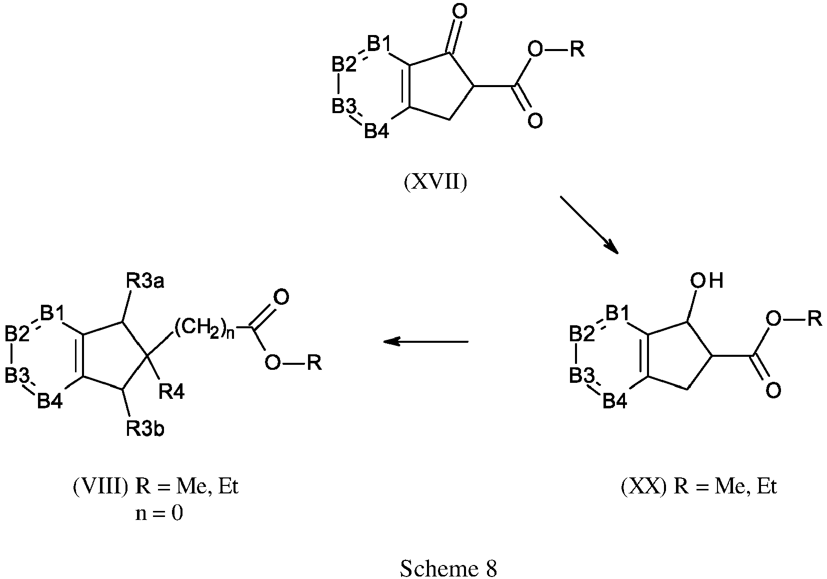

Reduction of ketones of formula (XVII) affords alcohols of formula (XX). Typical conditions include a reducing agent such as sodium borohydride in a solvent such as ethanol at a temperature such as 0 °C. Alcohols of formula (XX) may be converted to esters of formula (VIII), wherein R₃ₐ is protected OH and R_{3b} and R₄ are H. Typical protecting groups include *tert-*butyldimethylsilyl. Typical conditions include tert-butyldimethylchlorosilane with a base such as imidazole in a solvent such as DCM at a temperature such as between 0 °C and 25 °C.

Esters of formula (VIII), wherein R_{3b} is protected OH and R₃ₐ and R₄ are H, may be prepared by an analogous method to that illustrated in scheme 8 starting from the appropriate regioisomer of the keto-esters of formula (XVII).

Intermediates of formula (VII) may be prepared as illustrated in scheme 9.

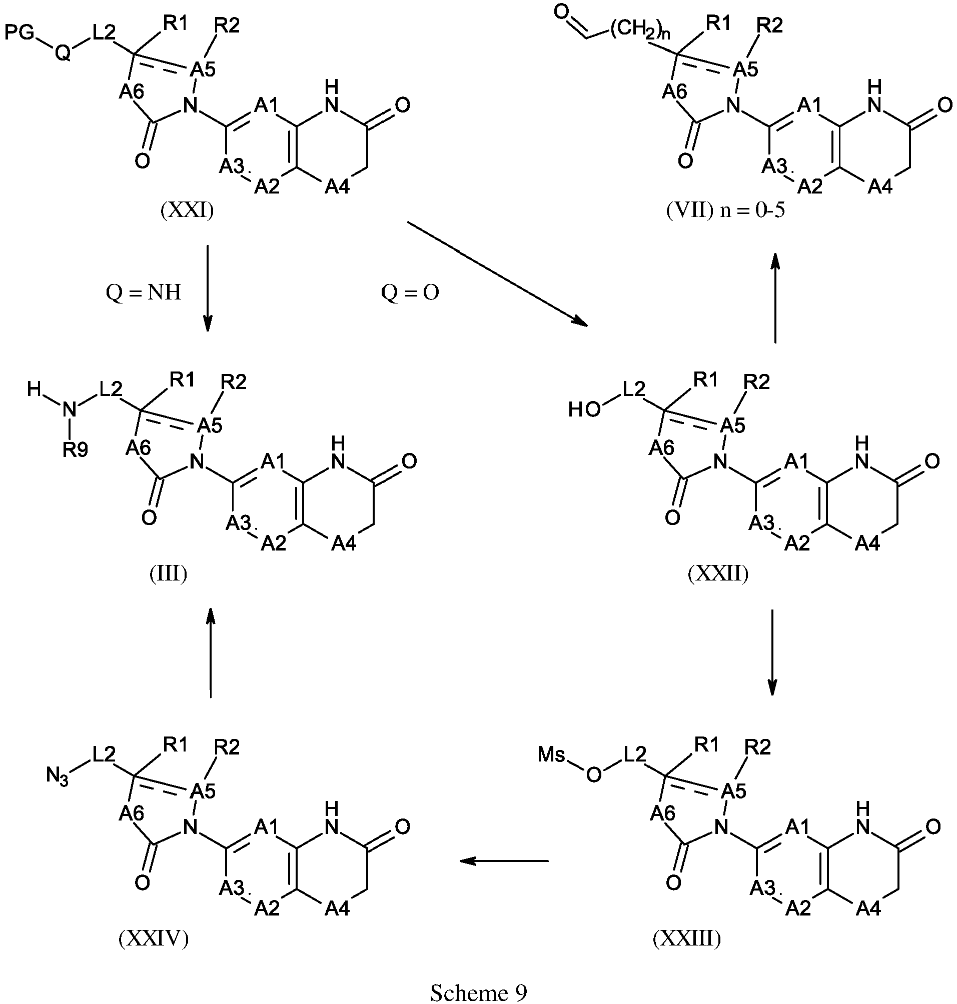

Protected alcohols of formula (XXI) wherein Q is O may be converted to alcohols of formula (XXII). Typical protecting groups include *tert*-butyldimethylsilyl and *tert-*butyldiphenylsilyl. Typical conditions include an acid such as hydrochloric acid in a solvent such as methanol at a temperature such as 20 °C, or a reagent such as TBAF in a solvent such as THF at a temperature such as 20 °C. Alcohols of formula (XXII) wherein L₂ is (CH₂)y and y is 1 to 6 may be oxidized to intermediates of formula (VII). Typical conditions include Dess-Martin periodiane in a solvent such as DCM at a temperature such as 0 to 25 °C.

Intermediates of formula (III), wherein L₂ is (CH₂)y and y is 1 to 6, may be prepared as illustrated in scheme 9. Alcohols of formua (XXII) may be converted to mesylates of formula (XXIII). Typical conditions include methanesulfonyl choride in the presence of a base such as trimethylamine in a solvent such as DCM at a temperature such as between 0 °C and 25 °C. Mesylates of formula (XXIII) may be converted to azides of formula (XXIV). Typical conditions include sodium azide in a solvent such as DMF at an elevated temperature such as 60 °C. Azides of formula (XXIV) may be converted to intermediates of formula (III), wherein L₂ is (CH₂)y and y is 1 to 6. Typical conditions include hydrogenation using a catalyst such as palladium on carbon in a solvent such as methanol at room temperature. Alternative conditions include trimethylphosphine in a solvent such as THF followed by addition of water at a temperature such as 20 °C.

Alternatively, intermediates of formula (III) may be prepared from protected amines of formula (XXI) wherein Q is NH as illustrated in scheme 9. Typical conditions when the protecting group is tert-butyloxycarbonyl include an acid such as hydrochloric acid in a solvent such as methanol or ethyl acetrate or trifluroacetic acid in a solvent such as DCM at a temperature such as 25 °C. Typical conditions when the protecting group is benzyloxycarbonyl or diphenylmethyl or bis[(4-methoxyphenyl)methyl include hydrogenation at a pressure such as between atmospheric pressue and 50 psi using a catalyst such as palladium on carbon or palladium hydroxide on carbon in a solvent such as methanol or THF at a temperature such as 25 °C, optionally in the presence of an acid such as trifluoroacetic acid or hydrochloric acid.

Intermediates of formula (XXI) may be prepared as illustrated in scheme 10.

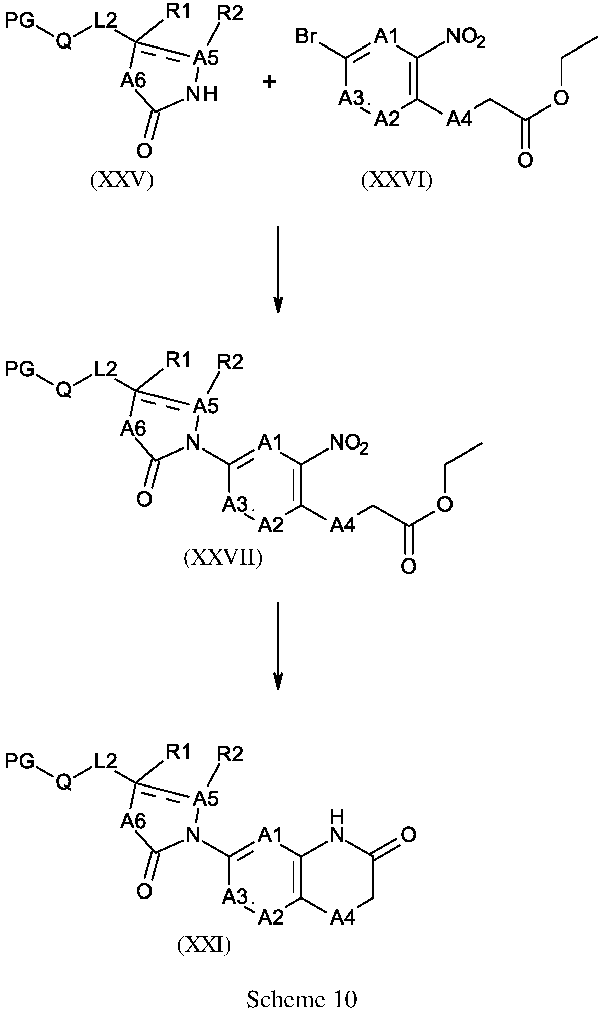

Coupling of compounds of formula (XXV) with heteroaryl bromides of formula (XXVI) affords compounds of formula (XXVII). Typical conditions include the use of a catalytic quantity of copper (I) iodide and *N,N*-dimethyl-ethylenediamine or of tris(dibenzylideneacetone)dipalladium (0) and 9,9-dimethyl-4,5-bis(diphenylphosphino)xanthene in the presence of a base such as potassium carbonate in a solvent such as 1,4-dioxane or toluene at a temperature such as 100 °C. Reduction of compounds of formula (XXVII) affords intermediates of formula (XXI). Typical conditions include iron in acetic acid at a temperature such as between 50 °C and 70 °C, or iron and hydrochloric acid in a solvent such as ethanol at a temperature such as 25 °C.

Alternatively, intermediates of formula (XXI) may be prepared as illustrated in scheme 11.

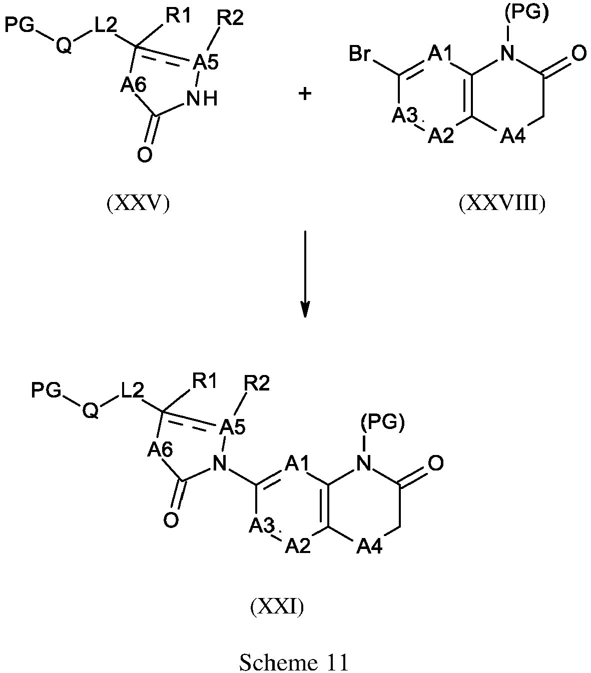

Coupling of compounds of formula (XXV) with heteroaryl bromides of formula (XXVIII), optionally protected, affords intermediates of formula (XXI) optionally protected. Typical conditions include the use of a catalytic quantity of copper (I) iodide and *trans-N,N'-*dimethylcyclohexane-1,2-diamine or of tris(dibenzylideneacetone)dipalladium (0) and 9,9-dimethyl-4,5-bis(diphenylphosphino)xanthene in the presence of a base such as potassium carbonate in a solvent such as 1,4-dioxane at a temperature such as 100 °C. Typical optional protecting groups in heteroaryl bromides of formula (XXVIII) include 2-trimethylsilylethoxymethyl.

Particular examples of compounds of formula (I) as described herein are selected from
6-[5-[2-[(4-chloro-2,3-dihydro-1H-inden-2-yl)methylamino]ethyl]-2-oxo-1,3-oxazolidin-3-yl]-4H-pyrido[3,2-b][1,4]oxazin-3-one; formic acid
6-[5-[3-[(4-chloro-2,3-dihydro-1H-inden-2-yl)methylamino]propyl]-2-oxo-1,3-oxazolidin-3-yl]-4H-pyrido[3,2-b][1,4]oxazin-3-one
6-[5-[2-[(4-chloro-2,3-dihydro-1H-inden-2-yl)amino]ethyl]-2-oxo-1,3-oxazolidin-3-yl]-4H-pyrido[3,2-b][1,4]oxazin-3-onehydrochloride
6-[5-[3-[(4-chloro-2,3-dihydro-1H-inden-2-yl)amino]propyl]-2-oxo-1,3-oxazolidin-3-yl]-4H-pyrido[3,2-b][1,4]oxazin-3-one
2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile
6-[5-[2-[(4-Chloro-2,3-dihydro-1H-inden-2-yl)methylamino]ethyl]-2-oxo-1,3-oxazolidin-3-yl]-4H-pyrido[3,2-b][1,4]oxazin-3-one, Isomer A
6-[5-[2-[(4-Chloro-2,3-dihydro-1H-inden-2-yl)methylamino]ethyl]-2-oxo-1,3-oxazolidin-3-yl]-4H-pyrido[3,2-b][1,4]oxazin-3-one, Isomer A; formic acid
6-[5-[3-[(4-fluoro-2,3-dihydro-1H-inden-2-yl)amino]propyl]-2-oxo-1,3-oxazolidin-3-yl]-4H-pyrido[3,2-b][1,4]oxazin-3-one; formic acid
6-[5-[3-(2,3-dihydro-1H-inden-2-ylamino)propyl]-2-oxo-1,3-oxazolidin-3-yl]-4H-pyrido[3,2-b][1,4]oxazin-3-one; formic acid
6-[5-[3-[(5-chloro-2,3-dihydro-1H-inden-2-yl)amino]propyl]-2-oxo-1,3-oxazolidin-3-yl]-4H-pyrido[3,2-b][1,4]oxazin-3-one; formic acid
6-[5-[2-[(5-chloro-2,3-dihydro-1H-inden-2-yl)amino]ethyl]-2-oxo-1,3-oxazolidin-3-yl]-4H-pyrido[3,2-b][1,4]oxazin-3-one
6-[5-[3-[(5-fluoro-2,3-dihydro-1H-inden-2-yl)amino]propyl]-2-oxo-1,3-oxazolidin-3-yl]-4H-pyrido[3,2-b][1,4]oxazin-3-one;formic acid
2-[3-[2-oxo-3-(3-oxo-4H-pyrido[3,2-b] [1,4]oxazin-6-yl)-1,3-oxazolidin-5-yl]propylamino]-2,3-dihydro-1H-indene-4-carbonitrile; formic acid
2-[[trans-3-[2-oxo-3-(3-oxo-4H-pyrido[3,2-b] [1,4]oxazin-6-yl)-1,3-oxazolidin-5-yl] cyclobutyl] amino] -2,3-dihydro-1H-indene-4-carbonitrile
6-[5-[2-[(4-chloro-2,3-dihydro-1H-inden-2-yl)methylamino]ethyl]-2-oxo-1,3-oxazolidin-3-yl]-4H-1,4-benzoxazin-3-one; formic acid
6-[5-[cis-3-[(4-chloro-2,3-dihydro-1H-inden-2-yl)amino]cyclobutyl]-2-oxo-1,3-oxazolidin-3-yl]-4H-pyrido[3,2-b][1,4]oxazin-3-one; formic acid
6-[5-[trans-3-[(4-chloro-2,3-dihydro-1H-inden-2-yl)amino]cyclobutyl]-2-oxo-1,3-oxazolidin-3-yl]-4H-pyrido[3,2-b][1,4]oxazin-3-one
2-[[2-[2-oxo-3-(3-oxo-4H-1,4-benzoxazin-6-yl)-1,3-oxazolidin-5-yl]ethylamino]methyl]-2,3-dihydro-1H-indene-4-carbonitrile; formic acid
2-[[cis-3-[2-oxo-3-(3-oxo-4H-pyrido[3,2-b][1,4]oxazin-6-yl)-1,3-oxazolidin-5-yl]cyclobutyl]amino]-2,3-dihydro-1H-indene-4-carbonitrile; formic acid
6-[5-[2-[2-(4-chloro-2,3-dihydro-1H-inden-2-yl)ethylamino]ethyl]-2-oxo-1,3-oxazolidin-3-yl]-4H-pyrido[3,2-b][1,4]oxazin-3-one; formic acid
6-[5-[[(4-chloro-2,3-dihydro-1H-inden-2-yl)methylamino]methyl]-2-oxo-1,3-oxazolidin-3-yl]-4H-pyrido[3,2-b][1,4]oxazin-3-one; formic acid
6-[5-[[(4-chloro-2,3-dihydro-1H-inden-2-yl)amino]methyl]-2-oxo-1,3-oxazolidin-3-yl]-4H-pyrido[3,2-b][1,4]oxazin-3-one
6-[(4R)-4-[[3-[(4-fluoro-2,3-dihydro-1H-inden-2-yl)amino]cyclobutyl]amino]-2-oxopyrrolidin-1-yl]-4H-pyrido[3,2-b][1,4]oxazin-3-one; formic acid
6-[(4S)-4-[[3-[(4-fluoro-2,3-dihydro-1H-inden-2-yl)amino]cyclobutyl]amino]-2-oxopyrrolidin-1-yl]-4H-pyrido[3,2-b][1,4]oxazin-3-one
6-[5-[[(5-chloro-2,3-dihydro-1H-inden-2-yl)amino]methyl]-2-oxo-1,3-oxazolidin-3-yl]-4H-pyrido[3,2-b][1,4]oxazin-3-one
6-[5-[[2-(4-chloro-2,3-dihydro-1H-inden-2-yl)ethylamino]methyl]-2-oxo-1,3-oxazolidin-3-yl]-4H-pyrido[3,2-b][1,4]oxazin-3-one; formic acid
6-[5-[3-[(4-methyl-6,7-dihydro-5H-cyclopenta[b]pyridin-6-yl)amino]propyl]-2-oxo-1,3-oxazolidin-3-yl]-4H-pyrido[3,2-b][1,4]oxazin-3-one
2-[[2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-indene-4-carbonitrile
2-[[[2-methyl-1-[2-oxo-3-(3-oxo-4H-pyrido[3,2-b][1,4]oxazin-6-yl)-1,3-oxazolidin-5-yl]propan-2-yl]amino]methyl]-2,3-dihydro-1H-indene-4-carbonitrile
6-[5-[2-[(1-chloro-6,7-dihydro-5H-cyclopenta[c]pyridin-6-yl)methylamino]-2-methylpropyl]-2-oxo-1,3-oxazolidin-3-yl]-4H-pyrido[3,2-b] [1,4] oxazin-3 -one; formic acid
2-[[[rac-3a,7a-trans-6,7a-cis-2-oxo-3-(3-oxo-4H-pyrido[3,2-b][1,4]oxazin-6-yl)-3a,4,5,6,7,7a-hexahydro-1,3-benzoxazol-6-yl]amino]methyl]-2,3-dihydro-1H-indene-4-carbonitrile
6-[rac-3a,7a-trans-6,7a-cis-6-[2-(4-chloro-2,3-dihydro-1H-inden-2-yl)ethylamino]-2-oxo-3a,4,5,6,7,7a-hexahydro-1,3-benzoxazol-3-yl]-4H-pyrido[3,2-b][1,4]oxazin-3-one
2-[2-[6-oxo-7-(3-oxo-4H-pyrido[3,2-b][1,4]oxazin-6-yl)-5-oxa-2,7-diazaspiro[3.4]octan-2-yl]ethyl]-2,3-dihydro-1H-indene-4-carbonitrile
2-[[trans-4-[2-oxo-3-(3-oxo-4H-pyrido[3,2-b] [1,4]oxazin-6-yl)-1,3-oxazolidin-5-yl]cyclohexyl]amino]-2,3-dihydro-1H-indene-4-carbonitrile; formic acid
2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-indene-4-carbonitrile
6-[5-[2-[(4-fluoro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one
4-fluoro-6-[[2-[2-oxo-3-(3-oxo-4H-pyrido[3,2-b] [1,4]oxazin-6-yl)-1,3-oxazolidin-5-yl]ethylamino]methyl]-6,7-dihydro-5H-cyclopenta[c]pyridine-1-carbonitrile; formic acid
6-[5-[2-[(1-chloro-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one
2-[[2-oxo-3-(3-oxo-4H-pyrido[3,2-b][1,4]oxazin-6-yl)-1-oxa-3-azaspiro[4.5]decan-8-yl]amino]-2,3-dihydro-1H-indene-4-carbonitrile, Isomer A; formic acid
2-[[2-oxo-3-(3-oxo-4H-pyrido[3,2-b][1,4]oxazin-6-yl)-1-oxa-3-azaspiro[4.5]decan-8-yl]amino]-2,3-dihydro-1H-indene-4-carbonitrile, Isomer B; formic acid
6-[2-oxo-5-[2-[[rac-(5,6-trans)-1-chloro-5-hydroxy-6,7-dihydro-5H-cyclopenta[c]pyridin-6-yl]methylamino]ethyl]-1,3-oxazolidin-3-yl]-4H-pyrido[3,2-b][1,4]oxazin-3-one; formic acid
2-[[2-[5-oxo-4-(3-oxo-4H-1,4-benzoxazin-6-yl)-1,3,4-oxadiazol-2-yl]ethylamino]methyl]-2,3-dihydro-1H-indene-4-carbonitrile; formic acid
6-[2-oxo-5-[2-[[rac-(5,6-trans)-5-hydroxy-1-methyl-6,7-dihydro-SH-cyclopenta[c]pyridin-6-yl]methylamino]ethyl]-1,3-oxazolidin-3-yl]-4H-pyrido[3,2-b][1,4]oxazin-3-one; formic acid
2-[[2-[5-oxo-4-(3-oxo-4H-pyrido[3,2-b] [1,4]oxazin-6-yl)-1,3,4-oxadiazol-2-yl]ethylamino]methyl]-2,3-dihydro-1H-indene-4-carbonitrile; formic acid
7-[5-[2-[(1-chloro-6,7-dihydro-5H-cyclopenta[c]pyridin-6-yl)methylamino]ethyl]-2-oxo-1,3-oxazolidin-3-yl]-1H-pyrido[2,3-b][1,4]oxazin-2-one;hydrochloride
2-[[2-[2-oxo-3-(2-oxo-1H-pyrido[2,3-b] [1,4]oxazin-7-yl)-1,3-oxazolidin-5-yl]ethylamino]methyl]-2,3-dihydro-1H-indene-4-carbonitrile
1-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-4-carbonitrile; formic acid
2-[[4-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide
6-[5-[2-[(1-chloro-3-methoxy-6,7-dihydro-5H-cyclopenta[c]pyridin-6-yl)methylamino]ethyl]-2-oxo-1,3-oxazolidin-3-yl]-4H-pyrido[3,2-b] [1,4] oxazin-3 -one
6-[5-[2-[(1-chloro-3-methoxy-6,7-dihydro-5H-cyclopenta[c]pyridin-6-yl)methylamino]ethyl]-2-oxo-1,3-oxazolidin-3-yl]-4H-pyrazino[2,3-b][1,4] oxazin-3 -one
6-[5-[2-[(4-fluoro-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one
5-[(1-methylpyrrolidin-2-yl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile; formic acid
3-methoxy-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile
5-(2-morpholin-4-ylethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile; formic acid
6-[5-[2-[(4-chloro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3 -one; formic acid
N-[2-[[4-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]acetamide
6-[5-[2-[(1-chloro-4-methoxy-6,7-dihydro-5H-cyclopenta[c]pyridin-6-yl)methylamino]ethyl]-2-oxo-1,3-oxazolidin-3-yl]-4H-pyrido[3,2-b] [1,4] oxazin-3 -one
6-[5-[2-[(1-chloro-4-methoxy-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one; formic acid
N-[2-[[4-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]methanesulfonamide
6-[5-[2-[(4-chloro-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;formic acid
2-[[[(2R)-2-hydroxy-2-[(5R)-2-oxo-3-(3-oxo-4H-1,4-benzoxazin-6-yl)-1,3-oxazolidin-5-yl]ethyl]amino]methyl]-2,3-dihydro-1H-indene-4-carbonitrile; formic acid
2-[[[(2S)-2-hydroxy-2-[(5S)-2-oxo-3-(3-oxo-4H-1,4-benzoxazin-6-yl)-1,3-oxazolidin-5-yl]ethyl]amino]methyl]-2,3-dihydro-1H-indene-4-carbonitrile; formic acid
5-(oxetan-3-yloxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile
2-[[2-[2-oxo-3-(3-oxo-4H-pyrido[3,2-b] [1,4]thiazin-6-yl)-1,3-oxazolidin-5-yl]ethylamino]methyl]-2,3-dihydro-1H-indene-4-carbonitrile
3-methoxy-6-[[2-[2-oxo-3-(3-oxo-4H-pyrazino[2,3-b] [1,4]oxazin-6-yl)oxazolidin-5-yl]ethylamino]methyl]-6,7-dihydro-5H-cyclopenta[c]pyridine-1-carbonitrile; formic acid
2-[[1-chloro-6-[[2-[2-oxo-3-(3-oxo-4H-pyrido[3,2-b] [1,4]oxazin-6-yl)oxazolidin-5-yl]ethylamino]methyl]-6,7-dihydro-5H-cyclopenta[c]pyridin-3-yl]oxy]-N-methyl-acetamide
6-[5-[2-[(4-fluoro-1-methyl-6,7-dihydro-5H-cyclopenta[c]pyridin-6-yl)methylamino]ethyl]-2-oxo-1,3,4-oxadiazol-3-yl]-4H-1,4-benzoxazin-3-one
6-[5-[2-[(4-fluoro-1-methyl-6,7-dihydro-5H-cyclopenta[c]pyridin-6-yl)methylamino]ethyl]-2-oxo-1,3,4-oxadiazol-3-yl]-4H-pyrido[3,2-b][1,4]oxazin-3-one
2-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide; formic acid
6-[5-[2-[(4-fluoro-1-methoxy-6,7-dihydro-5H-cyclopenta[c]pyridin-6-yl)methylamino]ethyl]-2-oxo-oxazolidin-3-yl]-4H-pyrido[3,2-b][1,4]oxazin-3-one
6-[5-[2-[(4-fluoro-1-methoxy-6,7-dihydro-5H-cyclopenta[c]pyridin-6-yl)methylamino]ethyl]-2-oxo-oxazolidin-3-yl]-4H-pyrazino[2,3-b][1,4]oxazin-3-one
6-[8-[(4-fluoro-1-methyl-6,7-dihydro-5H-cyclopenta[c]pyridin-6-yl)methyl]-2-oxo-1-oxa-3,8-diazaspiro[4.5]decan-3-yl]-4H-pyrido[3,2-b][1,4]oxazin-3-one
2-[[7-fluoro-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-inden-5-yl]oxy]acetonitrile; formic acid
2-[[7-fluoro-2-[[2-[2-oxo-3-(3-oxo-4H-pyrido[3,2-b] [1,4]thiazin-6-yl)-1,3-oxazolidin-5-yl]ethylamino]methyl]-2,3-dihydro-1H-inden-5-yl]oxy]-N-methylacetamide; formic acid
6-[5-[1-[(4-fluoro-1-methyl-6,7-dihydro-5H-cyclopenta[c]pyridin-6-yl)methyl]azetidin-3-yl]-2-oxo-oxazolidin-3-yl]-4H-pyrazino[2,3-b][1,4]oxazin-3-one; formic acid
2-[[3-[2-oxo-3-(3-oxo-4H-pyrazino[2,3-b] [1,4]oxazin-6-yl)-1,3-oxazolidin-5-yl]azetidin-1-yl]methyl]-2,3-dihydro-1H-indene-4-carbonitrile; formic acid
2-[[7-fluoro-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-inden-5-yl]oxy]-N,N-dimethylacetamide
6-[5-[3-[(4-fluoro-1-methyl-6,7-dihydro-5H-cyclopenta[c]pyridin-6-yl)amino]propyl]-2-oxo-1,3-oxazolidin-3-yl]-4H-pyrido[3,2-b] [1,4] oxazin-3 -one
6-[5-[3-[(4-fluoro-1-methyl-6,7-dihydro-5H-cyclopenta[c]pyridin-6-yl)amino]propyl]-2-oxo-1,3-oxazolidin-3-yl]-4H-pyrazino[2,3-b][1,4]oxazin-3-one
2-[3-[2-oxo-3-(3-oxo-4H-pyrazino[2,3-b][1,4]oxazin-6-yl)-1,3-oxazolidin-5-yl]propylamino]-2,3-dihydro-1H-indene-4-carbonitrile
2-[[2-hydroxy-3-[2-oxo-3-(3-oxo-4H-pyrazino[2,3-b] [1,4]oxazin-6-yl)-1,3-oxazolidin-5-yl]propyl]amino]-2,3-dihydro-1H-indene-4-carbonitrile, Isomer A; formic acid
2-[[2-hydroxy-3-[2-oxo-3-(3-oxo-4H-pyrazino[2,3-b][1,4]oxazin-6-yl)-1,3-oxazolidin-5-yl]propyl]amino]-2,3-dihydro-1H-indene-4-carbonitrile, Isomer B; formic acid
6-[5-[3-[(4-fluoro-2,3-dihydro-1H-inden-2-yl)amino]-2-hydroxypropyl]-2-oxo-1,3-oxazolidin-3-yl]-4H-pyrazino[2,3-b][1,4]oxazin-3-one, Isomer A; formic acid
6-[5-[3-[(4-fluoro-2,3-dihydro-1H-inden-2-yl)amino]-2-hydroxypropyl]-2-oxo-1,3-oxazolidin-3-yl]-4H-pyrazino[2,3-b][1,4]oxazin-3-one, Isomer B; formic acid
6-[5-[2-[(4-fluoro-1-methyl-6,7-dihydro-5H-cyclopenta[c]pyridin-6-yl)methylamino]ethyl]-2-oxo-1,3-oxazol-3-yl]-4H-pyrido[3,2-b][1,4]oxazin-3-one
6-[5-[2-[(1-chloro-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one
6-[5-[2-(2,3-dihydro-1H-inden-2-ylmethylamino)ethyl]-2-oxo-1,3-oxazolidin-3-yl]-4H-pyrido[3,2-b][1,4]oxazin-3-one
6-[5-[2-[(1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one
6-[5-[2-[(4-methyl-6,7-dihydro-5H-cyclopenta[b]pyridin-6-yl)methylamino]ethyl]-2-oxo-1,3-oxazolidin-3-yl]-4H-pyrido[3,2-b][1,4]oxazin-3-one
6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile; formic acid
6-[5-[2-[(4-fluoro-2,3-dihydro-1H-inden-2-yl)methylamino]ethyl]-2-oxo-1,3-oxazolidin-3-yl]- 4H-pyrido[3,2-b][1,4]oxazin-3-one
6-[5-[3-[(1-chloro-6,7-dihydro-5H-cyclopenta[c]pyridin-6-yl)amino]propyl]-2-oxo-1,3-oxazolidin-3-yl]-4H-pyrido[3,2-b][1,4]oxazin-3-one
6-[2-oxo-5-[2-[(1,3,4-trimethyl-6,7-dihydro-5H-cyclopenta[c]pyridin-6-yl)methylamino]ethyl]-1,3-oxazolidin-3-yl]-4H-pyrido [3,2-b][1,4]oxazin-3 -one
6-[5-[3-[(1-methyl-6,7-dihydro-5H-cyclopenta[c]pyridin-6-yl)amino]propyl]-2-oxo-1,3-oxazolidin-3-yl] -4H-pyrido [3,2-b] [1,4] oxazin-3 -one
6-[3-[2-oxo-3-(3-oxo-4H-pyrido[3,2-b] [1,4]oxazin-6-yl)-1,3-oxazolidin-5-yl]propylamino]-6,7-dihydro-5H-cyclopenta[c]pyridine-1-carbonitrile
2-[[[cis-3-[2-oxo-3-(3-oxo-4H-pyrido[3,2-b][1,4]oxazin-6-yl)-1,3-oxazolidin-5-yl]cyclobutyl]amino]methyl]-2,3-dihydro-1H-indene-4-carbonitrile; formic acid
2-[[[trans-3-[2-oxo-3-(3-oxo-4H-pyrido[3,2-b] [1,4]oxazin-6-yl)-1,3-oxazolidin-5-yl]cyclobutyl]amino]methyl]-2,3-dihydro-1H-indene-4-carbonitrile
6-[5-[2-[(1,4-dimethyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one
2-[[[rac-3a,7a-cis-6,7a-trans-2-oxo-3-(3-oxo-4H-pyrido[3,2-b][1,4]oxazin-6-yl)-3a,4,5,6,7,7a-hexahydro-1,3-benzoxazol-6-yl]amino]methyl]-2,3-dihydro-1H-indene-4-carbonitrile; formic acid
2-[[[trans-4-[2-oxo-3-(3-oxo-4H-pyrido[3,2-b] [1,4]oxazin-6-yl)-1,3-oxazolidin-5-yl]cyclohexyl]amino]methyl]-2,3-dihydro- 1H-indene-4-carbonitrile; formic acid
6-[5-[2-[[1,4-dimethyl-3-[(5-oxomorpholin-2-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[1,4-dimethyl-3-[(5-oxopyrrolidin-3-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[1,4-dimethyl-3-[(1-methyl-5-oxopyrrolidin-3-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[[[2,4-trans-6-oxo-7-(3-oxo-4H-pyrido[3,2-b][1,4]oxazin-6-yl)-5-oxa-7-azaspiro[3.4]octan-2-yl]amino]methyl]-6,7-dihydro-5H-cyclopenta[c]pyridine-1-carbonitrile
6-[5-[2-[[1,4-dimethyl-3-(3-methylsulfonylpropoxy)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[(3-methoxy-1,4-dimethyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one
2-[[7-cyano-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-inden-5-yl]oxy]-N-methylacetamide
2-[[4-chloro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide
2-[[4-chloro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide
2-[[1,4-dimethyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-3-yl]oxy]-N-methylacetamide
2-[[1,4-dimethyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridin-3-yl]oxy]-N-methylacetamide
2-[[4,7-dimethyl-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide
2-[[4,7-dimethyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide
2-[[4,7-dimethyl-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-5-yl]oxy]-N-methylacetamide
N-[2-[[7-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]methanesulfonamide
6-[5-[2-[[5-(2-aminoethoxy)-4-chloro-2,3-dihydro-1H-inden-2-yl]methylamino]ethyl]-2-oxo-1,3-oxazolidin-3-yl]-4H-pyrido[3,2-b][1,4]oxazin-3-one
6-[5-[2-[[5-[(1-aminocyclopropyl)methoxy]-4-chloro-2,3-dihydro-1H-inden-2-yl]methylamino]ethyl]-2-oxo-1,3-oxazolidin-3-yl]-4H-pyrido[3,2-b][1,4]oxazin-3-one; formic acid
6-[5-[3-[[5-(2-aminoethoxy)-4-chloro-2,3-dihydro-1H-inden-2-yl]amino]propyl]-2-oxo-1,3-oxazolidin-3-yl]-4H-pyrido[3,2-b][1,4]oxazin-3-one;formic acid
6-[5-[3-[[5-[(1-aminocyclopropyl)methoxy]-4-chloro-2,3-dihydro-1H-inden-2-yl]amino]propyl]-2-oxo-1,3-oxazolidin-3-yl]-4H-pyrido[3,2-b][1,4] oxazin-3 -one; formic acid
6-[4-[2-[(4-fluoro-2,3-dihydro-1H-inden-2-yl)amino]ethylamino]-2-oxopyrrolidin-1-yl]-4H-pyrido[3,2-b][1,4]oxazin-3-one; formic acid
2-[2-[[5-oxo-1-(3-oxo-4H-pyrido[3,2-b][1,4]oxazin-6-yl)pyrrolidin-3-yl]amino]ethylamino]-2,3- dihydro-1H-indene-4-carbonitrile
6-[5-[2-[[3-[(1-aminocyclopropyl)methoxy]-1-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one; formic acid
6-[2-oxo-5-[2-[[5,6-trans-5-amino-1-chloro-6,7-dihydro-5H-cyclopenta[c]pyridin-6-yl]methylamino]ethyl]-1,3-oxazolidin-3-yl]-4H-pyrido[3,2-b][1,4]oxazin-3-one; formic acid
5-[(1-aminocyclopropyl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile hydrochloride
3-[(1-aminocyclopropyl)methoxy]-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile
6-[5-[2-[[3-(2-aminoethoxy)-1-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
3-(2-aminoethoxy)-6-[[2-[2-oxo-3-(3-oxo-4H-pyrido[3,2-b] [1,4]oxazin-6-yl)-1,3-oxazolidin-5-yl]ethylamino]methyl]-6,7-dihydro-5H-cyclopenta[c]pyridine-1-carbonitrile; formic acid
5-(2-aminoethoxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; formic acid
5-(azetidin-3-ylmethoxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; formic acid
6-[2-oxo-5-[2-[(rac-5,6-trans-5-amino-1-methyl-6,7-dihydro-5H-cyclopenta[c]pyridin-6-yl)methylamino]ethyl]-1,3-oxazolidin-3-yl]-4H-pyrido[3,2-b][1,4]oxazin-3-one; formic acid
rac-1,2-trans-1-amino-2-[[2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-indene-4-carbonitrile; formic acid
5-(azetidin-3-yloxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; formic acid
rac-5,6-trans-5-amino-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile; formic acid
5-(2-aminopropoxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; formic acid
7-[2-oxo-5-[2-[[rac-1,2-trans-1-amino-4-chloro-2,3-dihydro-1H-inden-2-yl]methylamino]ethyl]-1,3-oxazolidin-3-yl]-1H-pyrido[2,3-b][1,4]oxazin-2-one; formic acid
6-[2-oxo-5-[2-[[rac-1,2-trans-1-amino-4-chloro-2,3-dihydro-1H-inden-2-yl]methylamino]ethyl]-1,3 -oxazolidin-3 -yl] -4H-pyrazino [2,3 -b] [1,4] oxazin-3 -one; formic acid
5-(azetidin-2-ylmethoxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; formic acid
2-[[2-[3-amino-5-oxo-1-(3-oxo-4H-pyrido[3,2-b][1,4]oxazin-6-yl)pyrrolidin-3-yl]ethylamino]methyl]-2,3-dihydro-1H-indene-4-carbonitrile
rac-1,2-trans-1-hydroxy-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile
6-[4-amino-4-[2-[(4-chloro-2,3-dihydro-1H-inden-2-yl)methylamino]ethyl]-2-oxopyrrolidin-1-yl]-4H-pyrido[3,2-b][1,4]oxazin-3-one
2-[[2-[2-oxo-3-(3-oxo-4H-pyrido[3,2-b] [1,4]oxazin-6-yl)-1,3-oxazolidin-5-yl]ethylamino]methyl]-5-(pyrrolidin-2-ylmethoxy)-2,3-dihydro-1H-indene-4-carbonitrile; formic acid
6-[5-[[2-(2-amino-4-chloro-1,3-dihydroinden-2-yl)ethylamino]methyl]-2-oxo-1,3-oxazolidin-3-yl]-4H-pyrido[3,2-b][1,4]oxazin-3-one; formic acid
2-[[2-[2-oxo-3-(3-oxo-4H-pyrido[3,2-b] [1,4]oxazin-6-yl)-1,3-oxazolidin-5-yl]ethylamino]methyl]-5-(2-piperazin-1-ylethoxy)-2,3-dihydro-1H-indene-4-carbonitrile; formic acid
6-[5-[2-[[3-(2-aminopropoxy)-1-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one; formic acid
6-[5-[2-[[3-(2-aminopropoxy)-1-chloro-6,7-dihydro-5H-cyclopenta[c]pyridin-6-yl] methylamino] ethyl] -2-oxo-oxazolidin-3 -yl] -4H-pyrazino [2,3 -b] [1,4] oxazin-3 -one; formic acid
6-[2-oxo-5-[2-[[rac-5,6-cis-5-amino-1-chloro-6,7-dihydro-5H-cyclopenta[c]pyridin-6-yl]methylamino]ethyl]-1,3-oxazolidin-3-yl]-4H-pyrido[3,2-b][1,4]oxazin-3-one; formic acid
6-[2-oxo-5-[2-[[rac-1,2-trans-1-amino-4-chloro-2,3-dihydro-1H-inden-2-yl]methylamino]ethyl]-1,3,4-oxadiazol-3-yl]-4H-1,4-benzoxazin-3-one; formic acid
2-[[2-[rac-4,5-cis-4-(aminomethyl)-2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; formic acid
2-[[2-[rac-4,5-trans-4-(aminomethyl)-2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile; formic acid
6-[5-[2-[[6-(azetidin-3-yloxy)-4-fluoro-2,3-dihydro-1H-inden-2-yl]methylamino]ethyl]-2-oxo-1,3-oxazolidin-3-yl]-4H-pyrido[3,2-b][1,4]oxazin-3-one
rac-2,3-trans-3-hydroxy-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile; formic acid
rac-2,3-trans-3-hydroxy-2-[[2-[2-oxo-3-(3-oxo-4H-pyrido[3,2-b] [1,4]thiazin-6-yl)-1,3-oxazolidin-5-yl]ethylamino]methyl]-2,3-dihydro-1H-indene-4-carbonitrile; formic acid
6-[5-[2-[(rac-1,2-trans-1-amino-4-chloro-2,3-dihydro-1H-inden-2-yl)methylamino]ethyl]-2-oxo-1,3,4-oxadiazol-3-yl]-4H-pyrido[3,2-b] [1,4] oxazin-3 -one
6-[5-[2-[[6-(2-aminopropoxy)-4-fluoro-2,3-dihydro-1H-inden-2-yl]methylamino]ethyl]-2-oxo-1,3-oxazolidin-3-yl] -4H-pyrido [3,2-b] [1,4] oxazin-3 -one
6-[5-[2-[[6-(1-aminopropan-2-yloxy)-4-fluoro-2,3-dihydro-1H-inden-2-yl]methylamino]ethyl]-2-oxo-1,3-oxazolidin-3-yl]-4H-pyrido[3,2-b][1,4] oxazin-3 -one; formic acid
rac-2,3-trans-3-amino-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile
rac-2,3-trans-3-amino-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-indene-4-carbonitrile
6-[5-[[[2-amino-2-(4-chloro-2,3-dihydro-1H-inden-2-yl)ethyl]amino]methyl]-2-oxo-1,3-oxazolidin-3-yl]-4H-pyrido[3,2-b][1,4]oxazin-3-one; formic acid
6-[2-[2-amino-2-(4-chloro-2,3-dihydro-1H-inden-2-yl)ethyl]-6-oxo-5-oxa-2,7-diazaspiro [3.4] octan-7-yl] -4H-pyrido [3,2-b] [1,4] oxazin-3 -one; formic acid
6-[5-[2-[[6-[(1-aminocyclopropyl)methoxy]-4-fluoro-2,3-dihydro-1H-inden-2-yl]methylamino]ethyl]-2-oxo-1,3-oxazolidin-3-yl]-4H-pyrido[3,2-b][1,4]oxazin-3-one
6-[5-[2-[[6-[(1-aminocyclopropyl)methoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one
6-[5-[2-[[6-[(3-aminooxetan-3-yl)methoxy]-4-fluoro-2,3-dihydro-1H-inden-2-yl]methylamino]ethyl]-2-oxo-1,3-oxazolidin-3-yl]-4H-pyrido[3,2-b][1,4]oxazin-3-one; formic acid
6-[5-[2-[(rac-1,2-trans-1-amino-4-fluoro-2,3-dihydro-1H-inden-2-yl)methylamino]ethyl]-2-oxo-1,3-oxazolidin-3-yl]-4H-pyrido[3,2-b][1,4]oxazin-3-one
6-[5-[2-[(rac-1,2-trans-1-amino-4-fluoro-2,3-dihydro-1H-inden-2-yl)methylamino]ethyl]-2-oxo-1,3 -oxazolidin-3 -yl] -4H-pyrazino [2,3 -b] [1,4] oxazin-3 -one; formic acid
5-[2-[(rac-1,2-trans-1-amino-4-fluoro-2,3-dihydro-1H-inden-2-yl)methylamino]ethyl]-3-(3-oxo-4H-pyrido[3,2-b][1,4]thiazin-6-yl)-1,3-oxazolidin-2-one; formic acid
2-[[rac-5,6-cis-5,8-trans-6-amino-2-oxo-3-(3-oxo-4H-pyrido[3,2-b][1,4]oxazin-6-yl)-1-oxa-3-azaspiro[4.5]decan-8-yl]amino]-2,3-dihydro-1H-indene-4-carbonitrile
6-[rac-5,6-cis-5,8-trans-6-amino-8-[(4-fluoro-2,3-dihydro-1H-inden-2-yl)amino]-2-oxo-1-oxa-3-azaspiro[4.5]decan-3-yl]-4H-pyrido[3,2-b][1,4]oxazin-3-one; 2,2,2-trifluoroacetic acid
6-[2-oxo-5-[2-[[rac-1,2-trans-1-amino-7-chloro-2,3-dihydro-1H-inden-2-yl]methylamino]ethyl]-1,3-oxazolidin-3-yl]-4H-pyrido[3,2-b][1,4]oxazin-3-one; formic acid
6-[2-oxo-5-[2-[[rac-1,2-trans-1-amino-7-chloro-2,3-dihydro-1H-inden-2-yl]methylamino]ethyl]-1,3-oxazolidin-3-yl] -4H-pyrazino[2,3-b] [1,4] oxazin-3 -one
6-[2-oxo-5-[2-[[rac-1,2-trans-1-amino-4-chloro-2,3-dihydro-1H-inden-2-yl]methylamino]ethyl]-1,3-oxazol-3-yl]-4H-pyrido[3,2-b][1,4]oxazin-3-one
6-[5-[2-[[3-(2-aminoethoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-[(1-aminocyclopropyl)methoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[1,4-dimethyl-3-[2-(methylamino)ethoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-(2-aminoethoxy)-4-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-[(1-aminocyclopropyl)methoxy]-1-methyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-(3-aminopropoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[6-(2-aminoethoxy)-4-chloro-2,3-dihydro-1H-inden-2-yl]methylamino]ethyl]-2-oxo-1,3-oxazolidin-3-yl] -4H-pyrido [3,2-b] [1,4] oxazin-3 -one
6-[5-[2-[[6-[(1-aminocyclopropyl)methoxy]-4-chloro-2,3-dihydro-1H-inden-2-yl]methylamino]ethyl]-2-oxo-1,3-oxazolidin-3-yl]-4H-pyrido[3,2-b][1,4]oxazin-3-one
6-[5-[3-[[6-(2-aminoethoxy)-4-chloro-2,3-dihydro-1H-inden-2-yl]amino]propyl]-2-oxo-1,3-oxazolidin-3-yl]-4H-pyrido[3,2-b][1,4]oxazin-3-one
6-[5-[3-[[6-[(1-aminocyclopropyl)methoxy]-4-chloro-2,3-dihydro-1H-inden-2-yl]amino]propyl]-2-oxo-1,3-oxazolidin-3-yl]-4H-pyrido[3,2-b] [1,4] oxazin-3 -one
6-[5-[2-[[3-[(1-aminocyclopropyl)methoxy]-4-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[[[2,4-cis-6-oxo-7-(3-oxo-4H-pyrido[3,2-b][1,4]oxazin-6-yl)-5-oxa-7-azaspiro[3.4]octan-2-yl]amino]methyl]-6,7-dihydro-5H-cyclopenta[c]pyridine-1-carbonitrile
2-[[[2,4-cis-6-oxo-7-(3-oxo-4H-pyrido[3,2-b][1,4]oxazin-6-yl)-5-oxa-7-azaspiro[3.4]octan-2-yl]amino]methyl]-2,3-dihydro-1H-indene-4-carbonitrile
6-[5-[2-[[3-(azetidin-3-ylmethoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one hydrochloride
6-[(1-aminocyclopropyl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile
6-((1-aminocyclopropyl)methoxy)-2-(((2-(2-oxo-3-(3-oxo-3,4-dihydro-2H-pyrazino[2,3-b] [1,4]oxazin-6-yl)oxazolidin-5-yl)ethyl)amino)methyl)-2,3-dihydro-1H-indene-4-carbonitrile
6-(azetidin-2-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile
6-(2-aminoethoxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile
6-[5-[2-[[3-[2-(cyclopropylamino)ethoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-(azetidin-3-yloxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; 2,2,2-trifluoroacetic acid
6-(azetidin-3-ylmethoxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; 2,2,2-trifluoroacetic acid
6-(2-aminopropoxy)-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile; 2,2,2-trifluoroacetic acid
6-(2-aminopropoxy)-2-(((2-(2-oxo-3-(3-oxo-3,4-dihydro-2H-pyrazino[2,3-b][1,4]oxazin-6-yl)oxazolidin-5-yl)ethyl)amino)methyl)-2,3-dihydro-1H-indene-4-carbonitrile; 2,2,2-trifluoroacetic acid
6-[2-oxo-5-[[2-[rac-1,2-trans-1-amino-4-chloro-2,3-dihydro-1H-inden-2-yl]ethylamino]methyl]-1,3-oxazolidin-3-yl]-4H-pyrido[3,2-b][1,4]oxazin-3-one
6-[6-oxo-2-[2-[rac-1,2-trans-1-amino-4-chloro-2,3-dihydro-1H-inden-2-yl]ethyl]-5-oxa-2,7-diazaspiro [3.4] octan-7-yl] -4H-pyrido [3,2-b] [1,4] oxazin-3 -one; 2,2,2-trifluoroacetic acid
6-((1-aminocyclopropyl)methoxy)-2-(2-(((2-oxo-3-(3-oxo-3,4-dihydro-2H-pyrido[3,2-b] [1,4]oxazin-6-yl)oxazolidin-5-yl)methyl)amino)ethyl)-2,3-dihydro-1H-indene-4-carbonitrile
6-((1-aminocyclopropyl)methoxy)-2-(2-(6-oxo-7-(3-oxo-3,4-dihydro-2H-pyrido[3,2-b][1,4]oxazin-6-yl)-5-oxa-2,7-diazaspiro[3.4]octan-2-yl)ethyl)-2,3-dihydro-1H-indene-4-carbonitrile
3-[(1-aminocyclopropyl)methoxy]-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-4-carbonitrile
6-[5-[2-[[1,4-dimethyl-3-(morpholin-3-ylmethoxy)-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-[[(2S)-azetidin-2-yl]methoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[1,4-dimethyl-3-[2-(propan-2-ylamino)ethoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-[2-(2-methoxyethylamino)ethoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-(2-amino-2-methylpropoxy)-2-(((2-(2-oxo-3-(3-oxo-3,4-dihydro-2H-pyrido[3,2-b][1,4]oxazin-6-yl)oxazolidin-5-yl)ethyl)amino)methyl)-2,3-dihydro-1H-indene-4-carbonitrile; 2,2,2-trifluoroacetic acid
6-(2-amino-2-methylpropoxy)-2-(((2-(2-oxo-3-(3-oxo-3,4-dihydro-2H-pyrazino[2,3-b] [1,4]oxazin-6-yl)oxazolidin-5-yl)ethyl)amino)methyl)-2,3-dihydro-1H-indene-4-carbonitrile
6-[5-[1-[(rac-1,2-trans-1-amino-4-chloro-2,3-dihydro-1H-inden-2-yl)methyl]azetidin-3-yl]-2-oxo-1,3-oxazolidin-3-yl]-4H-pyrazino[2,3-b][1,4]oxazin-3-one
6-[rac-5,6-cis-5,8-trans-6-amino-8-[(1-chloro-6,7-dihydro-5H-cyclopenta[c]pyridin-6-yl)methylamino]-2-oxo-1-oxa-3-azaspiro[4.5]decan-3-yl]-4H-pyrido[3,2-b][1,4]oxazin-3-one
6-[2-Oxo-5-[2-[[1,2-trans-1-amino-4-chloro-2,3-dihydro-1H-inden-2-yl]methylamino]ethyl]-1,3-oxazolidin-3-yl]-4H-pyrido[3,2-b][1,4]oxazin-3-one, Isomer A
6-[2-oxo-5-[2-[[1,2-trans-1-amino-4-chloro-2,3-dihydro-1H-inden-2-yl]methylamino]ethyl]-1,3-oxazolidin-3-yl]-4H-pyrido[3,2-b][1,4]oxazin-3-one, Isomer B
6-[5-[2-[[3-(2-aminoethoxy)-1-methyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
2,4-cis-6-[6-oxo-2-[[rac-1,2-trans-1-amino-4-chloro-2,3-dihydro-1H-inden-2-yl]methylamino]-5-oxa-7-azaspiro[3.4]octan-7-yl]-4H-pyrido[3,2-b][1,4]oxazin-3-one
6-[6-oxo-2-[[rac-(1R,2R)-1-amino-4-chloro-2,3-dihydro-1H-inden-2-yl]methylamino]-5-oxa-7-azaspiro [3.4] octan-7-yl] -4H-pyrido [3,2-b] [1,4] oxazin-3 -one
6-[2-oxo-8-[[rac-1,2-trans-1-amino-4-chloro-2,3-dihydro-1H-inden-2-yl]methyl]-1-oxa-3,8-diazaspiro[4.5]decan-3-yl]-4H-pyrido[3,2-b][1,4]oxazin-3-one
3-[(1-aminocyclopropyl)methoxy]-4-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile
3-[(1-aminocyclopropyl)methoxy]-1-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-4-carbonitrile
3-[(1-aminocyclopropyl)methoxy]-1-methyl-6-[[2-oxo-3-(3-oxo-4H-pyrido[3,2-b][1,4]oxazin-6-yl)-1-oxa-3,8-diazaspiro[4.5]decan-8-yl]methyl]-6,7-dihydro-5H-cyclopenta[c]pyridine-4-carbonitrile
6-[5-[2-[[3-[(1-aminocyclopropyl)methoxy]-4-methyl-1-(5-methyl-1,3-thiazol-2-yl)-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-[(1-aminocyclopropyl)methoxy]-4-methyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[3-[(1-aminocyclopropyl)methoxy]-4-methyl-1-(1,3-thiazol-2-yl)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[4-methyl-3-[[(2R)-azetidin-2-yl]methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
4-methyl-6-((2-oxo-3-(3-oxo-3,4-dihydro-2H-pyrido[3,2-b][1,4]oxazin-6-yl)-1-oxa-3,8-diazaspiro[4.5]decan-8-yl)methyl)-3-((5-oxopiperazin-2-yl)methoxy)-6,7-dihydro-5H-cyclopenta [c]pyridine-1-carbonitrile
2-((1-cyano-4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-SH-cyclopenta[c]pyridin-3-yl)oxy)-N-methylacetamide
2-((1-cyano-4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-SH-cyclopenta[c]pyridin-3-yl)oxy)-N-isopropylacetamide
N-methyl-2-((4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-SH-cyclopenta[c]pyridin-3-yl)oxy)acetamide
N-isopropyl-2-((4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-SH-cyclopenta[c]pyridin-3-yl)oxy)acetamide
ethyl 1-ethyl-6-fluoro-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylate
6-[2-oxo-5-[2-[[rac-1,2-trans-1-amino-4-chloro-2,3-dihydro-1H-inden-2-yl]methylamino]ethyl]-1,3-oxazolidin-3-yl] -4H-pyrido [3,2-b] [1,4] oxazin-3 -one
6-[2-oxo-5-[3-[[rac-1,2-trans-1-amino-4-chloro-2,3-dihydro-1H-inden-2-yl]methylamino]propyl]-1,3-oxazolidin-3-yl]-4H-pyrido[3,2-b][1,4]oxazin-3-one
rac-1,2-trans-1-amino-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile
rac-1,2-trans-1-amino-2-((((2-oxo-3-(3-oxo-3,4-dihydro-2H-pyrido[3,2-b][1,4]oxazin-6-yl)oxazolidin-5-yl)methyl)amino)methyl)-2,3-dihydro-1H-indene-4-carbonitrile
6-[2-oxo-5-[2-[[rac-1,2-trans-4-chloro-1-hydroxy-2,3-dihydro-1H-inden-2-yl]methylamino]ethyl]-1,3-oxazolidin-3-yl]-4H-pyrido[3,2-b][1,4]oxazin-3-one
6-[2-oxo-5-[2-[[rac-1,2-cis-4-chloro-1-hydroxy-2,3-dihydro-1H-inden-2-yl]methylamino]ethyl]- 1,3-oxazolidin-3-yl] -4H-pyrido[3,2-b] [1,4] oxazin-3 -one
6-[5-[2-[(4-chloro-2-hydroxy-1,3-dihydroinden-2-yl)methylamino]ethyl]-2-oxo-1,3-oxazolidin-3-yl]-4H-pyrido[3,2-b][1,4]oxazin-3-one; formic acid
2-[[2-aminoethyl-[2-[2-oxo-3-(3-oxo-4H-pyrido[3,2-b] [1,4]oxazin-6-yl)-1,3-oxazolidin-5-yl]ethyl]amino]methyl]-2,3-dihydro-1H-indene-4-carbonitrile; formic acid
6-[5-[2-[[1,4-dimethyl-3-[(2R)-2-amino-3-hydroxypropoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
3-[(2R)-2-amino-3-hydroxypropoxy]-4-methyl-6-[[2-[2-oxo-3-(3-oxo-4H-pyrido[3,2-b] [1,4]oxazin-6-yl)-1,3-oxazolidin-5-yl]ethylamino]methyl]-6,7-dihydro-5H-cyclopenta[c]pyridine-1-carbonitrile
6-[(2R)-2-amino-3-hydroxypropoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile
6-[5-[2-[[4-chloro-3-[rac-(2R)-2-amino-3-hydroxypropoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
2-[[2-Hydroxy-3-[2-oxo-3-(3-oxo-4H-pyrido[3,2-b] [1,4]oxazin-6-yl)-1,3-oxazolidin-5-yl] propyl] amino]-2,3-dihydro-1H-indene-4-carbonitrile hydrochloride
1-ethyl-6-fluoro-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylic acid
6-[5-[2-[(2-amino-4-chloro-1,3-dihydroinden-2-yl)methylamino]ethyl]-2-oxo-1,3-oxazolidin-3-yl]-4H-pyrido[3,2-b][1,4]oxazin-3-one; formic acid
2-[[rac-5,6-trans-5,8-cis-6-Amino-2-oxo-3-(3-oxo-4H-pyrido[3,2-b] [1,4]oxazin-6-yl)-1-oxa-3-azaspiro[4.5]decan-8-yl]amino]-2,3-dihydro-1H-indene-4-carbonitrile; formic acid
6-[5-(aminomethyl)-5-[2-[(4-fluoro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one
2-[[2-[5-(aminomethyl)-2-oxo-3-(3-oxo-4H-pyrido[3,2-b] [1,4]oxazin-6-yl)-1,3-oxazolidin-5-yl]ethylamino]methyl]-2,3-dihydro-1H-indene-4-carbonitrile
6-[2-oxo-5-[3-[[rac-1,2-trans-1-amino-4-chloro-2,3-dihydro-1H-inden-2-yl]amino]propyl]-1,3-oxazolidin-3-yl] -4H-pyrido [3,2-b] [1,4] oxazin-3 -one
6-[2-oxo-5-[3-[[rac-1,2-trans-1-amino-4-chloro-2,3-dihydro-1H-inden-2-yl]amino]propyl]-1,3-oxazolidin-3-yl]-4H-pyrazino[2,3-b][1,4]oxazin-3-one
5-[2-[(rac-1,2-trans-1-amino-4-chloro-2,3-dihydro-1H-inden-2-yl)methylamino]ethyl]-3-(3-oxo-4H-pyrido[3,2-b][1,4]thiazin-6-yl)-1,3-oxazolidin-2-one
rac-1,2-trans-1-amino-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-indene-4-carbonitrile
6-[5-[2-[[6-(2-amino-3-methoxypropoxy)-4-fluoro-2,3-dihydro-1H-inden-2-yl]methylamino]ethyl]-2-oxo-1,3-oxazolidin-3-yl]-4H-pyrido[3,2-b][1,4]oxazin-3-one
2-[[rac-5,6-trans-6-(Aminomethyl)-2-oxo-3-(3-oxo-4H-pyrido[3,2-b] [1,4]oxazin-6-yl)-1-oxa-3,8-diazaspiro[4.5]decan-8-yl]methyl]-2,3-dihydro-1H-indene-4-carbonitrile; formic acid
6-[rac-5,6-trans-6-(aminomethyl)-8-[(4-fluoro-1-methyl-6,7-dihydro-5H-cyclopenta[c]pyridin-6-yl)methyl]-2-oxo-1-oxa-3,8-diazaspiro[4.5]decan-3-yl]-4H-pyrido[3,2-b] [1,4]oxazin-3-one
6-[rac-5,6-cis-6-(aminomethyl)-8-[(4-fluoro-1-methyl-6,7-dihydro-SH-cyclopenta[c]pyridin-6-yl)methyl]-2-oxo-1-oxa-3,8-diazaspiro[4.5]decan-3-yl]-4H-pyrido[3,2-b] [1,4]oxazin-3-one
2-[[rac-5,6-cis-6-(Aminomethyl)-2-oxo-3-(3-oxo-4H-pyrido[3,2-b] [1,4]oxazin-6-yl)-1-oxa-3,8-diazaspiro[4.5]decan-8-yl]methyl]-2,3-dihydro-1H-indene-4-carbonitrile; formic acid
6-[5-[2-[[5-[(1-aminocyclopropyl)methoxy]-4,7-dimethyl-2,3-dihydro-1H-inden-2-yl]methylamino]ethyl]-2-oxo-1,3-oxazolidin-3-yl]-4H-pyrazino[2,3-b][1,4]oxazin-3-one;formic acid
2-[[2-[2-oxo-3-(3-oxo-4H-pyrido[3,2-b] [1,4]oxazin-6-yl)-1,3-oxazolidin-5-yl]ethylamino]methyl]-6-pyrrolidin-3-yloxy-2,3-dihydro-1H-indene-4-carbonitrile; formic acid
2-amino-3-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylpropanamide; formic acid
6-[5-[2-[(1-chloro-4-fluoro-3-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one
6-[8-[[6-[(1-aminocyclopropyl)methoxy]-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one; formic acid
6-[8-[(4-fluoro-1-methyl-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-one
6-[2-oxo-8-[[rac-1,2-trans-1-amino-4-chloro-2,3-dihydro-1H-inden-2-yl]methyl]-1-oxa-3,8-diazaspiro[4.5]decan-3-yl]-4H-pyrazino[2,3-b][1,4]oxazin-3-one; formic acid
6-[8-[[6-(2-aminopropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one; formic acid
2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-indene-4-carbonitrile; formic acid
6-[5-[2-[(rac-5,6-trans-5-amino-4-chloro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;formic acid
6-[2-oxo-5-[2-[[rac-5,6-trans-5-amino-4-chloro-1-methyl-6,7-dihydro-5H-cyclopenta[c]pyridin-6-yl]methylamino]ethyl]-1,3-oxazolidin-3-yl]-4H-pyrazino[2,3-b][1,4]oxazin-3-one
3-(3-oxo-4H-pyrido[3,2-b][1,4]thiazin-6-yl)-5-[2-[[rac-5,6-trans-5-amino-4-chloro-1-methyl-6,7-dihydro-5H-cyclopenta[c]pyridin-6-yl]methylamino]ethyl]-1,3-oxazolidin-2-one
6-[5-[[2-(4-fluoro-1-methyl-6,7-dihydro-5H-cyclopenta[c]pyridin-6-yl)ethylamino]methyl]-2-oxo-1,3-oxazolidin-3-yl]-4H-pyrazino[2,3-b][1,4]oxazin-3-one; formic acid
6-[2-oxo-5-[[2-[rac-1,2-trans-1-amino-4-chloro-2,3-dihydro-1H-inden-2-yl]ethylamino]methyl]-1,3-oxazolidin-3-yl]-4H-pyrazino[2,3-b][1,4]oxazin-3-one
2-amino-3-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]propanoic acid; formic acid
6-[5-[2-[(1,4-dimethyl-6,7-dihydro-5H-cyclopenta[c]pyridin-6-yl)methylamino]ethyl]-2-oxo-1,3-oxazolidin-3-yl] -4H-pyrazino[2,3-b] [1,4] oxazin-3 -one
6-[2-oxo-5-[2-[(1,3,4-trimethyl-6,7-dihydro-5H-cyclopenta[c]pyridin-6-yl)methylamino]ethyl]-1,3-oxazolidin-3-yl]-4H-pyrazino[2,3-b][1,4]oxazin-3-one
6-[5-[2-[(4-methyl-6,7-dihydro-5H-cyclopenta[b]pyridin-6-yl)methylamino]ethyl]-2-oxo-l,3-oxazolidin-3-yl]-4H-pyrazino[2,3-b][1,4]oxazin-3-one
6-[5-[2-[[6-[2-(dimethylamino)ethoxy]-4-fluoro-2,3-dihydro-1H-inden-2-yl]methylamino]ethyl]-2-oxo-1,3-oxazolidin-3-yl]-4H-pyrido[3,2-b] [1,4] oxazin-3 -one; formic acid
6-[5-[2-[[6-[2-(dimethylamino)ethoxy]-4-fluoro-2,3-dihydro-1H-inden-2-yl]methylamino]ethyl]-2-oxo-1,3-oxazolidin-3-yl]-4H-pyrazino[2,3-b] [1,4] oxazin-3 -one; formic acid
6-[2-oxo-5-[2-[[rac-1,2-trans-1-amino-6-[(1-aminocyclopropyl)methoxy]-4-chloro-2,3-dihydro-1H-inden-2-yl]methylamino]ethyl]-1,3-oxazolidin-3-yl]-4H-pyrido[3,2-b][1,4]oxazin-3-one; formic acid
6-[2-oxo-8-[[rac-1,2-trans-1-amino-6-[(1-aminocyclopropyl)methoxy]-4-chloro-2,3-dihydro-1H-inden-2-yl]methyl]-1-oxa-3,8-diazaspiro[4.5]decan-3-yl]-4H-pyrido[3,2-b][1,4]oxazin-3-one; formic acid
1-ethyl-4-oxo-8-[[2-[2-oxo-3-(3-oxo-4H-pyrido[3,2-b] [1,4]oxazin-6-yl)-1,3-oxazolidin-5-yl]ethylamino]methyl]-8,9-dihydro-7H-cyclopenta[h]quinoline-3-carboxylic acid
1-ethyl-4-oxo-8-[[2-[2-oxo-3-(3-oxo-4H-pyrazino[2,3-b][1,4]oxazin-6-yl)-1,3-oxazolidin-5-yl]ethylamino]methyl]-8,9-dihydro-7H-cyclopenta[h]quinoline-3-carboxylic acid
6-[8-[(4-fluoro-1-methyl-6,7-dihydro-5H-cyclopenta[c]pyridin-6-yl)methyl]-6-(hydroxymethyl)-2-oxo-1-oxa-3,8-diazaspiro[4.5]decan-3-yl]-4H-pyrido[3,2-b][1,4]oxazin-3-one
6-[5-[[2-(4-fluoro-1-methyl-6,7-dihydro-5H-cyclopenta[c]pyridin-6-yl)ethylamino]methyl]-5-(2-hydroxyethyl)-2-oxo-1,3-oxazolidin-3-yl]-4H-pyrido[3,2-b][1,4]oxazin-3-one
6-[5-[2-[(1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;formic acid
6-[5-[[2-(6-amino-4-fluoro-1-methyl-5,7-dihydrocyclopenta[c]pyridin-6-yl)ethylamino]methyl]-2-oxo-1,3-oxazolidin-3-yl]-4H-pyrido[3,2-b] [1,4] oxazin-3 -one
6-[5-[2-[(4-chloro-2,3-dihydro-1H-inden-2-yl)methylamino]ethyl]-2-oxo-1,3-oxazolidin-3-yl]-4H-pyrazino[2,3-b][1,4]oxazin-3-one
6-[2-Oxo-5-[2-[[rac-1,2-trans-1-amino-6-[(1-aminocyclopropyl)methoxy]-4-chloro-2,3-dihydro-1H-inden-2-yl]methylamino]ethyl]-1,3-oxazolidin-3-yl]-4H-pyrazino[2,3-b][1,4]oxazin-3- one;formic acid
6-[5-[2-[(1,4-dimethyl-6,7-dihydro-5H-cyclopenta[d]pyridazin-6-yl)methylamino]ethyl]-2-oxo-1,3-oxazolidin-3-yl]-4H-pyrazino[2,3-b][1,4]oxazin-3-one
6-[5-[2-[(1,4-dimethyl-6,7-dihydro-5H-cyclopenta[d]pyridazin-6-yl)methylamino]ethyl]-2-oxo-1,3-oxazolidin-3-yl]-4H-pyrido[3,2-b][1,4]oxazin-3-one
5-[2-[(1,4-dimethyl-6,7-dihydro-5H-cyclopenta[d]pyridazin-6-yl)methylamino]ethyl]-3-(3-oxo-4H-pyrido[3,2-b] [1,4]thiazin-6-yl)-1,3-oxazolidin-2-one
2-[[4-cyano-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide
6-[5-[2-[(4-fluoro-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one
6-[5-[2-[(4-fluoro-6,7-dihydro-5H-cyclopenta[c]pyridin-6-yl)methylamino]ethyl] -2-oxo-1,3-oxazolidin-3-yl]-4H-pyrido[3,2-b][1,4]oxazin-3-one
2-[[2-[2-oxo-3-(7-oxo-8H-pyrimido[5,4-b][1,4]oxazin-2-yl)-1,3-oxazolidin-5-yl]ethylamino]methyl]-2,3-dihydro-1H-indene-4-carbonitrile
2-[5-[2-[(rac-1,2-trans-1-amino-4-chloro-2,3-dihydro-1H-inden-2-yl)methylamino]ethyl]-2-oxo-1,3-oxazolidin-3-yl]-8H-pyrimido[5,4-b] [1,4]oxazin-7-one
1-methyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridine-4-carbonitrile;2,2,2-trifluoroacetic acid
6-[5-[2-[[4-fluoro-6-(2-hydroxy-2-methylpropoxy)-2,3-dihydro-1H-inden-2-yl]methylamino]ethyl]-2-oxo-1,3-oxazolidin-3-yl]-4H-pyrazino[2,3-b][1,4]oxazin-3-one
6-[5-[2-[(4-fluoro-1-methyl-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-one
6-[5-[2-[[6-[2-(5-aminotetrazol-1-yl)ethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[[6-[2-(5-aminotetrazol-2-yl)ethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[[4-fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
trans-6-[5-[2-[[4-Fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
cis-6-[5-[2-[[4-Fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[(3-fluoro-4-methyl-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-one
6-[5-[2-[(3-fluoro-4-methyl-6,7-dihydro-5H-cyclopenta[b]pyridin-6-yl)methylamino]ethyl]-2-oxo-1,3-oxazolidin-3-yl]-4H-pyrido[3,2-b] [1,4]oxazin-3-one
6-[8-[(3-fluoro-4-methyl-6,7-dihydro-5H-cyclopenta[b]pyridin-6-yl)methyl]-2-oxo-1-oxa-3,8-diazaspiro[4.5]decan-3-yl]-4H-pyrazino[2,3-b][1,4]oxazin-3-one
6-[5-[3-[(4-fluoro-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid
6-[5-[3-[(4-fluoro-l-methyl-6,7-dihydro-5H-cyclopenta[c]pyridin-6-yl)-methylamino]propyl]-2-oxo-1,3-oxazol-3-yl]-4H-pyrazino[2,3-b][1,4]oxazin-3-one;formic acid
6-[5-[2-[[4-fluoro-6-[2-(2-oxo-1,3-oxazolidin-3-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
2-[[7-fluoro-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]acetamide
2-[[7-fluoro-2-[[2-[2-oxo-3-(3-oxo-4H-pyrazino[2,3-b] [1,4]oxazin-6-yl)-1,3-oxazol-5-yl]ethylamino]methyl]-2,3-dihydro-1H-inden-5-yl]oxy]acetamide;formic acid
6-[5-[2-[[6-[2-(azetidin-1-yl)-2-oxoethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid
N-ethyl-2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide;formic acid
2-[[7-fluoro-2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-inden-5-yl]oxy]-N-methylacetamide
2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;formic acid
2-[[7-fluoro-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]-N-methoxyacetamide;formic acid 2-[[4,7-difluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide
6-[2-cxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer A
6-[2-oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer B
6-[2-oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer C
6-[2-oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer D
6-[5-[2-[(4-fluoro-6-hydroxy-2,3-dihydro-1H-inden-2-yl)methylamino]ethyl]-2-oxo-1,3-oxazolidin-3-yl]-4H-pyrazino[2,3-b][1,4]oxazin-3-one
6-[5-[2-[(4-fluoro-6,7-dihydro-SH-cyclopenta[b]pyridin-6-yl)methylamino]ethyl]-2-oxo-1,3-oxazolidin-3-yl]-4H-pyrazino[2,3-b][1,4]oxazin-3-one
6-[5-[2-[(4-chloro-3-fluoro-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-one;formic acid
6-[5-[2-[[4-fluoro-6-(1H-pyrazol-3-ylmethoxy)-2,3-dihydro-1H-inden-2-yl]methylamino]ethyl]-2-oxo-1,3-oxazolidin-3-yl]-4H-pyrazino[2,3-b][1,4]oxazin-3-one;formic acid
1-methyl-6-[3-[2-oxo-3-(3-oxo-4H-pyrazino[2,3-b] [1,4]oxazin-6-yl)-1,3-oxazol-5-yl]propylamino]-6,7-dihydro-SH-cyclopenta[c]pyridine-4-carbonitrile, Enantiomer A
1-methyl-6-[3-[2-oxo-3-(3-oxo-4H-pyrazino[2,3-b] [1,4]oxazin-6-yl)-1,3-oxazol-5-yl]propylamino]-6,7-dihydro-SH-cyclopenta[c]pyridine-4-carbonitrile, Enantiomer B
6-[5-[2-[[4-fluoro-6-(oxetan-3-yloxy)-2,3-dihydro-1H-inden-2-yl]methylamino]ethyl]-2-oxo-1,3-oxazolidin-3-yl] -4H-pyrazino[2,3-b] [1,4] oxazin-3 -one
N-[2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]ethyl]methanesulfonamide
N-[2-[[7-fluoro-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]ethyl]-N-methylmethanesulfonamide
6-[2-[2-(4-fluoro-1-methyl-6,7-dihydro-5H-cyclopenta[c]pyridin-6-yl)ethyl]-6-oxo-5-oxa-2,7-diazaspiro [3.4] octan-7-yl] -4H-pyrazino [2,3 -b] [1,4] oxazin-3 -one
6-[2-[2-[6-(2-aminopropoxy)-4-fluoro-2,3-dihydro-1H-inden-2-yl]ethyl]-6-oxo-5-oxa-2,7-diazaspiro[3.4]octan-7-yl]-4H-pyrazino[2,3-b] [1,4]oxazin-3-one
6-[5-[3-[(4-fluoro-1-methyl-6,7-dihydro-5H-cyclopenta[c]pyridin-6-yl)amino]cyclobutyl]-2-oxo-1,3 -oxazolidin-3 -yl] -4H-pyrazino [2,3 -b] [1,4] oxazin-3 -one, Isomer A
6-[5-[3-[(4-fluoro-1-methyl-6,7-dihydro-5H-cyclopenta[c]pyridin-6-yl)amino]cyclobutyl]-2-oxo-1,3 -oxazolidin-3 -yl] -4H-pyrazino [2,3 -b] [1,4] oxazin-3 -one, Isomer B
6-[(5S)-5-[2-[1-(4-fluoro-1-methyl-6,7-dihydro-5H-cyclopenta[c]pyridin-6-yl)ethylamino]ethyl]-2-oxo-1,3-oxazolidin-3-yl]-4H-pyrazino[2,3-b][1,4]oxazin-3-one; formic acid
6-[(5R)-5-[2-[1-(4-fluoro-1-methyl-6,7-dihydro-5H-cyclopenta[c]pyridin-6-yl)ethylamino]ethyl]-2-oxo-1,3-oxazolidin-3-yl]-4H-pyrazino[2,3-b][1,4]oxazin-3-one; formic acid
6-[5-[2-[[4-fluoro-6-(2-imidazol-1-ylethoxy)-2,3-dihydro-1H-inden-2-yl]methylamino]ethyl]-2-oxo-1,3-oxazolidin-3-yl]-4H-pyrazino[2,3-b][1,4]oxazin-3-one
6-[5-[3-[(4-fluoro-1-methyl-6,7-dihydro-5H-cyclopenta[c]pyridin-6-yl)amino]butyl]-2-oxo-1,3-oxazolidin-3-yl]-4H-pyrazino[2,3-b][1,4]oxazin-3-one, Isomer A
6-[5-[3-[(4-fluoro-1-methyl-6,7-dihydro-5H-cyclopenta[c]pyridin-6-yl)amino]butyl]-2-oxo-1,3-oxazolidin-3-yl]-4H-pyrazino[2,3-b][1,4]oxazin-3-one, Isomer B
6-[5-[2-[(4-fluoro-1-methyl-6,7-dihydro-5H-cyclopenta[c]pyridin-6-yl)methylamino]ethyl]-2-oxo-1,3,4-oxadiazol-3-yl]-4H-pyrazino[2,3-b][1,4]oxazin-3-one
2-[[4-fluoro-2-[[2-oxo-3-(3-oxo-4H-pyrazino[2,3-b] [1,4]oxazin-6-yl)-1-oxa-3,8-diazaspiro[4.5]decan-8-yl]methyl]-2,3-dihydro-1H-inden-5-yl]oxy]-N-methylacetamide
6-[2-oxo-5-[2-[[rac-1,2-trans-1-amino-4-chloro-2,3-dihydro-1H-inden-2-yl]methylamino]ethyl]-1,3,4-oxadiazol-3-yl]-4H-pyrazino[2,3-b][1,4]oxazin-3-one; formic acid
6-[(5S)-5-[2-[(4-fluoro-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4] oxazin-3 -one
6-[(5R)-5-[2-[(4-fluoro-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one
6-[5-[2-[(4-fluoro-1-methyl-6,7-dihydro-5H-cyclopenta[c]pyridin-6-yl)methylamino]ethyl]-5-methyl-2-oxo-1,3-oxazolidin-3-yl]-4H-pyrazino[2,3-b] [1,4]oxazin-3-one
6-[(5S)-5-[2-[[6-(2-amino-2-methylpropoxy)-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[(5R)-5-[2-[[6-(2-amino-2-methylpropoxy)-4-fluoro-2,3-dihydro-1H-inden-2-yl]methylamino]ethyl]-2-oxo-1,3-oxazolidin-3-yl]-4H-pyrazino[2,3-b][1,4]oxazin-3-one; formic acid
6-[5-[2-[[4-fluoro-6-[2-(triazol-2-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[[4-fluoro-6-[2-(triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
1-[2-[[7-fluoro-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]ethyl]pyrrolidine-2,5-dione;formic acid
N-cyclopropyl-2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide;formic acid
6-[5-[2-[[4-fluoro-6-[2-(tetrazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[[4-fluoro-6-[2-(tetrazol-2-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid
2-[4-[2-oxo-3-(3-oxo-4H-pyrazino[2,3-b] [1,4]oxazin-6-yl)-1,3-oxazolidin-5-yl]piperidin-1-yl]-2,3-dihydro-1H-indene-4-carbonitrile
6-[5-[1-(4-fluoro-2,3-dihydro-1H-inden-2-yl)piperidin-4-yl]-2-oxo-1,3-oxazolidin-3-yl]-4H-pyrazino[2,3-b][1,4]oxazin-3-one
6-[5-[2-[[4-Fluoro-6-(2-hydroxypropoxy)-2,3-dihydro-1H-inden-2-yl]methylamino]ethyl]-2-oxo-1,3-oxazolidin-3-yl]-4H-pyrazino[2,3-b][1,4]oxazin-3-one;hydrochloride
6-[5-[2-[(4-fluoro-1,3-dimethyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;formic acid
6-[5-[2-[[4-fluoro-6-(2-methylsulfonylethoxy)-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[[6-(1-acetylazetidin-3-yl)oxy-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[[4-fluoro-6-(1-methylsulfonylazetidin-3-yl)oxy-2,3-dihydro-1H-inden-2-yl]methylamino]ethyl]-2-oxo-1,3-oxazolidin-3-yl]-4H-pyrazino[2,3-b][1,4]oxazin-3- one;hydrochloride
6-[8-[[6-[(2S)-2-aminopropoxy]-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid
6-[8-[[6-[(2R)-2-aminopropoxy]-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid
6-[5-[2-[[6-(azetidin-3-yloxy)-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;hydrochloride
6-[5-[2-[(rac-1,2-trans-1-amino-4-chloro-2,3-dihydro-1H-inden-2-yl)methylamino]ethyl]-2-oxo-1,3-oxazol-3-yl]-4H-pyrazino[2,3-b][1,4]oxazin-3-one;formic acid
6-[(5R)-5-[(1R)-2-[(4-fluoro-1-methyl-6,7-dihydro-5H-cyclopenta[c]pyridin-6-yl)methylamino]-1-hydroxyethyl]-2-oxo-1,3-oxazolidin-3-yl]-4H-pyrazino[2,3-b] [1,4]oxazin-3-one;formic acid
6-[5-[trans-3-[(4-fluoro-1-methyl-6,7-dihydro-5H-cyclopenta[c]pyridin-6-yl)methylamino]cyclobutyl]-2-oxo-1,3-oxazolidin-3-yl]-4H-pyrazino[2,3-b] [1,4]oxazin-3-one
6-[5-[cis-3-[(4-fluoro-1-methyl-6,7-dihydro-5H-cyclopenta[c]pyridin-6-yl)methylamino]cyclobutyl]-2-oxo-1,3-oxazolidin-3-yl]-4H-pyrazino[2,3-b][1,4]oxazin-3-one
4-fluoro-3-methyl-6-[[2-[2-oxo-3-(3-oxo-4H-pyrazino[2,3-b] [1,4]oxazin-6-yl)-1,3-oxazolidin-5-yl]ethylamino]methyl]-6,7-dihydro-5H-cyclopenta[c]pyridine-1-carbonitrile;formic acid
1-methyl-6-[[2-[(5S)-2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridine-4-carbonitrile
1-methyl-6-[[2-[(5R)-2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridine-4-carbonitrile
2-[[7-fluoro-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]acetonitrile;formic acid
6-[5-[3-[(4-fluoro-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one, Enantiomer A
6-[5-[3-[(4-fluoro-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one, Enantiomer B
6-[5-[3-[(1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid, Enantiomer A
6-[5-[3-[(1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid, Enantiomer B
6-[5-[2-[(1-methyl-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-one
6-[5-[2-[(4-fluoro-6,7-dihydro-5H-cyclopenta[c]pyridin-6-yl)methylamino]ethyl] -2-oxo-1,3-oxazol-3-yl]-4H-pyrazino[2,3-b][1,4]oxazin-3-one
6-[8-[(1-chloro-6,7-dihydro-SH-cyclopenta[c]pyridin-6-yl)methyl]-2-oxo-1-oxa-3,8-diazaspiro[4.5]decan-3-yl]-4H-pyrazino[2,3-b][1,4]oxazin-3-one
6-[8-[(1-methyl-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-one
6-[5-methyl-2-oxo-5-[2-[(rac-1,2-trans-1-amino-4-chloro-2,3-dihydro-1H-inden-2-yl)methylamino]ethyl]-1,3-oxazolidin-3-yl]-4H-pyrazino[2,3-b][1,4]oxazin-3-one
6-[5-[2-[(4-Fluoro-1-methyl-6,7-dihydro-5H-cyclopenta[c]pyridin-6-yl)methyl-methylamino]ethyl]-2-oxo-1,3-oxazolidin-3-yl]-4H-pyrazino[2,3-b][1,4]oxazin-3-one
6-[8-[[6-(2-amino-2-methylpropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid, Enantiomer A
6-[8-[[6-(2-amino-2-methylpropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid, Enantiomer B
2-[[7-fluoro-2-[[2-[(5S)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer A
2-[[7-fluoro-2-[[2-[(SS)-2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]acetamide, Isomer B
2-[[7-fluoro-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer A
2-[[7-fluoro-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer B
2-[[7-cyano-4-methyl-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]-N-methylacetamide
2-[[4-cyano-7-methyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide
6-[5-[2-[(2-chloro-4-methyl-6,7-dihydro-5H-cyclopenta[d]pyrimidin-6-yl)methylamino]ethyl]-2-oxo-1,3-oxazolidin-3-yl]-4H-pyrido[3,2-b][1,4]oxazin-3-one
6-(1,3-oxazol-4-ylmethoxy)-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile
6-[(3-methyl-1,2,4-oxadiazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile
6-[(1-methylpyrazol-3-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile
6-[(2-methyltetrazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile
6-[(1-methylpyrazol-4-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile
6-[(1-methyltetrazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile
6-[(4-methyl-1,2,5-oxadiazol-3-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile
2-[[4,7-difluoro-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]-N-methylacetamide
2-[[4,7-difluoro-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-inden-5-yl]oxy]-N-methylacetamide
6-[5-[2-[[4,7-difluoro-5-(1H-pyrazol-3-ylmethoxy)-2,3-dihydro-1H-inden-2-yl]methylamino]ethyl]-2-oxo-1,3-oxazolidin-3-yl]-4H-pyrazino[2,3-b][1,4]oxazin-3-one
6-[8-[[4,7-difluoro-5-(1H-pyrazol-3-ylmethoxy)-2,3-dihydro-1H-inden-2-yl]methyl]-2-oxo-1-oxa-3,8-diazaspiro[4.5]decan-3-yl]-4H-pyrazino[2,3-b] [1,4]oxazin-3-one
6-[5-[2-[(4-fluoro-3-methoxy-1-methyl-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-one
6-[5-[2-[(3-chloro-4-fluoro-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one
2-[[rac-2,3-trans-3-Amino-7-chloro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;formic acid
N-methyl-2-[[rac-2,3-trans-3-amino-7-chloro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]acetamide; formic acid
6-[(5S)-5-[2-[[(1R,2R)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one
6-[(5S)-5-[2-[[(1S,2S)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one
6-[(5R)-5-[2-[[(1R,2R)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one
2-[[2-[2-oxo-3-(3-oxo-4H-pyrazino[2,3-b] [1,4]oxazin-6-yl)-1,3-oxazolidin-5-yl]ethylamino]methyl]-6-(1H-pyrazol-3-ylmethoxy)-2,3-dihydro-1H-indene-4-carbonitrile; formic acid
2-[[2-[2-oxo-3-(3-oxo-4H-pyrazino[2,3-b] [1,4]oxazin-6-yl)-1,3-oxazolidin-5-yl]ethylamino]methyl]-6-(1H-pyrazol-4-ylmethoxy)-2,3-dihydro-1H-indene-4-carbonitrile; formic acid
6-(1H-imidazol-5-ylmethoxy)-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-indene-4-carbonitrile; formic acid
6-(2-amino-2-methylpropoxy)-2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-indene-4-carbonitrile
6-[5-[2-[[5-[(1-aminocyclopropyl)methoxy]-4,7-difluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[5-[2-[[5-[(1-aminocyclopropyl)methoxy]-4,7-difluoro-2,3-dihydro-1H-inden-2-yl]methylamino]ethyl]-2-oxo-1,3-oxazolidin-3-yl]-4H-pyrido[3,2-b][1,4]oxazin-3-one;formic acid
6-[8-[[5-[(1-aminocyclopropyl)methoxy]-4,7-difluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid
6-[5-[2-[[5-(2-amino-2-methylpropoxy)-4,7-difluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid
6-[8-[[5-(2-amino-2-methylpropoxy)-4,7-difluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid
2-[[4-fluoro-2-[[2-[2-oxo-3-(3-oxo-4H-pyrazino[2,3-b][1,4]oxazin-6-yl)-1,3-oxazolidin-5-yl] ethylamino] methyl] -2,3 -dihydro-1 H-inden-5-yl] oxy] acetamide
2-[[7-cyano-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]acetamide
6-[5-[2-[[1-methyl-3-(1H-pyrazol-3-ylmethoxy)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one
6-[5-[2-[(5,8-dimethyl-5,7-diazatricyclo[7.3.0.02,6]dodeca-1,6,8-trien-11-yl)methylamino]ethyl]-2-oxo-1,3-oxazolidin-3-yl]-4H-pyrido[3,2-b][1,4]oxazin-3-one
6-[4-[2-[(4-fluoro-1-methyl-6,7-dihydro-5H-cyclopenta[c]pyridin-6-yl)methylamino]ethyl]-2-oxopyrrolidin-1-yl]-4H-pyrido[3,2-b][1,4]oxazin-3-one
6-[5-[2-(6,7-dihydro-5H-cyclopenta[c]pyridin-6-ylmethylamino)ethyl]-2-oxo-1,3-oxazolidin-3-yl]-4H-pyrido[3,2-b][1,4]oxazin-3-one
6-[5-[2-[[3-(dimethylamino)-1-methyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[[1-methyl-3-(methylamino)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one
6-[5-[2-[(4-methyl-6,7-dihydro-5H-cyclopenta[d]pyrimidin-6-yl)methylamino]ethyl]-2-oxo-1,3-oxazolidin-3-yl] -4H-pyrido [3,2-b] [1,4] oxazin-3 -one
6-[(5R)-5-[2-[[(1S,2S)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one
6-[5-[2-[(8-methyl-5,7-diazatricyclo[7.3.0.02,6]dodeca-1,6,8-trien-11-yl)methylamino]ethyl]-2-oxo-1,3-oxazolidin-3-yl]-4H-pyrido[3,2-b][1,4]oxazin-3-one
6-[5-[2-[(5,8-dimethyl-5,7-diazatricyclo[7.3.0.02,6]dodeca-1,6,8-trien-1 1-yl)methylamino]ethyl]-2-oxo-1,3-oxazolidin-3-yl]-4H-pyrazino[2,3-b][1,4]oxazin-3-one
2-[[2-[2-oxo-3-(3-oxo-4H-pyrazino[2,3-b] [1,4]oxazin-6-yl)-1,3-oxazolidin-5-yl]ethylamino]methyl]-6-(1H-triazol-5-ylmethoxy)-2,3-dihydro-1H-indene-4-carbonitrile
6-[5-[2-[[4-fluoro-1-(1H-pyrazol-5-yl)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one
6-[(5R)-5-[2-[[(6R)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one
6-[(5S)-5-[2-[[(6R)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one
6-[(5S)-5-[2-[[(6S)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one
6-[(5R)-5-[2-[[(6S)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one
6-[5-[2-[(1-chloro-4-fluoro-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;formic acid;
6-[5-[2-[[4-fluoro-1-(1H-pyrazol-4-yl)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one;
6-[5-[2-[[4-fluoro-1-(1H-imidazol-5-yl)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one

Further particular examples of compounds of formula (I) as described herein are selected from
6-[5-[2-[(1-chloro-6,7-dihydro-SH-cyclopenta[c]pyridin-6-yl)methylamino]ethyl]-2-oxo-1,3-oxazolidin-3-yl]-4*H*-pyrazino[2,3-b][1,4]oxazin-3-one;
6-[5-[2-[(4-fluoro-1-methyl-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-one;
2-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide; formic acid;
6-[5-[2-[(1-methyl-6,7-dihydro-5*H*-cyclopenta[c]pyridin-6-yl)methylamino]ethyl]-2-oxo-1,3-oxazolidin-3-yl]-4*H*-pyrido[3,2-b][1,4]oxazin-3-one;
6-[2-oxo-5-[2-[[*rac*-1,2-*trans*-1-amino-4-chloro-2,3-dihydro-1*H*-inden-2-yl]methylamino]ethyl]-1,3-oxazolidin-3-yl]-4H-pyrazino[2,3-b][1,4]oxazin-3-one; formic acid;
6-[5-[2-[[6-[(1-aminocyclopropyl)methoxy]-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;
6-[(1-aminocyclopropyl)methoxy]-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile;
6-(2-aminopropoxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile; 2,2,2-trifluoroacetic acid ;
6-[2-oxo-5-[2-[[*rac*-1,2-*trans*-1-amino-4-chloro-2,3-dihydro-1*H*-inden-2-yl]methylamino]ethyl]-1,3-oxazolidin-3-yl]-4*H*-pyrido[3,2-b][1,4]oxazin-3-one;
6-[5-[2-[(1-methyl-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-one;formic acid;
6-[5-[2-[(4-fluoro-6,7-dihydro-5*H*-cyclopenta[c]pyridin-6-yl)methylamino]ethyl]-2-oxo-1,3-oxazolidin-3-yl]-4H-pyrazino[2,3-b][1,4]oxazin-3-one;
1-methyl-6-[[2-[2-oxo-3-(3-oxo-4*H*-pyrazino[2,3-b][1,4]oxazin-6-yl)-1,3-oxazolidin-5-yl] ethylamino] methyl]-6,7-dihydro-SH-cyclopenta[c]pyridine-4-carbonitrile;2,2,2-trifluoroacetic acid;
6-[5-[2-[(4-fluoro-1-methyl-6,7-dihydro-SH-cyclopenta[c]pyridin-6-yl)methylamino]ethyl]-2-oxo-1,3-oxazol-3-yl]-4*H*-pyrazino[2,3-b][1,4]oxazin-3-one;
6-[5-[3-[(4-fluoro-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one;formic acid;
2-[[7-fluoro-2-[[2-[2-oxo-3-(3-oxo-4*H*-pyrazino[2,3-b][1,4]oxazin-6-yl)-1,3-oxazolidin-5-yl] ethylamino] methyl]-2,3-dihydro-1H-inden-5-yl] oxy] acetamide;
2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide;formic acid;
2-[[4,7-difluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide;
6-[8-[[6-[(2*S*)-2-aminopropoxy]-4-fluoro-2,3-dihydro-1*H*-inden-2-yl]methyl]-2-oxo-1-oxa-3,8-diazaspiro[4.5]decan-3-yl]-4H-pyrazino[2,3-b][1,4]oxazin-3-one;formic acid;
1-methyl-6-[[2-[(5*S*)-2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridine-4-carbonitrile;
1-methyl-6-[[2-[(SR)-2-oxo-3-(3-oxo-4H-pyrazino[2,3-b] [1,4]oxazin-6-yl)-1,3-oxazolidin-5-yl] ethylamino] methyl]-6,7-dihydro-SH-cyclopenta[c]pyridine-4-carbonitrile;
6-[5-[3-[(4-fluoro-1-methyl-6,7-dihydro-5*H*-cyclopenta[c]pyridin-6-yl)amino]propyl]-2-oxo-l,3-oxazol-3-yl]-4*H*-pyrazino[2,3-b][1,4]oxazin-3-one, Enantiomer A;
6-[5-[3-[(4-fluoro-1-methyl-6,7-dihydro-5*H*-cyclopenta[c]pyridin-6-yl)amino]propyl]-2-oxo-l,3-oxazol-3-yl]-4*H*-pyrazino[2,3-b][1,4]oxazin-3-one, Enantiomer B;
6-[5-[2-[(1-methyl-6,7-dihydro-5*H*-cyclopenta[c]pyridin-6-yl)methylamino]ethyl]-2-oxo-1,3-oxazol-3-yl]-4*H*-pyrazino[2,3-b][1,4]oxazin-3-one;
2-[[7-fluoro-2-[[2-[(SS)-2-oxo-3-(3-oxo-4H-pyrazino[2,3-b] [1,4]oxazin-6-yl)-1,3-oxazolidin-5-yl]ethylamino]methyl]-2,3-dihydro-1*H*-inden-5-yl]oxy]acetamide, Isomer A;
2-[[7-fluoro-2-[[2-[(5*S*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide, Isomer B;
2-[[7-fluoro-2-[[2-[(5R)-2-oxo-3-(3-oxo-4*H*-pyrazino[2,3-b][1,4]oxazin-6-yl)-1,3-oxazolidin-5-yl]ethylamino]methyl]-2,3-dihydro-1H-inden-5-yl]oxy]acetamide, Isomer A;
2-[[7-fluoro-2-[[2-[(5*R*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide, Isomer B;
2-[[4,7-difluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide;
2-[[4,7-difluoro-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1*H*-inden-5-yl]oxy]-*N*-methylacetamide;
6-[(5*R*)-5-[2-[[(1*R*,2*R*)-1-amino-4-chloro-2,3-dihydro-1*H*-inden-2-yl]methylamino]ethyl]-2-oxo-1,3-oxazolidin-3-yl]-4*H*-pyrazino[2,3-b][1,4]oxazin-3-one;
6-[(5*R*)-5-[2-[[(1*S*,2*S*)-1-amino-4-chloro-2,3-dihydro-1*H*-inden-2-yl]methylamino]ethyl]-2-oxo-1,3-oxazolidin-3-yl]-4*H*-pyrazino[2,3-b][1,4]oxazin-3-one;
6-[(5*R*)-5-[2-[[(6*R*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[(5*S*)-5-[2-[[(6*R*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[(5*S*)-5-[2-[[(6*S*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[(5*R*)-5-[2-[[(6*S*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[5-[2-[[4-fluoro-1-(1*H*-pyrazol-4-yl)-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[5-[2-[[4-fluoro-1-(1*H*-imidazol-5-yl)-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
or pharmaceutically acceptable salts.

Also an object of the present invention is a compound according to formula (I) as described herein for use as a therapeutically active substance.

Likewise an object of the present invention is a pharmaceutical composition comprising a compound according to formula (I) as described herein and a therapeutically inert carrier.

As described above, the compounds of formula (I) and their pharmaceutically acceptable salts possess valuable pharmacological properties for the treatment or prevention of infections and resulting diseases, particularly bacteremia, pneumonia, meningitis, urinary tract infection, and wound infection, caused by pathogens, particularly by bacteria, more particularly by *Pseudomonas aeruginosa*, *Klebsiella pneumoniae*, *Escherichia coli*, *Acinetobacter baumannii* or *Staphylococcus aureus,* more particulary by Gram negative bacterias, furthermore particularly by *Escherichia coli.*

The compounds of formula (I) and their pharmaceutically acceptable salts exhibit activity as antibiotics, particularly as antibiotics against *Pseudomonas aeruginosa, Klebsiella pneumoniae*, *Escherichia coli*, *Acinetobacter baumannii* or *Staphylococcus aureus,* more particulary against Gram negative bacterias, furthermore particularly against *Escherichia coli.*

The compounds of formula (I) and their pharmaceutically acceptable salts can be used as antibiotics, i.e. as antibacterial pharmaceutical ingredients suitable in the treatment and prevention of bacterial infections, particulalrly in the treatment and prevention of bacterial infections caused by pathogens, particularly by bacteria, more particularly by *Pseudomonas aeruginosa*, *Klebsiella pneumoniae*, *Escherichia coli*, *Acinetobacter baumannii* or *Staphylococcus aureus,* more particulary by Gram negative bacterias, furthermore particularly by *Escherichia coli.*

The compounds of the present invention can be used, either alone or in combination with other drugs, for the treatment or prevention of infections and resulting diseases, particularly bacteremia, pneumonia, meningitis, urinary tract infection, and wound infection, caused by pathogens, particularly by bacteria, more particularly by *Pseudomonas aeruginosa*, *Klebsiella pneumoniae*, *Escherichia coli*, *Acinetobacter baumannii* or *Staphylococcus aureus,* more particulary by Gram negative bacterias, furthermore particularly by *Escherichia coli.*

A particular embodiment of the present invention relates to pharmaceutical compositions comprising compounds of formula (I) as defined above or their pharmaceutically acceptable salts as defined above and one or more pharmaceutically acceptable excipients.

A particular embodiment of the present invention relates to pharmaceutical compositions comprising compounds of formula (I) or their pharmaceutically acceptable salts as defined above and one or more pharmaceutically acceptable excipients for the treatment or prevention of infections and resulting diseases, particularly bacteremia, pneumonia, meningitis, urinary tract infection, and wound infection, caused by pathogens, particularly by bacteria, more particularly by *Pseudomonas aeruginosa*, *Klebsiella pneumoniae*, *Escherichia coli*, *Acinetobacter baumannii* or *Staphylococcus aureus,* more particulary by Gram negative bacterias, furthermore particularly by *Escherichia coli.*

A particular embodiment of the present invention relates to compounds of formula (I) or their pharmaceutically acceptable salts as defined above for use as therapeutically active substances, especially for use as therapeutically active substances for the treatment or prevention of infections and resulting diseases, particularly bacteremia, pneumonia, meningitis, urinary tract infection, and wound infection, caused by pathogens, particularly by bacteria, more particularly by *Pseudomonas aeruginosa*, *Klebsiella pneumoniae*, *Escherichia coli*, *Acinetobacter baumannii* or *Staphylococcus aureus,* more particulary by Gram negative bacterias, furthermore particularly by *Escherichia coli.*

A particular embodiment of the present invention relates to compounds of formula (I) or their pharmaceutically acceptable salts as defined above for the use in the treatment or prevention of infections and resulting diseases, particularly bacteremia, pneumonia, meningitis, urinary tract infection, and wound infection, caused by pathogens, particularly by bacteria, more particularly by *Pseudomonas aeruginosa*, *Klebsiella pneumoniae*, *Escherichia coli*, *Acinetobacter baumannii* or *Staphylococcus aureus*, more particulary by Gram negative bacterias, furthermore particularly by *Escherichia coli.*

A particular embodiment of the present invention relates to a method for the treatment or prevention of infections and resulting diseases, particularly bacteremia, pneumonia, meningitis, urinary tract infection, and wound infection, caused by pathogens, particularly by bacteria, more particularly by *Pseudomonas aeruginosa*, *Klebsiella pneumoniae*, *Escherichia coli*, *Acinetobacter baumannii* or *Staphylococcus aureus,* more particulary by Gram negative bacterias, furthermore particularly by *Escherichia coli,* which method comprises administering compounds of formula (I) or their pharmaceutically acceptable salts as defined above to a subject.

A particular embodiment of the present invention relates to the use of compounds of formula (I) or their pharmaceutically acceptable salts as defined above for the treatment or prevention of infections and resulting diseases, particularly bacteremia, pneumonia, meningitis, urinary tract infection, and wound infection, caused by pathogens, particularly by bacteria, more particularly by *Pseudomonas aeruginosa*, *Klebsiella pneumoniae*, *Escherichia coli*, *Acinetobacter baumannii* or *Staphylococcus aureus*, more particulary by Gram negative bacterias, furthermore particularly by *Escherichia coli.*

A particular embodiment of the present invention relates to the use of compounds of formula (I) or their pharmaceutically acceptable salts as defined above for the preparation of medicaments for the treatment or prevention of infections and resulting diseases, particularly bacteremia, pneumonia, meningitis, urinary tract infection, and wound infection, caused by pathogens, particularly by bacteria, more particularly caused by pathogens, particularly by bacteria, more particularly by *Pseudomonas aeruginosa*, *Klebsiella pneumoniae*, *Escherichia coli*, *Acinetobacter baumannii* or *Staphylococcus aureus*, more particulary by Gram negative bacterias, furthermore particularly by *Escherichia coli.* Such medicaments comprise compounds of formula (I) or their pharmaceutically acceptable salts as defined above.

Also an embodiment of the present invention are compounds of formula (I) as described herein, when manufactured according to any one of the described processes.

### Assay procedures

### Antimicrobial susceptibility testing:

### 50% Growth Inhibitory Concentration (IC50) determination

The *in vitro* antimicrobial activity of the compounds was determined according to the following procedure:
The assay used a 10-point Iso-Sensitest broth dilution method to measure quantitatively the *in vitro* activity of the compounds against *Pseudomonas aeruginosa* ATCC 27853, *Escherichia coli* ATCC 25922, *Acinetobacter baumannii* ATCC 17961, *Klebsiella pneumoniae* ATCC 43816 and *Staphylococcus aureus* ATCC 29213.

Stock compounds in DMSO were serially twofold diluted (e.g. range from 50 to 0.097 µM final concentration) in 384 wells microtiter plates and inoculated with 50 µL of the bacterial suspension in Iso-Sensitest medium to have a final cell concentration of ~ 5×10⁽⁵⁾ CFU/mL in a final volume/well of 50 µL/well. Microtiter plates were incubated at 35 ± 2 °C.

Bacterial cell growth was determined with the measurement of optical density at λ=600 nm each 20 minutes over a time course of 16 h.

Growth inhibition was calculated during the logarithmic growth of the bacterial cells with determination of the concentration inhibiting 50% (IC50) and 90% (IC90) of the growth.

Table 1 provides the 50% growth inhibitory concentrations (IC50) in micromoles per liter of the compounds of present invention obtained against the strain *Escherichia coli* ATCC 25922.

Particular compounds of the present invention exhibit an IC50 (*Escherichia coli* ATCC 25922) ≤ 5 µmol/L.

More particular compounds of the present invention exhibit an IC50 (*Escherichia coli* ATCC 25922) ≤ 1 µmol/L.

Most particular compounds of the present invention exhibit an IC50 (*Escherichia coli* ATCC 25922) ≤ 0.1 µmol/L.

**Table 1**

| **Example** | ***Escherichia coli* growth inhibition IC50 (µM)** |
|---|---|
| 1 | <0.0977 |
| 2 | 0.332 |
| 3 | 0.94 |
| 4 | <0.0977 |
| 5 | <0.0977 |
| 6 | <0.0977 |
| 7 | <0.0977 |
| 8 | 0.167 |
| 9 | 0.439 |
| 10 | 0.447 |
| 11 | 2.4 |
| 12 | 0.471 |
| 13 | <0.0977 |
| 14 | 0.415 |
| 15 | 0.188 |
| 16 | 0.84 |
| 17 | 0.823 |
| 18 | <0.0977 |
| 19 | 0.449 |
| 20 | 0.139 |
| 21 | 0.453 |
| 22 | 8.53 |
| 23 | 2.17 |
| 24 | 1.81 |
| 25 | 4.07 |
| 26 | <0.0977 |
| 27 | 0.582 |
| 28 | <0.0977 |
| 29 | 0.945 |
| 30 | 3.4 |
| 31 | 0.156 |
| 32 | 0.71 |
| 33 | <0.0977 |
| 34 | 1.66 |
| 35 | <0.0977 |
| 36 | <0.0977 |
| 37 | <0.0977 |
| 38 | <0.0977 |
| 39 | 0.737 |
| 40 | 0.153 |
| 41 | 0.728 |
| 42 | 0.158 |
| 43 | 4.14 |
| 44 | 0.152 |
| 45 | 1.5 |
| 46 | 0.356 |
| 47 | <0.0977 |
| 48 | 0.106 |
| 49 | 0.193 |
| 50 | <0.0977 |
| 51 | <0.0977 |
| 52 | <0.0977 |
| 53 | <0.0977 |
| 54 | 0.357 |
| 55 | <0.0977 |
| 56 | 0.941 |
| 57 | 0.328 |
| 58 | 0.19 |
| 59 | 0.253 |
| 60 | <0.0977 |
| 61 | 0.164 |
| 62 | 0.208 |
| 63 | <0.0977 |
| 64 | <0.0977 |
| 65 | <0.0977 |
| 66 | 0.234 |
| 67 | 0.249 |
| 68 | 0.175 |
| 69 | 0.123 |
| 70 | 0.0977 |
| 71 | <0.0977 |
| 72 | 0.0697 |
| 73 | <0.0977 |
| 74 | 0.118 |
| 75 | 0.23 |
| 76 | <0.0977 |
| 77 | 0.393 |
| 78 | 0.155 |
| 79 | <0.0977 |
| 80 | <0.0977 |
| 81 | 0.164 |
| 82 | 0.152 |
| 83 | <0.0977 |
| 84 | 0.217 |
| 85 | <0.0977 |
| 86 | 0.134 |
| 87 | 0.584 |
| 88 | 0.168 |
| 89 | 0.304 |
| 90 | 0.17 |
| 91 | <0.0977 |
| 92 | 0.373 |
| 93 | <0.0977 |
| 94 | 0.485 |
| 95 | 0.232 |
| 96 | 0.187 |
| 97 | 0.185 |
| 98 | <0.0977 |
| 99 | 0.516 |
| 100 | 1.26 |
| 101 | 1.39 |
| 102 | 0.871 |
| 103 | 1.69 |
| 104 | 0.0994 |
| 105 | 1.11 |
| 106 | 0.259 |
| 107 | 0.235 |
| 108 | <0.0977 |
| 109 | 0.098 |
| 110 | 0.311 |
| 111 | 0.489 |
| 112 | 0.303 |
| 113 | 0.323 |
| 114 | 0.217 |
| 115 | 0.261 |
| 116 | <0.0977 |
| 117 | <0.0977 |
| 118 | <0.0977 |
| 119 | <0.0977 |
| 120 | 0.44 |
| 121 | 0.167 |
| 122 | <0.0977 |
| 123 | 0.248 |
| 124 | <0.0977 |
| 125 | 0.111 |
| 126 | 0.26 |
| 127 | 0.189 |
| 128 | 0.0981 |
| 129 | 0.692 |
| 130 | 0.868 |
| 131 | 0.24 |
| 132 | 0.145 |
| 133 | 1.38 |
| 134 | 0.114 |
| 135 | 5.8 |
| 136 | 0.112 |
| 137 | 0.801 |
| 138 | 0.359 |
| 139 | 0.513 |
| 140 | 0.314 |
| 141 | 0.186 |
| 142 | <0.0977 |
| 143 | 0.833 |
| 144 | 0.131 |
| 145 | 0.191 |
| 146 | 1.01 |
| 147 | <0.0977 |
| 148 | 0.598 |
| 149 | 0.904 |
| 150 | <0.0977 |
| 151 | 0.14 |
| 152 | 0.149 |
| 153 | 0.198 |
| 154 | <0.0977 |
| 155 | 0.364 |
| 156 | <0.0977 |
| 157 | <0.0977 |
| 158 | 0.164 |
| 159 | 0.17 |
| 160 | <0.0977 |
| 161 | <0.0977 |
| 162 | 0.195 |
| 163 | 0.131 |
| 164 | <0.0977 |
| 165 | <0.0977 |
| 166 | 0.172 |
| 167 | 0.19 |
| 168 | <0.0977 |
| 169 | <0.0977 |
| 170 | <0.0977 |
| 171 | 0.178 |
| 172 | <0.0977 |
| 173 | 0.194 |
| 174 | <0.0977 |
| 175 | 0.193 |
| 176 | 0.483 |
| 177 | <0.0977 |
| 178 | <0.0977 |
| 179 | 0.103 |
| 180 | <0.0977 |
| 181 | <0.0977 |
| 182 | 0.882 |
| 183 | 0.153 |
| 184 | 0.425 |
| 185 | <0.0977 |
| 186 | <0.0977 |
| 187 | 0.121 |
| 188 | <0.0977 |
| 189 | 0.0995 |
| 190 | <0.0977 |
| 191 | 0.295 |
| 192 | <0.0977 |
| 193 | <0.0977 |
| 194 | <0.0977 |
| 195 | <0.0977 |
| 196 | <0.0977 |
| 197 | <0.0977 |
| 198 | <0.0977 |
| 199 | 0.0884 |
| 200 | 0.32 |
| 201 | 0.157 |
| 202 | 0.135 |
| 203 | <0.0977 |
| 204 | <0.0977 |
| 205 | 0.123 |
| 206 | 1.16 |
| 207 | <0.0977 |
| 208 | <0.0977 |
| 209 | 0.845 |
| 210 | 0.295 |
| 211 | <0.0977 |
| 212 | <0.0977 |
| 213 | <0.0977 |
| 214 | <0.0977 |
| 215 | <0.0977 |
| 216 | 0.211 |
| 217 | <0.0977 |
| 218 | <0.0977 |
| 219 | 0.378 |
| 220 | 0.319 |
| 221 | 0.302 |
| 222 | 0.717 |
| 223 | 0.389 |
| 224 | 0.791 |
| 225 | 0.547 |
| 226 | <0.0977 |
| 227 | 0.132 |
| 228 | 0.174 |
| 229 | 1.57 |
| 230 | <0.0977 |
| 231 | 0.176 |
| 232 | 0.495 |
| 233 | 0.942 |
| 234 | 0.212 |
| 235 | 0.196 |
| 236 | <0.0977 |
| 237 | 0.14 |
| 238 | 0.17 |
| 239 | <0.0977 |
| 240 | 0.278 |
| 241 | 0.509 |
| 242 | 0.778 |
| 243 | 0.199 |
| 244 | <0.0977 |
| 245 | 0.119 |
| 246 | <0.0977 |
| 247 | <0.0977 |
| 248 | <0.0977 |
| 249 | 0.108 |
| 250 | 0.289 |
| 251 | 0.679 |
| 252 | 0.156 |
| 253 | <0.0977 |
| 254 | 0.188 |
| 255 | 0.18 |
| 256 | <0.0977 |
| 257 | <0.0977 |
| 258 | 0.221 |
| 259 | <0.0977 |
| 260 | <0.0977 |
| 261 | <0.0977 |
| 262 | <0.0977 |
| 263 | 0.127 |
| 264 | <0.0977 |
| 265 | <0.0977 |
| 266 | 0.206 |
| 267 | <0.0977 |
| 268 | <0.0977 |
| 269 | <0.0977 |
| 270 | <0.0977 |
| 271 | <0.0977 |
| 272 | <0.0977 |
| 273 | 0.148 |
| 274 | <0.0977 |
| 275 | 0.592 |
| 276 | 0.635 |
| 277 | 0.628 |
| 278 | 1.89 |
| 279 | <0.0977 |
| 280 | 0.844 |
| 281 | <0.0977 |
| 282 | 0.374 |
| 283 | 0.888 |
| 284 | 1.27 |
| 285 | 0.925 |
| 286 | <0.0977 |
| 287 | <0.0977 |
| 288 | <0.0977 |
| 289 | <0.0977 |
| 290 | 0.261 |
| 291 | <0.0977 |
| 292 | 0.403 |
| 293 | <0.0977 |
| 294 | 0.727 |
| 295 | 0.39 |
| 296 | 0.381 |
| 297 | 0.249 |
| 298 | 0.369 |
| 299 | <0.0977 |
| 300 | 0.156 |
| 301 | <0.0977 |
| 302 | <0.0977 |
| 303 | 0.163 |
| 304 | 0.446 |
| 305 | <0.0977 |
| 306 | <0.0977 |
| 307 | 0.359 |
| 308 | 0.471 |
| 309 | <0.0977 |
| 310 | 0.18 |
| 311 | 0.382 |
| 312 | <0.0977 |
| 313 | 1.9 |
| 314 | 1.18 |
| 315 | 0.668 |
| 316 | 0.957 |
| 317 | <0.0977 |
| 318 | <0.0977 |
| 319 | <0.0977 |
| 320 | 0.202 |
| 321 | <0.0977 |
| 322 | <0.0977 |
| 323 | 0.25 |
| 324 | 0.205 |
| 325 | 0.281 |
| 326 | 0.138 |
| 327 | <0.0977 |
| 328 | 0.885 |
| 329 | 0.453 |
| 330 | <0.0977 |
| 331 | <0.0977 |
| 332 | 0.184 |
| 333 | 0.265 |
| 334 | 0.321 |
| 335 | 0.225 |
| 336 | <0.0977 |
| 337 | 1.08 |
| 338 | <0.0977 |
| 339 | 0.145 |
| 340 | 0.358 |
| 341 | <0.0977 |
| 342 | 0.18 |
| 343 | 0.617 |
| 344 | 0.202 |
| 345 | 0.659 |
| 346 | 0.311 |
| 347 | 0.175 |
| 348 | 0.317 |
| 349 | <0.0977 |
| 350 | <0.0977 |
| 351 | 0.144 |
| 352 | <0.0977 |
| 353 | 0.17 |
| 354 | 0.392 |
| 355 | 0.162 |
| 356 | <0.0977 |
| 357 | <0.0977 |
| 358 | <0.0977 |
| 359 | <0.0977 |
| 360 | 0.319 |
| 361 | 0.336 |
| 362 | 0.157 |
| 363 | <0.0977 |
| 364 | <0.0977 |
| 365 | <0.0977 |
| 366 | 0.144 |
| 367 | <0.0977 |
| 368 | <0.0977 |
| 369 | 0.172 |
| 370 | 0.195 |
| 371 | <0.0977 |
| 372 | <0.0977 |
| 373 | <0.0977 |
| 374 | 0.207 |
| 375 | 0.351 |
| 376 | <0.0977 |
| 377 | <0.0977 |
| 378 | <0.0977 |
| 379 | <0.0977 |
| 380 | <0.0977 |
| 381 | 0.105 |
| 382 | 0.158 |
| 383 | 0.139 |
| 384 | 0.167 |
| 385 | 0.923 |
| 386 | <0.0977 |
| 387 | <0.0977 |
| 388 | 0.192 |
| 389 | <0.0977 |
| 390 | 0.161 |
| 391 | 0.258 |
| 392 | <0.0977 |
| 393 | <0.0977 |
| 394 | <0.0977 |
| 395 | 0.166 |
| 396 | 0.161 |
| 397 | <0.0977 |
| 398 | <0.0977 |
| 399 | 1.12 |
| 400 | 0.365 |
| 401 | <0.0977 |
| 402 | <0.0977 |
| 403 | <0.0977 |
| 404 | <0.0977 |
| 405 | <0.0977 |
| 406 | 0.139 |
| 407 | <0.0977 |
| 408 | <0.0977 |
| 409 | <0.0977 |
| 410 | <0.0977 |
| 411 | <0.0977 |
| 412 | <0.0977 |
| 413 | <0.0977 |
| 414 | 0.127 |
| 415 | 0.221 |
| 416 | 0.0951 |
| 417 | <0.0977 |
| 418 | 0.751 |
| 419 | 0.225 |
| 420 | 0.196 |
| 421 | 0.618 |
| 422 | <.0977 |
| 423 | 0.468 |
| 424 | 0.227 |
| 425 | 0.102 |
| 426 | 0.156 |
| 427 | <0.0977 |
| 428 | <0.0977 |
| 429 | <0.0977 |
| 430 | <0.0977 |
| 431 | <0.0977 |
| 432 | 0.120 |
| 433 | <0.0977 |

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays), rectally (e.g. in the form of suppositories) or topical ocularly (e.g. in the form of solutions, ointments, gels or water soluble polymeric inserts). However, the administration can also be effected parenterally, such as intramuscularly, intravenously, or intraocularly (e.g. in the form of sterile injection solutions).

The compounds of formula (I) and their pharmaceutically acceptable salts can be processed with pharmaceutically inert, inorganic or organic adjuvants for the production of tablets, coated tablets, dragees,hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

### Preparation of pharmaceutical compositions comprising compounds of the invention:

Tablets of the following composition are manufactured in the usual manner:

| **Ingredient** | **mg/tablet** | | | |
|---|---|---|---|---|
| | 5 | 25 | 100 | 500 |
| Compound of formula I | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 1161 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

Capsules of the following composition are manufactured:

| **ingredient** | **mg/capsule** | | | |
|---|---|---|---|---|
| | 5 | 25 | 100 | 500 |
| Compound of formula I | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

A compound of formula I lactose and corn starch are firstly mixed in a mixer and then in a comminuting machine. The mixture is returned to the mixer; the talc is added thereto and mixed thoapproximatively. The mixture is filled by machine into suitable capsules, e.g. hard gelatin capsules.

Injection solutions of the following composition are manufactured:

| **ingredient** | **mg/injection solution.** |
|---|---|
| Compound of formula I | 3 |
| Polyethylene Glycol 400 | 150 |
| acetic acid | q.s. ad pH 5.0 |
| water for injection solutions | ad 1.0 ml |

The invention is illustrated hereinafter by Examples, which have no limiting character.

In case the preparative examples are obtained as a mixture of enantiomers, the pure enantiomers can be obtained by methods described herein or by methods known to those skilled in the art, such as e.g. chiral chromatography or crystallization.

### Examples

### ABBREVIATIONS

MeOH: methanol, AcOH: acetic acid, prep-HPLC: preparative reversed phase high-performance liquid chromatography, MS: mass spectrum, M: molecular mass, ESP: electrospray ionisation (positive charge detection), ESN: electrospray ionisation (negative charge detection), DCM: dichloromethane, EtOAc: ethyl acetate, DMF: *N*,*N*-dimethylformamide, RT, r.t. or rt: room temperature, DIPEA: *N*,*N*-diisopropylethylamine, i-PrOH: 2-propanol, h: hours, THF: tetrahydrofuran, xantphos: 4,5-bis(diphenylphosphino)-9,9-dimethylxanthene, TFA: trifluoroacetic acid, dioxane: 1,4-dioxane, sat.: saturated, quant.: quantitative, EI: electron ionization, dppf: 1,1'-ferrocenediyl-bis(diphenylphosphine), HATU: 1-[bis(dimethylamino)methylene]-1H-1,2,3-triazolo[4,5-b]pyridinium 3-oxide hexafluorophosphate, RM: reaction mixture, t-BuOH: 2-methylpropan-2-ol, prep-TLC: preparative thin layer chromatography, SFC: supercritical fluid chromatography, ether: diethyl ether, BOC: tert-butyloxycarbonyl, HV: high vacuum, Pd/C: palladium on carbon, TMSCl: trimethylsilyl chloride, MeCN: acetonitrile, NMR: nuclear magnetic resonance spectroscopy, s: singlet, d (NMR): doublet, t: triplet, q: quartet, m: multiplet, dd: doublet of doublets, dt: doublet of triplets, br: broad, J: coupling constant, δ, chemical shift in parts per million, PE: petroleum ether, NMP: N-methyl-2-pyrrolidone, DMA: *N*,*N*-dimethylacetamide, Dess-Martin periodinane or DMP: 1,1,1-triacetoxy-1,1-dihydro-1,2-benziodoxol-3(1H)-one, LDA: lithium diisopropylamide, MOMCl: methylchloromethyl ether, DMAP: 4-dimethylaminopyridine, tBu: tert-butyl, LiHMDS: lithium bis(trimethylsilyl)amide, TsCl: tosyl chloride, HPLC: preparative reversed phase high-performance liquid chromatography, TBSOTf: tert-butyldimethylsilyl trifluoromethanesulfonate, Grubbs Catalyst 2nd Generation: 1,3-Bis(2,4,6-trimethylphenyl)-2-imidazolidinylidene)dichloro(phenylmethylene)(tricyclohexylphosphine)ruthenium, DMSO: dimethyl sulfoxide, AcOEt: ethyl acetate, d (time): days, TEMPO: (2,2,6,6-tetramethyl-piperidin-1-yl)oxyl, TBSCl: *tert*-butyldimethylsilyl chloride, dba: dibenzylideneacetone, t-BuXphos: 2-di-*tert*-butylphosphino-2',4',6'-triisopropylbiphenyl, TBAF: tetra-n-butylammonium fluoride, t-BuXPhos-Pd-G3: [(2-di-tert-butylphosphino-2',4',6'-triisopropyl-1,1'-biphenyl)-2-(2'-amino-1,1'-biphenyl)] palladium(II) methanesulfonate.

### Example 1

### 6-[5-[2-[(4-Chloro-2,3-dihydro-1H-inden-2-yl)methylamino]ethyl]-2-oxo-1,3-oxazolidin-3-yl]-4H-pyrido[3,2-b][1,4]oxazin-3-one; formic acid

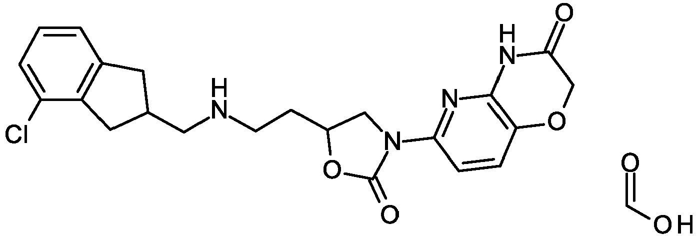

To a solution of 6-[5-(2-aminoethyl)-2-oxo-1,3-oxazolidin-3-yl]-4*H*-pyrido[3,2-b][1,4]oxazin-3-one (Intermediate 1, 30.0 mg, 0.110 mmol, 1 eq) in methanol (3 mL) was added 4-chloro-2,3-dihydro-1H-indene-2-carbaldehyde (CAS# 1823964-97-1, 25.97 mg, 0.130 mmol, 1.2 eq) and sodium cyanoborohydride (13.5 mg, 0.220 mmol, 2 eq). The mixture was stirred at 25 °C for 16 h. The solvent was removed by evaporation to give a residue, which was dissolved in DMF (1 mL) and was purified by prep-HPLC (formic acid) to give the title compound (12.2 mg, 0.03 mmol, 23.1 % yield) as a white solid. MS (ESP): m/z = 443.2 [M+H]⁺.

The following examples were prepared by analogy to example 1.

| **Ex.** | **Name, Structure, MS** | **Starting Materials** |
|---|---|---|
| 2 | 6-[5-[3-[(4-chloro-2,3-dihydro-1*H*-inden-2-yl)methylamino]propyl]-2-oxo-1,3-oxazolidin-3-yl]-4*H-*pyrido[3,2-b][1,4]oxazin-3-one | Intermediate 2 and 4-chloro-2,3-dihydro-1*H*-indene-2-carbaldehyde [CAS# 1823964-97-1] |
| | 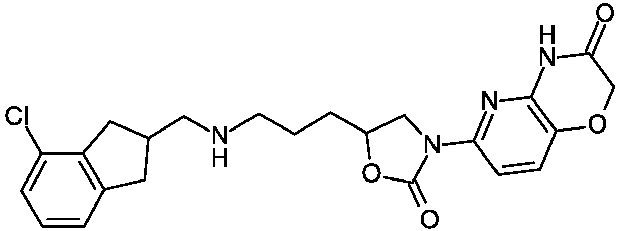 | |
| | ESP [M+H]⁺: 457.3 | |
| 3 | 6-[5-[2-[(4-chloro-2,3-dihydro-1H-inden-2-yl)amino]ethyl]-2-oxo-1,3-oxazolidin-3-yl]-4*H*-pyrido[3,2-b][1,4]oxazin-3-one hydrochloride | Intermediate 1 and 4-chloro-1,3-dihydroinden-2-one [CAS# 74124-90-6] |
| | 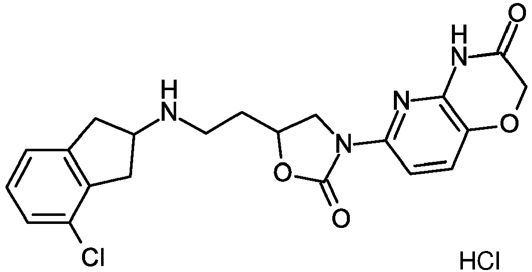 | |
| | ESP [M+H]⁺: 429.0 | |
| 4 | 6-[5-[3-[(4-chloro-2,3-dihydro-1H-inden-2-yl)amino]propyl]-2-oxo-1,3-oxazolidin-3-yl]-4H-pyrido[3,2-b][1,4]oxazin-3-one | Intermediate 2 and 4-chloro-1,3-dihydroinden-2-one [CAS# 74124-90-6] |
| | 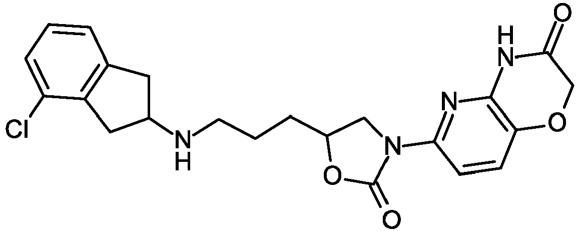 | |
| | ESP [M+H]⁺: 443.1 | |
| 5 | 2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile | Intermediate 1 and Intermediate 5 |
| | 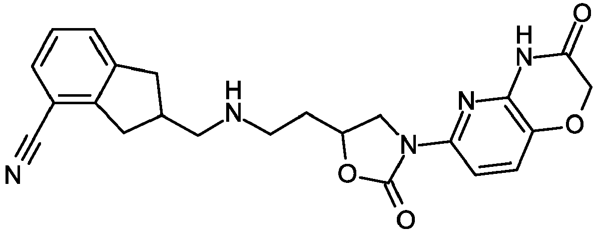 | |
| | ESP [M+H]⁺: 434.2 | |
| 6 | 6-[5-[2-[(4-chloro-2,3-dihydro-1*H*-inden-2-yl)methylamino]ethyl]-2-oxo-1,3-oxazolidin-3-yl]-4*H-*pyrido[3,2-b][1,4]oxazin-3-one, Isomer A | Intermediate 3 and 4-chloro-2,3-dihydro-1*H*-indene-2-carbaldehyde [CAS# 1823964-97-1] |
| | 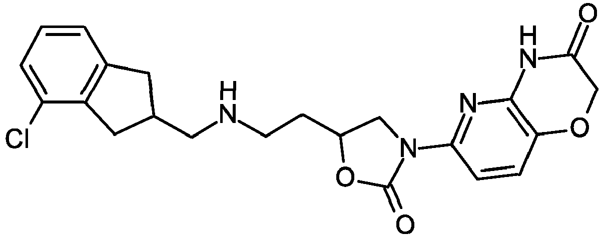 | |
| | ESP [M+H]⁺: 443.0 | |
| 7 | 6-[5-[2-[(4-chloro-2,3-dihydro-1H-inden-2-yl)methylamino]ethyl]-2-oxo-1,3-oxazolidin-3-yl]-4*H-*pyrido[3,2-b][1,4]oxazin-3-one, Isomer B; formic acid | Intermediate 4 and 4-chloro-2,3-dihydro-1*H*-indene-2-carbaldehyde [CAS# 1823964-97-1] |
| | 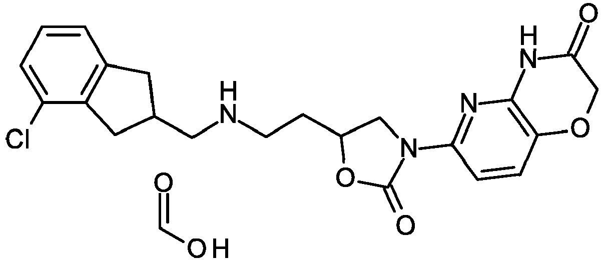 | |
| | ESP [M+H]⁺: 443.1 | |
| 8 | 6-[5-[3-[(4-fluoro-2,3-dihydro-1*H*-inden-2-yl)amino]propyl]-2-oxo-1,3-oxazolidin-3-yl]-4*H*-pyrido[3,2-b][1,4]oxazin-3-one; formic acid | Intermediate 2 and 4-fluoro-1,3-dihydroinden-2-one [CAS# 1210824-58-0] |
| | 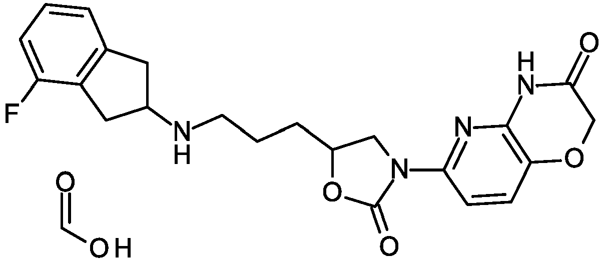 | |
| | ESP [M+H]⁺: 427.2 | |
| 9 | 6-[5-[3-(2,3-dihydro-1*H*-inden-2-ylamino)propyl]-2-oxo-1,3-oxazolidin-3-yl]-4*H*-pyrido[3,2-b][1,4]oxazin-3-one; formic acid | Intermediate 2 and 1,3-dihydroinden-2-one [CAS# 615-13-4] |
| | 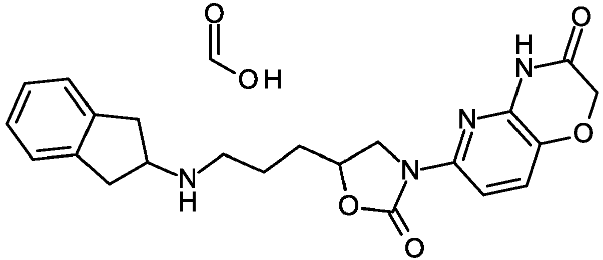 | |
| | ESP [M+H]⁺: 409.2 | |
| 10 | 6-[5-[3-[(5-chloro-2,3-dihydro-1*H*-inden-2-yl)amino]propyl]-2-oxo-1,3-oxazolidin-3-yl]-4*H*-pyrido[3,2-b][1,4]oxazin-3-one; formic acid | Intermediate 2 and 5-chloro-1,3-dihydroinden-2-one [CAS# 74444-81-8] |
| | 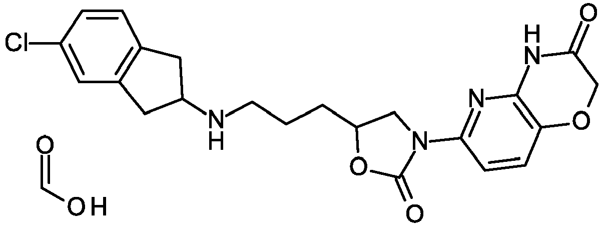 | |
| | ESP [M+H]⁺: 443.2 | |
| **11** | 6-[5-[2-[(5-chloro-2,3-dihydro-1*H*-inden-2-yl)amino]ethyl]-2-oxo-1,3-oxazolidin-3-yl]-4*H*-pyrido[3,2-b][1,4]oxazin-3-one | Intermediate 1 and 5-chloro-1,3-dihydroinden-2-one [CAS# 74444-81-8] |
| | 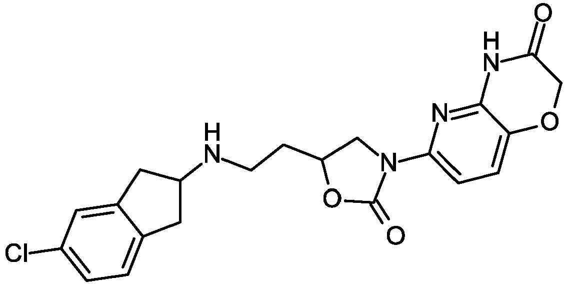 | |
| | ESP [M+H]⁺: 429.1 | |
| **12** | 6-[5-[3-[(5-fluoro-2,3-dihydro-1*H*-inden-2-yl)amino]propyl]-2-oxo-1,3-oxazolidin-3-yl]-4*H*-pyrido[3,2-b][1,4]oxazin-3-one; formic acid | Intermediate 2 and 5-fluoro-1,3-dihydroinden-2-one [CAS# 57584-69-7] |
| | 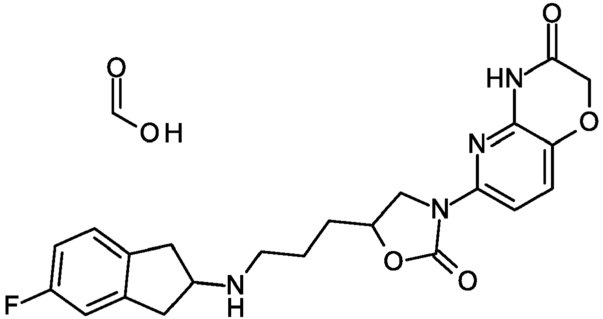 | |
| | ESP [M+H]⁺: 427.2 | |
| **13** | 2-[3-[2-oxo-3-(3-oxo-4*H*-pyrido[3,2-b][1,4]oxazin-6-yl)-1,3-oxazolidin-5-yl]propylamino]-2,3-dihydro-1*H*-indene-4-carbonitrile; formic acid | Intermediate 2 and Intermediate 6 |
| | 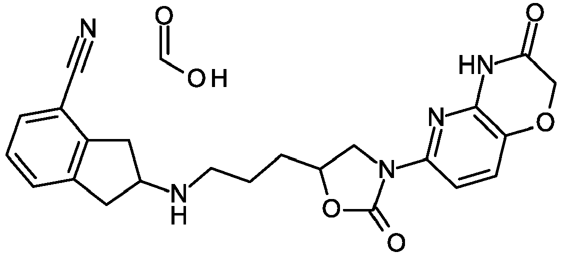 | |
| | ESP [M+H]⁺: 434.2 | |
| **14** | 2-[[trans-3-[2-oxo-3-(3-oxo-4H-pyrido[3,2-b] [1,4]oxazin-6-yl)-1,3-oxazolidin-5-yl] cyclobutyl] amino]-2,3-dihydro-1H-indene-4-carbonitrile | Intermediate 7 and Intermediate 6 |
| | 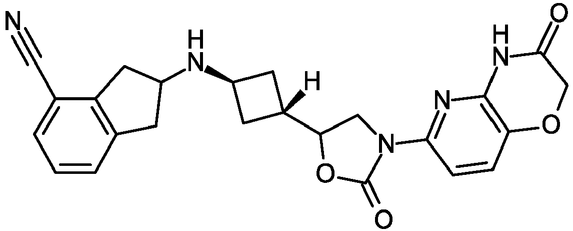 | |
| | ESP [M+H]⁺: 446.1 | |
| **15** | 6-[5-[2-[(4-chloro-2,3-dihydro-1H-inden-2-yl)methylamino]ethyl]-2-oxo-1,3-oxazolidin-3-yl]-4*H*-1,4-benzoxazin-3-one; formic acid | Intermediate 9 and 4-chloro-2,3-dihydro-1*H*-indene-2-carbaldehyde [CAS# 1823964-97-1] |
| | 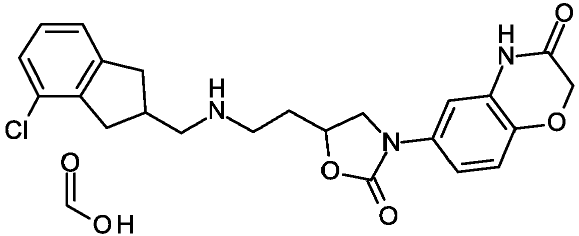 | |
| | ESP [M+H]⁺: 442.3 | |
| **16** | 6-[5-[cis-3-[(4-chloro-2,3-dihydro-1H-inden-2-yl)amino]cyclobutyl]-2-oxo-1,3-oxazolidin-3-yl]-4*H-*pyrido[3,2-b][1,4]oxazin-3-one; formic acid | Intermediate 10 and 4-chloro-1,3-dihydroinden-2-one [CAS# 74124-90-6] |
| | 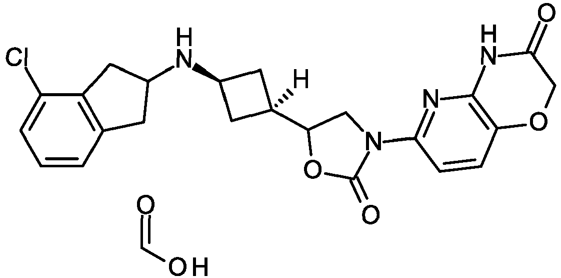 | |
| | ESP [M+H]⁺: 455.2 | |
| **17** | 6-[5-[trans-3-[(4-chloro-2,3-dihydro-1H-inden-2-yl)amino]cyclobutyl]-2-oxo-1,3-oxazolidin-3-yl]-4*H-*pyrido[3,2-b][1,4]oxazin-3-one | Intermediate 7 and 4-chloro-1,3-dihydroinden-2-one [CAS# 74124-90-6] |
| | 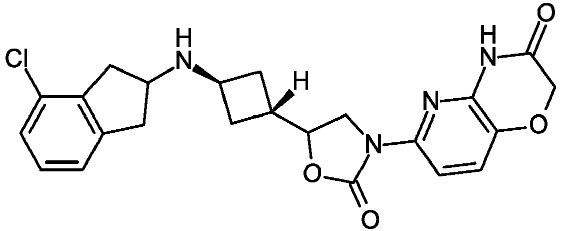 | |
| | ESP [M+H]⁺: 455.1 | |
| **18** | 2-[[2-[2-oxo-3-(3-oxo-4*H*-1,4-benzoxazin-6-yl)-1,3-oxazolidin-5-yl]ethylamino]methyl]-2,3-dihydro-1*H*-indene-4-carbonitrile; formic acid | Intermediate 9 and Intermediate 5 |
| | 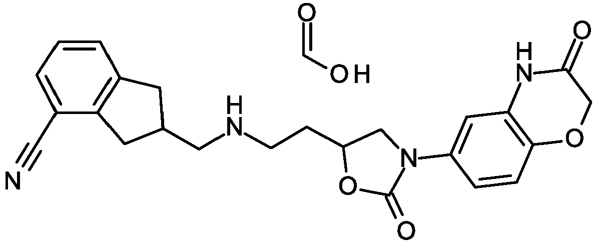 | |
| | ESP [M+H]⁺: 433.2 | |
| **19** | 2-[[cis-3-[2-oxo-3-(3-oxo-4H-pyrido[3,2-b] [1,4]oxazin-6-yl)-1,3-oxazolidin-5-yl]cyclobutyl]amino]-2,3-dihydro-1*H*-indene-4-carbonitrile; formic acid | Intermediate 10 and Intermediate 6 |
| | 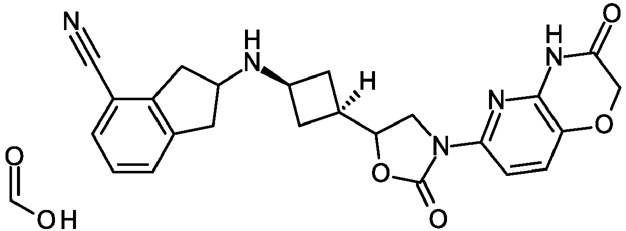 | |
| | ESP [M+H]⁺: 446.2 | |
| **20** | 6-[5-[2-[2-(4-chloro-2,3-dihydro-1H-inden-2-yl)ethylamino]ethyl]-2-oxo-1,3-oxazolidin-3-yl]-4*H-*pyrido[3,2-b][1,4]oxazin-3-one; formic acid | Intermediate 1 and Intermediate 11 |
| | 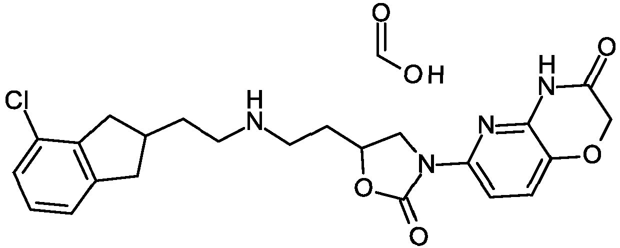 | |
| | ESP [M+H]⁺: 457.1 | |
| **21** | 6-[5-[[(4-chloro-2,3-dihydro-1H-inden-2-yl)methylamino]methyl]-2-oxo-1,3-oxazolidin-3-yl]-4*H-*pyrido[3,2-b][1,4]oxazin-3-one; formic acid | Intermediate 12 and 4-chloro-2,3-dihydro-1/7-indcnc-2-carbaldehyde [CAS# 1823964-97-1] |
| | 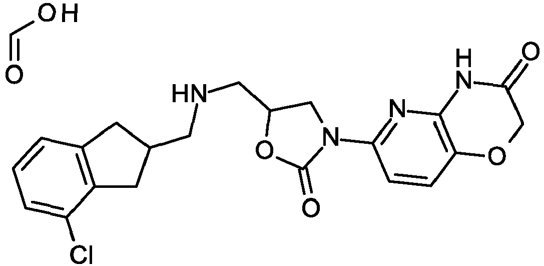 | |
| | ESP [M+H]⁺: 429.2 | |
| **22** | 6-[5-[[(4-chloro-2,3-dihydro-1H-inden-2-yl)amino]methyl]-2-oxo-1,3-oxazolidin-3-yl]-4*H*-pyrido[3,2-b][1,4]oxazin-3-one | Intermediate 12 and 4-chloro-1,3-dihydroinden-2-one [CAS# 74124-90-6] |
| | 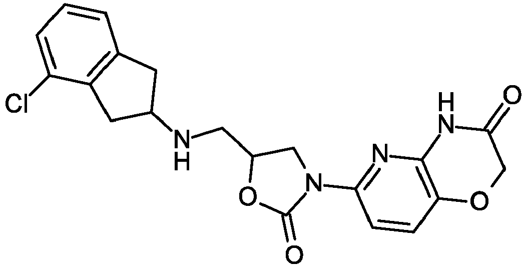 | |
| | ESP [M+H]⁺: 415.1 | |
| **23** | 6-[(4*R*)-4-[[3-[(4-fluoro-2,3-dihydro-1*H*-inden-2-yl)amino]cyclobutyl]amino]-2-oxopyrrolidin-1-yl]-4*H-*pyrido[3,2-b][1,4]oxazin-3-one; formic acid | Intermediate 13 and 4-fluoro-1,3-dihydroinden-2-one [CAS# 1210824-58-0] |
| | 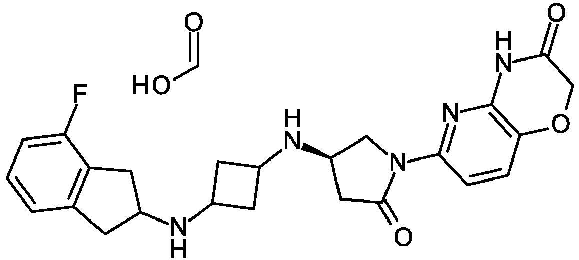 | |
| | ESN [M-H]⁻: 450.4 | |
| **24** | 6-[(4S)-4-[[3-[(4-fluoro-2,3-dihydro-1H-inden-2-yl)amino]cyclobutyl]amino]-2-oxopyrrolidin-1-yl]-4*H-*pyrido[3,2-b] [1,4]oxazin-3-one | Intermediate 14 and 4-fluoro-1,3-dihydroinden-2-one [CAS# 1210824-58-0] |
| | 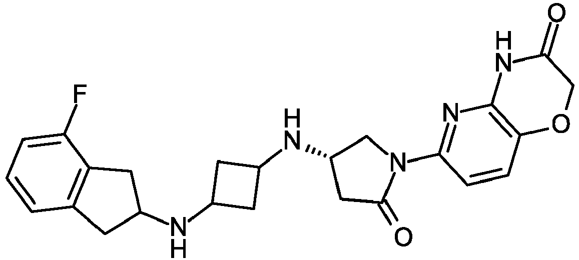 | |
| | ESN [M-H]⁻: 450.4 | |
| **25** | 6-[5-[[(5-chloro-2,3-dihydro-1H-inden-2-yl)amino]methyl]-2-oxo-1,3-oxazolidin-3-yl]-4*H*-pyrido[3,2-b][1,4]oxazin-3-one | Intermediate 12 and 5-chloro-1,3-dihydroinden-2-one [CAS# 74444-81-8] |
| | 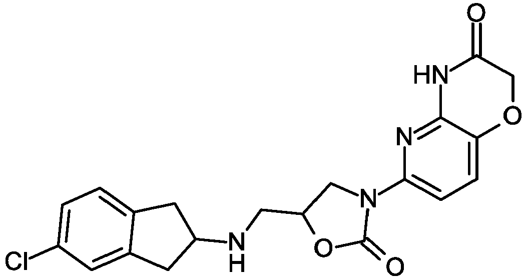 | |
| | ESP [M+H]⁺: 415.1 | |
| **26** | 6-[5-[[2-(4-chloro-2,3-dihydro-1*H*-inden-2-yl)ethylamino]methyl]-2-oxo-1,3-oxazolidin-3-yl]-4*H-*pyrido[3,2-b][1,4]oxazin-3-one; formic acid | Intermediate 12 and Intermediate 11 |
| | 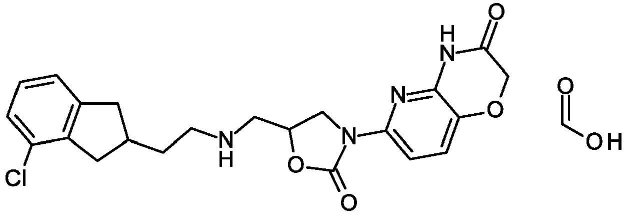 | |
| | ESP [M+H]⁺: 443.2 | |
| **27** | 6-[5-[3-[(4-methyl-6,7-dihydro-5H-cyclopenta[b]pyridin-6-yl)amino]propyl]-2-oxo-1,3-oxazolidin-3-yl]-4H-pyrido[3,2-b][1,4]oxazin-3-one | Intermediate 15 and Intermediate 16 |
| | 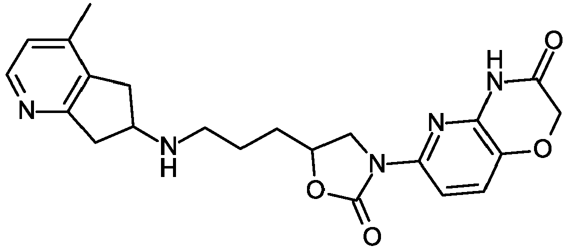 | |
| | ESP [M+H]⁺: 424.2 | |
| **28** | 2-[[2-oxo-3-(3-oxo-4*H*-pyrido[3,2-b][1,4]oxazin-6-yl)-1-oxa-3,8-diazaspiro[4.5]decan-8-yl]methyl]-2,3-dihydro-1*H*-indene-4-carbonitrile | Intermediate 17 and Intermediate 5 |
| | 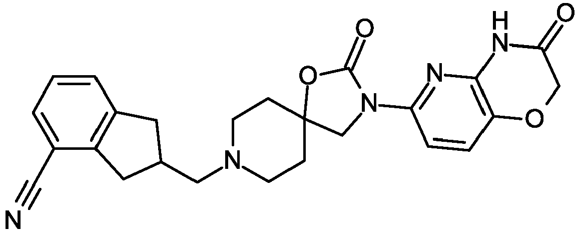 | |
| | ESP [M+H]⁺: 460.3 | |
| **29** | 2-[[[2-methyl-1-[2-oxo-3-(3-oxo-4*H*-pyrido[3,2-b][1,4]oxazin-6-yl)-1,3-oxazolidin-5-yl]propan-2-yl]amino]methyl]-2,3-dihydro-1H-indene-4-carbonitrile | Intermediate 18 and Intermediate 5 |
| | 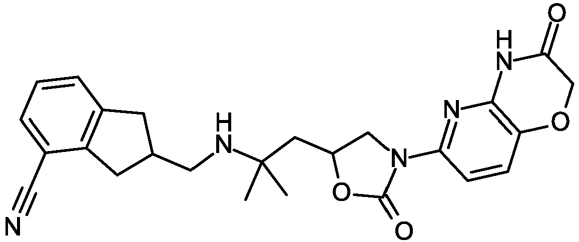 | |
| | ESP [M+H]⁺: 462.3 | |
| **30** | 6-[5-[2-[(1-chloro-6,7-dihydro-5*H*-cyclopenta[c]pyridin-6-yl)methylamino]-2-methylpropyl]-2-oxo-1,3-oxazolidin-3-yl]-4*H*-pyrido[3,2-b][1,4]oxazin-3-one; formic acid | Intermediate 18 and Intermediate 19 |
| | 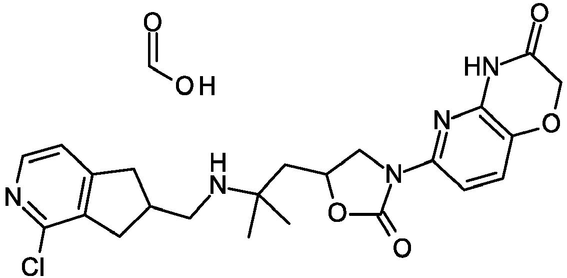 | |
| | ESP [M+H]⁺: 427.2 | |
| **31** | 2-[[[*rac*-3a,7a-*trans*-6,7a-*cis*-2-oxo-3-(3-oxo-4*H*-pyrido[3,2-b][1,4]oxazin-6-yl)-3a,4,5,6,7,7a-hexahydro-1,3-benzoxazol-6-yl]amino]methyl]-2,3-dihydro-1*H*-indene-4-carbonitrile | Intermediate 20 and Intermediate 5 |
| | 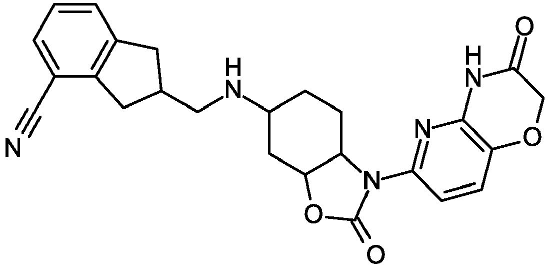 | |
| | ESP [M+H]⁺: 460.3 | |
| **32** | 6-[*rac*-3a,7a-*trans*-6,7a-*cis*-6-[2-(4-chloro-2,3-dihydro-1*H-*inden-2-yl)ethylamino]-2-oxo-3a,4,5,6,7,7a-hexahydro-1,3-benzoxazol-3-yl]-4*H*-pyrido[3,2-b][1,4]oxazin-3-one | Intermediate 20 and Intermediate 11 |
| | 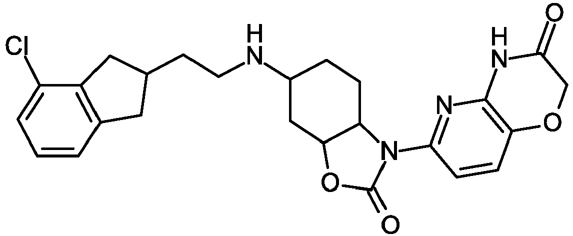 | |
| | ESP [M+H]⁺: 483.2 | |
| **33** | 2-[2-[6-oxo-7-(3-oxo-4*H*-pyrido[3,2-b][1,4]oxazin-6-yl)-5-oxa-2,7-diazaspiro[3.4]octan-2-yl]ethyl]-2,3-dihydro-1*H*-indene-4-carbonitrile | Intermediate 21 and Intermediate 22 |
| | 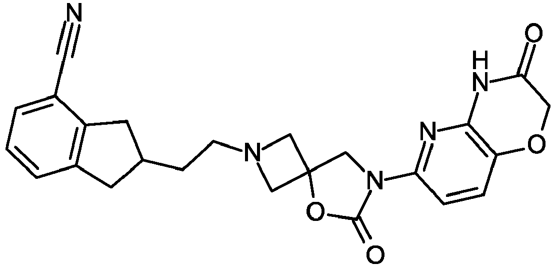 | |
| | ESP [M+H]⁺: 446.0 | |
| **34** | 2-[[trans-4-[2-oxo-3-(3-oxo-4H-pyrido[3,2-b] [1,4]oxazin-6-yl)-1,3-oxazolidin-5-yl] cyclohexyl] amino]-2,3-dihydro-1H-indene-4-carbonitrile; formic acid | Intermediate 23 and Intermediate 6 |
| | 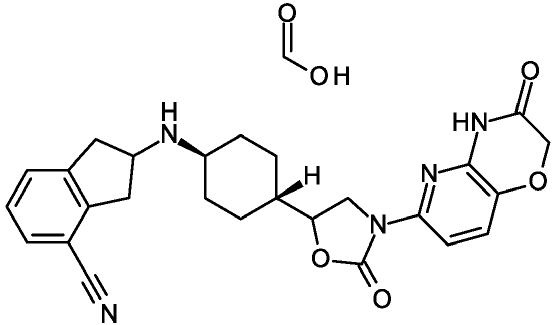 | |
| | ESP [M+H]⁺: 474.1 | |
| **35** | 2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H-*indene-4-carbonitrile | Intermediate 24 and Intermediate 5 |
| | 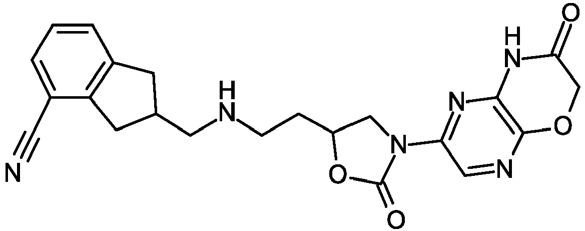 | |
| | ESP [M+H]⁺: 435.2 | |
| **36** | 6-[5-[2-[(4-fluoro-1-methyl-6,7-dihydro-5*H-*cyclopenta [c]pyridin-6-yl)methylamino] ethyl]-2-oxo-1,3-oxazolidin-3-yl]-4*H*-pyrido[3,2-b][1,4]oxazin-3-one | Intermediate 1 and Intermediate 27 |
| | 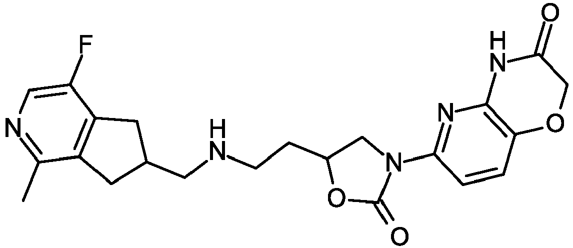 | |
| | ESP [M+H]⁺: 442.3 | |
| **37** | 4-fluoro-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H-*cyclopenta[c]pyridine-1-carbonitrile; formic acid | Intermediate 1 and Intermediate 28 |
| | 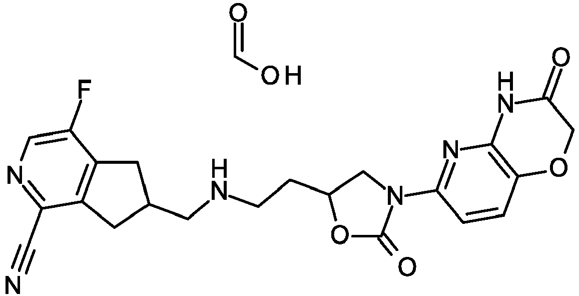 | |
| | ESP [M+H]⁺: 453.1 | |
| **38** | 6-[5-[2-[(1-chloro-6,7-dihydro-5*H*-cyclopenta[c]pyridin-6-yl)methylamino]ethyl]-2-oxo-1,3-oxazolidin-3-yl]-4*H-*pyrazino[2,3-b][1,4]oxazin-3-one | Intermediate 24 and Intermediate 19 |
| | 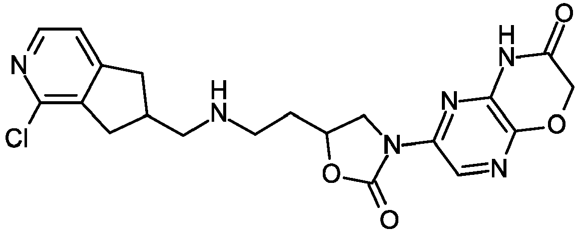 | |
| | ESP [M+H]⁺: 445.1 | |
| **39** | 2-[[2-oxo-3-(3-oxo-4*H*-pyrido[3,2-b][1,4]oxazin-6-yl)-1-oxa-3-azaspiro[4.5]decan-8-yl]amino]-2,3-dihydro-1*H*-indene-4-carbonitrile, Isomer A; formic acid | Intermediate 29 and Intermediate 6 |
| | 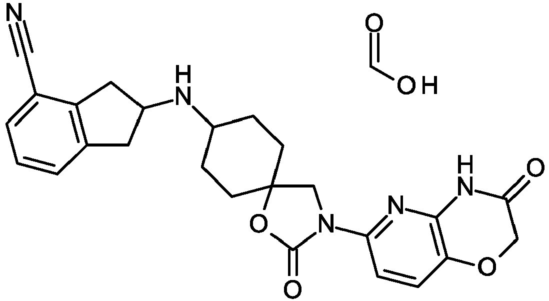 | |
| | ESP [M+H]⁺: 460.3 | |
| **40** | 2-[[2-oxo-3-(3-oxo-4*H*-pyrido[3,2-b][1,4]oxazin-6-yl)-1-oxa-3-azaspiro[4.5]decan-8-yl]amino]-2,3-dihydro-1*H*-indene-4-carbonitrile, Isomer B; formic acid | Intermediate 29 and Intermediate 6 |
| | 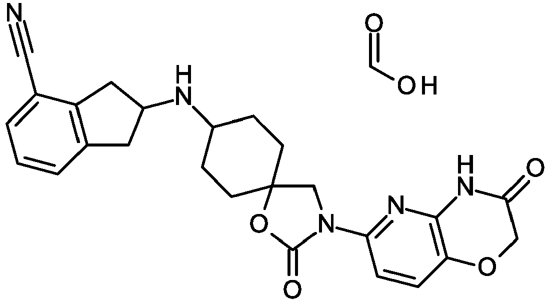 | |
| | ESP [M+H]⁺: 460.2 | |
| **41** | 6-[2-oxo-5-[2-[[*rac*-(5,6-*trans*)-1-chloro-5-hydroxy-6,7-dihydro-5*H*-cyclopenta[c]pyridin-6-yl]methylamino]ethyl]-1,3-oxazolidin-3-yl]-4*H*-pyrido[3,2-b][1,4]oxazin-3-one; formic acid | Intermediate 1 and Intermediate 30 |
| | 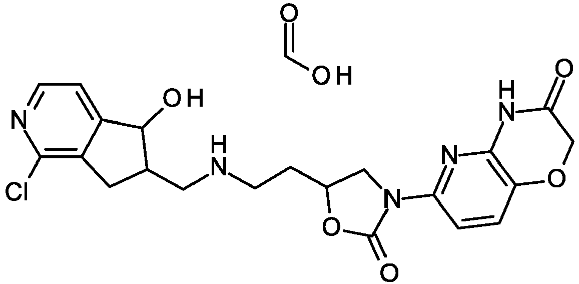 | |
| | ESP [M+H]⁺: 460.0 | |
| **42** | 2-[[2-[5-oxo-4-(3-oxo-4H-1,4-benzoxazin-6-yl)-1,3,4-oxadiazol-2-yl]ethylamino]methyl]-2,3-dihydro-1*H*-indene-4-carbonitrile; formic acid | Intermediate 31 and Intermediate 5 |
| | 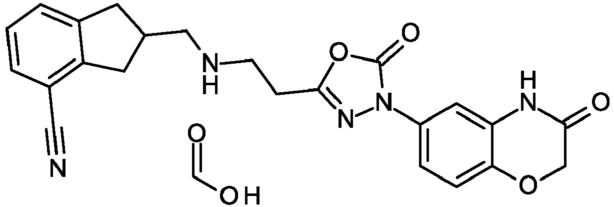 | |
| | ESP [M+H]⁺: 432.1 | |
| **43** | 6-[2-oxo-5-[2-[[*rac*-(5,6-*trans*)-5-hydroxy-1-methyl-6,7-dihydro-5*H*-cyclopenta[c]pyridin-6-yl]methylamino]ethyl]-1,3-oxazolidin-3-yl]-4*H*-pyrido[3,2-b][1,4]oxazin-3-one; formic acid | Intermediate 1 and Intermediate 32 |
| | 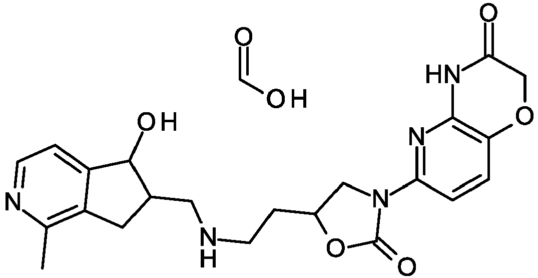 | |
| | ESP [M+H]⁺: 440.1 | |
| **44** | 2-[[2-[5-oxo-4-(3-oxo-4H-pyrido[3,2-b] [1,4]oxazin-6-yl)-1,3,4-oxadiazol-2-yl]ethylamino]methyl]-2,3-dihydro-1*H*-indene-4-carbonitrile; formic acid | Intermediate 33 and Intermediate 5 |
| | 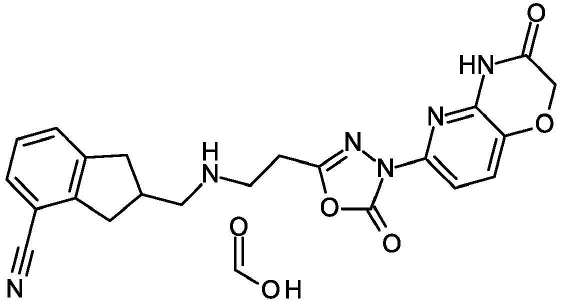 | |
| | ESP [M+H]⁺: 433.1 | |
| **45** | 7-[5-[2-[(1-chloro-6,7-dihydro-5*H*-cyclopenta[c]pyridin-6-yl)methylamino] ethyl]-2-oxo-1,3-oxazolidin-3-yl]-1H-pyrido[2,3-b][1,4]oxazin-2-one hydrochloride | Intermediate 34 and Intermediate 19 |
| | 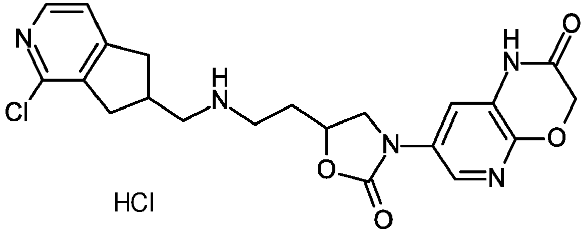 | |
| | ESP [M+H]⁺: 444.0 | |
| **46** | 2-[[2-[2-oxo-3-(2-oxo-1H-pyrido[2,3-b][1,4]oxazin-7-yl)-1,3-oxazolidin-5-yl]ethylamino]methyl]-2,3-dihydro-1H-indene-4-carbonitrile | Intermediate 34 and Intermediate 5 |
| | 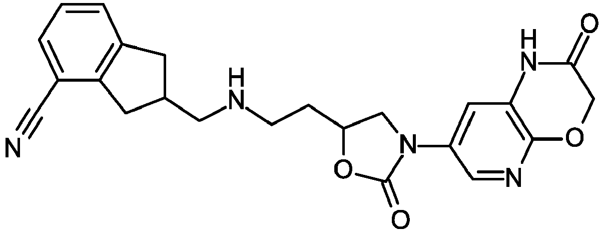 | |
| | ESP [M+H]⁺: 434.2 | |
| **47** | 1-methyl-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H-*cyclopenta[c]pyridine-4-carbonitrile; formic acid | Intermediate 1 and Intermediate 35 |
| | 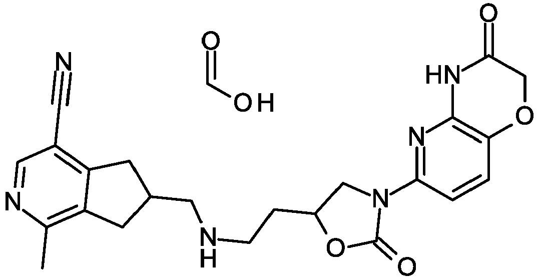 | |
| | ESP [M+H]⁺: 449.2 | |
| **48** | 2-[[4-cyano-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide | Intermediate 1 and Intermediate 36 |
| | 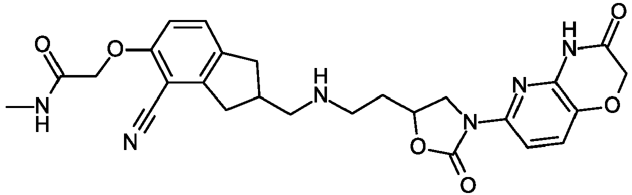 | |
| | ESP [M+Na]⁺: 543.3 | |
| **49** | 6-[5-[2-[(1-chloro-3-methoxy-6,7-dihydro-5*H-*cyclopenta[c]pyridin-6-yl)methylamino]ethyl]-2-oxo-1,3-oxazolidin-3-yl]-4*H*-pyrido[3,2-b][1,4]oxazin-3-one | Intermediate 1 and Intermediate 37 |
| | 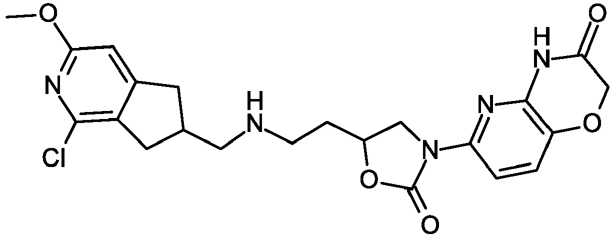 | |
| | ESP [M+H]⁺: 472.2 | |
| **50** | 6-[5-[2-[(1-chloro-3-methoxy-6,7-dihydro-5*H-*cyclopenta[c]pyridin-6-yl)methylamino]ethyl]-2-oxo-1,3-oxazolidin-3-yl]-4*H*-pyrazino[2,3-b][1,4]oxazin-3-one | Intermediate 24 and Intermediate 37 |
| | 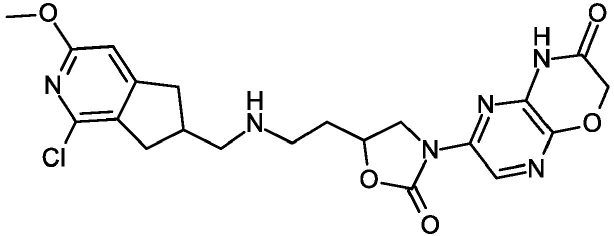 | |
| | ESP [M+H]⁺: 475.2 | |
| **51** | 6-[5-[2-[(4-fluoro-1-methyl-6,7-dihydro-5*H-*cyclopenta [c]pyridin-6-yl)methylamino] ethyl]-2-oxo-1,3-oxazolidin-3-yl]-4*H*-pyrazino[2,3-b][1,4]oxazin-3-one | Intermediate 27 and Intermediate 24 |
| | 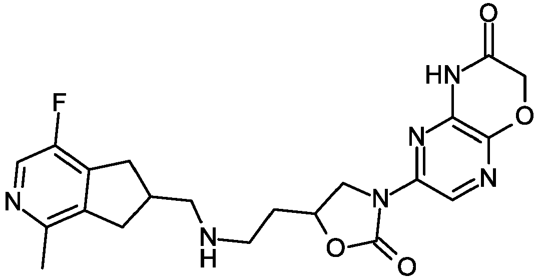 | |
| | ESP [M+H]⁺: 443.2 | |
| **52** | 5-[(1-methylpyrrolidin-2-yl)methoxy]-2-[[2-[2-oxo-3-(3-oxo-4*H*-pyrido[3,2-b][1,4]oxazin-6-yl)-1,3-oxazolidin-5-yl] ethylamino] methyl]-2,3-dihydro-1*H*-indene-4-carbonitrile; formic acid | Intermediate 1 and Intermediate 38 |
| | 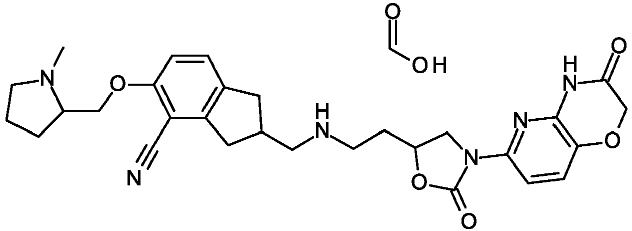 | |
| | ESP [M+H]⁺: 547.3 | |
| **53** | 3-methoxy-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H*-cyclopenta[c]pyridine-1-carbonitrile | Intermediate 1 and Intermediate 39 |
| | 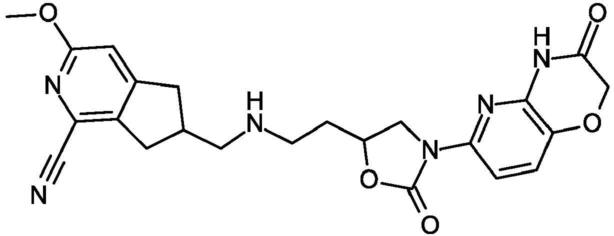 | |
| | ESP [M+H]⁺: 465.2 | |
| **54** | 5-(2-morpholin-4-ylethoxy)-2-[[2-[2-oxo-3-(3-oxo-4*H-*pyrido[3,2-b][1,4]oxazin-6-yl)-1,3-oxazolidin-5-yl] ethylamino] methyl]-2,3-dihydro-1H-indene-4-carbonitrile; formic acid | Intermediate 1 and Intermediate 40 |
| | 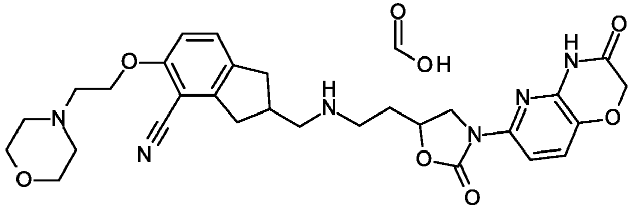 | |
| | ESP [M+H]⁺: 563.2 | |
| **55** | 6-[5-[2-[(4-chloro-1-methyl-6,7-dihydro-5*H-*cyclopenta[c]pyridin-6-yl)methylamino]ethyl]-2-oxo-1,3-oxazolidin-3-yl]-4*H*-pyrido[3,2-b][1,4]oxazin-3-one; formic acid | Intermediate 1 and Intermediate 41 |
| | 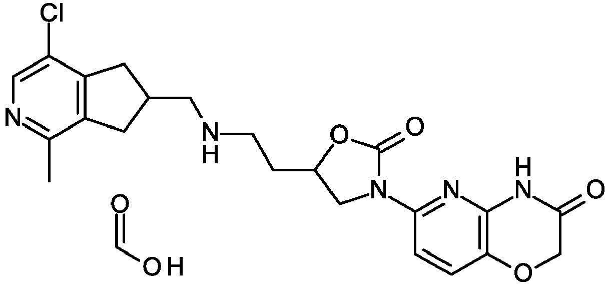 | |
| | ESP [M+H]⁺: 458.2 | |
| **56** | N-[2-[[4-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1*H*-inden-5-yl]oxy]ethyl]acetamide | Intermediate 1 and Intermediate 42 |
| | 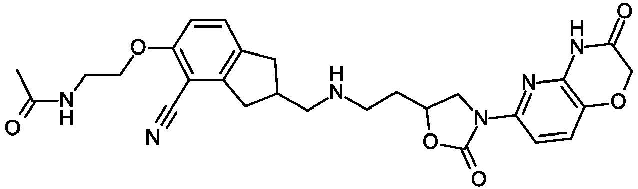 | |
| | ESP [M+H]⁺: 535.3 | |
| **57** | 6-[5-[2-[(1-chloro-4-methoxy-6,7-dihydro-5*H-*cyclopenta[c]pyridin-6-yl)methylamino]ethyl]-2-oxo-1,3-oxazolidin-3-yl]-4*H*-pyrido[3,2-b][1,4]oxazin-3-one | Intermediate 1 and Intermediate 43 |
| | 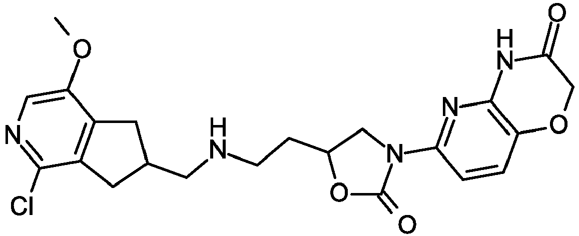 | |
| | ESP [M+H]⁺: 474.2 | |
| **58** | 6-[5-[2-[(1-chloro-4-methoxy-6,7-dihydro-5*H-*cyclopenta[c]pyridin-6-yl)methylamino]ethyl]-2-oxo-1,3-oxazolidin-3-yl]-4*H*-pyrazino[2,3-b][1,4]oxazin-3-one; formic acid | Intermediate 24 and Intermediate 43 |
| | 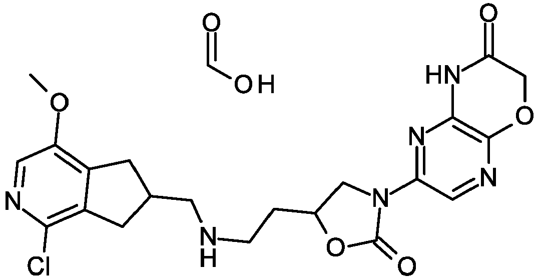 | |
| | ESP [M+H]⁺: 475.2 | |
| **59** | *N*-[2-[[4-cyano-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]ethyl]methanesulfonamide | Intermediate 1 and Intermediate 44 |
| | 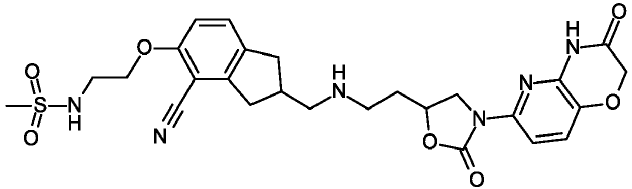 | |
| | ESP [M+H]⁺: 571.1 | |
| **60** | 6-[5-[2-[(4-chloro-1-methyl-6,7-dihydro-5H-cyclopenta[c]pyridin-6-yl)methylamino]ethyl]-2-oxo-1,3-oxazolidin-3-yl]-4*H*-pyrazino[2,3-b][1,4]oxazin-3-one; formic acid | Intermediate 24 and Intermediate 41 |
| | 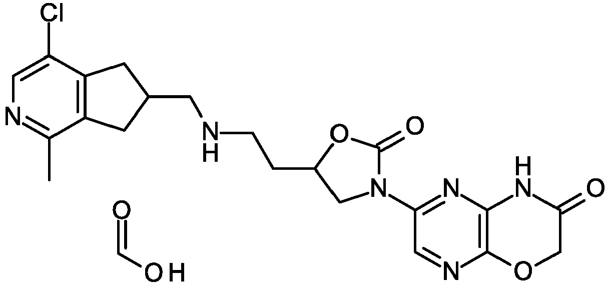 | |
| | ESP [M+H]⁺: 459.2 | |
| **61** | 2-[[[(2*R*)-2-hydroxy-2-[(5*R*)-2-oxo-3-(3-oxo-4*H*-1,4-benzoxazin-6-yl)-1,3-oxazolidin-5-yl]ethyl]amino]methyl]-2,3-dihydro-1*H*-indene-4-carbonitrile; formic acid | Intermediate 45 and Intermediate 5 |
| | 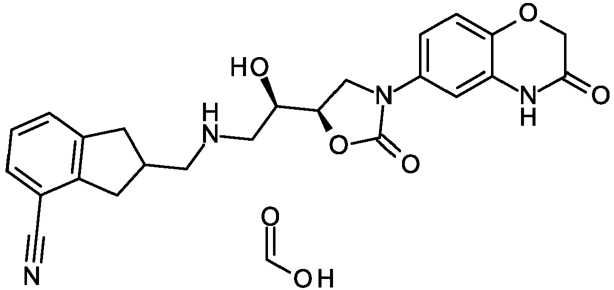 | |
| | ESP [M+H]⁺: 449.2 | |
| **62** | 2-[[[(2*S*)-2-hydroxy-2-[(5*S*)-2-oxo-3-(3-oxo-4*H*-1,4-benzoxazin-6-yl)-1,3-oxazolidin-5-yl]ethyl]amino]methyl]-2,3-dihydro-1*H*-indene-4-carbonitrile; formic acid | Intermediate 46 and Intermediate 5 |
| | 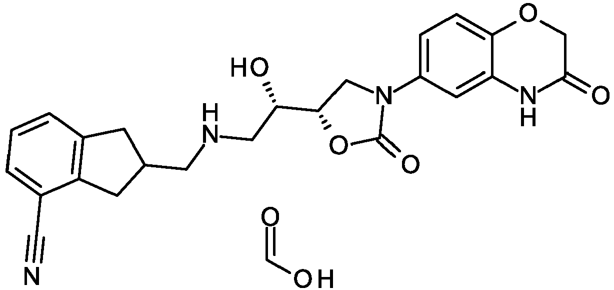 | |
| | ESP [M+H]⁺: 449.2 | |
| **63** | 5-(oxetan-3-yloxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile | Intermediate 1 and Intermediate 47 |
| | 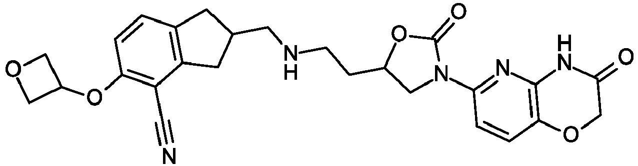 | |
| | ESP [M+H]⁺: 506.3 | |
| **64** | 2-[[2-[2-oxo-3-(3-oxo-4*H*-pyrido[3,2-b][1,4]thiazin-6-yl)-1,3-oxazolidin-5-yl]ethylamino]methyl]-2,3-dihydro-1*H*-indene-4-carbonitrile | Intermediate 48 and Intermediate 5 |
| | 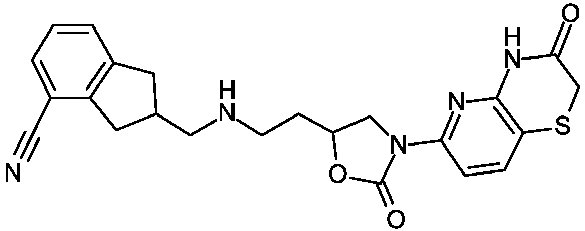 | |
| | ESP [M+H]⁺: 450.4 | |
| **65** | 3-methoxy-6-[[2-[2-oxo-3-(3-oxo-4*H*-pyrazino[2,3-b][1,4]oxazin-6-yl)oxazolidin-5-yl]ethylamino]methyl]-6,7-dihydro-5*H*-cyclopenta[c]pyridine-1-carbonitrile; formic acid | Intermediate 24 and Intermediate 39 |
| | 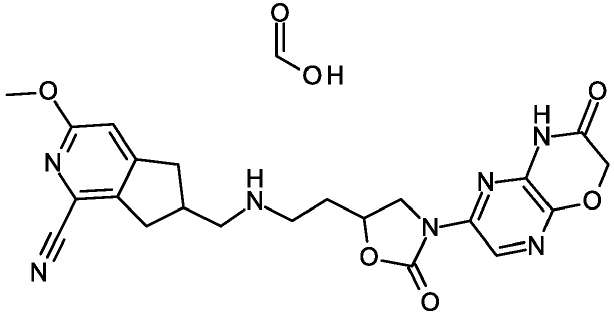 | |
| | ESP [M+H]⁺: 466.1 | |
| **66** | 2-[[1-chloro-6-[[2-[2-oxo-3-(3-oxo-4*H*-pyrido[3,2-b][1,4]oxazin-6-yl)oxazolidin-5-yl]ethylamino]methyl]-6,7-dihydro-5*H*-cyclopenta[c]pyridin-3-yl]oxy]-N-methylacetamide | Intermediate 1 and Intermediate 49 |
| | 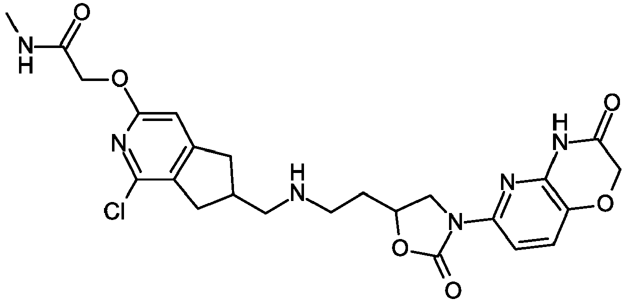 | |
| | ESP [M+H]⁺: 531.1 | |
| **67** | 6-[5-[2-[(4-fluoro-1-methyl-6,7-dihydro-5*H-*cyclopenta[c]pyridin-6-yl)methylamino]ethyl]-2-oxo-1,3,4-oxadiazol-3-yl]-4*H*-1,4-benzoxazin-3-one | Intermediate 31 and Intermediate 27 |
| | 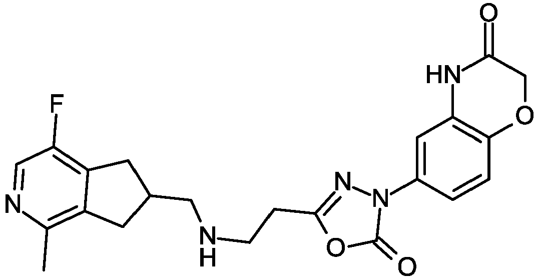 | |
| | ESP [M+H]⁺: 440.2 | |
| **68** | 6-[5-[2-[(4-fluoro-1-methyl-6,7-dihydro-5*H-*cyclopenta[c]pyridin-6-yl)methylamino]ethyl]-2-oxo-1,3,4-oxadiazol-3-yl]-4*H*-pyrido[3,2-b][1,4] oxazin-3-one | Intermediate 33 and Intermediate 27 |
| | 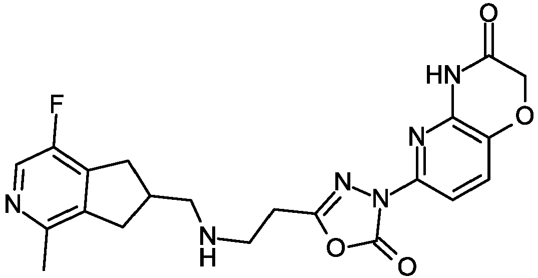 | |
| | ESP [M+H]⁺: 441.0 | |
| **69** | 2-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide; formic acid | Intermediate 1 and Intermediate 50 |
| | 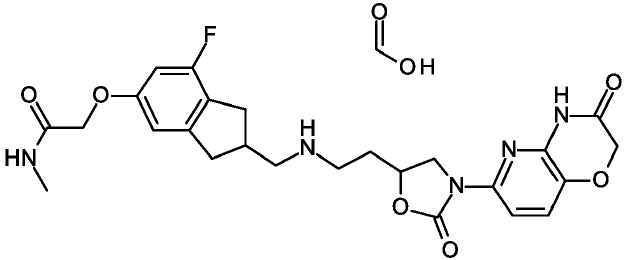 | |
| | ESP [M+H]⁺: 514.3 | |
| **70** | 6-[5-[2-[(4-fluoro-1-methoxy-6,7-dihydro-5*H-*cyclopenta[c]pyridin-6-yl)methylamino]ethyl]-2-oxo-oxazolidin-3-yl]-4*H*-pyrido[3,2-b][1,4]oxazin-3-one | Intermediate 1 and Intermediate 51 |
| | 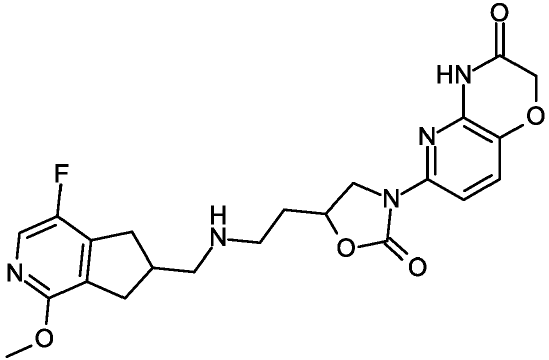 | |
| | ESP [M+H]⁺: 458.3 | |
| **71** | 6-[5-[2-[(4-fluoro-1-methoxy-6,7-dihydro-5*H-*cyclopenta[c]pyridin-6-yl)methylamino]ethyl]-2-oxo-oxazolidin-3-yl]-4*H*-pyrazino[2,3-b][1,4]oxazin-3-one | Intermediate 24 and Intermediate 51 |
| | 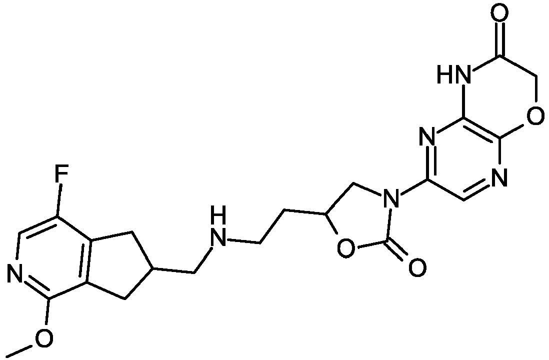 | |
| | ESP [M+H]⁺: 459.3 | |
| **72** | 6-[8-[(4-fluoro-1-methyl-6,7-dihydro-5*H*-cyclopenta[c]pyridin-6-yl)methyl]-2-oxo-1-oxa-3,8-diazaspiro[4.5]decan-3-yl]-4*H-*pyrido[3,2-b][1,4]oxazin-3-one | Intermediate 17 and Intermediate 27 |
| | 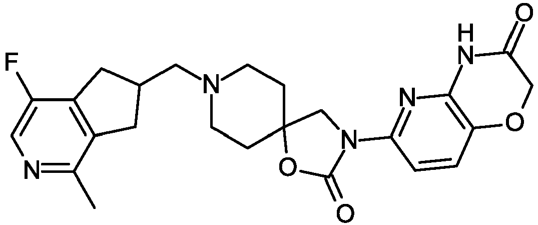 | |
| | ESP [M+H]⁺: 468.4 | |
| **73** | 2-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]acetonitrile; formic acid | Intermediate 1 and Intermediate 52 |
| | 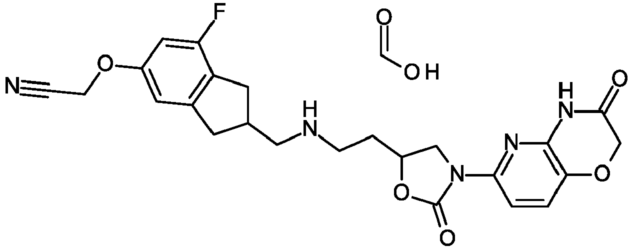 | |
| | ESP [M+H]⁺: 482.2 | |
| **74** | 2-[[7-fluoro-2-[[2-[2-oxo-3-(3-oxo-4*H*-pyrido[3,2-b][1,4]thiazin-6-yl)-1,3-oxazolidin-5-yl]ethylamino]methyl]-2,3-dihydro-1*H*-inden-5-yl]oxy]-*N*-methylacetamide; formic acid | Intermediate 48 and Intermediate 50 |
| | 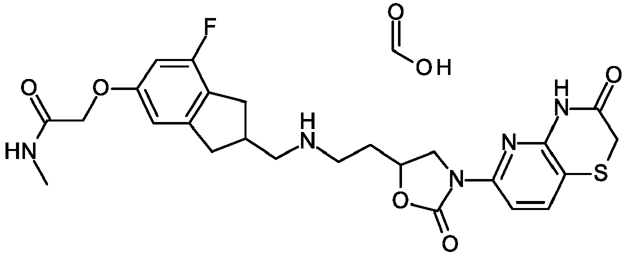 | |
| | ESP [M+H]⁺: 530.2 | |
| **75** | 6-[5-[1-[(4-fluoro-1-methyl-6,7-dihydro-5*H-*cyclopenta[c]pyridin-6-yl)methyl]azetidin-3-yl]-2-oxo-oxazolidin-3-yl]-4*H*-pyrazino[2,3-b][1,4]oxazin-3-one; formic acid | Intermediate 53 and Intermediate 27 |
| | 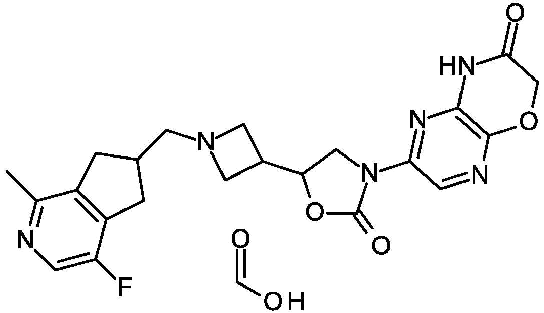 | |
| | ESP [M+H]⁺: 455.1 | |
| **76** | 2-[[3-[2-oxo-3-(3-oxo-4*H*-pyrazino[2,3-b][1,4]oxazin-6-yl)-1,3-oxazolidin-5-yl]azetidin-1-yl]methyl]-2,3-dihydro-1*H-*indene-4-carbonitrile; formic acid | Intermediate 53 and Intermediate 5 |
| | 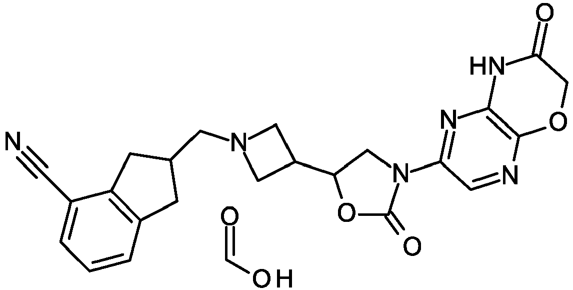 | |
| | ESP [M+H]⁺: 447.3 | |
| **77** | 2-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N,N*-dimethylacetamide | Intermediate 1 and Intermediate 54 |
| | 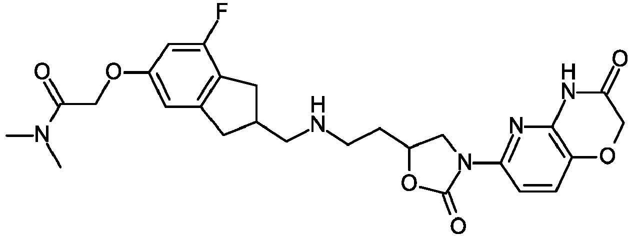 | |
| | ESP [M+Na]⁺: 550.3 | |
| **78** | 6-[5-[3-[(4-fluoro-1-methyl-6,7-dihydro-5*H-*cyclopenta[c]pyridin-6-yl)amino]propyl]-2-oxo-1,3-oxazolidin-3-yl]-4*H*-pyrido[3,2-b][1,4]oxazin-3-one | Intermediate 16 and Intermediate 153 |
| | 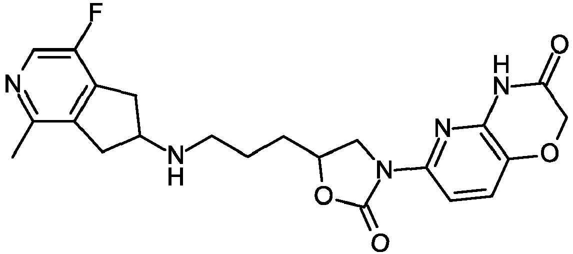 | |
| | ESP [M+H]⁺: 442.2 | |
| **79** | 6-[5-[3-[(4-fluoro-1-methyl-6,7-dihydro-5*H-*cyclopenta[c]pyridin-6-yl)amino]propyl]-2-oxo-1,3-oxazolidin-3-yl]-4*H*-pyrazino[2,3-b][1,4]oxazin-3-one | Intermediate 154 and Intermediate 153 |
| | 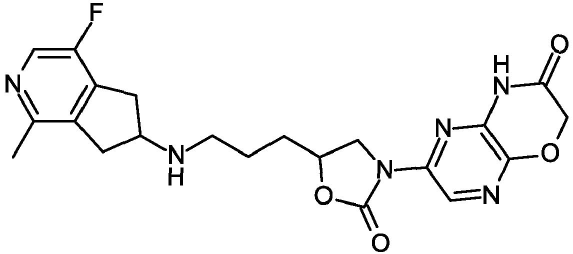 | |
| | ESP [M+H]⁺: 443.1 | |
| **80** | 2-[3-[2-oxo-3-(3-oxo-4*H*-pyrazino[2,3-b][1,4]oxazin-6-yl)-1,3-oxazolidin-5-yl]propylamino]-2,3-dihydro-1*H*-indene-4-carbonitrile | Intermediate 155 and Intermediate 6 |
| | 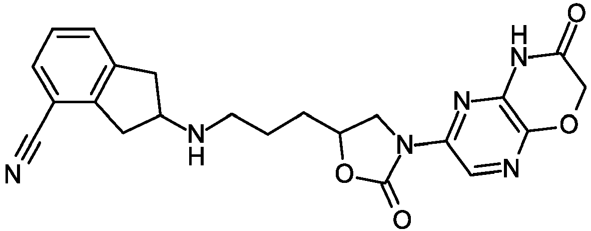 | |
| | ESP [M+H]⁺: 435.1 | |
| **81** | 2-[[2-hydroxy-3-[2-oxo-3-(3-oxo-4*H*-pyrazino[2,3-b][1,4]oxazin-6-yl)-1,3-oxazolidin-5-yl]propyl]amino]-2,3-dihydro-1*H*-indene-4-carbonitrile, Isomer A; formic acid | Intermediate 156 and Intermediate 6 |
| | 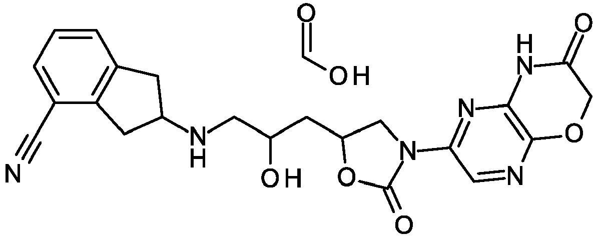 | |
| | ESP [M+H]⁺: 451.1 | |
| **82** | 2-[[2-hydroxy-3-[2-oxo-3-(3-oxo-4*H*-pyrazino[2,3-b][1,4]oxazin-6-yl)-1,3-oxazolidin-5-yl]propyl]amino]-2,3-dihydro-1*H*-indene-4-carbonitrile, Isomer B; formic acid | Intermediate 157 and Intermediate 6 |
| | 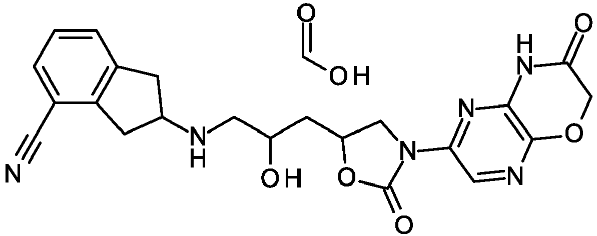 | |
| | ESP [M+H]⁺: 451.1 | |
| **83** | 6-[5-[3-[(4-fluoro-2,3-dihydro-1*H*-inden-2-yl)amino]-2-hydroxypropyl]-2-oxo-1,3-oxazolidin-3-yl]-4*H*-pyrazino[2,3-b][1,4]oxazin-3-one, Isomer A; formic acid | Intermediate 156 and 4-fluoro-1,3-dihydroinden-2-one [CAS# 1210824-58-0] |
| | 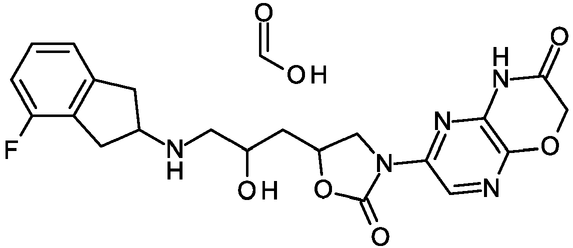 | |
| | ESP [M+H]⁺: 444.1 | |
| **84** | 6-[5-[3-[(4-fluoro-2,3-dihydro-1*H*-inden-2-yl)amino]-2-hydroxypropyl]-2-oxo-1,3-oxazolidin-3-yl]-4*H*-pyrazino[2,3-b][1,4]oxazin-3-one, Isomer B; formic acid | Intermediate 157 and 4-fluoro-1,3-dihydroinden-2-one [CAS# 1210824-58-0] |
| | 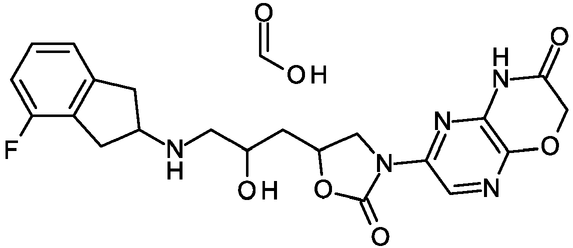 | |
| | ESP [M+H]⁺: 444.3 | |
| **85** | 6-[5-[2-[(4-fluoro-1-methyl-6,7-dihydro-5*H-*cyclopenta[c]pyridin-6-yl)methylamino]ethyl]-2-oxo-1,3-oxazol-3-yl]-4*H*-pyrido[3,2-b] [1,4]oxazin-3-one | Intermediate 183 and Intermediate 27 |
| | 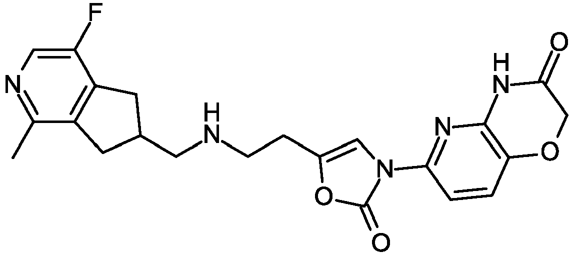 | |
| | ESP [M+H]⁺: 440.2 | |
| **277** | 6-[8-[(4-fluoro-1-methyl-6,7-dihydro-5*H*-cyclopenta[c]pyridin-6-yl)methyl]-6-(hydroxymethyl)-2-oxo-1-oxa-3,8-diazaspiro[4.5]decan-3-yl]-4*H*-pyrido[3,2-b][1,4]oxazin-3-one | Intermediate 196 and Intermediate 27 |
| | 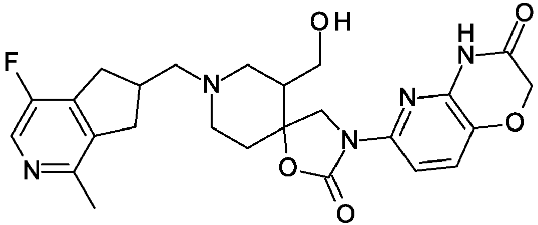 | |
| | ESP [M+H]⁺: 498.3 | |
| **278** | 6-[5-[[2-(4-fluoro-1-methyl-6,7-dihydro-5*H-*cyclopenta[c]pyridin-6-yl)ethylamino]methyl]-5-(2-hydroxyethyl)-2-oxo-1,3-oxazolidin-3-yl]-4*H*-pyrido[3,2-b][1,4]oxazin-3-one | Intermediate 197 and Intermediate 188 |
| | 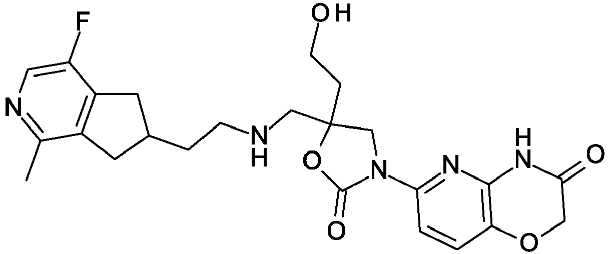 | |
| | ESP [M+H]⁺: 486.2 | |
| **279** | 6-[5-[2-[(1-methyl-6,7-dihydro-5*H*-cyclopenta[c]pyridin-6-yl)methylamino]ethyl]-2-oxo-1,3-oxazolidin-3-yl]-4*H-*pyrazino[2,3-b][1,4]oxazin-3-one;formic acid | Intermediate 24 and Intermediate 55 |
| | 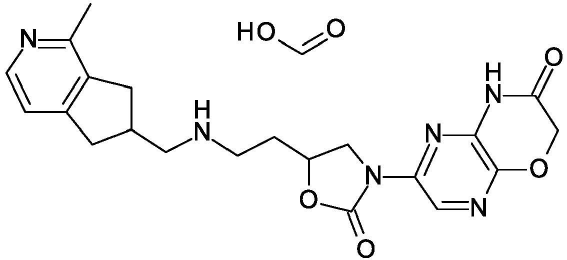 | |
| | ESP [M+H]⁺: 425.1 | |
| **283** | 6-[5-[2-[(1,4-dimethyl-6,7-dihydro-5*H*-cyclopenta[d]pyridazin-6-yl)methylamino]ethyl]-2-oxo-1,3-oxazolidin-3-yl]*-*4*H-*pyrazino[2,3-b][1,4]oxazin-3-one | Intermediate 24 and Intermediate 199 |
| | 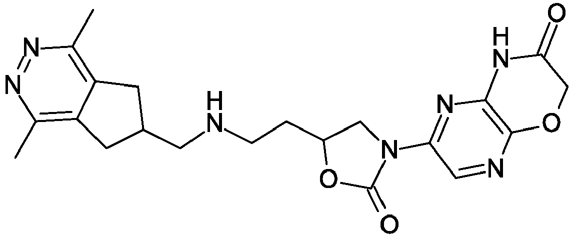 | |
| | ESP [M+H]⁺: 440.3 | |
| **284** | 6-[5-[2-[(1,4-dimethyl-6,7-dihydro-5*H*-cyclopenta[d]pyridazin-6-yl)methylamino]ethyl]-2-oxo-1,3-oxazolidin-3-yl]-4*H-*pyrido[3,2-b][1,4]oxazin-3-one | Intermediate 1 and Intermediate 199 |
| | 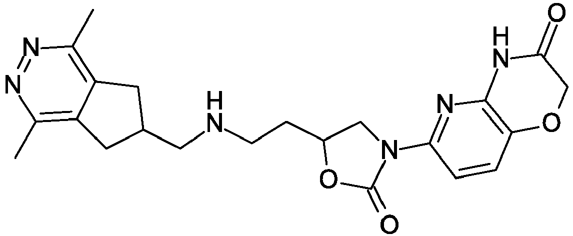 | |
| | ESP [M+H]⁺: 439.1 | |
| **285** | 5-[2-[(1,4-dimethyl-6,7-dihydro-5*H*-cyclopenta[d]pyridazin-6-yl)methylamino]ethyl]-3-(3-oxo-4*H*-pyrido[3,2-b][1,4]thiazin-6-yl)-1,3-oxazolidin-2-one | Intermediate 48 and Intermediate 199 |
| | 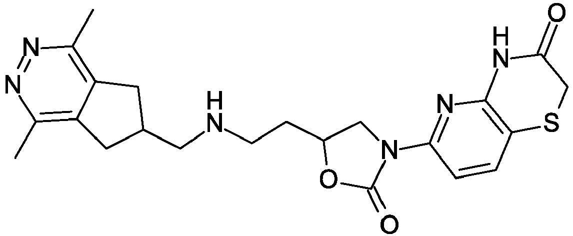 | |
| | ESP [M+H]⁺: 455.2 | |
| **286** | 2-[[4-cyano-2-[[2-[2-oxo-3-(3-oxo-4*H*-pyrazino[2,3-b] [1,4]oxazin-6-yl)-1,3-oxazolidin-5-yl]ethylamino]methyl]-2,3-dihydro-1*H*-inden-5-yl]oxy]-*N*-methylacetamide | Intermediate 24 and Intermediate 36 |
| | 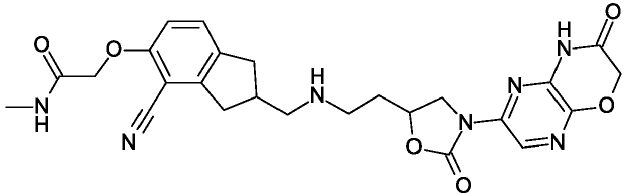 | |
| | ESP [M+H]⁺: 522.2 | |
| **287** | 6-[5-[2-[(4-fluoro-6,7-dihydro-5*H*-cyclopenta[c]pyridin-6-yl)methylamino]ethyl]-2-oxo-1,3-oxazolidin-3-yl]-4*H-*pyrazino[2,3-b][1,4]oxazin-3-one | Intermediate 24 and Intermediate 200 |
| | 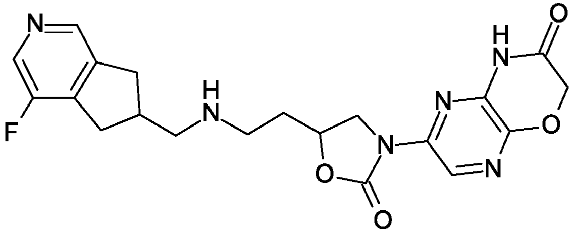 | |
| | ESP [M+H]⁺: 429.1 | |
| **288** | 6-[5-[2-[(4-fluoro-6,7-dihydro-5*H*-cyclopenta[c]pyridin-6-yl)methylamino]ethyl]-2-oxo-1,3-oxazolidin-3-yl]-4*H-*pyrido[3,2-b][1,4]oxazin-3-one | Intermediate 1 and Intermediate 200 |
| | 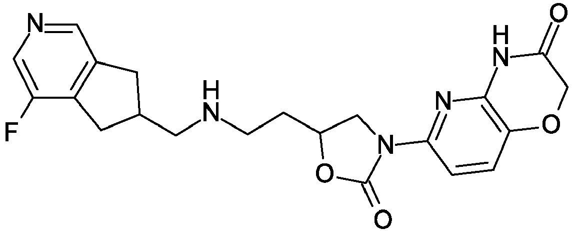 | |
| | ESP [M+H]⁺: 428.2 | |
| **289** | 2-[[2-[2-oxo-3-(7-oxo-8*H*-pyrimido[5,4-b][1,4]oxazin-2-yl)-1,3-oxazolidin-5-yl]ethylamino]methyl]-2,3-dihydro-1*H-*indene-4-carbonitrile | Intermediate 201 and Intermediate 5 |
| | 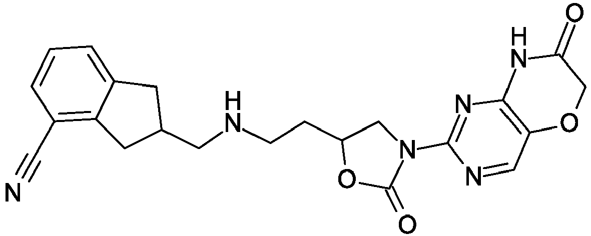 | |
| | ESP [M+H]⁺: 435.1 | |
| **290** | 2-[5-[2-[(*rac*-1,2-*trans*-1-amino-4-chloro-2,3-dihydro-1*H-*inden-2-yl)methylamino]ethyl]-2-oxo-1,3-oxazolidin-3-yl]-8*H-*pyrimido[5,4-b][1,4]oxazin-7-one | Intermediate 201 and Intermediate 92 |
| | 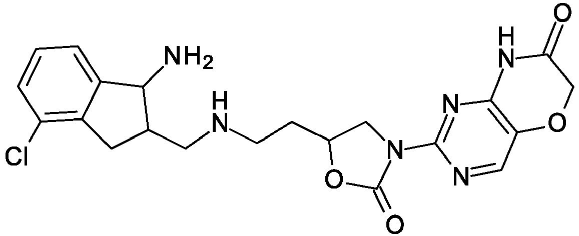 | |
| | ESP [M+H]⁺: 459.1 | |
| **291** | 1-methyl-6-[[2-[2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridine-4-carbonitrile;2,2,2-trifluoroacetic acid | Intermediate 24 and Intermediate 35 |
| | 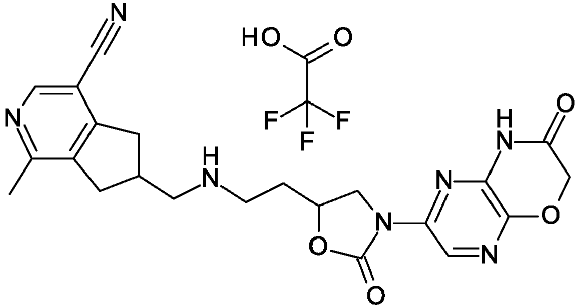 | |
| | ESP [M+H]⁺: 450.2 | |
| **292** | 6-[5-[2-[[4-fluoro-6-(2-hydroxy-2-methylpropoxy)-2,3-dihydro-1*H*-inden-2-yl]methylamino]ethyl]-2-oxo-1,3-oxazolidin-3-yl]-4*H*-pyrazino[2,3-b][1,4]oxazin-3-one | Intermediate 24 and Intermediate 202 |
| | 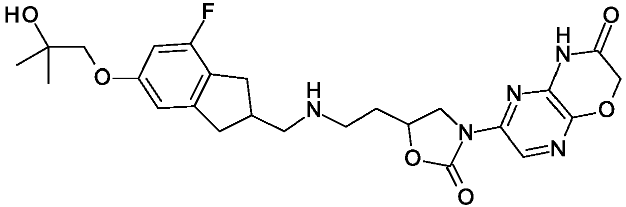 | |
| | ESP [M+H]⁺: 516.4 | |
| **293** | 6-[5-[2-[(4-fluoro-1-methyl-6,7-dihydro-5*H-*cyclopenta[c]pyridin-6-yl)methylamino]ethyl]-2-oxo-1,3-oxazol-3-yl]-4*H*-pyrazino[2,3-b][1,4]oxazin-3-one | Intermediate 27 and Intermediate 203 |
| | 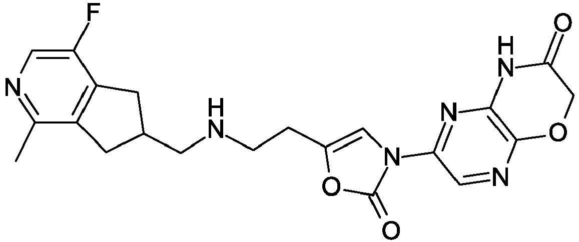 | |
| | ESP [M+H]⁺: 441.2 | |
| | 6-[5-[2-[[6-[2-(5-aminotetrazol-1-yl)ethoxy]-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;formic | |
| **294** | 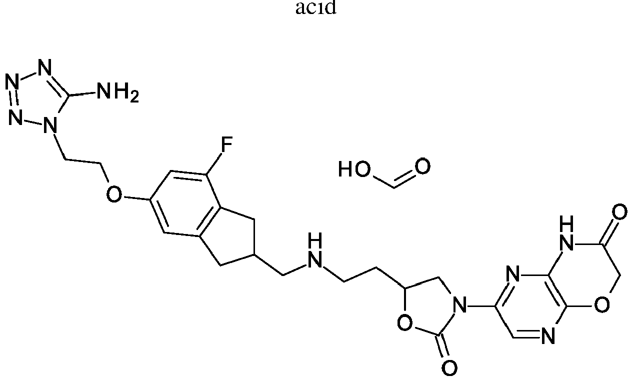 | Intermediate 24 and Intermediate 205 |
| | ESP [M+H]⁺: 555.3 | |
| | 6-[5-[2-[[6-[2-(5-aminotetrazol-2-yl)ethoxy]-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;formic acid | |
| **295** | 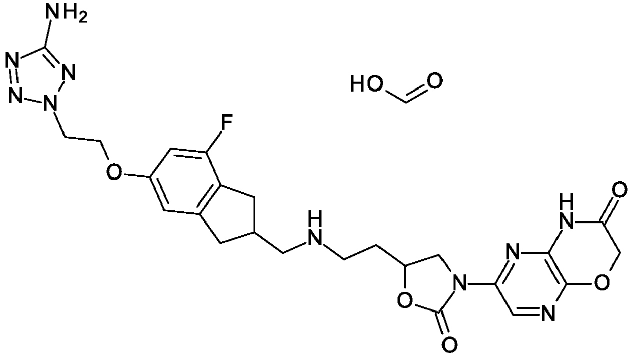 | Intermediate 24 and Intermediate 206 |
| | ESP [M+H]⁺: 555.3 | |
| **296** | 6-[5-[2-[[4-fluoro-6-[2-(1,2,4-triazol-1-yl)ethoxy]-2,3-dihydro-1*H*-inden-2-yl]methylamino]ethyl]-2-oxo-1,3-oxazolidin-3-yl]-4*H*-pyrazino[2,3-b][1,4]oxazin-3-one;formic acid | Intermediate 24 and Intermediate 207 |
| | 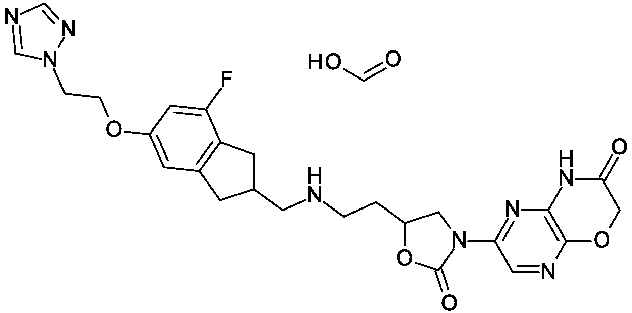 | |
| | ESP [M+H]⁺: 539.3 | |
| **299** | 6-[5-[2-[(3-fluoro-4-methyl-6,7-dihydro-5*H-*cyclopenta[b]pyridin-6-yl)methylamino]ethyl]-2-oxo-1,3-oxazolidin-3-yl]-4*H*-pyrazino[2,3-b][1,4]oxazin-3-one | Intermediate 24 and Intermediate 209 |
| | 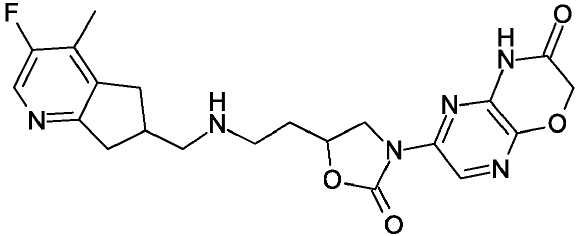 | |
| | ESP [M+H]⁺: 443.2 | |
| **300** | 6-[5-[2-[(3-fluoro-4-methyl-6,7-dihydro-5*H-*cyclopenta[b]pyridin-6-yl)methylamino]ethyl]-2-oxo-1,3-oxazolidin-3-yl]-4*H*-pyrido[3,2-b][1,4]oxazin-3-one | Intermediate 1 and Intermediate 209 |
| | 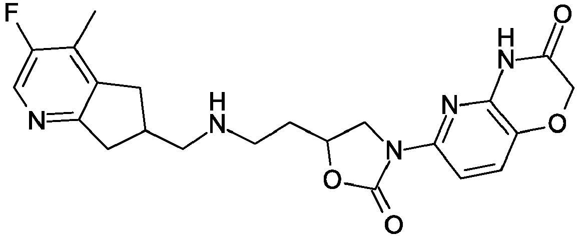 | |
| | ESP [M+H]⁺: 442.2 | |
| **301** | 6-[8-[(3-fluoro-4-methyl-6,7-dihydro-5*H*-cyclopenta[b]pyridin-6-yl)methyl]-2-oxo-1-oxa-3,8-diazaspiro[4.5]decan-3-yl]-4*H-*pyrazino[2,3-b][1,4]oxazin-3-one | Intermediate 186 and Intermediate 209 |
| | 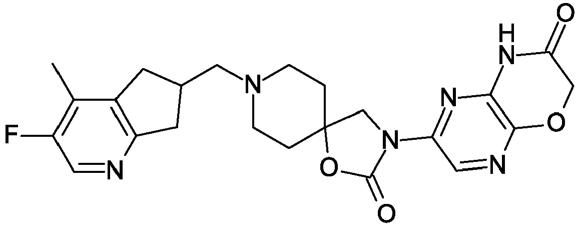 | |
| | ESP [M+H]⁺: 469.3 | |
| **304** | 6-[5-[2-[[4-fluoro-6-[2-(2-oxo-1,3-oxazolidin-3-yl)ethoxy]-2,3-dihydro-1*H*-inden-2-yl]methylamino]ethyl]-2-oxo-1,3-oxazolidin-3-yl]-4*H*-pyrazino[2,3-b][1,4]oxazin-3-one;formic acid | Intermediate 24 and Intermediate 211 |
| | 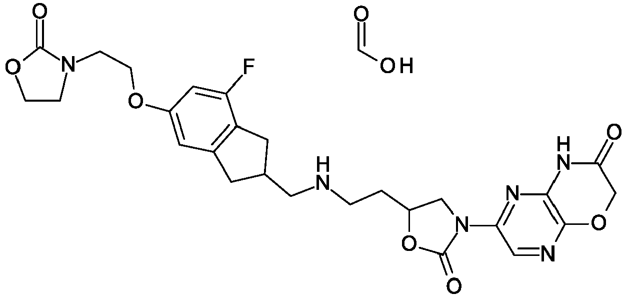 | |
| | ESP [M+H]⁺: 557.3 | |
| **305** | 2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy] acetamide | Intermediate 24 and Intermediate 212 |
| | 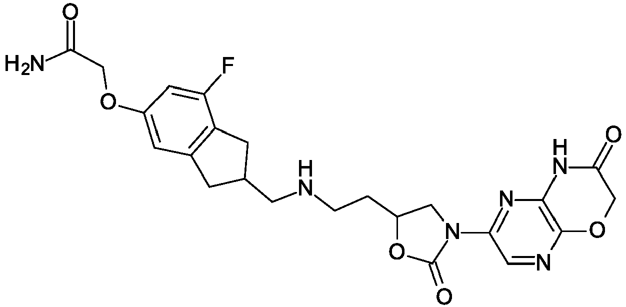 | |
| | ESP [M+H]⁺: 501.2 | |
| **306** | 2-[[7-fluoro-2-[[2-[2-oxo-3-(3-oxo-4*H*-pyrazino[2,3-b][1,4]oxazin-6-yl)-1,3-oxazol-5-yl]ethylamino]methyl]-2,3-dihydro-1*H*-inden-5-yl]oxy]acetamide;formic acid | Intermediate 203 and Intermediate 212 |
| | 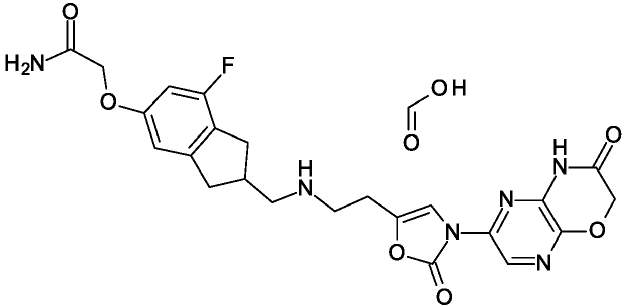 | |
| | ESP [M+Na]⁺: 521.3 | |
| **307** | 6-[5-[2-[[6-[2-(azetidin-1-yl)-2-oxoethoxy]-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;formic acid | Intermediate 24 and Intermediate 213 |
| | 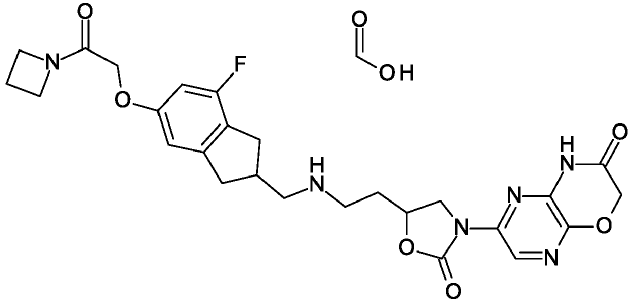 | |
| | ESP [M+H]⁺: 541.2 | |
| **308** | *N*-ethyl-2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide;formic acid | Intermediate 24 and Intermediate 214 |
| | 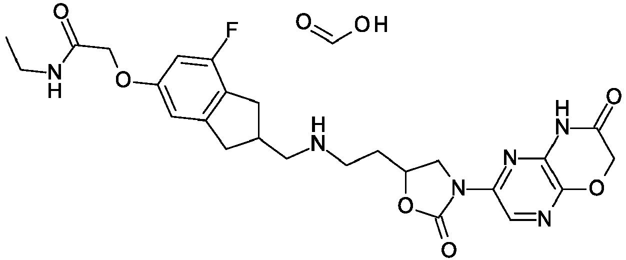 | |
| | ESP [M+H]⁺: 529.1 | |
| **309** | 2-[[7-fluoro-2-[[2-oxo-3-(3-oxo-4*H*-pyrazino[2,3-b][1,4]oxazin-6-yl)-1-oxa-3,8-diazaspiro[4.5]decan-8-yl]methyl]-2,3-dihydro-1*H*-inden-5-yl]oxy]-N-methylacetamide | Intermediate 186 and Intermediate 50 |
| | 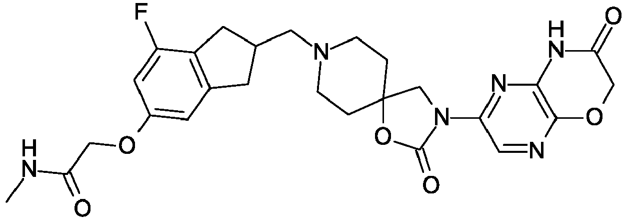 | |
| | ESP [M+H]⁺: 541.3 | |
| **310** | 2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide;formic acid | Intermediate 24 and Intermediate 50 |
| | 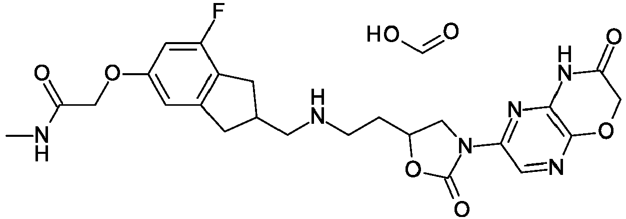 | |
| | ESP [M+H]⁺: 515.3 | |
| **312** | 2-[[4,7-difluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide | Intermediate 24 and Intermediate 216 |
| | 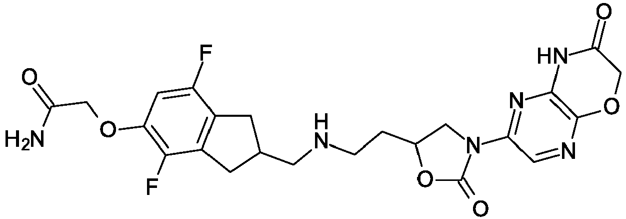 | |
| | ESP [M+H]⁺: 519.3 | |
| **318** | 6-[5-[2-[(4-fluoro-6,7-dihydro-5*H*-cyclopenta[b]pyridin-6-yl)methylamino]ethyl]-2-oxo-1,3-oxazolidin-3-yl]-4*H-*pyrazino[2,3-b][1,4]oxazin-3-one | Intermediate 24 and Intermediate 223 |
| | 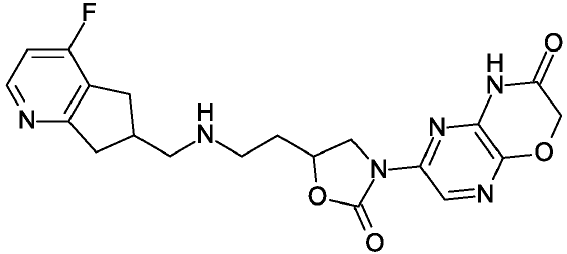 | |
| | ESP [M+H]⁺: 429.1 | |
| **319** | 6-[5-[2-[(4-chloro-3-fluoro-6,7-dihydro-5*H-*cyclopenta[b]pyridin-6-yl)methylamino]ethyl]-2-oxo-1,3-oxazolidin-3-yl]-4*H*-pyrazino[2,3-b][1,4]oxazin-3-one;formic acid | Intermediate 24 and Intermediate 224 |
| | 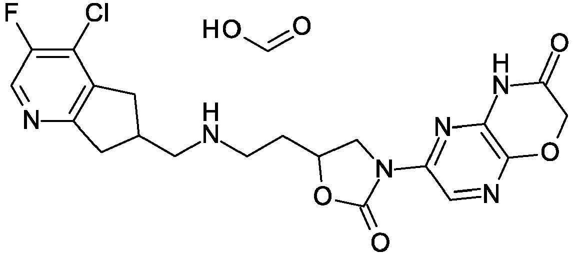 | |
| | ESP [M+H]⁺: 463.2 | |
| **323** | 6-[5-[2-[[4-fluoro-6-(oxetan-3-yloxy)-2,3-dihydro-1*H*-inden-2-yl]methylamino]ethyl]-2-oxo-1,3-oxazolidin-3-yl]-4*H-*pyrazino[2,3-b][1,4]oxazin-3-one | Intermediate 24 and Intermediate 229 |
| | 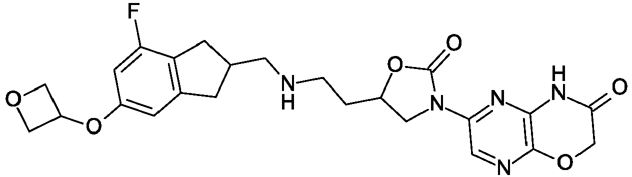 | |
| | ESP [M+H]⁺: 500.3 | |
| **324** | N-[2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]ethyl]methanesulfonamide | |
| | 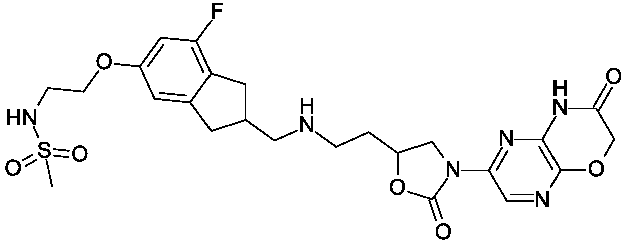 | Intermediate 24 and Intermediate 230 |
| | ESP [M+H]⁺: 565.2 | |
| **325** | *N*-[2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]ethyl]-*N-*methylmethanesulfonamide | Intermediate 24 and Intermediate 232 |
| | 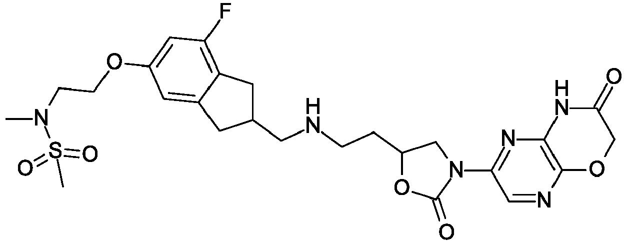 | |
| | ESP [M+H]⁺: 579.3 | |
| **326** | 6-[2-[2-(4-fluoro-1-methyl-6,7-dihydro-5*H-*cyclopenta[c]pyridin-6-yl)ethyl]-6-oxo-5-oxa-2,7-diazaspiro[3.4]octan-7-yl]-4*H*-pyrazino[2,3-b][1,4]oxazin-3-one | Intermediate 226 and Intermediate 188 |
| | 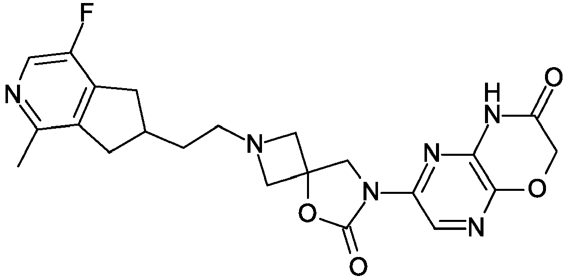 | |
| | ESP [M+H]⁺: 455.2 | |
| **330** | 6-[(5*S*)-5-[2-[1-(4-fluoro-1-methyl-6,7-dihydro-5*H-*cyclopenta[c]pyridin-6-yl)ethylamino]ethyl]-2-oxo-1,3-oxazolidin-3-yl]-4*H*-pyrazino[2,3-b][1,4]oxazin-3-one; formic acid | Intermediate 236 and Intermediate 240 |
| | 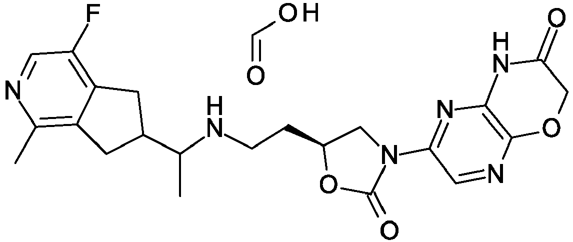 | |
| | ESP [M+H]⁺: 457.1 | |
| **331** | 6-[(5*R*)-5-[2-[1-(4-fluoro-1-methyl-6,7-dihydro-5*H-*cyclopenta[c]pyridin-6-yl)ethylamino]ethyl]-2-oxo-1,3-oxazolidin-3-yl]-4*H*-pyrazino[2,3-b][1,4]oxazin-3-one; formic acid | Intermediate 236 and Intermediate 241 |
| | 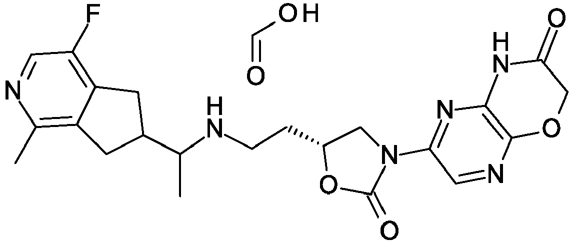 | |
| | ESP [M+H]⁺: 457.3 | |
| **332** | 6-[5-[2-[[4-fluoro-6-(2-imidazol-1-ylethoxy)-2,3-dihydro-1*H-*inden-2-yl]methylamino]ethyl]-2-oxo-1,3-oxazolidin-3-yl]-4*H-*pyrazino[2,3-b][1,4]oxazin-3-one | Intermediate 24 and Intermediate 237 |
| | 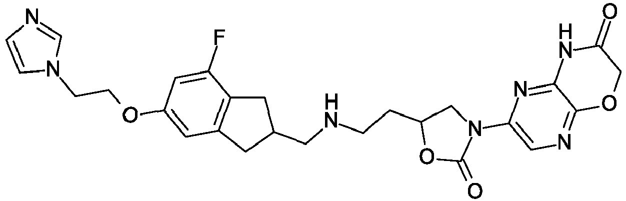 | |
| | ESP [M+H]⁺: 538.3 | |
| **333** | 6-[5-[3-[(4-fluoro-1-methyl-6,7-dihydro-5*H-*cyclopenta[c]pyridin-6-yl)amino]butyl]-2-oxo-1,3-oxazolidin-3-yl]-4*H*-pyrazino[2,3-b][1,4]oxazin-3-one, Isomer A | Intermediate 153 and Intermediate 238 |
| | 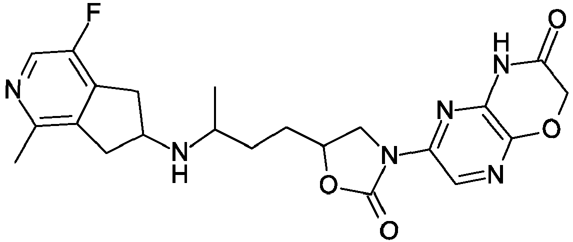 | |
| | ESP [M+H]⁺: 457.1 | |
| **334** | 6-[5-[3-[(4-fluoro-1-methyl-6,7-dihydro-5*H-*cyclopenta[c]pyridin-6-yl)amino]butyl]-2-oxo-1,3-oxazolidin-3-yl]-4*H*-pyrazino[2,3-b][1,4]oxazin-3-one, Isomer B | Intermediate 153 and Intermediate 239 |
| | 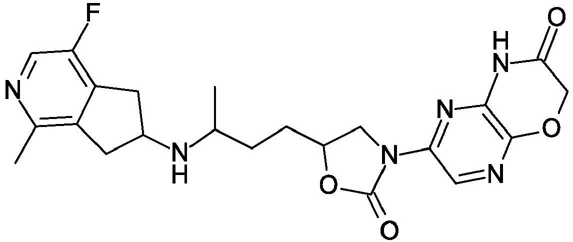 | |
| | ESP [M+H]⁺: 457.1 | |
| **336** | 2-[[4-fluoro-2-[[2-oxo-3-(3-oxo-4*H*-pyrazino[2,3-b][1,4]oxazin-6-yl)-1-oxa-3,8-diazaspiro[4.5]decan-8-yl]methyl]-2,3-dihydro-1*H*-inden-5-yl]oxy]-N-methylacetamide | Intermediate 186 and Intermediate 243 |
| | 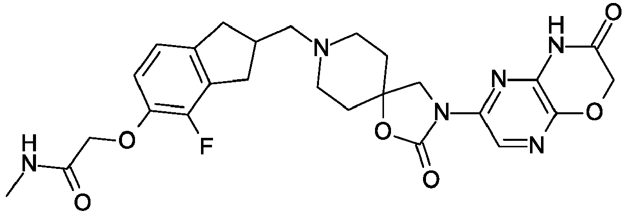 | |
| | ESP [M+H]⁺: 541.2 | |
| **338** | 6-[(5*S*)-5-[2-[(4-fluoro-3-methyl-6,7-dihydro-5*H-*cyclopenta[c]pyridin-6-yl)methylamino]ethyl]-2-oxo-1,3-oxazolidin-3-yl]-4*H*-pyrazino[2,3-b][1,4]oxazin-3-one | Intermediate 240 and Intermediate 245 |
| | 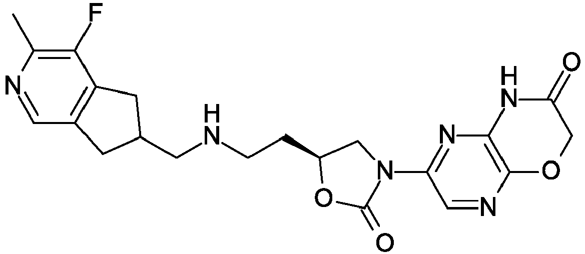 | |
| | ESP [M+H]⁺: 443.0 | |
| **339** | 6-[(5*R*)-5-[2-[(4-fluoro-3-methyl-6,7-dihydro-5*H-*cyclopenta[c]pyridin-6-yl)methylamino]ethyl]-2-oxo-1,3-oxazolidin-3-yl]-4*H*-pyrazino[2,3-b][1,4]oxazin-3-one | Intermediate 241 and Intermediate 245 |
| | 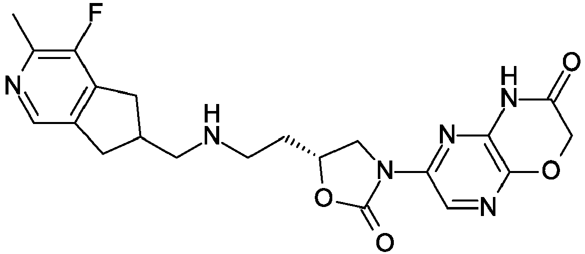 | |
| | ESP [M+H]⁺: 443.1 | |
| **340** | 6-[5-[2-[(4-fluoro-1-methyl-6,7-dihydro-5*H-*cyclopenta[c]pyridin-6-yl)methylamino]ethyl]-5-methyl-2-oxo-1,3-oxazolidin-3-yl]-4*H*-pyrazino[2,3-b][1,4]oxazin-3-one | Intermediate 246 and Intermediate 27 |
| | 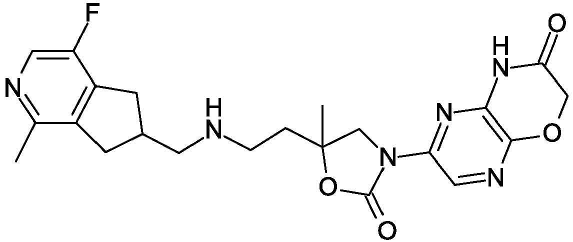 | |
| | ESP [M+H]⁺: 457.3 | |
| **343** | 6-[5-[2-[[4-fluoro-6-[2-(triazol-2-yl)ethoxy]-2,3-dihydro-1*H-*inden-2-yl]methylamino]ethyl]-2-oxo-1,3-oxazolidin-3-yl]-4*H-*pyrazino[2,3-b][1,4]oxazin-3-one;formic acid | Intermediate 24 and Intermediate 248 |
| | 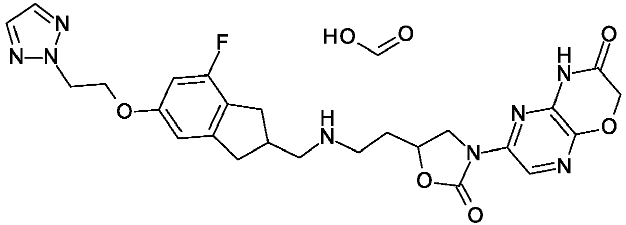 | |
| | ESP [M+H]⁺: 539.3 | |
| **344** | 6-[5-[2-[[4-fluoro-6-[2-(triazol-1-yl)ethoxy]-2,3-dihydro-1*H-*inden-2-yl]methylamino]ethyl]-2-oxo-1,3-oxazolidin-3-yl]-4*H-*pyrazino[2,3-b][1,4]oxazin-3-one;formic acid | Intermediate 24 and Intermediate 249 |
| | 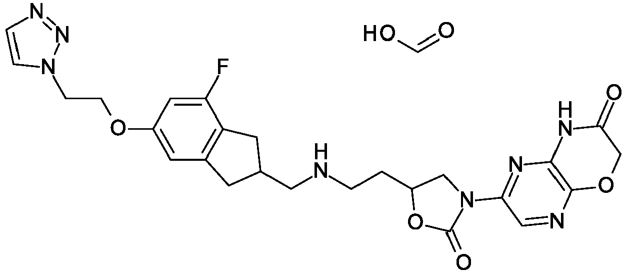 | |
| | ESP [M+H]⁺: 539.3 | |
| **345** | 1-[2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]ethyl]pyrrolidine-2,5-dione;formic acid | Intermediate 24 and Intermediate 250 |
| | 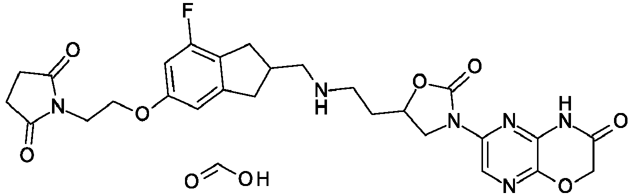 | |
| | ESP [M+H]⁺: 569.3 | |
| **346** | *N*-cyclopropyl-2-[[7-fluoro-2-[[2-[2-oxo-3-(3-oxo-4*H-*pyrazino[2,3-b][1,4]oxazin-6-yl)-1,3-oxazolidin-5-yl]ethylamino]methyl]-2,3-dihydro-1*H*-inden-5-yl]oxy]acetamide;formic acid | Intermediate 24 and Intermediate 251 |
| | 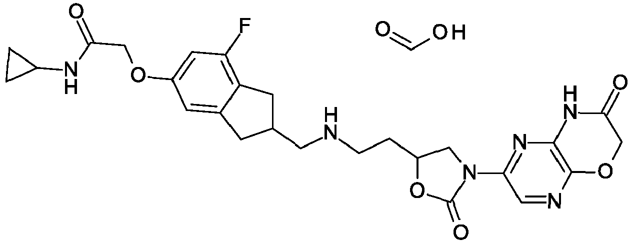 | |
| | ESP [M+H]⁺: 541.3 | |
| **347** | 6-[5-[2-[[4-fluoro-6-[2-(tetrazol-1-yl)ethoxy]-2,3-dihydro-1*H-*inden-2-yl]methylamino]ethyl]-2-oxo-1,3-oxazolidin-3-yl]-4*H-*pyrazino[2,3-b][1,4]oxazin-3-one;formic acid | Intermediate 24 and Intermediate 252 |
| | 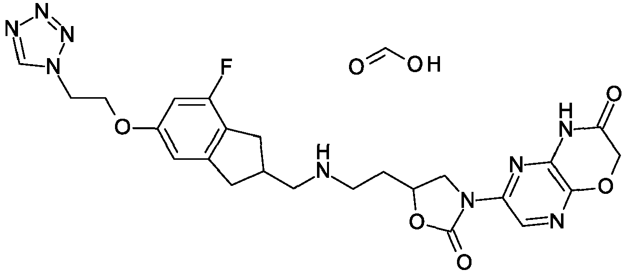 | |
| | ESP [M+H]⁺: 540.3 | |
| **348** | 6-[5-[2-[[4-fluoro-6-[2-(tetrazol-2-yl)ethoxy]-2,3-dihydro-1*H-*inden-2-yl]methylamino]ethyl]-2-oxo-1,3-oxazolidin-3-yl]-4*H-*pyrazino[2,3-b][1,4]oxazin-3-one;formic acid | Intermediate 24 and Intermediate 254 |
| | 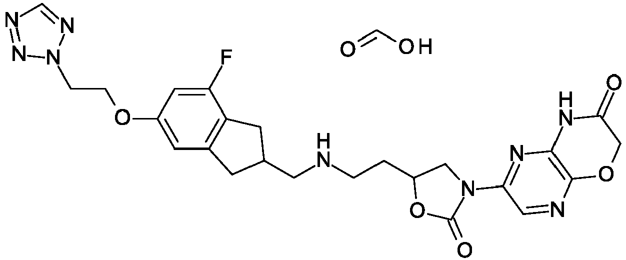 | |
| | ESP [M+H]⁺: 540.3 | |
| **349** | 2-[4-[2-oxo-3-(3-oxo-4*H*-pyrazino[2,3-b][1,4]oxazin-6-yl)-1,3-oxazolidin-5-yl]piperidin-1-yl]-2,3-dihydro-1*H*-indene-4-carbonitrile | Intermediate 6 and Intermediate 253 |
| | 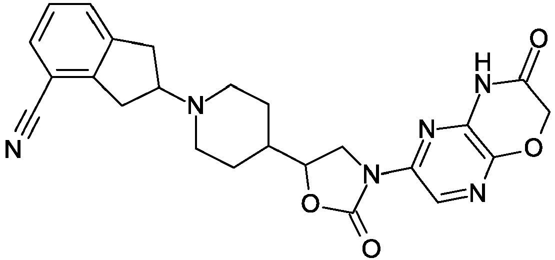 | |
| | ESP [M+H]⁺: 461.3 | |
| **350** | 6-[5-[1-(4-fluoro-2,3-dihydro-1*H*-inden-2-yl)piperidin-4-yl]-2-oxo-1,3-oxazolidin-3-yl]-4*H*-pyrazino[2,3-b][1,4]oxazin-3-one | 4-fluoro-1,3-dihydroinden-2-one [CAS# 1210824-58-0] and Intermediate 253 |
| | 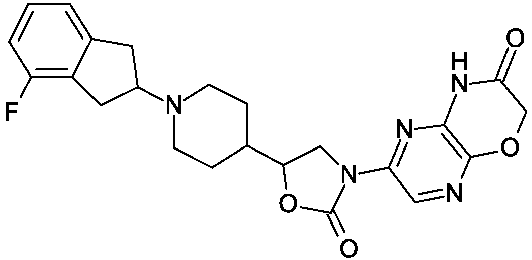 | |
| | ESP [M+H]⁺: 454.1 | |
| **352** | 6-[5-[2-[(4-fluoro-1,3-dimethyl-6,7-dihydro-5*H-*cyclopenta [c]pyridin-6-yl)methylamino] ethyl]-2-oxo-1,3-oxazolidin-3-yl]-4*H*-pyrazino[2,3-b][1,4]oxazin-3-one;formic acid | Intermediate 24 and Intermediate 256 |
| | 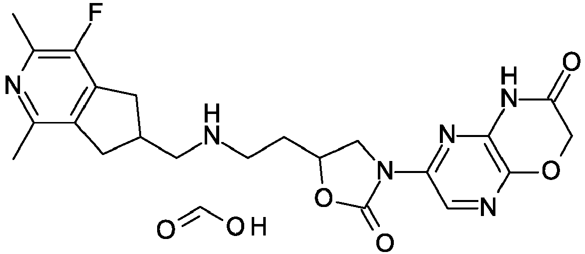 | |
| | ESP [M+H]⁺: 457.2 | |
| **353** | 6-[5-[2-[[4-fluoro-6-(2-methylsulfonylethoxy)-2,3-dihydro-1*H-*inden-2-yl]methylamino]ethyl]-2-oxo-1,3-oxazoldin-3-yl]-4*H-*pyrazino[2,3-b][1,4]oxazin-3-one;formic acid | Intermediate 24 and Intermediate 257 |
| | 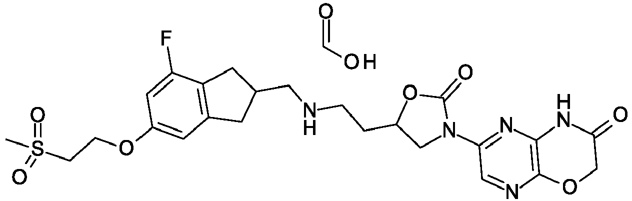 | |
| | ESP [M+H]⁺: 550.1 | |
| **354** | 6-[5-[2-[[6-(1-acetylazetidin-3-yl)oxy-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;formic acid | Intermediate 24 and Intermediate 258 |
| | 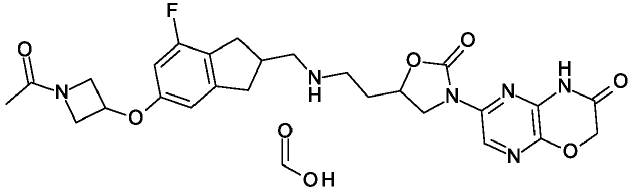 | |
| | ESP [M+H]⁺: 541.4 | |
| **355** | 6-[5-[2-[[4-fluoro-6-(1-methylsulfonylazetidin-3-yl)oxy-2,3-dihydro-1*H*-inden-2-yl]methylamino]ethyl]-2-oxo-1,3-oxazolidin-3-yl]-4*H*-pyrazino[2,3-b][1,4]oxazin-3-one;hydrochloride | Intermediate 24 and Intermediate 259 |
| | 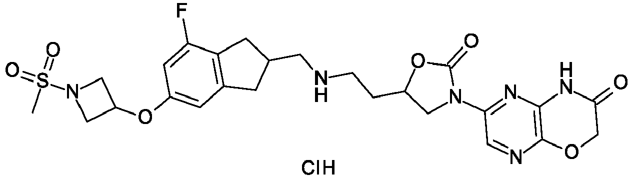 | |
| | ESP [M+H]⁺: 577.0 | |
| **361** | 6-[5-[*trans*-3-[(4-fluoro-1-methyl-6,7-dihydro-5*H-*cyclopenta[c]pyridin-6-yl)methylamino]cyclobutyl]-2-oxo-1,3-oxazolidin-3-yl]-4*H*-pyrazino[2,3-b][1,4]oxazin-3 -one | Intermediate 27 and Intermediate 263 |
| | 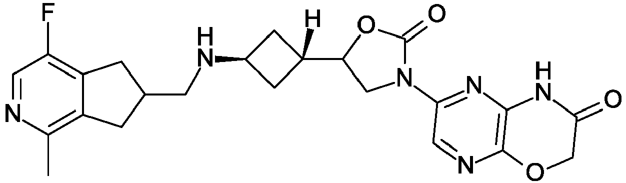 | |
| | ESP [M+H]⁺: 469.1 | |
| **362** | 6-[5-[*cis*-3-[(4-fluoro-1-methyl-6,7-dihydro-5*H-*cyclopenta[c]pyridin-6-yl)methylamino]cyclobutyl]-2-oxo-1,3-oxazolidin-3-yl]-4*H*-pyrazino[2,3-b][1,4]oxazin-3-one | Intermediate 27 and Intermediate 264 |
| | 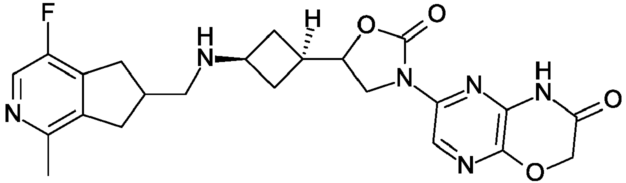 | |
| | ESP [M+H]⁺: 469.2 | |
| **363** | 4-fluoro-3-methyl-6-[[2-[2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridine-1-carbonitrile;formic acid | Intermediate 24 and Intermediate 266 |
| | 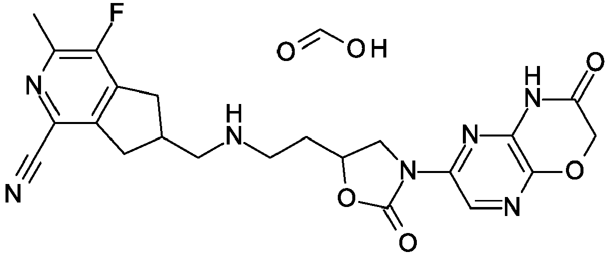 | |
| | ESP [M+H]⁺: 468.2 | |
| **364** | 1-methyl-6-[[2-[(5*S*)-2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridine-4-carbonitrile | Intermediate 240 and Intermediate 35 |
| | 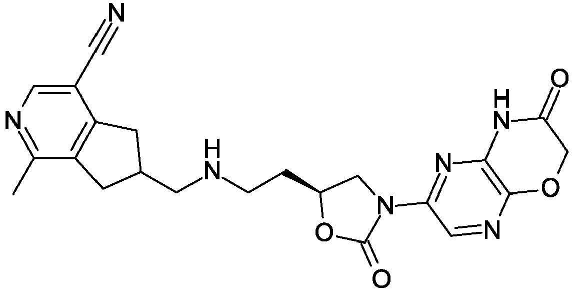 | |
| | ESP [M+H]⁺: 450.1 | |
| **365** | 1-methyl-6-[[2-[(5R)-2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridine-4-carbonitrile | Intermediate 241 and Intermediate 35 |
| | 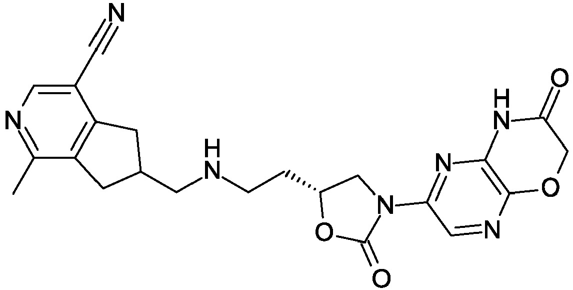 | |
| | ESP [M+H]⁺: 450.1 | |
| **366** | 2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetonitrile;formic acid | Intermediate 24 and Intermediate 52 |
| | 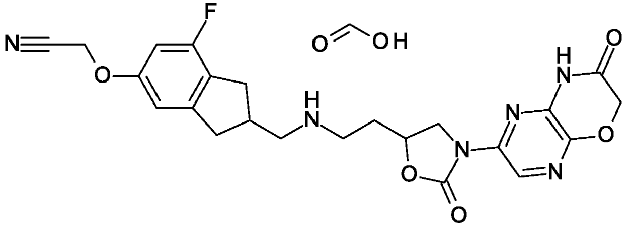 | |
| | ESP [M+H]⁺: 483.2 | |
| **371** | 6-[5-[2-[(1-methyl-6,7-dihydro-5*H*-cyclopenta[c]pyridin-6-yl)methylamino]ethyl]-2-oxo-1,3-oxazol-3-yl]-4*H-*pyrazino[2,3-b] [1,4]oxazin-3-one | Intermediate 203 and Intermediate 55 |
| | 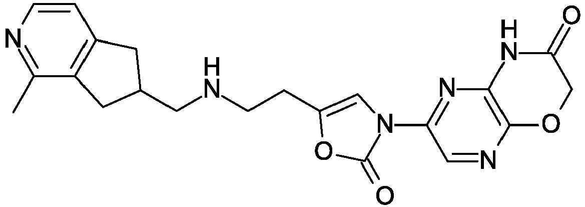 | |
| | ESP [M+H]⁺: 423.1 | |
| **372** | 6-[5-[2-[(4-fluoro-6,7-dihydro-5*H*-cyclopenta[c]pyridin-6-yl)methylamino]ethyl]-2-oxo-1,3-oxazol-3-yl]-4*H-*pyrazino[2,3-b][1,4]oxazin-3-one | Intermediate 203 and Intermediate 200 |
| | 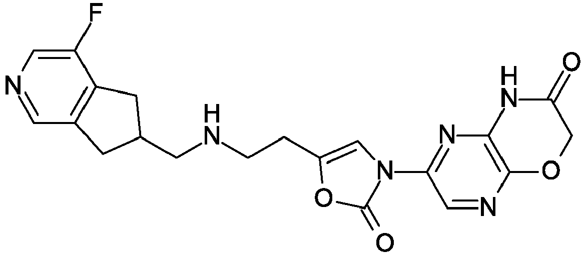 | |
| | ESP [M+H]⁺: 427.2 | |
| **373** | 6-[8-[(1-chloro-6,7-dihydro-5*H*-cyclopenta[c]pyridin-6-yl)methyl]-2-oxo-1-oxa-3,8-diazaspiro[4.5]decan-3-yl]-4*H-*pyrazino[2,3-b][1,4]oxazin-3-one | Intermediate 186 and Intermediate 19 |
| | 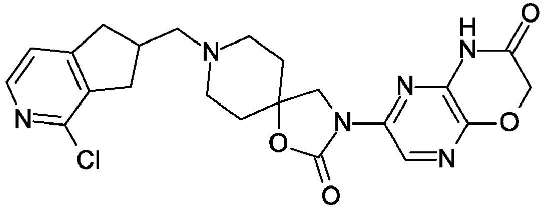 | |
| | ESP [M+H]⁺: 471.2 | |
| **374** | 6-[8-[(1-methyl-6,7-dihydro-5*H*-cyclopenta[c]pyridin-6-yl)methyl]-2-oxo-1-oxa-3,8-diazaspiro[4.5]decan-3-yl]-4*H-*pyrazino[2,3-b][1,4]oxazin-3-one | Intermediate 186 and Intermediate 55 |
| | 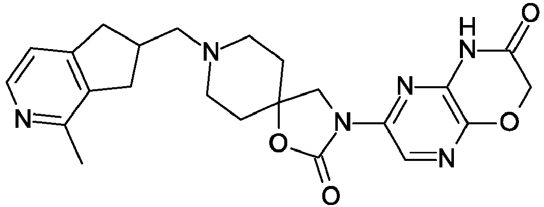 | |
| | ESP [M+H]⁺: 451.2 | |
| **375** | 6-[5-methyl-2-oxo-5-[2-[(*rac*-1,2-*trans*-1-amino-4-chloro-2,3-dihydro-1*H*-inden-2-yl)methylamino]ethyl]-1,3-oxazolidin-3-yl]-4*H*-pyrazino[2,3-b][1,4]oxazin-3-one | Intermediate 246 and Intermediate 92 |
| | 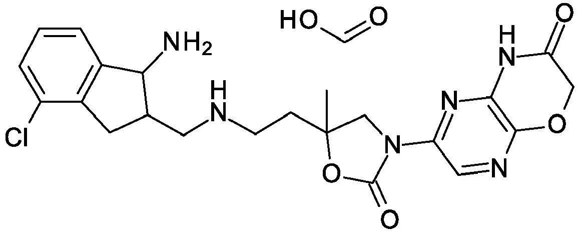 | |
| | ESP [M+H]⁺: 473.3 | |
| **431** | 6-[5-[2-[(1-chloro-4-fluoro-3-methyl-6,7-dihydro-5*H-*cyclopenta[c]pyridin-6-yl)methylamino]ethyl]-2-oxo-1,3-oxazolidin-3-yl]-4*H*-pyrazino[2,3-b][1,4]oxazin-3-one;formic acid | Intermediate 24 and Intermediate 265 |
| | 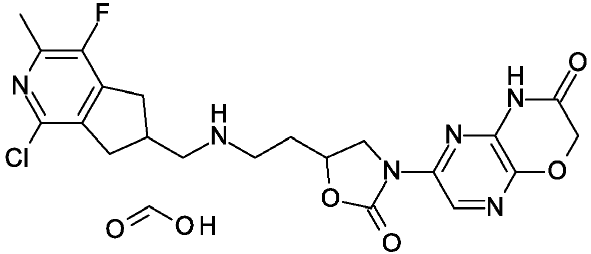 | |
| | ESP [M+H]⁺: 477.2 | |

### Example 86

### 6-[5-[2-[(1-Chloro-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one

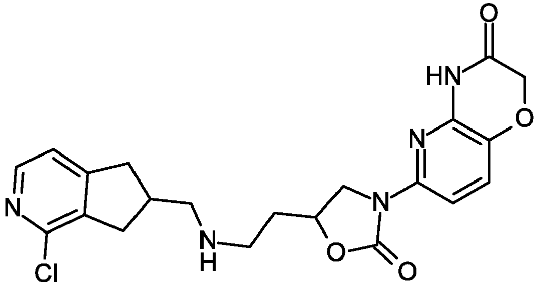

6-[5-(2-Aminoethyl)-2-oxo-1,3-oxazolidin-3-yl]-4*H*-pyrido[3,2-b] [1,4]oxazin-3-one (Intermediate 1, 30 mg, 108 µmol, 1 eq) was dissolved in THF (3.5 mL) and methanol (3.5 mL) with powdered mol sieves 3Å (30 mg). 1-Chloro-6,7-dihydro-5*H*-cyclopenta[c]pyridine-6-carbaldehyde (Intermediate 19, 20.6 mg, 113 µmol, 1.05 eq) and acetic acid (7.77 mg, 7.41 µL, 129 µmol, 1.2 eq) were added and the reaction mixture stirred at RT for 1 h. Sodium triacetoxyborohydride (27.4 mg, 129 µmol, 1.2 eq) was added and the mixture stirred for 2 h. Triethylamine (54.5 mg, 75.1 µL, 539 µmol, 5 eq) was added and the reaction was evaporated onto silica and purified by silica cartridge chromatography (ISCO 20g; 0% to 30% MeOH in DCM) to give the impure title compound which was further purified by prep-HPLC followed by lyophilisation to give the title compound (11.1 mg, 25 µmol, 23.2 % yield) as a white solid. MS (ESN): m/z = 442.3 [M-H]⁻.

The following examples were prepared by analogy to example 86.

| **Ex.** | **Name, Structure, MS** | **Starting Materials** |
|---|---|---|
| **87** | 6-[5-[2-(2,3-dihydro-1*H*-inden-2-ylmethylamino)ethyl]-2-oxo-1,3-oxazolidin-3-yl]-4*H*-pyrido[3,2-b][1,4]oxazin-3-one | Intermediate 1 and 2,3-dihydro-1*H-*indene-2-carbaldehyde [CAS# 37414-44-1] |
| | 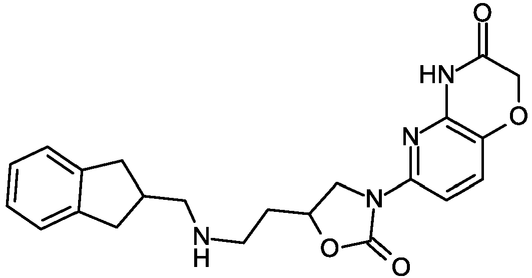 ESP [M+H]⁺: 409.2 | |
| **88** | 6-[5-[2-[(1-methyl-6,7-dihydro-5*H*-cyclopenta[c]pyridin-6-yl)methylamino]ethyl]-2-oxo-1,3-oxazolidin-3-yl]-4*H-*pyrido[3,2-b][1,4]oxazin-3-one | Intermediate 1 and Intermediate 55 |
| | 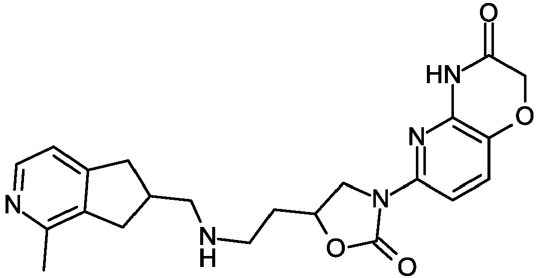 ESN [M-H]⁻: 422.3 | |
| **89** | 6-[5-[2-[(4-methyl-6,7-dihydro-5*H*-cyclopenta[b]pyridin-6-yl)methylamino]ethyl]-2-oxo-1,3-oxazolidin-3-yl]-4*H-*pyrido[3,2-b][1,4]oxazin-3-one | Intermediate 1 and Intermediate 56 |
| | 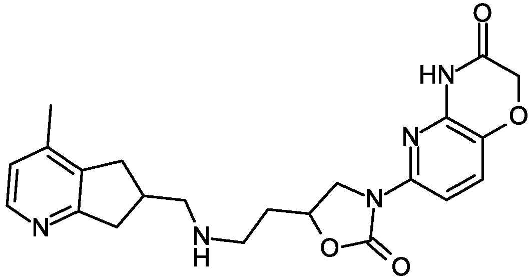 ESN [M-H]⁻: 422.3 | |
| **90** | 6-[[2-[2-oxo-3-(3-oxo-4*H*-pyrido[3,2-b] [1,4]oxazin-6-yl)-1,3-oxazolidin-5-yl]ethylamino]methyl]-6,7-dihydro-5*H-*cyclopenta[c]pyridine-1-carbonitrile; formic acid | Intermediate 1 and Intermediate 57 |
| | 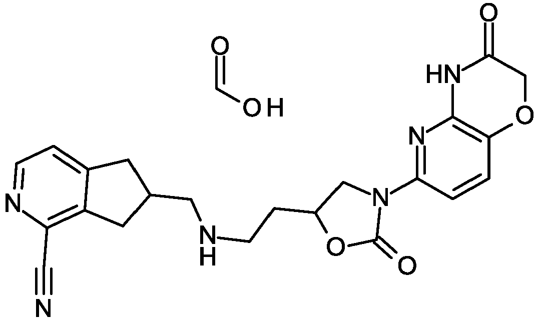 ESN [M-H]⁻: 433.3 | |
| **91** | 6-[5-[2-[(4-fluoro-2,3-dihydro-1*H*-inden-2-yl)methylamino]ethyl]-2-oxo-1,3-oxazolidin-3-yl]-4*H-*pyrido[3,2-b][1,4]oxazin-3-one | Intermediate 1 and 4-fluoro-2,3-dihydro-1H-indene-2-carbaldehyde [CAS# 1823964-81-3] |
| | 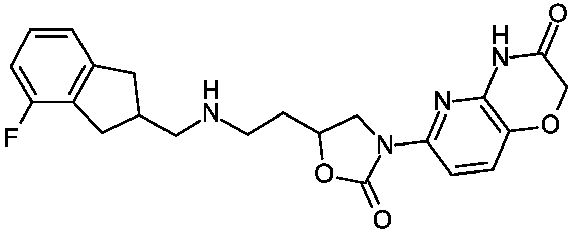 ESP [M+H]⁺: 427.3 | |
| **92** | 6-[5-[3-[(1-chloro-6,7-dihydro-5*H*-cyclopenta[c]pyridin-6-yl)amino]propyl]-2-oxo-1,3-oxazolidin-3-yl]-4*H*-pyrido[3,2-b][1,4]oxazin-3-one | Intermediate 58 and Intermediate 16 |
| | 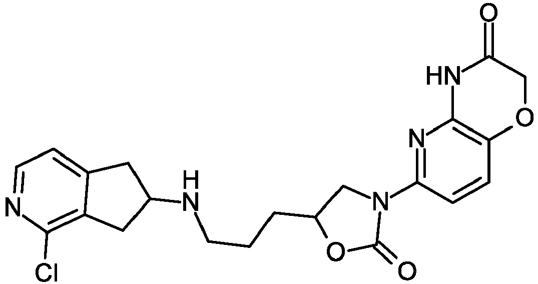 ESP [M+H]⁺: 444.2 | |
| **93** | 6-[2-oxo-5-[2-[(1,3,4-trimethyl-6,7-dihydro-5*H-*cyclopenta[c]pyridin-6-yl)methylamino]ethyl]-1,3-oxazolidin-3-yl]-4*H*-pyrido[3,2-b][1,4]oxazin-3-one | Intermediate 1 and Intermediate 59 |
| | 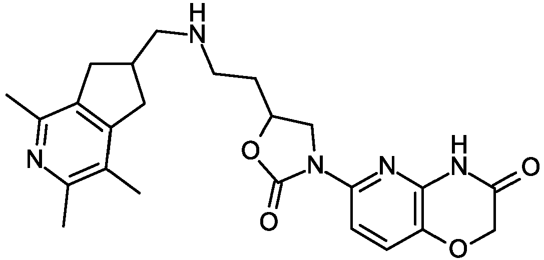 ESN [M-H]⁻: 450.4 | |
| **94** | 6-[5-[3-[(1-methyl-6,7-dihydro-5*H*-cyclopenta[c]pyridin-6-yl)amino]propyl]-2-oxo-1,3-oxazolidin-3-yl]-4*H*-pyrido[3,2-b][1,4]oxazin-3-one | Intermediate 60 and Intermediate 16 |
| | 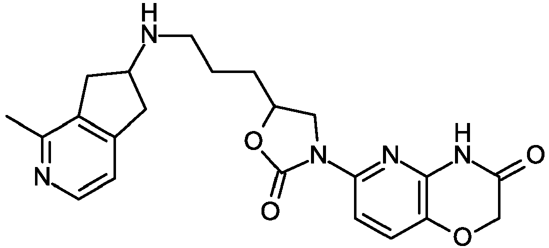 ESN [M-H]⁻: 422.4 | |
| **95** | 6-[3-[2-oxo-3-(3-oxo-4*H*-pyrido[3,2-b] [1,4]oxazin-6-yl)-1,3-oxazolidin-5-yl]propylamino]-6,7-dihydro-5*H-*cyclopenta[c]pyridine-1-carbonitrile | Intermediate 61 and Intermediate 16 |
| | 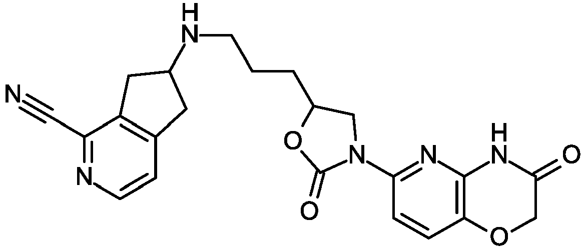 ESP [M+H]⁺: 435.3 | |
| **96** | 2-[[[*cis*-3-[2-oxo-3-(3-oxo-4*H*-pyrido[3,2-b][1,4]oxazin-6-yl)-1,3-oxazolidin-5-yl]cyclobutyl]amino]methyl]-2,3-dihydro-1*H-*indene-4-carbonitrile; formic acid | Intermediate 10 and Intermediate 5 |
| | 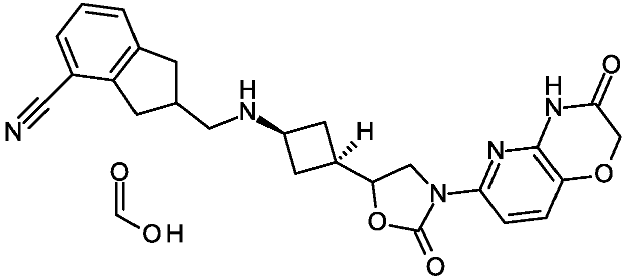 ESP [M+H]⁺: 460.2 | |
| **97** | 2-[[[*trans*-3-[2-oxo-3-(3-oxo-4*H*-pyrido[3,2-b][1,4]oxazin-6-yl)-1,3-oxazolidin-5-yl]cyclobutyl]amino]methyl]-2,3-dihydro-1*H*-indene-4-carbonitrile | Intermediate 7 and Intermediate 5 |
| | 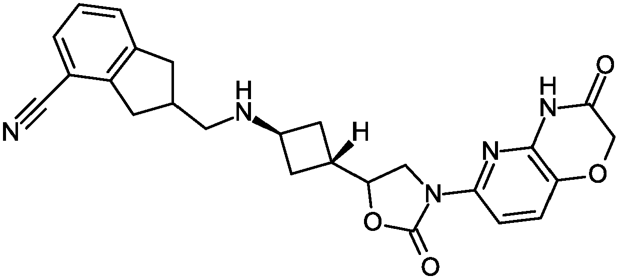 ESP [M+H]⁺: 460.3 | |
| **98** | 6-[5-[2-[(1,4-dimethyl-6,7-dihydro-5*H*-cyclopenta[c]pyridin-6-yl)methylamino]ethyl]-2-oxo-1,3-oxazolidin-3-yl]-4*H-*pyrido[3,2-b][1,4]oxazin-3-one | Intermediate 1 and Intermediate 62 |
| | 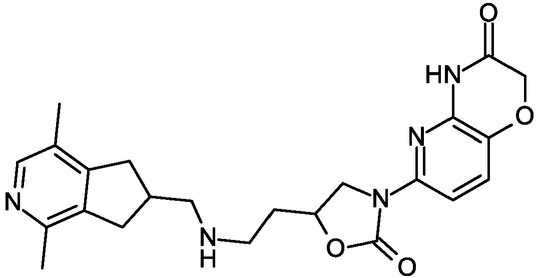 ESN [M-H]⁻: 463.3 | |
| **99** | 2-[[[*rac*-3a,7a-*cis*-6,7a-*trans*-2-oxo-3-(3-oxo-4*H*-pyrido[3,2-b][1,4]oxazin-6-yl)-3a,4,5,6,7,7a-hexahydro-1,3-benzoxazol-6-yl]amino]methyl]-2,3-dihydro-1*H*-indene-4-carbonitrile; formic acid | Intermediate 63 and Intermediate 5 |
| | 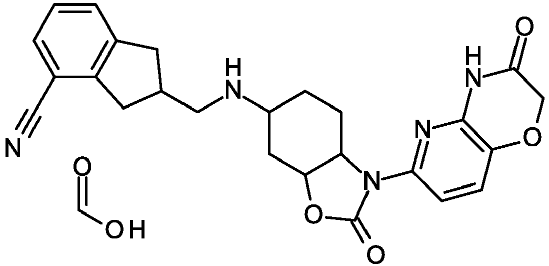 ESP [M+H]⁺: 460.1 | |
| **100** | 2-[[[*trans*-4-[2-oxo-3-(3-oxo-4*H*-pyrido[3,2-b][1,4]oxazin-6-yl)-1,3-oxazolidin-5-yl]cyclohexyl]amino]methyl]-2,3-dihydro-1*H*-indene-4-carbonitrile; formic acid | Intermediate 23 and Intermediate 5 |
| | 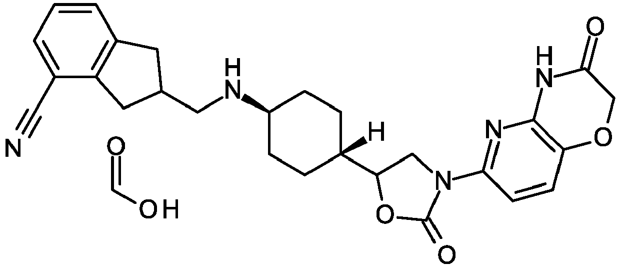 ESP [M+H]⁺: 488.1 | |
| **101** | 6-[5-[2-[[1,4-dimethyl-3-[(5-oxomorpholin-2-yl)methoxy]-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 64 |
| | 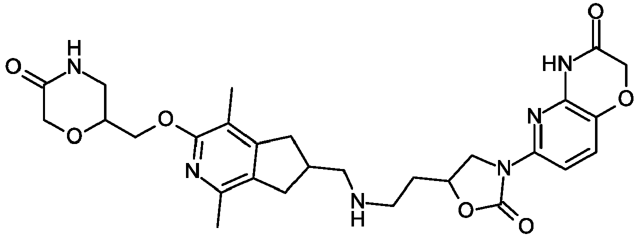 ESN [M-H]⁻: 565.5 | |
| **102** | 6-[5-[2-[[1,4-dimethyl-3-[(5-oxopyrrolidin-3-yl)methoxy]-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 65 |
| | 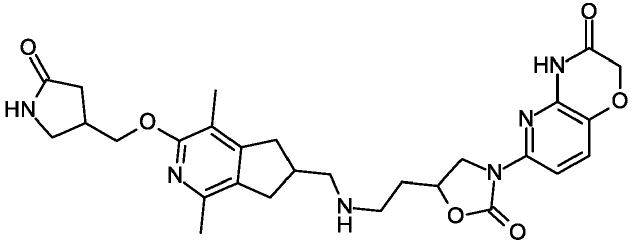 ESN [M-H]⁻: 549.6 | |
| **103** | 6-[5-[2-[[1,4-dimethyl-3-[(1-methyl-5-oxopyrrolidin-3-yl)methoxy]-6,7-dihydro-5*H*-cyclopenta[c]pyridin-6-yl]methylamino]ethyl]-2-oxo-1,3-oxazolidin-3-yl]-4*H-*pyrido[3,2-b][1,4]oxazin-3-one | Intermediate 1 and Intermediate 66 |
| | 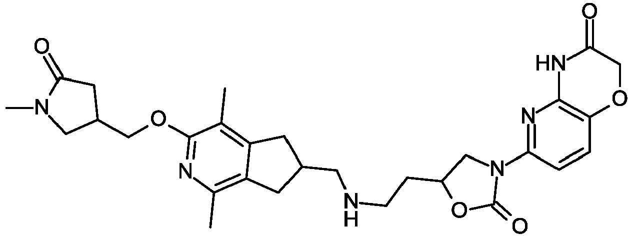 ESN [M-H]⁻: 563.6 | |
| **104** | 6-[[[2,4-*trans*-6-oxo-7-(3-oxo-4*H*-pyrido[3,2-b][1,4]oxazin-6-yl)-5-oxa-7-azaspiro[3.4]octan-2-yl]amino]methyl]-6,7-dihydro-5*H*-cyclopenta[c]pyridine-1-carbonitrile | Intermediate 67 and Intermediate 57 |
| | 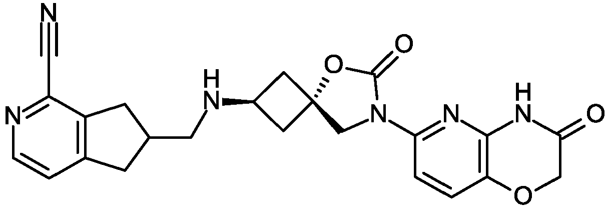 ESP [M+H]⁺: 447.3 | |
| **105** | 6-[5-[2-[[1,4-dimethyl-3-(3-methylsulfonylpropoxy)-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 68 |
| | 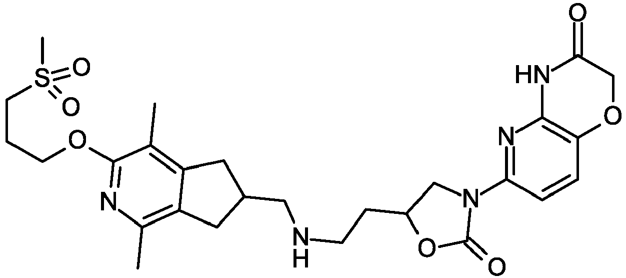 ESN [M-H]⁻: 572.5 | |
| **106** | 6-[5-[2-[(3-methoxy-1,4-dimethyl-6,7-dihydro-5*H-*cyclopenta[c]pyridin-6-yl)methylamino]ethyl]-2-oxo-1,3-oxazolidin-3-yl]-4*H*-pyrido[3,2-b][1,4]oxazin-3-one | Intermediate 1 and Intermediate 69 |
| | 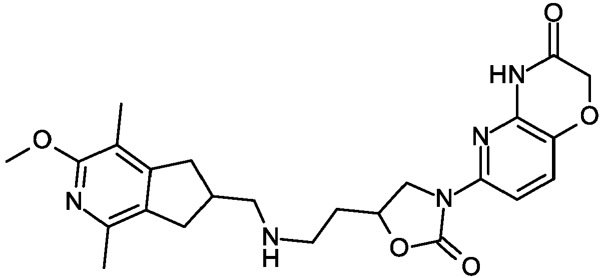 ESN [M-H]⁻: 465.5 | |
| **107** | 2-[[7-cyano-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide | Intermediate 1 and Intermediate 70 |
| | 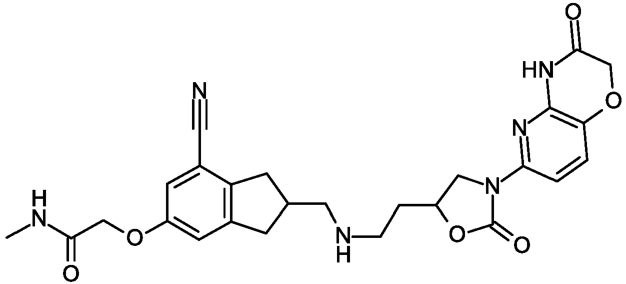 ESP [M+H]⁺: 521.4 | |
| **108** | 2-[[4-chloro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide | Intermediate 1 and Intermediate 71 |
| | 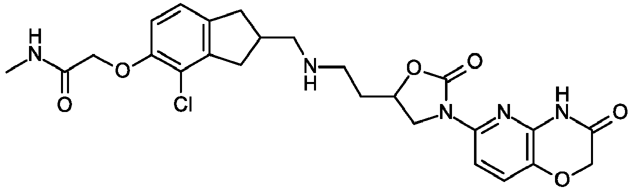 ESP [M+H]⁺: 530.3 | |
| **109** | 2 2-[[4-chloro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide | Intermediate 24 and Intermediate 71 |
| | 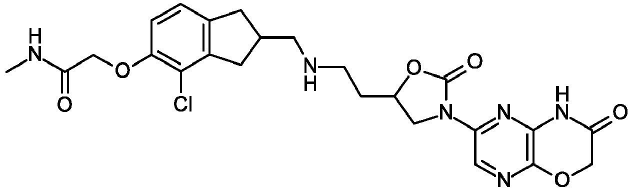 ESP [M+H]⁺: 531.3 | |
| **110** | 2-[[1,4-dimethyl-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H*-cyclopenta[c]pyridin-3-yl]oxy]-*N-*methylacetamide | Intermediate 1 and Intermediate 72 |
| | 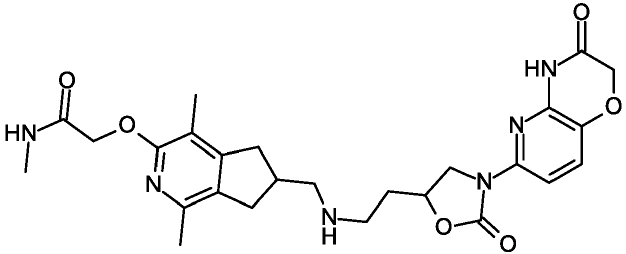 ESN [M-H]⁻: 523.4 | |
| **111** | 2-[[1,4-dimethyl-6-[[2-[2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridin-3-yl]oxy]-*N-*methylacetamide | Intermediate 24 and Intermediate 72 |
| | 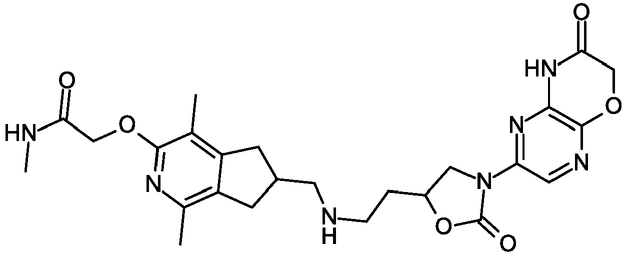 ESP [M+H]⁺: 526.3 | |
| **112** | 2 2-[[4,7-dimethyl-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide | Intermediate 1 and Intermediate 73 |
| | 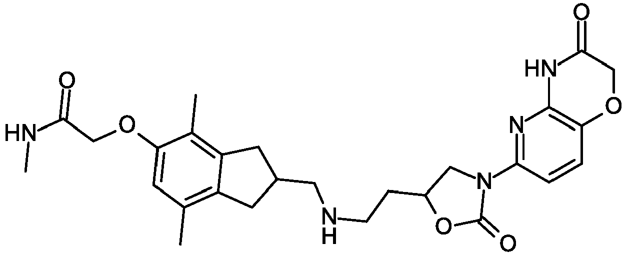 ESP [M+H]⁺: 524.3 | |
| **113** | 2-[[4,7-dimethyl-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide | Intermediate 24 and Intermediate 73 |
| | 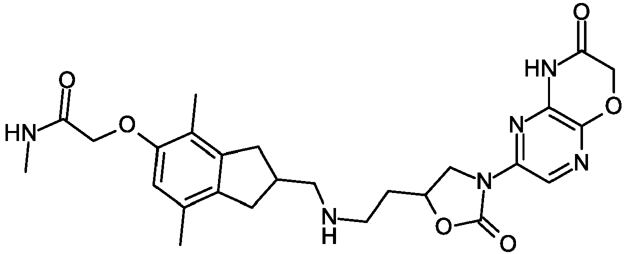 ESP [M+H]⁺: 525.3 | |
| **114** | 2-[[4,7-dimethyl-2-[[2-[2-oxo-3-(3-oxo-4*H*-pyrido[3,2-b][1,4]thiazin-6-yl)-1,3-oxazolidin-5-yl]ethylamino]methyl]-2,3-dihydro-1*H*-inden-5-yl]oxy]-*N*-methylacetamide | Intermediate 48 and Intermediate 73 |
| | 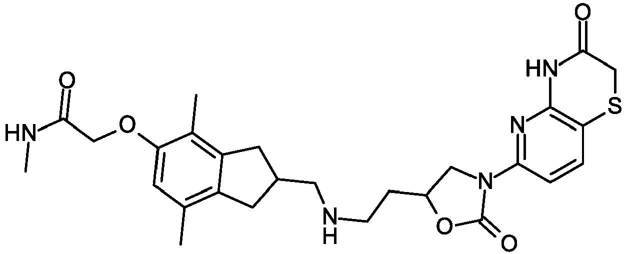 ESP [M+H]⁺: 540.3 | |
| **115** | *N*-[2-[[7-cyano-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]ethyl]methanesulfonamide | Intermediate 1 and Intermediate 181 |
| | 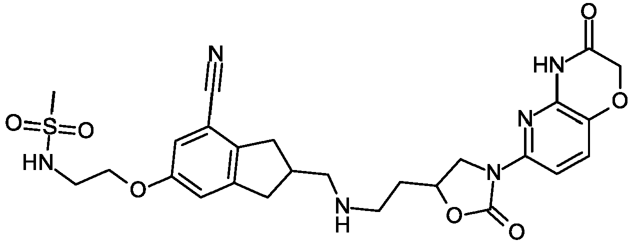 ESP [M+H]⁺: 571.3 | |
| **383** | 2-[[7-cyano-4-methyl-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide | Intermediate 24 and Intermediate 271 |
| | 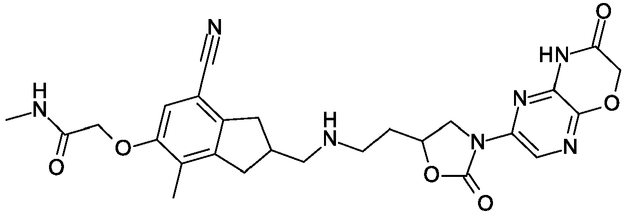 ESP [M+H]⁺: 536.3 | |
| **384** | 2-[[4-cyano-7-methyl-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide | Intermediate 24 and Intermediate 272 |
| | 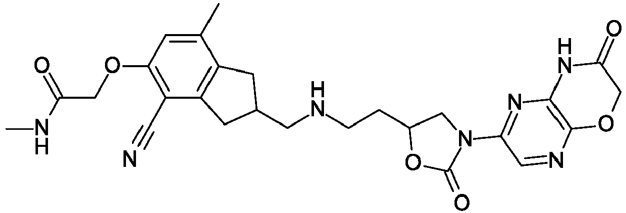 ESP [M+H]⁺: 536.3 | |
| **385** | 6-[5-[2-[(2-chloro-4-methyl-6,7-dihydro-5*H-*cyclopenta[d]pyrimidin-6-yl)methylamino]ethyl]-2-oxo-1,3-oxazolidin-3-yl]-4*H*-pyrido[3,2-b][1,4]oxazin-3-one | Intermediate 1 and Intermediate 273 |
| | 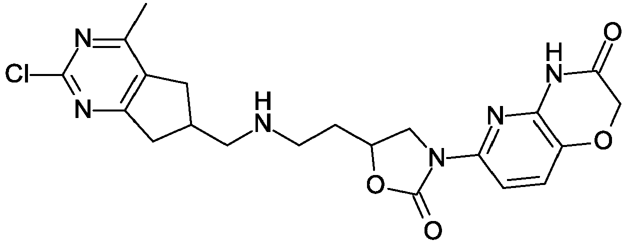 ESP [M+H]⁺: 459.2 | |
| **386** | 6-(1,3-oxazol-4-ylmethoxy)-2-[[2-[2-oxo-3-(3-oxo-4*H-*pyrazino[2,3-b][1,4]oxazin-6-yl)-1,3-oxazolidin-5-yl]ethylamino]methyl]-2,3-dihydro-1*H*-indene-4-carbonitrile | Intermediate 24 and Intermediate 274 |
| | 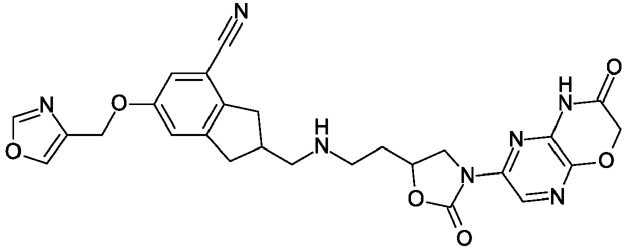 ESP [M+H]⁺: 532.3 | |
| **387** | 6-[(3-methyl-1,2,4-oxadiazol-5-yl)methoxy]-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-indene-4-carbonitrile | Intermediate 24 and Intermediate 275 |
| | 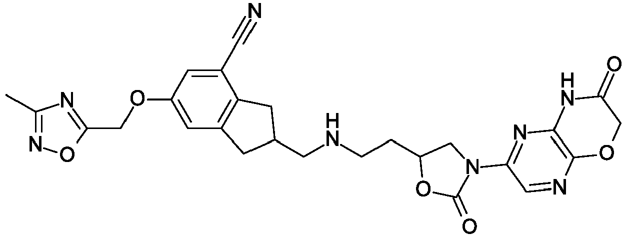 ESP [M+H]⁺: 547.3 | |
| **388** | 6-[(1-methylpyrazol-3-yl)methoxy]-2-[[2-[2-oxo-3-(3-oxo-4*H-*pyrazino[2,3-b][1,4]oxazin-6-yl)-1,3-oxazolidin-5-yl]ethylamino]methyl]-2,3-dihydro-1*H*-indene-4-carbonitrile | Intermediate 24 and Intermediate 276 |
| | 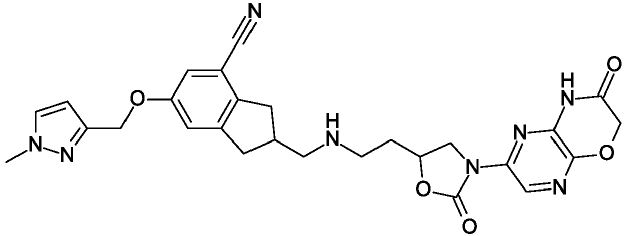 ESP [M+H]⁺: 545.4 | |
| **389** | 6-[(2-methyltetrazol-5-yl)methoxy]-2-[[2-[2-oxo-3-(3-oxo-4*H-*pyrazino[2,3-b][1,4]oxazin-6-yl)-1,3-oxazolidin-5-yl]ethylamino]methyl]-2,3-dihydro-1*H*-indene-4-carbonitrile | Intermediate 24 and Intermediate 277 |
| | 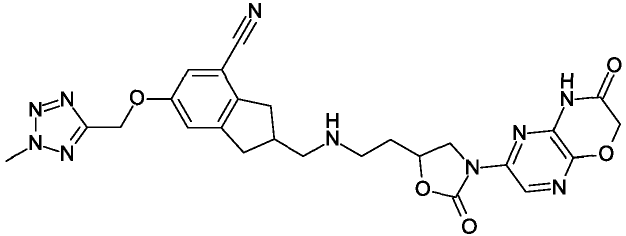 ESP [M+H]⁺: 547.4 | |
| **390** | 6-[(1-methylpyrazol-4-yl)methoxy]-2-[[2-[2-oxo-3-(3-oxo-4*H-*pyrazino[2,3-b][1,4]oxazin-6-yl)-1,3-oxazolidin-5-yl]ethylamino]methyl]-2,3-dihydro-1*H*-indene-4-carbonitrile | Intermediate 24 and Intermediate 278 |
| | 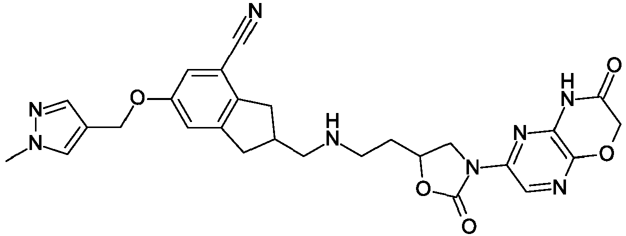 ESN [M-H]⁻: 543.3 | |
| **391** | 6-[(1-methyltetrazol-5-yl)methoxy]-2-[[2-[2-oxo-3-(3-oxo-4*H-*pyrazino[2,3-b][1,4]oxazin-6-yl)-1,3-oxazolidin-5-yl]ethylamino]methyl]-2,3-dihydro-1*H*-indene-4-carbonitrile | Intermediate 24 and Intermediate 279 |
| | 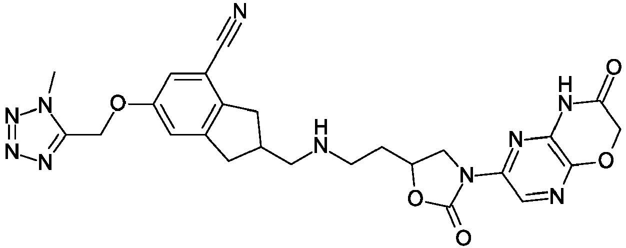 ESP [M+H]⁺: 547.4 | |
| **392** | 6-[(4-methyl-1,2,5-oxadiazol-3-yl)methoxy]-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-indene-4-carbonitrile | Intermediate 24 and Intermediate 280 |
| | 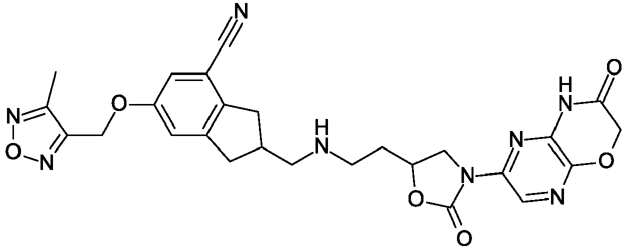 ESP [M+H]⁺: 547.3 | |
| **393** | 2-[[4,7-difluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide | Intermediate 24 and Intermediate 281 |
| | 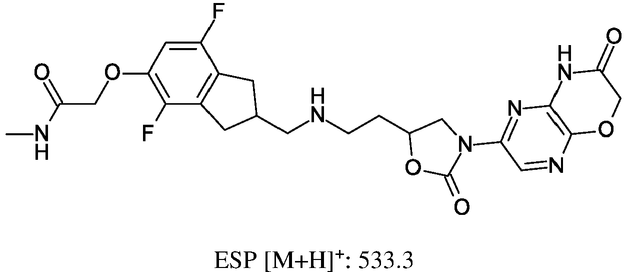 | |
| **394** | 2-[[4,7-difluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide | Intermediate 1 and Intermediate 281 |
| | 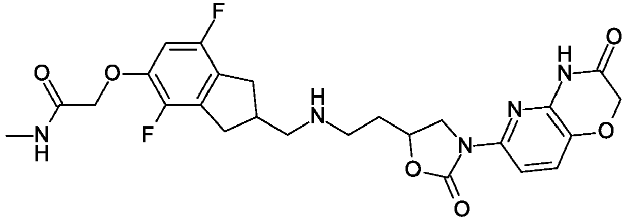 ESP [M+H]⁺: 532.3 | |
| **395** | 6-[5-[2-[[4,7-difluoro-5-(1*H*-pyrazol-3-ylmethoxy)-2,3-dihydro-1*H*-inden-2-yl] methylamino] ethyl]-2-oxo-1,3-oxazolidin-3-yl]-4*H*-pyrazino[2,3-b][1,4]oxazin-3-one | Intermediate 24 and Intermediate 282 |
| | 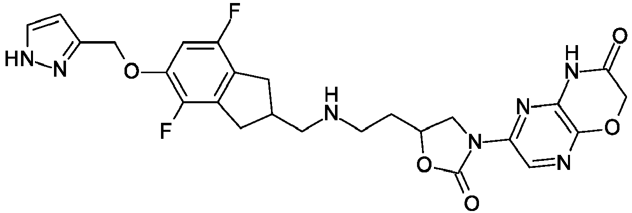 ESP [M+H]⁺: 542.3 | |
| **396** | 6-[8-[[4,7-difluoro-5-(1*H*-pyrazol-3-ylmethoxy)-2,3-dihydro-1*H*-inden-2-yl]methyl]-2-oxo-1-oxa-3,8-diazaspiro[4.5]decan-3-yl]-4*H*-pyrazino[2,3-b][1,4]oxazin-3-one | Intermediate 186 and Intermediate 282 |
| | 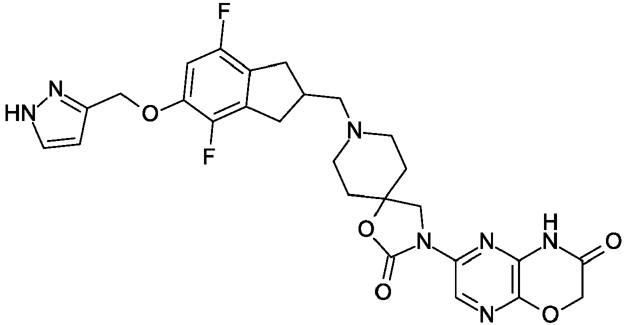 ESP [M+H]⁺: 568.3 | |
| **397** | 6-[5-[2-[(4-fluoro-3-methoxy-1-methyl-6,7-dihydro-5*H-*cyclopenta [c]pyridin-6-yl)methylamino] ethyl]-2-oxo-1,3-oxazolidin-3-yl]-4*H*-pyrazino[2,3-b][1,4]oxazin-3-one | Intermediate 24 and Intermediate 283 |
| | 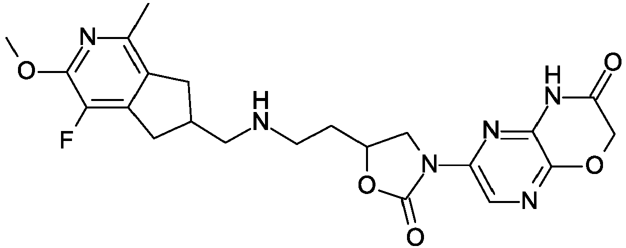 ESN [M-H]⁻: 471.3 | |
| **398** | 6-[5-[2-[(3-chloro-4-fluoro-1-methyl-6,7-dihydro-5*H-*cyclopenta[c]pyridin-6-yl)methylamino]ethyl]-2-oxo-1,3-oxazolidin-3-yl]-4*H*-pyrazino[2,3-b][1,4]oxazin-3-one | Intermediate 24 and Intermediate 284 |
| | 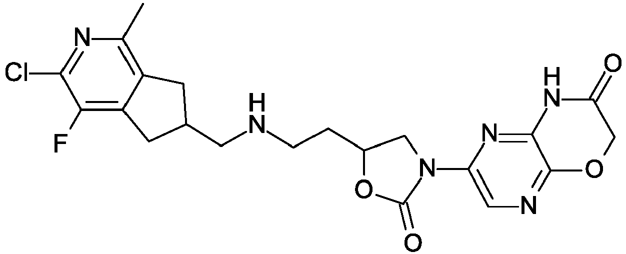 ESP [M+H]⁺: 477.2 | |
| **413** | 2-[[4-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide | Intermediate 24 and Intermediate 293 |
| | 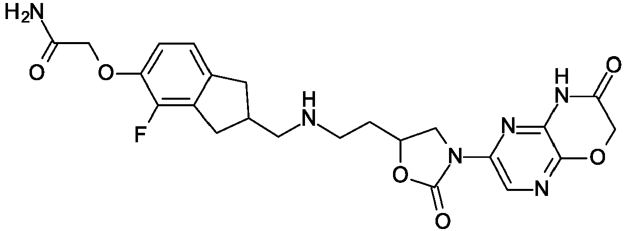 ESP [M+H]⁺: 501.0 | |
| **414** | 2-[[7-cyano-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy] acetamide | Intermediate 24 and Intermediate 294 |
| | 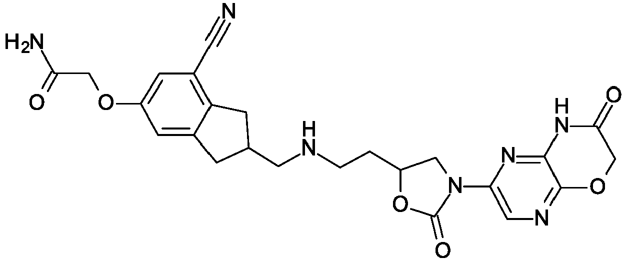 ESP [M+H]⁺: 508.1 | |

### Example 116

### 6-[5-[2-[[5-(2-Aminoethoxy)-4-chloro-2,3-dihydro-1H-inden-2-yl]methylamino]ethyl]-2-oxo-1,3-oxazolidin-3-yl]-4H-pyrido[3,2-b][1,4] oxazin-3 -one

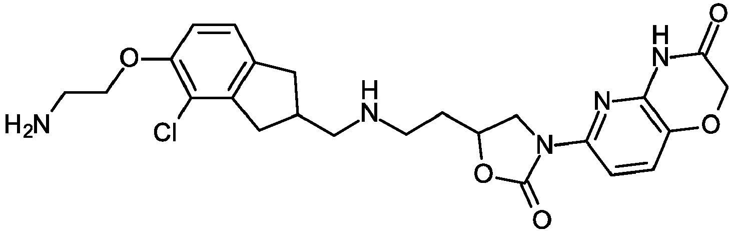

To a mixture of 6-[5-(2-aminoethyl)-2-oxo-1,3-oxazolidin-3-yl]-4*H*-pyrido[3,2-b][1,4]oxazin-3-one (Intermediate 1, 25.0 mg, 0.090 mmol, 1 eq) and *tert*-butyl N-[2-[(4-chloro-2-formyl-2,3-dihydro-1*H*-inden-5-yl)oxy]ethyl]carbamate (Intermediate 74, 52.3 mg, 0.110 mmol, 1.2 eq) in methanol (5 mL) was added acetic acid (5.39 mg, 0.090 mmol, 1 eq) and the mixture stirred at 20 °C for 2 h, then the mixture was cooled to 0°C and sodium cyanoborohydride (11.29 mg, 0.180 mmol, 2 eq) was added. The mixture was warmed to 20 °C and stirred for another 14 h. The mixture was filtered and the filtrate was purified by prep-HPLC (formic acid) and then freeze-drying to give *tert*-butyl *N*-[2-[[4-chloro-2-[[2-[2-oxo-3-(3-oxo-4*H*-pyrido[3,2-b] [1,4]oxazin-6-yl)-1,3-oxazolidin-5-yl]ethylamino]methyl]-2,3-dihydro-1*H*-inden-5-yl]oxy]ethyl]carbamate (50 mg) as a white solid. To a mixture of this material in DCM (3 mL) was added trifluoroacetic acid (0.2 mL, 2.6 mmol, 31.2 eq), then the reaction was stirred 20 °C for 2 h. The mixture was concentrated under vacuum to give the residue. The residue was purified by prep-HPLC (basic system) and then freeze-drying to give the title compound (3.1 mg, 0.010 mmol, 11 % yield) as a white solid. MS (ESP): m/z = 502.3 [M+H]⁺.

The following examples were prepared by analogy to Example 116.

| **Ex.** | **Name, Structure, MS** | **Starting Materials** |
|---|---|---|
| **117** | 6-[5-[2-[[5-[(1-aminocyclopropyl)methoxy]-4-chloro-2,3-dihydro-1*H*-inden-2-yl]methylamino]ethyl]-2-oxo-1,3-oxazolidin-3-yl]-4*H*-pyrido[3,2-b][1,4]oxazin-3-one; formic acid | Intermediate 1 and Intermediate 75 |
| | 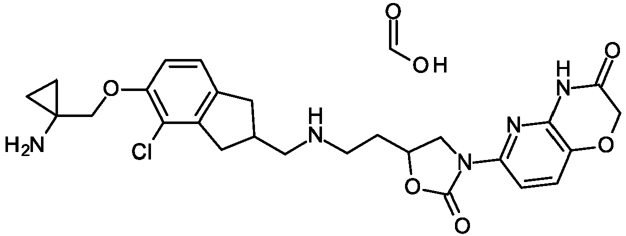 ESP [M+H]+: 528.1 | |
| **118** | 6-[5-[3-[[5-(2-aminoethoxy)-4-chloro-2,3-dihydro-1H-inden-2-yl]amino]propyl]-2-oxo-1,3-oxazolidin-3-yl]-4H-pyrido[3,2-b][1,4]oxazin-3-one; formic acid | Intermediate 16 and Intermediate 76 |
| | 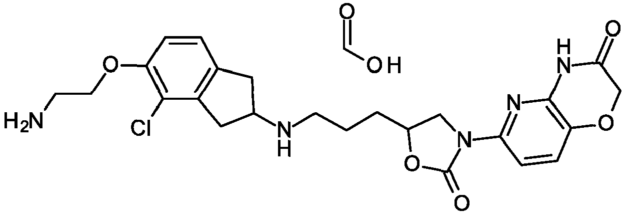 ESP [M+H]⁺: 502.1 | |
| **119** | 6-[5-[3-[[5-[(1-aminocyclopropyl)methoxy]-4-chloro-2,3-dihydro-1*H*-inden-2-yl]amino]propyl]-2-oxo-1,3-oxazolidin-3-yl]-4*H*-pyrido[3,2-b][1,4]oxazin-3-one; formic acid | Intermediate 16 and Intermediate 77 |
| | 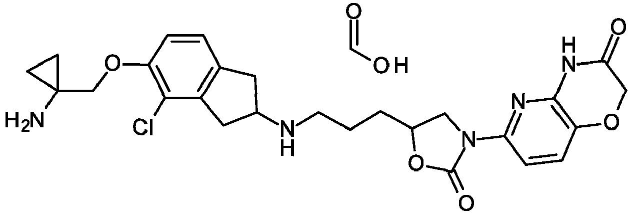 ESP [M+H]⁺: 528.3 | |
| **120** | 6-[4-[2-[(4-fluoro-2,3-dihydro-1*H*-inden-2-yl)amino]ethylamino]-2-oxopyrrolidin-1-yl]-4*H*-pyrido[3,2-b][1,4]oxazin-3-one; formic acid | Intermediate 78 and 4-fluoro-1,3-dihydroinden-2-one [CAS# 1210824-58-0] |
| | 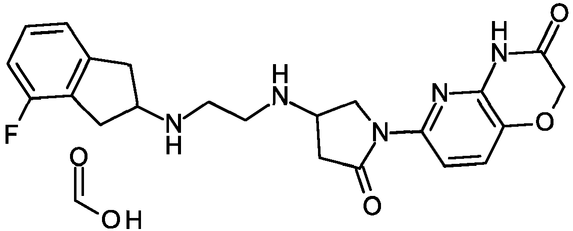 ESN [M-H]⁻: 424.4 | |
| **121** | 2-[2-[[5-oxo-1-(3-oxo-4*H*-pyrido[3,2-b] [1,4]oxazin-6-yl)pyrrolidin-3-yl]amino]ethylamino]-2,3-dihydro-1*H*-indene-4-carbonitrile | Intermediate 78 and Intermediate 6 |
| | 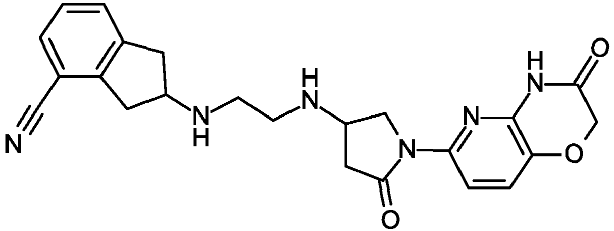 ESN [M-H]⁻: 431.5 | |
| **122** | 6-[5-[2-[[3-[(1-aminocyclopropyl)methoxy]-1-chloro-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one; formic acid | Intermediate 1 and Intermediate 79 |
| | 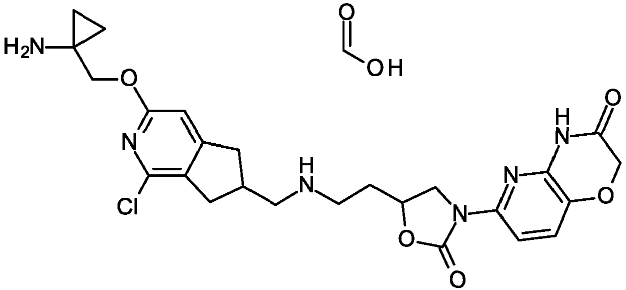 ESP [M+H]⁺: 529.3 | |
| **123** | 6-[2-oxo-5-[2-[[5,6-*trans*-5-amino-1-chloro-6,7-dihydro-5*H-*cyclopenta[c]pyridin-6-yl]methylamino]ethyl]-1,3-oxazolidin-3-yl]-4*H*-pyrido[3,2-b][1,4]oxazin-3-one; formic acid | Intermediate 1 and Intermediate 80 |
| | 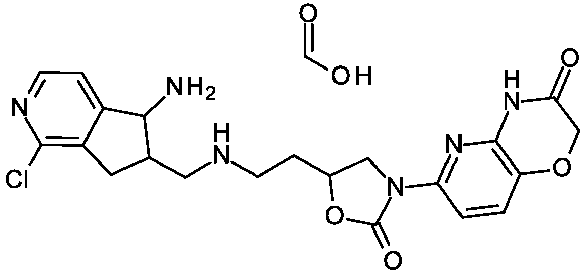 ESP [M+H]⁺: 459.1 | |
| **124** | 5-[(1-aminocyclopropyl)methoxy]-2-[[2-[2-oxo-3-(3-oxo-4*H-*pyrido[3,2-b][1,4]oxazin-6-yl)-1,3-oxazolidin-5-yl]ethylamino]methyl]-2,3-dihydro-1*H*-indene-4-carbonitrile hydrochloride | Intermediate 1 and Intermediate 81 |
| | 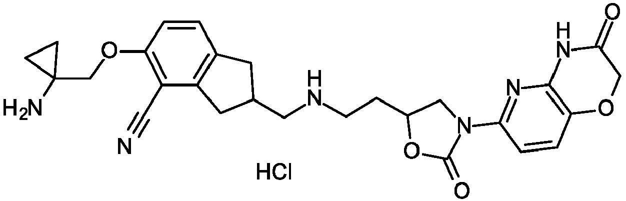 ESP [M+H]⁺: 519.2 | |
| **125** | 3-[(1-aminocyclopropyl)methoxy]-6-[[2-[2-oxo-3-(3-oxo-4*H-*pyrido[3,2-b] [1,4]oxazin-6-yl)-1,3-oxazolidin-5-yl]ethylamino]methyl]-6,7-dihydro-5*H*-cyclopenta[c]pyridine-1-carbonitrile | Intermediate 1 and Intermediate 82 |
| | 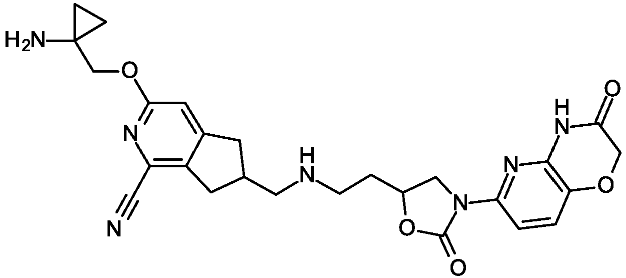 ESP [M+H]⁺: 520.3 | |
| **126** | 6-[5-[2-[[3-(2-aminoethoxy)-1-chloro-6,7-dihydro-5*H-*cyclopenta [c]pyridin-6-yl] methylamino] ethyl]-2-oxo-1,3-oxazolidin-3-yl]-4*H*-pyrido[3,2-b][1,4]oxazin-3-one | Intermediate 1 and Intermediate 83 |
| | 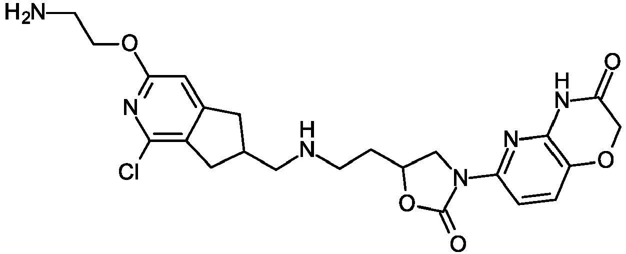 ESP [M+H]⁺: 503.2 | |
| **127** | 3-(2-aminoethoxy)-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H*-cyclopenta[c]pyridine-1-carbonitrile; formic acid | Intermediate 1 and Intermediate 84 |
| | 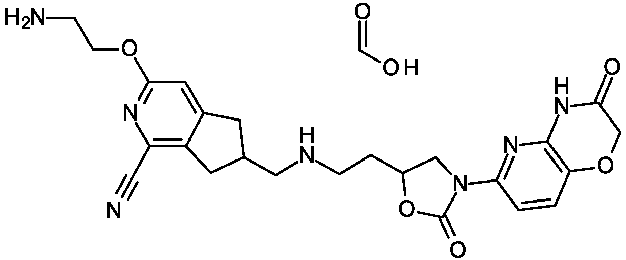 ESP [M+H]⁺: 494.1 | |
| **128** | 5-(2-aminoethoxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile; formic acid | Intermediate 1 and Intermediate 85 |
| | 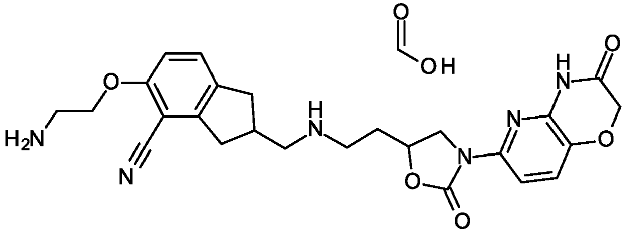 ESP [M+H]⁺: 493.2 | |
| **129** | 5-(azetidin-3-ylmethoxy)-2-[[2-[2-oxo-3-(3-oxo-4*H-*pyrido[3,2-b][1,4]oxazin-6-yl)-1,3-oxazolidin-5-yl]ethylamino]methyl]-2,3-dihydro-1*H*-indene-4-carbonitrile; formic acid | Intermediate 1 and Intermediate 86 |
| | 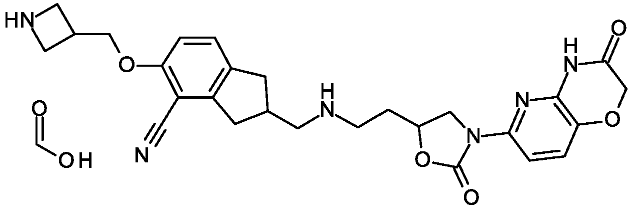 ESP [M+H]⁺: 519.2 | |
| **130** | 6-[2-oxo-5-[2-[(*rac*-5,6-*trans*-5-amino-1-methyl-6,7-dihydro-5*H*-cyclopenta[c]pyridin-6-yl)methylamino]ethyl]-1,3-oxazolidin-3-yl]-4*H*-pyrido[3,2-b][1,4]oxazin-3-one; formic acid | Intermediate 1 and Intermediate 87 |
| | 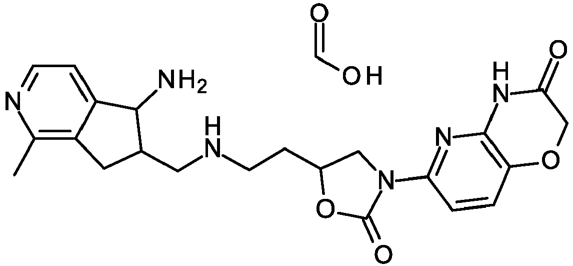 ESP [M+H]⁺: 439.1 | |
| **131** | *rac*-1,2-*trans*-1-amino-2-[[2-oxo-3-(3-oxo-4*H*-pyrido[3,2-b][1,4]oxazin-6-yl)-1-oxa-3,8-diazaspiro[4.5]decan-8-yl]methyl]-2,3-dihydro-1*H*-indene-4-carbonitrile; formic acid | Intermediate 17 and Intermediate 88 |
| | 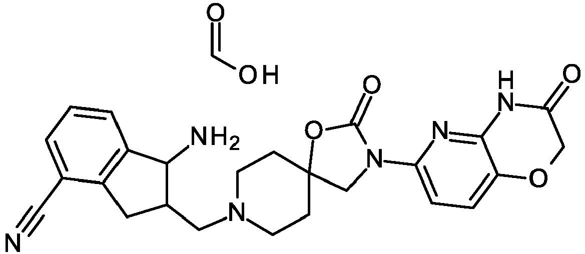 ESP [M+H]⁺: 475.2 | |
| **132** | 5-(azetidin-3-yloxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile; formic acid | Intermediate 1 and Intermediate 89 |
| | 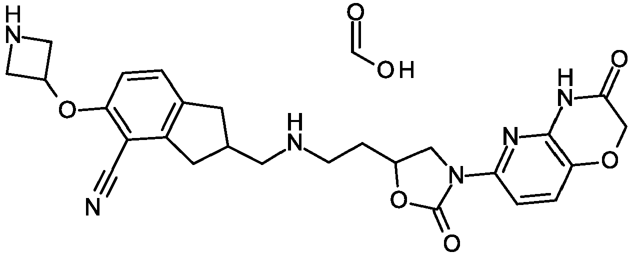 ESP [M+H]⁺: 505.2 | |
| **133** | *rac*-5,6-*trans*-5-amino-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H*-cyclopenta[c]pyridine-1-carbonitrile; formic acid | Intermediate 1 and Intermediate 90 |
| | 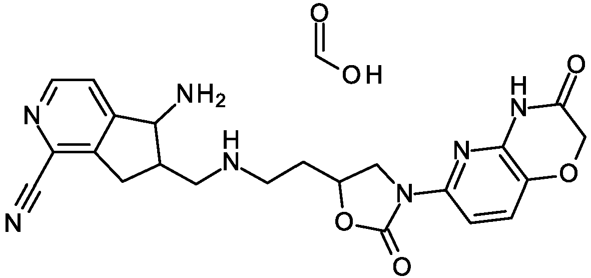 ESP [M+H]⁺: 450.1 | |
| **134** | 5-(2-aminopropoxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile; formic acid | Intermediate 1 and Intermediate 91 |
| | 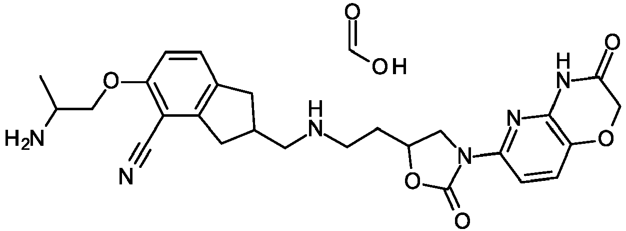 ESP [M+H]⁺: 507.2 | |
| **135** | 7-[2-oxo-5-[2-[[*rac*-1,2-*trans*-1-amino-4-chloro-2,3-dihydro-1*H*-inden-2-yl]methylamino]ethyl]-1,3-oxazolidin-3-yl]-1*H-*pyrido[2,3-b][1,4]oxazin-2-one; formic acid | Intermediate 34 and Intermediate 92 |
| | 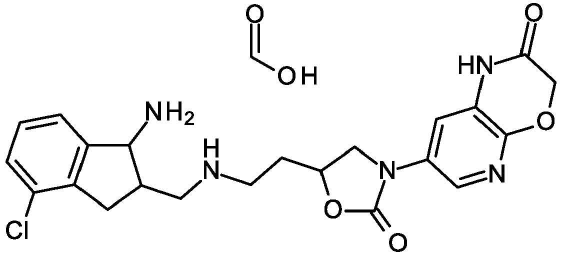 ESP [M+H]⁺: 458.1 | |
| **136** | 6-[2-oxo-5-[2-[[*rac*-1,2-*trans*-1-amino-4-chloro-2,3-dihydro-1*H*-inden-2-yl]methylamino] ethyl]-1,3-oxazolidin-3-yl]-4*H-*pyrazino[2,3-b][1,4]oxazin-3-one; formic acid | Intermediate 24 and Intermediate 92 |
| | 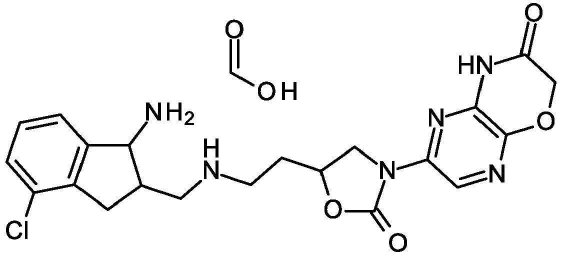 ESP [M+H]⁺: 459.1 | |
| **137** | 5-(azetidin-2-ylmethoxy)-2-[[2-[2-oxo-3-(3-oxo-4*H-*pyrido[3,2-b][1,4]oxazin-6-yl)-1,3-oxazolidin-5-yl]ethylamino]methyl]-2,3-dihydro-1*H*-indene-4-carbonitrile; formic acid | Intermediate 1 and Intermediate 93 |
| | 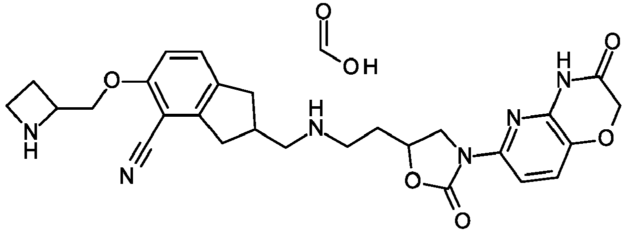 ESP [M+H]⁺: 519.1 | |
| **138** | 2-[[2-[3-amino-5-oxo-1-(3-oxo-4*H*-pyrido[3,2-b][1,4]oxazin-6-yl)pyrrolidin-3-yl]ethylamino]methyl]-2,3-dihydro-1*H-*indene-4-carbonitrile | Intermediate 94 and Intermediate 5 |
| | 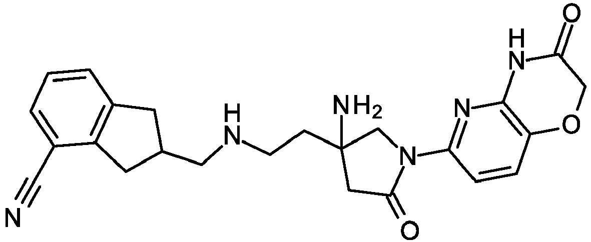 ESP [M+H]⁺: 447.2 | |
| **139** | *rac*-1,2-*trans*-1-hydroxy-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile | Intermediate 1 and Intermediate 95 |
| | 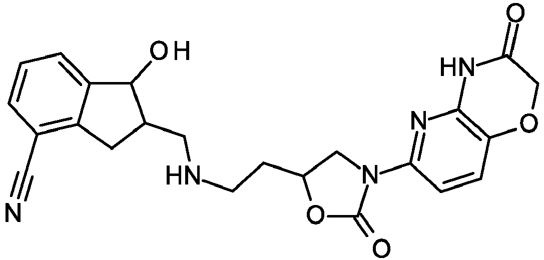 ESP [M+H]⁺: 450.4 | |
| **140** | 6-[4-amino-4-[2-[(4-chloro-2,3-dihydro-1*H*-inden-2-yl)methylamino] ethyl]-2-oxopyrrolidin-1-yl]-4*H*-pyrido[3,2-b][1,4]oxazin-3-one | Intermediate 94 and 4-chloro-2,3-dihydro- 1H-indene-2-carbaldehyde [CAS# 1823964-97-1] |
| | 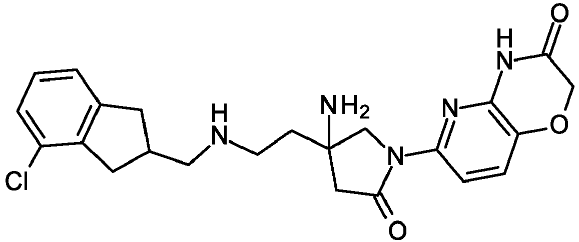 ESP [M+H]⁺: 456.1 | |
| **141** | 2-[[2-[2-oxo-3-(3-oxo-4*H*-pyrido[3,2-b] [1,4]oxazin-6-yl)-1,3-oxazolidin-5-yl]ethylamino]methyl]-5-(pyrrolidin-2-ylmethoxy)-2,3-dihydro-1*H*-indene-4-carbonitrile; formic acid | Intermediate 1 and Intermediate 96 |
| | 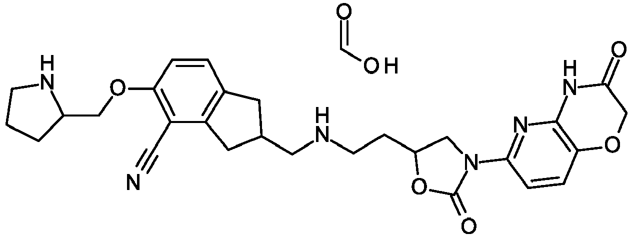 ESP [M+H]⁺: 533.3 | |
| **142** | 6-[5-[[2-(2-amino-4-chloro-1,3-dihydroinden-2-yl)ethylamino]methyl]-2-oxo-1,3-oxazolidin-3-yl]-4*H-*pyrido[3,2-b][1,4]oxazin-3-one; formic acid | Intermediate 12 and Intermediate 97 |
| | 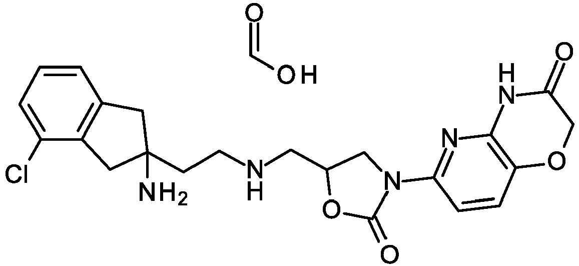 ESP [M+H]⁺: 458.2 | |
| **143** | 2-[[2-[2-oxo-3-(3-oxo-4*H*-pyrido[3,2-b][1,4]oxazin-6-yl)-1,3-oxazolidin-5-yl]ethylamino]methyl]-5-(2-piperazin-1-ylethoxy)-2,3-dihydro-1*H*-indene-4-carbonitrile; formic acid | Intermediate 1 and Intermediate 98 |
| | 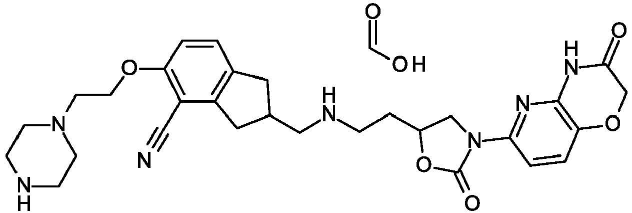 ESP [M+H]⁺: 562.2 | |
| **144** | 6-[5-[2-[[3-(2-aminopropoxy)-1-chloro-6,7-dihydro-5*H-*cyclopenta[c]pyridin-6-yl]methylamino]ethyl]-2-oxo-1,3-oxazolidin-3-yl]-4*H*-pyrido[3,2-b][1,4]oxazin-3-one; formic acid | Intermediate 1 and Intermediate 99 |
| | 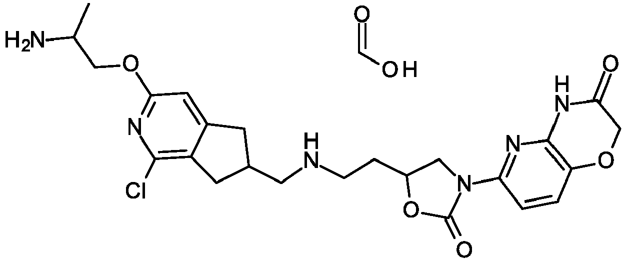 ESP [M+H]⁺: 517.2 | |
| **145** | 6-[5-[2-[[3-(2-aminopropoxy)-1-chloro-6,7-dihydro-5*H-*cyclopenta[c]pyridin-6-yl]methylamino]ethyl]-2-oxo-oxazolidin-3-yl]-4*H*-pyrazino[2,3-b][1,4]oxazin-3-one; formic acid | Intermediate 24 and Intermediate 99 |
| | 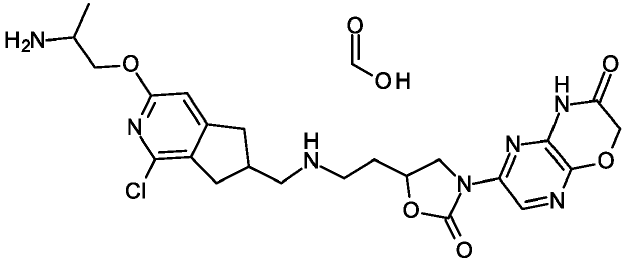 ESP [M+H]⁺: 518.2 | |
| **146** | 6-[2-oxo-5-[2-[[*rac*-5,6-*cis*-5-amino-1-chloro-6,7-dihydro-5*H-*cyclopenta[c]pyridin-6-yl]methylamino]ethyl]-1,3-oxazolidin-3-yl]-4*H*-pyrido[3,2-b][1,4]oxazin-3-one; formic acid | Intermediate 1 and Intermediate 100 |
| | 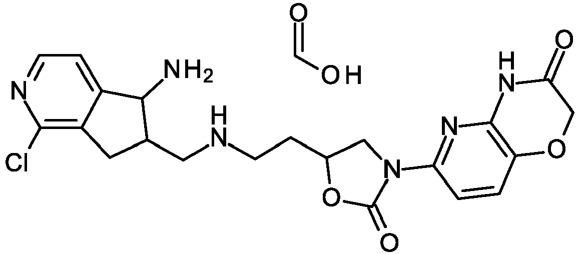 ESP [M+H]⁺: 459.2 | |
| **147** | 6-[2-oxo-5-[2-[[*rac*-1,2-*trans*-1-amino-4-chloro-2,3-dihydro-1*H*-inden-2-yl]methylamino]ethyl]-1,3,4-oxadiazol-3-yl]-4*H-*1,4-benzoxazin-3-one; formic acid | Intermediate 31 and Intermediate 92 |
| | 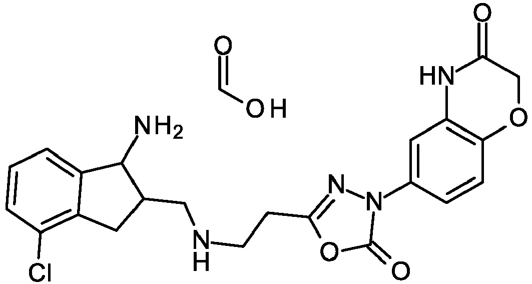 ESP [M+H]⁺: 456.2 | |
| **148** | 2-[[2-[*rac*-4,5-*cis*-4-(aminomethyl)-2-oxo-3-(3-oxo-4*H-*pyrido[3,2-b] [1,4]oxazin-6-yl)-1,3-oxazolidin-5-yl]ethylamino]methyl]-2,3-dihydro-1*H*-indene-4-carbonitrile; formic acid | Intermediate 101 and Intermediate 5 |
| | 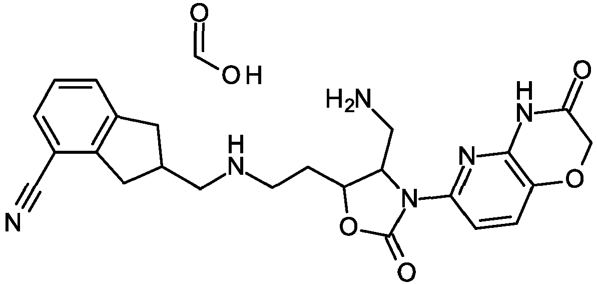 ESP [M+H]⁺: 463.4 | |
| **149** | 2-[[2-[*rac*-4,5-*trans*-4-(aminomethyl)-2-oxo-3-(3-oxo-4*H-*pyrido[3,2-b] [1,4]oxazin-6-yl)-1,3-oxazolidin-5-yl]ethylamino]methyl]-2,3-dihydro-1*H*-indene-4-carbonitrile; formic acid | Intermediate 102 and Intermediate 5 |
| | 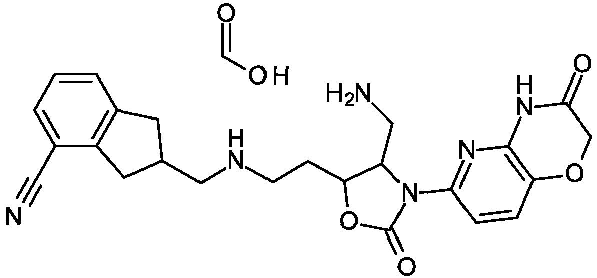 ESP [M+H]⁺: 463.2 | |
| **150** | 6-[5-[2-[[6-(azetidin-3-yloxy)-4-fluoro-2,3-dihydro-1*H*-inden-2-yl]methylamino]ethyl]-2-oxo-1,3-oxazolidin-3-yl]-4H-pyrido[3,2-b][1,4]oxazin-3-one | Intermediate 1 and Intermediate 103 |
| | 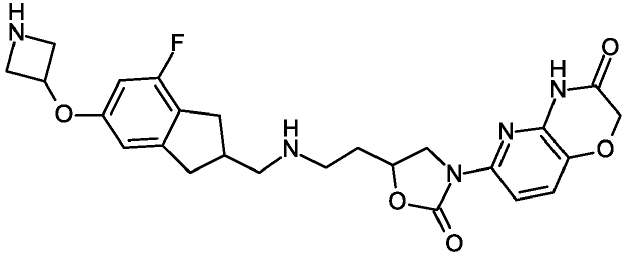 ESP [M+H]⁺: 498.2 | |
| **151** | *rac*-2,3-*trans*-3-hydroxy-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile; formic acid | Intermediate 1 and Intermediate 104 |
| | 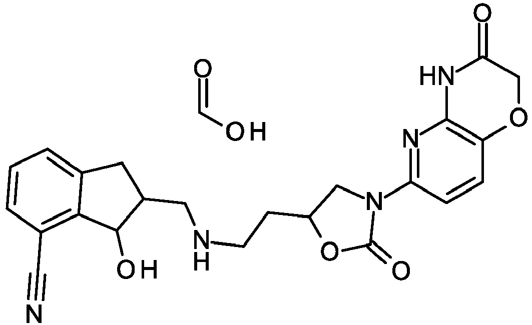 ESP [M+H]⁺: 450.2 | |
| **152** | *rac*-2,3-*trans*-3-hydroxy-2-[[2-[2-oxo-3-(3-oxo-4*H*-pyrido[3,2-b][1,4]thiazin-6-yl)-1,3-oxazolidin-5-yl]ethylamino]methyl]-2,3-dihydro-1*H*-indene-4-carbonitrile; formic acid | Intermediate 48 and Intermediate 104 |
| | 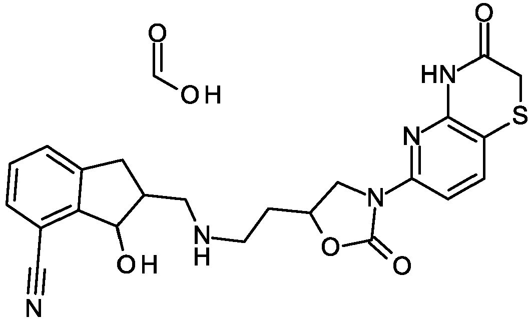 ESP [M+H]⁺: 466.1 | |
| **153** | 6-[5-[2-[(*rac*-1,2-*trans*-1-amino-4-chloro-2,3-dihydro-1*H-*inden-2-yl)methylamino]ethyl]-2-oxo-1,3,4-oxadiazol-3-yl]-4*H*-pyrido[3,2-b][1,4]oxazin-3 -one | Intermediate 33 and Intermediate 92 |
| | 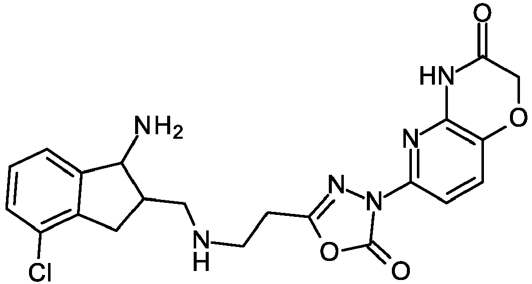 ESP [M+H]⁺: 457.1 | |
| **154** | 6-[5-[2-[[6-(2-aminopropoxy)-4-fluoro-2,3-dihydro-1*H*-inden-2-yl] methylamino]ethyl]-2-oxo-1,3-oxazolidin-3-yl]-4*H-*pyrido[3,2-b][1,4]oxazin-3-one | Intermediate 1 and Intermediate 105 |
| | 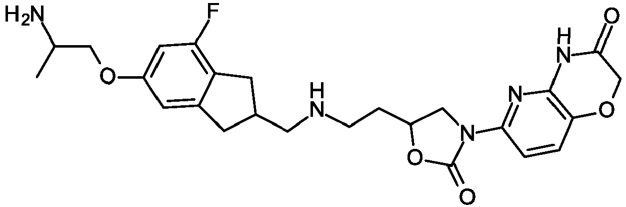 ESP [M+H]⁺: 500.3 | |
| **155** | 6-[5-[2-[[6-(1-aminopropan-2-yloxy)-4-fluoro-2,3-dihydro-1*H-*inden-2-yl]methylamino]ethyl]-2-oxo-1,3-oxazolidin-3-yl]-4H-pyrido[3,2-b][1,4]oxazin-3-one; formic acid | Intermediate 1 and Intermediate 106 |
| | 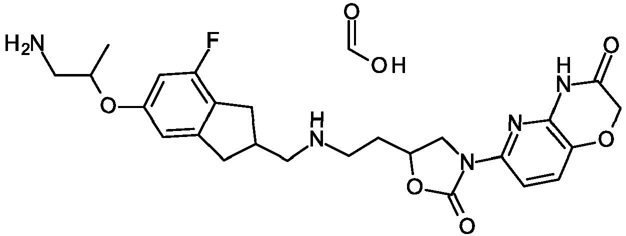 ESP [M+H]⁺: 500.3 | |
| **156** | *rac*-2,3-*trans*-3-amino-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile | Intermediate 1 and Intermediate 107 |
| | 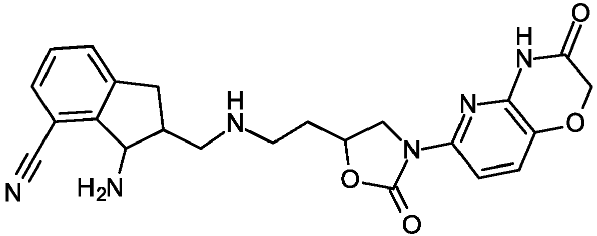 ESP [M+H]⁺: 449.4 | |
| **157** | *rac*-2,3-*trans*-3-amino-2-[[2-[2-oxo-3-(3-oxo-4*H*-pyrido[3,2-b][1,4]thiazin-6-yl)-1,3-oxazolidin-5-yl]ethylamino]methyl]-2,3-dihydro-1*H*-indene-4-carbonitrile | Intermediate 48 and Intermediate 107 |
| | 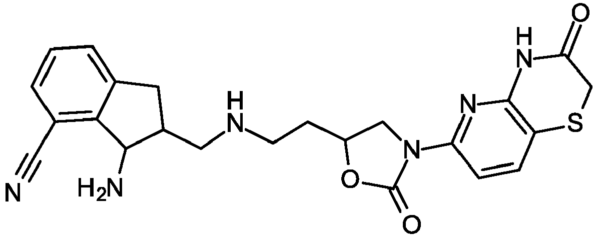 ESP [M+H]⁺: 465.2 | |
| **158** | 6-[5-[[[2-amino-2-(4-chloro-2,3-dihydro-1*H*-inden-2-yl)ethyl] amino] methyl]-2-oxo-1,3-oxazolidin-3-yl]-4*H-*pyrido[3,2-b][1,4]oxazin-3-one; formic acid | Intermediate 12 and Intermediate 108 |
| | 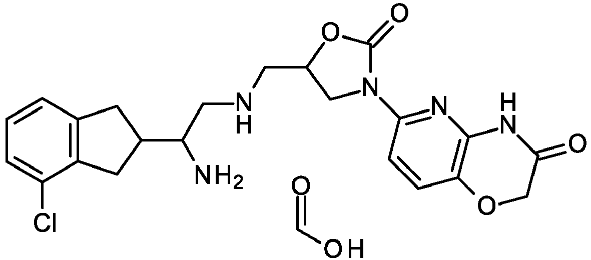 ESP [M+H]⁺: 458.2 | |
| **159** | 6-[2-[2-amino-2-(4-chloro-2,3-dihydro-1*H*-inden-2-yl)ethyl]-6-oxo-5-oxa-2,7-diazaspiro[3.4]octan-7-yl]-4*H*-pyrido[3,2-b][1,4]oxazin-3-one; formic acid | Intermediate 21 and Intermediate 108 |
| | 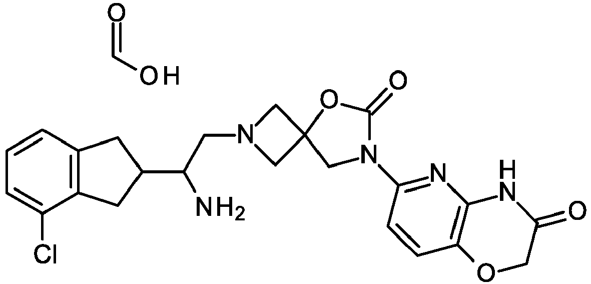 ESP [M+H]⁺: 470.2 | |
| **160** | 6-[5-[2-[[6-[(1-aminocyclopropyl)methoxy]-4-fluoro-2,3-dihydro-1*H*-inden-2-yl]methylamino]ethyl]-2-oxo-1,3-oxazolidin-3-yl]-4*H*-pyrido[3,2-b][1,4]oxazin-3-one | Intermediate 1 and Intermediate 109 |
| | 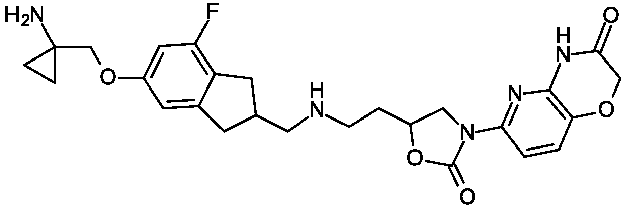 ESP [M+H]⁺: 512.4 | |
| **161** | 6-[5-[2-[[6-[(1-aminocyclopropyl)methoxy]-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one | Intermediate 24 and Intermediate 109 |
| | 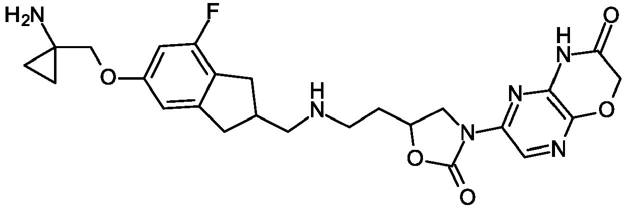 ESP [M+H]⁺: 513.3 | |
| **162** | 6-[5-[2-[[6-[(3-aminooxetan-3-yl)methoxy]-4-fluoro-2,3-dihydro-1*H*-inden-2-yl]methylamino]ethyl]-2-oxo-1,3-oxazolidin-3-yl]-4*H*-pyrido[3,2-b][1,4]oxazin-3-one; formic acid | Intermediate 1 and Intermediate 110 |
| | 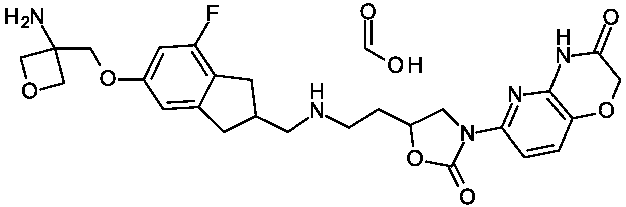 ESP [M+H]⁺: 528.3 | |
| **163** | 6-[5-[2-[(*rac*-1,2-*trans*-1-amino-4-fluoro-2,3-dihydro-1*H-*inden-2-yl)methylamino]ethyl]-2-oxo-1,3-oxazolidin-3-yl]-4H-pyrido[3,2-b][1,4]oxazin-3-one | Intermediate 1 and Intermediate 111 |
| | 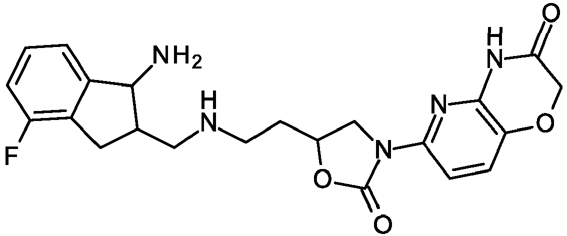 ESP [M+H]⁺: 442.2 | |
| **164** | 6-[5-[2-[(*rac*-1,2-*trans*-1-amino-4-fluoro-2,3-dihydro-1H-inden-2-yl)methylamino]ethyl]-2-oxo-1,3-oxazolidin-3-yl]-4H-pyrazino[2,3-b][1,4]oxazin-3-one; formic acid | Intermediate 24 and Intermediate 111 |
| | 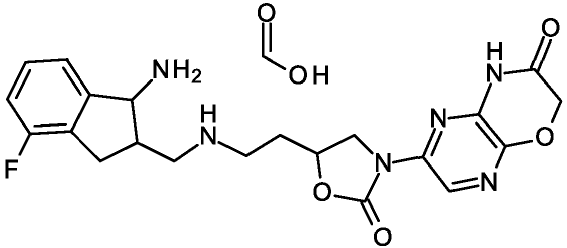 ESP [M+H]⁺: 443.2 | |
| **165** | 5-[2-[(*rac*-1,2-*trans*-1-amino-4-fluoro-2,3-dihydro-1*H*-inden-2-yl)methylamino]ethyl]-3-(3-oxo-4*H*-pyrido[3,2-b][1,4]thiazin-6-yl)-1,3-oxazolidin-2-one; formic acid | Intermediate 48 and Intermediate 111 |
| | 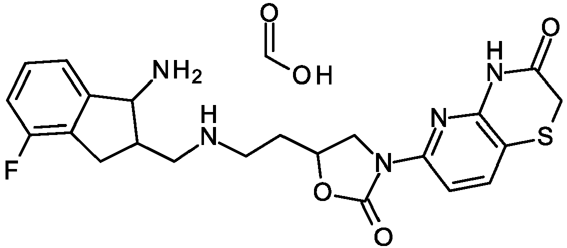 ESP [M+H]⁺: 458.3 | |
| **166** | 2-[[*rac*-5,6-*cis*-5,8-*trans*-6-amino-2-oxo-3-(3-oxo-4*H-*pyrido[3,2-b][1,4]oxazin-6-yl)-1-oxa-3-azaspiro[4.5]decan-8-yl]amino]-2,3-dihydro-1*H*-indene-4-carbonitrile | Intermediate 112 and Intermediate 6 |
| | 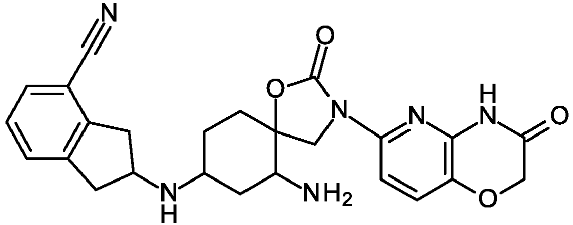 ESP [M+H]⁺: 475.3 | |
| **167** | 6-[*rac*-5,6-*cis*-5,8-*trans*-6-amino-8-[(4-fluoro-2,3-dihydro-1*H-*inden-2-yl)amino]-2-oxo-1-oxa-3-azaspiro[4.5]decan-3-yl]-4*H-*pyrido[3,2-b][1,4]oxazin-3-one; 2,2,2-trifluoroacetic acid | Intermediate 112 and 4-fluoro-1,3-dihydroinden-2-one [CAS# 1210824-58-0] |
| | 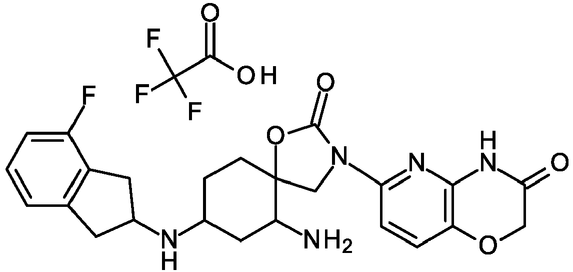 ESP [M+H]⁺: 468.2 | |
| **168** | 6-[2-oxo-5-[2-[[*rac*-1,2-*trans*-1-amino-7-chloro-2,3-dihydro-1*H*-inden-2-yl]methylamino]ethyl]-1,3-oxazolidin-3-yl]-4*H-*pyrido[3,2-b][1,4]oxazin-3-one; formic acid | Intermediate 1 and Intermediate 152 |
| | 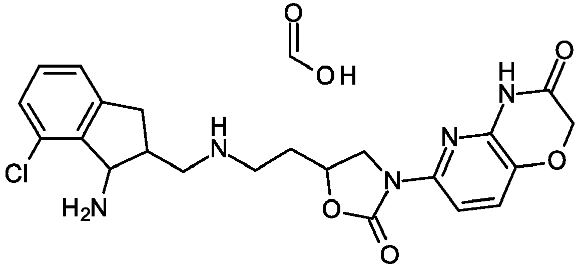 ESP [M+H]⁺: 458.1 | |
| **169** | 6-[2-oxo-5-[2-[[*rac*-1,2-*trans*-1-amino-7-chloro-2,3-dihydro-1*H*-inden-2-yl]methylamino] ethyl]-1,3-oxazolidin-3-yl]-4H-pyrazino[2,3-b][1,4]oxazin-3-one | Intermediate 24 and Intermediate 152 |
| | 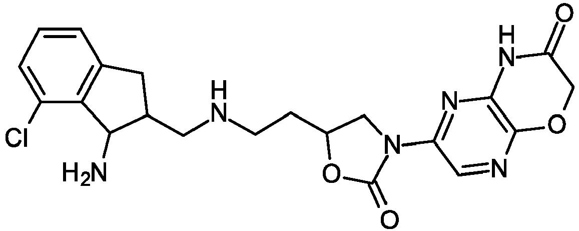 ESP [M+H]⁺: 459.2 | |
| **170** | 6-[2-oxo-5-[2-[[*rac*-1,2-*trans*-1-amino-4-chloro-2,3-dihydro-1*H*-inden-2-yl]methylamino]ethyl]-1,3-oxazol-3-yl]-4*H-*pyrido[3,2-b][1,4]oxazin-3-one | Intermediate 183 and Intermediate 92 |
| | 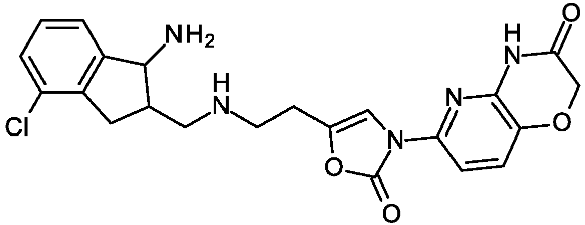 ESP [M+H]⁺: 456.2 | |
| **280** | 6-[5-[[2-(6-amino-4-fluoro-1-methyl-5,7-dihydrocyclopenta[c]pyridin-6-yl)ethylamino]methyl]-2-oxo-1,3-oxazolidin-3-yl]-4*H*-pyrido[3,2-b][1,4]oxazin-3-one | Intermediate 12 and Intermediate 198 |
| | 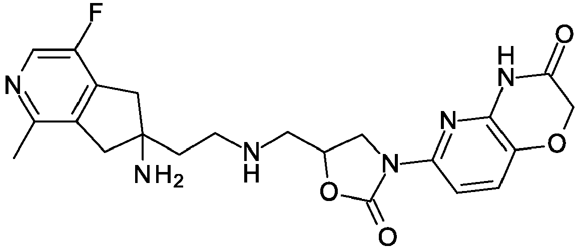 ESP [M+H]⁺: 457.2 | |

### Example 171

### 6-[5-[2-[[3-(2-Aminoethoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one

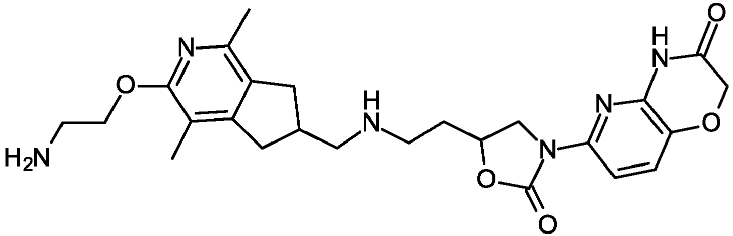

A suspension of 6-[5-(2-aminoethyl)-2-oxo-1,3-oxazolidin-3-yl]-4*H*-pyrido[3,2-b][1,4]oxazin-3-one (Intermediate 1, 59 mg, 0.212 mmol, 1 eq), *tert*-butyl *N*-[2-[(6-formyl-1,4-dimethyl-6,7-dihydro-5*H*-cyclopenta[c]pyridin-3-yl)oxy]ethyl]carbamate (Intermediate 113, 71 mg, 0.212 mmol, 1 eq), acetic acid (19.1 mg, 18.2 µL, 0.318 mmol, 1.5 eq) and sodium triacetoxyborohydride (67.4 mg, 0.318 mmol, 1.5 eq) in tetrahydrofuran (4 mL) was stirred at RT for 72 h. Additional sodium triacetoxyborohydride (22.5 mg, 0.106 mmol, 0.5 eq) was added and the mixture was stirred at RT for 5 h. Additional sodium triacetoxyborohydride (22.5 mg, 0.106 mmol, 0.5 eq) was added and the mixture was stirred at RT for 80 min. Water (basic with NaHCO₃) was added. The mixture was extracted with EtOAc (3 x). The combined organic layers were washed with brine (basic), dried over Na₂SO₄ and evaporated. The crude product was purified by flash column chromatography (20 g silica gel; DCM/MeOH 98:2 to 90:10) to give tert-butyl *N*-[2-[[1,4-dimethyl-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H*-cyclopenta[c]pyridin-3-yl]oxy]ethyl]carbamate (62 mg) as a white semi-solid. MS (ESN): m/z = 595.5 [M-H]⁻. A solution of this material in dichloromethane (1 mL) and trifluoroacetic acid (250 µL) was stirred at RT for 1 h. The mixture was concentrated. The crude product was purified by prep-HPLC to give the title compound (22 mg, 21 % yield) as a white solid. MS (ESN): m/z = 495.4 [M-H]⁻.

The following examples were prepared by analogy to Example 171.

| **Ex.** | **Name, Structure, MS** | **Starting Materials** |
|---|---|---|
| **172** | 6-[5-[2-[[3-[(1-aminocyclopropyl)methoxy]-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 114 |
| | 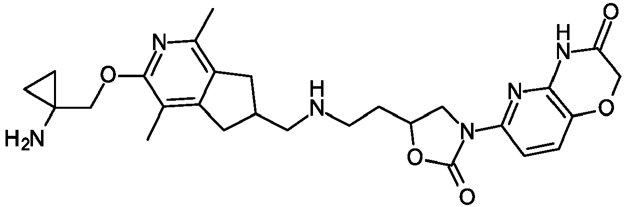 ESN [M-H]⁻: 521.1 | |
| **173** | 6-[5-[2-[[1,4-dimethyl-3-[2-(methylamino)ethoxy]-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 115 |
| | 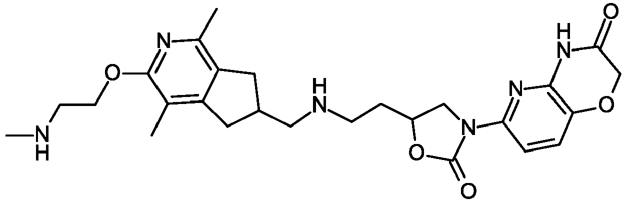 ESN [M-H]⁻: 509.4 | |
| **174** | 6-[5-[2-[[3-(2-aminoethoxy)-4-chloro-6,7-dihydro-5*H-*cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 116 |
| | 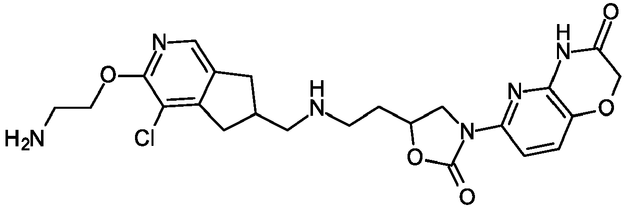 ESN [M-H]⁻: 501.3 | |
| **175** | 6-[5-[2-[[3-[(1-aminocyclopropyl)methoxy]-1-methyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 117 |
| | 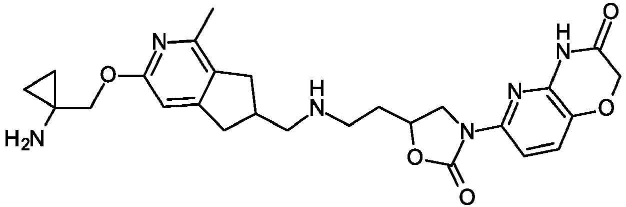 ESN [M-H]⁻: 507.3 | |
| **176** | 6-[5-[2-[[3-(3-aminopropoxy)-1,4-dimethyl-6,7-dihydro-5*H-*cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 118 |
| | 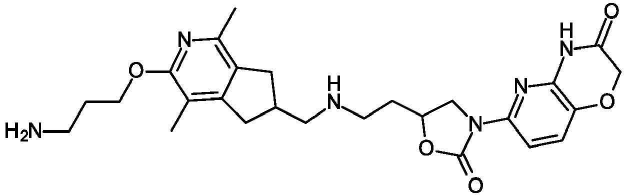 ESN [M-H]⁻: 509.2 | |
| **177** | 6-[5-[2-[[6-(2-aminoethoxy)-4-chloro-2,3-dihydro-1*H*-inden-2-yl]methylamino]ethyl]-2-oxo-1,3-oxazolidin-3-yl]-4*H-*pyrido[3,2-b][1,4]oxazin-3-one | Intermediate 1 and Intermediate 119 |
| | 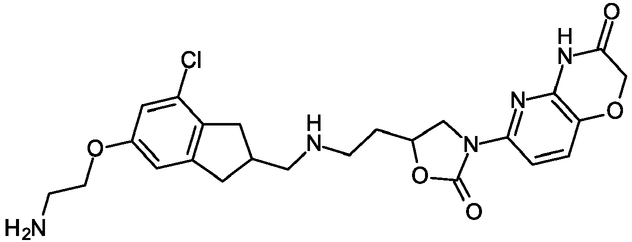 ESN [M-H]⁻: 500.3 | |
| **178** | 6-[5-[2-[[6-[(1-aminocyclopropyl)methoxy]-4-chloro-2,3-dihydro-1*H*-inden-2-yl]methylamino]ethyl]-2-oxo-1,3-oxazolidin-3-yl]-4*H*-pyrido[3,2-b][1,4] oxazin-3 -one | Intermediate 1 and Intermediate 120 |
| | 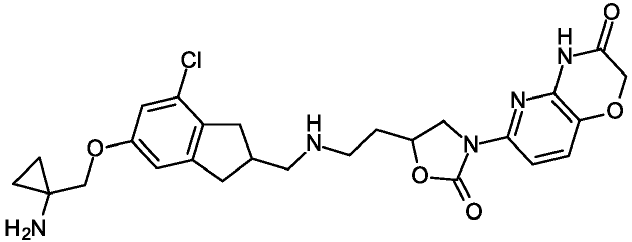 ESN [M-H]⁻: 526.3 | |
| **179** | 6-[5-[3-[[6-(2-aminoethoxy)-4-chloro-2,3-dihydro-1*H*-inden-2-yl]amino]propyl]-2-oxo-1,3-oxazolidin-3-yl]-4H-pyrido[3,2-b][1,4]oxazin-3-one | Intermediate 16 and Intermediate 121 |
| | 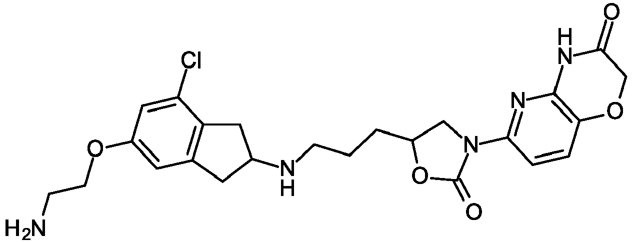 ESN [M-H]⁻: 500.3 | |
| **180** | 6-[5-[3-[[6-[(1-aminocyclopropyl)methoxy]-4-chloro-2,3-dihydro-1*H*-inden-2-yl]amino]propyl]-2-oxo-1,3-oxazolidin-3-yl]-4*H*-pyrido[3,2-b][1,4]oxazin-3-one | Intermediate 16 and Intermediate 122 |
| | 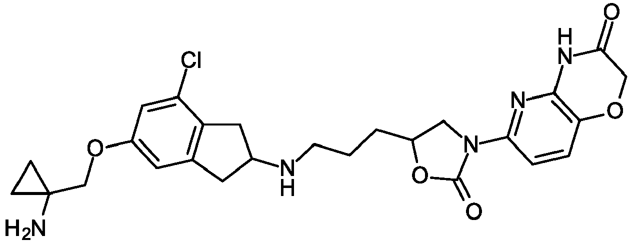 ESN [M-H]⁻: 526.3 | |
| **181** | 6-[5-[2-[[3-[(1-aminocyclopropyl)methoxy]-4-chloro-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 123 |
| | 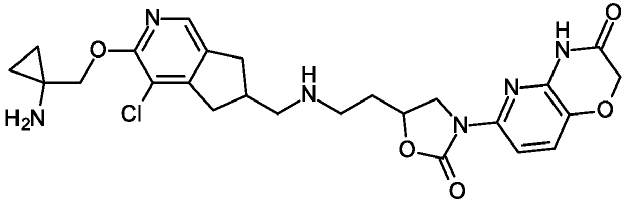 ESN [M-H]⁻: 527.3 | |
| **182** | 6-[[[2,4-*cis*-6-oxo-7-(3-oxo-4*H*-pyrido[3,2-b][1,4]oxazin-6-yl)-5-oxa-7-azaspiro[3.4]octan-2-yl]amino]methyl]-6,7-dihydro-5*H*-cyclopenta[c]pyridine-1-carbonitrile | Intermediate 124 and Intermediate 57 |
| | 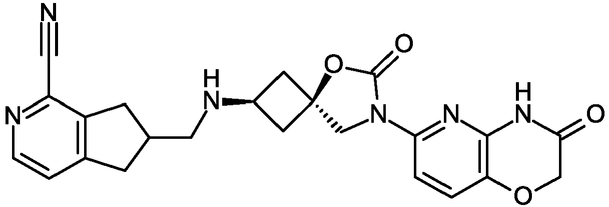 ESN [M-H]⁻: 445.4 | |
| **183** | 2-[[[2,4-*cis*-6-oxo-7-(3-oxo-4*H*-pyrido[3,2-b][1,4]oxazin-6-yl)-5-oxa-7-azaspiro[3.4]octan-2-yl]amino]methyl]-2,3-dihydro-1*H*-indene-4-carbonitrile | Intermediate 124 and Intermediate 5 |
| | 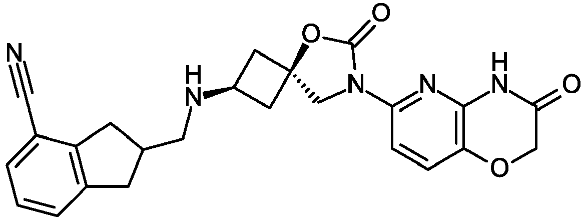 ESP [M+H]⁺: 446.2 | |
| **184** | 6-[5-[2-[[3-(azetidin-3-ylmethoxy)-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one hydrochloride | Intermediate 1 and Intermediate 125 |
| | 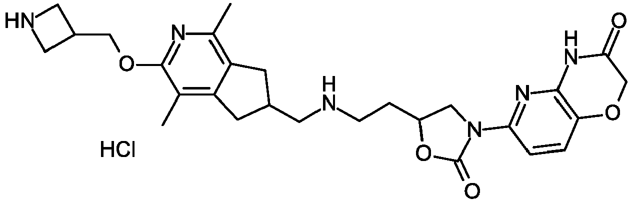 ESP [M+H]⁺: 523.2 | |
| **185** | 6-[(1-aminocyclopropyl)methoxy]-2-[[2-[2-oxo-3-(3-oxo-4*H-*pyrido[3,2-b][1,4]oxazin-6-yl)-1,3-oxazolidin-5-yl]ethylamino]methyl]-2,3-dihydro-1*H*-indene-4-carbonitrile | Intermediate 1 and Intermediate 126 |
| | 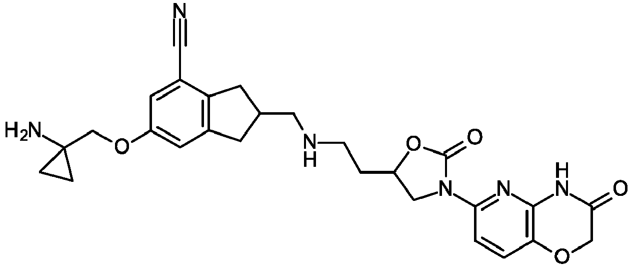 ESN [M-H]⁻: 517.5 | |
| **186** | 6-((1-aminocyclopropyl)methoxy)-2-(((2-(2-oxo-3-(3-oxo-3,4-dihydro-2*H*-pyrazino[2,3-b][1,4]oxazin-6-yl)oxazolidin-5-yl)ethyl)amino)methyl)-2,3-dihydro-1*H*-indene-4-carbonitrile | Intermediate 24 and Intermediate 126 |
| | 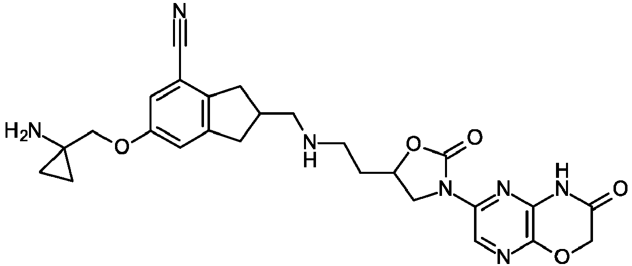 ESN [M-H]⁻: 518.4 | |
| **187** | 6-(azetidin-2-ylmethoxy)-2-[[2-[2-oxo-3-(3-oxo-4*H-*pyrido[3,2-b][1,4]oxazin-6-yl)-1,3-oxazolidin-5-yl]ethylamino]methyl]-2,3-dihydro-1*H*-indene-4-carbonitrile | Intermediate 1 and Intermediate 127 |
| | 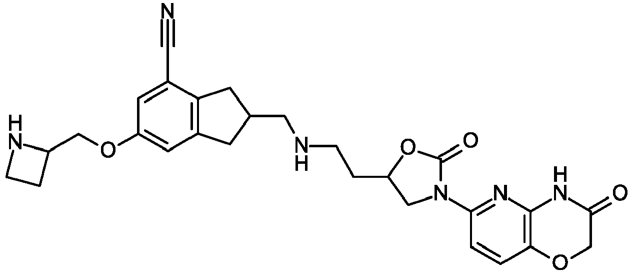 ESN [M-H]⁻: 517.5 | |
| **188** | 6-(2-aminoethoxy)-2-[[2-[2-oxo-3-(3-oxo-4*H*-pyrido[3,2-b] [1,4]oxazin-6-yl)-1,3-oxazolidin-5-yl]ethylamino]methyl]-2,3-dihydro-1*H*-indene-4-carbonitrile | Intermediate 1 and Intermediate 128 |
| | 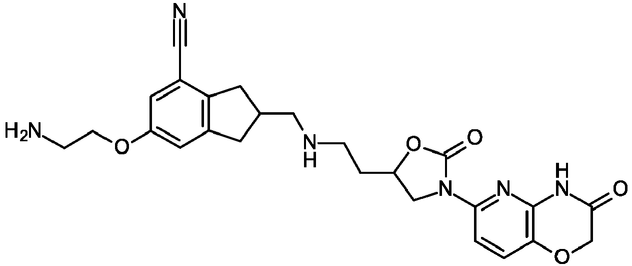 ESN [M-H]⁻: 491.5 | |
| **189** | 6-[5-[2-[[3-[2-(cyclopropylamino)ethoxy]-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 129 |
| | 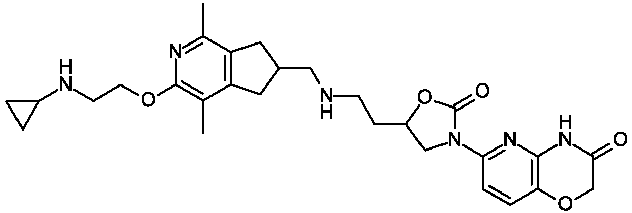 ESN [M-H]⁻: 535.5 | |
| **190** | 6-(azetidin-3-yloxy)-2-[[2-[2-oxo-3-(3-oxo-4*H*-pyrido[3,2-b] [1,4]oxazin-6-yl)-1,3-oxazolidin-5-yl]ethylamino]methyl]-2,3-dihydro-1*H*-indene-4-carbonitrile; 2,2,2-trifluoroacetic acid | Intermediate 1 and Intermediate 130 |
| | 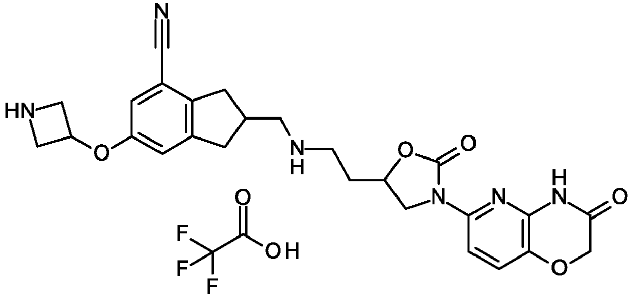 ESP [M+H]⁺: 505.4 | |
| **191** | 6-(azetidin-3-ylmethoxy)-2-[[2-[2-oxo-3-(3-oxo-4*H-*pyrido[3,2-b][1,4]oxazin-6-yl)-1,3-oxazolidin-5-yl]ethylamino]methyl]-2,3-dihydro-1*H*-indene-4-carbonitrile; 2,2,2-trifluoroacetic acid | Intermediate 1 and Intermediate 131 |
| | 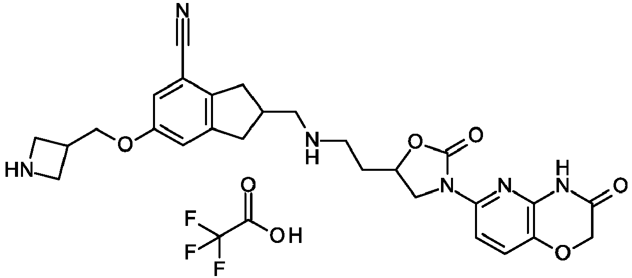 ESN [M-H]⁻: 517.5 | |
| **192** | 6-(2-aminopropoxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile; 2,2,2-trifluoroacetic acid | Intermediate 1 and Intermediate 132 |
| | 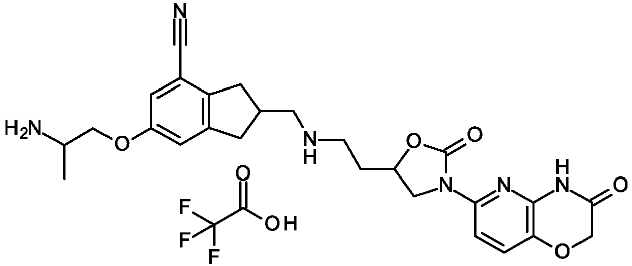 ESN [M-H]⁻: 505.5 | |
| **193** | 6-(2-aminopropoxy)-2-(((2-(2-oxo-3-(3-oxo-3,4-dihydro-2*H-*pyrazino[2,3-b][1,4]oxazin-6-yl)oxazolidin-5-yl)ethyl)amino)methyl)-2,3-dihydro-1*H*-indene-4-carbonitrile; 2,2,2-trifluoroacetic acid | Intermediate 24 and Intermediate 132 |
| | 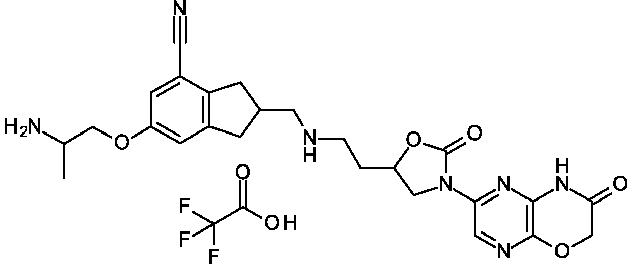 ESN [M-H]⁻: 506.4 | |
| **194** | 6-[2-oxo-5-[[2-[rac-1,2-*trans*-1-amino-4-chloro-2,3-dihydro-1*H*-inden-2-yl]ethylamino]methyl]-1,3-oxazolidin-3-yl]-4*H-*pyrido[3,2-b][1,4]oxazin-3-one | Intermediate 12 and Intermediate 133 |
| | 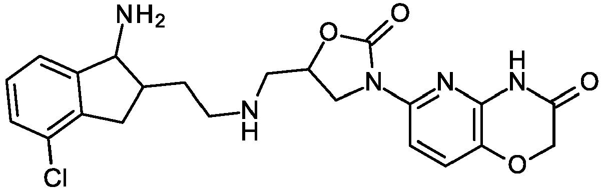 ESP [M+H]⁺: 458.2 | |
| **195** | 6-[6-oxo-2-[2-[*rac*-1,2-trans-1-amino-4-chloro-2,3-dihydro-1*H*-inden-2-yl]ethyl]-5-oxa-2,7-diazaspiro[3.4]octan-7-yl]-4*H-*pyrido[3,2-b][1,4]oxazin-3-one; 2,2,2-trifluoroacetic acid | Intermediate 21 and Intermediate 133 |
| | 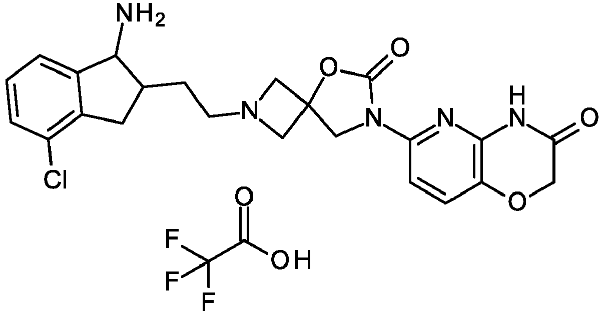 ESP [M+H]⁺: 470.4 | |
| **196** | 6-((1-aminocyclopropyl)methoxy)-2-(2-(((2-oxo-3-(3-oxo-3,4-dihydro-2*H*-pyrido[3,2-b][1,4]oxazin-6-yl)oxazolidin-5-yl)methyl)amino)ethyl)-2,3-dihydro-1*H*-indene-4-carbonitrile | Intermediate 12 and Intermediate 134 |
| | 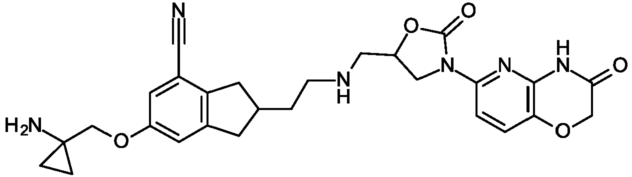 ESN [M-H]⁻: 517.5 | |
| **197** | 6-((1-aminocyclopropyl)methoxy)-2-(2-(6-oxo-7-(3-oxo-3,4-dihydro-2*H*-pyrido[3,2-b][1,4]oxazin-6-yl)-5-oxa-2,7-diazaspiro[3.4]octan-2-yl)ethyl)-2,3-dihydro-1*H*-indene-4-carbonitrile | Intermediate 21 and Intermediate 134 |
| | 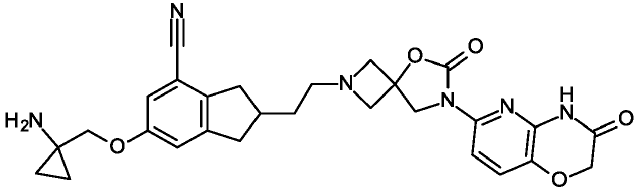 ESN [M-H]⁻: 529.5 | |
| **198** | 3-[(1-aminocyclopropyl)methoxy]-6-[[2-[2-oxo-3-(3-oxo-4*H-*pyrido[3,2-b][1,4]oxazin-6-yl)-1,3-oxazolidin-5-yl]ethylamino]methyl]-6,7-dihydro-5*H*-cyclopenta[c]pyridine-4-carbonitrile | Intermediate 1 and Intermediate 135 |
| | 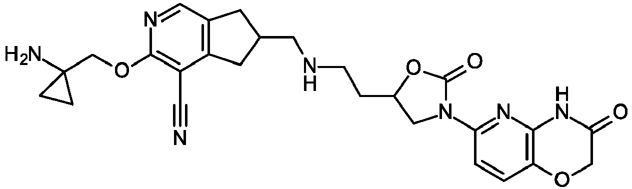 ESN [M-H]⁻: 518.5 | |
| **199** | 6-[5-[2-[[1,4-dimethyl-3-(morpholin-3-ylmethoxy)-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 136 |
| | 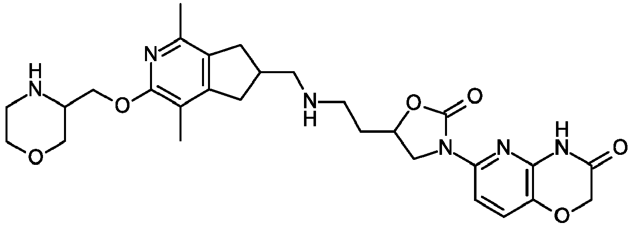 ESN [M-H]⁻: 551.4 | |
| 200 | 6-[5-[2-[[3-[[(2*S*)-azetidin-2-yl]methoxy]-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 137 |
| | 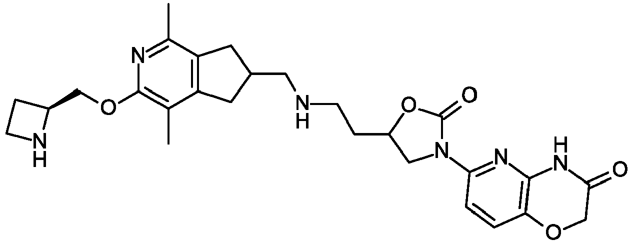 ESN [M-H]⁻: 521.5 | |
| **201** | 6-[5-[2-[[1,4-dimethyl-3-[2-(propan-2-ylamino)ethoxy]-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 138 |
| | 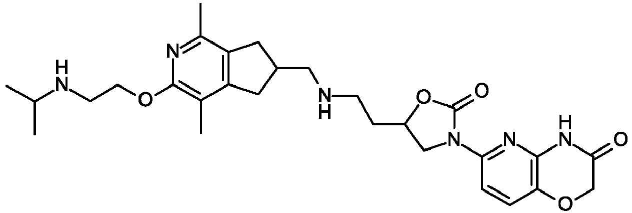 ESN [M-H]⁻: 537.7 | |
| **202** | 6-[5-[2-[[3-[2-(2-methoxyethylamino)ethoxy]-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 139 |
| | 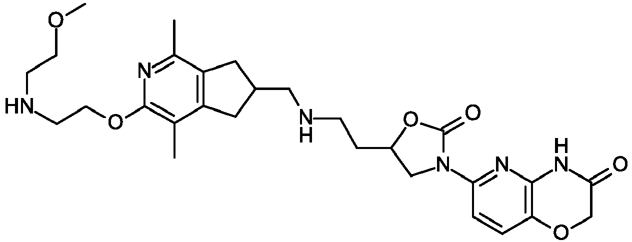 ESN [M-H]⁻: 553.6 | |
| 203 | 6-(2-amino-2-methylpropoxy)-2-(((2-(2-oxo-3-(3-oxo-3,4-dihydro-2*H*-pyrido[3,2-b][1,4]oxazin-6-yl)oxazolidin-5-yl)ethyl)amino)methyl)-2,3-dihydro-1*H*-indene-4-carbonitrile; 2,2,2-trifluoroacetic acid | Intermediate 1 and Intermediate 140 |
| | 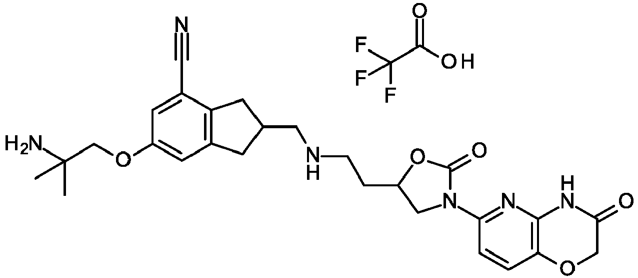 ESP [M+H]⁺: 521.3 | |
| **204** | 6-(2-amino-2-methylpropoxy)-2-(((2-(2-oxo-3-(3-oxo-3,4-dihydro-2*H*-pyrazino[2,3-b][1,4]oxazin-6-yl)oxazolidin-5-yl)ethyl)amino)methyl)-2,3-dihydro-1*H*-indene-4-carbonitrile | Intermediate 24 and Intermediate 140 |
| | 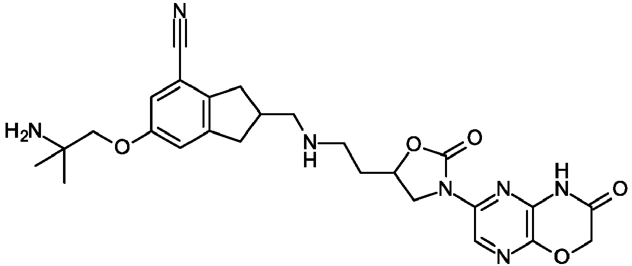 ESN [M-H]⁻: 520.5 | |
| **205** | 6-[5-[1-[(*rac*-1,2-*trans*-1-amino-4-chloro-2,3-dihydro-1*H-*inden-2-yl)methyl]azetidin-3-yl]-2-oxo-1,3-oxazolidin-3-yl]-4*H*-pyrazino[2,3-b][1,4]oxazin-3-one | Intermediate 92 and Intermediate 53 |
| | 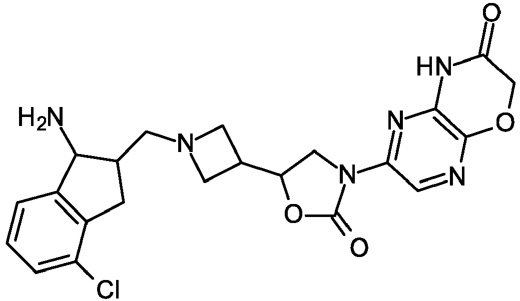 ESP [M+H]⁺: 471.1 | |
| **206** | 6-[*rac-*5,6-*cis*-5,8-*trans*-6-amino-8-[(1-chloro-6,7-dihydro-5*H-*cyclopenta[c]pyridin-6-yl)methylamino]-2-oxo-1-oxa-3-azaspiro[4.5]decan-3-yl]-4*H*-pyrido[3,2-b][1,4]oxazin-3-one | Intermediate 112 and Intermediate 19 |
| | 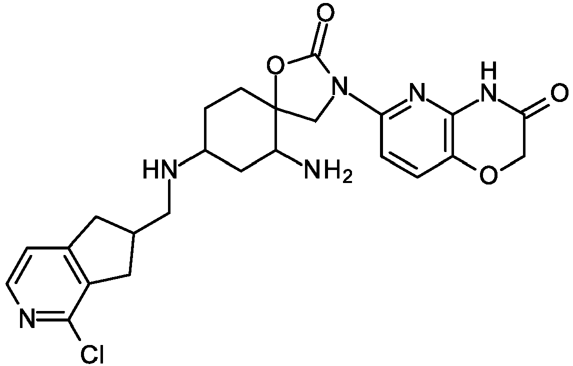 ESP [M+H]⁺: 499.3 | |
| **402** | 6-[(5*S*)-5-[2-[[(1*S*,2*S*)-1-amino-4-chloro-2,3-dihydro-1*H*-inden-2-yl]methylamino]ethyl]-2-oxo-1,3-oxazolidin-3-yl]*-*4*H-*pyrazino[2,3-b][1,4]oxazin-3-one | Intermediate 240 and Intermediate 287 |
| | 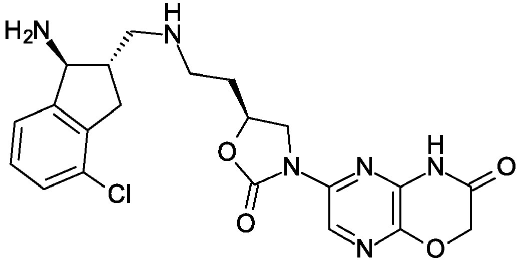 ESP [M+H]⁺: 459.2 | |
| **403** | 6-[(5*R*)-5-[2-[[(1*R*,2*R*)-1-amino-4-chloro-2,3-dihydro-1*H-*inden-2-yl]methylamino]ethyl]-2-oxo-1,3-oxazolidin-3-yl]-4*H-*pyrazino[2,3-b][1,4]oxazin-3-one | Intermediate 241 and Intermediate 286 |
| | 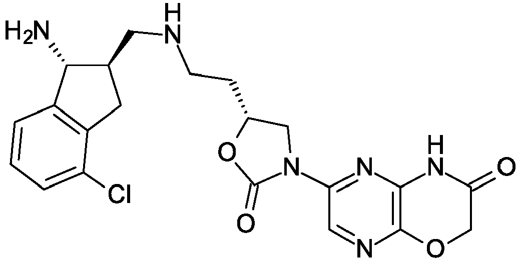 ESP [M+H]⁺: 459.2 | |
| **407** | 6-(2-amino-2-methylpropoxy)-2-[[2-oxo-3-(3-oxo-4*H-*pyrazino[2,3-b][1,4]oxazin-6-yl)-1-oxa-3,8-diazaspiro[4.5]decan-8-yl]methyl]-2,3-dihydro-1*H*-indene-4-carbonitrile | Intermediate 186 and Intermediate 140 |
| | 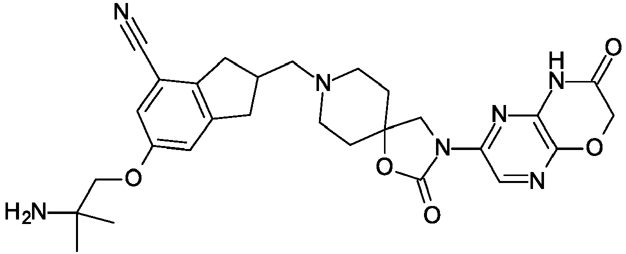 ESN [M-H]⁻: 546.4 | |
| **415** | 6-[5-[2-[[1-methyl-3-(1*H*-pyrazol-3-ylmethoxy)-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one | Intermediate 24 and Intermediate 295 |
| | 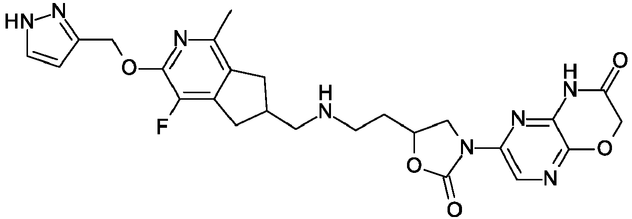 ESN [M-H]⁻: 537.3 | |
| 426 | 2-[[2-[2-oxo-3-(3-oxo-4*H*-pyrazino[2,3-b][1,4]oxazin-6-yl)-1,3-oxazolidin-5-yl]ethylamino]methyl]-6-(1*H*-triazol-5-ylmethoxy)-2,3-dihydro-1H-indene-4-carbonitrile | Intermediate 24 and Intermediate 304 |
| | 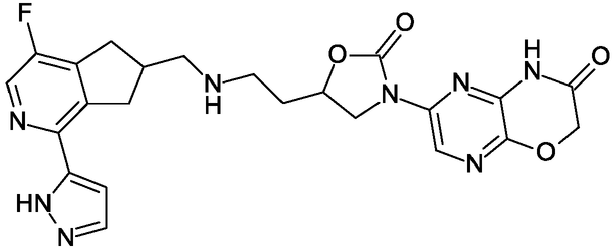 ESP [M+H]⁺: 495.1 | |
| **432** | 6-[5-[2-[[4-fluoro-1-(1*H*-pyrazol-4-yl)-6,7-dihydro-5*H-*cyclopenta[c]pyridin-6-yl]methylamino]ethyl]-2-oxo-1,3-oxazolidin-3-yl]-4*H*-pyrazino[2,3-b][1,4]oxazin-3-one | Intermediate 24 and Intermediate 307 |
| | 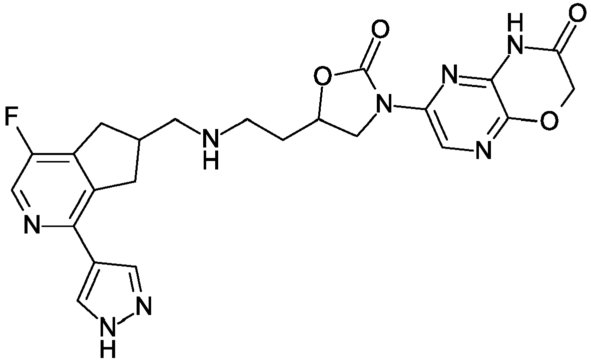 ESP [M+H]⁺: 495.1 | |

### Example 207

### 6-[2-Oxo-5-[2-[[1,2-trans-1-amino-4-chloro-2,3-dihydro-1H-inden-2-yl]methylamino]ethyl]-1,3-oxazolidin-3-yl]-4H-pyrido[3,2-b][1,4]oxazin-3-one, Isomer A

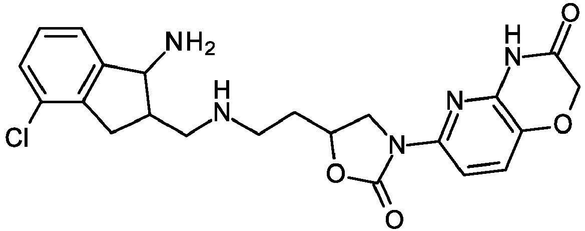

[(1,2-*trans*)-4-Chloro-1-[(2-methylpropan-2-yl)oxycarbonylamino]-2,3-dihydro-1*H*-inden-2-yl]methyl 4-methylbenzenesulfonate, Enantiomer A (Intermediate 141, 68 mg, 150 µmol, 1 eq), 6-[5-(2-aminoethyl)-2-oxo-1,3-oxazolidin-3-yl]-4*H*-pyrido[3,2-b] [1,4]oxazin-3-one (Intermediate 1, 41.9 mg, 150 µmol, 1 eq), sodium iodide (226 mg, 1.5 mmol, 10 eq) and triethylamine (91.3 mg, 126 µL, 903 µmol, 6 eq) were combined with DMF (1 mL) to give a white suspension. The reaction mixture was heated to 90 °C and was stirred at 90 °C for 3.5 h. The reaction mixture was poured into sat. aq. NaHCO₃ (3 mL) and was extracted with EtOAc (2 × 7 mL). The organic layers were combined, dried over Na₂SO₄ and evaporated. The crude material was purified by flash chromatography (silica gel, 5g, 1% to 10% MeOH in DCM) to give *tert*-butyl *N*-[(1,2-*trans*)-4-chloro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-1-yl]carbamate, Isomer A (45.4 mg) as a light brown foam. MS (ESP): m/z = 558.3 [M+H]⁺. This material and TFA (622 mg, 420 µL) were combined with DCM (1 mL) to give a light yellow solution. The reaction mixture was stirred at RT for 2 h. The crude reaction mixture was concentrated in vacuo. The crude product was purified by reverse phase preparative HPLC to give the title compound (6.4 mg, 9.3 % yield) as a colourless waxy solid. MS (ESP): m/z = 458.2 [M+H]⁺.

The following examples were prepared by analogy to Example 207.

| **Ex.** | **Name, Structure, MS** | **Starting Materials** |
|---|---|---|
| **208** | 6-[2-oxo-5-[2-[[1,2-*trans*-1-amino-4-chloro-2,3-dihydro-1*H-*inden-2-yl]methylamino]ethyl]-1,3-oxazolidin-3-yl]-4*H-*pyrido[3,2-b][1,4]oxazin-3-one, Isomer B | Intermediate 1 and Intermediate 142 |
| | 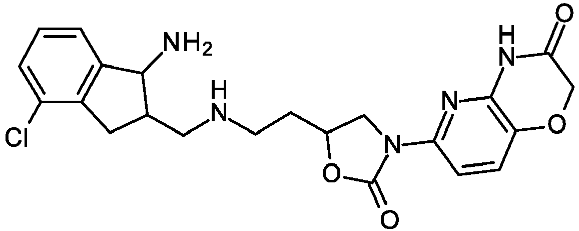 | |
| | ESP [M+H]⁺: 458.2 | |
| **209** | 6-[5-[2-[[3-(2-aminoethoxy)-1-methyl-6,7-dihydro-5*H-*cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 143 |
| | 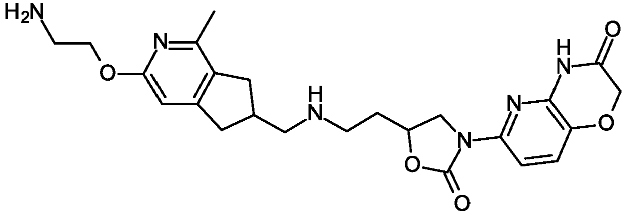 | |
| | ESN [M-H]⁻: 481.3 | |
| **210** | 2,4-*cis*-6-[6-oxo-2-[[*rac*-1,2-*trans*-1-amino-4-chloro-2,3-dihydro-1*H*-inden-2-yl]methylamino]-5-oxa-7-azaspiro[3.4]octan-7-yl]-4*H*-pyrido[3,2-b][1,4]oxazin-3-one | Intermediate 124 and Intermediate 144 |
| | 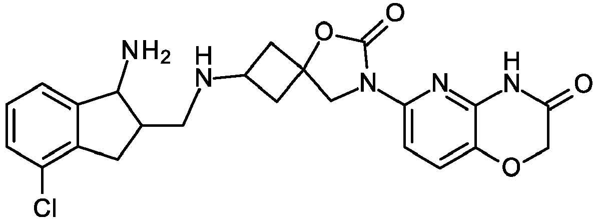 | |
| | ESN [M-H]⁻: 468.4 | |
| **211** | 2,4-*trans*-6-[6-oxo-2-[[*rac*-1,2-*trans*-1-amino-4-chloro-2,3-dihydro-1*H*-inden-2-yl]methylamino]-5-oxa-7-azaspiro[3.4]octan-7-yl]-4*H*-pyrido[3,2-b][1,4]oxazin-3-one | Intermediate 67 and Intermediate 144 |
| | 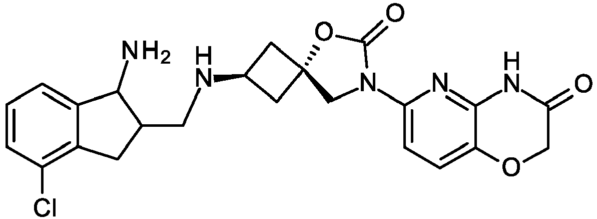 | |
| | ESN [M-H]⁻: 468.4 | |
| **212** | 6-[2-oxo-8-[[*rac*-1,2-*trans*-1-amino-4-chloro-2,3-dihydro-1*H-*inden-2-yl]methyl]-1-oxa-3,8-diazaspiro[4.5]decan-3-yl]-4*H-*pyrido[3,2-b][1,4]oxazin-3-one | Intermediate 17 and Intermediate 144 |
| | 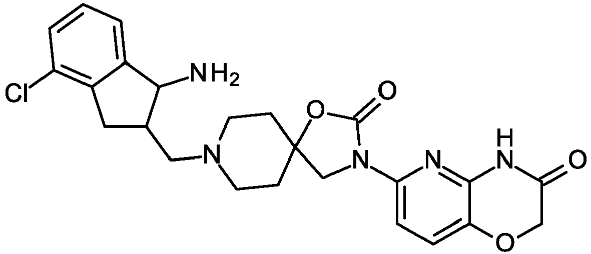 | |
| | ESN [M-H]⁻: 482.4 | |
| **213** | 3-[(1-aminocyclopropyl)methoxy]-4-methyl-6-[[2-[2-oxo-3-(3-oxo-4*H*-pyrido[3,2-b][1,4]oxazin-6-yl)-1,3-oxazolidin-5-yl]ethylamino]methyl]-6,7-dihydro-5*H*-cyclopenta[c]pyridine-1-carbonitrile | Intermediate 1 and Intermediate 145 |
| | 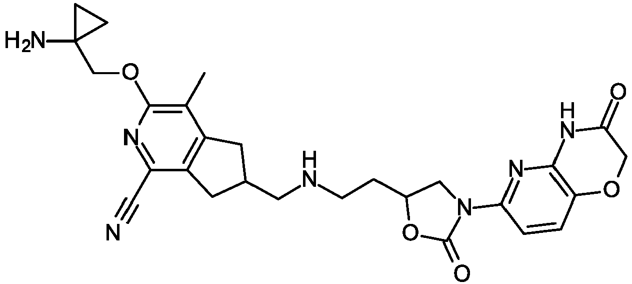 | |
| | ESP [M+H]⁺: 534.4 | |
| 214 | 3-[(1-aminocyclopropyl)methoxy]-1-methyl-6-[[2-[2-oxo-3-(3-oxo-4*H-*pyrido[3,2-b][1,4]oxazin-6-yl)-1,3-oxazolidin-5-yl]ethylamino]methyl]-6,7-dihydro-5*H*-cyclopenta[c]pyridine-4-carbonitrile | Intermediate 1 and Intermediate 146 |
| | 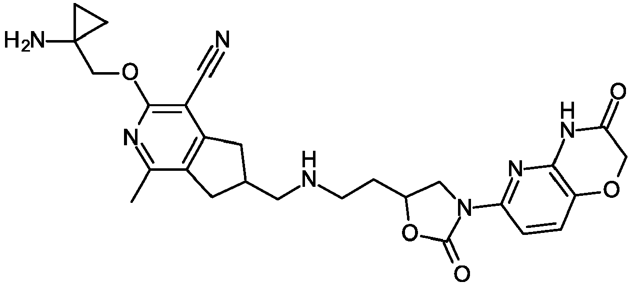 | |
| | ESP [M+H]⁺: 534.5 | |
| **215** | 3-[(1-aminocyclopropyl)methoxy]-1-methyl-6-[[2-oxo-3-(3-oxo-4*H*-pyrido[3,2-b][1,4]oxazin-6-yl)-1-oxa-3,8-diazaspiro[4.5]decan-8-yl]methyl]-6,7-dihydro-5*H-*cyclopenta[c]pyridine-4-carbonitrile | Intermediate 17 and Intermediate 146 |
| | 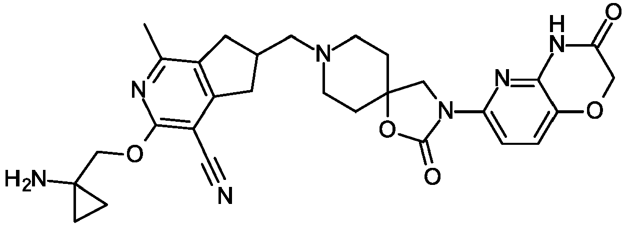 | |
| | ESP [M+H]⁺: 560.5 | |
| **216** | 6-[5-[2-[[3-[(1-aminocyclopropyl)methoxy]-4-methyl-1-(5-methyl-1,3-thiazol-2-yl)-6,7-dihydro-5*H*-cyclopenta[c]pyridin-6-yl]methylamino]ethyl]-2-oxo-1,3-oxazolidin-3-yl]-4*H-*pyrido[3,2-b][1,4]oxazin-3-one | Intermediate 1 and Intermediate 147 |
| | 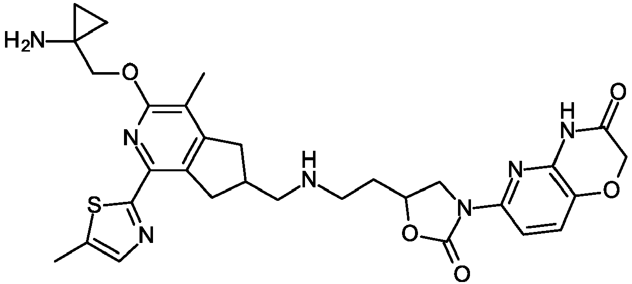 | |
| | ESP [M+H]⁺: 606.3 | |
| **217** | 6-[5-[2-[[3-[(1-aminocyclopropyl)methoxy]-4-methyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 148 |
| | 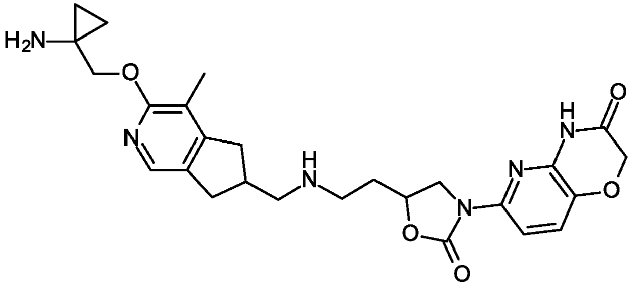 | |
| | ESP [M+H]⁺: 509.3 | |
| **218** | 6-[5-[2-[[3-[(1-aminocyclopropyl)methoxy]-4-methyl-1-(1,3-thiazol-2-yl)-6,7-dihydro-5*H*-cyclopenta[c]pyridin-6-yl]methylamino]ethyl]-2-oxo-1,3-oxazolidin-3-yl]-4*H-*pyrido[3,2-b][1,4]oxazin-3-one | Intermediate 1 and Intermediate 149 |
| | 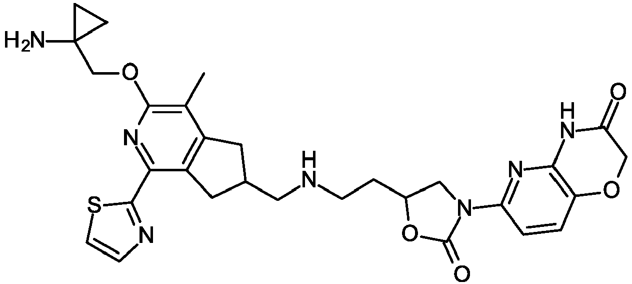 | |
| | ESP [M+H]⁺: 592.6 | |
| **219** | 6-[5-[2-[[4-methyl-3-[[(2*R*)-azetidin-2-yl]methoxy]-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one | Intermediate 1 and Intermediate 150 |
| | 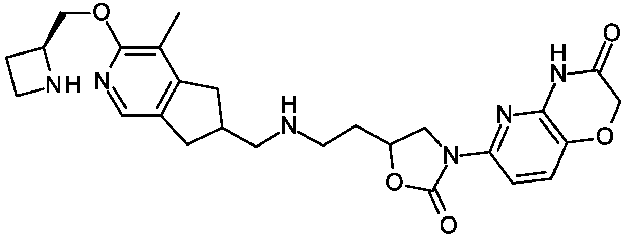 | |
| | ESP [M+H]⁺: 509.4 | |
| **220** | 4-methyl-6-((2-oxo-3-(3-oxo-3,4-dihydro-2*H*-pyrido[3,2-b][1,4]oxazin-6-yl)-1-oxa-3,8-diazaspiro[4.5]decan-8-yl)methyl)-3-((5-oxopiperazin-2-yl)methoxy)-6,7-dihydro-5*H-*cyclopenta[c]pyridine-1-carbonitrile | Intermediate 17 and Intermediate 151 |
| | 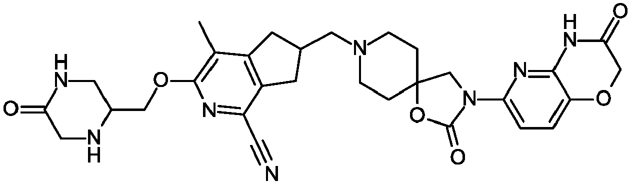 | |
| | ESP [M+H]⁺: 603.5 | |

### Example 221

### 2-((1-Cyano-4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)-N-methylacetamide

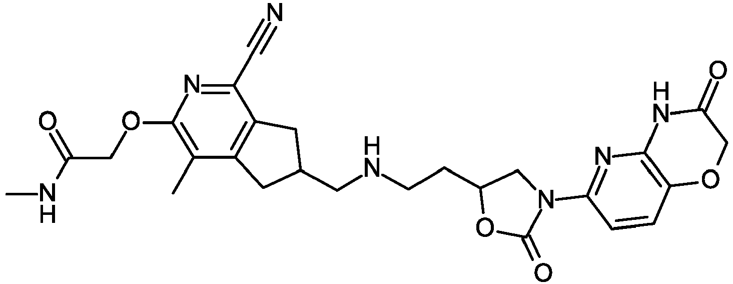

[1-Cyano-4-methyl-3-[2-(methylamino)-2-oxoethoxy]-6,7-dihydro-5*H*-cyclopenta[c]pyridin-6-yl]methyl 4-methylbenzenesulfonate (Intermediate 158, 76 mg, 166 µmol, 1 eq), 6-[5-(2-aminoethyl)-2-oxo-1,3-oxazolidin-3-yl]-4*H*-pyrido[3,2-b][1,4]oxazin-3-one (Intermediate 1, 57.1 mg, 166 µmol, 1 eq), sodium iodide (249 mg, 1.66 mmol, 10 eq) and triethylamine (101 mg, 139 µL, 998 µmol, 6 eq) were combined with DMF (1.5 mL). The reaction mixture was heated to 90 °C and stirred at 90 °C for 17 h. The reaction mixture was poured into NaHCO₃ (2 mL) and was extracted with EtOAc (2 × 5 mL). The precipitated solid was filtered and then dissolved in DCM/MeOH (9:1) and was extracted again with the previous aqueous phase. The organic layers were combined, dried over Na₂SO₄ and evaporated. The crude product was purified by reverse phase HPLC to give an impure product which was purified by prep-TLC (silica gel, 9:1 DCM/MeOH) to give the title compound (13.5 mg, 14.4 % yield) as a brown waxy solid. MS (ESP): m/z = 536.3 [M+H]⁺.

The following examples were prepared by analogy to Example 221.

| **Ex.** | **Name, Structure, MS** | **Starting Materials** |
|---|---|---|
| 222 | 2-((1-cyano-4-methyl-6-(((2-(2-oxo-3-(3-oxo-3,4-dihydro-2*H-*pyrido[3,2-b][1,4]oxazin-6-yl)oxazolidin-5-yl)ethyl)amino)methyl)-6,7-dihydro-5*H*-cyclopenta[c]pyridin-3-yl)oxy)-*N*-isopropylacetamide | Intermediate 1 and Intermediate 159 |
| | 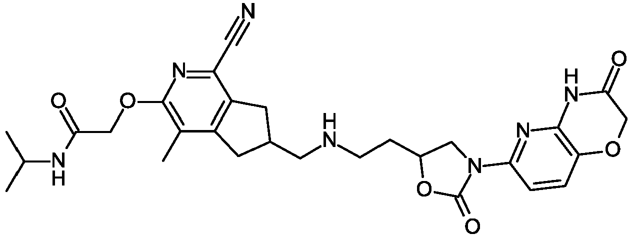 | |
| | ESP [M+H]⁺: 564.3 | |
| **223** | *N*-methyl-2-((4-methyl-6-(((2-(2-oxo-3-(3-oxo-3,4-dihydro-2*H-*pyrido[3,2-b][1,4]oxazin-6-yl)oxazolidin-5-yl)ethyl)amino)methyl)-6,7-dihydro-5*H*-cyclopenta[c]pyridin-3-yl)oxy)acetamide | Intermediate 1 and Intermediate 160 |
| | 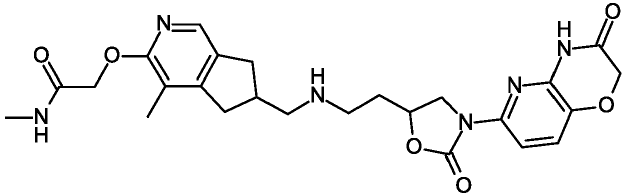 | |
| | ESP [M+H]⁺: 511.3 | |
| **224** | *N*-isopropyl-2-((4-methyl-6-(((2-(2-oxo-3-(3-oxo-3,4-dihydro-2*H*-pyrido[3,2-b][1,4]oxazin-6-yl)oxazolidin-5-yl)ethyl)amino)methyl)-6,7-dihydro-5*H*-cyclopenta[c]pyridin-3-yl)oxy)acetamide | Intermediate 1 and Intermediate 161 |
| | 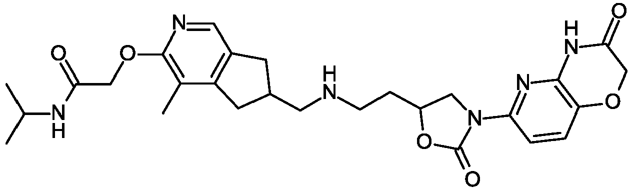 | |
| | ESP [M+H]⁺: 539.3 | |
| 225 | ethyl 1-ethyl-6-fluoro-4-oxo-8-[[2-[2-oxo-3-(3-oxo-4*H-*pyrido[3,2-b][1,4]oxazin-6-yl)-1,3-oxazolidin-5-yl]ethylamino]methyl]-8,9-dihydro-7*H*-cyclopenta[h]quinoline-3-carboxylate | Intermediate 1 and Intermediate 172 |
| | 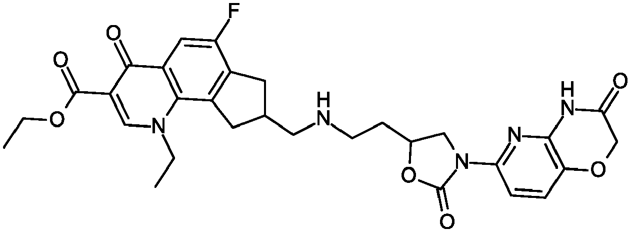 | |
| | ESP [M+H]⁺: 594.4 | |
| **416** | 6-[5-[2-[(5,8-dimethyl-5,7-diazatricyclo[7.3.0.0^{2,6}]dodeca-1,6,8-trien-11-yl)methylamino]ethyl]-2-oxo-1,3-oxazolidin-3-yl]-4*H*-pyrido[3,2-b][1,4]oxazin-3-one | Intermediate 1 and Intermediate 296 |
| | 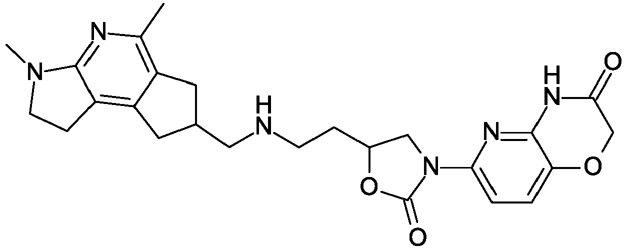 | |
| | ESP [M+H]⁺: 479.3 | |
| **421** | 6-[5-[2-[(4-methyl-6,7-dihydro-5*H*-cyclopenta[d]pyrimidin-6-yl)methylamino]ethyl]-2-oxo-1,3-oxazolidin-3-yl]-4*H-*pyrido[3,2-b][1,4]oxazin-3-one | Intermediate 1 and Intermediate 300 |
| | 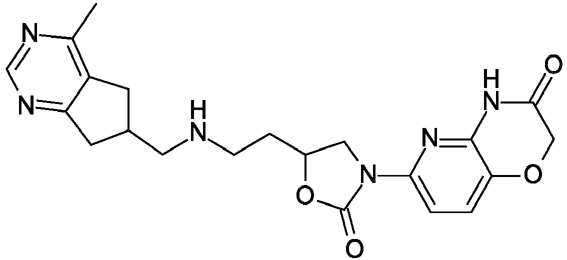 | |
| | ESP [M+H]⁺: 425.3 | |

### Example 226

### 6-[2-Oxo-5-[2-[[rac-1,2-trans-1-amino-4-chloro-2,3-dihydro-1H-inden-2-yl]methylamino]ethyl]-1,3-oxazolidin-3-yl]-4H-pyrido[3,2-b][1,4]oxazin-3-one

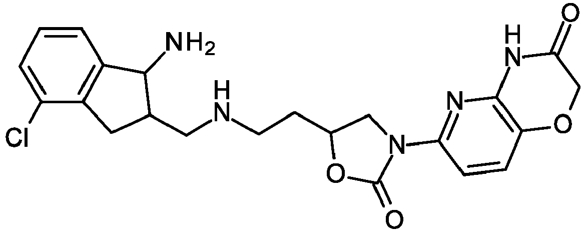

A solution of [*rac*-1,2-*trans*-4-chloro-1-[(2-methylpropan-2-yl)oxycarbonylamino]-2,3-dihydro-1*H*-inden-2-yl]methyl 4-methylbenzenesulfonate (Intermediate 144, 48.7 mg, 108 µmol, 1 eq), 6-[5-(2-aminoethyl)-2-oxo-1,3-oxazolidin-3-yl]-4*H*-pyrido[3,2-b] [1,4]oxazin-3-one (Intermediate 1, 30 mg, 108 µmol, 1 eq), sodium iodide (162 mg, 1.08 mmol, 10 eq) and triethylamine (65.5 mg, 90.2 µL, 647 µmol, 6 eq) in DMF (1 mL) was stirred at 90 °C for 3 h. The reaction mixture was poured into NaHCO₃ (3 mL) and extracted with EtOAc (2 × 7 mL). The organic layers were combined, dried over Na₂SO₄ and evaporated. The crude product was purified by flash chromatography (silica gel, 3g; 0% to 100% EtOAc in heptane then 0 to 15% MeOH in DCM) to give *rac-tert*-butyl (1,2-*trans*-4-chloro-2-(((2-(2-oxo-3-(3-oxo-3,4-dihydro-2*H*-pyrido[3,2-b][1,4]oxazin-6-yl)oxazolidin-5-yl)ethyl)amino)methyl)-2,3-dihydro-1*H*-inden-1-yl)carbamate (35 mg, 62.7 µmol, 53 % yield) as an off-white foam. A mixture of this material and 4N HCl in dioxane (157 µL, 627 µmol, 10 eq) in dioxane (1 mL) was stirred at RT for 2.5 h. To the reaction was added further 4N HCl in dioxane (157 µl, 627 µmol, 10 eq) and MeOH (0.5 mL) and the reaction was stirred at RT for 2 h. The mixture was concentrated in vacuo. The crude product was purified by reverse phase HPLC to give the title compound (8.5 mg, 29 % yield) as a colourless waxy solid. MS (ESP): m/z = 458.2 [M+H]⁺.

### Example 227

### 6-[2-Oxo-5-[3-[[rac-1,2-trans-1-amino-4-chloro-2,3-dihydro-1H-inden-2-yl]methylamino]propyl]-1,3-oxazolidin-3-yl]-4H-pyrido[3,2-b][1,4]oxazin-3-one

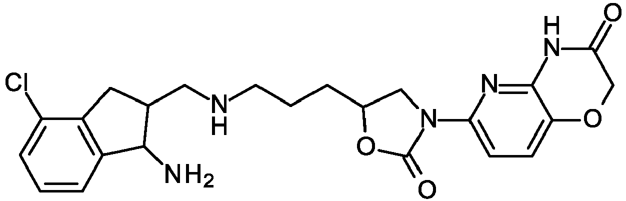

The title compound was prepared from 6-[5-(3-aminopropyl)-2-oxo-1,3-oxazolidin-3-yl]-4*H-*pyrido[3,2-b][1,4]oxazin-3-one (Intermediate 2) and [*rac*-1,2*-trans*-4-chloro-1-[(2-methylpropan-2-yl)oxycarbonylamino]-2,3-dihydro-1*H*-inden-2-yl]methyl 4-methylbenzenesulfonate (Intermediate 144) by analogy with Example 226 and was obtained as a colourless waxy solid. MS (ESP): m/z = 472.2 [M+H]⁺.

### Example 228

### rac-1,2-trans-1-Amino-2-[[2-[2-oxo-3-(3-oxo-4H-pyrido[3,2-b][1,4]oxazin-6-yl)-1,3-oxazolidin-5-yl] ethylamino] methyl] -2,3-dihydro-1H-indene-4-carbonitrile

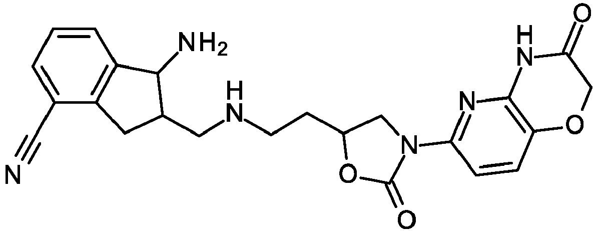

The title compound was prepared from 6-[5-(2-aminoethyl)-2-oxo-1,3-oxazolidin-3-yl]-4*H-*pyrido[3,2-b][1,4]oxazin-3-one (Intermediate 1) and [*rac*-1,2-*trans*-4-*cyano*-1-[(2-methylpropan-2-yl)oxycarbonylamino]-2,3-dihydro-1*H*-inden-2-yl]methyl 4-methylbenzenesulfonate (Intermediate 162) by analogy with Example 226 and was obtained as a white solid. MS (ESP): m/z = 449.1 [M+H]⁺.

### Example 229

### rac-1,2-trans-1-Amino-2-((((2-oxo-3-(3-oxo-3,4-dihydro-2H-pyrido[3,2-b][1,4]oxazin-6-yl)oxazolidin-5-yl)methyl)amino)methyl)-2,3-dihydro-1H-indene-4-carbonitrile

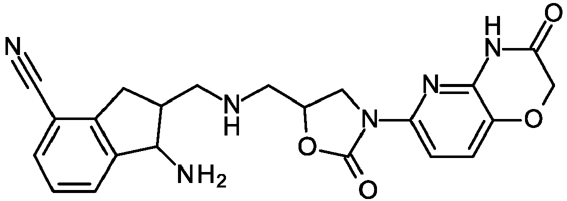

The title compound was prepared from 6-[5-(aminomethyl)-2-oxo-1,3-oxazolidin-3-yl]-4*H-*pyrido[3,2-b][1,4]oxazin-3-one (Intermediate 12) and [*rac*-1,2-*trans*-4-cyano-1-[(2-methylpropan-2-yl)oxycarbonylamino]-2,3-dihydro-1*H*-inden-2-yl]methyl 4-methylbenzenesulfonate (Intermediate 162) by analogy with Example 226 and was obtained as a colourless waxy solid. MS (ESP): m/z = 435.2 [M+H]⁺.

### Example 230

### 6-[2-Oxo-5-[2-[[rac-1,2-trans-4-chloro-1-hydroxy-2,3-dihydro-1H-inden-2-yl]methylamino]ethyl]-1,3-oxazolidin-3-yl]-4H-pyrido[3,2-b][1,4]oxazin-3-one

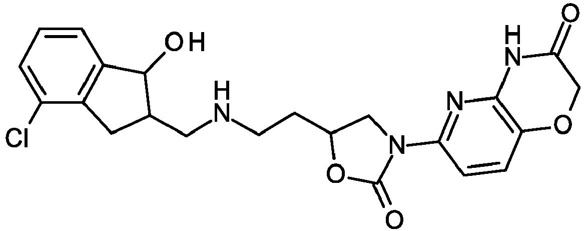

A suspension of *rac-*1,2-*trans*-1-[*tert*-butyl(dimethyl)silyl]oxy-4-chloro-2,3-dihydro-1*H*-indene-2-carbaldehyde (Intermediate 163, 50 mg, 0.161 mmol, 1 eq), 6-[5-(2-aminoethyl)-2-oxo-1,3-oxazolidin-3-yl]-4*H*-pyrido[3,2-b] [1,4]oxazin-3-one (Intermediate 1, 44.8 mg, 0.161 mmol, 1 eq), acetic acid (14.5 mg, 13.8 µl, 0.241 mmol, 1.5 eq) and sodium triacetoxyborohydride (51.1 mg, 0.241 mmol, 1.5 eq) in tetrahydrofuran (3 mL) was stirred at RT for 18 h. Additional sodium triacetoxyborohydride (17 mg, 0.0804 mmol, 0.5 eq) was added and the mixture was stirred at RT for 2 h. Water (basic with NaHCO₃) was added and the mixture was extracted with EtOAc (3 x). The organic layers were washed with brine (basic), dried over Na₂SO₄ and evaporated. The residue was purified by flash chromatography (20 g silica gel; DCM/MeOH 100:0 to 95:5) to give 6-[5-[2-[[*rac*-1,2-*trans*-1-[*tert*-butyl(dimethyl)silyl]oxy-4-chloro-2,3-dihydro-1*H*-inden-2-yl]methylamino]ethyl]-2-oxo-1,3-oxazolidin-3-yl]-4*H*-pyrido[3,2-b][1,4]oxazin-3-one (45 mg) as a yellow semi-solid. To a solution of this material in THF (2 mL) was added tetrabutylammonium fluoride solution 1 M in THF (65.9 µL, 0.0659 mmol, 1 eq) and the mixture stirred at 0 °C for 30 min. then at RT for 30 min. Additional tetrabutylammonium fluoride solution 1 M in THF (46 µl, 0.046 mmol, 0.7 eq) was added and the solution was stirred at RT for 18 h. The volatiles were removed. The residue was purified by preparative HPLC to give the title compound (11 mg) as an off-white solid. (ESP): m/z = 459.2 [M+H]⁺.

### Example 231

### 6-[2-Oxo-5-[2-[[rac-1,2-cis-4-chloro-1-hydroxy-2,3-dihydro-1H-inden-2-yl]methylamino]ethyl]-1,3-oxazolidin-3-yl]-4H-pyrido[3,2-b][1,4]oxazin-3-one

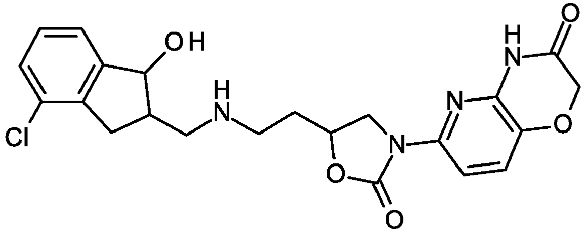

A solution of [*rac-*1,2-*cis*-1-[*tert*-butyl(dimethyl)silyl]oxy-4-chloro-2,3-dihydro-1*H*-inden-2-yl]methyl 4-methylbenzenesulfonate (Intermediate 164, 120 mg, 0.257 mmol, 1 eq), 6-[5-(2-aminoethyl)-2-oxo-1,3-oxazolidin-3-yl]-4*H*-pyrido[3,2-b][1,4]oxazin-3-one (Intermediate 1, 71.5 mg, 0.257 mmol, 1 eq), sodium iodide (385 mg, 2.57 mmol, 10 eq) and triethylamine (156 mg, 215 µl, 1.54 mmol, 6 eq) in *N,N*-dimethylacetamide (2 mL) was stirred at 90 °C for 2.5 h. The mixture was cooled to RT, diluted with water (basic with NaHCO₃) and extracted with EtOAc (3 x). The organic layers were washed with water (basic, 3 x) and brine (basic), dried over Na₂SO₄ and evaporated. The residue was purified by flash column chromatography (20 g silica gel; DCM/MeOH 100:0 - 95:5) to give 6-[2-oxo-5-[2-[[*rac*-1,2-*cis*-1-[*tert-*butyl(dimethyl)silyl]oxy-4-chloro-2,3-dihydro-1*H*-inden-2-yl]methylamino]ethyl]-1,3-oxazolidin-3-yl]-4*H*-pyrido[3,2-b] [1,4]oxazin-3-one (18 mg) as a yellow solid. A solution of this material (24 mg, 0.0419 mmol, 1 eq) and tetrabutylammonium fluoride solution 1 M in THF (62.8 µl, 0.0628 mmol, 1.5 eq) in THF (1 mL) was stirred at RT overnight. The volatiles were removed. The crude material was taken up in a small amount of water (basic with NaHCO₃) and extracted with EtOAc (3 x). The organic layers were washed with water (basic, 3 x) and brine (basic), dried over Na₂SO₄ and evaporated. The residue was purified by silica column chromatography (10 g silica gel; DCM/MeOH 100:0 to 98:2) to give the title compound as a light yellow solid. MS (ESP): m/z = 459.1 [M+H]⁺.

### Example 232

### 6-[5-[2-[(4-Chloro-2-hydroxy-1,3-dihydroinden-2-yl)methylamino]ethyl]-2-oxo-1,3-oxazolidin-3-yl]-4H-pyrido[3,2-b][1,4]oxazin-3-one; formic acid

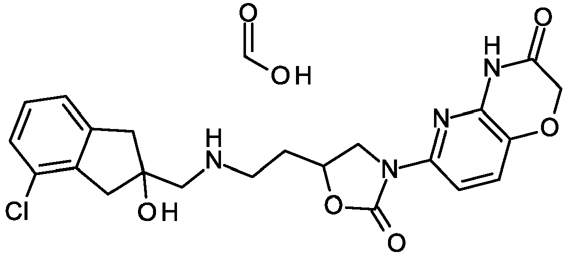

To a mixture of 2-[2-oxo-3-(3-oxo-4*H*-pyrido[3,2-b][1,4]oxazin-6-yl)-1,3-oxazolidin-5-yl]ethyl methanesulfonate (Intermediate 1, step 7, 20.0 mg, 0.060 mmol, 1 eq) and 2-(aminomethyl)-4-chloro-1,3-dihydroinden-2-ol (Intermediate 165, 13.3 mg, 0.070 mmol, 1.2 eq) in DMSO (3 mL) was added *N,N-*diisopropylethylamine (0.02 mL, 0.110 mmol, 2 eq) and the reaction mixture was stirred at 80 °C for 16 h. The mixture was quenched with brine (10 mL) and then extracted with EtOAc (3 × 15 mL), the organic phase was concentrated to give the crude product (30 mg). The crude product was purified by preparative HPLC (formic acid system) and then freeze-dried to give the title compound (2.3 mg, 0.005 mmol, 9.0 % yield) as a white solid. MS (ESP): m/z = 459.1 [M+H]⁺.

### Example 233

### 2-[[2-Aminoethyl-[2-[2-oxo-3-(3-oxo-4H-pyrido[3,2-b][1,4]oxazin-6-yl)-1,3-oxazolidin-5-yl]ethyl]amino]methyl]-2,3-dihydro-1H-indene-4-carbonitrile; formic acid

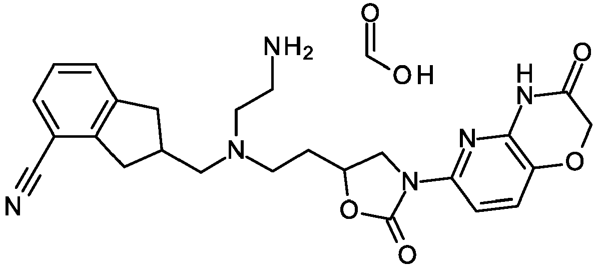

To a solution of 2-[2-[2-[2-oxo-3-(3-oxo-4*H*-pyrido[3,2-b][1,4]oxazin-6-yl)-1,3-oxazolidin-5-yl]ethylamino]ethyl]isoindole-1,3-dione (Intermediate 166, 20.0 mg, 0.040 mmol, 1 eq) in methanol (1.5 mL) was added 2-formyl-2,3-dihydro-1*H*-indene-4-carbonitrile (Intermediate 5, 9.1 mg, 0.050 mmol, 1.2 eq). The mixture was stirred at 25 °C for 1 h. Sodium cyanoborohydride (8.35 mg, 0.130 mmol, 3 eq) was added and the mixture was stirred at 25 °C for 16 h. The solvent was removed by evaporation to give 2-[[2-(1,3-dioxoisoindol-2-yl)ethyl-[2-[2-oxo-3-(3-oxo-4*H*-pyrido[3,2-b][1,4]oxazin-6-yl)-1,3-oxazolidin-5-yl]ethyl]amino]methyl]-2,3-dihydro-1*H*-indene-4-carbonitrile as a light yellow semi-solid, which was used directly without further purification. To a solution of this material (20.0 mg, 0.030 mmol, 1 eq) in ethanol (0.5 mL) was added hydrazine hydrate (6.6 mg, 0.130 mmol, 4 eq). The mixture was stirred at 25 °C for 16 h. The mixture was purified by preparative HPLC (formic acid system) and freeze dried to give the title compound (3.4 mg, 0.010 mmol, 21.4 % yield) as a light yellow solid. MS (ESP): m/z = 477.3 [M+H]⁺.

### Example 234

### 6-[5-[2-[[3-[(2R)-2-Amino-3-hydroxypropoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one

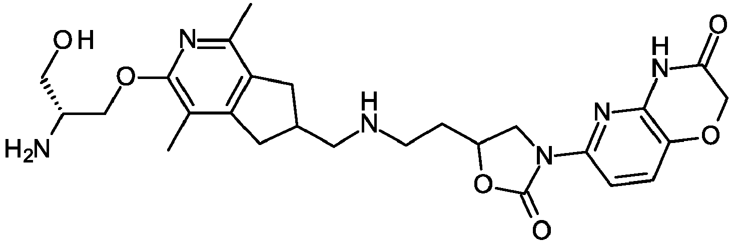

A suspension of *tert*-butyl (4*R*)-4-[(6-formyl-1,4-dimethyl-6,7-dihydro-5*H*-cyclopenta[c]pyridin-3-yl)oxymethyl]-2,2-dimethyl-1,3-oxazolidine-3-carboxylate (Intermediate 167, 142 mg, 0.316 mmol, 1 eq), 6-[5-(2-aminoethyl)-2-oxo-1,3-oxazolidin-3-yl]-4*H*-pyrido[3,2-b][1,4]oxazin-3-one (Intermediate 1, 87.9 mg, 0.316 mmol, 1 eq), acetic acid (28.5 mg, 27.1 µL, 0.474 mmol, 1.5 eq) and sodium triacetoxyborohydride (100 mg, 0.474 mmol, 1.5 eq) in tetrahydrofuran (6 mL) and methanol (1 mL) was stirred at RT for 18 h. Additional sodium triacetoxyborohydride (100 mg, 0.474 mmol, 1.5 eq) was added and the RM was stirred at RT for 18 h. Water (basic with NaHCO₃) was added. The mixture was extracted with EtOAc (3 x). The organic layers were washed with brine (basic), dried over Na₂SO₄ and evaporated. The residue was purified by flash column chromatography (20 g silica gel; DCM/MeOH 98:2 to 95:5) to give *tert-butyl* (4*R*)-4-[[1,4-dimethyl-6-[[2-[2-oxo-3-(3-oxo-4*H*-pyrido[3,2-b] [1,4]oxazin-6-yl)-1,3-oxazolidin-5-yl]ethylamino]methyl]-6,7-dihydro-5*H*-cyclopenta[c]pyridin-3-yl]oxymethyl]-2,2-dimethyl-1,3-oxazolidine-3-carboxylate (108 mg) as a white solid. This material was dissolved in dioxane (1 mL). At RT 4 N HCl in dioxane (787 µl, 3.15 mmol, 20 eq) was added dropwise followed by methanol (0.5 mL) The solution was stirred at RT for 1 h. The mixture was concentrated to dryness. DCM was added and removed three times. The crude product was adsorbed on adsorbent and purified by silica column chromatography (10 g silica gel; DCM/MeOH/NH4OH 95:4.5:0.5 to 92.5:6.75:0.75) to give the title compound (59 mg) as a white solid. MS (ESN): m/z = 525.5 [M-H]⁻.

### Example 235

### 3-[(2R)-2-Amino-3-hydroxypropoxy]-4-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile

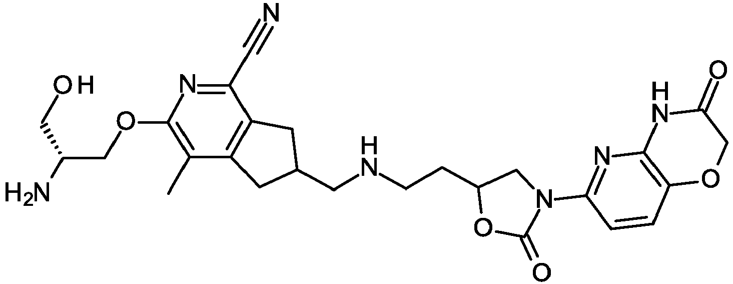

The title compound was prepared by analogy with Example 235 using *tert-butyl* (4*R*)-4-[(1-cyano-6-formyl-4-methyl-6,7-dihydro-5*H*-cyclopenta[c]pyridin-3-yl)oxymethyl]-2,2-dimethyl-1,3-oxazolidine-3-carboxylate (Intermediate 168) in place of *tert*-butyl (4*R*)-4-[(6-formyl-1,4-dimethyl-6,7-dihydro-5*H*-cyclopenta[c]pyridin-3-yl)oxymethyl]-2,2-dimethyl-1,3-oxazolidine-3-carboxylate and was obtained as a white foam. MS (ESP): m/z = 538.4 [M+H]⁺.

### Example 236

### 6-[(2R)-2-Amino-3-hydroxypropoxyl-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile

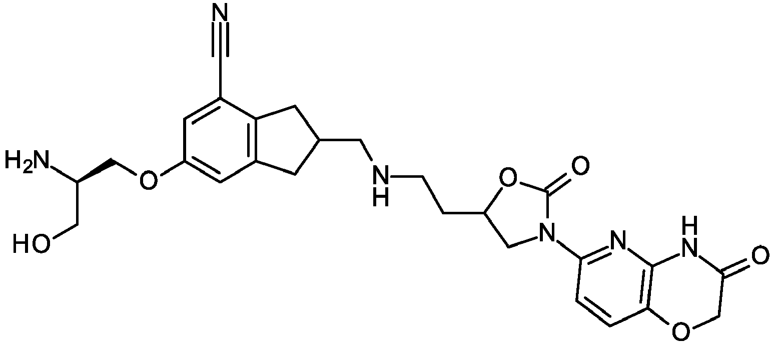

The title compound was prepared by analogy with Example 235 using *tert-butyl* (4*R*)-4-[(7-cyano-2-formyl-2,3-dihydro-1*H*-inden-5-yl)oxymethyl]-2,2-dimethyl-1,3-oxazolidine-3-carboxylate (Intermediate 169) in place of *tert*-butyl (4*R*)-4-[(6-formyl-1,4-dimethyl-6,7-dihydro-5*H*-cyclopenta[c]pyridin-3-yl)oxymethyl]-2,2-dimethyl-1,3-oxazolidine-3-carboxylate and was obtained as a white solid. MS (ESN): m/z = 521.5 [M-H]⁻.

### Example 237

### 6-[5-[2-[[4-Chloro-3-[(2R)-2-amino-3-hydroxypropoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one

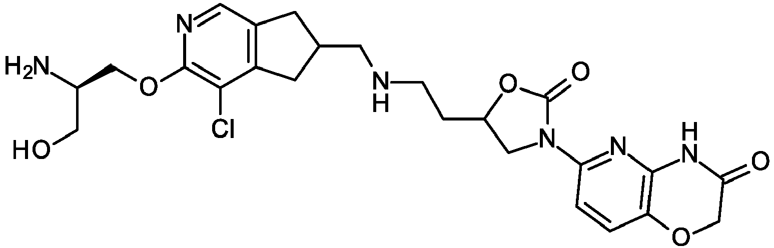

The title compound was prepared by analogy with Example 234 using *tert*-butyl (4*R*)-4-[(4-chloro-6-formyl-6,7-dihydro-5*H*-cyclopenta[c]pyridin-3-yl)oxymethyl]-2,2-dimethyl-1,3-oxazolidine-3-carboxylate (Intermediate 170) in place of *tert*-butyl (4*R*)-4-[(6-formyl-1,4-dimethyl-6,7-dihydro-5*H*-cyclopenta[c]pyridin-3-yl)oxymethyl]-2,2-dimethyl-1,3-oxazolidine-3-carboxylate and was obtained as a white solid. MS (ESN): m/z = 531.5 [M-H]⁻.

### Example 238

### 2-[[2-Hydroxy-3-[2-oxo-3-(3-oxo-4H-pyrido[3,2-b][1,4]oxazin-6-yl)-1,3-oxazolidin-5-yl]propyl]amino]-2,3-dihydro-1H-indene-4-carbonitrile hydrochloride

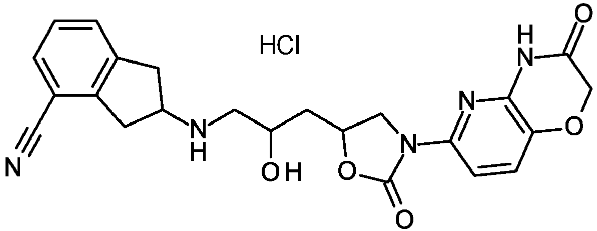

To a solution of 6-[5-[3-amino-2-[*tert*-butyl(dimethyl)silyl]oxypropyl]-2-oxo-1,3-oxazolidin-3-yl]-4*H*-pyrido[3,2-b][1,4]oxazin-3-one (Intermediate 171, 110.0 mg, 0.260 mmol, 1 eq) and 2-oxo-1,3-dihydroindene-4-carbonitrile (Intermediate 6, 40.9 mg, 0.260 mmol, 1 eq) in methanol (20 mL) was added a drop of AcOH, and then to the solution was added sodium cyanoborohydride (49.1 mg, 0.780 mmol, 3 eq), and the solution was stirred at 25 °C for 16 h. The solvent was removed and the residue was taken up in brine (30 mL) and extracted with DCM (30 mL × 2), combined with the organics and dried by Na₂SO₄. The solvent was removed to give the crude, which was purified by preparative TLC (DCM: MeOH=10:1) to give 2-[[2-[*tert*-butyl(dimethyl)silyl]oxy-3-[2-oxo-3-(3-oxo-4*H*-pyrido[3,2-b] [1,4]oxazin-6-yl)-1,3-oxazolidin-5-yl]propyl]amino]-2,3-dihydro-1*H*-indene-4-carbonitrile (40 mg, 0.070 mmol, 27.3 % yield) as a brown oil. A solution of this material in HCl (gas) in MeOH (1.0 mL) was stirred at 25 °C for 1 h. The solvent was removed to give the crude, which was purified by preparative TLC (DCM: MeOH=10:1) and then was purified by preparative HPLC (HCl system) to give the title compound (1.5 mg, 4.7 % yield) as a white solid. MS (ESP): m/z = 450.1 [M+H]⁺.

### Example 239

### 1-Ethyl-6-fluoro-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylic acid

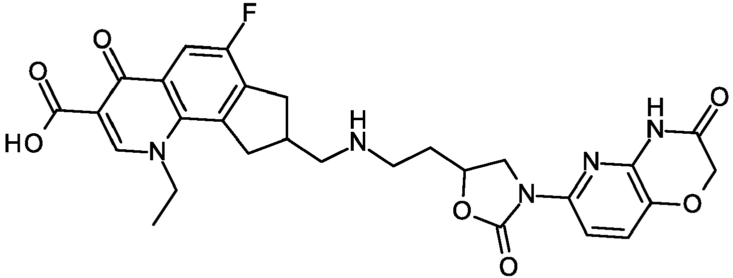

To a solution of ethyl 1-ethyl-6-fluoro-4-oxo-8-[[2-[2-oxo-3-(3-oxo-4*H*-pyrido[3,2-b] [1,4]oxazin-6-yl)-1,3-oxazolidin-5-yl]ethylamino]methyl]-8,9-dihydro-7*H-*cyclopenta[h]quinoline-3-carboxylate (Example 225, 22.1 mg, 37.2 µmol, 1 eq) in THF (0.5 mL), MeOH (0.5 mL) and H₂O (0.2 mL) was added 1N LiOH in H₂O (149 µL, 149 µmol, 4 eq). The reaction mixture was stirred at RT for 5 h. The crude reaction mixture was concentrated in vacuo. To the reaction mixture was added 1N HCl (149 µL) to neutralize the reaction (pH 6). The product precipitated and was filtered off. The precipitate was triturated with a mix of H₂O, THF and MeOH, filtered and washed with the solvent-mixture. The solid was dried to give the title compound (17 mg, 72.7 % yield) as a dark brown solid. (ESP): m/z = 566.3 [M+H]⁺.

### Example 240

### 6- [5- [2- [(2-Amino-4-chloro-1,3 -dihydroinden-2-yl)methylamino] ethyl] -2-oxo-1,3 -oxazolidin-3 - yl]-4H-pyrido[3,2-b][1,4]oxazin-3-one; formic acid

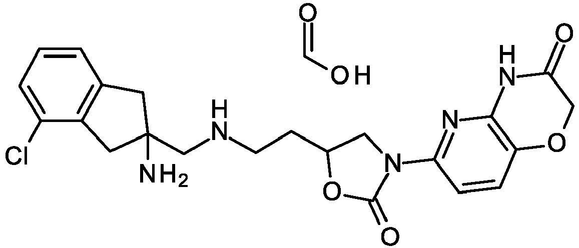

A solution of 9*H*-fluoren-9-ylmethyl *N*-(4-chloro-2-formyl-1,3-dihydroinden-2-yl)carbamate (Intermediate 173, 600.0 mg, 1.44 mmol, 1 eq) and 6-[5-(2-aminoethyl)-2-oxo-oxazolidin-3-yl]-4*H*-pyrido[3,2-b][1,4]oxazin-3-one (Intermediate 1, 422.79 mg, 1.52 mmol, 1.06 eq) in 2-propanol (3 mL) and chloroform (21 mL) was stirred at 20 °C for 14 h. Then to the mixture was added sodium triacetoxyborohydride (472.5 mg, 2.23 mmol, 1.55 eq) and the mixture stirred at 20 °C for 2 h. H₂O (5 mL) was added and the mixture concentrated in vacuo to give a crude. The crude was purified by preparative HPLC (formic acid system) and lyophilized to give 9*H-*fluoren-9-ylmethyl *N*-[4-chloro-2-[[2-[2-oxo-3-(3-oxo-4*H*-pyrido[3,2-b][1,4]oxazin-6-yl)-1,3-oxazolidin-5-yl]ethylamino]methyl]-1,3-dihydroinden-2-yl]carbamate (100 mg, 0.150 mmol, 10.2 % yield) as a white solid. A solution of 9*H*-fluoren-9-ylmethyl *N*-[4-chloro-2-[[2-[2-oxo-3-(3-oxo-4*H*-pyrido[3,2-b][1,4]oxazin-6-yl)-1,3-oxazolidin-5-yl]ethylamino]methyl]-1,3-dihydroinden-2-yl]carbamate (50.0 mg, 0.070 mmol, 1 eq) and 20% piperidine in DMF (5.0 mL, 0.070 mmol, 1 eq) was stirred at 25 °C for 1 h. The mixture was concentrated in vacuo to give a crude. The crude was purified by preparative HPLC (formic acid system) and lyophilized to give the title compound (9.3 mg, 0.020 mmol, 23.5 % yield) as a light yellow solid. MS (ESP): m/z = 458.1 [M+H]⁺.

### Example 241

### 2-[[rac-5,6-trans-5,8-cis-6-Amino-2-oxo-3-(3-oxo-4H-pyrido[3,2-b][1,4]oxazin-6-yl)-1-oxa-3-azaspiro[4.5]decan-8-yl]amino]-2,3-dihydro-1H-indene-4-carbonitrile; formic acid

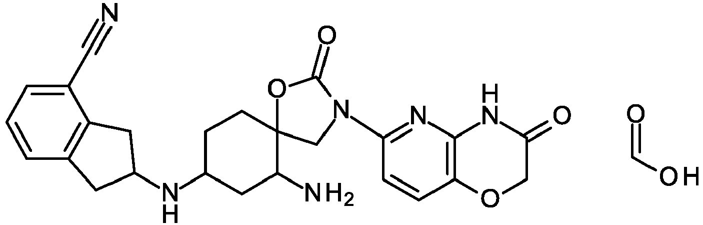

To a solution of *tert*-butyl *N*-[*rac-*5,6-*trans*-5,8-*cis*-8-amino-2-oxo-3-(3-oxo-4*H*-pyrido[3,2-b][1,4]oxazin-6-yl)-1-oxa-3-azaspiro[4.5]decan-6-yl]carbamate (Intermediate 174, 16.7 mg, 36.6 µmol, 1 eq) in THF (2 mL) and MeOH (2 mL) under argon was added 3Å powdered mol sieves (25 mg), then 2-oxo-1,3-dihydroindene-4-carbonitrile (Intermediate 6, 7.48 mg, 47.6 µmol, 1.3 eq) was added, followed by addition of acetic acid (3.74 mg, 3.56 µl, 62.2 µmol, 1.7 eq) and the mixture was stirred for 30 min. at RT. Sodium cyanoborohydride (3.91 mg, 62.2 µmol, 1.7 eq) was added and the mixture was stirred at 40 °C for 14 h. SiO₂ (3 g) was added and the mixture evaporated totally. The crude product was purified by silica cartridge chromatography (ISCO 10 g, CH₂Cl₂ to 5% MeOH/CH₂Cl₂) to give *tert*-butyl N-[8-[(4-cyano-2,3-dihydro-1*H*-inden-2-yl)amino]-2-oxo-3-(3-oxo-4*H*-pyrido[3,2-b] [1,4]oxazin-6-yl)-1-oxa-3-azaspiro[4.5]decan-6-yl]carbamate (12.4 mg, 19.9 µmol, 54.2 % yield) as an off-white solid. This material was dissolved in formic acid (91.4 mg, 76.1 µL, 1.99 mmol, 100 eq) and the mixture stirred for 1 h at RT. The mixture was evaporated totally, MeCN (1 mL) and water (20 mL) were added and the mixture lyophilized overnight to give the title compound (7.1 mg, 12.5 µmol, 63.2 % yield) as a white solid. MS (ESN): m/z = 473.4 [M-H]⁻.

### Example 242

### 6-[5-(Aminomethyl)-5-[2-[(4-fluoro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one

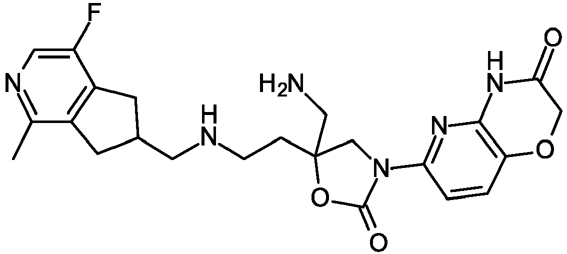

To a solution of *tert*-butyl *N*-[[5-(2-aminoethyl)-2-oxo-3-(3-oxo-4*H*-pyrido[3,2-b][1,4]oxazin-6-yl)-1,3-oxazolidin-5-yl]methyl]carbamate (Intermediate 175, 60.0 mg, 0.150 mmol, 1 eq) and 4-fluoro-1-methyl-6,7-dihydro-5*H*-cyclopenta[c]pyridine-6-carbaldehyde (Intermediate 27, 43.98 mg, 0.150 mmol, 1 eq) in THF (2 mL) and 1-propanol (2 mL) was added acetic acid (8.84 mg, 0.150 mmol, 1 eq). After 30 min. sodium cyanoborohydride (27.8 mg, 0.440 mmol, 3 eq) was added and the mixture stirred for 16 h at 30 °C. The mixture was poured into water (10 mL), extracted with ethyl acetate (10 mL × 2), the organic layer was washed with brine (10 mL), dried over anhydrous Na₂SO₄ and concentrated in vacuo to afford *tert*-butyl *N*-[[5-[2-[(4-fluoro-1-methyl-6,7-dihydro-5*H*-cyclopenta[c]pyridin-6-yl)methylamino]ethyl]-2-oxo-3-(3-oxo-4*H-*pyrido[3,2-b][1,4]oxazin-6-yl)-1,3-oxazolidin-5-yl]methyl]carbamate (80 mg, 0.140 mmol, 95.2 % yield) as a colourless oil. To a solution of this material in ethyl acetate (3 mL) was added HCl/EtOAc (3.0 mL, 12 mmol). The mixture was stirred at 25 °C for 2 h. The mixture was concentrated in vacuo, purified by preparative HPLC (NH₄OH), and lyophilised to afford the title compound (19 mg, 0.040 mmol, 27.2 % yield) as a white solid. MS (ESP): m/z = 471.2 [M+H]⁺.

The following examples were prepared by analogy to Example 242.

| **Ex.** | **Name, Structure, MS** | **Starting Materials** |
|---|---|---|
| **243** | 2-[[2-[5-(aminomethyl)-2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile | Intermediate 175 and Intermediate 5 |
| | 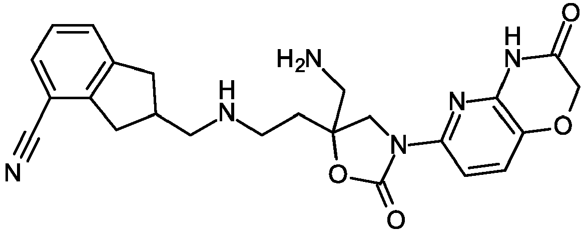 ESP [M+H]⁺: 463.3 | |
| **244** | 6-[2-oxo-5-[3-[[*rac*-1,2-*trans*-1-amino-4-chloro-2,3-dihydro-1*H*-inden-2-yl]amino]propyl]-1,3-oxazolidin-3-yl]-4*H-*pyrido[3,2-b][1,4]oxazin-3-one | Intermediate 16 and Intermediate 176 |
| | 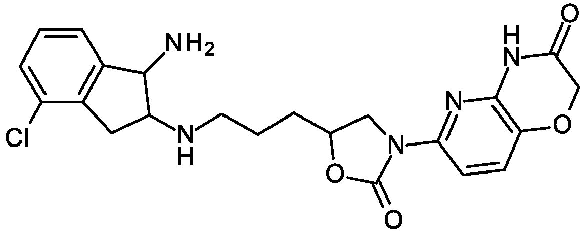 ESP [M+H]⁺: 458.1 | |
| **245** | 6-[2-oxo-5-[3-[[*rac*-1,2-*trans*-1-amino-4-chloro-2,3-dihydro-1*H*-inden-2-yl]amino]propyl]-1,3-oxazolidin-3-yl]-4*H-*pyrazino[2,3-b][1,4]oxazin-3-one | Intermediate 154 and Intermediate 176 |
| | 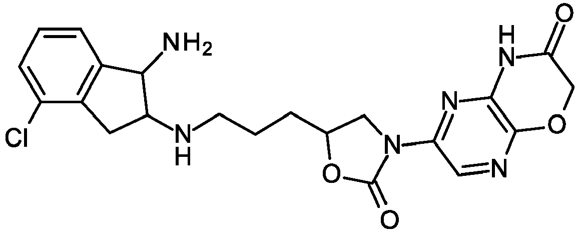 ESP [M+H]⁺: 459.1 | |
| **246** | 5-[2-[(*rac*-1,2-*trans*-1-amino-4-chloro-2,3-dihydro-1*H*-inden-2-yl)methylamino]ethyl]-3-(3-oxo-4*H*-pyrido[3,2-b][1,4]thiazin-6-yl)-1,3-oxazolidin-2-one | Intermediate 48 and Intermediate 92 |
| | 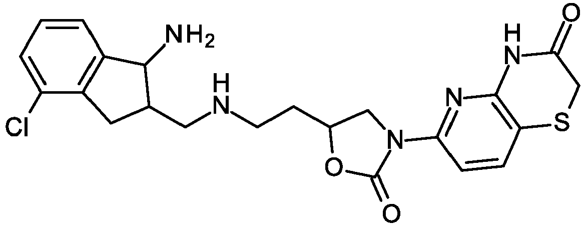 ESP [M+H]⁺: 474.3 | |
| 247 | *rac-*1,2-*trans*-1-amino-2-[[2-[2-oxo-3-(3-oxo-4*H*-pyrido[3,2-b][1,4]thiazin-6-yl)-1,3-oxazolidin-5-yl]ethylamino]methyl]-2,3-dihydro-1*H*-indene-4-carbonitrile | Intermediate 48 and Intermediate 88 |
| | 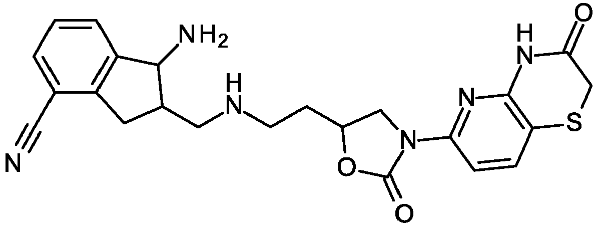 ESP [M+H]⁺: 465.1 | |
| **248** | 6-[5-[2-[[6-(2-amino-3-methoxypropoxy)-4-fluoro-2,3-dihydro-1*H*-inden-2-yl]methylamino]ethyl]-2-oxo-1,3-oxazolidin-3-yl]-4*H*-pyrido[3,2-b][1,4]oxazin-3-one | Intermediate 1 and Intermediate 180 |
| | 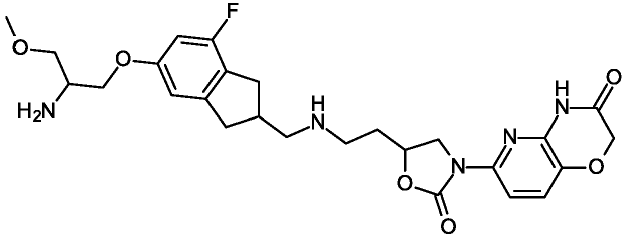 ESP [M+H]⁺: 530.2 | |

### Example 249

### 2-[[rac-5,6-trans-6-(Aminomethyl)-2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-indene-4-carbonitrile; formic acid

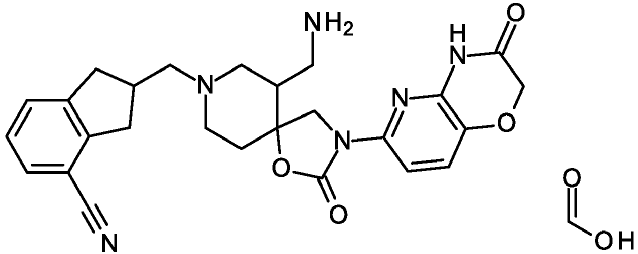

To a mixture of 6-[*rac*-5,6-*trans*-6-(azidomethyl)-2-oxo-1-oxa-3,8-diazaspiro[4.5]decan-3-yl]-4*H*-pyrido[3,2-b][1,4]oxazin-3-one hydrochloride (Intermediate 177, 40.0 mg, 0.100 mmol, 1 eq) in methanol (5.19 mL) was added Et₃N (one drop) and the mixture stirred at 15 °C for 5 min., then 2-formyl-2,3-dihydro-1*H*-indene-4-carbonitrile (Intermediate 5, 34.6 mg, 0.200 mmol, 2 eq) and sodium cyanoborohydride (19.05 mg, 0.300 mmol, 3 eq) were added. The mixture was stirred at 15 °C for another 16 h. The mixture was filtered and the filtrate was concentrated under vacuum to give a residue. The residue was purified by prep-TLC (PE/EtOAc=1/3) to give 2-[[*rac*-5,6-*trans*-6-(azidomethyl)-2-oxo-3-(3-oxo-4*H*-pyrido[3,2-b][1,4]oxazin-6-yl)-1-oxa-3,8-diazaspiro[4.5]decan-8-yl]methyl]-2,3-dihydro-1*H*-indene-4-carbonitrile (40 mg, 0.080 mmol, 79.8 % yield) as a white solid. A mixture of this material and Pd/C (20.0 mg) in THF (4 mL) under H₂ balloon was stirred at 30 °C for 4 h. The mixture was filtered and the filtrate was concentrated under vacuum to give the crude product. The crude product was purified by prep-HPLC (formic acid system) to give the title compound (4.7 mg, 0.010 mmol, 12.3 % yield) as a white solid. MS (ESP): m/z = 489.2 [M+H]⁺.

### Example 250

### 6-[rac-5,6-trans-6-(Aminomethyl)-8-[(4-fluoro-l-methyl-6,7-dihydro-5H-cyclopenta[c]pyridin-6-yl)methyl]-2-oxo-1-oxa-3,8-diazaspiro[4.5]decan-3-yl]-4H-pyrido[3,2-b][1,4]oxazin-3-one

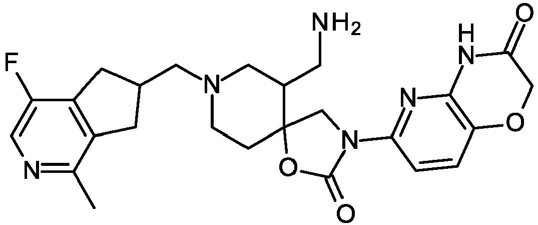

To a solution of 6-[*rac*-5,6-*trans*-6-(azidomethyl)-2-oxo-1-oxa-3,8-diazaspiro[4.5]decan-3-yl]-4*H*-pyrido[3,2-b][1,4]oxazin-3-one hydrochloride (Intermediate 177, 35.0 mg, 0.090 mmol, 1 eq) in methanol (3 mL) was added Et₃N (one drop) and the mixture stirred for 5 min. 4-Fluoro-1-methyl-6,7-dihydro-5*H*-cyclopenta[c]pyridine-6-carbaldehyde (Intermediate 27, 31.7 mg, 0.180 mmol, 2 eq) and sodium cyanoborohydride (16.7 mg, 0.270 mmol, 3 eq) were added and the reaction mixture was stirred at 15 °C for 16 h. The mixture was concentrated under vacuum to give a residue. The residue was purified by prep-TLC (DCM/CH₃OH=10/1) to give 6-[*rac*-5,6-*trans*-6-(azidomethyl)-8-[(4-fluoro-1-methyl-6,7-dihydro-5*H*-cyclopenta[c]pyridin-6-yl)methyl]-2-oxo-1-oxa-3,8-diazaspiro[4.5]decan-3-yl]-4*H*-pyrido[3,2-b][1,4]oxazin-3-one (40 mg, 0.080 mmol, 86.6 % yield) as a white solid. To this material in THF (4 mL) was added trimethylphosphine in THF (0.15 mL, 0.150 mmol, 2 eq) and then the reaction was stirred at 20 °C for 1h. After 1 h, water (2 mL) was added and then then the reaction was stirred for another 2 h. The mixture was filtered and concentrated under vacuum to give the residue. The residue was purified by prep-HPLC (neutral system) and then freeze-dried to give the title compound (6.8 mg, 0.010 mmol, 15.9 % yield) as a white solid. MS (ESP): m/z = 497.3 [M+H]⁺.

### Example 251

### 6-[rac-5,6-cis-6-(Aminomethyl)-8-[(4-fluoro-1-methyl-6,7-dihydro-5H-cyclopenta[c]pyridin-6-yl)methyl]-2-oxo-1-oxa-3,8-diazaspiro[4.5]decan-3-yl]-4H-pyrido[3,2-b][1,4]oxazin-3-one

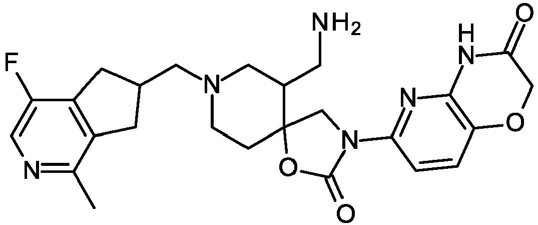

The title compound was prepared from 4-fluoro-1-methyl-6,7-dihydro-5*H-*cyclopenta[c]pyridine-6-carbaldehyde (Intermediate 27) and 6-[*rac*-5,6-*cis*-6-(azidomethyl)-2-oxo-1-oxa-3,8-diazaspiro[4.5]decan-3-yl]-4*H*-pyrido[3,2-b][1,4]oxazin-3-one hydrochloride (Intermediate 178) by analogy with Example 249 and was obtained as a white solid. MS (ESP): m/z = 497.3 [M+H]⁺.

### Example 252

### 2-[[rac-5,6-trans-6-(Aminomethyl)-2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-indene-4-carbonitrile; formic acid

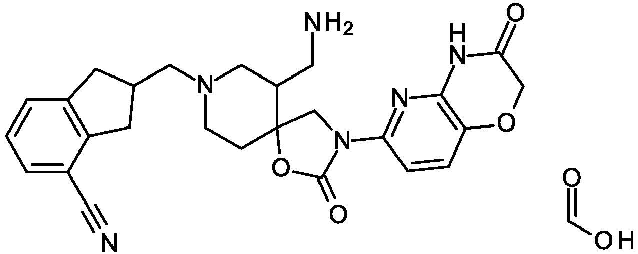

The title compound was prepared from 2-formyl-2,3-dihydro-1*H*-indene-4-carbonitrile (Intermediate 5) and 6-[*rac*-5,6-*cis*-6-(azidomethyl)-2-oxo-1-oxa-3,8-diazaspiro[4.5]decan-3-yl]-4*H*-pyrido[3,2-b][1,4]oxazin-3-one hydrochloride (Intermediate 178) by analogy with Example 250 and was obtained as a white solid.MS (ESP): m/z = 489.3 [M+H]⁺.

### Example 253

### 6-[5-[2-[[5-[(1-Aminocyclopropyl)methoxy]-4,7-dimethyl-2,3-dihydro-1H-inden-2-yl]methylamino]ethyl]-2-oxo-1,3-oxazolidin-3-yl]-4H-pyrazino[2,3-b][1,4]oxazin-3-one; formic acid

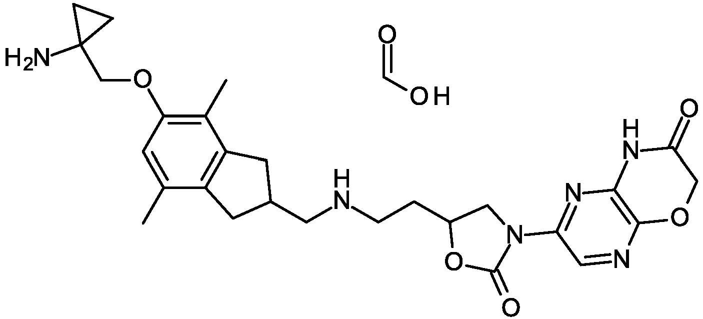

A solution of *tert-butyl N*-[1-[(2-formyl-4,7-dimethyl-2,3-dihydro-1*H*-inden-5-yl)oxymethyl]cyclopropyl]carbamate (Intermediate 179, 72.0 mg, 200 µmol, 1 eq), 6-[5-(2-aminoethyl)-2-oxo-1,3-oxazolidin-3-yl]-4*H*-pyrazino[2,3-b][1,4]oxazin-3-one; 2,2,2-trifluoroacetic acid (Intermediate 24, 78.8 mg, 200 µmol, 1 eq), acetic acid (18 mg, 17.2 µL, 300 µmol, 1.5 eq) and sodium triacetoxyborohydride (63.7 mg, 300 µmol, 1.5 eq) in tetrahydrofuran (3.5 mL) and methanol (0.5 mL) was stirred at RT for 18 h. The reaction mixture was treated with sat. aq. NaHCO₃ (5 mL) and extracted with EtOAc (2 × 10 mL). The organic layers were dried (Na₂SO₄) and evaporated. The crude material was purified by flash chromatography (silica gel, 5 g; 2% to 10% MeOH in DCM) to give tert-*butyl N*-[1-[[4,7-dimethyl-2-[[2-[2-oxo-3-(3-oxo-4*H*-pyrazino[2,3-b] [1,4]oxazin-6-yl)-1,3-oxazolidin-5-yl]ethylamino]methyl]-2,3-dihydro-1*H*-inden-5-yl]oxymethyl]cyclopropyl]carbamate (40 mg, 28 % yield) as a light brown waxy solid. A mixture of this material and formic acid (591 mg, 493 µL, 12.8 mmol) was stirred at RT for 3 h. The reaction mixture was evaporated. The crude product was dissolved in MeCN (0.5 mL) and H₂O (5 mL) and lyophilised overnight to give the title compound (30 mg, 80.5 % yield) as a white powder. MS (ESP): m/z = 523.3 [M+H]⁺.

### Example 254

### 2-[[2-[2-Oxo-3-(3-oxo-4H-pyrido[3,2-b] [1,4]oxazin-6-yl)-1,3-oxazolidin-5-yl]ethylamino]methyl]-6-pyrrolidin-3-yloxy-2,3-dihydro-1H-indene-4-carbonitrile; formic acid

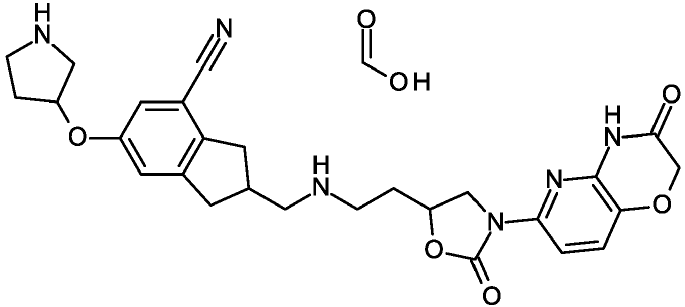

The title compound was prepared from *tert*-butyl 3-[(7-cyano-2-formyl-2,3-dihydro-1*H*-inden-5-yl)oxy]pyrrolidine-1-carboxylate (Intermediate 182) and 6-[5-(2-aminoethyl)-2-oxo-1,3-oxazolidin-3-yl]-4*H*-pyrido[3,2-b] [1,4]oxazin-3-one (Intermediate 1) by analogy with Example 253 and was obtained as a white solid. MS (ESN): m/z = 517.4 [M-H]⁻.

### Example 255

### 2-Amino-3-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylpropanamide; formic acid

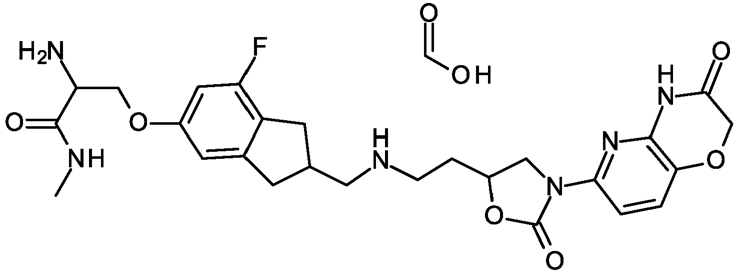

To a mixture of *tert*-butyl N-[3-[(7-fluoro-2-formyl-2,3-dihydro-1*H*-inden-5-yl)oxy]-1-(methylamino)-1-oxopropan-2-yl]carbamate (Intermediate 184, 20.5 mg, 0.050 mmol, 1 eq) and 6-[5-(2-aminoethyl)-2-oxo-1,3-oxazolidin-3-yl]-4*H*-pyrido[3,2-b] [1,4]oxazin-3-one (Intermediate 1, 15.0 mg, 0.050 mmol, 1 eq) in methanol (2 mL) was added sodium cyanoborohydride (10.16 mg, 0.160 mmol, 3 eq) then the reaction was stirred at 20 °C for 5 h. The mixture was diluted with DCM (30 mL) and washed with brine (10 mL × 2), then dried over Na₂SO₄ and concentrated under vacuum to give *tert*-butyl *N*-[3-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H-*inden-5-yl]oxy]-1-(methylamino)-1-oxopropan-2-yl]carbamate (18 mg, 0.030 mmol, 52 % yield) as a white solid. To a mixture of this material in methanol (1 mL) was added HCl/CH₃OH (1.0 mL) and the RM stirred at 20 °C for 2 h. The mixture was concentrated under vacuum. The crude product was purified by prep-HPLC (formic acid system) to give the title compound (6.7 mg, 0.010 mmol, 33.9 % yield) as a white waxy solid. MS (ESP): m/z = 543.3 [M+H]⁺.

### Example 256

### 6-[5-[2-[(1-Chloro-4-fluoro-3-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one

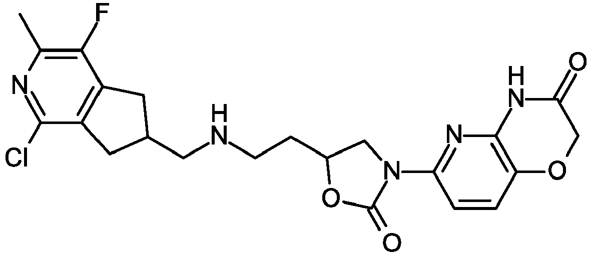

The title compound was prepared from (1-chloro-4-fluoro-3-methyl-6,7-dihydro-5*H-*cyclopenta[c]pyridin-6-yl)methyl 4-methylbenzenesulfonate (Intermediate 185) and 6-[5-(2-aminoethyl)-2-oxo-1,3-oxazolidin-3-yl]-4*H*-pyrido[3,2-b][1,4]oxazin-3-one (Intermediate 1) by analogy with 6-[5-[2-[(4-chloro-2-hydroxy-1,3-dihydroinden-2-yl)methylamino]ethyl]-2-oxo-1,3-oxazolidin-3-yl]-4*H*-pyrido[3,2-b][1,4]oxazin-3-one; formic acid (Example 232) and was obtained as a light yellow solid. MS (ESP): m/z = 476.3 [M+H]⁺.

### Example 257

### 6-[8-[[6-[(1-Aminocyclopropyl)methoxy]-4-fluoro-2,3-dihydro-1H-inden-2-y1]methyl]-2-oxo-1-oxa-3,8-diazaspiro[4.5]decan-3-yl]-4H-pyrazino[2,3-b][1,4]oxazin-3-one; formic acid

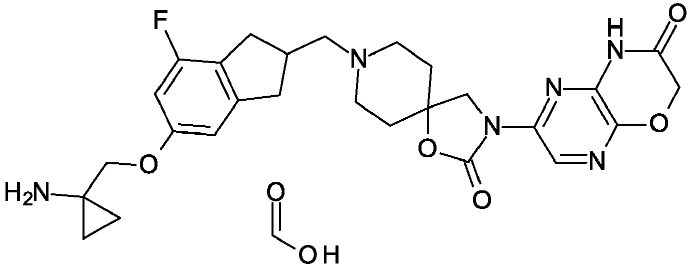

The title compound was prepared from 6-(2-oxo-1-oxa-3,8-diazaspiro[4.5]decan-3-yl)-4*H-*pyrazino[2,3-b][1,4]oxazin-3-one; formic acid (Intermediate 186) and *tert*-butyl *N*-[1-[(7-fluoro-2-formyl-2,3-dihydro-1*H*-inden-5-yl)oxymethyl]cyclopropyl]carbamate (Intermediate 109) by analogy with 6-[5-[2-[[3-(2-aminoethoxy)-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4] oxazin-3 -one (Example 171) and was obtained as a white solid. MS (ESP): m/z = 539.5 [M+H]⁺.

### Example 258

### 6-[8-[(4-Fluoro-1-methyl-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-one

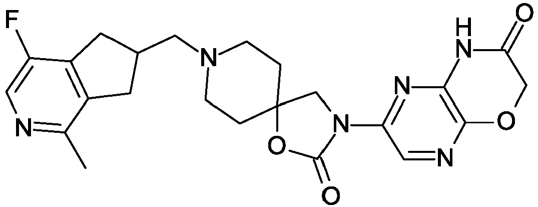

The title compound was prepared from 6-(2-oxo-1-oxa-3,8-diazaspiro[4.5]decan-3-yl)-4*H-*pyrazino[2,3-b][1,4]oxazin-3-one; formic acid (Intermediate 186) and 4-fluoro-1-methyl-6,7-dihydro-5*H*-cyclopenta[c]pyridine-6-carbaldehyde (Intermediate 27) by analogy with 6-[5-[2-[(4-chloro-2,3-dihydro-1*H*-inden-2-yl)methylamino]ethyl]-2-oxo-1,3-oxazolidin-3-yl]-4*H-*pyrido[3,2-b][1,4]oxazin-3-one; formic acid (Example 1) and was obtained as a white solid. MS (ESP): m/z = 469.3 [M+H]⁺.

### Example 259

### 6-[2-Oxo-8-[[rac-1,2-trans-1-amino-4-chloro-2,3-dihydro-1H-inden-2-yl]methyl]-1-oxa-3,8-diazaspiro[4.5]decan-3-yl]-4H-pyrazino[2,3-b][1,4]oxazin-3-one; formic acid

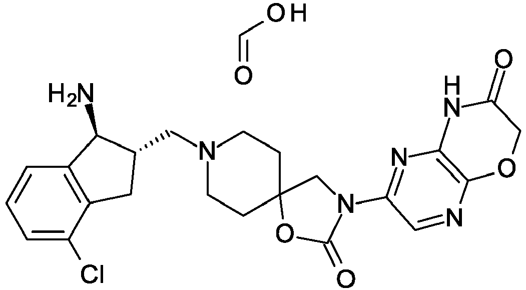

The title compound was prepared from 6-(2-oxo-1-oxa-3,8-diazaspiro[4.5]decan-3-yl)-4*H-*pyrazino[2,3-b][1,4]oxazin-3-one; formic acid (Intermediate 186) and *tert*-butyl *N*-(*rac*-1,2-trans-4-chloro-2-formyl-2,3-dihydro-1*H*-inden-1-yl)carbamate (Intermediate 92) by analogy with 6-[5-[2-[[3-(2-aminoethoxy)-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3 -one (Example 171) and was obtained as a white solid. MS (ESP): m/z = 485.2 [M+H]⁺.

### Example 260

### 6-[8-[[6-(2-Aminopropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one; formic acid

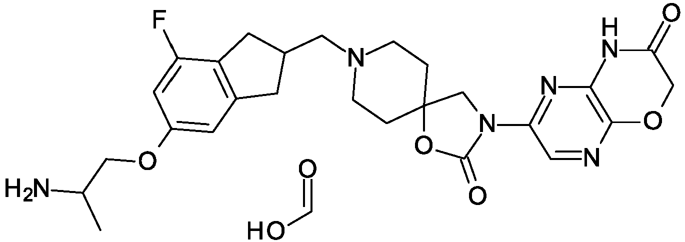

The title compound was prepared from 6-(2-oxo-1-oxa-3,8-diazaspiro[4.5]decan-3-yl)-4*H-*pyrazino[2,3-b][1,4]oxazin-3-one; formic acid (Intermediate 186) and *tert*-butylN-[1-[(7-fluoro-2-formyl-2,3-dihydro-1*H*-inden-5-yl)oxy]propan-2-yl]carbamate (Intermediate 105) by analogy with 6-[5-[2-[[3-(2-aminoethoxy)-1,4-dimethyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one (Example 171) and was obtained as a white solid. MS (ESP): m/z = 527.2 [M+H]⁺.

### Example 261

### 2-[[2-Oxo-3-(3-oxo-4H-pyrazino[2,3-b][1,4]oxazin-6-yl)-1-oxa-3,8-diazaspiro[4.5]decan-8-yl]methyl]-2,3-dihydro-1H-indene-4-carbonitrile; formic acid

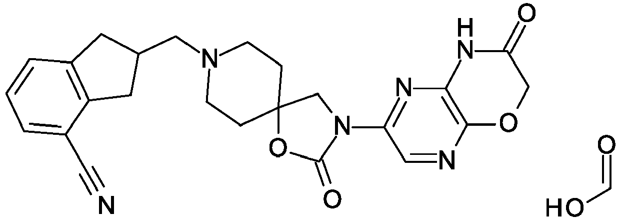

The title compound was prepared from 6-(2-oxo-1-oxa-3,8-diazaspiro[4.5]decan-3-yl)-4*H-*pyrazino[2,3-b][1,4]oxazin-3-one; formic acid (Intermediate 186) and 2-formyl-2,3-dihydro-1*H-*indene-4-carbonitrile (Intermediate 5) by analogy with 6-[5-[2-[(4-chloro-2,3-dihydro-1*H*-inden-2-yl)methylamino]ethyl]-2-oxo-1,3-oxazolidin-3-yl]-4*H*-pyrido[3,2-b][1,4]oxazin-3-one; formic acid (Example 1) and was obtained as a white solid. MS (ESP): m/z = 461.1 [M+H]⁺.

### Example 262

### 6-[5-[2-[(rac-5,6-trans-5-Amino-4-chloro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one; formic acid

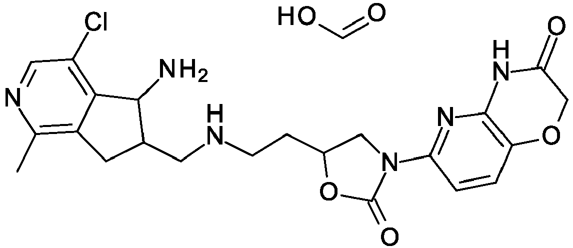

The title compound was prepared from 6-[5-(2-aminoethyl)-2-oxo-1,3-oxazolidin-3-yl]-4H-pyrido[3,2-b][1,4]oxazin-3-one (Intermediate 1) and *rac*-5,6-*trans*-5-azido-4-chloro-1-methyl-6,7-dihydro-5*H*-cyclopenta[c]pyridine-6-carbaldehyde (Intermediate 187) by analogy with 6-[*rac*-5,6-*trans*-6-(aminomethyl)-8-[(4-fluoro-1-methyl-6,7-dihydro-5*H*-cyclopenta[c]pyridin-6-yl)methyl]-2-oxo-1-oxa-3,8-diazaspiro[4.5]decan-3-yl]-4*H*-pyrido[3,2-b][1,4]oxazin-3-one (Example 250) and was obtained as a white solid. MS (ESP): m/z = 473.2 [M+H]⁺.

### Example 263

### 6-[2-oxo-5-[2-[[rac-5,6-trans-5-amino-4-chloro-1-methyl-6,7-dihydro-5H-cyclopenta[c]pyridin-6-yl]methylamino]ethyl]-1,3-oxazolidin-3-yl]-4H-pyrazino[2,3-b][1,4]oxazin-3-one

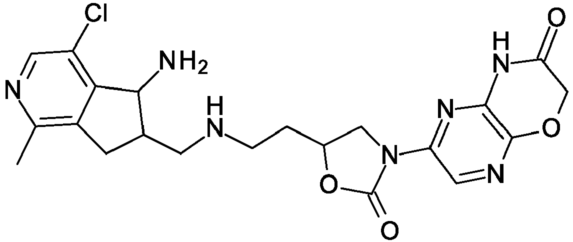

The title compound was prepared by analogy with 6-[5-[2-[(*rac-*5,6-*trans*-5-amino-4-chloro-1-methyl-6,7-dihydro-5*H*-cyclopenta[c]pyridin-6-yl)methylamino]ethyl]-2-oxo-1,3-oxazolidin-3-yl]-4*H*-pyrido[3,2-b][1,4]oxazin-3-one; formic acid (Example 262) using 6-[5-(2-aminoethyl)-2-oxo-1,3-oxazolidin-3-yl]-4*H*-pyrazino[2,3-b][1,4]oxazin-3-one (Intermediate 24) in place of 6-[5-(2-aminoethyl)-2-oxo-1,3-oxazolidin-3-yl]-4H-pyrido[3,2-b][1,4]oxazin-3 -one and was obtained as a white solid. MS (ESP): m/z = 474.2 [M+H]⁺.

### Example 264

### 3-(3-Oxo-4H-pyrido[3,2-b][1,4]thiazin-6-yl)-5-[2-[[rac-5,6-trans-5-amino-4-chloro-1-methyl-6,7-dihydro-5H-cyclopenta[c]pyridin-6-yl]methylamino]ethyl]-1,3-oxazolidin-2-one

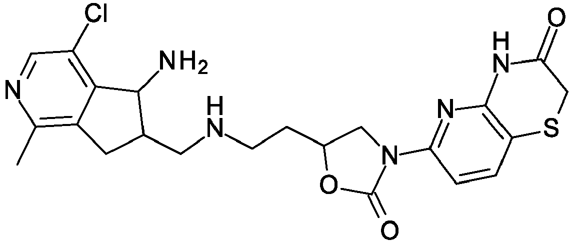

The title compound was prepared by analogy with 6-[5-[2-[(*rac*-5,6-*trans*-5-amino-4-chloro-1-methyl-6,7-dihydro-5*H*-cyclopenta[c]pyridin-6-yl)methylamino]ethyl]-2-oxo-1,3-oxazolidin-3-yl]-4*H*-pyrido[3,2-b][1,4]oxazin-3-one; formic acid (Example 262) using 5-(2-aminoethyl)-3-(3-oxo-4*H*-pyrido[3,2-b][1,4]thiazin-6-yl)-1,3-oxazolidin-2-one hydrochloride (Intermediate 48) in place of 6-[5-(2-aminoethyl)-2-oxo-1,3-oxazolidin-3-yl]-4H-pyrido[3,2-b][1,4]oxazin-3-one and was obtained as a white solid. MS (ESP): m/z = 489.2 [M+H]⁺.

### Example 265

### 6-[5-[[2-(4-Fluoro-1-methyl-6,7-dihydro-5H-cyclopenta[c]pyridin-6-yl)ethylamino]methyl]-2-oxo-1,3-oxazolidin-3-yl]-4H-pyrazino[2,3-b][1,4]oxazin-3-one; formic acid

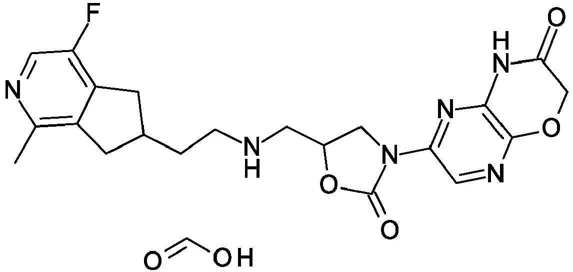

The title compound was prepared from 2-(4-fluoro-1-methyl-6,7-dihydro-5*H-*cyclopenta[c]pyridin-6-yl)acetaldehyde (Intermediate 188) and 6-[5-(aminomethyl)-2-oxo-1,3-oxazolidin-3-yl]-4*H*-pyrazino[2,3-b][1,4]oxazin-3-one; 2,2,2-trifluoroacetic acid (Intermediate 189) by analogy with 6-[5-[2-[(4-chloro-2,3-dihydro-1*H*-inden-2-yl)methylamino]ethyl]-2-oxo-1,3-oxazolidin-3-yl]-4*H*-pyrido[3,2-b][1,4]oxazin-3-one; formic acid (Example 1) and was obtained as a white solid. MS (ESP): m/z = 443.2 [M+H]⁺.

### Example 266

### 6-[2-Oxo-5-[[2-[rac-1,2-trans-1-amino-4-chloro-2,3-dihydro-1H-inden-2-yl]ethylamino]methyl]-1,3-oxazolidin-3-yl]-4H-pyrazino[2,3-b][1,4]oxazin-3-one

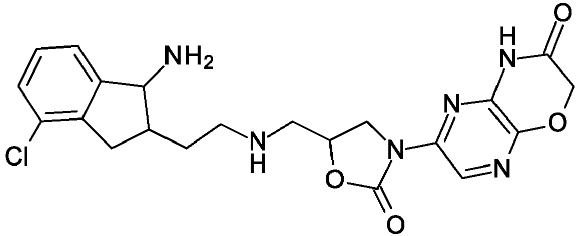

The title compound was prepared from 6-[5-(aminomethyl)-2-oxo-1,3-oxazolidin-3-yl]-4*H-*pyrazino[2,3-b][1,4]oxazin-3-one; 2,2,2-trifluoroacetic acid (Intermediate 189) and *tert*-butyl *N-*[*rac*-1,2-*trans*-4-chloro-2-(2-oxoethyl)-2,3-dihydro-1*H*-inden-1-yl]carbamate (Intermediate 133) by analogy with 6-[5-[2-[[3-(2-aminoethoxy)-1,4-dimethyl-6,7-dihydro-5*H-*cyclopenta[c]pyridin-6-yl]methylamino]ethyl]-2-oxo-1,3-oxazolidin-3-yl]-4*H*-pyrido[3,2-b][1,4]oxazin-3-one (Example 171) and was obtained as a light yellow solid. MS (ESP): m/z = 459.1 [M+H]⁺.

### Example 267

### 2-Amino-3-[[7-fluoro-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-inden-5-yl]oxy]propanoic acid; formic acid

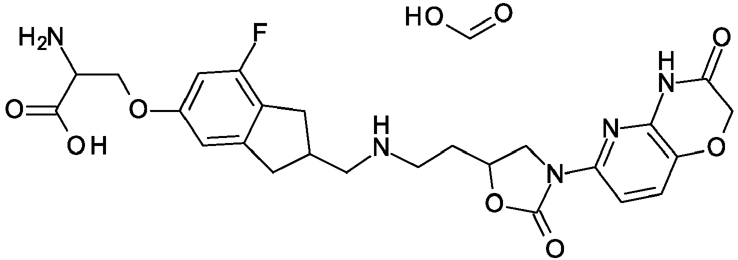

To a mixture of 3-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-2-[(2-methylpropan-2-yl)oxycarbonylamino]propanoic acid (Intermediate 190, 20.0 mg, 0.030 mmol, 1 eq) in DCM (4 mL) at 0 °C was added TFA (1.0 mL, 0.030 mmol, 1 eq), then the RM was stirred at 20 °C for 16 h. The mixture was concentrated under vacuum to give a residue. The residue was purified by Prep-HPLC (formic acid system) to give the title compound (1.7 mg, 9.3 % yield) as a white solid. MS (ESP): m/z = 530.3 [M+H]⁺.

### Example 268

### 6-[5-[2-[(1,4-Dimethyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one

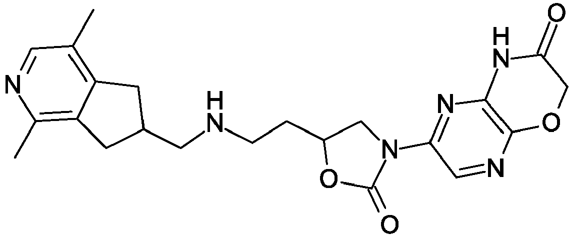

A solution of (1,4-dimethyl-6,7-dihydro-5*H*-cyclopenta[c]pyridin-6-yl)methyl 4-methylbenzenesulfonate (Intermediate 191, 71 mg, 214 µmol, 1 eq), 6-[5-(2-aminoethyl)-2-oxo-1,3-oxazolidin-3-yl]-4*H*-pyrazino[2,3-b][1,4]oxazin-3-one; 2,2,2-trifluoroacetic acid (Intermediate 24, 84.2 mg, 214 µmol, 1 eq), sodium iodide (321 mg, 2.14 mmol, 10 eq) and TEA (130 mg, 179 µL, 1.29 mmol, 6 eq) in DMF (1.5 mL) was heated to 90 °C for 2 h. The reaction mixture was poured into NaHCOs (2 mL) and was extracted with EtOAc (2 x 5 mL). The organic layers were combined, dried over Na₂SO₄ and evaporated. The crude material was purified by flash chromatography (silica gel, 5 g; 2% to 20% MeOH in DCM) to give a brown waxy solid. This material was purified again by preparative TLC (9:1 DCM/MeOH) to give the title compound (5.8 mg, 6 % yield) as a light brown waxy solid. MS (ESP): m/z = 439.3 [M+H]⁺.

### Example 269

### 6-[2-Oxo-5-[2-[(1,3,4-trimethyl-6,7-dihydro-5H-cyclopenta[c]pyridin-6-yl)methylamino]ethyl]-1,3-oxazolidin-3-yl]-4H-pyrazino[2,3-b] [1,4]oxazin-3-one

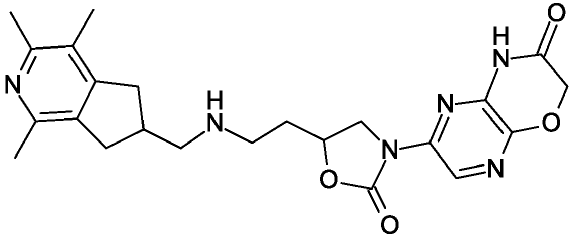

The title compound was prepared from 1,3,4-trimethyl-6,7-dihydro-5*H*-cyclopenta[c]pyridine-6-carbaldehyde (Intermediate 59) and 6-[5-(2-aminoethyl)-2-oxo-1,3-oxazolidin-3-yl]-4*H-*pyrazino[2,3-b][1,4]oxazin-3-one; 2,2,2-trifluoroacetic acid (Intermediate 24) by analogy with 6-[5-[2-[(1-chloro-6,7-dihydro-5*H*-cyclopenta[c]pyridin-6-yl)methylamino]ethyl]-2-oxo-1,3-oxazolidin-3-yl]-4*H*-pyrido[3,2-b][1,4]oxazin-3-one (Example 86) and was obtained as a colourless waxy solid. MS (ESP): m/z = 453.3 [M+H]⁺.

### Example 270

### 6-[5-[2-[(4-Methyl-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-one

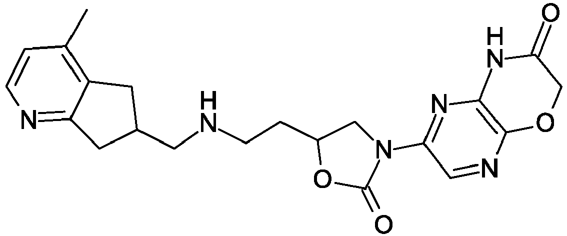

The title compound was prepared from 4-methyl-6,7-dihydro-5*H*-cyclopenta[b]pyridine-6-carbaldehyde (Intermediate 56) and 6-[5-(2-aminoethyl)-2-oxo-1,3-oxazolidin-3-yl]-4*H-*pyrazino[2,3-b][1,4]oxazin-3-one; 2,2,2-trifluoroacetic acid (Intermediate 24) by analogy with 6-[5-[2-[(1-chloro-6,7-dihydro-5*H*-cyclopenta[c]pyridin-6-yl)methylamino]ethyl]-2-oxo-1,3-oxazolidin-3-yl]-4*H*-pyrido[3,2-b] [1,4]oxazin-3-one (Example 86) and was obtained as a white solid. MS (ESP): m/z = 425.3 [M+H]⁺.

### Example 271

### 6-[5-[2-[[6-[2-(Dimethylamino)ethoxy]-4-fluoro-2,3-dihydro-1H-inden-2-yl]methylamino]ethyl]-2-oxo-1,3-oxazolidin-3-yl]-4H-pyrido[3,2-b][1,4]oxazin-3-one; formic acid

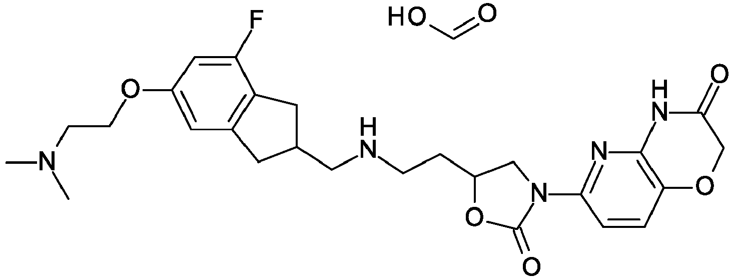

The title compound was prepared by analogy to 6-[5-[2-[(4-chloro-2,3-dihydro-1*H*-inden-2-yl)methylamino]ethyl]-2-oxo-1,3-oxazolidin-3-yl]-4*H*-pyrido[3,2-b][1,4]oxazin-3-one; formic acid (Example 1) using 6-[2-(dimethylamino)ethoxy]-4-fluoro-2,3-dihydro-1*H*-indene-2-carbaldehyde (Intermediate 192) in place of 4-chloro-2,3-dihydro-1*H*-indene-2-carbaldehyde and was obtained as a white solid. MS (ESP): m/z = 514.3 [M+H]⁺.

### Example 272

### 6-[5-[2-[[6-[2-(Dimethylamino)ethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one; formic acid

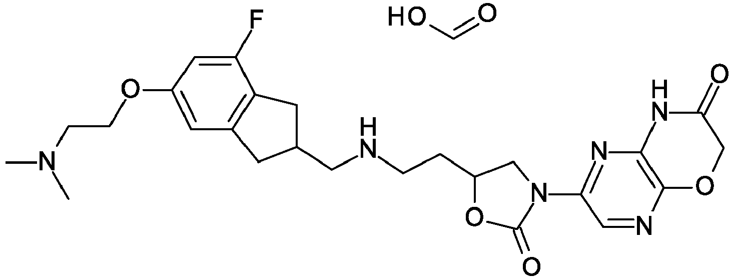

The title compound was prepared by analogy to 6-[5-[2-[[6-[2-(dimethylamino)ethoxy]-4-fluoro-2,3-dihydro-1*H*-inden-2-yl]methylamino]ethyl]-2-oxo-1,3-oxazolidin-3-yl]-4*H*-pyrido[3,2-b][1,4]oxazin-3-one; formic acid (Example 271) using 6-[5-(2-aminoethyl)-2-oxo-1,3-oxazolidin-3-yl]-4*H*-pyrazino[2,3-b][1,4]oxazin-3-one (Intermediate 24) in place of 6-[5-(2-aminoethyl)-2-oxo-1,3-oxazolidin-3-yl]-4*H*-pyrido[3,2-b][1,4]oxazin-3-one and was obtained as a white solid. MS (ESP): m/z = 515.3 [M+H]⁺.

### Example 273

### 6-[2-Oxo-5-[2-[[rac-1,2-trans-1-amino-6-[(1-aminocyclopropyl)methoxy]-4-chloro-2,3-dihydro-1H-inden-2-yl]methylamino]ethyl]-1,3-oxazolidin-3-yl]-4H-pyrido[3,2-b][1,4]oxazin-3-one; formic acid

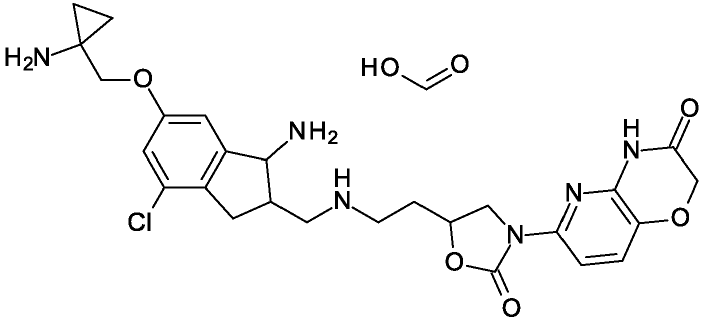

A mixture of [*rac*-1,2-*trans*-4-chloro-1-[(2-methylpropan-2-yl)oxycarbonylamino]-6-[[1-[(2-methylpropan-2-yl)oxycarbonylamino]cyclopropyl]methoxy]-2,3-dihydro-1*H*-inden-2-yl]methyl 4-methylbenzenesulfonate (Intermediate 193, 65 mg, 0.102 mmol, 1 eq), 6-[5-(2-aminoethyl)-2-oxo-1,3-oxazolidin-3-yl]-4H-pyrido[3,2-b][1,4]oxazin-3-one (Intermediate 1, 35.5 mg, 0.102 mmol, 1 eq), triethylamine (61.9 mg, 85.3 µl, 0.612 mmol, 6 eq) and sodium iodide (76.5 mg, 0.510 mmol, 5 eq) in DMA (0.8 mL) was stirred at 90 °C for 18 h. The mixture was cooled to RT, diluted with water (basic with NaHCOs) and extracted with EtOAc (3 x). The organic layers were washed with water (basic with NaHCO₃, 2 x) and brine (basic with NaHCO₃), dried over Na₂SO₄ and evaporated. The residue was purified by flash chromatography (10 g silica gel; DCM/MeOH 98:2 to 95:5) to give *tert*-butyl *N*-[*rac*-1,2-*trans*-4-chloro-6-[[1-[(2-methylpropan-2-yl)oxycarbonylamino]cyclopropyl]methoxy]-2-[[2-[2-oxo-3-(3-oxo-4*H*-pyrido[3,2-b] [1,4]oxazin-6-yl)-1,3-oxazolidin-5-yl]ethylamino]methyl]-2,3-dihydro-1H-inden-1-yl]carbamate (30 mg) as an off-white solid. A solution of this material in formic acid (800 µL) was stirred at RT for 3 h. The volatiles were removed by evaporation. Toluene was added and removed by evaporation five times. The residue was dissolved in water and lyophilized to give the title compound (18 mg, 30 % yield) as a white solid. MS (ESN): m/z = 541.4 [M-H]⁻.

### Example 274

### 6-[2-Oxo-8-[[rac-1,2-trans-1-amino-6-[(1-aminocyclopropyl)methoxy]-4-chloro-2,3-dihydro-1H-inden-2-yl]methyl]-1-oxa-3,8-diazaspiro[4.5]decan-3-yl]-4H-pyrido[3,2-b][1,4]oxazin-3-one; formic acid

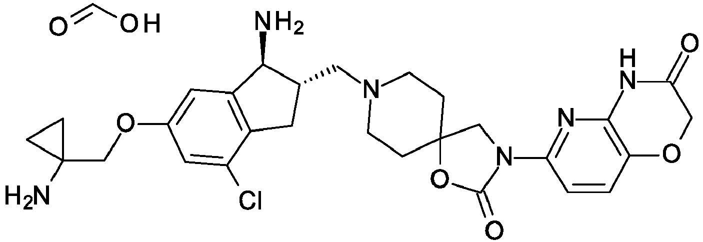

The title compound was prepared by analogy to 6-[2-oxo-5-[2-[[*rac*-1,2-*trans*-1-amino-6-[(1-aminocyclopropyl)methoxy]-4-chloro-2,3-dihydro-1*H*-inden-2-yl]methylamino]ethyl]-1,3-oxazolidin-3-yl]-4*H*-pyrido[3,2-b][1,4]oxazin-3-one; formic acid (Example 273) using 6-(2-oxo-1-oxa-3,8-diazaspiro[4.5]decan-3-yl)-4*H*-pyrido[3,2-b][1,4]oxazin-3-one (Intermediate 17) in place of 6-[5-(2-aminoethyl)-2-oxo-1,3-oxazolidin-3-yl]-4*H*-pyrido[3,2-b][1,4]oxazin-3-one and was obtained as a white solid. MS (ESN): m/z = 567.3 [M-H]⁻.

### Example 275

### 1-Ethyl-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylic acid

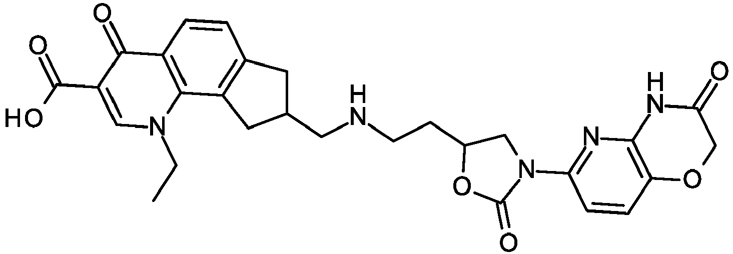

The title compound was prepared from ethyl 1-ethyl-4-oxo-8-[[2-[2-oxo-3-(3-oxo-4*H-*pyrido[3,2-b][1,4]oxazin-6-yl)-1,3-oxazolidin-5-yl]ethylamino]methyl]-8,9-dihydro-7*H-*cyclopenta[h]quinoline-3-carboxylate (Intermediate 194) by analogy with 1-ethyl-6-fluoro-4-oxo-8-[[2-[2-oxo-3-(3-oxo-4*H*-pyrido[3,2-b][1,4]oxazin-6-yl)-1,3-oxazolidin-5-yl]ethylamino]methyl]-8,9-dihydro-7*H*-cyclopenta[h]quinoline-3-carboxylic acid (Example 239) and was obtained as a white solid. MS (ESN): m/z = 546.4 [M-H]⁻.

### Example 276

### 1-Ethyl-4-oxo-8-[[2-[2-oxo-3-(3-oxo-4H-pyrazino[2,3-b][1,4]oxazin-6-yl)-1,3-oxazolidin-5-yl]ethylamino]methyl]-8,9-dihydro-7H-cyclopenta[h]quinoline-3-carboxylic acid

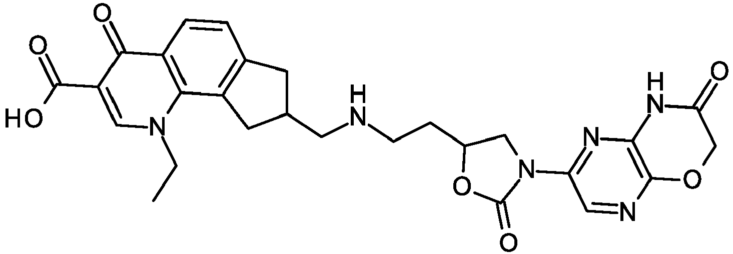

The title compound was prepared from ethyl 1-ethyl-4-oxo-8-[[2-[2-oxo-3-(3-oxo-4*H-*pyrazino[2,3-b][1,4]oxazin-6-yl)-1,3-oxazolidin-5-yl]ethylamino]methyl]-8,9-dihydro-7*H-*cyclopenta[h]quinoline-3-carboxylate (Intermediate 195) by analogy with 1-ethyl-6-fluoro-4-oxo-8-[[2-[2-oxo-3-(3-oxo-4*H*-pyrido[3,2-b][1,4]oxazin-6-yl)-1,3-oxazolidin-5-yl]ethylamino]methyl]-8,9-dihydro-7*H*-cyclopenta[h]quinoline-3-carboxylic acid (Example 239) and was obtained as a white solid. MS (ESN): m/z = 547.4 [M-H]⁻.

### Example 281

### 6-[5-[2-[(4-Chloro-2,3-dihydro-1H-inden-2-yl)methylamino]ethyl]-2-oxo-1,3-oxazolidin-3-yl]-4H-pyrazino[2,3-b][1,4]oxazin-3-one

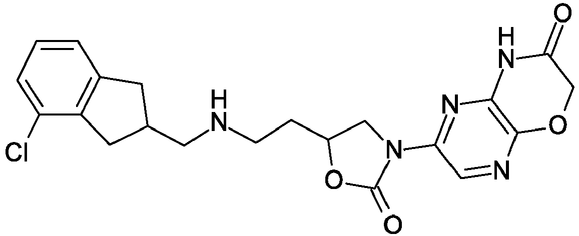

### Step 1:

### (4-Chloro-2,3-dihydro-1H-inden-2-yl)methyl methanesulfonate

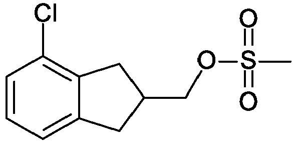

(4-Chloro-2,3-dihydro-1*H*-inden-2-yl)methanol (CAS# 1823383-27-2) (300 mg, 1.56 mmol, 1 eq) was dissoved in DCM (10 mL). Triethylamine (237 mg, 326 µL, 2.34 mmol, 1.5 eq) and methanesulfonyl chloride (214 mg, 146 µL, 1.87 mmol, 1.2 eq) were added and the mixture was left stirring overnight. The mixture was poured onto water and extracted twice with ethyl acetate. The combined organic layers were washed with water, dried over Na₂SO₄ and evaporated. The residue was purified by column chromatography on silica gel eluting with 0% to 30% ethyl acetate in heptane to give the title compound. (370 mg, 1.42 mmol, 90.9 % yield) as a brown oil. ¹H NMR (300 MHz, CDCl₃) δ ppm 2.74 - 3.00 (m, 3 H) 3.03 (s, 3 H) 3.11 - 3.28 (m, 2 H) 4.15 - 4.31 (m, 2 H) 7.02 - 7.20 (m, 3 H).

### Step 2:

### 6-[5-[2-[(4-Chloro-2,3-dihydro-1H-inden-2-yl)methylamino]ethyl]-2-oxo-1,3-oxazolidin-3-yl]-4H-pyrazino[2,3-b][1,4]oxazin-3-one

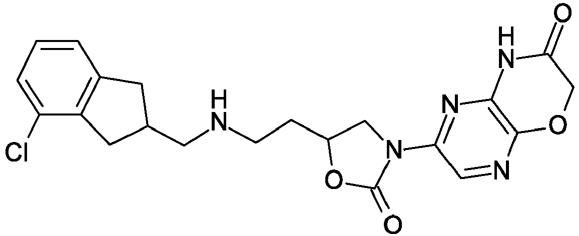

Intermediate 24 (50 mg, 127 µmol, 1 eq) and (4-chloro-2,3-dihydro-1*H*-inden-2-yl)methyl methanesulfonate (33.1 mg, 127 µmol, 1 eq) were suspended in acetonitrile (1 mL). Potassium carbonate (35 mg, 254 µmol, 2 eq) and sodium iodide (114 mg, 763 µmol, 6 eq) were added. This mixture was stirred at room temperature for 1 h. Then the mixture was heated to 60 °C for 2 h, after which the temperature was increased to 100 °C for 16.5 h. The reaction mixture was cooled to room temperature and formic acid (600 mg, 500 µl, 13 mmol, 103 eq) was added. After evaporation the title compound was obtained by preparative HPLC (3 mg, 7 µmol, 5.5 % yield) as a white solid. MS (ESP): m/z = 444.2 [M+H]⁺.

### Example 282

### 6-[2-Oxo-5-[2-[[rac-1,2-trans-1-amino-6-[(1-aminocyclopropyl)methoxy]-4-chloro-2,3-dihydro-1H-inden-2-yl]methylamino]ethyl]-1,3-oxazolidin-3-yl]-4H-pyrazino[2,3-b][1,4]oxazin-3-one;formic acid

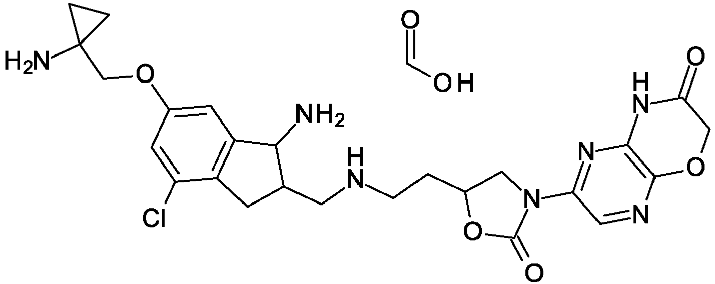

The title compound was prepared by analogy to Example 273 using Intermediate 24 in place of Intermediate 1 and was obtained as a light brown solid. MS (ESN): m/z = 542.3 [M-H]⁻.

### Example 297

### trans-6-[5-[2-[[4-Fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid

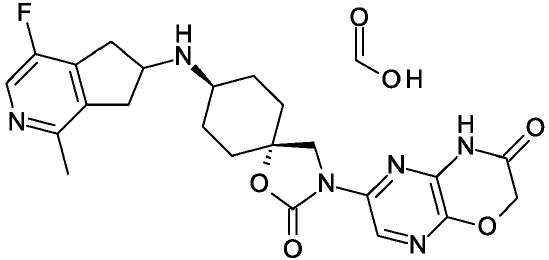

### Step 1:

### trans-6-[8-[(4-Fluoro-1-methyl-6,7-dihydro-5H-cyclopenta[c]pyridin-6-yl)amino]-2-oxo-1-oxa-3-azaspiro[4.5]decan-3-yl]-4-(2-trimethylsilylethoxymethyl)pyrazino[2,3-b][1,4]oxazin-3-one

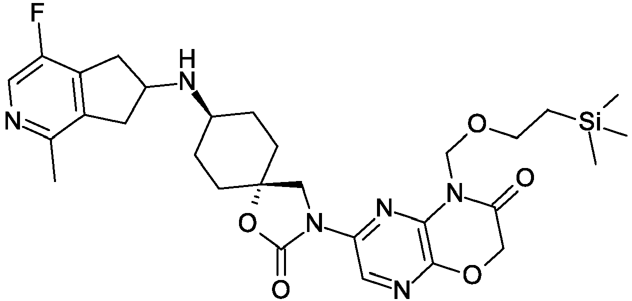

and
*cis*-6-[8-[(4-fluoro-1-methyl-6,7-dihydro-5*H*-cyclopenta[c]pyridin-6-yl)amino]-2-oxo-1-oxa-3-azaspiro[4.5]decan-3-yl]-4-(2-trimethylsilylethoxymethyl)pyrazino[2,3-b] [1,4]oxazin-3-one

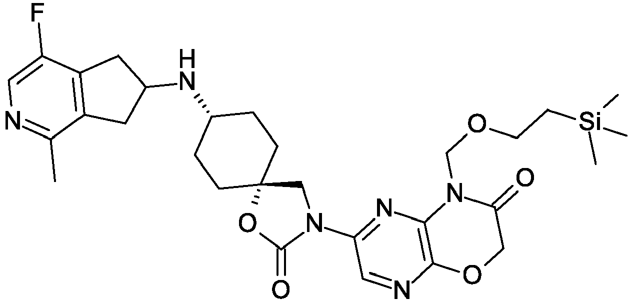

A mixture of 4-fluoro-1-methyl-6,7-dihydro-5*H*-cyclopenta[c]pyridin-6-amine (Intermediate 153, 70.0 mg, 0.420 mmol, 1 eq) and 3-[3-oxo-4-(2-trimethylsilylethoxymethyl)pyrazino[2,3-b][1,4]oxazin-6-yl]-1-oxa-3-azaspiro[4.5]decane-2,8-dione (Intermediate 208, 188.9 mg, 0.420 mmol, 1 eq) in THF (3 mL) and methanol (3 mL) was stirred at 25 °C for 1 h . After 1 h, to the mixture was added sodium cyanoborohydride (105.9 mg, 1.68 mmol, 4 eq) and 4Å molecular sieve (200 mg) at 0 °C. Then the solution was stirred at 50 °C for futher 16 h. The reaction solution was concentrated under vacuum to give the crude product which was purified by Prep-TLC (DCM/MeOH=10/1) to give *trans*-6-[8-[(4-fluoro-1-methyl-6,7-dihydro-5*H-*cyclopenta [c]pyridin-6-yl)amino]-2-oxo-1-oxa-3-azaspiro [4.5] decan-3-yl]-4-(2-trimethylsilyl ethoxymethyl)pyrazino[2,3-b][1,4]oxazin-3-one (40 mg, 0.070 mmol, 15.9% yield) as a yellow solid and cis-6-[8-[(4-fluoro-1-methyl-6,7-dihydro-5*H*-cyclopenta[c]pyridin-6-yl)amino]-2-oxo-1-oxa-3-azaspiro[4.5]decan-3-yl]-4-(2-trimethylsilylethoxymethyl)pyrazino[2,3-b] [1,4]oxazin-3-one (80 mg, 0.130 mmol, 31.7% yield) as a yellow solid. MS (ESP): m/z = 599.3 [M+H]⁺.

### Step 2:

### trans-6-[8-[(4-Fluoro-1-methyl-6,7-dihydro-5H-cyclopenta[c]pyridin-6-yl)amino]-2-oxo-1-oxa-3-azaspiro[4.5]decan-3-yl]-4H-pyrazino[2,3-b][1,4]oxazin-3-one; formic acid

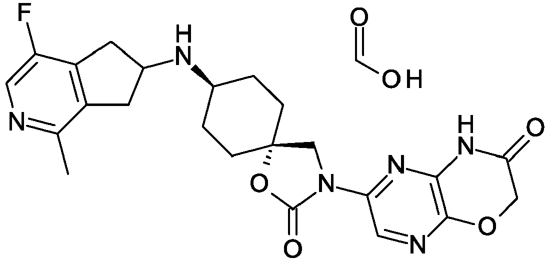

A mixture of *trans*-6-[8-[(4-fluoro-1-methyl-6,7-dihydro-5*H*-cyclopenta[c]pyridin-6-yl)amino]-2-oxo-1-oxa-3-azaspiro[4.5]decan-3-yl]-4-(2-trimethylsilylethoxymethyl)pyrazino[2,3-b] [1,4] oxazin-3-one (40.0 mg, 0.070 mmol, 1 eq) in TFA (0.5 mL) and DCM (2.5 mL) was stirred at 25 °C for 12 h. The reaction solution was concentrated under vacuum at 25 °C. The residue and potassium carbonate (33.27 mg, 0.24 mmol, 3 eq) in methanol (8 mL) was stirred at 25 °C for 4 h. The mixture was diluted with DMF (2.0 mL) and concentrated under reduced pressure at 25 °C. After methanol was removed, the solution was purified by prep-HPLC (formic acid system) and then freeze-dried to give the title compound (9 mg, 0.020 mmol, 21.5% yield) as a pink solid. MS (ESP): m/z = 469.2 [M+H]⁺. ¹H NMR (400 MHz, DMSO-*d*₆) δ 8.39 (d, *J* = 2.0 Hz, 1H), 8.23 (s, 1H), 8.14 (s, 1H), 4.85 (d, *J* = 2.1 Hz, 2H), 3.88 - 3.76 (m, 3H), 3.20 - 3.10 (m, 4H), 2.71 - 2.63 (m, 3H), 2.34 (s, 3H), 2.03 - 1.69 (m, 6H), 1.42 - 1.21 (m, 2H).

### Example 298

### cis-6-[5-[2-[[4-Fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid

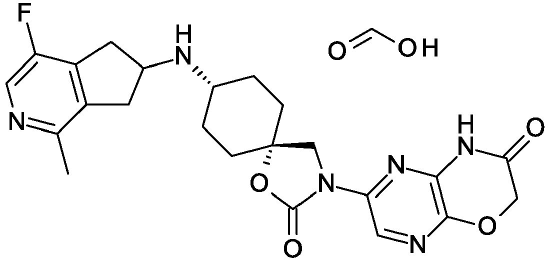

The title compound was prepared by analogy with *trans*-6-[5-[2-[[4-fluoro-6-[2-(1,2,4-triazol-1-yl)ethoxy]-2,3-dihydro-1*H*-inden-2-yl]methylamino]ethyl]-2-oxo-1,3-oxazolidin-3-yl]-4*H-*pyrazino[2,3-b][1,4]oxazin-3-one;formic acid (Example 297) using *cis*-6-[8-[(4-fluoro-1-methyl-6,7-dihydro-5*H*-cyclopenta[c]pyridin-6-yl)amino]-2-oxo-1-oxa-3-azaspiro[4.5]decan-3-yl]-4-(2-trimethylsilylethoxymethyl)pyrazino[2,3-b][1,4]oxazin-3-one in place of *trans*-6-[8-[(4-fluoro-1-methyl-6,7-dihydro-5*H*-cyclopenta[c]pyridin-6-yl)amino]-2-oxo-1-oxa-3-azaspiro[4.5]decan-3-yl]-4-(2-trimethylsilylethoxymethyl)pyrazino[2,3-b][1,4]oxazin-3-one and was obtained as a pink solid. MS (ESP): m/z = 469.3 [M+H]⁺. ¹H NMR (400 MHz, DMSO-*d*₆) δ 8.40 (s, 1H), 8.16 (d, *J* = 7.5 Hz, 2H), 4.85 (s, 2H), 3.87 - 3.77 (m, 3H), 3.20 - 3.09 (m, 4H), 2.76 - 2.66 (m, 3H), 2.35 (s, 3H), 2.04-2.01 (br d, *J* = 13.8 Hz, 2H), 1.85 - 1.82 (m, 2H), 1.75 - 1.68 (m, 2H), 1.46 - 1.37(m, 2H).

### Example 302

### 6-[5-[3-[(4-Fluoro-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid

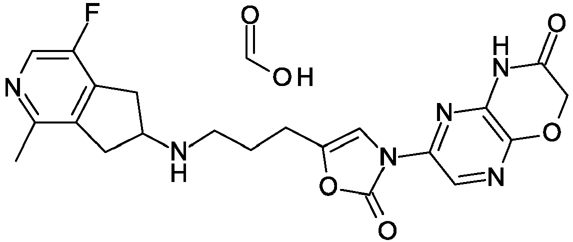

The title compound was prepared by analogy with *trans*-6-[5-[2-[[4-fluoro-6-[2-(1,2,4-triazol-1-yl)ethoxy]-2,3-dihydro-1*H*-inden-2-yl]methylamino]ethyl]-2-oxo-1,3-oxazolidin-3-yl]-4*H-*pyrazino[2,3-b][1,4]oxazin-3-one;formic acid (Example 297) using 3-[2-oxo-3-[3-oxo-4-(2-trimethylsilylethoxymethyl) pyrazino[2,3-b][1,4]oxazin-6-yl]oxazol-5-yl]propanal (Intermediate 210) in place of 3-[3-oxo-4-(2-trimethylsilylethoxymethyl)pyrazino[2,3-b][1,4]oxazin-6-yl]-1-oxa-3-azaspiro[4.5]decane-2,8-dione, and was obtained as a brown waxy solid. MS (ESP): m/z = 441.3 [M+H]⁺.

### Example 303

### 6-[5-[3-[(4-Fluoro-1-methyl-6,7-dihydro-5H-cyclopenta[c]pyridin-6-yl)-methylamino]propyl]-2-oxo-1,3-oxazol-3-yl]-4H-pyrazino[2,3-b][1,4]oxazin-3-one;formic acid

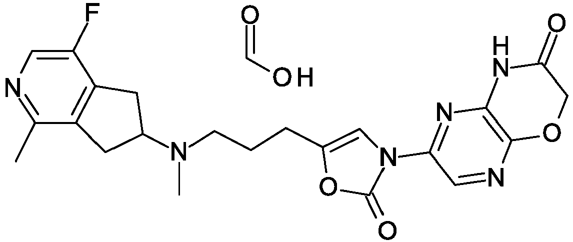

The title compound was isolated together with 6-[5-[3-[(4-fluoro-1-methyl-6,7-dihydro-5*H-*cyclopenta[c]pyridin-6-yl)amino]propyl]-2-oxo-1,3-oxazol-3-yl]-4*H*-pyrazino[2,3-b][1,4]oxazin-3-one;formic acid in Step 2 of the preparation of Example 302 and was obtained as a pink solid. MS (ESP): m/z = 455.3 [M+H]⁺.

### Example 311

### 2-[[7-Fluoro-2-[[2-[2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]-N-methoxyacetamide;formic acid

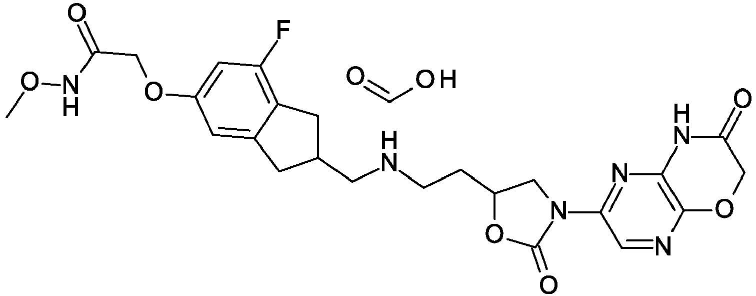

### Step 1:

### tert-Butyl N-[[4-fluoro-6-[2-(methoxyamino)-2-oxo-ethoxy]indan-2-yl]methyl]-N-[2-[2-oxo-3-(3-oxo-4H-pyrazino[2,3-b][1,4]oxazin-6-yl)oxazolidin-5-yl]ethyl]carbamate

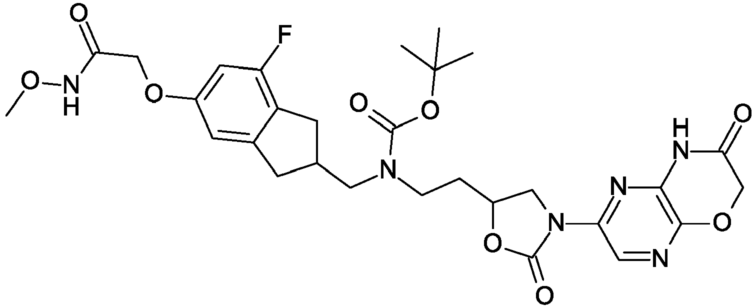

To a solution of 2-[2-[[*tert*-butoxycarbonyl-[2-[2-oxo-3-(3-oxo-4*H*-pyrazino[2,3-b][1,4]oxazin-6-yl)oxazolidin-5-yl]ethyl]amino]methyl]-7-fluoro-indan-5-yl]oxyacetic acid (Intermediate 215, 20.0 mg, 0.030 mmol, 1 eq) in DMF (0.500 mL) was added DIPEA (21.5 mg, 0.170 mmol, 5 eq) and HATU (19.0 mg, 0.050 mmol, 1.5 eq), then O-methylhydroxylamine (6.26 mg, 0.130 mmol, 4 eq) was added. The solution was stirred at 25 °C for 3 h. The solution was dried under reduced pressure to give the crude, which was purified by Prep-HPLC (formic acid system) and freeze-dried to give the title compound (20 mg, 0.030 mmol, 95.4% yield) as a white solid. MS (ESP): m/z = 575.1, [M+H-Bu]⁺.

### Step 2:

### 2-[[7-Fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methoxyacetamide;formic acid

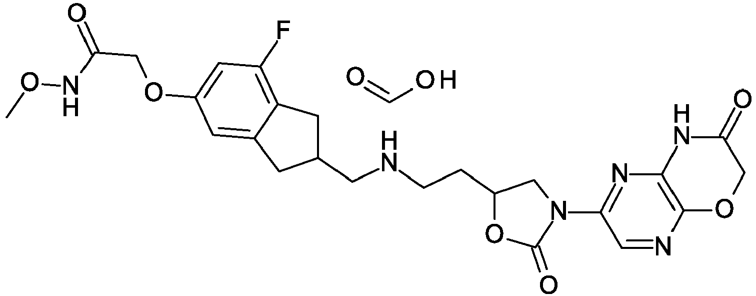

A solution of *tert*-butyl *N*-[[4-fluoro-6-[2-(methoxyamino)-2-oxo-ethoxy]indan-2-yl]methyl]-N-[2-[2-oxo-3-(3-oxo-4*H*-pyrazino[2,3-b][1,4]oxazin-6-yl)oxazolidin-5-yl]ethyl]carbamate (15.0 mg, 0.020 mmol, 1 eq) in formic acid (5.0 mL, 0.020 mmol, 1 eq) was stirred at 25 °C for 4 h. After 4 h, the solvent was evaporated to give the crude, to which was added water (20 mL) followed by freeze-drying to give the title compound (11.5 mg, 0.020 mmol, 79.7% yield) as a white solid. MS (ESP): m/z = 531.2 [M+H]⁺.

### Example 313

### 6-[2-Oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer A

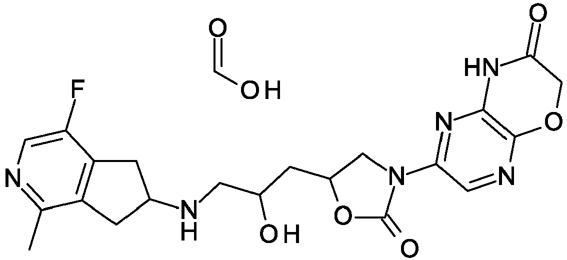

### Step 1:

### 5-[3-[(4-Fluoro-1-methyl-6,7-dihydro-5H-cyclopenta[c]pyridin-6-yl)amino]-2-hydroxy-propyl]-2-oxo-oxazolidin-3-yl]-4-(2-trimethylsilylethoxymethyl)pyrazino[2,3-b][1,4]oxazin-3-one, Isomer A

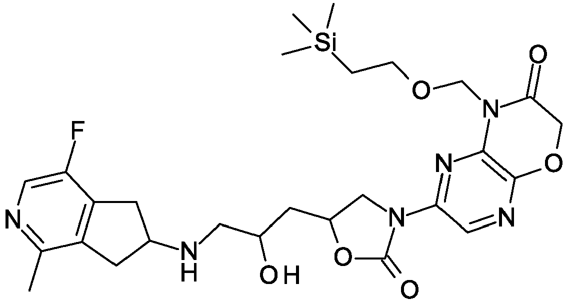

To a solution of 6-[5-(oxiran-2-ylmethyl)-2-oxo-oxazolidin-3-yl]-4-(2-trimethylsilyl ethoxymethyl)pyrazino[2,3-b][1,4]oxazin-3-one, Isomer A (Intermediate 218, 40.0 mg, 0.090 mmol, 1 eq) and 4-fluoro-1-methyl-6,7-dihydro-5H-cyclopenta[c]pyridin-6-amine (Intermediate 153, 15.7 mg, 0.090 mmol, 1 eq) in acetonitrile (1 mL) was added LiClO₄ (30.1 mg, 0.280 mmol, 3 eq). The mixture was stirred at 50 °C for 16 h. The mixture was filtered and purified by prep-HPLC (formic acid system) and evaporated to give the title compound (15 mg, 0.030 mmol, 26.9% yield) as a dark brown oil. MS (ESP): m/z = 589.4 [M+H]⁺.

### Step 2:

### 3-[(4-Fluoro-1-methyl-6,7-dihydro-5H-cyclopenta[c]pyridin-6-yl)amino]-2-hydroxy-propyl]-2-oxo-oxazolidin-3-yl]-4H-pyrazino[2,3-b][1,4]oxazin-3-one; formic acid, Isomer A

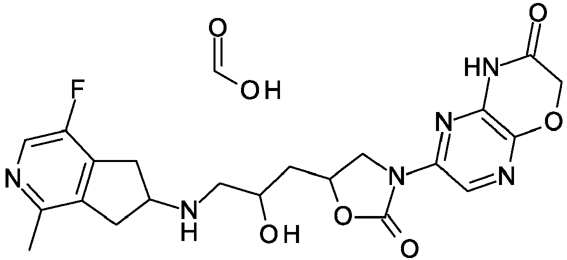

A solution of 5-[3-[(4-fluoro-1-methyl-6,7-dihydro-5*H*-cyclopenta[c]pyridin-6-yl)amino]-2-hydroxy-propyl]-2-oxo-oxazolidin-3-yl]-4-(2-trimethylsilylethoxymethyl)pyrazino[2,3-b][1,4]oxazin-3-one, Isomer A (15.0 mg, 0.030 mmol, 1 eq) in HCOOH (1.0 mL) was stirred at 40 °C for 16 h, then evaporated under high vacuum to give a residue. Methanol (1.0 mL) and K₂CO₃ (7.04 mg, 0.050 mmol, 2 eq) were added, the mixture was stirred at 25°C for another 2 h. The crude was purified by prep-HPLC (NH₄HCO₃ system), then freeze-dried to give the title compound (2.5 mg, 0.005 mmol, 19.3% yield) as a white solid. MS (ESP): m/z = 459.1 [M+H]⁺.

### Example 314

### 6-[2-Oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer B

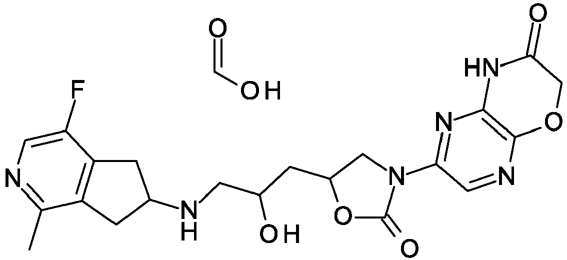

The title compound was prepared from 6-[5-(oxiran-2-ylmethyl)-2-oxo-oxazolidin-3-yl]-4-(2-trimethylsilyl ethoxymethyl)pyrazino[2,3-b][1,4]oxazin-3-one, Isomer B (Intermediate 219) by analogy with 5-[3-[(4-fluoro-1-methyl-6,7-dihydro-5*H*-cyclopenta[c]pyridin-6-yl)amino]-2-hydroxy-propyl]-2-oxo-oxazolidin-3-yl]-4-(2-trimethylsilylethoxymethyl)pyrazino[2,3-b][1,4]oxazin-3-one, Isomer A (Example 313) and was obtained as a white solid. MS (ESP): m/z = 459.0 [M+H]⁺.

### Example 315

### 6-[2-Oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer C

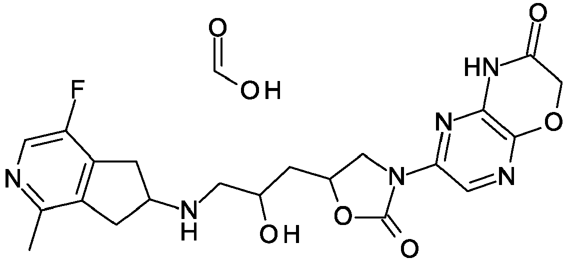

The title compound was prepared from 6-[5-(oxiran-2-ylmethyl)-2-oxo-oxazolidin-3-yl]-4-(2-trimethylsilyl ethoxymethyl)pyrazino[2,3-b][1,4]oxazin-3-one, Isomer C (Intermediate 220) by analogy with 5-[3-[(4-fluoro-1-methyl-6,7-dihydro-5*H*-cyclopenta[c]pyridin-6-yl)amino]-2-hydroxy-propyl]-2-oxo-oxazolidin-3-yl]-4-(2-trimethylsilylethoxymethyl)pyrazino[2,3-b][1,4]oxazin-3-one, Isomer A (Example 313) and was obtained as a white solid. MS (ESP): m/z = 459.1 [M+H]⁺.

### Example 316

### 6-[2-Oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer D

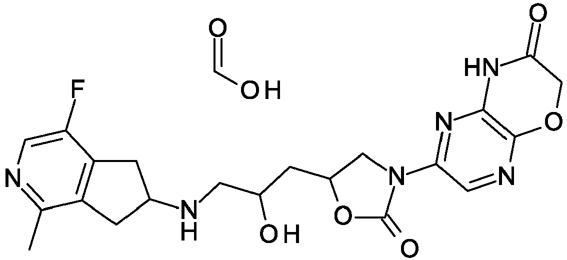

The title compound was prepared from 6-[5-(oxiran-2-ylmethyl)-2-oxo-oxazolidin-3-yl]-4-(2-trimethylsilyl ethoxymethyl)pyrazino[2,3-b][1,4]oxazin-3-one, Isomer D (Intermediate 221) by analogy with 5-[3-[(4-fluoro-1-methyl-6,7-dihydro-5*H*-cyclopenta[c]pyridin-6-yl)amino]-2-hydroxy-propyl]-2-oxo-oxazolidin-3-yl]-4-(2-trimethylsilylethoxymethyl)pyrazino[2,3-b][1,4]oxazin-3-one, Isomer A (Example 313) and was obtained as a white solid. MS (ESP): m/z = 459.2 [M+H]⁺.

### Example 317

### 6-[5-[2-[(4-Fluoro-6-hydroxy-2,3-dihydro-1H-inden-2-yl)methylamino]ethyl]-2-oxo-1,3-oxazolidin-3-yl]-4H-pyrazino[2,3-b][1,4]oxazin-3-one

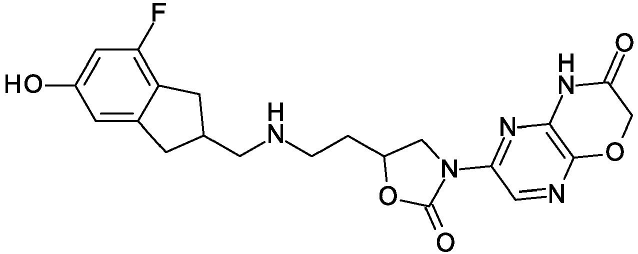

### Step 1:

### 6-[5-[2-[(6-Benzyloxy-4-fluoro-indan-2-yl)methylamino]ethyl]-2-oxo-oxazolidin-3 -yl]-4H-pyrazino[2,3-b][1,4]oxazin-3-one

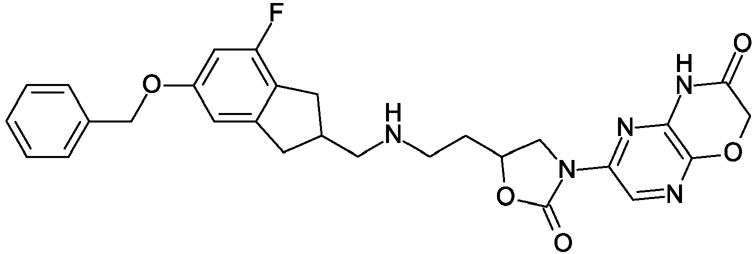

A mixture of 6-benzyloxy-4-fluoro-indane-2-carbaldehyde (Intermediate 222, 68.73 mg, 0.250 mmol, 1 eq) and 6-[5-(2-aminoethyl)-2-oxo-oxazolidin-3-yl]-4*H*-pyrazino[2,3-b][1,4]oxazin-3-one; 2,2,2-trifluoroacetic acid (Intermediate 24, 100.0 mg, 0.250 mmol, 1 eq) in 1-propanol (4 mL) and THF (4 mL) was stirred at stirred at 25 °C for 1h. After 1 h, NaBH₃CN (64.11 mg, 1.02 mmol, 4 eq) was added and the reaction was stirred at 25 °C for another 16 h. The mixture was concentrated under vacuum to give the crude. The crude product was triturated with CH₃OH (4 mL) and filtered, the filter cake was dried under vacuum to give the title compound (80 mg, 0.150 mmol, 59.0% yield) as a white solid. MS (ESP): m/z = 534.3[M+H]⁺.

### Step 2:

### 6-[5-[2-[(4-Fluoro-6-hydroxy-2,3-dihydro-1H-inden-2-yl)methylamino]ethyl]-2-oxo-1,3-oxazolidin-3-yl]-4H-pyrazino[2,3-b][1,4]oxazin-3-one

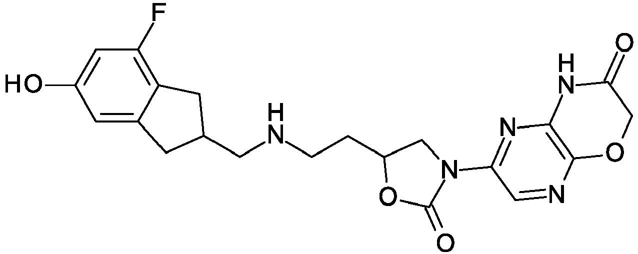

A mixture of 6-[5-[2-[(6-benzyloxy-4-fluoro-indan-2-yl)methylamino]ethyl]-2-oxo-oxazolidin-3-yl]-4*H*-pyrazino[2,3-b][1,4]oxazin-3-one (60.0 mg, 0.110 mmol, 1 eq) in CF₃COOH (5.0 mL) was stirred at stirred at 60 °C for 16 h. The mixture was concentrated under vacuum to give a crude product. The crude product was purified by Prep-HPLC (NH₄HCO₃ system) and freeze-dried to give the title compound (9.7 mg, 0.020 mmol, 17.9% yield) as a white solid. MS (ESP): m/z = 444.1 [M+H]⁺.

### Example 320

### 6-[5-[2-[[4-Fluoro-6-(1H-pyrazol-3-ylmethoxy)-2,3-dihydro-1H-inden-2-yl]methylamino]ethyl]-2-oxo-1,3-oxazolidin-3-yl]-4H-pyrazino[2,3-b][1,4]oxazin-3-one;formic acid

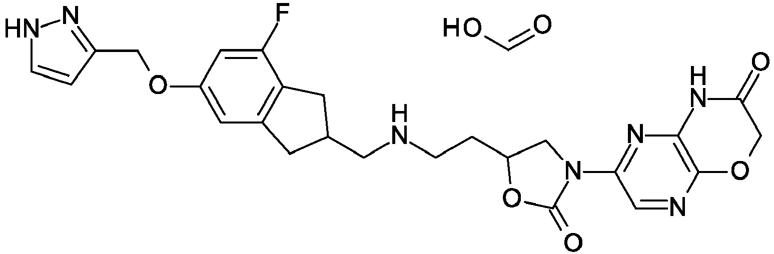

A mixture of *tert*-butyl 3-[(7-fluoro-2-formyl-indan-5-yl)oxymethyl]pyrazole-1-carboxylate (Intermediate 225, 82.47 mg, 0.230 mmol, 1 eq) and 6-[5-(2-aminoethyl)-2-oxo-oxazolidin-3-yl]-4*H*-pyrazino[2,3-b][1,4]oxazin-3-one; 2,2,2-trifluoroacetic acid (Intermediate 24, 90.0 mg, 0.230 mmol, 1 eq) in THF (9 mL) and 2-propanol (9 mL) was stirred at 25 °C for 1 h. After 1 h, sodium cyanoborohydride (43.14 mg, 0.690 mmol, 3 eq) was added and then the solution was stirred at 25 °C for 16 h. The mixture concentrated under vacuum to give a residue. The residue was re-dissolved in EtOAc (20 mL) and washed with water (15 mL), dried over Na₂SO₄ and concentrated under vacuum to give a crude product. A mixture of this crude product and HCOOH (2.0 mL) was stirred at 25 °C for 2 h. The mixture was concentrated under vacuum to give a residue. The residue was purified by Prep-HPLC (formic acid system) and freeze-dried to give the title compound as a white solid. MS (ESP): m/z = 524.3[M+H]⁺.

The following examples were prepared by analogy to Example 320.

| **Ex.** | **Name, Structure, MS** | **Starting Materials** |
|---|---|---|
| **327** | 6-[2-[2-[6-(2-aminopropoxy)-4-fluoro-2,3-dihydro-1*H*-inden-2-yl]ethyl]-6-oxo-5-oxa-2,7-diazaspiro[3.4]octan-7-yl]-4*H-*pyrazino[2,3-b][1,4]oxazin-3-one | Intermediate 233 and Intermediate 226 |
| | 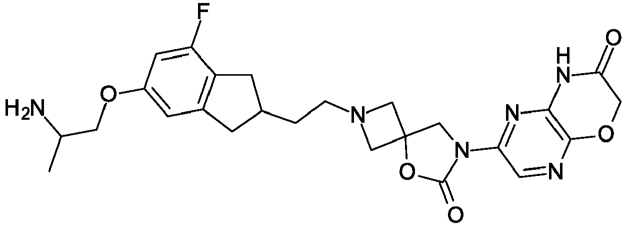 ESP [M+H]⁺: 513.3 | |
| 328 | 6-[5-[3-[(4-fluoro-1-methyl-6,7-dihydro-5*H-*cyclopenta[c]pyridin-6-yl)amino]cyclobutyl]-2-oxo-1,3-oxazolidin-3-yl]-4*H*-pyrazino[2,3-b][1,4]oxazin-3-one, Isomer A | Intermediate 153 and Intermediate 234 |
| | 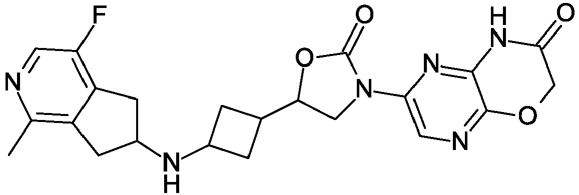 ESP [M+H]⁺: 455.2 | |
| **329** | 6-[5-[3-[(4-fluoro-1-methyl-6,7-dihydro-5*H-*cyclopenta[c]pyridin-6-yl)amino]cyclobutyl]-2-oxo-1,3-oxazolidin-3-yl]-4*H*-pyrazino[2,3-b][1,4]oxazin-3-one, Isomer B | Intermediate 153 and Intermediate 235 |
| | 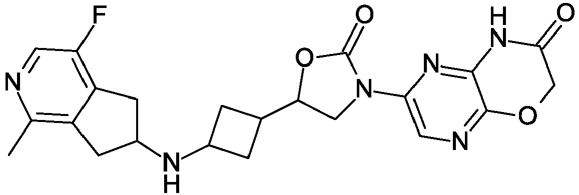 ESP [M+H]⁺: 455.1 | |
| **335** | 6-[5-[2-[(4-fluoro-1-methyl-6,7-dihydro-5*H-*cyclopenta[c]pyridin-6-yl)methylamino]ethyl]-2-oxo-1,3,4-oxadiazol-3-yl]-4*H*-pyrazino[2,3-b][1,4]oxazin-3-one | Intermediate 27 and Intermediate 242 |
| | 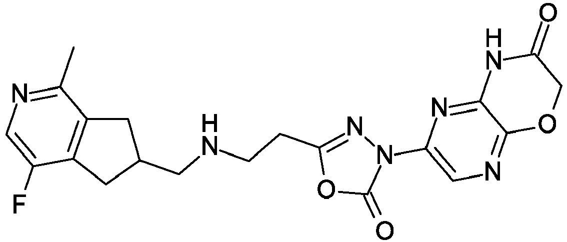 ESP [M+H]⁺: 442.2 | |
| **337** | 6-[2-oxo-5-[2-[[*rac*-1,2-*trans*-1-amino-4-chloro-2,3-dihydro-1*H*-inden-2-yl]methylamino]ethyl]-1,3,4-oxadiazol-3-yl]-4*H-*pyrazino[2,3-b][1,4]oxazin-3-one; formic acid | Intermediate 244 and Intermediate 92 |
| | 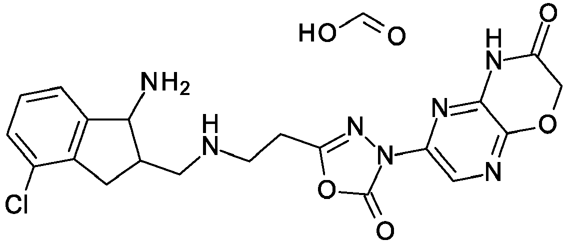 ESP [M+H]⁺: 458.1 | |
| **341** | 6-[(5*S*)-5-[2-[[6-(2-amino-2-methylpropoxy)-4-fluoro-2,3-dihydro-*1H*-inden-2-yl]methylamino]ethyl]-2-oxo-1,3-oxazolidin-3-yl]-4*H*-pyrazino[2,3-b][1,4]oxazin-3-one; formic acid | Intermediate 240 and Intermediate 247 |
| | 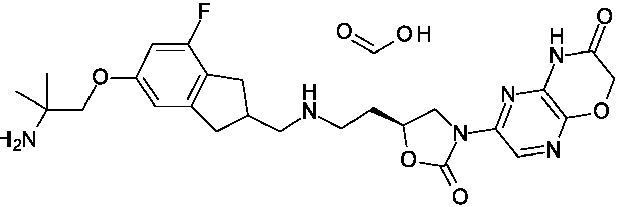 ESP [M+H]⁺: 515.3 | |
| **342** | 6-[(57?)-5-[2-[[6-(2-amino-2-methylpropoxy)-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one; formic acid | Intermediate 241 and Intermediate 247 |
| | 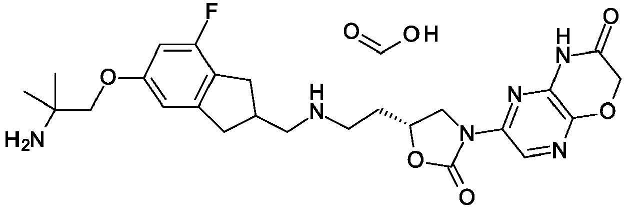 ESP [M+H]⁺: 515.3 | |
| **356** | 6-[8-[[6-[(2*S*)-2-aminopropoxy]-4-fluoro-2,3-dihydro-1*H-*inden-2-yl]methyl]-2-oxo-1-oxa-3,8-diazaspiro[4.5]decan-3-yl]-4*H*-pyrazino[2,3-b][1,4]oxazin-3-one;formic acid | Intermediate 186 and Intermediate 260 |
| | 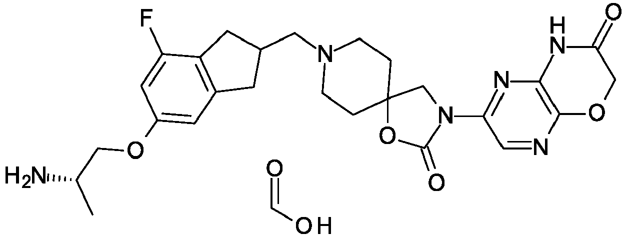 ESP [M+H]⁺: 527.1 | |
| **357** | 6-[8-[[6-[(2*R*)-2-aminopropoxy]-4-fluoro-2,3-dihydro-1*H-*inden-2-yl]methyl]-2-oxo-1-oxa-3,8-diazaspiro[4.5]decan-3-yl]-4*H*-pyrazino[2,3-b][1,4]oxazin-3-one;formic acid | Intermediate 186 and Intermediate 261 |
| | 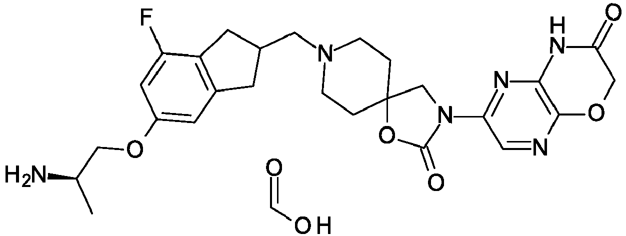 ESP [M+H]⁺: 527.4 | |
| **358** | 6-[5-[2-[[6-(azetidin-3-yloxy)-4-fluoro-2,3-dihydro-1*H*-inden-2-yl]methylamino]ethyl]-2-oxo-1,3-oxazolidin-3-yl]-4*H-*pyrazino[2,3-b][1,4]oxazin-3-one;hydrochloride | Intermediate 24 and Intermediate 103 |
| | 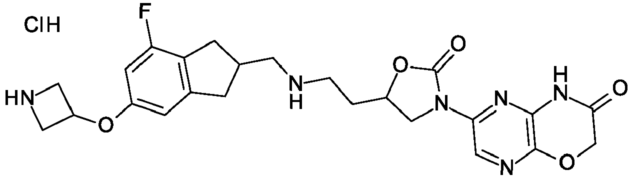 ESP [M+H]⁺: 499.2 | |
| **359** | 6-[5-[2-[(*rac*-1,2-*trans*-1-amino-4-chloro-2,3-dihydro-1*H-*inden-2-yl)methylamino]ethyl]-2-oxo-1,3-oxazol-3-yl]-4*H-*pyrazino[2,3-b][1,4]oxazin-3-one;formic acid | Intermediate 203 and Intermediate 92 |
| | 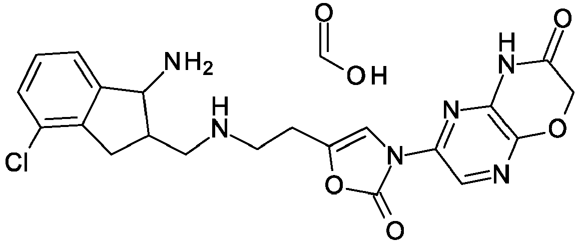 ESP [M+H]⁺: 457.1 | |

### Example 321

### 1-Methyl-6-[3-[2-oxo-3-(3-oxo-4H-pyrazino[2,3-b] [1,4]oxazin-6-yl)-1,3-oxazol-5-yl]propylamino]-6,7-dihydro-5H-cyclopenta[c]pyridine-4-carbonitrile, Enantiomer A

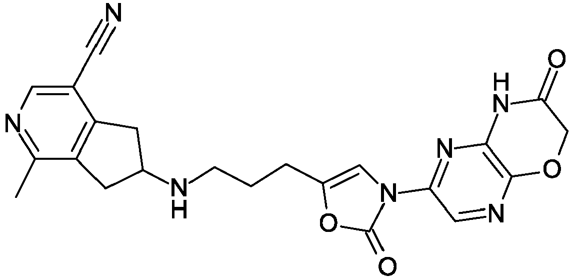

The title compound was prepared from 6-amino-1-methyl-6,7-dihydro-5*H-*cyclopenta[c]pyridine-4-carbonitrile; 2,2,2-trifluoroacetic acid, Enantiomer A (Intermediate 227) and 3-[2-oxo-3-[3-oxo-4-(2-trimethylsilylethoxymethyl)pyrazino[2,3-b][1,4]oxazin-6-yl]oxazol-5-yl]propanal (Intermediate 210) by analogy with *trans*-6-[5-[2-[[4-fluoro-6-[2-(1,2,4-triazol-1-yl)ethoxy]-2,3-dihydro-1*H*-inden-2-yl]methylamino]ethyl]-2-oxo-1,3-oxazolidin-3-yl]-4*H-*pyrazino[2,3-b][1,4]oxazin-3-one; formic acid (Example 297) and was obtained as a light brown solid. MS (ESP): m/z = 448.2 [M+H]⁺.

### Example 322

### 1-Methyl-6-[3-[2-oxo-3-(3-oxo-4H-pyrazino[2,3-b] [1,4]oxazin-6-yl)-1,3-oxazol-5-yl]propylamino]-6,7-dihydro-5H-cyclopenta[c]pyridine-4-carbonitrile, Enantiomer B

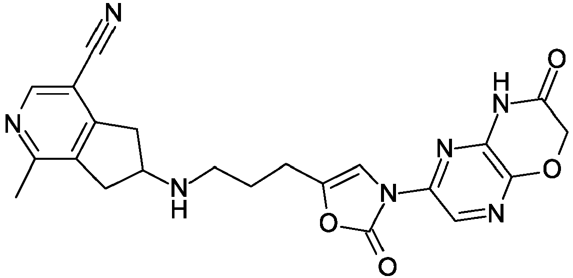

The title compound was prepared from 6-amino-1-methyl-6,7-dihydro-5*H-*cyclopenta[c]pyridine-4-carbonitrile; 2,2,2-trifluoroacetic acid, Enantiomer B (Intermediate 228) and 3-[2-oxo-3-[3-oxo-4-(2-trimethylsilylethoxymethyl)pyrazino[2,3-b][1,4]oxazin-6-yl]oxazol-5-yl]propanal (Intermediate 210) by analogy with *trans*-6-[5-[2-[[4-fluoro-6-[2-(1,2,4-triazol-1-yl)ethoxy]-2,3-dihydro-1*H*-inden-2-yl]methylamino]ethyl]-2-oxo-1,3-oxazolidin-3-yl]-4*H-*pyrazino[2,3-b][1,4]oxazin-3-one; formic acid (Example 297) and was obtained as a light brown solid. MS (ESP): m/z = 448.2 [M+H]⁺.

### Example 351

### 6-[5-[2-[[4-Fluoro-6-(2-hydroxypropoxy)-2,3-dihydro-1H-inden-2-yl]methylamino]ethyl]-2-oxo-1,3-oxazolidin-3-yl]-4H-pyrazino[2,3-b][1,4]oxazin-3-one;hydrochloride

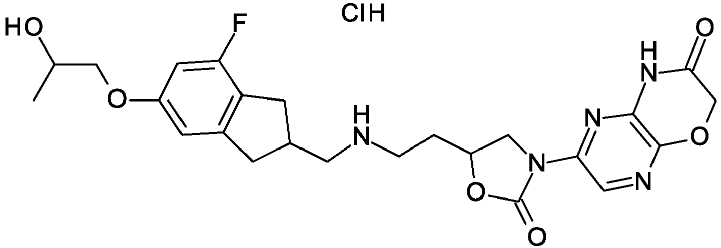

### Step 1:

### 6-[5-[2-[[6-(2-Benzyloxypropoxy)-4-fluoro-indan-2-yl]methylamino]ethyl]-2-oxo-oxazolidin-3-yl]-4H-pyrazino[2,3-b][1,4]oxazin-3-one

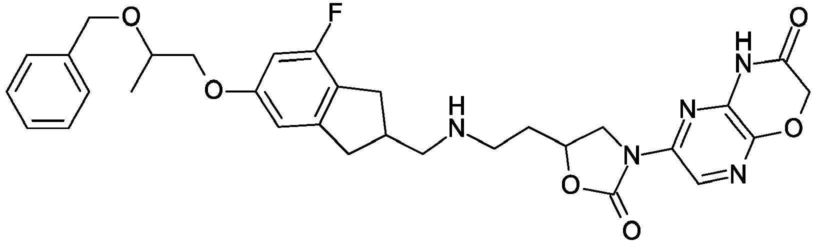

To a solution of 6-[5-(2-aminoethyl)-2-oxo-oxazolidin-3-yl]-4*H*-pyrazino[2,3-b][1,4]oxazin-3-one; 2,2,2-trifluoroacetic acid (Intermediate 24, 120 mg, 0.300 mmol, 1 eq) in THF (3 mL) and 2-propanol (5 mL) was added triethylamine (0.01 mL), the mixture was stirred at 25 °C for 10 min. After 10 min, 6-(2-benzyloxypropoxy)-4-fluoro-indane-2-carbaldehyde (Intermediate 255, 100 mg, 0.300 mmol, 1 eq) in THF (2 mL) was added and the mixture was stirred at 25 °C for 20 min. Then sodium cyanoborohydride (76.5 mg, 1.22 mmol, 4 eq) was added and the resulting mixture was stirred at 25 °C for another 16 h. The solvent was removed by evaporated to give a residue, which was purified by Prep-TLC (DCM: MeOH =10:1) to give the title compound (80 mg, 0.140 mmol, 42.2% yield) as an off-white solid. MS (ESP): m/z = 592.3 [M+H]⁺.

### Step 2:

### 6-[5-[2-[[4-Fluoro-6-(2-hydroxypropoxy)-2,3-dihydro-1H-inden-2-yl]methylamino]ethyl]-2-oxo-1,3-oxazolidin-3-yl]-4H-pyrazino[2,3-b][1,4]oxazin-3-one;hydrochloride

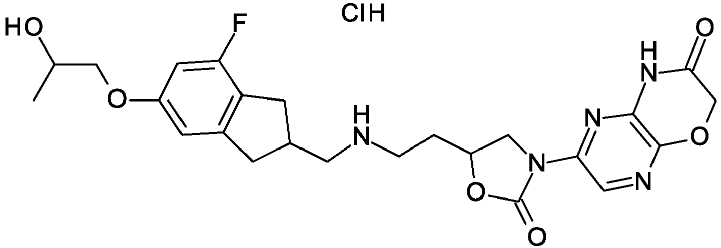

To a solution of 6-[5-[2-[[6-(2-benzyloxypropoxy)-4-fluoro-indan-2-yl]methylamino]ethyl]-2-oxo-oxazolidin-3-yl]-4*H*-pyrazino[2,3-b][1,4]oxazin-3-one (70.0 mg, 0.120 mmol, 1 eq) in DCM (10 mL) was added ferric chloride (96.0 mg, 0.590 mmol, 5 eq), then the solution was stirred at 25 °C for 16 h. The solid product was collected by filtration, washed with 3 mL MeOH, then was purified by Prep-HPLC (HCl system) then dried by lyophilization to give the title compound (5.2 mg, 0.010 mmol, 8.4% yield) as a yellow solid. MS (ESP): m/z = 502.1 [M+H]⁺.

### Example 360

### 6-[(5R)-5-[(1R)-2-[(4-Fluoro-1-methyl-6,7-dihydro-5H-cyclopenta[c]pyridin-6-yl)methylamino]-1-hydroxyethyl]-2-oxo-1,3-oxazolidin-3-yl]-4H-pyrazino[2,3-b][1,4]oxazin-3-one;formic acid

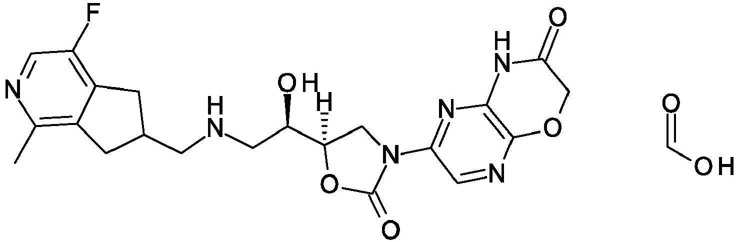

### Step 1:

### tert-Butyl N-[(4-fluoro-1-methyl-6,7-dihydro-5H-cyclopenta[c]pyridin-6-yl)methyl]-N-[(2R)-2-hydroxy-2-[(5R)-2-oxo-3-(3-oxo-4H-pyrazino[2,3-b][1,4]oxazin-6-yl)oxazolidin-5-yl]ethyl] carbamate

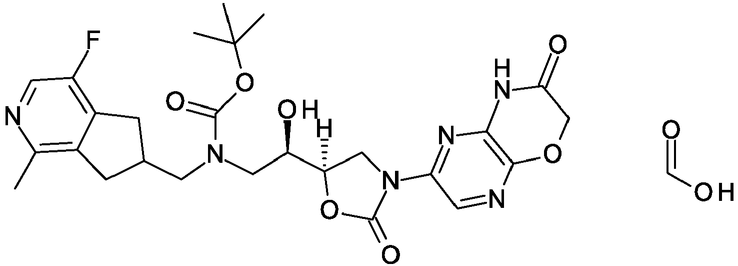

To a solution of 6-bromo-4*H*-pyrazino[2,3-b][1,4]oxazin-3-one (Intermediate 25, Step 1, 34.1 mg, 0.150 mmol, 1.01 eq) and *tert*-butyl *N*-[(4-fluoro-1-methyl-6,7-dihydro-5*H-*cyclopenta [c]pyridin-6-yl)methyl]-*N*-[(2*R*)-2-hydroxy-2-[(SR)-2-oxooxazolidin-5-yl]ethyl]carbamate (Intermediate 262, 60.0 mg, 0.150 mmol, 1 eq) in 1,4-dioxane (6 mL) was added *trans*-*N,N*'-dimethylcyclohexane-1,2-diamine (12.8 mg, 0.090 mmol, 0.61 eq), potassium carbonate (142 mg, 1.03 mmol, 7.01 eq) and CuI (27.9 mg, 0.150 mmol, 1 eq). The mixture was degassed with N₂ three times and stirred at 100 °C for 3 h. The solid was filtered off and the filtrate was concentrated to give a crude, which was purified by Prep-HPLC (formic acid system) and dried by lyophilization to give the title compound (30 mg, 0.050 mmol, 36.7% yield) as a white solid. MS (ESP): m/z = 559.3 [M+H]⁺.

### Step 2:

### 6-[(5R)-5-[(1R)-2-[(4-Fluoro-1-methyl-6,7-dihydro-5H-cyclopenta[c]pyridin-6-yl)methylamino]-1-hydroxyethyl]-2-oxo-1,3-oxazolidin-3-yl]-4H-pyrazino[2,3-b][1,4]oxazin-3-one;formic acid

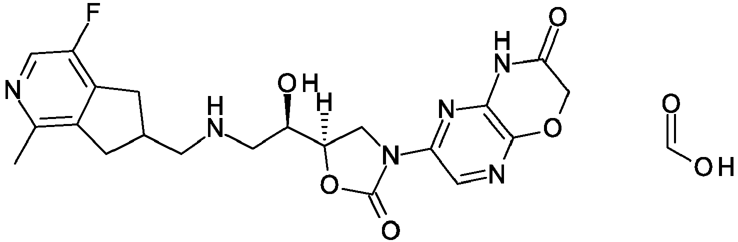

A solution of *tert*-butyl *N*-[(4-fluoro-1-methyl-6,7-dihydro-5*H*-cyclopenta[c]pyridin-6-yl)methyl]-*N*-[(2*R*)-2-hydroxy-2-[(5*R*)-2-oxo-3-(3-oxo-4H-pyrazino[2,3-b][1,4]oxazin-6-yl)oxazolidin-5-yl]ethyl] carbamate (30.0 mg, 0.050 mmol, 1 eq) in formic acid (3.0 mL) was stirred at 25 °C for 2 h. The solvent was removed and the residue was taken up in MeOH (5 mL) and water (50 mL), then the mixture was dried by lyophilization to give the title compound (18 mg, 0.040 mmol, 63.1% yield) as a white solid. MS (ESP): m/z = 459.1 [M+H]⁺.

### Example 367

### 6-[5-[3-[(4-Fluoro-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one, Enantiomer A

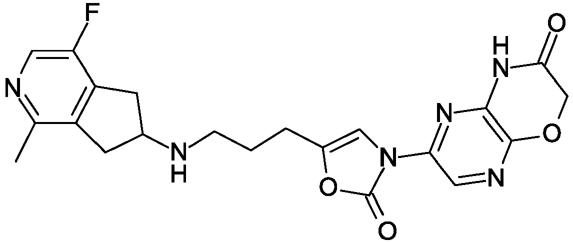

To a mixture of *tert-butyl N*-[(4-fluoro-1-methyl-6,7-dihydro-5*H*-cyclopenta[c]pyridin-6-yl]-*N-*[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer A (Intermediate 267, 120.0 mg, 0.180 mmol, 1 eq) in DCM (5 mL) was added CF₃COOH (1.0 mL) and the mixture was stirred at 25 °C for 2 h. The mixture was concentrated under high vacuum to give a residue. The residue was redissolved in ethanol (16 mL) then ethylamine (24 mg, 0.540 mmol, 3 eq) and K₂CO₃ (49 mg, 0.360 mmol, 2 eq) was added and the mixture was stirred at 25 °C for 2 h. The mixture was acidified with CF₃COOH (two drops) and then concentrated under vacuum to give a crude product. The crude product was purified by Prep-HPLC (formic acid system) to give the title compound (41.8 mg, 0.090 mmol, 53.1% yield) as a white solid. MS (ESP): m/z = 441.2 [M+H]⁺.

### Example 368

### 6-[5-[3-[(4-Fluoro-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one, Enantiomer B

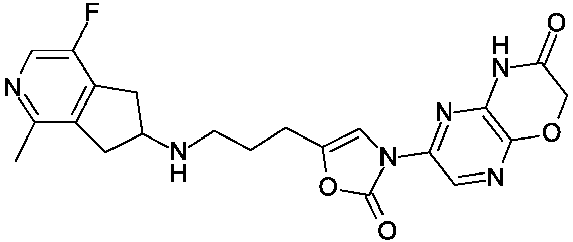

The title compound was prepared from *tert*-butyl *N*-[(4-fluoro-1-methyl-6,7-dihydro-5*H-*cyclopenta[c]pyridin-6-yl]-*N*-[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer B (Intermediate 268) by analogy with 6-[5-[3-[(4-fluoro-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one, Enantiomer A (Example 367) and was obtained as a white solid. MS (ESP): m/z = 441.2 [M+H]⁺.

### Example 369

### 6-[5-[3-[(1-Methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid, Enantiomer A

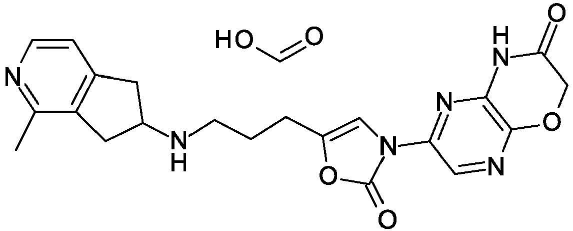

The title compound was prepared from 6-[5-[3-[(1-methyl-6,7-dihydro-5*H*-cyclopenta[c]pyridin-6-yl)amino]propyl]-2-oxo-oxazol-3-yl]-4-(2-trimethylsilylethoxymethyl)pyrazino[2,3-b][1,4]oxazin-3-one, Enantiomer A (Intermediate 269) by analogy with 6-[5-[3-[(4-fluoro-1-methyl-6,7-dihydro-5*H*-cyclopenta[c]pyridin-6-yl)amino]propyll-2-oxo-1,3-oxazol-3-yl]-4*H-*pyrazino[2,3-b][1,4]oxazin-3-one, Enantiomer A (Example 367) and was obtained as a purple solid. MS (ESP): m/z = 423.3 [M+H]⁺.

### Example 370

### 6-[5-[3-[(1-Methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid, Enantiomer B

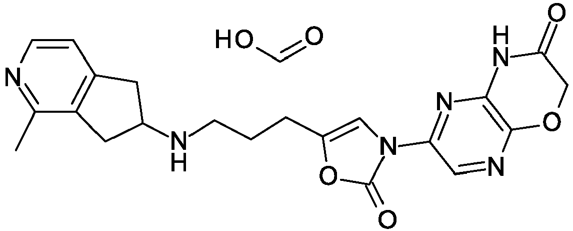

The title compound was prepared from 6-[5-[3-[(1-methyl-6,7-dihydro-5*H*-cyclopenta[c]pyridin-6-yl)amino]propyl]-2-oxo-oxazol-3-yl]-4-(2-trimethylsilylethoxymethyl)pyrazino[2,3-b][1,4]oxazin-3-one, Enantiomer B (Intermediate 270) by analogy with 6-[5-[3-[(4-fluoro-1-methyl-6,7-dihydro-5*H*-cyclopenta[c]pyridin-6-yl)amino]propyl]-2-oxo-1,3-oxazol-3-yl]-4*H-*pyrazino[2,3-b][1,4]oxazin-3-one, Enantiomer A (Example 367) and was obtained as a purple solid. MS (ESP): m/z = 423.2 [M+H]⁺.

### Example 376 6-[5-[2-[(4-Fluoro-1-methyl-6,7-dihydro-5H-cyclopenta[c]pyridin-6-yl)methyl-methylamino]ethyl]-2-oxo-1,3-oxazolidin-3-yl]-4H-pyrazino[2,3-b][1,4]oxazin-3-one

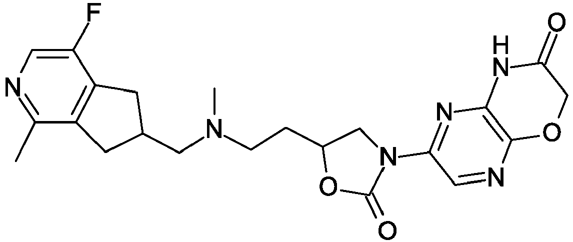

A solution of 4-fluoro-1-methyl-6,7-dihydro-5*H*-cyclopenta[c]pyridine-6-carbaldehyde (Intermediate 27, 22.8 mg, 0.130 mmol, 1 eq) and 6-[5-(2-aminoethyl)-2-oxo-oxazolidin-3-yl]-4*H*-pyrazino[2,3-b][1,4]oxazin-3-one; 2,2,2-trifluoroacetic acid (Intermediate 24, 50.0 mg, 0.130 mmol, 1 eq) in methanol (5 mL) was stirred at 20 °C for 2 h. After 2 h, sodium cyanoborohydride (24.0 mg, 0.380 mmol, 3 eq) was added and the reaction was stirred for another 16 h. After 16 h, formaldehyde in water (0.05 mL, 0.720 mmol, 5.66 eq) was added and the mixture was stirred at 20 °C for another 1h, The mixture was filtered and purified by Prep-HPLC (NH₄HCO₃ system) then freeze-dried to give the title compound (19 mg, 0.040 mmol, 32.3% yield) as a white solid. MS (ESP): m/z = 457.2 [M+H]⁺.

### Example 377

### 6-[8-[[6-(2-Amino-2-methylpropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid, Enantiomer A

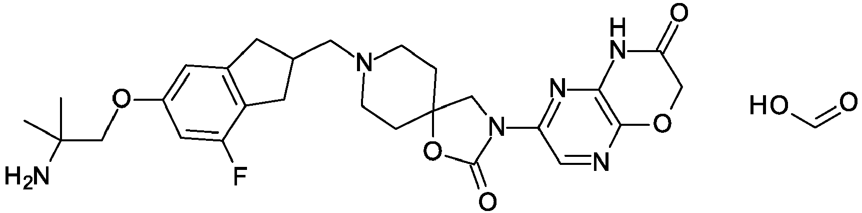

### Step 1:

### tert-Butyl N-[2-[7-fluoro-2-[[2-oxo-3-(3-oxo-4H-pyrazino[2,3-b][1,4]oxazin-6-yl)-1-oxa-3,8-diazaspiro[4.5]decan-8-yl]methyl]indan-5-yl]oxy-1,1-dimethyl-ethyl]carbamate, Enantiomer A and tert-butyl N-[2-[7-fluoro-2-[[2-oxo-3-(3-oxo-4H-pyrazino[2,3-b] [1,4]oxazin-6-yl)-1-oxa-3,8-diazaspiro[4.5]decan-8-yl]methyl]indan-5-yl]oxy-1,1-dimethyl-ethyl]carbamate, Enantiomer B

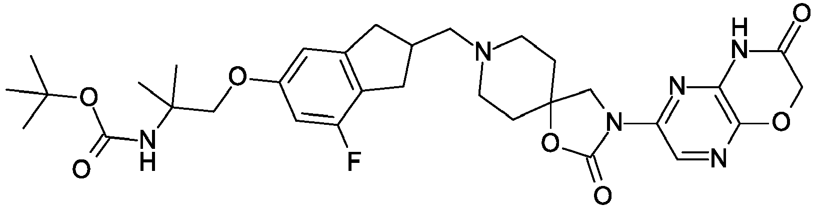

To a solution of 6-(2-oxo-1-oxa-3,8-diazaspiro[4.5]decan-3-yl)-4*H*-pyrazino[2,3-b] [1,4]oxazin-3-one; formic acid (Intermediate 186, 50.0 mg, 0.140 mmol, 1 eq) in THF (3 mL), 2-propanol (3 mL) and DMA (2 mL) was added *tert*-butyl N-[2-(7-fluoro-2-formyl-indan-5-yl)oxy-1,1-dimethyl-ethyl]carbamate (Intermediate 247, 50.0 mg, 0.140 mmol, 1 eq) and the resulting mixture stirred at 25 °C for 1h. Then sodium cyanoborohydride (35.8 mg, 0.570 mmol, 4 eq) was added to the mixture and the mixture was stirred at 25 °C for 16 h. The mixture was evaporated and purified by prep-HPLC (formic acid system) and freeze-dried to give a racemic mixture of the title compounds (80 mg, 0.120 mmol, 87.8% yield) as a white solid. MS (ESP): m/z = 641.3 [M+H]⁺.

The two isomers were separated by chiral SFC separation to give *tert*-butyl *N*-[2-[7-fluoro-2-[[2-oxo-3-(3-oxo-4*H*-pyrazino[2,3-b][1,4]oxazin-6-yl)-1-oxa-3,8-diazaspiro[4.5]decan-8-yl]methyl]indan-5-yl]oxy-1,1-dimethyl-ethyl]carbamate, Enantiomer A (40 mg, 0.060 mmol, 36.4% yield) as a white solid and *tert*-butyl *N*-[2-[7-fluoro-2-[[2-oxo-3-(3-oxo-4H-pyrazino[2,3-b] [1,4]oxazin-6-yl)-1-oxa-3,8-diazaspiro[4.5]decan-8-yl]methyl]indan-5-yl]oxy-1,1-dimethylethyl]carbamate, Enantiomer B (26 mg, 0.040 mmol, 23.6% yield) as a white solid.

### Step 2:

### 6-[8-[[6-(2-Amino-2-methylpropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid, Enantiomer A

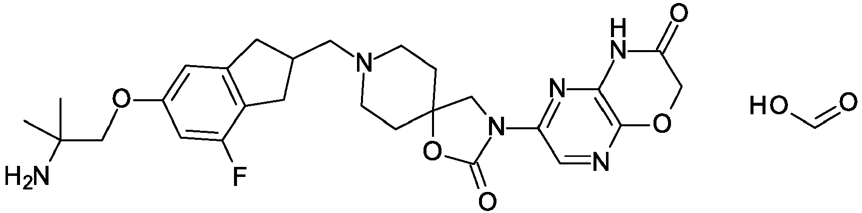

A solution *tert*-butyl *N*-[2-[7-fluoro-2-[[2-oxo-3-(3-oxo-4*H*-pyrazino[2,3-b][1,4]oxazin-6-yl)-1-oxa-3,8-diazaspiro[4.5]decan-8-yl]methyl]indan-5-yl]oxy-1,1-dimethyl-ethyl]carbamate, Enantiomer A (40.0 mg, 0.060 mmol, 1 eq) in HCOOH (3.0 mL, 0.060 mmol, 1 eq) was stirred at 25 °C for 2 h. The mixture was evaporated, then H₂O (20 mL) was added and the mixture freeze-dried to give the title compound (34.7 mg, 0.060 mmol, 94.8% yield) as a white solid. MS (ESP): m/z = 541.4 [M+H]⁺.

### Example 378

### 6-[8-[[6-(2-Amino-2-methylpropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid, Enantiomer B

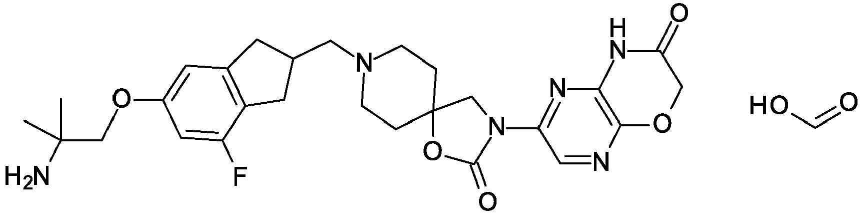

The title compound was prepared from *tert*-butyl *N*-[2-[7-fluoro-2-[[2-oxo-3-(3-oxo-4*H-*pyrazino[2,3-b][1,4]oxazin-6-yl)-1-oxa-3,8-diazaspiro[4.5]decan-8-yl]methyl]indan-5-yl]oxy-1,1-dimethyl-ethyl]carbamate, Enantiomer B (Example 377, Step 1) by analogy with 6-[8-[[6-(2-amino-2-methylpropoxy)-4-fluoro-2,3-dihydro-1*H*-inden-2-yl]methyl]-2-oxo-1-oxa-3,8-diazaspiro[4.5]decan-3-yl]-4*H*-pyrazino[2,3-b][1,4]oxazin-3-one;formic acid, Enantiomer A (Example 377) and was obtained as a white solid. MS (ESP): m/z = 541.4 [M+H]⁺.

### Example 379

### 2-[[7-Fluoro-2-[[2-[(SS)-2-oxo-3-(3-oxo-4H-pyrazino[2,3-b] [1,4]oxazin-6-yl)-1,3-oxazolidin-5-yl]ethylamino]methyl]-2,3-dihydro-1H-inden-5-yl]oxy]acetamide, Isomer A

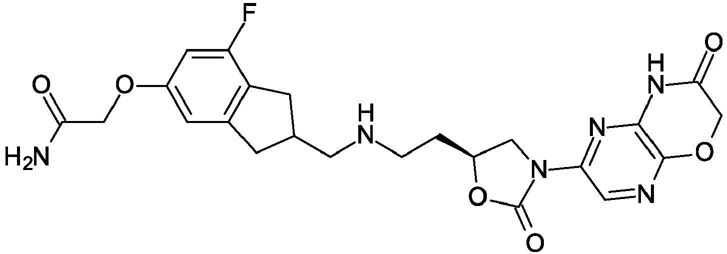

and

### Example 380

### 2-[[7-Fluoro-2-[[2-[(5S)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer B

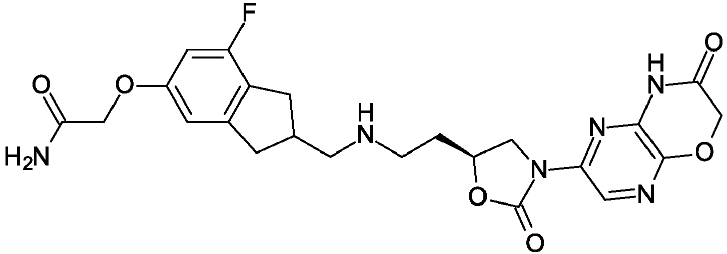

To a solution of 6-[(5*S*)-5-(2-aminoethyl)-2-oxo-oxazolidin-3-yl]-4*H*-pyrazino[2,3-b][1,4]oxazin-3-one (Intermediate 240, 51.8 mg, 0.190 mmol, 1.1 eq) in THF (12 mL) and 2-propanol (11.58 mL) was added triethylamine (0.04 mL, 0.250 mmol, 1.5 eq) and stirred at 25 °C for 10 min, then 2-(7-fluoro-2-formyl-indan-5-yl)oxyacetamide (Intermediate 212, 40.0 mg, 0.170 mmol, 1 eq) was added and the mixture stirred at 25 °C for 1h . After 1 h, sodium cyanoborohydride (42.4 mg, 0.670 mmol, 4 eq) was added to the mixture at 0 °C. Then the mixture was stirred at 25 °C for another 16 h. The solvent was evaporated and purified by Prep-HPLC (formic acid system), with freeze-drying to give 2-[[7-fluoro-2-[[2-[(5S)-2-oxo-3-(3-oxo-4*H*-pyrazino[2,3-b][1,4]oxazin-6-yl)-1,3-oxazolidin-5-yl]ethylamino]methyl]-2,3-dihydro-1*H-*inden-5-yl]oxy]acetamide (20 mg, 0.040 mmol, 23.7% yield) as a white solid. MS (ESP): m/z = 501.2 [M+H]⁺. This material was separated by chiral SFC and concentrated under vacuum, with freeze-drying to give 2-[[7-fluoro-2-[[2-[(SS)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide, Isomer A (17 mg, 0.030 mmol, 24.8% yield) as a light brown solid; m/z = 501.2 [M+H]⁺, and 2-[[7-fluoro-2-[[2-[(SS)-2-oxo-3-(3-oxo-4*H*-pyrazino[2,3-b] [1,4]oxazin-6-yl)-1,3-oxazolidin-5-yl]ethylamino]methyl]-2,3-dihydro-1*H*-inden-5-yl]oxy]acetamide, Isomer B (14.5 mg, 0.030 mmol, 21.9% yield) as a light brown solid; MS (ESP): m/z = 501.2 [M+H]⁺.

### Example 381

### 2-[[7-Fluoro-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer A

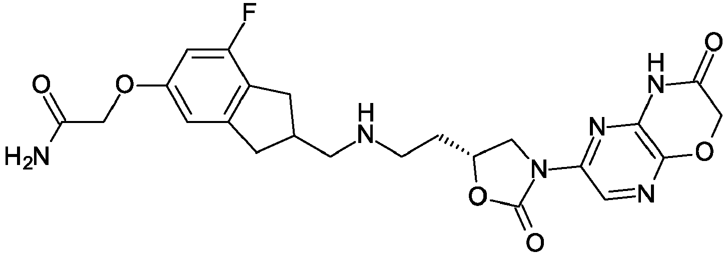

and

### Example 382

### 2-[[7-Fluoro-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer B

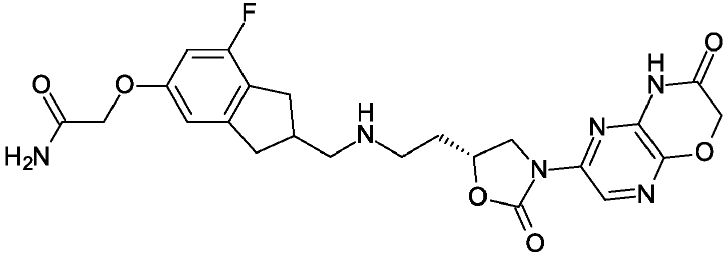

The title compounds were prepared by analogy with 2-[[7-fluoro-2-[[2-[(5*S*)-2-oxo-3-(3-oxo-4*H-*pyrazino[2,3-b][1,4]oxazin-6-yl)-1,3-oxazolidin-5-yl]ethylamino]methyl]-2,3-dihydro-1*H*-inden-5-yl]oxy]acetamide, Isomer A and Isomer B (Examples 379 and 380) using 6-[(5R)-5-(2-aminoethyl)-2-oxo-oxazolidin-3-yl]-4*H*-pyrazino[2,3-b][1,4]oxazin-3-one (Intermediate 241) in place of 6-[(5*S*)-5-(2-aminoethyl)-2-oxo-oxazolidin-3-yl]-4*H*-pyrazino[2,3-b][1,4]oxazin-3-one and were each obtained as a light yellow solid. MS (ESP): m/z = 501.2 [M+H]⁺ for both compounds.

### Example 399

### 2-[[rac-2,3-trans-3-Amino-7-chloro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;formic acid

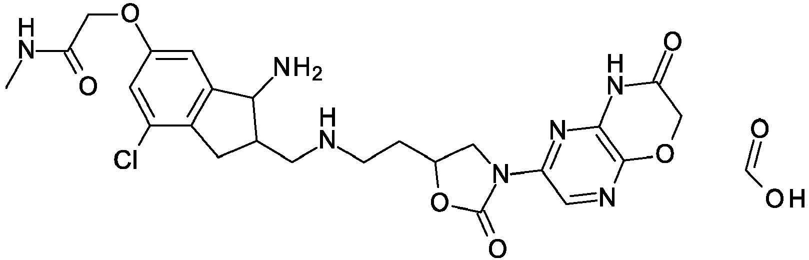

A white suspension of 6-[5-(2-aminoethyl)-2-oxo-1,3-oxazolidin-3-yl]-4*H*-pyrazino[2,3-b][1,4]oxazin-3-one trifluoroacetate (Intermediate 24, 105 mg, 0.266 mmol, 1 eq), *tert*-butyl *N-*[1,2-trans-4-chloro-2-formyl-6-[2-(methylamino)-2-oxoethoxy]-2,3-dihydro-1*H*-inden-1-yl]carbamate (Intermediate 285, 120 mg, 0.266 mmol, 1 eq), acetic acid (24 mg, 22.9 µL, 0.400 mmol, 1.5 eq) and sodium triacetoxyborohydride (84.7 mg, 0.400 mmol, 1.5 eq) in THF (4 mL) and EtOH (6.5 mL) was stirred at RT for 3 days. Additional acetic acid (16 mg, 15.3 µL, 0.266 mmol, 1 eq) and sodium triacetoxyborohydride (56.5 mg, 0.266 mmol, 1 eq) were added and the mixture was stirred for another 18 h. Water (basic with NaHCO₃) was added to the mixture. The mixture was extracted with EtOAc (3 x). The organic layers were washed with brine (basic), dried over Na₂SO₄ and evaporated. The residue was purified by flash chromatography (10 g silica gel; DCM/MeOH 98:2 - 90:10) to give an off-white solid (119 mg). A solution of this material (103 mg, 0.143 mmol, 1 eq) in formic acid (1.32 g, 1.1 mL, 28.7 mmol, 200 eq) was stirred at RT for 4 h. Additional formic acid (1.32 g, 1.1 ml, 28.7 mmol, 200 eq) was added and the solution was stirred at RT overnight. Acetonitrile was added and removed by evaporation 5 times to give the title compound (78 mg) as an off-white solid. MS (ESP): m/z = 546.2 [M+H]⁺.

The following examples were prepared by analogy to Example 399:

| **Ex.** | **Name, Structure, MS** | **Starting Materials** |
|---|---|---|
| **400** | *N*-methyl-2-[[*rac*-2,3-*trans*-3-amino-7-chloro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]acetamide; formic acid | Intermediate 1 and Intermediate 285 |
| | 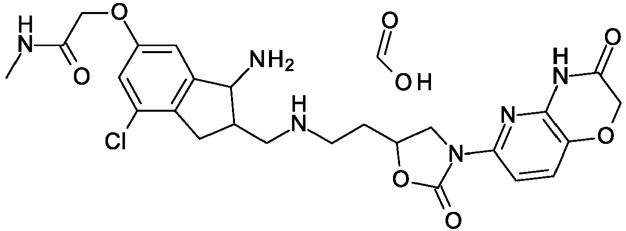 ESP [M+H]⁺: 545.2 | |
| **401** | 6-[(5*S*)-5-[2-[[(1*R*,2*R*)-1-amino-4-chloro-2,3-dihydro-1*H-*inden-2-yl]methylamino]ethyl]-2-oxo-1,3-oxazolidin-3-yl]-4*H-*pyrazino[2,3-b][1,4]oxazin-3-one | Intermediate 240 and Intermediate 286 |
| | 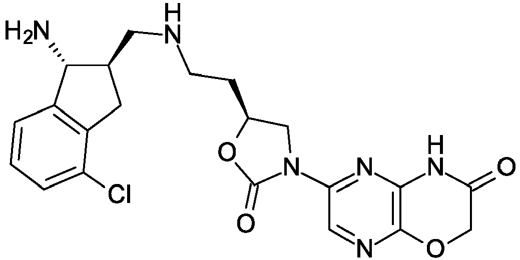 ESP [M+H]⁺: 459.2 | |
| **404** | 2-[[2-[2-oxo-3-(3-oxo-4*H*-pyrazino[2,3-b][1,4]oxazin-6-yl)-1,3-oxazolidin-5-yl]ethylamino]methyl]-6-(1*H*-pyrazol-3-ylmethoxy)-2,3-dihydro-1*H*-indene-4-carbonitrile; formic acid | Intermediate 24 and Intermediate 288 |
| | 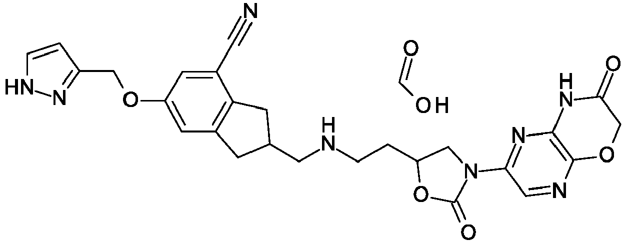 ESN [M-H]⁻: 529.3 | |
| **405** | 2-[[2-[2-oxo-3-(3-oxo-4*H*-pyrazino[2,3-b][1,4]oxazin-6-yl)-1,3-oxazolidin-5-yl]ethylamino]methyl]-6-(1*H*-pyrazol-4-ylmethoxy)-2,3-dihydro-1*H*-indene-4-carbonitrile; formic acid | Intermediate 24 and Intermediate 289 |
| | 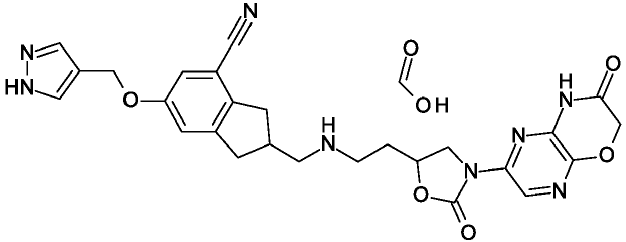 ESN [M-H]⁻: 529.4 | |
| **406** | 6-(1*H*-imidazol-5-ylmethoxy)-2-[[2-[2-oxo-3-(3-oxo-4*H-*pyrazino[2,3-b] [1,4]oxazin-6-yl)-1,3-oxazolidin-5-yl]ethylamino]methyl]-2,3-dihydro-1*H*-indene-4-carbonitrile; formic acid | Intermediate 24 and Intermediate 290 |
| | 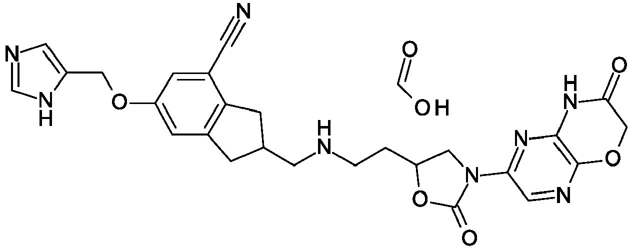 ESN [M-H]⁻: 529.4 | |
| **408** | 6-[5-[2-[[5-[(1-aminocyclopropyl)methoxy]-4,7-difluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;formic acid | Intermediate 24 and Intermediate 291 |
| | 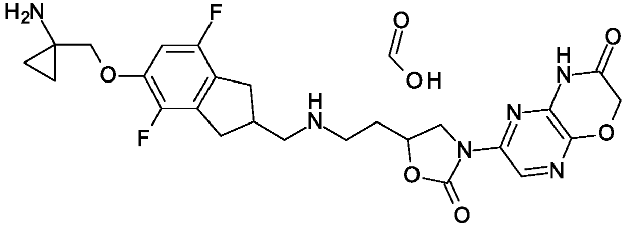 ESP [M+H]⁺: 531.4 | |
| **409** | 6-[5-[2-[[5-[(1-aminocyclopropyl)methoxy]-4,7-difluoro-2,3-dihydro-1*H*-inden-2-yl]methylamino]ethyl]-2-oxo-1,3-oxazolidin-3-yl]-4*H*-pyrido[3,2-b][1,4]oxazin-3-one;formic acid | Intermediate 1 and Intermediate 291 |
| | 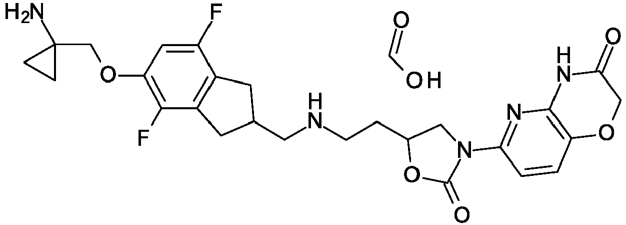 ESP [M+H]⁺: 530.4 | |
| **410** | 6-[8-[[5-[(1-aminocyclopropyl)methoxy]-4,7-difluoro-2,3-dihydro-1*H*-inden-2-yl]methyl]-2-oxo-1-oxa-3,8-diazaspiro[4.5]decan-3-yl]-4*H*-pyrazino[2,3-b][1,4]oxazin-3-one;formic acid | Intermediate 186 and Intermediate 291 |
| | 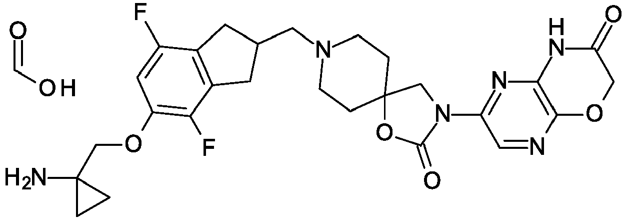 ESP [M+H]⁺: 557.4 | |
| **411** | 6-[5-[2-[[5-(2-amino-2-methylpropoxy)-4,7-difluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;formic acid | Intermediate 24 and Intermediate 292 |
| | 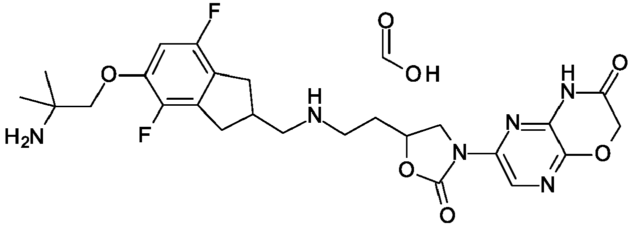 ESP [M+H]⁺: 533.5 | |
| **412** | 6-[8-[[5-(2-amino-2-methylpropoxy)-4,7-difluoro-2,3-dihydro-1*H*-inden-2-yl]methyl]-2-oxo-1-oxa-3,8-diazaspiro[4.5]decan-3-yl]-4*H*-pyrazino[2,3-b][1,4]oxazin-3-one;formic acid | Intermediate 186 and Intermediate 292 |
| | 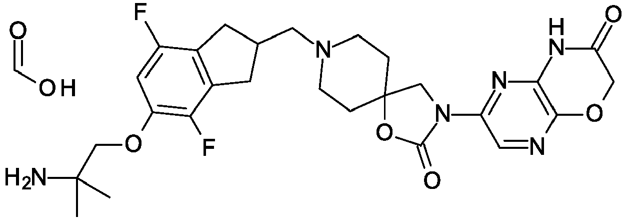 ESP [M+H]⁺: 559.5 | |

### Example 417

### 6-[4-[2-[(4-Fluoro-1-methyl-6,7-dihydro-5H-cyclopenta[c]pyridin-6-yl)methylamino]ethyl]-2-oxopyrrolidin-1-yl]-4H-pyrido[3,2-b][1,4]oxazin-3-one

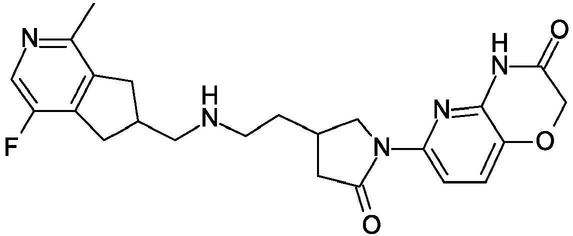

To a solution of 4-fluoro-1-methyl-6,7-dihydro-5*H*-cyclopenta[c]pyridine-6-carbaldehyde (Intermediate 27, 41 mg, 229 µmol, 1 eq) in methanol (915 µL) was added 6-(4-(2-aminoethyl)-2-oxopyrrolidin-1-yl)-4-(4-methoxybenzyl)-2*H*-pyrido[3,2-b][1,4]oxazin-3(4*H*)-one (Intermediate 297, 90.7 mg, 229 µmol, 1 eq) at room temperature and the mixture stirred at 55 °C for 2 h. The reaction mixture was cooled to room temperature. 3Å molecular sieves, acetic acid (13.7 mg, 13.1 µL, 229 µmol, 1 eq) were added. The reaction mixture was cooled to 0-5°C and sodium borohydride (10.4 mg, 275 µmol, 1.2 eq) was added. The mixture was stirred at RT for 15 min. The reaction was quenched with 1 M aqueous sodium hydroxide solution at room temperature and stirred for 5 min. The mixture was partitioned between dichloromethane (20 mL) and 1 M aqueous sodium hydroxide solution (10 mL). The layers were separated. The aqueous layer was extracted with DCM (3 x 20 mL). The combined organic layers were dried over anhydrous sodium sulfate, filtered and concentrated in vacuo. The crude product was purified by flash chromatography (heptane/isopropyl acetate 100:0 to 0:100) to give crude 6-(4-(2-(((4-fluoro-1-methyl-6,7-dihydro-5*H*-cyclopenta[c]pyridin-6-yl)methyl)amino)ethyl)-2-oxopyrrolidin-1-yl)-4-(4-methoxybenzyl)-2*H*-pyrido[3,2-b][1,4]oxazin-3(4*H*)-one (66 mg, 118 µmol. To a solution of this material in dichloromethane (2 mL) was added trifluoromethansulfonic acid (63.7 mg, 37.3 µl, 425 µmol, 3.6 eq) at 0-5 °C. The ice bath was removed and stirring was continued at room temperature 16 h. To the reaction mixture was added potassium carbonate (60.3 mg, 436 µmol, 3.7 eq) and the mixture stirred for 30 h. The solvent was removed by decantation. The residue was partitioned between DCM (15 mL) and water (5 mL) (pH 9). The aqueous layer was extracted with DCM (4 x 15 mL). The combined organic layers were dried over anhydrous sodium sulfate, filtered and concentrated in vacuo. The crude product was purified by flash chromatography (heptane/isopropyl acetate 100:0 to 0:100) to give the title compound (12 mg) as a white solid. MS (ESP): m/z = 440.2 [M+H]⁺.

### Example 418

### 6-[5-[2-(6,7-Dihydro-5H-cyclopenta[c]pyridin-6-ylmethylamino)ethyl]-2-oxo-1,3-oxazolidin-3-yl] -4H-pyrido[3,2-b] [1,4]oxazin-3-one

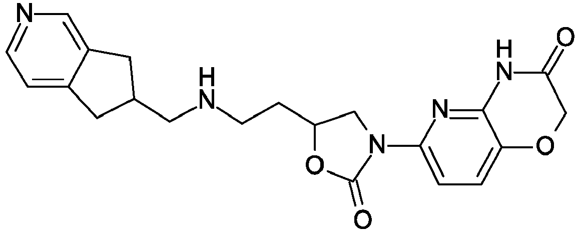

The title compound was prepared by analogy with 6-[4-[2-[(4-fluoro-1-methyl-6,7-dihydro-5*H-*cyclopenta [c]pyridin-6-yl)methylamino] ethyl]-2-oxopyrrolidin-1-yl]-4*H*-pyrido [3,2-b][1,4]oxazin-3-one (Example 417) using 6,7-dihydro-5*H*-cyclopenta[c]pyridine-6-carbaldehyde (CAS# 932035-62-6) in place of 4-fluoro-1-methyl-6,7-dihydro-5*H*-cyclopenta[c]pyridine-6-carbaldehyde and was obtained as a light yellow solid. MS (ESP): m/z = 410.4 [M+H]⁺.

### Example 419

### 6-[5-[2-[[3-(Dimethylamino)-1-methyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one

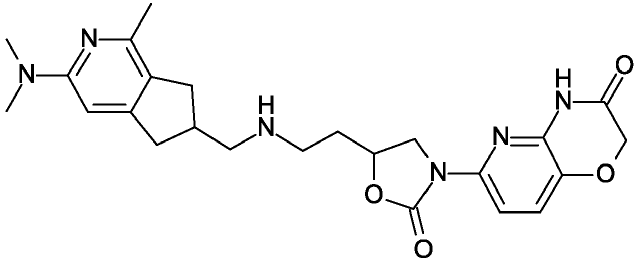

To a colorless solution of *tert*-butyl ((3-(dimethylamino)-1-methyl-6,7-dihydro-5*H-*cyclopenta[c]pyridin-6-yl)methyl)(2-(3-(4-(4-methoxybenzyl)-3-oxo-3,4-dihydro-2*H-*pyrido[3,2-b][1,4]oxazin-6-yl)-2-oxooxazolidin-5-yl)ethyl)carbamate (Intermediate 298, 54 mg, 62.9 µmol, 1 eq) in dichloromethane (1.22 mL) was added trifluoromethanesulfonic acid (66.1 mg, 38.9 µL, 440 µmol, 7 eq) at 22 °C. The mixture was stirred at RT for 5 min. Trifluoromethanesulfonic acid (34 mg, 20 µL, 226 µmol, 3.6 eq) was added and the mixture stirred for 3 hours at room temperature. To the reaction mixture was added potassium carbonate (86.9 mg, 629 µmol, 10 eq) and the mixture stirred for 30 min. at room temperature. The mixture was partitioned between dichloromethane (10 mL) and water (5 mL). The aqueous layer (pH 9-10) was extracted with dichloromethane (3 x 10 mL). The combined organic layers were dried over anhydrous sodium sulfate, filtered and concentrated in vacuo. The crude was purified over a silica gel column (4 g, 0-100% ethyl acetate in heptane and switch to 0-20% methanol in dichloromethane) to give the title compound (20.9 mg, 67.7% yield) as a light yellow solid. MS (ESN): m/z = 465.5 [M-H]⁻.

### Example 420

### 6-[5-[2-[[1-Methyl-3-(methylamino)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one

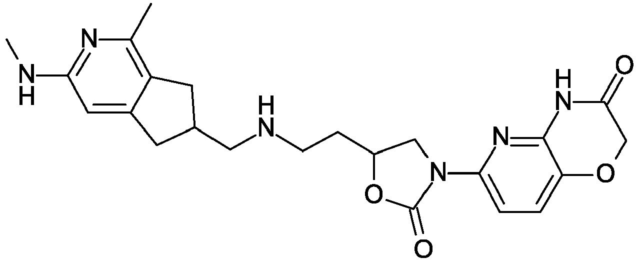

The title compound was prepared from *tert*-butyl (2-(3-(4-(4-methoxybenzyl)-3-oxo-3,4-dihydro-2*H*-pyrido[3,2-b][1,4]oxazin-6-yl)-2-oxooxazolidin-5-yl)ethyl)((1-methyl-3-(methylamino)-6,7-dihydro-5*H*-cyclopenta[c]pyridin-6-yl)methyl)carbamate (Intermediate 299) by analogy with 6-[5-[2-[[3-(dimethylamino)-1-methyl-6,7-dihydro-5*H*-cyclopenta[c]pyridin-6-yl]methylamino]ethyl]-2-oxo-1,3-oxazolidin-3-yl]-4*H*-pyrido[3,2-b][1,4]oxazin-3-one (Example 419) and was obtained as a white solid. MS (ESP): m/z = 453.4 [M+H]⁺.

### Example 422

### 6-[(5R)-5-[2-[[(1S,2S)-1-Amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one

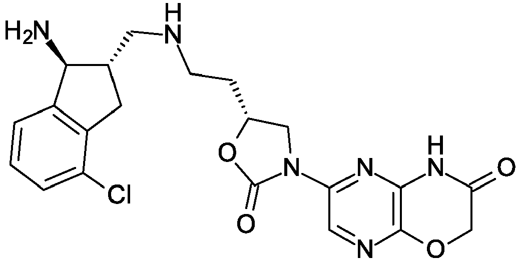

### Step 1:

### tert-Butyl N-[(1S,2S)-4-chloro-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-1-yl]carbamate

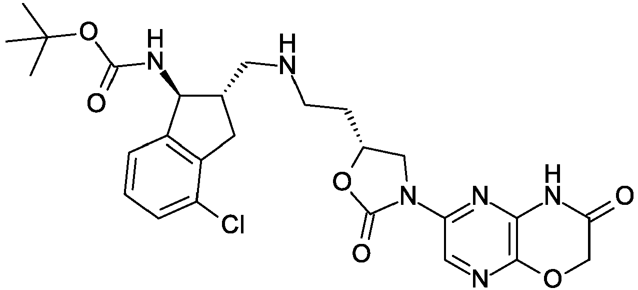

A suspension of *tert*-butyl *N*-[(1*S*,2*R*)-4-chloro-2-formyl-2,3-dihydro-1*H*-inden-1-yl]carbamate (Intermediate 287, 194 mg, 0.623 mmol, 1 eq), 6-[(5*R*)-5-(2-aminoethyl)-2-oxo-oxazolidin-3-yl]-4*H*-pyrazino[2,3-b][1,4]oxazin-3-one; formic acid (Intermediate 241, 203 mg, 0.623 mmol, 1 eq), acetic acid (56.1 mg, 53.5 µL, 0.935 mmol, 1.5 eq) and sodium triacetoxyborohydride (198 mg, 0.935 mmol, 1.5 eq) in THF (10 mL) and EtOH (19 mL) was stirred at RT overnight. Additional sodium triacetoxyborohydride (132 mg, 0.623 mmol, 1 eq) was added and the mixture was stirred at RT for 4 h. The mixture was concentrated. Water (basic with NaHCO₃) was added and mixture extracted with EtOAc (3 x). The combined organic layers were washed with brine (basic), dried over Na₂SO₄ and evaporated to give a residue which was purified by flash column chromatography (20 g silica gel; DCM/MeOH 98:2 - 90:10) to give the title compound (149 mg) as an off-white solid. MS (ESP): m/z = 559.4 [M+H]⁺.

### Step 2:

### 6-[(5R)-5-[2-[[(1S,2S)-1-Amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one

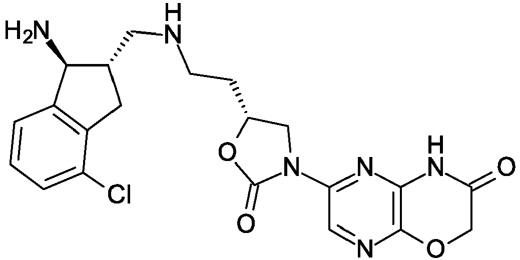

A mixture of *tert*-butyl *N*-[(1*S*,2*S*)-4-chloro-2-[[2-[(5*R*)-2-oxo-3-(3-oxo-4*H*-pyrazino[2,3-b] [1,4]oxazin-6-yl)-1,3-oxazolidin-5-yl]ethylamino]methyl]-2,3-dihydro-1*H*-inden-1-yl]carbamate (50 mg, 0.0894 mmol) in hexafluoroisopropanol (3 mL) was stirred in the microwave at 145 °C for 90 min. The volatiles were removed. MeCN was added and removed by evaporation several times. Water (12 mL) and MeCN (1 mL) were added and the mixture was lyophilized to give the title compound (31 mg) as a light brown solid. MS (ESP): m/z = 459.1 [M+H]⁺.

### Example 423

### 6-[5-[2-[(8-Methyl-5,7-diazatricyclo[7.3.0.0^{2,6}]dodeca-1,6,8-trien-11-yl)methylamino]ethyl]-2-oxo-1,3-oxazolidin-3-yl]-4H-pyrido[3,2-b][1,4]oxazin-3-one

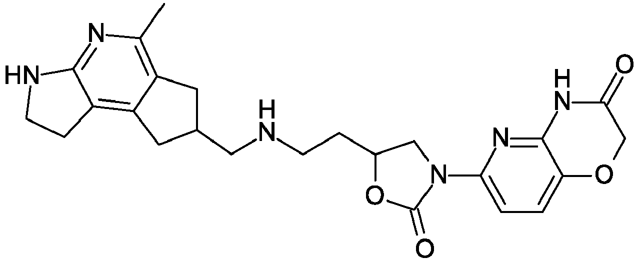

To a solution of 6-(5-(2-(((3-benzyl-5-methyl-1,2,3,6,7,8-hexahydrocyclopenta[d]pyrrolo[2,3-b]pyridin-7-yl)methyl)amino)ethyl)-2-oxooxazolidin-3-yl)-2*H*-pyrido[3,2-b][1,4]oxazin-3(4*H*)-one (Intermediate 301, 21.9 mg, 39.5µmol, 1 eq) and AcOH (2 mL) was added Pd-C 10% (3.2 mg, 29.6 µmol, 0.75 eq) under Ar. The reaction mixture was stirred under H₂ at 50 °C for 4h. The reaction mixture was filtered and the residue washed thoroughly with MeOH and then the filtrate was concentrated in vacuo at RT to give a residue. The residue was purified by flash chromatography (silica gel, 2g, 1% to 8% MeOH+NH₄OH in DCM then 20% MeOH+NH₄OH in DCM) to give the title compound (5.4 mg, 23% yield) as a light brown waxy solid. MS (ESP): m/z = 465.4 [M+H]⁺.

### Example 424

### 6-[5-[2-[(5,8-Dimethyl-5,7-diazatricyclo[7.3.0.0^{2,6}]dodeca-1,6,8-trien-11-yl)methylamino]ethyl]-2-oxo-1,3-oxazolidin-3-yl]-4H-pyrazino[2,3-b][1,4] oxazin-3 -one

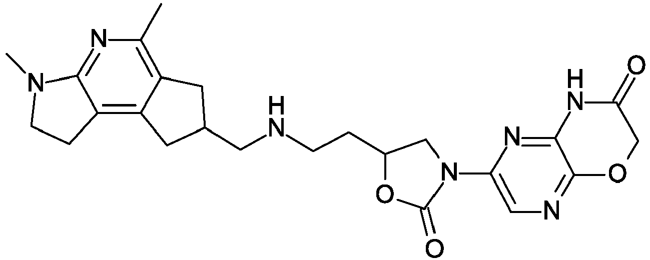

### Step 1:

### 6-[5-[2-[(5,8-Dimethyl-5,7-diazatricyclo[7.3.0.0^{2,6}]dodeca-1,6,8-trien-11-yl)methylamino]ethyl]-2-oxo-1,3-oxazolidin-3-yl]-4-(2-trimethylsilylethoxymethyl)pyrazino[2,3-b] [1,4] oxazin-3 -one

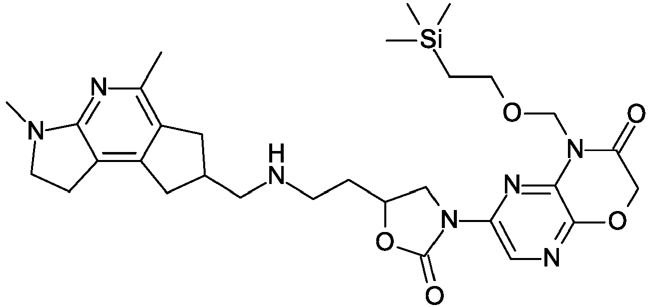

The title compound was prepared by analogy with 6-[5-[2-[(5,8-dimethyl-5,7-diazatricyclo[7.3.0.0^{2,6}]dodeca-1,6,8-trien-11-yl)methylamino]ethyl]-2-oxo-1,3-oxazolidin-3-yl]-4*H*-pyrido[3,2-b][1,4]oxazin-3-one (Example 416) using 6-[5-(2-aminoethyl)-2-oxo-oxazolidin-3-yl]-4-(2-trimethylsilylethoxymethyl)pyrazino[2,3-b][1,4]oxazin-3-one (Intermediate 302) in place of 6-[5-(2-aminoethyl)-2-oxo-1,3-oxazolidin-3-yl]-4*H*-pyrido[3,2-b][1,4]oxazin-3-one and was obtained as a light brown solid. MS (ESP): m/z = 610.5 [M+H]⁺.

### Step 2:

### 6-[5-[2-[(5,8-Dimethyl-5,7-diazatricyclo[7.3.0.0^{2,6}]dodeca-1,6,8-trien-11-yl)methylamino]ethyl]-2-oxo-1,3-oxazolidin-3-yl]-4H-pyrazino[2,3-b][1,4] oxazin-3 -one

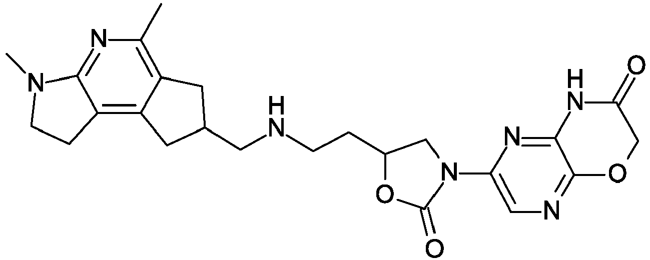

The title compound was prepared by analogy with *trans*-6-[8-[(4-fluoro-1-methyl-6,7-dihydro-5*H*-cyclopenta[c]pyridin-6-yl)amino]-2-oxo-1-oxa-3-azaspiro[4.5]decan-3-yl]-4*H*-pyrazino[2,3-b][1,4]oxazin-3-one; formic acid (Example 297, Step 2) and was obtained as a light brown solid. MS (ESP): m/z = 480.4 [M+H]⁺.

### Example 425

### 2-[[2-[2-Oxo-3-(3-oxo-4H-pyrazino[2,3-b][1,4]oxazin-6-yl)-1,3-oxazolidin-5-yl]ethylamino]methyl]-6-(1H-triazol-5-ylmethoxy)-2,3-dihydro-1H-indene-4-carbonitrile

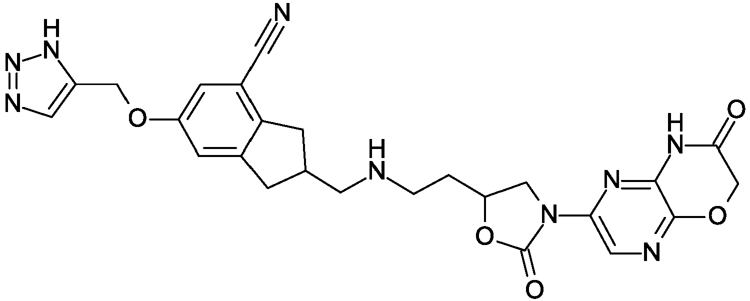

A mixture of 2-formyl-6-((1-trityl-1*H*-1,2,3-triazol-4-yl)methoxy)-2,3-dihydro-1*H*-indene-4-carbonitrile (Intermediate 303, 105 mg, 185 µmol, 1 eq), 6-(5-(2-aminoethyl)-2-oxooxazolidin-3-yl)-2H-pyrazino[2,3-b][1,4]oxazin-3(4H)-one 2,2,2-trifluoroacetate (Intermediate 24, 80.9 mg, 185 µmol, 1 eq) and *N,N*-diisopropylethylamine (35.9 mg, 48.5 µL, 278 µmol, 1.5 eq) in THF (1.5 mL) and DMF (0.2 mL) was stirred at RT for 15 min. Acetic acid (27.8 mg, 26.5 µL, 463 µmol, 2.5 eq) was added and the mixture stirred for 15 min. Then sodium triacetoxyborohydride (58.8 mg, 278 µmol, 1.5 eq) was added and the mixture stirred overnight. The mixture was diluted with water (basic with NaHCO₃) and extracted with EtOAc (3 x). The organic layers were washed with water (basic, 2 x) and brine (basic), dried over Na₂SO₄ and evaporated to give a residue. The residue was purified by silica cartridge chromatography (20 g; heptane to EtOAc to 10% MeOH-NH₄OH 10:1) to give 2-(((2-(2-oxo-3-(3-oxo-3,4-dihydro-2*H*-pyrazino[2,3-b] [1,4]oxazin-6-yl)oxazolidin-5-yl)ethyl)amino)methyl)-6-((1-trityl-1*H*-1,2,3-triazol-4-yl)methoxy)-2,3-dihydro-1*H*-indene-4-carbonitrile (90 mg, 93 µmol, 50.3 % yield) as a light brown oil. To a solution of this material in ethanol (2 mL) and THF (1 mL) was added HCl in water 1M (465 µL, 465 µmol, 5 eq) at RT and the mixture stirred for 1 h. Concentrated HCl (500 µL) was added and the mixture stirred for 1 h. Saturated aqueous NaHCO₃ was added and the mixture extracted with DCM. The organic phase was dried over Na₂SO₄, filtered and evaporated. The residue was purified by silica cartridge chromatography (10 g; DCM to 10% MeOH-NH₄OH 10:1) to give after lyophilisation the title compound (22 mg, 40.4 µmol, 43.4 % yield) as a white solid. MS (ESN): m/z = 530.4 [M-H]⁻.

### Example 427

### 6-[(5R)-5-[2-[[(6R)-4-Fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one

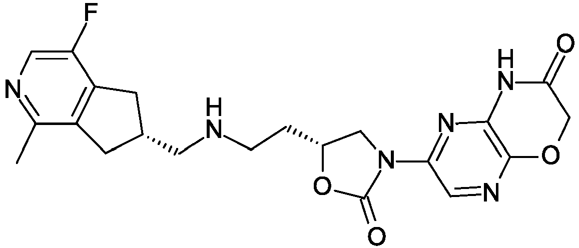

### Step 1:

### 6-((R)-5-(2-((((R)-4-Fluoro-1-methyl-6,7-dihydro-5H-cyclopenta[c]pyridin-6-yl)methyl)amino)ethyl)-2-oxooxazolidin-3-yl)-4-((2-(trimethylsilyl)ethoxy)methyl)-2H-pyrazino[2,3-b][1,4]oxazin-3(4H)-one

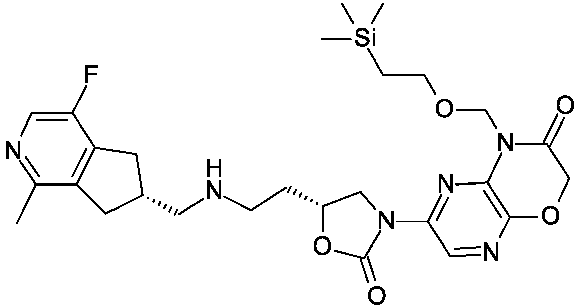

To a solution of (*R*)-6-(5-(2-aminoethyl)-2-oxooxazolidin-3-yl)-4-((2-(trimethylsilyl)ethoxy)methyl)-2*H*-pyrazino[2,3-b][1,4]oxazin-3(4*H*)-one hydrochloride (Intermediate 241 hydrochloride salt, 200 mg, 448 µmol, 1 eq) and (*R*)-4-fluoro-6-(iodomethyl)-1-methyl-6,7-dihydro-5*H*-cyclopenta[c]pyridine (Intermediate 305, 131 mg, 448 µmol, 1 eq) in MeCN (3.9 mL) was added potassium carbonate (155 mg, 1.12 mmol, 2.5 eq) and the mixture stirred at 90 °C for 2 days. SiO₂ (5 g) was added and the mixture evaporated. The residue was purified by silica cartridge chromatography (50 g; DCM to 10% MeOH/DCM) to give the title compound (58 mg, 101 µmol, 22.6 % yield) as a light brown oil. MS (ESN): m/z = 617.4 [M+HCOO]⁻.

### Step 2:

### 6-[(5R)-5-[2-[[(6R)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one

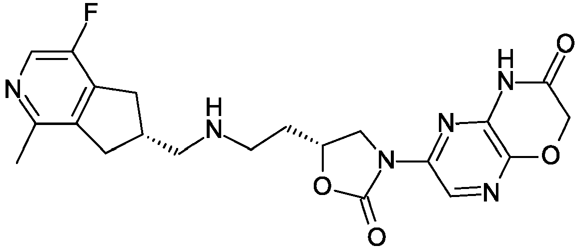

6-((*R*)-5-(2-((((*R*)-4-Fluoro-1-methyl-6,7-dihydro-5*H*-cyclopenta[c]pyridin-6-yl)methyl)amino)ethyl)-2-oxooxazolidin-3-yl)-4-((2-(trimethylsilyl)ethoxy)methyl)-2*H-*pyrazino[2,3-b][1,4]oxazin-3(4*H*)-one (58 mg, 101 µmol, 1 eq) was dissolved in TFA (1.15 g, 780 µl, 10.1 mmol, 100 eq) and the mixture stirred at RT for 1 h. The mixture was evaporated to dryness. The residue was dissolved in methanol (2 mL) and potassium carbonate (70 mg, 506 µmol, 5 eq) was added, then the mixture was stirred at RT for 1 h. Formic acid (46.6 mg, 38.8 µL, 1.01 mmol, 10 eq) was added and the mixture stirred at RT for 5 mins. 3g SiO₂ was added and the mixture evaporated totally. The residue was purified by silica cartridge chromatography (10 g, DCM to 14% MeOH/DCM). The product was mixed with MeCN (2 mL) and water (20 mL) and lyophilized overnight to give a mixture of formate and TFA salts of the title compound. Saturated aqueous NaHCO₃ (50 mL) was added and the mixture stirred with EtOAc for 10 mins. The organic phase was washed once with sat. NaHCO₃, water and brine, dried over Na₂SO₄, filtered and evaporated to give a residue which was purified by silica cartridge chromatography (10 g; DCM to 14% MeOH/DCM). The product was mixed with MeCN (1 mL) and water (20 mL) then lyophilized overnight to give the title compound (21.3 mg, 48.1 µmol, 47.5 % yield) as a white solid. MS (ESN): m/z = 441.3 [M-H]⁻.

The following examples were prepared by analogy to Example 427:

| **Ex.** | **Name, Structure, MS** | **Starting Materials** |
|---|---|---|
| **428** | 6-[(5*S*)-5-[2-[[(6*R*)-4-fluoro-1-methyl-6,7-dihydro-5*H-*cyclopenta[c]pyridin-6-yl]methylamino]ethyl]-2-oxo-1,3-oxazolidin-3-yl]-4*H*-pyrazino[2,3-b][1,4]oxazin-3-one | Intermediate 240 hydrochloride salt and Intermediate 305 |
| | 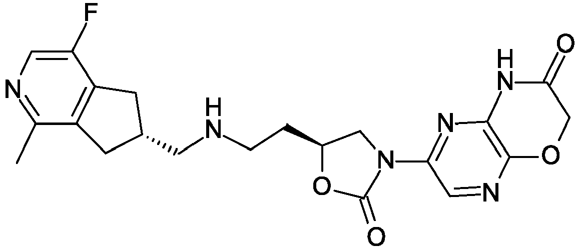 ESN [M-H]⁻: 441.3 | |
| **429** | 6-[(5*S*)-5-[2-[[(6*S*)-4-fluoro-1-methyl-6,7-dihydro-5*H-*cyclopenta[c]pyridin-6-yl]methylamino]ethyl]-2-oxo-1,3-oxazolidin-3-yl]-4*H*-pyrazino[2,3-b][1,4]oxazin-3-one | Intermediate 240 hydrochloride salt and Intermediate 306 |
| | 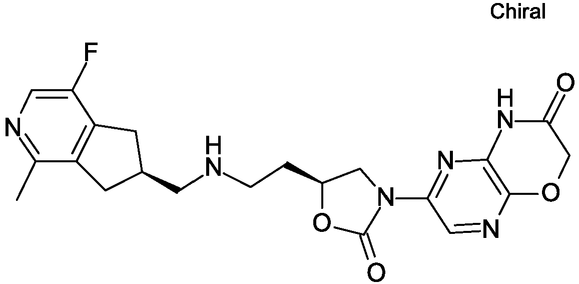 ESN [M-H]⁻: 441.3 | |
| **430** | 6-[(5*R*)-5-[2-[[(6*S*)-4-fluoro-1-methyl-6,7-dihydro-5*H-*cyclopenta[c]pyridin-6-yl]methylamino]ethyl]-2-oxo-1,3-oxazolidin-3-yl]-4*H*-pyrazino[2,3-b][1,4]oxazin-3-one | Intermediate 241 hydrochloride salt and Intermediate 306 |
| | 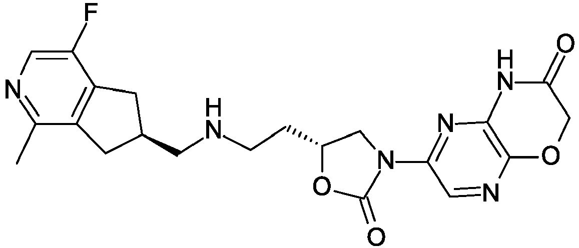 ESN [M-H]⁻: 441.3 | |

### Example 433

### 6-[5-[2-[[4-Fluoro-1-(1H-imidazol-5-yl)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one

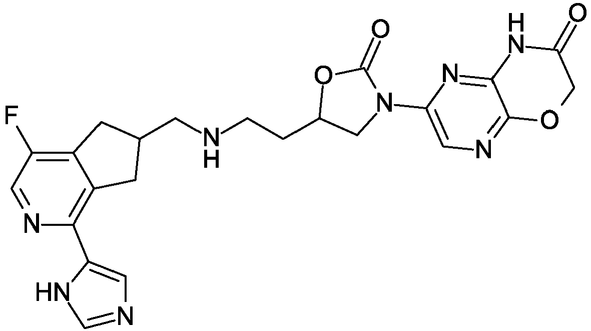

A solution of 4-fluoro-1-(1-tritylimidazol-4-yl)-6,7-dihydro-5*H*-cyclopenta[c]pyridine-6-carbaldehyde (Intermediate 308, 200.0 mg, 0.420 mmol, 1 eq), 6-[5-(2-aminoethyl)-2-oxo-oxazolidin-3-yl]-4*H*-pyrazino[2,3-b][1,4]oxazin-3-one;2,2,2-trifluoroacetic acid (Intermediate 24, 166.1 mg, 0.420 mmol, 1 eq) and *N*-ethyldiisopropylamine (0.11 mL, 0.630 mmol, 1.5 eq) in DMA (1.3 mL) and tetrahydrofuran (4 mL) was stirred at 25 °C for 15 min. Acetic acid (0.06 mL, 1.06 mmol, 2.5 eq) was added followed by sodium triacetoxyborohydride (134.3 mg, 0.630 mmol, 1.5 eq). The mixture was stirred at 25 °C for 18h. The reaction mixture was diluted with sat. aq. NaHCO₃ solution at 0 °C, stirred for 15 min. at room temperature and extracted with EtOAc (3 x). The combined organic layers were dried over Na₂SO₄, filtered and and evaporated. The resulting crude was purified by flash chromatography (SiO₂, 5g, DCM:MeOH, from 100% DCM to 98:2) to afford 6-[5-[2-[[4-fluoro-1-(1-tritylimidazol-4-yl)-6,7-dihydro-5*H-*cyclopenta[c]pyridin-6-yl]methylamino]ethyl]-2-oxo-oxazolidin-3-yl]-4*H*-pyrazino[2,3-b][1,4]oxazin-3-one (70 mg) as an off-white solid. To a solution of this material in DCM (3.7 mL) was added triethylsilane (18.2 µL, 0.110 mmol, 1.2 eq) followed by trifluoroacetic acid (36.6 µL, 0.480 mmol, 5 eq) and the mixture was stirred for 1 h. Sat. aq. NaHCO₃ was added and the mixture extracted with EtOAc (x 2) and DCM:MeOH (9:1). The combined organic phases were dried over Na₂SO₄, filtered and concentrated under vacuum. The resulting crude was purified by by flash chromatography (SiO₂, 5g, DCM:MeOH, from 100% DCM to 95:5) to give the title compound (20 mg, 0.040 mmol, 42.6% yield) as a white solid. MS (ESP): m/z = 495.1 [M+H]⁺.

### INTERMEDIATES

### Intermediate 1

### 6-[5-(2-Aminoethyl)-2-oxo-1,3-oxazolidin-3-yl]-4H-pyrido[3,2-b] [1,4]oxazin-3-one

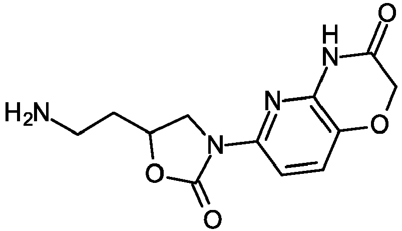

### Step 1:

### tert-Butyl-dimethyl-[2-(oxiran-2-yl)ethoxy] silane

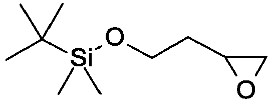

To a stirred solution of but-3-enoxy-tert-butyl-dimethyl-silane (CAS# 108794-10-1, 6.0 g, 32.19 mmol, 1 eq) in DCM (100 mL), cooled to 0 °C, was added portionwise meta chloroperbenzoic acid (16.67 g, 96.58 mmol, 3 eq) and the mixture stirred at 25 °C for 16 h. The reaction mixture was filtered to remove inorganics. Then, the filtrate was quenched with 10% sodium thiosulphate and extracted with DCM (50.0 mL × 3). The organic layer was combined and washed with 10% sodium bicarbonate (100.0 mL), dried over Na₂SO₄, filtered and concentrated to provide the crude title compound (6 g, 29.65 mmol, 92.1 % yield) as a light yellow oil.

### Step 2:

### 1-Amino-4-[tert-butyl(dimethyl)silyl]oxybutan-2-ol

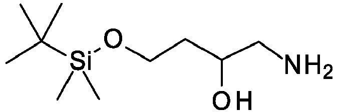

To a solution of *tert-*butyl-dimethyl-[2-(oxiran-2-yl)ethoxy]silane (6.0 g, 29.65 mmol, 1 eq) in methanol (50 mL) was added ammonium hydroxide (80.0 mL) at 20°C. The reaction mixture was stirred at 25 °C for 16 h. The reaction mixture was concentrated to provide the crude product. The crude product was purified by column chromatography (PE:EtOAc=2:1 to 1:3) to give the title compound (4 g, 18.23 mmol, 61.5% yield) as a yellow oil. MS (ESP): m/z = 220.3 [M+H]⁺.

### Step 3:

### 5-[2-[tert-Butyl(dimethyl)silyl]oxyethyl]-1,3-oxazolidin-2-one

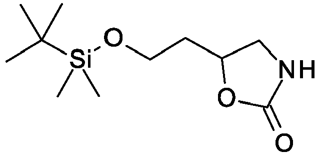

To a solution of 1-amino-4-[*tert*-butyl(dimethyl)silyl]oxybutan-2-ol (1000.0 mg, 4.56 mmol, 1 eq) and triethylamine (1.91 mL, 13.67 mmol, 3 eq) in DCM (20 mL) was added bis(trichloromethyl)carbonate (541.02 mg, 1.82 mmol, 0.400 eq) at 0 °C. The reaction mixture was stirred at 20 °C for 4 h. The reaction was quenched with water (20.0 mL) and extract with DCM (30.0 mL × 3). The organic layer was washed with brine, dried over Na₂SO₄, and concentrated under reduced pressure to give the crude product. It was purified by column chromatography on silica gel (PE:EA =3:1 to 1:1) to give the title compound (230 mg, 0.940 mmol, 20.6% yield) as a yellow oil.

### Step 4:

### Ethyl 2-[6-[5-[2-[tert-butyl(dimethyl)silyl]oxyethyl]-2-oxo-1,3-oxazolidin-3-yl]-2-nitropyridin-3-yl]oxyacetate

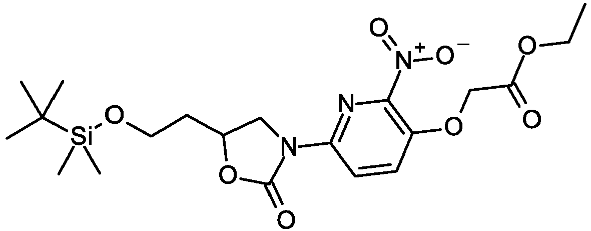

To a solution of 5-[2-[*tert*-butyl(dimethyl)silyl]oxyethyl]-1,3-oxazolidin-2-one (1327.17 mg, 5.41 mmol, 1.1 eq) and ethyl 2-[(6-bromo-2-nitro-3-pyridyl)oxy]acetate (CAS# 443956-09-0, 1500.0 mg, 4.92 mmol, 1 eq) in 1,4-dioxane (30 mL) was added potassium carbonate (1362.1 mg, 9.86 mmol, 2 eq), copper (I) iodide (93.64 mg, 0.490 mmol, 0.100 eq) and N,N-dimethylethylenediamine (0.01 mL, 0.490 mmol, 0.100 eq). The mixture was degassed with nitrogen three times and stirred at 100 °C for 4 h. The mixture was filtered and the filtrate was concentrated under vacuum to give the crude product (3.0 g). The crude product was purified by flash chromatography on silica gel (PE/EtOAc=10/1 to 3/1) to give the title compound (1100 mg, 2.34 mmol, 47.7 % yield) as a light yellow solid. MS (ESP): m/z = 492.2 [M+Na]⁺.

### Step 5:

### 6-[5-[2-[tert-Butyl(dimethyl)silyl]oxyethyl]-2-oxo-1,3-oxazolidin-3-yl]-4H-pyrido[3,2-b][1,4]oxazin-3-one

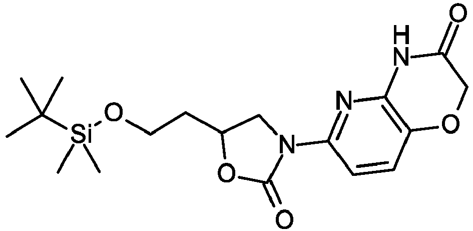

A mixture of ethyl 2-[6-[5-[2-[*tert*-butyl(dimethyl)silyl]oxyethyl]-2-oxo-1,3-oxazolidin-3-yl]-2-nitropyridin-3-yl]oxyacetate (1100.0 mg, 2.34 mmol, 1 eq) and iron (654.1 mg, 11.71 mmol, 5 eq) in acetic acid (20 mL) was stirred at 70 °C for 2 h. The reaction was cooled to room temperature and concentrated under vacuum to give the residue. The residue was re-dissolved in EtOAc (20 mL) and then filtered, the filtrate was concentrated under vacuum to give the crude product (1.2 g). The crude product was purified by flash chromatography on silica gel (PE/EtOAc=3/1 to 1/1) to give the title compound (850 mg, 2.16 mmol, 85.0 % yield) as a white solid. MS (ESP): m/z = 394.2 [M+H]⁺.

### Step 6:

### 6-[5-(2-Hydroxyethyl)-2-oxo-1,3-oxazolidin-3-yl]-4H-pyrido[3,2-b][1,4]oxazin-3-one

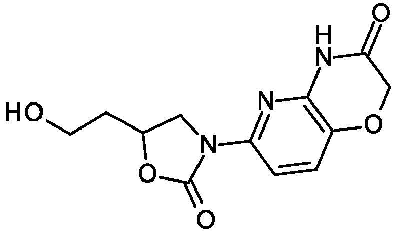

To a mixture of 6-[5-[2-[*tert*-butyl(dimethyl)silyl]oxyethyl]-2-oxo-1,3-oxazolidin-3-yl]-4*H-*pyrido[3,2-b][1,4]oxazin-3-one (850.0 mg, 2.16 mmol, 1 eq) in methanol (10 mL) was added HCl/MeOH (5.0 mL, 10 mmol, 4.63 eq) and then the reaction was stirred at 20 °C for 2 h. The mixture was concentrated under vacuum to give the crude product (0.7 g). The crude product was triturated with EtOAc (5 mL) and then filtered and dried under vacuum to give the title compound (480 mg, 1.72 mmol, 79.6% yield) as a white solid. MS (ESP): m/z = 280.1 [M+H]⁺.

### Step 7:

### 2-[2-Oxo-3-(3-oxo-4H-pyrido[3,2-b][1,4]oxazin-6-yl)-1,3-oxazolidin-5-yl]ethyl methanesulfonate

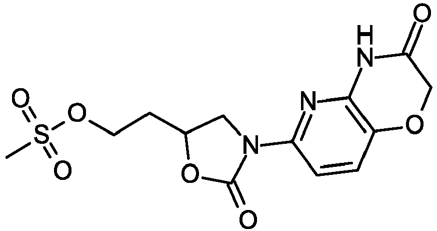

To a mixture of 6-[5-(2-hydroxyethyl)-2-oxo-1,3-oxazolidin-3-yl]-4*H*-pyrido[3,2-b][1,4]oxazin-3-one (480.0 mg, 1.72 mmol, 1 eq) and triethylamine (0.72 mL, 5.16 mmol, 3 eq) in DCM (30 mL) under ice-bath cooling was added methanesulfonyl chloride (0.16 mL, 2.06 mmol, 1.2 eq) then the reaction was warmed to 25 °C and stirred for 2 h. Further methanesulfonyl chloride (0.13 mL, 1.72 mmol, 1 eq) was added and the reaction was stirred at 25 °C for another 2 h. The reaction was quenched with saturated NaHCO₃ solution (20 mL) and then extracted with DCM (2 × 20 mL). The organic phase was concentrated to give the title compound (650 mg, 1.82 mmol, 90.1 % yield) as a light yellow solid. The crude product was used for the next step directly. MS (ESP): m/z = 358.1 [M+H]⁺.

### Step 8:

### 6-[5-(2-Azidoethyl)-2-oxo-1,3-oxazolidin-3-yl]-4H-pyrido[3,2-b][1,4]oxazin-3-one

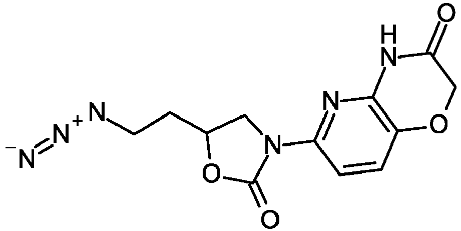

To a mixture of 2-[2-oxo-3-(3-oxo-4*H*-pyrido[3,2-b][1,4]oxazin-6-yl)-1,3-oxazolidin-5-yl]ethyl methanesulfonate (650.0 mg, 1.82 mmol, 1 eq) in DMF (15 mL) was added sodium azide (0.32 mL, 9.09 mmol, 5 eq) and the reaction was stirred at 60 °C for 3 h. The reaction was cooled to 25 °C and then quenched with brine solution (20 mL). The mixture was extracted with EtOAc (2 × 30 mL) and then washed brine solution (3 × 20 mL). The organic phase was concentrated under vacuum to give the title compound (500 mg, 1.64 mmol, 74.4 % yield) as a white solid. MS (ESP): m/z = 305.1 [M+H]⁺.

### Step 9:

### 6-[5-(2-Aminoethyl)-2-oxo-1,3-oxazolidin-3-yl]-4H-pyrido[3,2-b] [1,4]oxazin-3-one

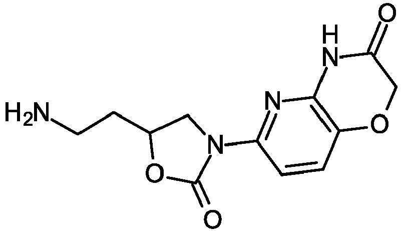

A mixture of 6-[5-(2-azidoethyl)-2-oxo-1,3-oxazolidin-3-yl]-4*H*-pyrido[3,2-b] [1,4]oxazin-3-one (500.0 mg, 1.64 mmol, 1 eq) and Pd/C (50.0 mg) in methanol (10 mL) under an atmosphere of hydrogen was stirred at 20 °C for 2 h. The mixture was filtered and the filtrate was concentrated under vacuum to give the crude title compound (400 mg, 1.44 mmol, 87.5 % yield) as a white solid which was used for the next step without purification. MS (ESP): m/z = 279.1 [M+H]⁺.

### Intermediate 2

### 6-[5-(3-Aminopropyl)-2-oxo-1,3-oxazolidin-3-yl]-4H-pyrido[3,2-b][1,4]oxazin-3-one

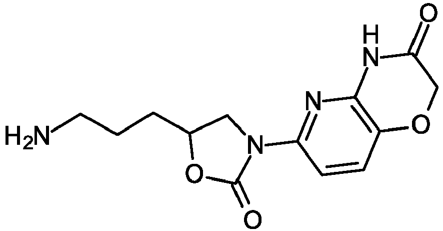

The title compound was prepared by analogy to Intermediate 1 using *tert*-butyl-dimethyl-pent-4-enoxysilane (CAS# 127154-84-1) in place of but-3-enoxy-*tert*-butyl-dimethyl-silane, and was obtained as an off-white solid. MS (ESP): m/z = 293.1 [M+H]⁺.

### Intermediate 3

### 6-[5-(2-Aminoethyl)-2-oxo-1,3-oxazolidin-3-yl]-4H-pyrido[3,2-b][1,4]oxazin-3-one, Enantiomer A and

### Intermediate 4

### 6-[5-(2-Aminoethyl)-2-oxo-1,3-oxazolidin-3-yl]-4H-pyrido[3,2-b][1,4]oxazin-3-one, Enantiomer B

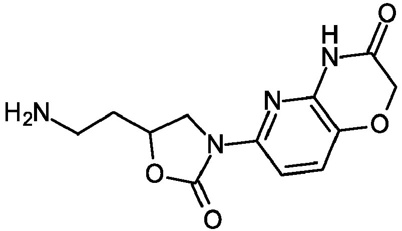

The title compounds were prepared by analogy to 6-[5-(2-aminoethyl)-2-oxo-1,3-oxazolidin-3-yl]-4*H*-pyrido[3,2-b][1,4]oxazin-3-one (Intermediate 1, Steps 6 to 9) using 6-[5-(2-hydroxyethyl)-2-oxo-1,3-oxazolidin-3-yl]-4*H*-pyrido[3,2-b][1,4]oxazin-3-one, Enantiomer A and 6-[5-(2-aminoethyl)-2-oxo-1,3-oxazolidin-3-yl]-4*H*-pyrido[3,2-b][1,4] oxazin-3 -one, Enantiomer B respectively in place of racemic 6-[5-(2-hydroxyethyl)-2-oxo-1,3-oxazolidin-3-yl]-4*H*-pyrido[3,2-b][1,4]oxazin-3-one.

The 6-[5-(2-hydroxyethyl)-2-oxo-1,3-oxazolidin-3-yl]-4*H*-pyrido[3,2-b][1,4]oxazin-3-one, Enantiomer A and 6-[5-(2-aminoethyl)-2-oxo-1,3-oxazolidin-3-yl]-4*H*-pyrido[3,2-b][1,4]oxazin-3-one, Enantiomer B used as starting materials were prepared by chiral SFC separation of racemic 6-[5-(2-hydroxyethyl)-2-oxo-1,3-oxazolidin-3-yl]-4*H*-pyrido[3,2-b][1,4]oxazin-3 -one (1000.0 mg, 3.58 mmol, 1 eq) eluting with 0.1% NH₃.H₂O-MeOH, column: DAICEL CHIRALPAK AD (250mm*30mm, 10 µm), to give two isomers: peak 1 (hold time: 1.002): 6-[5-(2-hydroxyethyl)-2-oxo-1,3-oxazolidin-3-yl]-4*H*-pyrido[3,2-b][1,4]oxazin-3-one, Enantiomer A (450 mg, 1.61 mmol, 44.1% yield) as a yellow solid; peak 2 (hold time: 1.499): 6-[5-(2-aminoethyl)-2-oxo-1,3-oxazolidin-3-yl]-4*H*-pyrido[3,2-b][1,4]oxazin-3-one, Enantiomer B (450 mg, 1.61 mmol, 44.1 % yield) as a yellow solid.

### Intermediate 5

### 2-Formyl-2,3-dihydro-1H-indene-4-carbonitrile

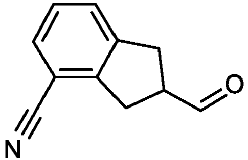

### Step 1:

### (4-Bromo-2,3-dihydro-1H-inden-2-yl)methanol

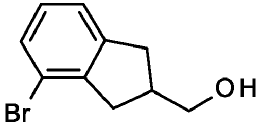

To a solution of 4-bromoindane-2-carboxylic acid (CAS# 209224-95-3, 14.13 g, 58.59 mmol, 1 eq) in THF (400 mL) was added dropwise borane-methyl sulfide complex (17.58 mL, 175.78 mmol, 3 eq) at -78 °C, and then the mixture was warmed slowly to 20 °C during 2 h. The reaction was quenched slowly by addition of MeOH (100 mL) and refluxed for 0.5 h. The mixture was purified directly by column chromatography (PE/ethyl acetate=5:1) to give the title compound (10.2 g, 44.91 mmol, 76.7 % yield) as a light yellow oil. ¹H-NMR (400 MHz, CDCl₃) δ 7.32 (d, J=7.91 Hz, 1H), 7.15 (d, J=7.40 Hz, 1H), 6.99-7.06 (m, 1H), 3.58-3.78 (m, 2H), 3.04-3.30 (m, 2H), 2.61-2.95 (m, 3H).

### Step 2:

### 2-(Hydroxymethyl)-2,3-dihydro-1H-indene-4-carbonitrile

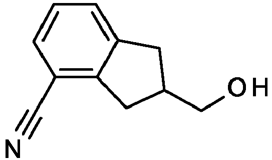

A suspension of (4-bromo-2,3-dihydro-1*H*-inden-2-yl)methanol (10.02 g, 44.13 mmol, 1 eq), zinc cyanide (1.68 mL, 26.48 mmol, 0.60 eq), zinc (288.6 mg, 4.41 mmol, 0.10 eq) and 1,1'-bis(diphenylphosphino)ferrocene (2.45 g, 4.41 mmol, 0.10 eq), tetrakis(triphenylphosphine)palladium(0) (5.1 g, 4.41 mmol, 0.10 eq) in *N,N*-dimethylacetamide (400 mL) was stirred at 100 °C under nitrogen atmosphere for 16 h. The reaction was quenched with water (400 mL) and the mixture was extracted with ethyl acetate (500 mL × 2). The combined organic phase was washed with H₂O (300 mL × 3) and brine (500 mL). The combined organic layer was dried over sodium sulfate, then concentrated to give a residue, which was purified by column chromatography (PE/EtOAc=5:1 to 3:1) to give the title compound (7.5 g, 43.3 mmol, 98.1 % yield) as a light brown oil. ¹H-NMR (400 MHz, CDCl₃) δ 7.44 (dd, J=2.20, 7.59 Hz, 2H), 7.20-7.27 (m, 1H), 3.63-3.76 (m, 2H), 3.09-3.33 (m, 2H), 3.03 (s, 1H), 2.99 (br d, J=6.15 Hz, 1H), 2.76-2.92 (m, 1H).

### Step 3:

### 2-Formyl-2,3-dihydro-1H-indene-4-carbonitrile

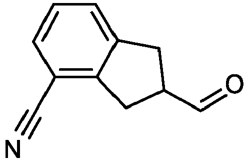

To a solution of 2-(hydroxymethyl)-2,3-dihydro-1H-indene-4-carbonitrile (60.0 mg, 0.350 mmol, 1 eq) in DCM (2.5 mL), was added Dess-Martin periodinane (191.0 mg, 0.450 mmol, 1.3 eq) at 0 °C. Then the solution was stirred at 25 °C for 2 h. The mixture was filtered and the filtrate was concentrated under vacuum to give the crude product which was purified by TLC (EtOAc:PE=1:1) to give the title compound (50 mg, 0.290 mmol, 50.1 % yield) as a colourless oil. m/z = 172.2 [M+H]⁺.

### Intermediate 6

### 2-Oxo-1,3-dihydroindene-4-carbonitrile

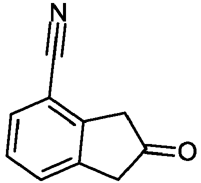

### Step 1:

### 4-Bromospiro[1,3-dihydroindene-2,2'-1,3-dioxolane]

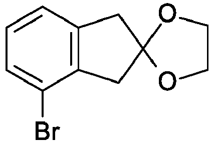

To a mixture of 4-bromo-1,3-dihydroinden-2-one (3.0 g, 14.2 mmol, 1 eq, CAS# 846032-36-8) in toluene (20 ml) was added p-toluenesulfonic acid monohydrate (541 mg, 2.8 mmol, 0.2 eq) followed by ethylene glycol (1.6 ml, 28.4 mmol, 2 eq). Using a Dean-Stark apparatus the mixture was refluxed for 2.5 h. The mixture was partitioned between sat. aq. NaHCO₃ and ethyl acetate. The organic layer was washed with water (2 x) and evaporated to give the title compound (3.1 g, 12.2 mmol, 85.5 % yield) as a brown liquid. MS (ESP): m/z = 255.1 [M+H]⁺.

### Step 2:

### Spiro [1,3 -dihydroindene-2,2'-1,3 -dioxolane] -4-carbonitrile

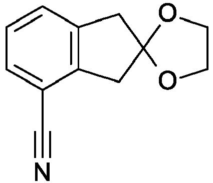

4-Bromospiro[1,3-dihydroindene-2,2'-1,3-dioxolane] (3.1 g, 11.3 mmol, 1 eq) was dissolved in *N,N*-dimethylformamide (75 mL) and zinc cyanide (2.7 g, 22.6 mmol, 2 eq) was added. The mixture was stirred for about 10 min. while degasing with argon. The mixture was inserted into a preheated oilbath at 160 °C. Then tetrakis(triphenylphosphine)palladium(0) (653 mg, 565 µmol, 0.05 eq) was added and the mixture was stirred for 1 h at 160 °C and then poured onto water. The solids were filtered off and the fitrate was extracted with methyl *tert-butyl* ether (3 x) and the combined organics were washed with water (2 x) and dried over Na₂SO₄. After evaporation the residue was purified by flash chromatography on silica gel (0-100% ethyl acetate in heptane) to give the title compound (1.9 g, 9.3 mmol, 82 % yield) as a light brown solid. MS (ESP): m/z = 202.1 [M+H]⁺.

### Step 3:

### 2-Oxo-1,3-dihydroindene-4-carbonitrile

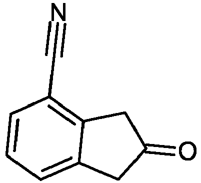

Spiro[1,3-dihydroindene-2,2'-1,3-dioxolane]-4-carbonitrile (1 g, 5.0 mmol, 1 eq) was dissolved acetonitrile (20 mL) followed by the addition of 1M HCl (20 ml, 20 mmol, 4 eq) while cooling in an ice bath. After stirring at room temperature for 4 days sat. aq. NaHCO₃ and water were added. The mixtue was extracted with ethyl acetate (2 x). The combined organic layers were washed once with water and brine, dried over Na₂SO₄ and evaporated to give the title compound (780 mg, 5.0 mmol, quant. yield) as a light brown solid MS (ESN): m/z = 156.0 [M-H]⁻.

### Intermediate 7

### 6-[5-(trans-3-Aminocyclobutyl)-2-oxo-1,3-oxazolidin-3-yl]-4H-pyrido[3,2-b][1,4]oxazin-3-one; 2,2,2-trifluoroacetic acid

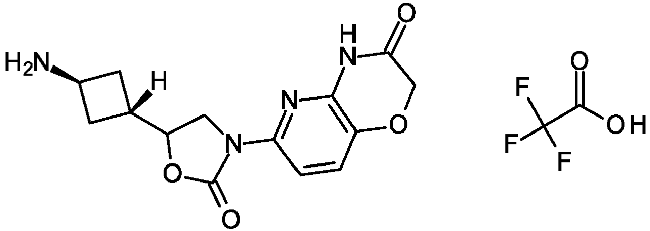

### Step 1:

### tert-butyl N-[trans-3-(1-hydroxy-2-nitroethyl)cyclobutyl]carbamate

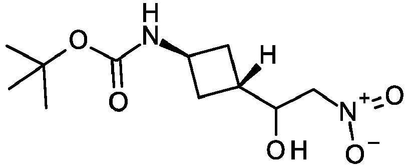

To a solution of *tert-butyl N*-(*trans*-3-formylcyclobutyl)carbamate (CAS# 171549-92-1, 1400.0 mg, 7.03 mmol, 1 eq) in nitromethane (14.0 mL, 258.49 mmol, 36.79 eq) was added triethylamine (0.49 mL, 3.51 mmol, 0.500 eq) at 20 °C slowly. Then the mixture was stirred at 20°C for 2 h. The mixture was purified by silica gel column chromatography (petroleum ether:acetate ethyl = 5:1 to 1:1) to give the title compound (1300 mg, 4.99 mmol, 71.1% yield) as a white solid. ¹H-NMR (400 MHz, CDCl₃) δ 4.73 (br s, 1H), 4.37 (m, 3H), 4.13 (m, 1H), 2.97 (br s, 1H), 2.45 (m, 1H), 2.27 (m, 2H), 2.06 (m, 2H), 1.43 (s, 9H).

### Step 2:

### tert-Butyl N-[trans-3-(2-amino-1-hydroxyethyl)cyclobutyl]carbamate

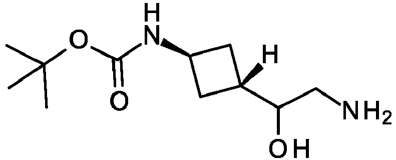

To a solution of *tert*-butyl *N*-[*trans*-3-(1-hydroxy-2-nitroethyl)cyclobutyl]carbamate (1300.0 mg, 4.99 mmol, 1 eq) in methanol (60 mL) was added Pd(OH)₂/C (360.0 mg). The mixture was stirred under H₂ at 30°C for 12 h at 50 psi. The mixture was filtered and the filtrate was concentrated in vacuo to give the title compound (1200 mg, 5.21 mmol, quant. yield) as a white solid. The crude was used for next step directly.

### Step 3:

### tert-Butyl N-[trans-3-(2-oxo-1,3-oxazolidin-5-yl)cyclobutyl]carbamate

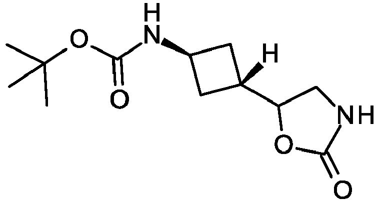

To a solution of *tert*-butyl *N*-[*trans*-3-(2-amino-1-hydroxyethyl)cyclobutyl]carbamate (500.0 mg, 2.17 mmol, 1 eq) in THF (10 mL) was added *N,N*'-carbonyldiimidazole (528.06 mg, 3.26 mmol, 1.5 eq). The mixture was heated to 50 °C and stirred for 0.250 h. The mixture was purified by silica gel column chromatography (petroleum ether:acetate ethyl =3:1 to 1:1) to give the title compound (410 mg, 1.6 mmol, 73.7 % yield) as a white solid.

### Step 4:

### tert-Butyl N-[trans-3-[2-oxo-3-[3-oxo-4-(2-trimethylsilylethoxymethyl)pyrido[3,2-b][1,4]oxazin-6-yl]-1,3-oxazolidin-5-yl]cyclobutyl]carbamate

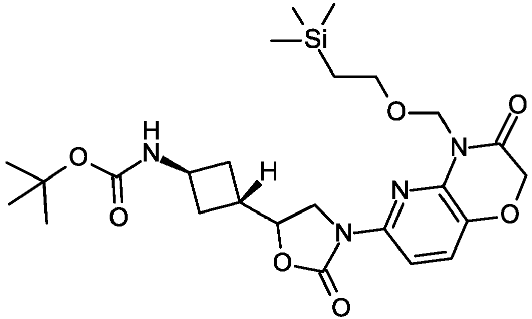

To a solution of 6-bromo-4-(2-trimethylsilylethoxymethyl)pyrido[3,2-b][1,4]oxazin-3-one (Intermediate 8, 574.75 mg, 1.6 mmol, 1 eq) and *tert*-butyl *N*-[*trans*-3-(2-oxo-1,3-oxazolidin-5-yl)cyclobutyl]carbamate (410.0 mg, 1.6 mmol, 1 eq) in 1,4-dioxane (15 mL) was added *trans-N,N*'-dimethylcyclohexane-1,2-diamine (136.44 mg, 0.960 mmol, 0.600 eq), potassium carbonate (1548 mg, 11.2 mmol, 7 eq) and copper (I) iodide (91.4 mg, 0.480 mmol, 0.300 eq). The mixture was heated to 100 °C and stirred for 4 h. The mixture was filtered through silica and concentrated in vacuo to give a crude product. The crude product was purified by silica gel column chromatography (petroleum ether:acetate ethyl = 5:1 to 1:1) to give the title compound (520 mg, 0.970 mmol, 60.8 % yield) as a white solid. ¹H-NMR (400 MHz, CDCl₃) δ 7.86 (d, 1H), 7.33 (m, 1H), 5.62 (s, 2H), 4.65 (m, 3H), 4.26 (m, 1H), 3.71 (m, 3H), 2.54 (m, 2H), 2.30 (m, 1H), 2.13 (m, 2H), 1.44 (s, 9H), 0.92 (t, 2H), -0.04 (s, 9H).

### Step 5:

### 6-[5-(trans-3-Aminocyclobutyl)-2-oxo-1,3-oxazolidin-3-yl]-4H-pyrido[3,2-b] [1,4]oxazin-3-one; 2,2,2-trifluoroacetic acid

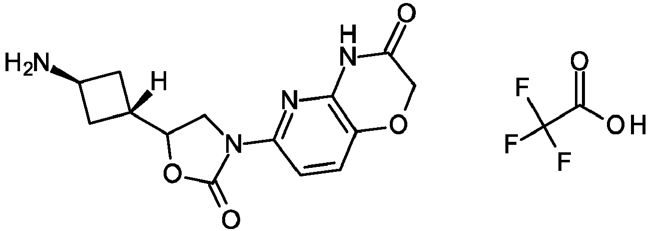

A mixture of *tert*-butyl *N*-[*trans*-3-[2-oxo-3-[3-oxo-4-(2-trimethylsilylethoxymethyl)pyrido[3,2-b][1,4]oxazin-6-yl]-1,3-oxazolidin-5-yl]cyclobutyl]carbamate (350.0 mg, 0.650 mmol, 1 eq) in trifluoroacetic acid (4.0 mL, 51.92 mmol, 79.31 eq) was stirred at 60 °C for 12 h. The mixture was concentrated in vacuo to give the title compound (300 mg, 0.990 mmol, 75.3% yield) as dark brown oil. The crude product was used for next step directly. MS (ESP): m/z = 305.2 [M+H]⁺.

### Intermediate 8

### 6-Bromo-4-(2-trimethylsilylethoxymethyl)pyrido[3,2-b][1,4]oxazin-3-one

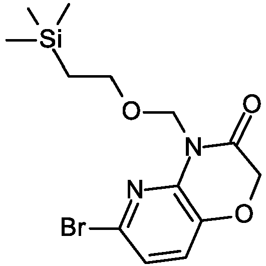

To a solution of 6-bromo-4*H*-pyrido[3,2-b][1,4]oxazin-3-one (CAS# 337463-88-4, 2000.0 mg, 8.73 mmol, 1 eq) in DMF (30 mL) was added sodium hydride, 60% in oil (523.9 mg, 13.1 mmol, 1.5 eq) at 0 °C and the reaction mixture was stirred at room temperature for 1 h. To the mixture was added slowly dropwise 2-(trimethylsilyl)ethoxymethyl chloride (2.32 mL, 13.1 mmol, 1.5 eq) and the resulting mixture was stirred at 25 °C for another 3 h. The reaction was quenched with aq. NH₄Cl (30 mL) and extracted with EtOAc (3 × 50 mL). The combined organic layers were collected, washed with brine (2 × 30 mL), 5% citric acid (20 mL), dried and concentrated to give a crude product, which was purified by flash chromatography (15% EA/PE) to give the title compound (2360 mg, 6.57 mmol, 71.7% yield) as a white solid. MS (ESP): m/z = 381.0 [M+Na]⁺.

### Intermediate 9

### 6-[5-(2-Aminoethyl)-2-oxo-1,3-oxazolidin-3-yl]-4H-1,4-benzoxazin-3-one

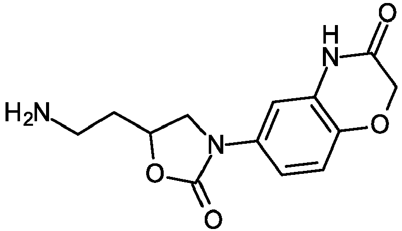

### Step 1:

### tert-Butyl N-[3-hydroxy-4-[(3-oxo-4H-1,4-benzoxazin-6-yl)amino]butyl]carbamate

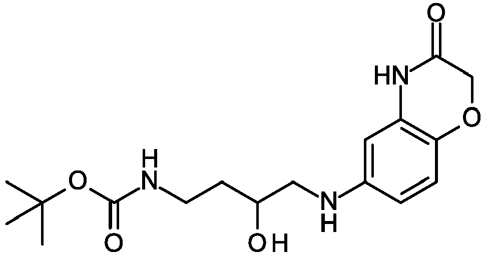

To a solution of *tert*-butyl *N*-[2-(oxiran-2-yl)ethyl]carbamate (CAS# 1072793-83-9, 260.06 mg, 1.39 mmol, 1.2 eq) in ethanol (9 mL)/water (1 mL) was added 6-amino-4*H*-1,4-benzoxazin-3-one (CAS# 24036-52-0, 190.0 mg, 1.16 mmol, 1 eq) at 20 °C. The reaction mixture was stirred at 90 °C for 16 h. The reaction mixture was concentrated to provide the crude product. The crude product was dissolved into DMF (2.0 mL) and purified by prep-HPLC (formic acid) to give the title compound (100 mg, 0.280 mmol, 24.6% yield) as yellow solid. MS (ESP): m/z = 352.3 [M+H]⁺.

### Step 2:

### tert-Butyl N- [2- [2-oxo-3 -(3 -oxo-4/7-1,4-benzoxazin-6-yl)-1,3 -oxazolidin-5-yl] ethyl] carbamate

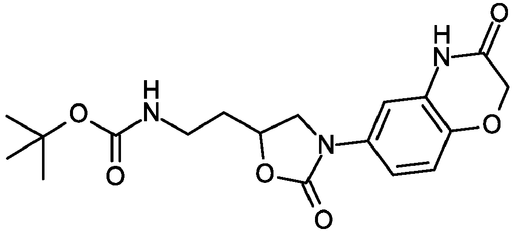

To a solution of *tert*-butyl *N*-[3-hydroxy-4-[(3-oxo-4*H*-1,4-benzoxazin-6-yl)amino]butyl]carbamate (100.0 mg, 0.280 mmol, 1 eq) in THF (10 mL) was added *N.N'-*carbonyldiimidazole (69.22 mg, 0.430 mmol, 1.5 eq) at 20 °C. The reaction mixture was stirred at 50 °C for 16 h. The reaction mixture was concentrated to provide the crude product. The crude product was purified by TLC (DCM: MeOH=10:1) to give the title compound (90 mg, 0.240 mmol, 83.8 % yield) as colourless oil. MS (ESP): m/z = 278.2 [M-Boc+2H]⁺.

### Step 3:

### 6-[5-(2-Aminoethyl)-2-oxo-1,3-oxazolidin-3-yl]-4H-1,4-benzoxazin-3-one

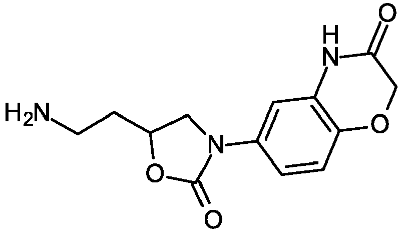

To a solution of *tert*-butyl *N*-[2-[2-oxo-3-(3-oxo-4*H*-1,4-benzoxazin-6-yl)-1,3-oxazolidin-5-yl]ethyl]carbamate (90.0 mg, 0.240 mmol, 1 eq) in EtOAc (5 mL) was added HCl in EtOAc (5.0 mL) at 20 °C. The reaction mixture was stirred at 20 °C for 1 h. The reaction mixture was concentrated to provide the title compound (60 mg, 0.220 mmol, 90.7 % yield) as a white solid. The crude product was used in the next step without further purification. MS (ESP): m/z = 278.2 [M+H]⁺.

### Intermediate 10

### 6-[5-(cis-3-Aminocyclobutyl)-2-oxo-1,3-oxazolidin-3-yl]-4H-pyrido[3,2-b][1,4]oxazin-3-one; formic acid

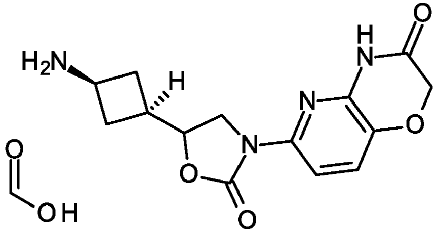

The title compound was prepared by analogy with Intermediate 7 using *tert*-butyl *N*-(*cis*-3-formylcyclobutyl)carbamate (CAS# 171549-91-0) in place of *tert-*butyl *N-*(*trans*-3-formylcyclobutyl)carbamate, and was obtained as a yellow solid. MS (ESP): m/z = 305.0 [M+H]⁺.

### Intermediate 11

### 2-(4-Chloro-2,3-dihydro-1H-inden-2-yl)acetaldehyde

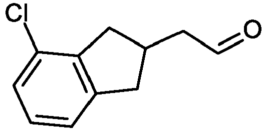

### Step 1:

### (4-Chloro-2,3-dihydro-1H-inden-2-yl)methyl methanesulfonate

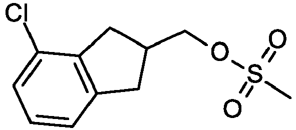

To a solution of (4-chloro-2,3-dihydro-1*H*-inden-2-yl)methanol (CAS# 1823383-27-2, 200.0 mg, 1.09 mmol, 1 eq) in DCM (5 mL) was added *N*,*N*-diisopropylethylamine (0.38 mL, 2.19 mmol, 2 eq) and methanesulfonyl chloride (0.1 mL, 1.31 mmol, 1.2 eq) at 0 °C . The mixture was stirred at 20 °C for 3 h. The mixture was poured into water (20 mL), extracted with DCM (15 mL × 3), the organic layer was washed with HCl (0.5 M, 10 mL), saturated NaHCO₃ (10 mL), brine (10 mL), dried over anhydrous Na₂SO₄ and concentrated in vacuo to afford the title compound (260 mg, 1 mmol, 91.1 % yield) as a yellow oil. ¹H-NMR (400 MHz, CDCl₃) δ 7.12 (m, 3H), 4.25 (d, 2H), 3.20 (m, 2H), 3.03 (s, 3H), 2.88 (m, 3H).

### Step 2:

### 2-(4-Chloro-2,3-dihydro-1H-inden-2-yl)acetonitrile

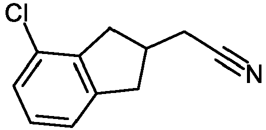

To a solution of (4-chloro-2,3-dihydro-1*H*-inden-2-yl)methyl methanesulfonate (140.0 mg, 0.540 mmol, 1 eq) in DMF (5 mL) was addded sodium cyanide (79.04 mg, 1.61 mmol, 3 eq). The mixture was stirred at 90 °C for 16 h. The mixture was poured into water (20 mL), extracted with ethyl acetate (10 mL × 3), the organic layer was washed with brine (10 mL × 2), dried over anhydrous Na₂SO₄, filtered and concentrated to the title compound (100 mg, 0.520 mmol, 97.2 % yield) as a colorless oil.

### Step 3:

### Ethyl 2-(4-chloro-2,3-dihydro-1H-inden-2-yl)acetate

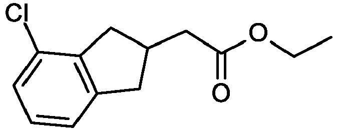

To a solution of 2-(4-chloro-2,3-dihydro-1*H*-inden-2-yl)acetonitrile (100.0 mg, 0.520 mmol, 1 eq) in ethanol (6 mL) was added concentrated sulfuric acid (2.0 mL). The mixture was stirred at 100 °C for 16 h. The mixture was poured into water (20 mL), extracted with ethyl acetate (10 mL × 3), the organic layer was washed with brine (10 mL × 2), dried over anhydrous Na₂SO₄, filtered and concentrated to afford the title compound (100 mg, 0.420 mmol, 80.3 % yield) as a colourless oil.

### Step 4:

### 2-(4-Chloro-2,3-dihydro-1H-inden-2-yl)ethanol

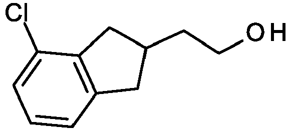

To a solution of ethyl 2-(4-chloro-2,3-dihydro-1*H*-inden-2-yl)acetate (200.0 mg, 0.840 mmol, 1 eq) in THF (2 mL) was added lithium aluminum hydride (63.6 mg, 1.68 mmol, 2 eq) at 0 °C. The mixture was stirred at 0 °C for 2 h. Water (0.1 mL) and 15% aqueous NaOH (0.1 mL) were added to the reaction mixture drop-wise, the mixture was filtered, then the filtrate was concentrated in vacuo to afford the title compound (170 mg, 0.860 mmol, 82.5 % yield).

### Step 5:

### 2-(4-Chloro-2,3-dihydro-1H-inden-2-yl)acetaldehyde

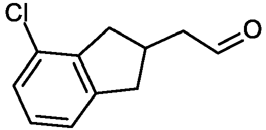

To a solution of 2-(4-chloro-2,3-dihydro-1*H*-inden-2-yl)ethanol (170.0 mg, 0.860 mmol, 1 eq) in DCM (5 mL) was added Dess-Martin periodinane (549.9 mg, 1.3 mmol, 1.5 eq). The mixture was stirred at 20 °C for 2 h. The mixture was poured into saturated aqueous Na₂SO₃ (20 mL), extracted with DCM (10 mL × 3), the organic layer was washed with brine (10 mL), dried over anhydrous Na₂SO₄ and concentrated in vacuo, and the residue purified by flash column chromatography (20% ethyl acetate/Petroleum ether) to afford the title compound (100 mg, 0.510 mmol, 59.4 % yield) as a colourless oil. ¹H-NMR (400 MHz, CDCl₃) δ 9.85 (s, 1H), 7.10 (m, 3H), 3.25 (m, 2H), 2.98 (m, 1H), 2.80 (m, 4H).

### Intermediate 12

### 6-[5-(Aminomethyl)-2-oxo-1,3-oxazolidin-3-yl]-4H-pyrido[3,2-b][1,4]oxazin-3-one

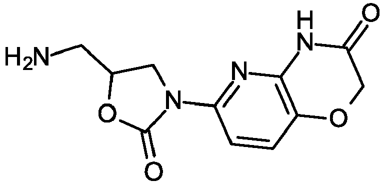

The title compound was prepared in analogy to Intermediate 1, steps 4-8 using 5-[[*tert-*butyl(dimethyl)silyl]oxymethyl]-1,3-oxazolidin-2-one in place of 5-[2-[*tert-*butyl(dimethyl)silyl]oxyethyl]-1,3-oxazolidin-2-one. The title compound was obtained as a white solid. MS (ESP): m/z = 265.1 [M+H]⁺.

The 5-[[*tert-*butyl(dimethyl)silyl]oxymethyl]-1,3-oxazolidin-2-one used as starting material was prepared as follows:
To a solution of 5-(hydroxymethyl)-1,3-oxazolidin-2-one (CAS# 7517-99-9, 10.0 g, 85.4 mmol, 1 eq) and imidazole (11627.21 mg, 170.79 mmol, 2 eq) in DCM (150 mL) was added *tert-* butyldimethylchlorosilane (19307 mg, 128.1 mmol, 1.5 eq) in an ice-bath. After addition, the mixture was stirred at 15 °C for 16 h. The mixture was filtrated through celite and the filtrated was collected. The solvent was removed by evaporated to give a residue, which was dissolved in EtOAc (500 mL) and washed with H₂O (300 mL) and brine (300 mL). The organic layer was collected, dried and concentrated to give a crude product, which was purified by column chromatography on silica gel (PE:EtOAc = 20:1-5:1) to give the title compound (9.2 g, 40 mmol, 45 % yield) as a white solid. MS (ESP): m/z = 232.1 [M+H]⁺.

### Intermediate 13

### 6-[(4R)-4-[(3-Aminocyclobutyl)amino]-2-oxopyrrolidin-1-yl]-4H-pyrido[3,2-b] [1,4]oxazin-3-one bis 2,2,2-trifluoroacetate

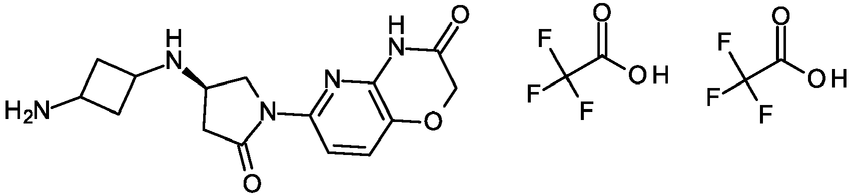

### Step 1:

### tert-Butyl N-[(3R)-5-oxo-1-[3-oxo-4-(2-trimethylsilylethoxymethyl)pyrido[3,2-b][1,4]oxazin-6-yl]pyrrolidin-3-yl]carbamate

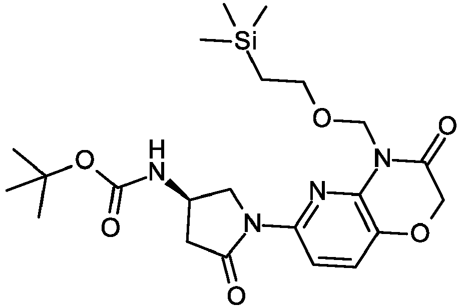

Through a mixture of 6-bromo-4-(2-trimethylsilylethoxymethyl)pyrido[3,2-b][1,4]oxazin-3-one (Intermediate 8, 4.73 g, 13.2 mmol, 1.05 eq), *tert*-butyl *N*-[(3*R*)-5-oxopyrrolidin-3-yl]carbamate (CAS# 1346773-63-4, 2.51 g, 12.5 mmol, 1 eq), K₂CO₃ (12.1 g, 87.7 mmol, 7 eq) and copper (I) iodide (716 mg, 3.76 mmol, 0.3 eq) in toluene (75 ml) argon was bubbled for 5 minutes. *trans-N,N*'-Dimethylcyclohexane-1,2-diamine (1.07 g, 1.19 ml, 7.52 mmol, 0.6 eq) was added and the mixture was heated up to 120 °C. The mixture was stirred for 90 minutes. Silica (10 g) was added and the reaction mixture evaporated. The residue was purified by silica cartridge chromatography (ISCO: 50 g from CH₂Cl₂ to 5% MeOH in CH₂Cl₂) to give the title compound (5 g, 10.4 mmol, 83.3 % yield) as a white foam. MS (ESP): m/z = 479.3 [M+H]⁺.

### Step 2:

### 6-[(4R)-4-Amino-2-oxopyrrolidin-1-yl]-4-(2-trimethylsilylethoxymethyl)pyrido[3,2-b][1,4] oxazin-3 -one

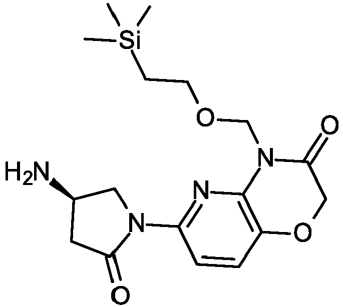

*tert*-Butyl *N*-[(3*R*)-5-oxo-1-[3-oxo-4-(2-trimethylsilylethoxymethyl)pyrido[3,2-b][1,4]oxazin-6-yl]pyrrolidin-3-yl]carbamate (5 g, 10.4 mmol, 1 eq) was mixed with 4M HCl in dioxane (26.1 mL, 104 mmol, 10 eq). After 10 minutes dioxane (20 mL) was added and the mixture stirred for 1 h. The mixture was poured onto sat. NaHCO₃ and extracted 3 times with EtOAc. The organic layer was dried over Na₂SO₄, filtered and evaporated to give the title compound (3.28 g, 8.67 mmol, 83 % yield) as a light yellow oil. MS (ESP): m/z = 379.2 [M+H]⁺.

### Step 3:

### tert-Butyl N-[3-[[(3R)-5-oxo-1-[3-oxo-4-(2-trimethylsilylethoxymethyl)pyrido[3,2-b][1,4]oxazin-6-yl]pyrrolidin-3-yl]amino]cyclobutyl]carbamate

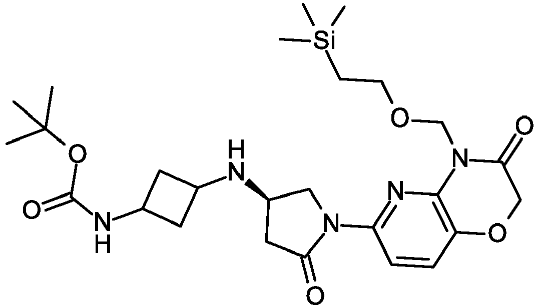

6-[(4*R*)-4-Amino-2-oxopyrrolidin-1-yl]-4-(2-trimethylsilylethoxymethyl)pyrido[3,2-b][1,4]oxazin-3-one (3.2 g, 8.45 mmol, 1 eq) was suspended in tetrahydrofuran (75 mL) under argon followed by powdered mol sieves 3Å (3.2 g), then *tert*-butyl (3-oxocyclobutyl)carbamate (2.04 g, 11 mmol, 1.3 eq) was added, followed by addition of acetic acid (863 mg, 823 µL, 14.4 mmol, 1.7 eq). and the mixture was stirred 30 minutes at RT, then sodium cyanoborohydride (903 mg, 14.4 mmol, 1.7 eq) was added and the mixture was stirred at 40 °C for 3 hours. NaHCO₃ was added and the mixture extracted 3 times with EtOAc, dried over Na₂SO₄, filtered and evaporated. The crude product was purified by prep-HPLC to give the title compound (670 mg, 14.5 % yield) as a white foam. MS (ESP): m/z = 548.4 [M+H]⁺.

### Step 4:

### 6-[(4R)-4-[(3-Aminocyclobutyl)amino]-2-oxopyrrolidin-1-yl]-4H-pyrido[3,2-b][1,4]oxazin-3-one bis 2,2,2-trifluoroacetate

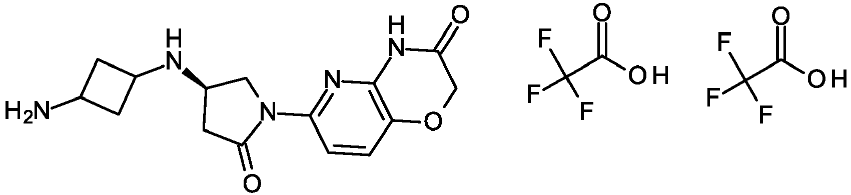

*tert*-Butyl *N*-[3-[[(3*R*)-5-oxo-1-[3-oxo-4-(2-trimethylsilylethoxymethyl)pyrido[3,2-b][1,4]oxazin-6-yl]pyrrolidin-3-yl]amino]cyclobutyl]carbamate (100 mg, 183 µmol, 1 eq) was dissolved in dichloromethane (1 mL), TFA (1.04 g, 703 µl, 9.13 mmol, 50 eq) was added and the mixture stirred at RT for 2 hours. The mixture was evaporated and the residue triturated with ether and filtered off, washed with ether and dried to give 110 mg off white solid. MeCN (1 mL) and water (15 mL) were added and the mixture was lyophilized over night to give the title compound (77 mg, 141 µmol, 77.3 % yield) as a light yellow solid. MS (ESP): m/z = 318.2 [M+H]⁺.

### Intermediate 14

### 6-[(4S)-4-[(3-Aminocyclobutyl)amino]-2-oxopyrrolidin-1-yl]-4H-pyrido[3,2-b][1,4]oxazin-3-one bis trifluoroacetate

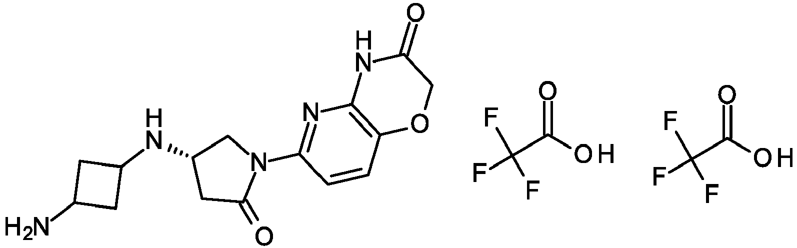

The title compound was prepared by analogy to Intermediate 13 using *tert*-butyl *N*-[(3*S*)-5-oxopyrrolidin-3-yl]carbamate (CAS# 672883-23-7) in place of *tert*-butyl *N*-[(*3R*)-5-oxopyrrolidin-3-yl]carbamate and was obtained as a white solid. MS (ESP): m/z = 318.1 [M+H]⁺.

### Intermediate 15

### 4-Methyl-6,7-dihydro-5H-cyclopenta[b]pyridin-6-amine

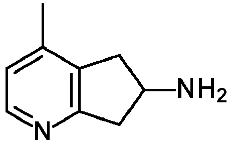

### Step 1:

### Dimethyl 4-methylpyridine-2,3-dicarboxylate

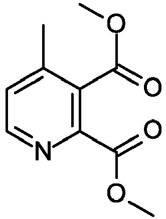

To a solution of 4-methylpyridine-2,3-dicarboxylic acid (CAS# 517-40-8, 1000.0 mg, 5.52 mmol, 1 eq) in DMF (20 mL) was added (trimethylsilyl)diazomethane (8.28 mL, 16.56 mmol, 3 eq) at 0 °C, and then the temperature was allowed to warm to 25 °C and stirred for 16 h. The solvent was removed to give the crude product, which was purified by chromatography on silica gel (PE:EtOAc =3:1) to give the title compound (800 mg, 3.82 mmol, 69.3 % yield) as a brown oil. MS (ESP): m/z = 210.1 [M+H]⁺.

### Step 2:

### [2-(Hydroxymethyl)-4-methyl-3-pyridyl]methanol

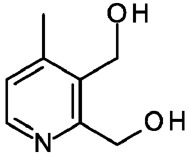

To a solution of dimethyl 4-methylpyridine-2,3-dicarboxylate (800.0 mg, 3.82 mmol, 1 eq) in ethanol (20 mL) was added sodium borohydride (868.0 mg, 22.94 mmol, 6 eq), and then the solution was stirred at 25 °C for 16 h. The pH of the solution was adjusted to around 6 by progressively adding formic acid, and then the solid was filtered off and the filtrate was concentrated to give the title compound (600 mg, 3.92 mmol, quant. yield) as a white solid. MS (ESP): m/z = 154.1 [M+H]⁺.

### Step 3:

### 2,3-Bis(chloromethyl)-4-methyl-pyridine

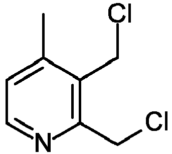

To a solution of [2-(hydroxymethyl)-4-methyl-3-pyridyl]methanol (0.6 g, 3.92 mmol, 1 eq) in DCM (10 mL) was added thionyl chloride (4.66 g, 39.17 mmol, 10 eq), and then the solution was stirred at 25 °C for 16 h. The solvent was removed by concentration and the residue was taken up in 50 mL DCM, and then the solution was poured into 20 mL ice-water, and the pH of the solution was adjusted to around 8 by progressively adding aq. NaHCO₃, and then the solution was extracted with DCM (20 mL × 2), combined with the organics and dried by Na₂SO₄. The solvent was removed to give the title compound (400 mg, 2.1 mmol, 53.7 % yield) as a brown oil.

### Step 4:

### Diethyl 4-methyl-5,7-dihydrocyclopenta[b]pyridine-6,6-dicarboxylate

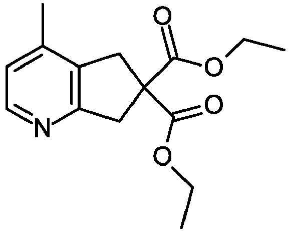

To a solution of diethyl malonate (0.337 g, 2.1 mmol, 1 eq) in 200 mL THF was added potassium tert-butoxide (0.5 g, 4.42 mmol, 2.1 eq) at 0 °C, and the solution was stirred at 0 °C for further 1 h. After that the solution was added to a stirred cooled to 0 °C solution of 2,3-bis(chloromethyl)-4-methyl-pyridine (0.4 g, 2.1 mmol, 1 eq) in 10.0 mL THF, and then the solution was stirred at 70 °C for 16 h. The mixture was poured into 50 mL ice-water and the solution was extracted with EtOAc (30 mL × 2), combined with the organics and dried by Na₂SO₄. The solvent was removed to give the crude product, which was purified by chromatography on silica gel (PE:EtOAc =10:1) to give the title compound (350 mg, 1.26 mmol, 60 % yield) as a colorless oil. MS (ESP): m/z = 278.1 [M+H]⁺.

### Step 5:

### 4-Methyl-6,7-dihydro-5H-cyclopenta[b]pyridine-6-carboxylic acid hydrochloride

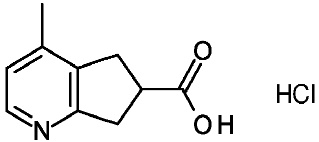

A solution of diethyl 4-methyl-5,7-dihydrocyclopenta[b]pyridine-6,6-dicarboxylate (350.0 mg, 1.26 mmol, 1 eq) in hydrochloric acid (0.13 mL, 1.26 mmol, 1 eq) was stirred at 100 °C for 16 h. The solvent was removed by concentration and to the residue was added 20 mL toluene, and water was removed to give the title compound (200 mg, 0.940 mmol, 74.2 % yield) as a yellow solid. MS (ESP): m/z = 178.1 [M+H]⁺.

### Step 6:

### 2-Trimethylsilylethyl N-(4-methyl-6,7-dihydro-5H-cyclopenta[b]pyridin-6-yl)carbamate

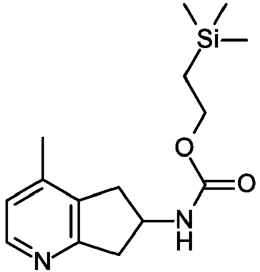

To a solution of 4-methyl-6,7-dihydro-5*H*-cyclopenta[b]pyridine-6-carboxylic acid hydrochloride (200.0 mg, 0.940 mmol, 1 eq) and 2-(trimethylsilyl)ethanol (0.67 mL, 4.68 mmol, 5 eq) in toluene (10 mL) was added diphenylphosphonic azide (0.4 mL, 1.87 mmol, 2 eq), and then the solution was stirred at 25 °C for 1 h. To the reaction was added triethylamine (0.39 mL, 2.81 mmol, 3 eq), and the solution was stirred at 100 °C for 16 h. The solvent was removed by concentration and the residue was taken up in 20 mL EtOAc, and then washed with 10 mL brine and dried by Na₂SO₄. The solvent was removed to give a crude product, which was purified by chromatography on silica gel (PE:EtOAc=1:2) to give the title compound (60 mg, 0.210 mmol, 21.9 % yield) as a brown oil. MS (ESP): m/z = 293.1 [M+H]⁺.

### Step 7:

### 4-Methyl-6,7-dihydro-5H-cyclopenta[b]pyridin-6-amine; 2,2,2-trifluoroacetic acid

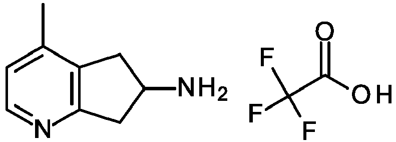

To a solution of 2-trimethylsilylethyl *N*-(4-methyl-6,7-dihydro-5*H*-cyclopenta[b]pyridin-6-yl)carbamate (60.0 mg, 0.210 mmol, 1 eq) in DCM (10 mL) was added trifluoroacetic acid (1.0 mL, 12.98 mmol, 63.3 eq), and then the solution was stirred at 25 °C for 2 h. The solvent was removed to give the crude title compound (30 mg, 0.200 mmol, 98.7 % yield) as a brown oil. MS (ESP): m/z = 149.1 [M+H]⁺.

### Intermediate 16

### 3-[2-Oxo-3-(3-oxo-4H-pyrido[3,2-b][1,4]oxazin-6-yl)-1,3-oxazolidin-5-yl]propanal

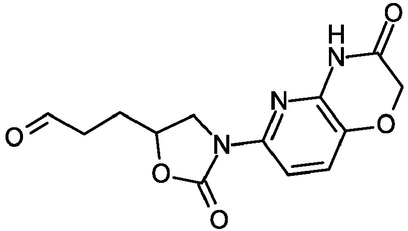

### Step 1:

### tert-Butyl-dimethyl-[3-(oxiran-2-yl)propoxy]silane

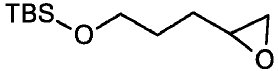

The title compound was prepared in analogy to *tert*-butyl-dimethyl-[2-(oxiran-2-yl)ethoxy]silane (Intermediate 1, step 1) using *tert*-butyl-dimethyl-pent-4-enoxysilane (CAS# 127154-84-1) in place of but-3-enoxy-*tert*-butyl-dimethyl-silane and was obtained as a light brown oil , which was not purified and used directly on next step.

### Step 2:

### 1-Azido-5-[tert-butyl(dimethyl)silyl]oxypentan-2-ol

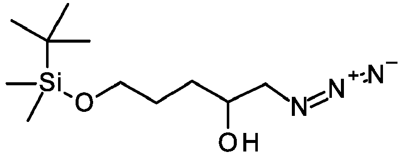

To a solution of *tert*-butyl-dimethyl-[3-(oxiran-2-yl)propoxy]silane (20.0 g, 92.43 mmol, 1 eq) in methanol (600 mL) was added sodium azide (9.74 mL, 277.3 mmol, 3 eq) and ammonium chloride (9.89 g, 184.8 mmol, 2 eq), and then the solution was stirred at 80 °C for 16 h. The solvent was evaporated under reduced pressure and the residue was partitioned between 200 mL EtOAc and 100 mL water. The organics layer was washed with brine, dried over Na₂SO₄ and evaporated under reduced pressure, affording the crude title compound (23 g, 88.66 mmol, 95.9 % yield) as a yellow oil.

### Step 3:

### 1-Amino-5-[tert-butyl(dimethyl)silyl]oxypentan-2-ol

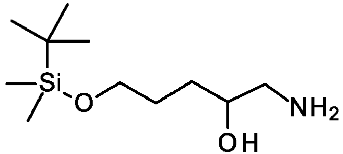

To a solution of 1-azido-5-[*tert-*butyl(dimethyl)silyl]oxy-pentan-2-ol (23.0 g, 88.66 mmol, 1 eq) in THF (500 mL) was added Pd/C catalyst (2 g), and then the mixture was stirred under hydrogen balloon at 25 °C for 16 h. The catalyst was filtered off and the filtrate was concentrated to give the crude title compound (18.5 g, 79.26 mmol, 89.4 % yield) as a brown oil.

### Steps 4 to 8:

### 6-[5-(3-hydroxypropyl)-2-oxo-1,3-oxazolidin-3-yl]-4H-pyrido[3,2-b][1,4]oxazin-3-one

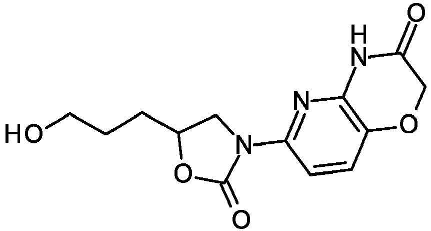

The title compound was prepared by analogy to 6-[5-(2-hydroxyethyl)-2-oxo-1,3-oxazolidin-3-yl]-4*H*-pyrido[3,2-b][1,4]oxazin-3-one (Intermediate 1, steps 2 to 6) using 1-amino-4-[*tert-*butyl(dimethyl)silyl]oxypentan-2-ol in place of 1-amino-4-[*tert*-butyl(dimethyl)silyl]oxybutan-2-ol and was obtained as a white solid. MS (ESP): m/z = 294.1 [M+H]+.

### Step 9:

### 3-[2-Oxo-3-(3-oxo-4H-pyrido[3,2-b][1,4]oxazin-6-yl)-1,3-oxazolidin-5-yl]propanal

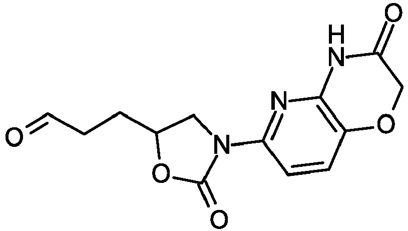

To a solution of 6-[5-(3-hydroxypropyl)-2-oxo-1,3-oxazolidin-3-yl]-4*H*-pyrido[3,2-b][1,4]oxazin-3-one (0.1 g, 0.340 mmol, 1 eq) in DCM (5 mL) and THF (5 mL), was added Dess-Martin periodinane (188.01 mg, 0.440 mmol, 1.3 eq) at 0 °C. The mixture was stirred at 20 °C for 3 h. The mixture was filtered and the filtrate was diluted with DCM (5 mL) and washed with 10 mL brine, and then dried with Na₂SO₄. The organic phase was concentrated under vacuum to give the title compound (80 mg, 0.270 mmol, 80.6 % yield) as a white solid. MS (ESP): m/z = 292.1 [M+H]⁺.

### Intermediate 17 6-(2-Oxo-1-oxa-3,8-diazaspiro[4.5]decan-3-yl)-4H-pyrido[3,2-b][1,4]oxazin-3-one hydrochloride

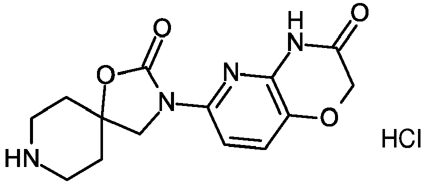

### Step 1:

### tert-Butyl 4-(2-ethoxy-2-oxoethyl)-4-hydroxypiperidine-1-carboxylate

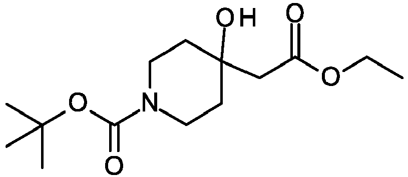

To a solution of lithium bis(trimethylsilyl)amide (60.23 mL, 60.23 mmol, 1.2 eq) in THF (100 mL) was added dropwise ethyl acetate (3.67 mL, 55.21 mmol, 1.1 eq) during 10 min. at -70°C, and stirred for 0.5 h. Then tert-butyl 4-oxopiperidine-1-carboxylate (10.0 g, 50.19 mmol, 1 eq) in THF (50 mL) was added to the mixture during 10 min. at -70°C, and stirred for 1 h at -70 °C. The mixture was quenched with aq. NH₄Cl (100 mL) and extracted with EtOAc (100 mL × 2). The organic layer was washed with brine (100 mL) and concentrated to give the title compound (14000 mg, 48.72 mmol, 97.1 % yield) as colorless oil. MS (ESP): m/z = 188.1 [M+H-Boc]⁺.

### Step 2:

### 2-[4-Hydroxy-1-[(2-methylpropan-2-yl)oxycarbonyl]piperidin-4-yl]acetic acid

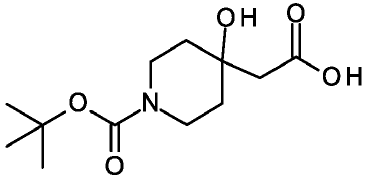

To a solution of *tert*-butyl 4-(2-ethoxy-2-oxo-ethyl)-4-hydroxy-piperidine-1-carboxylate (14.0 g, 48.72 mmol, 1 eq) in methanol (160 mL) were added water (40 mL) and sodium hydroxide (2.34 g, 58.47 mmol, 1.2 eq). The mixture was stirred for 2 h at 25 °C. The mixture was concentrated under reduced pressure. The residue was dissolved in water (100 mL) and extracted with EtOAc (100 mL). The aqueous layer was adjusted to pH=5 with HCl (1N) and then extracted with EtOAc (100 mL × 2). The organic layer was concentrated to give the title compound (11 g, 42.42 mmol, 87.1 % yield) as colorless oil. MS (ESP): m/z = 204.0 [M+H-tBu]⁺.

### Step 3:

### tert-Butyl 2-oxo-1-oxa-3,8-diazaspiro[4.5]decane-8-carboxylate

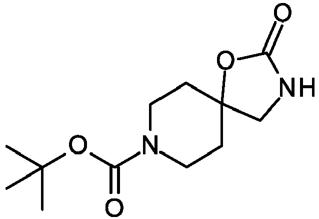

To a solution of triethylamine (6.5 mL, 46.66 mmol, 1.1 eq) in toluene (300 mL) was added 2-[4-hydroxy-1-[(2-methylpropan-2-yl)oxycarbonyl]piperidin-4-yl] acetic acid (11.0 g, 42.42 mmol, 1 eq) and diphenylphosphonic azide (11.88 mL, 55.15 mmol, 1.3 eq) . The mixture was stirred at 110 °C for 16 h. The reaction was quenched with EtOAc (200 mL) and washed with H₂O (2×100 mL). The organic layer was collected, dried and concentrated to give a residue, which was purified by column chromatography on silica gel (petroleum ether: EtOAc = 3:1) to give the title compound (4 g, 15.61 mmol, 33.1% yield) as white solid. MS (ESP): m/z = 201.0 [M+H]⁺.

### Steps 4 to 5:

### tert-Butyl 2-oxo-3-(3-oxo-4H-pyrido[3,2-b][1,4]oxazin-6-yl)-1-oxa-3,8-diazaspiro[4.5]decane-8-carboxylate

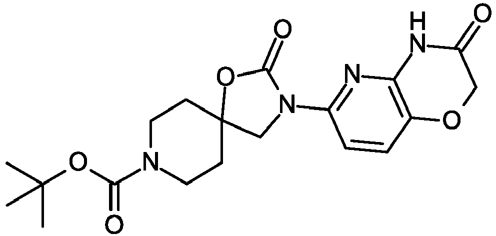

The title compound was prepared in analogy to 6-[5-[2-[*tert*-butyl(dimethyl)silyl]oxyethyl]-2-oxo-1,3-oxazolidin-3-yl]-4*H*-pyrido[3,2-b][1,4]oxazin-3-one (Intermediate 1, steps 4 and 5) using *tert*-butyl 2-oxo-1-oxa-3,8-diazaspiro[4.5]decane-8-carboxylate in place of 5-[2-[*tert-*butyl(dimethyl)silyl]oxyethyl]-1,3-oxazolidin-2-one and was obtained as a white solid. MS (ESP): m/z = 405.2 [M+H-tBu]⁺.

### Step 6:

### 6-(2-Oxo-1-oxa-3,8-diazaspiro[4.5]decan-3-yl)-4H-pyrido[3,2-b][1,4]oxazin-3-one hydrochloride

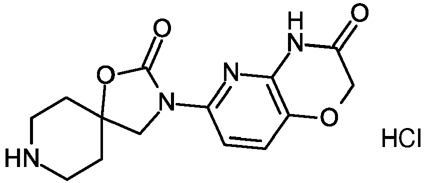

A solution of *tert*-butyl 2-oxo-3-(3-oxo-4*H*-pyrido[3,2-b][1,4]oxazin-6-yl)-1-oxa-3,8-diazaspiro[4.5]decane-8-carboxylate (500.0 mg, 1.24 mmol, 1 eq) in hydrochloric acid in methanol (20.0 mL, 80 mmol, 64.7 eq) was stirred for 4 h at 25 °C. The mixture was concentrated under reduced pressure. To the residue was added EtOAc (20 mL) and the mixture filtered. The filter cake was washed with EtOAc (10 mL) and then dried to give the title compound (238.8 mg, 0.700 mmol, 55.5 % yield) as a grey solid. The combined filtrate and washing were concentrated to give additional title compound (150 mg, 0.440 mmol, 35.0 % yield) as a yellow solid. MS (ESP): m/z = 305.0 [M+H]⁺.

### Intermediate 18

### 6-[5-(2-Amino-2-methylpropyl)-2-oxo-1,3-oxazolidin-3-yl]-4H-pyrido[3,2-b][1,4]oxazin-3-one

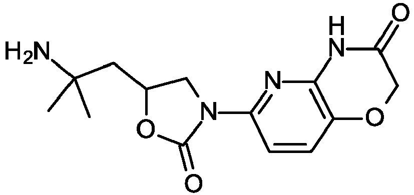

### Step 1:

### 1-Amino-4-methyl-4-nitropentan-2-ol

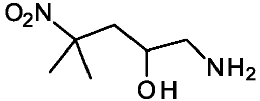

To a solution of ammonium hydroxide (231.33 mL, 5940 mmol, 479.0 eq) in THF (35 mL) was added dropwise a mixture of 2-(2-methyl-2-nitro-propyl)oxirane (CAS# 20030-57-3, 1800.0 mg, 12.4 mmol, 1 eq) in THF (105 mL) at 40 °C. After addition, the reaction was stirred at 40 °C for 16 h. The solvent was removed by evaporation and the residue dried to give the title compound (2100 mg, 12.95 mmol, 94.0 % yield) as a light brown oil.

### Step 2:

### 5-(2-Methyl-2-nitropropyl)-1,3-oxazolidin-2-one

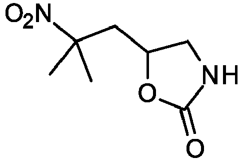

To a solution of 1-amino-4-methyl-4-nitropentan-2-ol (1900.0 mg, 11.71 mmol, 1 eq) and triethylamine (4.9 mL, 35.14 mmol, 3 eq) in DCM (40 mL) was added bis(trichloromethyl)carbonate (1390.5 mg, 4.69 mmol, 0.400 eq) at 0 °C. The reaction mixture was stirred at 20 °C for 2 h. The solvent was removed by evaporation to give a residue, which was dissolved in EtOAc (50 mL) and washed with aq. NaHCO₃ (3 × 20 mL) and brine (30 mL). The organic layer was collected, dried and concentrated to give a crude product, some solid was obtained. The crude product was dissolved in EtOAc (10 mL) and the solid was collected by filtration to give the title compound (270 mg, 1.43 mmol, 12.3 % yield) as a white solid and the filtrate was evaporated to give additional title compound (800 mg, 4.25 mmol, 36.3 % yield) as yellow semisolid.

### Steps 3 to 4:

### 6-[5-(2-Methyl-2-nitropropyl)-2-oxo-1,3-oxazolidin-3-yl]-4H-pyrido[3,2-b] [1,4]oxazin-3-one

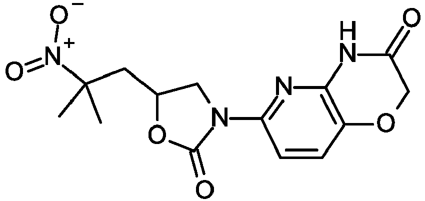

The title compound was prepared in analogy to 6-[5-[2-[*tert*-butyl(dimethyl)silyl]oxyethyl]-2-oxo-1,3-oxazolidin-3-yl]-4*H*-pyrido[3,2-b][1,4]oxazin-3-one (Intermediate 1, steps 4 and 5) using 5-(2-methyl-2-nitropropyl)-1,3-oxazolidin-2-one in place of 5-[2-[*tert-*butyl(dimethyl)silyl]oxyethyl]-1,3-oxazolidin-2-one and was obtained as a light yellow solid. MS (ESP): m/z 337.2 [M+H]⁺.

### Step 5:

### 6-[5-(2-Amino-2-methylpropyl)-2-oxo-1,3-oxazolidin-3-yl]-4H-pyrido[3,2-b] [1,4]oxazin-3-one

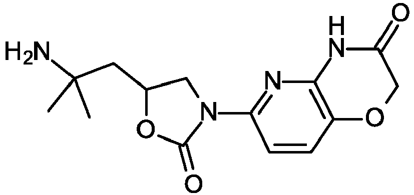

To a solution of 6-[5-(2-methyl-2-nitropropyl)-2-oxo-1,3-oxazolidin-3-yl]-4*H*-pyrido[3,2-b][1,4]oxazin-3-one (100.0 mg, 0.300 mmol, 1 eq) in ethanol (20 mL) was added tin(II) chloride dihydrate (402.59 mg, 1.78 mmol, 6 eq). The mixture was stirred at 80 °C for 16 h. To the mixture was added further tin(II) chloride dihydrate (268.39 mg, 1.19 mmol, 4 eq) and the mixture was stirred at same temperature for another 16 h. To the mixture was added thiourea resin (4 g) and the mixture was stirred at same temperature for another 3 h. The filtrate was collected by filtration and concentrated to give a residue, which was purified by preparative HPLC (formic acid) and freeze dried to give the title compound (30 mg, 0.100 mmol, 32.3 % yield) as a light yellow solid. MS (ESP): m/z = 307.2 [M+H]⁺.

### Intermediate 19

### 1-Chloro-6,7-dihydro-5H-cyclopenta[c]pyridine-6-carbaldehyde

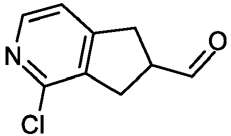

### Step 1:

### 2-Chloro-3,4-bis(chloromethyl)pyridine

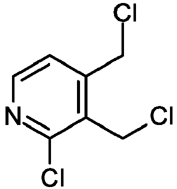

To a solution of [2-chloro-3-(hydroxymethyl)-4-pyridyl]methanol (for preparation, see WO 2013127268A1) (18.9 g, 108.88 mmol, 1 eq) in DCM (200 mL) was added thionyl chloride (129.53 g, 1089 mmol, 10 eq) at 0 °C, and then the solution was stirred at 25 °C for 16 h. The solvent was removed to give the crude product and the residue was poured into 100 mL ice-water, and then the pH of the solution was adjusted to around 8 by progressively adding 10 N aq. NaHCO₃. The solution was extracted with EtOAc (100 mL × 2), combined the organics and dried by Na₂SO₄. The solvent was removed to give the crude product, which was purified by chromatography on silica gel (PE:EA=10:1) to give the title compound (17 g, 80.76 mmol, 74.2 % yield) as a white solid. MS (ESP): m/z = 209.9 [M+H]⁺.

### Step 2:

### Diethyl 1-chloro-5,7-dihydrocyclopenta[c]pyridine-6,6-dicarboxylate

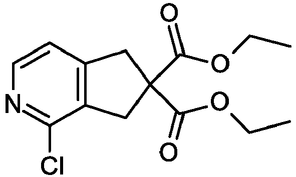

To a solution of 2-chloro-3,4-bis(chloromethyl)pyridine (17.0 g, 80.76 mmol, 1 eq) in 200 mL THF was added potassium *tert*-butoxide (19.03 g, 169.6 mmol, 2.1 eq) at 0 °C, and the solution was stirred at 0 °C for further 1 h. After that the solution was added to a stirred cooled to 0 °C solution of 2-chloro-3,4-bis(chloromethyl)pyridine (17.0 g, 80.76 mmol, 1 eq) in 100 mL THF, and then the solution was stirred at 70 °C for 16 h. The mixture was poured into 50 mL ice-water and the solution was extracted with EtOAc (30 mL × 2) and the combined organic layers were dried with Na₂SO₄. The solvent was removed to give the crude product, which was purified by chromatography on silica gel (PE:EA=10:1) to give the title compound (12.5 g, 41.98 mmol, 52.0 % yield) as a colorless oil. MS (ESP): m/z = 298.2 [M+H]⁺.

### Step 3:

### 1-Chloro-6,7-dihydro-5H-cyclopenta[c]pyridine-6-carboxylic acid

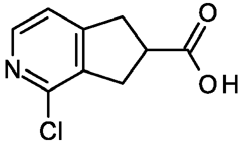

To a solution of diethyl 1-chloro-5,7-dihydrocyclopenta[c]pyridine-6,6-dicarboxylate (12.5 g, 41.98 mmol, 1 eq) was added hydrochloric acid (109.34 g, 1050 mmol, 25 eq), and then the solution was stirred at 100 °C for 16 h. The solvent was removed to give the crude title compound (10 g, quant. yield) as a brown solid, which was used in the next step without any further purification. MS (ESP): m/z = 198.1 [M+H]⁺.

### Step 4:

### Methyl 1-chloro-6,7-dihydro-5H-cyclopenta[c]pyridine-6-carboxylate

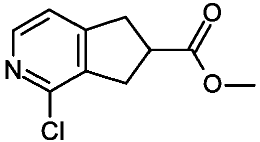

To a solution of 1-chloro-6,7-dihydro-5*H*-cyclopenta[c]pyridine-6-carboxylic acid (10000.0 mg, 50.6 mmol, 1 eq) was added HCl (gas) in MeOH (400.0 mL, 1600 mmol, 31.62 eq), and then the solution was stirred at 60 °C for 16 h. The solvent was removed by concentration and the residue was poured into 30 mL water. The pH of the solution was adjusted to around 10 by progressively adding NaHCO₃. The solution was extracted with EtOAc (20 mL × 3) and the combined organic layers were dried with Na₂SO₄. The solvent was removed to give the crude title compound (6.5 g, 30.71 mmol, 60.7 % yield) as brown oil. MS (ESP): m/z = 212.1 [M+H]⁺.

### Step 5:

### (1-Chloro-6,7-dihydro-5H-cyclopenta[c]pyridin-6-yl)methanol

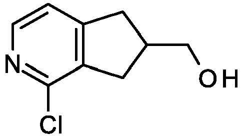

To a solution of methyl 1-chloro-6,7-dihydro-5*H*-cyclopenta[c]pyridine-6-carboxylate (2000.0 mg, 9.45 mmol, 1 eq) in THF (50 mL) was added lithium aluminum hydride (358.67 mg, 9.45 mmol, 1 eq) at 0 °C, and then the solution was stirred at 0 °C for 2 h. Drops of water were added and then EtOAc was added slowly until there was no more gas generated, and then 5 g Na₂SO₄ was added and the mixture was stirred at room temperature for 30 min. The solid was filtered off, the filter cake was washed with 20 mL EtOAc and the filtrate was evaporated to give the crude product, which was purified by chromtography on silica gel (PE:EA=1:1) to give the title compound (1500 mg, 8.17 mmol, 86.4 % yield) as a brown solid. MS (ESP): m/z = 184.0 [M+H]⁺.

### Step 6:

### 1-Chloro-6,7-dihydro-5H-cyclopenta[c]pyridine-6-carbaldehyde

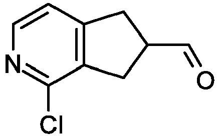

(1-Chloro-6,7-dihydro-5*H*-cyclopenta[c]pyridin-6-yl)methanol (304 mg, 1.66 mmol, 1 eq) was dissolved in DCM (16.6 mL) and the solution was cooled to 0 °C. Dess-Martin reagent (737 mg, 1.74 mmol, 1.05 eq) was added and the ice bath was removed. After 1 h at room temperature the reaction was quenched by the addition of sat. Na₂S₂O₃ and sat. NaHCO₃. The mixture was extracted 3 × with DCM and the combined organic layers were dried with MgSOa and evaporated. The residue was purified by silica cartridge chromatography eluting with 0-50% EtOAc in heptane to give the title compound (245 mg, 1.21 mmol, 73.3 % yield) as a colorless oil. MS (ESP): m/z = 182.1 [M+H]⁺.

### Intermediate 20

### 6-[rac-3a,7a-trans-3a,6-cis-6-Amino-2-oxo-3a,4,5,6,7,7a-hexahydro-1,3-benzoxazol-3-yl]-4H-pyrido[3,2-b][1,4]oxazin-3-one

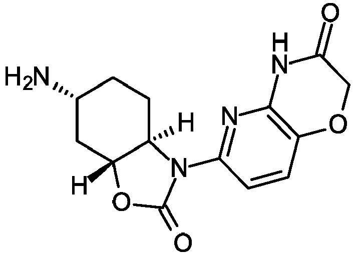

### Step 1:

### Benzyl N-[rac-1,3-cis-1,4-trans-3-hydroxy-4-(prop-2-enylamino)cyclohexyl]carbamate

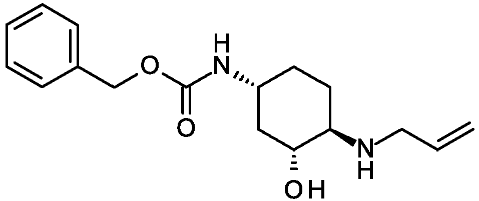

To a solution of benzyl *N*-[*rac*-1,4-*cis*-4,6-*cis*-7-oxabicyclo[4.1.0]heptan-4-yl]carbamate (CAS# 1932050-68-4, 3800.0 mg, 15.37 mmol, 1 eq) in MeCN (20 mL) was added lithium perchlorate (8174 mg, 76.83 mmol, 5 eq). The mixture was stirred for 15 min, then allylamine (4386 mg, 76.83 mmol, 5 eq) was added and the mixture was heated to 50 °C for 16 h. Water was added and the mixture was extracted with EtOAc. The combined extracts were dried over anhydrous Na₂SO₄ and concentrated in vacuo to afford the title compound as a yellow solid.

### Step 2:

### Benzyl N-[rac-1,3-cis-1,4-trans-4-amino-3-hydroxycyclohexyl]carbamate

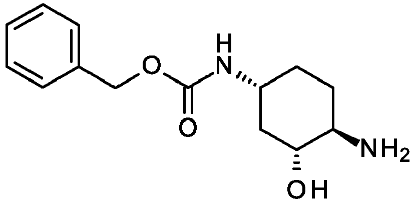

To a solution of benzyl *N-*[*rac-*1,3*-cis*-1,4*-trans*-3-hydroxy-4-(prop-2-enylamino)cyclohexyl]carbamate (8.0 g, 26.28 mmol, 1 eq) in CH₂Cl₂ (300 mL) under N₂ was added 1,3-dimethylbarbituric acid (24622.9 mg, 157.7 mmol, 6 eq) and tetrakis(triphenylphosphine)palladium(0) (6074.4 mg, 5.26 mmol, 0.200 eq). The mixture was stirred at 20 °C for 3 h.1N HCl (200 mL) was added and the layers were separated. The organic layer was washed with aq. HCl and the combined aqueous layers were then washed with CH₂Cl₂. The aqueous layer was basified with conc. NaOH and extracted twice with CH₂Cl₂. The combined organic layers from the basic extraction were dried over Na₂SO₄, filtered and evaporated to give the title compound (3.2 g, 12.11 mmol, 46.1 % yield) as a yellow solid. MS (ESP): m/z = 265.1 [M+H]⁺.

### Step 3:

### Benzyl N-[rac-3a,7a-trans-6,7a-cis-2-oxo-3a,4,5,6,7,7a-hexahydro-3H-1,3-benzoxazol-6-yl] carbamate

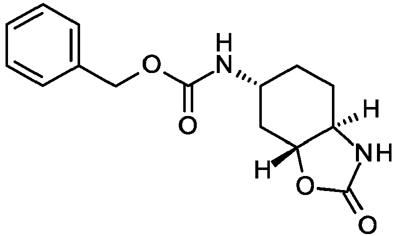

To a solution of benzyl *N*-[rac-1,3-cis-1,4-*trans*-4-amino-3-hydroxycyclohexyl]carbamate (1000.0 mg, 3.78 mmol, 1 eq) in THF (10 mL) was added N.N'-carbonyldiimidazole (920.19 mg, 5.67 mmol, 1.5 eq). The mixture was stirred at 50 °C for 2 h. The mixture was concentrated in vacuo and purified by flash chromatography (1: 1 ethyl acetate:hexane) to afford the title compound (450 mg, 1.55 mmol, 40.97% yield) as a white solid.

### Step 4:

### Ethyl 2-[2-nitro-6-[rac-3a,7a-trans-6,7a-cis-2-oxo-6-(phenylmethoxycarbonylamino)-3a,4,5,6,7,7a-hexahydro-1,3-benzoxazol-3-yl]pyridin-3-yl]oxyacetate

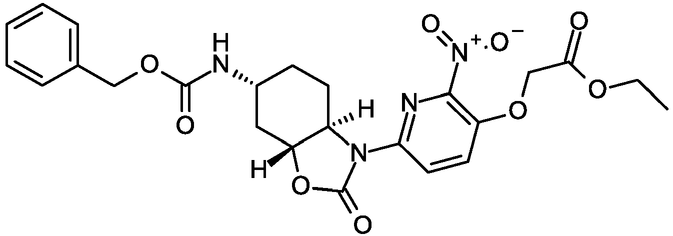

To a solution of benzyl *N*-[*rac*-3a,7a-*trans*-6,7a-*cis*-2-oxo-3a,4,5,6,7,7a-hexahydro-3H-1,3-benzoxazol-6-yl]carbamate (150.0 mg, 0.520 mmol, 1 eq), ethyl 2-[(6-bromo-2-nitro-3-pyridyl)oxy]acetate (173.4 mg, 0.570 mmol, 1.1 eq) and potassium carbonate (214.2 mg, 1.55 mmol, 3 eq), tris(dibenzylideneacetone)dipalladium (0) (94.6 mg, 0.100 mmol, 0.200 eq) in toluene (2 mL) was added 9,9-dimethyl-4,5-bis(diphenylphosphino)xanthene (119.6 mg, 0.210 mmol, 0.400 eq). The mixture was degassed with nitrogen three times and stirred at 100 °C for 2 h. The mixture was filtered and the filtrate was concentrated in vacuo and purified by flash column to afford the title compound (180 mg, 0.350 mmol, 67.7 % yield) as a white solid.

### Step 5:

### Benzyl N-[rac-3a,7a-trans-6,7a-cis-2-oxo-3-(3-oxo-4H-pyrido[3,2-b][1,4]oxazin-6-yl)-3a,4,5,6,7,7a-hexahydro-1,3-benzoxazol-6-yl]carbamate

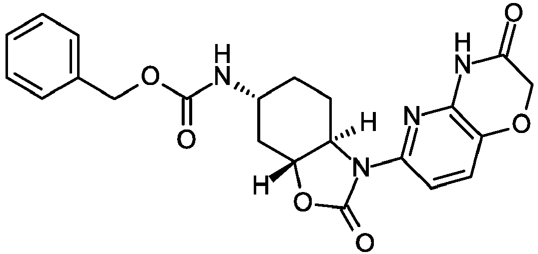

To a solution of ethyl 2-[2-nitro-6-[*rac*-3a,7a-*trans*-6,7a-*cis*-2-oxo-6-(phenylmethoxycarbonylamino)-3a,4,5,6,7,7a-hexahydro-1,3-benzoxazol-3-yl]pyridin-3-yl]oxyacetate (330.0 mg, 0.640 mmol, 1 eq) in acetic acid (5 mL) was added iron (179.1 mg, 3.21 mmol, 5 eq). The mixture was stirred at 60 °C for 2 h. The mixture was filtered, the pH of the filtrate was adjusted to 7 by addition of saturated NaHCO₃ solution and then extracted with ethyl acetate. The organic layer was washed with brine, dried over anhydrous Na₂SO₄ and concentrated in vacuo to afford the title compound (220 mg, 0.500 mmol, 78.2 % yield) as a white solid. MS (ESP): m/z = 439.2 [M+H]⁺.

### Step 6:

### 6-[rac-3a,7a-trans-6,7a-cis-6-Amino-2-oxo-3a,4,5,6,7,7a-hexahydro-1,3-benzoxazol-3-yl]-4H-pyrido[3,2-b][1,4]oxazin-3-one

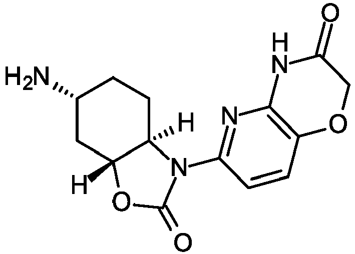

To a solution of benzyl *N*-[*rac*-3a,7a-*trans*-6,7a-cis-2-oxo-3-(3-oxo-4*H*-pyrido[3,2-b][1,4]oxazin-6-yl)-3a,4,5,6,7,7a-hexahydro-1,3-benzoxazol-6-yl]carbamate (200.0 mg, 0.460 mmol, 1 eq) in 1-propanol (50 mL) and THF (50 mL) was added Pd/C (50.0 mg). The mixture was stirred at 20 °C for 16 h under H₂ (15 psi). The mixture was filtered and concentrated in vacuo to afford the title compound (120 mg, 0.390 mmol, 86.5 % yield) as a yellow solid.

### Intermediate 21

### 6-(6-Oxo-5-oxa-2,7-diazaspiro[3.4]octan-7-yl)-4H-pyrido[3,2-b][1,4]oxazin-3-one

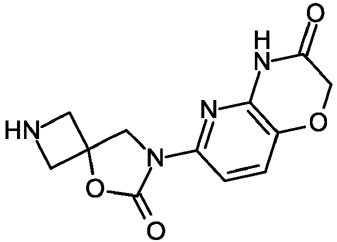

### Step 1:

### 3-(Aminomethyl)-1-benzhydrylazetidin-3-ol

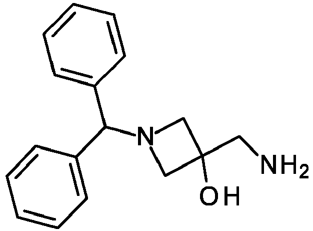

To a solution of 1-benzhydryl-3-trimethylsilyloxy-azetidine-3-carbonitrile (CAS# 151097-25-5, 7.5 g, 22.29 mmol, 1 eq) in methanol (365.9 mL) was added nickel chloride hexahydrate (6861.2 mg, 28.97 mmol, 1.3 eq) and sodium borohydride (4216.1 mg, 111.44 mmol, 5 eq) in several portions at 0 °C. The mixture was sitrred at 0°C for 1 h. Saturated NaHCO₃ was added, the mixture was filtered and the filtrate was concentrated in vacuo and the residue purified by flash column chromatography (10 % methanol/DCM with 1% NH₃•H₂O) to afford the title compound (2.7 g, 10.06 mmol, 45.1 % yield) as a white solid. MS (ESP): m/z = 269.2 [M+H]⁺.

### Step 2:

### 2-Benzhydryl-5-oxa-2,7-diazaspiro[3.4]octan-6-one

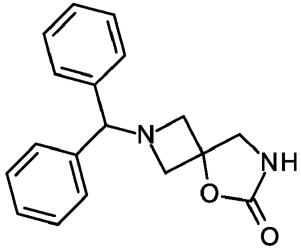

To a solution of 3-(aminomethyl)-1-benzhydrylazetidin-3-ol (2.7 g, 10.06 mmol, 1 eq) in THF (70 mL) was added *N*,*N*'-carbonyldiimidazole (2447.19 mg, 15.09 mmol, 1.5 eq). The mixture was stirred at 50 ° for 1 h. The mixture was poured into water and filtered to give the title compound (2.47 g, 8.39 mmol, 83.4 % yield) as a yellow solid. MS (ESP): m/z = 295.1 [M+H]⁺.

### Steps 3 to 4:

### 6-(2-Benzhydryl-6-oxo-5-oxa-2,7-diazaspiro[3.4]octan-7-yl)-4H-pyrido[3,2-b][1,4]oxazin-3-one

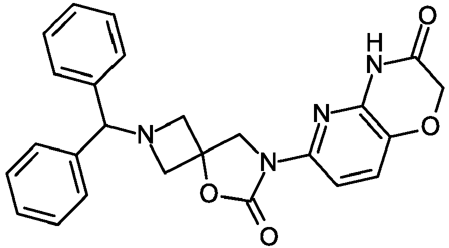

The title compound was prepared by analogy to benzyl *N-[rac-3a,7a-trans-6,7a-cis-2-oxo-3-(3-*oxo-4*H*-pyrido[3,2-b][1,4]oxazin-6-yl)-3a,4,5,6,7,7a-hexahydro-1,3-benzoxazol-6-yl]carbamate (Intermediate 20, steps 4 and 5) using 2-benzhydryl-5-oxa-2,7-diazaspiro[3.4]octan-6-one in place of benzyl *N*-[rac-3a,7a-*trans*-6,7a-cis-2-oxo-3a,4,5,6,7,7a-hexahydro-3*H*-1,3-benzoxazol-6-yl]carbamate and was obtained as a yellow solid. MS (ESP): m/z = 443.2 [M+H]⁺.

### Step 5:

### 6-(6-Oxo-5-oxa-2,7-diazaspiro[3.4]octan-7-yl)-4H-pyrido[3,2-b][1,4]oxazin-3-one

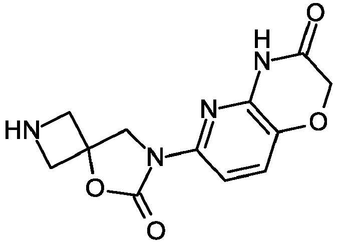

To a solution of 6-(2-benzhydryl-6-oxo-5-oxa-2,7-diazaspiro[3.4]octan-7-yl)-4*H*-pyrido[3,2-b][1,4]oxazin-3-one (100.0 mg, 0.230 mmol, 1 eq) in THF (50 mL) and 1-propanol (50 mL) was added Pd(OH)₂/C (20.0 mg). The mixture was stirred at 25 °C for 16 h under H₂ (50 psi). The mixture was filtered and concentrated in vacuo to give a residue which was triturated with TBME to afford the title compound (30 mg, 0.110 mmol, 48.1 % yield) as a yellow solid. MS (ESP): m/z = 277.0 [M+H]⁺.

### Intermediate 22

### 2-(2-Oxoethyl)-2,3-dihydro-1H-indene-4-carbonitrile

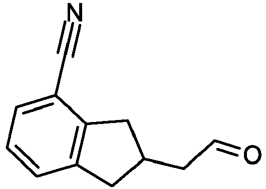

### Step 1:

### 2-(2-Hydroxyethyl)-2,3-dihydro-1H-indene-4-carbonitrile

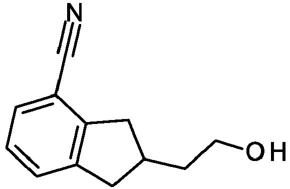

To a degassed solution of 2-(4-chloro-2,3-dihydro-1*H*-inden-2-yl)ethanol (Intermediate 11, step 4, 150.0 mg, 0.760 mmol, 1 eq) in DMA (6 mL) were added zinc cyanide (0.1 mL, 1.53 mmol, 2 eq), 2-di-*tert*-butylphosphino-2',4',6'-triisopropylbiphenyl (129.6 mg, 0.310 mmol, 0.40 eq) and tris(dibenzylideneacetone)dipalladium (0) (139.7 mg, 0.150 mmol, 0.20 eq). The mixture was stirred under N₂ at 120 °C for 16 h. Water was added and the mixture was extracted with ethyl acetate. The combined organic layers were washed with brine, dried over anhydrous Na₂SO₄ and concentrated in vacuo and purified by flash chromatography (30 % ethyl acetate/petroleum ether) to afford the title compound (100 mg, 0.530 mmol, 70.0% yield) as a yellow oil.

### Step 2:

### 2-(2-Oxoethyl)-2,3-dihydro-1H-indene-4-carbonitrile

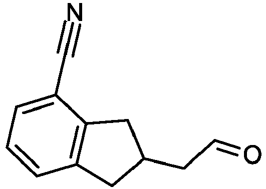

To a solution of 2-(2-hydroxyethyl)-2,3-dihydro-1*H*-indene-4-carbonitrile (100.0 mg, 0.530 mmol, 1 eq) in DCM (4.17 mL) was added Dess-Martin periodinane (453.0 mg, 1.07 mmol, 2 eq). The mixture was stirred at 25 °C for 2 h. The reaction was poured into water (20 mL), extracted with DCM (20 mL × 3) and the combined extracts were washed with 20 mL saturated brine solution. The organic layer was then separated, dried (MgSO₄) and concentrated to dryness. The crude product was then purified by flash column chromatography (20 % EtOAc/PE). The desired fractions were concentrated to afford the title compound (60 mg, 0.320 mmol, 50.5 % yield) as a colourless oil. ¹H-NMR (400 MHz, CDCl₃) δ 9.85 (s, 1H), 7.42 (m, 2H), 7.25 (m, 1H), 3.42 (m, 1H), 3.26 (m, 1H), 3.03 (m, 1H), 2.65 (m, 4H).

### Intermediate 23

### 6-[5-(trans-4-Aminocyclohexyl)-2-oxo-1,3-oxazolidin-3-yl]-4H-pyrido[3,2-b][1,4]oxazin-3-one hydrochloride

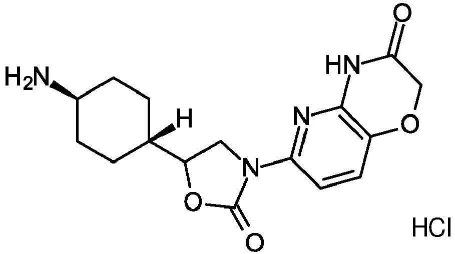

The title compound was prepared by analogy to 6-[5-(*trans*-3-aminocyclobutyl)-2-oxo-1,3-oxazolidin-3-yl]-4*H*-pyrido[3,2-b] [1,4]oxazin-3-one (Intermediate 7) using *tert-butyl N-(trans-4-*formylcyclohexyl)carbamate (CAS# 181308-57-6) in place of *tert-butyl N-(trans-3-*formylcyclobutyl)carbamate and was obtained as a yellow solid. MS (ESP): m/z = 333.1 [M+H]⁺.

### Intermediate 24

### 6-[5-(2-Aminoethyl)-2-oxo-1,3-oxazolidin-3-yl]-4H-pyrazino[2,3-b][1,4]oxazin-3-one trifluoroacetate

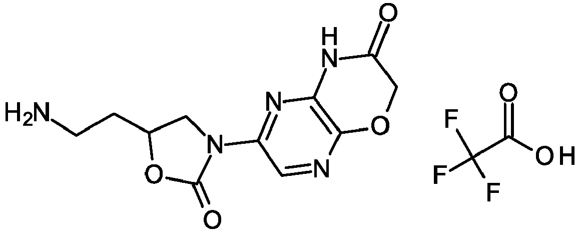

The title compound was prepared by analogy with 6-[5-(*trans*-3-Aminocyclobutyl)-2-oxo-1,3-oxazolidin-3-yl]-4*H*-pyrido[3,2-b] [1,4]oxazin-3-one (Intermediate 7, steps 4 and 5) using 6-bromo-4-(2-trimethylsilylethoxymethyl)pyrazino[2,3-b][1,4]oxazin-3-one (Intermediate 25) in place of 6-bromo-4-(2-trimethylsilylethoxymethyl)pyrido[3,2-b][1,4]oxazin-3-one and *tert*-butyl *N*-[2-(2-oxo-1,3-oxazolidin-5-yl)ethyl]carbamate (Intermediate 26) in place of *tert*-butyl *N-*[*trans*-3-(2-oxo-1,3-oxazolidin-5-yl)cyclobutyl]carbamate. MS (ESP): m/z = 280.1 [M+H]⁺.

### Intermediate 25

### 6-Bromo-4-(2-trimethylsilylethoxymethyl)pyrazino[2,3-b] [1,4]oxazin-3-one

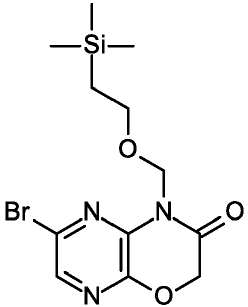

### Step 1:

### 6-Bromo-4H-pyrazino[2,3-b][1,4]oxazin-3-one

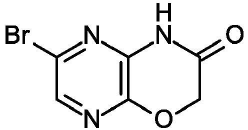

To a solution of 3,6-dibromopyrazin-2-amine (5.0 g, 19.77 mmol, 1 eq) in methyl glycolate (17.81 g, 197.71 mmol, 10 eq) was added potassium *tert*-butoxide (6.66 g, 59.31 mmol, 3 eq) at 60 °C under N₂. The reaction mixture was stirred at 60 °C for 3 hr. The pH of the reaction mixture was then adjusted to 4 by addition of 1M HCl, the mixture was diluted with H₂O (100 mL) and extracted with EtOAc (50 mL × 3). The combined organic layers were dried over anhydrous Na₂SO₄, filtered and concentrated and the resulting residue was purified by flash column chromatography (40% EtOAc/petroleum ether) to give the title compound (2.7 g, 11.74 mmol, 59.4% yield). ¹H-NMR (400 MHz, methanol-d4) δ 7.86 (s, 1H), 4.93 (s, 2H).

### Step 2:

### 6-Bromo-4-(2-trimethylsilylethoxymethyl)pyrazino[2,3-b][1,4]oxazin-3-one

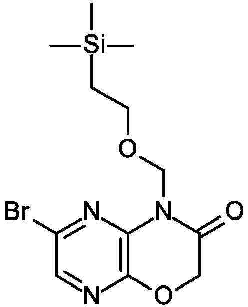

To a solution of 6-bromo-4*H*-pyrazino[2,3-b][1,4]oxazin-3-one (2.7 g, 11.74 mmol, 1 eq), potassium carbonate (4.87 g, 35.21 mmol, 3 eq) in DMF (30 mL) was added 2-(trimethylsilyl)ethoxymethyl chloride (3.12 mL, 17.61 mmol, 1.5 eq) at 0 °C. Then the reaction was stirred at 25 °C for 0.5 hr. The reaction mixture was diluted with H₂O (100 mL), extracted with ethyl acetate (40 mL × 3). The combined extracts were dried over anhydrous Na₂SO₄ and concentrated and the resulting residue was purified by flash column chromatography (20 % EA/petroleum ether) to give the title compound (3.9 g, 10.82 mmol, 92.2% yield) as a light yellow solid. ¹H-NMR (400 MHz, CDCl₃) δ 8.02 (s, 1H), 5.51 (s, 2H), 4.93 (s, 2H), 3.72 (m, 2H), 0.97 (m, 2H), 0.00 (s, 9H).

### Intermediate 26

### tert-Butyl N-[2-(2-oxo-1,3-oxazolidin-5-yl)ethyl]carbamate

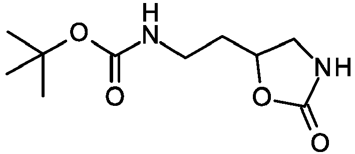

### Step 1:

### 5-(2-Hydroxyethyl)oxazolidin-2-one

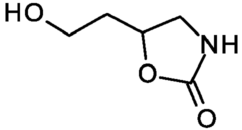

A solution of 5-[2-[*tert*-butyl(dimethyl)silyl]oxyethyl]oxazolidin-2-one (Intermediate 1, step 3, 1.0 g, 4.08 mmol, 1 eq) in HCl (gas) in MeOH (10.19 mL, 40.75 mmol, 10 eq) was stirred at 25 °C for 1 h. The pH of the solution was adjusted to around 8 by progressively adding NH₃.H₂O. The solvent was removed to give the crude product, which was washed with 20 mL petroleum ether, and then the solid was dried under reduced pressure to give a crude title compound (500 mg, 3.81 mmol, 93.6 % yield) as a yellow solid.

### Step 2:

### 2-(2-Oxo-1,3-oxazolidin-5-yl)ethyl methanesulfonate

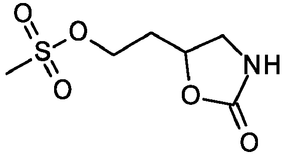

To a solution of 5-(2-hydroxyethyl)oxazolidin-2-one (500.0 mg, 3.81 mmol, 1 eq) in THF (10 mL) was added methanesulfonyl chloride (0.59 mL, 7.63 mmol, 2 eq), and then the mixture was stirred at 50 °C for 2 h. The solution was washed with water (10 mL) and dried with Na₂SO₄. The solvent was removed to give the crude title compound (800 mg, 3.82 mmol, 100% yield) as a yellow solid.

### Step 3:

### 5-(2-Azidoethyl)oxazolidin-2-one

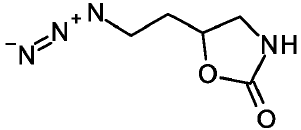

To a solution of 2-(2-oxo-1,3-oxazolidin-5-yl)ethyl methanesulfonate (800.0 mg, 3.82 mmol, 1 eq) in DMF (40 mL) was added sodium azide (1242.9 mg, 19.12 mmol, 5 eq), and then the mixture was stirred at 50 °C for 16 h. The solution was poured into 50 mL water and the mixture was extracted with EtOAc (50 mL × 2). the combined organic layers were dried with Na₂SO₄ and the solvent was removed to give the crude title compound (500 mg, 3.2 mmol, 83.8% yield) as a brown oil.

### Step 4:

### 5-(2-Aminoethyl)-1,3-oxazolidin-2-one

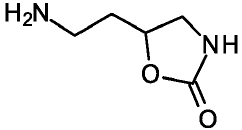

To a solution of 5-(2-azidoethyl)oxazolidin-2-one (500.0 mg, 3.2 mmol, 1 eq) in THF (50 mL) was added Pd/C (100 mg) and then the mixture was stirred at 25 °C under an atmosphere of hydrogen for 3 h. The catalyst was filtered off and the filtrate was concentrated to give the title compound (400 mg, 3.07 mmol, 96.0% yield) as a brown oil. MS (ESP): m/z = 131.1 [M+H]⁺.

### Step 5:

### tert-Butyl N-[2-(2-oxo-1,3-oxazolidin-5-yl)ethyl]carbamate

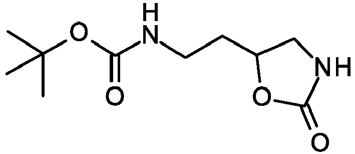

To a solution of 5-(2-aminoethyl)-1,3-oxazolidin-2-one (400.0 mg, 3.07 mmol, 1 eq) and triethylamine (0.86 mL, 6.15 mmol, 2 eq) in DCM (20 mL) was added di-t-butyldicarbonate (1006.1 mg, 4.61 mmol, 1.5 eq) and the solution was stirred at 25°C for 16 h. The solvent was removed to give the crude product which was purified by chromatography on silica gel (DCM : MeOH=10:1) to give the title compound (250 mg, 1.09 mmol, 35.3 % yield) as a brown oil. MS (ESP): m/z = 253.1 [M+Na]⁺.

### Intermediate 27

### 4-Fluoro-1-methyl-6,7-dihydro-5H-cyclopenta[c]pyridine-6-carbaldehyde

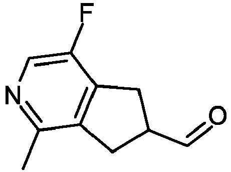

### Step 1:

### 2-Bromo-5-fluoro-3-methylpyridine-4-carbaldehyde

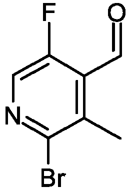

To a solution of 2-bromo-5-fluoro-3-methyl-pyridine (CAS# 38186-85-5, 5000.0 mg, 26.31 mmol, 1 eq) in THF (100 mL) was added lithium diisopropylamide (2M in THF, 26.31 mL, 52.6 mmol, 2 eq) at -78°C. The reaction mixture was stirred at -78°C for 1 h. DMF (5 mL) was added and the reaction mixture was stirred at -78°C for 3 h. Sat. aq. NH₄Cl solution (100.0 mL) was added and the mixture was extracted with DCM (100 mL × 3). The organic layers were combined and dried with Na₂SO₄. The solvent was removed to give the crude title compound (5000 mg, 22.93 mmol, 87.2 % yield) as a yellow oil. The crude product was used in the next step without further purification.

### Step 2:

### (2-Bromo-5-fluoro-3-methylpyridin-4-yl)methanol

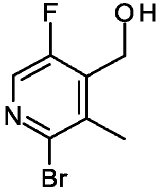

To a solution of 2-bromo-5-fluoro-3-methyl-pyridine-4-carbaldehyde (5000.0 mg, 22.93 mmol, 1 eq) in THF (100 mL) was added sodium borohydride (1735.28 mg, 45.87 mmol, 2 eq) at 0°C. The reaction mixture was stirred at 20 °C for 16 h. 6 M HCl (20.0 mL) was added and the mixture was extracted with DCM (50 mL × 3). The organic layers were combined, dried with Na₂SO₄ and concentrated to provide the crude product. The crude product was purified by silica column chromatography (PE: EtOAc=15:1 to 1:1) to give the title compound (3600 mg, 16.36 mmol, 71.3 % yield) as a light yellow solid. MS (ESP): m/z = 220.0 [M+H]⁺.

### Step 3:

### 2-Bromo-4-(chloromethyl)-5-fluoro-3-methylpyridine

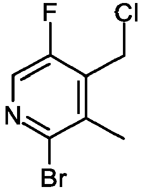

A mixture of (2-bromo-5-fluoro-3-methyl-4-pyridyl)methanol (3600.0 mg, 16.36 mmol, 1 eq) and DMF (3 mL) in ethyl acetate (100 mL) was stirred at 20°C for 0.5 h to form a clear solution. Thionyl chloride (3892.9 mg, 32.72 mmol, 2 eq) was added to the reaction mixture at 20 °C. The reaction mixture was stirred at 20°C for 15.5 h. 15 wt% Na₂CO₃ solution (50 mL) was added to adjust the pH of the mixture to 8.0. The layers were separated and the organic layer was dried with Na₂SO₄ and concentrated to provide the crude title compound (3500 mg, 14.68 mmol, 89.7 % yield) as yellow oil. The crude product was used in the next step without further purification. MS (ESP): m/z = 238.0 [M+H]⁺.

### Step 4:

### 2-Bromo-3-(bromomethyl)-4-(chloromethyl)-5-fluoropyridine

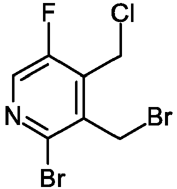

To a solution of *N*-bromosuccinimide (3134.5 mg, 17.61 mmol, 1.2 eq) and 2-bromo-4-(chloromethyl)-5-fluoro-3-methylpyridine (3500.0 mg, 14.68 mmol, 1 eq) in carbon tetrachloride (100 mL) was added benzoyl peroxide (355.5 mg, 1.47 mmol, 0.100 eq) portionwise at 0 °C. At the end of the addition, the reaction mixture was stirred at 20 °C for 0.5 h and then at 80°C for another 15.5 h. The reaction mixture was concentrated to provide the crude product. The crude product was dissolved in EtOAc (100.0 mL) and the solution was washed with water (50 mL × 2) and brine (50 mL × 2), dried over Na₂SO₄ and concentrated to give the title compound (3500 mg, 11.03 mmol, 75.1 % yield) as yellow oil. The crude product was used in the next step without further purification. MS (ESP): m/z = 317.9 [M+H]⁺.

### Step 5:

### Diethyl 1-bromo-4-fluoro-5,7-dihydrocyclopenta[c]pyridine-6,6-dicarboxylate

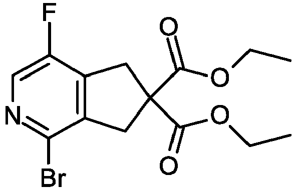

To a solution of diethyl malonate (0.96 mL, 6.3 mmol, 1 eq) in THF (15.0 mL) was added potassium *tert*-butoxide (1484.9 mg, 13.23 mmol, 2.1 eq) at 0°C, and the solution was stirred at 0 °C for further 1 h. After that, the solution was added to a stirred cooled (0 °C) solution of 2-bromo-3-(bromomethyl)-4-(chloromethyl)-5-fluoropyridine (2000.0 mg, 6.3 mmol, 1 eq) in THF (5.0 mL), and then the solution was stirred at 70 °C for 16 h. The mixture was poured into 100 mL ice-water and the solution was extracted with EtOAc (100 mL × 2) and the combined organic extracts were dried with Na₂SO₄. The solvent was removed to give the crude product, which was purified by silica column chromatography (PE: EtOAc=20:1-2:1) to give the title compound (1.8 g, 5 mmol, 79.3 % yield) as yellow oil. MS (ESP): m/z = 360.0 [M+H]⁺.

### Step 6:

### 1-Bromo-4-fluoro-6,7-dihydro-5H-cyclopenta[c]pyridine-6-carboxylic acid

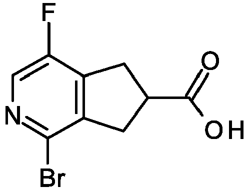

A solution of diethyl 1-bromo-4-fluoro-5,7-dihydrocyclopenta[c]pyridine-6,6-dicarboxylate (1800.0 mg, 5 mmol, 1 eq) in hydrochloric acid (50.0 mL, 600 mmol, 120.06 eq) was stirred at 100 °C for 16 h. The reaction mixture was concentrated and to the residue were added 100 mL toluene. The solvent was removed to give the crude title compound (1200 mg, 4.61 mmol, 92.3 % yield) as yellow oil. MS (ESP): m/z = 260.0 and 262.0 [M+H]⁺.

### Step 7:

### Ethyl 1-bromo-4-fluoro-6,7-dihydro-5H-cyclopenta[c]pyridine-6-carboxylate

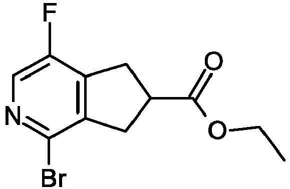

To a solution of 1-bromo-4-fluoro-6,7-dihydro-5*H*-cyclopenta[c]pyridine-6-carboxylic acid (1200.0 mg, 4.61 mmol, 1 eq) in ethanol (50 mL) was added sulfuric acid (1.0 mL, 18.4 mmol, 3.99 eq) at 20 °C. The reaction mixture was stirred at 80 °C for 16 h. The reaction mixture was concentrated to provide the crude product which was purified by silica column chromatography (PE:EtOAc=20:1 to 2:1) to give the title compound (1200 mg, 4.17 mmol, 90.3 % yield) as yellow oil. MS (ESP): m/z = 288.0 and 290.0 [M+H]⁺.

### Step 8:

### Ethyl 4-fluoro-1-methyl-6,7-dihydro-5H-cyclopenta[c]pyridine-6-carboxylate

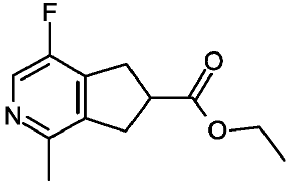

A solution of ethyl 1-bromo-4-fluoro-6,7-dihydro-5*H*-cyclopenta[c]pyridine-6-carboxylate (600.0 mg, 2.08 mmol, 1 eq) and [1,1'-bis(diphenylphosphino)ferrocene]dichloropalladium(II) (152.38 mg, 0.210 mmol, 0.100 eq) in 1,4-dioxane (20.0 mL) was degassed with N₂ three times, and then 1.0M Me₂Zn in toluene (6.25 mL, 6.25 mmol, 3 eq) was added and the solution was stirred at 110 °C for 2 h. The solution was poured into ice-water (20.0 mL) and the mixture was extracted with EtOAc (30 mL × 3). The combined extracts were dried with Na₂SO₄ and the solvent was removed to give the crude product, which was purified by preparative TLC (PE:EA=3:1) to give the title comopund (300 mg, 1.34 mmol, 64.5 % yield) as a yellow oil. MS (ESP): m/z = 224.3 [M+H]⁺.

### Step 9:

### (4-Fluoro-1-methyl-6,7-dihydro-5H-cyclopenta[c]pyridin-6-yl)methanol

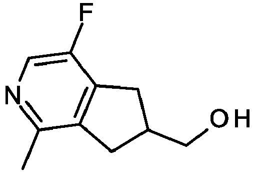

To a solution of ethyl 4-fluoro-1-methyl-6,7-dihydro-5*H*-cyclopenta[c]pyridine-6-carboxylate (300.0 mg, 1.34 mmol, 1 eq) in THF (15 mL) was added lithium aluminum hydride (102.01 mg, 2.69 mmol, 2 eq) at 0°C. The reaction mixture was stirred at 20°C for 2 h. The reaction was quenched with saturated aqueous NH₄Cl (10.0 mL). The mixture was diluted with water (10.0 mL) and extracted with EtOAc (20.0 mL × 2). The combined organic layers were concentrated to provide the crude product which was purified by preparative TLC (PE: EtOAc=1:1) to give the title compound (210 mg, 1.16 mmol, 86.2 % yield) as a yellow oil. MS (ESP): m/z = 182.3 [M+H]⁺.

### Step 10:

### 4-Fluoro-1-methyl-6,7-dihydro-5H-cyclopenta[c]pyridine-6-carbaldehyde

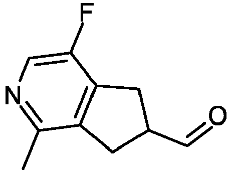

To a solution of (4-fluoro-1-methyl-6,7-dihydro-5*H*-cyclopenta[c]pyridin-6-yl)methanol (100.0 mg, 0.550 mmol, 1 eq) in DCM (5 mL) was added Dess-Martin periodine (468.13 mg, 1.1 mmol, 2 eq) at 20 °C. The reaction mixture was stirred at 20 °C for 16 h. The reaction mixture was filtered and concentrated to provide the crude product which was purified by preparative TLC (DCM: MeOH=10:1) to give the title compound (35 mg, 0.200 mmol, 35.4 % yield) as yellow oil. MS (ESP): m/z = 180.3 [M+H]⁺.

### Intermediate 28

### 4-Fluoro-6-formyl-6,7-dihydro-5H-cyclopenta[c]pyridine-1-carbonitrile

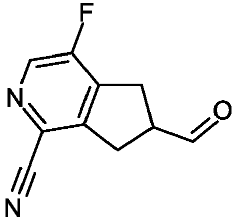

The title compound was prepared by analogy to 2-(2-oxoethyl)-2,3-dihydro-1*H*-indene-4-carbonitrile (Intermediate 22) using (1-bromo-4-fluoro-6,7-dihydro-5*H*-cyclopenta[c]pyridin-6-yl)methanol in place of 2-(4-chloro-2,3-dihydro-1*H*-inden-2-yl)ethanol and was obtained as an off-white solid, which was used directly without further purification.

The (1-bromo-4-fluoro-6,7-dihydro-5*H*-cyclopenta[c]pyridin-6-yl)methanol used as starting material was prepared as follows:

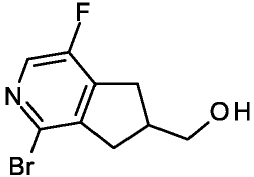

To a solution of ethyl 1-bromo-4-fluoro-6,7-dihydro-5*H*-cyclopenta[c]pyridine-6-carboxylate (Intermediate 27, step 7, 350.0 mg, 1.21 mmol, 1 eq) in THF (23.33 mL) was added lithium aluminum hydride-THF (1.34 mL, 1.34 mmol, 1.1 eq) at 0 °C, and then the solution was stirred at 0 °C for 2 h. The reaction was quenched with Na₂SO₄•10H₂O and diluted with EtOAc (50 mL). The mixture was filtered and the filtrate was concentrated to give the title compound (300 mg, 1.22 mmol, 87.3 % yield) as a green solid, which was used directly without further purification. MS (ESP): m/z = 246.0 and 248.0 [M+H]⁺.

### Intermediate 29

### 6-(8-Amino-2-oxo-1-oxa-3-azaspiro[4.5]decan-3-yl)-4H-pyrido[3,2-b][1,4]oxazin-3-one

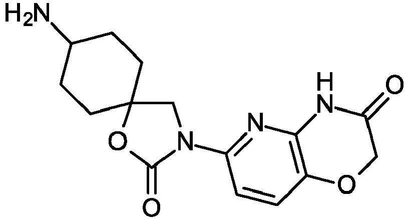

### Steps 1 to 3:

### tert-Butyl N-(2-oxo-1-oxa-3-azaspiro[4.5]decan-8-yl)carbamate

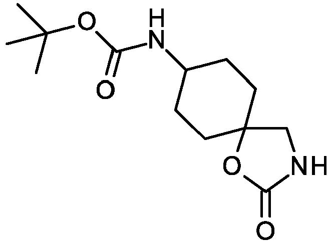

The title compound was prepared in analogy to *tert*-butyl *N*-[*trans*-3-(2-oxo-1,3-oxazolidin-5-yl)cyclobutyl]carbamate (Intermediate 7, steps 1 to 3) using *tert*-butyl N-(4-oxocyclohexyl)carbamate (CAS# 179321-49-4) in place of *tert*-butyl *N*-(*trans*-3-formylcyclobutyl)carbamate and was obtained as a white solid.

### Step 4:

### Ethyl 2-[6-[8-[(2-methylpropan-2-yl)oxycarbonylamino]-2-oxo-1-oxa-3-azaspiro[4.5]decan-3-yl]-2-nitropyridin-3-yl]oxyacetate

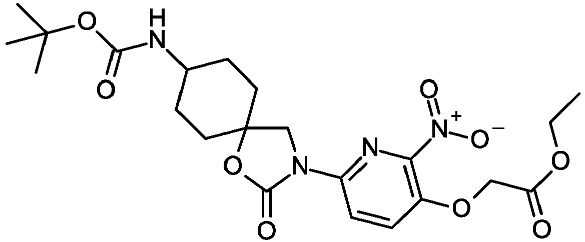

To a solution of *tert*-butyl *N*-(2-oxo-1-oxa-3-azaspiro[4.5]decan-8-yl)carbamate (1.1 g, 4.07 mmol, 1 eq) and ethyl 2-[(6-bromo-2-nitro-3-pyridyl)oxy]acetate (1.24 g, 4.07 mmol, 1 eq) in 1,4-dioxane (22 mL) was added copper (I) iodide (155.0 mg, 0.810 mmol, 0.200 eq), *N,N-*dimethyl-ethylenediamine (0.01 mL, 1.63 mmol, 0.400 eq) and potassium carbonate (1.12 g, 8.14 mmol, 2 eq). The mixture was degassed with nitrogen for three times and stirred at 100 °C for 2 h. The reaction was filtered and the filtrate was concentrated to dryness. The residue was diluted with EtOAc (50 mL) and the solution was washed with water (30 mL) and brine (30 mL), dried over anhydrous Na₂SO₄ and concentrated under reduced pressure to afford a crude product. The residue was purified by column gel eluted with (PE/EtOAc= 1/1). The desired fractions were concentrated to dryness in vacuo to give the title compound (1.3 g, 2.63 mmol, 64.6% yield) as yellow solid.

### Step 5:

### tert-Butyl N-[2-oxo-3-(3-oxo-4H-pyrido[3,2-b][1,4]oxazin-6-yl)-1-oxa-3-azaspiro[4.5]decan-8-yl] carbamate

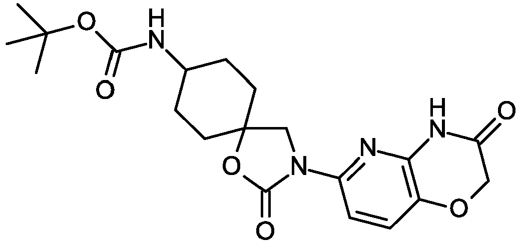

To a solution of ethyl 2-[6-[8-[(2-methylpropan-2-yl)oxycarbonylamino]-2-oxo-1-oxa-3-azaspiro[4.5]decan-3-yl]-2-nitropyridin-3-yl]oxyacetate (1.3 g, 2.71 mmol, 1 eq) in acetic acid (20 mL) was added iron (755.5 mg, 13.53 mmol, 5 eq) and the mixture was stirred for 2 h at 60 °C. The reaction was filtered and concentrated to dryness. The residue was diluted with EtOAc (50 mL) and the organic layer was washed with water (30 mL) and brine (30 mL), dried over anhydrous Na₂SO₄ and concentrated under reduced pressure to afford crude product. The residue was purified by column gel eluted with (PE/EtOAc= 1/1). The desired fractions were concentrated to dryness in vacuo to afford the title compound (700 mg, 1.73 mmol, 62.1% yield) as yellow solid. MS (ESP): m/z = 419.2 [M+H]⁺.

### Step 6:

### 6-(8-Amino-2-oxo-1-oxa-3-azaspiro[4.5]decan-3-yl)-4H-pyrido[3,2-b][1,4]oxazin-3-one

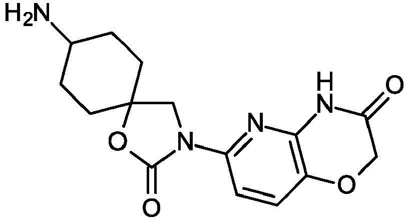

To a solution of *tert*-butyl *N*-[2-oxo-3-(3-oxo-4H-pyrido[3,2-b][1,4]oxazin-6-yl)-1-oxa-3-azaspiro[4.5]decan-8-yl]carbamate (200.0 mg, 0.480 mmol, 1 eq) in ethyl acetate (5 mL) was added hydrochloric acid in ethyl acetate (5.0 mL, 20 mmol, 41.84 eq). Then the mixture was stirred for 4h at 25 °C. The mixture was concentrated under reduced pressure. Ethyl acetate (20 mL) was added, the mixture was filtered and the filter cake was washed with ethyl acetate (10 mL). The solvent was removed and the residue was diluted with methanol (20 mL). To this mixture was added basic resin (5 g) and the mixture was stirred for 2h. The mixture was filtered and concentrated to give the title compound (150 mg, 0.470 mmol, 98.6% yield) as white solid. MS (ESP): m/z = 319.2 [M+H]⁺.

### Intermediate 30

### [rac-(5,6-trans)-1-Chloro-6-formyl-6,7-dihydro-5H-cyclopenta[c]pyridin-5-yl] acetate

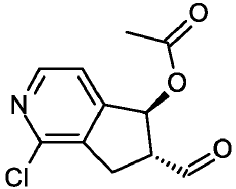

### Step 1:

### tert-Butyl-[(1-chloro-6,7-dihydro-5H-cyclopenta[c]pyridin-6-yl)methoxy]-diphenylsilane

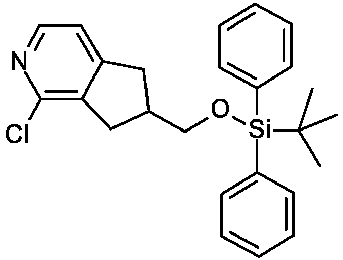

To a mixture of (1-chloro-6,7-dihydro-5*H*-cyclopenta[c]pyridin-6-yl)methanol (Intermediate 19, step 5, 2400.0 mg, 13.07 mmol, 1 eq) and imidazole (1779.51 mg, 26.14 mmol, 2 eq) in DMF (15 mL) was added tert-butylchlorodiphenylsilane (4670.06 mg, 16.99 mmol, 1.3 eq) at 0°C and then the reaction mixture was stirred at 25 °C for 16 h. The mixture was quenched with saturated NaHCO₃ (60 mL) and then extracted with DCM (100 mL). The organic phase was dried over Na₂SO₄, concentrated under vacuum to give the residue. The residue was purified by chromatography column on silica (PE/EtOAc=30/1 to 3/1) to give the title compound (5000 mg, 11.85 mmol, 90.7 % yield) as colourless oil. MS (ESP): m/z = 422.2 [M+H]⁺.

### Step 2:

### tert-Butyl-[(1-chloro-2-oxido-6,7-dihydro-5H-cyclopenta[c]pyridin-2-ium-6-yl)methoxy]-diphenylsilane

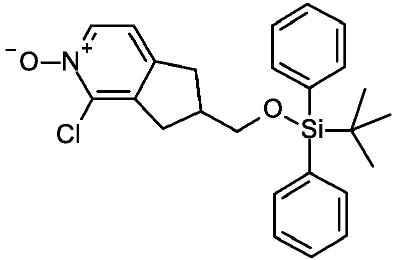

To a solution of tert-butyl-[(1-chloro-6,7-dihydro-SH-cyclopenta[c]pyridin-6-yl)methoxy]-diphenylsilane (5000.0 mg, 11.85 mmol, 1 eq) in DCM (100 mL) was added 3-chloroperbenzoic acid (3595.86 mg, 17.77 mmol, 1.5 eq) at 0°C and then the reaction was stirred at 25 °C for 16 h. The reaction was concentrated under vacuum to give the crude product (11 g). The crude product was purified by chromatography column on silica gel (PE/EtOAc=1011-112) to give the title compound (4900 mg, 11.19 mmol, 94.4% yield) as a white solid. MS (ESP): m/z = 438.2 [M+H]⁺.

### Step 3:

### 6-[[tert-Butyl(diphenyl)silyl]oxymethyl]-1-chloro-6,7-dihydro-5H-cyclopenta[c]pyridin-5-yl] acetate and [6-[[tert-Butyl(diphenyl)silyl]oxymethyl]-1-chloro-6,7-dihydro-5H-cyclopenta[c]pyridin-4-yl] acetate

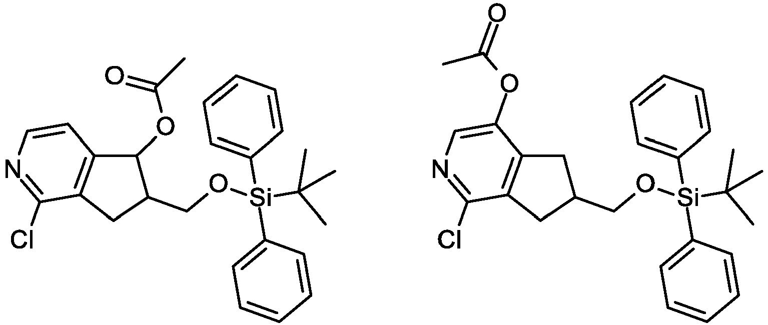

A mixture of *tert*-butyl-[(1-chloro-2-oxido-6,7-dihydro-5*H*-cyclopenta[c]pyridin-2-ium-6-yl)methoxy]-diphenylsilane (4900.0 mg, 11.19 mmol, 1 eq) in Ac₂O (50.0 mL, 111.86 mmol, 10 eq) was stirred at 100 °C for 3 h. The mixture was concentrated under vacuum to give a residue which was dissolved in EtOAc (100 mL) and the solution was washed with NaHCO₃ (2 × 50 mL). The combined organic layers were dried over Na₂SO₄ and concentrated under vacuum to give the crude product (7.0 g). The crude product was purified by chromatography column on silica gel (PE/EtOAc=10/1 to 3/1) to give a mixture of the title compounds (3700 mg, 7.71 mmol, 68.9 % yield) as a colourless oil. MS (ESP): m/z = 480.3 [M+H]⁺.

### Step 4:

### [rac-(5,6-trans)-1-Chloro-6-(hydroxymethyl)-6,7-dihydro-5H-cyclopenta[c]pyridin-5-yl] acetate

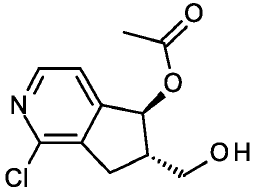

To a mixture of *tert*-butyl(diphenyl)silyl]oxymethyl]-1-chloro-6,7-dihydro-5*H-*cyclopenta[c]pyridin-5-yl] acetate and [6-[[*tert*-butyl(diphenyl)silyl]oxymethyl]-1-chloro-6,7-dihydro-5*H*-cyclopenta[c]pyridin-4-yl] acetate (150.0 mg, 0.310 mmol, 1 eq) in THF (5.04 mL) was added TBAF in THF (0.62 mL, 0.620 mmol, 2 eq) and the reaction mixture was stirred at 25 °C for 16 h. The mixture was quenched with brine (10 mL) then extracted with EtOAc (2 × 20 mL). The combined extracts were dried over Na₂SO₄, concentrated under vacuum to give a residue which was purified by preparative TLC (PE/EtOAc=1/2) to give title compound (25 mg, 0.100 mmol, 33.1 % yield) as a colourless oil. MS (ESP): m/z = 242.1 [M+H]⁺.

### Step 5:

### [rac-(5,6-trans)-1-Chloro-6-formyl-6,7-dihydro-5H-cyclopenta[c]pyridin-5-yl] acetate

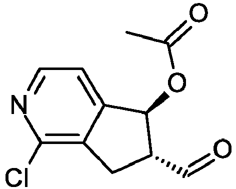

To a solution of [*rac*-(5,6-*trans*)-1-chloro-6-(hydroxymethyl)-6,7-dihydro-5*H-*cyclopenta[c]pyridin-5-yl] acetate (80.0 mg, 0.330 mmol, 1 eq) in DCM (15.36 mL) was added Dess-Martin periodinane (280.81 mg, 0.660 mmol, 2 eq) at 0 °C. After addition, the reaction was stirred at 20 °C for 2 h. Celite (0.5 g) was added and the mixture was stirred for 5 min. The mixture was filtered and the filtrate was concentrated under vacuum to give the crude title compound (100 mg, 0.420 mmol, 63.0% yield) as a light brown solid. The crude product was used for the next step directly. MS (ESP): m/z = 240.1 [M+H]⁺.

### Intermediate 31

### 6-[5-(2-Aminoethyl)-2-oxo-1,3,4-oxadiazol-3-yl]-4H-1,4-benzoxazin-3-one; formic acid

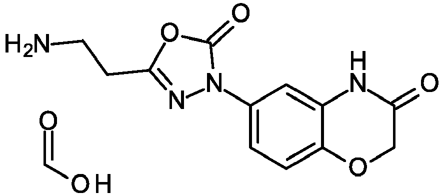

### Step 1:

### 6-Bromo-4-(2-trimethylsilylethoxymethyl)-1,4-benzoxazin-3-one

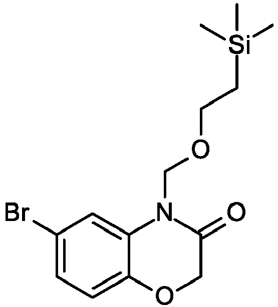

To a solution of 6-bromo-2*H*-1,4-benzoxazin-3(4H)-one (2000.0 mg, 8.77 mmol, 1 eq) in tetrahydrofuran (60 mL) was added potassium *tert*-butoxide (1968.3 mg, 17.54 mmol, 2 eq) at 0 °C and the mixture stirred for 10 min. Then 2-(trimethylsilyl)ethoxymethyl chloride (2.33 mL, 13.16 mmol, 1.5 eq) was added dropwise and the mixture was allowed to warm to 25 °C and stirred for another 50 min. The reaction was poured into H₂O (50 mL) and extracted with ethyl acetate (100 mL × 3). The combined organic layers were dried over anhydrous Na₂SO₄ and concentrated. The residue was purified by silica column chromatography(PE:EtOAc=30:1 to 20:1) to give the title compound (2300 mg, 6.42 mmol, 73.2 % yield) as a white solid. ¹H-NMR (400 MHz, d6-DMSO) δ 7.41 (s, 1H), 7.20 (d, 1H), 6.97 (d, 1H), 5.31 (s, 2H), 4.71 (s, 2H), 3.57 (t, 2H), 0.86 (t, 2H), -0.05 (s, 9H).

### Step 2:

### Methyl 3 - [bis [(4-methoxyphenyl)methyl] amino]propanoate

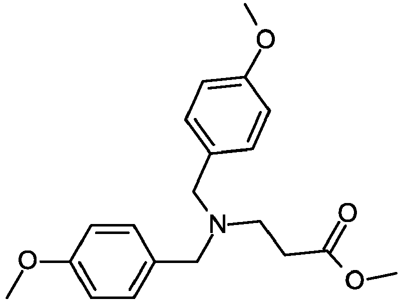

A mixture of methyl 3-aminopropanoate (11000.0 mg, 106.67 mmol, 1 eq) in 4-methoxybenzylchloride (36.16 mL, 266.68 mmol, 2.5 eq) and potassium carbonate (44228.1 mg, 320.02 mmol, 3 eq) was stirred at 30 °C for 4 h. The reaction mixture was poured into H₂O (300 mL) and extracted with ethyl acetate (100 mL × 3). The combined organic layers were washed with brine and dried over anhydrous Na₂SO₄, filtered and concentrated. The residue was purified by silica column chromatography (PE:EtOAc=10:1 to 8:1) to give the title compound (18000 mg, 52.41 mmol, 49.1 % yield) as a colourless oil.

### Step 3:

### 3-[bis[(4-Methoxyphenyl)methyl]amino]propanehydrazide

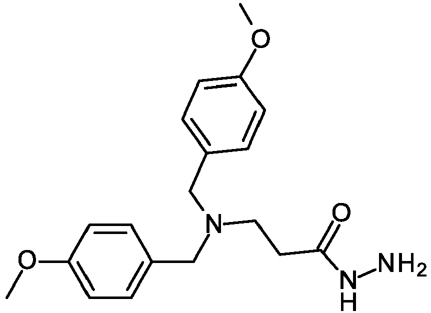

To a solution of methyl 3-[bis[(4-methoxyphenyl)methyl]amino]propanoate (10000.0 mg, 29.12 mmol, 1 eq) in ethanol (200 mL) was added hydrazine hydrate (14874.4 mg, 291.19 mmol, 10 eq) and the mixture stirred at 80 °C for 12 h. The reaction mixture was concentrated. The residue was purified by prep-HPLC and lyophilized to give the title compound (8000 mg, 23.3 mmol, 80 % yield) as a colourless oil. MS (ESP): m/z = 344.2 [M+H]⁺.

### Step 4:

### 5-[2-[bis[(4-Methoxyphenyl)methyl]amino]ethyl]-3H-1,3,4-oxadiazol-2-one

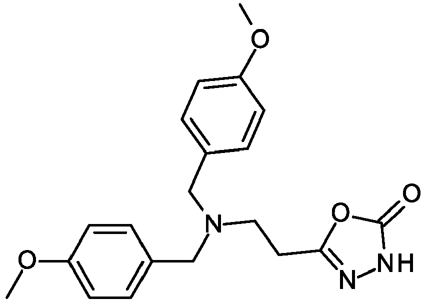

To a solution of 3-[bis[(4-methoxyphenyl)methyl]amino]propanehydrazide (2900.0 mg, 8.44 mmol, 1 eq) in dichloroethane (87 mL) was added bis(trichloromethyl)carbonate (1039.9 mg, 3.5 mmol, 0.420 eq) at 25 °C. The mixture was heated to 80 °C and stirred for 2 h. The reaction mixture was filtered and the filter cake was washed with dichloroethane (50 mL) to give the title compound (3200 mg, 8.66 mmol, 99.2% yield) as a white solid. MS (ESP): m/z = 370.1 [M+H]⁺.

### Step 5:

### 6-[5-[2-[bis[(4-Methoxyphenyl)methyl]amino]ethyl]-2-oxo-1,3,4-oxadiazol-3-yl]-4-(2-trimethylsilylethoxymethyl)-1,4-benzoxazin-3-one

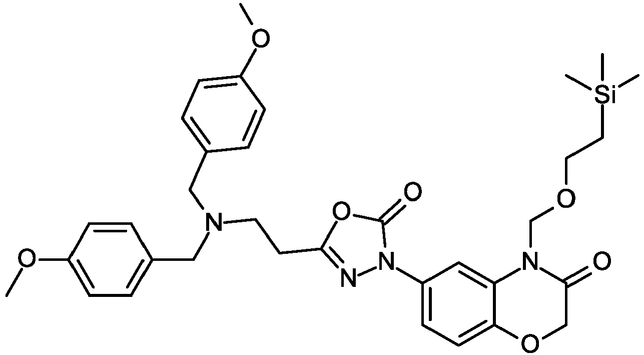

To a solution of 5-[2-[bis[(4-methoxyphenyl)methyl]amino]ethyl]-3*H*-1,3,4-oxadiazol-2-one (200.0 mg, 0.540 mmol, 1 eq)in 3-methyl-1-butanol (6.67 mL) was added 6-bromo-4-(2-trimethylsilylethoxymethyl)-1,4-benzoxazin-3-one (232.78 mg, 0.650 mmol, 1.2 eq), sodium tert-butoxide (156.1 mg, 1.62 mmol, 3 eq) and t-BuXPhos-Pd-G3 (43.0 mg, 0.050 mmol, 0.100 eq) at 25 °C under an atmosphere of nitrogen. The reaction mixture was heated to 90 °C and stirred for 12 h. The reaction mixture was filtered and filtrate was concentrated. The residue was purified by silica column chromatography (PE:EtOAc=10:1 to 8:1) and concentrated to give the title compound (120 mg, 0.190 mmol, 34.3 % yield) as a light yellow oil. MS (ESP): m/z = 647.3 [M+H]⁺.

### Step 6:

### 6-[5-[2-[bis[(4-Methoxyphenyl)methyl]amino]ethyl]-2-oxo-1,3,4-oxadiazol-3-yl]-4H-1,4-benzoxazin-3-one

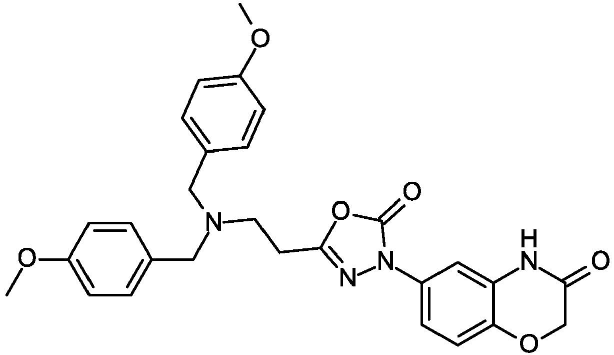

To a solution of 6-[5-[2-[bis[(4-methoxyphenyl)methyl]amino]ethyl]-2-oxo-1,3,4-oxadiazol-3-yl]-4-(2-trimethylsilylethoxymethyl)-1,4-benzoxazin-3-one (650.0 mg, 1 mmol, 1 eq) in ethanol (50 mL) was added hydrochloric acid (50.0 mL, 300 mmol, 298.53 eq) and the mixture stirred at 90 °C for 2 h. The pH of the reaction mixture was adjusted to 9 with NaHCO₃ and the mixture was extracted with EtOAc (50 mL × 3). The combined organic layers were dried over anhydrous Na₂SO₄ and concentrated to give the title compound (450 mg, 0.870 mmol, 86.7 % yield) as a light brown solid. MS (ESP): m/z = 517.2 [M+H]⁺.

### Step 7:

### 6-[5-(2-Aminoethyl)-2-oxo-1,3,4-oxadiazol-3-yl]-4H-1,4-benzoxazin-3-one; formic acid

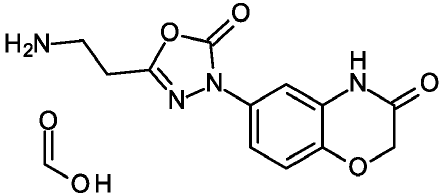

To a solution of 6-[5-[2-[bis[(4-methoxyphenyl)methyl]amino]ethyl]-2-oxo-1,3,4-oxadiazol-3-yl]-4*H*-1,4-benzoxazin-3-one (440.0 mg, 0.850 mmol, 1 eq) in methanol (44 mL) was added palladium/C (500.0 mg) and trifluoroacetic acid (0.33 mL, 4.26 mmol, 5 eq) and the reaction mixture was stirred under an atmosphere of hydrogen at 25 °C for 4 h. The reaction mixture was filtered and filtrate was concentrated. The residue was purified by preparative HPLC (formic acid) and lyophilized to give the title compound (90 mg, 0.280 mmol, 32 % yield) as a white solid. MS (ESP): m/z = 277.0 [M+H]⁺.

### Intermediate 32

### [rac-(5,6-trans)-6-Formyl-1-methyl-6,7-dihydro-5H-cyclopenta[c]pyridin-5-yl] acetate

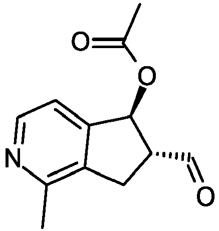

The title compound was prepared by analogy to [*rac*-(5,6-*trans*)-1-chloro-6-formyl-6,7-dihydro-5*H*-cyclopenta[c]pyridin-5-yl] acetate (Intermediate 30, steps 4 and 5) using [*rac*-(5,6-*trans*)-6-[[*tert*-butyl(diphenyl)silyl]oxymethyl]-1-methyl-6,7-dihydro-5*H*-cyclopenta[c]pyridin-5-yl] acetate in place of [*rac*-(5,6-*trans*)-6-[[*tert*-butyl(diphenyl)silyl]oxymethyl]-1-chloro-6,7-dihydro-5*H*-cyclopenta[c]pyridin-5-yl] acetate and was obtained as a light brown oil. The crude product was used for the next step directly.

The [*rac*-(5,6-*trans*)-6-[[*tert*-butyl(diphenyl)silyl]oxymethyl]-1-methyl-6,7-dihydro-5*H-*cyclopenta[c]pyridin-5-yl] acetate used as starting material was prepared as follows:

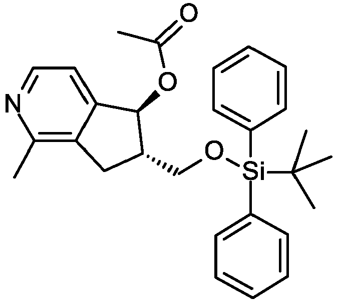

To a solution of [rac-(5,6-*trans*)-6-[[*tert*-butyl(diphenyl)silyl]oxymethyl]-1-chloro-6,7-dihydro-5*H*-cyclopenta[c]pyridin-5-yl] acetate (Intermediate 30, step 3, 100.0 mg, 0.210 mmol, 1 eq) in 1,4-dioxane (4 mL) was added [1,1'-bis(diphenylphosphino)ferrocene] dichloropalladium(II) (30.5 mg, 0.040 mmol, 0.200 eq), and 1.0M Me₂Zn in toluene (0.62 mL, 0.620 mmol, 3 eq). The mixture was stirred for 16 h at 100 °C under N₂. The reaction was quenched with NH₄Cl. EtOAc (20 mL) was added, the layers were separated and the organic layer was washed with saturated brine solution (2 × 20 mL). The organic layer was dried (MgSO₄) before concentration to dryness. The crude product was then purified by preparative TLC (PE/EtOAc=1/1) to give the title compound (70 mg, 0.150 mmol, 73.1 % yield) as a white solid. MS (ESP): m/z = 460.3 [M+H]⁺.

### Intermediate 33

### 6-[5-(2-Aminoethyl)-2-oxo-1,3,4-oxadiazol-3-yl]-4H-pyrido[3,2-b][1,4]oxazin-3-one;formic acid

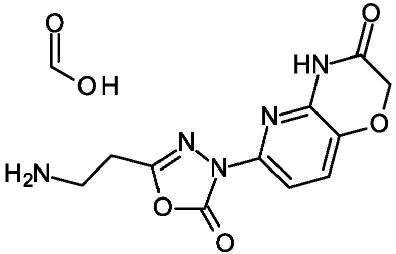

### Steps 1 to 3:

### 5-[2-(Dibenzylamino)ethyl]-3H-1,3,4-oxadiazol-2-one

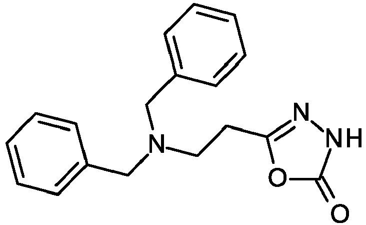

The title compound was prepared by analogy to 5-[2-[bis[(4-Methoxyphenyl)methyl]amino]ethyl]-3*H*-1,3,4-oxadiazol-2-one (Intermediate 31, steps 2 to 4) using benzylchloride in place of 4-methoxybenzylchloride and was obtained as a white solid. MS (ESP): m/z = 310.1 [M+H]⁺.

### Step 4:

### Ethyl 2- [6- [5- [2-(dibenzylamino)ethyl] -2-oxo-1,3,4-oxadiazol-3 -yl] -2-nitropyridin-3 - yl]oxyacetate

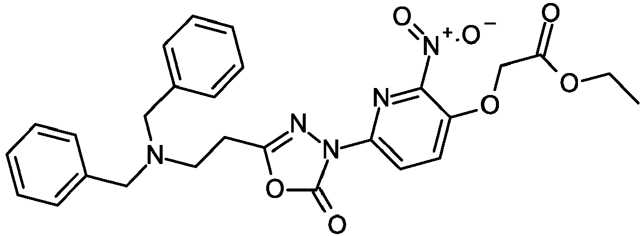

The title compound was prepared by analogy to ethyl 2-[6-[5-[2-[*tert-*butyl(dimethyl)silyl]oxyethyl]-2-oxo-1,3-oxazolidin-3-yl]-2-nitropyridin-3-yl]oxyacetate (Intermediate 1, step 4) using 5-[2-(dibenzylamino)ethyl]-3*H*-1,3,4-oxadiazol-2-one in place of 5-[2-[*tert*-butyl(dimethyl)silyl]oxyethyl]-1,3-oxazolidin-2-one and was obtained as a light yellow oil. MS (ESP): m/z = 534.1 [M+H]⁺.

### Step 5:

### 6-[5-[2-(Dibenzylamino)ethyl]-2-oxo-1,3,4-oxadiazol-3-yl]-4H-pyrido[3,2-b][1,4]oxazin-3-one

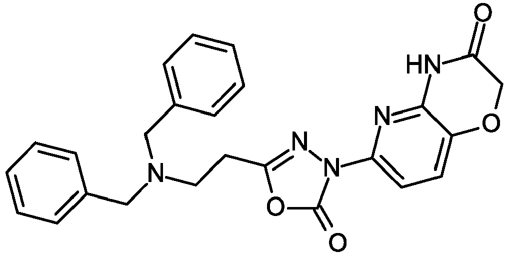

To a solution of ethyl 2-[6-[5-[2-(dibenzylamino)ethyl]-2-oxo-1,3,4-oxadiazol-3-yl]-2-nitropyridin-3-yl]oxyacetate (950.0 mg, 1.78 mmol, 1 eq) in ethanol (31.67 mL) were added iron (497.19 mg, 8.9 mmol, 5 eq) and hydrochloric acid (5.94 mL, 17.81 mmol, 10 eq). The reaction mixture was stirred at 25 °C for 12 h. The reaction mixture was diluted with EtOAc (100 mL) neutralised by the addition of NaHCO₃. The mixture was filtered and filtrate was concentrated to give the title compound (700 mg, 1.53 mmol, 85.9% yield) as a dark brown oil which was used directly in the next step without further purification. MS (ESP): m/z = 458.1 [M+H]⁺.

### Step 6:

### 6-[5-(2-Aminoethyl)-2-oxo-1,3,4-oxadiazol-3-yl]-4H-pyrido[3,2-b][1,4]oxazin-3-one; formic acid

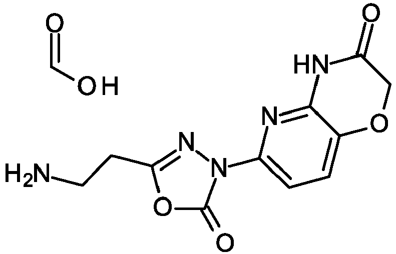

The title compound was prepared by analogy to 6-[5-(2-aminoethyl)-2-oxo-1,3,4-oxadiazol-3-yl]-4*H*-1,4-benzoxazin-3-one; formic acid (Intermediate 31, step 7) using 6-[5-[2-(dibenzylamino)ethyl]-2-oxo-1,3,4-oxadiazol-3-yl]-*4*H-pyrido[3,2-b][1,4]oxazin-3-one in place of 6-[5-[2-[bis[(4-methoxyphenyl)methyl]amino]ethyl]-2-oxo-1,3,4-oxadiazol-3-yl]-4*H*-1,4-benzoxazin-3-one and was obtained as a light brown solid. MS (ESP): m/z = 278.0 [M+H]⁺.

### Intermediate 34

### 7-[5-(2-Aminoethyl)-2-oxo-1,3-oxazolidin-3-yl]-1H-pyrido[2,3-b][1,4]oxazin-2-one; 2,2,2-trifluoroacetic acid

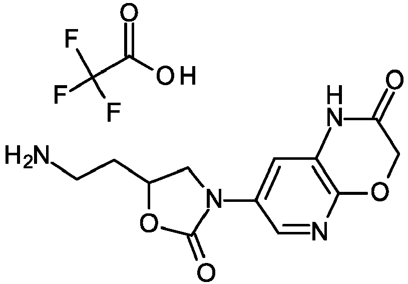

### Step 1:

### Ethyl 2-(5-bromo-3-nitropyridin-2-yl)oxyacetate

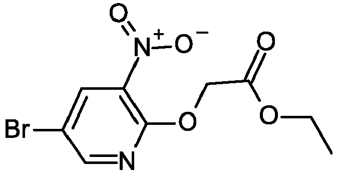

5-Bromo-2-chloro-3-nitropyridine (5000.0 mg, 21.06 mmol, 1 eq) and ethyl glycolate (2411.35 mg, 23.16 mmol, 1.1 eq) were dissolved in THF (100 mL) and cooled to 0 °C. Sodium hydride, 60% in oil (926.46 mg, 23.16 mmol, 1.1 eq) was added and the reaction mixture was stirred at 20°C for 1 h. The reaction mixture was poured into ice water and extracted with EtOAc (50.0 mL × 3). The combined organic layers were washed with brine (50.0 mL × 2), dried with Na₂SO₄ and concentrated to provide the crude product. The crude product was purified by column (PE: EtOAc=50:1 to 5:1) to give title compound (6 g, 19.67 mmol, 93.4 % yield) as a yellow oil. MS (ESP): m/z = 305.0 and 307.0 [M+H]⁺.

### Step 2:

### Ethyl 2-[5-[5-[2-[(2-methylpropan-2-yl)oxycarbonylamino]ethyl]-2-oxo-1,3-oxazolidin-3-yl]-3-nitropyridin-2-yl]oxyacetate

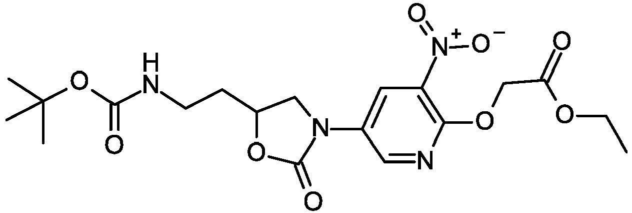

To a solution of ethyl 2-[(5-bromo-3-nitro-2-pyridyl)oxy] acetate (0.79 g, 2.61 mmol, 1.2 eq) and *tert*-butyl *N*-[2-(2-oxooxazolidin-5-yl)ethyl]carbamate (Intermediate 26, 500.0 mg, 2.17 mmol, 1 eq) in 1,4-dioxane (10 mL) were added potassium carbonate (600.22 mg, 4.34 mmol, 2 eq), copper (I) iodide (82.71 mg, 0.430 mmol, 0.200 eq) and *N,N*'-dimethyl-ethylenediamine (0.01 mL, 0.870 mmol, 0.400 eq). The mixture was degassed with nitrogen three times and stirred at 100 °C for 2 h. The solid was filtered off and the filtrate was concentrated to give the crude product, which was purified by flash column chromatography to give the title compound (800 mg, 1.76 mmol, 81.1 % yield) as a yellow oil. MS (ESP): m/z = 477.2 [M+H]⁺.

### Step 3:

### tert-Butyl N-[2-[2-oxo-3-(2-oxo-1H-pyrido[2,3-b][1,4]oxazin-7-yl)-1,3-oxazolidin-5-yl]ethyl]carbamate

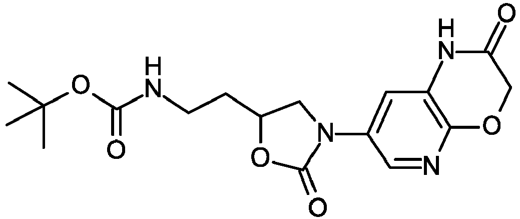

A mixture of ethyl 2-[[5-[5-[2-(*tert*-butoxycarbonylamino)ethyl]-2-oxo-oxazolidin-3-yl]-3-nitro-2-pyridyl]oxy]acetate (0.8 g, 1.76 mmol, 1 eq) and iron (491.56 mg, 8.8 mmol, 5 eq) in acetic acid (10 mL) was stirred at 50 °C for 2 h. The solid was filtered off and washed with EtOAc (100 mL), filtrate was concentrated and the residue was taken up in EtOAc (50 mL) The pH of the mixture was adjusted to 8 by progressively adding NaHCO₃ aq. The mixture was extracted with EtOAc (50 mL × 3) and the combined organic layers were dried with Na₂SO₄. The solvent was removed to give the crude product, which was purified by flash chromatography on silica gel (PE:EA=2:1) to give the title compound (600 mg, 1.59 mmol, 90.1% yield) as a yellow solid. MS (ESP): m/z = 379.0 [M+H]⁺.

### Step 4:

### 7-[5-(2-Aminoethyl)-2-oxo-1,3-oxazolidin-3-yl]-1H-pyrido[2,3-b][1,4]oxazin-2-one; 2,2,2-trifluoroacetic acid

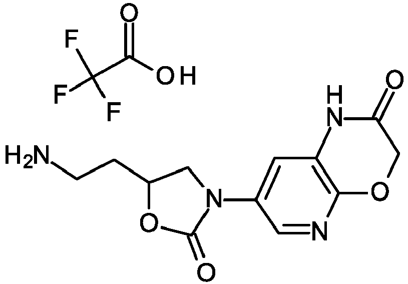

To a solution of *tert*-butyl *N*-[2-[2-oxo-3-(2-oxo-1*H*-pyrido[2,3-b][1,4]oxazin-7-yl)-1,3-oxazolidin-5-yl]ethyl]carbamate (300.0 mg, 0.790 mmol, 1 eq) in DCM (10 mL) was added trifluoroacetic acid (0.61 mL, 7.93 mmol, 10 eq) and then the solution was stirred at 25 °C for 16 h. The solvent was removed to give the crude title compound (300 mg, 0.760 mmol, 96.5% yield) as a brown solid which was used directly in the next step without further purification.

### Intermediate 35

### 6-Formyl-1-methyl-6,7-dihydro-5H-cyclopenta[c]pyridine-4-carbonitrile

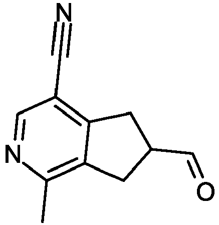

### Steps 1 to 7:

### Ethyl 1,4-dibromo-6,7-dihydro-5H-cyclopenta[c]pyridine-6-carboxylate

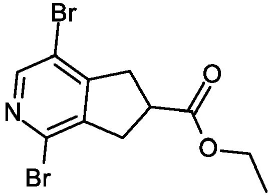

The title compound was prepared by analogy to ethyl 1-bromo-4-fluoro-6,7-dihydro-5*H-*cyclopenta[c]pyridine-6-carboxylate (Intermediate 27, steps 1 to 7) using 2,5-dibromo-3-methylpyridine (CAS# 3430-18-0) in place of 2-bromo-5-fluoro-3-methyl-pyridine and was obtained as a yellow oil. MS (ESP): m/z = 350.0 [M+H]⁺.

### Step 8:

### (1,4-Dibromo-6,7-dihydro-5H-cyclopenta[c]pyridin-6-yl)methanol

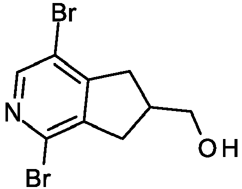

To a mixture of ethyl 1,4-dibromo-6,7-dihydro-5*H*-cyclopenta[c]pyridine-6-carboxylate (450.0 mg, 1.34 mmol, 1 eq) in THF (30 mL) under ice-water was added LiAlH₄/THF (1.61 mL, 1.61 mmol, 1.2 eq) and then the reaction was stirred at 0 °C for another 1 h. The mixture was quenched with water (0.1 mL) and then diluted with EtOAc (3 mL), dried over Na₂SO₄ and filtered. The filtrate was concentrated under vacuum to give the title compound (300 mg, 0.980 mmol, 72.7 % yield) as a colourless oil. The crude product was used for the next step directly. MS (ESP): m/z = 307.9 [M+H]⁺.

### Step 9:

### 1-Bromo-6-(hydroxymethyl)-6,7-dihydro-5H-cyclopenta[c]pyridine-4-carbonitrile

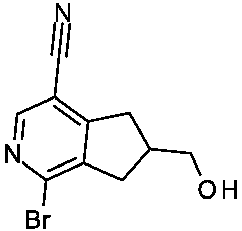

A mixture of (1,4-dibromo-6,7-dihydro-5*H*-cyclopenta[c]pyridin-6-yl)methanol (200.0 mg, 0.650 mmol, 1 eq) and copper(1) cyanide (0.03 mL, 0.650 mmol, 1 eq) in DMA (9.1 mL) was degassed and filled with N₂ three times. The reaction mixture was stirred at 120 °C for 5 h. The reaction was quenched with brine (15 mL) and extracted with EtOAc (2 × 20 mL). The organic layers were washed with brine (2 × 20 mL), then dried over Na₂SO₄ and concentrated under vacuum to give a residue which was purified by prep-TLC (PE/EtOAc=1/1) to give the title compound (50 mg, 0.200 mmol, 30.3 % yield) as a white solid.

### Step 10:

### 6-(Hydroxymethyl)-1-methyl-6,7-dihydro-5H-cyclopenta[c]pyridine-4-carbonitrile

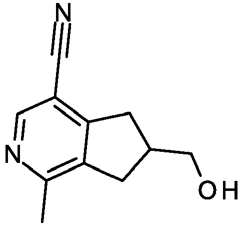

To a solution of 1-bromo-6-(hydroxymethyl)-6,7-dihydro-5*H*-cyclopenta[c]pyridine-4-carbonitrile (50.0 mg, 0.200 mmol, 1 eq) in 1,4-dioxane (3 mL) and water (0.865 mL) were added K₂CO₃ (81.79 mg, 0.590 mmol, 3 eq), trimethylboroxine (496 mg, 3.95 mmol, 20 eq) and [1,1'-bis(diphenylphosphino)ferrocene]dichloropalladium(II) (14.5 mg, 0.020 mmol, 0.100 eq). The mixture was stirred for 16 h at 100 °C under N₂. The reaction mixture was concentrated to dryness and the residue was taken up in EtOAc (20 mL) and the solution was washed with saturated brine solution (2 × 20 mL), dried (MgSO₄) and concentrated to dryness. The crude product was then purified by prep-TLC (PE/EtOAc=1/1) to give the title compound (40 mg, 0.210 mmol, 107 % yield) as a light yellow solid. MS (ESP): m/z = 189.1 [M+H]⁺.

### Step 11:

### 6-Formyl-1-methyl-6,7-dihydro-5H-cyclopenta[c]pyridine-4-carbonitrile

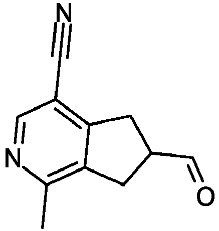

To a mixture of 6-(hydroxymethyl)-1-methyl-6,7-dihydro-5*H*-cyclopcnta[c]pyridinc-4-carbonitrile (40.0 mg, 0.210 mmol, 1 eq) in DCM (4 mL) was added Dess-Martin periodinane (117.17 mg, 0.280 mmol, 1.3 eq) and then the reaction was stirred at 25 °C for 2 h. The mixture was filtered and the filtrate was concentrated under vacuum to give the crude title compound (20 mg, 0.110 mmol, 50.5% yield) as a light brown solid. MS (ESP): m/z = 187.1 [M+H]⁺.

### Intermediate 36

### 2-[(4-Cyano-2-formyl-2,3-dihydro-1H-inden-5-yl)oxy]-N-methylacetamide

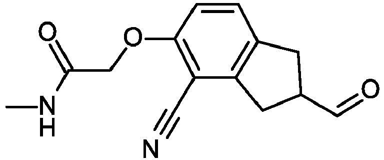

### Step 1:

### Ethyl 7-bromo-6-methoxy-3-oxo-1,2-dihydroindene-2-carboxylate

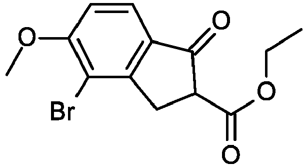

To a solution of 4-bromo-5-methoxy-indan-1-one (CAS# 436803-36-0, 6278.0 mg, 15.62 mmol, 1 eq) in toluene (90 mL) was added sodium hydride, 60% in oil (1874.78 mg, 46.87 mmol, 3 eq) at 0°C. The mixture was heated to reflux (about 110°C). A solution of diethyl carbonate (4245 mg, 35.9 mmol, 2.3 eq) in toluene (90 mL) was added dropwise over 1 h. After the addition, the mixture was kept at the same temperature for 16 h. The reaction was cooled to room temperature and poured into saturated aq. NH₄Cl (100 mL) and extracted with EtOAc (3 × 150 mL). The combined organic layers were concentrated under reduced pressure and the obtained crude product was purified by flash chromatography (35% EA/PE) to give the title compound (4 g, 12.77 mmol, 74.7 % yield) as brown solid. MS (ESP): m/z = 313.0 [M+H]⁺.

### Step 2:

### Ethyl 4-bromo-5-methoxy-2,3-dihydro-1H-indene-2-carboxylate

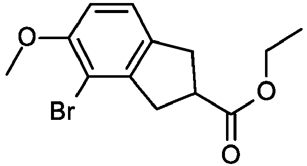

To a mixture of ethyl 7-bromo-6-methoxy-3-oxo-1,2-dihydroindene-2-carboxylate (4.0 g, 12.77 mmol, 1 eq) in trifluoroacetic acid (70.0 mL) was added triethylsilane (14.3 mL, 89.42 mmol, 7 eq) at 0 °C. Then the mixture was warmed to 25 °C and stirred for 16 h. All volatiles were removed in vacuo and the residue was diluted with EtOAc (150 mL). The solution was washed with water (50 mL), saturated aq. NaHCO₃ (100 mL), brine (100 mL) and dried over Na₂SO₄. After filtration, the filtrate was concentrated under reduced pressure and the crude product was purified by flash chromatography (15% EA/PE) to give the impure title compound (5600 mg) as light yellow oil. MS (ESP): m/z = 298.9 [M+H]⁺.

### Step 3:

### Ethyl 4-bromo-5-hydroxy-2,3-dihydro-1H-indene-2-carboxylate

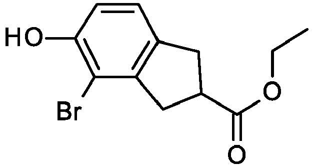

To a solution of ethyl 4-bromo-5-methoxy-2,3-dihydro-1*H*-indene-2-carboxylate (5600.0 mg, 11.23 mmol, 1 eq) in DCM (120 mL) was added boron tribromide (5.21 mL, 56.16 mmol, 5 eq) and the solution was stirred at 25 °C for 3 h. The solution was cooled to 0 °C and EtOH (2 mL) was added. Then all solvents were removed in vacuo to give a residue which was purified by flash chromatography (25% PE:EA) to give the title compound (2500 mg, 8.77 mmol, 77.3% yield) as yellow solid. MS (ESP): m/z = 285.0 and 287.0 [M+H]⁺.

### Step 4:

### Ethyl 4-bromo-5-[2-(methylamino)-2-oxoethoxy]-2,3-dihydro-1H-indene-2-carboxylate

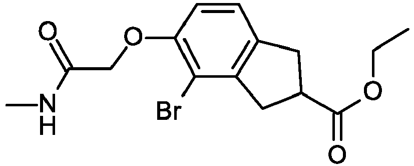

A mixture of ethyl 4-bromo-5-hydroxy-2,3-dihydro-1*H*-indene-2-carboxylate (350.0 mg, 1.23 mmol, 1 eq), potassium carbonate (254.47 mg, 1.84 mmol, 1.5 eq) and 2-chloro-*N-*methylacetamide (158.41 mg, 1.47 mmol, 1.2 eq) in MeCN (25 mL) was stirred at 80 °C for 3 h. The reaction was filtered and the filtrate was concentrated to give a residue which was purified by flash chromatography (85% EA:PE) to give title compound (320 mg, 0.900 mmol, 70.8% yield) as a white solid. MS (ESP): m/z = 355.9 and 357.9 [M+H]⁺.

### Step 5:

### 2-[[4-Bromo-2-(hydroxymethyl)-2,3-dihydro-1H-inden-5-yl]oxy]-N-methylacetamide

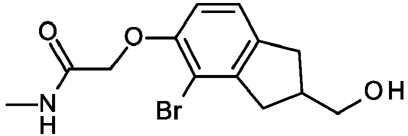

To a solution of ethyl 4-bromo-5-[2-(methylamino)-2-oxoethoxy]-2,3-dihydro-1*H*-indene-2-carboxylate (345.0 mg, 0.970 mmol, 1 eq) in THF (17 mL) was added lithium aluminium hydride (44.1 mg, 1.16 mmol, 1.2 eq) at 0 °C and then the mixture was stirred at -15 °C for 2 h. The reaction was quenched with Na2SO₄•10H₂O and diluted with EtOAc (50 mL). After filtration the filtrate was concentrated to give a crude product, which was purified by flash chromatography (1:1 EtOAc /PE) to give the title compound (125 mg, 0.400 mmol, 40.4% yield) as a white solid. MS (ESP): m/z = 314.1 and 316.1 [M+H]⁺.

### Step 6:

### 2-[[4-Cyano-2-(hydroxymethyl)-2,3-dihydro-1H-inden-5-yl]oxy]-N-methylacetamide

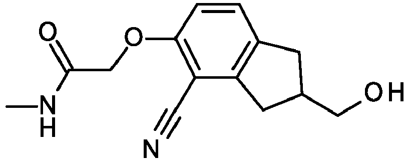

A mixture of zinc cyanide (233.57 mg, 1.99 mmol, 5 eq), tris(dibenzylideneacetone)dipalladium (0) (155.46 mg, 0.170 mmol, 0.430 eq), 2-di-*tert*-butylphosphino-2',4',6'-triisopropylbiphenyl (108.13 mg, 0.250 mmol, 0.640 eq) and 2-[[4-bromo-2-(hydroxymethyl)-2,3-dihydro-1*H*-inden-5-yl]oxy]-*N*-methylacetamide (125.0 mg, 0.400 mmol, 1 eq) in *N*,*N*-dimethylacetamide (5 mL) was degassed with N₂ three times. The mixture was directly stirred at 110 °C for 2 h. The mixture was diluted with EtOAc (10 mL) and filtered through celite. The filtrate was washed with H₂O (2 × 10 mL), brine (10 mL), dried and concentrated to give a residue which was purified by flash chromatography (95% PE/EA) to give title compound (96 mg, 0.370 mmol, 89.6% yield) as a white solid. MS (ESP): m/z = 261.1 [M+H]⁺.

### Step 7:

### 2-[(4-Cyano-2-formyl-2,3-dihydro-1H-inden-5-yl)oxy]-N-methylacetamide

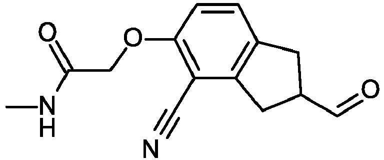

To a mixture of 2-[[4-cyano-2-(hydroxymethyl)-2,3-dihydro-1*H*-inden-5-yl]oxy]-*N-*methylacetamide (96.0 mg, 0.370 mmol, 1 eq) in DCM (5 mL) was added Dess-Martin periodinane (187.72 mg, 0.440 mmol, 1.2 eq) at 0 °C , and then the mixture was stirred at 25 °C for 2 h. The mixture was filtrated and the filtrate was concentrated to give the crude title compound (150 mg, 0.580 mmol, 78.7% yield) as light yellow oil, which was used directly without further purification. MS (ESP): m/z = 259.1 [M+H]⁺.

### Intermediate 37

### 1-Chloro-3-methoxy-6,7-dihydro-5H-cyclopenta[c]pyridine-6-carbaldehyde

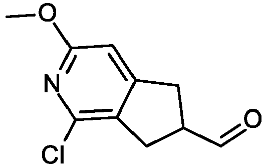

### Step 1:

### Dimethyl 2-benzyl-3-oxo-5,7-dihydrocyclopenta[c]pyridine-6,6-dicarboxylate

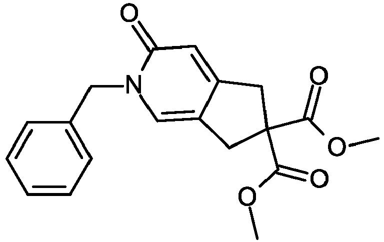

To a solution of dimethyl 2,2-bis(prop-2-ynyl)propanedioate (2.0 g, 9.61 mmol, 1 eq) and benzyl isocyanate (2.56 g, 19.21 mmol, 2 eq) in 1,2-dichloroethane (50 mL) was added chloro(1,5-cyclooctadiene(pentamethylcyclopentadienyl)ruthenium(II) (182.47 mg, 0.480 mmol, 0.050 eq). The mixture was heated to 90 °C and stirred for 5 h. The reaction mixture was concentrated in vacuo to give a crude product which was first purified by silica gel column chromatography (PE:EtOAc =3:1 to 1:1) and then triturated with DCM (30 mL), filtered and dried under vacuum to give the title compound (2500 mg, 7.32 mmol, 76.2 % yield) as a light yellow solid. MS (ESP): m/z = 342.1 [M+H]⁺.

### Step 2:

### Dimethyl 3-oxo-5,7-dihydro-2H-cyclopenta[c]pyridine-6,6-dicarboxylate

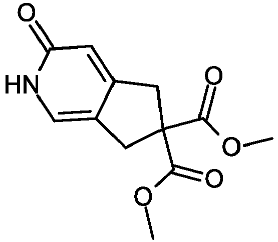

To a solution of dimethyl 2-benzyl-3-oxo-5,7-dihydrocyclopenta[c]pyridine-6,6-dicarboxylate (0.5 g, 1.46 mmol, 1 eq) in acetic acid (10 mL) was added Pd(OH)₂/C (155.87 mg, 0.290 mmol, 0.200 eq). The mixture was stirred at 20 °C under H₂ (15 psi) for 24 h. The mixture was filtered through celite and the filter cake was washed with MeOH (50 mL). The filtrate was concentrated in vacuo to give a crude product which was triturated with ethyl acetate (5 mL). The mixture was filtered to give the title compound (250 mg, 1 mmol, 67.9% yield) as a white solid. MS (ESP): m/z = 252.1 [M+H]⁺.

### Step 3:

### Dimethyl 3-methoxy-5,7-dihydrocyclopenta[c]pyridine-6,6-dicarboxylate

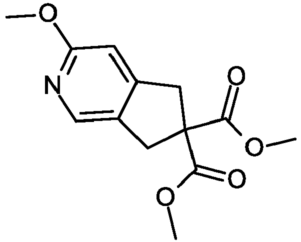

To a mixture of dimethyl 3-oxo-5,7-dihydro-2*H*-cyclopenta[c]pyridine-6,6-dicarboxylate (10000.0 mg, 39.8 mmol, 1 eq) and iodomethane (2.97 mL, 47.76 mmol, 1.2 eq) in 1,2-dichloroethane (100 mL) was added Ag₂CO₃ (21795.1 mg, 79.61 mmol, 2 eq) and then the reaction was stirred at 30 °C for another 16 h. The mixture was filtrated and the filtrate was diluted with DCM (150 mL), washed with H₂O (2 × 50 mL), brine (50 mL), dried over Na₂SO₄ and concentrated under vacuum to give the crude product which was purified by flash chromatography (35%PE/EA) to give the title compound (6.4 g, 24.13 mmol, 57.6 % yield) as light yellow oil. MS (ESP): m/z = 266.1 [M+H]⁺.

### Step 4:

### Dimethyl 3-methoxy-2-oxido-5,7-dihydrocyclopenta[c]pyridin-2-ium-6,6-dicarboxylate

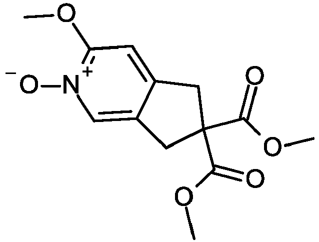

To a mixture of dimethyl 3-methoxy-5,7-dihydrocyclopenta[c]pyridine-6,6-dicarboxylate (6400.0 mg, 24.13 mmol, 1 eq) in DCM (128 mL) was added 3-chloroperbenzoic acid (7322.9 mg, 36.19 mmol, 1.5 eq) at 0°C and then the reaction was stirred at 25 °C for 16 h. The mixture was concentrated and purified by chromatography column on silica gel (PE/EtOAc=1/1 to DCM/CH₃OH=10/1) to give the title compound (4300 mg, 15.29 mmol, 58.9 % yield) as a white solid. MS (ESP): m/z = 282.1 [M+H]⁺.

### Step 5:

### Dimethyl 1-chloro-3-methoxy-5,7-dihydrocyclopenta[c]pyridine-6,6-dicarboxylate

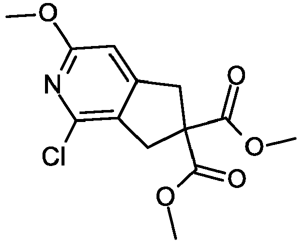

A mixture of dimethyl 3-methoxy-2-oxido-5,7-dihydrocyclopenta[c]pyridin-2-ium-6,6-dicarboxylate (1.8 g, 6.4 mmol, 1 eq) in phosphorus oxychloride (18.0 mL, 193.11 mmol, 30.17 eq) was stirred at 95 °C for 4 h. The mixture was concentrated under vacuum and the resulting residue was diluted with EtOAc (10 mL). The mixture was quenched with NaHCO₃ (2 × 30 mL) and then extracted with EtOAc (3 × 20 mL), the organics were dried over Na₂SO₄, and concentrated under vacuum to give the crude product, which was purified by chromatography column on silica gel (PE/EtOAc=10/1 to 3/1) to give the title compound (500 mg, 1.67 mmol, 25.8 % yield) as a colourless semisolid. MS (ESP): m/z = 300.0 [M+H]⁺.

### Step 6:

### Methyl 1-chloro-3-methoxy-6,7-dihydro-5H-cyclopenta[c]pyridine-6-carboxylate

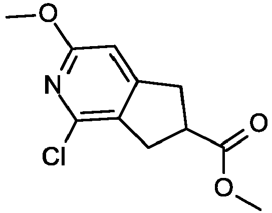

To a solution of dimethyl 1-chloro-3-methoxy-5,7-dihydrocyclopenta[c]pyridine-6,6-dicarboxylate (200.0 mg, 0.670 mmol, 1 eq) in DMSO (2 mL) and water (0.200 mL) was added lithium chloride (28.3 mg, 0.670 mmol, 1 eq). The mixture was heated to 160 °C and stirred for 5 h. The mixture was diluted with EtOAc (30 mL) and washed with brine solution (4 × 20 mL), the organic phase was dried over Na₂SO₄, concentrated under vacuum to give the crude title compound (170 mg, quant. yield) as a light brown oil. The crude product was used for the next step directly. MS (ESP): m/z = 242.1 [M+H]⁺.

### Steps 7 to 8:

### 1-Chloro-3-methoxy-6,7-dihydro-5H-cyclopenta[c]pyridine-6-carbaldehyde

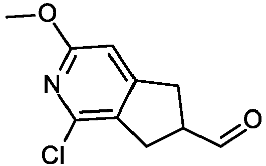

The title compound was prepared by analogy to 4-fluoro-1-methyl-6,7-dihydro-5*H-*cyclopenta[c]pyridine-6-carbaldehyde (Intermediate 27, steps 9 and 10) using methyl 1-chloro-3-methoxy-6,7-dihydro-5*H*-cyclopenta[c]pyridine-6-carboxylate in place of ethyl 4-fluoro-1-methyl-6,7-dihydro-5*H*-cyclopenta[c]pyridine-6-carboxylate and was obtained as a light yellow oil. ¹H-NMR (400 MHz, CDCl₃) δ 9.74 (s, 1H), 6.47 (s, 1H), 3.82 (s, 3H), 3.27 (m, 2H), 3.1 (m, 3H).

### Intermediate 38

### 2-Formyl-5-[(1-methylpyrrolidin-2-yl)methoxy]-2,3-dihydro-1H-indene-4-carbonitrile

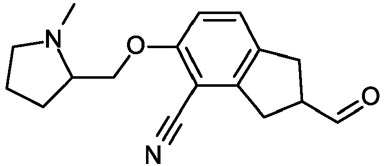

### Step 1:

### tert-Butyl 2-[(4-bromo-2-ethoxycarbonyl-indan-5-yl)oxymethyl]pyrrolidine-1-carboxylate

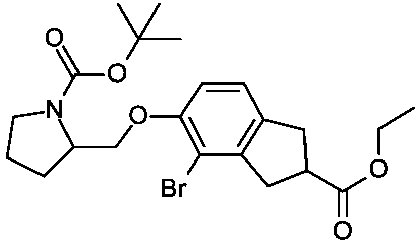

To a mixture of ethyl 4-bromo-5-hydroxy-indane-2-carboxylate (Intermediate 36, step 3, 600.0 mg, 2.1 mmol, 1 eq) and caesium carbonate (1371.16 mg, 4.21 mmol, 2 eq) in DMF (12 mL) was added *tert*-butyl 2-(*p*-tolylsulfonyloxymethyl)pyrrolidine-1-carboxylate (897.57 mg, 2.53 mmol, 1.2 eq) and then the mixture was stirred at 100°C for 1 h. The mixture was poured into 50 mL water and then extracted with EtOAc (30 mL × 2) and the combined organic layers were dried with Na₂SO₄. The solvent was removed to give the crude product which was purified by chromatography on silica gel (PE: EtOAc =10:1) to give the title compound (900 mg, 1.92 mmol, 91.3 % yield) as a colourless oil.

### Step 2:

### tert-Butyl 2-[[4-bromo-2-(hydroxymethyl)-2,3-dihydro-1H-inden-5-yl]oxymethyl]pyrrolidine-1-carboxylate

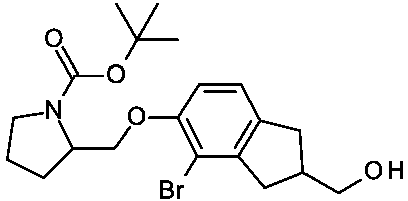

To a solution of *tert*-butyl 2-[(4-bromo-2-ethoxycarbonyl-indan-5-yl)oxymethyl]pyrrolidine-1-carboxylate (900.0 mg, 1.92 mmol, 1 eq) in THF (40 mL) was added lithium aluminum hydride (87.52 mg, 2.31 mmol, 1.2 eq) at 0 °C, and then the solution was stirred at 0 °C for 0.5 h. Drops of water were added and EtOAc was added slowly until there no more gas was generated. Then 5 g Na₂SO₄ was added and the mixture was stirred at room temperature for 30 min. The solid was filtered off and the cake was washed with 20 mL EtOAc. The filtrate was concentrated to give the crude product which was purified by chromtography on silica gel (PE:EA=1:1) to give the title compound (650 mg, 1.52 mmol, 79.3 % yield) as a colorless oil. MS (ESP): m/z = 448.1 [M+Na]⁺.

### Step 3:

### tert-butyl 2-[[4-cyano-2-(hydroxymethyl)-2,3-dihydro-1H-inden-5-yl]oxymethyl]pyrrolidine-1-carboxylate

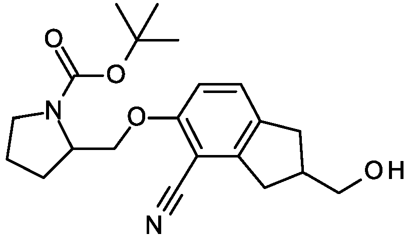

A mixture of *tert*-butyl 2-[[4-bromo-2-(hydroxymethyl)indan-5-yl]oxymethyl]pyrrolidine-1-carboxylate (600.0 mg, 1.41 mmol, 1 eq), zinc cyanide (0.18 mL, 2.81 mmol, 2 eq), tris(dibenzylideneacetone)dipalladium (0) (128.87 mg, 0.140 mmol, 0.100 eq) and *2-*di*-tert-*butylphosphino-2',4',6'-triisopropylbiphenyl (119.51 mg, 0.280 mmol, 0.200 eq) in DMA (12 mL) was degassed and filled with N₂ three times. Then it was stirred at 120 °C for 3 h. The mixture was poured into 30 mL water and then the mixture was extracted with EtOAc (20 mL × 2) and the combined organic extracts were dried with Na₂SO₄. The solvent was removed to give the crude product which was purified by chromatography (PE:EA=2:1)to give the title compound (350 mg, 0.940 mmol, 66.8 % yield) as a yellow oil. MS (ESP): m/z = 395.1 [M+Na]⁺.

### Step 4:

### 2-(Hydroxymethyl)-5-(pyrrolidin-2-ylmethoxy)-2,3-dihydro-1H-indene-4-carbonitrile; 2,2,2-trifluoroacetic acid

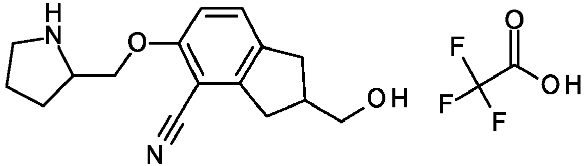

To a solution of *tert*-butyl 2-[[4-cyano-2-(hydroxymethyl)indan-5-yl]oxymethyl]pyrrolidine-1-carboxylate (60.0 mg, 0.160 mmol, 1 eq) in DCM (6 mL) was added trifluoroacetic acid (0.6 mL, 7.79 mmol, 48.34 eq), and then the solution was stirred at 25 °C for 2 h. The solvent was removed to give the title compound (60 mg, 0.160 mmol, 96.4% yield) as a yellow oil.

### Step 5:

### 2-(Hydroxymethyl)-5-[(1-methylpyrrolidin-2-yl)methoxy]-2,3-dihydro-1H-indene-4-carbonitrile

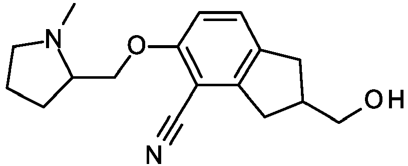

To a mixture of sodium cyanoborohydride (29.28 mg, 0.470 mmol, 3 eq) and 2-(hydroxymethyl)-5-(pyrrolidin-2-ylmethoxy)indane-4-carbonitrile; 2,2,2-trifluoroacetic acid (60.0 mg, 0.160 mmol, 1 eq) in methanol (5 mL) was added a drop of AcOH and the mixture was stirred for 30 min. Formaldehyde (126.02 mg, 1.55 mmol, 10 eq) was added and the mixture was stirred at 25 °C for 16 h. The solvent was removed and 20 mL DCM were added. The mixture was washed with 10 mL brine and dried with Na₂SO₄. The solvent was removed to give the crude product which was purified by prep-TLC (DCM: MeOH=10:1) to the title compound (30 mg, 0.100 mmol, 67.5 % yield) as a white solid. MS (ESP): m/z = 287.1 [M+H]⁺.

### Step 6:

### 2-Formyl-5-[(1-methylpyrrolidin-2-yl)methoxy]-2,3-dihydro-1H-indene-4-carbonitrile

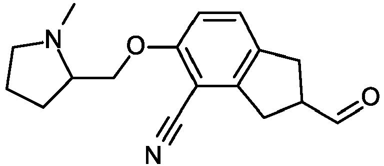

To a solution of 2-(hydroxymethyl)-5-[(1-methylpyrrolidin-2-yl)methoxy]indane-4-carbonitrile (30.0 mg, 0.100 mmol, 1 eq) in DCM (10 mL) was added Dess-Martin periodinane (132.68 mg, 0.310 mmol, 3 eq) and then the solution was stirred at 25 °C for 3 h. The reaction was quenched with 10 mL10% aq. NaHCO₃. The mixture was extracted with DCM (10 mL ×2) and the combined extracts were dried with Na₂SO₄. The solvent was removed to give the crude title compound (30 mg, 0.110 mmol, 90.6 % yield) as a yellow oil.

### Intermediate 39

### 6-Formyl-3-methoxy-6,7-dihydro-5H-cyclopenta[c]pyridine-1-carbonitrile

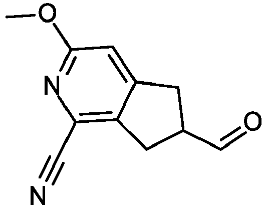

### Step 1:

### 6-(Hydroxymethyl)-3-methoxy-6,7-dihydro-5H-cyclopenta[c]pyridine-1-carbonitrile

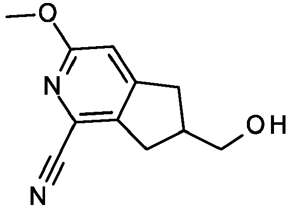

A mixture of (1-chloro-3-methoxy-6,7-dihydro-5*H*-cyclopenta[c]pyridin-6-yl)methanol (Intermediate 37, step 7, 130.0 mg, 0.610 mmol, 1 eq), zinc cyanide (0.08 mL, 1.22 mmol, 2 eq), tris(dibenzylideneacetone)dipalladium (0) (55.72 mg, 0.060 mmol, 0.100 eq) and 2-di*-tert-*butylphosphino-2',4',6'-triisopropylbiphenyl (51.67 mg, 0.120 mmol, 0.200 eq) in DMA (5 mL) was degassed and filled with N₂ three times. Then it was stirred at 120 °C for 3 h. The solution was poured into 30 mL water and then extracted with EtOAc (20 mL × 2) and the combined extracts were dried with Na₂SO₄. The solvent was removed to give the crude product which was purified by prep-TLC (PE:EA=1:1) to give the title compound (100 mg, 0.490 mmol, 80.5 % yield) as a colourless oil. MS (ESP): m/z = 205.1 [M+H]⁺.

### Step 2:

### 6-Formyl-3-methoxy-6,7-dihydro-5H-cyclopenta[c]pyridine-1-carbonitrile

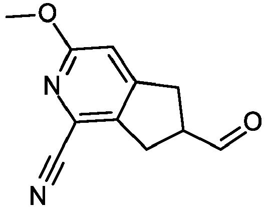

To a solution of 6-(hydroxymethyl)-3-methoxy-6,7-dihydro-5*H*-cyclopenta[c]pyridine-1-carbonitrile (50.0 mg, 0.240 mmol, 1 eq) in DCM (10 mL) was added Dess-Martin periodinane (155.03 mg, 0.370 mmol, 1.5 eq) at 0 °C, and then the solution was stirred at 25 °C for 3 h. The reaction was quenched with 10 mL saturated aq. NaHCO₃. The mixture was extracted with DCM (10 mL × 2) and the combined extracts were dried with Na₂SO₄. The solvent was removed to give the crude title compound (50 mg, 0.250 mmol, 90.9 % yield) as yellow oil.

### Intermediate 40

### 2-Formyl-5-(2-morpholin-4-ylethoxy)-2,3-dihydro-1H-indene-4-carbonitrile

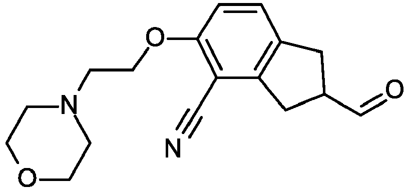

The title compound was prepared by analogy to 2-formyl-5-[(1-methylpyrrolidin-2-yl)methoxy]-2,3-dihydro-1*H*-indene-4-carbonitrile (Intermediate 38, steps 1 to 3 and 6) using 4-(2-chloroethyl)morpholine hydrochloride in place of *tert-*butyl 2-(*p-*tolylsulfonyloxymethyl)pyrrolidine-1-carboxylate and was obtained as an off-white semisolid.

### Intermediate 41

### 4-Chloro-l-methyl-6,7-dihydro-5H-cyclopenta[c]pyridine-6-carbaldehyde

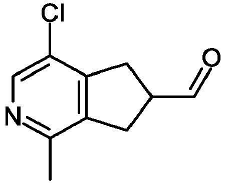

The title compound was prepared by analogy to 4-fluoro-1-methyl-6,7-dihydro-5*H-*cyclopenta[c]pyridine-6-carbaldehyde (Intermediate 27) using 2-bromo-5-chloro-3-methylpyridine in place of 2-bromo-5-fluoro-3-methyl-pyridine and was obtained as a dark green semisolid. MS (ESP): m/z = 196.1 [M+H]⁺.

### Intermediate 42

### N-[2-[(4-Cyano-2-formyl-2,3-dihydro-1H-inden-5-yl)oxy] ethyl] acetamide

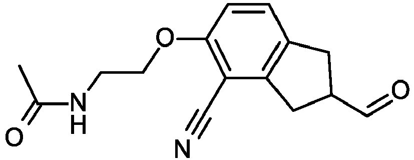

### Step 1:

### Ethyl 4-bromo-5-[2-(tert-butoxycarbonylamino)ethoxy]indane-2-carboxylate

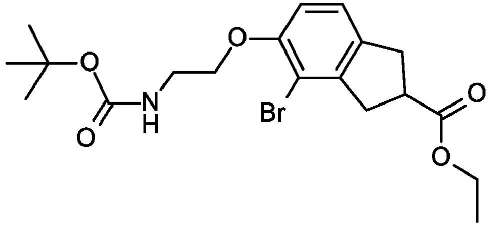

The title compound was prepared by analogy to *tert*-butyl 2-[(4-bromo-2-ethoxycarbonyl-indan-5-yl)oxymethyl]pyrrolidine-1-carboxylate (Intermediate 38, step 1) using *tert-*butyl *N-*(2-chloroethyl)carbamate in place of 4-(2-chloroethyl)morpholine hydrochloride and was obtained as a yellow oil. ¹H-NMR (400 MHz, CDCl₃) δ 7.07 (d, 1H), 6.72 (d, 1H), 5.13 (br s, 1H), 4.22 (q, 2H), 4.06 (m, 2H), 3.58 (m, 2H), 3.3 (m, 5H), 1.47 (s, 9H), 1.30 (t, 3H).

### Step 2:

### Methyl 5-(2-aminoethoxy)-4-bromo-2,3-dihydro-1H-indene-2-carboxylate hydrochloride

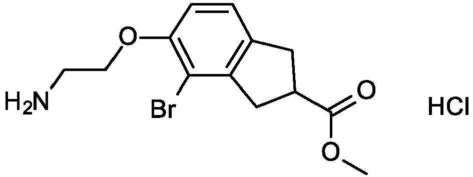

To a solution of ethyl 4-bromo-5-[2-(*tert*-butoxycarbonylamino)ethoxy]indane-2-carboxylate (950.0 mg, 2.22 mmol, 1 eq) in methanol (40 mL) was added HCl (gas) in MeOH (5.0 mL, 20 mmol, 9.02 eq) at 0 °C, then the reaction mixture was stirred at 25 °C for 1 h. The solvent was removed to give the crude title compound (750 mg, 2.14 mmol, 92.7% yield) as a yellow solid. MS (ESP): m/z = 316.0 [M+H]⁺.

### Step 3:

### Methyl 5-(2-acetamidoethoxy)-4-bromo-2,3-dihydro-1H-indene-2-carboxylate

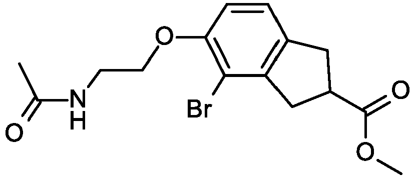

To a solution of methyl 5-(2-aminoethoxy)-4-bromo-indane-2-carboxylate hydrochloride (288.47 mg, 0.820 mmol, 1 eq) in 1,2-dichloroethane (30 mL) was added triethylamine (0.23 mL, 1.65 mmol, 2 eq). The solution was cooled to 0 °C, acetic anhydride (0.15 mL, 1.65 mmol, 2 eq) was added dropwise and the reaction mixture was stirred at 0 °C for 0.5 h. The solution was washed with 10 mL brine and dried with Na₂SO₄. The solvent was removed to give the title compound (300 mg, 0.840 mmol, 98.5% yield) as a yellow solid. MS (ESP): m/z = 356.0 [M+H]⁺.

### Steps 4 to 6:

### N-[2-[(4-Cyano-2-formyl-2,3-dihydro-1H-inden-5-yl)oxy]ethyl]acetamide

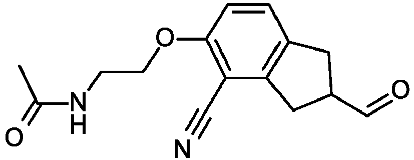

The title compound was prepared by analogy to 2-formyl-5-[(1-methylpyrrolidin-2-yl)methoxy]-2,3-dihydro-1*H*-indene-4-carbonitrile (Intermediate 38, steps 2, 3 and 6) using methyl 5-(2-acetamidoethoxy)-4-bromo-2,3-dihydro-1*H*-indene-2-carboxylate in place of *tert*-butyl 2-[(4-bromo-2-ethoxycarbonyl-indan-5-yl)oxymethyl]pyrrolidine-1-carboxylate and was obtained as a yellow oil.

### Intermediate 43

### 1-Chloro-4-methoxy-6,7-dihydro-5H-cyclopenta[c]pyridine-6-carbaldehyde

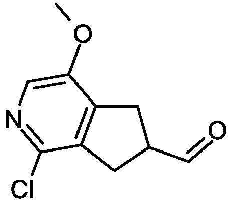

### Step 1:

### 6-[[tert-Butyl(diphenyl)silyl]oxymethyl]-1-chloro-6,7-dihydro-5H-cyclopenta[c]pyridin-4-ol

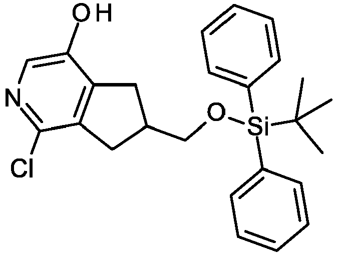

To a mixture of [6-[[*tert*-butyl(diphenyl)silyl]oxymethyl]-1-chloro-6,7-dihydro-5H-cyclopenta[c]pyridin-4-yl] acetate and [6-[[*tert*-butyl(diphenyl)silyl]oxymethyl]-1-chloro-6,7-dihydro-5*H*-cyclopenta[c]pyridin-5-yl] acetate (Intermediate 30, step 3, 1600.0 mg, 3.33 mmol, 1 eq) in THF (20 mL) and water (20 mL) was added LiOH.H₂O (699.19 mg, 16.66 mmol, 5 eq) at 0 °C and then the reaction was stirred at 25 °C for 3 h. Water (30 mL) was added and the mixture was extracted with DCM (100 mL). The combined extracts were dried with Na₂SO₄ and concentrated under vacuum to give the crude product. The crude product was purified by preparative TLC (PE/EtOAc=3/1) to give the title compound (250 mg, 0.570 mmol, 17.1% yield) as a light yellow solid. MS (ESP): m/z = 438.2 [M+H]⁺.

### Step 2:

### tert-Butyl-[(1-chloro-4-methoxy-6,7-dihydro-5H-cyclopenta[c]pyridin-6-yl)methoxy]-diphenylsilane

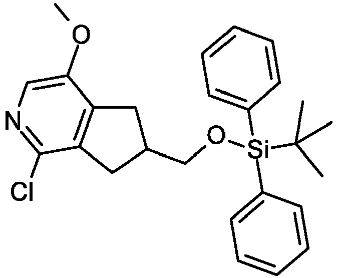

To a mixture of 6-[[*tert*-butyl(diphenyl)silyl]oxymethyl]-1-chloro-6,7-dihydro-5H-cyclopenta[c]pyridin-4-ol (200.0 mg, 0.460 mmol, 1 eq) in DMF (8 mL) was added potassium carbonate (189.31 mg, 1.37 mmol, 3 eq) and the mixture stirred at 25 °C for 30 min. After 30 min. dimethyl sulfate (0.09 mL, 0.910 mmol, 2 eq) was added and then the reaction was stirred at 30 °C for 12 h. The mixture was quenched with brine (10 mL) and then extracted with EtOAc (15 mL × 2). The combined organic layers were washed with brine (15 mL × 3), dried over Na₂SO₄ and then concentrated under vacuum. The resulting residue was purified by flash chromatography column on silica gel to give the title compound (150 mg, 0.330 mmol, 72.7 % yield) as a white semi-solid. MS (ESP): m/z = 452.2 [M+H]⁺.

### Step 3:

### (1-Chloro-4-methoxy-6,7-dihydro-5H-cyc1openta[c]pyridin-6-yl)methanol

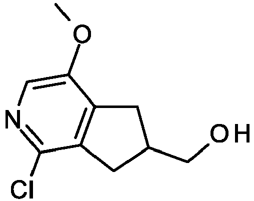

To a mixture of *tert*-butyl-[(1-chloro-4-methoxy-6,7-dihydro-5*H*-cyclopenta[c]pyridin-6-yl)methoxy]-diphenyl-silane (150.0 mg, 0.330 mmol, 1 eq) in THF (5 mL) was added TBAF (0.66 mL, 0.660 mmol, 2 eq) and the mixture stirred at 25 °C for 5 h. The mixture was concentrated under vacuum to give a residue which was purified by preparative TLC (PE/EtOAc=1/1) to give the title compound (50 mg, 0.230 mmol, 70.5% yield) as a colourless oil.

### Step 4:

### 1-Chloro-4-methoxy-6,7-dihydro-5H-cyc1openta[c]pyridine-6-carbaldehyde

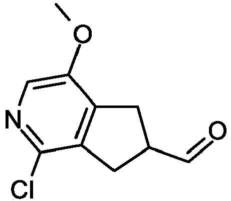

To a solution of (1-chloro-4-methoxy-6,7-dihydro-5*H*-cyclopenta[c]pyridin-6-yl)methanol (50.0 mg, 0.230 mmol, 1 eq) in DCM (3 mL) was added Dess-Martin periodinane (148.88 mg, 0.350 mmol, 1.5 eq) at 0 °C. The mixture was filtrated and the filtrate was concentrated to give the title compound (50 mg, 0.240 mmol, 90.9% yield) as a yellow oil which was used directly without further purification. ¹H-NMR (400 MHz, CDCl₃) δ 9.71 (s, 1H), 8.21 (d, 1H), 3.84 (s, 3H), 3.2 (m, 5H).

### Intermediate 44

### N-[2-[(4-Cyano-2-formyl-2,3-dihydro-1H-inden-5-yl)oxy]ethyl]methanesulfonamide

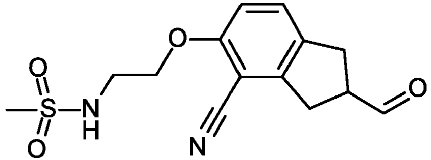

### Step 1:

### N-[2-[[4-Bromo-2-(hydroxymethyl)-2,3-dihydro-1H-inden-5-yl]oxy]ethyl]methanesulfonamide

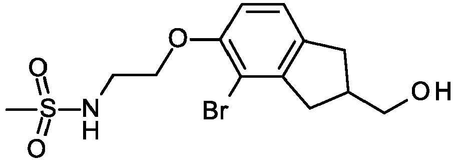

To a solution of methyl 5-(2-aminoethoxy)-4-bromo-indane-2-carboxylate hydrochloride (Intermediate 42, step 2, 432.7 mg, 1.23 mmol, 1 eq) in 1,2-dichloroethane (45 mL) was added triethylamine (0.34 mL, 2.47 mmol, 2 eq) and the solution was cooled to 0 °C. Methanesulfonyl chloride (0.14 mL, 1.85 mmol, 1.5 eq) was added dropwise and the reaction mixture was stirred at 0 °C for 0.5 h. The solution was washed with 10 mL brine and dried with Na₂SO₄. The solvent was removed to give the crude product which was purified by chromatography on silica gel (PE:EA=2:1) to give the title compound (550 mg, 1.4 mmol, 113.6% yield) as a yellow solid.

### Steps 2 to 4:

### N-[2-[(4-cyano-2-formyl-2,3-dihydro-1H-inden-5-yl)oxy]ethyl]methanesulfonamide

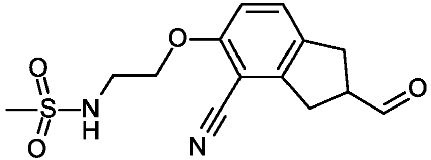

The title compound was prepared by analogy to 2-formyl-5-[(1-methylpyrrolidin-2-yl)methoxy]-2,3-dihydro-1*H*-indene-4-carbonitrile (Intermediate 38, steps 2, 3 and 6) using *N*-[2-[[4-bromo-2-(hydroxymethyl)-2,3-dihydro-1*H*-inden-5-yl]oxy]ethyl]methanesulfonamide in place of *tert-*butyl 2-[(4-bromo-2-ethoxycarbonyl-indan-5-yl)oxymethyl]pyrrolidine-1-carboxylate and was obtained as an off-white solid.

### Intermediate 45

### 6-[(5R)-2-Oxo-5-[(1R)-2-amino-1-hydroxyethyl]-1,3-oxazolidin-3-yl]-4H-1,4-benzoxazin-3-one; 2,2,2-trifluoroacetic acid

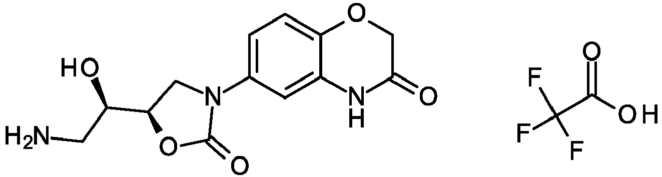

### Step 1:

### tert-Butyl N-tert-butoxycarbonyl-N-[(2R,3S)-4-chloro-2,3-dihydroxy-butyl]carbamate

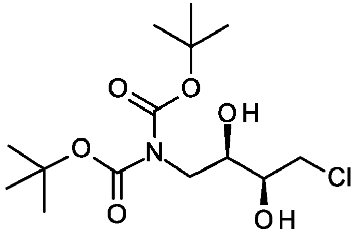

A solution of AD-mix-beta (6409.42 mg, 8.23 mmol, 1.8 eq) and methanesulfonamide (435.48 mg, 4.58 mmol, 1 eq) in *tert*-butanol (20 mL) and water (20 mL) was cooled to 0 °C and potassium osmate (VI) dihydrate (168.66 mg, 0.460 mmol, 0.100 eq) was added. Then *tert-butyl N*-*tert*-butoxycarbonyl-N-[(E)-4-chlorobut-2-enyl]carbamate (CAS# 121030-31-7, 1400.0 mg, 4.58 mmol, 1 eq) was added and the mixture was stirred at 0 °C for 6 h and then poured into 20 mL brine. The mixture was extracted with EtOAc (20 mL × 2) and the combined extracts were dried with Na₂SO₄. The solvent was removed to give the crude title compound (300 mg, 0.880 mmol, 88.3 % yield) as a yellow oil.

### Step 2:

### tert-Butyl (5R)-5-[(1S)-2-chloro-1-hydroxy-ethyl]-2-oxo-oxazolidine-3-carboxylate

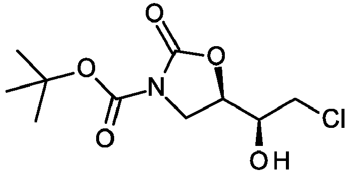

To a solution of *tert*-butyl *N*-*tert*-butoxycarbonyl-*N*-[(2*R*,3*S*)-4-chloro-2,3-dihydroxybutyl]carbamate (1500.0 mg, 4.41 mmol, 1 eq) in THF (50 mL) was added sodium hydride (17.66 mg, 0.440 mmol, 0.100 eq) at 0 °C and reaction mixture was stirred at 0 °C for 0.5 h. The mixture was poured into 10 mL ice-water and then extracted with EA (20 mL × 2). The combined organic layers were dried with Na₂SO₄ and the solvent was removed to give the crude product which was purified by chromatography on silica gel (PE:EA=10:1) to give the title compound (600 mg, 2.26 mmol, 51.2% yield) as a white solid.

### Step 3:

### tert-Butyl N-[(2R)-2-hydroxy-2-[(2R)-oxiran-2-yl]ethyl]carbamate

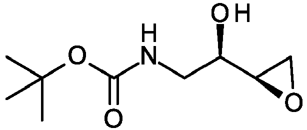

To a solution of tert-butyl (5*R*)-5-[(1*S*)-2-chloro-1-hydroxy-ethyl]-2-oxo-oxazolidine-3-carboxylate (400.0 mg, 1.51 mmol, 1 eq) in methanol (16 mL) was added K₂CO₃ (415.8 mg, 3.01 mmol, 2 eq) and the mixture was stirred at 25 °C for 16 h. The solvent was removed and the residue was taken up in 20 mL DCM, the solid was filtered off and the filtrate was concentrated to give the crude title compound (300 mg, 1.48 mmol, 98.1% yield) as a colourless oil.

### Step 4:

### tert-Butyl N-[(2R,3R)-2,3-dihydroxy-4-[(3-oxo-4H-1,4-benzoxazin-6-yl)amino]butyl]carbamate

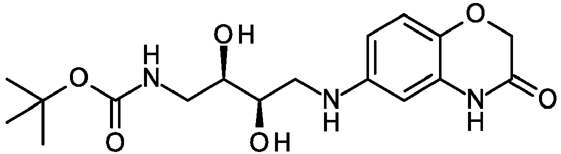

A solution of *tert-butyl N*-[(2*R*)-2-hydroxy-2-[(2*R*)-oxiran-2-yl]ethyl]carbamate (300.0 mg, 1.48 mmol, 1 eq) and 6-amino-4*H*-1,4-benzoxazin-3-one (242.31 mg, 1.48 mmol, 1 eq) in ethanol (10 mL) and water (1 mL) was stirred at 80 °C for 16 h under N₂ atmosphere. The solvent was removed to give the crude product which was purified by chromatography on silica gel (DCM:MeOH=20 :1) to give the title compound (130 mg, 0.350 mmol, 24 % yield) as a colourless oil. MS (ESP): m/z = 368.2 [M+H]⁺.

### Step 5:

### tert-Butyl N-[[(4R,5R)-2-oxo-5-[[(3-oxo-4H-1,4-benzoxazin-6-yl)amino]methyl]-1,3-dioxolan-4-yl] methyl] carbamate

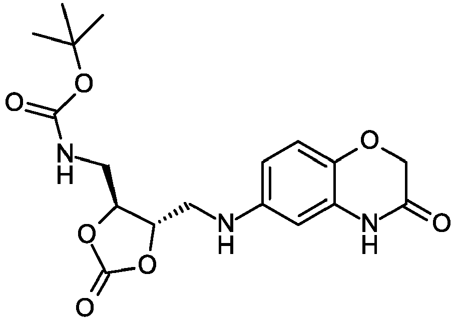

To a solution of *tert*-butyl *N*-[(2*R*,3*R*)-2,3-dihydroxy-4-[(3-oxo-4*H*-1,4-benzoxazin-6-yl)amino]butyl]carbamate (130.0 mg, 0.350 mmol, 1 eq) in THF (10 mL) was added *N,N'-*carbonyldiimidazole (68.85 mg, 0.420 mmol, 1.2 eq), and then the solution was stirred at 25 °C for 16 h. The solvent was removed and the crude product was purified by prep-TLC (DCM: MeOH=10:1) to give the title compound (60 mg, 0.150 mmol, 43.1 % yield) as a white solid. MS (ESP): m/z = 338.0 [M+H-tBu]⁺.

### Step 6:

### Methyl N-[[(4S,5S)-5-[(tert-butoxycarbonylamino)methyl]-2-oxo-1,3-dioxolan-4-yl]methyl]-N-(3-oxo-4H-1,4-benzoxazin-6-yl)carbamate

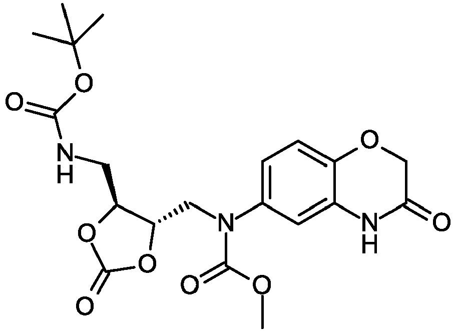

To a solution of *tert*-butyl *N*-[[(4*R*,5*R*)-2-oxo-5-[[(3-oxo-4*H*-1,4-benzoxazin-6-yl)amino]methyl]-1,3-dioxolan-4-yl]methyl]carbamate (60.0 mg, 0.150 mmol, 1 eq) and *N,N-*diisopropylethylamine (0.13 mL, 0.760 mmol, 5 eq) in DCM (5 mL) was added methyl chloroformate (36.03 mg, 0.380 mmol, 2.5 eq) and then the solution was stirred at 25 °C for 16 h. The solution was washed with 10 mL brine and dried with Na₂SO₄. The solvent was removed to give the crude title compound (60 mg, 0.130 mmol, 87.1% yield) as a yellow oil. MS (ESP): m/z = 474.1 [M+Na]⁺.

### Step 7:

### tert-Butyl N-[(2R)-2-hydroxy-2-[(5R)-2-oxo-3-(3-oxo-4H-1,4-benzoxazin-6-yl)oxazolidin-5-yl]ethyl]carbamate

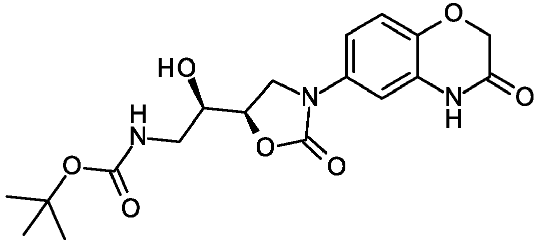

To a solution of methyl *N*-[[(4*S*,5*S*)-5-[(*tert*-butoxycarbonylamino)methyl]-2-oxo-1,3-dioxolan-4-yl]methyl]-*N*-(3-oxo-4*H*-1,4-benzoxazin-6-yl)carbamate (60.0 mg, 0.130 mmol, 1 eq) in methanol (5 mL) was added potassium carbonate (9.18 mg, 0.070 mmol, 0.500 eq) and the solution was stirred at 25 °C for 2 h. The solvent was removed to give the crude product which was purified by preparative TLC (DCM:MeOH=10:1) to give the title compound (50 mg, 0.130 mmol, 95.6% yield) as a white solid. MS (ESP): m/z = 416.1 [M+Na]⁺.

### Step 8:

### 6-[(5R)-2-Oxo-5-[(1R)-2-amino-1-hydroxyethyl]-1,3-oxazolidin-3-yl]-4H-1,4-benzoxazin-3-one; 2,2,2-trifluoroacetic acid

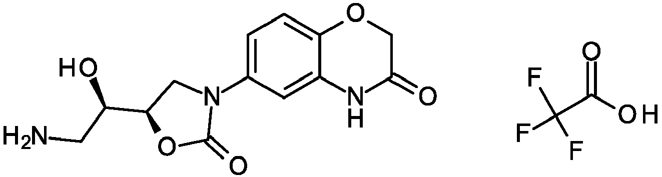

To a solution of *tert-*butyl *N*-[(2*R*)-2-hydroxy-2-[(5*R*)-2-oxo-3-(3-oxo-4*H*-1,4-benzoxazin-6-yl)oxazolidin-5-yl]ethyl]carbamate (50.0 mg, 0.130 mmol, 1 eq) in DCM (5 mL) was added trifluoroacetic acid (1.0 mL, 12.98 mmol, 102.12 eq) and the solution was stirred at 25 °C for 3 h. The solvent was removed to give the crude title compound (50 mg, 0.120 mmol, 96.6% yield) as a white solid. MS (ESP): m/z = 294.1 [M+H]⁺.

### Intermediate 46

### 6-[(5S)-5-[(1S)-2-amino-1-hydroxyethyl]-2-oxo-1,3-oxazolidin-3-yl]-4H-1,4-benzoxazin-3-one; 2,2,2-trifluoroacetic acid

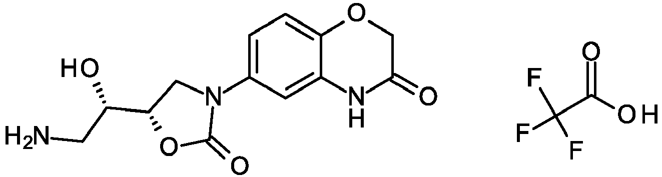

The title compound was prepared by analogy to 6-[(5*R*)-2-oxo-5-[(1*R*)-2-amino-1-hydroxyethyl]-1,3-oxazolidin-3-yl]-4*H*-1,4-benzoxazin-3-one; 2,2,2-trifluoroacetic acid (Intermediate 45) using AD-mix-alpha in place of AD-mix-beta and was obtained as a white solid. MS (ESP): m/z = 294.1 [M+H]⁺.

### Intermediate 47

### 2-Formyl-5-(oxetan-3-yloxy)-2,3-dihydro-1H-indene-4-carbonitrile

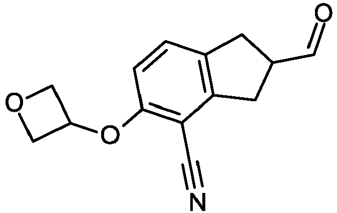

The title compound was prepared by analogy to 2-formyl-5-[(1-methylpyrrolidin-2-yl)methoxy]-2,3-dihydro-1*H*-indene-4-carbonitrile (Intermediate 38, steps 1 to 3 and 6) using oxetan-3-yl 4-methylbenzenesulfonate in place of *tert*-butyl 2-(*p*-tolylsulfonyloxymethyl)pyrrolidine-1-carboxylate and was obtained as a light yellow semisolid which was used directly without further purification. ¹H-NMR (400 MHz, CDCl₃) δ 9.70 (s, 1H), 7.23 (d, 1H), 6.25 (d, 1H), 5.20 (m, 1H), 4.91 (dd, 2H), 4.75 (dd, 2H), 3.2 (m, 5H).

### Intermediate 48

### 5-(2-Aminoethyl)-3-(3-oxo-4H-pyrido[3,2-b] [1,4]thiazin-6-yl)-1,3-oxazolidin-2-one;2,2,2-trifluoroacetic acid

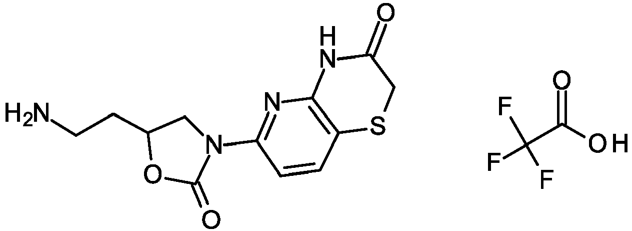

### Step 1:

### Methyl 2-[[2-(tert-butoxycarbonylamino)-6-chloro-3-pyridyl] sulfanyl] acetate

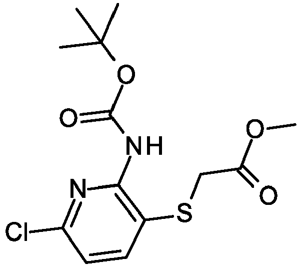

A solution of tetramethylethylenediamine (14.91 mL, 98.82 mmol, 2.26 eq) in THF (200 mL) was cooled to -20 °C and butyllithium solution (41.67 mL, 104.17 mmol, 2.38 eq) was added dropwise. After stirring for 30 min. the solution was cooled to -78 °C and *tert-*butyl *N-*(6-chloro-2-pyridyl)carbamate (10.0 g, 43.73 mmol, 1 eq) in THF(10 mL) was added dropwise. The solution was further stirred for 1 h and sulfur (7011.11 mg, 218.65 mmol, 5 eq) was added. The solution was stirred for another 30 min. and methyl bromoacetate (8.28 mL, 87.46 mmol, 2 eq) was added. The mixture was further stirred for 2 h and then quenched by the addition of EtOAc and water. The organic phase separated and sequentially washed with 1N HCl, conc. NH₄Cl solution and brine. The combined organic phase was dried over MgSO₄ and the solvents were evaporated. The residue was purified by flash column chromatography (20 % ethyl acetate / Petroleum ether) to afford the title compound (6.1 g, 18.33 mmol, 38.1 % yield) as a yellow solid. MS (ESP): m/z = 277.0 [M+H-tBu]⁺.

### Step 2:

### Methyl 2-[(2-amino-6-chloro-3-pyridyl) sulfanyl] acetate

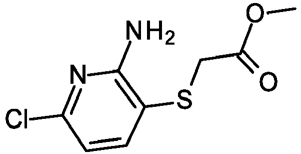

A mixture of methyl 2-[[2-(*tert*-butoxycarbonylamino)-6-chloro-3-pyridyl]sulfanyl]acetate (6.0 g, 18.03 mmol, 1 eq) in DCM (40 mL) was stirred at 25 °C for 16 h in the presence of trifluoroacetic acid (40.0 mL, 519.19 mmol, 28.8 eq). The mixture was concentrated in vacuo to afford the crude title compound (9 g, 38.68 mmol) as a yellow oil. MS (ESP): m/z = 233.1 [M+H]⁺.

### Step 3:

### 6-Chloro-4H-pyrido[3,2-b][1,4]thiazin-3-one

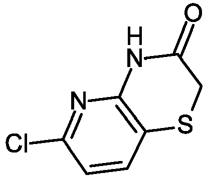

A mixture of methyl 2-[(2-amino-6-chloro-3-pyridyl)sulfanyl]acetate (9000.0 mg, 19.34 mmol, 1 eq) in ethanol (112.5 mL) was stirred for 48 h at 80 °C. The mixture was concentrated in vacuo. The residue was dissolved in ethyl acetate and the solution was washed with saturated Na₂CO₃ and concentrated in vacuo. The resulting residue was purified by flash chromatography (PE: EA:DCM=2:1:1) to afford the title compound (1.5 g, 7.48 mmol, 33.6 % yield) as a white solid. MS (ESP): m/z = 201.5 [M+H]⁺.

### Step 4:

### tert-Butyl N-[2-[2-oxo-3-(3-oxo-4H-pyrido[3,2-b] [1,4]thiazin-6-yl)oxazolidin-5-yl] ethyl] carbamate

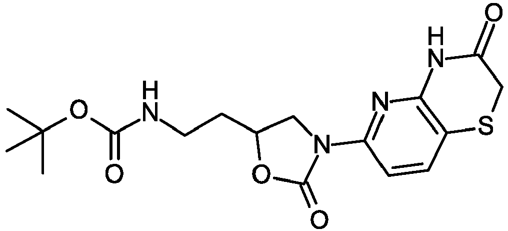

To a solution of *tert*-butyl *N*-[2-(2-oxooxazolidin-5-yl)ethyl]carbamate (Intermediate 26, 114.76 mg, 0.500 mmol, 1 eq) and 6-chloro-4*H*-pyrido[3,2-b][1,4]thiazin-3-one (100.0 mg, 0.500 mmol, 1 eq) in 1,4-dioxane (10 mL) were added 9,9-dimethyl-4,5-bis(diphenylphosphino)xanthene (115.35 mg, 0.200 mmol, 0.400 eq), tris(dibenzylideneacetone)dipalladium (0) (91.28 mg, 0.100 mmol, 0.200 eq) and potassium carbonate (206.64 mg, 1.5 mmol, 3 eq). The mixture was stirred at 100 °C for 2 h. The mixture was filtered and concentrated in vacuo to give the residue which was purified by flash chromatography (60 % ethyl acetate / petroleum ether) to give the title compound (90 mg, 0.230 mmol, 44.4% yield) as a yellow solid. MS (ESP): m/z = 339.4 [M+H-tBu]⁺.

### Step 5:

### 5-(2-Aminoethyl)-3-(3-oxo-4H-pyrido[3,2-b][1,4]thiazin-6-yl)-1,3-oxazolidin-2-one; 2,2,2-trifluoroacetic acid

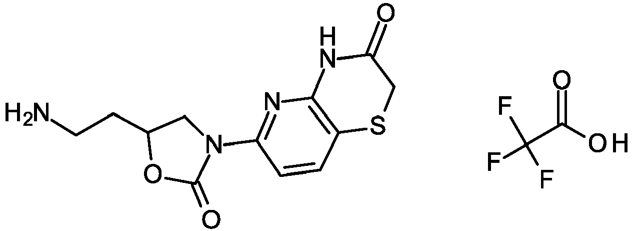

To a solution of *tert*-butyl *N*-[2-[2-oxo-3-(3-oxo-4*H*-pyrido[3,2-b][1,4]thiazin-6-yl)oxazolidin-5-yl]ethyl]carbamate (190.0 mg, 0.480 mmol, 1 eq) in DCM (4 mL) was added trifluoroacetic acid (4.0 mL, 51.92 mmol, 107.79 eq). The mixture was stirred at 25 °C for 2 h. The mixture was concentrated in vacuo to afford the title compound (300 mg, 0.730 mmol, 99.1% yield) as a yellow solid.

### Intermediate 49

### 2-[(1-Chloro-6-fonnyl-6,7-dihydro-5H-cyclopenta[c]pyridin-3-yl)oxy]-N-methylacetamide

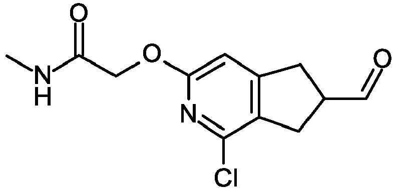

### Step 1:

### Ethyl 1-chloro-3-oxo-2,5,6,7-tetrahydrocyclopenta[c]pyridine-6-carboxylate

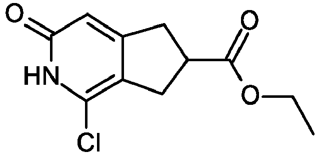

A mixture of dimethyl 1-chloro-3-methoxy-5,7-dihydrocyclopenta[c]pyridine-6,6-dicarboxylate (Intermediate 37, step 5, 400.0 mg, 1.33 mmol, 1 eq) in HCl/H₂O (8.0 mL, 96 mmol, 72 eq) was stirred at 100 °C for 16 h. The mixture was concentrated under vacuum to give a residue which was re-dissolved in ethanol (8 mL), sulfuric acid (0.5 mL, 98.3%) was added and the mixture was stirred at 80 °C for 2 h. After 2 h the mixture was concentrated, saturated NaHCO₃ solution (15 mL) was added and the mixture was extracted with EtOAc (2 × 15 mL). The combined organic layers were concentrated under vacuum to give the title compound (300 mg, 1.24 mmol, 78.0% yield) as a white solid. MS (ESP): m/z = 242.1 [M+H]⁺.

### Steps 2 to 4:

### 2-[(1-Chloro-6-formyl-6,7-dihydro-5H-cyclopenta[c]pyridin-3-yl)oxy]-N-methylacetamide

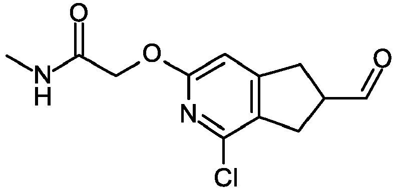

The title compound was prepared by analogy to 2-formyl-5-[(1-methylpyrrolidin-2-yl)methoxy]-2,3-dihydro-1*H*-indene-4-carbonitrile (Intermediate 38, steps 1, 2 and 6) using 2-chloro-*N-*methylacetamide in place of *tert*-butyl 2-(*p*-tolylsulfonyloxymethyl)pyrrolidine-1-carboxylate and ethyl 1-chloro-3-oxo-2,5,6,7-tetrahydrocyclopenta[c]pyridine-6-carboxylate in place of ethyl 4-bromo-5-hydroxy-indane-2-carboxylate and was obtained as a light yellow semi-solid.

### Intermediate 50

### 2-[(7-Fluoro-2-formyl-2,3-dihydro-1H-inden-5-yl)oxy]-N-methylacetamide

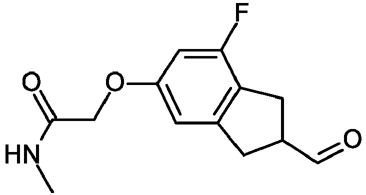

### Step 1:

### Ethyl 4-fluoro-1-oxo-indane-2-carboxylate

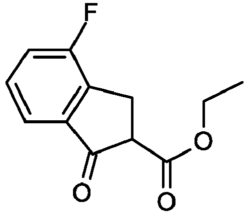

To a dried three-necked flask equipped with a condenser and a magnetic stirrer were added sodium hydride, 60% in oil (3995.6 mg, 99.9 mmol, 3 eq), diethylcarbonate (8.89 g, 75.26 mmol, 2.26 eq) and toluene (55 mL). The mixture was heated to reflux (about 110°C). A solution of 4-fluoroindan-1-one (5.0 g, 33.3 mmol, 1 eq) in toluene (55 mL) was added drop-wise over 1 h. After addition, the mixture was keep at the same temperature for 2 h. The reaction was cooled to room temperature and poured into saturated aq. NH₄Cl (300 mL) and treated with AcOH (about 15 g). The obtained mixture was extracted with toluene (2 × 100 mL). The combined organic layers were concentrated under reduced pressure and the obtained crude product was purified by flash chromatography (22% PE/EA) to give the title compound (5.8 g, 26.1 mmol, 78.4 % yield) as a red oil. MS (ESP): m/z = 223.1 [M+H]⁺.

### Step 2:

### Ethyl 4-fluoroindane-2-carboxylate

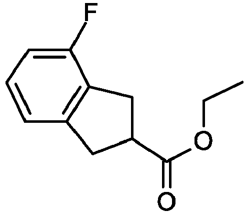

To a mixture of ethyl 4-fluoro-1-oxo-indane-2-carboxylate (5.8 g, 26.1 mmol, 1 eq) in trifluoroacetic acid (100.0 mL) was added triethylsilane (20.85 mL, 130.51 mmol, 5 eq) at 0 °C. Then the mixture was warmed to 25 °C and stirred for 20 h. The solvent was removed in vacuo and the residue was diluted with EtOAc (300 mL). The solution was washed with water (200 mL), saturated aq. NaHCO₃ (200 mL) and brine (200 mL) and it was dried with Na₂SO₄. After filtration, the filtrate was concentrated under reduced pressure to get the crude product (15 g). It was purified by flash chromatography (8% PE:EA) to give the title compound (4.7 g, 22.57 mmol, 86.5 % yield) as light yellow oil. MS (ESP): m/z = 209.0 [M+H]⁺.

### Step 3:

### Ethyl 4-fluoro-6-hydroxy-indane-2-carboxylate; ethyl 4-fluoro-5-hydroxy-indane-2-carboxylate; ethyl 4-fluoro-7-hydroxy-indane-2-carboxylate

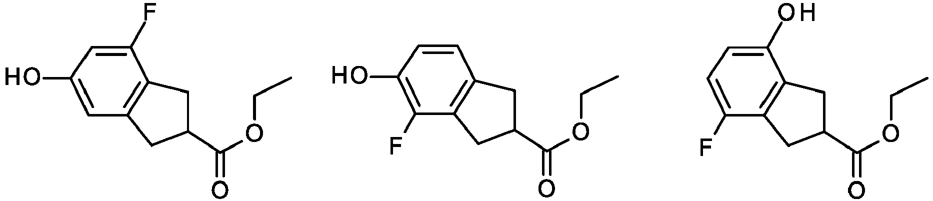

To a stirred solution of 4,4,5,5-tetramethyl-1,3,2-dioxaborolane (11984.59 mg, 93.65 mmol, 1.5 eq) were added methoxy(cyclooctadiene)iridium(I) dimer (1655.32 mg, 2.5 mmol, 0.040 eq) and 4,4'-di-*tert*-butyl-2,2-bipyridinyl (669.73 mg, 2.5 mmol, 0.040 eq) under an atmosphere of nitrogen and the solution was stirred for further 5 min. Then ethyl 4-fluoroindane-2-carboxylate (13 g, 62.45 mmol, 1 eq) was added and the solution was stirred at 150 °C for 4 h. The reaction mixture was cooled to room temperature, then a solution of sodium perborate monohydrate (18691.8 mg, 187.29 mmol, 3 eq) in 20 mL water was added and the reaction was stirred for further 30 min. The solution was poured into 50 mL brine and the mixture was extracted with EtOAc (20 mL × 2). The combined organic extracts were dried with Na₂SO₄ and the solvent was removed to give the crude product which was purified by chromatography on silica gel (PE:EA=5:1) to give the title compounds in a ratio of 2:1:1 (7000 mg, 31.22 mmol, 25 % yield).

### Step 4:

### Ethyl 6-benzyloxy-4-fluoro-indane-2-carboxylate; ethyl 5-benzyloxy-4-fluoro-indane-2-carboxylate; ethyl 7-benzyloxy-4-fluoro-indane-2-carboxylate

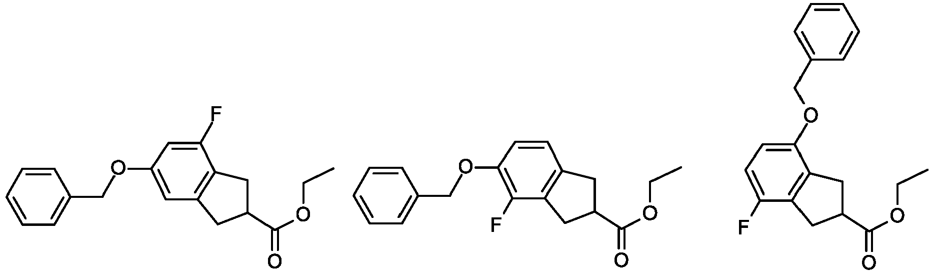

To a mixture of ethyl 4-fluoro-6-hydroxy-indane-2-carboxylate, ethyl 4-fluoro-5-hydroxy-indane-2-carboxylate and ethyl 4-fluoro-7-hydroxy-indane-2-carboxylate (6000.0 mg, 26.76 mmol, 1 eq) and potassium carbonate (7390.6 mg, 53.52 mmol, 2 eq) in MeCN (133.3 mL) was added benzyl bromide (3.82 mL, 32.11 mmol, 1.2 eq) and the solution was stirred at 80°C for 16 h. The solid was filtered off and the filtrate was concentrated to give the crude product which was purified by chromatography on silica gel (PE:EA=10:1) to give the title compound mixture (6500 mg, 20.68 mmol, 77.4 % yield) as a colorless oil.

### Step 5:

### (6-Benzyloxy-4-fluoro-indan-2-yl)methanol

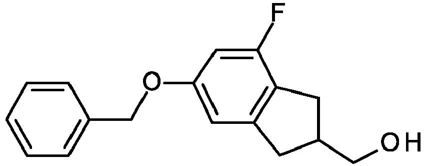

To a solution of ethyl 6-benzyloxy-4-fluoro-indane-2-carboxylate, ethyl 5-benzyloxy-4-fluoro-indane-2-carboxylate and ethyl 7-benzyloxy-4-fluoro-indane-2-carboxylate (1500.0 mg, 4.77 mmol, 1 eq) in THF (30 mL) was added lithium aluminium hydride in THF (7.16 mL, 7.16 mmol, 1.5 eq) at 0 °C and the solution was stirred at 0 °C for 0.5 h. A few drops of water were added and then EtOAc was added slowly until the gas evolution stopped. Then 5 g Na₂SO₄ were added and the mixture was stirred at room temperature for 30 min. The solid was filtered off, the filter cake was washed with 20 mL EtOAc and the filtrate was evaporated to give the crude product which was purified by chromatography on silica gel (PE:EA=3:1) to give the title compound (600 mg, 2.2 mmol, 46.2 % yield) as a white solid. ¹H-NMR (400 MHz, d6-DMSO) δ 7.39 (m, 5H), 6.74 (s, 1H), 6.63 (d, 1H), 5.07 (s, 2H), 4.69 (t, 1H), 2.88 (m, 2H), 2.61 (m, 1H), 2.58 (m, 2H).

### Step 6:

### 7-Fluoro-2-(hydroxymethyl)indan-5-ol

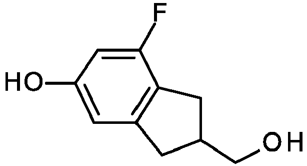

To a solution of (6-benzyloxy-4-fluoro-indan-2-yl)methanol (600.0 mg, 2.2 mmol, 1 eq) in methanol (50 mL) was added Pd/C (100 mg) and the mixture was stirred at 25 °C under an atmosphere of hydrogen for 16 h. The catalyst was filtered off and the filtrate was concentrated to give the crude title compound (400 mg, 2.2 mmol, 99.6% yield) as a colourless oil.

### Step 7:

### 2-[7-Fluoro-2-(hydroxymethyl)indan-5-yl]oxy-N-methyl-acetamide

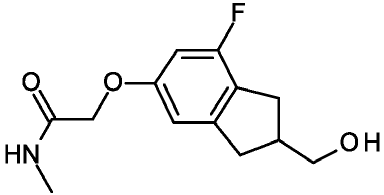

A mixture of 2-chloro-*N*-methylacetamide (177.08 mg, 1.65 mmol, 2 eq), potassium carbonate (227.57 mg, 1.65 mmol, 2 eq) and 7-fluoro-2-(hydroxymethyl)indan-5-ol (150.0 mg, 0.820 mmol, 1 eq) in MeCN (15 mL) was stirred at 80 °C for 16 h. The mixture was filtered and the filtrate concentrated to give a residue which was purified by preparative TLC (PE:EA = 1:3) to give the title compound (150 mg, 0.590 mmol, 66.2 % yield) as an off-white solid. MS (ESP): m/z = 254.6 [M+H]⁺.

### Step 8:

### 2-[(7-Fluoro-2-formyl-2,3-dihydro-1H-inden-5-yl)oxy]-N-methylacetamide

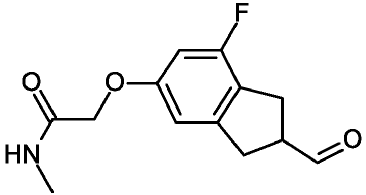

To a mixture of 2-[7-fluoro-2-(hydroxymethyl)indan-5-yl]oxy-*N*-methyl-acetamide (90.0 mg, 0.360 mmol, 1 eq) in DCM (4 mL) was added Dess-Martin Periodinane (226.08 mg, 0.530 mmol, 1.5 eq) and then the mixture was stirred at 25 °C for 2 h. After filtration the filtrate was concentrated to give the title compound (65 mg, 0.260 mmol, 72.8% yield) as a light yellow semisolid, which was used directly without further purification. ¹H-NMR (400 MHz, CDCl₃) δ 9.90 (s, 1H), 6.52 (s, 1H), 6.40 (d, 1H), 4.39 (s, 2H), 3.2 (m, 5H), 2.84 (s, 3H).

### Intermediate 51

### 4-Fluoro-1-methoxy-6,7-dihydro-5H-cyclopenta[c]pyridine-6-carbaldehyde

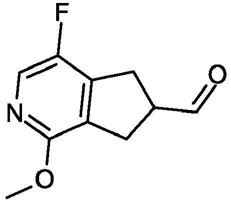

### Step 1:

### 4-Fluoro-1-methoxy-6,7-dihydro-5H-cyclopenta[c]pyridine-6-carboxylic acid

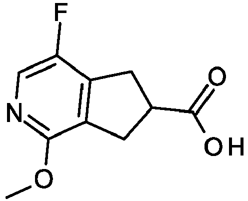

To a mixture of ethyl 1-bromo-4-fluoro-6,7-dihydro-5*H*-cyclopenta[c]pyridine-6-carboxylate (Intermediate 27, step 7, 1000.0 mg, 3.47 mmol, 1 eq), palladium (II) acetate (77.93 mg, 0.350 mmol, 0.100 eq) and 5-(di-*tert*-butylphosphino)-1,3,5-triphenyl-1H-[1,4]bipyrazole (351.7 mg, 0.690 mmol, 0.200 eq) in methanol (10 mL)/toluene (10 mL) was added cesium carbonate (1696.33 mg, 5.21 mmol, 1.5 eq) at 20 °C. The reaction mixture was stirred at 80 °C for 16 h under N₂. The reaction mixture was filtered and concentrated to provide the crude product. The crude product was purified by column chromatography (PE: EtOAc=10:1-1:1(PE: EtOAc=1:1) to give the title compound (420 mg, 1.99 mmol, 57.3 % yield) as a yellow oil. MS (ESP): m/z = 212.1 [M+H]⁺.

### Steps 2 to 4:

### 4-Fluoro-1-methoxy-6,7-dihydro-5H-cyclopenta[c]pyridine-6-carbaldehyde

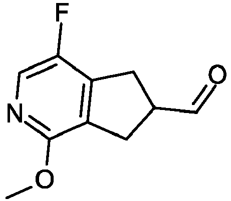

The title compound was prepared by analogy with 4-fluoro-1-methyl-6,7-dihydro-5*H-*cyclopenta[c]pyridine-6-carbaldehyde (Intermediate 27, steps 7, 9 and 10) using 4-fluoro-1-methoxy-6,7-dihydro-5*H*-cyclopenta[c]pyridine-6-carboxylic acid in place of 1-bromo-4-fluoro-6,7-dihydro-5*H*-cyclopenta[c]pyridine-6-carboxylic acid and was obtained as a yellow oil. The crude product was used in the next step directly without further purification. MS (ESP): m/z = 214.2 [M+H]⁺.

### Intermediate 52

### 2-[(7-Fluoro-2-formyl-2,3-dihydro-1H-inden-5-yl)oxy] acetonitrile

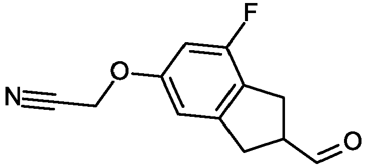

### Step 1:

### tert-Butyl-[(4-fluoro-6-phenylmethoxy-2,3-dihydro-1H-inden-2-yl)methoxy]-dimethylsilane

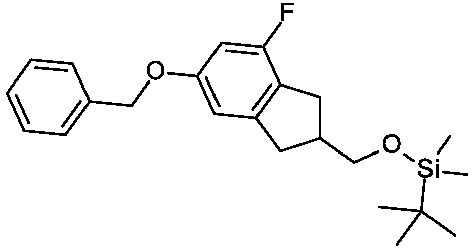

To a solution of (6-benzyloxy-4-fluoro-indan-2-yl)methanol (Intermediate 50, step 5, 400.0 mg, 1.47 mmol, 1 eq) and imidazole (200.0 mg, 2.94 mmol, 2 eq) in DMF (4 mL) was added *tert*butyldimethylsilyl chloride (0.33 g, 2.2 mmol, 1.5 eq) at 0°C and the solution was stirred at 25 °C for 16 h . The solution was poured into 50 mL water and the mixture was extracted with EtOAc (20 mL × 2). The combined extracts were dried with Na₂SO₄ and the solvent was removed to give the crude product which was purified by chromatography on silica gel (PE:EA=10:1) to give the title compound (550 mg, 1.42 mmol, 96.9% yield) as a colourless oil.

### Step 2:

### 2-[[tert-Butyl(dimethyl)silyl]oxymethyl]-7-fluoro-2,3-dihydro-1H-inden-5-ol

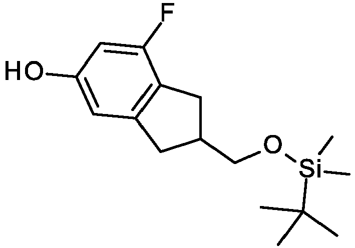

To a solution of *tert*-butyl-[(4-fluoro-6-phenylmethoxy-2,3-dihydro-1*H*-inden-2-yl)methoxy]-dimethylsilane (550.0 mg, 1.42 mmol, 1 eq) in methanol (30 mL) was added Pd/C (100 mg), and then the mixture was stirred at 25 °C under an atmosphere of hydrogen for 16 h. The catalyst was filtered off and the filtrate was concentrated to give the crude product which was purified by chromatography on silica gel (PE:EA=20:1) to give the title compound (120 mg, 0.400 mmol, 27.0% yield) as a white solid. ¹H-NMR (400 MHz, d6-DMSO) δ 9.48 (s, 1H), 6.42 (s, 1H), 6.25 (d, 1H), 3.50 (d, 2H), 2.83 (m, 2H), 2.5 (m, 3H), 0.83 (s, 9H), 0.01 (s, 6H).

### Step 3:

### 2-[[2-[[tert-Butyl(dimethyl)silyl]oxymethyl]-7-fluoro-2,3-dihydro-1H-inden-5-yl] oxy] acetonitrile

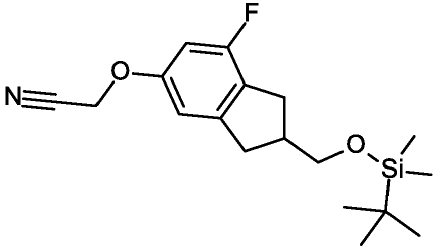

To a mixture of potassium carbonate (46.58 mg, 0.340 mmol, 2 eq) and bromoacetonitrile (30.35 mg, 0.250 mmol, 1.5 eq) in MeCN (5 mL) was added 2-[[*tert-*butyl(dimethyl)silyl]oxymethyl]-7-fluoro-2,3-dihydro-1*H*-inden-5-ol (50.0 mg, 0.170 mmol, 1 eq) and the solution was stirred at 80°C for 16 h. The solid was filtered off and the filtrate was concentrated to give the crude product which was purified by chromatography on silica gel (PE:EA=10:1) to give the title compound (55 mg, 0.160 mmol, 97.2% yield) as a colourless oil.

### Step 4:

### 2-[[7-Fluoro-2-(hydroxymethyl)-2,3-dihydro-1H-inden-5-yl] oxy] acetonitrile

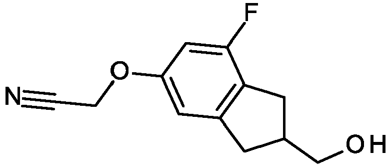

To a solution of 2-[[2-[[*tert*-butyl(dimethyl)silyl]oxymethyl]-7-fluoro-2,3-dihydro-1*H*-inden-5-yl]oxy]acetonitrile (55.0 mg, 0.160 mmol, 1 eq) in THF (10 mL) was added TBAF in THF (0.33 mL, 0.330 mmol, 2 eq) and the solution was stirred at 25 °C for 16 h. The solvent was removed to give the crude product which was purified by preparative TLC (PE:EA=1:1) to give the title compound (35 mg, 0.160 mmol, 96.5% yield) as a colourless oil.

### Step 5:

### 2-[(7-Fluoro-2-formyl-2,3-dihydro-1H-inden-5-yl)oxy]acetonitrile

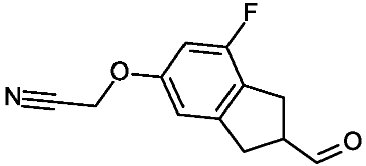

To a solution of Dess-Martin periodinane (100.18 mg, 0.240 mmol, 1.5 eq) in DCM (5 mL) was added 2-[[7-fluoro-2-(hydroxymethyl)-2,3-dihydro-1*H*-inden-5-yl]oxy]acetonitrile (35.0 mg, 0.160 mmol, 1 eq) and the solution was stirred at 25 °C for 3 h. The reaction was quenched with 10 mL10% aq. NaHCO₃ and the mixture was extracted with DCM (10 mL ×2). The combined extracts were dried with Na₂SO₄ and the solvent was removed to give the crude title compound (30 mg, 0.140 mmol, 86.5% yield) as a colourless oil.

### Intermediate 53

### 6-[5-(Azetidin-3-yl)-2-oxo-1,3-oxazolidin-3-yl]-4H-pyrazino[2,3-b][1,4]oxazin-3-one; 2,2,2-trifluoroacetic acid

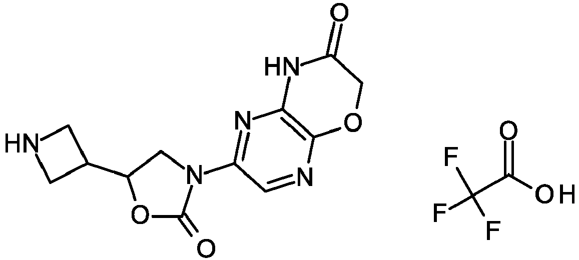

The title compound was prepared by analogy to 6-[5-(*trans*-3-Aminocyclobutyl)-2-oxo-1,3-oxazolidin-3-yl]-4*H*-pyrido[3,2-b][1,4]oxazin-3-one; 2,2,2-trifluoroacetic acid (Intermediate 7, steps 4 and 5) using *tert*-butyl 3-(2-oxo-1,3-oxazolidin-5-yl)azetidine-1-carboxylate in place of *tert-butyl* N-[*trans*-3-(2-oxo-1,3-oxazolidin-5-yl)cyclobutyl]carbamate and 6-bromo-4H-pyrazino[2,3-b][1,4]oxazin-3-one (Intermediate 25, step 1) in place of 6-bromo-4-(2-trimethylsilylethoxymethyl)pyrido[3,2-b][1,4]oxazin-3-one and was obtained as a brown oil. The crude product was used for next step directly. MS (ESP): m/z = 292.0 [M+H]⁺.

The *tert*-butyl 3-(2-oxo-1,3-oxazolidin-5-yl)azetidine-1-carboxylate used as a starting material was prepared by analogy with *tert-*butyl *N*-[*trans*-3-(2-oxo-1,3-oxazolidin-5-yl)cyclobutyl]carbamate (Intermediate 7, steps 1 to 3) using *tert*-butyl 3-formylazetidine-1-carboxylate (CAS# 177947-96-5) in place of *tert-butyl* N-(*trans*-3-formylcyclobutyl)carbamate and was obtained as a white solid. ¹H-NMR (400 MHz, CDCl₃) δ 6.33 (br s, 1H), 4.79 (dd, 1H), 4.02 (m, 2H), 3.82 (m, 1H), 3.71 (m, 2H), 3.22 (dd, 1H), 2.81 (m, 1H), 1.43 (s, 9H).

### Intermediate 54

### 2-[(7-Fluoro-2-formyl-2,3-dihydro-1H-inden-5-yl)oxy]-N,N-dimethylacetamide

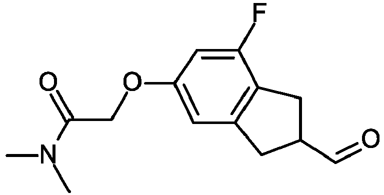

The title compound was prepared by analogy with 2-[(7-fluoro-2-formyl-2,3-dihydro-1*H*-inden-5-yl)oxy]acetonitrile (Intermediate 52, steps 3 to 5) using 2-chloro-*N,N*-dimethylacetamide in place of bromoacetonitrile and was obtained as a light yellow oil which was used directly without further purification.

### Intermediate 55

### 1-Methyl-6,7-dihydro-5H-cyclopenta[c]pyridine-6-carbaldehyde

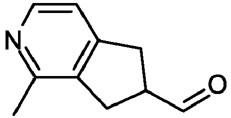

### Step 1:

### (1-Methyl-6,7-dihydro-5H-cyclopenta[c]pyridin-6-yl)methanol

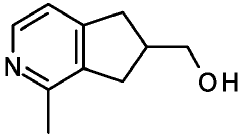

To (1-chloro-6,7-dihydro-5*H*-cyclopenta[c]pyridin-6-yl)methanol (Intermediate 19, step 5) (500 mg, 2.7 mmol, 1 eq) in dioxane (13.6 ml) under argon was added Pd(dppf) dichloromethane adduct (222 mg, 272 µmol, 0.1 eq) followed by the dropwise addition of 1.2 M Me₂Zn in toluene (6.8 ml, 8.2 mmol, 3 eq) and the mixture was heated at 80 °C for 2 h. The mixture was carefully poured onto water and the mixture was extracted twice with EtOAc. The combined organic layers were dried over Na₂SO₄, evaporated and the residue was purified on SiO₂ (0 to 20 % EtOAc in heptane) to give the title compound (215 mg, 1.3 mmol, 48 % yield) as a light brown solid. MS (ESP): m/z = 164.0 [M+H]⁺.

### Step 2:

### 1-Methyl-6,7-dihydro-5H-cyclopenta[c]pyridine-6-carbaldehyde

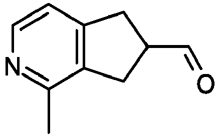

(1-Methyl-6,7-dihydro-5*H*-cyclopenta[c]pyridin-6-yl)methanol (200 mg, 1.2 mmol, 1 eq) was dissolved in dichloromethane (12 ml) at ambient temperature. Iodobenzene diacetate (407 mg, 1.3 mmol, Eq: 1.0) was added and after dissolution the reaction mixture was cooled to 0 °C. TEMPO (9.6 mg, 61 µmol, 0.05 eq) was added and the ice bath was removed. After 16 hours TEMPO (9.6 mg, 61 µmol, 0.05 eq) was added again and the reaction was stirred for 3 more hours. To the reaction mixture was added Et₃N (248 mg, 342 µl, 2.45 mmol) and the mixture was evaporated. The crude residue was purified on SiO₂ eluting with dichloromethane to 5% MeOH to give the title compound (100 mg, 620 µmol, 51 % yield) as a light brown liquid. ¹H-NMR (CDCl₃, 300 MHz): δ ppm 2.43 - 2.56 (m, 3 H) 3.11 - 3.41 (m, 5 H) 7.06 (d, J=5.44 Hz, 1 H) 8.32 (d, J=5.04 Hz, 1 H) 9.79 (d, J=1.21 Hz, 1 H)

### Intermediate 56

### 4-Methyl-6,7-dihydro-5H-cyclopenta[b]pyridine-6-carbaldehyde

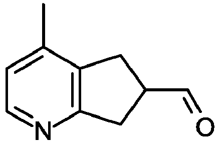

The title compound was prepared by analogy to 1-chloro-6,7-dihydro-5*H*-cyclopcnta[c]pyridinc-6-carbaldehyde (Intermediate 19) using [2-(hydroxymethyl)-4-methyl-3-pyridyl]methanol (Intermediate 15, step 2) in place of [2-chloro-3-(hydroxymethyl)-4-pyridyl]methanol and was obtained as a brown oil. MS (ESP): m/z = 180.1 [M+H+H₂O]⁺.

### Intermediate 57

### 6-Fonnyl-6,7-dihydro-5H-cyclopenta[c]pyridine-1-carbonitrile

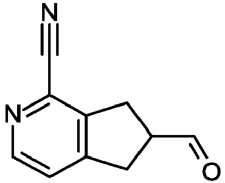

(1-Chloro-6,7-dihydro-5*H*-cyclopenta[c]pyridin-6-yl)methanol (Intermediate 19, step 5) (500 mg, 2.73 mmol, 1 eq) in *N*,*N*-dimethylformamide (50 ml) was combined with zinc cyanide (640 mg, 5.45 mmol, 2 eq) and heated to 160 °C. The mixture was degased with argon for 5 minutes and then tetrakis(triphenylphosphine)palladium(0) (157 mg, 136 µmol, 0.05 eq) was added. The mixture was stirred at 160 °C for 60 min. Zinc cyanide (640 mg, 5.45 mmol, 2 eq) and tetrakis(triphenylphosphine)palladium(0) (157 mg, 136 µmol, 0.05 eq) were added and the resulting mixture was stirred at 160 °C for 4 hours. The mixture was poured onto water, the solids were filtered off and the filtrate was extracted with ethyl acetate. The organic layer was dried over Na₂SO₄ and evaporated. The residue was purified on SiO₂ eluting with 0-50% ethyl acetate in heptane to give a mixture of 6-(hydroxymethyl)-6,7-dihydro-5*H-*cyclopenta[c]pyridine-1-carbonitrile (500mg, 1.23 mmol, 45 % yield, 43 % pure according to ¹H-NMR) and triphenylphosphine oxide as a light orange oil. Without further purification this mixture was dissolved in dichloromethane (29 ml) at ambient temperature. Iodobenzene diacetate (952 mg, 2.96 mmol, 2.4 eq) was added and after dissolution the reaction mixture was cooled to 0 °C. TEMPO (22 mg, 144 µmol, 0.1 eq) was added and the ice bath was removed. After 16 hours TEMPO (22 mg, 144 µmol, 0.1 eq) was added again and the reaction mixture was stirred for 3 more hours. The reaction mixture was washed with sat. NaHCO₃ and the organic phase was dried over Na₂SO₄ and evaporated. The residue was purified on SiO₂ eluting with 0% to 30% ethyl acetate in heptane to give the title compound (90 mg, 523 µmol, 42 % yield) as a light brown liquid. ¹H-NMR (CDC13, 300 MHz) δ ppm 9.81 (s, 1 H) 8.51 (d, J=5.04 Hz, 1 H) 7.40 (d, J=5.04 Hz, 1 H) 3.38 - 3.63 (m, 4 H) 3.16 - 3.33 (m, 1 H).

### Intermediate 58

### 1-Chloro-6,7-dihydro-5H-cyclopenta[c]pyridin-6-amine

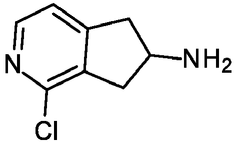

### Step 1:

### (4-Methoxyphenyl)methyl N-(1-chloro-6,7-dihydro-5H-cyclopcnta[c]pyridin-6-yl)carbamatc

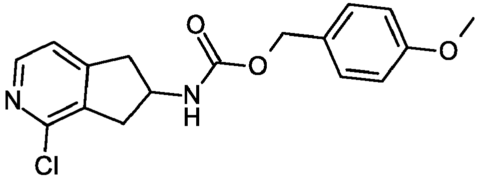

To a solution of 1-chloro-6,7-dihydro-5*H*-cyclopenta[c]pyridine-6-carboxylic acid (Intermediate 19, step 3, 1 g, 5.06 mmol, 1 eq) in toluene (30 ml) was added triethylamine (870 mg, 1.2 ml, 8.6 mmol, 1.7 eq) and this mixture was stirred for 10 minutes. Then diphenylphosphoryl azide (1.46 g, 1.15 ml, 5.31 mmol, 1.05 eq) was added and the mixture was stirred for 2 hours at 100 °C. (4-Methoxyphenyl)methanol (1.19 g, 1.07 ml, 8.6 mmol, 1.7 eq) was added and the mixture stirred for 1 day at 100 °C. Water was added and the mixture was extracted with CH₂Cl₂ (x 2). The combined extracts were dried over Na₂SO₄ and evaporated. The crude product was purified by silica cartridge chromatography (ISCO: 70 g from heptane to 50% EtOAc/heptane) to give the title compound (1.02 g, 3.07 mmol, 60.6 % yield) as a light yellow solid. MS (ESP): m/z = 333.1 [M+H]⁺.

### Step 2:

### 1-Chloro-6,7-dihydro-5H-cyclopenta[c]pyridin-6-amine

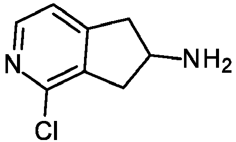

(4-Methoxyphenyl)methyl *N*-(1-chloro-6,7-dihydro-5*H*-cyclopenta[c]pyridin-6-y1)carbamate (1.02 g, 3.07 mmol, 1 eq) was dissolved in dichloromethane (27.6 mL), TFA was added (6.29 g, 4.25 ml, 55.2 mmol, 18 eq) and the mixture was stirred for 2 hours. The mixture was evaporated, water was added and the mixture was washed twice with EtOAc. The water layer was basified with 2M NaOH and extracted 3 times with CH₂Cl₂. The combined organic layers were dried with Na₂SO₄, filtered and evaporated to give the title compound (408 mg, 2.42 mmol, 78.9 % yield) as a yellow oil. MS (ESP): m/z = 169.0 [M+H]⁺.

### Intermediate 59

### 1,3,4-Trimethyl-6,7-dihydro-5H-cyclopenta[c]pyridine-6-carbaldehyde

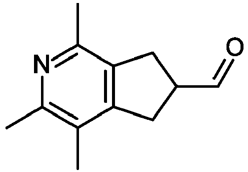

### Step 1:

### Dimethyl 2,2-bis(but-2-ynyl)propanedioate

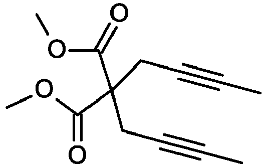

Dimethyl malonate (3.96 g, 3.43 ml, 30 mmol) was added dropwise to a suspension of sodium hydride (3.27 g, 75 mmol, 55% in mineral oil) in THF (75 ml) at 0°C and the mixture was stirred for 0.5 h at 0°C. 1-Bromobut-2-yne (9.97 g, 6.57 ml, 75 mmol) was added dropwise and the suspension was stirred at RT for 2.5 h. Sat. NH₄Cl solution was added and the mixture was extracted with diethylether. The combined organic layers were washed with brine, dried with Na₂SO₄ and evaporated. The resulting residue was purified by chromatography (silica gel; heptane/EtOAc 98:2 to 90: 10) to give the title compound (6.533 g) as light yellow solid. MS (ESP): m/z = 237.1 [M+H]⁺.

### Step 2:

### Dimethyl 1,3,4-trimethyl-5,7-dihydrocyclopenta[c]pyridine-6,6-dicarboxylate

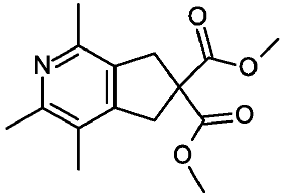

To a mixture of chloro(1,5-cyclooctadiene)iridium(I) dimer (22.7 mg, 0.0339 mmol) and 1,1'-bis(diphenylphosphino)ferrocene (37.5 mg, 0.0677 mmol) in benzene (17 ml) were added acetonitrile (1.39 g, 1.77 ml, 33.9 mmol) and dimethyl 2,2-bis(but-2-ynyl)propanedioate (800 mg, 3.39 mmol) and the reaction vessel was evacuated and refilled with argon. The yellow solution was heated to reflux for 2.5 h. The volatiles were removed and the resulting residue was purified by chromatography (silica gel; heptane/EtOAc 90:10 - 50:50) to give the title compound (731 mg) as light brown solid. MS (ESP): m/z = 278.2 [M+H]⁺.

### Steps 3 to 6:

### 1,3,4-Trimethyl-6,7-dihydro-5H-cyclopenta[clpyridine-6-carbaldehyde

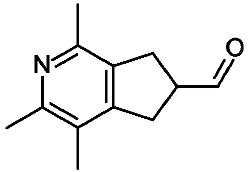

The title compound was prepared in analogy to 1-chloro-3-methoxy-6,7-dihydro-5*H-*cyclopenta[c]pyridine-6-carbaldehyde (Intermediate 37, steps 6 to 8) from dimethyl 1,3,4-trimethyl-5,7-dihydrocyclopenta[c]pyridine-6,6-dicarboxylate and was obtained as brown oil. MS (ESP): m/z = 190.2 [M+H]⁺.

### Intermediate 60

### 1-Methyl-6,7-dihydro-5H-cyclopenta[c]pyridin-6-amine; 2,2,2-trifluoroacetic acid

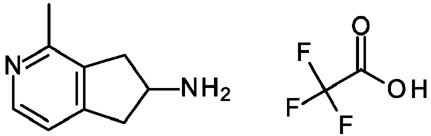

### Step 1:

### tert-Butyl N-(1-chloro-6,7-dihydro-5H-cyclopenta[c]pyridin-6-yl)carbamate

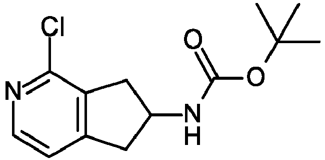

1-Chloro-6,7-dihydro-5*H*-cyclopenta[c]pyridin-6-amine (Intermediate 58, 230 mg, 1.36 mmol, 1 eq) was suspended in dichloromethane (5 mL) and triethylamine (207 mg, 285 µl, 2.05 mmol, 1.5 eq) followed by Boc₂O (313 mg, 333 µl, 1.43 mmol, 1.05 eq) were added at 0 °C. The ice bath was removed and the mixture stirred for 2 hours at RT. The reaction mixture was concentrated and purified by silica cartridge chromatography (ISCO: 20 g from CH₂Cl₂ to 30% EtOAc/ CH₂Cl₂) to give the title compound (340 mg, 1.27 mmol, 92.8 % yield) as an off-white solid. MS (ESP): m/z = 269.2 [M+H]⁺.

### Step 2:

### tert-Butyl N-(1-methyl-6,7-dihydro-5H-cyclopenta[c]pyridin-6-yl)carbamate

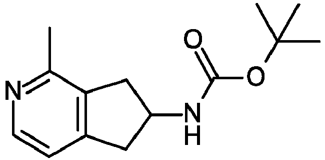

To a solution of *tert*-butyl (1-chloro-6,7-dihydro-5*H*-cyclopenta[c]pyridin-6-yl)carbamate (75 mg, 279 µmol, 1 eq) in dioxane (3 mL) in a pressure tube were added trimethylboroxine (59.6 mg, 66.3 µl, 474 µmol, 1.7 eq) and K₂CO₃ (116 mg, 837 µmol, 3 eq). Argon was bubbled through this mixture and 1,1'-bis(diphenylphosphino)ferrocene-palladium(II)chloride dichloromethane complex (22.8 mg, 27.9 µmol, 0.1 eq) was added. This mixture was stirred for 3 hours at 100 °C. The mixture was poured onto water and the mixture was extracted 3 times with CH₂Cl₂. The combined extracts were dried over Na₂SO₄ and evaporated. The crude product was purified by preparative HPLC to give the title compound (56 mg, 226 µmol, 80.8 % yield) as a light brown solid. MS (ESP): m/z = 249.2 [M+H]⁺.

### Step 3:

### 1-Methyl-6,7-dihydro-5H-cyclopenta[c]pyridin-6-amine; 2,2,2-trifluoroacetic acid

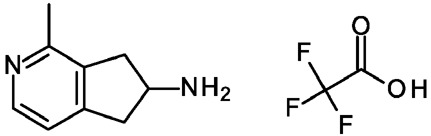

*tert*-Butyl *N*-(1-mcthyl-6,7-dihydro-5*H*-cyclopcnta[c]pyridin-6-yl(carbamate (56 mg, 226 µmol, 1 eq) was dissolved in dichloromethane (2 mL), TFA (514 mg, 347 µl, 4.51 mmol, 20 eq) was added and the mixture was stirred for 2 hours. The mixture was evaporated, diethyl ether was added and decanted 2 times. From the residue water was removed by azeotropic distillation with toluene (3 times) to give the title compound (73 mg, 194 µmol, 86 % yield) as a light brown solid which was used directly in the next reaction without purification.

### Intermediate 61

### 6-Amino-6,7-dihydro-5H-cyclopenta[c]pyridine-1-carbonitrile; 2,2,2-trifluoroacetic acid

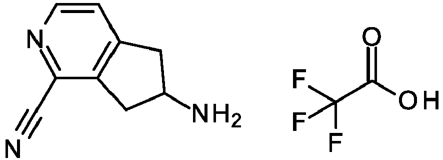

### Step 1:

### tert-Butyl N-(1-cyano-6,7-dihydro-5H-cyclopenta[c]pyridin-6-yl)carbamate

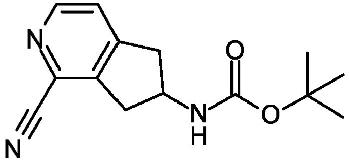

tert-Butyl (1-chloro-6,7-dihydro-5*H*-cyclopenta[c]pyridin-6-yl)carbamate (Intermediate 60, step 1, 75 mg, 279 µmol, 1 eq) was disolved in *N*,*N*-dimethylacetamide (2 ml) and zinc cyanide (45.9 mg, 391 µmol, 1.4 eq) and tBuX-PHOS (23.7 mg, 55.8 µmol, 0.2 eq) were added. Argon was bubbled through the solution, tris(dibenzylideneacetone)dipalladium (0) (25.6 mg, 27.9 µmol, 0.1 eq) was added and the mixture was stirred at 120 °C for 1 hour and then poured onto water. The mixture was extracted 3 times with CH₂Cl₂ and the extracts were dried over Na₂SO₄ and evaporated. The crude product was purified by preparative HPLC to give the title compound (54 mg, 208 µmol, 74.6 % yield) as a light yellow solid. MS (ESP): m/z = 260.2 [M+H]⁺.

### Step 2:

### 6-Amino-6,7-dihydro-5H-cyclopenta[c]pyridine-1-carbonitrile; 2,2,2-trifluoroacetic acid

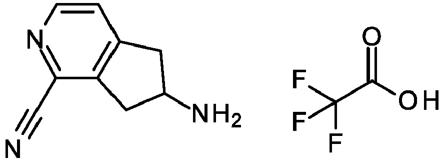

*tert*-Butyl *N*-(1-cyano-6,7-dihydro-5*H*-cyclopenta[c]pyridin-6-yl)carbamate (54 mg, 208 µmol, 1 eq) was disolved in dichloromethane (2 mL), TFA (475 mg, 321 µl, 4.16 mmol, 20 eq) was added and the mixture stirred for 2 hours. The mixture was evaporated, diethyl ether was added and decanted 2 times. From the residue water was removed by azeotropic distillation with toluene (3 times) to give the title compound (62 mg, 160 µmol, 76.9 % yield) as a colorless oil which was used directly in the next reaction without purification.

### Intermediate 62

### 1,4-Dimethyl-6,7-dihydro-5H-cyclopenta[c]pyridine-6-carbaldehyde

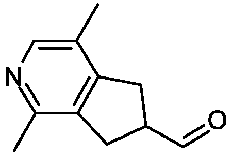

### Step 1:

### 7-(Hydroxymethyl)-4-methyl-1H-furo[3,4-c]pyridin-3-one; hydrochloride

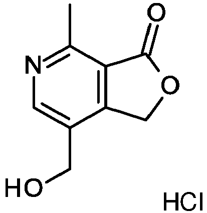

A mixture of ethyl 6-methyl-1,3-dihydrofuro[3,4-c]pyridine-7-carboxylate (30 g, 143.4 mmol, CAS# 92757-50-1, Hel. Chim. Acta 1984, 67, 1274-82) and KOH (9.6 g, 145.4 mmol, 85%) in EtOH (120 ml) was heated to 165°C for 1.5 h. The mixture was cooled to RT, 2N HCl (78.8 ml) was added and the reaction mixture was again heated to 165°C for 20 h. The mixture was then evaporated and EtOH (800 ml) and charcoal were added to the resulting residue. The resulting mixture was heated to 55°C, then cooled to RT, filtered and the filtrate was evaporated. 15 g of the remaining material were dissolved in MeOH (70 ml) and 25% HCl (24.79 ml) was added and the mixture was heated to 60°C for 0.5 h. The mixture was then cooled to 0°C and the precipitate was filtered off to give the title compound (11.1 g) as off-white crystals. Melting point 206 °C.

### Step 2:

### 7-[[tert-Butyl(diphenyl)silyl]oxymethyl]-4-methyl-1H-furo[3,4-c]pyridin-3-one

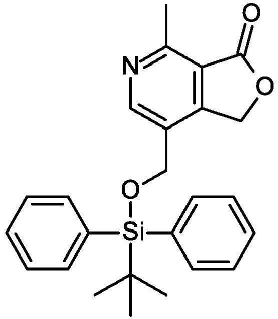

To a mixture of 7-(hydroxymethyl)-4-methyl-1*H*-furo[3,4-c]pyridin-3-one; hydrochloride (4.48 g, 20.8 mmol) and imidazole (7.07 g, 104 mmol) in DMF (40 ml) was added *tert-*butylchlorodiphenylsilane (6.85 g, 6.26 ml, 24.9 mmol) at 0°C. After the addition, the solution was stirred at RT for 3 h. The solution was diluted with water and extracted with EtOAc. The combined organic layers were washed with water (basified with Na₂CO₃) and brine (basified with Na₂CO₃), dried with Na₂SO₄ and evaporated. The resulting residue was purified by chromatography (silica gel; heptane/EtOAc 90:10 to 75:25) to give the title compound (8.82 g) as colorless gum. MS (ESP): m/z = 418.1 [M+H]⁺.

### Step 3:

### [5-[[tert-Butyl(diphenyl)silyl]oxymethyl]-4-(hydroxymethyl)-2-methyl-3-pyridyl]methanol

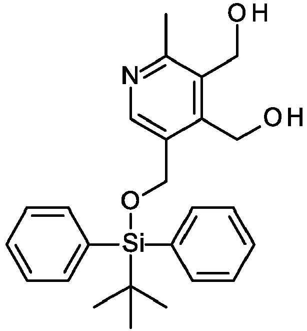

Sodium borohydride (1.76 g, 46.5 mmol) was added to a solution of 7*-*[[*tert-*butyl(diphenyl)silyl]oxymethyl]-4-methyl-1*H*-furo[3,4-c]pyridin-3-one (8.82 g, 21.1 mmol) in EtOH (55 ml) at 35-40 °C. After the addition, the solution was stirred at RT for 6 h. The solution was stored in the fridge overnight. Water was added and the pH was adjusted to 3 by addition of 2N HCl. Then sat. Na₂CO₃ solution was added until the pH was adjusted to 10 and the mixture was extracted with EtOAc. The combined organic layers were washed with water (basified with Na₂CO₃) and brine (basified with Na₂CO₃), dried with Na₂SO₄ and evaporated. The resulting residue was purified by chromatography (silica gel; DCM/MeOH 98:2 to 90:10) to give the title compound (7.057 g) as a white solid. MS (ESP): m/z = 422.3 [M+H]⁺.

### Steps 4 to 5:

### Diethyl 4-[[tert-butyl(diphenyl)silyl]oxymethyl]-1-methyl-5,7-dihydrocyclopenta[c]pyridine-6,6-dicarboxylate

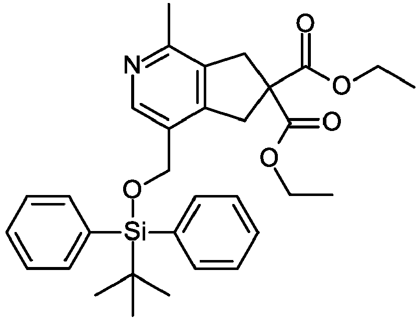

The title compound was prepared by analogy to diethyl 4-methyl-5,7-dihydrocyclopenta[b]pyridine-6,6-dicarboxylate (Intermediate 15, steps 3 and 4) using [5*-*[[*tert-*butyl(diphenyl)silyl]oxymethyl]-4-(hydroxymethyl)-2-methyl-3-pyridyl]methanol in place of [2-(hydroxymethyl)-4-methyl-3-pyridyl]methanol and was obtained as a yellow oil. MS (ESP): m/z = 546.5 [M+H]⁺.

### Step 6:

### Ethyl 4-[[tert-butyl(diphenyl)silyl]oxymethyl]-1-methyl-6,7-dihydro-5H-cyclopenta[c]pyridine-6-carboxylate

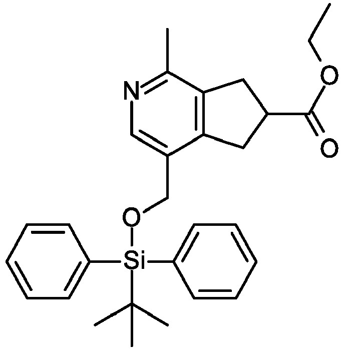

A mixture of diethyl 4-[[*tert*-butyl(diphenyl)silyl]oxymethyl]-1-methyl-5,7-dihydrocyclopenta[c]pyridine-6,6-dicarboxylate (7.943 g, 14.6 mmol, 1 eq) and lithium chloride (617 mg, 14.6 mmol, 1 eq) in DMSO (20 ml) and water (0.5 mL) was stirred at 170 °C for 10 h. The black mixture was cooled to RT overnight, diluted with water (basified with Na₂CO₃) and extracted with EtOAc (3 x). The organic layers were washed with water (basic, 3 x) and brine (basic), dried over Na₂SO₄ and evaporated. The crude product was purified by flash column chromatography (50g silica gel; heptane/EtOAc 90:10 to 75:25) to give the title compound (4.48 g, 9.46 mmol, 65 % yield) as a brown oil. MS (ESP): m/z = 474.5 [M+H]⁺.

### Step 7:

### [4-[[tert-Butyl(diphenyl)silyl]oxymethyl]-1-methyl-6,7-dihydro-5H-cyclopenta[c]pyridin-6-yl]methanol

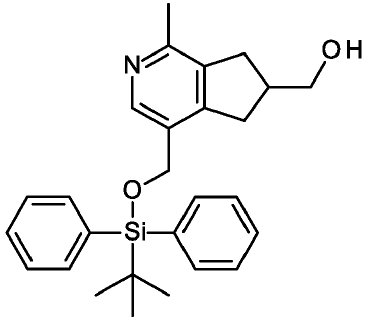

The title compound was prepared in analogy to 1-chloro-6,7-dihydro-*5H*-cyclopenta[c]pyridine-6-carboxylic acid (Intermediate 19, step 3) using ethyl 4-[[*tert*-butyl(diphenyl)silyl]oxymethyl]-1-methyl-6,7-dihydro-5*H*-cyclopenta[c]pyridine-6-carboxylate in place of diethyl 1-chloro-5,7-dihydrocyclopenta[c]pyridine-6,6-dicarboxylate and was obtained as yellow gum. MS (ESP): m/z = 432.4 [M+H]⁺.

### Step 8:

### (1,4-Dimethyl-6,7-dihydro-5H-cyclopenta[c]pyridin-6-yl)methanol

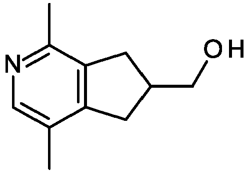

To a mixture of [4-[[*tert*-butyl(diphenyl)silyl]oxymethyl]-1-methyl-6,7-dihydro-5*H-*cyclopenta[c]pyridin-6-yl]methanol (300 mg, 0.695 mmol) and 10% palladium on carbon (120 mg) in MeOH (16 ml) under an atmosphere of hydrogen was added 4N HCl in dioxane (1.74 ml, 6.95 mmol) via syringe and the black suspension was vigorously stirred at RT for 5 h. Additional 4N HCl in dioxane (869 µl, 3.48 mmol) was added and the mixture was stirred at RT overnight. The mixture was filtered through a pad of celite, which was washed well with MeOH. The filtrate was concentrated, sat. Na₂CO₃ solution was added to the resulting residue and the mixture was extracted with DCM. The combined organic layers were washed with a small amount of brine (basified with Na₂CO₃), dried with Na₂SO₄ and evaporated. The resulting residue was purified by chromatography (silica gel; DCM/MeOH 98:2 to 95:5) to give the title compound (105mg) as white solid. MS (ESP): m/z = 178.1 [M+H]⁺.

### Step 9:

### 1,4-Dimethyl-6,7-dihydro-5H-cyclopenta[c]pyridine-6-carbaldehyde

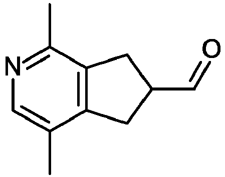

The title compound was obtained from (1,4-dimethyl-6,7-dihydro-5*H*-cyclopenta[c]pyridin-6-yl)methanol (137 mg, 0.773 mmol) in analogy to 1-chloro-6,7-dihydro-5*H-*cyclopenta[c]pyridine-6-carbaldehyde (Intermediate 19, step 4) as dark brown oil (93 mg). MS (ESP): m/z = 176.1 [M+H]⁺.

### Intermediate 63

### 6-[rac-7a-cis-3a,6-trans-6-Amino-2-oxo-3a,4,5,6,7,7a-hexahydro-1,3-benzoxazol-3-yl]-4H-pyrido[3,2-b][1,4]oxazin-3-one

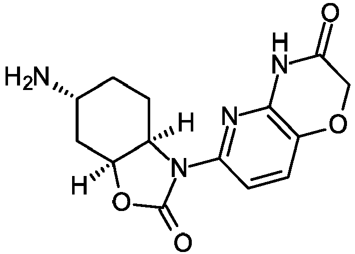

### Step 1:

### Benzyl N-[rac-1,3-trans-3,4-cis-3-hydroxy-4-[(2-methylpropan-2-yl)oxycarbonylamino]cyclohexyl]carbamate

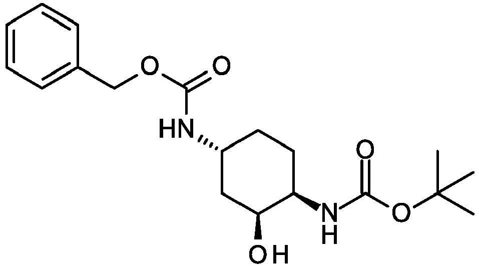

To a solution of benzyl *N*-[*rac*-1,3-*cis*-1,4-*tras*-4-amino-3-hydroxycyclohexyl]carbamate (Intermediate 20, step 2, 3300.0 mg, 12.48 mmol, 1 eq) in 1,4-dioxane (50 mL) and water (20 mL) was added sodium hydrogen carbonate (2097.62 mg, 24.97 mmol, 2 eq) and di-t-butyldicarbonate (3542.27 mg, 16.23 mmol, 1.3 eq). The mixture was stirred at 20 °C for 16 h. Water was added and the mixture was extracted with ethyl acetate. The combined organic layers were washed with brine, dried over anhydrous Na₂SO₄ and concentrated in vacuo to give the title compound (5000 mg, 13.72 mmol, 109.9% yield) as yellow solid. MS (ESP): m/z = 265.1 [M+H-BOC].

### Step 2:

### [rac-1,2-cis-1,5-trans-2-[(2-Methylpropan-2-yl)oxycarbonylamino]-5-(phenylmethoxycarbonylamino)cyclohexyl] 4-nitrobenzoate

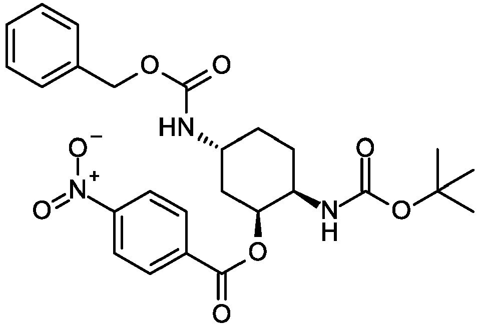

To a solution of triphenylphosphine (3958.31 mg, 15.09 mmol, 1.1 eq) in THF (10 mL) was added diisopropyl azodicarboxylate (2.97 mL, 15.09 mmol, 1.1 eq) in THF (10 mL) dropwise at 0 °C under N₂ and the resulting mixture was stirred for 10 min. 4-Nitrobenzoic acid (2522.12 mg, 15.09 mmol, 1.1 eq) was added in several portions at 0 °C and then benzyl *N-*[*rac-*1,3*-trans-*3,4-cis-3-hydroxy-4-[(2-methylpropan-2-yl)oxycarbonylamino]cyclohexyl]carbamate (5.0 g, 13.72 mmol, 1 eq) in THF (10 mL) was added at 0°C. The mixture was stirred at 20 °C for 3 h. The mixture was washed with brine, dried over anhydrous Na₂SO₄ and concentrated in vacuo to give a residue which was triturated with MeOH to afford the title compound (3500 mg, 6.82 mmol, 49.7% yield) as a white solid.

### Step 3:

### Benzyl N-[rac-3,4-cis-1,3-trans-3-hydroxy-4-[(2-methylpropan-2-yl)oxycarbonylamino]cyclohexyl]carbamate

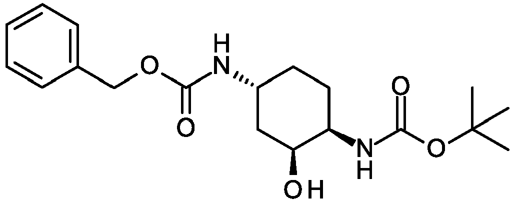

To a solution of [*rac-*1,2-*cis*-1,5-*trans*-2-[(2-methylpropan-2-yl)oxycarbonylamino]-5-(phenylmethoxycarbonylamino)cyclohexyl] 4-nitrobenzoate (3.5 g, 6.82 mmol, 1 eq) in methanol (15 mL), THF (15 mL) and water (10 mL) was added potassium carbonate (1883.87 mg, 13.63 mmol, 2 eq). The mixture was stirred at 30 °C for 3 h. Water (30 mL) and petroleum ether (30 mL) were added and the white solid that precipitated was filtered off to give the title compound (1700 mg, 4.66 mmol, 68.4% yield). MS (ESP): m/z = 365.3 [M+H]⁺.

### Step 4:

### Benzyl N-[rac-3,4-cis-1,3-trans-4-amino-3-hydroxycyclohexy1]carbamate hydrochloride

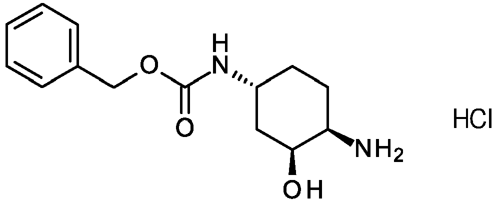

A mixture of benzyl *N*-[*rac*-3,4-*cis*-1,3-*trans*-3-hydroxy-4-[(2-methylpropan-2-yl)oxycarbonylamino]cyclohexyl]carbamate (600.0 mg, 1.65 mmol, 1 eq) and HCl in 1,4-dioxane (10.0 mL, 40 mmol, 24.3 eq) was stirred at 20 °C for 2h. The mixture was filtered and the filter cake was washed with ethyl acetate (30 mL). The filter cake was dried in vacuo to give the title compound (350 mg, 1.16 mmol, 70.7% yield) as a white solid. MS (ESP): m/z = 265.1 [M+H]⁺.

### Steps 5 to 8:

### 6-[rac-7a-cis-3a,6-trans-6-Amino-2-oxo-3a,4,5,6,7,7a-hexahydro-1,3-benzoxazol-3-yl]-4H-pyrido[3,2-b][1,4]oxazin-3-one

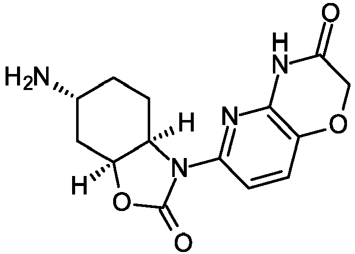

The title compound was prepared by analogy to 6-[*rac*-3a,7a-*trans*-6,7a-*cis*-6-amino-2-oxo-3a,4,5,6,7,7a-hexahydro-1,3-benzoxazol-3-yl]-4*H*-pyrido[3,2-b][1,4]oxazin-3-one (Intermediate 20, steps 3 to 6) using benzyl *N*-[*rac*-3,4-*cis*-1,3-*trans*-4-amino-3-hydroxycyclohexyl]carbamate hydrochloride in place of benzyl *N-*[*rac-*1*,*3*-cis-*1*,*4*-trans-*4*-amino-*3-hydroxycyclohexyl]carbamate and was obtained as a white solid which was used for next step directly.

### Intermediate 64

### 1,4-Dimethyl-3-[(5-oxomorpholin-2-yl)methoxy]-6,7-dihydro-5H-cyclopenta[c]pyridine-6-carbaldehyde

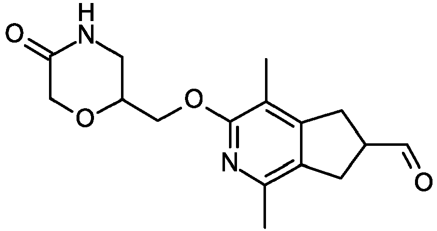

### Steps 1 and 2:

### Dimethyl 1,4-dimethyl-3-oxo-5,7-dihydro-2H-cyclopenta[c]pyridine-6,6-dicarboxylate

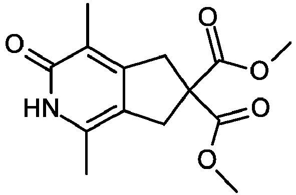

The title compound was prepared by analogy with dimethyl 3-oxo-5,7-dihydro-2*H-*cyclopenta[c]pyridine-6,6-dicarboxylate (Intermediate 37, steps 1 and 2) using dimethyl 2,2-bis(but-2-ynyl)propanedioate in place of dimethyl 2,2-bis(prop-2-ynyl)propanedioate and was obtained as an off-white solid. MS (ESP): m/z = 280.1 [M+H]⁺.

### Step 3:

### Dimethyl 1,4-dimethyl-3-[(5-oxomorpholin-2-yl)methoxy]-5,7-dihydrocyclopenta[c]pyridine-6,6-dicarboxylate

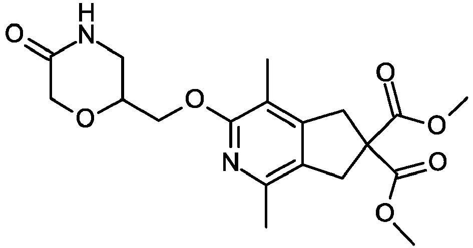

A yellow solution (at 70 °C) of dimethyl 1,4-dimethyl-3-oxo-5,7-dihydro-2*H-*cyclopenta[c]pyridine-6,6-dicarboxylate (50 mg, 0.179 mmol, 1 eq), 6-(hydroxymethyl)morpholin-3-one (23.5 mg, 0.179 mmol, 1 eq), triphenylphosphine (58.7 mg, 0.224 mmol, 1.25 eq) and di-tert-butyl azodicarboxylate (49.5 mg, 0.215 mmol, 1.2 eq) in tetrahydrofuran (2.5 mL) was stirred at RT for 5 h. The mixture was cooled to RT, water was added and the mixture was extracted with EtOAc (3 x). The organic layers were washed with water and brine, dried over Na₂SO₄ and evaporated. The residue was purified by flash column chromatography (20 g silica gel; DCM/MeOH 100:0 - 95:5) to give the title compound (31 mg, 0.075 mmol, 41.9 % yield) as a colourless gum. MS (ESP): m/z = 393.3 [M+H]⁺.

### Steps 4 to 6:

### 1,4-Dimethyl-3-[(5-oxomorphofin-2-yl)methoxy]-6,7-dihydro-5H-cyclopenta[c]pyridine-6-carbaldehyde

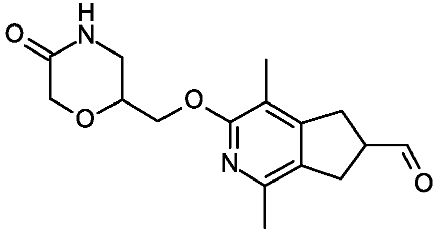

The title compound was prepared by analogy to 1-chloro-3-methoxy-6,7-dihydro-5*H-*cyclopenta[c]pyridine-6-carbaldehyde (Intermediate 37, steps 6 to 8) using dimethyl 1,4-dimethyl-3-[(5-oxomorpholin-2-yl)methoxy]-5,7-dihydrocyclopenta[c]pyridine-6,6-dicarboxylate in place of dimethyl 1-chloro-3-methoxy-5,7-dihydrocyclopenta[c]pyridine-6,6-dicarboxylate and was obtained as a white solid, which was used without purification for the next step. MS (ESP): m/z = 305.3 [M+H]⁺.

### Intermediate 65

### 1,4-Dimethyl-3-[(5-oxopyrrolidin-3-yl)methoxy]-6,7-dihydro-5H-cyclopenta[c]pyridine-6-carbaldehyde

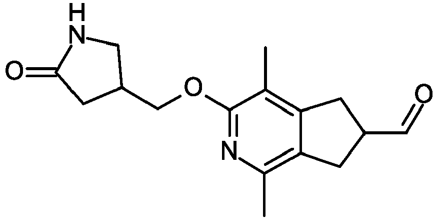

The title compound was prepared by analogy to 1,4-dimethyl-3-[(5-oxomorpholin-2-yl)methoxy]-6,7-dihydro-5*H*-cyclopenta[c]pyridine-6-carbaldehyde (Intermediate 64, steps 3 to 6) using 4-(hydroxymethyl)pyrrolidin-2-one in place of 6-(hydroxymethyl)morpholin-3-one and was obtained as a white solid, which was used without purification for the next step. MS (ESP): m/z = 289.3 [M+H]⁺.

### Intermediate 66

### 1,4-Dimethyl-3-[(1-methyl-5-oxopyrroldin-3-yl)methoxy]-6,7-dihydro-5H-cyclopenta[c]pyridine-6-carbaldehyde

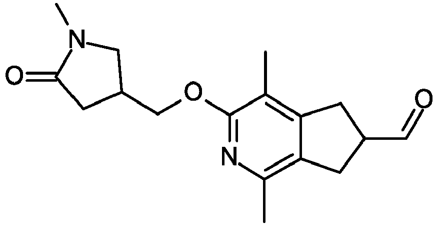

The title compound was prepared by analogy to 1,4-dimethyl-3-[(5-oxomorpholin-2-yl)methoxy]-6,7-dihydro-5*H*-cyclopenta[c]pyridine-6-carbaldehyde (Intermediate 64, steps 3 to 6) using 4-(hydroxymethyl)-1-methylpyrrolidin-2-one in place of 6-(hydroxymethyl)morpholin-3-one and was obtained as a colourless gum, which was used without purification for the next step. MS (ESP): m/z = 303.3 [M+H]⁺.

### Intermediate 67

### 6-(2,4-trans-2-Amino-6-oxo-5-oxa-7-azaspiro[3.4]octan-7-yl)-4H-pyrido[3,2-b][1,4]oxazin-3-one

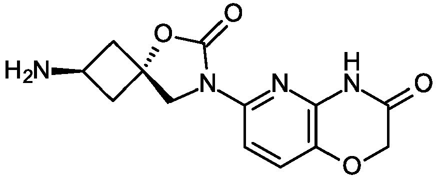

### Step 1:

### Benzyl N-(3,5-trans-1-oxaspiro[2.3]hexan-5-yl)carbamate and benzyl N-(3,5-cis-1-oxaspiro[2.3]hexan-5-yl)carbamate

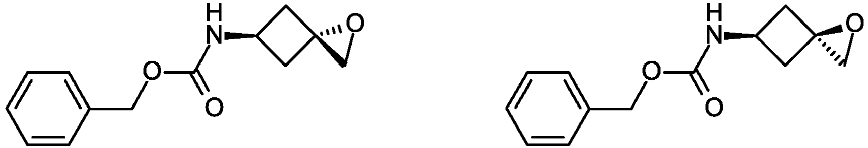

To a stirred solution of benzyl *N*-(3-methylenecyclobutyl)carbamate (5.0 g, 23.01 mmol, 1 eq) in chloroform (85 mL) at 0°C was added m-chloroperbenzoic acid (4.17 g, 24.16 mmol, 1.5 eq) and the reaction mixture was stirred for 30 minutes at 0°C and was then allowed to warm to 25 °C and stirred at that temperature for 1.5h. The reaction mixture was diluted with chloroform (150 mL) and washed with 10% aq. Na₂SO₃ (2 × 200 mL), 10% aq. NaHCO₃ (2 × 200 mL) and brine (200 mL). The organic layer was dried over MgSO₄, filtered and concentrated. The crude product was purified by flash choromatography (silica gel/hexanes: ethyl acetate 0-25%) to afford a 1:1 mixture of the title compounds (4.2 g, 18.01 mmol, 75.8% yield) as an off-white solid. The two isomers were separated by normal phase preparative HPLC (column used- Luna Silica-2(250x21.2)5µ; mobile phase- Hexane/ Iso propanol :98/02; Flow rate- 21.0 ml/min; Run time- 25min; Wave Length- 210nm) to afford benzyl N-(3,5-cis-1-oxaspiro[2.3]hexan-5-yl)carbamate (1.5 g, 6.43 mmol, 81.2% yield) and benzyl *N*-(3,5-*trans*-1-oxaspiro[2.3]hexan-5-yl)carbamate (1.2 g, 5.14 mmol, 64.9% yield) both as white solids. MS (ESP): m/z = 234.2 [M+H]⁺.

### Step 2:

### Benzyl N-[trans-3-(azidomethyl)-3-hydroxycyclobutyl]carbamate

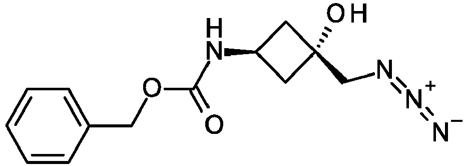

Sodium azide (331 mg, 5.08 mmol, 2 eq) and ammonium chloride (272 mg, 5.08 mmol, 2 eq) were added to benzyl *N*-(3,5-*trans*-1-oxaspiro[2.3]hexan-5-yl)carbamate (593mg, 2.54 mmol, 1 eq) dissolved in MeOH (10 mL) and the mixture was stirred at RT overnight. The reaction mixture was diluted with EtOAc and washed with water and sat. NaHCO₃. The organic phase was concentrated in vacuo and purified by silica cartridge chromatography (ISCO 20 g, 0% to 10% MeOH in DCM) to give the title compound (689.5 mg, 2.5 mmol, 98.2 % yield). MS (ESP): m/z = 277.3 [M+H]⁺.

### Step 3:

### Benzyl N-[trans-3-(aminomethyl)-3-hydroxycyclobutyl]carbamate

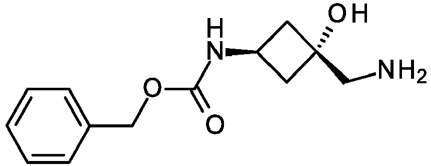

Trimethylphosphine in THF 1M (384 µl, 384 µmol, 2 eq) was added to benzyl *N*-[trans-3-(azidomethyl)-3-hydroxycyclobutyl]carbamate (53 mg, 192 µmol, 1 eq) in THF (4 mL) and the mixture was stirred at RT for 1.5 hours. Water (4 g, 4 mL, 222 mmol) was added and the mixture was stirred for 2 hours. The mixture was diluted with CH₂Cl₂ and sat. NaHCO₃ and then extracted three times with CH₂Cl₂. The aqueous phase was saturated with NaCl and re-extracted with THF. The combined organic phases were concentrated in vacuo and purified by reverse phase cartridge chromatography (ISCO 20 g, 10% to 50% MeCN in water+0.1% formic acid) to give the title compound (45.2 mg, 181 µmol, 94.1 % yield) as a yellow oil. MS (ESP): m/z = 251.3 [M+H]⁺.

### Steps 4 to 7:

### 6-(2,4-trans-2-Amino-6-oxo-5-oxa-7-azaspiro[3.4]octan-7-yl)-4H-pyrido[3,2-b][1,4]oxazin-3-one

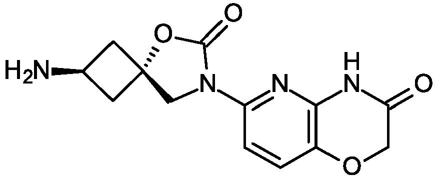

The title compound was prepared by analogy to 6-(6-oxo-5-oxa-2,7-diazaspiro[3.4]octan-7-yl)-4*H*-pyrido[3,2-b] [1,4]oxazin-3-one (Intermediate 21, steps 2 to 5) using benzyl *N*-[trans-3-(aminomethyl)-3-hydroxycyclobutyl]carbamate in place of 3-(aminomethyl)-1-benzhydrylazetidin-3-ol. MS (ESP): m/z = 291.1 [M+H]⁺.

### Intermediate 68

### 1,4-Dimethyl-3-(3-methylsulfonylpropoxy)-6,7-dihydro-5H-cyclopenta[c]pyridine-6-carbaldehyde

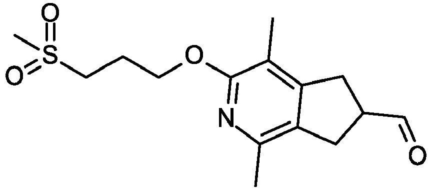

The title compound was prepared by analogy to 1,4-dimethyl-3-[(5-oxomorpholin-2-yl)methoxy]-6,7-dihydro-5*H*-cyclopenta[c]pyridine-6-carbaldehyde (Intermediate 64, steps 3 to 6) using 3-(methylsulfonyl)propan-1-ol in place of 6-(hydroxymethyl)morpholin-3-one and was obtained as a light brown semi-solid, which was used without purification for the next step. MS (ESP): m/z = 312.2 [M+H]⁺.

### Intermediate 69

### 3-Methoxy-1,4-dimethyl-6,7-dihydro-5H-cyclopenta[c]pyridine-6-carbaldehyde

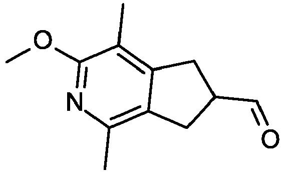

The title compound was prepared by analogy to 1,4-dimethyl-3-[(5-oxomorpholin-2-yl)methoxy]-6,7-dihydro-5*H*-cyclopenta[c]pyridine-6-carbaldehyde (Intermediate 64, steps 4 to 6) using dimethyl 3-methoxy-1,4-dimethyl-5,7-dihydrocyclopenta[c]pyridine-6,6-dicarboxylate in place of dimethyl 1,4-dimethyl-3-[(5-oxomorpholin-2-yl)methoxy]-5,7-dihydrocyclopenta[c]pyridine-6,6-dicarboxylate and was obtained as a yellow gum. The material was used without purification for the next step. MS (ESP): m/z = 206.2 [M+H]⁺.

The dimethyl 3-methoxy-1,4-dimethyl-5,7-dihydrocyclopenta[c]pyridine-6,6-dicarboxylate used as starting material was prepared as follows:
Dimethyl 1,4-dimethyl-3-oxo-5,7-dihydro-2H-cyclopenta[c]pyridine-6,6-dicarboxylate (Intermediate 64, step 2, 50 mg, 0.179 mmol, 1 eq) was dissolved in chloroform (EtOH-free) (2.5 mL). Silver carbonate (98.7 mg, 0.358 mmol, 2 eq) and iodomethane (254 mg, 111 µl, 1.79 mmol, 10 eq) were added and the resulting yellow suspension was stirred in the dark at RT for 2 h. Additional iodomethane (254 mg, 111 µl, 1.79 mmol, 10 eq) was added and the suspension was stirred at RT overnight. The solid was filtered off, washed well with EtOAc. The filtrate was concentrated. The crude material was adsorbed on adsorbent and purified by flashc column hromatography (20 g silica gel; heptane/EtOAc 98:2 - 90:10) to give dimethyl 3-methoxy-1,4-dimethyl-5,7-dihydrocyclopenta[c]pyridine-6,6-dicarboxylate as a colourless gum, which solidified after a while. MS (ESP): m/z = 294.2 [M+H]⁺.

### Intermediate 70

### 2-[(7-Cyano-2-formyl-2,3-dihydro-1H-inden-5-yl)oxy]-N-methylacetamide

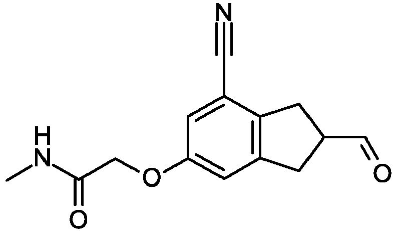

### Step 1:

### Diethyl 4-bromo-1,3-dihydroindene-2,2-dicarboxylate

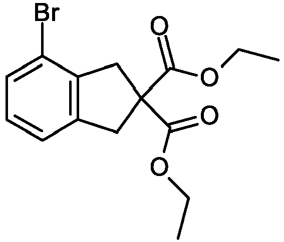

Diethyl malonate (11.7 g, 11.1 ml, 72.9 mmol, 1 eq) was dissolved in tetrahydrofuran (438 mL), then NaH (5.83 g, 146 mmol, 2 eq) was added portionwise at 0°C and the mixture (solution 1) stirred for 1 h at 0 °C. 1-Bromo-2,3-bis(bromomethyl)benzene (25 g, 72.9 mmol, 1 eq) was dissolved in tetrahydrofuran (292 mL) and cooled to 0 °C. Solution 1 was added by cannula and the mixture warmed to RT over night. The reaction mixture was poured onto water and extracted twice with EtOAc. The extracts were washed with brine, dried over Na₂SO₄, filtered and evaporated. The residue was purified by silica column chromatography (heptane to 10% EtOAc/heptane) to give the title compound (14.2 g, 41.6 mmol, 57.1 % yield) as a light yellow oil. MS (ESP): m/z = 343.2 [M+H]⁺.

### Step 2:

### Ethyl 4-bromo-2,3-dihydro-1H-indene-2-carboxylate

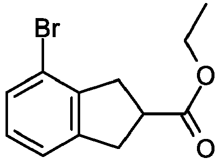

The title compound was prepared by analogy to methyl 1-chloro-3-methoxy-6,7-dihydro-5*H-*cyclopenta[c]pyridine-6-carboxylate (Intermediate 37, step 6) using diethyl 4-bromo-1,3-dihydroindene-2,2-dicarboxylate in place of dimethyl 1-chloro-3-methoxy-5,7-dihydrocyclopenta[c]pyridine-6,6-dicarboxylate and was obtained as a light yellow liquid. MS (ESP): m/z = 271.1 [M+H]⁺.

### Step 3:

### Ethyl 4-cyano-2,3-dihydro-1H-indene-2-carboxylate

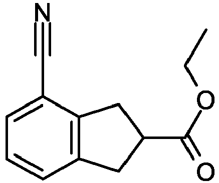

The title compound was prepared by analogy to 2-(hydroxymethyl)indane-4-carbonitrile (Intermediate 5, step 2) using ethyl 4-bromo-2,3-dihydro-1H-indene-2-carboxylate in place of (4-bromoindan-2-yl)methanol and was obtained as a light yellow oil.

### Step 4:

### Ethyl 4-cyano-6-hydroxy-2,3-dihydro-1H-indene-2-carboxylate

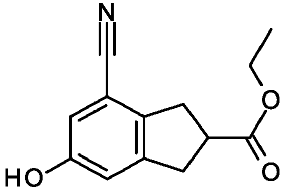

The title compound was prepared by analogy to ethyl 4-fluoro-6-hydroxy-2,3-dihydro-1*H-*indene-2-carboxylate (Intermediate 50, step 3) using ethyl 4-cyano-2,3-dihydro-1*H*-indene-2-carboxylate in place of ethyl 4-fluoro-2,3-dihydro-1*H*-indene-2-carboxylate and was obtained as a colorless oil. MS (ESP): m/z = 230.2 [M+H]⁺.

### Steps 5 to 7:

### 2-[(7-Cyano-2-formyl-2,3-dihydro-1H-inden-5-yl)oxy]-N-methylacetamide

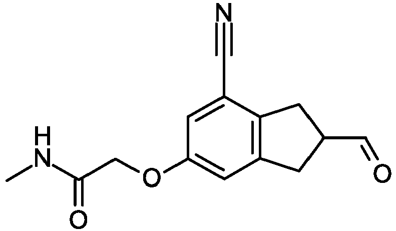

The title compound was prepared by analogy to 2-[(4-cyano-2-formyl-2,3-dihydro-1*H*-inden-5-yl)oxy]-*N*-methylacetamide (Intermediate 36, steps 4, 5 and 7) using ethyl 4-cyano-6-hydroxy-2,3-dihydro-1*H*-indene-2-carboxylate in place of ethyl 4-bromo-5-hydroxy-2,3-dihydro-1*H-*indene-2-carboxylate and was obtained as a white solid. MS (ESP): m/z = 259.1 [M+H]⁺.

### Intermediate 71

### 2-[(4-Chloro-2-formyl-2,3-dihydro-1H-inden-5-yl)oxy]-N-methylacetamide

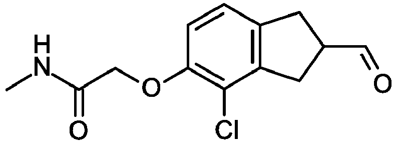

### Steps 1 to 3:

### Ethyl 4-chloro-5-hydroxy-2,3-dihydro-1H-indene-2-carboxylate

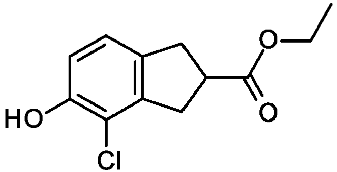

The title compound was prepared by analogy to ethyl 4-bromo-5-hydroxy-2,3-dihydro-1*H-*indene-2-carboxylate (Intermediate 36, steps 1 to 3) using 4-chloro-5-methoxy-2,3-dihydroinden-1-one (CAS# 944109-65-3) in place of 4-bromo-5-methoxy-2,3-dihydroinden-1-one and was obtained as a colourless oil. MS (ESP): m/z = 241.2 [M+H]⁺.

### Step 4:

### 4-Chloro-2-(hydroxymethyl)-2,3-dihydro-1H-inden-5-ol

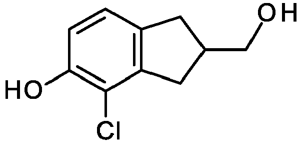

Ethyl 4-chloro-5-hydroxy-2,3-dihydro-1*H*-indene-2-carboxylate (619.5 mg, 2.57 mmol, 1 eq) was dissolved in THF (10 mL) and the mixture was cooled to -78 °C before lithium aluminum hydride 1 M in THF (3.09 mL, 3.09 mmol, 1.2 eq) was added. The resulting solution was stirred at -78 °C for 2.5 h. Sat. aq. NH₄Cl was slowly added until gas development stopped. The mixture was extracted twice with DCM and the combined organic phases were dried over Na₂SO₄ and concentrated in vacuo. The resulting residue was purified by flash chromatography (ISCO 50 g, 0% to 100% EtOAc in heptane) to give the title compound (456.8 mg, 2.3 mmol, 89.3 % yield) as a light yellow oil. MS (ESP): m/z = 197.2 [M+H]⁺.

### Step 5:

### 4-Chloro-2-(trimethylsilyloxymethyl)-2,3-dihydro-1H-inden-5-ol

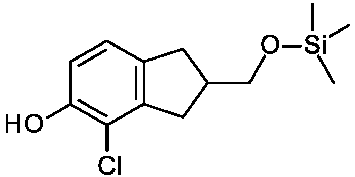

4-Chloro-2-(hydroxymethyl)-2,3-dihydro-1*H*-inden-5-ol (456.8 mg, 2.3 mmol, 1 eq) was dissoved in DMF (10 mL) and imidazole (157 mg, 2.3 mmol, 1 eq) and chlorotrimethylsilane (250 mg, 292 µl, 2.3 mmol, 1 eq) were added at 0°C. The reaction mixture was stirred overnight allowing to warm to RT. The reaction mixture was diluted with TBME, washed twice with water and concentrated in vacuo to give the title compound (463 mg, 43% yield) which was used in the next step without purification.

### Step 6:

### 2-[[4-Chloro-2-(hydroxymethyl)-2,3-dihydro-1H-inden-5-yl]oxy]-N-methylacetamide

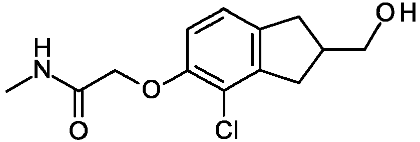

The title compound was prepared by analogy to ethyl 4-bromo-5-[2-(methylamino)-2-oxoethoxy]-2,3-dihydro-1*H*-indene-2-carboxylate (Intermediate 36, step 4) using 4-chloro-2-(trimethylsilyloxymethyl)-2,3-dihydro-1*H*-inden-5-ol in place of ethyl 4-bromo-5-hydroxy-2,3-dihydro-1*H*-indene-2-carboxylate and was obtained as a white solid. MS (ESP): m/z = 270.2 [M+H]⁺.

### Step 7:

### 2-[(4-Chloro-2-formyl-2,3-dihydro-1H-inden-5-yl)oxy]-N-methylacetamide

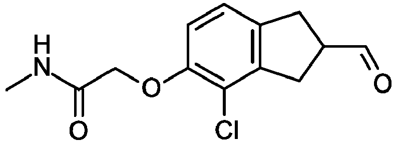

2-[[4-Chloro-2-(hydroxymethyl)-2,3-dihydro-1*H*-inden-5-yl]oxy]-*N*-methylacetamide (50 mg, 185 µmol, 1 eq) was dissolved in dichloromethane (4 mL). TEMPO (2.9 mg, 18.5 µmol, 0.1 eq) and iodobenzene diacetate (59.7 mg, 185 µmol, 1 eq) were added and the mixture was stirred for 2 h. Further TEMPO (2.9 mg, 18.5 µmol, 0.1 eq) and iodobenzene diacetate (59.7 mg, 185 µmol, 1 eq) were added and the mixture stirred for 2 h. The reaction mixture was concentrated and the residue was purified by silica cartridge chromatography (ISCO: 20g from CH₂Cl₂ to 5% MeOH in CH₂Cl₂) to give the title compound (50 mg, 174 µmol, 93.7 % yield) as a light red oil. MS (ESP): m/z = 268.2 [M+H]⁺.

### Intermediate 72

### 2-[(6-Formyl-1,4-dimethyl-6,7-dihydro-5H-cyclopenta[c]pyridin-3-yl)oxy]-N-methylacetamide

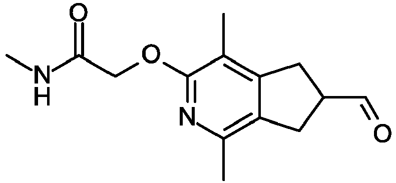

### Step 1:

### Dimethyl 2-(3-bromoprop-2-ynyl)-2-but-2-ynylpropanedioate

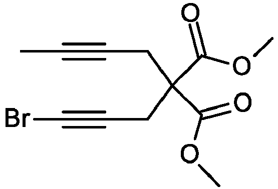

Dimethyl 2-but-2-ynyl-2-prop-2-ynylpropanedioate (CAS# 188025-73-2, 5 g, 22.5 mmol, 1 eq) was dissolved in acetone (100 mL). N-Bromsuccinimide (6.01 g, 33.7 mmol, 1.5 eq) and AgNO₃ (573 mg, 3.37 mmol, 0.15 eq) were added and the resulting mixture was stirred at RT for 1.5 h in the dark. The reaction mixture was treated with 30 mL H₂O and extracted with EtOAc (2 × 200 mL). The organic layers were dried (Na₂SO₄) and evaporated. The crude material was purified by flash chromatography (silica gel, 120g, 2% to 30% EtOAc in heptane) to give the title compound (6.76 g, 97.8 % yield) as a yellow oil. MS (ESP): m/z = 301.1 and 303.1 [M+H]⁺.

### Step 2:

### Dimethyl 1-bromo-2-[(4-methoxyphenyl)methyl]-4-methyl-3-oxo-5,7-dihydrocyclopenta[c]pyridine-6,6-dicarboxylate and dimethyl 4-bromo-2-[(4-methoxyphenyl)methyl]-1-methyl-3-oxo-5,7-dihydrocyclopenta[c]pyridine-6,6-dicarboxylate

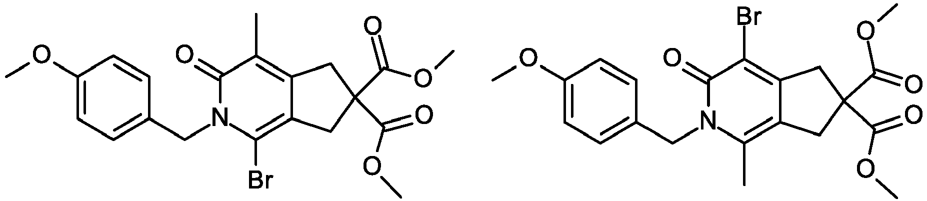

To a solution of chloro(1,5-cyclooctadiene)(pentamethylcyclopentadienyl)ruthenium(II) (214 mg, 562 µmol, 0.05 eq) and 4-methoxybenzylisocyanate (3.67 g, 3.21 mL, 22.5 mmol, 2 eq) in degassed 1,2-dichloroethane (35 mL) was added dropwise a solution of dimethyl 2-(3-bromoprop-2-ynyl)-2-but-2-ynylpropanedioate (3.385 g, 11.2 mmol, 1 eq) in 1,2-dichloroethane (132 mL) at 90 °C. The solution was stirred at 90 °C for 2 h. The mixture was evaporated and the crude residue was purified in two portions by flash chromatography (silica gel, 120 g, 5% to 100% EtOAc in heptane) to give a mixture of the title compounds (4.99 g, 96 % yield) as a light brown solid. The compounds were separated by SFC to give dimethyl 1-bromo-2-[(4-methoxyphenyl)methyl]-4-methyl-3-oxo-5,7-dihydrocyclopenta[c]pyridine-6,6-dicarboxylate (2.544 g) as a light brown solid and dimethyl 4-bromo-2-[(4-methoxyphenyl)methyl]-1-methyl-3-oxo-5,7-dihydrocyclopenta[c]pyridine-6,6-dicarboxylate (2.048 g) as a light brown foam. MS (ESP): m/z = 464.3 and 466.3 [M+H]⁺ for both products.

### Step 3:

### Dimethyl 1-bromo-4-methyl-3-oxo-5,7-dihydro-2H-cyclopenta[c]pyridine-6,6-dicarboxylate

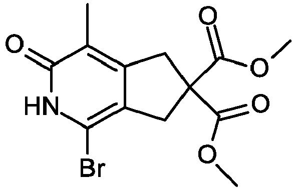

Dimethyl 1-bromo-2-[(4-methoxyphenyl)methyl]-4-methyl-3-oxo-5,7-dihydrocyclopenta[c]pyridine-6,6-dicarboxylate (1.335 g, 2.88 mmol, 1 eq) was combined with TFA (13.1 mL) to give a dark brown solution. The reaction mixture was heated to 100 °C for 1.5h. The reaction mixture was concentrated in vacuo, diluted with DCM and evaporated again (4 x). The crude material was purified by flash chromatography (silica gel, 50g, 10% to 100% EtOAc in heptane) to give the title compound (1.123 g, 99.9% yield) as a light yellow solid. MS (ESP): m/z = 344.2 and 346.2 [M+H]⁺.

### Step 4:

### Dimethyl 1-bromo-4-methyl-3-[2-(methylamino)-2-oxoethoxy]-5,7-dihydrocyclopenta[c]pyridine-6,6-dicarboxylate

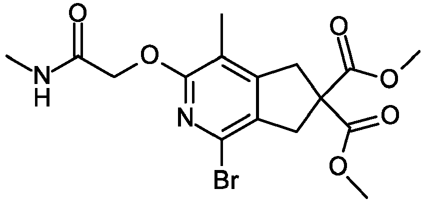

A mixture of dimethyl 1 -bromo-4-methyl-3-oxo-5,7-dihydro-2*H*-cyclopenta[c]pyridine-6,6-dicarboxylate (150 mg, 0.436 mmol, 1 eq), 2-chloro-*N*-methylacetamide (56.2 mg, 0.523 mmol, 1.2 eq), sodium iodide (16.3 mg, 0.109 mmol, 0.25 eq) and potassium carbonate (108 mg, 0.785 mmol, 1.8 eq) in *N*,*N*-dimethylacetamide (2 mL) was stirred at 50 °C for 1 h 45 min. Additional 2-chloro-*N*-methylacetamide (23.4 mg, 0.218 mmol, 0.5 eq) and potassium carbonate (54.2 mg, 0.392 mmol, 0.9 eq) were added and the mixture was stirred at 50 °C for 2 h. The mixture was cooled to RT, diluted with water and extracted with EtOAc (3 x). The organic layers were washed with water (2 x) and brine, dried over Na₂SO₄ and evaporated. The crude material was purified by flash column chromatography (20 g silica gel; DCM/MeOH 100:0 - 90:10) to give the title compound (90 mg,49.7 % yield) as a light brown semisolid. MS (ESP): m/z = 415.2 [M+H]⁺.

### Step 5:

### Dimethyl 1,4-dimethyl-3-[2-(methylamino)-2-oxoethoxy]-5,7-dihydrocyclopenta[c]pyridine-6,6-dicarboxylate

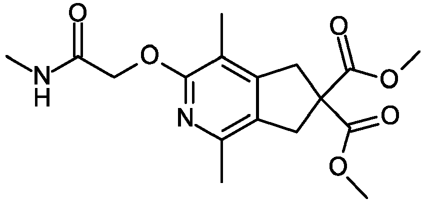

The title compound was prepared by analogy to 6-(hydroxymethyl)-1-methyl-6,7-dihydro-5*H-*cyclopenta[c]pyridine-4-carbonitrile (Intermediate 35, step 10) using dimethyl 1-bromo-4-methyl-3-[2-(methylamino)-2-oxoethoxy]-5,7-dihydrocyclopenta[c]pyridine-6,6-dicarboxylate in place of 1-bromo-6-(hydroxymethyl)-6,7-dihydro-5*H*-cyclopenta[c]pyridine-4-carbonitrile and was obtained as a light brown solid. MS (ESP): m/z = 351.2 [M+H]⁺.

### Step 6:

### Methyl 1,4-dimethyl-3-[2-(methylamino)-2-oxoethoxy]-6,7-dihydro-5H-cyclopenta[c]pyridine-6-carboxylate

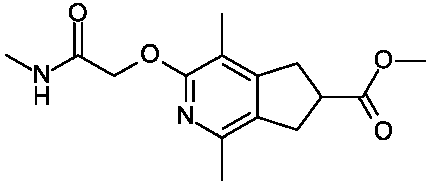

The title compound was prepared by analogy to methyl 1-chloro-3-methoxy-6,7-dihydro-5*H-*cyclopenta[c]pyridine-6-carboxylate (Intermediate 37, step 6) using dimethyl 1,4-dimethyl-3-[2-(methylamino)-2-oxoethoxy]-5,7-dihydrocyclopenta[c]pyridine-6,6-dicarboxylate in place of dimethyl 1-chloro-3-methoxy-5,7-dihydrocyclopenta[c]pyridine-6,6-dicarboxylate and was obtained as a light brown solid. MS (ESP): m/z = 293.2 [M+H]⁺.

### Step 7:

### 2-[(6-Formyl-1,4-dimethyl-6,7-dihydro-5H-cyclopenta[c]pyridin-3-yl)oxy]-N-methylacetamide

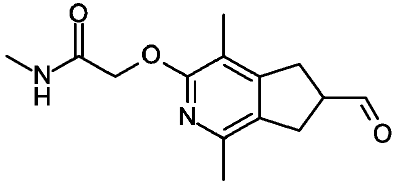

Methyl 1,4-dimethyl-3-[2-(methylamino)-2-oxoethoxy]-6,7-dihydro-5*H*-cyclopenta[c]pyridine-6-carboxylate (127 mg, 0.413 mmol, 1 eq) was dissolved in tetrahydrofuran (4 mL). At -78 °C Lithium aluminium hydride 1 M in THF (495 µL, 0.495 mmol, 1.2 eq) was added. The resulting solution was stirred at -78 °C for 1 h. Et₂O and THF were added. Na₂SO₄ sat. sol. was added dropwise until gas development ceased. Solid Na₂SO₄ was added and the mixture was filtered. The solid was washed well with THF and Et₂O. The filtrate was concentrated. The residue was adsorbed on adsorbent and purified by flashchromatography (20 g silica gel; heptane/EtOAc 80:20 - 5:95) to give the title compound (55 mg, 45.7 % yield) as a colourless gum. MS (ESP): m/z = 263.2 [M+H]⁺.

### Intermediate 73

### 2-[(2-Formyl-4,7-dimethyl-2,3-dihydro-1H-inden-5-yl)oxy]-N-methylacetamide

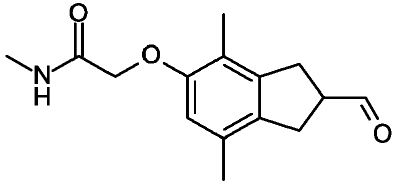

### Step 1:

### Dimethyl 4,7-dimethyl-5-(4,4,5,5-tetramethyl-1,3,2-dioxaborolan-2-yl)-1,3-dihydroindene-2,2-dicarboxylate

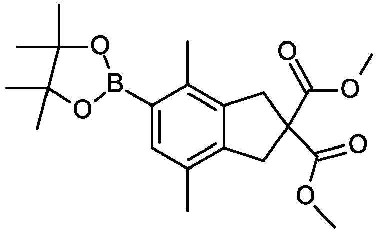

A solution of dimethyl 2,2-bis(but-2-ynyl)propanedioate (CAS# 107428-05-7, 213 mg, 0.9 mmol, 1 eq) in dry degassed 1,2-dichloroethane (6 mL) was added over 1 h via syringe pump at RT to a solution of chloro(1,5-cyclooctadiene)(pentamethylcyclopentadienyl)ruthenium(II) (68.4 mg, 0.18 mmol, 0.2 eq) and 2-ethynyl-4,4,5,5-tetramethyl-1,3,2-dioxaborolane (274 mg, 1.8 mmol, 2 eq) in dry degassed 1,2-dichloroethane (4.5 mL) . The solution was stirred at RT under an Ar atmosphere overnight. Further 2-ethynyl-4,4,5,5-tetramethyl-1,3,2-dioxaborolane (137 mg, 0.9 mmol, 1 eq) and chloro(1,5-cyclooctadiene)(pentamethylcyclopentadienyl)ruthenium(II) (68.4 mg, 0.180 mmol, 0.2 eq) were added and the solution was stirred at RT under Ar atmosphere for 2 hours. The solvent was removed. The crude product was purified by flash column chromatography (20 g silica gel; heptane/EtOAc 98:2 - 90:10) to give the title compound (159 mg, 27.3 % yield) as a light yellow solid. MS (ESP): m/z = 389.4 [M+H]⁺.

### Step 2:

### Dimethyl 5-hydroxy-4,7-dimethyl-1,3-dihydroindene-2,2-dicarboxylate

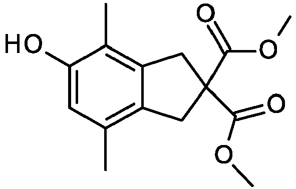

Sodium perborate monohydrate (118 mg, 1.18 mmol, 3 eq) was added to a solution of dimethyl 4,7-dimethyl-5-(4,4,5,5-tetramethyl-1,3,2-dioxaborolan-2-yl)-1,3-dihydroindene-2,2-dicarboxylate (153 mg, 0.394 mmol, 1 eq) in THF (3 mL) and water (3 mL) and the mixture was stirred for 35 minutes. 3 mL sat. NH₄Cl sol. were added to the brown solution. The resulting mixture was stirred for 3 min, then poured into a mixture of water and EtOAc and stirred for 10 min. The mixture was filtered through a pad of Celite, which was washed well with EtOAc. The pH of the aqueous layer was adjusted to 3 with 0.1 N HCl. The layers were separated and the aqueous layer was extracted with EtOAc (3 x). The combined organic layers were washed with water (pH 3) and brine, dried over Na₂SO₄ and evaporated. The crude product was purified by flash column chromatography (20 g silica gel; heptane/EtOAc 90:10 - 80:20) to give the title compound (32 mg, 29.2 % yield) as a white solid. MS (ESP): m/z = 279.2 [M+H]⁺.

### Steps 3 to 5:

### 2-[(2-Formyl-4,7-dimethyl-2,3-dihydro-1H-inden-5-yl)oxy]-N-methylacetamide

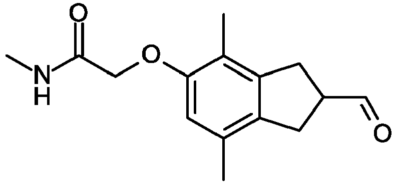

The title compound was prepared by analogy to 2-[(6-formyl-1,4-dimethyl-6,7-dihydro-5*H-*cyclopenta[c]pyridin-3-yl)oxy]-N-methylacetamide (Intermediate 72, steps 4, 6 and 7) using dimethyl 5-hydroxy-4,7-dimethyl-1,3-dihydroindene-2,2-dicarboxylate in place of dimethyl 1-bromo-4-methyl-3-oxo-5,7-dihydro-2H-cyclopenta[c]pyridine-6,6-dicarboxylate and was obtained as a white solid. MS (ESP): m/z = 262.2 [M+H]⁺.

### Intermediate 74

### tert-Butyl N-[2-[(4-chloro-2-formyl-2,3-dihydro-1H-inden-5-yl)oxy]ethyl]carbamate

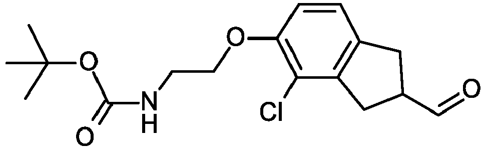

### Steps 1 to 3:

### Ethyl 4-chloro-5-hydroxy-2,3-dihydro-1H-indene-2-carboxylate

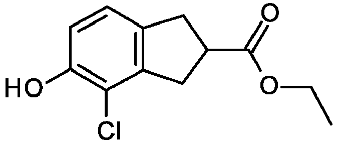

The title compound was prepared by analogy to ethyl 4-bromo-5-hydroxy-2,3-dihydro-1*H-*indene-2-carboxylate (Intermediate 36, steps 1 to 3) using 4-fluoro-5-methoxy-indan-1-one in place of 4-bromo-5-methoxy-indan-1-one and was obtained as a light brown oil. ¹H-NMR (400 MHz, CDCl₃) δ 7.00 (d, 1H), 6.84 (d, 1H), 5.38 (s, 1H), 4.20 (q, 2H), 3.38 (m, 1H), 3.25 (m, 4H), 1.30 (t, 3H).

### Step 4:

### Ethyl 4-chloro-5-[2-[(2-methylpropan-2-yl)oxycarbonylamino]ethoxy]-2,3-dihydro-1H-indene-2-carboxylate

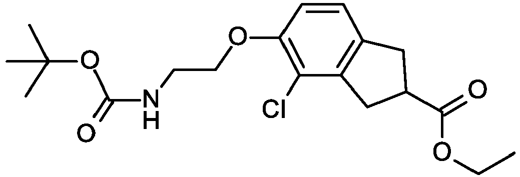

To a solution of ethyl 4-chloro-5-hydroxy-2,3-dihydro-1*H*-indene-2-carboxylate (150.0 mg, 0.620 mmol, 1 eq), diisopropyl azodicarboxylate (0.25 mL, 1.25 mmol, 2 eq) and *tert*-butyl N-(2-hydroxyethyl)carbamate (100.47 mg, 0.620 mmol, 1 eq) in THF (5 mL) was added dropwise tri-n-butylphosphine (189.14 mg, 0.930 mmol, 1.5 eq) at 0 °C. After addition, the mixture was stirred at 20 °C for 16 h. The reaction mixture was diluted with EtOAc (25 mL) and filtered. The filtrate was evaporated to give a residue, which was purified by flash chromatography (PE: EtOAc =50/1 to 10/1) to give the title compound (120 mg, 0.310 mmol, 50.2% yield) as a white solid. MS (ESP): m/z = 284.1 [M+H-BOC]⁺.

### Steps 5 and 6:

### tert-Butyl N-[2-[(4-chloro-2-formyl-2,3-dihydro-1H-inden-5-yl)oxy]ethyl]carbamate

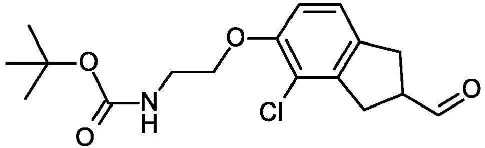

The title compound was prepared by analogy to 4-fluoro-1-methyl-6,7-dihydro-5*H-*cyclopenta[c]pyridine-6-carbaldehyde (Intermediate 27, steps 9 and 10) and was obtained as a white solid. The crude product was used for the next step directly. MS (ESP): m/z = 362.1 [M+Na]⁺.

### Intermediate 75

### tert-Butyl N-[1-[(4-chloro-2-formyl-2,3-dihydro-1H-inden-5-yl)oxymethyl]cyclopropyl]carbamate

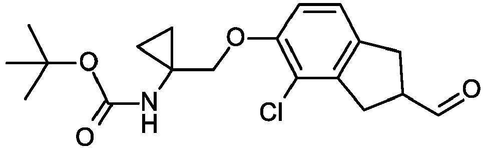

### Step 1:

### Methyl 4-chloro-5-[[1-[(2-methylpropan-2-yl)oxycarbonylamino]cyclopropyl]methoxy]-2,3-dihydro-1H-indene-2-carboxylate

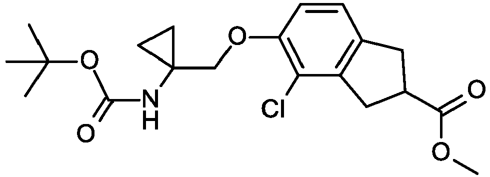

To a solution of ethyl 4-chloro-5-hydroxy-2,3-dihydro-1*H*-indene-2-carboxylate (Intermediate 74, step 3, 500.0 mg, 2.08 mmol, 1 eq) and *tert*-butyl *N*-[1-(bromomethyl)cyclopropyl]carbamate (CAS# 387845-49-0, 623.56 mg, 2.49 mmol, 1.2 eq) in acetonitrile (25 mL) was added potassium carbonate (574.23 mg, 4.15 mmol, 2 eq). After addition, the mixture was stirred at 50 °C for 16 h. *tert*-Butyl *N*-[1-(bromomethyl)cyclopropyl]carbamate (155.87 mg, 0.620 mmol, 0.3 eq) was added and the reaction was heated to 80 °C and stirred for another 6 h. The reaction mixture was diluted with EtOAc (50 mL) and filtered. The filtrate was evaporated to give a residue, which was purified by flash chromatography (PE:EA=50/1-10/1) to give the title compound (780 mg, 1.9 mmol, 91.6% yield) as a colourless oil. MS (ESP): m/z = 432.2 [M+Na]⁺.

### Steps 2 and 3:

### tert-Butyl N-[1-[(4-chloro-2-formyl-2,3-dihydro-1H-inden-5-yl)oxymethyl]cyclopropyl]carbamate

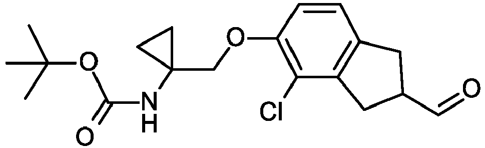

The title compound was prepared by analogy to 4-fluoro-1-methyl-6,7-dihydro-5*H-*cyclopenta[c]pyridine-6-carbaldehyde (Intermediate 27, steps 9 and 10) and was obtained as a white solid. MS (ESP): m/z = 388.2 [M+Na]⁺.

### Intermediate 76

### tert-Butyl N-[2-[(2-amino-4-chloro-2,3-dihydro-1H-inden-5-yl)oxy]ethyl]carbamate

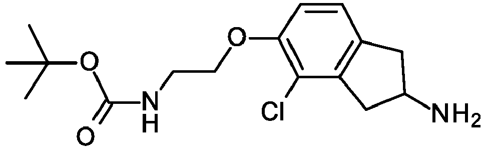

### Step 1:

### 4-Chloro-5-[2-[(2-methylpropan-2-yl)oxycarbonylamino]ethoxy]-2,3-dihydro-1H-indene-2-carboxylic acid

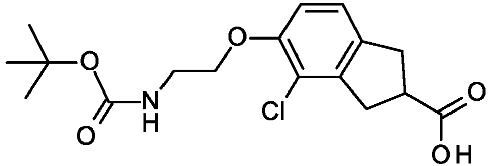

To a solution of ethyl 4-chloro-5-[2-[(2-methylpropan-2-yl)oxycarbonylamino]ethoxy]-2,3-dihydro-1*H*-indene-2-carboxylate (Intermediate 74, step 4, 350.0 mg, 0.910 mmol, 1 eq) in THF (5 mL) and water (5 mL) was added lithium hydroxide (109.2 mg, 4.56 mmol, 5 eq) at 0 °C. After addition, the mixture was stirred at 20 °C for 16 h. The reaction mixture was diluted with EtOAc (25 mL) and filtered. The filtrate was concentrated and then the pH was was adjusted to 2 by addition of HCl (1 M). The precipitate was filtered, dried under vacuum to give the title compound (220 mg, 0.620 mmol, 67.8% yield) as a white solid. MS (ESP): m/z = 378.1 [M+Na]⁺.

### Step 2:

### 2-Trimethylsilylethyl N-[4-chloro-5-[2-[(2-methylpropan-2-yl)oxycarbonylamino]ethoxy]-2,3-dihydro-1H-inden-2-yl]carbamate

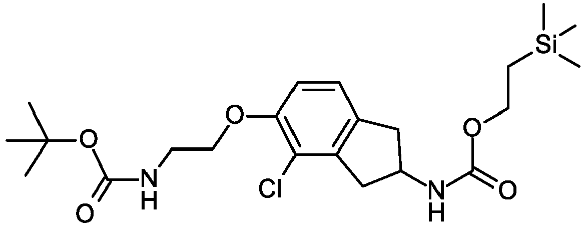

The title compound was prepared by analogy to 2-trimethylsilylethyl *N*-(4-methyl-6,7-dihydro-5*H*-cyclopenta[b]pyridin-6-yl)carbamate (Intermediate 15, step 6) using 4-chloro-5-[2-[(2-methylpropan-2-yl)oxycarbonylamino]ethoxy]-2,3-dihydro-1*H*-indene-2-carboxylic acid in place of 4-methyl-6,7-dihydro-5*H*-cyclopenta[b]pyridine-6-carboxylic acid and was obtained as a colorless oil. MS (ESP): m/z = 471.2 [M+H]⁺.

### Step 3:

### tert-Butyl N-[2-[(2-amino-4-chloro-2,3-dihydro-1H-inden-5-yl)oxy]ethyl]carbamate

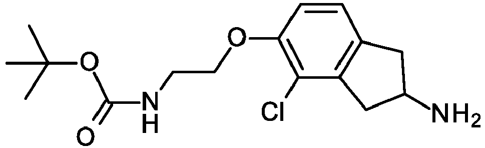

To a solution of 2-trimethylsilylethyl *N*-[4-chloro-5-[2-[(2-methylpropan-2-yl)oxycarbonylamino]ethoxy]-2,3-dihydro-1*H*-inden-2-yl]carbamate (150.0 mg, 0.320 mmol, 1 eq) in THF (10 mL) was added TBAF (0.96 mL, 0.960 mmol, 3 eq) and the solution was stirred at 50 °C for 16 h. The solvent was removed to give a residue which was dissolved in water (10 mL) and then extracted with DCM (3 × 15 mL). The combined extracts were dried over Na₂SO₄ and concentrated under vacuum to give the crude title compound (450 mg) as a light brown oil. The crude product was used for the next step directly.

### Intermediate 77

### tert-Butyl N-[1-[(2-amino-4-chloro-2,3-dihydro-1H-inden-5-yl)oxymethyl]cyclopropyl]carbamate

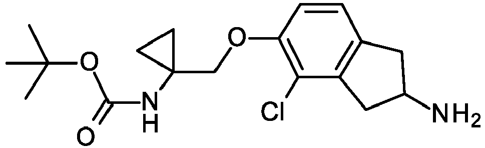

The title compound was prepared by analogy to *tert*-butyl *N*-[2-[(2-amino-4-chloro-2,3-dihydro-1*H*-inden-5-yl)oxy]ethyl]carbamate (Intermediate 76) using ethyl 4-chloro-5-[[1-[(2-methylpropan-2-yl)oxycarbonylamino]cyclopropyl]methoxy]-2,3-dihydro-1*H*-indene-2-carboxylate (Intermediate 75, step 1) in place of ethyl 4-chloro-5-[2-[(2-methylpropan-2-yl)oxycarbonylamino]ethoxy]-2,3-dihydro-1*H*-indene-2-carboxylate and was obtained as a light brown oil. MS (ESP): m/z = 353.2 [M+H]⁺.

### Intermediate 78

### tert-Butyl N-(2-aminoethyl)-N-[5-oxo-1-[3-oxo-4-(2-trimethylsilylethoxymethyl)pyrido[3,2-b][1,4]oxazin-6-yl]pyrrolidin-3-yl]carbamate

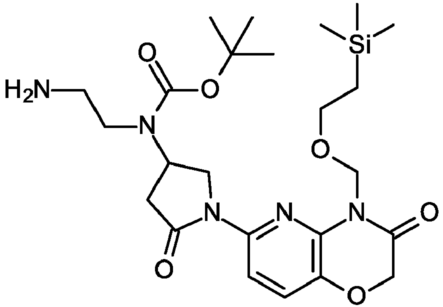

### Stes 1 and 2:

### 6-(4-Amino-2-oxopyrrolidin-1-yl)-4-(2-trimethylsilylethoxymethyl)pyrido[3,2-b][1,4]oxazin-3-one

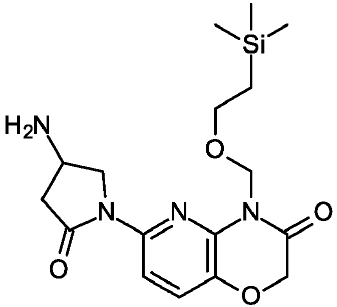

The title compound was prepared by analogy to 6-[(4*R*)-4-amino-2-oxopyrrolidin-1-yl]-4-(2-trimethylsilylethoxymethyl)pyrido[3,2-b][1,4]oxazin-3-one (Intermediate 13, steps 1 and 2) using *tert*-butyl *N*-(5-oxopyrrolidin-3-yl)carbamate (CAS# 1245648-84-3) in place of *tert-butyl N*-[(3*R*)-5-oxopyrrolidin-3-yl]carbamate and was obtained as a brown oil. MS (ESP): m/z = 379.2 [M+H]⁺.

### Step 3:

### 2-[[5-Oxo-1-[3-oxo-4-(2-trimethylsilylethoxymethyl)pyrido[3,2-b][1,4]oxazin-6-yl]pyrrolidin-3-yl] amino] acetonitrile

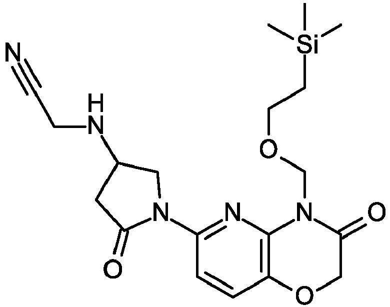

To a solution 6-(4-amino-2-oxopyrrolidin-1-yl)-4-(2-trimethylsilylethoxymethyl)pyrido[3,2-b][1,4]oxazin-3-one (5.15 g, 13.6 mmol, 1 eq) in *N*-methyl-2-pyrrolidinone (149 mL) was added 2-chloroacetonitrile (1.03 g, 859 µl, 13.6 mmol, 1 eq). This solution was then stirred at 100 °C for 1.5 h. Then the temperature was raised to 135 °C for 45 min. Further 2-chloroacetonitrile (514 mg, 429 µl, 6.8 mmol, 0.5 eq) was added and the mixture was stirred for 1 h. Potassium carbonate (940 mg, 6.8 mmol, 0.5 eq) was added. The reaction mixture was poured onto water and the mixture was extracted with TBME (x 2). The organic phases were combined, washed with brine and evaporated. The resulting residue was purified by silica column chromatography (DCM to 5% MeOH in DCM) to give the title compound (1.13 g, 2.71 mmol, 19.9 % yield) as a brown oil. MS (ESP): m/z = 418.0 [M+H]⁺.

### Step 4:

### tert-Butyl N-(cyanomethyl)-N-[5-oxo-1-[3-oxo-4-(2-trimethylsilylethoxymethyl)pyrido[3,2-b][1,4]oxazin-6-yl]pyrrolidin-3-yl]carbamate

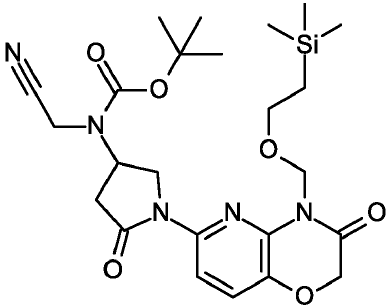

2-[[5-Oxo-1-[3-oxo-4-(2-trimethylsilylethoxymethyl)pyrido[3,2-b][1,4]oxazin-6-yl]pyrrolidin-3-yl]amino]acetonitrile (1.02 g, 2.44 mmol, 1 eq) was dissolved in THF (12.2 mL). Boc-Anhydride (800 mg, 851 µl, 3.66 mmol, 1.5 eq) and Et₃N (247 mg, 340 µl, 2.44 mmol, 1 eq) were added and the mixture stirred at 50°C for 16 h. Further Boc-Anhydride (800 mg, 851 µl, 3.66 mmol, 1.5 eq) was added and the mixture stirred for 2 h. The mixture was cooled to RT and evaporated. The crude product was purified by silica cartridge chromatography (ISCO 70 g, from heptane to 50% EtOAc/heptane) to give the title compound (670 mg, 1.29 mmol, 53 % yield) as an orange foam. MS (ESP): m/z = 518.2 [M+H]⁺.

### Step 5:

### tert-Butyl N-(2-aminoethyl)-N-[5-oxo-1-[3-oxo-4-(2-trimethylsilylethoxymethyl)pyrido[3,2-b][1,4]oxazin-6-yl]pyrrolidin-3-yl]carbamate

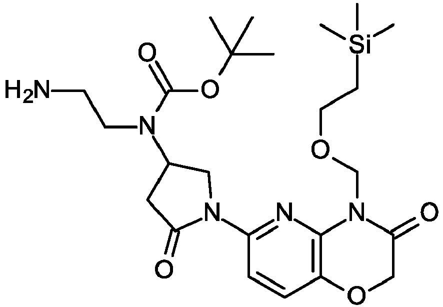

To a solution of *tert*-butyl *N*-(cyanomethyl)-N-[5-oxo-1-[3-oxo-4-(2-trimethylsilylethoxymethyl)pyrido[3,2-b][1,4]oxazin-6-yl]pyrrolidin-3-yl]carbamate (100 mg, 193 µmol, 1 eq) in methanol (12 mL) was added nickel (II) chloride (1.25 mg, 9.66 µmol, 0.05 eq) at 0°C. Then sodium borohydride (27.8 mg, 734 µmol, 3.8 eq) was added portionwise (3 portions in 5 minutes) and the mixture stirred for 2 h at 0 °C. Further sodium borohydride (27.8 mg, 734 µmol, 3.8 eq) was added again in 3 portions and the mixture stirred for 2 h at 0 °C. Water was added and the mixture was extracted with CH₂Cl₂. The combined extracts were washed with brine, dried over Na₂SO₄, filtered and evaporated to give 110 mg brown solid which was dissolved in CH₂Cl₂ (5 mL) and filtered through a syringe filter. The filtrate was evaporated to give the title compound (96 mg, 184 µmol, 95.3 % yield) as a light green foam. MS (ESP): m/z = 522.4 [M+H]⁺.

### Intermediate 79

### tert-Butyl N-[1-[(1-chloro-6-formyl-6,7-dihydro-SH-cyclopenta[c]pyridin-3-yl)oxymethyl]cyclopropyl]carbamate

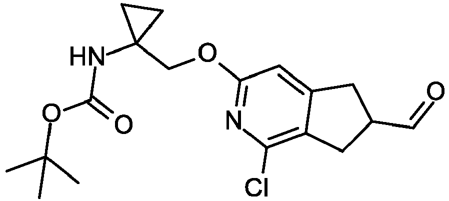

### Step 1:

### Dimethyl 1-chloro-3-oxo-5,7-dihydro-2H-cyclopenta[c]pyridine-6,6-dicarboxylate

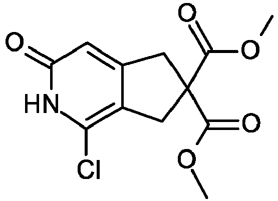

To a solution of dimethyl 1-chloro-3-methoxy-5,7-dihydrocyclopenta[c]pyridine-6,6-dicarboxylate (Intermediate 37, step 5, 20.0 mg, 0.070 mmol, 1 eq) in MeCN (2 mL) were added trimethylchlorosilane (72.5 mg, 0.670 mmol, 10 eq) and sodium iodide (100.03 mg, 0.670 mmol, 10 eq) and the mixture was stirred at 25 °C for 16 h. The mixture was quenched with brine (10 mL) and then extracted with DCM (20 mL). The organic extracts were dried over Na₂SO₄ and concentrated under vacuum to give crude title compound (20 mg, 0.070 mmol, 104.9% yield) as a light brown solid.

### Step 2:

### Methyl 1-chloro-3-oxo-2,5,6,7-tetrahydrocyclopenta[c]pyridine-6-carboxylate

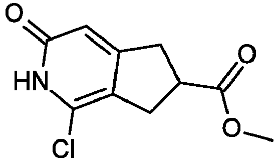

A mixture of dimethyl 1-chloro-3-oxo-5,7-dihydro-2H-cyclopenta[c]pyridine-6,6-dicarboxylate (150.0 mg, 0.550 mmol, 1 eq) in HCl/H₂O (2.1 mL, 25.24 mmol, 45.71 eq) was stirred at 100 °C for 5 h. The mixture was concentrated and the residue was dissolved in HCl/MeOH (2.1 mL, 8.41 mmol, 15.24 eq) and then stirred at 80 °C for 2 h. After 2 h, the mixture was concentrated under vacuum to give the crude product. Saturated NaHCO₃ solution (15 mL) was added and the mixture was extracted with EtOAc (2 × 15 mL). The combined organic extracts were concentrated under vacuum to give the crude product (100 mg). The crude product was purified by chromatography column on silica gel (PE/EtOAc=3/1-1/2) to give the title compound (70 mg, 0.310 mmol, 58.6% yield) as a light brown solid. MS (ESP): m/z = 228.0 [M+H]⁺.

### Step 3:

### Methyl 1-chloro-3-[[1-[(2-methylpropan-2-yl)oxycarbonylamino]cyclopropyl]methoxy]-6,7-dihydro-5H-cyclopenta[c]pyridine-6-carboxylate

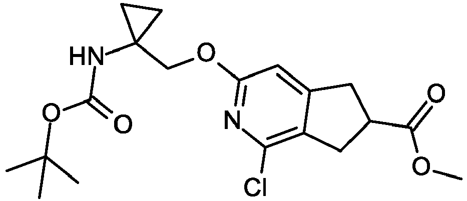

To a mixture of methyl 1-chloro-3-oxo-2,5,6,7-tetrahydrocyclopenta[c]pyridine-6-carboxylate (40.0 mg, 0.180 mmol, 1 eq) and *tert*-butyl *N*-[1-(bromomethyl)cyclopropyl]carbamate (2.48 mL, 0.350 mmol, 2 eq) in MeCN (0.400 mL) was added Cs₂CO₃ (114.5 mg, 0.350 mmol, 2 eq) and then the reaction mixture was stirred at 70 °C for 2 h. The mixture was filtered and the filtrate was concentrated under vacuum to give the crude product. The crude product was purified by preparative TLC (PE/EtOAc=3/1) to give the title compound (30 mg, 0.080 mmol, 43.0% yield) as a white solid. MS (ESP): m/z = 397.2 [M+H]⁺.

### Steps 4 and 5:

### tert-Butyl N-[1-[(1-chloro-6-formyl-6,7-dihydro-5H-cyclopenta[c]pyridin-3-yl)oxymethyl]cyclopropyl]carbamate

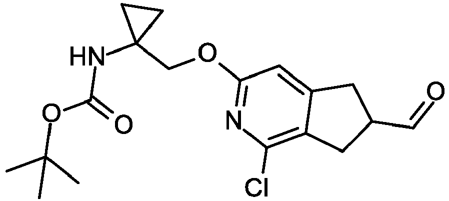

The title compound was prepared by analogy to 4-fluoro-1-methyl-6,7-dihydro-5*H-*cyclopenta[c]pyridine-6-carbaldehyde (Intermediate 27, steps 9 and 10) and was obtained as a light brown solid. MS (ESP): m/z = 367.2 [M+H]⁺.

### Intermediate 80

### tert-butyl N-[(2-methylpropan-2-yl)oxycarbonyl]-N-[rac-5,6-trans-1-chloro-6-fonnyl-6,7-dihydro-5H-cyclopenta[c]pyridin-5-yl]carbamate

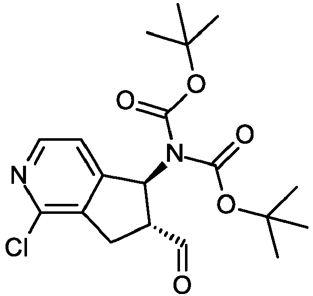

### Step 1:

### rac-5,6-cis-6-[[tert-Butyl(diphenyl)silyl]oxymethyl]-1-chloro-6,7-dihydro-5H-cyclopenta[c]pyridin-5-ol and rac-5,6-trans-6-[[tert-butyl(diphenyl)silyl]oxymethyl]-1-chloro-6,7-dihydro-5H-cyclopenta[c]pyridin-5-ol

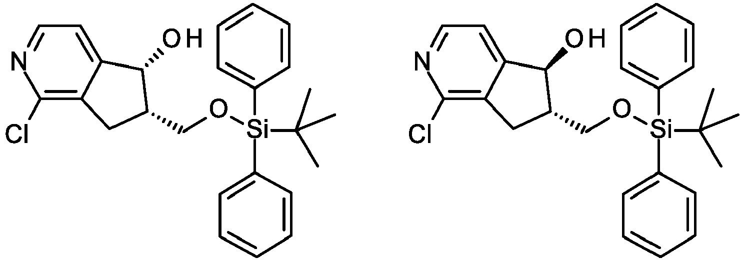

To a solution of [6-[[*tert*-butyl(diphenyl)silyl]oxymethyl]-1-chloro-6,7-dihydro-5*H-*cyclopenta[c]pyridin-5-yl] acetate (Intermediate 30, step 3, 500.0 mg, 1.04 mmol, 1 eq) in THF (10 mL) and water (10 mL) was added LiOH.H₂O (218.51 mg, 5.21 mmol, 5 eq) at 0°C and then the reaction was stirred at 25 °C for 3 h. To the mixture were added EtOAc (30 mL) and water (20 mL), then the organic layer was separated, dried over Na₂SO₄ and concentrated under vacuum to give the crude product. The crude product was purified by preparative TLC (PE/EtOAc=3/1) to give *rac*-5,6-*trans*-6-[[*tert*-butyl(diphenyl)silyl]oxymethyl]-1-chloro-6,7-dihydro-5*H*-cyclopenta[c]pyridin-5-ol (130 mg, 0.300 mmol, 18.6% yield) and *rac*-5,6-*cis*-6-[[*tert*-butyl(diphenyl)silyl]oxymethyl]-1-chloro-6,7-dihydro-5*H*-cyclopenta[c]pyridin-5-ol (200 mg, 0.460 mmol, 43.8% yield) both as white solids. MS (ESP): m/z = 438.1 [M+H]⁺ for both products.

### Step 2:

### tert-Butyl-diphenyl-[(rac-5,6-trans-5-azido-1-chloro-6,7-dihydro-5H-cyclopenta[c]pyridin-6-yl)methoxy] silane

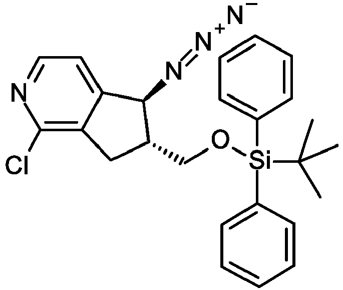

To a mixture of *rac*-5,6-*cis*-6-[[*tert*-butyl(diphenyl)silyl]oxymethyl]-1-chloro-6,7-dihydro-5*H-*cyclopenta[c]pyridin-5-ol (200.0 mg, 0.460 mmol, 1 eq) in toluene (13.33 mL) was added diphenylphosphonic azide (0.3 mL, 1.37 mmol, 3 eq) and the mixture was stirred at 25 °C for 5 min. 1,8-diazabicyclo[5.4.0]undec-7-ene (0.2 mL, 1.37 mmol, 3 eq) was added and the mixture was stirred at 25 °C for 10 min and then heated to 70 °C for 16 h. The mixture was quenched with brine (20 mL) and extracted with EtOAc (2 × 20 mL). The combined organic layers were dried over Na₂SO₄ and concentrated under vacuum to give the title compound (110 mg, 0.240 mmol, 52.0% yield) as a colourless oil. MS (ESP): m/z = 463.2 [M+H]⁺.

### Step 3:

### rac-5,6-trans-6-[[tert-Butyl(diphenyl)silyl]oxymethyl]-1-chloro-6,7-dihydro-5H-cyclopenta[c]pyridin-5-amine

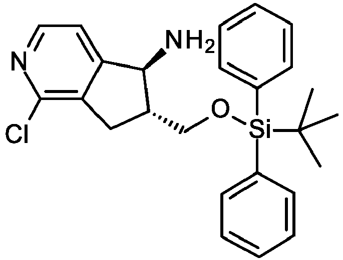

A mixture of *tert*-butyl-diphenyl-[(*rac*-5,6-*trans*-5-azido-1-chloro-6,7-dihydro-5*H-*cyclopenta[c]pyridin-6-yl)methoxy]silane (130.0 mg, 0.280 mmol, 1 eq), ZnBr₂ (62.26 mg, 0.280 mmol, 1 eq) and Pd/C ( mg, 0.420 mmol, 1.5 eq) in ethyl acetate (10 mL) was stirred at 25 °C for 16 h under an atmosphere of hydrogen. The mixture was filtered and the filtrate was concentrated under vacuum to give the crude title compound (100 mg, 0.230 mmol, 81.5% yield) as a white solid. MS (ESP): m/z = 437.2 [M+H]⁺.

### Step 4:

### tert-Butyl N-[(2-methylpropan-2-yl)oxycarbonyl]-N-[rac-5,6-trans-1-chloro-6-(hydroxymethyl)-6,7-dihydro-5H-cyclopenta[c]pyridin-5-yl]carbamate

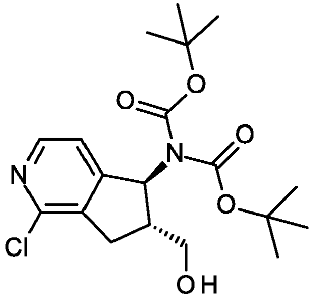

To a mixture of *rac*-5,6-*trans*-6-[[*tert*-butyl(diphenyl)silyl]oxymethyl]-1-chloro-6,7-dihydro-5*H-*cyclopenta[c]pyridin-5-amine (60.0 mg, 0.140 mmol, 1 eq), 4-dimethylaminopyridine (4.0 mg, 0.030 mmol, 0.240 eq) and triethylamine (0.1 mL, 0.690 mmol, 5 eq) in DCM (3 mL) was added di-t-butyldicarbonate (89.89 mg, 0.410 mmol, 3 eq) and the mixture was stirred at 25 °C for 16 h. The mixture was diluted with DCM (20 mL) and then washed with NaHCO₃ solution (2 × 15 mL), dried over Na₂SO₄ and concentrated under vacuum to give a residue. The residue was dissolved in THF (3 mL), TBAF in THF (0.37 mL, 0.370 mmol, 2 eq) was added and the mixture was stirred at 25 °C for 16 h. The mixture was quenched with brine (10 mL) then extracted with EtOAc (2 × 20 mL). The combine extracts were dried over Na₂SO₄ and concentrated under vacuum to give a residue. The residue was purified by preparative TLC (PE/EtOAc=1/1) to give the title compound (30 mg, 0.080 mmol, 40.4% yield) as a white solid. MS (ESP): m/z = 399.1 [M+H]⁺.

### Step 5:

### tert-butyl N-[(2-methylpropan-2-yl)oxycarbonyl]-N-[rac-5,6-trans-1-chloro-6-formyl-6,7-dihydro-5H-cyclopenta[c]pyridin-5-yl]carbamate

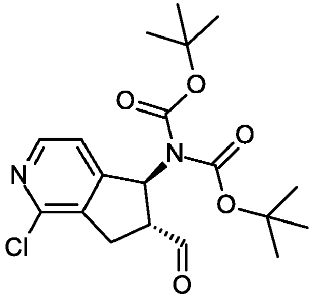

The title compound was prepared by analogy to 4-fluoro-1-methyl-6,7-dihydro-5*H-*cyclopenta[c]pyridine-6-carbaldehyde (Intermediate 27, step 10). MS (ESP): m/z = 397.2 [M+H]⁺.

### Intermediate 81

### tert-Butyl N-[1-[(4-cyano-2-formyl-2,3-dihydro-1H-inden-5-yl)oxymethyl]cyclopropyl]carbamate

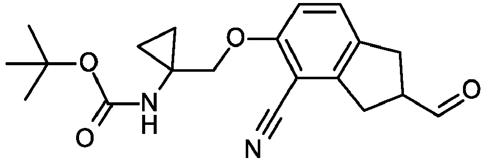

### Steps 1 and 2:

### tert-Butyl N-[1-[[4-bromo-2-(hydroxymethyl)-2,3-dihydro-1H-inden-5-yl] oxymethyl] cyclopropyl] carbamate

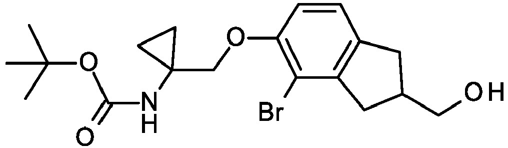

The title compound was prepared by analogy to tert-Butyl *N*-[1-[[4-chloro-2-(hydroxymethyl)-2,3-dihydro-1*H*-inden-5-yl]oxymethyl]cyclopropyl]carbamate (Intermediate 75, steps 1 and 2) using ethyl 4-bromo-5-hydroxy-2,3-dihydro-1*H*-indene-2-carboxylate (Intermediate 36, step 3) in place of ethyl 4-chloro-5-hydroxy-2,3-dihydro-1*H*-indene-2-carboxylate and was obtained as a colorless oil.

### Step 3:

### tert-Butyl N-[1-[[4-bromo-2-[[tert-butyl(dimethyl)silyl]oxymethyl]-2,3-dihydro-1H-inden-5-yl]oxymethyl]cyclopropyl]carbamate

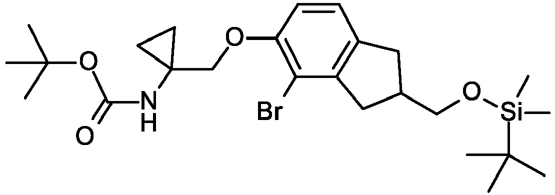

To a solution of *tert*-butyl *N*-[1-[[4-bromo-2-(hydroxymethyl)-2,3-dihydro-1*H*-inden-5-yl]oxymethyl]cyclopropyl]carbamate (67.47 mL, 1.09 mmol, 1 eq) in DCM (150 mL) was added triethylamine (0.21 mL, 1.52 mmol, 1.39 eq) at 0°C. The mixture was stirred at this temperature for 15 min. Then *tert*-butyldimethylsilyl chloride (0.33 g, 2.18 mmol, 2 eq) and 4-dimethylaminopyridine (1.88 mg, 0.020 mmol, 0.010 eq) in DCM (50 mL) were slowly added. The mixture was stirred at 25 °C for 24 h. The white precipitate was filtered off and the filtrate was concentrated to give the crude product which was purified by chromatography on silica gel (PE) to give the title compound (450 mg, 0.850 mmol, 78.3% yield) as a colorless oil. MS (ESP): m/z = 548.2 [M+Na]⁺.

### Step 4:

### tert-Butyl N-[1-[[2-[[tert-butyl(dimethyl)silyl]oxymethyl]-4-cyano-2,3-dihydro-1H-inden-5-yl]oxymethyl]cyclopropyl]carbamate

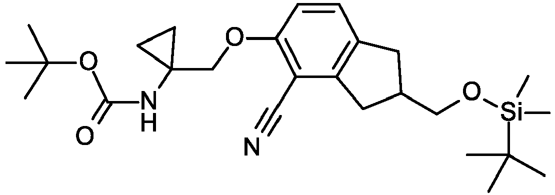

The title compound was prepared by analogy to tert-butyl 2-[[4-cyano-2-(hydroxymethyl)-2,3-dihydro-1*H*-inden-5-yl]oxymethyl]pyrrolidine-1-carboxylate (Intermediate 38, step 3) and was obtained as a yellow oil. MS (ESP): m/z = 495.2 [M+Na]⁺.

### Steps 5 and 6:

### tert-Butyl N-[1-[(4-cyano-2-formyl-2,3-dihydro-1H-inden-5-yl)oxymethyl]cyclopropyl]carbamate

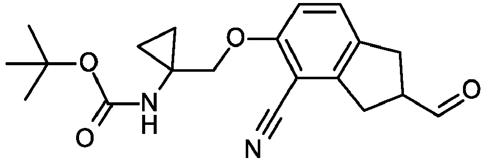

The title compound was prepared by analogy to 1-chloro-4-methoxy-6,7-dihydro-5*H-*cyclopenta[c]pyridine-6-carbaldehyde (Intermediate 43, steps 3 and 4) and was obtained as a yellow oil.

### Intermediate 82

### tert-Butyl N-[1-[(1-cyano-6-formyl-6,7-dihydro-5H-cyclopenta[c]pyridin-3-yl)oxymethyl]cyclopropyl]carbamate

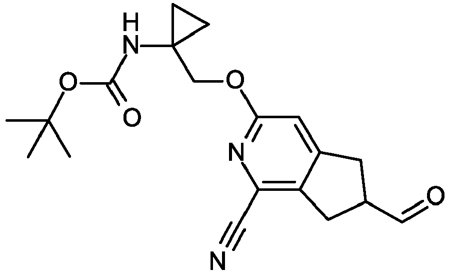

### Step 1:

The title compound was prepared by analogy to *tert*-butyl 2-[[4-cyano-2-(hydroxymethyl)-2,3-dihydro-1*H*-inden-5-yl]oxymethyl]pyrrolidine-1-carboxylate (Intermediate 37, step 3) using *tert-*butyl *N*-[1-[[1-chloro-6-(hydroxymethyl)-6,7-dihydro-5*H*-cyclopenta[c]pyridin-3-yl]oxymethyl]cyclopropyl]carbamate (Intermediate 79, step 4) in place of *tert-butyl* 2-[[4-bromo-2-(hydroxymethyl)indan-5-yl]oxymethyl]pyrrolidine-1-carboxylate and was obtained as a white solid. MS (ESP): m/z = 382.2 [M+H]⁺.

### Step 2:

### tert-Butyl N-[1-[(1-cyano-6-formyl-6,7-dihydro-5H-cyclopenta[c]pyridin-3-yl)oxymethyl]cyclopropyl]carbamate

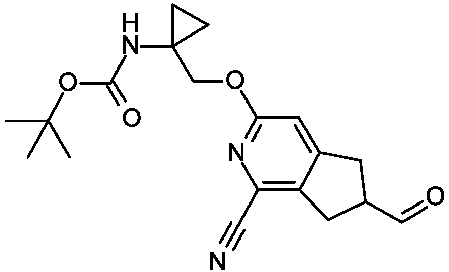

The title compound was prepared by analogy to 4-fluoro-1-methyl-6,7-dihydro-5*H-*cyclopenta[c]pyridine-6-carbaldehyde (Intermediate 27, step 10) and was obtained as a white solid.

### Intermediate 83

### tert-Butyl N-[2-[(1-chloro-6-formyl-6,7-dihydro-5H-cyclopenta[c]pyridin-3-yl)oxy] ethyl] carbamate

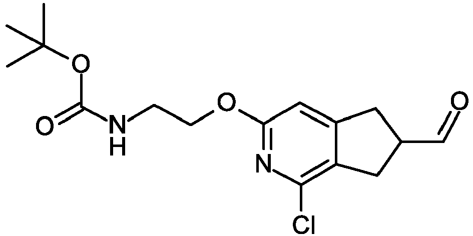

The title compound was prepared by analogy to *tert*-butyl *N*-[1-[(1-chloro-6-formyl-6,7-dihydro-5*H*-cyclopenta[c]pyridin-3-yl)oxymethyl]cyclopropyl]carbamate (Intermediate 79) using *tert-*butyl *N*-(2-bromoethyl)carbamate in place of *tert*-butyl *N*-[1-(bromomethyl)cyclopropyl]carbamate in step 3 and was obtained as a white solid. MS (ESP): m/z = 363.1 [M+Na]⁺.

### Intermediate 84

### tert-Butyl N-[2-[(1-cyano-6-formyl-6,7-dihydro-5H-cyclopenta[c]pyridin-3-yl)oxy]ethyl]carbamate

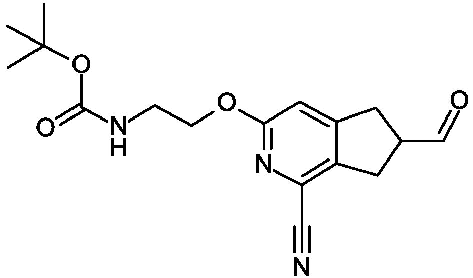

The title compound was prepared by analogy to *tert*-butyl *N*-[1-[(1-cyano-6-formyl-6,7-dihydro-5*H*-cyclopenta[c]pyridin-3-yl)oxymethyl]cyclopropyl]carbamate (Intermediate 82) using *tert-*butyl *N*-[2-[[1-chloro-6-(hydroxymethyl)-6,7-dihydro-5*H*-cyclopenta[c]pyridin-3-yl]oxy]ethyl]carbamate (Intermediate 83, step 4) in place of *tert*-butyl *N*-[1-[[1-chloro-6-(hydroxymethyl)-6,7-dihydro-5*H*-cyclopenta[c]pyridin-3-yl]oxymethyl]cyclopropyl]carbamate and was obtained as a light brown oil. MS (ESP): m/z = 276.1 [M+H-tBu]⁺.

### Intermediate 85

### tert-Butyl N-[2-[(4-cyano-2-formyl-2,3-dihydro-1H-inden-5-yl)oxy]ethyl]carbamate

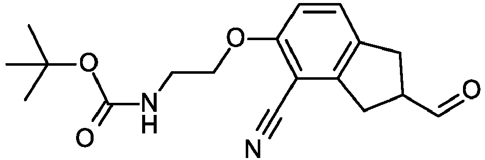

The title compound was prepared by analogy to 2-[(4-cyano-2-formyl-2,3-dihydro-1*H*-inden-5-yl)oxy]-N-methylacetamide (Intermediate 36, steps 4 to 7) using *tert*-butyl *N*-(2-bromoethyl)carbamate in place of 2-chloro-*N*-methylacetamide in the first step and was obtained as a yellow oil.

### Intermediate 86

### tert-Butyl 3-[(4-cyano-2-formyl-2,3-dihydro-1H-inden-5-yl)oxymethyl] azetidine-1-carboxylate

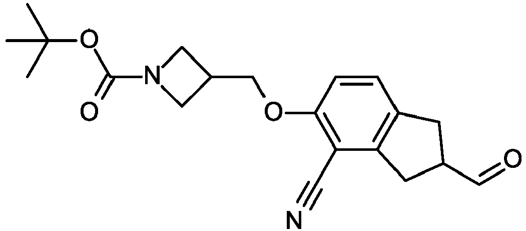

The title compound was prepared by analogy to 2-[(4-cyano-2-formyl-2,3-dihydro-1*H*-inden-5-yl)oxy]-*N*-methylacetamide (Intermediate 36, steps 4 to 7) using *tert*-butyl 3-(bromomethyl)azetidine-1-carboxylate (CAS# 253176-93-1) in place of 2-chloro-*N-*methylacetamide in step 4 and was obtained as a yellow oil.

### Intermediate 87

### tert-Butyl N-[rac-5,6-trans-6-formyl-1-methyl-6,7-dihydro-5H-cyclopenta[c]pyridin-5-yl]carbamate

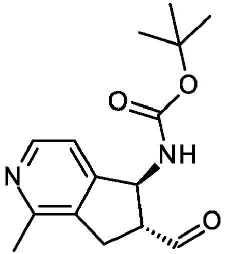

### Step 1:

### tert-Butyl N-[rac-5,6-trans-6-[[tert-butyl(diphenyl)silyl]oxymethyl]-1-chloro-6,7-dihydro-5H-cyclopenta[c]pyridin-5-yl] carbamate

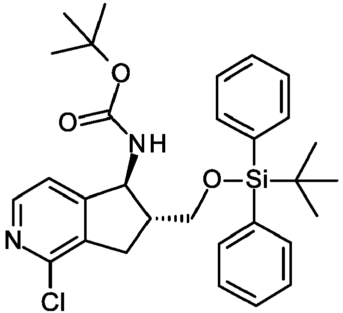

To a mixture of *rac*-5,6-t*rans*-6-[[*tert*-butyl(diphenyl)silyl]oxymethyl]-1-chloro-6,7-dihydro-5*H-*cyclopenta[c]pyridin-5-amine (Intermediate 80, step 3, 180.0 mg, 0.410 mmol, 1 eq) and triethylamine (0.11 mL, 0.820 mmol, 2 eq) in DCM (5 mL) was added di-t-butyldicarbonate (179.77 mg, 0.820 mmol, 2 eq) and the mixture was stirred at 25 °C for 16 h. The mixture was diluted with DCM (20 mL) and then washed with NaHCO₃ (2 × 10 mL), dried over Na₂SO₄, concentrated under vacuum to give the crude title compound (150 mg, 0.280 mmol, 67.8% yield) as a light yellow waxy solid. MS (ESP): m/z = 537.3 [M+H]⁺.

### Step 2:

### tert-Butyl N-[rac-5,6-trans-6-[[tert-butyl(diphenyl)silyl]oxymethyl]-1-methyl-6,7-dihydro-5H-cyclopenta[c]pyridin-5-yl]carbamate

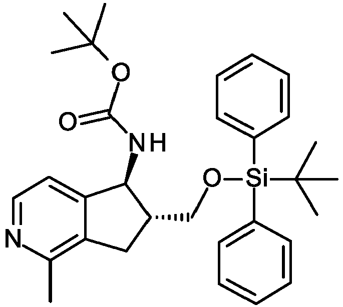

To a solution of *tert*-butyl *N*-[*rac*-5,6-*trans*-6-[[*tert*-butyl(diphenyl)silylloxymethyl]-1-chloro-6,7-dihydro-5*H*-cyclopenta[c]pyridin-5-yl]carbamate (100.0 mg, 0.190 mmol, 1 eq) in 1,4-dioxane (4 mL) and water (0.200 mL) were added K₂CO₃ (77.07 mg, 0.560 mmol, 3 eq), trimethylboroxine (467.38 mg, 3.72 mmol, 20 eq) and [1,1'-bis(diphenylphosphino)ferrocene]dichloropalladium(II) (13.62 mg, 0.020 mmol, 0.100 eq). The mixture was stirred for 16 h at 100 °C under N₂. The reaction was concentrated to dryness and the residue was taken up in EtOAc (20 mL) and the mixture was washed with saturated brine solution (2 × 20 mL). The organic layer was then dried (MgSO₄) and concentrated to dryness. The crude product was then purified by prep-TLC (PE/EtOAc=1/1) to give the title compound (60 mg, 0.120 mmol, 55.5 % yield) as a white solid. MS (ESP): m/z = 517.4 [M+H]⁺.

### Steps 3 and 4:

### tert-Butyl N-[rac-5,6-trans-6-formyl-1-methyl-6,7-dihydro-5H-cyclopenta[c]pyridin-5-yl] carbamate

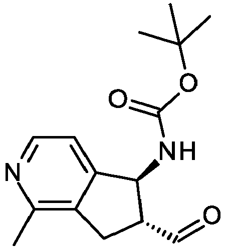

The title compound was prepared by analogy to [*rac*-(5,6-*trans*)-1-chloro-6-formyl-6,7-dihydro-5*H*-cyclopenta[c]pyridin-5-yl] acetate (Intermediate 30, steps 4 and 5) and was obtained as a light brown solid.

### Intermediate 88

### tert-Butyl N-[rac-1,2-trans-4-cyano-2-formyl-2,3-dihydro-1H-inden-1-yl]carbamate

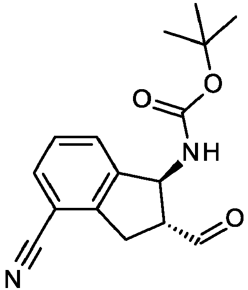

### Step 1:

### Ethyl 7-chloro-3-oxo-1,2-dihydroindene-2-carboxylate

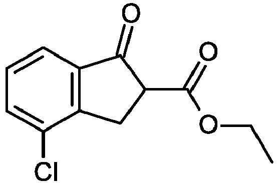

The title compound was prepared by analogy with Intermediate 50, step 1 from 4-chloro-2,3-dihydroinden-1-one (CAS# 15115-59-0) and was obtained as a white solid.

### Step 2:

### Ethyl 3-amino-7-chloro-1H-indene-2-carboxylate

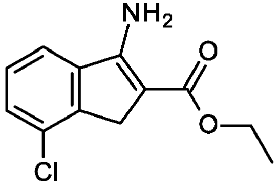

A mixture of ethyl 4-chloro-1-oxo-indane-2-carboxylate (10000.0 mg, 41.9 mmol, 1 eq) and ammonium acetate (32296.5 mg, 419.0 mmol, 10 eq) in ethanol (600 mL) was stirred at 80 °C for 16 h. The mixture was poured into saturated NH₄Cl (1500 mL) and extracted with ethyl acetate (600 mL × 3). The extracts were washed with brine (500 mL), dried over anhydrous Na₂SO₄ and concentrated in vacuo. The remaining residue was then purified by flash chromatography (25% ethyl acetate/petroleum ether) to afford the title compound (9 g, 37.87 mmol, 81.3% yield) as a grey solid. MS (ESP): m/z = 238.5 [M+H]⁺.

### Step 3:

### Ethyl rac-1,2-trans-1-amino-4-chloro-2,3-dihydro-1H-indene-2-carboxylate

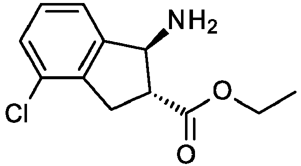

To a solution of ethyl 3-amino-7-chloro-1*H*-indene-2-carboxylate (9000.0 mg, 37.87 mmol, 1 eq) in THF (250 mL) was added trifluoroacetic acid (50.0 mL, 649.0 mmol, 17.1 eq) at 0 °C in portions over a period of 30 min. After 10 min. sodium borohydride (2148.8 mg, 56.8 mmol, 1.5 eq) was added at 0 °C in portions and the mixture was stirred for 2 h. The mixture was poured into saturated NaHCO₃ (500 mL) and extracted with ethyl acetate (500 mL × 2). The organic extracts were washed with brine, dried over anhydrous Na₂SO₄ and concentrated in vacuo to give the crude product which was used for next step without purification.

### Step 4:

### Ethyl rac-1,2-trans-4-chloro-1-[(2-methylpropan-2-yl)oxycarbonylamino]-2,3-dihydro-1H-indene-2-carboxylate

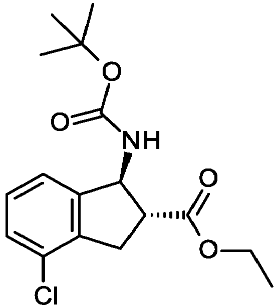

To a solution of the crude ethyl *rac*-1,2-*trans*-1-amino-4-chloro-2,3-dihydro-1*H*-indene-2-carboxylate (20000.0 mg, 83.44 mmol, 1 eq) in DCM (200 mL) were added triethylamine (23.26 mL, 166.88 mmol, 2 eq) and di-t-butyldicarbonate (21852.0 mg, 100.13 mmol, 1.2 eq) and the mixture was stirred at 25 °C for 16 h. The mixture was concentrated in vacuo and the remaining residue was purified by flash chromatography to afford the title compound (5.2 g, 15.3 mmol, 18.3% yield) as a yellow solid. MS (ESP): m/z = 339.1 [M+Na]⁺.

### Step 5:

### tert-Butyl N-[rac-1,2-trans-4-chloro-2-(hydroxymethyl)-2,3-dihydro-1H-inden-1-yl]carbamate

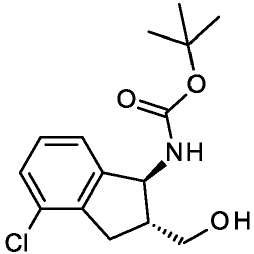

To a solution of ethyl *rac*-1,2-*trans*-4-chloro-1-[(2-methylpropan-2-yl)oxycarbonylamino]-2,3-dihydro-1*H*-indene-2-carboxylate (5200.0 mg, 15.3 mmol, 1 eq) in THF (132 mL) was added lithium aluminium hydride (1163.92 mg, 30.61 mmol, 2 eq) at -20 °C and the mixture stirred for 2 h. Water (1.2 mL), 10% aqueous NaOH (1.2 mL) and ethyl acetate (200 mL) were added into the reaction mixture at 0°C. The mixture was filtered and the filtrate was concentrated in vacuo to afford the title compound (4300 mg, 14.44 mmol, 90.6% yield) as an a off-white solid. MS (ESP): m/z = 320.5 [M+Na]⁺.

### Steps 6 and 7:

### tert-Butyl N-[rac-1,2-trans-4-cyano-2-formyl-2,3-dihydro-1H-inden-1-yl]carbamate

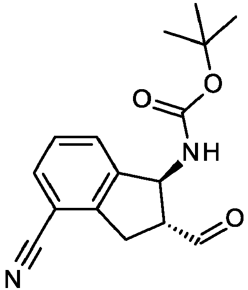

The title compound was prepared by analogy to 2-(2-oxoethyl)-2,3-dihydro-1*H*-indene-4-carbonitrile (Intermediate 22) as a light brown solid.

### Intermediate 89

### tert-Butyl 3-[(4-cyano-2-formyl-2,3-dihydro-1H-inden-5-yl)oxy] azetidine-1-carboxylate

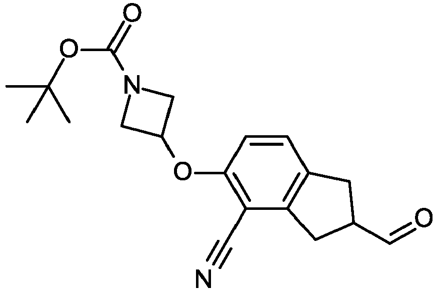

The title compound was prepared by analogy to 2-[(4-cyano-2-formyl-2,3-dihydro-1*H*-inden-5-yl)oxy]-*N*-methylacetamide (Intermediate 36, steps 4 to 7) using *tert*-butyl 3-bromoazetidine-1-carboxylate (CAS# 1064194-10-0) in place of 2-chloro-N-methylacetamide in the first step and was obtained as a yellow oil.

### Intermediate 90

### tert-Butyl N-[rac-5,6-trans-1-cyano-6-formyl-6,7-dihydro-5H-cyclopenta[c]pyridin-5-yl]carbamate

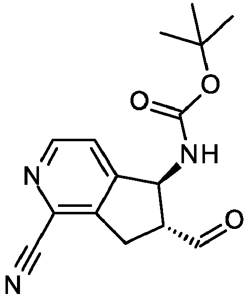

### Step 1:

### tert-Butyl N-[rac-5,6-trans-6-[[tert-butyl(diphenyl)silyl]oxymethyl]-1-cyano-6,7-dihydro-5H-cyclopenta[c]pyridin-5-yl]carbamate

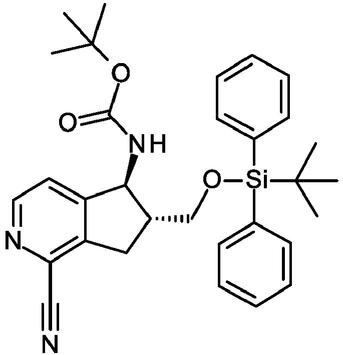

The title compound was prepared by analogy with 6-(hydroxymethyl)-3-methoxy-6,7-dihydro-5*H*-cyclopenta[c]pyridine-1-carbonitrile (Intermediate 39, step 1) using *tert*-butyl *N*-[*rac*-5,6-*trans*-6-[[*tert*-butyl(diphenyl)silyl]oxymethyl]-1-chloro-6,7-dihydro-5*H*-cyclopenta[c]pyridin-5-yl]carbamate (Intermediate 87, step 1) in place of (1-chloro-3-methoxy-6,7-dihydro-5*H-*cyclopenta[c]pyridin-6-yl)methanol and was obtained as a white solid. MS (ESP): m/z = 528.3 [M+H]⁺.

### Steps 2 and 3:

### tert-Butyl N-[rac-5,6-trans-1-cyano-6-formyl-6,7-dihydro-5H-cyclopenta[c]pyridin-5-yl] carbamate

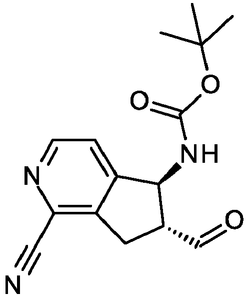

The title compound was prepared by analogy to [*rac*-(5,6-*trans*)-1-chloro-6-formyl-6,7-dihydro-5*H*-cyclopenta[c]pyridin-5-yl] acetate (Intermediate 30, steps 4 and 5) and was obtained as a light brown solid.

### Intermediate 91

### tert-Butyl N-[1-[(4-cyano-2-formyl-2,3-dihydro-1H-inden-5-yl)oxy]propan-2-yl]carbamate

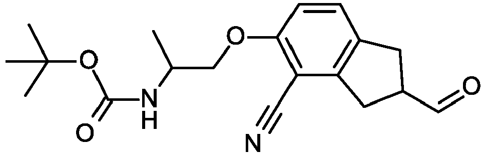

The title compound was prepared by analogy to 2-[(4-cyano-2-formyl-2,3-dihydro-1*H*-inden-5-yl)oxy]-*N*-methylacetamide (Intermediate 36, steps 4 to 7) using 2-[(2-methylpropan-2-yl)oxycarbonylamino]propyl methanesulfonate (CAS# 149602-63-1) in place of 2-chloro-*N-*methylacetamide in the first step and was obtained as a light yellow oil.

### Intermediate 92

### tert-Butyl N-(rac-1,2-trans-4-chloro-2-formyl-2,3-dihydro-1H-inden-1-yl)carbamate

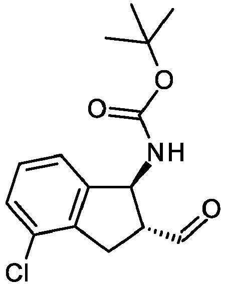

To a solution of tert-butyl *N*-[*rac*-1,2-*trans*-4-chloro-2-(hydroxymethyl)-2,3-dihydro-1*H*-inden-1-yl]carbamate (Intermediate 88, step 5, 100.0 mg, 0.340 mmol, 1 eq) in DCM (20 mL) was added Dess-Martin periodinane (184.3 mg, 0.440 mmol, 1.3 eq), and then the mixture was stirred at 25 °C for 2 h. The reaction was quenched with 10% aq. NaHCO₃ (10 mL). The mixture was extracted with DCM (10 mL × 2) and the combined organic layers were dried with Na₂SO₄. The solvent was removed to give the crude title compound (100 mg, 0.340 mmol, 100% yield) as a yellow oil.

### Intermediate 93

### tert-Butyl 2-[(4-cyano-2-formyl-2,3-dihydro-1H-inden-5-yl)oxymethyl] azetidine-1-carboxylate

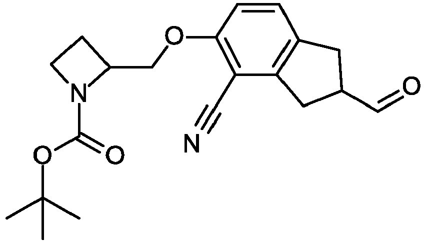

The title compound was prepared by analogy to 2-[(4-cyano-2-formyl-2,3-dihydro-1*H*-inden-5-yl)oxy]-*N*-methylacetamide (Intermediate 36, steps 4 to 7) using *tert*-butyl 2-[(4-methylphenyl)sulfonyloxymethyl]azetidine-1-carboxylate (CAS# 128542-76-7) in place of 2-chloro-*N*-methylacetamide in the first step and was obtained as a light yellow oil.

### Intermediate 94

### tert-Butyl N-[3-(2-aminoethyl)-5-oxo-1-(3-oxo-4H-pyrido[3,2-b][1,4]oxazin-6-yl)pyrrolidin-3-yl]carbamate

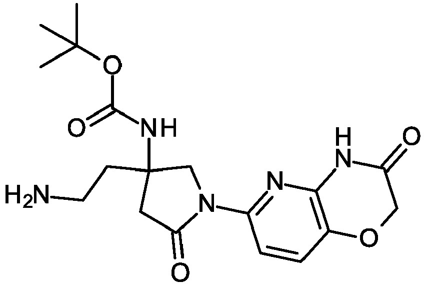

### Step 1:

### Methyl 2-[5-oxo-3-(phenylmethoxycarbonylamino)pyrrolidin-3-yl] acetate

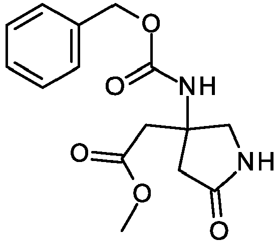

To a solution of 3-(2-methoxy-2-oxo-ethyl)-5-oxo-pyrrolidine-3-carboxylic acid (CAS# 362706-47-6, 2100.0 mg, 10.44 mmol, 1 eq) in toluene (116.67 mL) were added triethylamine (2.18 mL, 15.66 mmol, 1.5 eq) and diphenylphosphonic azide (2.47 mL, 11.48 mmol, 1.1 eq). The mixture was stirred at 110 °C for 4 h under N₂. Benzyl alcohol (1693.22 mg, 15.66 mmol, 1.5 eq) was added and the mixture stirred for 16 h. Water was added and the mixture was extracted with ethyl acetate. The combinrd organic layers were washed with brine, dried over anhydrous Na₂SO₄ and concentrated in vacuo to give a residue which was purified by flash chromatography to afford the title compound (500 mg, 1.63 mmol, 15.6% yield) as a colorless oil.

### Step 2:

### Benzyl N-[3-(2-hydroxyethyl)-5-oxopyrrolidin-3-yl]carbamate

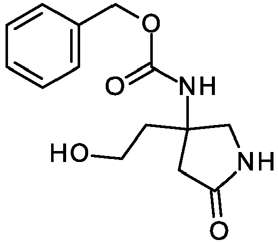

To a solution of methyl 2-[3-(benzyloxycarbonylamino)-5-oxo-pyrrolidin-3-yl]acetate (600.0 mg, 1.96 mmol, 1 eq) in THF (50 mL) was added lithium borohydride (128 mg, 5.88 mmol, 3 eq) at 25 °C and the mixture was stirred for 3 h. Saturated NH₄Cl was added and the mixture extracted with ethyl acetate. The combined organic layers were wahshed with brine, dried over anhydrous Na₂SO₄ and concentrated in vacuo to afford the title compound (500 mg, 1.8 mmol, 91.7% yield) as a colorless oil.

### Step 3:

### Benzyl N-[3-[2-[tert-butyl(dimethyl)silyl]oxyethyl]-5-oxopyrrolidin-3-yl]carbamate

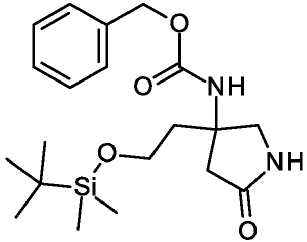

To a solution of benzyl *N*-[3-(2-hydroxyethyl)-5-oxo-pyrrolidin-3-yl]carbamate (500.0 mg, 1.8 mmol, 1 eq) in DCM (20 mL) were added imidazole (244.62 mg, 3.59 mmol, 2 eq) and *tert-*butyldimethylchlorosilane (406.18 mg, 2.69 mmol, 1.5 eq) and the mixture was stirred at 25°C for 16 h. Water was added and the mixture was extracted with DCM. The combined organic layers were concentrated and the remaining residue was purified by flash column chromatography to afford the title compound (500 mg, 1.27 mmol, 70.9% yield) as a colorless oil. ¹H-NMR (400 MHz, CDCl₃) δ 7.35 (m, 5H), 6.32 (br s, 1H), 5.67 (br s, 1H), 5.07 (s, 2H), 3.82 (m, 3H), 3.27 (d, 1H), 3.03 (d, 1H), 2.33 (d, 1H), 1.95 (m, 2H), 0.89 (s, 9H), 0.06 (s, 6H).

### Step 4:

### Ethyl 2-[6-[4-[2-[tert-butyl(dimethyl)silyl]oxyethyl]-2-oxo-4-(phenylmethoxycarbonylamino)pyrrolidin-1-yl]-2-nitropyridin-3-ylloxyacetate

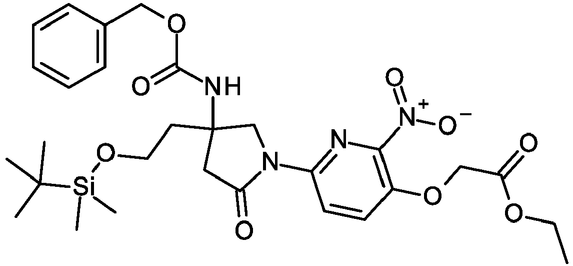

To a solution of benzyl *N*-[3-[2-[*tert*-butyl(dimethyl)silyl]oxyethyl]-5-oxo-pyrrolidin-3-yl]carbamate (300.0 mg, 0.760 mmol, 1 eq) and ethyl 2-[(6-bromo-2-nitro-3-pyridyl)oxy] acetate (233.15 mg, 0.760 mmol, 1 eq) in toluene (20 mL) were added 9,9-dimethyl-4,5-bis(diphenylphosphino)xanthene (176.88 mg, 0.310 mmol, 0.400 eq), tris(dibenzylideneacetone)dipalladium (0) (139.96 mg, 0.150 mmol, 0.200 eq) and potassium carbonate (211.24 mg, 1.53 mmol, 2 eq). The mixture was stirred at 100 °C for 2 h under N₂. The mixture was filtered and the residue was purified by flash chromatography to afford the title compound (450 mg, 0.730 mmol, 95.5% yield) as a yellow oil.

### Step 5:

### Benzyl N-[3-[2-[tert-butyl(dimethyl)silyl]oxyethyl]-5-oxo-1-(3-oxo-4H-pyrido[3,2-b][1,4]oxazin-6-yl)pyrrolidin-3-yl]carbamate

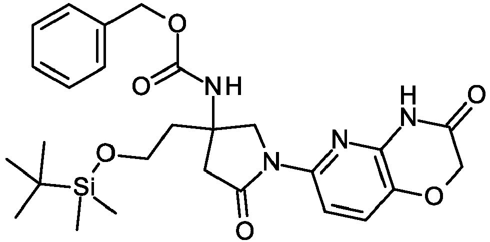

To a solution of ethyl 2-[[6-[4-(benzyloxycarbonylamino)-4-[2-[*tert-*butyl(dimethyl)silyl]oxyethyl]-2-oxo-pyrrolidin-1-yl]-2-nitro-3-pyridyl]oxy]acetate (500.0 mg, 0.810 mmol, 1 eq) in acetic acid (20 mL) was added iron (452.7 mg, 8.11 mmol, 10 eq). The mixture was stirred at 70 °C for 3 h. The mixture was poured into saturated NaHCOs (200mL) and extracted with ethyl acetate. The combined organic layers were washed with brine, dried over anhydrous Na₂SO₄ and concentrated in vacuo to obtain the crude title compound (500 mg) as a yellow oil. MS (ESP): m/z = 541.2 [M+H]⁺.

### Step 6:

### Benzyl N-[3-(2-hydroxyethyl)-5-oxo-1-(3-oxo-4H-pyrido[3,2-b][1,4]oxazin-6-yl)pyrrolidin-3-yl]carbamate

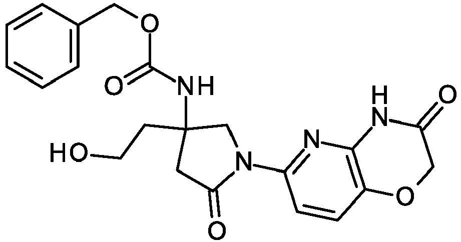

To a solution of benzyl *N*-[3-[2-[*tert*-butyl(dimethyl)silyl]oxyethyl]-5-oxo-1-(3-oxo-4*H-*pyrido[3,2-b][1,4]oxazin-6-yl)pyrrolidin-3-yl]carbamate (500.0 mg, 0.920 mmol, 1 eq) in methanol (20 mL) was added HCl/MeOH (4.0 mL, 16 mmol, 17.3 eq) and the mixture was stirred at 25 °C for 2 h. The mixture was concentrated in vacuo to afford the title compound (350 mg, 0.820 mmol, 88.8% yield) as a yellow solid. MS (ESP): m/z = 427.1 [M+H]⁺.

### Step 7:

### tert-Butyl N-[3-(2-hydroxyethyl)-5-oxo-1-(3-oxo-4H-pyrido[3,2-b] [1,4]oxazin-6-yl)pyrrolidin-3-yl] carbamate

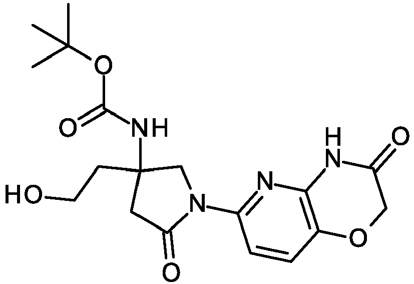

To a solution of benzyl *N*-[3-(2-hydroxyethyl)-5-oxo-1-(3-oxo-4*H*-pyrido[3,2-b][1,4]oxazin-6-yl)pyrrolidin-3-yl]carbamate (350.0 mg, 0.820 mmol, 1 eq) in DMF (5 mL) and methanol (5 mL) was addded di-*tert*-butyl dicarbonate (268.55 mg, 1.23 mmol, 1.5 eq) and Pd/C (100.0 mg). The mixture was stirred at 25 °C under an atmosphere of hydrogen for 16 h. The mixture was filtered and concentrated in vacuo to give a residue which was purified by flash chromatography to afford the title compound (180 mg, 0.460 mmol, 55.9% yield) as a yellow oil. MS (ESP): m/z = 393.1 [M+H]⁺.

### Step 8:

### 2-[3-[(2-Methylpropan-2-yl)oxycarbonylamino]-5-oxo-1-(3-oxo-4H-pyrido[3,2-b] [1,4]oxazin-6-yl)pyrrolidin-3-yl]ethyl methanesulfonate

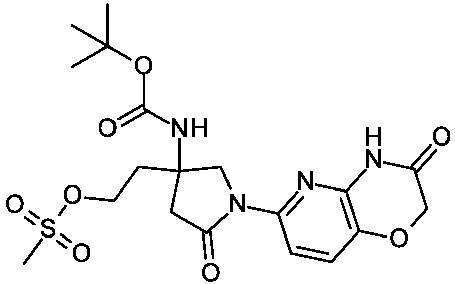

To a solution of *tert*-butyl *N*-[3-(2-hydroxyethyl)-5-oxo-1-(3-oxo-4*H*-pyrido[3,2-b][1,4]oxazin-6-yl)pyrrolidin-3-yl]carbamate (150.0 mg, 0.380 mmol, 1 eq) in DCM (5 mL) and THF (5 mL) were added *N*,*N*-diisopropylethylamine (0.27 mL, 1.53 mmol, 4 eq) and methylsufonyl chloride (87.12 mg, 0.760 mmol, 2 eq) at 0 °C and the mixture was stirred for 2 h. The mixture was poured into water (20 mL) and extracted with DCM (20 mL × 3). The combined organic layers were washed with brine (20 mL), dried over anhydrous Na₂SO₄ and concentrated in vacuo to afford the crude title compound (180 mg, 0.380 mmol, 100% yield) as colorless oil which was used for the next step without purification. MS (ESP): m/z = 471.1 [M+H]⁺.

### Step 9:

### tert-Butyl N-[3-(2-azidoethyl)-5-oxo-1-(3-oxo-4H-pyrido[3,2-b][1,4]oxazin-6-yl)pyrrolidin-3-yl]carbamate

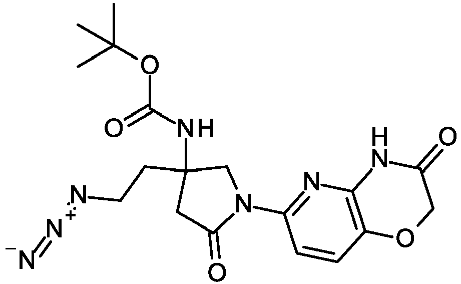

To a solution of 2-[3-[(2-methylpropan-2-yl)oxycarbonylamino]-5-oxo-1-(3-oxo-4*H*-pyrido[3,2-b][1,4]oxazin-6-yl)pyrrolidin-3-yl]ethyl methanesulfonate (180.0 mg, 0.380 mmol, 1 eq) in DMF (5 mL) was added sodium azide (0.04 mL, 1.15 mmol, 3 eq) and the mixture was stirred at 70 °C for 16 h. The mixture was poured into water (20 mL) and extracted with ethyl acetate (20 mL × 2). The combined organic layers were washed with brine, dried over anhydrous Na₂SO₄ and concentrated in vacuo to afford the title compound (120 mg, 0.290 mmol, 75.1% yield) as a yellow oil. MS (ESP): m/z = 418.1 [M+H]⁺.

### Step 10:

### tert-Butyl N-[3-(2-aminoethyl)-5-oxo-1-(3-oxo-4H-pyrido[3,2-b][1,4]oxazin-6-yl)pyrrolidin-3-yl]carbamate

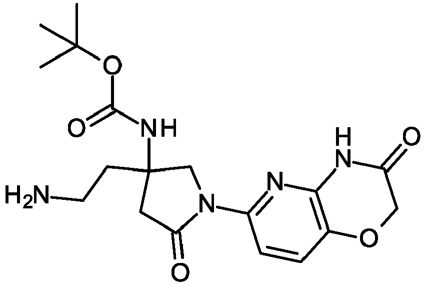

To a solution of *tert*-butyl *N*-[3-(2-azidoethyl)-5-oxo-1-(3-oxo-4*H*-pyrido[3,2-b][1,4]oxazin-6-yl)pyrrolidin-3-yl]carbamate (120.0 mg, 0.290 mmol, 1 eq) in THF (8 mL) and water (8 mL) was added PPh₃ (113.1 mg, 0.430 mmol, 1.5 eq). The mixture was stirred at 25 °C for 16 h. The mixture was concentrated in vacuo and the residue was purified by flash chromatography (10 % MeOH/DCM) to afford the title compound (52 mg, 0.130 mmol, 46.2% yield) as a colorless oil. MS (ESP): m/z = 392.1 [M+H]⁺.

### Intermediate 95

### rac-1,2-trans-1-[tert-Butyl(dimethyl)silyl]oxy-2-formyl-2,3-dihydro-1H-indene-4-carbonitrile

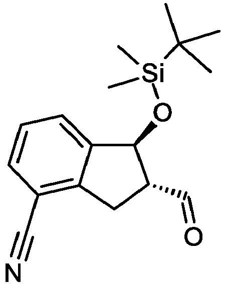

### Step 1:

### Ethyl 7-bromo-3-oxo-1,2-dihydroindene-2-carboxylate

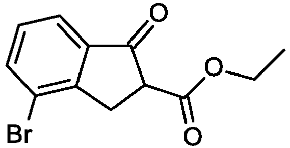

The title compound was prepared by analogy to ethyl 7-bromo-6-methoxy-3-oxo-1,2-dihydroindene-2-carboxylate (Intermediate 36, step 1) using 4-bromo-2,3-dihydroinden-1-one in place of 4-bromo-5-methoxy-indan-1-one and was obtained as a yellow solid. MS (ESP): m/z = 283.0 [M+H]⁺.

### Step 2:

### Ethyl rac-1,2-trans-4-bromo-1-hydroxy-2,3-dihydro-1H-indene-2-carboxylate

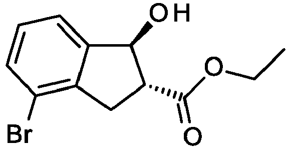

To a solution of ethyl 7-bromo-3-oxo-1,2-dihydroindene-2-carboxylate (600.0 mg, 2.12 mmol, 1 eq) in ethanol (15 mL) was added sodium borohydride (120.3 mg, 3.18 mmol, 1.5 eq) at 0 °C. The reaction mixture was stirred at 0 °C for 3 h. The mixture was quenched with saturated NH₄Cl solution (5.0 mL). The reaction mixture was concentrated to provide the crude product. The crude product was purified by preparative HPLC (formic acid) to give the title compound (450 mg, 1.58 mmol, 74.5% yield) as a yellow solid. MS (ESP): m/z = 267.0, 269.0 [M+H-H₂O]⁺.

### Step 3:

### Ethyl rac-1,2-trans-4-bromo-1-[tert-butyl(dimethyl)silyl]oxy-2,3-dihydro-1H-indene-2-carboxylate

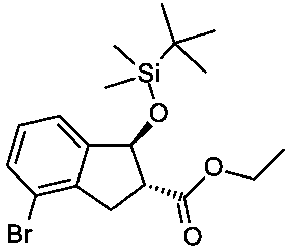

To a solution of ethyl *rac*-1,2-*trans*-4-bromo-1-hydroxy-2,3-dihydro-1*H*-indene-2-carboxylate (450.0 mg, 1.58 mmol, 1 eq) and imidazole (214.88 mg, 3.16 mmol, 2 eq) in DCM (10 mL) was added *tert*-butyldimethylchlorosilane (356.8 mg, 2.37 mmol, 1.5 eq) at 0 °C. The reaction mixture was stirred at 20 °C for 16 h. The white precipitate was filtered off and the filtrate was washed with aq. NaHCOs (20.0 mL) and brine (20.0 mL) and then the organic layer was dried over Na₂SO₄. The organic layer was concentrated to provide the crude title compound (580 mg, 1.45 mmol, 92.0% yield) as a yellow oil. The crude product was used in the next step without further purification. MS (ESP): m/z = 421.2 [M+Na]⁺.

### Steps 4 to 6:

### rac-1,2-trans-1-[tert-Butyl(dimethyl)silyl]oxy-2-formyl-2,3-dihydro-1H-indene-4-carbonitrile

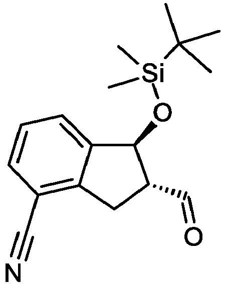

The title compound was prepared by analogy to 2-[(4-cyano-2-formyl-2,3-dihydro-1*H*-inden-5-yl)oxy]-N-methylacetamide (Intermediate 36, steps 5 to 7) and was obtained as a yellow oil.

### Intermediate 96

### tert-Butyl 2-[(4-cyano-2-formyl-2,3-dihydro-1H-inden-5-yl)oxymethyl]pyrrolidine-1-carboxylate

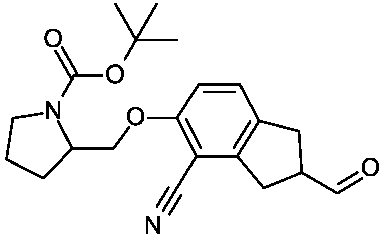

The title compound was prepared by analogy to 2-[(4-cyano-2-formyl-2,3-dihydro-1*H*-inden-5-yl)oxy]-N-methylacetamide (Intermediate 36, steps 4 to 7) using *tert*-butyl 2-[(4-methylphenyl)sulfonyloxymethyl]pyrrolidine-1-carboxylate (CAS# 136235-11-5) in place of 2-chloro-*N*-methylacetamide in the first step and was obtained as a light yellow oil.

### Intermediate 97

### 2-Trimethylsilylethyl N-[4-chloro-2-(2-oxoethyl)-1,3-dihydroinden-2-yl]carbamate

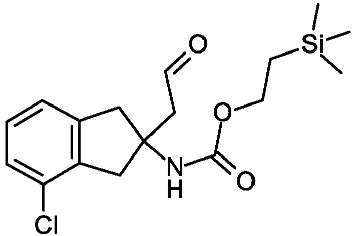

### Step 1:

### Ethyl 2-(4-chloro-2-hydroxy-1,3-dihydroinden-2-yl)acetate

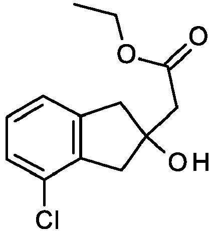

THF (10 mL) was cooled to -78°C, then ethyl acetate (1.6 mL, 24.01 mmol, 2 eq) in THF (3 mL) was added, followed by slow addition of lithium diisopropylamide (12.0 mL, 24.01 mmol, 2 eq) in THF (3 mL) at -78°C. The reaction mixture was stirred at -78°C for 0.5 hr, then 4-chloroindan-2-one (2.0 g, 12 mmol, 1 eq) in THF (2 mL) was slowly added. The mixture was stirred at -78°C for 1 hr. The mixture was quenched with saturated NH₄Cl and extracted with EtOAc (30 mL × 3).The combined extracts were dried over anhydrous Na₂SO₄, filtered and concentrated to give a residue which was purified by flash column chromatography (10% EtOAc) to give the title compound (2 g, 7.85 mmol, 65.4% yield). ¹H-NMR (400 MHz, CDCl₃) δ 7.05 (m, 3H), 4.13 (dd, 2H), 3.72 (br s, 1H), 3.14 (dd, 2H), 2.95 (q, 2H), 2.70 (s, 2H), 1.20 (q, 3H).

### Step 2:

### Ethyl 2-(2-acetamido-4-chloro-1,3-dihydroinden-2-yl)acetate

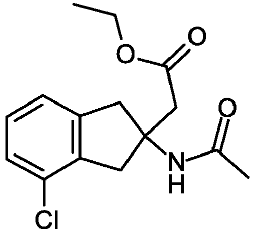

To a solution of ethyl 2-(4-chloro-2-hydroxy-indan-2-yl)acetate (2500.0 mg, 9.82 mmol, 1 eq) in MeCN (50 mL) at 0°C was added ClSO₃H (2.5 mL, 3.93 mmol, 0.400 eq) and the reaction mixture was stirred at 0 °C for 0.5 hr. The mixture was poured into ice-water (50 mL) and then extracted with EtOAc (50 mL × 2). the combined organic layers were dried over Na₂SO₄ and concentrated under vacuum to give a residue. The residue was purified by falsh chromatography column on silica gel to give the title compound (1500 mg, 5.07 mmol, 47.8% yield) as light brown solid. MS (ESP): m/z = 296.1 [M+H]⁺.

### Step 3:

### 2-(2-Amino-4-chloro-1,3-dihydroinden-2-yl)acetic acid hydrochloride

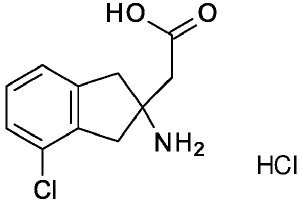

A mixture of ethyl 2-(2-acetamido-4-chloro-indan-2-yl)acetate (700.0 mg, 2.37 mmol, 1 eq) in hydrogen chloride (20.0 mL, 240 mmol, 101.4 eq) was stirred at 110 °C for 16 h. The mixture was concentrated under vacuum to give the crude title compound as a white solid. The crude product was used for the next step directly. MS (ESP): m/z = 226.0 [M+H]⁺.

### Step 4:

### Methyl 2-(2-amino-4-chloro-1,3-dihydroinden-2-yl)acetate hydrochloride

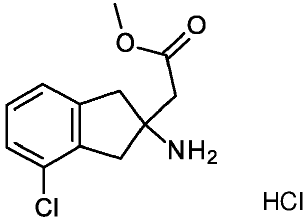

A mixture of 2-(2-amino-4-chloro-1,3-dihydroinden-2-yl)acetic acid (700.0 mg, 3.1 mmol, 1 eq) in HCl/CH₃OH (10.0 mL, 40 mmol, 12.9 eq) was stirred at 70 °C for 2 h. The mixture was concentrated under vacuum to give the crude title compound (540 mg, 1.96 mmol, 63.0% yield) as a white solid. The crude product was used for the next step directly. MS (ESP): m/z = 240.1 [M+H]⁺.

### Step 5:

### Methyl 2-[4-chloro-2-(2-trimethylsilylethoxycarbonylamino)-1,3-dihydroinden-2-yl] acetate

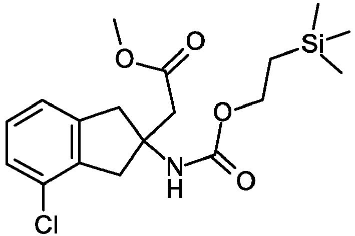

To a mixture of methyl 2-(2-amino-4-chloro-1,3-dihydroinden-2-yl)acetate hydrochloride (150.0 mg, 0.540 mmol, 1 eq) and triethylamine (0.23 mL, 1.63 mmol, 3 eq) in 1,4-dioxane (10 mL) and water (10 mL) was added 2-trimethylsilylethyl 2,5-dioxopyrrolidine-1-carboxylate (264.34 mg, 1.09 mmol, 2 eq) and the reaction was stirred at 25 °C for 2 h. The mixture was extracted with EtOAc (20 mL × 2) and the combined extracts were concentrated under vacuum to give the crude product (0.3 g). The crude product was purified by flash chromatography column on silica gel (PE/EtOAc=100/1-20/1) to give the title compound (140 mg, 0.360 mmol, 67.1% yield) as colorless oil. MS (ESP): m/z = 406.1 [M+Na]⁺.

### Steps 6 and 7:

### 2-Trimethylsilylethyl N-[4-chloro-2-(2-oxoethyl)-1,3-dihydroinden-2-yl]carbamate

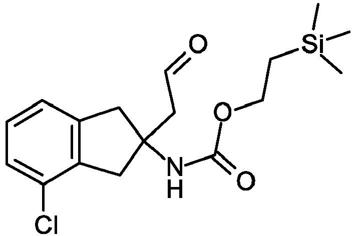

The title compound was prepared by analogy to 2-(4-chloro-2,3-dihydro-1*H*-inden-2-yl)acetaldehyde (Intermediate 11, Steps 4 and 5) and was obtained as a light brown solid.

### Intermediate 98

### tert-Butyl 4-[2-[(4-cyano-2-formyl-2,3-dihydro-1H-inden-5-yl)oxy]ethyl]piperazine-1- carboxylate

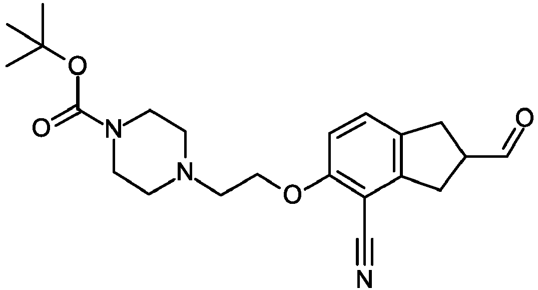

The title compound was prepared by analogy to 2-[(4-cyano-2-formyl-2,3-dihydro-1*H*-inden-5-yl)oxy]-*N*-methylacetamide (Intermediate 36, steps 4 to 7) using *tert*-butyl 4-(2-chloroethyl)piperazine-1-carboxylate (CAS# 208167-83-3) in place of 2-chloro-N-methylacetamide in the first step and was obtained as a light yellow oil.

### Intermediate 99

### tert-Butyl N-[1-[(1-chloro-6-formyl-6,7-dihydro-5H-cyclopenta[c]pyridin-3-yl)oxy]propan-2-yl] carbamate

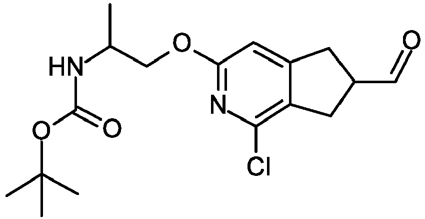

The title compound was prepared by analogy to *tert*-butyl *N*-[1-[(1-chloro-6-formyl-6,7-dihydro-5*H*-cyclopenta[c]pyridin-3-yl)oxymethyl]cyclopropyl]carbamate (Intermediate 79, steps 3 to 5) using 2-[(2-methylpropan-2-yl)oxycarbonylamino]propyl 4-methylbenzenesulfonate in place of *tert*-butyl *N*-[1-(bromomethyl)cyclopropyl]carbamate in the first step and was obtained as a light yellow oil.

The 2-[(2-methylpropan-2-yl)oxycarbonylamino]propyl 4-methylbenzenesulfonate used as starting material was prepared as follows:
To a solution of *tert*-butyl *N*-(1-hydroxypropan-2-yl)carbamate (CAS# 147252-84-4, 1.5 g, 8.56 mmol, 1 eq) and triethylamine (2.39 mL, 17.12 mmol, 2 eq) in DCM (15 mL) was added 4-methylbenzenesulfonyl chloride (2439.52 mg, 12.84 mmol, 1.5 eq). The mixture was stirred at 25 °C for 5 h. The solvent was removed by evaporation to give a residue which was purified by flash chromatography (30% PE/EA) to give 2-[(2-methylpropan-2-yl)oxycarbonylamino]propyl 4-methylbenzenesulfonate (1680 mg, 5.1 mmol, 53.3% yield) as colourless semi-solid. MS (ESP): m/z = 230.1 [M+H-BOC]⁺.

### Intermediate 100

### tert-Butyl N-(rac-5,6-cis-1-chloro-6-formyl-6,7-dihydro-5H-cyclopenta[c]pyridin-5-yl)carbamate

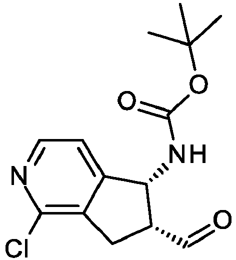

The title compound was prepared by analogy to *tert*-butyl *N*-[(2-methylpropan-2-yl)oxycarbonyl]-*N*-[*rac*-5,6-*trans*-1-chloro-6-formyl-6,7-dihydro-5*H*-cyclopenta[c]pyridin-5-yl]carbamate (Intermediate 80, steps 2 to 5) using *rac*-5,6-*trans-*6-[[*tert-*butyl(diphenyl)silyl]oxymethyl]-1-chloro-6,7-dihydro-5*H*-cyclopenta[c]pyridin-5-ol (Intermediate 80, step 1) in place of *rac*-5,6-*cis*-6-[[*tert*-butyl(diphenyl)silyl]oxymethyl]-1-chloro-6,7-dihydro-5*H*-cyclopenta[c]pyridin-5-ol and was obtained as a light brown semi-solid.

### Intermediate 101

### tert-Butyl N-[[rac-4,5-cis-5-(2-aminoethyl)-2-oxo-3-(3-oxo-4H-pyrido[3,2-b][1,4]oxazin-6-yl)-1,3-oxazolidin-4-yl]methyl]carbamate

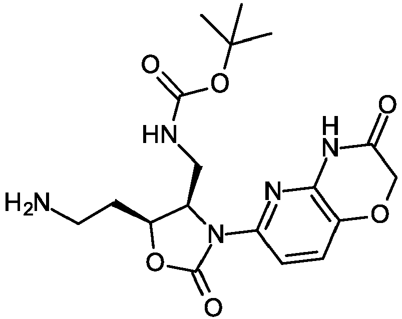

### Step 1:

### 1-(Oxiran-2-yl)-3-phenylmethoxypropan-1-ol

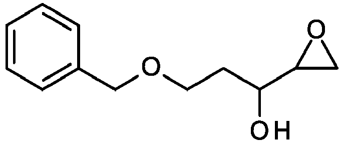

To a solution of 5-benzyloxypent-1-en-3-ol (CAS# 93553-66-3, 12.5 g, 65.02 mmol, 1 eq) in DCM (180 mL) was added 3-chloroperbenzoic acid (15.7 g, 78.02 mmol, 1.2 eq) at 0°C. The mixture was stirred at 25 °C for 16 h. The mixture was filtered and the filtrate was washed with 5% aq. NaOH (2 × 50 mL). The organic layer was concentrated to give a residue which was purified by flash chromatography (40% PE/EA) to give the title compound (7.5 g, 36.01 mmol, 55.4% yield) as a colorless oil. ¹H-NMR (400 MHz, CDCl₃) δ 7.35 (m, 5H), 4.55 (s, 2H), 3.75 (m, 3H), 3.03 (m, 1H), 2.79 (m, 3H), 1.94 (m, 2H).

### Step 2:

### [1-(Oxiran-2-yl)-3-phenylmethoxypropyl] N-benzoylcarbamate

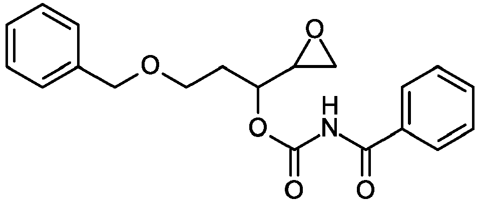

To a solution of benzoyl isocyanate (6.36 g, 43.22 mmol, 1.2 eq) in DCM (200 mL) was added 1-(oxiran-2-yl)-3-phenylmethoxypropan-1-ol (7.5 g, 36.01 mmol, 1 eq). The mixture was stirred at 25 °C for 1 h under nitrogen atmosphere. The solvent was removed by evaporation to give a residue which was purified by flash chromatography (60% PE/EA) to give the title compound (13 g, 36.58 mmol, 94.5 % yield) as a colorless oil. MS (ESP): m/z = 356.1 [M+H]⁺.

### Step 3:

### [2-Oxo-5-(2-phenylmethoxyethyl)-1,3-oxazolidin-4-yl]methyl benzoate

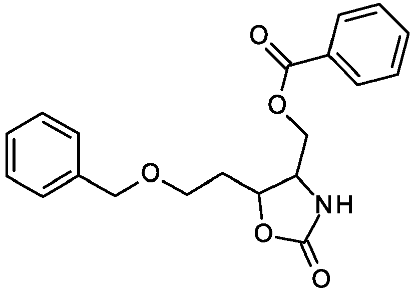

To a solution of [1-(oxiran-2-yl)-3-phenylmethoxypropyl] N-benzoylcarbamate (13.0 g, 36.58 mmol, 1 eq) in DCM (500 mL) was added 1,8-diazabicyclo[5.4.0]undec-7-ene (1.09 mL, 7.32 mmol, 0.200 eq). The mixture was stirred at 50 °C for 2 h. The solvent was removed by evaporation to give a residue which was purified by flash chromatography (60% PE/EA) to give the title compound (11 g, 30.95 mmol, 71.9 % yield) as a light yellow oil. MS (ESP): m/z = 356.1 [M+H]⁺.

### Steps 4 to 5:

### [2-Oxo-3-(3-oxo-4H-pyrido[3,2-b][1,4]oxazin-6-yl)-5-(2-phenylmethoxyethyl)-1,3-oxazolidin-4-yl]methyl benzoate

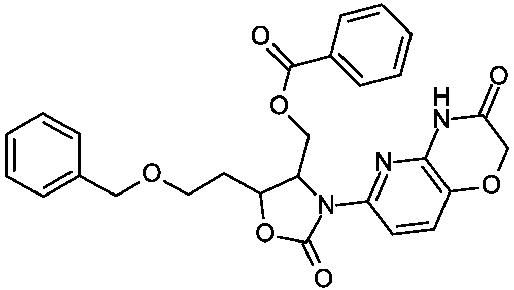

The title compound was prepared by analogy to 6-[5-[2-[*tert*-butyl(dimethyl)silyl]oxyethyl]-2-oxo-1,3-oxazolidin-3-yl]-4*H*-pyrido[3,2-b][1,4]oxazin-3-one (Intermediate 1, steps 4 and 5) and was obtained as a yellow viscous oil which was used directly without further purification. MS (ESP): m/z = 504.1 [M+H]⁺.

### Step 6:

### 6-[4-(Hydroxymethyl)-2-oxo-5-(2-phenylmethoxyethyl)-1,3-oxazolidin-3-yl]-4H-pyrido[3,2-b][1,4]oxazin-3-one

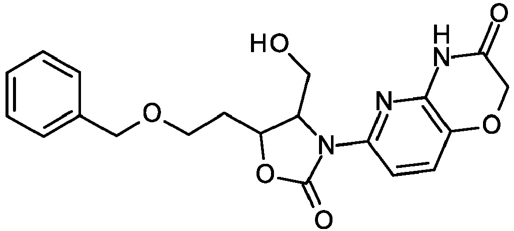

To a solution of [2-oxo-3-(3-oxo-4*H*-pyrido[3,2-b][1,4]oxazin-6-yl)-5-(2-phenylmethoxyethyl)-1,3-oxazolidin-4-yl]methyl benzoate (2600.0 mg, 5.16 mmol, 1 eq) in methanol (52 mL) was added lithium hydroxide (0.1 mL, 10.33 mmol, 2 eq). The mixture was stirred at 25 °C for 2 h. The mixture was extracted with EtOAc (15 mL) and the organic extracts were washed with 1N HCl (5 mL) and brine (5 mL). The organic layer was dried and concentrated to give the title compound (2000 mg, 5.01 mmol, 90.2 % yield) as a light yellow solid, which was used directly without further purification. MS (ESP): m/z = 400.1 [M+H]⁺.

### Steps 7 to 10:

### tert-Butyl N-[[2-oxo-3-(3-oxo-4H-pyrido[3,2-b][1,4]oxazin-6-yl)-5-(2-phenylmethoxyethyl)-1,3-oxazolidin-4-yl]methyl]carbamate

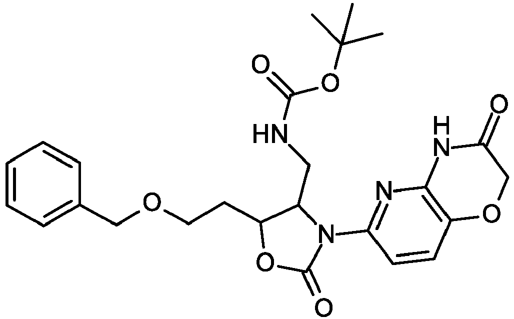

The title compound was prepared by analogy to *tert*-butyl *N*-[2-(2-oxo-1,3-oxazolidin-5-yl)ethyl]carbamate (Intermediate 26, steps 2 to 5) and was obtained as a light yellow viscous oil. MS (ESP): m/z = 499.2 [M+H]⁺.

### Step 11:

### tert-Butyl N-[[rac-4,5-cis-5-(2-hydroxyethyl)-2-oxo-3-(3-oxo-4H-pyrido[3,2-b][1,4]oxazin-6-yl)-1,3-oxazolidin-4-yl]methyl]carbamate and tert-butyl N-[[rac-4,5-trans-5-(2-hydroxyethyl)-2-oxo-3-(3-oxo-4H-pyrido[3,2-b][1,4]oxazin-6-yl)-1,3-oxazolidin-4-yl]methyl]carbamate

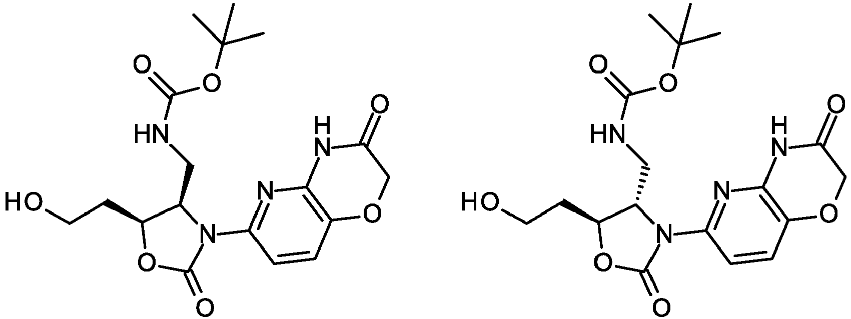

To a solution of *tert*-butyl *N*-[[2-oxo-3-(3-oxo-4*H*-pyrido[3,2-b][1,4]oxazin-6-yl)-5-(2-phenylmethoxyethyl)-1,3-oxazolidin-4-yl]methyl]carbamate (350.0 mg, 0.700 mmol, 1 eq) in THF (10 mL) was added Pd-C (700.0 mg). The mixture was stirred at 25 °C for 16 h under an hydrogen atmosphere. The mixture was filtrated and the residue was purified by preparative TLC (PE:EA = 1:3) to give *tert*-butyl *N*-[[*rac*-4,5-*trans*-5-(2-hydroxyethyl)-2-oxo-3-(3-oxo-4*H-*pyrido[3,2-b][1,4]oxazin-6-yl)-1,3-oxazolidin-4-yl]methyl]carbamate (80 mg, 0.200 mmol, 23.7% yield) and *tert*-butyl *N*-[[*rac*-4,5-*cis*-5-(2-hydroxyethyl)-2-oxo-3-(3-oxo-4*H*-pyrido[3,2-b][1,4]oxazin-6-yl)-1,3-oxazolidin-4-yl]methyl]carbamate (100 mg, 0.240 mmol, 31.4% yield) as colorless oils. MS (ESP): m/z = 409.4 [M+H]⁺ for both products.

### Steps 12 to 14:

### tert-Butyl N-[[rac-4,5-cis-5-(2-aminoethyl)-2-oxo-3-(3-oxo-4H-pyrido[3,2-b][1,4]oxazin-6-yl)-1,3-oxazolidin-4-yl]methyl]carbamate

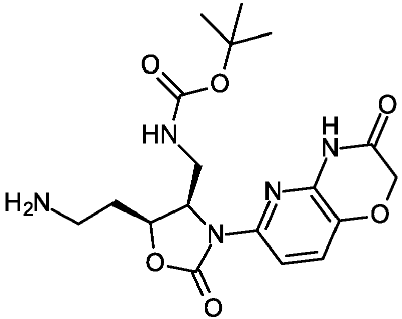

The title compound was prepared by analogy to 6-[5-(2-aminoethyl)-2-oxo-1,3-oxazolidin-3-yl]-4*H*-pyrido[3,2-b][1,4]oxazin-3-one (Intermediate 1, steps 7 to 9) and was obtained as a light yellow semi-solid. MS (ESP): m/z = 408.4 [M+H]⁺.

### Intermediate 102

### tert-Butyl N-[[rac-4,5-trans-5-(2-aminoethyl)-2-oxo-3-(3-oxo-4H-pyrido[3,2-b][1,4]oxazin-6-yl)-1,3-oxazolidin-4-yl]methyl]carbamate

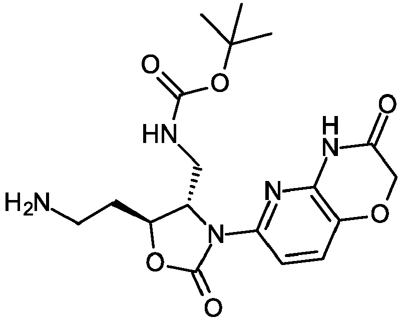

The title compound was prepared by analogy to *tert*-butyl *N*-[[*rac*-4,5-*cis*-5-(2-aminoethyl)-2-oxo-3-(3-oxo-4*H*-pyrido[3,2-b][1,4]oxazin-6-yl)-1,3-oxazolidin-4-yl]methyl]carbamate (Intermediate 101, steps 12 to 14) using *tert*-butyl *N*-[[*rac*-4,5-*trans*-5-(2-hydroxyethyl)-2-oxo-3-(3-oxo-4*H*-pyrido[3,2-b][1,4]oxazin-6-yl)-1,3-oxazolidin-4-yl]methyl]carbamate (Intermediate 101, step 11) in place of *tert*-butyl *N*-[[*rac*-4,5-*cis*-5-(2-hydroxyethyl)-2-oxo-3-(3-oxo-4*H-*pyrido[3,2-b][1,4]oxazin-6-yl)-1,3-oxazolidin-4-yl]methyl]carbamate and was obtained as a light yellow semi-solid. MS (ESP): m/z = 408.4 [M+H]⁺.

### Intermediate 103

### tert-Butyl 3-[(7-fluoro-2-formyl-2,3-dihydro-1H-inden-5-yl)oxy] azetidine-1-carboxylate

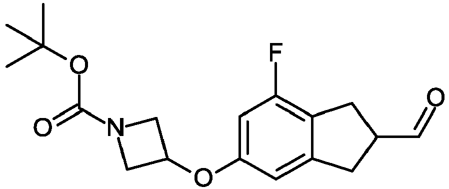

### Step 1:

### tert-Butyl 3-[(2-ethoxycarbonyl-7-fluoro-2,3-dihydro-1H-inden-5-yl)oxy]azetidine-1-carboxylate

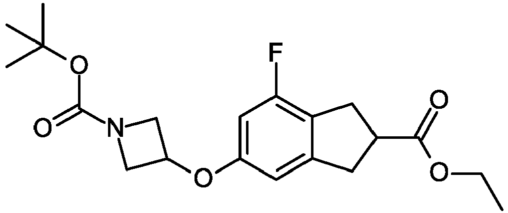

To a mixture of ethyl 4-fluoro-6-hydroxy-indane-2-carboxylate; ethyl 4-fluoro-5-hydroxy-indane-2-carboxylate; ethyl 4-fluoro-7-hydroxy-indane-2-carboxylate (Intermediate 50, step 4, 500.0 mg, 2.23 mmol, 1 eq) and potassium carbonate (615.89 mg, 4.46 mmol, 2 eq) in NMP (10 mL) was added *tert*-butyl 3-bromoazetidine-1-carboxylate (631.79 mg, 2.68 mmol, 1.2 eq) and the mixture was stirred at 80 °C for 16 h. The solid was filtered off and the filtrate was concentrated to give the crude product which was purified by chromatography on silica gel (PE:EA=10:1) to give a mixture of *tert*-butyl 3-[(2-ethoxycarbonyl-4-fluoro-2,3-dihydro-1*H-*inden-5-yl)oxy]azetidine-1-carboxylate and *tert*-butyl 3-[(2-ethoxycarbonyl-6-fluoro-2,3-dihydro-1*H*-inden-5-yl)oxy]azetidine-1-carboxylate (200 mg, 0.550 mmol, 24.7% yield) as colorless oils and the title compound (500 mg, 1.32 mmol, 59.2% yield) as a colorless oil.

### Steps 2 and 3:

### tert-Butyl 3-[(7-fluoro-2-formyl-2,3-dihydro-1H-inden-5-yl)oxy] azetidine-1-carboxylate

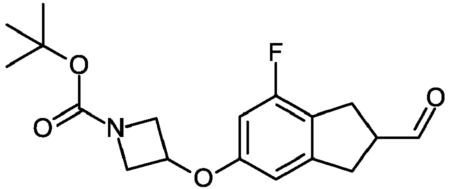

The title compound was prepared by analogy to 4-fluoro-1-methyl-6,7-dihydro-5*H-*cyclopenta[c]pyridine-6-carbaldehyde (Intermediate 27, steps 3 and 4) and was obtained as a colorless oil.

### Intermediate 104

### rac-2,3-trans-3-[tert-Butyl(dimethyl)silyl]oxy-2-formyl-2,3-dihydro-1H-indene-4-carbonitrile

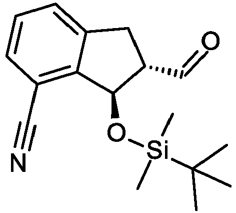

The title compound was prepared by analogy to *rac*-1,2-*trans*-1-[*tert*-butyl(dimethyl)silyl]oxy-2-formyl-2,3-dihydro-1*H*-indene-4-carbonitrile (Intermediate 95) using 7-bromo-2,3-dihydroinden-1-one in place of 4-bromo-2,3-dihydroinden-1-one in the first step and was obtained as a yellow oil. ¹H-NMR (400 MHz, CDCl₃) δ 9.57 (s, 1H), 7.28 (m, 2H), 7.15 (m, 1H), 5.53 (s, 1H), 3.1 (m, 3H), 0.68 (s, 9H), 0.09 (s, 3H), 0.01 (s, 3H).

### Intermediate 105

### tert-Butyl N-[1-[(7-fluoro-2-formyl-2,3-dihydro-1H-inden-5-yl)oxy]propan-2-yl]carbamate

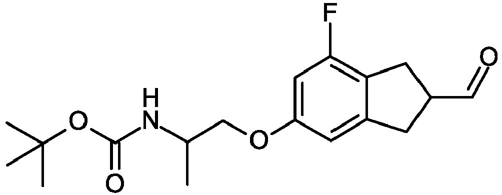

### Step 1:

### Ethyl 4-fluoro-6-[2-[(2-methylpropan-2-yl)oxycarbonylamino]propoxy]-2,3-dihydro-1H-indene-2-carboxylate

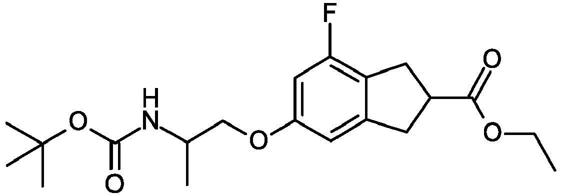

To a mixture of ethyl 4-fluoro-6-hydroxy-indane-2-carboxylate; ethyl 4-fluoro-5-hydroxy-indane-2-carboxylate; ethyl 4-fluoro-7-hydroxy-indane-2-carboxylate (Intermediate 50 step 2, 394.13 mg, 1.76 mmol, 1.1 eq), *tert*-butyl *N*-(1-hydroxypropan-2-yl)carbamate (280.0 mg, 1.6 mmol, 1 eq) and triphenylphosphine (838.21 mg, 3.2 mmol, 2 eq) in THF (20 mL) was added diethylazodicarboxlyate (417.41 mg, 2.4 mmol, 1.5 eq) dropwise at 0 °C under N₂ and the reaction mixture was stirred at 25 °C for 16 h. The mixture was diluted with EtOAc (30 mL) and washed with H₂O (2 × 10 mL) and brine (10 mL). The organic layer was dried and concentrated to give a residue which was purified by flash chromatography (25% PE/EA) to give the title compound (250 mg, 0.660 mmol, 34.0% yield) as a colorless oil. MS (ESP): m/z = 282.6 [M+H-BOC]⁺.

### Steps 2 and 3:

### tert-Butyl N-[1-[(7-fluoro-2-formyl-2,3-dihydro-1H-inden-5-yl)oxy]propan-2-yl]carbamate

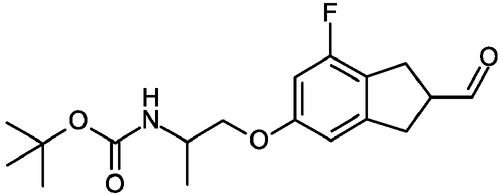

The title compound was prepared by analogy to 4-fluoro-1-methyl-6,7-dihydro-5*H-*cyclopenta[c]pyridine-6-carbaldehyde (Intermediate 27, steps 3 and 4) and was obtained as a light yellow semi-solid.

### Intermediate 106

### tert-Butyl N-[2-[(7-fluoro-2-formyl-2,3-dihydro-1H-inden-5-yl)oxy]propyl]carbamate

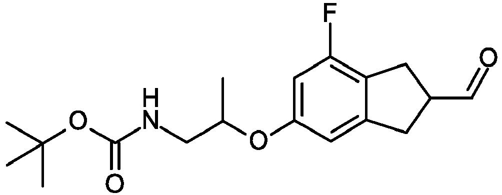

### Step 1:

### tert-Butyl N-[2-[[2-[[tert-butyl(dimethyl)silyl]oxymethyl]-7-fluoro-2,3-dihydro-1H-inden-5-yl]oxy]propyl]carbamate

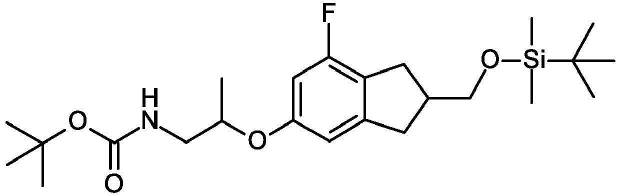

To a solution of triphenylphosphine (132.71 mg, 0.510 mmol, 1.5 eq), *tert-butyl N-(2-*hydroxypropyl)carbamate (70.93 mg, 0.400 mmol, 1.2 eq) and diethylazodicarboxlyate (88.12 mg, 0.510 mmol, 1.5 eq) in toluene (10 mL) was added 2-[[*tert*-butyl(dimethyl)silyl]oxymethyl]-7-fluoro-2,3-dihydro-1*H*-inden-5-ol (Intermediate 52, step 2, 100.0 mg, 0.340 mmol, 1 eq) dropwise at 0 °C under N₂ and the reaction mixture was stirred at 25 °C for 16 h. The mixture was poured into 20 mL water and then extracted with 20 mL EtOAc. The organic layer was dried with Na₂SO₄ and the solvent was removed to give the crude product which was purified first by flash column chromatography on silica gel (PE:EA=1:1) and then further purified by preparative TLC (PE:EA=3:1) to give the title compound (100 mg, 0.220 mmol, 65.35% yield) as a colorless oil.

### Steps 2 and 3:

### tert-Butyl N-[2-[(7-fluoro-2-formyl-2,3-dihydro-1H-inden-5-yl)oxy]propyl]carbamate

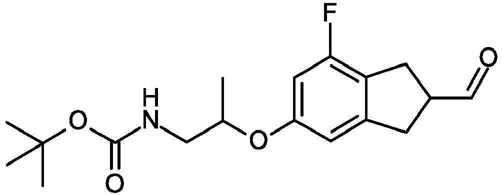

The title compound was prepared by analogy to 2-[(7-fluoro-2-formyl-2,3-dihydro-1*H*-inden-5-yl)oxy]acetonitrile (Intermediate 52, steps 4 and 5) and was obtained as an off-white semisolid.

### Intermediate 107

### tert-Butyl N-(rac-1,2-trans-7-cyano-2-formyl-2,3-dihydro-1H-inden-1-yl)carbamate

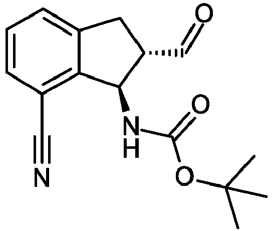

The title compound was prepared by analogy to *tert*-butyl *N*-[*rac*-1,2-*trans*-4-cyano-2-formyl-2,3-dihydro-1*H*-inden-1-yl]carbamate (Intermediate 88) using 7-bromo-2,3-dihydroinden-1-one in place of 4-chloroindan-1-one and was obtained as a yellow oil.

### Intermediate 108

### 2-Trimethylsilylethyl N-[1-(4-chloro-2,3-dihydro-1H-inden-2-yl)-2-oxoethyl]carbamate

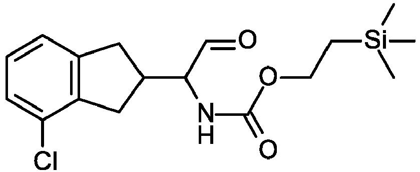

### Step 1:

### 5-(4-Chloro-2,3-dihydro-1H-inden-2-yl)imidazolidine-2,4-dione

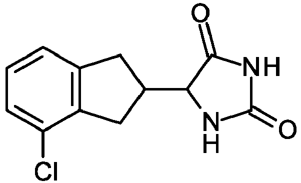

To a solution of 4-chloro-2,3-dihydro-1*H*-indene-2-carbaldehyde (CAS# 1823964-97-1, 2800.0 mg, 15.5 mmol, 1 eq) in ethanol (40 mL) and water (40 mL) were added ammonium carbonate (5957.01 mg, 61.99 mmol, 4 eq) and potassium cyanide (1549.51 mg, 23.79 mmol, 1.54 eq). The mixture was heated to 50 °C and stirred for 12 h. The mixture was filtered. The filtrate was extracted with ethyl acetate (50 mL × 3) and the combined extracts were concentrated to give a crude product. The crude product was purified by flash chromatography (70% EA/PE) to give the title compound (1700 mg, 6.78 mmol, 43.2 % yield) as a light brown solid. MS (ESP): m/z = 251.0 [M+H]⁺.

### Step 2:

### Ethyl 2-amino-2-(4-chloro-2,3-dihydro-1H-inden-2-yl)acetate

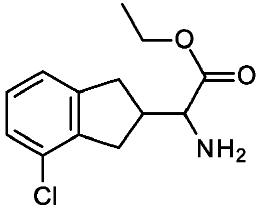

To a mixture of 5-(4-chloro-2,3-dihydro-1*H*-inden-2-yl)imidazolidine-2,4-dione (300.0 mg, 1.2 mmol, 1 eq) in 1,4-dioxane (3 mL) was added aq. 3M NaOH (15.0 mL, 45 mmol, 37.6 eq). The mixture was stirred at 90 °C for 12 h. To the mixture was added 1N HCl adjusting the pH to 7. The mixture was extracted with ethyl acetate (50 mL × 2). The organic extracts were washed with brine, dried over anhydrous magnesium sulfate and concentrated to give a brown solid (150 mg). The aqueous phase was lyophilized to give a white solid (3000 mg). The white solid was dissolved in ethanol (40 mL) and 98% sulfuric acid (10.0 mL) was added. The mixture was stirred at 80 °C for 12 h. The pH of the reaction solution was adjusted to around 10 by addition of Na₂CO₃. The mixture was extracted with EtOAc (3 × 50 mL) and the combined organic layers were washed with brine, dried over anhydrous magnesium sulfate and concentrated under vacuum to give the title compound (147 mg, 0.580 mmol, 48 % yield) as a white semi-solid. The crude was used for next step directly. MS (ESP): m/z = 254.1 [M+H]⁺.

### Step 3:

### Ethyl 2-(4-chloro-2,3-dihydro-1H-inden-2-yl)-2-(2-trimethylsilylethoxycarbonylamino)acetate

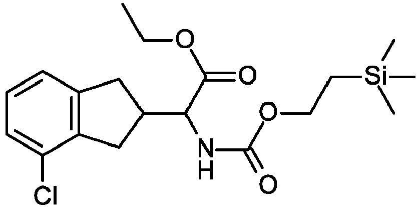

The title compound was prepared by analogy to methyl 2-[4-chloro-2-(2-trimethylsilylethoxycarbonylamino)-1,3-dihydroinden-2-yl]acetate (Intermediate 97) and was obtained as a light yellow oil. MS (ESP): m/z = 420.1 [M+Na]⁺.

### Step 4:

### 2-Trimethylsilylethyl N-[1-(4-chloro-2,3-dihydro-1H-inden-2-yl)-2-hydroxyethyl]carbamate

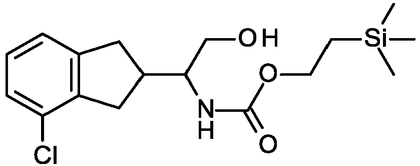

To a solution of ethyl 2-(4-chloroindan-2-yl)-2-(2-trimethylsilylethoxycarbonylamino)acetate (200.0 mg, 0.500 mmol, 1 eq) in THF (14 mL) was added lithium borohydride (21.89 mg, 1.01 mmol, 2 eq) at 0°C. Then the reaction was stirred at 25 °C for 3 h. The reaction mixture was quenched by addition of formic acid. The reaction mixture was concentrated under vacuum to give the crude title compound (200 mg, 0.560 mmol, 83.8% yield) as a light yellow oil which was used directly without further purification. MS (ESP): m/z = 378.0 [M+Na]⁺.

### Step 5:

### 2-Trimethylsilylethyl N-[1-(4-chloro-2,3-dihydro-1H-inden-2-yl)-2-oxoethyl]carbamate

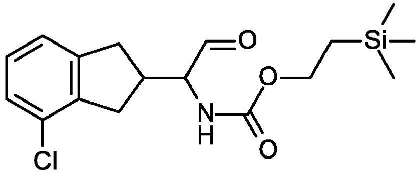

The title compound was prepared by analogy to 2-formyl-2,3-dihydro-1*H*-indene-4-carbonitrile (Intermediate 5, step 3) and was obtained as a light yellow oil.

### Intermediate 109

### tert-Butyl N-[1-[(7-fluoro-2-formyl-2,3-dihydro-1H-inden-5-yl)oxymethyl]cyclopropyl]carbamate

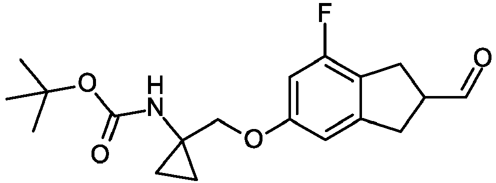

The title compound was prepared by analogy to 2-[(7-fluoro-2-formyl-2,3-dihydro-1*H*-inden-5-yl)oxy]acetonitrile (Intermediate 52, steps 3 to 5) using *tert*-butyl N-[1-(bromomethyl)cyclopropyl]carbamate in place of bromoacetonitrile and was obtained as a light yellow oil.

### Intermediate 110

### tert-Butyl N-[3-[(7-fluoro-2-formyl-2,3-dihydro-1H-inden-5-yl)oxymethyl]oxetan-3-yl] carbamate

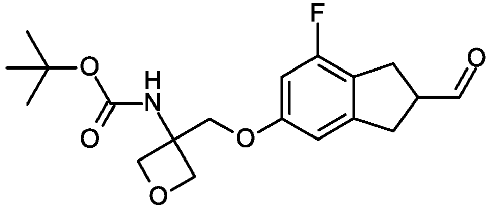

The title compound was prepared by analogy to 2-[(7-fluoro-2-formyl-2,3-dihydro-1*H*-inden-5-yl)oxy]acetonitrile (Intermediate 52, steps 3 to 5) using [3-[(2-methylpropan-2-yl)oxycarbonylamino]oxetan-3-yl]methyl 4-methylbenzenesulfonate in place of bromoacetonitrile and was obtained as a light yellow oil.

The [3-[(2-methylpropan-2-yl)oxycarbonylamino]oxetan-3-yl]methyl 4-methylbenzenesulfonate used as starting material was prepared as follows:
To a solution of *tert*-butyl *N*-[3-(hydroxymethyl)oxetan-3-yl]carbamate (CAS# 1363382-11-9, 0.5 g, 2.46 mmol, 1 eq) in pyridine (4.95 mL) was added 4-methylbenzenesulfonyl chloride (703.5 mg, 3.69 mmol, 1.5 eq) and the solution was stirred at 25 °C for 16 h. The solution was poured into 20 mL water and the mixture was extracted with EtOAc (10 mL × 2) The combined extracts were dried with Na₂SO₄. The solvent was removed to give the crude product which was purified by chromatography on silica gel (PE:EA=10:1) to give the title compound (600 mg, 1.68 mmol, 68.2% yield) as a colorless oil. ¹H-NMR (400 MHz, d6-DMSO) δ 7.80 (d, 2H), 7.50 (d, 2H), 4.43 (d, 2H), 4.30 (m, 4H), 2.43 (s, 3H), 1.33 (s, 9H).

### Intermediate 111

### tert-Butyl N-(rac-1,2-trans-4-fluoro-2-formyl-2,3-dihydro-1H-inden-1-yl)carbamate

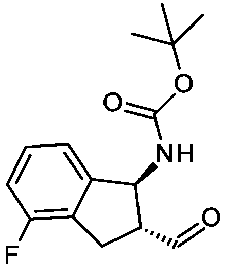

The title compound was prepared by analogy to *tert*-Butyl *N*-(*rac*-1,2-*trans*-4-chloro-2-formyl-2,3-dihydro-1*H*-inden-1-yl)carbamate (Intermediate 92) starting from 4-fluoro-2,3-dihydroinden-1-one (CAS# 699-99-0) and was obtained as a light yellow oil.

### Intermediate 112

### tert-Butyl N-[rac-5,6-cis-5,8-trans-8-amino-2-oxo-3-(3-oxo-4H-pyrido[3,2-b][1,4]oxazin-6-yl)-1-oxa-3-azaspiro[4.5]decan-6-yl]carbamate

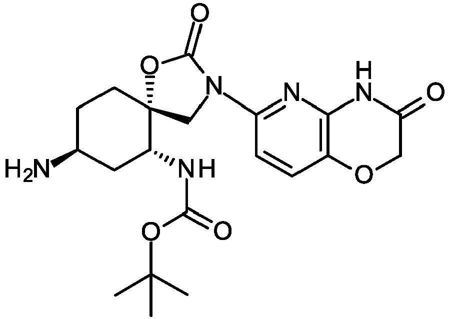

### Step 1:

### Benzyl N-[rac-1,3-cis-1,6-cis-7-oxabicyclo[4.1.0]heptan-3-yl]carbamate

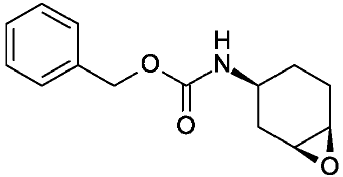

To a solution of benzyl cyclohex-3-en-1-ylcarbamate (6.5 g, 28.1 mmol, 1 eq, CAS# 210574-45-1) in DCM (140 mL) was added 70% 3-chloroperbenzoic acid (7.3 g, 29.5 mmol, 1.05 eq) at 0 °C. After 4 hours, the reaction mixture was quenched with sat. Na₂S₂O₃ and extracted with DCM (2 x). The combined organic phases were washed with sat. NaHCO₃ (2 x), dried over Na₂SO₄ and evaporated. The residue was purified by flash chromatography on silica gel eluting with 0-40% EtOAc in heptane to give a mixture of isomers. The diastereomers were separated via non-chiral SFC using a Viridis column eluting with 5% MeOH with ethylpyridine as modifier. Using NOESY the major isomer was unambiguously assigned to have the *cis* configuration. The title compound (3.3 g, 13.3 mmol, 48% yield) was obtained as white solid. (ESP): m/z = 378.2 [M+H]⁺.

### Step 2:

### Benzyl N-[rac-1,3-trans-1,4-cis-3-amino-4-hydroxycyclohexyl]carbamate

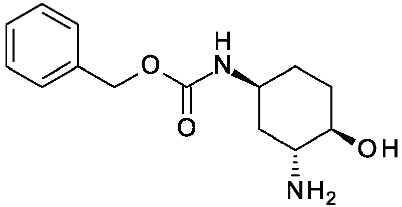

Benzyl *N*-[*rac*-1,3-*cis*-1,6-*cis*-7-oxabicyclo[4.1.0]heptan-3-yl]carbamate (10 g, 40.4 mmol, 1 eq) was dissolved in EtOH (100 mL) and 25% ammonia in water (70 mL, 809 mmol, 20 eq) was added and the mixture was stirred at 45 °C overnight. The mixture was evaporated and the residue was purified by flash chromatography eluting on silica gel (CH₂Cl₂ to CH₂Cl₂:10% NH₄OH in MeOH = 10:1) to give the title compound (7.7 g, 29.1 mmol, 72 % yield) as a white solid. The structure of the product was unambiguously assigned with COSY. (ESP): m/z = 265.2 [M+H]⁺. The other regioisomer was also isolated as minor product.

### Step 3:

### Benzyl N-[rac-1,3-trans-1,4-cis-4-hydroxy-3-[(2-methylpropan-2-yl)oxycarbonylamino]cyclohexyl]carbamate

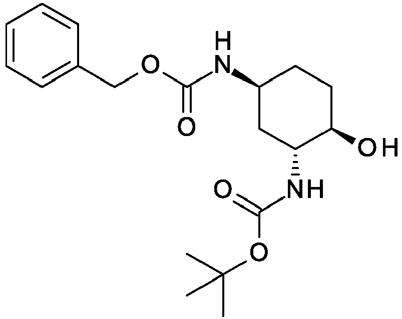

Benzyl *N*-[*rac*-1,3-*trans*-1,4-*cis*-3-amino-4-hydroxycyclohexyl]carbamate (7.7 g, 29.1 mmol, 1 eq) was dissolved in ethanol (44 mL) and cooled to 0 °C. Di-*tert*-butyl dicarbonate (6.4 g, 29.1 mmol, 1 eq) was added and the ice bath was removed. The mixture was stirred for 16 hours. Water (50 mL) and 4-dimethylaminopyridine (1.1 g, 8.7 mmol, 0.3 eq) were added and the mixture was stirred for 30 minutes. 10% citric acid was added and the mixture was stirred for 10 minutes. The mixture was extracted with methyl *tert*-butyl ether (2 x) and the combined organic layers were washed with brine, dried over Na₂SO₄ and evaporated to give the title compound (11.0 g, 29.1 mmol, quant. yield) as a white foam. (ESP): m/z = 465.3 [M+H]⁺.

### Step 4:

### Benzyl N-[rac-1,3-trans-3-[(2-methylpropan-2-yl)oxycarbonylamino]-4-oxocyclohexyl] carbamate

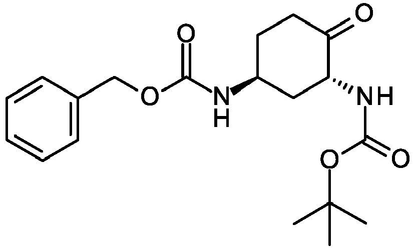

Benzyl *N*-[*rac*-1,3-*trans*-1,4-*cis*-4-hydroxy-3-[(2-methylpropan-2-yl)oxycarbonylamino]cyclohexyl]carbamate (5.351 g, 14.7 mmol, 1 eq) was dissolved in dichloromethane (134 mL). Dess-Martin periodinane (6.85 g, 16.2 mmol, 1.1 eq) was added and the mixture stirred for 2 h at RT. Further Dess-Martin periodinane (3.11 g, 7.34 mmol, 0.5 eq) was added and the mixture stirred for one more hour. The mixture was filtered and the residue washed with CH₂Cl₂. To the combined filtrate and washings was added 10g SiO₂ and the mixture evaporated to dryness. The residue was purified by silica cartridge chromatography (ISCO: 50g, from heptane to EtOAc) to give the title compound (4.36 g, 12 mmol, 81.9 % yield). MS (ESP): m/z = 263.2 [M+2H-BOC]⁺.

### Step 5:

### Ethyl 2-[rac-2,4-trans-1-hydroxy-2-[(2-methylpropan-2-yl)oxycarbonylamino]-4-(phenylmethoxycarbonylamino)cyclohexyl] acetate

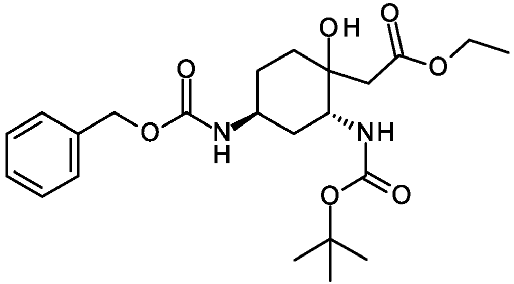

To a solution of 1M LHMDS in THF (3.04 mL, 3.04 mmol, 1.1 eq) under argon was slowly added dropwise 1M ethyl acetate in THF (3.04 mL, 3.04 mmol, 1.1 eq) at -78°C. After stirring for 15 min. at -78°C, benzyl *N*-[*rac*-1,3-*trans*-3-[(2-methylpropan-2-yl)oxycarbonylamino]-4-oxocyclohexyl]carbamate (1 g, 2.76 mmol, 1 eq) (coevaporated twice with toluene) in THF (40 mL) was added dropwise. The reaction was stirred at -78°C for 4 hours. 5% citric acid (50 mL) was added and the mixture was stirred at room temperature. The mixture was diluted with water (50 mL) and extracted with ethyl acetate (2 × 100 mL). The combined organic phases were dried over Na₂SO₄ and evaporated to give 1.43g light yellow oil. The residue was purified by silica cartridge chromatography (ISCO: 50g SiO₂ eluting with 0-50% EtOAc in heptane) to give the title compound (1.07 g, 2.37 mmol, 86.1 % yield). MS (ESP): m/z = 351.2 [M+2H-BOC]⁺.

### Step 6:

### 2-[rac-1,2-cis-1,4-trans-1-Hydroxy-2-[(2-methylpropan-2-yl)oxycarbonylamino]-4-(phenylmethoxycarbonylamino)cyclohexyl]acetic acid and 2-[rac-1,2-trans-1,4-cis-1-hydroxy-2-[(2-methylpropan-2-yl)oxycarbonylamino] -4-(phenylmethoxycarbonylamino)cyclohexyl] acetic acid

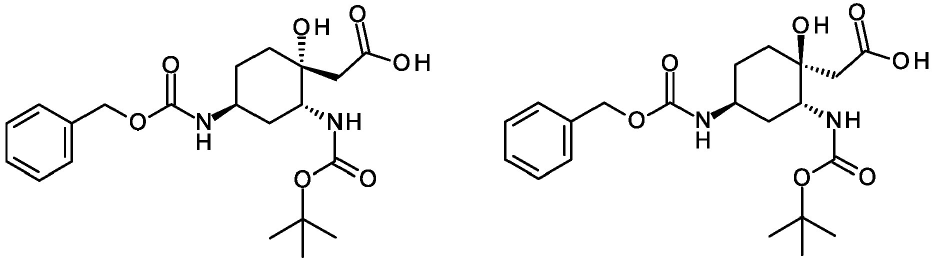

To a solution of ethyl 2-[*rac*-2,4-*trans*-1-hydroxy-2-[(2-methylpropan-2-yl)oxycarbonylamino]-4-(phenylmethoxycarbonylamino)cyclohexyl]acetate (1.07 g, 2.37 mmol, 1 eq) in tetrahydrofuran (75 mL) and water (50 mL) at room temperature was added LiOH (569 mg, 23.7 mmol, 10 eq) and the mixture was stirred for one hour. The mixture was acidified to pH 1 by addition of 3M HCl and then extracted with EtOAc (3 × 50 mL). The combined organic layers were dried with Na₂SO₄, filtered, evaporated and dried in HV to give the mixture of products which were separated by preparative HPLC to give *2*-[*rac*-1,2-*cis*-1,4-*trans*-1-hydroxy-2-[(2-methylpropan-2-yl)oxycarbonylamino]-4-(phenylmethoxycarbonylamino)cyclohexyl] acetic acid (450 mg, 1.07 mmol, 45 % yield) and 2-[*rac*-1,2-*trans*-1,4-*cis*-1-hydroxy-2-[(2-methylpropan-2-yl)oxycarbonylamino]-4-(phenylmethoxycarbonylamino)cyclohexyl]acetic acid (50 mg, 0.12 mmol, 5 % yield) as white solids. MS (ESP): m/z = 323.2 [M+2H-BOC]⁺.

### Step 7:

### Benzyl N-[rac-5,6-cis-5,8-trans-6-[(2-methylpropan-2-yl)oxycarbonylamino]-2-oxo-1-oxa-3-azaspiro[4.5]decan-8-yl]carbamate

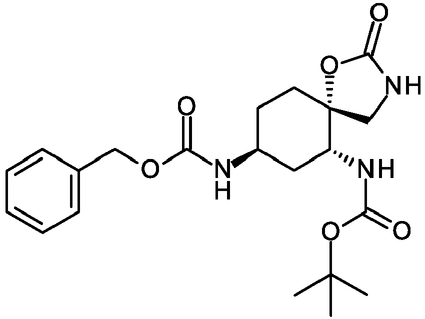

A mixture of 2-[*rac*-1,2-*cis*-1,4-*trans*-1-Hydroxy-2-[(2-methylpropan-2-yl)oxycarbonylamino]-4-(phenylmethoxycarbonylamino)cyclohexyl]acetic acid (350 mg, 828 µmol, 1 eq), Et₃N (126 mg, 173 µl, 1.24 mmol, 1.5 eq) and diphenylphosphoryl azide (342 mg, 267 µL, 1.24 mmol, 1.5 eq) in toluene (38.9 mL) was stirred for 3 h at 80 °C. SiO₂ (3 g) was added and the mixture evaporated to dryness. The reside was purified by silica cartridge chromatography (ISCO: 20 g, from CH₂Cl₂ to 3% MeOH-NH₄OH 10:1/CH₂Cl₂) to give the title compound (364 mg, 781 µmol, 94.3 % yield) as a white solid. MS (ESN): m/z = 464.4 [M+HCOO]⁻.

### Step 8:

### Ethyl 2-[2-nitro-6-[rac-5,6-cis-5,8-trans-6-[(2-methylpropan-2-yl)oxycarbonylamino]-2-oxo-8-(phenylmethoxycarbonylamino)-1-oxa-3-azaspiro[4.5]decan-3-yl]pyridin-3-yl]oxyacetate

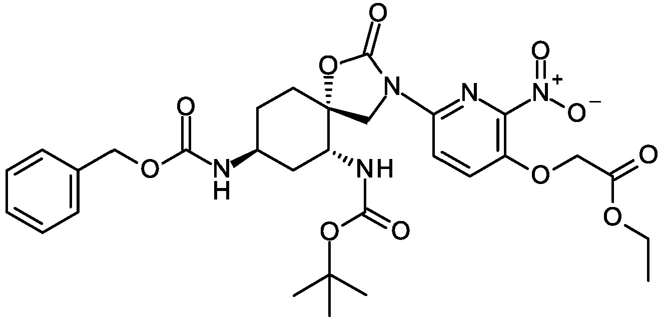

The title compound was prepared by analogy to *tert*-butyl *N*-[(3*R*)-5-oxo-1-[3-oxo-4-(2-trimethylsilylethoxymethyl)pyrido[3,2-b] [1,4]oxazin-6-yl]pyrrolidin-3-yl]carbamate (Intermediate 13, step 1) using ethyl 2-(6-bromo-2-nitropyridin-3-yl)oxyacetate in place of 6-bromo-4-(2-trimethylsilylethoxymethyl)pyrido[3,2-b][1,4]oxazin-3-one and benzyl *N*-[*rac*-5,6-*cis*-5,8-*trans*-6-[(2-methylpropan-2-yl)oxycarbonylamino]-2-oxo-1-oxa-3-azaspiro[4.5]decan-8-yl]carbamate in place of *tert*-butyl *N*-[(3*R*)-5-oxopyrrolidin-3-yl]carbamate and was obtained as an off-white solid. MS (ESN): m/z = 688.54 [M+HCOO]⁻.

### Step 9:

### Benzyl N-[rac-5,6-cis-5,8-trans-6-[(2-methylpropan-2-yl)oxycarbonylamino]-2-oxo-3-(3-oxo-4H-pyrido[3,2-b][1,4]oxazin-6-yl)-1-oxa-3-azaspiro[4.5]decan-8-yl]carbamate

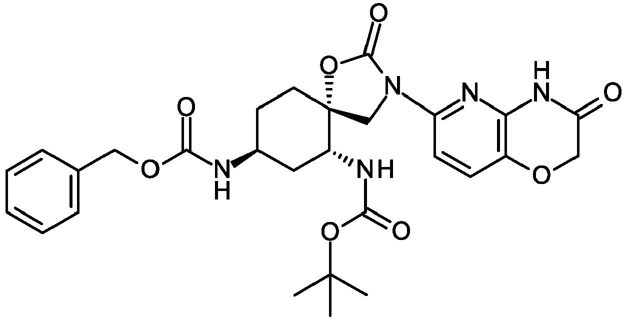

The title compound was prepared by analogy to 6-[5-[2-[*tert*-butyl(dimethyl)silyl]oxyethyl]-2-oxo-1,3-oxazolidin-3-yl]-4*H*-pyrido[3,2-b][1,4]oxazin-3-one (Intermediate 1, step 5) and was obtained as an off-white solid. MS (ESN): m/z = 566.4 [M-H]⁻.

### Step 10:

### tert-Butyl N-[rac-5,6-cis-5,8-trans-8-amino-2-oxo-3-(3-oxo-4H-pyrido[3,2-b][1,4]oxazin-6-yl)-1-oxa-3-azaspiro[4.5]decan-6-yl]carbamate

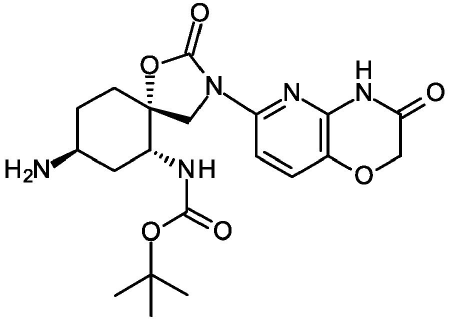

To a solution of benzyl *N*-[*rac*-5,6-*cis*-5,8-*trans*-6-[(2-methylpropan-2-yl)oxycarbonylamino]-2-oxo-3-(3-oxo-4*H*-pyrido[3,2-b][1,4]oxazin-6-yl)-1-oxa-3-azaspiro[4.5]decan-8-yl]carbamate (361 mg, 636 µmol, 1 eq) in THF (82.9 mL) and water (829 µL) under argon was added Pd/C 10% (67.7 mg). The mixture was stirred under an atmosphere of hydrogen for 1 h. The mixture was filtered and the filtrate was evaporated to give a residue which was triturated with diethyl ether, filtered and dried to give the title compound (195 mg, 450 µmol, 70.7 % yield) as an off white solid. MS (ESP): m/z = 434.3 [M+H]⁺.

### Intermediate 113

### tert-Butyl N-[2-[(6-formyl-1,4-dimethyl-6,7-dihydro-5H-cyclopenta[c]pyridin-3-yl)oxy]ethyl]carbamate

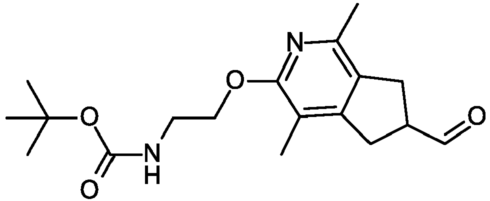

### Step 1:

### Dimethyl 1,4-dimethyl-3-[2-[(2-methylpropan-2-yl)oxycarbonylamino]ethoxy]-5,7-dihydrocyclopenta[c]pyridine-6,6-dicarboxylate

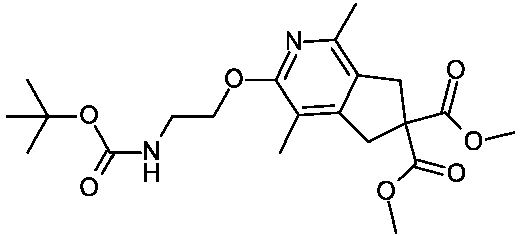

Dimethyl 1,4-dimethyl-3-oxo-5,7-dihydro-2H-cyclopenta[c]pyridine-6,6-dicarboxylate (Intermediate 64, step 2, 610 mg, 2.18 mmol, 1 eq) was dissolved in dichloromethane (30 mL) and dioxane (30 mL). *tert*-Butyl (2-hydroxyethyl)carbamate (528 mg, 3.28 mmol, 1.5 eq) as solution in dichloromethane (2 mL), tri-n-butylphosphine (663 mg, 808 µl, 3.28 mmol, 1.5 eq) and azodicarboxylic dipiperidide (827 mg, 3.28 mmol, 1.5 eq) were added. The resulting solution was stirred at RT for 18 h. A solution of tert-butyl (2-hydroxyethyl)carbamate (352 mg, 2.18 mmol, 1 eq), tri-*n*-butylphosphine (442 mg, 539 µl, 2.18 mmol, 1 eq) and azodicarboxylic dipiperidide (551 mg, 2.18 mmol, 1 eq) in dichloromethane (3 mL) was added to the now yellow-white suspension. The mixture was stirred for 1 h 45 min. at RT, then water was added and the mixture was extracted with EtOAc (3 x). The organic layers were washed with water and brine, dried over Na₂SO₄ and evaporated. The crude product was adsorbed on adsorbent and purified by flash column chromatography (120 g silica gel; heptane/EtOAc 90:10 to 80:20 to DCM/MeOH 95:5) to give the title compound (578 mg, 1.37 mmol, 62.6 % yield) as a colorless gum. MS (ESP): m/z = 423.3 [M+H]⁺.

### Steps 2 to 4:

### tert-Butyl N-[2-[(6-formyl-1,4-dimethyl-6,7-dihydro-5H-cyclopenta[c]pyridin-3-yl)oxy]ethyl]carbamate

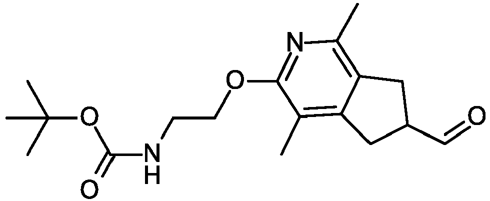

The title compound was prepared by analogy to 1-chloro-3-methoxy-6,7-dihydro-5*H-*cyclopenta[c]pyridine-6-carbaldehyde (Intermediate 37, steps 6 to 8) and was obtained as a colourless gum. MS (ESP): m/z = 335.2 [M+H]⁺.

### Intermediate 114

### tert-Butyl N-[1-[(6-formyl-1,4-dimethyl-6,7-dihydro-5H-cyclopenta[c]pyridin-3-yl)oxymethyl]cyclopropyl]carbamate

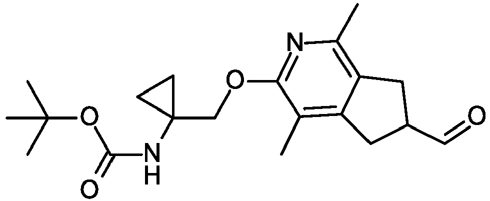

### Steps 1 and 2:

### Methyl 1,4-dimethyl-3-[[1-[(2-methylpropan-2-yl)oxycarbonylamino]cyclopropyl]methoxy]-6,7-dihydro-5H-cyclopenta [c]pyridine-6-carboxylate

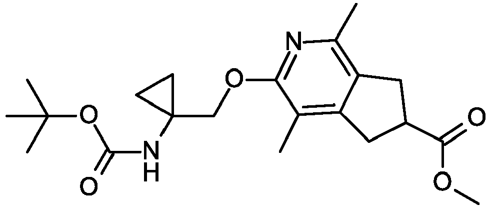

The title compound was prepared by analogy to *tert*-butyl N-[2-[(6-formyl-1,4-dimethyl-6,7-dihydro-5*H*-cyclopenta[c]pyridin-3-yl)oxy]ethyl]carbamate (Intermediate 113, steps 1 and 2) using *tert*-butyl *N*-[1-(hydroxymethyl)cyclopropyl]carbamate (CAS# 107017-73-2) in place of *tert*-butyl (2-hydroxyethyl)carbamate and was obtained as a white solid. MS (ESP): m/z = 391.3 [M+H]⁺.

### Step 3:

### tert-Butyl N-[1-[(6-formyl-1,4-dimethyl-6,7-dihydro-5H-cyclopenta[c]pyridin-3-yl)oxymethyl]cyclopropyl]carbamate

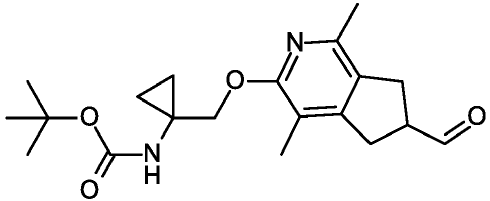

The title compound was prepared by analogy to 2-[(6-formyl-1,4-dimethyl-6,7-dihydro-5*H-*cyclopenta[c]pyridin-3-yl)oxy]-*N*-methylacetamide (Intermediate 72, step 7) and was obtained as a colourless gum. MS (ESP): m/z = 361.3 [M+H]⁺.

### Intermediate 115

### tert-Butyl N-[2-[(6-formyl-1,4-dimethyl-6,7-dihydro-SH-cyclopenta[c]pyridin-3-yl)oxy]ethyl]-N-methylcarbamate

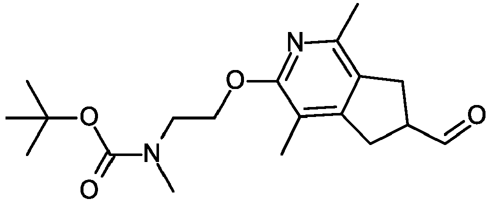

The title compound was prepared by analogy to *tert*-butyl N-[2-[(6-formyl-1,4-dimethyl-6,7-dihydro-5H-cyclopenta[c]pyridin-3-yl)oxy]ethyl]carbamate (Intermediate 113) using *tert*-butyl N-(2-hydroxyethyl)-N-methylcarbamate (CAS# 57561-39-4) in place of *tert*-butyl (2-hydroxyethyl)carbamate and was obtained as a colorless oil. MS (ESP): m/z = 411.3 [M+H]⁺.

### Intermediate 116

### tert-Butyl N-[2-[(4-chloro-6-formyl-6,7-dihydro-SH-cyclopenta[c]pyridin-3-yl)oxy]ethyl]carbamate

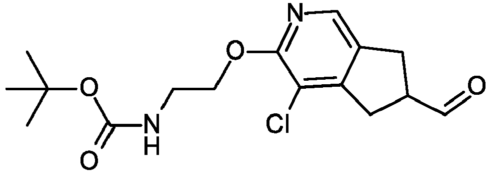

### Step 1:

### Dimethyl 4-chloro-3-oxo-5,7-dihydro-2H-cyclopenta[c]pyridine-6,6-dicarboxylate

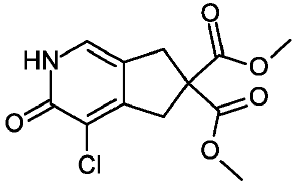

*N*-Chlorosuccinimide (63.8 mg, 0.478 mmol, 1.2 eq) was added to a solution of dimethyl 3-oxo-5,7-dihydro-2*H*-cyclopenta[c]pyridine-6,6-dicarboxylate (Intermediate 37, step 2, 100 mg, 0.398 mmol, 1 eq) in *N*,*N*-dimethylformamide (1.5 mL) at 0 °C. The mixture was stirred at RT for 90 min. The mixture was stirred at 40 °C for 4 h. Additional *N*-chlorosuccinimide (26.6 mg, 0.199 mmol, 0.5 eq) was added and the solution was stirred at 40 °C for 30 min. The reaction mixture was allowed to cool to RT overnight. Water was added and the mixture was extracted with EtOAc (3 x). The organic layers were washed with water (2 x) and brine, dried over Na₂SO₄ and evaporated. The crude product was purified by flash column chromatography (20 g silica gel; DCM/MeOH 100:0 to 97:3) to give the title compound (41 mg, 0.144 mmol, 36.1 % yield) as a white solid. MS (ESP): m/z = 286.1 [M+H]⁺.

### Steps 2 to 4:

### tert-Butyl N-[2-[(4-chloro-6-formyl-6,7-dihydro-5H-cyclopenta[c]pyridin-3-yl)oxy]ethyl]carbamate

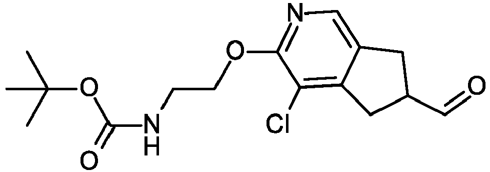

The title compound was prepared by analogy to *tert*-butyl *N*-[1-[(6-formyl-1,4-dimethyl-6,7-dihydro-5*H*-cyclopenta[c]pyridin-3-yl)oxymethyl]cyclopropyl]carbamate (Intermediate 114) using dimethyl 4-chloro-3-oxo-5,7-dihydro-2*H*-cyclopenta[c]pyridine-6,6-dicarboxylate in place of dimethyl 1,4-dimethyl-3-oxo-5,7-dihydro-2*H*-cyclopenta[c]pyridine-6,6-dicarboxylate and *tert*-butyl (2-hydroxyethyl)carbamate in place of *tert*-butyl *N*-[1-(hydroxymethyl)cyclopropyl]carbamate and was obtained as a colorless gum. MS (ESP): m/z = 341.1 [M+H]⁺.

### Intermediate 117

### tert-Butyl N-[1-[(6-formyl-1-methyl-6,7-dihydro-5H-cyclopenta[c]pyridin-3-yl)oxymethyl]cyclopropyl]carbamate

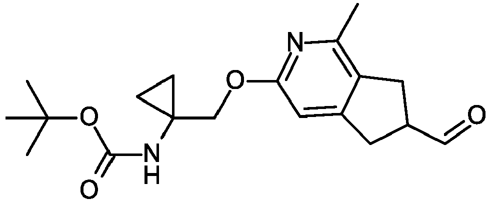

### Step 1:

### Dimethyl 1-methyl-3-phenylmethoxy-5,7-dihydrocyclopenta[c]pyridine-6,6-dicarboxylate

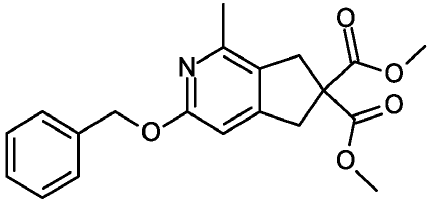

The title compound was prepared by analogy to dimethyl 1-bromo-2-[(4-methoxyphenyl)methyl]-4-methyl-3-oxo-5,7-dihydrocyclopenta[c]pyridine-6,6-dicarboxylate (Intermediate 72, step 1) using benzylisocyanate in place of 4-methoxybenzylisocyanate and dimethyl 2-but-2-ynyl-2-prop-2-ynylpropanedioate (CAS# 188025-73-2) in place of dimethyl 2-(3-bromoprop-2-ynyl)-2-but-2-ynylpropanedioate and was obtained as a light yellow solid. MS (ESP): m/z = 356.3 [M+H]⁺.

### Step 2:

### Dimethyl 1-methyl-3-oxo-5,7-dihydro-2H-cyclopenta[c]pyridine-6,6-dicarboxylate

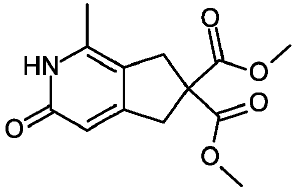

The title compound was prepared by analogy to dimethyl 3-oxo-5,7-dihydro-2*H-*cyclopenta[c]pyridine-6,6-dicarboxylate (Intermediate 37, step 2) and was obtained as an off-white solid. MS (ESP): m/z = 266.2 [M+H]⁺.

### Steps 3 to 5:

### tert-Butyl N-[1-[(6-formyl-1-methyl-6,7-dihydro-5H-cyclopenta[c]pyridin-3-yl)oxymethyl]cyclopropyl]carbamate

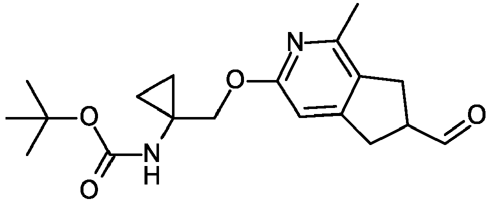

The title compound was prepared by analogy to *tert-*butyl *N*-[1-[(6-formyl-1,4-dimethyl-6,7-dihydro-5*H*-cyclopenta[c]pyridin-3-yl)oxymethyl]cyclopropyl]carbamate (Intermediate 114) using dimethyl 1-methyl-3-oxo-5,7-dihydro-2*H*-cyclopenta[c]pyridine-6,6-dicarboxylate in place of dimethyl 1,4-dimethyl-3-oxo-5,7-dihydro-2*H*-cyclopenta[c]pyridine-6,6-dicarboxylate and was obtained as a colorless oil. MS (ESP): m/z = 347.3 [M+H]⁺.

### Intermediate 118

### tert-Butyl N-[3-[(6-formyl-1,4-dimethyl-6,7-dihydro-5H-cyclopenta[c]pyridin-3-yl)oxy]propyl]carbamate

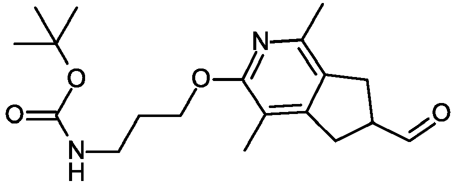

### Step 1:

### Dimethyl 3-[3-(1,3-dioxoisoindol-2-yl)propoxy]-1,4-dimethyl-5,7-dihydrocyclopenta[c]pyridine-6,6-dicarboxylate

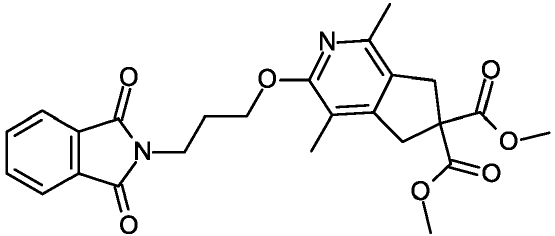

The title compound was prepared by analogy to dimethyl 1,4-dimethyl-3-[2-[(2-methylpropan-2-yl)oxycarbonylamino]ethoxy]-5,7-dihydrocyclopenta[c]pyridine-6,6-dicarboxylate (Intermediate 113, step 1) using 2-(3-hydroxypropyl)isoindole-1,3-dione (CAS# 883-44-3) in place of *tert-*butyl (2-hydroxyethyl)carbamate and was obtained as a white solid. MS (ESP): m/z = 467.2 [M+H]⁺.

### Step 2:

### Dimethyl 3-(3-aminopropoxy)-1,4-dimethyl-5,7-dihydrocyclopenta[c]pyridine-6,6-dicarboxylate

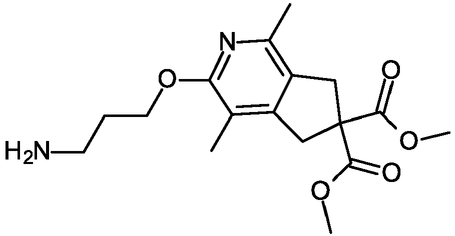

A suspension of dimethyl 3-[3-(1,3-dioxoisoindol-2-yl)propoxy]-1,4-dimethyl-5,7-dihydrocyclopenta[c]pyridine-6,6-dicarboxylate (378 mg, 0.810 mmol, 1 eq) and hydrazine hydrate (42.6 mg, 41.3 µl, 0.851 mmol, 1.05 eq) was stirred at reflux 5 h. Additional hydrazine hydrate (12.2 mg, 11.8 µl, 0.243 mmol, 0.3 eq) was added and the now colorless solution was stirred at reflux for 18 h. A white solid precipitated, which was filtered off, washed with DCM/MeOH 95:5 and dried to give the title compound (127 mg) as a white solid. The mother liquor was concentrated to give the title compound (234 mg) as a white solid. The two crude products were combined and used without purification for the next step. MS (ESP): m/z = 337.2 [M+H]⁺.

### Step 3:

### Dimethyl 1,4-dimethyl-3-[3-[(2-methylpropan-2-yl)oxycarbonylamino]propoxy]-5,7-dihydrocyclopenta[c]pyridine-6,6-dicarboxylate

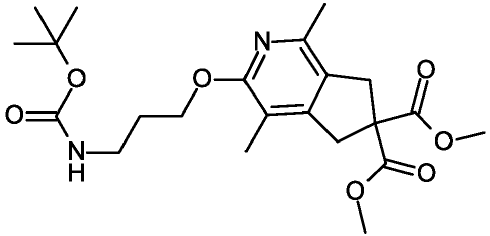

Dimethyl 3-(3-aminopropoxy)-1,4-dimethyl-5,7-dihydrocyclopenta[c]pyridine-6,6-dicarboxylate (356 mg, 0.688 mmol, 1 eq) was suspended in a mixture of dichloromethane (8 mL) and tetrahydrofuran (5 mL). Di-tert.-butyl-dicarbonate (165 mg, 0.757 mmol, 1.1) eq was added and the white suspension was stirred at RT for 2 h. Water was added and the mixture was extracted with EtOAc (3 x). The organic layers were washed with water and brine, dried over Na₂SO₄ and evaporated. The residue was adsorbed on adsorbent and purified by flash chromatography (50 g silica gel; heptane/EtOAc 95:5 to 80:20) to give impure title compound. The material was purified by preparative HPLC to give the title compound which still contained some impurities. The material was taken up in water and extracted with EtOAc (3 x). The organic layers were washed with brine, dried over Na₂SO₄ and evaporated to give the title compound (282 mg) as a colorless gum. MS (ESP): m/z = 437.3 [M+H]⁺.

### Steps 4 to 5:

### tert-Butyl N-[3-[(6-formyl-1,4-dimethyl-6,7-dihydro-SH-cyclopenta[c]pyridin-3-yl)oxy]propyl]carbamate

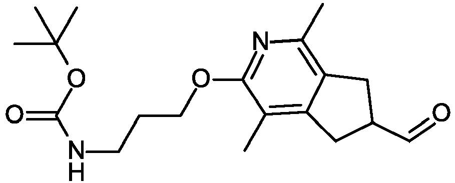

The title compound was prepared by analogy to *tert*-butyl *N*-[1-[(6-formyl-1,4-dimethyl-6,7-dihydro-5*H*-cyclopenta[c]pyridin-3-yl)oxymethyl]cyclopropyl]carbamate (Intermediate 114, steps 2 and 3) and was obtained as a colorless gum. MS (ESP): m/z = 349.2 [M+H]⁺.

### Intermediate 119

### tert-Butyl N-[2-[(7-chloro-2-formyl-2,3-dihydro-1H-inden-5-yl)oxy]ethyl]carbamate

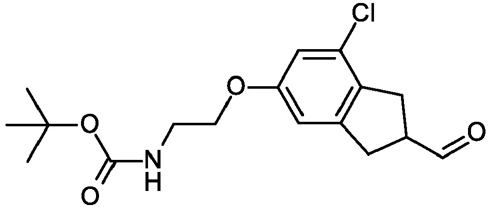

### Steps 1 to 3:

### Ethyl 4-chloro-6-hydroxy-2,3-dihydro-1H-indene-2-carboxylate

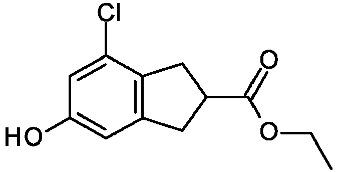

The title compound was prepared by analogy to ethyl 4-bromo-5-hydroxy-2,3-dihydro-1*H-*indene-2-carboxylate (Intermediate 36, steps 1 to 3) using 4-chloro-6-methoxy-2,3-dihydroinden-1-one (CAS# 1092347-56-2) in place of 4-bromo-5-methoxy-indan-1-one and was obtained as an off white solid. MS (ESN): m/z = 239.1 [M-H]⁻.

### Step 4:

### Ethyl 4-chloro-6-[2-[(2-methylpropan-2-yl)oxycarbonylamino]ethoxy]-2,3-dihydro-1H-indene-2-carboxylate

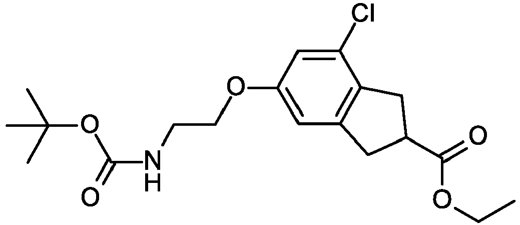

The title compound was prepared by analogy to dimethyl 1,4-dimethyl-3-[2-[(2-methylpropan-2-yl)oxycarbonylamino]ethoxy]-5,7-dihydrocyclopenta[c]pyridine-6,6-dicarboxylate (Intermediate 113, step 1) and was obtained as a colorless oil. MS (ESP): m/z = 328.1 [M+H-tBu]⁺.

### Steps 5 and 6:

### tert-Butyl N-[2-[(7-chloro-2-formyl-2,3-dihydro-1H-inden-5-yl)oxy]ethyl]carbamate

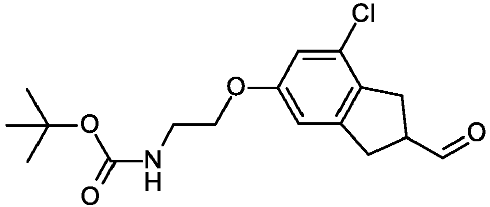

The title compound was prepared by analogy to 1-chloro-6,7-dihydro-5*H*-cyclopcnta[c]pyridinc-6-carbaldehyde (Intermediate 19, steps 3 and 4) and was obtained as a colorless oil. MS (ESP): m/z = 284.1 [M+H-tBu]⁺.

### Intermediate 120

### tert-Butyl N-[1-[(7-chloro-2-formyl-2,3-dihydro-1H-inden-5-yl)oxymethyl]cyclopropyl]carbamate

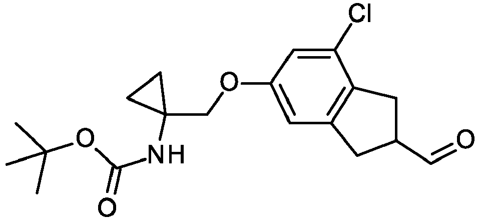

The title compound was prepared by analogy to tert-butyl *N*-[2-[(7-chloro-2-formyl-2,3-dihydro-1*H*-inden-5-yl)oxy]ethyl]carbamate (Intermediate 119, steps 4 to 6) using *tert*-butyl N-[1-(hydroxymethyl)cyclopropyl]carbamate in place of *tert*-butyl (2-hydroxyethyl)carbamate and was obtained as a colorless oil. MS (ESP): m/z = 310.2 [M+H-tBu]⁺.

### Intermediate 121

### tert-Butyl N-[2-[(2-amino-7-chloro-2,3-dihydro-1H-inden-5-yl)oxy]ethyl]carbamate

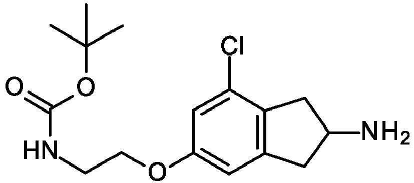

The title compound was prepared by analogy to *tert*-butyl *N*-[2-[(2-amino-4-chloro-2,3-dihydro-1*H*-inden-5-yl)oxy]ethyl]carbamate (Intermediate 76) using ethyl 4-chloro-6-[2-[(2-methylpropan-2-yl)oxycarbonylamino]ethoxy]-2,3-dihydro-1*H*-indene-2-carboxylate (Intermediate 119, step 4) in place of ethyl 4-chloro-5-[2-[(2-methylpropan-2-yl)oxycarbonylamino]ethoxy]-2,3-dihydro-1*H*-indene-2-carboxylate and was obtained as a brown oil. MS (ESP): m/z = 327.2 [M+H]⁺.

### Intermediate 122

### tert-Butyl N-[1-[(2-amino-7-chloro-2,3-dihydro-1H-inden-5-yl)oxymethyl]cyclopropyl]carbamate

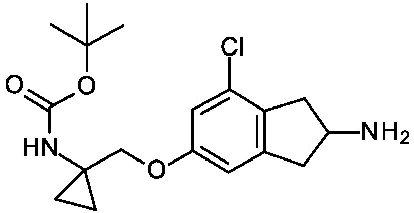

The title compound was prepared by analogy to *tert*-butyl *N*-[2-[(2-amino-7-chloro-2,3-dihydro-1*H*-inden-5-yl)oxy]ethyl]carbamate (Intermediate 121) using *tert*-butyl *N*-[1-(hydroxymethyl)cyclopropyl]carbamate in place of *tert*-butyl *N*-(2-hydroxyethyl)carbamate and was obtained as a white solid. MS (ESP): m/z = 353.2 [M+H]⁺.

### Intermediate 123

### tert-Butyl N-[1-[(4-chloro-6-formyl-6,7-dihydro-5H-cyclopenta[c]pyridin-3-yl)oxymethyl]cyclopropyl]carbamate

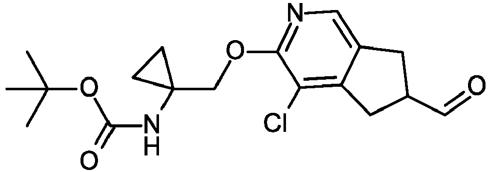

The title compound was prepared by analogy to *tert*-butyl *N*-[2-[(4-chloro-6-formyl-6,7-dihydro-5*H*-cyclopenta[c]pyridin-3-yl)oxy]ethyl]carbamate (Intermediate 116, steps 2 to 4) using *tert-*butyl *N*-[1-(hydroxymethyl)cyclopropyl]carbamate in place of *tert*-butyl *N*-(2-hydroxyethyl)carbamate and was obtained as a colorless gum. MS (ESP): m/z = 367.2 [M+H]⁺.

### Intermediate 124

### 6-(2,4-trans-2-Amino-6-oxo-5-oxa-7-azaspiro[3.4]octan-7-yl)-4-(2-trimethylsilylethoxymethyl)pyrido[3,2-b][1,4]oxazin-3-one

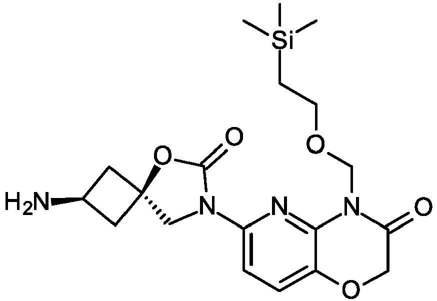

### Steps 1 to 3:

### Benzyl N-(2,4-cis-6-oxo-5-oxa-7-azaspiro[3.4]octan-2-yl)carbamate

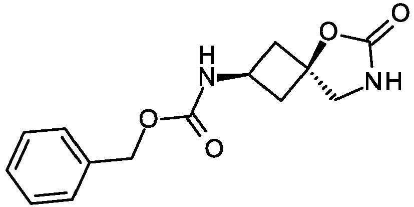

The title compound was prepared by analogy to benzyl *N*-(2,4-*trans*-6-oxo-5-oxa-7-azaspiro[3.4]octan-2-yl)carbamate (Intermediate 67, steps 2 to 4) using benzyl *N-*(3,5*-cis*-1-oxaspiro[2.3]hexan-5-yl)carbamate (Intermediate 67, step 1) in place of benzyl *N*-(3,5-*trans*-1-oxaspiro[2.3]hexan-5-yl)carbamate and was obtained as a white solid. MS (ESP): m/z = 277.1 [M+H]⁺.

### Step 4:

### Benzyl N-[6-oxo-7-[3-oxo-4-(2-trimethylsilylethoxymethyl)pyrido[3,2-b][1,4]oxazin-6-yl]-5-oxa-7-azaspiro[3.4]octan-2-yl]carbamate

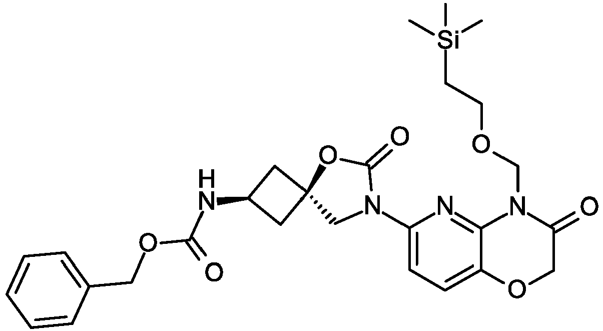

The title compound was prepared by analogy to *tert*-butyl *N*-[(3*R*)-5-oxo-1-[3-oxo-4-(2-trimethylsilylethoxymethyl)pyrido[3,2-b] [1,4]oxazin-6-yl]pyrrolidin-3-yl]carbamate (Intermediate 13, step 1) and was obtained as a white waxy solid. MS (ESP): m/z = 555.3 [M+H]⁺.

### Step 5:

### 6-(2,4-trans-2-Amino-6-oxo-5-oxa-7-azaspiro[3.4]octan-7-yl)-4-(2-trimethylsilylethoxymethyl)pyrido[3,2-b] [1,4]oxazin-3-one

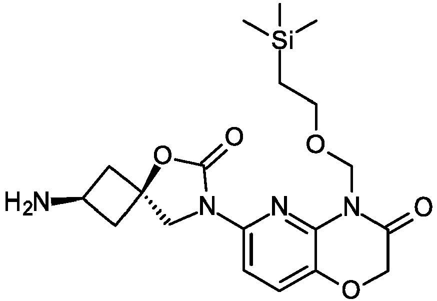

The title compound was prepared by analogy to 6-[*rac*-3a,7a-*trans*-3a,6-*cis*-6-amino-2-oxo-3a,4,5,6,7,7a-hexahydro-1,3-benzoxazol-3-yl]-4*H*-pyrido[3,2-b][1,4]oxazin-3-one (Intermediate 20, step 6) and was obtained as a dark brown oil. MS (ESP): m/z = 421.2 [M+H]⁺.

### Intermediate 125

### tert-Butyl 3-[(6-formyl-1,4-dimethyl-6,7-dihydro-5H-cyclopenta[c]pyridin-3-yl)oxymethyl]azetidine-1-carboxylate

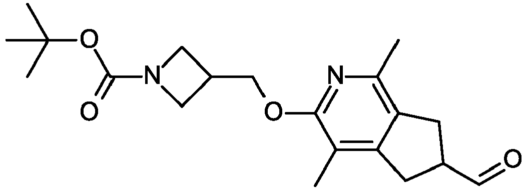

### Step 1:

### Dimethyl 1,4-dimethyl-3-[[1-[(2-methylpropan-2-yl)oxycarbonyl]azetidin-3-yl]methoxy]-5,7-dihydrocyclopenta[c]pyridine-6,6-dicarboxylate

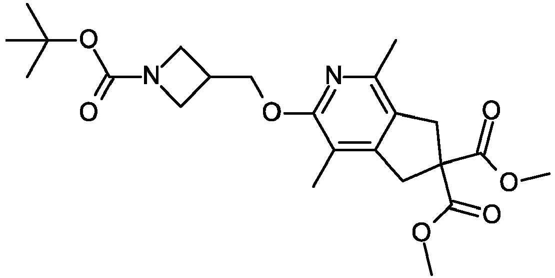

To a mixture of dimethyl 1,4-dimethyl-3-oxo-5,7-dihydro-2*H*-cyclopenta[c]pyridine-6,6-dicarboxylate (Intermediate 64, step 2, 400.0 mg, 1.43 mmol, 1 eq) and *tert*-butyl 3-(bromomethyl)azetidine-1-carboxylate (CAS# 253176-93-1, 540 mg, 2.2 mmol, 1.51 eq) in DMF (13.33 mL) was added caseium carbonate (1170.0 mg, 3.59 mmol, 2.51 eq). The reaction mixture was heated to 50 °C and stirred for 12 h. The reaction was filtered and the filter cake was washed with ethyl acetate (30 mL). The filtrate was concentrated in vacuo to give a crude product which was purified by silica gel column chromatography (petroleum ether:ethyl acetate = 20:1 to 1:1 ) to give the title compound (400 mg, 0.890 mmol, 62.3 % yield) as a colorless oil. MS (ESP): m/z = 449.1 [M+H]⁺.

### Steps 2 to 4:

### tert-Butyl 3-[(6-formyl-1,4-dimethyl-6,7-dihydro-5H-cyclopenta[c]pyridin-3-yl)oxymethyl]azetidine-1-carboxylate

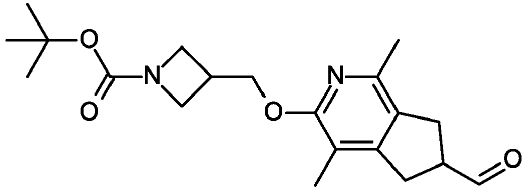

The title compound was prepared by analogy to 1-chloro-3-methoxy-6,7-dihydro-5*H-*cyclopenta[c]pyridine-6-carbaldehyde (Intermediate 37, steps 6 to 8) and was obtained as a colorless oil. MS (ESP): m/z = 361.1 [M+H]⁺.

### Intermediate 126

### tert-Butyl N-[1-[(7-cyano-2-formyl-2,3-dihydro-1H-inden-5-yl)oxymethyl]cyclopropyl]carbamate

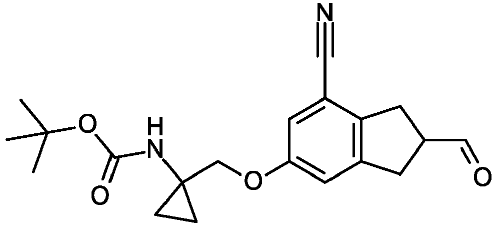

The title compound was prepared by analogy to *tert*-butyl *N*-[1-[(7-chloro-2-formyl-2,3-dihydro-1*H*-inden-5-yl)oxymethyl]cyclopropyl]carbamate (Intermediate 120) using ethyl 4-cyano-6-hydroxy-2,3-dihydro-1*H*-indene-2-carboxylate (Intermediate 70, step 4) in place of ethyl 4-chloro-6-hydroxy-2,3-dihydro-1*H*-indene-2-carboxylate and was obtained as a white foam. MS (ESP): m/z = 301.2 [M+H-tBu]⁺.

### Intermediate 127

### tert-Butyl 2-[(7-cyano-2-formyl-2,3-dihydro-1H-inden-5-yl)oxymethyl]azetidine-1-carboxylate

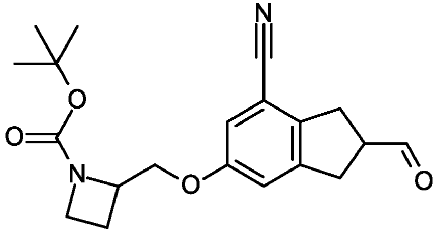

The title compound was prepared by analogy to *tert*-butyl N-[1-[(7-cyano-2-formyl-2,3-dihydro-1*H*-inden-5-yl)oxymethyl]cyclopropyl]carbamate (Intermediate 126) using *tert*-butyl 2-(hydroxymethyl)azetidine-1-carboxylate (CAS# 174346-82-8) in place of *tert*-butyl *N*-[1-(hydroxymethyl)cyclopropyl]carbamate and was obtained as a colorless oil. MS (ESP): m/z = 301.2 [M+H-tBu]⁺.

### Intermediate 128

### tert-Butyl N-[2-[(7-cyano-2-formyl-2,3-dihydro-1H-inden-5-yl)oxy]ethyl]carbamate

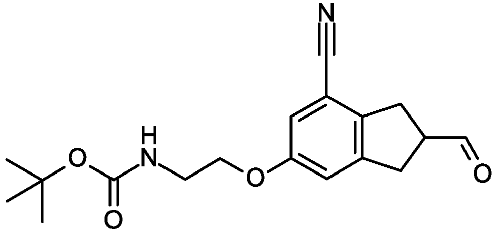

The title compound was prepared by analogy to *tert*-butyl *N*-[1-[(7-cyano-2-formyl-2,3-dihydro-1*H*-inden-5-yl)oxymethyl]cyclopropyl]carbamate (Intermediate 126) using *tert*-butyl *N*-(2-hydroxyethyl)carbamate in place of *tert*-butyl *N*-[1-(hydroxymethyl)cyclopropyl]carbamate and was obtained as a white foam. MS (ESP): m/z = 275.2 [M+H-tBu]⁺.

### Intermediate 129

### tert-Butyl N-cyclopropyl-N-[2-[(6-formyl-1,4-dimethyl-6,7-dihydro-5H-cyclopenta[c]pyridin-3-yl)oxy]ethyl]carbamate

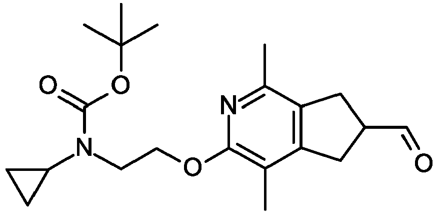

### Step 1:

### Dimethyl 3-[2-[cyclopropyl-[(2-methylpropan-2-yl)oxycarbonyl]amino]ethoxy]-1,4-dimethyl-5,7-dihydrocyclopenta[c]pyridine-6,6-dicarboxylate

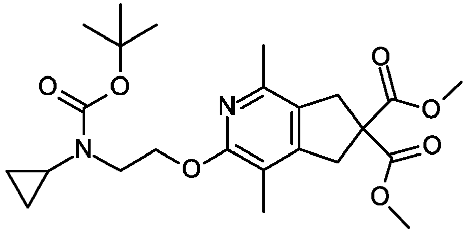

The title compound was prepared by analogy to dimethyl 1,4-dimethyl-3-[(5-oxomorpholin-2-yl)methoxy]-5,7-dihydrocyclopenta[c]pyridine-6,6-dicarboxylate (Intermediate 64, step 3) using *tert*-butyl *N*-cyclopropyl-N-(2-hydroxyethyl)carbamate (CAS# 1153134-59-8) in place of 6-(hydroxymethyl)morpholin-3-one and was obtained as a colorless gum. MS (ESP): m/z = 463.4 [M+H]⁺.

### Steps 2 and 3:

### tert-Butyl N-cyclopropyl-N-[2-[(6-formyl-1,4-dimethyl-6,7-dihydro-5H-cyclopenta[c]pyridin-3-yl)oxy]ethyl]carbamate

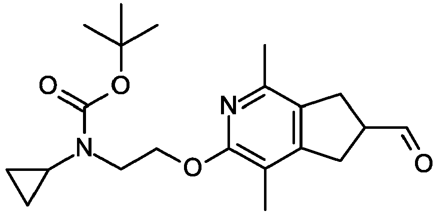

The title compound was prepared by analogy to *tert*-butyl *N*-[1-[(6-formyl-1,4-dimethyl-6,7-dihydro-5*H*-cyclopenta[c]pyridin-3-yl)oxymethyl]cyclopropyl]carbamate (Intermediate 114, steps 2 and 3) and was obtained as a colorless gum. MS (ESP): m/z = 375.4 [M+H]⁺.

### Intermediate 130

### tert-Butyl 3-[(7-cyano-2-formyl-2,3-dihydro-1H-inden-5-yl)oxy]azetidine-1-carboxylate

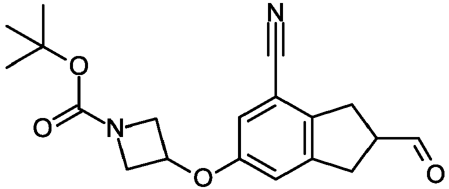

The title compound was prepared by analogy to *tert*-butyl 3-[(7-fluoro-2-formyl-2,3-dihydro-1*H-*inden-5-yl)oxy]azetidine-1-carboxylate (Intermediate 103) using ethyl 4-cyano-6-hydroxy-2,3-dihydro-1*H*-indene-2-carboxylate (Intermediate 70, step 4) in place of ethyl 4-fluoro-6-hydroxy-indane-2-carboxylate and was obtained as a colorless gum. MS (ESP): m/z = 287.2 [M+H-tBu]⁺.

### Intermediate 131

### tert-Butyl 3-[(7-cyano-2-formyl-2,3-dihydro-1H-inden-5-yl)oxymethyl] azetidine-1-carboxylate

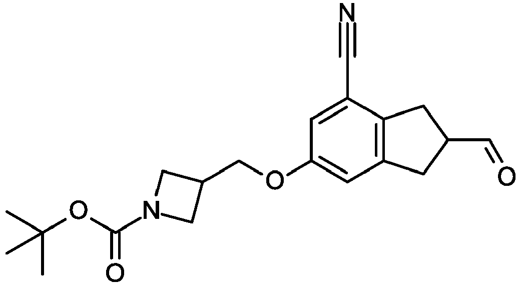

The title compound was prepared by analogy to *tert*-butyl 3-[(7-cyano-2-formyl-2,3-dihydro-1*H-*inden-5-yl)oxy]azetidine-1-carboxylate (Intermediate 130) using *tert*-butyl 3-(bromomethyl)azetidine-1-carboxylate (CAS# 253176-93-1) in place of *tert*-butyl 3-bromoazetidine-1-carboxylate and was obtained as a colorless oil. MS (ESP): m/z = 301.2 [M+H-tBu]⁺.

### Intermediate 132

### tert-Butyl N-[1-[(7-cyano-2-formyl-2,3-dihydro-1H-inden-5-yl)oxy]propan-2-yl]carbamate

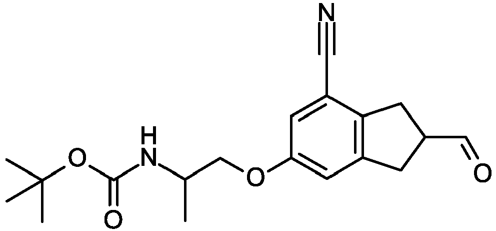

The title compound was prepared by analogy to *tert*-butyl 3-[(7-cyano-2-formyl-2,3-dihydro-1*H-*inden-5-yl)oxy]azetidine-1-carboxylate (Intermediate 130) using *tert*-butyl *N*-(1-bromopropan-2-yl)carbamate (CAS# 1391026-59-7) in place of *tert*-butyl 3-bromoazetidine-1-carboxylate and was obtained as a colorless oil. MS (ESP): m/z = 289.2 [M+H-tBu]⁺.

### Intermediate 133

### tert-Butyl N-[rac-1,2-trans-4-chloro-2-(2-oxoethyl)-2,3-dihydro-1H-inden-1-yl]carbamate

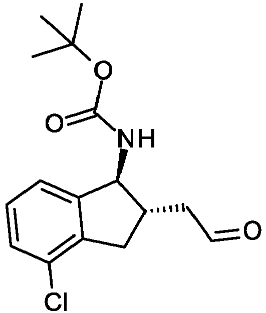

### Step 1:

### tert-Butyl N-(rac-1,2-trans-4-chloro-2-ethenyl-2,3-dihydro-1H-inden-1-yl)carbamate

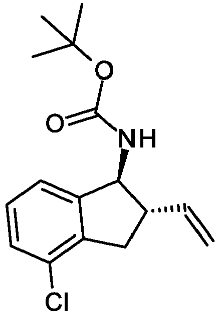

Methyltriphenylphosphonium bromide (264 mg, 738 µmol, 2.4 eq) was co-evaporated with toluene 3 times before suspending in THF (2.5 mL) at RT under argon. Potassium *tert*-butoxide (1M solution, 615 µL, 615 µmol, 2 eq) was added dropwise and the solution was stirred for 45 min. before adding dropwise *tert*-butyl *N*-(*rac*-1,2-*trans*-4-chloro-2-formyl-2,3-dihydro-1*H-*inden-1-yl)carbamate (Intermediate 92, 91 mg, 308 µmol, 1 eq) in THF (2.5 mL) and stirring for a further 18 h. The reaction mixture was quenched with ice water then the mixture extracted three times with DCM before concentrating in vacuo and purifying the residue by chromatography (ISCO 10 g, 0% to 30% EtOAc in heptane) to give the title compound (76.7 mg, 261 µmol, 84.9 % yield) as a white solid. MS (ESN): m/z = 238.1 [M+HCOO]⁻.

### Step 2:

### tert-Butyl N-[rac-1,2-trans-4-chloro-2-(2-hydroxyethyl)-2,3-dihydro-1H-inden-1-yl]carbamate

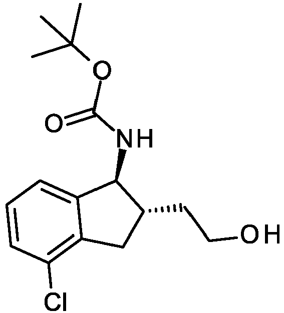

To *tert*-butyl *N*-(*rac*-1,2-*trans*-4-chloro-2-ethenyl-2,3-dihydro-1*H*-inden-1-yl)carbamate (76.7 mg, 261 µmol, 1 eq) dissolved in THF (4 mL) was added borane tetrahydrofuran complex (287 µL, 287 µmol, 1.1 eq) at -10 °C under argon. The mixture was warmed to RT and stirred for 3 hours. The reaction mixture was cooled to -10 °C before adding NaOH (300 µL, 600 µmol, 2.3 eq) and hydrogen peroxide (78.7 mg, 70.9 µL, 809 µmol, 3.1 eq). The mixture was stirred for 72 h. The reaction mixture was diluted with water and extracted three times with DCM. The combined extracts were concentrated in vacuo and the crude product was purified by flash chromatography (ISCO 10 g, 0% to 60% EtOAc in heptane) to give the title compound (48 mg, 154 µmol, 59 % yield) as a waxy colourless solid. MS (ESN): m/z = 356.3 [M+HCOO]⁻.

### Step 3:

### tert-Butyl N-[rac-1,2-trans-4-chloro-2-(2-oxoethyl)-2,3-dihydro-1H-inden-1-yl]carbamate

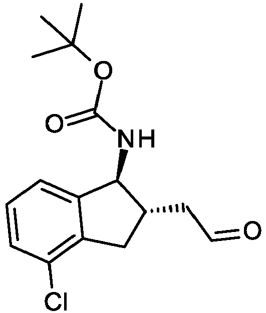

To a suspension of *tert*-butyl *N*-[*rac*-1,2-*trans*-4-chloro-2-(2-hydroxyethyl)-2,3-dihydro-1*H-*inden-1-yl]carbamate (48 mg, 154 µmol, 1 eq) in DCM (2 mL) at 0 °C were added Dess-Martin periodinane (68.6 mg, 162 µmol, 1.05 eq) and pyridine (24.4 mg, 24.9 µL, 308 µmol, 2 eq). The reaction mixture was stirred for 6 hours allowing to warm to RT. The reaction mixture was quenched with sat. Na₂S₂O₃/sat. NaHCO₃ (1:5) and water (50%), before extracting three times with DCM. The organic phases were dried over Na₂SO₄ and concentrated in vacuo. The remaining residue was purified by flash chromatography (ISCO 10 g, 0% to 100% EtOAc in heptane) to give the title compound (40.8 mg, 132 µmol, 85.6 % yield) as a white powder. MS (ESN): m/z = 354.3 [M+HCOO]⁻.

### Intermediate 134

### tert-Butyl N-[1-[[7-cyano-2-(2-oxoethyl)-2,3-dihydro-1H-inden-5-yl] oxymethyl] cyclopropyl] carbamate

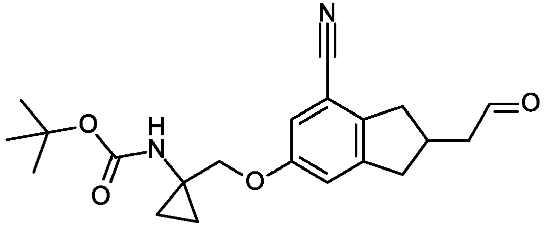

The title compound was prepared by analogy to *tert*-butyl *N*-[*rac*-1,2*-trans*-4-chloro-2-(2-oxoethyl)-2,3-dihydro-1*H*-inden-1-yl]carbamate (Intermediate 133) using *tert-*butyl *N*-[1-[(7-cyano-2-formyl-2,3-dihydro-1*H*-inden-5-yl)oxymethyl]cyclopropyl]carbamate (Intermediate 126) in place of *tert*-butyl *N*-(*rac*-1,2-*trans*-4-chloro-2-formyl-2,3-dihydro-1*H*-inden-1-yl)carbamate and was obtained as a light brown solid. MS (ESN): m/z = 315.2 [M+HCOO]⁻.

### Intermediate 135

### tert-Butyl N-[1-[(4-cyano-6-formyl-6,7-dihydro-5H-cyclopenta[c]pyridin-3-yl)oxymethyl]cyclopropyl]carbamate

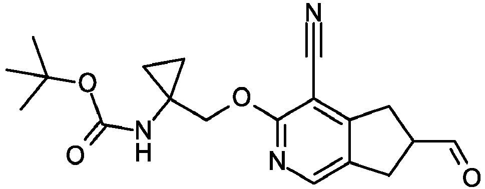

### Step 1:

### Dimethyl 4-bromo-3-oxo-5,7-dihydro-2H-cyclopenta[c]pyridine-6,6-dicarboxylate

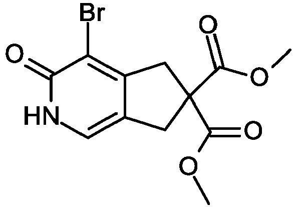

A mixture of dimethyl 3-oxo-5,7-dihydro-2*H*-cyclopenta[c]pyridine-6,6-dicarboxylate (Intermediate 37, step 2, 1.259 g, 5.01 mmol, 1 eq) and N-bromosuccinimide (1.16 g, 6.51 mmol, 1.3 eq) in tetrahydrofuran (48 mL) was stirred in the dark at 60 °C for 1 h. The mixture was cooled to RT. Water was added and the mixture was extracted with EtOAc (3 x). The organic layers were washed with water (2 x) and brine, dried over Na₂SO₄ and evaporated. The residue was adsorbed on adsorbent and purified by flashchromatography (120 g silica gel; DCM/MeOH 100:0 to 98:2) to give the title compound (1.192 g, 3.61 mmol, 72.1 % yield) as a white solid. MS (ESP): m/z = 330.0 [M+H]⁺.

### Steps 2 and 3:

### Methyl 4-bromo-3-[[1-[(2-methylpropan-2-yl)oxycarbonylamino]cyclopropyl]methoxy]-6,7-dihydro-5H-cyclopenta [c]pyridine-6-carboxylate

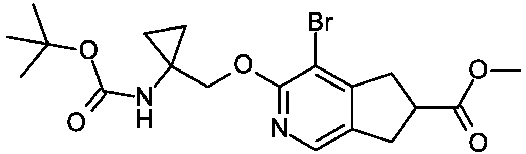

The title compound was prepared by analogy to methyl 1,4-dimethyl-3-[(5-oxomorpholin-2-yl)methoxy]-6,7-dihydro-5*H*-cyclopenta[c]pyridine-6-carboxylate (Intermediate 64, steps 3 and 4) using dimethyl 4-bromo-3-oxo-5,7-dihydro-2*H*-cyclopenta[c]pyridine-6,6-dicarboxylate in place of dimethyl 1,4-dimethyl-3-oxo-5,7-dihydro-2*H*-cyclopenta[c]pyridine-6,6-dicarboxylate and *tert*-butyl *N*-[1-(hydroxymethyl)cyclopropyl]carbamate in place of 6-(hydroxymethyl)morpholin-3-one and was obtained as a white gum. MS (ESP): m/z = 441.3 and 443.3 [M+H]⁺.

### Steps 4 to 6:

### tert-Butyl N-[1-[(4-cyano-6-formyl-6,7-dihydro-5H-cyclopenta[c]pyridin-3-yl)oxymethyl]cyclopropyl]carbamate

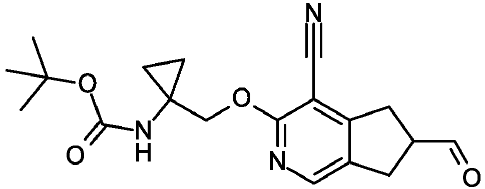

The title compound was prepared by analogy to 2-[(4-cyano-2-formyl-2,3-dihydro-1*H*-inden-5-yl)oxy]-*N*-methylacetamide (Intermediate 36, steps 5 to 7) using methyl 4-bromo-3-[[1-[(2-methylpropan-2-yl)oxycarbonylamino]cyclopropyl]methoxy]-6,7-dihydro-5*H-*cyclopenta[c]pyridine-6-carboxylate in place of ethyl 4-bromo-5-[2-(methylamino)-2-oxoethoxy]-2,3-dihydro-1*H*-indene-2-carboxylate and was obtained as a yellow solid. MS (ESP): m/z = 302.3 [M+H-tBu]⁺.

### Intermediate 136

### tert-Butyl 3-[(6-formyl-1,4-dimethyl-6,7-dihydro-5H-cyclopenta[c]pyridin-3-yl)oxymethyl]morpholine-4-carboxylate

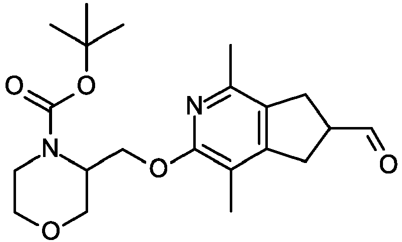

The title compound was prepared by analogy to 1,4-dimethyl-3-[(5-oxomorpholin-2-yl)methoxy]-6,7-dihydro-5*H*-cyclopenta[c]pyridine-6-carbaldehyde (Intermediate 64, steps 3 to 6) using *tert*-butyl 3-(hydroxymethyl)morpholine-4-carboxylate (CAS# 473923-56-7) in place of 6-(hydroxymethyl)morpholin-3-one and was obtained as a light yellow gum. MS (ESP): m/z = 391.4 [M+H]⁺.

### Intermediate 137

### tert-Butyl (2S)-2-[(6-formyl-1,4-dimethyl-6,7-dihydro-5H-cyclopenta[c]pyridin-3-yl)oxymethyl]azetidine-1-carboxylate

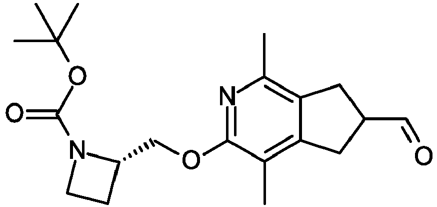

The title compound was prepared by analogy to *tert*-butyl 3-[(6-formyl-1,4-dimethyl-6,7-dihydro-5*H*-cyclopenta[c]pyridin-3-yl)oxymethyl]morpholine-4-carboxylate (Intermediate 136) using *tert*-butyl (2*S*)-2-(hydroxymethyl)azetidine-1-carboxylate (CAS# 161511-85-9) in place of *tert*-butyl 3-(hydroxymethyl)morpholine-4-carboxylate and was obtained as a light yellow oil. MS (ESP): m/z = 361.4 [M+H]⁺.

### Intermediate 138

### tert-Butyl N-[2-[(6-formyl-1,4-dimethyl-6,7-dihydro-5H-cyclopenta[c]pyridin-3-yl)oxy]ethyl]-N-propan-2-ylcarbamate

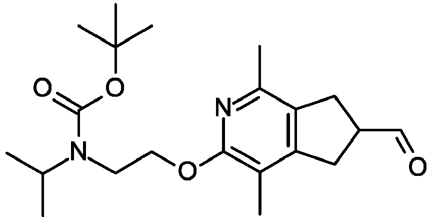

### Step 1:

### Dimethyl 1,4-dimethyl-3-[2-[(2-methylpropan-2-yl)oxycarbonyl-propan-2-ylamino]ethoxy]-5,7-dihydrocyclopenta[c]pyridine-6,6-dicarboxylate

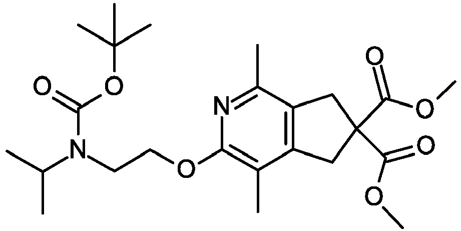

The title compound was prepared by analogy to dimethyl 1,4-dimethyl-3-[(5-oxomorpholin-2-yl)methoxy]-5,7-dihydrocyclopenta[c]pyridine-6,6-dicarboxylate (Intermediate 54, step 3) using *tert-*butyl *N*-(2-hydroxyethyl)-*N*-propan-2-ylcarbamate (CAS# 610309-73-4) in place of 6-(hydroxymethyl)morpholin-3-one and was obtained as a colourless gum. MS (ESP): m/z = 465.5 [M+H]⁺.

### Steps 2 and 3:

### tert-Butyl N-[2-[(6-formyl-1,4-dimethyl-6,7-dihydro-5H-cyclopenta[c]pyridin-3-yl)oxy]ethyl]-N-propan-2-ylcarbamate

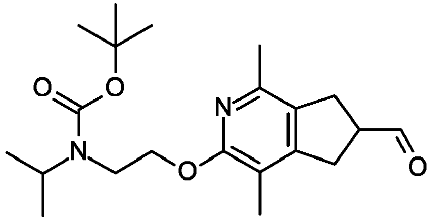

The title compound was prepared by analogy to *tert*-butyl *N*-[1-[(6-formyl-1,4-dimethyl-6,7-dihydro-5*H*-cyclopenta[c]pyridin-3-yl)oxymethyl]cyclopropyl]carbamate (Intermediate 114, steps 2 and 3) and was obtained as a colorless semi-solid. MS (ESP): m/z = 377.4 [M+H]⁺.

### Intermediate 139

### tert-Butyl N-[2-[(6-formyl-1,4-dimethyl-6,7-dihydro-5H-cyclopenta[c]pyridin-3-yl)oxy]ethyl]-N-(2-methoxyethyl)carbamate

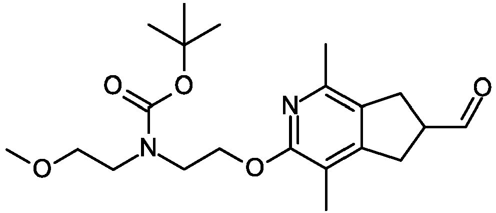

The title compound was prepared by analogy to *tert*-butyl *N*-[2-[(6-formyl-1,4-dimethyl-6,7-dihydro-5*H*-cyclopenta[c]pyridin-3-yl)oxy]ethyl]-*N*-propan-2-ylcarbamate (Intermediate 138) using *tert*-butyl *N*-(2-hydroxyethyl)-*N*-(2-methoxyethyl)carbamate (CAS# 1596688-68-4) in place of *tert*-butyl *N*-(2-hydroxyethyl)-*N*-propan-2-ylcarbamate and was obtained as a colorless gum. MS (ESP): m/z = 393.4 [M+H]⁺.

### Intermediate 140

### tert-Butyl N-[1-[(7-cyano-2-formyl-2,3-dihydro-1H-inden-5-yl)oxy]-2-methylpropan-2-yl]carbamate

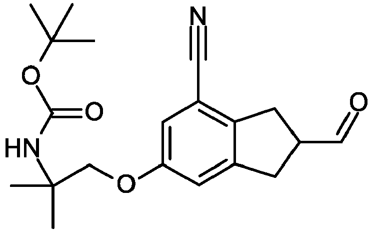

### Step 1:

### Ethyl 4-cyano-6-[2-methyl-2-[(2-methylpropan-2-yl)oxycarbonylamino]propoxy]-2,3-dihydro-1H-indene-2-carboxylate

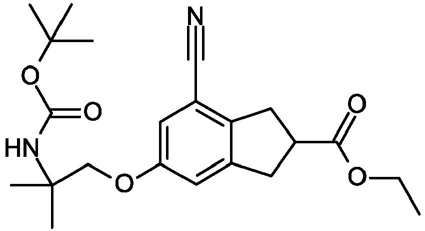

A mixture of ethyl 4-cyano-6-hydroxy-2,3-dihydro-1*H*-indene-2-carboxylate (Intermediate 70, step 4, 50 mg, 0.216 mmol, 1 eq), *tert*-butyl 4,4-dimethyl-1,2,3-oxathiazolidine-3-carboxylate 2,2-dioxide (CAS# 454248-55-6, 65.2 mg, 0.259 mmol, 1.2 eq) and potassium carbonate (89.6 mg, 0.649 mmol, 3 eq) in *N,N*-dimethylacetamide (1 mL) was stirred at 80 °C for 20 h. The reaction mixture was cooled to RT, diluted with NH₄Cl sat. sol. and extracted with EtOAc (3 x). The organic layers were washed with water (2 x) and brine, dried over Na₂SO₄ and evaporated. The crude material was adsorbed on adsorbent and purified by flash chromatography (20 g silica gel; heptane/DCM 70:30 to 0:100 then EtOAc 100 %) to give the title compound (63 mg) as a colorless gum. MS (ESP): m/z = 303.1 M+H-BOC]⁺.

### Step 2:

### tert-Butyl N-[1-[(7-cyano-2-formyl-2,3-dihydro-1H-inden-5-yl)oxy]-2-methylpropan-2-yl] carbamate

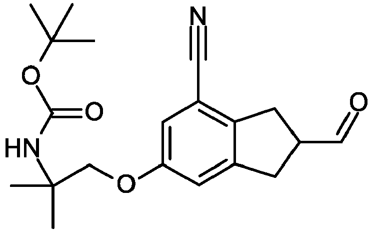

Ethyl 4-cyano-6-[2-methyl-2-[(2-methylpropan-2-yl)oxycarbonylamino]propoxy]-2,3-dihydro-1*H*-indene-2-carboxylate (61 mg, 0.144 mmol, 1 eq) was dissolved in tetrahydrofuran (1.5 mL). At -78 °C lithium aluminium hydride 1 M in THF (173 µL, 0.173 mmol, 1.2 eq) was added. The resulting solution was stirred at -78 °C for 1 h. Et₂O and THF were added. Na₂SO₄ sat. sol. was added dropwise until gas development ceased. Solid Na₂SO₄ was added and the mixture was filtered. The solid was washed well with THF and Et₂O. The filtrate was concentrated. The crude product was adsorbed on adsorbent and purified by flashchromatography (10 g silica gel; heptane/EtOAc 90:10 - 50:50) to give the title compound (41 mg) as a colorless gum. MS (ESP): m/z = 259.2 [M+H-BOC]⁺.

### Intermediate 141

### [(1,2-trans)-4-Chloro-1-[(2-methylpropan-2-yl)oxycarbonylamino]-2,3-dihydro-1H-inden-2-yl]methyl 4-methylbenzenesulfonate, Enantiomer A

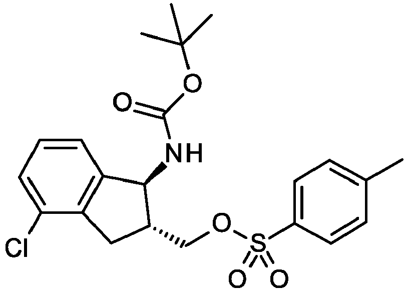

### Step 1:

### tert-Butyl N-[1,2-trans-4-chloro-2-(hydroxymethyl)-2,3-dihydro-1H-inden-1-yl]carbamate, Enantiomer A and tert-butyl N-[1,2-trans-4-chloro-2-(hydroxymethyl)-2,3-dihydro-1H-inden-1-yl]carbamate, Enantiomer B

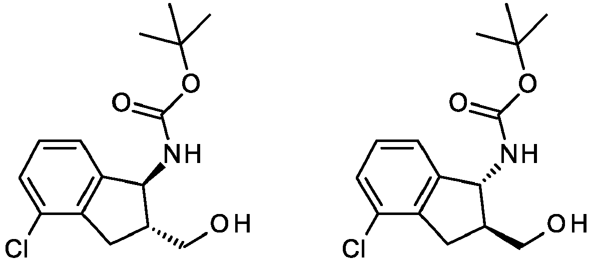

*tert*-Butyl *N*-[*rac*-1,2-*trans*-4-chloro-2-(hydroxymethyl)-2,3-dihydro-1*H*-inden-1-yl]carbamate (Intermediate 88, step 5, 500 mg) was separated by chiral HPLC (Chiralpak AD, to give *tert-*butyl *N*-[1,2-*trans*-4-chloro-2-(hydroxymethyl)-2,3-dihydro-1*H*-inden-1-yl]carbamate, Enantiomer A (215 mg) as a white solid (100% ee) and *tert*-butyl *N*-[1,2-*trans*-4-chloro-2-(hydroxymethyl)-2,3-dihydro-1*H*-inden-1-yl]carbamate, Enantiomer B (232 mg) as a white solid (97.86% ee). MS (ESP): m/z = 242.1 [M+H-tBu]⁺ for both products.

### Step 2:

### [(1,2-trans)-4-Chloro-1-[(2-methylpropan-2-yl)oxycarbonylamino]-2,3-dihydro-1H-inden-2-yl]methyl 4-methylbenzenesulfonate, Enantiomer A

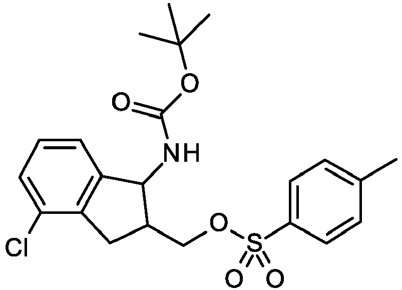

To a solution of *tert*-butyl *N*-[1,2-*trans*-4-chloro-2-(hydroxymethyl)-2,3-dihydro-1*H*-inden-1-yl]carbamate, Enantiomer A (50 mg, 168 µmol, 1 eq) in DCM (2 mL) were added *p-*toluenesulfonyl chloride (128 mg, 672 µmol, 4 eq), *N,N-*diisopropylethylamine (109 mg, 144 µl, 840 µmol, 5 eq) and DMAP (82.1 mg, 672 µmol, 4 eq). The reaction mixture was stirred at RT for 4h. The reaction mixture was treated with water (2 x 5 mL) and extracted with DCM (2 x 15 mL). The organic layers were dried (Na₂SO₄) and evaporated. The crude material was purified by flash chromatography (silica gel, 5 g, 10% to 70% EtOAc in heptane) to give the title compound (68.7 mg, 88.7 % yield) as a white solid. MS (ESP): m/z = 352.1 [M+H-BOC]⁺.

### Intermediate 142

### [(1,2-trans)-4-Chloro-1-[(2-methylpropan-2-yl)oxycarbonylamino]-2,3-dihydro-1H-inden-2-yl]methyl 4-methylbenzenesulfonate, Enantiomer B

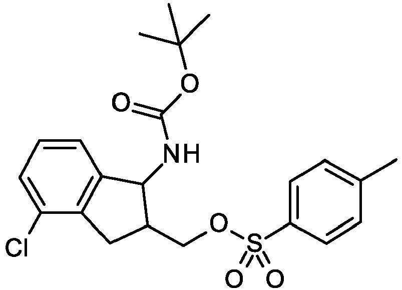

The title compound was prepared by analogy with [(1,2-*trans*)-4-chloro-1-[(2-methylpropan-2-yl)oxycarbonylamino]-2,3-dihydro-1*H*-inden-2-yl]methyl4-methylbenzenesulfonate, Enantiomer A (Intermediate 141, step 2) using *tert*-butyl *N-*[1,2-*trans-*4-chloro-2-(hydroxymethyl)-2,3-dihydro-1*H*-inden-1-yl]carbamate, Enantiomer B (Intermediate 141, step 1) in place of *tert*-butyl *N*-[1,2-*trans-*4-chloro-2-(hydroxymethyl)-2,3-dihydro-1*H*-inden-1-yl]carbamate, Enantiomer A and was obtained as a white solid. MS (ESP): m/z = 352.1 [M+H-BOC]⁺.

### Intermediate 143

### [1-Methyl-3-[2-[(2-methylpropan-2-yl)oxycarbonylamino]ethoxy]-6,7-dihydro-5H-cyclopenta[c]pyridin-6-yl]methyl 4-methylbenzenesulfonate

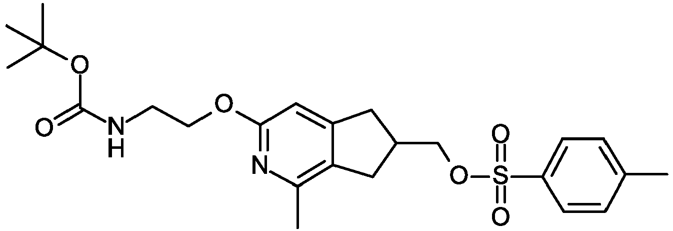

### Steps 1 and 2:

### Methyl 1-methyl-3-[2-[(2-methylpropan-2-yl)oxycarbonylamino]ethoxy]-6,7-dihydro-5H-cyclopenta[c]pyridine-6-carboxylate

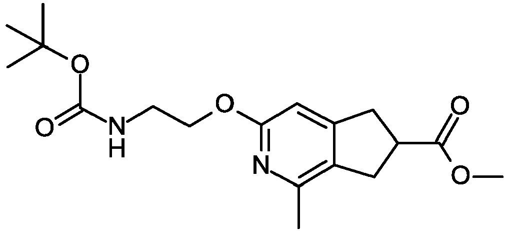

The title compound was prepared by analogy to methyl 1-methyl-3-[[1-[(2-methylpropan-2-yl)oxycarbonylamino]cyclopropyl]methoxy]-6,7-dihydro-5*H*-cyclopenta[c]pyridine-6-carboxylate (Intermediate 117, steps 3 and 4) using *tert*-butyl (2-hydroxyethyl)carbamate in place of *tert*-butyl *N*-[1-(hydroxymethyl)cyclopropyl]carbamate and was obtained as a light yellow solid. MS (ESP): m/z = 351.2 [M+H]⁺.

### Step 3:

### tert-Butyl N-[2-[[6-(hydroxymethyl)-1-methyl-6,7-dihydro-5H-cyclopenta[c]pyridin-3-yl] oxy] ethyl] carbamate

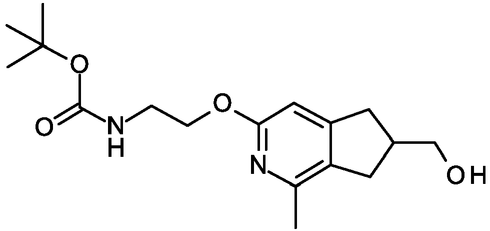

Lithium aluminium hydride in THF (1.04 ml, 1.04 mmol, 1.2 eq) was added at -78 °C to a solution of methyl 1-methyl-3-[2-[(2-methylpropan-2-yl)oxycarbonylamino]ethoxy]-6,7-dihydro-5*H*-cyclopenta[c]pyridine-6-carboxylate (303.7 mg, 867 µmol, 1 eq) in tetrahydrofuran (5.4 mL). The resulting solution was stirred at -40°C for 1h. Na₂SO₄ sat. sol. was added dropwise until gas evolution ceased. Solid Na₂SO₄ was added. The mixture was filtered and the solid was washed very well with Et₂O and THF. The filtrate was concentrated. The crude material was purified by flash chromatography (silica gel, 5g; 10% to 55% EtOAc in heptane) to give the title compound (310mg, 99.8 % yield) as a colorless waxy solid. MS (ESP): m/z = 323.3 [M+H]⁺.

### Step 4:

### [1-Methyl-3-[2-[(2-methylpropan-2-yl)oxycarbonylamino]ethoxy]-6,7-dihydro-5H-cyclopenta[c]pyridin-6-yl]methyl 4-methylbenzenesulfonate

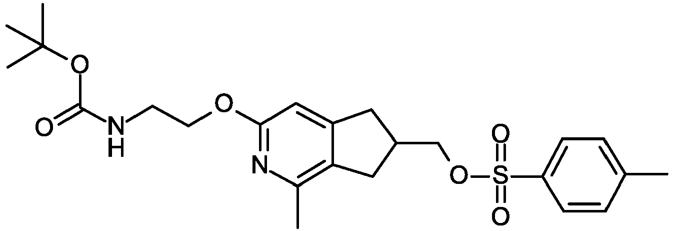

The title compound was prepared by analogy with [(1,2-*trans*)-4-chloro-1-[(2-methylpropan-2-yl)oxycarbonylamino] -2,3-dihydro-1*H*-inden-2-yl]methyl 4-methylbenzenesulfonate, Enantiomer A (Intermediate 141, step 2) and was obtained as a light yellow waxy solid. MS (ESP): m/z = 477.3 [M+H]⁺.

### Intermediate 144

### [rac-1,2-trans-4-Chloro-1-[(2-methylpropan-2-yl)oxycarbonylamino]-2,3-dihydro-1H-inden-2-yl] methyl 4-methylbenzenesulfonate

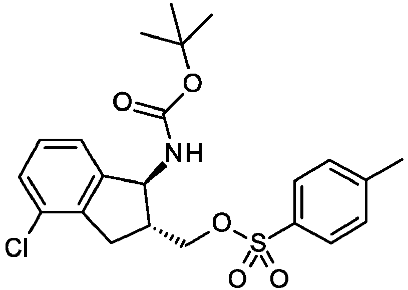

The title compound was prepared by analogy with [(1,2-trans)-4-chloro-1-[(2-methylpropan-2-yl)oxycarbonylamino]-2,3-dihydro-1*H*-inden-2-yl]methyl4-methylbenzenesulfonate, Enantiomer A (Intermediate 141, step 2) using *tert*-butyl *N*-[*rac*-1,2-*trans*-4-chloro-2-(hydroxymethyl)-2,3-dihydro-1*H*-inden-1-yl]carbamate (Intermediate 88, step 5) in place of *tert*-butyl *N*-[1,2-*trans*-4-chloro-2-(hydroxymethyl)-2,3-dihydro-1*H*-inden-1-yl]carbamate, Enantiomer A.

### Intermediate 145

### [1-Cyano-4-methyl-3-[[1-[(2-methylpropan-2-yl)oxycarbonylamino]cyclopropyl]methoxy]-6,7-dihydro-5H-cyclopenta[c]pyridin-6-yl]methyl4-methylbenzenesulfonate

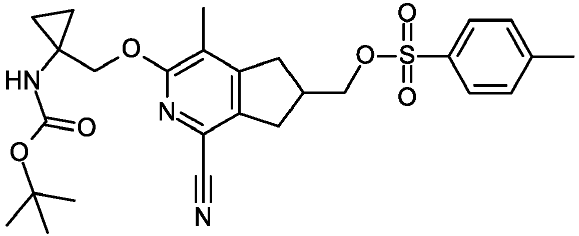

### Steps 1 to 3:

### tert-Butyl N-[1-[[1-bromo-6-(hydroxymethyl)-4-methyl-6,7-dihydro-SH-cyclopenta[c]pyridin-3-yl]oxymethyl]cyclopropyl]carbamate

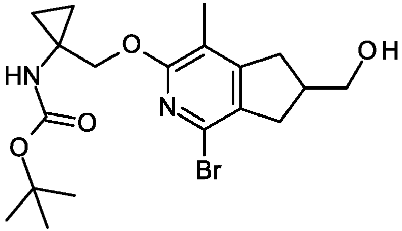

The title compound was prepared by analogy with *tert*-butyl *N*-[2-[[6-(hydroxymethyl)-1,4-dimethyl-6,7-dihydro-5*H*-cyclopenta[c]pyridin-3-yl]oxy]ethyl]carbamate (Intermediate 113, steps 1 to 3) using *tert*-butyl *N*-[1-(hydroxymethyl)cyclopropyl]carbamate (CAS# 107017-73-2) in place of *tert*-butyl (2-hydroxyethyl)carbamate and dimethyl 1-bromo-4-methyl-3-oxo-5,7-dihydro-2*H*-cyclopenta[c]pyridine-6,6-dicarboxylate (Intermediate 72, step 3) in place of dimethyl 1,4-dimethyl-3-oxo-5,7-dihydro-2*H*-cyclopenta[c]pyridine-6,6-dicarboxylate and was obtained as a colorless waxy solid. MS (ESP): m/z = 427.2 and 429.2 [M+H]⁺.

### Step 4:

### tert-Butyl N-[1-[[1-cyano-6-(hydroxymethyl)-4-methyl-6,7-dihydro-5H-cyclopenta[c]pyridin-3-yl]oxymethyl]cyclopropyl]carbamate

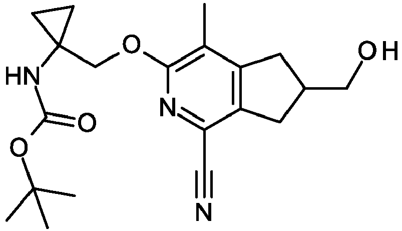

The title compound was prepared by analogy with 2-(2-hydroxyethyl)-2,3-dihydro-1*H*-indene-4-carbonitrile (Intermediate 22, step 1) and was obtained as a light brown waxy solid. MS (ESP): m/z = 318.2 [M+H-tBu]⁺.

### Step 5:

### [1-Cyano-4-methyl-3-[[1-[(2-methylpropan-2-yl)oxycarbonylamino]cyclopropyl]methoxy]-6,7-dihydro-5H-cyclopenta[c]pyridin-6-yl]methyl 4-methylbenzenesulfonate

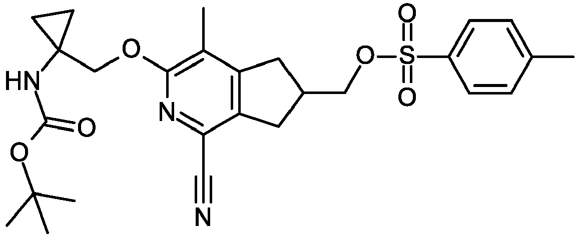

The title compound was prepared by analogy with [(1,2-*trans*)-4-chloro-1-[(2-methylpropan-2-yl)oxycarbonylamino]-2,3-dihydro-1*H*-inden-2-yl]methyl 4-methylbenzenesulfonate, Enantiomer A (Intermediate 141, step 2) and was obtained as a light brown foam. MS (ESP): m/z = 472.3 [M+H-tBu]⁺.

### Intermediate 146

### [4-Cyano-1-methyl-3-[[1-[(2-methylpropan-2-yl)oxycarbonylamino]cyclopropyl]methoxy]-6,7-dihydro-5H-cyclopenta[c]pyridin-6-yl]methyl 4-methylbenzenesulfonate

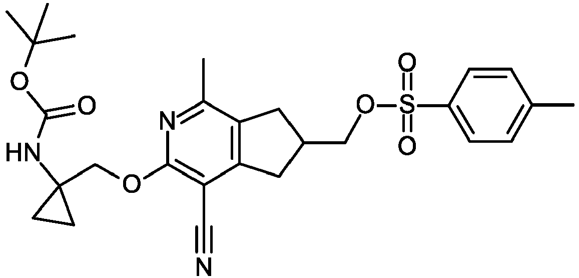

The title compound was prepared by analogy with [1-cyano-4-methyl-3-[[1-[(2-methylpropan-2-yl)oxycarbonylamino]cyclopropyl]methoxy]-6,7-dihydro-5*H*-cyclopenta[c]pyridin-6-yl]methyl 4-methylbenzenesulfonate (Intermediate 145) using dimethyl 4-bromo-1-methyl-3-oxo-5,7-dihydro-2*H*-cyclopenta[c]pyridine-6,6-dicarboxylate in place of dimethyl 1-bromo-4-methyl-3-oxo-5,7-dihydro-2*H*-cyclopenta[c]pyridine-6,6-dicarboxylate and was obtained as a light brown foam. MS (ESP): m/z = 528.3 [M+H]⁺.

The dimethyl 4-bromo-1-methyl-3-oxo-5,7-dihydro-2*H*-cyclopenta[c]pyridine-6,6-dicarboxylate used as starting material was prepared by analogy with dimethyl 1-bromo-4-methyl-3-oxo-5,7-dihydro-2*H*-cyclopenta[c]pyridine-6,6-dicarboxylate (Intermediate 72, step 3) using dimethyl 4-bromo-2-[(4-methoxyphenyl)methyl]-1-methyl-3-oxo-5,7-dihydrocyclopenta[c]pyridine-6,6-dicarboxylate (Intermediate 72, step 2) in place of dimethyl 1-bromo-2-[(4-methoxyphenyl)methyl]-4-methyl-3-oxo-5,7-dihydrocyclopenta[c]pyridine-6,6-dicarboxylate and was obtained as a light brown solid. MS (ESP): m/z = 344.1, 346.1[M+H]⁺.

### Intermediate 147

### [4-Methyl-3-[[1-[(2-methylpropan-2-yl)oxycarbonylamino]cyclopropyl]methoxy]-1-(5-methyl-1,3-thiazol-2-yl)-6,7-dihydro-5H-cyclopenta[c]pyridin-6-yl]methyl 4-methylbenzenesulfonate

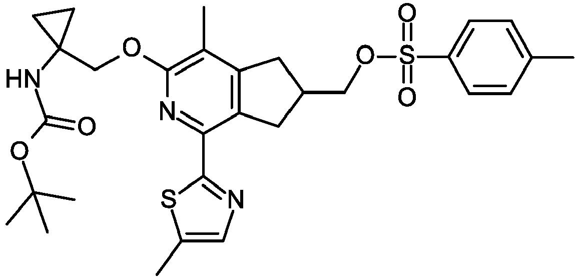

and **Intermediate 148**

### [4-Methyl-3-[[1-[(2-methylpropan-2-yl)oxycarbonylamino]cyclopropyl]methoxy]-6,7-dihydro-5H-cyclopenta[c]pyridin-6-yl]methyl 4-methylbenzenesulfonate

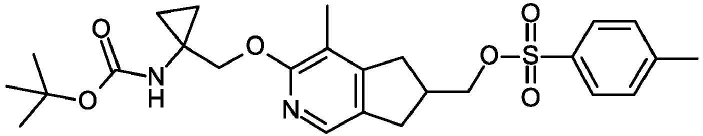

### Step 1:

### tert-Butyl N-[1-[[6-(hydroxymethyl)-4-methyl-1-(5-methyl-1,3-thiazol-2-yl)-6,7-dihydro-5H-cyclopenta[c]pyridin-3-yl]oxymethyl]cyclopropyl]carbamate and tert-butyl N-[1-[[6-(hydroxymethyl)-4-methyl-6,7-dihydro-5H-cyclopenta[c]pyridin-3-yl]oxymethyl]cyclopropyl]carbamate

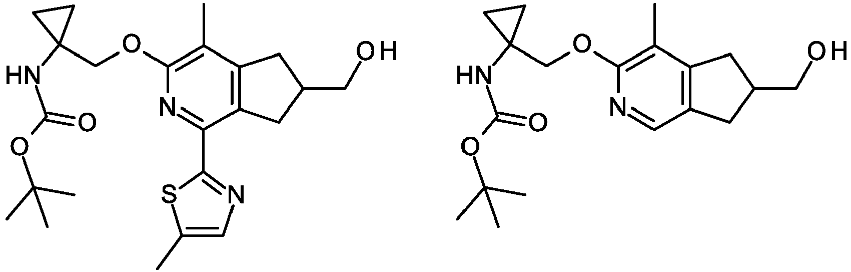

In a microwave tube, *tert*-butyl *N*-[1-[[1-bromo-6-(hydroxymethyl)-4-methyl-6,7-dihydro-5*H-*cyclopenta[c]pyridin-3-yl]oxymethyl]cyclopropyl]carbamate (Intermediate 145, step 3, 40 mg, 93.6 µmol, 1 eq) was disolved in DMF (1 mL). 5-Methyl-2-(4,4,5,5-tetramethyl-1,3,2-dioxaborolan-2-yl)-1,3-thiazole (42.1 mg, 187 µmol, 2 eq), Pd(OAc)₂ (1.05 mg, 4.68 µmol, 0.05 eq), DPPF (5.19 mg, 9.36 µmol, 0.1 eq), copper (I) chloride (9.27 mg, 93.6 µmol, 1 eq) and Cs₂CO₃ (61 mg, 187 µmol, 2 eq) were added. Argon was bubbled through the solution (2-3min). The reaction mixture was heated to 100 °C and stirred for 6 h. The reaction mixture was treated with 5 mL H₂O and extracted with EtOAc (3 x 10 mL). The organic layers were washed with 5 mL H₂O. The organic layers were dried (Na₂SO₄) and evaporated. The crude material was purified by flash chromatography (silica gel, 5g, 0% to 80% EtOAc in heptane) to give the title compound mixture (24 mg) as a brown waxy solid. MS (ESP): m/z = 446.3 and 349.2 [M+H]⁺.

### Step 2:

### [4-Methyl-3-[[1-[(2-methylpropan-2-yl)oxycarbonylamino]cyclopropyl]methoxy]-1-(5-methyl-1,3-thiazol-2-yl)-6,7-dihydro-5H-cyclopenta[c]pyridin-6-yl]methyl 4-methylbenzenesulfonate and [4-methyl-3-[[l-[(2-methylpropan-2-yl)oxycarbonylamino]cyclopropyl]methoxy]-6,7-dihydro-5H-cyclopenta[c]pyridin-6-yl]methyl 4-methylbenzenesulfonate

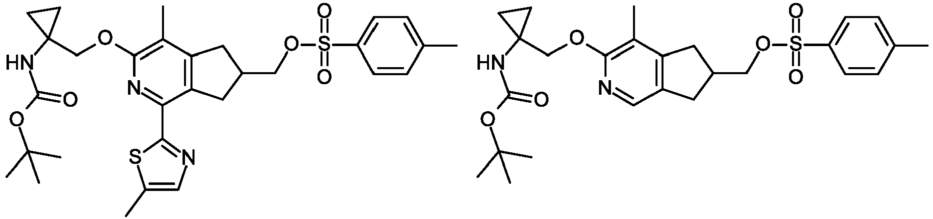

The mixture of the title compounds was prepared by analogy with [(1,2-*trans*)-4-chloro-1-[(2-methylpropan-2-yl)oxycarbonylamino]-2,3-dihydro-1*H*-inden-2-yl]methyl 4-methylbenzenesulfonate, Enantiomer A (Intermediate 141, step 2). The mixture was separated by flash column chromatography to give [4-methyl-3-[[1-[(2-methylpropan-2-yl)oxycarbonylamino]cyclopropyl]methoxy]-1-(5-methyl-1,3-thiazol-2-yl)-6,7-dihydro-5*H-*cyclopenta[c]pyridin-6-yl]methyl 4-methylbenzenesulfonate as a white solid; MS (ESP): m/z = 600.4 [M+H]⁺ and [4-methyl-3-[[1-[(2-methylpropan-2-yl)oxycarbonylamino]cyclopropyl]methoxy]-6,7-dihydro-5*H*-cyclopenta[c]pyridin-6-yl]methyl 4-methylbenzenesulfonate as an off-white waxy solid; MS (ESP): m/z = 503.4 [M+H]⁺.

### Intermediate 149

### [4-Methyl-3-[[1-[(2-methylpropan-2-yl)oxycarbonylamino]cyclopropyl]methoxy]-1-(1,3-thiazol-2-yl)-6,7-dihydro-5H-cyclopenta[c]pyridin-6-yl]methyl 4-methylbenzenesulfonate

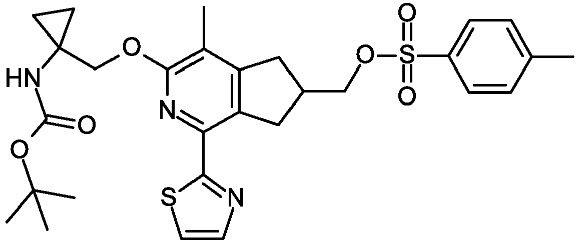

### Step 1:

### Methyl 4-methyl-3-[[1-[(2-methylpropan-2-yl)oxycarbonylamino]cyclopropyl]methoxy]-1-(1,3-thiazol-2-yl)-6,7-dihydro-5H-cyclopenta[c]pyridine-6-carboxylate

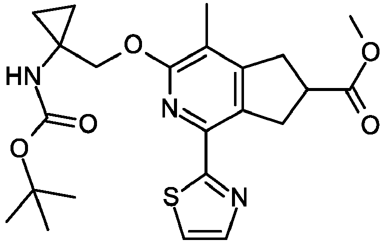

In a 10 mL microwave tube, 2-(tributylstannyl)thiazole (24.7 mg, 65.9 µmol, 1 eq) and methyl 1-bromo-4-methyl-3-[[1-[(2-methylpropan-2-yl)oxycarbonylamino]cyclopropyl]methoxy]-6,7-dihydro-5*H*-cyclopenta[c]pyridine-6-carboxylate (Intermediate 145, step 2, 30 mg, 65.9 µmol, 1 eq) were combined with toluene (1.5 mL) to give a colorless solution. Ar was bubbled through the reaction mixture during 10 min. Tetrakis(triphenylphosphine)palladium (0) (3.81 mg, 3.29 µmol, 0.05 eq) was added. Again Ar was bubbled through the mixture during 3 min. The reaction mixture was heated to 100 °C and stirred for 4.5 h. The crude reaction mixture was concentrated in vacuo. The residue was disolved in DCM (2mL) and saturated potassium fluoride solution (2 mL). This mixture was stirred for 1 h at RT. The mixture was diluted with DCM (5 mL) and saturated potassium fluoride solution (2 mL) and extracted with DCM (2 x 7 mL). The organic layers were washed with saturated potassium fluoride solution (5 mL). The organic layers were dried (Na₂SO₄) and evaporated. The crude material was purified by flash chromatography (silica gel, 5g, 0% to 40% EtOAc in heptane) to give the crude product. The material was purified by reverse phase column HPLC to give the title compound (14.1 mg, 45.6 % yield) as a white solid. MS (ESP): m/z = 460.3 [M+H]⁺.

### Steps 2 and 3:

### [4-Methyl-3-[[1-[(2-methylpropan-2-yl)oxycarbonylamino]cyclopropyl]methoxy]-1-(1,3-thiazol-2-yl)-6,7-dihydro-5H-cyclopenta[c]pyridin-6-yl]methyl 4-methylbenzenesulfonate

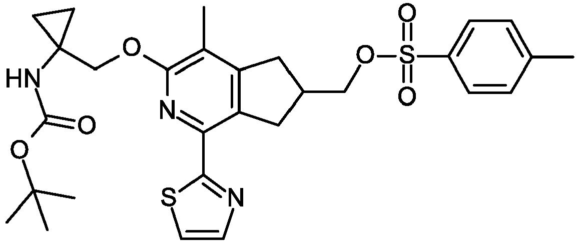

The title compound was prepared by analogy with [1-cyano-4-methyl-3-[[1-[(2-methylpropan-2-yl)oxycarbonylamino]cyclopropyl]methoxy]-6,7-dihydro-5*H*-cyclopenta[c]pyridin-6-yl]methyl 4-methylbenzenesulfonate (Intermediate 145, steps 4 and 5) and was obtained as a white solid. MS (ESP): m/z = 586.4 [M+H]⁺.

### Intermediate 150

### tert-Butyl (2S)-2-[[4-methyl-6-[(4-methylphenyl)sulfonyloxymethyl]-6,7-dihydro-5H-cyclopenta[c]pyridin-3-yl]oxymethyl]azetidine-1-carboxylate

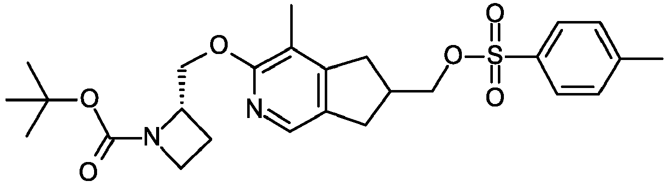

### Step 1:

### Dimethyl 1-bromo-4-methyl-3-[[(2S)-1-[(2-methylpropan-2-yl)oxycarbonyl]azetidin-2-yl]methoxy]-5,7-dihydrocyclopenta[c]pyridine-6,6-dicarboxylate

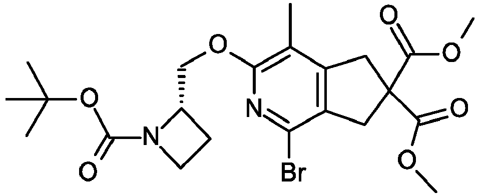

A solution of dimethyl 1-bromo-4-methyl-3-oxo-5,7-dihydro-2H-cyclopenta[c]pyridine-6,6-dicarboxylate (Intermediate 72, step 3, 300 mg, 655 µmol, 1 eq), *tert*-butyl (2*S*)-2-(hydroxymethyl)azetidine-1-carboxylate (CAS# 161511-85-9, 123 mg, 655 µmol, 1 eq), triphenylphosphine (215 mg, 818 µmol, 1.25 eq) and di-*tert*-butyl azodicarboxylate (181 mg, 786 µmol, 1.2 eq) in tetrahydrofuran (18 ml) was stirred at 70 °C for 5 h. The mixture was cooled to RT, H₂O was added and the mixture was extracted with EtOAc (3 x). The organic layers were washed with water and brine, dried over Na₂SO₄ and evaporated. The crude material was purified by flash chromatography (silica gel, 50g; 2% to 60% EtOAc in heptane) to give the impure title compound which was further purified by SFC to give the title compound (336 mg, 73.8 % yield) as a colorless oil. MS (ESP): m/z = 513.5, 517.5 [M+H]⁺.

### Step 2:

### Methyl 1-bromo-4-methyl-3-[[(2S)-1-[(2-methylpropan-2-yl)oxycarbonyl]azetidin-2-yl]methoxy]-6,7-dihydro-5H-cyclopenta[c]pyridine-6-carboxylate

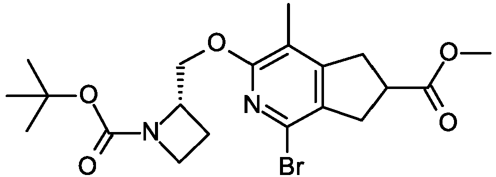

To a solution of dimethyl 1-bromo-4-methyl-3-[[(2S)-1-[(2-methylpropan-2-yl)oxycarbonyl]azetidin-2-yl]methoxy]-5,7-dihydrocyclopenta[c]pyridine-6,6-dicarboxylate (336 mg, 654 µmol, 1 eq) in dimethyl sulfoxide (5 mL) were added lithium chloride (27.7 mg, 654 µmol, 1 eq) and water (1 mL) and the resulting mixture was stirred at 170 °C for 2.5h. The mixture was cooled to RT, diluted with H₂O (15 mL) and extracted with EtOAc (2 x 30 mL). The organic layers were washed with H₂O (10 mL), dried over Na₂SO₄ and evaporated. The crude material was purified by flash chromatography (silica gel, 10g; 0% to 50% EtOAc in heptane) to give the title compound (213 mg, 66.8 % yield) as a colorless oil. MS (ESP): m/z = 455.3, 457.3 [M+H]⁺.

### Step 3:

### Methyl 4-methyl-3-[[(2S)-1-[(2-methylpropan-2-yl)oxycarbonyl]azetidin-2-yl]methoxy]-6,7-dihydro-5H-cyclopenta[c]pyridine-6-carboxylate

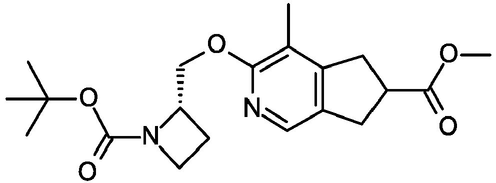

Methyl 1-bromo-4-methyl-3-[[(2*S*)-1-[(2-methylpropan-2-yl)oxycarbonyl]azetidin-2-yl]methoxy]-6,7-dihydro-5*H*-cyclopenta[c]pyridine-6-carboxylate (153 mg, 336 µmol, 1 eq) was combined with MeOH (5 mL) to give a colorless solution. The reaction flask was evacuated by vacuum pump and refilled with argon (balloon). This was repeated 3 times. Palladium on activated carbon 10% (7.15 mg, 67.2 µmol, 0.2 eq) was added. The flask was evacuated by vacuum pump and refilled with hydrogen (balloon). This was repeated 3 times. The reaction mixture was stirred for 5 days at RT under an atmosphere of hydrogen. The reaction mixture was filtered and washed thoroughly with MeOH (30 mL) and then the filtrate was concentrated in vacuo at RT. The crude material was purified by flash chromatography (silica gel, 5 g, 0% to 60% EtOAc in heptane) to give the title compound (103 mg) as a colorless oil. MS (ESP): m/z = 377.3 [M+H]⁺.

### Steps 4 and 5:

### tert-Butyl (2S)-2-[[4-methyl-6-[(4-methylphenyl)sulfonyloxymethyl]-6,7-dihydro-5H-cyclopenta[c]pyridin-3-yl]oxymethyl]azetidine-1-carboxylate

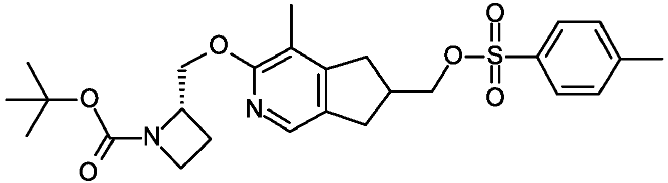

The title compound was prepared by analogy with [1-methyl-3-[2-[(2-methylpropan-2-yl)oxycarbonylamino]ethoxy]-6,7-dihydro-5*H*-cyclopenta[c]pyridin-6-yl]methyl 4-methylbenzenesulfonate (Intermediate 143, steps 3 and 4) and was obtained as a colorless viscous oil. MS (ESP): m/z = 503.4 [M+H]⁺.

### Intermediate 151

### tert-Butyl 2-[[1-cyano-4-methyl-6-[(4-methylphenyl)sulfonyloxymethyl]-6,7-dihydro-5H-cyclopenta[c]pyridin-3-yl]oxymethyl]-5-oxopiperazine-1-carboxylate

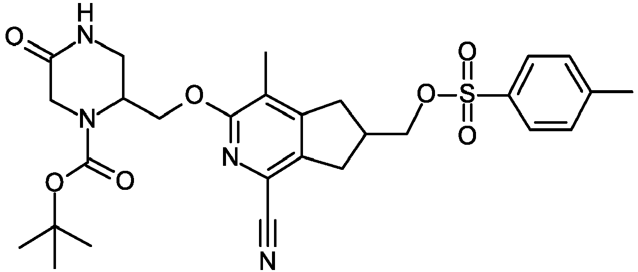

### Step 1:

### Dimethyl 1-bromo-4-methyl-3-[[1-[(2-methylpropan-2-yl)oxycarbonyl]-5-oxopiperazin-2-yl] methoxy] -5,7-dihydrocyclopenta[c]pyridine-6,6-dicarboxylate

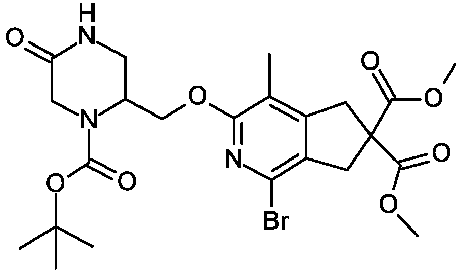

The title compound was prepared by analogy with dimethyl 1-bromo-4-methyl-3-[[(2*S*)-1-[(2-methylpropan-2-yl)oxycarbonyl]azetidin-2-yl]methoxy]-5,7-dihydrocyclopenta[c]pyridine-6,6-dicarboxylate (Intermediate 150, step 1) using *tert*-butyl 2-(hydroxymethyl)-5-oxopiperazine-1-carboxylate in place of *tert*-butyl (2*S*)-2-(hydroxymethyl)azetidine-1-carboxylate and was obtained as a white foam. MS (ESP): m/z = 556.3, 558.3 [M+H]⁺.

The *tert*-butyl 2-(hydroxymethyl)-5-oxopiperazine-1-carboxylate used as starting material was prepared as follows:
To a solution of 1-[(2-methylpropan-2-yl)oxycarbonyl]-5-oxopiperazine-2-carboxylic acid (1.00 g, 4.09 mmol, 1 eq) in THF (6 mL) were added at 0°C isobutylchloroformate (559 mg, 536 µl, 4.09 mmol, 1 eq) and N-methylmorpholine (414 mg, 450 µl, 4.09 mmol, 1 eq) dropwise. The mixture was stirred 1.5 h at 0 °C. Then the mixture was filtered. The filtrate was cooled again to 0 °C. Sodium borohydride (232 mg, 6.14 mmol, 1.5 eq) dissolved in H₂O (3 mL) was added dropwise (strong bubbling). After 10 min. at 0°C, the reaction was warmed up to RT. The reaction was stirred for 4 h at RT. The reaction mixture was treated with 2 mL H₂O and extracted with EtOAc (3 x 15 mL). The organic layers were dried (Na₂SO₄) and evaporated. The crude material was purified by flash chromatography (silica gel, 20g; 2% to 15% MeOH in DCM) to give the title compound (548 mg, 63.5 % yield) as a white solid. MS (ESP): m/z = 175.1 [M+H-tBu]⁺.

### Steps 2 to 5:

### tert-Butyl 2-[[1-cyano-4-methyl-6-[(4-methylphenyl)sulfonyloxymethyl]-6,7-dihydro-5H-cyclopenta[c]pyridin-3-yl]oxymethyl]-5-oxopiperazine-1-carboxylate

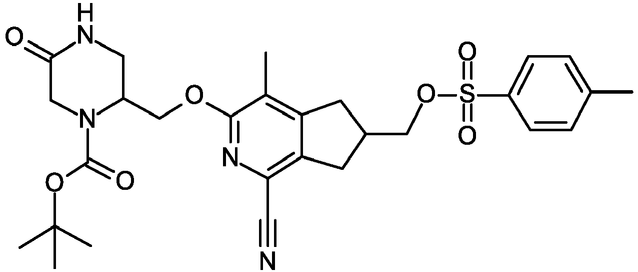

The title compound was prepared by analogy with [1-cyano-4-methyl-3-[[1-[(2-methylpropan-2-yl)oxycarbonylamino]cyclopropyl]methoxy]-6,7-dihydro-5*H*-cyclopenta[c]pyridin-6-yl]methyl 4-methylbenzenesulfonate (Intermediate 145, steps 2 to 5) and was obtained as a yellow foam. MS (ESP): m/z = 515.3 [M+H-tBu]⁺.

### Intermediate 152

### tert-Butyl N-[rac-1,2-trans-7-chloro-2-formyl-2,3-dihydro-1H-inden-1-yl]carbamate

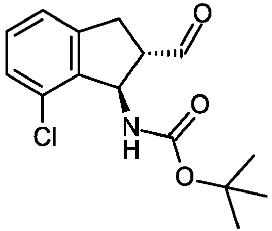

The title compound was prepared by analogy with *tert*-butyl *N*-(*rac*-1,2-*trans*-4-chloro-2-formyl-2,3-dihydro-1*H*-inden-1-yl)carbamate (Intermediate 92) using 7-chloro-2,3-dihydroinden-1-one (CAS# 34911-25-6) in place of 4-chloro-2,3-dihydroinden-1-one and was obtained as a light yellow oil.

### Intermediate 153

### 4-Fluoro-1-methyl-6,7-dihydro-5H-cyclopenta[c]pyridin-6-amine

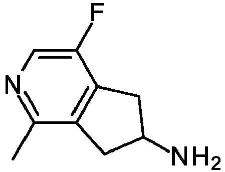

### Step 1:

### 4-Fluoro-1-methyl-6,7-dihydro-5H-cyclopenta[c]pyridine-6-carboxylic acid

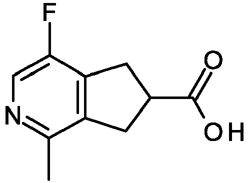

A solution of ethyl 4-fluoro-1-methyl-6,7-dihydro-5*H*-cyclopenta[c]pyridine-6-carboxylate (Intermediate 27, step 8, 450.0 mg, 2.02 mmol, 1 eq) in THF (3 mL), methanol (3 mL) and water (1 mL) was added lithium hydroxide (0.04 mL, 4.03 mmol, 2 eq) at 20 °C. The reaction mixture was stirred at 20 °C for 2 h. The reaction mixture was concentrated to provide the title compound (330 mg, 1.69 mmol, 83.9% yield) as a yellow oil. The crude product was used in the next step without further purification. MS (ESP): m/z = 196.1 [M+H]⁺.

### Step 2:

### (4-Methoxyphenyl)methyl N-(4-fluoro-1-methyl-6,7-dihydro-5H-cyclopenta[c]pyridin-6-yl)carbamate

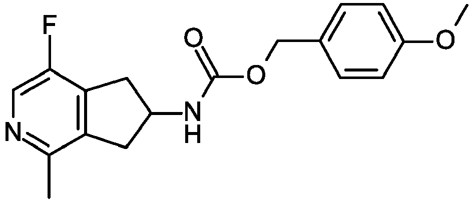

The title compound was prepared by analogy with (4-methoxyphenyl)methyl *N*-(1-chloro-6,7-dihydro-5*H*-cyclopenta[c]pyridin-6-yl)carbamate (Intermediate 58, step 1) and was obtained as a white solid. MS (ESP): m/z = 331.4 [M+H]⁺.

### Step 3:

### 4-Fluoro-1-methyl-6,7-dihydro-5H-cyclopenta[c]pyridin-6-amine

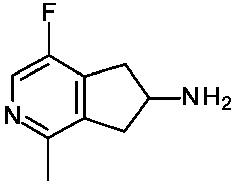

To a solution of (4-methoxyphenyl)methyl *N*-(4-fluoro-1-methyl-6,7-dihydro-5*H-*cyclopenta[c]pyridin-6-yl)carbamate (62.66 mg, 0.190 mmol, 1 eq) in THF (10 mL) was added Pd/C catalyst (60 mg) and then the mixture was stirred at 25 °C under an atmosphere of hydrogen for 3 h. The catalyst was filtered off and then filtrate was concentrated to give the crude title compound (30 mg, 0.180 mmol, 95.2% yield) as an off-white solid. MS (ESP): m/z = 167.1 [M+H]⁺.

### Intermediate 154

### 3-[2-Oxo-3-(3-oxo-4H-pyrazino[2,3-b] [1,4]oxazin-6-yl)-1,3-oxazolidin-5-yl]propanal

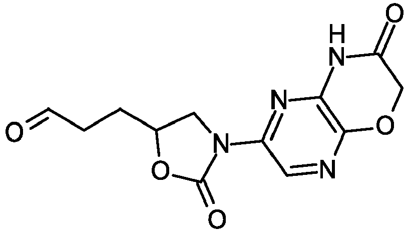

### Step 1:

### 6-[5-[3-[tert-Butyl(dimethyl)silyl]oxypropyl]-2-oxo-1,3-oxazolidin-3-yl]-4H-pyrazino[2,3-b][1,4]oxazin-3-one

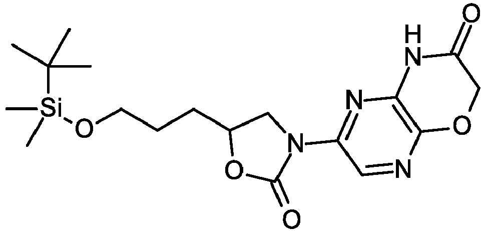

To a solution of 5-[3-[*tert*-butyl(dimethyl)silyl]oxypropyl]-1,3-oxazolidin-2-one (Intermediate 16, step 5, 400.0 mg, 1.54 mmol, 1 eq) and 6-bromo-4*H*-pyrazino[2,3-b][1,4]oxazin-3-one (Intermediate 25, step 1, 529.41 mg, 2.31 mmol, 1.5 eq) in toluene (16 mL) were added copper(I) iodide (0.05 mL, 1.54 mmol, 1 eq), potassium carbonate (639.3 mg, 4.63 mmol, 3 eq) and *trans-N,N'*-dimethylcyclohexane-1,2-diamine (219.3 mg, 1.54 mmol, 1 eq). The mixture was stirred at 100 °C for 3 h. The reaction mixture was filtered and concentrated to give a residue which was purified by flash column (50% PE\EA) to give the title compound (200 mg, 0.490 mmol, 31.7 % yield). MS (ESP): m/z = 409.2 [M+H]⁺.

### Steps 2 and 3:

### 3-[2-Oxo-3-(3-oxo-4H-pyrazino[2,3-b][1,4]oxazin-6-yl)-1,3-oxazolidin-5-yl]propanal

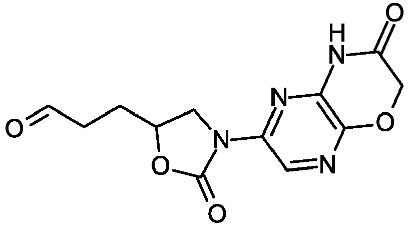

The title compound was prepared by analogy with 3-[2-oxo-3-(3-oxo-4*H*-pyrido[3,2-b][1,4]oxazin-6-yl)-1,3-oxazolidin-5-yl]propanal (Intermediate 16, steps 8 and 9) and was obtained as a white solid.

### Intermediate 155

### 6-[5-(3-Aminopropyl)-2-oxo-1,3-oxazolidin-3-yl]-4H-pyrazino[2,3-b] [1,4]oxazin-3-one

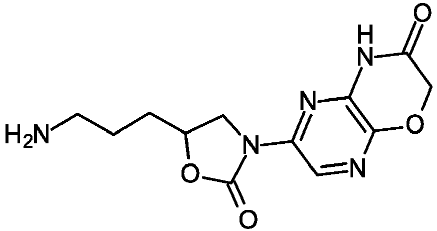

The title compound was prepared by analogy with 6-[5-(2-aminoethyl)-2-oxo-1,3-oxazolidin-3-yl]-4*H*-pyrido[3,2-b][1,4]oxazin-3-one (Intermediate 1, steps 7 to 9) using 6-[5-(3-hydroxypropyl)-2-oxo-1,3-oxazolidin-3-yl]-4*H*-pyrazino[2,3-b][1,4]oxazin-3-one (Intermediate 154, step 2) in place of 6-[5-(2-hydroxyethyl)-2-oxo-1,3-oxazolidin-3-yl]-4*H*-pyrido[3,2-b][1,4]oxazin-3-one and was obtained as a white solid. MS (ESP): m/z = 294.2 [M+H]⁺.

### Intermediate 156

### 6-[2-Oxo-5-[3-amino-2-hydroxypropyl]-1,3-oxazolidin-3-yl]-4H-pyrazino[2,3-b][1,4]oxazin-3-one, Isomer A 2,2,2-trifluoroacetate

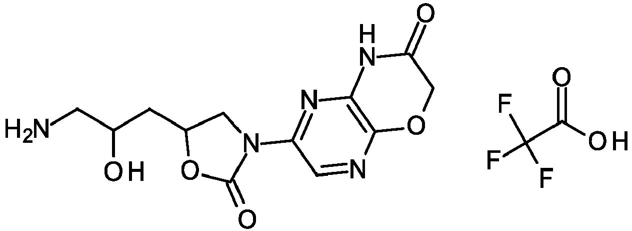

### Step 1:

### tert-Butyl N-[2-[tert-butyl(diphenyl)silylloxypent-4-enyl]carbamate

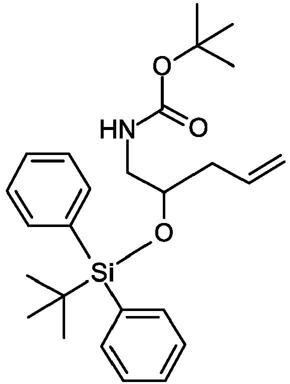

To a solution of *tert*-butyl *N*-(2-hydroxypent-4-enyl)carbamate (CAS# 113525-94-3, 67.47 mL, 26.83 mmol, 1 eq) in DCM (150 mL) was added imidazole (3653.2 mg, 53.66 mmol, 2 eq) at 0°C and the mixture was stirred at this temperature for 15 min. Then *tert-*butylchlorodiphenylsilane (11062 mg, 40.25 mmol, 1.5 eq) in DCM (50 mL) was slowly added. The mixture was stirred at 25 °C for 24 h. The white precipitate was filtered off and the filtrate was concentrated to give the crude product which was purified by chromatography on silica gel (PE) to give the title compound (9 g, 20.47 mmol, 76 % yield) as a colorless oil.

### Step 2:

### tert-Butyl N-[2-[tert-butyl(diphenyl)silyl]oxy-3-(oxiran-2-yl)propyl]carbamate

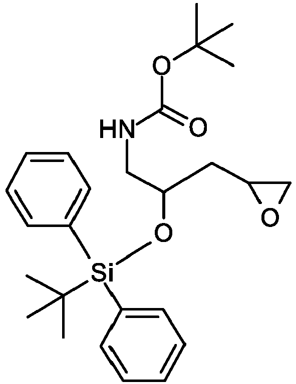

To a solution of *tert*-butyl *N*-[2*-*[*tert*-butyl(diphenyl)silyl]oxypent-4-enyl]carbamate (4.6 g, 10.46 mmol, 1 eq) in DCM (50 mL) was added 3-chloroperbenzoic acid (2761.4 mg, 13.6 mmol, 1.3 eq) at 0°C and the mixture was stirred at 25 °C for 16 h. The precipitate was filtered off and 10% Na₂S₂O₃ solution was added. The mixture was extracted with DCM and the combined organic layers were washed with 10% NaHCOs solution and brine, dried over anhydrous magnesium sulfate then concentrated to give the crude product which was purified by chromatography on silica (PE:EA=10:1) to give the title compound (3.6 g, 7.9 mmol, 75.5% yield) as a colorless oil.

### Step 3:

### tert-Butyl N-[5-azido-2-[tert-butyl(diphenyl)silyl]oxy-4-hydroxypentyl]carbamate

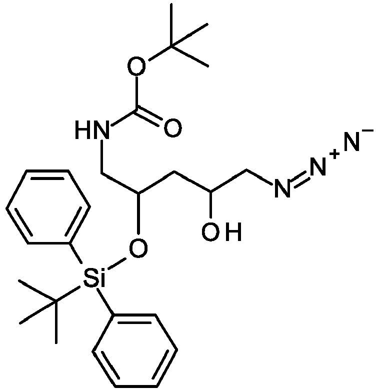

To a solution of *tert*-butyl *N*-[2-[*tert*-butyl(diphenyl)silyl]oxy-3-(oxiran-2-yl)propyl]carbamate (1.0 g, 2.19 mmol, 1 eq) in methanol (20 mL) were added sodium azide (0.23 mL, 6.58 mmol, 3 eq) and ammonium chloride (0.35 g, 6.58 mmol, 3 eq) and then the mixture was stirred at 80 °C for 16 h. The solvent was evaporated under reduced pressure and the residue was partitioned between 200 mL EtOAc and 100 mL water. The organic layer was washed with brine, dried over Na₂SO₄ and evaporated under reduced pressure, affording the crude title compound (1 g, 2.01 mmol, 91.4% yield) as a yellow oil. MS (ESP): m/z = 399.1 [M+H-BOC]⁺.

### Step 4:

### tert-Butyl N-[2-[tert-butyl(diphenyl)silyl]oxy-3-(2-oxo-1,3-oxazolidin-5-yl)propyl]carbamate

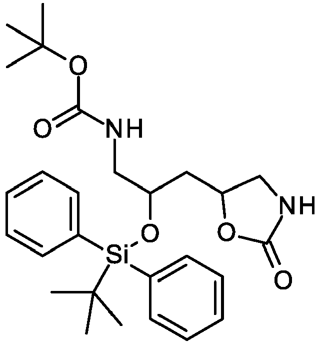

To a solution of *tert*-butyl *N*-[5-azido-2-[*tert*-butyl(diphenyl)silyl]oxy-4-hydroxypentyl]carbamate (1.0 g, 2.01 mmol, 1 eq) in toluene (50 mL) was added triphenylphosphine (578.5 mg, 2.21 mmol, 1.1 eq) at 0°C under an atmosphere of carbon dioxide for 30 min. Then the solution was heated to 110 °C for 16 h. The solvent was removed to give the crude product which was purified by chromatography on silica gel (PE:EA=1:1) to give the title compound (700 mg, 1.4 mmol, 70% yield) as a colorless oil. MS (ESP): m/z = 399.2 [M+H-BOC]⁺.

### Step 5:

### tert-Butyl N-[2-[tert-butyl(diphenyl)silyl]oxy-3-[2-oxo-3-(3-oxo-4H-pyrazino[2,3-b][1,4]oxazin-6-yl)-1,3-oxazolidin-5-yl]propyl]carbamate

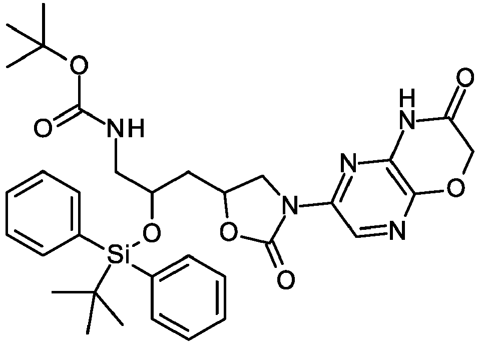

The title compound was prepared by analogy with 6-[5-[3-[*tert*-butyl(dimethyl)silyl]oxypropyl]-2-oxo-1,3-oxazolidin-3-yl]-4*H*-pyrazino[2,3-b][1,4]oxazin-3-one (Intermediate 154, step 1) and was obtained as a white solid. MS (ESP): m/z = 548.1 [M+H-BOC]⁺.

### Step 6:

### tert-Butyl N-[2-hydroxy-3-[2-oxo-3-(3-oxo-4H-pyrazino[2,3-b] [1,4]oxazin-6-yl)-1,3-oxazolidin-5-yl]propyl]carbamate, Isomer A and tert-butyl N-[2-hydroxy-3-[2-oxo-3-(3-oxo-4H-pyrazino[2,3-b][1,4]oxazin-6-yl)-1,3-oxazolidin-5-yl]propyl]carbamate, Isomer B

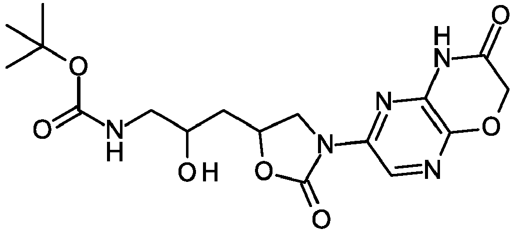

To a solution of *tert*-butyl *N*-[2-[*tert-*butyl(diphenyl)silyl]oxy-3-[2-oxo-3-(3-oxo-4*H-*pyrazino[2,3-b][1,4]oxazin-6-yl)-1,3-oxazolidin-5-yl]propyl]carbamate (100.0 mg, 0.150 mmol, 1 eq) in THF (20 mL) was added TBAF in THF (0.31 mL, 0.310 mmol, 2 eq) and the solution was stirred at 25 °C for 16 h. The solvent was removed to give the crude product which was purified by chromatography on silica gel (DCM:MeOH=10:1) to give 50 mg of a mixture of isomers. The isomers were separated by chiral SFC to give peak 1, title compound Isomer A (20 mg, 0.050 mmol, 31.6% yield) as a white solid and peak 2, title compound Isomer B (20 mg, 0.050 mmol, 31.6% yield) as a white solid. MS (ESP): m/z = 310.1 [M+H-BOC]⁺ for both products.

### Step 7:

### 6-[2-Oxo-5-[3-amino-2-hydroxypropyl]-1,3-oxazolidin-3-yl]-4H-pyrazino[2,3-b] [1,4]oxazin-3-one, Isomer A 2,2,2-trifluoroacetate

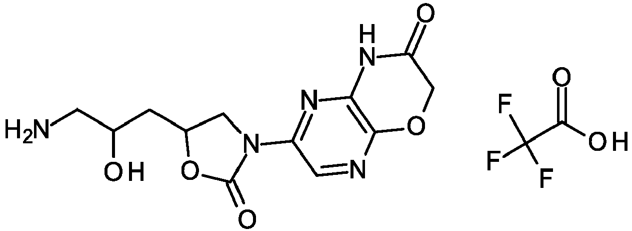

To a solution of *tert*-butyl *N*-[2-hydroxy-3-[2-oxo-3-(3-oxo-4*H*-pyrazino[2,3-b][1,4]oxazin-6-yl)-1,3-oxazolidin-5-yl]propyl]carbamate, Isomer A (20.0 mg, 0.050 mmol, 1 eq) in DCM (2 mL) was added trifluoroacetic acid (0.5 mL, 6.49 mmol) and the solution was stirred at 25 °C for 3 h. The solvent was removed to give the crude title compound (20 mg, 0.050 mmol, 96.7% yield) as a brown solid. MS (ESP): m/z = 310.1 [M+H]⁺.

### Intermediate 157

### 6-[2-Oxo-5-[3-amino-2-hydroxypropyl]-1,3-oxazolidin-3-yl]-4H-pyrazino[2,3-b] [1,4]oxazin-3-one, Isomer B 2,2,2-trifluoroacetate

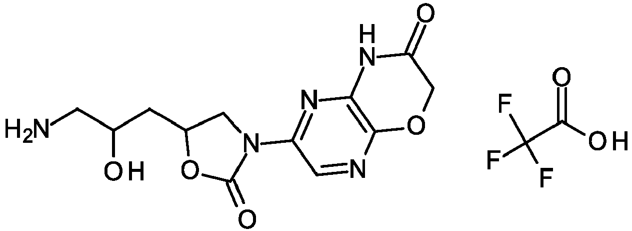

The title compound was prepared by analogy with 6-[2-oxo-5-[3-amino-2-hydroxypropyl]-1,3-oxazolidin-3-yl]-4*H*-pyrazino[2,3-b][1,4]oxazin-3-one, Isomer A 2,2,2-trifluoroacetate (Intermediate 156, step 7) using *tert*-butyl *N*-[2-hydroxy-3-[2-oxo-3-(3-oxo-4*H*-pyrazino[2,3-b][1,4]oxazin-6-yl)-1,3-oxazolidin-5-yl]propyl]carbamate, Isomer B (Intermediate 156, step 6) in place of *tert*-butyl *N*-[2-hydroxy-3-[2-oxo-3-(3-oxo-4*H*-pyrazino[2,3-b][1,4]oxazin-6-yl)-1,3-oxazolidin-5-yl]propyl]carbamate, Isomer A and was obtained as a brown solid. MS (ESP): m/z = 310.1 [M+H]⁺.

### Intermediate 158

### [1-Cyano-4-methyl-3-[2-(methylamino)-2-oxoethoxy]-6,7-dihydro-5H-cyclopenta[c]pyridin-6-yl]methyl4-methylbenzenesulfonate

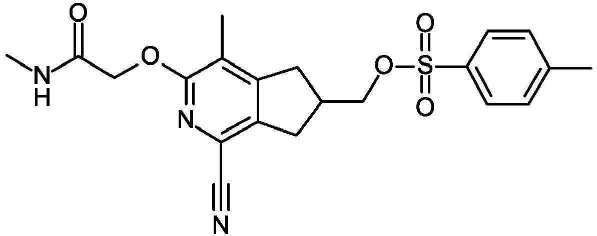

### Step 1:

### Dimethyl 1-bromo-4-methyl-3-[2-(methylamino)-2-oxoethoxy]-5,7-dihydrocyclopenta[c]pyridine-6,6-dicarboxylate

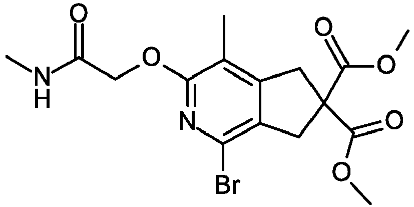

Dimethyl 1-bromo-4-methyl-3-oxo-2,3,5,7-tetrahydro-6*H*-cyclopenta[c]pyridine-6,6-dicarboxylate (Intermediate 145, step 2, 50 mg, 109 µmol, 1 eq), 2-chloro-*N*-methylacetamide (18 mg, 167 µmol, 1.53 eq), sodium iodide (4.18 mg, 27.9 µmol, 0.256 eq) and potassium carbonate (28 mg, 203 µmol, 1.86 eq) were combined with DMA (1 mL). The reaction mixture was stirred for 5.5 h at 50 °C. Further 2-chloro-N-methylacetamide (5.87 mg, 54.6 µmol, 0.5 eq) and potassium carbonate (15.1 mg, 109 µmol, 1 eq) were added to the mixture. The reaction mixture was stirred at 50°C for 18 h. The reaction mixture was treated with 5 mL sat. aq. NaHCOs and extracted with EtOAc (2 x 15 mL). The organic layers were dried (Na₂SO₄) and evaporated. The crude material was purified by flash chromatography (silica gel, 5g, 0% to 100% EtOAc in heptane) to give the title compound (38.4 mg, 72 % yield) as a light brown waxy solid. MS (ESP): m/z = 415.2 [M+H]⁺.

### Steps 2 to 5:

### [1-Cyano-4-methyl-3-[2-(methylamino)-2-oxoethoxy]-6,7-dihydro-5H-cyclopenta[c]pyridin-6-yl]methyl4-methylbenzenesulfonate

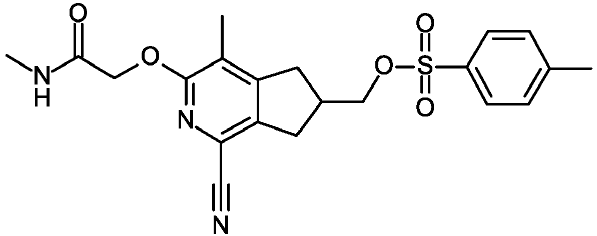

The title compound was prepared by analogy with [1-cyano-4-methyl-3-[[1-[(2-methylpropan-2-yl)oxycarbonylamino]cyclopropyl]methoxy]-6,7-dihydro-5*H*-cyclopenta[c]pyridin-6-yl]methyl 4-methylbenzenesulfonate (Intermediate 145, steps 2 to 5) and was obtained as a yellow foam. MS (ESP): m/z = 430.3 [M+H]⁺.

### Intermediate 159

### [1-Cyano-4-methyl-3-[2-oxo-2-(propan-2-ylamino)ethoxy]-6,7-dihydro-5H-cyclopenta[c]pyridin-6-yl]methyl 4-methylbenzenesulfonate

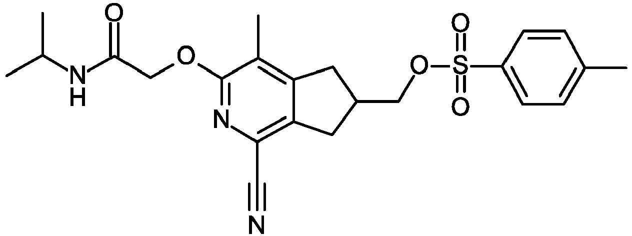

The title compound was prepared by analogy with [1-cyano-4-methyl-3-[2-(methylamino)-2-oxoethoxy]-6,7-dihydro-5*H*-cyclopenta[c]pyridin-6-yl]methyl 4-methylbenzenesulfonate (Intermediate 158) using 2-chloro-*N*-isopropylacetamide in place of 2-chloro-*N*-methylacetamide and was obtained as an off-white foam. MS (ESP): m/z = 458.3 [M+H]⁺.

### Intermediate 160

### [4-Methyl-3-[2-(methylamino)-2-oxoethoxy]-6,7-dihydro-5H-cyclopenta[c]pyridin-6-yl]methyl 4-methylbenzenesulfonate

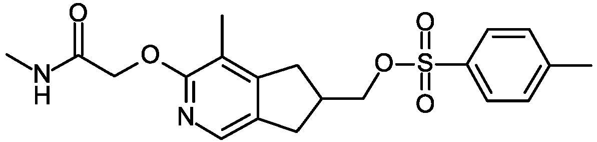

### Step 1:

### Methyl 2-[[6-(hydroxymethyl)-4-methyl-6,7-dihydro-5H-cyclopenta[c]pyridin-3-yl]oxy]acetate

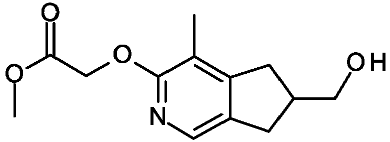

The title compound was prepared by analogy with methyl 4-methyl-3-[[(2*S*)-1-[(2-methylpropan-2-yl)oxycarbonyl]azetidin-2-yl]methoxy]-6,7-dihydro-5*H*-cyclopenta[c]pyridine-6-carboxylate (Intermediate 150, step 3) using 2-[[1-bromo-6-(hydroxymethyl)-4-methyl-6,7-dihydro-5*H*-cyclopenta[c]pyridin-3-yl]oxy]-*N*-methylacetamide (Intermediate 158, step 3) in place methyl 1-bromo-4-methyl-3-[[(2*S*)-1-[(2-methylpropan-2-yl)oxycarbonyl]azetidin-2-yl]methoxy]-6,7-dihydro-5*H*-cyclopenta[c]pyridine-6-carboxylate and was obtained as a light yellow oil which also contained 2-[[6-(hydroxymethyl)-4-methyl-6,7-dihydro-5*H-*cyclopenta[c]pyridin-3-yl]oxy]-*N*-methylacetamide. MS (ESP): m/z = 252.2 [M+H]⁺.

### Step 2:

### Methyl 2-[[4-methyl-6-[(4-methylphenyl)sulfonyloxymethyl]-6,7-dihydro-5H-cyclopenta[c]pyridin-3-yl]oxy]acetate

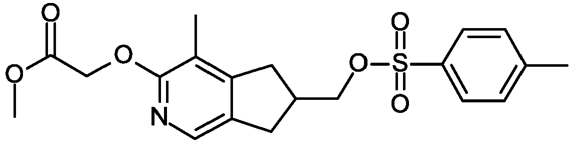

The title compound was prepared by analogy with [(1,2-*trans*)-4-chloro-1-[(2-methylpropan-2-yl)oxycarbonylamino]-2,3-dihydro-1*H*-inden-2-yl]methyl4-methylbenzenesulfonate, Enantiomer A (Intermediate 141, step 2) and was obtained as a colourless oil. MS (ESP): m/z = 406.1 [M+H]⁺.

### Step 3:

### 2-[[4-Methyl-6-[(4-methylphenyl)sulfonyloxymethyl]-6,7-dihydro-5H-cyclopenta[c]pyridin-3-yl]oxy]acetic acid

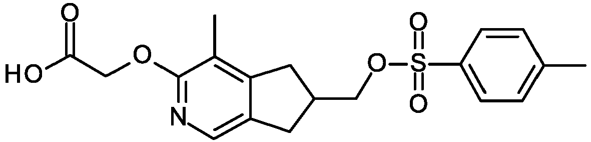

To a solution of methyl 2-[[4-methyl-6-[(4-methylphenyl)sulfonyloxymethyl]-6,7-dihydro-5*H-*cyclopenta[c]pyridin-3-yl]oxy]acetate (55 mg, 136 µmol, 1 eq) in MeOH (1 mL) was added LiOH 1N in H₂O (407 µL, 407 µmol, 3 eq). The reaction mixture was stirred at RT for 5 h. The crude reaction mixture was concentrated in vacuo. The reaction mixture was treated with 3 mL 1 M HCl and extracted with EtOAc (3 x 7 mL). The organic layers were dried (Na₂SO₄) and evaporated to give the title compound (45 mg, 60 % yield) as an off-white foam. MS (ESP): m/z = 392.2 [M+H]⁺.

### Step 4:

### [4-Methyl-3-[2-(methylamino)-2-oxoethoxy]-6,7-dihydro-5H-cyclopenta[c]pyridin-6-yl]methyl 4-methylbenzenesulfonate

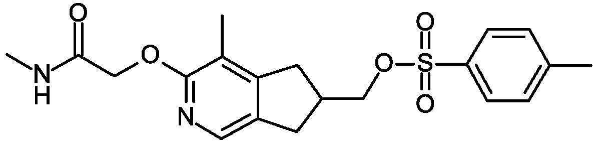

To a solution of 2-[[4-methyl-6-[(4-methylphenyl)sulfonyloxymethyl]-6,7-dihydro-5*H-*cyclopenta[c]pyridin-3-yl]oxy]acetic acid (45 mg, 115 µmol, 1 eq) in DMF (1 mL) were added methanamine hydrochloride (9.31 mg, 138 µmol, 1.2 eq), HATU (52.5 mg, 138 µmol, 1.2 eq) and *N,N-*diisopropylethylamine (44.6 mg, 59 µl, 345 µmol, 3 eq) and the resulting mixture was stirred for 3 h at RT. The reaction mixture was treated with 2 mL H₂O and extracted with EtOAc (2 x 10 mL). The organic layers were washed with 2 mL H₂O. The organic layers were dried (Na₂SO₄) and evaporated. The crude material was purified by flash chromatography (silica gel, 5g; 20% to 100% EtOAc in heptane) to give the title compound (16.3 mg, 28.7 % yield) as an off-white foam. MS (ESP): m/z = 405.2 [M+H]⁺.

### Intermediate 161

### [4-Methyl-3-[2-oxo-2-(propan-2-ylamino)ethoxy]-6,7-dihydro-5H-cyclopenta[c]pyridin-6-yl] methyl 4-methylbenzenesulfonate

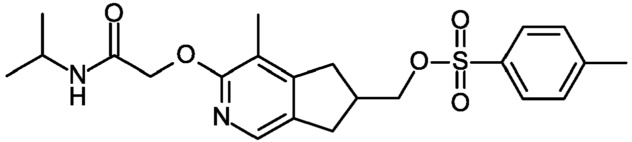

### Steps 1 to 3:

### 2-[[1-Bromo-6-(hydroxymethyl)-4-methyl-6,7-dihydro-5H-cyclopenta[c]pyridin-3-yl]oxy]-N-propan-2-ylacetamide

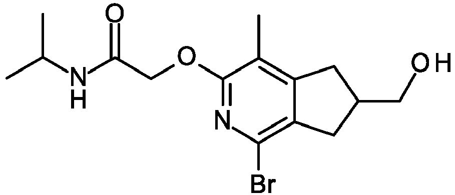

The title compound was prepared by analogy with 2-[[1-bromo-6-(hydroxymethyl)-4-methyl-6,7-dihydro-5*H*-cyclopenta[c]pyridin-3-yl]oxy]-N-methylacetamide (Intermediate 158, steps 1 to 3) using 2-chloro-*N*-isopropylacetamide in place of 2-chloro-*N*-methylacetamide and was obtained as a white solid. MS (ESP): m/z = 357.1, 359.1 [M+H]⁺.

### Step 4:

### 2-[[6-(Hydroxymethyl)-4-methyl-6,7-dihydro-5H-cyclopenta[c]pyridin-3-yl]oxy]-N-propan-2-ylacetamide

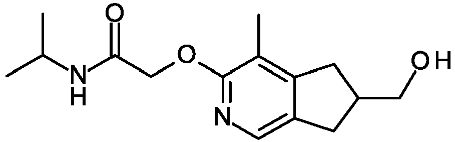

To a solution of 2-[[1-bromo-6-(hydroxymethyl)-4-methyl-6,7-dihydro-5*H-*cyclopenta[c]pyridin-3-yl]oxy]-*N*-propan-2-ylacetamide (97 mg, 272 µmol, 1 eq) in THF (2 mL) and H₂O (0.2 mL) under Ar was added 10% palladium on carbon (8.67 mg). The reaction mixture was stirred for 3.5 days at RT under an atmosphere of hydrogen. The reaction mixture was filtered and the filter cake washed thoroughly with MeOH (10 mL). The filtrate was concentrated in vacuo at RT and the remaining material was purified by flash chromatography (silica gel, 5g; 2% to 15% MeOH in DCM) to give the title compound (34 mg, 31.5 % yield) as a colorless waxy solid. MS (ESP): m/z = 279.2 [M+H]⁺.

### Step 5:

### [4-Methyl-3-[2-oxo-2-(propan-2-ylamino)ethoxy]-6,7-dihydro-5H-cyclopenta[c]pyridin-6-yl] methyl 4-methylbenzenesulfonate

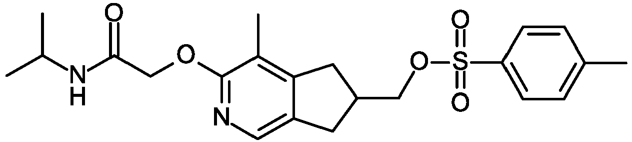

The title compound was prepared by analogy with [4-methyl-3-[2-(methylamino)-2-oxoethoxy]-6,7-dihydro-5*H*-cyclopenta[c]pyridin-6-yl]methyl 4-methylbenzenesulfonate (Intermediate 160, step 4) and was obtained as a light yellow waxy solid. MS (ESP): m/z = 433.2 [M+H]⁺.

### Intermediate 162

### [rac-1,2-trans-4-Cyano-1-[(2-methylpropan-2-yl)oxycarbonylamino]-2,3-dihydro-1H-inden-2-yl] methyl 4-methylbenzenesulfonate

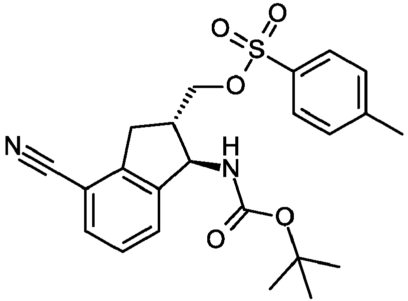

The title compound was prepared by analogy with [(1,2-*trans*)-4-chloro-1-[(2-methylpropan-2-yl)oxycarbonylamino]-2,3-dihydro-1*H*-inden-2-yl]methyl 4-methylbenzenesulfonate, Enantiomer A (Intermediate 141, step 2) using *tert*-butyl *N*-[*rac*-1,2-*trans*-4-cyano-2-(hydroxymethyl)-2,3-dihydro-1*H*-inden-1-yl]carbamate (Intermediate 88, step 6) in place of *tert-*butyl *N*-[1,2-*trans*-4-chloro-2-(hydroxymethyl)-2,3-dihydro-1*H*-inden-1-yl]carbamate, Enantiomer A and was obtained as a white foam. MS (ESP): m/z = 343.1 [M+H-BOC]⁺.

### Intermediate 163

### rac-1,2-trans-1-[tert-Butyl(dimethyl)silyl]oxy-4-chloro-2,3-dihydro-1H-indene-2-carbaldehyde

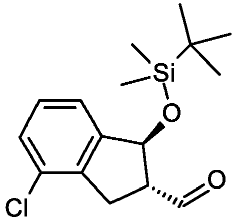

### Step 1:

### Methyl 7-chloro-3-oxo-1,2-dihydroindene-2-carboxylate

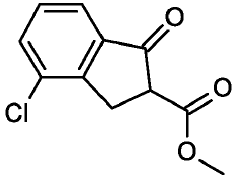

A solution of 4-chloro-2,3-dihydro-1*H*-inden-1-one (2 g, 12 mmol, 1 eq) in toluene (10 mL) was added dropwise slowly to a refluxing mixture of sodium hydride 55% in mineral oil (1.47 g, 33.6 mmol, 2.8 eq) and dimethyl carbonate (2.16 g, 2.02 ml, 24 mmol, 2 eq) in toluene (10 mL). The mixture was stirred at reflux for 1 h. The mixtre was cooled to RT, then acetic acid (2.88 g, 2.75 ml, 48 mmol, 4 eq) was added slowly. The mixture was poured onto ice water and extracted with EtOAc (3 x). The organic layers were washed with brine, dried over Na₂SO₄ and evaporated. The residue was adsorbed on adsorbent and purified by flash column chromatography (70 g silica gel; heptane/EtOAc 98:2 to 90:10) to give the title compound (1.14 g) as a light brown solid. MS (ESP): m/z = 225.1 [M+H]⁺.

### Step 2:

### Methyl 4-chloro-1-hydroxy-2,3-dihydro-1H-indene-2-carboxylate

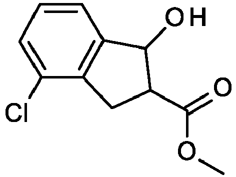

To a solution of methyl 7-chloro-3-oxo-1,2-dihydroindene-2-carboxylate (1.14 g, 5.07 mmol, 1 eq) in methanol (90 mL) was added sodium borohydride (384 mg, 10.1 mmol, 2 eq) portionwise at 0 °C. The mixture was stirred at RT for 30 min. The mixture was partially concentrated, quenched with 1N HCl and the pH was adjusted to 8 with NaHCO₃. The mixture was extracted with EtOAc (3 x) and the combined organic layers were washed with brine (with some NaHCO₃), dried over Na₂SO₄ and evaporated. The remaining residue was adsorbed on adsorbent and purified by flash column chromatography (50 g silica gel; heptane/EtOAc 90:10 to 70:30) to give the title compound (917 mg) as a white solid. MS (ESP): m/z = 209.1 [M+H-H₂O]⁺.

### Step 3:

### Methyl rac-1,2-trans-1-[tert-butyl(dimethyl)silyl]oxy-4-chloro-2,3-dihydro-1H-indene-2-carboxylate and methyl rac-1,2-cis-1-[tert-butyl(dimethyl)silyl]oxy-4-chloro-2,3-dihydro-1H-indene-2-carboxylate

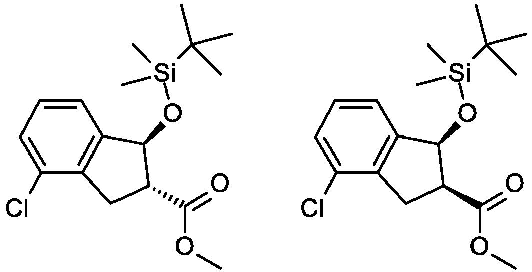

*tert*-Butyldimethylsilyl trifluoromethanesulfonate (1.76 g, 6.64 mmol, 2 eq) was added to a colorless solution of methyl 4-chloro-1-hydroxy-2,3-dihydro-1*H*-indene-2-carboxylate (mixture of *cis* and *trans* isomers) (753 mg, 3.32 mmol, 1 eq) and 2,6-dimethylpyridine (783 mg, 7.31 mmol, 2.2 eq) in dichloromethane (15 mL) at 0 °C. The mixture was stirred at 0 °C for 30 min.

The mixture was poured onto 40 mL water and the pH was adjusted to 5 with 0.1 N HCl. The mixture was extracted with EtOAc (3 x) and the organic layers were washed with brine, dried over Na₂SO₄ and evaporated. The residue was adsorbed on adsorbent and purified by flash column chromatography (50 g silica gel; heptane/EtOAc 98:2 to 95:5) to give methyl *rac*-1,2-*trans*-1-[*tert*-butyl(dimethyl)silyl]oxy-4-chloro-2,3-dihydro-1*H*-indene-2-carboxylate (401 mg) as a colorless oil and methyl *rac*-1,2-*cis*-1-[*tert*-butyl(dimethyl)silyl]oxy-4-chloro-2,3-dihydro-1*H*-indene-2-carboxylate as a white solid (516 mg). MS (ESP): m/z = 209.1 [M+H-TBSOH]⁺ for both products.

### Step 4:

### rac-1,2-trans-1-[tert-Butyl(dimethyl)silyl]oxy-4-chloro-2,3-dihydro-1H-indene-2-carbaldehyde

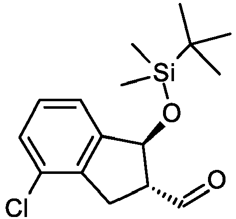

The title compound was prepared by analogy to 2-[(6-formyl-1,4-dimethyl-6,7-dihydro-5*H-*cyclopenta[c]pyridin-3-yl)oxy]-*N*-methylacetamide (Intermediate 72, step 7) and was obtained as a white solid. MS (ESP): m/z = 179.0 [M+H-TBSOH]⁺.

### Intermediate 164

### [rac-1,2-cis-1-[tert-Butyl(dimethyl)silyl]oxy-4-chloro-2,3-dihydro-1H-inden-2-yl]methyl 4-methylbenzenesulfonate

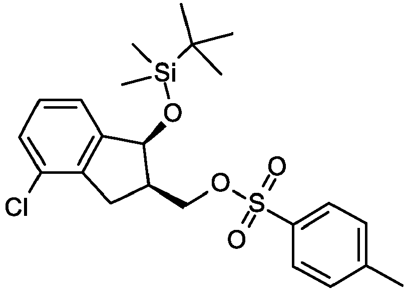

The title compound was prepared from methyl *rac*-1,2-*cis*-1-[*tert*-butyl(dimethyl)silyl]oxy-4-chloro-2,3-dihydro-1*H*-indene-2-carboxylate (Intermediate 164, step 3) by analogy with [1-methyl-3-[2-[(2-methylpropan-2-yl)oxycarbonylamino]ethoxy]-6,7-dihydro-5*H-*cyclopenta[c]pyridin-6-yl]methyl 4-methylbenzenesulfonate (Intermediate 143, steps 3 and 4) and was obtained as a colorless gum.

### Intermediate 165

### 2-(Aminomethyl)-4-chloro-1,3-dihydroinden-2-ol

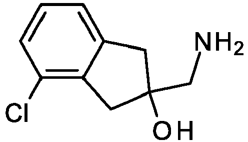

### Step 1:

### 4-Chloro-2-hydroxy-1,3-dihydroindene-2-carbonitrile

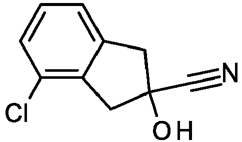

A mixture of 4-chloro-1,3-dihydroinden-2-one (200.0 mg, 1.2 mmol, 1 eq) and trimethylsilyl cyanide (119.1 mg, 1.2 mmol, 1 eq) in DCM (2 mL) was stirred at 25 °C for 2 h. The reaction was quenched with sat. aq. NH₄Cl (0.1 mL), celite (100 mg) was added and the mixture filtered. The filtrate was concentrated under vacuum to give the crude product. The crude product was purified by flash chromatography on silica gel (PE/EtOAc=50/1 to 10/1) to give the title compound (55 mg, 0.280 mmol, 23.7 % yield) as a colorless oil. ¹H-NMR (400 MHz, CDCl₃) δ 7.23 (m, 3H), 3.68 (dd, 2H), 3.44 (dd, 2H).

### Step 2:

### 2-(Aminomethyl)-4-chloro-1,3-dihydroinden-2-ol

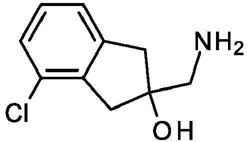

A mixture of 4-chloro-2-hydroxy-1,3-dihydroindene-2-carbonitrile (55.0 mg, 0.280 mmol, 1 eq) and lithium aluminium hydride 1M in THF (0.31 mL, 0.310 mmol, 1.1 eq) in THF (2 mL) was stirred at 0 °C for 0.5 h. The reaction was quenched with sat. aq. NH₄Cl (0.1 mL), celite (100 mg) was added and the mixture filtered. The filtrate was concentrated under vacuum to give crude title compound (30 mg, 0.150 mmol, 53.4 % yield) as a light brown oil. The crude product was used for the next step directly.

### Intermediate 166

### 2-[2-[2-[2-Oxo-3-(3-oxo-4H-pyrido[3,2-b][1,4]oxazin-6-yl)-1,3-oxazolidin-5-yl]ethylamino]ethyl]isoindole-1,3-dione

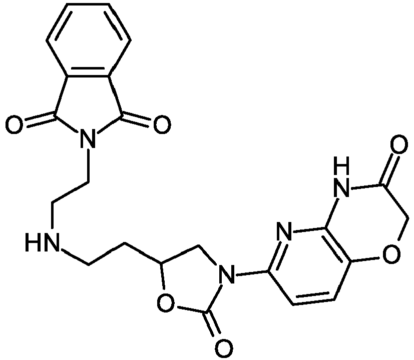

To a solution of *N*-(2-oxoethyl)phthalimide (CAS# 2913-97-5, 67.98 mg, 0.360 mmol, 1 eq) and 6-[5-(2-aminoethyl)-2-oxo-1,3-oxazolidin-3-yl]-4*H*-pyrido[3,2-b][1,4]oxazin-3-one (Intermediate 1, 100.0 mg, 0.360 mmol, 1 eq) in methanol (5 mL), was added sodium cyanoborohydride (90.3 mg, 1.44 mmol, 4 eq) at 0 °C . Then the solution was stirred at 25 °C for 16 h. The mixture was concentrated and the remaining residue was purified by preparative HPLC (formic acid system) followed by freeze drying to give the title compound (20 mg, 0.040 mmol, 11.8% yield) as an off-white solid. MS (ESP): m/z = 452.2 [M+H]⁺.

### Intermediate 167

### tert-Butyl (4R)-4-[(6-formyl-1,4-dimethyl-6,7-dihydro-5H-cyclopenta[c]pyridin-3-yl)oxymethyl] -2,2-dimethyl-1,3-oxazolidine-3-carboxylate

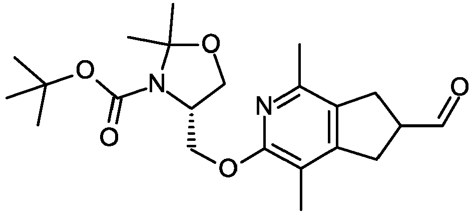

### Step 1:

### Dimethyl 3-[[(4R)-2,2-dimethyl-3-[(2-methylpropan-2-yl)oxycarbonyl]-1,3-oxazolidin-4-yl] methoxy] -1,4-dimethyl-5,7-dihydrocyclopenta[c]pyridine-6,6-dicarboxylate

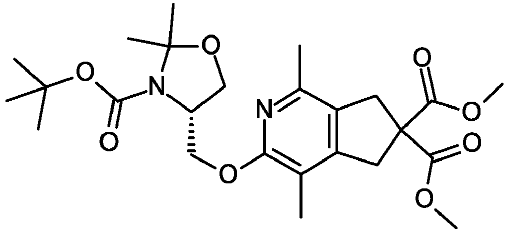

The title compound was prepared by analogy with dimethyl 1,4-dimethyl-3-[(5-oxomorpholin-2-yl)methoxy]-5,7-dihydrocyclopenta[c]pyridine-6,6-dicarboxylate (Intermediate 64, step 3) using *tert*-butyl (4*S*)-4-(hydroxymethyl)-2,2-dimethyl-1,3-oxazolidine-3-carboxylate (CAS# 108149-65-1) in place of 6-(hydroxymethyl)morpholin-3-one and was obtained as a colourless gum. MS (ESP): m/z = 493.3 [M+H]⁺.

### Steps 2 and 3:

### tert-Butyl (4R)-4-[(6-formyl-1,4-dimethyl-6,7-dihydro-5H-cyclopenta[c]pyridin-3-yl)oxymethyl] -2,2-dimethyl-1,3-oxazolidine-3-carboxylate

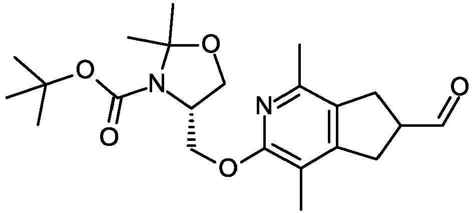

The title compound was prepared by analogy with 2-[(6-formyl-1,4-dimethyl-6,7-dihydro-5*H-*cyclopenta[c]pyridin-3-yl)oxy]-*N*-methylacetamide (Intermediate 72, steps 6 and 7) and was obtained as a colorless gum. MS (ESP): m/z = 405.4 [M+H]⁺.

### Intermediate 168

### tert-Butyl (4R)-4-[(1-cyano-6-formyl-4-methyl-6,7-dihydro-5H-cyclopenta[c]pyridin-3-yl)oxymethyl] -2,2-dimethyl-1,3-oxazolidine-3-carboxylate

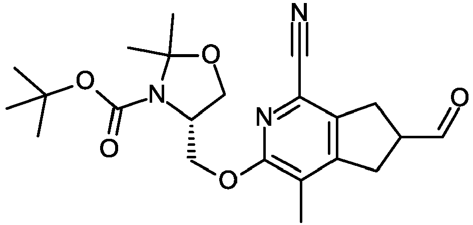

### Steps 1 and 2:

### tert-Butyl (4R)-4-[(1-bromo-6-methoxycarbonyl-4-methyl-6,7-dihydro-5H-cyclopenta[c]pyridin-3-yl)oxymethyl]-2,2-dimethyl-1,3-oxazolidine-3-carboxylate

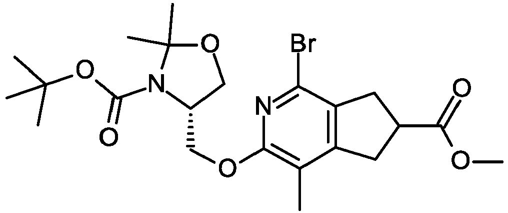

The title compound was prepared by analogy with *tert*-butyl (4*R*)-4-[(6-methoxycarbonyl-1,4-dimethyl-6,7-dihydro-5*H*-cyclopenta[c]pyridin-3-yl)oxymethyl]-2,2-dimethyl-1,3-oxazolidine-3-carboxylate (Intermediate 167, steps 1 and 2) using dimethyl 1-bromo-4-methyl-3-oxo-5,7-dihydro-2*H*-cyclopenta[c]pyridine-6,6-dicarboxylate (Intermediate 72, step 3) in place of dimethyl 1,4-dimethyl-3-oxo-5,7-dihydro-2*H*-cyclopenta[c]pyridine-6,6-dicarboxylate and was obtained as a colorless waxy solid. MS (ESP): m/z = 499.3, 501.3 [M+H]⁺.

### Steps 3 to 5:

### tert-Butyl (4R)-4-[(1-cyano-6-formyl-4-methyl-6,7-dihydro-5H-cyclopenta[c]pyridin-3-yl)oxymethyl]-2,2-dimethyl-1,3-oxazolidine-3-carboxylate

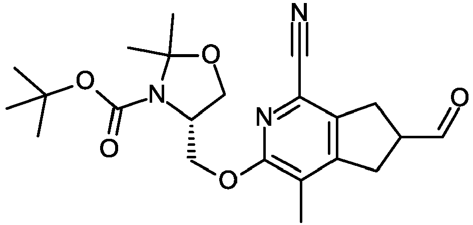

The title compound was prepared by analogy with 2-[(4-cyano-2-formyl-2,3-dihydro-1*H*-inden-5-yl)oxy]-*N*-methylacetamide (Intermediate 36, steps 5 to7) and was obtained as a colorless waxy solid. MS (ESP): m/z = 316.3 [M+H-BOC]⁺.

### Intermediate 169

### tert-Butyl (4R)-4-[(7-cyano-2-formyl-2,3-dihydro-1H-inden-5-yl)oxymethyl]-2,2-dimethyl-1,3-oxazolidine-3-carboxylate

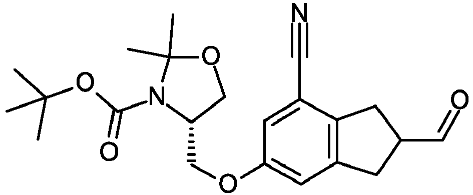

### Step 1:

### tert-Butyl (4R)-4-[(7-cyano-2-ethoxycarbonyl-2,3-dihydro-1H-inden-5-yl)oxymethyl]-2,2-dimethyl-1,3-oxazolidine-3-carboxylate

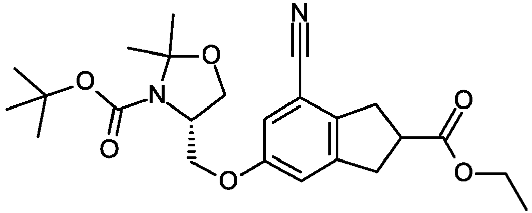

The title compound was prepared by analogy with dimethyl 3-[[(4*R*)-2,2-dimethyl-3-[(2-methylpropan-2-yl)oxycarbonyl]-1,3-oxazolidin-4-yl]methoxy]-1,4-dimethyl-5,7-dihydrocyclopenta[c]pyridine-6,6-dicarboxylate (Intermediate 167, step 1) using ethyl 4-cyano-6-hydroxy-2,3-dihydro-1*H*-indene-2-carboxylate (Intermediate 70, step 4) in place of dimethyl 1,4-dimethyl-3-oxo-5,7-dihydro-2*H*-cyclopenta[c]pyridine-6,6-dicarboxylate and was obtained as a colorless oil. MS (ESP): m/z = 389.3 [M+H-tBu]⁺.

### Steps 2 and 3:

### tert-Butyl (4R)-4-[(7-cyano-2-formyl-2,3-dihydro-1H-inden-5-yl)oxymethyl]-2,2-dimethyl-1,3-oxazolidine-3-carboxylate

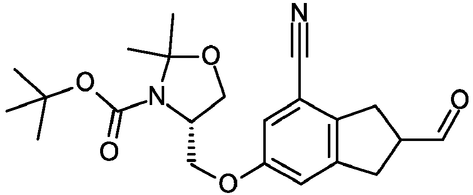

The title compound was prepared by analogy with 1-chloro-6,7-dihydro-5*H-*cyclopenta[c]pyridine-6-carbaldehyde (Intermediate 19, steps 3 and 4) and was obtained as a white foam. MS (ESP): m/z = 345.2 [M+H-tBu]⁺.

### Intermediate 170

### tert-butyl (4R)-4-[(4-chloro-6-formyl-6,7-dihydro-5H-cyclopenta[c]pyridin-3-yl)oxymethyl]-2,2-dimethyl-1,3-oxazolidine-3-carboxylate

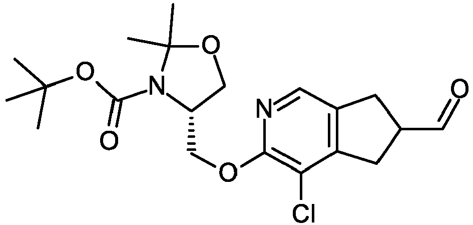

The title compound was prepared by analogy with *tert*-butyl (4*R*)-4-[(6-formyl-1,4-dimethyl-6,7-dihydro-5*H*-cyclopenta[c]pyridin-3-yl)oxymethyl]-2,2-dimethyl-1,3-oxazolidine-3-carboxylate (Intermediate 167) using dimethyl 4-chloro-3-oxo-5,7-dihydro-2*H*-cyclopenta[c]pyridine-6,6-dicarboxylate (Intermediate 116, step 1) in place of dimethyl 1,4-dimethyl-3-oxo-5,7-dihydro-2*H*-cyclopenta[c]pyridine-6,6-dicarboxylate and was obtained as a colorless gum. MS (ESP): m/z = 411.3 [M+H]⁺.

### Intermediate 171

### 6-[5-[3-Amino-2-[tert-butyl(dimethyl)silyl]oxypropyl]-2-oxo-1,3-oxazolidin-3-yl]-4H-pyrido[3,2-b][1,4]oxazin-3-one

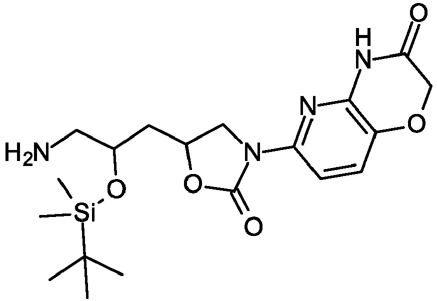

### Steps 1 and 2:

### tert-Butyl N-[2-[tert-butyl(dimethyl)silyl]oxy-3-(oxiran-2-yl)propyl]carbamate

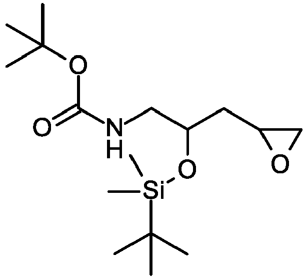

The title compound was prepared by analogy with tert-butyl *N*-[2-[*tert*-butyl(diphenyl)silyl]oxy-3-(oxiran-2-yl)propyl]carbamate (Intermediate 156, steps 1 and 2) using *tert-*butylchlorodimethylsilane in place of tert-butylchlorodiphenylsilane and was obtained as a colorless oil.

### Step 3:

### tert-Butyl N-[5-amino-2-[tert-butyl(dimethyl)silyl]oxy-4-hydroxypentyl]carbamate

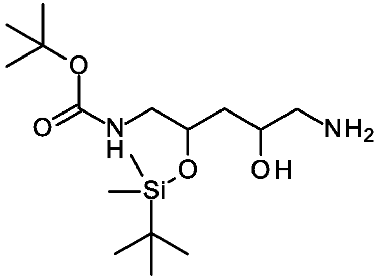

To a solution of *tert*-butyl *N*-[2-[*tert*-butyl(dimethyl)silyl]oxy-3-(oxiran-2-yl)propyl]carbamate (2.0 g, 6.03 mmol, 1 eq) in THF (30 mL) was added ammonium hydroxide, 28% solution (20.0 mL, 6.03 mmol, 1 eq) and the solution was stirred at 25 °C for 16 h. The solution was concentrated to give the crude title compound (2 g, 5.74 mmol, 95.1% yield) as a light yellow oil.

### Steps 4 to 6:

### tert-Butyl N-[2-[tert-butyl(dimethyl)silyl]oxy-3-[2-oxo-3-(3-oxo-4H-pyrido[3,2-b][1,4]oxazin-6-yl)-1,3-oxazolidin-5-yl]propyl]carbamate

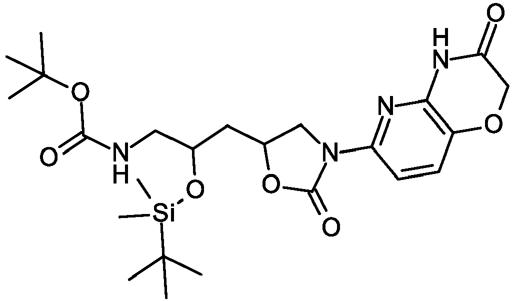

The title compound was prepared by analogy with benzyl *N*-[*rac*-3a,7a-*trans*-6,7a-*cis*-2-oxo-3-(3-oxo-4*H*-pyrido[3,2-b][1,4]oxazin-6-yl)-3a,4,5,6,7,7a-hexahydro-1,3-benzoxazol-6-yl]carbamate (Intermediate 20, steps 3 to 5) and was obtained as a yellow solid. MS (ESP): m/z 545.3 [M+Na]⁺.

### Step 7:

### 6-[5-[3-Amino-2-[tert-butyl(dimethyl)silyl]oxypropyl]-2-oxo-1,3-oxazolidin-3-yl]-4H-pyrido[3,2-b][1,4]oxazin-3-one

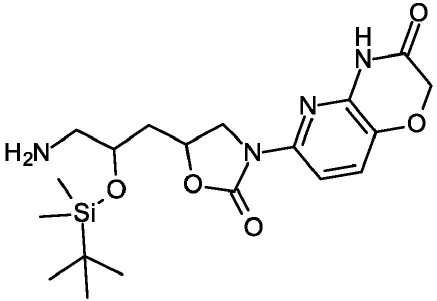

To a solution of *tert*-butyl *N*-[2-[*tert*-butyl(dimethyl)silyl]oxy-3-[2-oxo-3-(3-oxo-4*H*-pyrido[3,2-b][1,4]oxazin-6-yl)-1,3-oxazolidin-5-yl]propyl]carbamate (140.0 mg, 0.270 mmol, 1 eq) in DCM (20 mL) was added trifluoroacetic acid (0.41 mL, 5.36 mmol, 20 eq) and the solution was stirred at 25 °C for 2 h. The solvent was removed to give the crude title compound (110 mg, 0.260 mmol, 97.2 % yield) as a brown oil. MS (ESP): m/z = 423.2 [M+H]⁺.

### Intermediate 172

### Ethyl 1-ethyl-6-fluoro-8-[(4-methylphenyl)sulfonyloxymethyl]-4-oxo-8,9-dihydro-7H-cyclopenta[h]quinoline-3-carboxylate

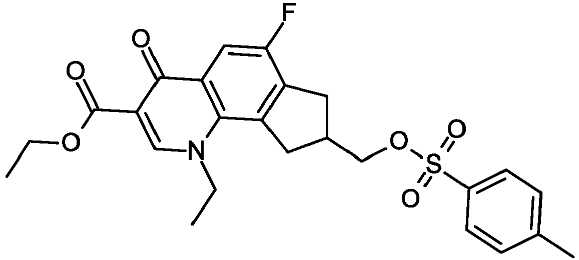

### Step 1:

### 2,3-Bis(bromomethyl)-1-fluoro-4-nitrobenzene

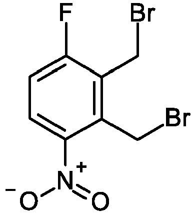

The title compound was prepared from 1-fluoro-2,3-dimethyl-4-nitrobenzene (CAS# 1736-87-4) by analogy with 2-bromo-3-(bromomethyl)-4-(chloromethyl)-5-fluoropyridine (Intermediate 27, step 4) and was obtained as a light-yellow solid.

### Step 2:

### Dimethyl 4-fluoro-7-nitro-1,3-dihydroindene-2,2-dicarboxylate

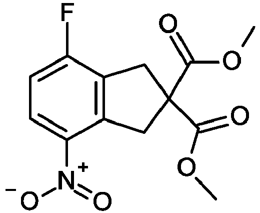

To a solution of dimethyl malonate (35.56 g, 269.15 mmol, 1.1 eq) in THF (100.0 mL) and methanol (44.4 mL) was added sodium methoxide (39.66 g, 734.06 mmol, 3 eq) at 16 °C and the mixture stirred at 16 °C for 30 min. Then 2,3-bis(bromomethyl)-1-fluoro-4-nitrobenzene (80.0 g, 244.69 mmol, 1 eq) dissolved in THF (50.0 mL) was added quickly at 16 °C and the resulting mixture stirred at 16 °C for 15.5 hrs. The reaction mixture was concentrated and diluted with DCM (30.0 mL). The organic phase was washed with brine, dried with anhdrous Na₂SO₄ and concentrated. The crude product was purified by silical column chromatography (PE: EtOAc=50:1~30:1) to give the title compound (50 g, 168.21 mmol, 60.3 % yield) as a white solid. MS (ESP): m/z = 298.1 [M+H]⁺.

### Step 3:

### Methyl 4-fluoro-7-nitro-2,3-dihydro-1H-indene-2-carboxylate

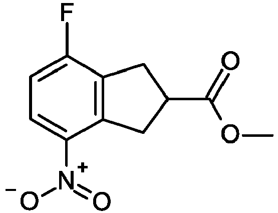

The title compound was prepared by analogy with methyl 1-chloro-3-methoxy-6,7-dihydro-5*H-*cyclopenta[c]pyridine-6-carboxylate (Intermediate 36, step 6) and was obtained as an off-white solid. MS (ESP): m/z = 240.1 [M+H]⁺.

### Step 4:

### Methyl 4-amino-7-fluoro-2,3-dihydro-1H-indene-2-carboxylate

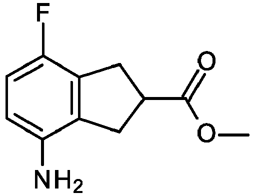

To a solution of methyl 4-fluoro-7-nitro-2,3-dihydro-1*H*-indene-2-carboxylate (9.0 g, 37.63 mmol, 1 eq) in EtOAc (4.18 mL) was added Pd/C (2.5 g) at 15 °C and the mixture stirred at 30 °C for 16 h under hydrogen (30 psi). The reaction was filtered and the filtrate was concentrated and the remaining residue was purified by silica column (PE: EtOAc=10:1~3:1) to give the title compound (8 g) as a white solid. MS (ESP): m/z = 210.1 [M+H]⁺.

### Step 5:

### (4-Amino-7-fluoro-2,3-dihydro-1H-inden-2-yl)methanol

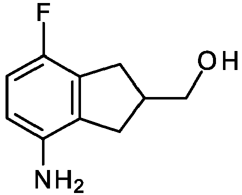

To a solution of methyl 4-amino-7-fluoro-2,3-dihydro-1*H*-indene-2-carboxylate (7.9 g, 37.76 mmol, 1 eq) in tetrahydrofuran (100.08 mL) was added lithium aluminum hydride (2.87 g, 75.52 mmol, 2 eq) at 0 °C and the mixture stirred at 0 °C for 1 h. 0.76 mL water, 0.76 mL 10% NaOH aqueous and 2.28 mL water were added to quench the reaction. After 30 mins, 100 mL EtOAc and excess anhydrous Na₂SO₄ were added. After stirring at room temperature for 30 min. the reaction mixture was filtered, the filtrate concentrated and the remaining residue purified by silica column (PE: EtOAc=5:1~1:1) to give the title compound as a white solid. ¹H-NMR (400 MHz, d6-DMSO) δ 6.62 (dd, 1H), 6.34 (dd, 1H), 4.66 (br m, 3H), 3.36 (m, 2H), 2.86 (m, 1H), 2.71 (m, 1H), 2.5 (m, 3H).

### Step 6:

### Diethyl 2-[[[7-fluoro-2-(hydroxymethyl)-2,3-dihydro-1H-inden-4-yl]amino]methylidene]propanedioate

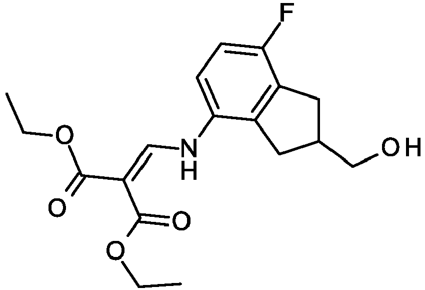

To solution of (4-amino-7-fluoro-2,3-dihydro-1*H*-inden-2-yl)methanol (3.0 g, 16.56 mmol, 1 eq) in ethanol (50 mL) was added diethyl 2-(methoxymethylene)propanedioate (4.35 g, 21.52 mmol, 1.3 eq) at 15 °C and the mixture was stirred at 85 °C for 12 h. The reaction was concentrated and the remaining residue was purified by silica column chromatography (PE: EtOAc =5:1~2:1) to give the title compound (5 g, 14.23 mmol, 86.0 % yield) as white solid.

### Step 7:

### Diethyl 2-[[ethyl-[7-fluoro-2-(hydroxymethyl)-2,3-dihydro-1H-inden-4-yl]amino]methylidene]propanedioate

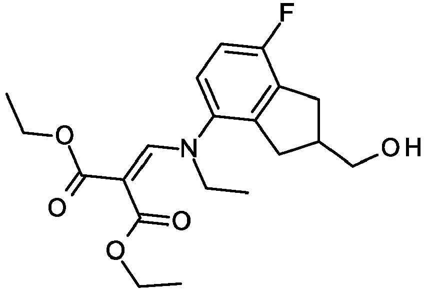

To a solution of diethyl 2-[[[7-fluoro-2-(hydroxymethyl)-2,3-dihydro-1*H*-inden-4-yl]amino]methylidene]propanedioate (1.0 g, 2.85 mmol, 1 eq) in MeCN (80 mL) were added iodoethane (2.3 mL, 28.46 mmol, 10 eq) and potassium carbonate (1573.3 mg, 11.38 mmol, 4 eq). The mixture was stirred at 90 °C for 12 h. The mixture was filtered and concentrated to give the title compound (1.1 g) as a light yellow solid. MS (ESP): m/z = 380.1 [M+H]⁺.

### Step 8:

### Ethyl 1-ethyl-6-fluoro-8-(hydroxymethyl)-4-oxo-8,9-dihydro-7H-cyclopenta[h]quinoline-3-carboxylate

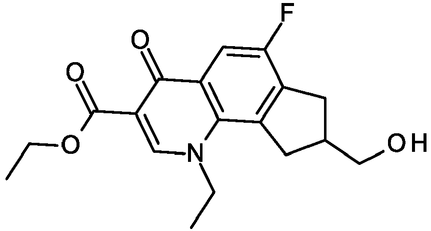

A solution of diethyl 2-[[ethyl-[7-fluoro-2-(hydroxymethyl)-2,3-dihydro-1*H*-inden-4-yl]amino]methylidene]propanedioate (800.0 mg, 2.11 mmol, 1 eq) in Eaton's reagent (7.7 wt% phosphorus pentoxide solution in methanesulfonic acid) (12.0 mL, 2.11 mmol, 1 eq) was stirred at 50 °C for 0.5 h. To the mixture was added aqueous sodium hydrogen carbonate (pH=7) and the mixture extracted with EtOAc (3 × 25 mL). The combined extracts were concentratied and the remaining residue was purified by preparative HPLC (formic acid system) to give the title compound (114.7 mg, 0.340 mmol, 15.6% yield) as a light yellow solid. MS (ESP): m/z = 334.1 [M+H]⁺.

### Step 9:

### Ethyl 1-ethyl-6-fluoro-8-[(4-methylphenyl)sulfonyloxymethyl]-4-oxo-8,9-dihydro-7H-cyclopenta [h] quinoline-3-carboxylate

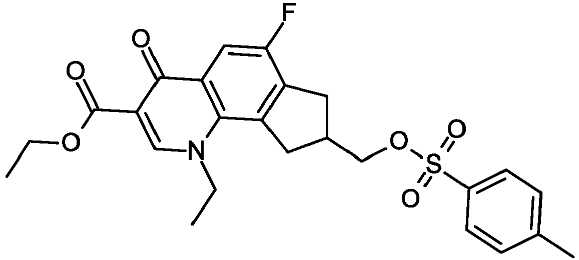

To a solution of ethyl 1-ethyl-6-fluoro-8-(hydroxymethyl)-4-oxo-4,7,8,9-tetrahydro-1*H-*cyclopenta[h]quinoline-3-carboxylate (111 mg, 333 µmol, 1 eq) in DCM (2 mL) were added *p-*toluenesulfonyl chloride (254 mg, 1.33 mmol, 4 eq), *N*,*N*-diisopropylethylamine (215 mg, 285 µl, 1.66 mmol, 5 eq) and DMAP (163 mg, 1.33 mmol, 4 eq). The reaction mixture was stirred at RT for 4 h. The reaction mixture was treated with H₂O (5 mL) and extracted with DCM (2 × 5 mL). The organic layers were dried (Na₂SO₄) and evaporated. The residue was first purified by flash chromatography (silica gel, 20g; 10% to 100% EtOAc in heptane) then purified further by flash chromatography (silica gel, 10g, 0% to 10% MeOH in DCM) to give the title compound (142 mg, 85.7 % yield) as a light green solid. MS (ESP): m/z = 488.3 [M+H]⁺.

### Intermediate 173

### 9H-Fluoren-9-ylmethyl N-(4-chloro-2-formyl-1,3-dihydroinden-2-yl)carbamate

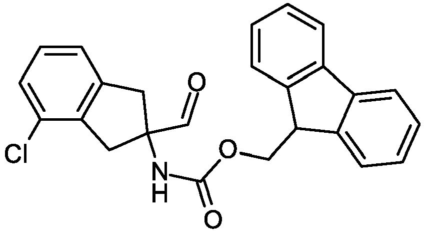

### Step 1:

### 4-Chlorospiro[1,3-dihydroindene-2,5'-imidazolidine]-2',4'-dione

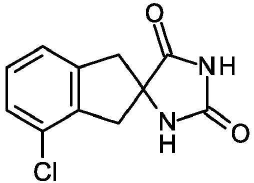

To a solution of 4-chloro-1,3-dihydroinden-2-one (3000.0 mg, 18.01 mmol, 1 eq) in ethanol (45 mL) and water (45 mL) were added ammonium carbonate (6920.0 mg, 72.02 mmol, 4 eq) and potassium cyanide (1800.0 mg, 27.64 mmol, 1.54 eq). The mixture was heated to 50 °C and stirred for 12 h. The mixture was filtered, the filtrate was extracted with ethyl acetate (100 mL × 3) and the combined extracts were concentrated to give a crude product. The crude product was triturated with PE/EA=10:1 and dried in vacuo to give the title compound (4000 mg, 16.9 mmol, 93.9 % yield) as brown solid. The crude product was used for next step directly. MS (ESP): m/z = 237 [M+H]⁺.

### Step 2:

### Methyl 2-amino-4-chloro-1,3-dihydroindene-2-carboxylate

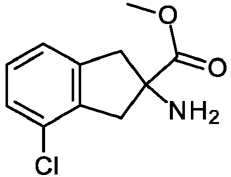

A mixture of 4-chlorospiro[1,3-dihydroindene-2,5'-imidazolidine]-2',4'-dione (4000.0 mg, 16.9 mmol, 1 eq) in NaOH (100.0 mL, 300 mmol, 17.75 eq) and 1,4-dioxane (20 mL) was stirred at 90 °C for 12 h. To the mixture was added 1N HCl to adjust the pH to 6-7. The mixture was extracted with ethyl acetate (150 mL × 2). The aqueous phase was lyophilized to give crude 2-amino-4-chloro-1,3-dihydroindene-2-carboxylic acid as a light yellow solid. This material was dissolved in methanol (50 mL) and the solution cooled to 0°C. To the mixture was added 98% sulfuric acid (10.0 mL, 94.5 mmol, 1 eq) slowly at 0°C. Then the mixture was heated to 80 °C and stirred for 12 h. To the mixture was added sat. aq. Na₂CO₃ to adjust the pH to 8-9. The mixture was extracted with EtOAC (150 mL × 2). The combined organic layers were dried over anhydrous Na₂SO₄ and concentrated in vacuo to give the title compound (700 mg) as a brown oil. MS (ESP): m/z = 226 [M+H]⁺.

### Step 3:

### Methyl 4-chloro-2-(9//-fluoren-9-ylmethoxycarbonylamino)-1,3-dihydroindene-2-carboxylate

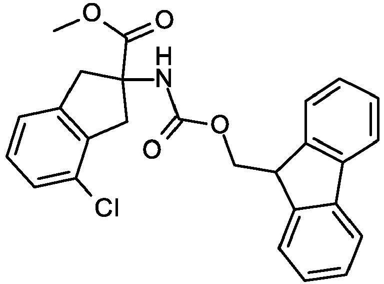

To a solution of methyl 2-amino-4-chloro-1,3-dihydroindene-2-carboxylate (660.0 mg, 2.92 mmol, 1 eq) in methanol (33 mL) were added 9*H*-fluoren-9-ylmethyl 2,5-dioxopyrrolidine-1-carboxylate (1200.0 mg, 3.56 mmol, 1.22 eq) and pyridine (0.94 mL, 11.68 mmol, 3.99 eq) at 25°C. The mixture was stirred for 12 h at 25°C. The reaction mixture was added to H₂O (100 mL) and extracted with EtOAc (100 mL × 3). The combined organic phases were concentrated under reduced pressure to give a crude product. The crude product was purified by silica gel column chromatography (petroleum ether to petroleum ether:EtOAc=20:1) to give the title compound (950 mg) as a light yellow solid. MS (ESP): m/z = 470.0 [M+Na]⁺.

### Step 4:

### 9H-Fluoren-9-ylmethyl N-[4-chloro-2-(hydroxymethyl)-1,3-dihydroinden-2-yl]carbamate

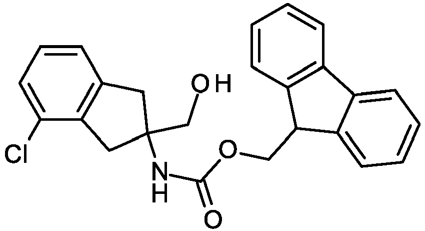

A solution of methyl 4-chloro-2-(9*H*-fluoren-9-ylmethoxycarbonylamino)-1,3-dihydroindene-2-carboxylate (100.0 mg, 0.220 mmol, 1 eq) in THF (5 mL) was cooled to 0°C. To the mixture was added lithium borohydride (0.3 mL, 0.600 mmol, 2.69 eq) and the mixture stirred at 20 °C for 3 h. The mixture was added to water (20 mL) and extracted with ethyl acetate (30 mL × 2). The organic extracts were concentrated in vacuo to give a crude product. The crude product was purified by silica gel column chromatography (PE:EA=20:1 to 5:1) to give the title compound (80 mg, 0.190 mmol, 85.3 % yield) as a light yellow solid. MS (ESP): m/z = 442.0 [M+Na]⁺.

### Step 5:

### 9H-Fluoren-9-ylmethyl N-(4-chloro-2-formyl-1,3-dihydroinden-2-yl)carbamate

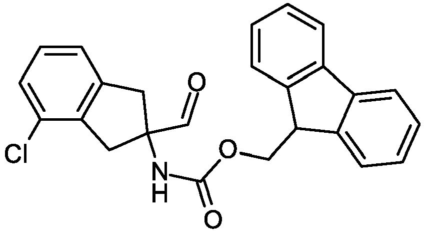

The title compound was prepared by analogy to 1-chloro-6,7-dihydro-5*H*-cyclopenta[c]pyridine-6-carbaldehyde (Intermediate 19, step 4) and was obtained as a light yellow solid.

### Intermediate 174

### tert-Butyl N-[rac-5,6-trans-5,8-cis-8-amino-2-oxo-3-(3-oxo-4H-pyrido[3,2-b][1,4]oxazin-6-yl)-1-oxa-3-azaspiro[4.5]decan-6-yl]carbamate

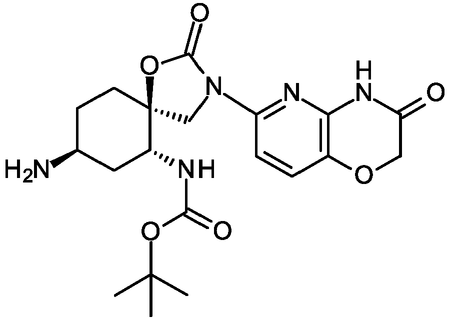

The title compound was prepared from 2-[*rac*-1,2-*trans*-1,4-*cis*-1-hydroxy-2-[(2-methylpropan-2-yl)oxycarbonylamino] -4-(phenylmethoxycarbonylamino)cyclohexyl] acetic acid (Intermediate 112, step 6) by analogy to *tert*-butyl *N*-[*rac*-5,6-*cis*-5,8-*trans*-8-amino-2-oxo-3-(3-oxo-4*H-*pyrido[3,2-b][1,4]oxazin-6-yl)-1-oxa-3-azaspiro[4.5]decan-6-yl]carbamate(Intermediate 112, steps 7 to 10) and was obtained as a white solid. MS (ESP): m/z = 432.4 [M+H]⁺.

### Intermediate 175

### tert-Butyl N-[[5-(2-aminoethyl)-2-oxo-3-(3-oxo-4H-pyrido[3,2-b][1,4]oxazin-6-yl)-1,3-oxazolidin-5-yl] methyl] carbamate

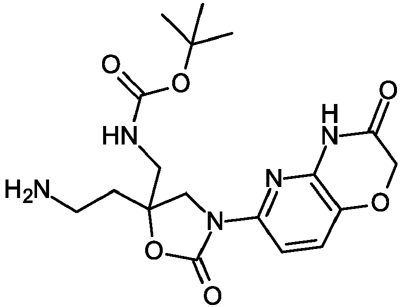

### Step 1:

### 3-(Chloromethyl)but-3-enoxymethylbenzene

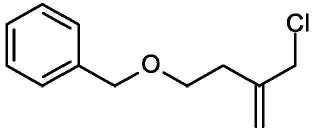

To a solution of 3-methylbut-3-enoxymethylbenzene (CAS# 58558-53-5, 3.0 g, 17.02 mmol, 1 eq) and cerium (III) chloride heptahydrate (12.68 g, 34.04 mmol, 2 eq) in DCM (50 mL) and water (50 mL) was added sodium hypochlorite (35.9 g, 28.94 mmol, 1.7 eq) dropwise. The mixture was stirred at 25 °C for 30 min. The mixture was poured into saturated Na₂SO₃, extracted with DCM and the organic layers were concentrated in vacuo to give the crude title compound (3800 mg, 18.04 mmol, quant. yield) as a colorless oil which was used for the next step directly.

### Step 2:

### 2-(Chloromethyl)-2-(2-phenylmethoxyethyl)oxirane

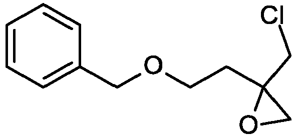

To a solution of 3-(chloromethyl)but-3-enoxymethylbenzene (3.8 g, 18.04 mmol, 1 eq) in DCM (60 mL) was added *m*-chloroperoxybenzoic acid (5835.6 mg, 27.05 mmol, 1.5 eq). The mixture was stirred at 25 °C for 3 h. The mixture was poured into water (100 mL) and extracted with DCM (50 mL × 3). The organic layers were washed with sat. aq. NaHCO₃ (100 mL), brine (100 mL), filtered and concentrated in vacuo. The remaining residue was purified by flash chromatography (10 % ethyl acetate/petroleum ether) to afford the title compound (1500 mg, 6.62 mmol, 36.7 % yield) as a colorless oil.

### Step 3:

### 1-Azido-2-(chloromethyl)-4-phenylmethoxybutan-2-ol

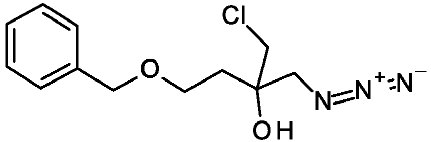

To a solution of 2-(chloromethyl)-2-(2-phenylmethoxyethyl)oxirane (6.0 g, 26.47 mmol, 1 eq) in methanol (100 mL) were added sodium azide (3.44 g, 52.93 mmol, 2 eq) and ammonium chloride (4.25 g, 79.4 mmol, 3 eq). The mixture was stirred at 80 °C for 16 h. The mixture was poured into water (100 mL) and extracted with ethyl acetate (50 mL × 3). The organic layers were washed with brine (50 mL), concentrated in vacuo and the remaining residue was purified by flash chromatography (10 % ethyl acetate/ petroleum ether) to afford the title compound (5.1 g, 18.91 mmol, 71.4 % yield) as a colorless oil.

### Step 4:

### 5-(Chloromethyl)-5-(2-phenylmethoxyethyl)-1,3-oxazolidin-2-one

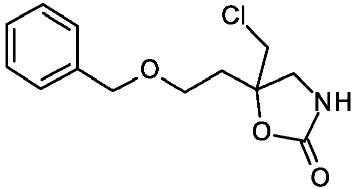

To a solution of 1-azido-2-(chloromethyl)-4-phenylmethoxybutan-2-ol (5.1 g, 18.91 mmol, 1 eq) and triphenylphosphine (5951.08 mg, 22.69 mmol, 1.2 eq) in toluene (200 mL) was added dry ice (8319.4 mg, 189.1 mmol, 10 eq) in several portions. The mixture was heated slowly to 100 °C under CO₂ and stirred for another 16 h. The mixture was concentrated in vacuo and the remaining residue was purified by flash chromatography (50 % ethyl acetate/petroleum ether) to afford the title compound (2.7 g, 10.01 mmol, 52.9 % yield) as a colourless oil and further title compound (1.7 g, 6.3 mmol, 16.7 % yield) as a white solid. ¹H-NMR (400 MHz, CDCl₃) δ 7.31 (m, 5H), 5.13 (br s, 1H), 4.50 (s, 2H), 3.70 (m, 4H), 3.61 (s, 2H), 2.19 (t, 2H).

### Step 5:

### 5-(Azidomethyl)-5-(2-phenylmethoxyethyl)-1,3-oxazolidin-2-one

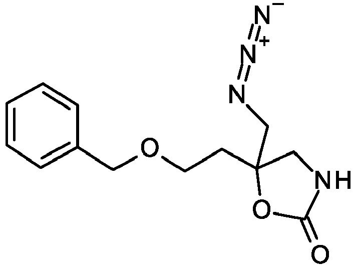

To a solution of 5-(chloromethyl)-5-(2-phenylmethoxyethyl)-1,3-oxazolidin-2-one (2700.0 mg, 10.01 mmol, 1 eq) in DMF (50 mL) were added sodium azide (1952.32 mg, 30.03 mmol, 3 eq) and tetrabutylammonium chloride (2641.6 mg, 10.01 mmol, 1 eq) and the mixture was stirred at 122 °C for 72 h. The mixture was poured into water (400 mL) and extracted with ethyl acetate (100 mL × 3). The organic layers were washed with brine (100 mL), dried over anhydrous Na₂SO₄ and concentrated in vacuo and the remaining residue was purified by flash chromatography (70 % ethyl acetate/petroleum ether) to afford the title compound (2600 mg, 9.41 mmol, 65.8 % yield) as a colorless oil. MS (ESP): m/z = 553.2 [2M+H]⁺.

### Step 6:

### tert-Butyl N-[[2-oxo-5-(2-phenylmethoxyethyl)-1,3-oxazolidin-5-yl]methyl]carbamate

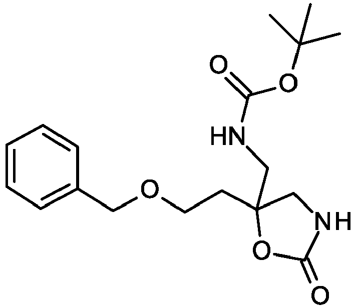

To a solution of 5-(azidomethyl)-5-(2-phenylmethoxyethyl)-1,3-oxazolidin-2-one (2600.0 mg, 6.59 mmol, 1 eq) in THF (30 mL) and water (30 mL) was added triphenylphosphine (3464.9 mg, 13.17 mmol, 2 eq). The mixture was stirred at 25 °C for 16 h. The mixture was acidified with 0.5 M HCl to pH=-3. The mixture was washed with ethyl acetate (30 mL × 2), the water layer was basified by sat. aq. NaHCO₃ to pH=9. Then di-*tert*-butyldicarbonate (2156.5 mg, 9.88 mmol, 1.5 eq) was added and the mixture stirred for 16 h. The mixture was extracted with ethyl acetate (30 mL × 3), the combined extracts were concentrated in vacuo and the remaining residue was purified by flash chromatography (70 % ethyl acetate/ petroleum ether) to afford the title compound (1.2 g, 3.42 mmol, 52 % yield) as a colorless oil. ¹H-NMR (400 MHz, CDCl₃) δ 7.35 (m, 5H), 5.00 (br s, 2H), 4.95 (s, 2H), 3.67 (m, 2H), 3.50 (m, 2H), 3.44 (m, 2H), 2.10 (m, 1H), 2.03 (m, 1H), 1.44 (s, 9H).

### Steps 7 and 8:

### tert-Butyl N-[[2-oxo-3-(3-oxo-4H-pyrido[3,2-b][1,4]oxazin-6-yl)-5-(2-phenylmethoxyethyl)-1,3-oxazolidin-5-yl] methyl] carbamate

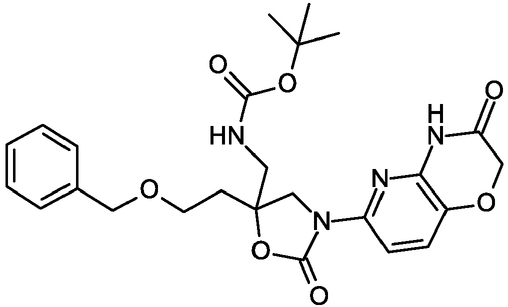

The title compound was prepared by analogy to benzyl *N*-[*rac*-3a,7a-*trans*-6,7a-*cis*-2-oxo-3-(3-oxo-4*H*-pyrido[3,2-b][1,4]oxazin-6-yl)-3a,4,5,6,7,7a-hexahydro-1,3-benzoxazol-6-yl]carbamate (Intermediate 20, steps 4 and 5) and was obtained as a yellow solid. MS (ESP): m/z = 499.3 [M+H]⁺.

### Step 9:

### tert-Butyl N-[[5-(2-hydroxyethyl)-2-oxo-3-(3-oxo-4H-pyrido[3,2-b][1,4]oxazin-6-yl)-1,3-oxazolidin-5-yl] methyl] carbamate

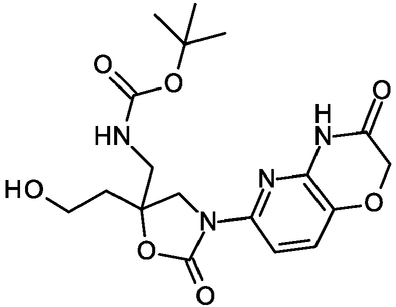

To a solution of *tert*-butyl *N*-[[2-oxo-3-(3-oxo-4*H*-pyrido[3,2-b][1,4]oxazin-6-yl)-5-(2-phenylmethoxyethyl)-1,3-oxazolidin-5-yl]methyl]carbamate (1100.0 mg, 2.21 mmol, 1 eq) in methanol (200 mL) was added acetic acid (0.5 mL) and Pd/C (1000.0 mg). The mixture was stirred at 30 °C under hydrogen (2280 mm Hg) for 40 h. The mixture was filtered and the filtrate was concentrated in vacuo to afford the title compound (700 mg, 1.71 mmol, 77.7% yield) as a white solid. MS (ESP): m/z = 353.3 [M+H-tBu]⁺.

### Steps 10 to 12:

### tert-Butyl N-[[5-(2-aminoethyl)-2-oxo-3-(3-oxo-4H-pyrido[3,2-b][1,4]oxazin-6-yl)-1,3-oxazolidin-5-yl] methyl] carbamate

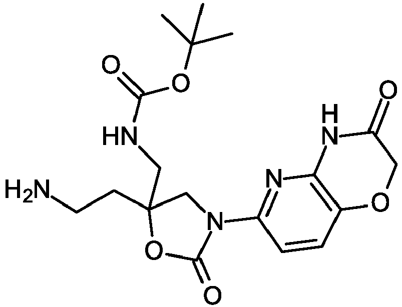

The title compound was prepared by analogy to 5-(2-aminoethyl)-1,3-oxazolidin-2-one (Intermediate 26, steps 2 to 4) and was obtained as a white solid. MS (ESP): m/z = 408.4 [M+H]⁺.

### Intermediate 176

### tert-Butyl N-(rac-1,2-trans-2-amino-4-chloro-2,3-dihydro-1H-inden-1-yl)carbamate

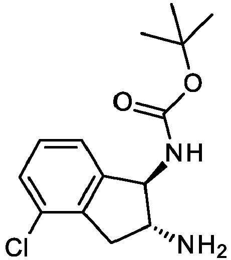

### Step 1:

### rac-1,2-trans-1-Amino-4-chloro-2,3-dihydro-1H-indene-2-carboxylic acid

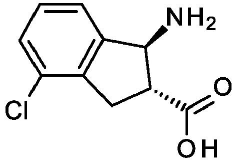

The title compound was prepared from ethyl *rac*-1,2-*trans*-1-amino-4-chloro-2,3-dihydro-1*H-*indene-2-carboxylate (Intermediate 88, step 3) by analogy with 4-fluoro-1-methyl-6,7-dihydro-5*H*-cyclopenta[c]pyridine-6-carboxylic acid (Intermediate 153, step 1).

### Step 2:

### rac-1,2-trans-4-Chloro-1-[(2-methylpropan-2-yl)oxycarbonylamino]-2,3-dihydro-1H-indene-2-carboxylic acid

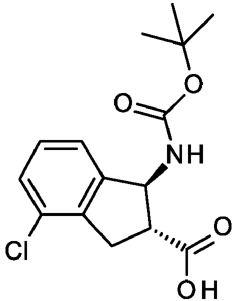

To a solution of *rac*-1,2-*trans*-1-amino-4-chloro-2,3-dihydro-1*H*-indene-2-carboxylic acid (22.0 g, 103.95 mmol, 1 eq) in *tert*-butanol (250 meL) and water (150 mL) were added sodium hydroxide (103.95 mL, 207.9 mmol, 2 eq) and di-*tert*-butyldicarbonate (45374 mg, 207.9 mmol, 2 eq). The mixture was stirred at 25 °C for 16 h. The mixture was acidified with 3N HCl to pH=3. The resulting white precipitate was filtered, washed with water and petroleum ether, then co-evaporated with toluene three times in vacuo to afford the title compound (22.7 g, 72.81 mmol, 67.9 % yield) as a white solid. MS (ESP): m/z = 334.3 [M+Na]⁺.

### Steps 3 and 4:

### tert-Butyl N-(rac-1,2-trans-2-amino-4-chloro-2,3-dihydro-1H-inden-1-yl)carbamate

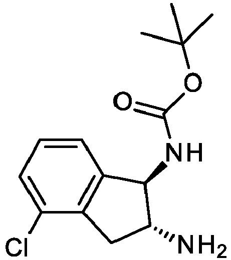

The title compound was prepared by analogy to tert-butyl *N*-[2-[(2-amino-4-chloro-2,3-dihydro-1*H*-inden-5-yl)oxylethyl]carbamate (Intermediate 76, steps 2 and 3) using *rac*-1,2-*trans*-4-chloro-1-[(2-methylpropan-2-yl)oxycarbonylamino]-2,3-dihydro-1*H*-indene-2-carboxylic acid in place of 4-chloro-5-[2-[(2-methylpropan-2-yl)oxycarbonylamino]ethoxy]-2,3-dihydro-1*H-*indene-2-carboxylic acid and was obtained as a yellow solid. MS (ESP): m/z = 283.5 [M+H]⁺.

### Intermediate 177

### 6-[rac-5,6-trans-6-(Azidomethyl)-2-oxo-1-oxa-3,8-diazaspiro[4.5]decan-3-yl]-4H-pyrido[3,2-b][1,4]oxazin-3-one hydrochloride

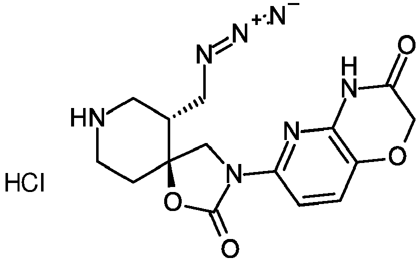

### Step 1:

### tert-Butyl 3-(hydroxymethyl)-4-hydroxypiperidine-1-carboxylate

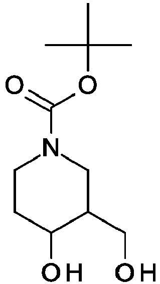

To a mixture of N-BOC-3-carboethoxy-4-piperidone (CAS# 98977-34-5, 30000.0 mg, 110.57 mmol, 1 eq) in ethanol (500 mL) was added NaBH₄ (21025.8 mg, 552.87 mmol, 5 eq) in batches at 0°C, then the reaction was warmed to 25 °C and stirred for 48 h. The mixture was quenched with ice-cold saturated NH₄Cl (1000 mL) and extracted with EtOAc (600 mL × 3). The combined organic layers were washed with brine (500 mL × 2), dried over Na₂SO₄ and concentrated under vacuum to give the title compound (17000 mg, 73.5 mmol, 66.5 % yield) as a white solid. MS (ESP): m/z = 176.1 [M+H-tBu]⁺.

### Step 2:

### tert-Butyl 3-[[tert-butyl(diphenyl)silyl]oxymethyl]-4-hydroxypiperidine-1-carboxylate

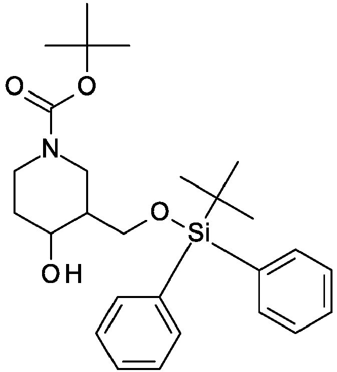

To a mixture of *tert*-butyl 3-(hydroxymethyl)-4-hydroxypiperidine-1-carboxylate (17000.0 mg, 73.5 mmol, 1 eq) and imidazole (10007.5 mg, 147 mmol, 2 eq) in DMF (100 mL) was added *tert*-butylchlorodiphenylsilane (22222.7 mg, 80.85 mmol, 1.1 eq) at 0°C and then the reaction was stirred at 25 °C for 16 h. The mixture was quenched with saturated NaHCO₃ (200 mL) and then extracted with DCM (300 mL). The organic phase was dried over Na₂SO₄ and concentrated under vacuum to give a residue which was purified by flash chromatography (20% EA/PE) to give the title compound (13000 mg, 27.68 mmol, 37.7 % yield) as colorless oil. MS (ESP): m/z = 492.3 [M+Na]⁺.

### Step 3:

### tert-Butyl 3-[[tert-butyl(diphenyl)silyl]oxymethyl]-4-oxo-piperidine-1-carboxylate

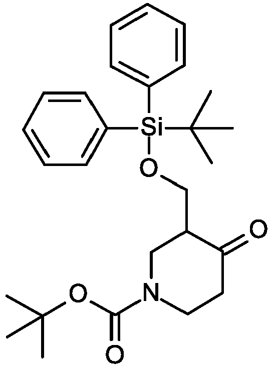

To a mixture of *tert*-butyl 3-[[*tert*-butyl(diphenyl)silyl]oxymethyl]-4-hydroxy-piperidine-1-carboxylate (13000.0 mg, 27.68 mmol, 1 eq) in DCM (200 mL) was added Dess-Martin periodinane (17608.9 mg, 41.52 mmol, 1.5 eq) at 0°C and then the reaction was stirred at 25 °C for 16 h. The mixture was concentrated under vacuum to give a residue. EtOAc (30 mL) was added to the residue and the mixture was filtered. The filtrate was concentrated under vacuum to give the crude product (20 g) which was purified by flash chromatography column to give the title compound (12000 mg, 25.66 mmol, 92.7 % yield) as a colorless oil. MS (ESP): m/z = 490.2 [M+Na]⁺.

### Step 4:

### tert-butyl 3-[[tert-butyl(diphenyl)silyl]oxymethyl]-4-(2-ethoxy-2-oxoethyl)-4-hydroxypiperidine-1 -carboxylate

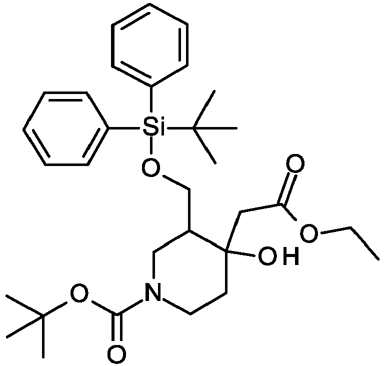

To a mixture of lithium diisopropylamide (38.49 mL, 76.98 mmol, 3 eq) in THF (300 mL) under nitorgen at -78 °C was added dropwise ethyl acetate (3.41 mL, 51.32 mmol, 2 eq) in THF (60 mL). After addition, the mixture was stirred at -78 °C for 30 min. *tert*-butyl 3-[[*tert-*butyl(diphenyl)silyl]oxymethyl]-4-oxopiperidine-1-carboxylate (12000.0 mg, 25.66 mmol, 1 eq) in THF (60 mL) was added solwly and the reaction was stirred at -78 °C for another 1.5 h. The reaction was quenched with saturated NH₄Cl solution (200 mL) then extracted with EtOAc (300 mL × 2) The combined extracts were dried over Na₂SO₄ and concentrated to give a residue which was purified by flash chromatography column on silica gel to give the title compound (12300 mg, 22.13 mmol, 86.3 % yield) as a colorless oil. MS (ESP): m/z = 578.2 [M+Na]⁺.

### Step 5:

### 2-[3-[[tert-Butyl(diphenyl)silyl]oxymethyl]-4-hydroxy-1-[(2-methylpropan-2-yl)oxycarbonyl]piperidin-4-yl]acetic acid

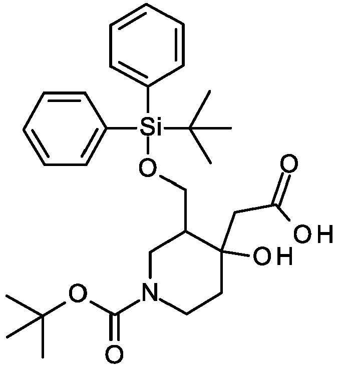

To a mixture of *tert*-butyl 3-[[*tert*-butyl(diphenyl)silyl]oxymethyl]-4-(2-ethoxy-2-oxoethyl)-4-hydroxypiperidine-1-carboxylate (12000.0 mg, 21.59 mmol, 1 eq) in THF (200 mL) was added LiOH (2593 mg, 107.96 mmol, 5 eq) in water (100 mL) at 0°C. The mixture was stirred at 30 °C for 16 h. The reaction mixture was concentrated in vacuo and the aqueous phase was washed with PE (100 mL). Then the aqueous phase was neutralized with HCl (1 N) to pH 6-7 and extracted with EtOAc (150 mL× 2). The extracts were washed with brine (100 mL), dried over Na₂SO₄ and concentrated to give the title compound (8000 mg, 15.16 mmol, 70.2 % yield) as a white solid. MS (ESP): m/z = 550.3 [M+Na]⁺.

### Step 6:

### tert-Butyl 6-[[tert-butyl(diphenyl)silyl]oxymethyl]-2-oxo-1-oxa-3,8-diazaspiro[4.5]decane-8-carboxylate

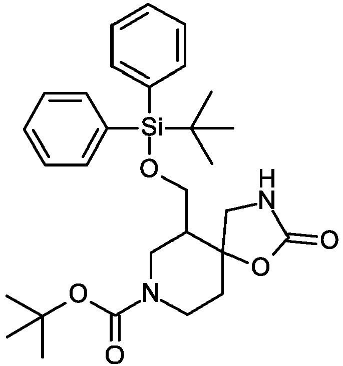

The title compound was prepared by analogy to tert-butyl 2-oxo-1-oxa-3,8-diazaspiro[4.5]decane-8-carboxylate (Intermediate 17, step 3) and was obtained as a white solid. MS (ESP): m/z = 547.4 [M+Na]⁺.

### Steps 7 and 8:

### tert-Butyl 6-[[tert-butyl(diphenyl)silyl]oxymethyl]-2-oxo-3-(3-oxo-4H-pyrido[3,2-b][1,4]oxazin-6-yl)-1-oxa-3,8-diazaspiro[4.5]decane-8-carboxylate

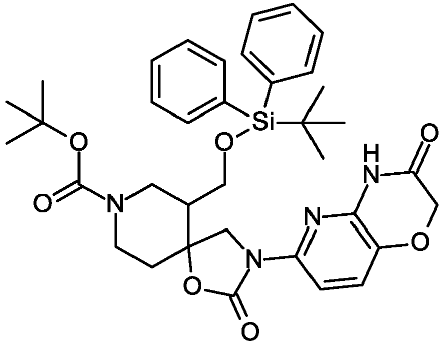

The title compound was prepared by analogy to 6-[5-[2-*[tert*-butyl(dimethyl)silyl]oxyethyl]-2-oxo-1,3-oxazolidin-3-yl]-4*H*-pyrido[3,2-b][1,4]oxazin-3-one (Intermediate 1, steps 4 and 5) and was obtained as a light yellow solid. MS (ESP): m/z = 673.4 [M+H]⁺.

### Step 9:

### tert-Butyl 6-(hydroxymethyl)-2-oxo-3-(3-oxo-4H-pyrido[3,2-b][1,4]oxazin-6-yl)-1-oxa-3,8-diazaspiro[4.5]decane-8-carboxylate

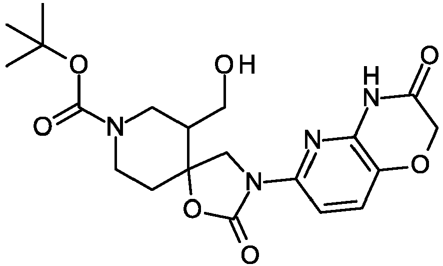

The title compound was prepared by analogy to *tert*-butyl *N*-[2-hydroxy-3-[2-oxo-3-(3-oxo-4*H-*pyrazino[2,3-b][1,4]oxazin-6-yl)-1,3-oxazolidin-5-yl]propyl]carbamate (Intermediate 156, step 6) and was obtained as a white solid. MS (ESP): m/z = 457.2 [M+Na]⁺.

### Steps 10 and 11:

### tert-Butyl rac-5,6-trans-6-(azidomethyl)-2-oxo-3-(3-oxo-4H-pyrido[3,2-b][1,4]oxazin-6-yl)-1-oxa-3,8-diazaspiro[4.5]decane-8-carboxylate and tert-butyl rac-5,6-cis-6-(azidomethyl)-2-oxo-3-(3-oxo-4H-pyrido[3,2-b][1,4]oxazin-6-yl)-1-oxa-3,8-diazaspiro[4.5]decane-8-carboxylate

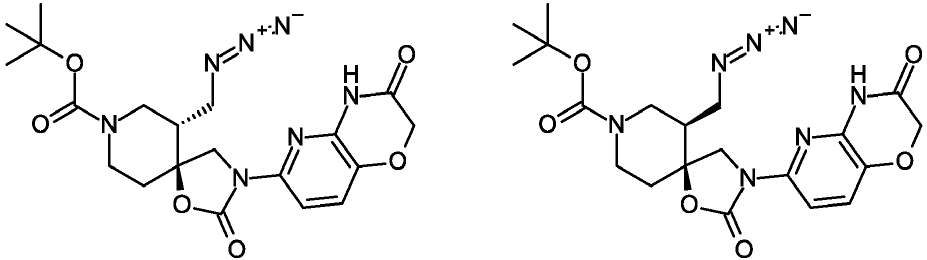

The title compounds were prepared by analogy to 6-[5-(2-azidoethyl)-2-oxo-1,3-oxazolidin-3-yl]-4*H*-pyrido[3,2-b][1,4]oxazin-3-one (Intermediate 1, steps 7 and 8) and were obtained as white solids. The isomers were separated using SFC. MS (ESP): m/z = 460.2 [M+H]⁺ for both products.

### Step 12:

### 6-[rac-5,6-trans-6-(Azidomethyl)-2-oxo-1-oxa-3,8-diazaspiro[4.5]decan-3-yl]-4H-pyrido[3,2-b][1,4]oxazin-3-one hydrochloride

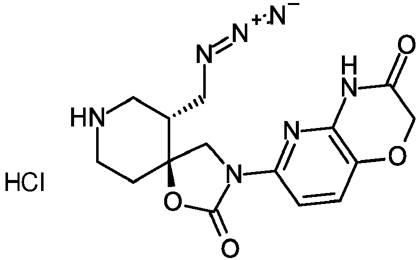

To a mixture of *tert-*butyl 5,6-*trans*-6-(azidomethyl)-2-oxo-3-(3-oxo-4*H*-pyrido[3,2-b][1,4]oxazin-6-yl)-1-oxa-3,8-diazaspiro[4.5]decane-8-carboxylate (150.0 mg, 0.330 mmol, 1 eq) in EtOAc (4 mL) was added HCl/EtOAc (4.0 mL, 0.330 mmol, 1 eq) at 0°C and then the reaction was stirred at 25 °C for 2 h. The mixture was concentrated under vacuum to give the crude title compound (120 mg, 0.300 mmol, 92.9 % yield) as a white solid. The crude product was used for the next step directly. MS (ESP): m/z = 360.2 [M+H]⁺.

### Intermediate 178

### 6-[rac-5,6-cis-6-(Azidomethyl)-2-oxo-1-oxa-3,8-diazaspiro[4.5]decan-3-yl]-4H-pyrido[3,2-b][1,4]oxazin-3-one hydrochloride

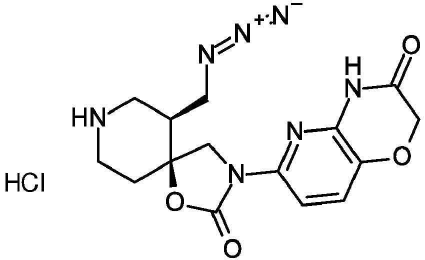

The title compound was prepared from *tert*-butyl *rac*-5,6-*trans*-6-(azidomethyl)-2-oxo-3-(3-oxo-4*H*-pyrido[3,2-b][1,4]oxazin-6-yl)-1-oxa-3,8-diazaspiro[4.5]decane-8-carboxylate (Intermediate 177, step 11) by analogy with 6-[*rac*-5,6-*trans*-6-(azidomethyl)-2-oxo-1-oxa-3,8-diazaspiro[4.5]decan-3-yl]-4*H*-pyrido[3,2-b][1,4]oxazin-3-one hydrochloride (Intermediate 177, step 12) and was obtained as a white solid. MS (ESP): m/z = 360.2 [M+H]⁺.

### Intermediate 179

### tert-Butyl N-[1-[(2-formyl-4,7-dimethyl-2,3-dihydro-1H-inden-5-yl)oxymethyl]cyclopropyl]carbamate

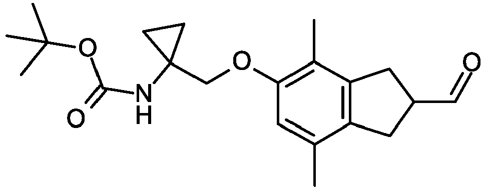

The title compound was prepared by analogy with *tert*-butyl *N*-[2-[(6-formyl-1,4-dimethyl-6,7-dihydro-5*H*-cyclopenta[c]pyridin-3-yl)oxy]ethyl]carbamate (Intermediate 113) using dimethyl 5-hydroxy-4,7-dimethyl-1,3-dihydroindene-2,2-dicarboxylate (Intermediate 73, step 2) in place of dimethyl 1,4-dimethyl-3-oxo-5,7-dihydro-2*H*-cyclopenta[c]pyridine-6,6-dicarboxylate and *tert-*butyl *N*-[1-(hydroxymethyl)cyclopropyl]carbamate in place of *tert*-butyl (2-hydroxyethyl)carbamate was obtained as a colorless waxy solid. MS (ESP): m/z = 304.2 [M+H-tBu]⁺.

### Intermediate 180

### tert-Butyl N-[1-[(7-fluoro-2-formyl-2,3-dihydro-1H-inden-5-yl)oxy]-3-methoxypropan-2-yl] carbamate

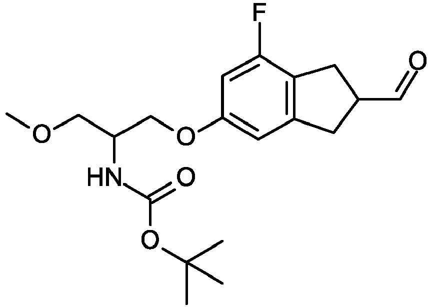

The title compound was prepared by analogy with *tert*-butyl *N*-[1-[(7-fluoro-2-formyl-2,3-dihydro-1*H*-inden-5-yl)oxy]propan-2-yl]carbamate (Intermediate 105) using *tert*-butyl *N*-(1-hydroxy-3-methoxypropan-2-yl)carbamate (CAS# 1334171-66-2) in place of *tert*-butyl *N*-(1-hydroxypropan-2-yl)carbamate and was obtained as a colorless oil.

### Intermediate 181

### N-[2-[(7-Cyano-2-formyl-2,3-dihydro-1H-inden-5-yl)oxy]ethyl]methanesulfonamide

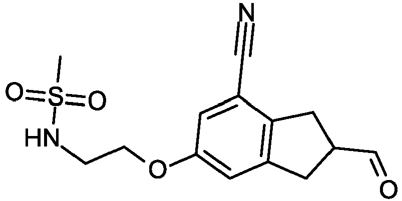

The title compound was prepared by analogy with *tert*-butyl 3-[(2-ethoxycarbonyl-7-fluoro-2,3-dihydro-1*H*-inden-5-yl)oxy]azetidine-1-carboxylate (Intermediate 103) using ethyl 4-cyano-6-hydroxy-2,3-dihydro-1*H*-indene-2-carboxylate (Intermediate 70, step 4) in place of ethyl 4-fluoro-6-hydroxy-indane-2-carboxylate and *N*-(2-bromoethyl)methanesulfonamide (CAS# 63132-74-1) in place of tert-butyl 3-bromoazetidine-1-carboxylate and was obtained as a grey oil. MS (ESP): m/z = 309.1 [M+H]⁺.

### Intermediate 182

### tert-Butyl 3-[(7-cyano-2-formyl-2,3-dihydro-1H-inden-5-yl)oxy]pyrolidine-1-carboxylate

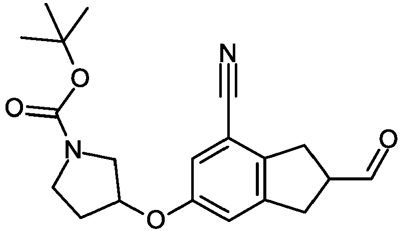

The title compound was prepared by analogy with *N*-[2-[(7-cyano-2-formyl-2,3-dihydro-1*H-*inden-5-yl)oxy]ethyl]methanesulfonamide (Intermediate 182) using tert-butyl 3-bromopyrrolidine-1-carboxylate (CAS# 939793-16-5) in place of *N*-(2-bromoethyl)methanesulfonamide and was obtained as a brown oil. MS (ESP): m/z = 257.1 [M+H-BOC]⁺.

### Intermediate 183

### 6-[5-(2-Aminoethyl)-2-oxo-1,3-oxazol-3-yl]-4H-pyrido[3,2-b][1,4]oxazin-3-one

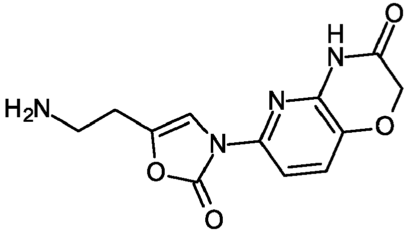

### Step 1:

### tert-Butyl N-[3-oxo-4-(2-trimethylsilylethoxymethyl)pyrido[3,2-b][1,4]oxazin-6-yl]carbamate

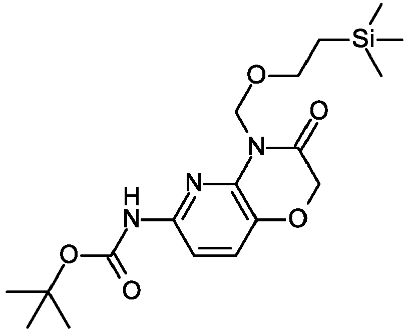

A mixture of 9,9-dimethyl-4,5-bis(diphenylphosphino)xanthene (170.0 mg, 0.290 mmol, 0.110 eq), tris(dibenzylideneacetone)dipalladium (0) (260.0 mg, 0.280 mmol, 0.100 eq), *tert*-butyl carbamate (652.0 mg, 5.57 mmol, 2 eq), 6-bromo-4-(2-trimethylsilylethoxymethyl)pyrido[3,2-b][1,4]oxazin-3-one (Intermediate 8, 0.06 mL, 2.78 mmol, 1 eq) and potassium carbonate (770.0 mg, 5.57 mmol, 2 eq) in 1,4-dioxane (60 mL) was degassed with nitrogen 3 times and the mixture was stirred at 100 °C for 12 h . The mixture was filtered and the filtrate was concentrated in vacuo to give a residue which was purified by flash chromatography (8% EtOAC/PE) to give the title compound (1000 mg, 2.53 mmol, 90.8 % yield) as a light yellow solid.

### Step 2:

### tert-Butyl N-[4-[tert-butyl(dimethyl)silyl]oxybut-1-ynyl]-N-[3-oxo-4-(2-trimethylsilylethoxymethyl)pyrido[3,2-b][1,4]oxazin-6-yl]carbamate

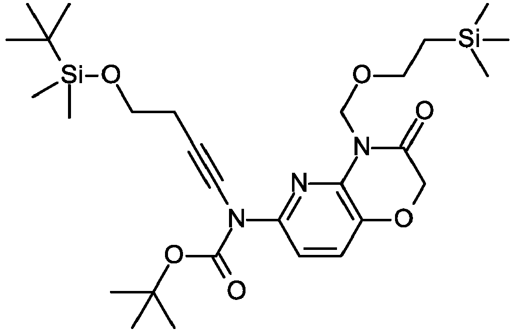

To a solution of *tert*-butyl *N*-[3-oxo-4-(2-trimethylsilylethoxymethyl)pyrido[3,2-b][1,4]oxazin-6-yl]carbamate (900.0 mg, 2.28 mmol, 1 eq) and 4-bromobut-3-ynoxy-*tert*-butyl-dimethyl-silane (CAS# 448944-55-6, 1200.0 mg, 4.56 mmol, 2 eq) in toluene (18 mL) were added 1,10-phenanthroline monohydrate (234.0 mg, 1.18 mmol, 0.520 eq), copper(II)sulfate (180.0 mg, 1.13 mmol, 0.500 eq) and phosphoric acid, potassium salt (972.0 mg, 4.58 mmol, 2.01 eq). The mixture was stirred at 85 °C for 12 h. The mixture was concentrated in vacuo to give a crude product which was purified by preparative HPLC (PE: EtOAC=3:1) to the title compound (600 mg, 1.04 mmol, 45.6 % yield) as colorless oil.

### Step 3:

### 6-[5-[2-[tert-Butyl(dimethyl)silyl]oxyethyl]-2-oxo-1,3-oxazol-3-yl]-4-(2-trimethylsilylethoxymethyl)pyrido[3,2-b][1,4]oxazin-3-one

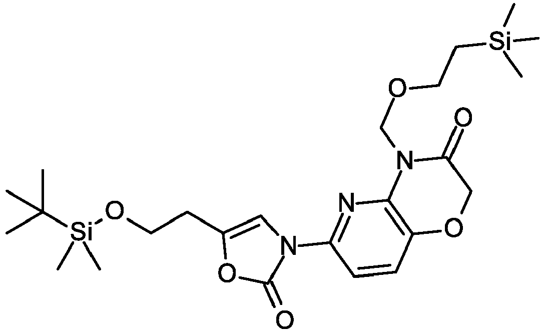

To a solution of tert-butyl *N*-[4-[*tert*-butyl(dimethyl)silyl]oxybut-1-ynyl]-*N*-[3-oxo-4-(2-trimethylsilylethoxymethyl)pyrido[3,2-b][1,4]oxazin-6-yl]carbamate (700.0 mg, 1.21 mmol, 1 eq) in DCM (20 mL) was added a mixture of silver hexafluoroantimonate (V) (120.0 mg, 0.350 mmol, 0.290 eq) and chloro(triphenylphosphine)gold(I) (180 mg, 0.400 mmol, 0.30 eq) in MeCN (2 mL). The mixture was stirred at 40 °C for 3 h. The mixture was concentrated in vacuo to give a crude product which was purified by flash column (10% EtOAC in PE) to give the title compound (600 mg, 1.15 mmol, 94.9 % yield) as a white solid. ¹H-NMR (400 MHz, d6-DMSO) δ 7.82 (d, 1H), 7.71 (d, 1H), 7.59 (s, 1H), 5.57 (s, 2H), 4.91 (s, 2H), 3.90 (t, 2H), 3.71 (t, 2H), 2.77 (m, 2H), 0.96 (m, 2H), 0.94 (s, 9H), 0.12 (s, 3H), 0.00 (s, 3H).

### Step 4:

### 6-[5-(2-Hydroxyethyl)-2-oxo-1,3-oxazol-3-yl]-4H-pyrido[3,2-b][1,4] oxazin-3 -one

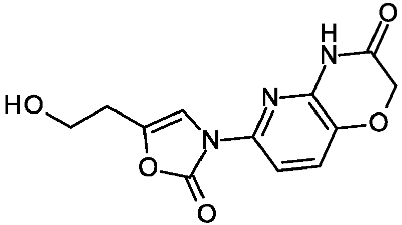

A mixture of 6-[5-[2-[*tert*-butyl(dimethyl)silyl]oxyethyl]-2-oxo-1,3-oxazol-3-yl]-4-(2-trimethylsilylethoxymethyl)pyrido[3,2-b][1,4]oxazin-3-one (600.0 mg, 1.15 mmol, 1 eq) in TFA (10.0 mL, 1.15 mmol, 1 eq) was stirred at 50 °C for 4 h. The mixture was concentrated in vacuo to give a residue. To the residue were added MeOH (5 mL) and K₂CO₃ (200 mg) in H₂O. The mixture was stirred at 20 °C for 1 h. The mixture was concentrated in vacuo to give a crude product. To the crude product was added EtOAC (50 mL) and the mixture was stirred for 0.5 h at 20°C. The mixture was filtered and the filtrate was concentrated in vacuo to give the title compound (300 mg, 1.08 mmol, 67.7 % yield) as a yellow solid. MS (ESP): m/z = 278.0 [M+H]⁺.

### Steps 5 and 6:

### 6-[5-(2-Azidoethyl)-2-oxo-1,3-oxazol-3-yl]-4H-pyrido[3,2-b][1,4] oxazin-3 -one

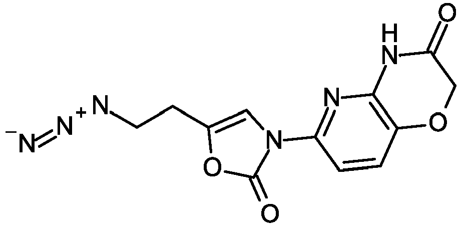

The title compound was prepared by analogy to 6-[5-(2-azidoethyl)-2-oxo-1,3-oxazolidin-3-yl]-4*H*-pyrido[3,2-b][1,4]oxazin-3-one (Intermediate 1, steps 7 and 8) and was obtained as a yellow solid. MS (ESP): m/z = 303.0 [M+H]⁺.

### Step 7:

### 6-[5-(2-Aminoethyl)-2-oxo-1,3-oxazol-3-yl]-4H-pyrido[3,2-b][1,4]oxazin-3-one

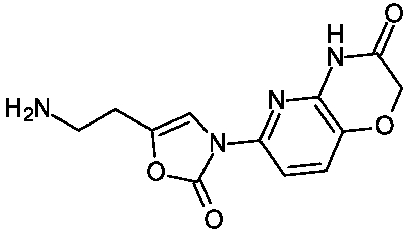

To a mixture of 6-[5-(2-azidoethyl)-2-oxo-1,3-oxazol-3-yl]-4*H*-pyrido[3,2-b][1,4]oxazin-3-one (60.0 mg, 0.200 mmol, 1 eq) in THF (3 mL) and water (0.600 mL) was added triphenylphosphine (156.0 mg, 0.590 mmol, 3 eq). The mixture was stirred at 20 °C for 2 h. The mixture was concentrated in vacuo to give a crude product which was purified by preparative TLC (EtOAc) to give the title compound (40 mg, 0.140 mmol, 72.9 % yield) as yellow solid. MS (ESP): m/z = 277.0 [M+H]⁺.

### Intermediate 184

### tert-Butyl N-[3-[(7-fluoro-2-formyl-2,3-dihydro-1H-inden-5-yl)oxy]-1-(methylamino)-1-oxopropan-2-yl]carbamate

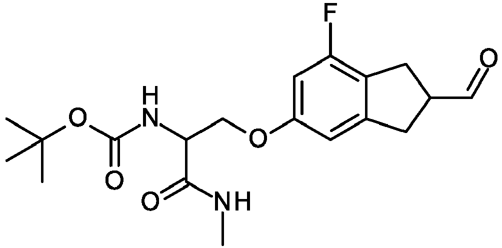

### Step 1:

### 4-O-Benzyl 3-O-tert-butyl 2-oxooxathiazolidine-3,4-dicarboxylate

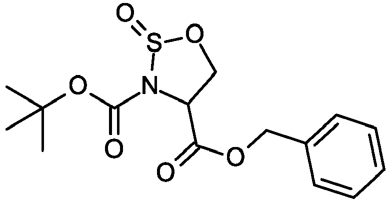

Dry DCM (50 mL) and thionyl chloride (3050.0 mg, 25.64 mmol, 2.52 eq) were placed in a round-bottomed flask under an argon atmosphere. Then benzyl 3-hydroxy-2-[(2-methylpropan-2-yl)oxycarbonylamino]propanoate (CAS# 3850-40-6, 3000.0 mg, 10.16 mmol, 1 eq) in DCM (100 mL) was added dropwise to the reaction mixture at -40 °C and stirring was continued for 30 min. Pyridine (4.19 mL, 51.83 mmol, 5.1 eq) was added to the reaction mixture and the temperature slowly rasised to 20 °C. The reaction mixture was stirred at 20 °C for 1 h. The reaction mxiture was quenched with ice-water (250 mL), washed with sodium bicarbonate (100 mL x2) and brine (100 mL), dried over sodium sulfate and evaporated to get the crude title compound (3000 mg, 8.79 mmol, 86.5 % yield) as a light brown oil. ¹H-NMR (400 MHz, DMSO-d6) δ 7.28 - 7.43 (m, 5H) 4.74 - 5.32 (m, 5H) 1.23 - 1.51 (m, 9H).

### Step 2:

### 4-O-Benzyl 3-O-tert-butyl 2,2-dioxooxathiazolidine-3,4-dicarboxylate

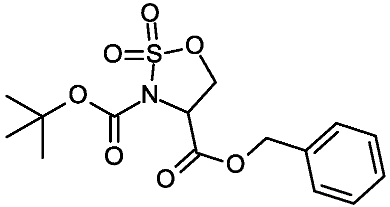

To a solution of 4-O-benzyl 3-*O*-*tert*-butyl 2-oxooxathiazolidine-3,4-dicarboxylate (3000.0 mg, 8.79 mmol, 1 eq) in MeCN (50 mL) were added RuCl₃·H2O (40.0 mg, 0.180 mmol, 0.020 eq), NaIO₄ (3000.0 mg, 14.03 mmol, 1.6 eq) and water (50 mL) at 0 °C and the reaction mixture was stirred at the same temperature for 1 h. The reaction mixture was quenched with sat. aq. NaHCO₃ (200 mL) and extracted with ethyl acetate (3 × 200 mL). The extracts were washed with brine (100 mL), dried over Na₂SO₄, and concentrated. The remaining residue was purified by flash column chromatography (PE/EA= 3:1) to give the title compound (2300 mg, 6.44 mmol, 73.2 % yield) as a light yellow solid. ¹H-NMR (400 MHz, DMSO-*d*6) δ 7.25 - 7.49 (m, 5H), 5.23 - 5.36 (m, 1H), 4.86 - 5.19 (m, 2H), 4.72 - 4.83 (m, 1H), 4.44 - 4.57 (m, 1H), 1.26 - 1.49 (m, 9H).

### Step 3:

### 3-[(2-Methylpropan-2-yl)oxycarbonyl]-2,2-dioxooxathiazolidine-4-carboxylic acid

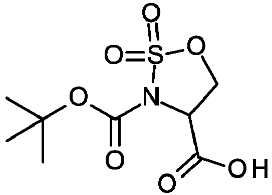

To a mixture of 4-*O*-benzyl 3-*O*-*tert*-butyl 2,2-dioxooxathiazolidine-3,4-dicarboxylate (300.0 mg, 0.840 mmol, 1 eq) in methanol (6 mL) was added Pd/C/ (30.0 mg) and the mixture was stirred under an atmosphere of hydrogen at 20 °C for 0.5 h. The mixture was filtered and the filtrate was concentrated under vacuum to give the title compound (200 mg, 0.750 mmol, 89.2 % yield) as a white solid.

### Step 4:

### 3-[[2-[[tert-Butyl(dimethyl)silyl]oxymethyl]-7-fluoro-2,3-dihydro-1H-inden-5-yl]oxy]-2-[(2-methylpropan-2-yl)oxycarbonylamino]propanoic acid

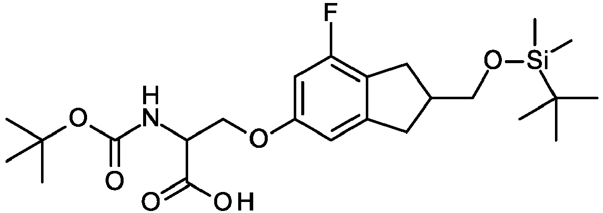

To a suspension of 2-[[*tert*-butyl(dimethyl)silyl]oxymethyl]-7-fluoro-2,3-dihydro-1*H*-inden-5-ol (Intermediate 52, step 2, 180.0 mg, 0.610 mmol, 1 eq) in THF (8 mL) was added NaH (73.02 mg, 1.83 mmol, 3.01 eq) at -15°C. The reaction mixture was stirred for 10 min and 3-[(2-methylpropan-2-yl)oxycarbonyl]-2,2-dioxooxathiazolidine-4-carboxylic acid (194.73 mg, 0.730 mmol, 1.2 eq) in THF (8 mL) was slowly added and the reaction mixture was stirred at -15 °C for 4 h. The mixture was quenched with NH₄Cl (20 mL) and then extracted with EtOAc (20 mL × 2). The extracts were dried over Na₂SO₄ and concentrated under vacuum to give the crude title compound (250 mg, 0.520 mmol, 17.0 % yield) as a colorless oil. MS (ESP): m/z = 428.2 [M+H-tBu]⁺.

### Step 5:

### Methyl 3-[[2-[[tert-butyl(dimethyl)silyl]oxymethyl]-7-fluoro-2,3-dihydro-1H-inden-5-yl]oxy]-2-[(2-methylpropan-2-yl)oxycarbonylamino]propanoate

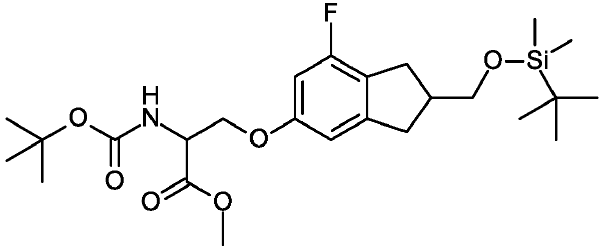

To a suspension of 3-[[2-[[*tert*-butyl(dimethyl)silyl]oxymethyl]-7-fluoro-2,3-dihydro-1*H*-inden-5-yl]oxy]-2-[(2-methylpropan-2-yl)oxycarbonylamino]propanoic acid (250.0 mg, 0.100 mmol, 1 eq) and Na₂CO₃ (0.05 mL, 0.310 mmol, 3 eq) in DMF (3 mL) was added iodomethane (29.35 mg, 0.210 mmol, 2 eq) at 0°C. The reaction mixture was stirred at 20 °C for 2 h. The mixture was quenched with brine (20 mL) and extracted with EtOAc (30 mL). The organic extracts were washed with brine (20 mL × 2), dried over Na₂SO₄ and concentrated under vacuum to give the residue which was purified by prep-TLC (PE/EtOAc=3/1) to give the title compound (150 mg, 0.300 mmol, 61.2 % yield) as a white solid. MS (ESP): m/z = 398.2 [M+H-BOC]⁺.

### Step 6:

### tert-Butyl N-[3-[[2-[[tert-butyl(dimethyl)silyl]oxymethyl]-7-fluoro-2,3-dihydro-1H-inden-5-yl]oxy]-1-(methylamino)-1-oxopropan-2-yl]carbamate

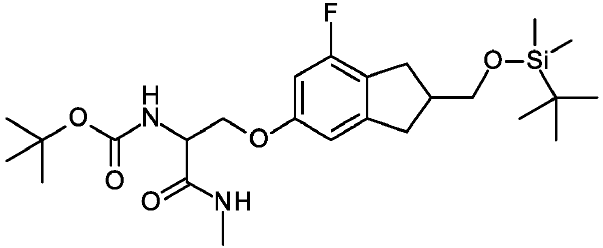

A solution of methyl 3-[[2-[[*tert*-butyl(dimethyl)silyl]oxymethyl]-7-fluoro-2,3-dihydro-1*H-*inden-5-yl]oxy]-2-[(2-methylpropan-2-yl)oxycarbonylamino]propanoate (150.0 mg, 0.060 mmol, 1 eq) in monomethylamine in EtOH (578.16 mg, 6.33 mmol, 100 eq) was stirred at 20 °C for 2 h. The mixture was concentrated under vacuum to give a residue which was purified by prep-TLC (PE/EtOAc=1/1) to give the title compoud (30 mg, 0.060 mmol, 95.4 % yield) as a white solid. MS (ESP): m/z = 397.4 [M+H-BOC]⁺.

### Steps 7 and 8:

### tert-Butyl N-[3-[(7-fluoro-2-formyl-2,3-dihydro-1H-inden-5-yl)oxy]-1-(methylamino)-1-oxopropan-2-yl]carbamate

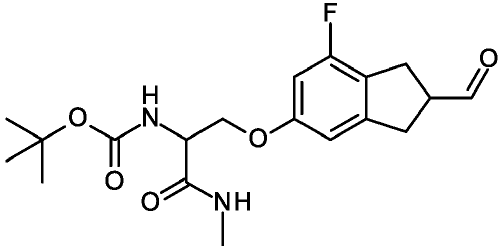

The title compound was prepared by analogy with [*rac*-(5,6-*trans*)-1-chloro-6-formyl-6,7-dihydro-5*H*-cyclopenta[c]pyridin-5-yl] acetate (Intermediate 30, steps 3 and 4) and was obtained as a light brown oil. MS (ESP): m/z = 325.1 [M+H-tBu]⁺.

### Intermediate 185

### (1-Chloro-4-fluoro-3-methyl-6,7-dihydro-5H-cyclopenta[c]pyridin-6-yl)methyl 4-methylbenzenesulfonate

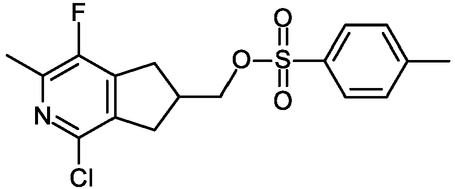

### Step 1:

### Diethyl 2-(6-chloro-3-fluoro-5-methoxycarbonylpyridin-2-yl)propanedioate

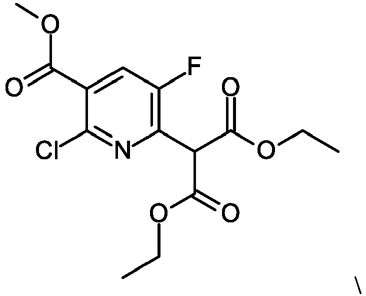

Dimethyl malonate (5.69 g, 5.39 ml, 34.8 mmol, 1.2 eq) was added dropwise to a suspension of sodium hydride (60% dispersion in mineral oil, 1.16 g, 29 mmol, 1 eq) in DMF (25 mL) at 0 °C. The reaction mixure was stirred at RT for 1 h. A solution of diethyl 2-(6-chloro-3-fluoro-5-methoxycarbonylpyridin-2-yl)propanedioate (6.5 g, 29 mmol, 1 eq) in DMF (25 mL) was added at 0 °C. The reaction mixture was stirred at room temperature for 4 h. The mixture was concentrated in vacuo to a volume of 15 mL. The mixture was quenched with sat. aq. NH₄Cl (150 mL). The solution was extracted with EtOAc (4 × 200 mL). The combined organic layers were washed with water (150 mL) and brine (150 mL), dried over Na₂SO₄, filtered and concentrated in vacuo. The crude material was purified by flash chromatography (120 g SiO₂ cartridge, n-heptane/ethyl acetate: 100:0 to 60:40) to give the title compound (5.5 g) as a coloress oil. MS (ESP): m/z = 348.1 [M+H]⁺.

### Step 2:

### 2-Chloro-5-fluoro-6-methylpyridine-3-carboxylic acid

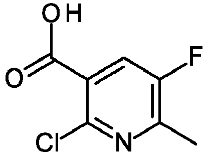

To a solution of diethyl 2-(5-chloro-2-fluoro-4-methoxycarbonylphenyl)propanedioate (7.5 g, 21.6 mmol, 1 eq) in DMA (40 mL) was added magnesium chloride hexahydrate (8.77 g, 43.1 mmol, 2 eq) at room temperature. The reaction mixture was heated to 150 °C for 20 h. The reaction mixture was cooled to room temperature and concentrated in vacuo. The residue was partitioned between EtOAc (250 mL) and cooled 10 % citric acid solution (150 mL). The aqueous layer was extracted with EtOAc (3 × 250 mL). The combined organic layers were washed with brine (150 mL), dried over Na₂SO₄, filtered and concentrated in vacuo. The crude material was purified by flash chromatography (80 g SiO₂ cartridge; ethyl acetate/isopropanol 100: 0 to 90:10) to give the title compound (3.7 g) as an off-white solid. MS (ESP): m/z = 190.1 [M+H]⁺.

### Step 3:

### 2-Chloro-5-fluoro-6-methylpyridine-3,4-dicarboxylic acid

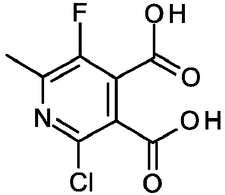

To a solution of N,N-diisopropylamine (3.1 g, 4.3 ml, 30.6 mmol, 2.15 eq) in tetrahydrofuran (30 mL) was added n-butyllithium 1.6 M in hexane (19.6 ml, 31.3 mmol, 2.2 eq) at -78 °C. The reaction mixture was stirred at -78 to -40 °C for 20 min. The reaction mixture was cooled to -78 °C and a solution of 2-chloro-5-fluoro-6-methylpyridine-3-carboxylic acid (2.7 g, 14.2 mmol, 1 eq) in tetrahydrofuran (18 mL) was added dropwise at -78 °C. The reaction mixture was stirred at -78 °C for 30 min. Carbon dioxide (472 mg, 10.7 mmol, 4.5 eq) was slowly added to the reaction mixture at -78°C. After 15 min. the reaction mixture was allowed to warmed to room temperature. At 0°C 1M aqueous hydrogene chloride solution (71.2 ml, 71.2 mmol, 5 eq) was added to adjust to pH 1 and the mixture was warmed to room temperature. To the mixture was added ethyl acetate (120 mL). The aqueous layer was extracted with EtOAc (2 × 200 mL). The combined organic layers were dried over Na₂SO₄, filtered and concentrated in vacuo. The residue was triturated in dichloromethane (20 mL) and hot toluene (20 mL) and dried in vacuo to give the title compound (2.89 g) as a green solid. MS (ESP): m/z = 234.0 [M+H]⁺.

### Step 4:

### [2-Chloro-5-fluoro-3-(hydroxymethyl)-6-methylpyridin-4-yl]methanol

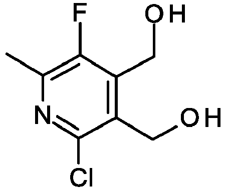

To a solution of 2-chloro-5-fluoro-6-methylpyridine-3,4-dicarboxylic acid (2.8 g, 12 mmol, 1 eq) in tetrahydrofuran (20 mL) was added borane dimethyl sulfide complex 2 M in THF (24 ml, 47.9 mmol, 4 eq) at 0 °C. After 15 min. the ice bath was removed and the reaction mixture was stirred at room temperature for 16 h. Further borane dimethyl sulfide complex 2 M in THF (5.99 ml, 12 mmol, 1 eq) and tetrahydrofuran (20 mL) were added and the reaction mixture was heated to 50 °C for 6 h. The reaction mixture was cooled to 0 °C and methanol (100 mL) was added dropwise. The mixture was preabsorbed onto absorbant and concentrated in vacuo. The crude material was first purified by cartridge chromatography (80 g SiO₂; ethyl acetate/isopropanol 100:0 to 90:10) and the resulting material was further purified by cartridge chromatography (40 g SiO₂; n-heptane/ethyl acetate: 100:0 to 75:25 to 50:50) to give the title compound as a light yellow oil. MS (ESP): m/z = 206.1 [M+H]⁺.

### Step 5:

### 3,4-Bis(bromomethyl)-2-chloro-5-fluoro-6-methylpyridine

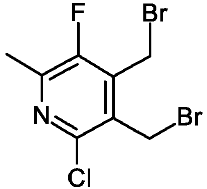

To a solution of [2-chloro-5-fluoro-3-(hydroxymethyl)-6-methylpyridin-4-yl]methanol (698 mg, 3.39 mmol, 1 eq) in dichloromethane (15 mL) was added a solution of phosphorus tribromide (1.1 g, 384 µl, 4.07 mmol, 1.2 eq) in dichloromethane (7 mL) dropwise at 0 °C. The reaction mixture was stirred at 0 °C for 2 h. The reaction mixture was allowed to warm to room temperature and stirring was continued for 2 h. To the reaction mixture were added 50 mL dichloromethane and the mixture was cooled to 0 °C. The mixture was quenched with sat. aq. NaHCO₃ (10 mL) at 0 °C then extracted with dichloromethane (3 × 60 mL). The combined organic layers were dried over anhydrous sodium sulfate, filtered and concentrated in vacuo to give the title compound (670 mg) as a light yellow oil. MS (ESP): m/z = 329.9, 331.9 [M+H]⁺.

### Steps 6 and 7:

### Ethyl 1-chloro-4-fluoro-3-methyl-6,7-dihydro-5H-cyclopenta[c]pyridine-6-carboxylate

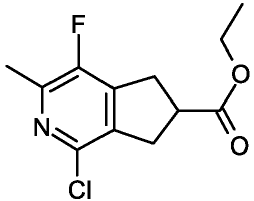

The title compound was prepared by analogy with ethyl 4-bromo-2,3-dihydro-1*H*-indene-2-carboxylate (Intermediate 79, steps 1 and 2) and was obtained as a light yellow liquid. MS (ESP): m/z = 258.1 [M+H]⁺.

### Steps 8 and 9:

### (1-Chloro-4-fluoro-3-methyl-6,7-dihydro-5H-cyclopenta[c]pyridin-6-yl)methyl 4-methylbenzenesulfonate

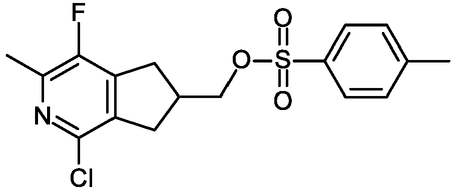

The title compound was prepared by analogy with [1-methyl-3-[2-[(2-methylpropan-2-yl)oxycarbonylamino]ethoxy]-6,7-dihydro-5*H*-cyclopenta[c]pyridin-6-yl]methyl 4-methylbenzenesulfonate (Intermediate 143, steps 3 and 4) and was obtained as a light yellow oil. MS (ESP): m/z = 370.1 [M+H]⁺.

### Intermediate 186

### 6-(2-Oxo-1-oxa-3,8-diazaspiro[4.5]decan-3-yl)-4H-pyrazino[2,3-b][1,4]oxazin-3-one; formic acid

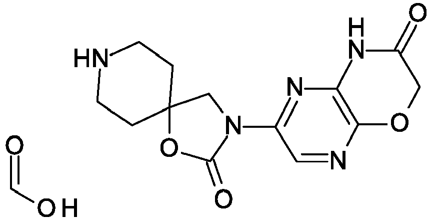

### Step 1:

### tert-Butyl 2-oxo-3-(3-oxo-4H-pyrazino[2,3-b][1,4]oxazin-6-yl)-1-oxa-3,8-diazaspiro[4.5]decane-8-carboxylate

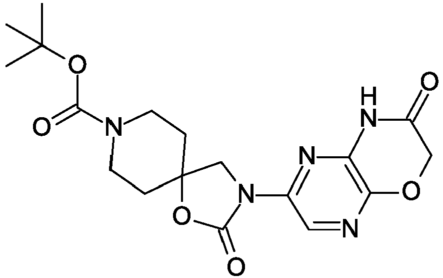

The title compound was prepared by analogy with *tert*-butyl *N*-[2-[2-oxo-3-(3-oxo-4*H-*pyrido[3,2-b][1,4]thiazin-6-yl)oxazolidin-5-yl]ethyl]carbamate (Intermediate 48, step 4) using *tert*-butyl 2-oxo-1-oxa-3,8-diazaspiro[4.5]decane-8-carboxylate (Intermediate 17, step 3) in place of *tert*-butyl *N*-[2-(2-oxooxazolidin-5-yl)ethyl]carbamate and 6-bromo-4*H*-pyrazino[2,3-b][1,4]oxazin-3-one (Intermediate 25, step 1) in place of 6-chloro-4*H*-pyrido[3,2-b][1,4]thiazin-3-one and was obtained as a yellow solid. MS (ESP): m/z = 406.3 [M+H]⁺.

### Step 2:

### 6-(2-Oxo-1-oxa-3,8-diazaspiro[4.5]decan-3-yl)-4H-pyrazino[2,3-b][1,4]oxazin-3-one; formic acid

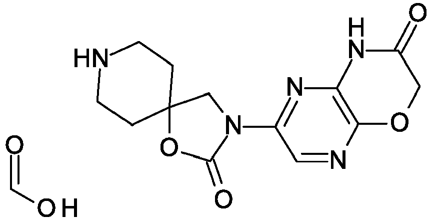

A solution of *tert*-butyl 2-oxo-3-(3-oxo-4*H*-pyrazino[2,3-b][1,4]oxazin-6-yl)-1-oxa-3,8-diazaspiro[4.5]decane-8-carboxylate (1000.0 mg, 2.47 mmol, 1 eq) in formic acid (20.0 mL, 2.47 mmol, 1 eq) was stirred at 35 °C for 5 h. The reaction mixture was concentrated to provide the crude title compound (700 mg, 1.99 mmol, 93 % yield) as a yellow solid. MS (ESP): m/z = 306.2 [M+H]⁺.

### Intermediate 187

### rac-5,6-trans-5-Azido-4-chloro-1-methyl-6,7-dihydro-5H-cyclopenta[c]pyridine-6-carbaldehyde

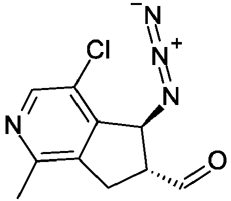

### Steps 1 to 8:

### (1-Bromo-4-chloro-6,7-dihydro-5H-cyclopenta[c]pyridin-6-yl)methanol

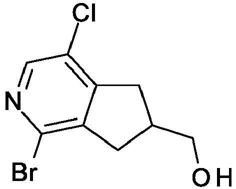

The title compound was prepared by analogy with (4-fluoro-1-methyl-6,7-dihydro-5*H-*cyclopenta[c]pyridin-6-yl)methanol (Intermediate 27, steps 1 to 7 and 9) using 2-bromo-5-chloro-3-methyl-pyridine (CAS# 65550-77-8) in place of 2-bromo-5-fluoro-3-methyl-pyridine and was obtained as a light yellow oil. MS (ESP): m/z = 264.0 [M+H]⁺.

### Steps 9 to 11:

### [1-Bromo-6-[[tert-butyl(diphenyl)silyl]oxymethyl]-4-chloro-6,7-dihydro-5H-cyclopenta[c]pyridin-5-yl] acetate

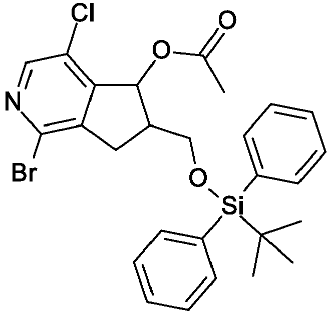

The title compound was prepared by analogy with 6-[[tert-butyl(diphenyl)silyl]oxymethyl]-1-chloro-6,7-dihydro-5*H*-cyclopenta[c]pyridin-5-yl] acetate (Intermediate 30, steps 1 to 3) and was obtained as a colourless oil. MS (ESP): m/z = 558.0, 560.0 [M+H]⁺.

### Step 12:

### [6-[[tert-Butyl(diphenyl)silyl]oxymethyl]-4-chloro-1-methyl-6,7-dihydro-5H-cyclopenta[c]pyridin-5-yl] acetate

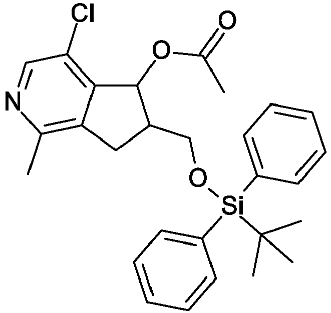

To a solution of [1-bromo-6-[[tert-butyl(diphenyl)silyl]oxymethyl]-4-chloro-6,7-dihydro-5H-cyclopenta[c]pyridin-5-yl] acetate (3000.0 mg, 5.37 mmol, 1 eq) in 1,4-dioxane (100 mL) were added K₂CO₃ (1481.3 mg, 10.73 mmol, 2 eq), trimethylboroxine (2021.2 mg, 8.05 mmol, 1.5 eq) and [1,1'-bis(diphenylphosphino)ferrocene]dichloropalladium(II) (392.7 mg, 0.540 mmol, 0.100 eq). The reaction mixture was stirred for 16 h at 100 °C under N₂. The reaction was filtered and the filtrate was concentrated to dryness. The residue was taken up in EtOAc (100 mL) and the organic phase was washed with saturated NaHCO₃ solution (60 mL × 2). The organics were then separated and dried (Na₂SO₄) and concentrated to dryness. The crude product was then purified by flash chromatography column on silica gel (PE/EtOAc=10/1 to 3/1) to give the title compound (1500 mg, 3.04 mmol, 56.6 % yield) as a colourless oil. MS (ESP): m/z = 494.2 [M+H]⁺.

### Steps 13 and 14:

### tert-Butyl-diphenyl-[[rac-5,6-trans-5-azido-4-chloro-1-methyl-6,7-dihydro-5H-cyclopenta[c]pyridin-6-yl]methoxy]silane

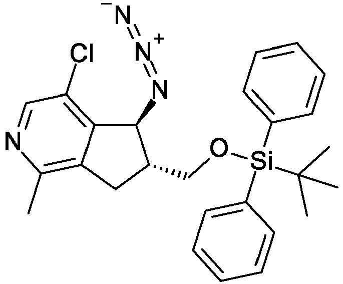

The title compound was prepared by analogy with *tert*-butyl-diphenyl-[(*rac*-5,6-trans-5-azido-1-chloro-6,7-dihydro-5*H*-cyclopenta[c]pyridin-6-yl)methoxy]silane (Intermediate 80, steps 1 and 2) and was obtained as a colourless oil. MS (ESP): m/z = 477.2 [M+H]⁺.

### Steps 15 and 16:

### rac-5,6-trans-5-Azido-4-chloro-1-methyl-6,7-dihydro-5H-cyclopenta[c]pyridine-6-carbaldehyde

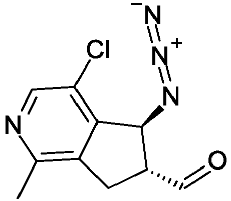

The title compound was prepared by analogy with 2-[(7-fluoro-2-formyl-2,3-dihydro-1*H*-inden-5-yl)oxy]acetonitrile (Intermediate 52, steps 4 and 5) and was obtained as a light brown oil. MS (ESP): m/z = 237.5 [M+H]⁺.

### Intermediate 188

### 2-(4-Fluoro-1-methyl-6,7-dihydro-5H-cyclopenta[c]pyridin-6-yl)acetaldehyde

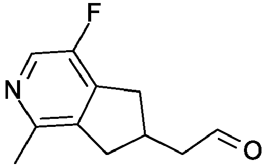

The title compound was prepared by analogy to 2-(4-chloro-2,3-dihydro-1*H*-inden-2-yl)acetaldehyde (Intermediate 11) using (4-fluoro-1-methyl-6,7-dihydro-5*H-*cyclopenta[c]pyridin-6-yl)methanol (Intermediate 27, step 9) in place of (4-chloro-2,3-dihydro-1*H*-inden-2-yl)methanol and was obtained as an off-white semi-solid.

### Intermediate 189

### 6-[5-(Aminomethyl)-2-oxo-1,3-oxazolidin-3-yl]-4H-pyrazino[2,3-b][1,4]oxazin-3-one; 2,2,2-trifluoroacetic acid

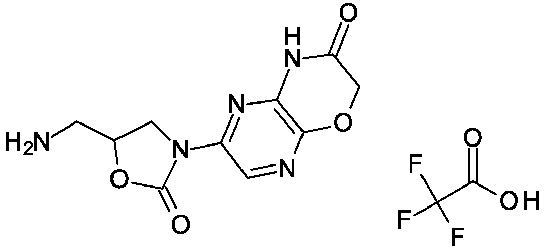

### Step 1:

### tert-Butyl N-[2-hydroxy-3-[(2-methylpropan-2-yl)oxycarbonylamino]propyl]carbamate

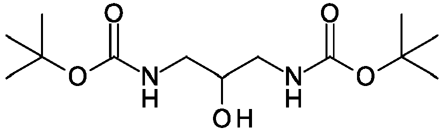

To a solution of 1,3-diamino-2-propanol (5.0 g, 55.48 mmol, 1 eq) in methanol (100 mL) were added di-*tert*-butyldicarbonate (24.22 g, 110.96 mmol, 2 eq) and triethylamine (50.0 mL, 358.73 mmol, 6.47 eq). The reaction mixture was stirred at 50 °C for 0.5 h and then at 25 °C for 1 h. The reaction mixture was concentrated to give a residue which was purified by flash column chromatography (PE:EA=1:1) to give the title compound (15 g, 51.66 mmol, 93.1 % yield) as a white solid.

### Step 2:

### tert-Butyl N-[(2-oxo-1,3-oxazolidin-5-yl)methyl]carbamate

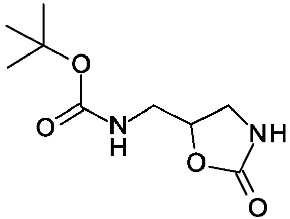

To a solution of *tert*-butyl N-[2-hydroxy-3-[(2-methylpropan-2-yl)oxycarbonylamino]propyl]carbamate (1.0 g, 3.44 mmol, 1 eq) in THF (10 mL) was added NaH (413.3 mg, 10.33 mmol, 3 eq) at 0 °C. The reaction mixture was stirred at 25 °C for 12 h. The reaction mixture was quenched with saturated NH₄Cl (100mL) at 0 °C and extracted with EtOAc (100 mL × 3). The combined organic phases were washed with brine, dried over anhydrous Na₂SO₄, filtered and concentracted to give the title compound (500 mg, 2.31 mmol, 67.1 % yield) as a light yellow solid.

### Steps 3 and 4:

### 6-[5-(Aminomethyl)-2-oxo-1,3-oxazolidin-3-yl]-4H-pyrazino[2,3-b] [1,4]oxazin-3-one;2,2,2-trifluoroacetic acid

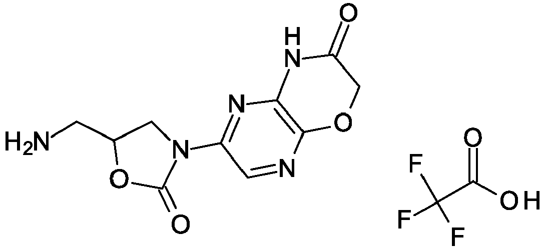

The title compound was prepared by analogy with 6-[5-(*trans*-3-aminocyclobutyl)-2-oxo-1,3-oxazolidin-3-yl]-4*H*-pyrido[3,2-b][1,4]oxazin-3-one; 2,2,2-trifluoroacetic acid (Intermediate 7, steps 4 and 5) using *tert*-butyl *N*-[(2-oxo-1,3-oxazolidin-5-yl)methyl]carbamate in place of *tert-*butyl *N*-[*trans*-3-(2-oxo-1,3-oxazolidin-5-yl)cyclobutyl]carbamate and 6-bromo-4*H-*pyrazino[2,3-b][1,4]oxazin-3-one (Intermediate 25, step 1) in place of 6-bromo-4-(2-trimethylsilylethoxymethyl)pyrido[3,2-b][1,4]oxazin-3-one and was obtained as a white solid. MS (ESP): m/z = 266.1 [M+H]⁺.

### Intermediate 190

### 3-[[7-Fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-2-[(2-methylpropan-2-yl)oxycarbonylamino]propanoic acid

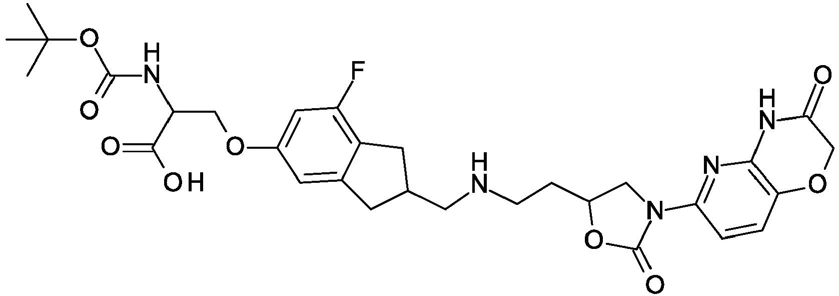

### Step 1:

### Methyl 3-[(7-fluoro-2-formyl-2,3-dihydro-1H-inden-5-yl)oxy]-2-[(2-methylpropan-2-yl)oxycarbonylamino]propanoate

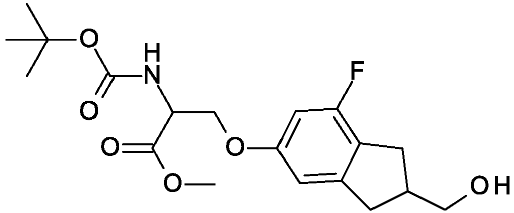

To a mixture of methyl 3-[[2-[[*tert*-butyl(dimethyl)silyl]oxymethyl]-7-fluoro-2,3-dihydro-1H-inden-5-yl]oxy]-2-[(2-methylpropan-2-yl)oxycarbonylamino]propanoate (Intermediate 184, step 5, 150.0 mg, 0.300 mmol, 1 eq) in THF (3 mL) were added TBAF (0.9 mL, 0.900 mmol, 2.99 eq) and acetic acid (36.0 mg, 0.600 mmol, 1.99 eq). The mixture was stirred at 30 °C for 16 h. The mixture was concentrated in vacuo to give a crude product which was purified by prep-TLC (PE: EtOAC=2:1) to give the title compound (100 mg, 0.260 mmol, 82.2 % yield) as a colourless oil. MS (ESP): m/z = 284.1 [M+H-BOC]⁺.

### Step 2:

### Methyl 3-[(7-fluoro-2-formyl-2,3-dihydro-1H-inden-5-yl)oxy]-2-[(2-methylpropan-2-yl)oxycarbonylamino]propanoate

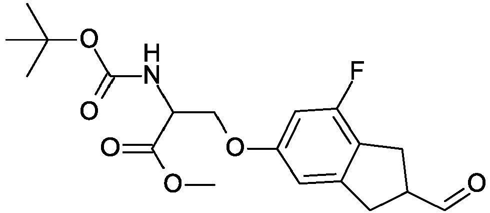

To a mixture of methyl 3-[(7-fluoro-2-formyl-2,3-dihydro-1*H*-inden-5-yl)oxy]-2-[(2-methylpropan-2-yl)oxycarbonylamino]propanoate (50.0 mg, 0.130 mmol, 1 eq) in DCM (1.5 mL) and THF (1.5 mL) was added Dess-Martin periodinane (72.5 mg, 0.170 mmol, 1.31 eq) at 0 °C. The mixture was stirred at 20 °C for 3 h. The mixture was concentrated in vacuo to give the title compound (40 mg, 0.100 mmol, 80.4 % yield) as a light yellow oil.

### Step 3:

### Methyl 3-[[7-fluoro-2-[[2-[2-oxo-3-(3-oxo-4H-pyrido[3,2-b][1,4]oxazin-6-yl)-1,3-oxazolidin-5-yl] ethylamino] methyl]-2,3-dihydro-1H-inden-5-yl] oxy]-2-[(2-methylpropan-2-yl)oxycarbonylamino]propanoate

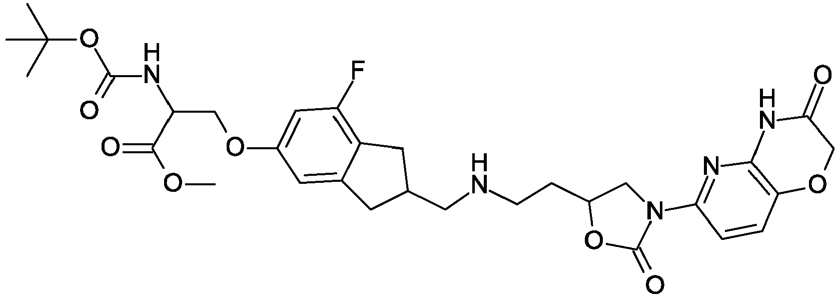

A mixture of methyl 3-[(7-fluoro-2-formyl-2,3-dihydro-1*H*-inden-5-yl)oxy]-2-[(2-methylpropan-2-yl)oxycarbonylamino]propanoate (60.0 mg, 0.160 mmol, 1 eq) and 6-[5-(2-aminoethyl)-2-oxo-1,3-oxazolidin-3-yl]-4*H*-pyrido[3,2-b][1,4]oxazin-3-one (Intermediate 1, 45.0 mg, 0.160 mmol, 1.03 eq) in methanol (2 mL) was stirred at 30 °C for 2 h. Then sodium cyanoborohydride (40.0 mg, 0.190 mmol, 1.2 eq) was added and the reaction mixture was stirred at 30 °C for 1 h. The reaction mixture was concentrated in vacuo to give a residue which was purified by prep-TLC (DCM:MeOH =10: 1) to give the title compound (25 mg, 0.040 mmol, 23 % yield) as a colourless solid. MS (ESP): m/z = 644.1 [M+H]⁺.

### Step 4:

### 3-[[7-Fluoro-2-[[2-[2-oxo-3-(3-oxo-4H-pyrido[3,2-b] [1,4]oxazin-6-yl)-1,3-oxazolidin-5-yl]ethylamino]methyl]-2,3-dihydro-1H-inden-5-yl]oxy]-2-[(2-methylpropan-2-yl)oxycarbonylamino]propanoic acid

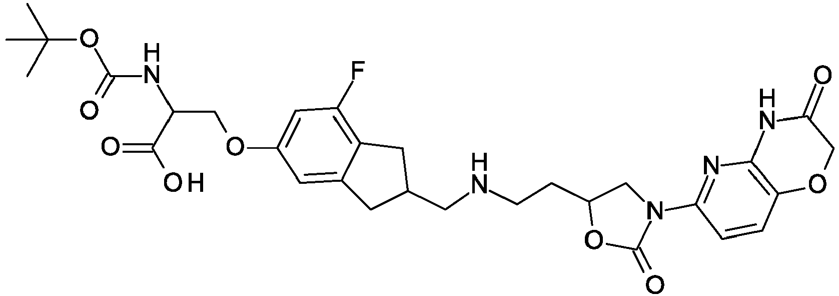

To a mixture of methyl 3-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-2-[(2-methylpropan-2-yl)oxycarbonylamino]propanoate (25.0 mg, 0.040 mmol, 1 eq) in THF (1 mL) and water (1 mL) was added LiOH.H₂O (7.96 mg, 0.190 mmol, 5 eq) at 0°C and the reaction mixture was stirred at 0 °C for 5 h. The mixture was neutralized with HCl (1 N) to pH 6-7 and then concentrated under vacuum to give the title compound (20 mg, 0.030 mmol, 81.8 % yield) as a light brown oil. The crude product was used for next step directly.

### Intermediate 191

### (1,4-Dimethyl-6,7-dihydro-5H-cyclopenta[c]pyridin-6-yl)methyl 4-methylbenzenesulfonate

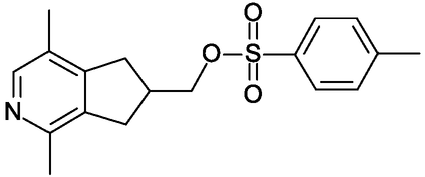

To a solution of (1,4-dimethyl-6,7-dihydro-5*H*-cyclopenta[c]pyridin-6-yl)methanol (Intermediate 62, step 8, 73 mg, 412 µmol, 1 eq) in DCM (6 mL) were added *p*-toluenesulfonylchloride (314 mg, 1.65 mmol, 4 eq) and *N*,*N*-diisopropylethylamine (213 mg, 282 µL, 1.65 mmol, 4 eq) and DMAP (201 mg, 1.65 mmol, 4 eq). The reaction mixture was stirred at RT for 3 h. The reaction mixture was treated with H₂O (5 mL) and extracted with DCM (2 × 15 mL). The organic layers were dried (Na₂SO₄) and evaporated. The crude material was purified by flash chromatography (silica gel, 10 g; 0% to 100% EtOAc in heptane) to give the title compound (107 mg, 76.8 % yield) as a light yellow solid. MS (ESP): m/z = 332.2 [M+H]⁺.

### Intermediate 192

### 6-[2-(Dimethylamino)ethoxy]-4-fluoro-2,3-dihydro-1H-indene-2-carbaldehyde\

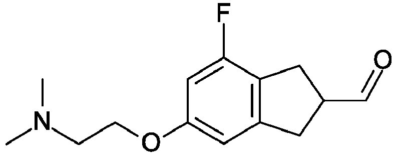

### Step 1:

### 2-[[2-[[tert-Butyl(dimethyl)silyl]oxymethyl]-7-fluoro-2,3-dihydro-1H-inden-5-yl]oxy]-N,N-dimethylethanamine

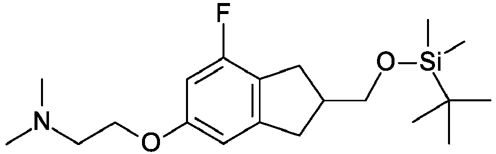

The title compound was prepared by analogy with 2-[[2-[[*tert*-butyl(dimethyl)silyl]oxymethyl]-7-fluoro-2,3-dihydro-1*H*-inden-5-yl]oxy]acetonitrile (Intermediate 52, step 3) using 2-chloro-*N,N*-dimethylethanamine hydrochloride (CAS# 4584-46-7) in place of bromoacetonitrile and was obtained as a brown oil.

### Step 2:

### [6-[2-(Dimethylamino)ethoxy]-4-fluoro-2,3-dihydro-1H-inden-2-yl]methanol

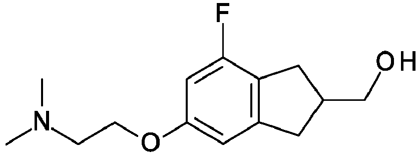

To a solution of 2-[[2-[[*tert*-butyl(dimethyl)silyl]oxymethyl]-7-fluoro-2,3-dihydro-1*H*-inden-5-yl]oxy]-*N,N*-dimethylethanamine (350.0 mg, 0.95 mmol, 1 eq) in methanol (7 mL) was added HCl in MeOH (7.0 mL). The mixture was stirred at 20 °C for 1 h. The mixture was concentrated in vacuo to give a residue. To the residue was added MeOH (30 mL) and basic resin (200 mg). The mixture was stirred at 20 °C for 1 h. The mixture was filtered and the filtrate was concentrated in vacuo to give the crude title compound (250 mg, quant. yield) as a light yellow solid. MS (ESP): m/z = 254.2 [M+H]⁺.

### Step 3:

### 6-[2-(Dimethylamino)ethoxy]-4-fluoro-2,3-dihydro-1H-indene-2-carbaldehyde

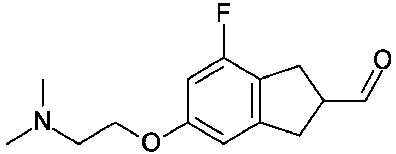

To a mixture of [6-[2-(dimethylamino)ethoxy]-4-fluoro-2,3-dihydro-1*H*-inden-2-yl]methanol (30.0 mg, 0.120 mmol, 1 eq) in DCM (3 mL) were added pyridine (1.0 mL, 12.36 mmol) and then Dess-Martin periodinane (65.3 mg, 0.150 mmol, 1.3 eq) at 25 °C. The mixture was stirred at 30 °C for 3 h. The mixture was filtered and the filtrate was concentrated under vacuum to give the crude title compound (35 mg) as a light brown oil. MS (ESP): m/z = 252.2 [M+H]⁺.

### Intermediate 193

### [rac-1,2-trans-4-Chloro-1-[(2-methylpropan-2-yl)oxycarbonylamino]-6-[[1-[(2-methylpropan-2-yl)oxycarbonylamino]cyclopropyl]methoxy]-2,3-dihydro-1H-inden-2-yl]methyl 4-methylbenzenesulfonate

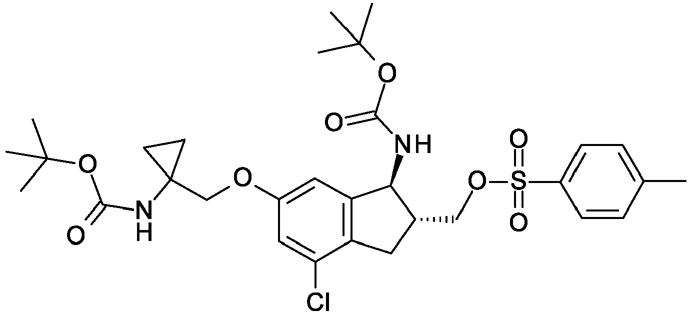

### Step 1:

### tert-Butyl N-[rac-1,2-trans-2-[[tert-butyl(dimethyl)silyl]oxymethyl]-4-chloro-2,3-dihydro-1H-inden-1-yl]carbamate

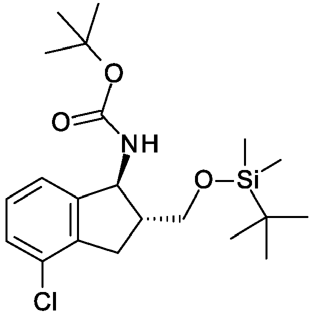

*tert*-Butyldimethylchlorosilane (601 mg, 3.99 mmol, 1.2 eq) was added to an ice-cold solution of *tert*-butyl *N*-[*rac*-1,2-trans-4-chloro-2-(hydroxymethyl)-2,3-dihydro-1H-inden-1-yl]carbamate (Intermediate 88, step 5, 990 mg, 3.32 mmol, 1 eq) and imidazole (1.13 g, 16.6 mmol, 5 eq) in DMF (12 mL). The reaction mixture was stirred at RT overnight. Water was added and the mixture was extracted with EtOAc (3 x). The combined organic layers were washed with water and brine, dried over Na₂SO₄ and evaporated. The residue was adsorbed on adsorbent and purified by flash chromatography (70 g silica gel; heptane/EtOAc 98:2 to 95:5) to give an impure product which was purified further by prep-HPLC to give the title compound (180 mg, 13 % yield) as a yellow gum. MS (ESP): m/z = 356.2 [M+H-tBu]⁺.

### Step 2:

### tert-Butyl N-[rac-1,2-trans-2-[[tert-butyl(dimethyl)silyl]oxymethyl]-4-chloro-6-hydroxy-2,3-dihydro-1H-inden-1-yl]carbamate

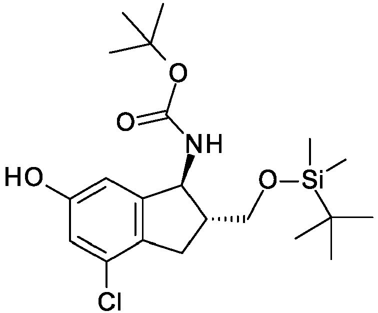

Ar was bubbled under sonication through a mixture of *tert*-butyl *N*-[*rac*-1,2-*trans*-2-[[*tert-*butyl(dimethyl)silyl]oxymethyl]-4-chloro-2,3-dihydro-1*H*-inden-1-yl]carbamate (150 mg, 0.364 mmol, 1 eq), 4,4,5,5-tetramethyl-1,3,2-dioxaborolane (277.2 mg, 1.092 mmol, 3 eq), (methoxy(cyclooctadiene)iridium(I) dimer (36.3 mg, 0.056 mmol, 0.15 eq) and 3,4,7,8-tetramethyl-1,10-phenanthroline (25.8 mg, 0.109 mmol, 0.3 eq) in THF (1.5 mL) at RT. The reaction mixture was stirred at 80 °C for 3 days. The reaction mixture was cooled to RT, diluted with THF (2.5 mL) and water (2.5 mL). Sodium perborate monohydrate (327 mg, 3.28 mmol, 9 eq) was added and the suspension was stirred well for 15 min at RT. The mixture was poured onto a stirred mixture of NH₄Cl and EtOAc. The pH of the aqueous layer was adjusted to 5 with 0.1 N HCl. The layers were separated. The aqueous layer was extracted with EtOAc (3 x). The organic layers were washed with water (pH 5) and brine (pH 5), dried over Na₂SO₄ and evaporated. The crude product was adsorbed on adsorbent and purified by flash chromatography (20 g silica gel; heptane/EtOAc 95:5 to 80:20) to give the title compound (69 mg, 0.161 mmol, 44 % yield) as a white solid. MS (ESN): m/z = 426.4 [M-H]⁻.

### Step 3:

### tert-Butyl N-[rac-1,2-trans-2-[[tert-butyl(dimethyl)silyl]oxymethyl]-4-chloro-6-[[1-[(2-methylpropan-2-yl)oxycarbonylamino] cyclopropyl] methoxy] -2,3-dihydro-1H-inden-1-yl] carbamate

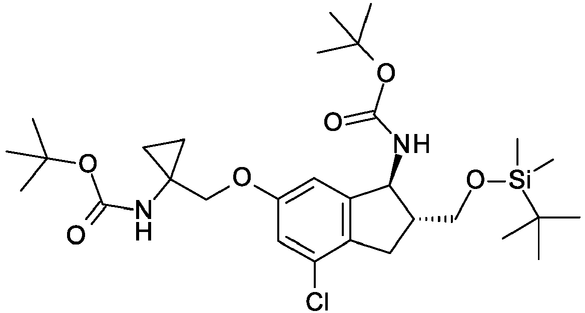

The title compound was prepared by analogy with dimethyl 1,4-dimethyl-3-[(5-oxomorpholin-2-yl)methoxy]-5,7-dihydrocyclopenta[c]pyridine-6,6-dicarboxylate (Intermediate 64, step 3) using *tert*-butyl *N*-[*rac*-1,2-*trans*-2-[[*tert*-butyl(dimethyl)silyl]oxymethyl]-4-chloro-6-hydroxy-2,3-dihydro-1*H*-inden-1-yl]carbamate in place of dimethyl 1,4-dimethyl-3-oxo-5,7-dihydro-2*H-*cyclopenta[c]pyridine-6,6-dicarboxylate and *tert*-butyl *N*-(1-methylcyclopropyl)carbamate in place of 6-(hydroxymethyl)morpholin-3-one and was obtained as a white solid. MS (ESP): m/z = 441.2 [M+2H-BOC-tBu]⁺.

### Step 4:

### tert-Butyl N-[rac-1,2-trans-4-chloro-2-(hydroxymethyl)-6-[[1-[(2-methylpropan-2-yl)oxycarbonylamino]cyclopropyl]methoxy]-2,3-dihydro-1H-inden-1-yl]carbamate

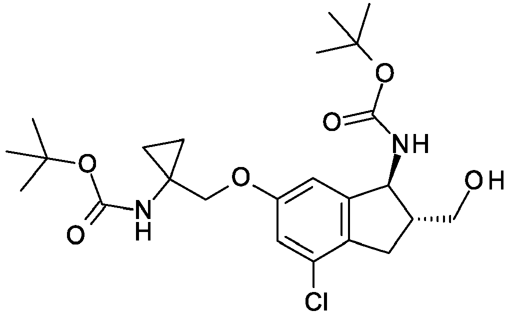

The title compound was prepared by analogy with [*rac*-(5,6-*trans*)-1-chloro-6-(hydroxymethyl)-6,7-dihydro-5*H*-cyclopenta[c]pyridin-5-yl] acetate (Intermediate 30, step 4) and was obtained as a white solid. MS (ESN): m/z = 527.3 [M-H+HCOOH]⁻.

### Step 5:

### [rac-1,2-trans-4-Chloro-1-[(2-methylpropan-2-yl)oxycarbonylamino]-6-[[1-[(2-methylpropan-2-yl)oxycarbonylamino]cyclopropyl]methoxy]-2,3-dihydro-1H-inden-2-yl]methyl 4-methylbenzenesulfonate

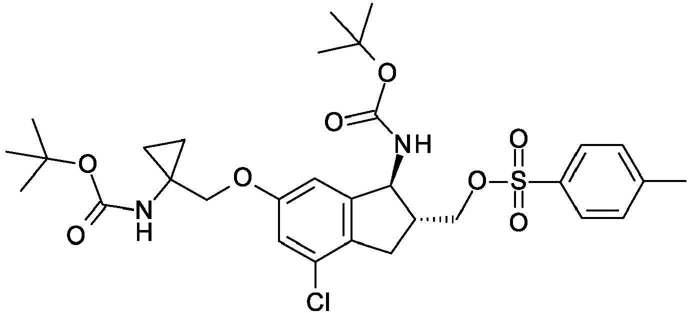

The title compound was prepared by analogy with [(1,2-*trans*)-4-chloro-1-[(2-methylpropan-2-yl)oxycarbonylamino]-2,3-dihydro-1*H*-inden-2-yl]methyl 4-methylbenzenesulfonate, Enantiomer A (Intermediate 141, step 2) using *tert*-butyl *N*-[*rac*-1,2-*trans*-4-*chloro*-2-(hydroxymethyl)-6-[[1-[(2-methylpropan-2-yl)oxycarbonylamino]cyclopropyl]methoxy]-2,3-dihydro-1*H*-inden-1-yl]carbamate in place of *tert*-butyl *N*-[1,2-*trans*-4-chloro-2-(hydroxymethyl)-2,3-dihydro-1*H*-inden-1-yl]carbamate, Enantiomer A and was obtained as a white solid. MS (ESN): m/z = 681.4 [M-H+HCOOH]⁻.

### Intermediate 194

### Ethyl 1-ethyl-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylate

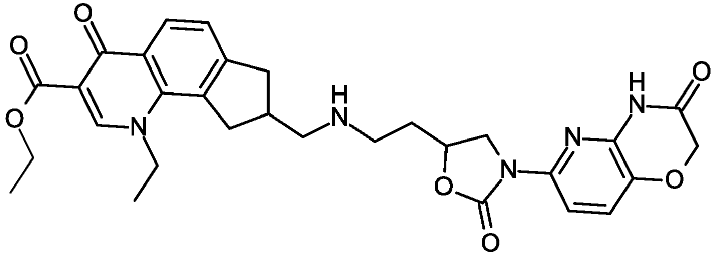

### Steps 1 to 3:

### Ethyl 1-ethyl-8-(methylsulfonyloxymethyl)-4-oxo-8,9-dihydro-7H-cyclopenta[h]quinoline-3-carboxylate

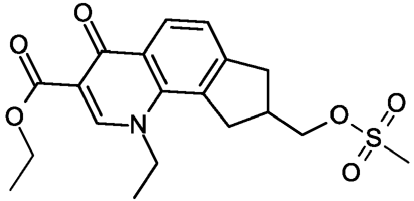

The title compound was prepared by analogy with ethyl 1-ethyl-6-fluoro-8-(hydroxymethyl)-4-oxo-8,9-dihydro-7*H*-cyclopenta[h]quinoline-3-carboxylate (Intermediate 172, steps 6 to 8) using (4-amino-2,3-dihydro-1*H*-inden-2-yl)methanol (CAS# 1780260-59-4) in place of (4-amino-7-fluoro-2,3-dihydro-1*H*-inden-2-yl)methanol and was obtained as a light yellow semi-solid.

### Step 4:

### Ethyl 1-ethyl-8-(hydroxymethyl)-4-oxo-8,9-dihydro-7H-cyclopenta[h]quinoline-3-carboxylate

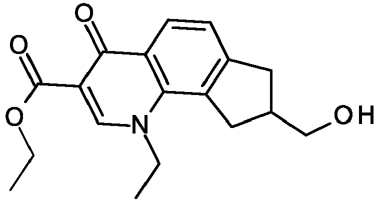

A mixture of ethyl 1-ethyl-8-(methylsulfonyloxymethyl)-4-oxo-8,9-dihydro-7*H-*cyclopenta[h]quinoline-3-carboxylate (640.0 mg, 1.63 mmol, 1 eq) and potassium carbonate (674.4 mg, 4.88 mmol, 3 eq) in MeCN (8 mL) and water (2 mL) was stirred at 90 °C for 32 h. The mixture was partitionated between EtOAc (150 mL) and water (100 mL). The organic layer was washed with saturated brine and concentrated to give a crude product which was purified by Prep-HPLC (formic acid). A mixture of this material (459.7 mg, 1.6 mmol, 1 eq), iodoethane (0.77 mL) and potassium carbonate (884.7 mg) in DMF (8 mL) was stirred at 50 °C for 4 h. The mixture was partitionated between EtOAc (100 mL) and water (80 mL). The organic layer was washed with saturated brine solution and concentrated to give the crude product which was purified by reverse phase chromatography (formic acid) to give the title compound (160.2 mg) as a light yellow semi-solid. MS (ESP): m/z = 316.1 [M+H]⁺.

### Step 5:

### Ethyl 1-ethyl-8-formyl-4-oxo-8,9-dihydro-7H-cyclopenta[h]quinoline-3-carboxylate

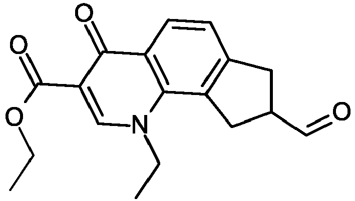

The title compound was prepared by analogy with 1-chloro-6,7-dihydro-5H-cyclopenta[c]pyridine-6-carbaldehyde (Intermediate 19, step 4) and was obtained as a light green solid. MS (ESP): m/z = 314.1 [M+H]⁺.

### Step 6:

### Ethyl 1-ethyl-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylate

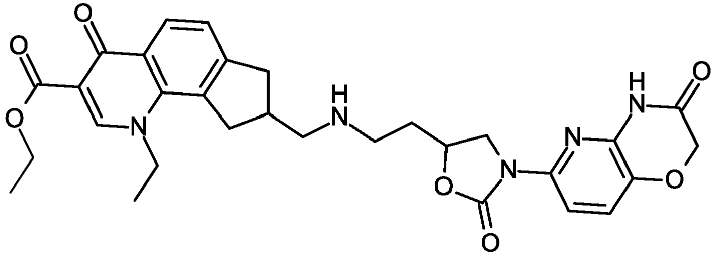

The title compound was prepared by analogy with 6-[5-[2-[(1-chloro-6,7-dihydro-5*H-*cyclopenta[c]pyridin-6-yl)methylamino]ethyl]-2-oxo-1,3-oxazolidin-3-yl]-4*H*-pyrido[3,2-b][1,4]oxazin-3-one (Example 86) using ethyl 1-ethyl-8-formyl-4-oxo-8,9-dihydro-7*H-*cyclopenta[h]quinoline-3-carboxylate in place of 1-chloro-6,7-dihydro-5*H-*cyclopenta[c]pyridine-6-carbaldehyde and was obtained as a grey foam. MS (ESN): m/z = 574.4 [M-H]⁻.

### Intermediate 195

### Ethyl 1-ethyl-4-oxo-8-[[2-[2-oxo-3-(3-oxo-4H-pyrazino[2,3-b] [1,4]oxazin-6-yl)-1,3-oxazolidin-5-yl] ethylamino] methyl] -8,9-dihydro-7H-cyclopenta[h] quinoline-3-carboxylate

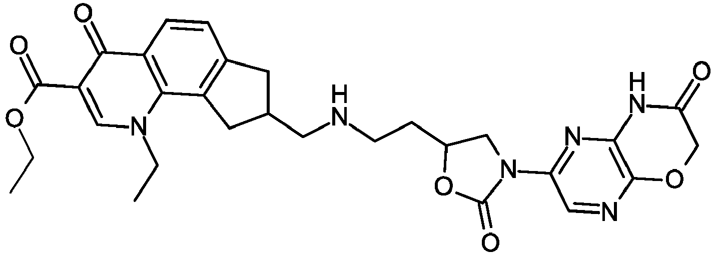

The title compound was prepared by analogy with 6-[5-[2-[(1-chloro-6,7-dihydro-5*H-*cyclopenta[c]pyridin-6-yl)methylamino]ethyl]-2-oxo-1,3-oxazolidin-3-yl]-4*H*-pyrido[3,2-b][1,4]oxazin-3-one (Example 86) using ethyl 1-ethyl-8-formyl-4-oxo-8,9-dihydro-7*H-*cyclopenta[h]quinoline-3-carboxylate (Intermediate 194, step 5) in place of 1-chloro-6,7-dihydro-5*H*-cyclopenta[c]pyridine-6-carbaldehyde and 6-[5-(2-aminoethyl)-2-oxo-1,3-oxazolidin-3-yl]-4*H*-pyrazino[2,3-b][1,4]oxazin-3-one (Intermediate 24) in place of 6-[5-(2-aminoethyl)-2-oxo-1,3-oxazolidin-3-yl]-4*H*-pyrido[3,2-b][1,4]oxazin-3-one and was obtained as a grey foam. MS (ESN): m/z = 575.4 [M-H]⁻.

### Intermediate 196

### 6-[6-(Hydroxymethyl)-2-oxo-1-oxa-3,8-diazaspiro[4.5]decan-3-yl]-4H-pyrido[3,2-b][1,4]oxazin-3-one;2,2,2-trifluoroacetic acid

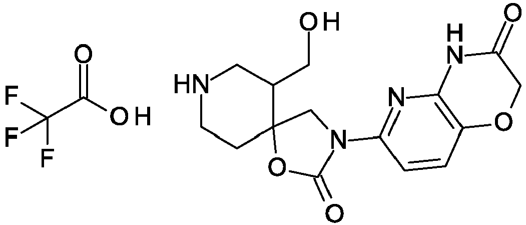

To a solution of *tert*-butyl 6-(hydroxymethyl)-2-oxo-3-(3-oxo-4*H*-pyrido[3,2-b][1,4]oxazin-6-yl)-1-oxa-3,8-diazaspiro[4.5]decane-8-carboxylate (Intermediate 177, Step 9) (50 mg, 0.12 mmol, 1 eq) in DCM (5 mL) was added trifluoroacetic acid (0.5 mL, 6.5 mmol, 56 eq) at 30 °C. The reaction mixture was stirred at 30 °C for 2 h. The reaction mixture was concentrated to provide the title compound (50 mg, 0.11 mmol, 82% yield) as a white solid, which was used without further purification . MS (ESP): m/z = 335.2 [M+H]⁺.

### Intermediate 197

### 6-[5-(Aminomethyl)-5-(2-hydroxyethyl)-2-oxo-1,3-oxazolidin-3-yl]-4H-pyrido[3,2-b][1,4]oxazin-3-one;2,2,2-trifluoroacetic acid

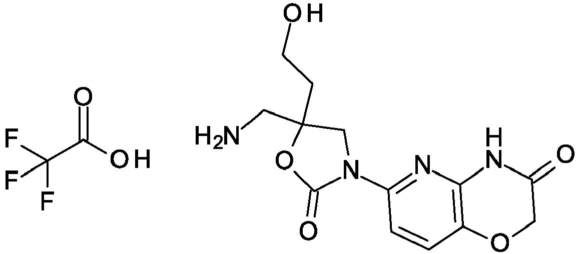

To a solution of *tert*-butyl *N*-[[5-(2-hydroxyethyl)-2-oxo-3-(3-oxo-4*H*-pyrido[3,2-b][1,4]oxazin-6-yl)-1,3-oxazolidin-5-yl]methyl]carbamate (Intermediate 175, Step 9) (80 mg, 0.20 mmol, 1 eq) in DCM (8 mL) was added trifluoroacetic acid (1.0 mL, 12.98 mmol, 66.26 eq) at 30 °C. The reaction mixture was stirred at 30 °C for 2 h. The reaction mixture was concentrated to provide the title compound (60 mg, 0.14 mmol, 70% yield) as light yellow oil, which was used directly without further purification. MS (ESP): m/z = 309.1 [M+H]⁺.

### Intermediate 198

### 2-Trimethylsilylethyl N-[4-fluoro-1-methyl-6-(2-oxoethyl)-5,7-dihydrocyclopenta[c]pyridin-6-yl]carbamate

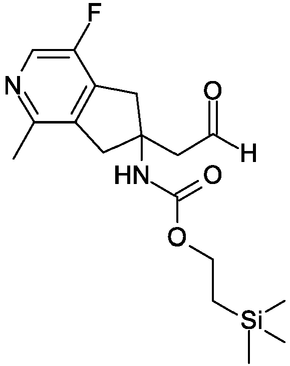

### Step 1:

### Ethyl 2-(4-fluoro-1-methyl-6,7-dihydro-5H-cyclopenta[c]pyridin-6-yl)pent-4-enoate

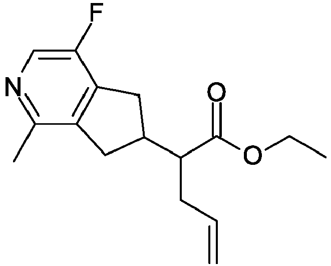

To a solution of ethyl 1-bromo-4-fluoro-6,7-dihydro-5*H*-cyclopenta[c]pyridine-6-carboxylate (Intermediate 27, Step 7) (230.0 mg, 1.03 mmol, 1 eq) in THF (5 mL) at -78 °C was dropwise added 1M sodium bis(trimethylsilyl)aminde in THF (1.24 mL, 1.24 mmol, 1.2 eq). The resulting yellow solution was stirred for 1 h, and then allyl bromide (187 mg, 1.55 mmol, 1.5 eq) was added in portions at -78 °C. The reaction mixture was stirred at -78 °C for 2 h. The mixture was diluted with ethyl acetate (20 mL) and washed with H₂O (5 mL) and sat. NaCl (5 mL). The organic layer was dried and concentrated. The residue was purified by preparative thin layer chromatography (petrol ether : ethyl acetate = 1:1) to give the title compound (200 mg, 0.76 mmol, 74% yield) as a light yellow oil. MS (ESP): m/z = 264.1 [M+H]⁺.

### Step 2:

### 2-(4-Fluoro-1-methyl-6,7-dihydro-5H-cyclopenta[c]pyridin-6-yl)pent-4-enoic acid

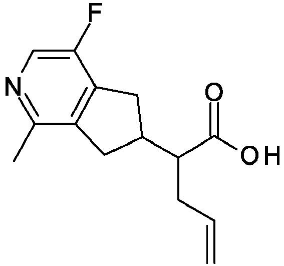

To a solution of ethyl 2-(4-fluoro-1-methyl-6,7-dihydro-5*H*-cyclopenta[c]pyridin-6-yl)pent-4-enoate (270 mg, 1.0 mmol, 1 eq) in methanol (2 mL) was added lithium hydroxide (25 mg, 1.0 mmol, 1.0 eq) in water (2 mL). The reaction mixture was stirred at 40 °C for 0.5 h. To the reaction mixture was added 0.5 M HCl until pH of 6-7 was reached and the reaction mixture was concentrated to afford the title compund (230 mg, 0.98 mmol, 93% yield) as a white solid. The compound was used as such without further purification. MS (ESP): m/z = 236.1 [M+H]⁺.

### Step 3:

### 2-Trimethylsilylethyl N-(4-fluoro-1-methyl-6-prop-2-enyl-5,7-dihydrocyclopenta[c]pyridin-6-yl)carbamate

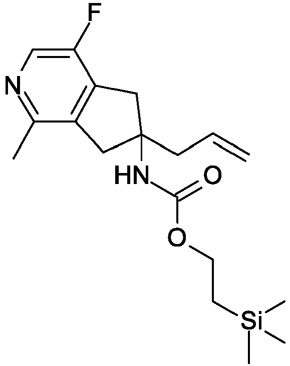

To a solution of triethylamine (0.16 mL, 1.17 mmol, 1.2 eq) in toluene (6 mL) was added diphenylphosphoryl azide (0.25 mL, 1.17 mmol, 1.2 eq) and 2-(4-fluoro-1-methyl-6,7-dihydro-5*H*-cyclopenta[c]pyridin-6-yl)pent-4-enoic acid (230 mg, 0.98 mmol, 1 eq). The mixture was stirred at 25 °C for 2 h. 2-(Trimethylsilyl)ethanol (0.21 mL, 1.47 mmol, 1.5 eq) was added and the mixture was stirred at 90 °C for 16 h. The mixture was diluted with ethyl acetate (40 mL) and washed with H₂O (10 mL) and sat. NaCl (5 mL). The aqueous layer was extracted with ethyl acetate (2 × 10 mL). The combined organic layers were concentrated to give a residue, which was purified by flash chromatography eluting with 20% ethyl acetate in petrol ether to give the title compound (250 mg, 0.710 mmol, 72.9% yield) as a light yellow oil. MS (ESP): m/z = 351.4 [M+H]⁺.

### Step 4:

### 2-Trimethylsilylethyl N-[4-fluoro-1-methyl-6-(2-oxoethyl)-5,7-dihydrocyclopenta[c]pyridin-6-yl] carbamate

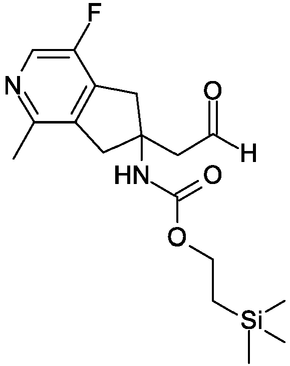

Ozone was bubbled through a suspension of 2-trimethylsilylethyl *N*-(4-fluoro-1-methyl-6-prop-2-enyl-5,7-dihydrocyclopenta[c]pyridin-6-yl)carbamate (100.0 mg, 0.290 mmol, 1 eq) in DCM (20 mL) at -65 °C until a pale blue appeared. The excess ozone was removed by bubbling oxygen and nitrogen. Dimethyl sulfide (0.1 mL, 1.43 mmol, 5 eq) was added to the suspension and the reaction was stirred at -60 °C for 3 h and then at 25 °C for 12 h. The solvent was removed by evaporation and the residue was purified by preparatory thin layer chromatography eluting with (petrol ether:ethyl acetate = 1:1) to give the title compound (50 mg, 0.28 mmol, 22% yield) as a yellow oil. MS (ESP): m/z = 353.1 [M+H]⁺.

### Intermediate 199

### 1,4-Dimethyl-6,7-dihydro-5H-cyclopenta[d]pyridazine-6-carbaldehyde

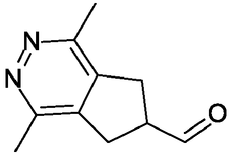

### Step 1:

### Ethyl 1,4-dichloro-6,7-dihydro-5H-cyclopenta[d]pyridazine-6-carboxylate

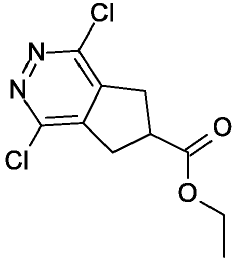

To a solution of 3,6-dichloro-1,2,4,5-tetrazine (CAS# 106131-61-7) (1000 mg, 6.62 mmol, 1 eq) in carbon tetrachloride (80 mL) was added drop wise of a solution of ethyl cyclopent-3-ene-1-carboxylate (CAS# 21622-01-5) (1003 mg, 7.15 mmol, 1.08 eq) in carbon tetrachloride (20 mL) at 0°C under N₂ atmosphere. The reaction was stirred at 0 °C for 2 h and then allowed to warm to room temperature. The reaction was stirred at 20 °C for 15 h and then concentrated. The residue was purified by flash chromatography column on silica gel eluting with pethrol ether : ethyl acetate 3:1 to afford the title compound (360 mg, 1.38 mmol, 21% yield) as light yellow oil. ¹H NMR (400 MHz, CDCl₃) δ ppm 1.32 (t, *J*=7.13 Hz, 4 H) 3.45 (m, 5 H) 4.24 (d, *J*=7.13 Hz, 2 H).

### Step 2:

### Ethyl 1,4-dimethyl-6,7-dihydro-5H-cyclopenta[d]pyridazine-6-carboxylate

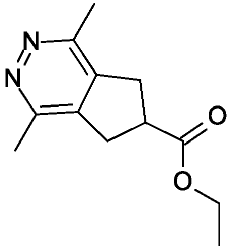

To a solution of ethyl 1,4-dichloro-6,7-dihydro-5*H*-cyclopenta[d]pyridazine-6-carboxylate (1550 mg, 5.94 mmol, 1 eq) in 1,4-dioxane (100 mL) was added K₂CO₃ (3277 mg, 23.8 mmol, 4 eq), trimethylboroxine (5962 mg, 23.8 mmol, 4 eq) and [1,1'-bis(diphenylphosphino)ferrocene]dichloropalladium(II) (869 mg, 1.19 mmol, 0.2 eq). The mixture was stirred for 2 h at 100 °C under nitrogen. The reaction was filtered and the filtrate was concentrated. The residue was purified by flash chromatography on silica gel eluting with ethyl acetate: methanol 10:1 to give the title compound (1100 mg, 5 mmol, 65% yield) as a dark brown solid. MS (ESP): m/z = 221.1 [M+H]⁺.

### Step 3:

### (1,4-Dimethyl-6,7-dihydro-5H-cyclopenta[d]pyridazin-6-yl)methanol

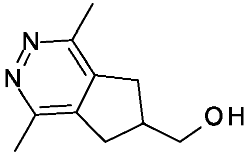

To a solution of ethyl 1,4-dimethyl-6,7-dihydro-5*H*-cyclopenta[d]pyridazine-6-carboxylate (300 mg, 1.36 mmol, 1 eq) in THF (15 mL) at -20 °C was added LiAlH₄ (104 mg, 2.7 mmol, 2 eq) and the mixture was stirred at -20 °C for 2 h. The reaction was quenched by addition of H₂O (0.1 mL) and 10% aq. NaOH (0.3 mL). Ethyl acetate (10 mL) and anhydrous magnesium sulfate was added and the mixture was stirred at 20 °C for 10 min. The mixture was filtered and the filtrate was evaporated to give the title compound (220 mg, 1.23 mmol, 82% yield) as a brown solid, which was used in the next step without further purification. ¹H NMR (400 MHz, CDCl₃) δ ppm 2.42 - 2.56 (m, 6 H) 2.64 - 2.81 (m, 3 H) 2.87 - 3.08 (m, 2 H) 3.55 - 3.69 (m, 2 H).

### Step 4:

### 1,4-Dimethyl-6,7-dihydro-5H-cyclopenta[d]pyridazine-6-carbaldehyde

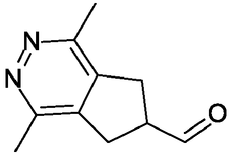

To a solution of (1,4-dimethyl-6,7-dihydro-5*H*-cyclopenta[d]pyridazin-6-yl)methanol (100 mg, 0.56 mmol, 1 eq) in DCM (6 mL) was added 1,1,1-tris(acetyloxy)-1,1-dihydro-1,2-benziodoxol-3-(1*H*)-one (357 mg, 0.84 mmol, 1.5 eq) at 0 °C. Then the mixture was stirred at 25 °C for 2 h. The mixture was filtered through filter membrane and the filtrate was collected and concentrated to give the title compound (90 mg, 0.51 mmol, 82% yield) as a dark brown oil. The purity was estimated to be 50% and this material was used as such in the next step. ¹H NMR (400 MHz, CDCl₃) δ ppm 2.54 - 2.74 (m, 6 H) 3.12 - 3.26 (m, 2 H) 3.30 - 3.40 (m, 2 H) 3.40 - 3.52 (m, 1 H) 9.70 - 9.91 (m, 1 H).

### Intermediate 200

### 4- Fluoro-6,7-dihydro-5H-cyclopenta[c]pyridine-6-carbaldehyde

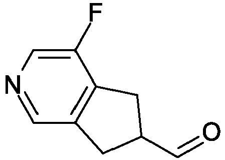

### Step 1:

### Diethyl 4-fluoro-5,7-dihydrocyclopenta[c]pyridine-6,6-dicarboxylate

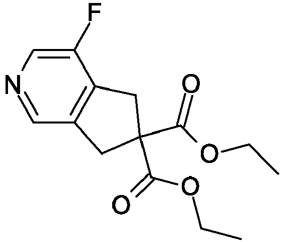

To a solution of diethyl 1-bromo-4-fluoro-5,7-dihydrocyclopenta[c]pyridine-6,6-dicarboxylate (Intermediate 27, Step 5) (510.0 mg, 1.42 mmol, 1 eq) in methanol (20 mL) was added 10% Pd/C (120 mg) at room temperature The reaction was stirred under an atmosphere of hydrogen for 3 hours rred at 20 °C. The mixture was filtered and the filtrate was evaporated to give the title compound (400 mg, 1.4 mmol, 89% yield) as a light yellow solid. MS (ESP): m/z = 282.0 [M+H]⁺.

### Step 2:

### 4-Fluoro-6,7-dihydro-5H-cyclopenta[c]pyridine-6-carboxylic acid;hydrochloride

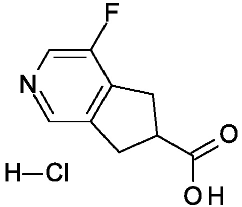

A solution of diethyl 4-fluoro-5,7-dihydrocyclopenta[c]pyridine-6,6-dicarboxylate (460.0 mg, 1.64 mmol, 1 eq) in 12M HCl (10 mL) was stirred at 100 °C for 16 h. The solvent was removed by evaporation to give the title compound (300 mg, 1.38 mmol, 42% yield) as grey solid, which was used directly without further purification. MS (ESP): m/z = 182.1 [M+H]⁺.

### Step 3:

### Ethyl 4-fluoro-6,7-dihydro-5H-cyclopenta[c]pyridine-6-carboxylate

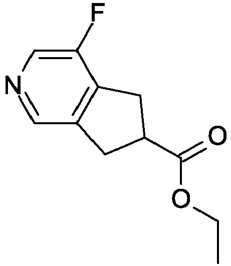

To a solution of 4-fluoro-6,7-dihydro-5*H*-cyclopenta[c]pyridine-6-carboxylic acid;hydrochloride (300 mg, 1.7 mmol, 1 eq) in ethanol (8 mL) was added sulfuric acid (0.2 mL). The mixture was stirred at 80 °C for 3 h. The mixture was diluted with ethyl acetate (30 mL) and washed with sat. NaHCOs (30 mL) and sat. NaCl (30 mL). The organic layer was dried and concentrated to give the title compound (200 mg, 0.96 mmol, 56% yield) as light grey oil, which was used in the next step without further purification. MS (ESP): m/z = 210.1 [M+H]⁺.

### Step 4:

### (4-Fluoro-6,7-dihydro-5H-cyclopenta[c]pyridin-6-yl)methanol

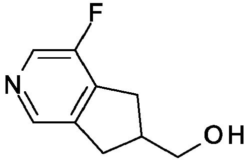

To a solution of ethyl 4-fluoro-6,7-dihydro-5*H*-cyclopenta[c]pyridine-6-carboxylate (200 mg, 0.96 mmol, 1 eq) in THF (10 mL) at -10 °C was added LiAlH₄ (73 mg, 1.9 mmol, 2 eq) and the mixture was stirred at -10 °C for 2 hours. The reaction was quenched by the addition of H₂O (0.05 mL) and 10% aq. NaOH (0.15 mL). Ethyl acetate was added (20 mL) and dried over anhydrous magnesium sulfate. The mixture was stirred at 20 °C for 10 min. The mixture was filtered and the filtrate was evaporated to give the title compound (160 mg, 0.96 mmol, 85% yield) as colorless oil, which was used in the next step without further purification. ¹H NMR (400 MHz, CDCl₃) δ ppm 2.55 - 2.75 (m, 3 H) 2.88 - 3.07 (m, 2 H) 3.44 - 3.60 (m, 2 H) 3.87 - 4.02 (m, 1 H) 7.97 - 8.16 (m, 2 H).

### Step 5:

### 4-Fluoro-6,7-dihydro-5H-cyclopenta[c]pyridine-6-carbaldehyde

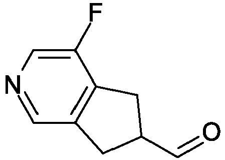

To a solution of (4-fluoro-6,7-dihydro-5*H*-cyclopenta[c]pyridin-6-yl)methanol (80 mg, 0.48 mmol, 1 eq) in DCM (5 mL) was added 1,1,1-tris(acetyloxy)-1,1-dihydro-1,2-benziodoxol-3-(1H)-one (304 mg, 0.72 mmol, 1.5 eq) at 0 °C. Then the mixture was stirred at 25 °C for 2 h. The mixture was filtered and the filtrate was evaporated to give the title compound (60 mg, 0.36 mmol, 61% yield) as brown semisolid. The purity was estimated to be 50% and this material was used as such in the next step.

### Intermediate 201

### 2-[5-(2-Aminoethyl)-2-oxo-1,3-oxazolidin-3-yl]-8H-pyrimido[5,4-b] [1,4]oxazin-7-one;formic acid

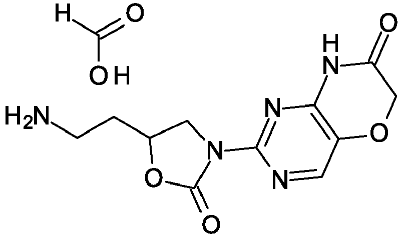

### Step 1:

### tert-butyl N-[2-[2-oxo-3-(7-oxo-8H-pyrimido[5,4-b][1,4]oxazin-2-yl)-1,3-oxazolidin-5-yl]ethyl]carbamate

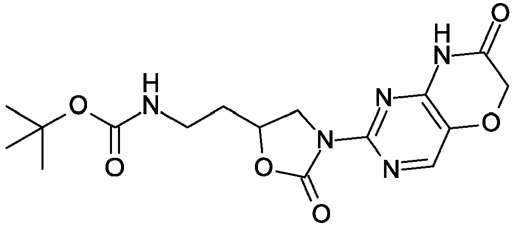

To a solution of 2-chloro-8*H*-pyrimido[5,4-b][1,4]oxazin-7-one (CAS# 943995-32-2) (170 mg, 0.92 mmol, 1 eq) and *tert*-butyl *N*-[2-(2-oxo-1,3-oxazolidin-5-yl)ethyl]carbamate (Iintermediate 26, 316 mg, 1.37 mmol, 1.5 eq) in 1,4-dioxane (10 mL) was added copper(I) iodide (0.03 mL, 0.92 mmol, 1 eq), potassium carbonate (380 mg, 2.75 mmol, 3 eq) and *trans-N,N'-*dimethylcyclohexane-1,2-diamine (130 mg, 0.92 mmol, 1 eq). The mixture was stirred at 100 °C for 12 h under nitrogen. The mixture was concentracted to give a residue and the residue was purified by flash column chromatography (DCM:MeOH=10:1) and prep-HPLC. The product was further purified by preparative thin layer chromatography (petrol ether : ethyl acetate 0:1) to give the title compound (240 mg, 0.63 mmol, 18% yield) as a white solid. MS (ESP): m/z = 380.2 [M+H]⁺.

### Step 2:

### 2-[5-(2-Aminoethyl)-2-oxo-1,3-oxazolidin-3-yl]-8H-pyrimido[5,4-b] [1,4]oxazin-7-one;formic acid

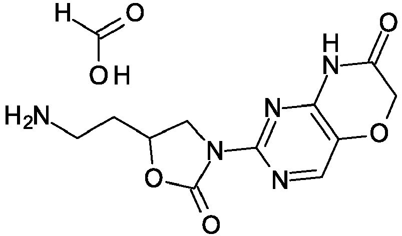

A mixture *tert*-butyl *N*-[2-[2-oxo-3-(7-oxo-8*H*-pyrimido[5,4-b] [1,4]oxazin-2-yl)-1,3-oxazolidin-5-yl]ethyl]carbamate (240 mg, 0.63 mmol, 1 eq) in formic acid (5.0 mL, 0.63 mmol, 1 eq) was stirred at 30 °C for 2 h. The mixture was concentracted to give the title compound (270 mg, quant. yield) as a colorless oil, which was used in the next step withouth further purification. MS (ESP): m/z = 280.1 [M+H]⁺.

### Intermediate 202

### 4-Fluoro-6-(2-hydroxy-2-methylpropoxy)-2,3-dihydro-1H-indene-2-carbaldehyde

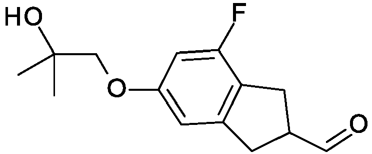

### Step 1:

### 1-[[2-[[tert-Butyl(dimethyl)silyl]oxymethyl]-7-fluoro-2,3-dihydro-1H-inden-5-yl]oxy]-2-methylpropan-2-ol

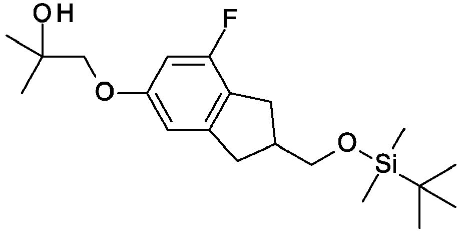

To a solution of 2-[[tert-butyl(dimethyl)silyl]oxymethyl]-7-fluoro-2,3-dihydro-1*H*-inden-5-ol (Intermediate 52, Step 2) (150 mg, 0.51 mmol, 1 eq) in ethanol (6 mL) was added 1-chloro-2-methyl-2-propanol (CAS# 558-42-9), potassium carbonate (420 mg, 3.0 mmol, 6 eq) and water (0.6 mL). The mixture was stirred at 90 °C for 16 h. The mixture was quenched with H₂O (10 mL) and extracted with ethyl acetate (3×15 mL). The combined organic layers were washed with sat. NaCl (10 mL), dried over Na₂SO₄ and concentrated. The residue was purified by flash chromatography (petrol ether : ethyl acetate 1:1) to give the title compound (160 mg, 0.43 mmol, 84% yield) as a colorless oil. MS (ESP): m/z = 351.6 [M-OH]⁺.

### Step 2:

### 4-Fluoro-6-(2-hydroxy-2-methylpropoxy)-2,3-dihydro-1H-indene-2-carbaldehyde

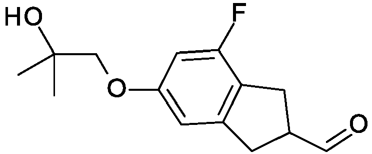

The title compound was prepared by analogy to intermediate 52 (steps 4 and 5) by using 1-[[2-[[*tert*-butyl(dimethyl)silyl]oxymethyl]-7-fluoro-2,3-dihydro-1*H*-inden-5-yl]oxy]-2-methylpropan-2-ol in place of 2-[[2-[[*tert*-butyl(dimethyl)silyl]oxymethyl]-7-fluoro-2,3-dihydro-1*H*-inden-5-yl]oxy]acetonitrile and was obtained as a light yellow semi-solid in estimated 50% purity. ¹H NMR (400 MHz, CDCl₃) δ ppm 1.26 (s, 7 H) 2.98 - 3.33 (m, 5 H) 3.67 (s, 2 H) 6.32 - 6.68 (m, 2 H) 9.51 - 9.84 (m, 1 H).

### Intermediate 203

### 6-[5-(2-Aminoethyl)-2-oxo-1,3-oxazol-3-yl]-4H-pyrazino[2,3-b][1,4]oxazin-3-one;formic acid

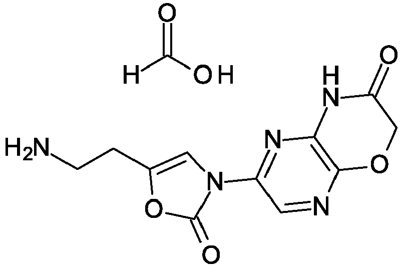

### Step 1:

### tert-Butyl N-[3-oxo-4-(2-trimethylsilylethoxymethyl)pyrazino[2,3-b] [1,4]oxazin-6-yl]carbamate

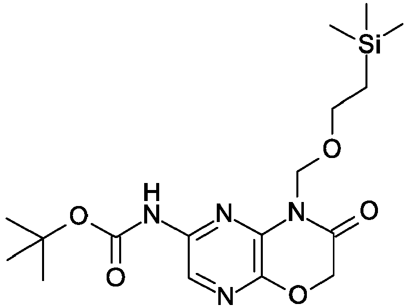

A solution of 9,9-dimethyl-4,5-bis(diphenylphosphino)xanthene (339 mg, 0.59 mmol, 0.11 eq), tris(dibenzylideneacetone)dipalladium (0) (519 mg, 0.57 mmol, 0.1 eq), *tert*-butyl carbamate (CAS# 4248-19-5) (1300.42 mg, 11.1 mmol, 2 eq), Intermediate 25 (2000 mg, 5.55 mmol, 1 eq) and potassium carbonate (1536 mg, 11.1 mmol, 2 eq) in 1,4-dioxane (40 mL) was degassed with nitrogen and stirred at 100 °C for 12 h. The mixture was filtered and the filtrate was evaporated to give a residue, which was purified by flash chromatography (20 g SiO₂, petrol ether : ethyl acetate 3:1). Further purification by reversed phase flash chromatography (MeCN/water) gave the title compound (1600 mg, 4.04 mmol, 72.69% yield) as a yellow solid. MS (ESP): m/z = 397.2 [M+H]⁺.

### Step 2:

### tert-Butyl N-[4-[tert-butyl(dimethyl)silyl]oxybut-1-ynyl]-N-[3-oxo-4-(2-trimethylsilylethoxymethyl)pyrazino[2,3-b][1,4] oxazin-6-yl] carbamate

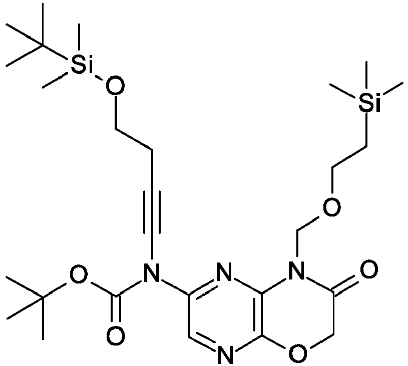

To a solution of freshly prepared 4-bromobut-3-ynoxy-tert-butyl-dimethyl-silane (CAS# 448944-55-6) (1394 mg, 5.3 mmol, 1.5 eq) and *tert*-butyl *N*-[4-[tert-butyl(dimethyl)silyl]oxybut-1-ynyl]-*N*-[3-oxo-4-(2-trimethylsilylethoxymethyl)pyrazino[2,3-b] [1,4]oxazin-6-yl]carbamate (1.4 g, 3.5 mmol, 1 eq) in 1,4-dioxane (28 mL) was added K₃PO₄ (1835 mg, 8.5 mmol, 2.4 eq), 3,4,7,8-tetramethyl-1,10-phenanthroline (334 mg, 1.4 mmol, 0.4 eq) and CuSO₄•5H₂O (881 mg, 3.5 mmol, 1 eq). The mixture was stirred at 85 °C for 12 h under nitrogen. The mixture was filtered and concentrated. The residue was purified by flash chromatography (40 g SiO₂, petrol ether : ethyl acetate 3:1) to give the title compound (625 mg, 1.08 mmol, 31% yield) as a colorless oil. MS (ESP): m/z = 579.4 [M+H]⁺.

### Step 3:

### 6-[5-[2-[tert-Butyl(dimethyl)silyl]oxyethyl]-2-oxo-1,3-oxazol-3-yl]-4-(2-trimethylsilylethoxymethyl)pyrazino[2,3-b] [1,4]oxazin-3-one

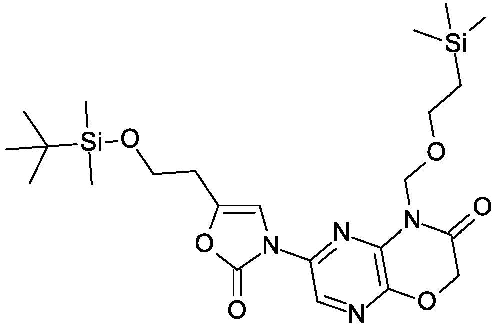

A mixture of *tert*-butyl *N*-[4-[*tert*-butyl(dimethyl)silyl]oxybut-1-ynyl]-N-[3-oxo-4-(2-trimethylsilylethoxymethyl)pyrazino[2,3-b][1,4]oxazin-6-yl]carbamate (3000 mg, 5.18 mmol, 1 eq) in DCM (55 mL) was added to a solution of chloro(triphenylphosphine)gold(I) (1026 mg, 2.1 mmol, 0.40 eq) and silver hexafluoroantimonate(V) (723 mg, 2.1 mmol, 0.40 eq) in acetonitrile (5.5 mL). The mixture was stirred at 40 °C for 12 h. The mixture was concentrated and the residue was purified by column chromatography (petrol ether : ethyl acetate 3:1) to give the title compound (2500 mg, 4.78 mmol, 92% yield) as a yellow oil. MS (ESP): m/z = 523.3 [M+H]⁺.

### Step 4:

### 6-[5-(2-Hydroxyethyl)-2-oxo-1,3-oxazol-3-yl]-4-(2-trimethylsilylethoxymethyl)pyrazino[2,3-b][1,4] oxazin-3 -one

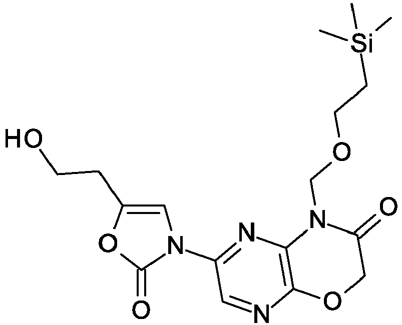

To a solution of 6-[5-[2-[*tert*-butyl(dimethyl)silyl]oxyethyl]-2-oxo-1,3-oxazol-3-yl]-4-(2-trimethylsilylethoxymethyl)pyrazino[2,3-b][1,4]oxazin-3-one (5000 mg, 9.56 mmol, 1 eq) in THF (50 mL) was added 1M hydrochloric acid (50 mL). The mixture was stirred for 3 h at 25 °C. The mixture was diluted with water (20 mL). The aqueous layer was adjusted to pH 7 with sat. NaHCOs and then extracted with ethyl acetate (2 × 50 mL). The combined organic layers were concentrated and the residue was purified by column chromatography (petrol ether : ethyl acetate 1:12) to give the title compound (3600 mg, 8.81 mmol, 92% yield) as a yellow semisolid. MS (ESP): m/z = 431.1 [M+Na]⁺.

### Step 5:

### 2-[2-Oxo-3-[3-oxo-4-(2-trimethylsilylethoxymethyl)pyrazino[2,3-b] [1,4]oxazin-6-yl]-1,3-oxazol-5-yl] ethyl methanesulfonate

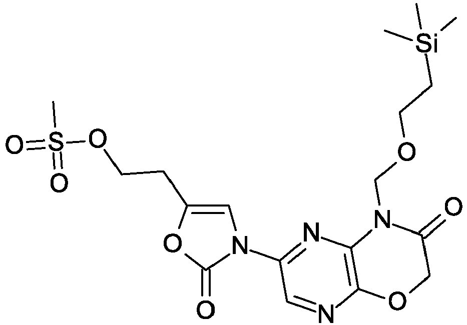

To a mixture of 6-[5-(2-hydroxyethyl)-2-oxo-1,3-oxazol-3-yl]-4-(2-trimethylsilylethoxymethyl)pyrazino[2,3-b][1,4]oxazin-3-one (1000 mg, 2.45 mmol, 1 eq) and *N,N*-diisopropylethylamine (1.28 mL, 7.35 mmol, 3 eq) in DCM (20 mL) at 0 °C was added methanesulfonyl chloride (0.38 mL, 4.89 mmol, 2 eq). The reaction was stirred at 25 °C for 2 h. The mixture was quenched with saturated NaHCOs (30 mL), extracted with DCM (30 mL) and the organic layer was dried over Na₂SO₄ and concentrated to give the title compound (1000 mg, 2.06 mmol, 84% yield) as a light brown oil. The product was used without futher purification in the next step. MS (ESP): m/z = 487.2 [M+H]⁺.

### Step 6:

### 6-[5-(2-Azidoethyl)-2-oxo-1,3-oxazol-3-yl]-4-(2-trimethylsilylethoxymethyl)pyrazino[2,3-b][1,4]oxazin-3-one

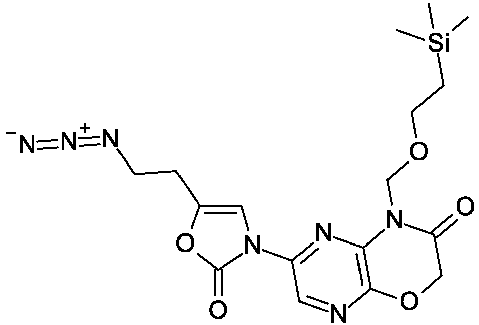

To a solution of 2-[2-oxo-3-[3-oxo-4-(2-trimethylsilylethoxymethyl)pyrazino[2,3-b][1,4]oxazin-6-yl]-1,3-oxazol-5-yl]ethyl methanesulfonate (3000 mg, 6.2 mmol, 1 eq) in DMF (60 mL) was added sodium azide (1200 mg, 18.5 mmol, 3 eq). The mixture was stirred at 60 °C for 16 h under nitrogen atmosphere. The mixture was diluted with EtOAC(100 mL) and washed with water (2 × 50 mL) and sat. NaCl (30 mL). The organic layer was dried and concentrated. The residue was purified by flash chromatography on silica gel to give the title compound (1500 mg, 3.5 mmol, 562% yield) as a colorless oil. MS (ESP): m/z = 434.1 [M+H]⁺.

### Step 7:

### 6-[5-(2-Azidoethyl)-2-oxo-1,3-oxazol-3-yl]-4H-pyrazino[2,3-b][1,4]oxazin-3-one

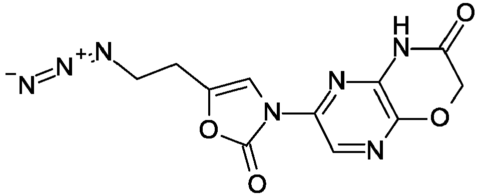

To a solution of 6-[5-(2-azidoethyl)-2-oxo-1,3-oxazol-3-yl]-4-(2-trimethylsilylethoxymethyl)pyrazino[2,3-b][1,4]oxazin-3-one (1000 mg, 2.3 mmol, 1 eq) in DCM (10 mL) was added trifluoroacetic acid (2.0 mL, 2.3 mmol, 1 eq). The mixture was stirred at 25 °C for 2 h under nitrogen atmosphere. The mixture was concentrated and the residue was dissolved in ethanol (10 mL) at 0 °C. K₂CO₃ (637 mg, 4.6 mmol, 2 eq) was added and the mixture was stirred at 0 °C 2 h. TFA (0.26 mL, 3.46 mmol, 1.5 eq) was added and the mixture was stirred for 5 min. The mixture was concentrated to about 1 mL, then 5 mL water was added. The precipitate was filtered and triturated with EtOAc:petrol ether 1:3 (2 × 3 mL). The solid was dried under vacuum to give the title compound (500 mg, 1.65 mmol, 71% yield) as a light yellow solid. MS (ESP): m/z = 304.1 [M+H]⁺.

### Step 8:

### tert-Butyl N-[2-[2-oxo-3-(3-oxo-4H-pyrazino[2,3-b][1,4]oxazin-6-yl)-1,3-oxazol-5-yl] ethyl] carbamate

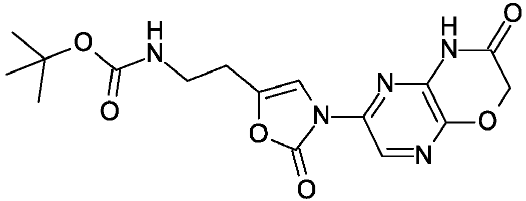

To a solution of 6-[5-(2-azidoethyl)-2-oxo-1,3-oxazol-3-yl]-4*H*-pyrazino[2,3-b][1,4]oxazin-3-one (1150 mg, 3.79 mmol, 1 eq) in THF (20 mL) and water (15 mL) was added Me₃P (4.17 mL, 4.17 mmol, 1.1 eq). The mixture was stirred at 25 °C for 1 h under nitrogen atmosphere. Triethylamine (1.06 mL, 7.59 mmol, 2 eq) and di-*tert*-butyl dicarbonate (1240 mg, 5.69 mmol, 1.5 eq) was added and the mixture was stirred at 25 °C for 1 h. The organic phase was removed under vacuum and the mixture was filtered. The filter cake was washed with water (2 × 10 mL) , triturated with EtOAc:petrol ether 1:1 (2 × 5 mL) and dried under high vacuum to give the title compound (750 mg, 1.99 mmol, 52% yield) as a light yellow solid. MS (ESP): m/z = 400.2 [M+Na]⁺.

### Step 9:

### 6-[5-(2-Aminoethyl)-2-oxo-1,3-oxazol-3-yl]-4H-pyrazino[2,3-b][1,4]oxazin-3-one;formic acid

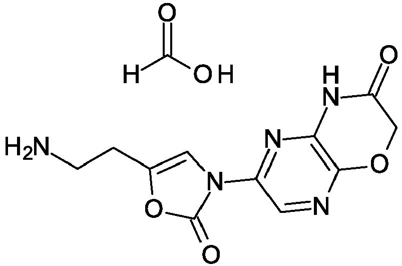

A solution of *tert*-butyl *N*-[2-[2-oxo-3-(3-oxo-4*H*-pyrazino[2,3-b][1,4]oxazin-6-yl)-1,3-oxazol-5-yl]ethyl]carbamate (750 mg, 2.0 mmol, 1 eq) in formic acid (5.0 mL, 1.99 mmol, 1 eq) was stirred at 25 °C for 1 h under nitrogen atmosphere. The mixture was concentrated under high vacuum to give a residue which was triturated with acetonitrile (10 mL). The precipitate was filtered and lyophilized to give the title compound (637 mg, 2.0 mmol, 95% yield) as a light yellow solid. MS (ESP): m/z = 278.1 [M+H]⁺

### Intermediate 204

### 2-[[tert-Butyl(dimethyl)silyl]oxymethyl]-7-fluoro-indan-5-ol

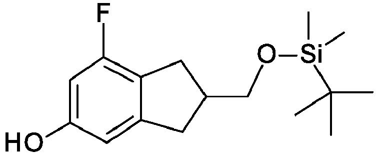

and

### Intermediate 204-A

### 2-[[tert-Butyl(dimethyl)silyl]oxymethyl]-4-fluoro-indan-5-ol

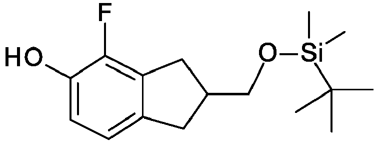

### Step 1:

### Ethyl 4-fluoro-6-hydroxy-indane-2-carboxylate and ethyl 4-fluoro-5-hydroxy-indane-2-carboxylate

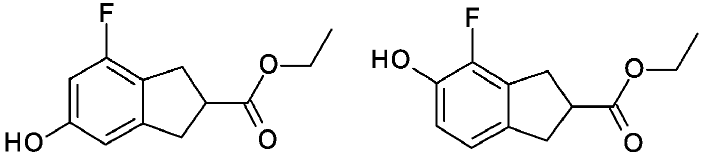

To a stirred mixture of 4,4,5,5-tetramethyl-1,3,2-dioxaborolane (18438 mg, 144.1 mmol, 2 eq) and 3,4,7,8-tetramethyl-1,10-phenanthroline (681 mg, 2.88 mmol, 0.040 eq) was added methoxy(cyclooctadiene)iridium(I) dimer (955 mg, 1.44 mmol, 0.020 eq) under N₂ atmosphere and the mixture was stirred for further 5 min, and then ethyl 4-fluoroindane-2-carboxylate (Intermediate 50, Step 2, 15.0 g, 72.04 mmol, 1 eq) was added and the mixture was stirred at 65 °C for 16 h. The reaction mixture was cooled to room temperature and the reaction mixture was dissolved in 200 mL EtOAc and the catalyst was filtered off, the filtrate was concentrated and the residue was taken up in THF (50 mL) and the solution was cooled to 0°C. Then a solution of sodium perborate monohydrate (43135 mg, 432 mmol, 6 eq) in water (50 mL) was added slowly, and the resulting mixture was stirred at room temperature for 1 h. The solid was filtered off and the filtrate was added to water (10 mL), extracted with EtOAc (100 mL × 3), combined with the organics and dried by Na₂SO₄. The solvent was removed to give the crude, which was purified by silica gel (PE: EtOAc=5:1) to give the title compound mixture (9.4 g, 41.9 mmol, 58.2% yield) as a colorless solid.

### Steps 2 to 4:

### (6-Benzyloxy-4-fluoro-indan-2-yl)methoxy-tert-butyl-dimethyl-silane and (5-benzyloxy-4-fluoro-indan-2-yl)methoxy-tert-butyl-dimethyl-silane

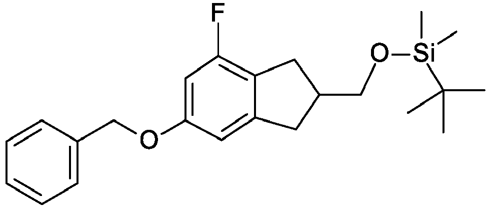

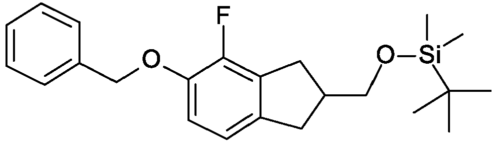

The title compound mixture was prepared by analogy with *tert*-butyl-[(4-fluoro-6-phenylmethoxy-2,3-dihydro-1*H*-inden-2-yl)methoxy]-dimethylsilane (Intermediate 50, Steps 4 and 5 then Intermediate 52, Step 1) and was obtained as a colorless oil.

### Step: 5

### 2-[[tert-Butyl(dimethyl)silyl]oxymethyl]-7-fluoro-indan-5-ol and 2-[[tert-butyl(dimethyl)silyl]oxymethyl]-4-fluoro-indan-5-ol

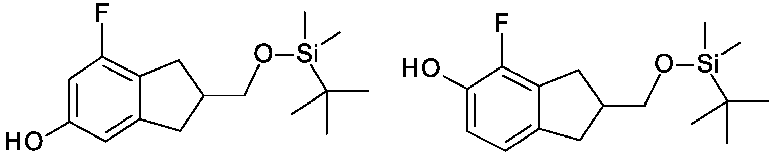

To a solution of (6-benzyloxy-4-fluoro-indan-2-yl)methoxy-tert-butyl-dimethyl-silane and (5-benzyloxy-4-fluoro-indan-2-yl)methoxy-tert-butyl-dimethyl-silane (18.5 g, 47.9 mmol, 1 eq) in methanol (500 mL) was added Pd/C catalyst (3 g), and then the mixture was stirred at 25 °C under hydrogen atmosphere for 4 h. The mixture was filtered and the filtrate was concentrated to give the crude, which was purified by chromatography on silica gel (PE:EtOAc=20:1 to 5:1) to give 2-[[tert-butyl(dimethyl)silyl]oxymethyl]-7-fluoro-indan-5-ol (Intermediate 204, 15.4 g, 52.0 mmol, 77.4% yield) as a white solid; ¹H-NMR (400 MHz, d6-DMSO) δ 9.48 (s, 1H), 6.42 (s, 1H), 6.25 (d, 1H), 3.50 (d, 2H), 2.83 (m, 2H), 2.5 (m, 3H), 0.83 (s, 9H), 0.01 (s, 6H) and 2-[[*tert-*butyl(dimethyl)silyl]oxymethyl]-4-fluoro-indan-5-ol (Intermediate 204-A, 1817 mg, 6.13 mmol, 12.8% yield) as a white solid; ¹H NMR (400 MHz, DMSO-*d*₆) δ 9.38 (s, 1H), 6.80 - 6.74 (m, 1H), 6.73 - 6.65 (m, 1H), 3.54 (d, *J* = 6.2 Hz, 2H), 2.98 - 2.80 (m, 2H), 2.68 - 2.54 (m, 3H), 0.85 (s, 9H), 0.06 - 0.01 (m, 6H).

### Intermediate 205

### 6-[2-(5-Aminotetrazol-1-yl)ethoxy]-4-fluoro-indane-2-carbaldehyde

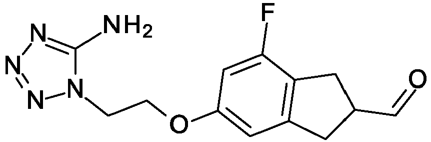

### Step 1:

### 1-[2-[2-[[tert-Butyl(dimethyl)silyl]oxymethyl]-7-fluoro-indan-5-yl]oxyethyl]tetrazol-5-amine

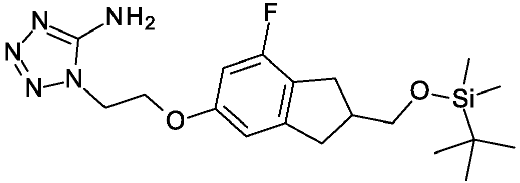

### 2-[2-[2-[[tert-Butyl(dimethyl)silyl]oxymethyl]-7-fluoro-indan-5-yl]oxyethyl]tetrazol-5-amine

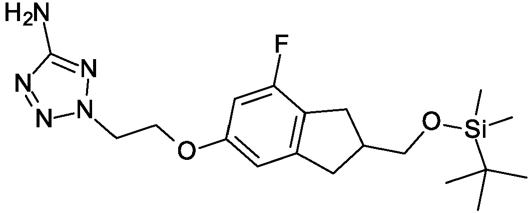

To a mixture of [6-(2-bromoethoxy)-4-fluoro-indan-2-yl]methoxy-tert-butyl-dimethyl-silane (Intermediate 217, 300.0 mg, 0.740 mmol, 1 eq) and 2*H*-tetrazol-5-amine (CAS# 4418-61-5, 0.02 mL, 2.23 mmol, 3 eq) in DMF (24 mL) was added K₂CO₃ (513.14 mg, 3.72 mmol, 5 eq) and NaI (110.81 mg, 0.740 mmol, 1 eq), then the mixture was heated to 100 °C for 16 h. The mixture filtered and concentrated under vacuum to give a residue. The residue was purified by Prep-TLC(PE/EtOAc=1/1) to give 2-[2-[2-[[*tert*-butyl(dimethyl)silyl]oxymethyl]-7-fluoro-indan-5-yl]oxyethyl]tetrazol-5-amine (140 mg, 0.340 mmol, 44.3% yield) as a white solid and 1-[2-[2-[[*tert*-butyl(dimethyl)silyl]oxymethyl]-7-fluoro-indan-5-yl]oxyethyl]tetrazol-5-amine (150 mg, 0.370 mmol, 43.1% yield) as a white solid. MS (ESP): m/z = 408.2 [M+H]⁺.

### Step 2:

### [6-[2-(5-Aminotetrazol-1-yl)ethoxy]-4-fluoro-indan-2-yl]methanol

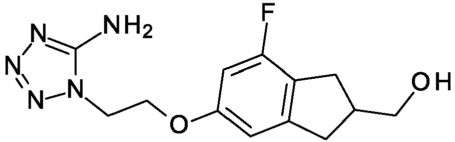

To a solution of 1-[2-[2-[[*tert*-butyl(dimethyl)silyl]oxymethyl]-7-fluoro-indan-5-yl]oxyethyl] tetrazol-5-amine (130.0 mg, 0.320 mmol, 1 eq) in THF (13 mL) was added TBAF in THF (1.3 mL, 1.3 mmol, 4.07 eq), and then the solution was stirred at 25 °C for 16 h. The mixture was concentrated and the residue was purified by prep-TLC (EtOAc /MeOH=10/1, Rf=0.05) to give the title compound (60 mg, 0.200 mmol, 64.2% yield) as a colorless oil. MS (ESP): m/z = 294.1 [M+H]⁺.

### Step 3:

### 6-[2-(5-Aminotetrazol-1-yl)ethoxy]-4-fluoro-indane-2-carbaldehyde

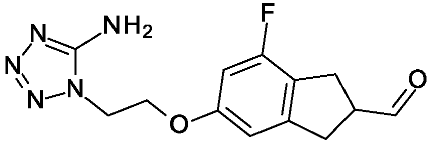

To a solution of [6-[2-(5-aminotetrazol-1-yl)ethoxy]-4-fluoro-indan-2-yl]methanol (30.0 mg, 0.100 mmol, 1 eq) in DCM (6 mL) and pyridine (0.02 mL, 0.200 mmol, 2 eq) was added Dess-Martin Periodinane (65.07 mg, 0.150 mmol, 1.5 eq) and the mixture stirred at 25 °C for 27 h. The mixture was filtrated and the filtrate evaporated. The residue was purified by prep-TLC (PE/ EtOAc =1/1, Rf=0.4) to give the title compound (15 mg, 0.050 mmol, 50.4% yield) as a yellow oil. MS (ESP): m/z = 292.1 [M+H]⁺.

### Intermediate 206

### 6-[2-(5-Aminotetrazol-2-yl)ethoxy]-4-fluoro-indane-2-carbaldehyde

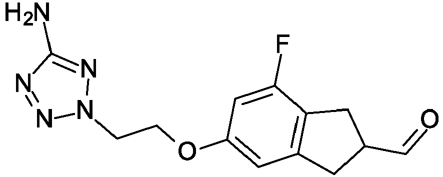

The title compound was prepared by analogy with 6-[2-(5-aminotetrazol-1-yl)ethoxy]-4-fluoro-indane-2-carbaldehyde (Intermediate 205) using 2-[2-[2-[[*tert*-butyl(dimethyl)silyl]oxymethyl]-7-fluoro-indan-5-yl]oxyethyl]tetrazol-5-amine (Intermediate 205, Step 1) in place of 1-[2-[2-[[*tert*-butyl(dimethyl)silyl]oxymethyl]-7-fluoro-indan-5-yl]oxyethyl] tetrazol-5-amine and was obtained as a yellow oil. MS (ESP): m/z = 292.2 [M+H]⁺.

### Intermediate 207

### 4-Fluoro-6-[2-(1,2,4-triazol-1-yl)ethoxy]indane-2-carbaldehyde

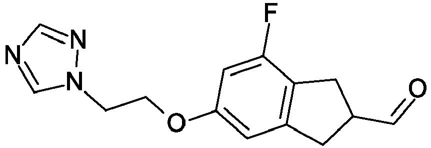

The title compound was prepared by analogy with 6-[2-(5-aminotetrazol-1-yl)ethoxy]-4-fluoro-indane-2-carbaldehyde (Intermediate 205) using 1,2,4-triazole (CAS# 288-88-0) in place of 2*H-*tetrazol-5-amine and was obtained as a yellow oil.

### Intermediate 208

### 3-[3-Oxo-4-(2-trimethylsilylethoxymethyl)pyrazino[2,3-b] [1,4]oxazin-6-yl]-1-oxa-3-azaspiro[4.5]decane-2,8-dione

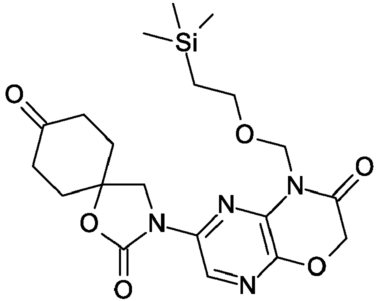

### Step 1:

### 8-(Nitromethyl)-1,4-dioxaspiro[4.5]decan-8-ol

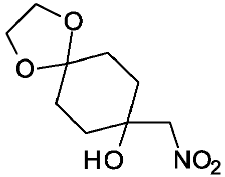

To a solution of 1,4-dioxaspiro[4.5]decan-8-one (CAS# 4746-97-8, 20.0 g, 128.06 mmol, 1 eq) and nitromethane (10.4 mL, 192.09 mmol, 1.5 eq) in ethanol (200 mL) was added sodium ethylate (435.72 mg, 6.4 mmol, 0.050 eq), and then the solution was stirred at 25 °C for 12 h. The reaction was concentrated to dryness. The residue was diluted with EtOAc (200 mL). The organic layer was washed with water (100 mL) and brine (100 mL), dried over anhydrous Na₂SO₄, concentrated under reduced pressure to afford a crude product. The residue was purified by column gel eluting with PE/EtOAc= 3/1. The desired fractions were concentrated to dryness in vacuo to give the title compound (10 g, 46.04 mmol, 35.9% yield) as a yellow oil. ¹H NMR (400 MHz, CDCl₃) δ 4.43 (s, 2H), 4.00 - 3.86 (m, 4H), 2.92 (s, 1H), 2.00 - 1.89 (m, 2H), 1.80 - 1.70 (m, 4H), 1.65 - 1.57 (m, 2H).

### Step 2:

### (8-Methyl-1,4-dioxaspiro[4.5]decan-8-yl) N-methylcarbamate

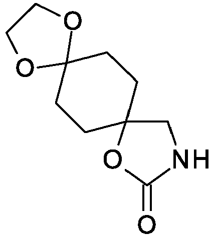

To a solution of 8-(nitromethyl)-1,4-dioxaspiro[4.5]decan-8-ol (3.96 g, 18.23 mmol, 1 eq) in methanol (50 mL) was added Pd(OH)₂/C (4 g) at 25 °C, and then the solution was stirred at 25 °C for 12 h under H₂ (50 Psi). The solid was filtered off and the cake was washed with 20 mL MeOH, combined with the organics, and the solvent was removed. The residue was dissolved in THF (50 mL). To the mixture was added *N,N'*-carbonyldiimidazole (3.9 g, 24.03 mmol, 1.5 eq), and then the solution was stirred at 25 °C for 12 h. Some white solid precipitated. The mixture was filtered and the filter cake was dissolved in THF (33 mL). To the mixture was added *N,N'*-carbonyldiimidazole (0.86 g, 5.33 mmol, 0.5 eq) and then the solution was stirred at 60 °C for 12 h. Some white solid precipitated. The mixture was filtered and the filter cake was concentrated to give the title compound (800 mg, 3.49 mmol, 32.7% yield) as a white solid. ¹H NMR (400 MHz, DMSO-*d*₆) δ 3.88 (s, 4H), 3.80 (s, 2H), 1.99 - 1.80 (m, 4H), 1.66 (br t, *J* = 4.8 Hz, 4H).

### Step 3:

### 3-[3-Oxo-4-(2-trimethylsilanyl-ethoxymethyl)-3,4-dihydro-2H-pyrazino[2 ,3-b][1,4]oxazin-6-yl]-1,9,12-trioxa-3-aza-dispiro[4.2.4.2]tetradecan- 2-one

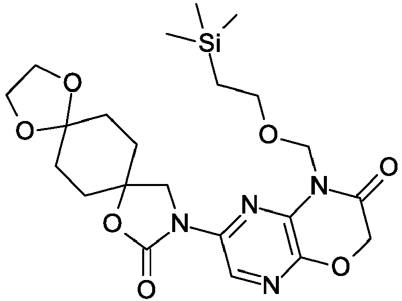

To a solution of (8-methyl-1,4-dioxaspiro[4.5]decan-8-yl) *N*-methylcarbamate (1000.0 mg, 4.69 mmol, 1 eq) and 6-bromo-4-(2-trimethylsilylethoxymethyl)pyrazino[2,3-b][1,4]oxazin-3-one (1689.6 mg, 4.69 mmol, 1 eq) in 1,4-dioxane (10 mL) was added copper(I) iodide (178.63 mg, 0.940 mmol, 0.200 eq), *N,N*-dimethyl-ethylenediamine (0.01 mL, 1.88 mmol, 0.400 eq) and potassium carbonate (1.3 g, 9.38 mmol, 2 eq). The mixture was degassed with nitrogen three times and stirred at 100 °C for 2 h. The reaction was filtered and the filtrate was concentrated to dryness. The resiude was diluted with EtOAc (50 mL). The organic layer was washed with water (30 mL) and brine (30 mL), dried over anhydrous Na₂SO₄, concentrated under reduced pressure to afford a crude product. The residue was purified by column gel eluted with (PE/EtOAc= 1/1, Rf= 0.5). The desired fractions were concentrated to dryness in vacuo to afford the title compound (1.5 g, 3.05 mmol, 64.9% yield) as a yellow solid. MS (ESP): m/z = 493.3 [M+H]⁺.

### Step 4:

### 3-[3-Oxo-4-(2-trimethylsilylethoxymethyl)pyrazino[2,3-b][1,4]oxazin-6-yl]-1-oxa-3-azaspiro[4.5] decane-2,8-dione

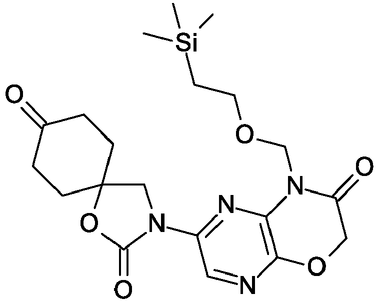

To a solution of 3-[3-oxo-4-(2-trimethylsilanyl-ethoxymethyl)-3,4-dihydro-2H-pyrazino[2 ,3-b][1,4]oxazin-6-yl]-1,9,12-trioxa-3-aza-dispiro[4.2.4.2]tetradecan- 2-one (500.0 mg, 1.02 mmol, 1 eq) in water (2 mL) was added TFA (2.0 mL). The mixture was stirreed at 25 °C for 24 h. The mixture was concentrated in vacuo. Then the residue was adjusted to pH 7 at 0 °C with aq. NaHCOs. The mixture was extracted with EtOAc (30 mL × 3). The combined organic layer was concentrated to give the title compound (380 mg, 0.850 mmol, 83.5% yield) as a yellow solid. MS (ESP): m/z = 449.2 [M+H]⁺.

### Intermediate 209

### 3-Fluoro-4-methyl-6,7-dihydro-5H-cyclopenta[b]pyridine-6-carbaldehyde

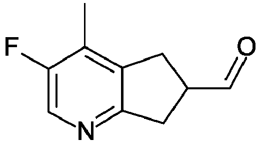

### Step 1:

### Methyl 2-chloro-5-fluoro-pyridine-3-carboxylate

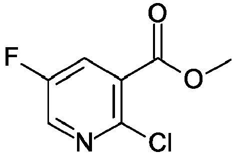

A solution of 2-chloro-5-fluoronicotinic acid (CAS#139911-30-1, 15000.0 mg, 85.45 mmol, 1 eq) in HCl-MeOH (150.0 mL, 4N) was stirred at 70 °C for 16 h. The mixture was concentrated to give crude title compound (16000 mg, 84.4 mmol, 98.8% yield) as a yellow oil. MS (ESP): m/z = 190.0 [M+H]⁺.

### Step 2:

### Methyl 5-fluoro-2-methyl-pyridine-3-carboxylate

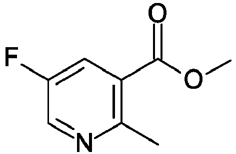

To a solution of methyl 2-chloro-5-fluoro-pyridine-3-carboxylate (14.0 g, 73.85 mmol, 1 eq) in 1,4-dioxane (140 mL) was added K₂CO₃ (30.57 g, 221.55 mmol, 3 eq), trimethylboroxine in THF (55.62 g, 221.55 mmol, 3 eq) and [1,1'-bis(diphenylphosphino)ferrocene]dichloropalladium (II) (5.4 g, 7.39 mmol, 0.100 eq). The mixture was stirred at 120 °C for 2 h under N₂. The mixture was diluted with EtOAc (30 mL) and filtered through celite; the filtrate was collected and concentrated to give a residue, which was purified by flash chromatography (10% PE/ EtOAc) to give the title compound (12000 mg, 70.94 mmol, 96.1% yield) as a yellow oil. MS (ESP): m/z = 170.1 [M+H]⁺.

### Step 3:

### 5-Fluoro-2-methyl-pyridine-3-carboxylic acid

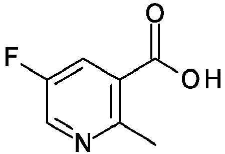

To a solution of methyl 5-fluoro-2-methyl-pyridine-3-carboxylate (13700.0 mg, 80.99 mmol, 1 eq) in methanol (97 mL) was added water (97 mL) and sodium hydroxide (3887.39 mg, 97.19 mmol, 1.2 eq). The mixture was stirred for 12 h at 25° C. The mixture was concentrated under reduced pressure to give a white solid. The solid was dissolved in water (20 mL). The aqueous layer was adjusted to pH 5 with HCl (1N) and then filtered. The filter cake was concentrated to give the title compound (10 g, 64.46 mmol, 79.6% yield) as a white solid. ¹H NMR (400 MHz, DMSO-*d*₆) δ 14.06 - 13.13 (m, 1H), 8.62 (d, *J* = 2.9 Hz, 1H), 8.00 (dd, *J* = 2.9, 9.0 Hz, 1H), 2.68 (d, *J* = 1.0 Hz, 3H).

### Step 4:

### 4-Chloro-5-fluoro-2-methyl-pyridine-3-carboxylic acid

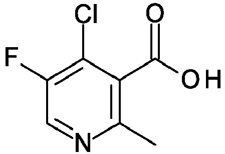

To a solution of n-BuLi (27.07 mL, 67.69 mmol, 3 eq) in THF (140 mL) was added bis(isopropyl)amine (9.49 mL, 67.69 mmol, 3 eq) at -75 °C and the mixture stirred for 1 h. Then 5-fluoro-2-methyl-pyridine-3-carboxylic acid (3.5 g, 22.56 mmol, 1 eq) in THF (35 mL) was added slowly at -75 °C. The mixture was stirred for 1 h. Then hexachloroethane (10.68 g, 45.12 mmol, 2 eq) was added slowly. The mixture was stirred at -75 °C for 4 h. This reaction was quenched with aq. NH₄Cl (20 mL) and concentrated. The residue was purified by prep-HPLC (formic acid system) to give the title compound (2200 mg, 11.61 mmol, 51.4% yield) as a yellow solid. MS (ESP): m/z = 190.1 [M+H]⁺.

### Step 5:

### Methyl 4-chloro-5-fluoro-2-methyl-pyridine-3-carboxylate

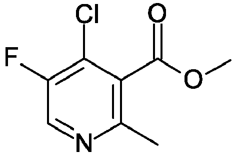

To a solution of 4-chloro-5-fluoro-2-methyl-pyridine-3-carboxylic acid (2.8 g, 14.77 mmol, 1 eq) in THF (56 mL) and methanol (5.6 mL) was added (trimethylsilyl)diazomethane 2.0 M in hexanes (14.77 mL, 29.54 mmol, 2 eq) at 0 °C. The mixture was stirred at 25 °C for 16 h. The mixture was diluted with water 15 mL and extracted with EtOAc (15 mL × 3). The combined EtOAc layer was concentrated and purified by column (PE/ EtOAc =6/1, Rf=0.2) to give the title compound (2200 mg, 10.81 mmol, 73.2% yield) as a yellow oil. MS (ESP): m/z = 204.1 [M+H]⁺.

### Step 6:

### (4-Chloro-5-fluoro-2-methyl-3-pyridyl) methanol

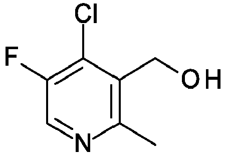

To a solution of methyl 4-chloro-5-fluoro-2-methyl-pyridine-3-carboxylate (2200.0 mg, 10.81 mmol, 1 eq) in THF (60 mL) was added LiAlH₄ in THF (21.61 mL, 21.61 mmol, 2 eq) at -78 °C, and then the solution was stirred at -30 °C for 4 h. The mixture was quenched with Na₂SO₄. 10H₂O and then filtered and the filtrate was concentrated to give the title compound (1500 mg, 8.54 mmol, 79.1% yield) as a yellow oil. MS (ESP): m/z = 176.1 [M+H]⁺.

### Step 7:

### (4-Chloro-5-fluoro-2-methyl-3-pyridyl) methyl acetate

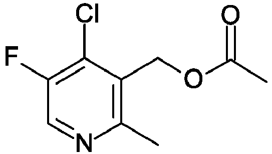

A solution of (4-chloro-5-fluoro-2-methyl-3-pyridyl)methanol (1500.0 mg, 8.54 mmol, 1 eq) in acetic anhydride (20.0 mL) was stirred at 70 °C for 12 h. The mixture was concentrated. The residue was diluted with EtOAc (50 mL) and washed with brine (50 mL). The organic layer was concentrated to give the title compound (1600 mg, 7.35 mmol, 86.1% yield) as a yellow oil. MS (ESP): m/z = 218.1 [M+H]⁺.

### Step 8:

### (4-Chloro-5-fluoro-2-methyl-1-oxido-pyridin-1-ium-3-yl) methyl propanoate

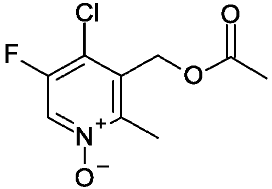

To a solution of (4-chloro-5-fluoro-2-methyl-3-pyridyl)methyl propanoate (1600.0 mg, 6.91 mmol, 1 eq) in DCM (40 mL) was added 3-chlorobenzoperoxoic acid (2376.0 mg, 13.81 mmol, 2 eq) at 0 °C. The mixture was stirred at 25 °C for 16 h. The mixture was washed with water (20 mL × 2). The organic layer was concentrated to give the title compound (1500 mg, 6.06 mmol, 87.7% yield) as a yellow solid.

### Step 9:

### [2-(Acetoxymethyl)-4-chloro-5-fluoro-3-pyridyl] methyl acetate

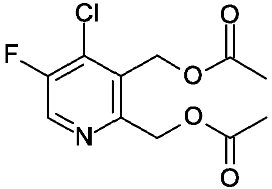

A solution of (4-chloro-5-fluoro-2-methyl-1-oxido-pyridin-1-ium-3-yl)methyl acetate (1500.0 mg, 6.42 mmol, 1 eq) in acetic anhydride (20.0 mL) was stirred at 70 °C for 12 h. The mixture was concentrated. The residue was purified by column (PE/ EtOAc =5/1) to give the title compound (1400 mg, 5.08 mmol, 79.1% yield) as a yellow oil. MS (ESP): m/z = 276.1 [M+H]⁺.

### Step 10:

### [2-(Acetoxymethyl)-5-fluoro-4-methyl-3-pyridyl] methyl acetate

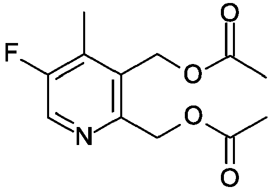

To a solution of [2-(acetoxymethyl)-4-chloro-5-fluoro-3-pyridyl]methyl acetate (1000.0 mg, 3.63 mmol, 1 eq) in 1,4-dioxane (10 mL) was added K₂CO₃ (1501.85 mg, 10.88 mmol, 3 eq), trimethylboroxine in THF (2732 mg, 10.88 mmol, 3 eq) and [1,1'-bis(diphenylphosphino) ferrocene]dichloropalladium(II) (265 mg, 0.360 mmol, 0.100 eq). The mixture was stirred at 100 °C for 2 h under N₂. The combined mixture was diluted with EtOAc (30 mL) and filtered through celite, the filtrate was collected and concentrated to give a residue, which was purified by flash chromatography (10% PE/ EtOAc) to give the title compound (660 mg, 2.58 mmol, 71% yield) as a yellow oil. MS (ESP): m/z = 256.1 [M+H]⁺.

### Step 11:

### [5-Fluoro-2-(hydroxymethyl)-4-methyl-3-pyridyl] methanol

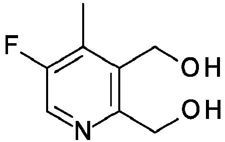

A mixture of [2-(acetoxymethyl)-5-fluoro-4-methyl-3-pyridyl]methyl acetate (900.0 mg, 3.53 mmol, 1 eq) and potassium carbonate (1461.98 mg, 10.58 mmol, 3 eq) in methanol (20 mL) was stirred at 25 °C for 4 h. The mixture was filtered and then filtrate was concentrated. The residue was diluted with water and extracted with EtOAc (20 mL × 2). The combined organic layer was concentrated. The residue was purified by column (PE/ EtOAc =1/1) to give the title compound (600 mg, 3.51 mmol, 99.4% yield) as a yellow oil. MS (ESP): m/z = 172.1 [M+H]⁺.

### Step 12:

### 2,3-Bis(chloromethyl)-5-fluoro-4-methyl-pyridine

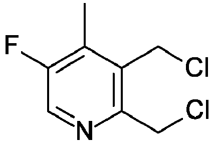

To a solution of [5-fluoro-2-(hydroxymethyl)-4-methyl-3-pyridyl]methanol (400.0 mg, 2.34 mmol, 1 eq) in DCM (16 mL) was added DMF (0.800 mL) and SOCl₂ (5561.7 mg, 46.7 mmol, 20 eq). The mixture was stirred at 25 °C for 12 h. The mixture was concentrated. The residue was diluted with EtOAc (20 mL) and poured onto ice water (20 mL). The mixture was adjusted to pH 7 with aq. NaHCOs, then extracted with EtOAc. The organic layer was washed with brine (20 mL). The organic layer was concentrated. The residue was purified by column (PE/ EtOAc =3/1) to give the title compound (380 mg, 1.83 mmol, 78.2% yield) as a yellow oil. MS (ESP): m/z = 208.1 [M+H]⁺.

### Step 13:

### Diethyl 3-fluoro-4-methyl-5,7-dihydrocyclopenta[b]pyridine-6,6-dicarboxylate

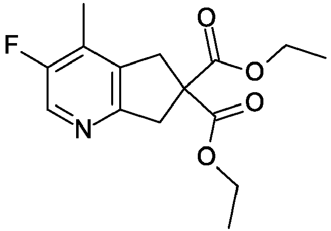

To a solution of diethyl malonate (0.31 mL, 2.01 mmol, 1.1 eq) in THF (32 mL) was added potassium tert-butoxide (450.88 mg, 4.02 mmol, 2.2 eq) under ice-water bath cooling and stirred for 1 h. To the mixture was added a solution of 2,3-bis(chloromethyl)-5-fluoro-4-methylpyridine (380.0 mg, 1.83 mmol, 1 eq) in THF (8 mL) under ice-water bath cooling. After addition, the reaction was heated to 70 °C and stirred for 16 h. The mixture was quenched with H₂O (20 mL) and extracted with EtOAc (20 mL × 2). The combined organic layer was washed with brine and concentrated to give a residue, which was purified by flash chromatography(15% PE in PE/ EtOAc) to give the title compound (300 mg, 1.25 mmol, 68.7% yield) as a yellow oil. MS (ESP): m/z = 296.2 [M+H]⁺.

### Step 14:

### Ethyl 3-fluoro-4-methyl-6,7-dihydro-5H-cyclopenta[b]pyridine-6-carboxylate

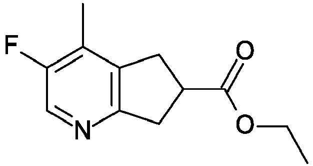

A solution of diethyl 3-fluoro-4-methyl-5,7-dihydrocyclopenta[b]pyridine-6,6-dicarboxylate (150.0 mg, 0.510 mmol, 1 eq) in HCl (6.0 mL) was stirred at 100 °C for 16 h. The solvent was removed by evaporation to give a residue, which was dissolved in toluene and concentrated. The residue was dissolved in ethanol (3.6 mL). To the mixture was added conc. H₂SO₄ (1.0 mL, 0.46 mmol, 1 eq). The mixture was stirred at 80 °C for 16 h. The solvent was removed by evaporation to give a residue, which was diluted with EtOAc (50 mL) and washed with aq.NaHCO₃ (30 mL × 2) and brine (30 mL). The organic layer was collected, dried and concentrated to give a residue, which was purified by flash chromatography (20% PE in PE/ EtOAc) to give the title compound (90 mg, 0.402 mmol, 78.7% yield) as a yellow oil. MS (ESP): m/z = 224.2 [M+H]⁺.

### Step 15:

### (3-Fluoro-4-methyl-6,7-dihydro-5H-cyclopenta[b]pyridin-6-yl)methanol

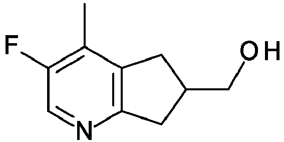

To a solution of ethyl 3-fluoro-4-methyl-6,7-dihydro-5*H*-cyclopenta[b]pyridine-6-carboxylate (50.0 mg, 0.220 mmol, 1 eq) in THF (2 mL) at -10 °C was added LiAlH₄ (17.04 mg, 0.450 mmol, 2 eq) and the mixture was stirred at -10 °C for 2 h. The reaction solution was quenched by adding H₂O (0.5 mL) and 10% aq. NaOH (0.5 mL). EtOAc (50 mL) was added and the mixture dried over anhydrous magnesium sulfate. The mixture was stirred at 20 °C for 10 min. The mixture was filtrated through filter membrane and the filtrate was collected and concentrated to give the crude product which was purified by flash chromatography (85% of EtOAc /PE) to give the title compound (40 mg, 0.220 mmol, 98.6% yield) as a white solid . MS (ESP): m/z = 182.1 [M+H]⁺.

### Step 16:

### 3-Fluoro-4-methyl-6,7-dihydro-5H-cyclopenta[b]pyridine-6-carbaldehyde

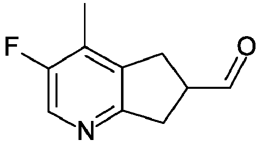

To a mixture of (3-fluoro-4-methyl-6,7-dihydro-5*H*-cyclopenta[b]pyridin-6-yl)methanol (40.0 mg, 0.220 mmol, 1 eq) in DCM (10 mL) was added Dess-Martin Periodinane (159.16 mg, 0.380 mmol, 1.7 eq) at 0 °C. The mixture was stirred at 25 °C for 4 h. The mixture was filtrated through a filter membrane and the filtrate was collected and concentrated to give the crude title compound (35 mg, 0.200 mmol, 88.5% yield) as a yellow oil.

### Intermediate 210

### 3-[2-Oxo-3-[3-oxo-4-(2-trimethylsilylethoxymethyl) pyrazino[2,3-b][1,4]oxazin-6-yl]oxazol-5-yl]propanal

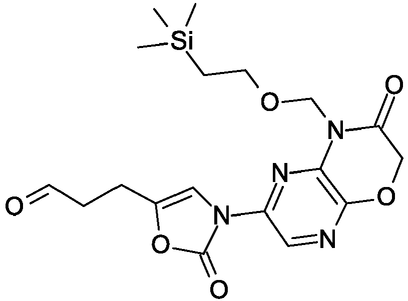

### Step 1:

### tert-Butyl-dimethyl-pent-4-ynoxy-silane

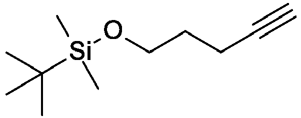

To a solution of 4-pentyn-1-ol (CAS# 5390-04-5, 3.0 g, 35.66 mmol, 1 eq), imidazole (3641.8 mg, 53.5 mmol, 1.5 eq) in DCM (30 mL) was added *tert*-butyldimethylchlorosilane (8.56 mL, 53.5 mmol, 1.5 eq) at 0 °C. Then the reaction was stirred at 25 °C for 2 h. The reaction mixture was diluted with H₂O (10 mL), and extracted with DCM (10 mL × 2). The combined organic layer was concentrated, and then the residue was purified by flash chromatography (20 g SiO₂, PE: EtOAC=20:1) to give the title compound (6 g, 30.24 mmol, 84.8% yield) as a colorless oil. ¹H NMR (400 MHz, CDCl₃) δ 3.70 (t, *J* = 6.1 Hz, 2H), 2.28 (dt, *J* = 2.6 Hz, 7.1 Hz, 2H), 1.93 (t, *J* = 2.7 Hz, 1H), 1.78 - 1.68 (m, 2H), 0.91 (s, 9H), 0.04 (s, 6H).

### Step 2:

### 5-Bromopent-4-ynoxy-tert-butyl-dimethyl-silane

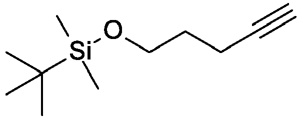

To a solution of tert-butyl-dimethyl-pent-4-ynoxy-silane (7000.0 mg, 35.29 mmol, 1 eq) and *N-*bromosuccinimide (6280.31 mg, 35.29 mmol, 1 eq) in acetone (70 mL) was added silver nitrate (325 mg, 1.91 mmol, 0.05 eq). The mixture was stirred at 30 °C for 12 h. The yelllow reaction mixture turned to grey and some grey solid was formed. The solvent was removed by evaporation to give a residue, to which was added petroleum ether (200 mL). The mixture was filtered and the filtrate was concentrated under vacuum. The residue was purified by silica column (PE) to give the title compound (6000 mg, 21.64 mmol, 61.3% yield) as a colorless oil.

### Step 3:

### tert-Butyl N-[3-oxo-4-(2-trimethylsilylethoxymethyl)pyrazino[2,3-b][1,4]oxazin-6-yl]carbamate

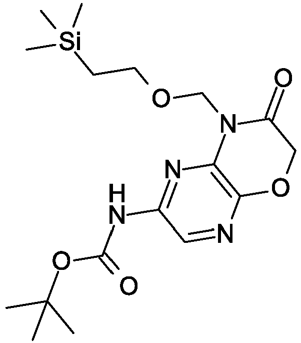

A solution of 9,9-dimethyl-4,5-bis(diphenylphosphino)xanthene (1356.26 mg, 2.34 mmol, 0.110 eq), tris(dibenzylideneacetone)dipalladium (0) (2074.28 mg, 2.27 mmol, 0.100 eq), *tert*-butyl carbamate (5201.67 mg, 44.4 mmol, 2 eq), 6-bromo-4-(2-trimethylsilylethoxymethyl)pyrazino [2,3-b][1,4]oxazin-3-one (Intermediate 25, 8000.0 mg, 22.2 mmol, 1 eq) and potassium carbonate (6143 mg, 44.45 mmol, 2 eq) in 1,4-dioxane (160 mL) was degassed with nitrogen 3 times and stirred at 100 °C for 12 h. The combined reaction mixture was filtered and the filtrate was concentrated. The residue was purified by flash chromatography (SiO₂ 80 g, PE/EtOAc=10/1) to give a crude product (24 g). The crude product was then purified by reversed phase column chromatography (CH₃CN, 0.5% formic acid in water) to give the title compound (5000 mg, 12.61 mmol, 56.8% yield) as a yellow solid. MS (ESP): m/z = 397.2 [M+H]⁺.

### Step 4:

### tert-Butyl N-[5-[tert-butyl(dimethyl)silyl]oxypent-1-ynyl]-N-[3-oxo-4-(2-trimethylsilylethoxymethyl)pyrazino[2,3-b][1,4]oxazin-6-yl]carbamate

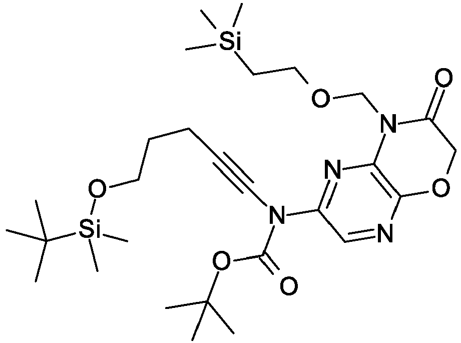

To a solution of *tert*-butyl *N*-[3-oxo-4-(2-trimethylsilylethoxymethyl)pyrazino[2,3-b][1,4] oxazin-6-yl]carbamate (1500.0 mg, 3.78 mmol, 1 eq) and 5-bromopent-4-ynoxy-*tert*-butyldimethyl-silane (1573.37 mg, 5.67 mmol, 1.5 eq) in 1,4-dioxane (30 mL) was added copper(II) sulfate (603.77 mg, 3.78 mmol, 1 eq), phosphoric acid, potassium salt (1927.21 mg, 9.08 mmol, 2.4 eq) and 3,4,7,8-tetramethyl-1,10-phenanthroline (357.58 mg, 1.51 mmol, 0.400 eq). The mixture was stirred at 85 °C for 12 h under N₂. The combined mixture was filtered and concentrated. The residue was purified by column (PE/ EtOAc =3/1) to give the title compound (400 mg, 0.67 mmol, 17.8% yield) as a colorless oil. MS (ESP): m/z = 593.4 [M+H]⁺.

### Step 5:

### 6-[5-[3-[tert-Butyl(dimethyl)silyl]oxypropyl]-2-oxo-oxazol-3-yl]-4-(2-trimethylsilylethoxy methyl)pyrazino[2,3-b][1,4]oxazin-3-one

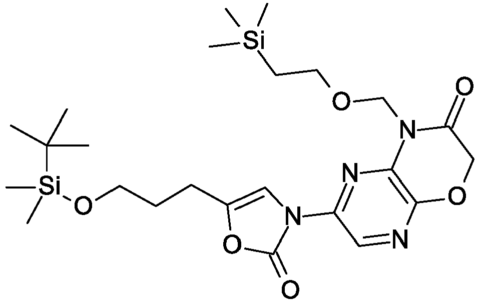

A mixture of *tert*-butyl *N*-[5-[*tert*-butyl(dimethyl)silyl]oxypent-1-ynyl]-*N*-[3-oxo-4-(2-trimethyl silylethoxymethyl)pyrazino[2,3-b][1,4]oxazin-6-yl]carbamate (1000.0 mg, 1.69 mmol, 1 eq) in DCM (49 mL) was added to a solution of silver hexafluoroantimonate(V) (CAS# 26042-64-8, 167.09 mg, 0.490 mmol, 0.290 eq) and chloro(triphenylphosphine)gold(I) (CAS# 14243-64-2, 250.63 mg, 0.510 mmol, 0.300 eq) in MeCN (4.87 mL). The mixture was stirred at 40 °C for 12 h. The mixture was concentrated in vacuum to give a crude. The crude was purified by column (PE: EtOAc =3:1) to give the title compound (750 mg, 1.4 mmol, 82.8% yield) as a yellow oil. MS (ESP): m/z = 537.1 [M+H]⁺.

### Step 6:

### 6-[5-(3-Hydroxypropyl)-2-oxo-oxazol-3-yl]-4-(2-trimethylsilylethoxymethyl)pyrazino[2,3-b][1,4]oxazin-3-one

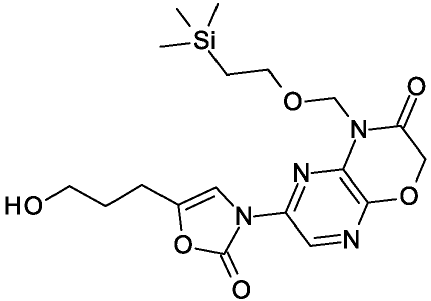

To a mixture of 6-[5-[3-[*tert*-butyl(dimethyl)silyl]oxypropyl]-2-oxo-oxazol-3-yl]-4-(2-trimethyl silylethoxymethyl)pyrazino[2,3-b][1,4]oxazin-3-one (750.0 mg, 1.4 mmol, 1 eq) in THF (10 mL) was added hydrochloric acid (10.0 mL, 10 mmol, 7.16 eq). Then the solution was stirred at 25 °C for 16 h. The solution was concentrated under vacuum to give the crude product. The combined crude product was purified by flash chromatography (20 g SiO₂, PE: EtOAc =1:1) to give the title compound (550 mg, 1.3 mmol, 74.5% yield) as a yellow solid. MS (ESP): m/z = 445.2 [M+Na]⁺.

### Step 7

### 3-[2-Oxo-3-[3-oxo-4-(2-trimethylsilylethoxymethyl)pyrazino[2,3-b] [1,4]oxazin-6-yl]oxazol-5-yl]propanal

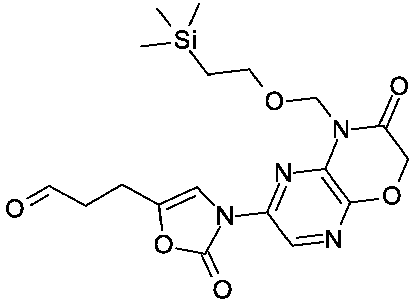

To a mixture of 6-[5-(3-hydroxypropyl)-2-oxo-oxazol-3-yl]-4-(2-trimethylsilylethoxymethyl) pyrazino[2,3-b][1,4]oxazin-3-one (200.0 mg, 0.470 mmol, 1 eq) in DCM (8 mL) was added Dess-Martin Periodinane (301.16 mg, 0.710 mmol, 1.5 eq) at 0 °C. The mixture was stirred at 25 °C for 2 h. The mixture was filtrated through filter membrane and the filtrate was collected and concentrated to give the crude product. The crude product was purified by prep-TLC (PE/ EtOAc =1/1) to give the title compound (150 mg, 0.360 mmol, 75.4% yield) as a colorless oil. ¹H NMR (400MHz, CDCl₃) δ 9.85 (s, 1H), 8.83 (s, 1H), 7.16 (s, 1H), 5.59 - 5.48 (m, 2H), 4.96 (s, 2H), 3.78 - 3.68 (m, 2H), 2.92 - 2.80 (m, 4H), 1.02 - 0.91 (m, 2H), 0.01 (s, 9H).

### Intermediate 211

### 4-Fluoro-6-[2-(2-oxooxazolidin-3-yl)ethoxy] indane-2-carbaldehyde

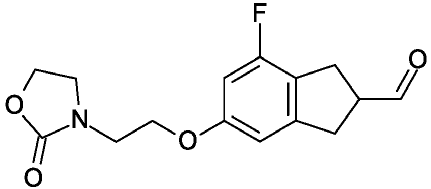

### Step 1:

### 3-[2-[2-[[tert-Butyl(dimethyl)silyl]oxymethyl]-7-fluoro-indan-5-yl]oxyethyl]oxazolidin-2-on

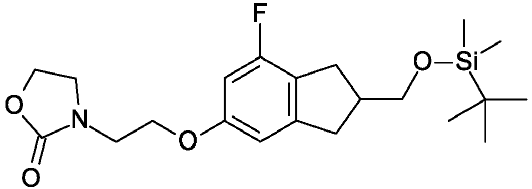

To a solution of [6-(2-bromoethoxy)-4-fluoro-indan-2-yl]methoxy-tert-butyl-dimethyl-silane (Intermediate 217, 250.0 mg, 0.620 mmol, 1 eq) in DMF (1 mL) was added potassium carbonate (342.6 mg, 2.48 mmol, 4 eq) and 2-oxazolidone (16.19 mg, 0.190 mmol, 1.5 eq). The solution was stirred at 70 °C for 16 h. The mixture was filtered and the filtrate was concentrated under reduced pressure to give the crude product which was purified by chromatography on silica gel (petroleum ether : ethyl acetate = 5 : 1) to give the title compound (150 mg, 0.370 mmol, 59.1% yield) as a colorless oil. MS (ESP): m/z = 410.2 [M+H]⁺.

### Step 2:

### 3-[2-[7-Fluoro-2-(hydroxymethyl)indan-5-yl]oxyethyl]oxazolidin-2-one

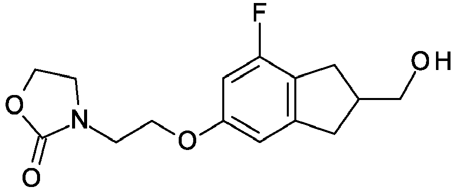

To a solution of 3-[2-[2-[[*tert*-butyl(dimethyl)silyl]oxymethyl]-7-fluoro-indan-5-yl] oxyethyl]oxazolidin-2-one (150.0 mg, 0.370 mmol, 1 eq) in THF (4.83 mL) was added TBAF in THF (0.7 mL, 0.730 mmol, 2 eq), and the solution was stirred at 25 °C for 16 h. The solvent was removed under reduced pressure to give the crude, which was purified by chromatography on silica gel (petroleum ether : ethyl acetate =1:1) to give the title compound (100 mg, 0.340 mmol, 92.5% yield) as a yellow oil. MS (ESP): m/z = 296.1 [M+H]⁺.

### Step 3:

### 4-Fluoro-6-[2-(2-oxooxazolidin-3-yl)ethoxy]indane-2-carbaldehyde

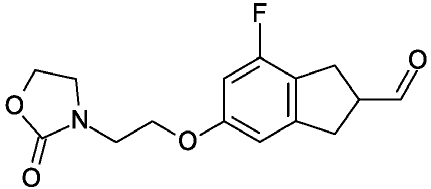

To a solution of 3-[2-[7-fluoro-2-(hydroxymethyl)indan-5-yl]oxyethyl]oxazolidin-2-one (100.0 mg, 0.340 mmol, 1 eq) in DCM (30 mL) was added Dess-Martin Periodinane (449.88 mg, 1.06 mmol, 1.2 eq) at 0 °C. Then the mixture was stirred at 25 °C for 2 h. The mixture was filtered, then the filtrate was collected and concentrated to give the crude product, which was purified by Prep-TLC(petroleum ether : ethyl acetate = 1:2) to give the title compound (250 mg, 0.740 mmol, 83.8% yield) as a colorless oil.

### Intermediate 212

### 2-(7-Fluoro-2-formyl-indan-5-yl)oxyacetamide

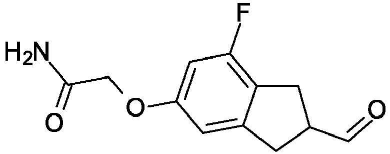

### Step 1:

### 2-[2-[[tert-Butyl(dimethyl)silyl]oxymethyl]-7-fluoro-indan-5-yl]oxyacetamide

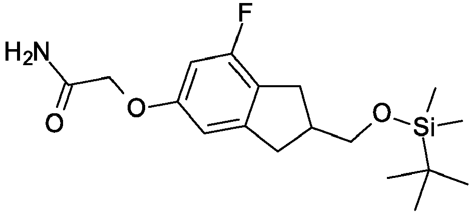

To a mixture of 2-[[*tert*-butyl(dimethyl)silyl]oxymethyl]-7-fluoro-indan-5-ol (Intermediate 204, 200.0 mg, 0.670 mmol, 1 eq), potassium carbonate (186.48 mg, 1.35 mmol, 2 eq) and potassium iodide (0.07 mL, 1.35 mmol, 2 eq) in MeCN (12 mL) was added 2-chloroacetamide (CAS#79-07-2, 0.13 mL, 1.35 mmol, 2 eq) and the mixture was stirred at 70 °C for 16 h. The solution was filtered and the filtrate was concentrated under reduced pressure to give a crude product. The crude product was purified by Prep-TLC (petroleum ether: ethyl acetate =1:1) to give the title compound (220 mg, 0.620 mmol, 92.3% yield) as a white solid. MS (ESP): m/z = 354.0 [M+H]⁺.

### Step 2:

### 2-[7-Fluoro-2-(hydroxymethyl)indan-5-yl]oxyacetamide

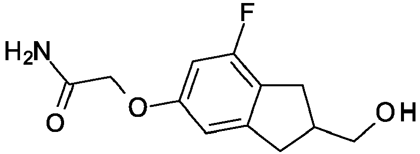

To a solution of 2-[2-[[*tert*-butyl(dimethyl)silyl]oxymethyl]-7-fluoro-indan-5-yl]oxyacetamide (220.0 mg, 0.620 mmol, 1 eq) in THF (8.25 mL) was added TBAF in THF (1.24 mL, 1.24 mmol, 2 eq), and then the solution was stirred at 25 °C for 16 h. The solvent was removed under reduced pressure to give the crude, which was purified by Prep-TLC (DCM: methanol =1:1) to give the title compound (140 mg, 0.590 mmol, 94.0% yield) as a white solid. MS (ESP): m/z = 240.2 [M+H]⁺.

### Step 3:

### 2-(7-Fluoro-2-formyl-indan-5-yl)oxyacetamide

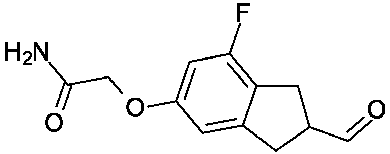

To a solution of 2-[7-fluoro-2-(hydroxymethyl)indan-5-yl]oxyacetamide (70.0 mg, 0.290 mmol, 1 eq) in DCM (30 mL) was added Dess-Martin Periodinane (449.88 mg, 1.06 mmol, 1.2 eq) at 0 °C . Then the mixture was stirred at 25 °C for 2 h. To the mixture was added sodium bicarbonate solution, which was extracted with DCM (20 mL × 2). Then the combined organic layers were dried over sodium sulphate anhydrous and filtered, the filtrate was collected and concentrated under reduced pressure to give the crude title compound (112 mg, 0.470 mmol, 96.8% yield) as a white solid.

### Intermediate 213

### 6-[2-(Azetidin-1-yl)-2-oxo-ethoxy]-4-fluoro-indane-2-carbaldehyde

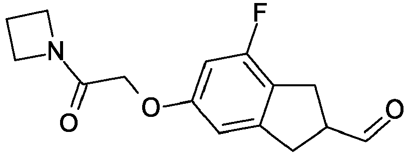

The title compound was prepared by analogy to 2-(7-fluoro-2-formyl-indan-5-yl)oxyacetamide (Intermediate 212) using 1-(azetidin-1-yl)-2-chloro-ethanone in place of 2-chloroacetamide and was obtained as a dark brown oil.

The 1-(azetidin-1-yl)-2-chloro-ethanone used as a starting material was prepared as follows:
To a mixture of potassium carbonate (8124.6 mg, 58.8 mmol, 2.2 eq) in water (28.7 mL) and DCM (28.7 mL) was added azetidine hydrochloride (CAS#36520-39-5, 2.5 g, 26.7 mmol, 1 eq), then the mixture was stirred for 30 min at 25 °C. After 30 min, the solution was cooled to 0 °C, then a solution of chloroacetyl chloride (CAS#79-07-2, 3621.42 mg, 32.06 mmol, 1.2 eq) in DCM (25 mL) was added over 15 min and the mixture stirred for a further 2 h at 25 °C. After 2 h, the solution was poured into water (30 mL) and extracted with DCM (10 mL × 2), combined with the organics and dried by Na₂SO₄. The solvent was removed under reduced pressure to give the crude, which was purified by flash chromatography on silica gel (Petroleum ether : Ethyl acetate =1:1, 12 g silica gel) to give 1-(azetidin-1-yl)-2-chloro-ethanone (1.8 g, 13.48 mmol, 50.4% yield) as a colorless oil. ¹H NMR (400 MHz, DMSO-*d*₆) δ 4.19 (t, *J* = 7.7 Hz, 2H), 4.09 (s, 2H), 3.90 (t, *J* = 7.8 Hz, 2H), 2.22 (s, 2H).

### Intermediate 214

### N-Ethyl-2-(7-fluoro-2-formyl-indan-5-yl)oxy-acetamide

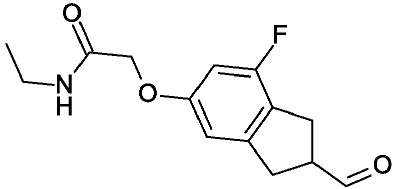

The title compound was prepared by analogy to 2-(7-fluoro-2-formyl-indan-5-yl)oxyacetamide (Intermediate 212) using 2-chloro-N-ethyl-acetamide (CAS# 105-35-1) in place of 2-chloroacetamide and was obtained as a colorless oil.

### Intermediate 215

### 2-[2-[[tert-Butoxycarbonyl-[2-[2-oxo-3-(3-oxo-4H-pyrazino[2,3-b] [1,4]oxazin-6-yl)oxazolidin-5-yl] ethyl] amino] methyl] -7-fluoro-indan-5-yl] oxyacetic acid

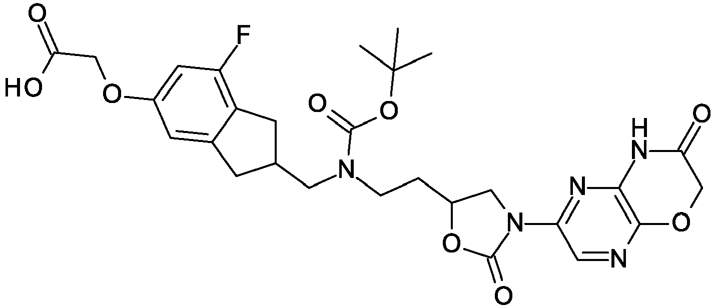

### Step 1:

### Benzyl 2-[2-[[tert-butyl(dimethyl)silyl]oxymethyl]-7-fluoro-indan-5-yl]oxyacetate

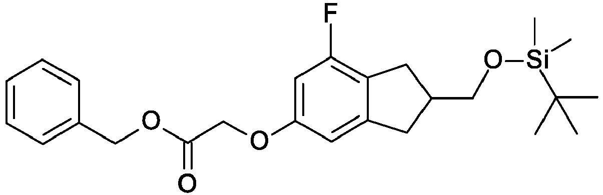

To a solution of 2-[[*tert*-butyl(dimethyl)silyl]oxymethyl]-7-fluoro-indan-5-ol (Intermediate 204, 500.0 mg, 1.69 mmol, 1 eq) and benzyl 2-bromoacetate (CAS#5437-45-6, 579.53 mg, 2.53 mmol, 1.5 eq) in MeCN (10 mL) was added potassium carbonate (466.2 mg, 3.37 mmol, 2 eq), and then the solution was stirred at 70 °C for 16 h. The mixture was filtered off and washed with ethyl acetate(50 mL), then the filtrate was concentrated to give a crude, which was purified by chromatography on silica gel (4g) (Petroleum ether : Ethyl acetate =20:1) to give the title compound (718 mg, 1.61 mmol, 95.8% yield) as a colorless oil. ¹H NMR (400 MHz, DMSO-*d*₆) δ 7.40-7.33 (m, 5H), 6.64 - 6.60 (m, 1H), 6.57 - 6.52 (m, 1H), 5.16 (s, 2H), 4.80 (s, 2H), 3.55 - 3.48 (m, 2H), 2.94 - 2.81 (m, 2H), 2.67 - 2.58 (m, 3H), 0.83 (s, 9H), 0.02 (m, 6H).

### Step 2:

### Benzyl 2-[7-fluoro-2-(hydroxymethyl)indan-5-yl]oxyacetate

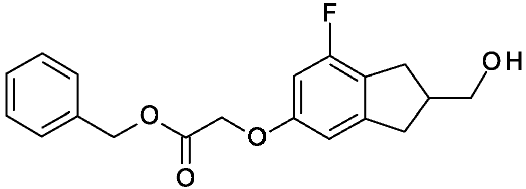

To a solution of benzyl 2-[2-[[*tert*-butyl(dimethyl)silyl]oxymethyl]-7-fluoro-indan-5-yl] oxyacetate (200.0 mg, 0.450 mmol, 1 eq) in THF (10 mL) was added concentrated hydrochloric acid (0.05 mL, 0.050 mmol, 0.110 eq) at 25 °C for 0.5 h. After 0.5 h, to the solution was added water (20mL) and the pH of the solution was adjusted to around 7 by progressively adding sodium bicarbonate, then the solution was extracted with ethyl acetate (30 mL × 3) and the organic layer was dried with anhydrous sodium sulfate. The solvent was evaporated to give the crude, which was purified by flash chromatography silica gel (4 g; Petroleum ether : Ethyl acetate =10:1) to give the title compound (112 mg, 0.340 mmol, 75.4% yield) as a colorless oil.

### Step 3:

### Benzyl 2-(7-fluoro-2-formyl-indan-5-yl)oxyacetate

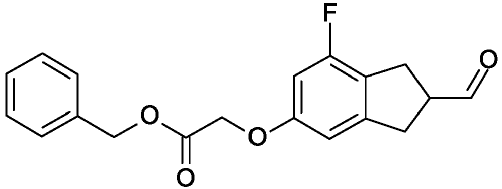

To a solution of benzyl 2-[7-fluoro-2-(hydroxymethyl)indan-5-yl]oxyacetate (202.0 mg, 0.610 mmol, 1 eq) in DCM (30 mL) was added Dess-Martin Periodinane (449.88 mg, 1.06 mmol, 1.2 eq) at 0 °C . Then the mixture was stirred at 25 °C for 2 h. The mixture was dried under reduced pressure to give the crude, which was purified by Prep-TLC (Petroleum ether : Ethyl acetate =1:2) to give the title compound (116 mg, 0.350 mmol, 57.8% yield) as a colorless oil.

### Step 4:

### Benzyl 2-[7 - fluoro-2-[[2-[2-oxo-3-(3-oxo-4H-pyrazino[2,3-b][1,4]oxazin-6-yl)oxazolidin-5-yl] ethylamino] methyl] indan-5-yl] oxyacetate

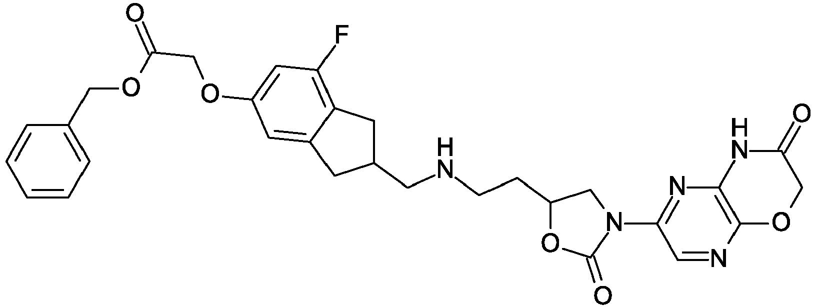

To a solution of 6-[5-(2-aminoethyl)-2-oxo-oxazolidin-3-yl]-4*H*-pyrazino[2,3-b][1,4]oxazin-3-one (Intermediate 24, 108.5 mg, 0.390 mmol, 1.1 eq) in THF (23.94 mL) and 2-propanol (23.9 mL) was added triethylamine (0.07 mL, 0.530 mmol, 1.5 eq) and the mixture stirred at 25°C for 10 min, then was added benzyl 2-(7-fluoro-2-formyl-indan-5-yl)oxyacetate (116.0 mg, 0.350 mmol, 1 eq) and the mixture stirred at 25 °C for 1h. Sodium cyanoborohydride (88.81 mg, 1.41 mmol, 4 eq) was added to the mixture at 0 °C. Then the mixture was stirred at 25 °C for another 16 h. Half of the solvent was evaporated and the mixture was extracted with ethyl acetate (40 mL × 3), the combined extracts were dried over Na₂SO₄ and the solvent was evaporated to give the crude title compound (190 mg, 0.320 mmol, 90.9% yield). MS (ESP): m/z = 592.2 [M+H]⁺.

### Step 5:

### Benzyl 2-[2-[[tert-butoxycarbonyl-[2-[2-oxo-3-(3-oxo-4H-pyrazino[2,3-b] [1,4]oxazin-6-yl)oxazolidin-5-yl] ethyl] amino] methyl] -7-fluoro-indan-5-yl] oxyacetate

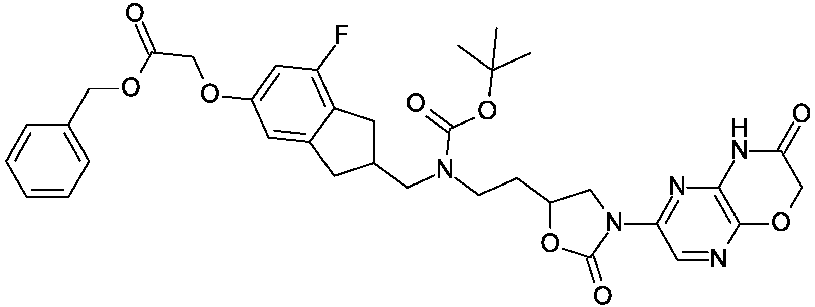

To a solution of benzyl 2-[7-fluoro-2-[[2-[2-oxo-3-(3-oxo-4*H*-pyrazino[2,3-b][1,4]oxazin-6-yl)oxazolidin-5-yl]ethylamino]methyl]indan-5-yl]oxyacetate (190.0 mg, 0.320 mmol, 1 eq) in THF (10 mL) was added triethylamine (0.09 mL, 0.640 mmol, 2 eq) and di-*tert-*butyldicarbonate (105.14 mg, 0.480 mmol, 1.5 eq). The solution was stirred at 25 °C for 16 h. The solvent was evaporated to give a crude, which was purified by Prep-TLC (DCM: Methanol =10:1) to give the title compound (84 mg, 0.120 mmol, 37.8% yield) as a colorless oil. MS (ESP): m/z = 692.1 [M+H]⁺.

### Step 6:

### 2-[2-[[tert-Butoxycarbonyl-[2-[2-oxo-3-(3-oxo-4H-pyrazino[2,3-b][1,4]oxazin-6-yl)oxazolidin-5-yl] ethyl] amino] methyl] -7-fluoro-indan-5-yl] oxyacetic acid

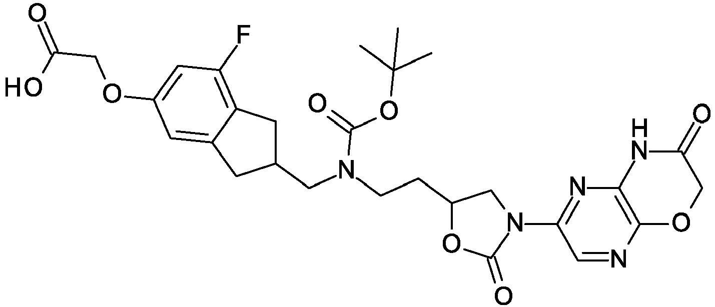

To a solution of benzyl 2-[2-[[*tert*-butoxycarbonyl-[2-[2-oxo-3-(3-oxo-4*H*-pyrazino[2,3-b][1,4]oxazin-6-yl)oxazolidin-5-yl]ethyl]amino]methyl]-7-fluoro-indan-5-yl]oxyacetate (84.0 mg, 0.120 mmol, 1 eq) in THF (20 mL) was added Pd/C (10.0 mg) under hydrogen, then the reaction was stirred at 25 °C for 1 h. The mixture was filtered, then the solvent was evaporated under reduced pressure to give the crude title compound (70 mg, 0.120 mmol, 95.8% yield) as a colorless solid. MS (ESP): m/z = 546.1 [M +H-Bu]⁺.

### Intermediate 216

### 2-(4,7-Difluoro-2-formyl-indan-5-yl)oxyacetamide

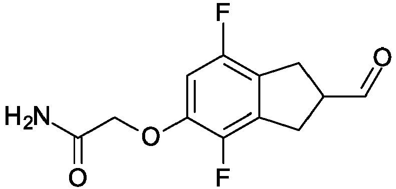

### Step 1:

### Dimethyl 3,6-difluorobenzene-1,2-dicarboxylate

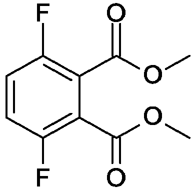

To a solution of 3,6-difluorophthalic acid (CAS# 651-97-8, 20000.0 mg, 98.96 mmol, 1 eq) in methanol (600 mL) was added conc. H₂SO₄ (20.0 mL, 98.96 mmol, 1 eq). The mixture was stirred at 80 °C for 16 h. The solvent was removed by evaporation to give a residue, which was diluted with EtOAc (200 mL) and the pH was adjusted to around 8 by addition of aq. NaHCO₃, and the mixture was extracted with EtOAc (3 × 200 mL). The organic layer was washed with brine (600 mL). The organic layer was dried and concentrated to give the crude title compound (18.7 g, 81.25 mmol, 78% yield) as a light yellow solid, which was used directly without further purification . ¹H NMR (400 MHz, CDCl₃) δ 7.30 - 7.23 (m, 2H), 3.96 (s, 6H).

### Step 2:

### [3,6-Difluoro-2-(hydroxymethyl)phenyl]methanol

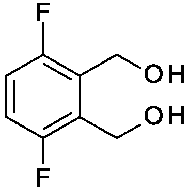

To a solution of dimethyl 3,6-difluorobenzene-1,2-dicarboxylate (18700 mg, 81.25 mmol, 1 eq) in THF (600 mL) was added lithium aluminum hydride (7400.89 mg, 195 mmol, 2.4 eq) at -15 °C, and then the solution was stirred at -15 °C for 2 h. The reaction was quenched with H₂O (7 mL). 10% aq. NaOH (14 mL), EtOAc (200 mL) and anhyrous magnesium sulfate (20 g) were added. The resulting mixture was filtered and the filtrate was collected and concentrated to give the title compound (14000 mg, 80.4 mmol, 94% yield) as a light brown solid which was used directly without further purification. ¹H NMR (400 MHz, CDCl₃) δ 7.07 - 6.99 (m, 2H), 4.80 (s, 4H).

### Steps 3 to 5:

### Ethyl 4,7-difluoroindane-2-carboxylate

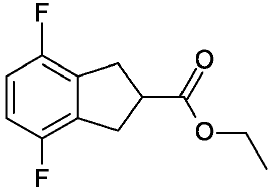

The title compound was prepared by analogy with ethyl 3-fluoro-4-methyl-6,7-dihydro-5*H-*cyclopenta[b]pyridine-6-carboxylate (Intermediate 209, Steps 12 to 14) and was obtained as a colorless oil. ¹H NMR (400 MHz, CDCl₃) δ 6.86-6.80 (m, 2H), 4.22 (q, *J* = 7.1 Hz, 2H), 3.48 - 3.37 (m, 1H), 3.34 - 3.27 (m, 4H), 1.31 (t, *J* = 7.2 Hz, 3H).

### Step 6:

### Ethyl 4,7-difluoro-5-hydroxy-indane-2-carboxylate

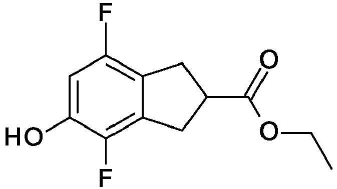

To a stirred mixture of 4,4,5,5-tetramethyl-1,3,2-dioxaborolane (4978 mg, 38.9 mmol, 4 eq) and 3,4,7,8-tetramethyl-1,10-phenanthroline (91.9 mg, 0.390 mmol, 0.040 eq) was added methoxy(cyclooctadiene)iridium(I) dimer (128.9 mg, 0.190 mmol, 0.020 eq) under N₂ atmosphere and the mixture was stirred for further 5 min, then ethyl 4,7-difluoroindane-2-carboxylate (2.2 g, 9.73 mmol, 1 eq) was added and the mixture was stirred at 80 °C for 16 h. The reaction mixture was cooled to room temperature, then the mixture was taken up in EtOAc (20 mL) and the catalyst was filtered off and washed with brine (10 mL), then the solvent was removed and the residue taken up in THF (30 mL), then a solution of sodium perborate monohydrate (2912 mg, 29.18 mmol, 3 eq) in water (50 mL) was added. The reaction was stirred for 30 min. The solution was poured into brine (50 mL) and extracted with EtOAc (100 mL × 2), combined with the organics and dried by Na₂SO₄. The solvent was removed to give the crude, which was purified by flash chromatography on silica gel (25 g) (PE: EtOAc =5:1) to give the title compound (1100 mg, 4.54 mmol, 46.7% yield) as a brown solid.

### Steps 7 to 8:

### (5-Benzyloxy-4,7-difluoro-indan-2-yl)methanol

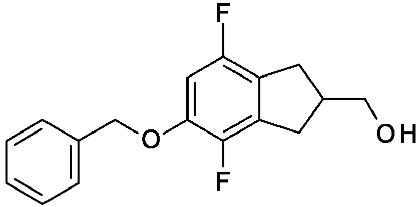

The title compound was prepared by analogy with (6-benzyloxy-4-fluoro-indan-2-yl)methanol (Intermediate 50, Steps 4 to 5) and was obtained as a white solid. ¹H NMR (400 MHz, CDCl₃) δ 7.40 - 7.19 (m, 5H), 6.47 (dd, *J* = 6.1, 9.6 Hz, 1H), 5.01 (s, 2H), 3.58 (d, *J* = 6.3 Hz, 2H), 3.09 - 2.92 (m, 2H), 2.76 - 2.58 (m, 3H).

### Steps 9 to 10:

### 2-[[tert-Butyl(dimethyl)silyl]oxymethyl]-4,7-difluoro-indan-5-ol

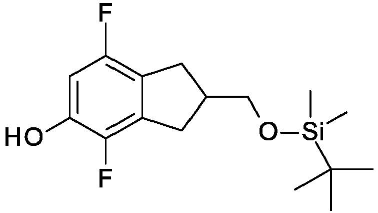

The title compound was prepared by analogy with 2-[[*tert*-butyl(dimethyl)silyl]oxymethyl]-7-fluoro-2,3-dihydro-1*H*-inden-5-ol (Intermediate 52, Steps 1 to 2) and was obtained as a colorless oil.

### Steps 11 to 13:

### 2-(4,7-Difluoro-2-formyl-indan-5-yl)oxyacetamide

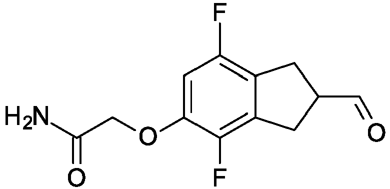

The title compound was prepared by analogy with 2-(7-fluoro-2-formyl-indan-5-yl)oxyacetamide (Intermediate 212, Steps 1 to 3) and was obtained as a light grey solid.

### Intermediate 217

### [6-(2-Bromoethoxy)-4-fluoro-indan-2-yl]methoxy-tert-butyl-dimethyl-silane

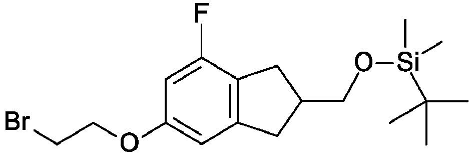

To a solution of 2-[[*tert*-butyl(dimethyl)silyl]oxymethyl]-7-fluoro-indan-5-ol (Intermediate 204, 400.0 mg, 1.35 mmol, 1 eq) and potassium carbonate (1117.22 mg, 8.1 mmol, 6 eq) in acetone (20 mL) was added 1,2-dibromoethane (CAS#106-93-4, 1267.4 mg, 6.75 mmol, 5 eq), then the solution was stirred at 70 °C for 16 h. Then to the reaction mixture was added 1,2-dibromoethane (760.44 mg, 4.05 mmol, 3 eq) and potassium carbonate (744.8 mg, 5.4 mmol, 4 eq) and the mixture stirred at 70 °C for another 32 h. The solid was filtered off and washed with ethyl acetate (20 mL), then the filtrate was concentrated to give a crude, which was purified by chromatography on silica gel (petroleum ether : ethyl acetate = 20 : 1) to give the title compound (400 mg, 0.990 mmol, 73.5% yield) as a colorless oil.

### Intermediates 218, 219, 220 and 221

### 6-[5-(Oxiran-2-ylmethyl)-2-oxo-oxazolidin-3-yl]-4-(2-trimethylsilylethoxymethyl)pyrazino[2,3-b][1,4]oxazin-3-one, Isomers A, B, C, D

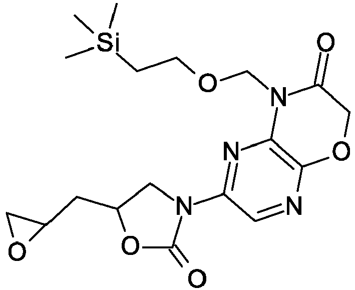

### Step 1:

### 6-(5-Allyl-2-oxo-oxazolidin-3-yl)-4-(2-trimethylsilylethoxymethyl)pyrazino[2,3-b][1,4]oxazin-3-one

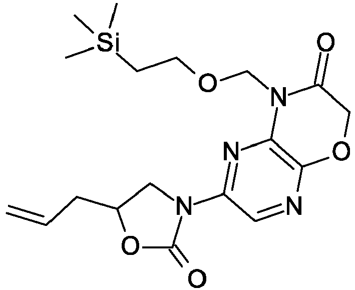

To a solution of 6-bromo-4-(2-trimethylsilylethoxymethyl)pyrazino[2,3-b][1,4]oxazin-3-one (Intermediate 25, 2833.7 mg, 7.87 mmol, 1 eq) and 5-allyloxazolidin-2-one (CAS# 50825-10-0, 1000.0 mg, 7.87 mmol, 1 eq) in 1,4-dioxane (50 mL) was added copper(I) iodide (449.4 mg, 2.36 mmol, 0.300 eq), trans-N,N'- dimethylcyclohexane-1,2-diamine (671.4 mg, 4.72 mmol, 0.600 eq) and potassium carbonate (3261.1 mg, 23.6 mmol, 3 eq). The mixture was stirred at 100 °C for 3 h. The mixture was concentrated to give a residue. The residue was diluted with EtOAc (50 mL) and washed with brine (30 mL × 2). The organic phase was dried over Na₂SO₄, concentrated to give the crude product (6 g). The crude was purified by flash column chromatography (PE/EtOAc=10/1 to 3/1) and concentrated to give the title compound (1700 mg, 4.18 mmol, 53.2% yield) as a light yellow solid. MS (ESP): m/z = 409.2 [M+Na] ⁺.

### Step 2:

### 6-[5-(Oxiran-2-ylmethyl)-2-oxo-oxazolidin-3-yl]-4-(2-trimethylsilylethoxymethyl)pyrazino[2,3-b][1,4]oxazin-3-one, Isomers A, B, C and D

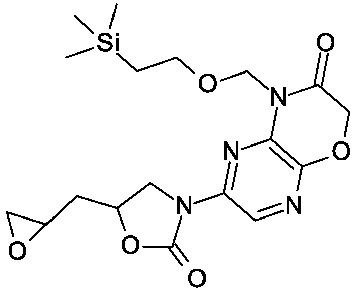

To a solution of 6-(5-allyl-2-oxo-oxazolidin-3-yl)-4-(2-trimethylsilylethoxymethyl)pyrazino[2,3-b][1,4]oxazin-3-one (1000 mg, 2.46 mmol, 1 eq) in DCM (15 mL) under ice-water bath cooling was added *m*-CPBA (1000 mg, 4.92 mmol, 2 eq) in batches. Then the reaction was stirred at 20 °C for 72 h. The mixture was cooled to 0 °C and a lot of precipitate appeared. The mixture was filtered and the filtrate was concentrated under vacuum to give a residue. The residue was purified by flash column chromatography (PE/EtOAc=3/1 to 1/2) and concentrated to give the title compound (300 mg, 0.710 mmol, 28.9% yield) as a white solid. MS (ESP): m/z = 423.3 [M+H]⁺.

Racemic 6-[5-(oxiran-2-ylmethyl)-2-oxo-oxazolidin-3-yl]-4-(2-trimethylsilyl ethoxymethyl)pyrazino[2,3-b][1,4]oxazin-3-one (200.0 mg, 0.47 mmol, 1 eq) was separated by chiral SFC to give four isomers:
Peak 1: (Isomer A, Intermediate 218, 30 mg, 0.070 mmol, 15% yield) as a light brown semisolid,
Peak 2: (Isomer B, Intermediate 219, 30 mg, 0.070 mmol, 15% yield) as a light brown semisolid,
Peak 3: (Isomer C, Intermediate 220, 30 mg, 0.070 mmol, 15% yield) as a white semisolid,
Peak 4: (Isomer D, Intermediate 221, 30 mg, 0.070 mmol, 15% yield) as a white semisolid.

### Intermediate 222

### 6-Benzyloxy-4-fluoro-indane-2-carbaldehyde

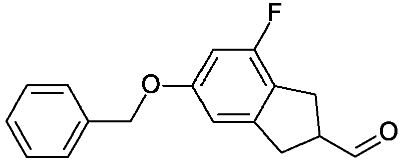

The title compound was prepared from (6-benzyloxy-4-fluoro-indan-2-yl)methanol (Intermediate 50, Step 5) by analogy with 2-formyl-2,3-dihydro-1*H*-indene-4-carbonitrile (Intermediate 5, Step 3) and was obtained as a white semi-solid. The crude product was used for the next step directly.

### Intermediate 223

### 4-Fluoro-6,7-dihydro-5H-cyclopenta[b]pyridine-6-carbaldehyde

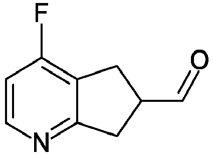

### Step 1:

### 2-chloro-4-fluoro-pyridine-3-carboxylate

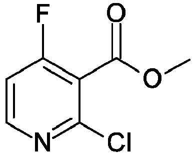

To a solution of 2-chloro-4-fluoronicotinic acid (CAS#1448155-98-3, 1.0 g, 5.7 mmol, 1 eq) in DCM (20 mL) and methanol (4 mL) at 0 °C was added trimethylsilyl diazomethane (hexanes) (4.27 mL, 8.55 mmol, 1.5 eq) drop-wise, the mixture was stirred for 1 h. The mixture was concentrated in vacuum and the residual crude product was purified by flash chromatography (ethyl acetate/petroleum ether=10/1) to afford the title compound (1.06 g, 5.59 mmol, 98.2% yield) as a colorless oil. ¹H NMR (400 MHz, CDCl₃) δ 8.29 (dd, *J* = 4.0, 8.0 Hz, 1H), 6.95 (dd, *J* = 4.0, 8.0 Hz, 1H), 3.85 (s, 3H).

### Step 2:

### 4-Fluoro-2-methyl-pyridine-3-carboxylate

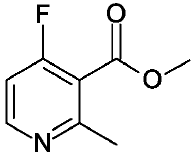

To a solution of methyl 2-chloro-4-fluoro-pyridine-3-carboxylate (5.0 g, 26.38 mmol, 1 eq) and trimethylboroxine (9.3 g, 36.93 mmol, 1.4 eq) in 1,4-dioxane (150 mL) was added [1,1'-bis(diphenylphosphino)ferrocene]dichloropalladium(II) (3.8 g, 5.28 mmol, 0.200 eq) and potassium carbonate (10.9 g, 79.1 mmol, 3 eq). The mixture was stirred at 120 °C for 3 h under N₂. The mixture was cooled and then diluted with EtOAc (100 mL), filtered and then concentrated in vacuo to give the crude product. The crude product was purified by flash chromatography (ethyl acetate/petroleum ether=10% to 30%) to afford the title compound (3.3 g, 19.51 mmol, 74.0% yield) as a colorless oil. ¹H NMR (400 MHz, CDCl₃) δ 8.51 (dd, *J* = 6.0, 8.0 Hz, 1H), 6.95 (dd, *J* = 6.0, 8.0 Hz, 1H), 3.96 (s, 3H), 2.65 (s, 3H).

### Step 3:

### (4-Fluoro-2-methyl-3-pyridyl)methanol

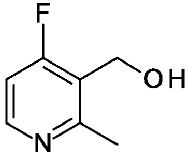

To a solution of methyl 4-fluoro-2-methyl-pyridine-3-carboxylate (2.8 g, 16.55 mmol, 1 eq) in THF (100 mL) was added lithium aluinium hydride (1.57 g, 41.38 mmol, 2.5 eq) in batches at -40 °C and the mixture stirred at -40 °C for another 1 h. Water (1 mL) was added dropwise, followed by 1 mL aqueous NaOH (10 %) and then ethyl acetate (50 mL). The mixture was filtered and filtrate was concentrated in vacuo to afford the title compound (2200 mg, 15.59 mmol, 94.2% yield) as a white solid. MS (ESP): m/z = 142.2 [M+H]⁺ .

### Steps 4 to 6:

### Dimethyl 4-fluoro-5,7-dihydrocyclopenta[b]pyridine-6,6-dicarboxylate

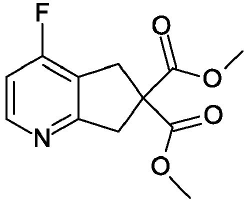

The title compound was prepared from (4-fluoro-2-methyl-3-pyridyl)methanol by analogy with diethyl 1-bromo-4-fluoro-5,7-dihydrocyclopenta[c]pyridine-6,6-dicarboxylate (Intermediate 27, Steps 3 to 5) using dimethyl malonate in place of diethyl malonate and sodium hydride in place of potassium tert-butoxide in Step 6, and was obtained as a white solid. MS (ESP): m/z = 254.1 [M+H]⁺ .

### Steps 7 to 9:

### 4-Fluoro-6,7-dihydro-5H-cyclopenta[b]pyridine-6-carbaldehyde

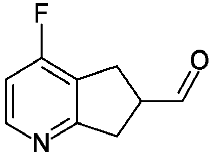

The title compound was prepared from dimethyl 4-fluoro-5,7-dihydrocyclopenta[b]pyridine-6,6-dicarboxylate by analogy with 1-chloro-3-methoxy-6,7-dihydro-5*H*-cyclopenta[c]pyridine-6-carbaldehyde (Intermediate 37, Steps 6 to 8) and was obtained as a white semi-solid. The crude product was used for the next step directly. MS (ESP): m/z = 166.1 [M+H]⁺.

### Intermediate 224

### 4-Chloro-3-fluoro-6,7-dihydro-5H-cyclopenta[b]pyridine-6-carbaldehyde

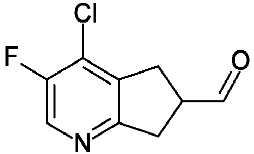

The title compound was prepared from (4-chloro-5-fluoro-2-methyl-3-pyridyl)methanol (Intermediate 209, Step 6) by analogy with 4-fluoro-6,7-dihydro-5*H*-cyclopenta[b]pyridine-6-carbaldehyde (Intermediate 223, Steps 4 to 9) and was obtained as a light brown oil. The crude product was used for the next step directly.

### Intermediate 225

### tert-Butyl 3-[(7-fluoro-2-formyl-indan-5-yl)oxymethyl]pyrazole-1-carboxylate

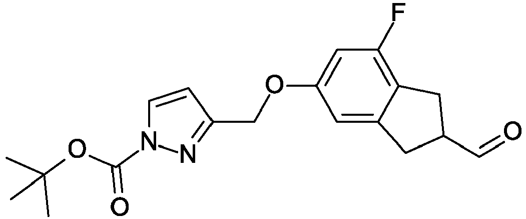

### Step 1:

### tert-Butyl 3-(hydroxymethyl)pyrazole-1-carboxylate

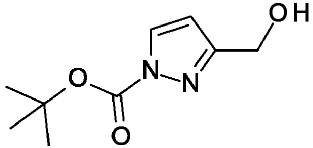

To a mixture of 1*H*-pyrazol-3-ylmethanol (CAS# 23585-49-1, 300.0 mg, 3.06 mmol, 1 eq) in MeCN (5 mL) under ice-water bath cooling was added Boc₂O (833.3 mg, 3.82 mmol, 1.25 eq) and 4-dimethylaminopyridine (747.2 mg, 6.12 mmol, 2 eq), then the mixture was warmed to 25 °C and stirred for 16 h. After 16 h, the mixture was concentrated under vacuum to give a residue. The residue was purified by flash chromatography (PE/EtOAc=1/1-1/2) to give the title compound (290 mg, 1.46 mmol, 47.8% yield) as a colorless oil. ¹H NMR (400 MHz, CDCl₃) δ 7.99 (d, *J* = 4.0 Hz, 1H), 6.37 (d, *J* = 4.0 Hz, 1H), 4.70 (s, 2H), 1.61 (s, 9H).

### Step 2:

### tert-Butyl 3-[[2-[[tert-butyl(dimethyl)silyl]oxymethyl]-7-fluoro-indan-5-yl]oxymethyl]pyrazole-1-carboxylate

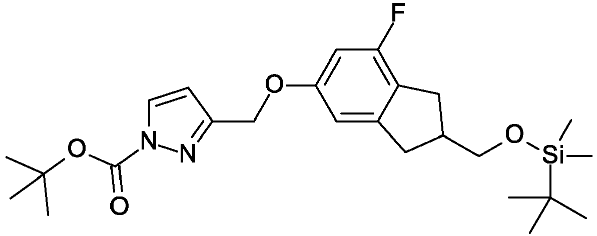

To a mixture of 2-[[*tert*-butyl(dimethyl)silyl]oxymethyl]-7-fluoro-indan-5-ol (Intermediate 204, 300.0 mg, 1.01 mmol, 1 eq), *tert*-butyl 3-(hydroxymethyl)pyrazole-1-carboxylate (240.71 mg, 1.21 mmol, 1.2 eq) and tributyl phosphine (408.84 mg, 2.02 mmol, 2 eq) in THF (6 mL) under nitrogen at 0 °C was added 1,1'-(azodicarbonyl)dipiperidine (ADDP) (510.4 mg, 2.02 mmol, 2 eq) in THF (3 mL) dropwise over 2 min, then the mixture was stirred at 25 °C for 3 h. The mixture was concentrated under vacuum to give a residue. The residue was purified by flash chromatography on silica gel (PE/EtOAc=0 to 10/1) to give the title compound (420 mg, 0.880 mmol, 74.6% yield) as a colorless oil. MS (ESP): m/z =377.4 [M +H-Boc]⁺.

### Steps 3 and 4:

### tert-Butyl 3-[(7-fluoro-2-formyl-indan-5-yl)oxymethyl]pyrazole-1-carboxylate

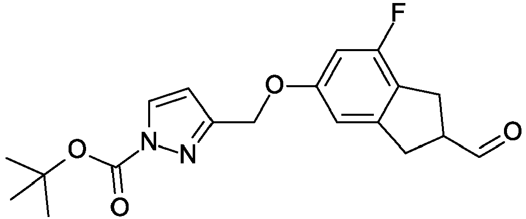

The title compound was prepared by analogy with 1-chloro-4-methoxy-6,7-dihydro-5*H-*cyclopenta[c]pyridine-6-carbaldehyde (Intermediate 43, Steps 3 and 4) and was obtained as a colorless oil. ¹H NMR (400 MHz, CDCl₃) δ 9.68 (s, 1H), 7.97 (d, *J* = 2.8, 1H), 6.67 (s, 1H), 6.54 (dd, *J* = 4.0, 12 Hz, 1H), 6.49 (d, *J* = 4 Hz, 1H), 5.12 (s, 2H), 3.41 - 3.07 (m, 5H), 1.68 (s, 9H).

### Intermediate 226

### 6-(6-Oxo-5-oxa-2,7-diazaspiro[3.4]octan-7-yl)-4H-pyrazino[2,3-b][1,4]oxazin-3-one;formic acid

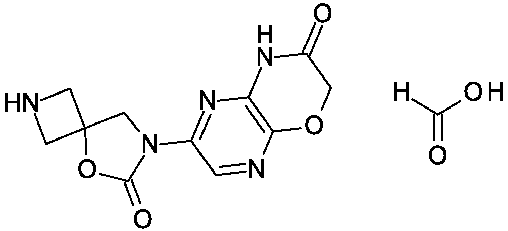

### Step 1:

### tert-Butyl 3-(aminomethyl)-3-hydroxy-azetidine-1-carboxylate

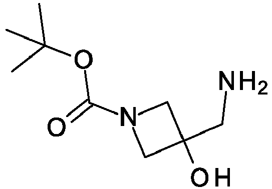

To a solutuion of *tert*-butyl 3-cyano-3-hydroxy-azetidine-1-carboxylate (CAS# 1493737-08-8, 1.0 g, 5.04 mmol, 1 eq) in methanol (30 mL) was added nickel chloride hexahydrate (1553 mg, 6.56 mmol, 1.3 eq) and sodium borohydride (954 mg, 25.22 mmol, 5 eq) in several portions at -40 °C. The mixture was sitrred at -40 °C for 1 h. Saturated NaHCO₃ (20 mL) was added, the mixture filtered and the filtrate concentrated in vacuum, then purified by flash column chromatography (70% methanol/ethyl acetate with 1% NH₃•H₂O) to afford the crude title compound (1.1 g, 5.44 mmol, 97.0% yield) as a yellow solid.

### Step 2:

### tert-Butyl 6-oxo-5-oxa-2,7-diazaspiro[3.4]octane-2-carboxylate

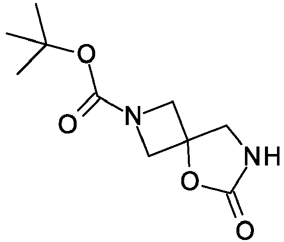

To a solutuion of *tert*-butyl 3-(aminomethyl)-3-hydroxy-azetidine-1-carboxylate (0.5 g, 2.22 mmol, 1 eq) in tetrahydrofuran (10 mL) was added *N,N'*-carbonyldiimidazole (469.0 mg, 2.89 mmol, 1.3 eq). The mixture was sitrred at 65 °C for 2 h. The mixture was concentrated in vacuo and purified by flash chromatography (ethyl acetate) to afford the title compound (240 mg, 1.05 mmol, 47.3% yield) as a white solid. ¹H NMR (400 MHz, CDCl₃) δ 5.71 (br s, 1H), 4.27-4.44 (m, 2H), 4.03 (d, *J*=10.64 Hz, 2H), 3.80 (s, 2H), 1.45 (s, 9H).

### Step 3:

### tert-Butyl 6-oxo-7-(3-oxo-4H-pyrazino[2,3-b][1,4]oxazin-6-yl)-5-oxa-2,7-diazaspiro[3.4]octane-2-carboxylate

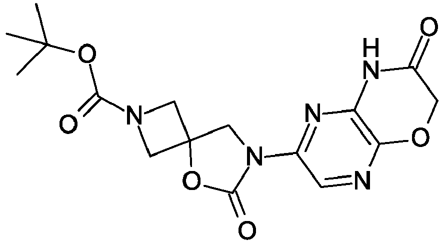

To a solution of 6-bromo-4*H*-pyrazino[2,3-b][1,4]oxazin-3-one (Intermediate 25, Step 1, 201.6 mg, 0.880 mmol, 1 eq) and *tert*-butyl 6-oxo-5-oxa-2,7-diazaspiro[3.4]octane-2-carboxylate (200.0 mg, 0.880 mmol, 1 eq) in 1,4-dioxane (10 mL) was added *trans-N,N'*-dimethylcyclohexane-1,2-diamine (74.8 mg, 0.530 mmol, 0.600 eq) potassium carbonate (363.3 mg, 2.63 mmol, 3 eq) and copper (I) iodide (50.1 mg, 0.260 mmol, 0.300 eq). The mixture was stirred at 120 °C for 2 h under N₂. The mixture was concentrated and purified by flash chromatography (100 % ethyl acetate) to afford the title compound (130 mg, 0.340 mmol, 27.5% yield) as a white solid. MS (ESP): m/z = 378.5 [M+H]⁺.

### Step 4:

### 6-(6-Oxo-5-oxa-2,7-diazaspiro[3.4]octan-7-yl)-4H-pyrazino[2,3-b][1,4]oxazin-3-one;formic acid

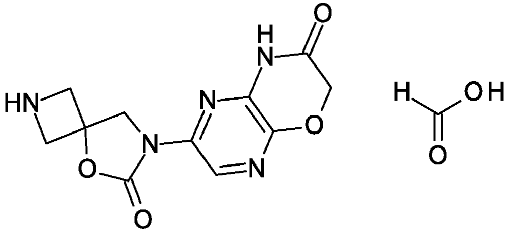

A solution of *tert*-butyl 6-oxo-7-(3-oxo-4*H*-pyrazino[2,3-b][1,4]oxazin-6-yl)-5-oxa-2,7-diazaspiro[3.4]octane-2-carboxylate (130.0 mg, 0.240 mmol, 1 eq) in formic acid (2.0 mL) was stirred at 35 °C for 2 h. The mixture was concentrated in vacuum to afford the title compound (110 mg, 0.340 mmol, 98.8% yield) as a yellow solid. MS (ESP): m/z = 278.6 [M+H]⁺.

### Intermediate 227

### 6-Amino-1-methyl-6,7-dihydro-5H-cyclopenta[c]pyridine-4-carbonitrile; 2,2,2-trifluoroacetic acid, Enantiomer A

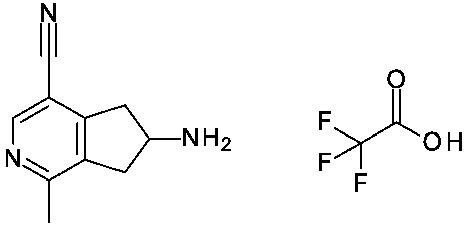

### Step 1:

### 2-Trimethylsilylethyl N-(4-chloro-1-methyl-6,7-dihydro-SH-cyclopenta[c]pyridin-6-yl)carbamate

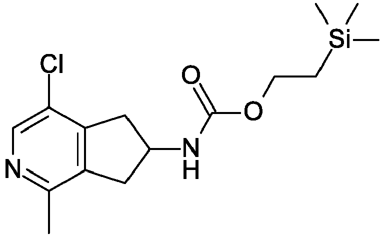

To a solution of 4-chloro-1-methyl-6,7-dihydro-5*H*-cyclopenta[c]pyridine-6-carboxylic acid hydrochloride (Intermediate 41, Step 6, 1000.0 mg, 4.03 mmol, 1 eq) and *N,N-*diisopropylethylamine (3.51 mL, 20.15 mmol, 5 eq) in toluene (100 mL) and DMA (10 mL) was added diphenylphosphonic azide (1.3 mL, 6.05 mmol, 1.5 eq), and then the solution was stirred at 25 °C for 1 h. To the reaction was added 2-(trimethylsilyl) ethanol (2.89 mL, 20.15 mmol, 5 eq), and the solution was stirred at 100 °C for 16 h. The solvent was removed by concentration and the residue was taken up in EtOAc (100 mL), and then washed with brine (100 mL) and dried by Na₂SO₄. The solvent was removed to give crude product, which was purified by chromatography on silica gel (50% of EtOAc/PE) to give the title compound (400 mg, 1.22 mmol, 25.8% yield) as a brown oil. ¹H NMR (400 MHz, CDCl₃) δ 8.23 (s, 1H), 4.80-4.75 (m, 1H), 4.55-4.50 (m, 1H), 4.23 - 4.08 (m, 2H), 3.43 - 3.20 (m, 2H), 2.93 - 2.75 (m, 2H), 2.39 (s, 3H), 1.04 - 0.87 (m, 2H), 0.01 (s, 9H).

### Step 2:

### 2-Trimethylsilylethyl N-(4-cyano-1-methyl-6,7-dihydro-5H-cyclopenta[c]pyridin-6-yl)carbamate

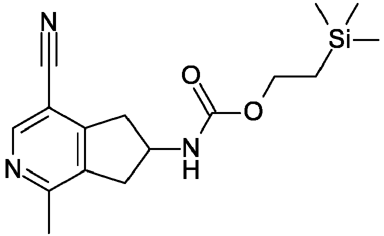

A mixture of 2-di-*tert*-butylphosphino-2',4',6'-triisopropylbiphenyl (320.0 mg, 0.750 mmol, 0.620 eq), zinc cyanide (1440.0 mg, 12.26 mmol, 10.02 eq), tris(dibenzylideneacetone)-dipalladium (0) (560.0 mg, 0.610 mmol, 0.500 eq), zinc (80.0 mg, 1.22 mmol, 1 eq) and 2-trimethylsilylethyl *N*-(4-chloro-1-methyl-6,7-dihydro-5*H*-cyclopenta[c]pyridin-6-yl)carbamate (400.0 mg, 1.22 mmol, 1 eq) in *N,N*-dimethylacetamide (28 mL) under nitrogen was stirred at 120 °C for 16 h. The mixture was filtered and the mixture was diluted with EtOAc (20 mL), washed with brine (20 mL × 2), dried over Na₂SO₄ and then concentrated under vacuum to give a residue. The residue was purified by Prep-TLC (PE/EtOAc=1/1) to give the title compound (150 mg, 0.470 mmol, 36.7% yield) as a light brown oil. MS (ESP): m/z = 318.4 [M+H]⁺.

### Step 3:

### 2-Trimethylsilylethyl N-(4-cyano-1-methyl-6,7-dihydro-5H-cyclopenta[c]pyridin-6-yl)carbamate, Enantiomer A and 2-trimethylsilylethyl N-(4-cyano-1-methyl-6,7-dihydro-5H-cyclopenta[c]pyridin-6-yl)carbamate, Enantiomer B

2-Trimethylsilylethyl *N*-(4-cyano-1-methyl-6,7-dihydro-5*H*-cyclopenta[c]pyridin-6-yl)carbamate (320.0 mg, 1.01 mmol, 1 eq) was separated by SFC to give 2-trimethylsilylethyl *N*-(4-cyano-1-methyl-6,7-dihydro-5*H*-cyclopenta[c]pyridin-6-yl)carbamate, Enantiomer B (90 mg, 0.280 mmol, 27.8% yield) as a yellow oil, MS (ESP): m/z =318.2 [M+H]⁺ and 2-trimethylsilylethyl *N-*(4-cyano-1-methyl-6,7-dihydro-5*H*-cyclopenta[c]pyridin-6-yl)carbamate, Enantiomer A (110 mg, 0.350 mmol, 32.7% yield) as a yellow oil. MS (ESP): m/z =318.2 [M+H]⁺.

### Step 4:

### 6-Amino-1-methyl-6,7-dihydro-5H-cyclopenta[c]pyridine-4-carbonitrile; 2,2,2-trifluoroacetic acid, Enantiomer A

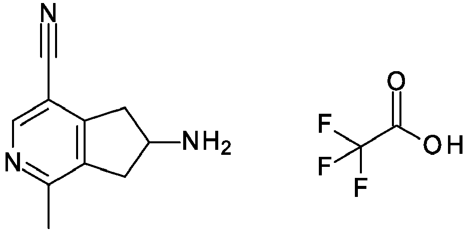

To a mixture of 2-trimethylsilylethyl *N*-(4-cyano-1-methyl-6,7-dihydro-5*H-*cyclopenta[c]pyridin-6-yl)carbamate, Enantiomer A (90.0 mg, 0.280 mmol, 1 eq) in DCM (9 mL) was added TFA (0.4 mL) and then the mixture was stirred at 20 °C for 16 h. The mixture was concentrated under high vacuum to give the crude title compound (80 mg, 0.280 mmol, 98.2% yield) as a light brown oil. The crude product was used for the next step directly.

### Intermediate 228

### 6-Amino-1-methyl-6,7-dihydro-5H-cyclopenta[c]pyridine-4-carbonitrile; 2,2,2-trifluoroacetic acid, Enantiomer B

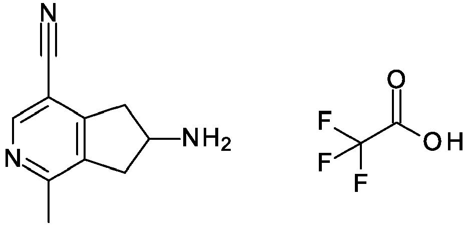

The title compound was prepared from 2-trimethylsilylethyl *N*-(4-cyano-1-methyl-6,7-dihydro-5*H*-cyclopenta[c]pyridin-6-yl)carbamate, Enantiomer B (Intermediate 227, Step 3) by analogy with 6-amino-1-methyl-6,7-dihydro-5*H*-cyclopenta[c]pyridine-4-carbonitrile; 2,2,2-trifluoroacetic acid, Enantiomer A (Intermediate 227, Step 4) and was obtained as a light brown oil. The crude product was used for the next step directly.

### Intermediate 229

### 4-Fluoro-6-(oxetan-3-yloxy) indane-2-carbaldehyde

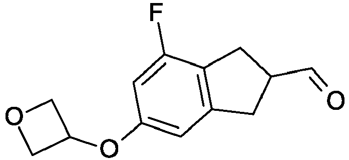

### Step 1:

### tert-Butyl-[[4-fluoro-6-(oxetan-3-yloxy)indan-2-yl]methoxy]-dimethyl-silane

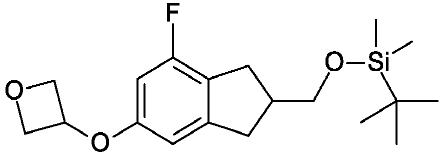

To a solution of 2-[[*tert*-butyl(dimethyl)silyl]oxymethyl]-7-fluoro-indan-5-ol (Intermediate 204, 230.0 mg, 0.780 mmol, 1 eq) in DMF (15 mL) was added caesium carbonate (505.5 mg, 1.55 mmol, 2 eq) and oxetan-3-yl 4-methylbenzenesulfonate (CAS# 26172-83-3,177 mg, 0.780 mmol, 1 eq), the reaction mixture was stirred at 120 °C for 2 h. The solution was poured into water (50 mL) and then extracted with EtOAc (50 mL × 2), combined with the organics and dried by Na₂SO₄. The solvent was removed to give the crude, which was purified by TLC (PE/EtOAc =3:1) to give the title compound (160 mg, 0.450 mmol, 55.6% yield) as a colorless oil. ¹H NMR (400MHz, CDCl₃) δ 6.29 (s, 1H), 6.17 (dd, *J*=2.0, 10.3 Hz, 1H), 5.09 (quin, *J*=5.7 Hz, 1H), 4.90 (t, J=6.9 Hz, 2H), 4.69 (dd, J=5.2, 7.8 Hz, 2H), 3.58 - 3.49 (m, 2H), 2.98 - 2.85 (m, 2H), 2.72 - 2.56 (m, 3H), 1.49 (s, 1H), 0.84 (s, 9H), 0.02 (s, 6H).

### Steps 2 and 3:

### 4-Fluoro-6-(oxetan-3-yloxy) indane-2-carbaldehyde

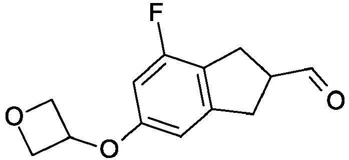

The title compound was prepared by analogy with 1-chloro-4-methoxy-6,7-dihydro-5*H-*cyclopenta[c]pyridine-6-carbaldehyde (Intermediate 43, Steps 3 and 4) and was obtained as a white semi-solid which was used directly without further purification. ¹H NMR (400MHz, CDCl₃) δ 9.73 (s, 1H), 6.38 - 6.28 (m, 1H), 6.40 - 6.28 (m, 1H), 6.26 - 6.20 (m, 1H), 6.23 (dd, *J*=1.8, 10.3 Hz, 1H), 5.11 (quin, *J*=5.6 Hz, 1H), 4.93 (t, *J*=6.7 Hz, 2H), 4.76 - 4.67 (m, 2H), 3.35 - 3.01 (m, 5H).

### Intermediate 230

### N-[2-(7-Fluoro-2-formyl-indan-5-yl) oxyethyl] methanesulfonamide

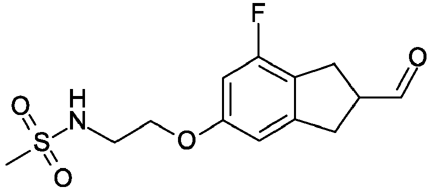

The title compound was prepared from *N*-[2-[7-fluoro-2-(hydroxymethyl)indan-5-yl]oxyethyl]methanesulfonamide (Intermediate 231) by analogy with 2-formyl-2,3-dihydro-1*H-*indene-4-carbonitrile (Intermediate 5, Step 3) and was obtained as a light yellow oil which was used directly without further purification. ¹H NMR (400MHz, CDCl₃) δ 9.69 (d, *J*=1.3 Hz, 1H), 6.53 - 6.47 (m, 1H), 6.36 (dd, *J*=1.9, 10.4 Hz, 1H), 4.71 (br s, 1H), 3.98 (t, *J*=5.0 Hz, 2H), 3.51 - 3.42 (m, 2H), 3.31 - 3.01 (m, 5H), 2.95 (s, 3H).

### Intermediate 231

### N-[2-[7-Fluoro-2-(hydroxymethyl)indan-5-yl]oxyethyl]methanesulfonamide

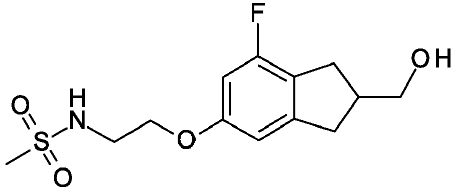

### Step 1:

### tert-Butyl N-[2-[2-[[tert-butyl(dimethyl)silyl]oxymethyl]-7-fluoro-indan-5-yl] oxyethyl] carbamate

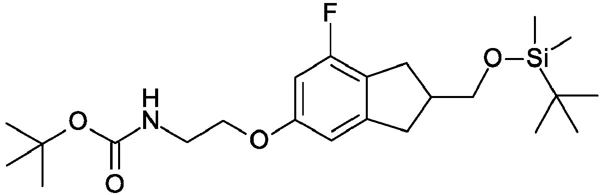

To a solution of *N*-BOC-2-chloroethylamine (CAS# 71999-74-1,363.58 mg, 2.02 mmol, 1.5 eq) in *N,N*-dimethylformamide (12 mL) was added 2-[[*tert*-butyl(dimethyl)silyl]oxymethyl]-7-fluoro-indan-5-ol (Intermediate 204, 400.0 mg, 1.35 mmol, 1 eq), caesium carbonate (1099 mg, 3.37 mmol, 2.5 eq) and potassium iodide (0.07 mL, 1.35 mmol, 1 eq), and then the solution was stirred at 70 °C for 16 h. The solution was poured into water (50 mL) and then extracted with EtOAc (80 mL × 2), combined with the organics and dried by Na₂SO₄. The solvent was removed to give the crude, which was purified by flash chromatography (25% of EtOAc/PE) to give the title compound (600 mg, 1.36 mmol, 89.2% yield) as a light yellow oil. ¹H NMR (400MHz, CDCl₃) δ 6.49 (s, 1H), 6.35 (dd, *J*=2.0, 10.6 Hz, 1H), 3.92 (t, *J*=5.1 Hz, 2H), 3.57 - 3.42 (m, 4H), 2.97 - 2.85 (m, 2H), 2.72 - 2.54 (m, 3H), 1.41 (s, 9H), 0.84 (s, 9H), 0.02 (s, 6H).

### Step 2:

### [6-(2-Aminoethoxy)-4-fluoro-indan-2-yl] methanol

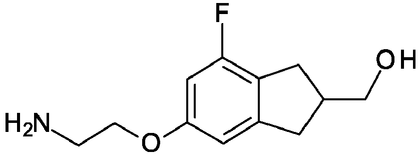

A mixture of *tert*-butyl *N*-[2-[2-[[*tert*-butyl(dimethyl)silyl]oxymethyl]-7-fluoro-indan-5-yl] oxyethyl]carbamate (100.0 mg, 0.230 mmol, 1 eq) in HCl-CH₃OH (8.0 mL, 0.230 mmol, 1 eq) was stirred at 30 °C for 2 h . The reaction solution was concentrated under vacuum to give the crude title compound (70 mg) as a white sollid, which was used directly without further purification. ¹H NMR (400MHz, CD₃OD) δ 6.72 (d, *J*=1.6 Hz, 1H), 6.57 (dd, *J*=2.0, 10.5 Hz, 1H), 4.24 - 4.16 (m, 2H), 3.55 (d, *J*=6.3 Hz, 2H), 3.39 - 3.34 (m, 2H), 3.10 - 2.95 (m, 2H), 2.81 - 2.62 (m, 3H).

### Step 3:

### N-[2-[7-Fluoro-2-(hydroxymethyl)indan-5-yl]oxyethyl]methanesulfonamide

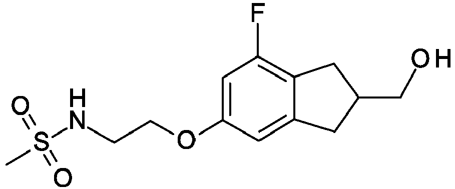

To a solution of [6-(2-aminoethoxy)-4-fluoro-indan-2-yl]methanol hydrochloride (150.0 mg, 0.570 mmol, 1 eq) in water (5 mL) and EtOAc (5 mL) was added potassium carbonate (118.8 mg, 0.860 mmol, 1.5 eq). The mixture was stirred at 0 °C for 30 min, then was added methanesulfonyl chloride (0.05 mL, 0.630 mmol, 1.1 eq) at 0 °C. The mixture was stirred at 0 °C for 4 h. The organic layer was washed with brine and dried over anhydrous magnesium sulfate and concentrated under vacuum to give the crude product. The crude product was purified by TLC (EtOAc:PE=2:1) to give the title compound (120 mg, 0.400 mmol, 64.2% yield) as a colorless oil. MS (ESP): m/z = 286.1 [M-H₂O+H]⁺.

### Intermediate 232

### N-[2-(7-Fluoro-2-formyl-indan-5-yl)oxyethyl]-N-methyl-methanesulfonamide

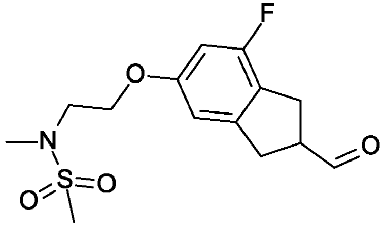

### Step 1:

### N-[2-[7-Fluoro-2-(hydroxymethyl)indan-5-yl]oxyethyl]-N-methyl-methanesulfonamide

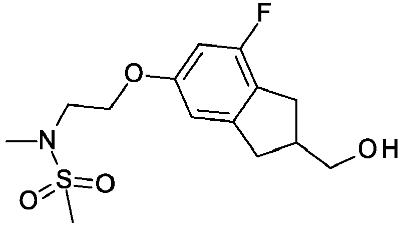

To a solution of N-[2-[7-fluoro-2-(hydroxymethyl)indan-5-yl]oxyethyl]methanesulfonamide (Intermediate 231, 110.0 mg, 0.360 mmol, 1 eq) in MeCN (10 mL) was added K₂CO₃ (150.3 mg, 1.09 mmol, 3 eq). The reaction mixture was stirred at 0 °C for 10 min. To the above solution was added methyl iodide (0.05 mL, 0.730 mmol, 2 eq) at 0 °C. The mixture was stirred at 65 °C for 16 h. The mixture was filtrated through a filter membrane and the filtrate was collected and concentrated to give the crude product which was purified by TLC (EtOAc:PE= 2:1 ,Rf= 0.4) to give the title compound (140 mg, 0.440 mmol, quant. yield) as a colorless semi-solid. ¹H NMR (400MHz, CD₃OD) δ 6.66 (d, *J*=1.7 Hz, 1H), 6.50 (dd, *J*=2.1, 10.6 Hz, 1H), 4.17 - 4.09 (m, 2H), 3.61 - 3.53 (m, 4H), 3.35-3.30 (m, 5H), 3.03 - 2.99 (m, 3H), 2.80 - 2.63 (m, 3H).

### Step 2:

### N-[2-(7-Fluoro-2-formyl-indan-5-yl)oxyethyl]-N-methyl-methanesulfonamide

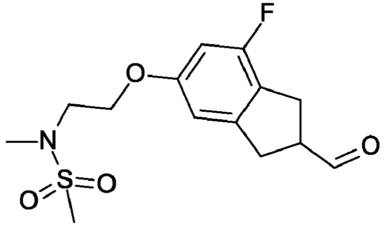

The title compound was prepared from *N*-[2-[7-fluoro-2-(hydroxymethyl)indan-5-yl]oxyethyl]-*N*-methyl-methanesulfonamide by analogy with 2-formyl-2,3-dihydro-1*H*-indene-4-carbonitrile (Intermediate 5, Step 3) and was obtained as a light yellow semi-solid which was used directly without further purification. ¹H NMR (400MHz, CDCl₃) δ 9.78 (d, *J*=1.2 Hz, 1H), 6.66 - 6.55 (m, 1H), 6.45 (dd, *J*=2.0, 10.5 Hz, 1H), 4.12 (t, *J*=5.2 Hz, 2H), 3.61 (t, *J*=5.3 Hz, 2H), 3.37 - 3.04 (m, 5H), 3.03 (s, 3H), 2.90 (s, 3H).

### Intermediate 233

### tert-Butyl N-[2-[7-fluoro-2-(2-oxoethyl)indan-5-yl]oxy-1-methyl-ethyl]carbamate

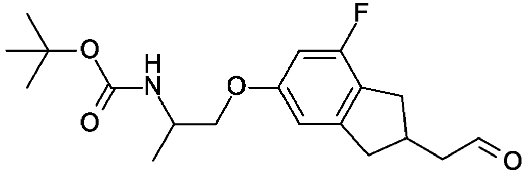

### Step 1:

### 1-[2-[[tert-Butyl(dimethyl)silyl]oxymethyl]-7-fluoro-indan-5-yl]oxypropan-2-one

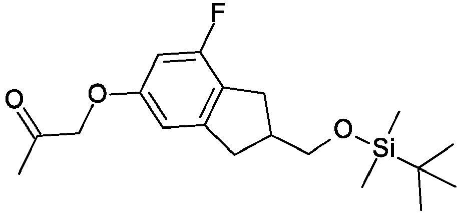

To a solution of 2-[[*tert*-butyl(dimethyl)silyl]oxymethyl]-7-fluoro-indan-5-ol (Intermediate 204, 1000.0 mg, 3.37 mmol, 1 eq) and potassium bicarbonate (931.7 mg, 6.75 mmol, 2 eq) in DMF (10 mL) was added chloroacetone (CAS# 78-95-5,374.51 mg, 4.05 mmol, 1.2 eq), and then the solution was stirred at 80 °C for 16 h. The solution was poured into water (20 mL) and then extracted with EtOAc (10 mL × 2), combined with the organics and dried by Na₂SO₄. The solvent was removed to give the crude, which was purified by chromatography on silica gel (PE: EtOAc=10:1) to give the title compound (1000 mg, 2.84 mmol, 84.1% yield) as a colorless oil. MS (ESP): m/z = 353.3 [M+H]⁺.

### Step 2:

### 1-[2-[[tert-Butyl(dimethyl)silyl]oxymethyl]-7-fluoro-indan-5-yl]oxypropan-2-amine

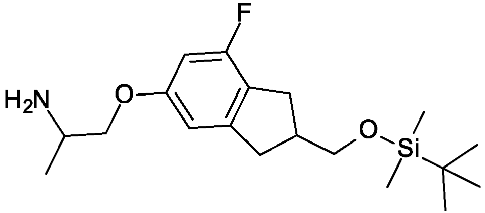

To a solution of sodium cyanoborohydride (891.3 mg, 14.18 mmol, 5 eq) in THF (3 mL) was added triethylamine (0.01 mL), the mixture was stirred at 25 °C for 10 min. Then 1-[2-[[*tert-*butyl(dimethyl)silyl]oxymethyl]-7-fluoro-indan-5-yl]oxypropan-2-one (1000.0 mg, 2.84 mmol, 1 eq) in THF (2 mL) was added. The mixture was stirred at 25 °C for 20 min. Then was added ammonium acetate (2186 mg, 28.37 mmol, 10 eq) at 0 °C. The mixture was stirred at 25 °C for another 16 h. The solvent was removed by evaopration and the residue was taken up in water (20 mL) and extracted with EtOAc (10 mL × 2), combined with the organics and dried by Na₂SO₄. The solvent was removed to give crude title compound (1000 mg, 2.83 mmol, 99.7% yield) as a yellow oil. MS (ESP): m/z = 354.3 [M+H]⁺.

### Step 3:

### tert-Butyl N-[2-[2-[[tert-butyl(dimethyl)silyl]oxymethyl]-7-fluoro-indan-5-yl]oxy-1-methylethyl] carbamate

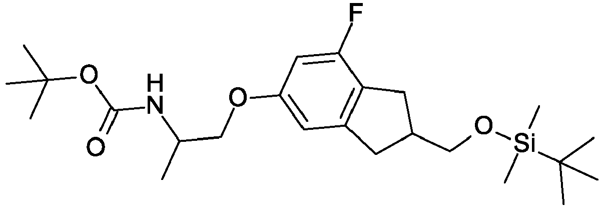

To a solution of 1-[2-[[*tert*-butyl(dimethyl)silyl]oxymethyl]-7-fluoro-indan-5-yl]oxypropan-2-amine (1000 mg, 2.83 mmol, 1 eq) and triethylamine (0.79 mL, 5.66 mmol, 2 eq) in methanol (30 mL) was added di-*tert*-butyldicarbonate (926 mg, 4.24 mmol, 1.5 eq), and then the solution was stirred at 25 °C for 6 h. The solvent was removed by concentration and the residue was taken up EtOAc (20 mL) and washed with brine (20 mL). The solvent was dried by Na₂SO₄ and the solvent was removed to give the crude, which was purified by chromatography on silica gel to give the title compound (500 mg, 1.1 mmol, 39.0% yield) as a colorless oil. MS (ESP): m/z = 476.3 [M+Na]⁺.

### Step 4:

### tert-Butyl N-[2-[7-fluoro-2-(hydroxymethyl)indan-5-yl]oxy-1-methyl-ethyl]carbamate

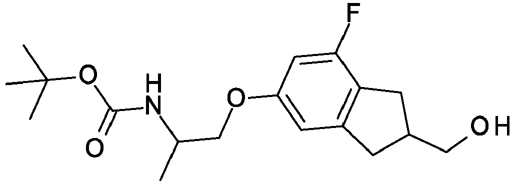

To a solution of *tert*-butyl *N*-[2-[2-[[*tert*-butyl(dimethyl)silyl]oxymethyl]-7-fluoro-indan-5-yl]oxy-1-methyl-ethyl]carbamate (500.0 mg, 1.1 mmol, 1 eq) in THF (10 mL) was added TBAF in THF (2.2 mL, 2.2 mmol, 2 eq), then the solution was stirred at 25 °C for 16 h. The solvent was removed to give the crude product. It was purified by chromatography on silica gel (PE:EtOAc=1:1) to give the title compound (300 mg, 0.880 mmol, 80.2% yield) as a colorless oil. MS (ESP): m/z = 362.2 [M+Na]⁺.

### Steps 5 to 7:

### Ethyl 2-[6-(2-aminopropoxy)-4-fluoro-indan-2-yl] acetate

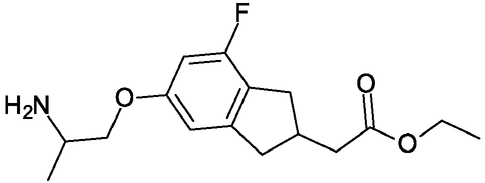

The title compound was prepared from *tert*-butyl *N*-[2-[7-fluoro-2-(hydroxymethyl)indan-5-yl]oxy-1-methyl-ethyl]carbamate by analogy with ethyl 2-(4-chloro-2,3-dihydro-1*H*-inden-2-yl)acetate (Intermediate 11, Steps 1 to 3) and was obtained as a white solid, which was used directly without future purification.

### Step 8:

### Ethyl 2-[6-[2-(tert-butoxycarbonylamino)propoxy]-4-fluoro-indan-2-yl] acetate

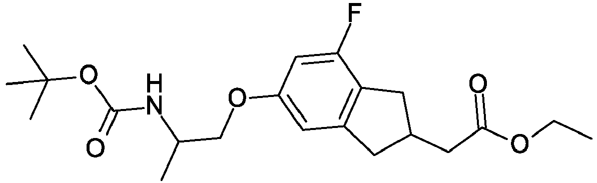

To a solution of ethyl 2-[6-(2-aminopropoxy)-4-fluoro-indan-2-yl]acetate (100.0 mg, 0.340 mmol, 1 eq) in ethanol (3 mL) was added saturated aq. NaHCO₃ (8.0 mL, 0.340 mmol, 1 eq). To the mixture was added di-tert-butyl dicarbonate (112 mg, 0.520 mmol, 1.52 eq) at 0 °C. Then the mixture was stirred at 25 °C for 16 h. The mixture was extracted with EtOAc (20 mL × 3). The organic layer was washed with brine and dried over anhydrous magnesium sulfate and concentrated under vacuum to give the crude product. The crude was purified by TLC ( PE: EtOAc= 3:1) to give the title compound (60 mg, 0.150 mmol, 39.4% yield) as a colorless oil. MS (ESP): m/z = 418.1 [M+Na]⁺.

### Steps 9 to 10:

### tert-Butyl N-[2-[7-fluoro-2-(2-oxoethyl)indan-5-yl]oxy-1-methyl-ethyl]carbamate

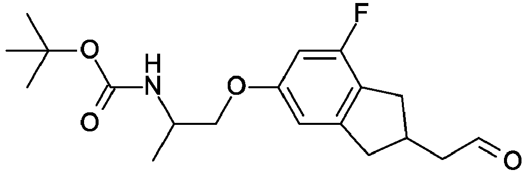

The title compound was prepared by analogy with 2-(4-chloro-2,3-dihydro-1*H*-inden-2-yl)acetaldehyde (Intermediate 11, Steps 4 to 5) and was obtained as a light yellow semi-solid. ¹H NMR (400MHz, CDCl₃) δ 9.75 (s, 1H), 6.49 (s, 1H), 6.36 (br d, *J*=10.4 Hz, 1H), 4.69 (br s, 1H), 4.00-3.96 (m, 1H), 3.80-3.79 (m, 2H), 3.15-3.05 (m, 2H), 2.95 - 2.83 (m, 1H), 2.64 - 2.43 (m, 4H), 1.40 - 1.36 (m, 9H), 1.23 - 1.19 (m, 4H).

### Intermediate 234

### 2-Oxo-5-(3-oxocyclobutyl)oxazolidin-3-yl]-4-(2-trimethylsilylethoxymethyl)pyrazino[2,3-b][1,4]oxazin-3-one, Enantiomer A and

### Intermediate 235

### 2-Oxo-5-(3-oxocyclobutyl)oxazolidin-3-yl]-4-(2-trimethylsilylethoxymethyl)pyrazino[2,3-b][1,4]oxazin-3-one, Enantiomer B

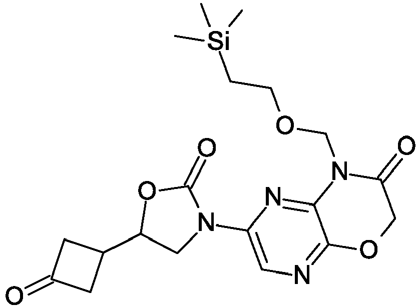

### Steps 1 to 4:

### 6-[5-[3-[tert-Butyl(dimethyl)silyl]oxycyclobutyl]-2-oxo-oxazolidin-3-yl]-4-(2-trimethylsilyl ethoxymethyl)pyrazino[2,3-b][1,4]oxazin-3-one

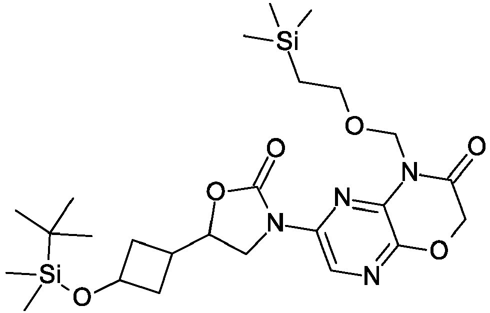

The title compound was prepared by analogy with *tert*-butyl *N*-[*trans*-3-(1-hydroxy-2-nitroethyl)cyclobutyl]carbamate (Intermediate 7, Steps 1 to 4) using 3-[*tert-*butyl(dimethyl)silyl]oxy cyclobutanecarbaldehyde (CAS# 1272656-62-8) in place of *tert*-butyl N-(trans-3-formylcyclobutyl)carbamate and was obtained as a white solid. MS (ESP): m/z = 573.2 [M+Na]⁺.

### Step 5:

### 6-[5-(3-Hydroxycyclobutyl)-2-oxo-oxazolidin-3-yl]-4-(2-trimethylsilylethoxymethyl) pyrazino[2,3-b][1,4]oxazin-3-one

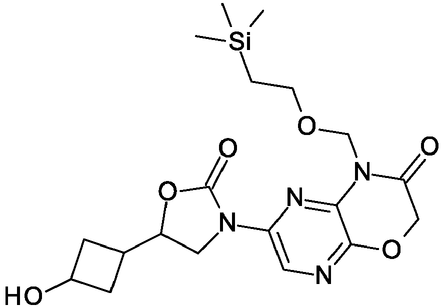

A solution of 6-[5-[3-[tert-butyl(dimethyl)silyl]oxycyclobutyl]-2-oxo-oxazolidin-3-yl]-4-(2-trimethylsilylethoxymethyl)pyrazino[2,3-b][1,4]oxazin-3-one (580.0 mg, 1.05 mmol, 1 eq) in formic acid (4.0 mL, 1.05 mmol, 1 eq) and water (10 mL) was stirred at 25 °C for16 h. The mixture was diluted with EtOAc (10 mL) and washed with aq.NaHCO₃ to adjust the pH to 8-9. The organic layer was collected, dried and concentrated to give the crude title compound (400 mg, 0.920 mmol, 87.0% yield) as a light yellow oil, which was used directly without further purification.

### Step 9:

### 2-Oxo-5-(3-oxocyclobutyl)oxazolidin-3-yl]-4-(2-trimethylsilylethoxymethyl)pyrazino[2,3-b][1,4]oxazin-3-one, Enantiomer A and 2-oxo-5-(3-oxocyclobutyl)oxazolidin-3-yl]-4-(2-trimethylsilylethoxymethyl)pyrazino[2,3-b][1,4]oxazin-3-one, Enantiomer B

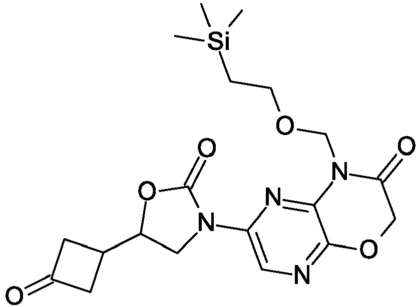

To a mixture of 6-[5-(3-hydroxycyclobutyl)-2-oxo-oxazolidin-3-yl]-4-(2-trimethylsilylethoxy methyl)pyrazino[2,3-b][1,4]oxazin-3-one (400.0 mg, 0.920 mmol, 1 eq) in DCM (20 mL) was added Dess-Martin Periodinane (777 mg, 1.83 mmol, 2 eq) The mixture was stirred at 20 °C for 2 h. The mixture was filtered and the filtrate was purified by TLC (PE:EtOAc = 1:1) to give a crude product. The crude product was purified by SFC to give 2-oxo-5-(3-oxocyclobutyl)oxazolidin-3-yl]-4-(2-trimethylsilylethoxymethyl)pyrazino[2,3-b] [1,4]oxazin-3-one, Enantiomer A (Intermediate 234, 65 mg, 0.150 mmol, 15.8% yield) as a white solid; MS (ESP): m/z = 457.2 [M+Na]⁺ and 2-oxo-5-(3-oxocyclobutyl)oxazolidin-3-yl]-4-(2-trimethylsilylethoxymethyl)pyrazino[2,3-b][1,4]oxazin-3-one, Enantiomer B (Intermediate 235, 65 mg, 0.150 mmol, 15.8% yield) as a white solid; MS (ESP): m/z = 435.2 [M+H]⁺.

### Intermediate 236

### 1-(4-Fluoro-1-methyl-6,7-dihydro-5H-cyclopenta[c]pyridin-6-yl)ethanone

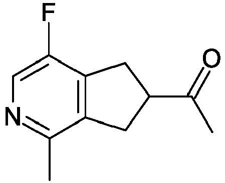

### Step 1:

### 4-Fluoro-N-methoxy-N,1-dimethyl-6,7-dihydro-5H-cyclopenta[c]pyridine-6-carboxamide

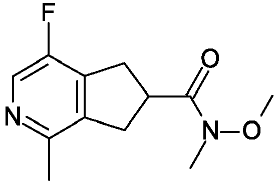

To a solution of 4-fluoro-1-methyl-6,7-dihydro-5*H*-cyclopenta[c]pyridine-6-carboxylic acid hydrochloride (Intermediate 153, step 1, 100.0 mg, 0.430 mmol, 1 eq) dissolved in DCM (5 mL) was added diisopropyl ethylamine (390.4 mg, 3.02 mmol, 7 eq), HOBT (70.0 mg, 0.520 mmol, 1.2 eq) and EDCI (120.4 mg, 0.780 mmol, 1.8 eq). Then *O,N*-dimethylhydroxylamine HCl (54.7 mg, 0.560 mmol, 1.3 eq) was added at 0 °C. After that the solution was stirred at 25 °C for 16 h. The solution was washed by water, extracted by EtOAc (50 mL), the organic layer was separated and concentrated, and the residue purified by TLC (EtOAc:PE= 3:2 ) to give the title compound (56 mg, 0.240 mmol, 51.3% yield) as a light brown solid. MS (ESP): m/z = 239.2 [M+H]⁺.

### Step 2:

### 1-(4-Fluoro-1-methyl-6,7-dihydro-5H-cyclopenta[c]pyridin-6-yl)ethanone

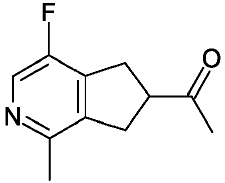

To a solution of 4-fluoro-N-methoxy-N,1-dimethyl-6,7-dihydro-SH-cyclopenta[c]pyridine-6-carboxamide (56.0 mg, 0.240 mmol, 1 eq) in THF (7 mL) was added methyl magnesium bromide in diethyl ether (0.47 mL, 1.41 mmol, 6 eq) at -78 °C. After that the solution was stirred at -20 °C for 2 h. The solution was quenched by adding water, extracted by EtOAc (3 × 20 mL). The organic layer was separated and concentrated, and the residue purified by TLC (EtOAc:PE=3:2) to give the title compound (20 mg, 0.100 mmol, 38.3% yield) as a light brown oil. MS(ESP): m/z = 194.1 [M+H]⁺.

### Intermediate 237

### 4-Fluoro-6-(2-imidazol-1-ylethoxy)indane-2-carbaldehyde

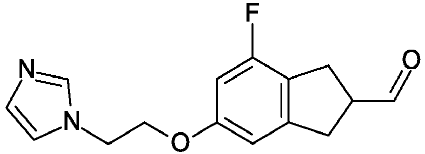

### Step 1:

### tert-Butyl-[[4-fluoro-6-(2-imidazol-1-ylethoxy)indan-2-yl]methoxy]-dimethyl-silane

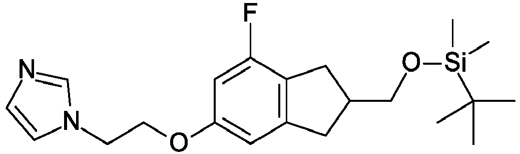

To a solution of [6-(2-bromoethoxy)-4-fluoro-indan-2-yl]methoxy-tert-butyl-dimethyl-silane (Intermediate 217, 220.0 mg, 0.550 mmol, 1 eq) in DMF (12 mL) was added imidazole (111.4 mg, 1.64 mmol, 3 eq). The mixture was stirred at 0 °C. Sodium hydride, 60% in oil (72.0 mg, 1.8 mmol, 3.3 eq) was added. Then the mixture was stirred at 25 °C for 2 h. The reaction was quenched by adding water (2 mL) and extracted with EtOAc (3 × 30 mL). The organic layer was washed with water, brine and dried over anhydrous magnesium sulfate and concentrated under vacuum to give the crude product, which was purified by TLC (EtOAc:PE=2:1) to give the title compound (170 mg, 0.440 mmol, 75.2% yield) as a colorless oil. MS (ESP): m/z = 391.3 [M+H]⁺.

### Steps 2 and 3:

### 4-Fluoro-6-(2-imidazol-1-ylethoxy)indane-2-carbaldehyde

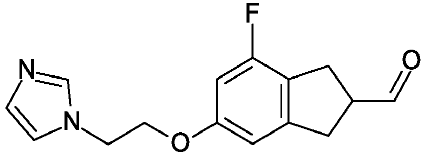

The title compound was prepared by analogy with 6-[2-(5-aminotetrazol-1-yl)ethoxy]-4-fluoro-indane-2-carbaldehyde (Intermediate 205, Steps 2 and 3) and was obtained as a white semi-solid.

### Intermediate 238

### 6-[2-Oxo-5-(3-oxobutyl)oxazolidin-3-yl]-4H-pyrazino[2,3-b][1,4] oxazin-3-one, Enantiomer A

### Intermediate 239

### 6-[2-Oxo-5-(3-oxobutyl)oxazolidin-3-yl]-4H-pyrazino[2,3-b][1,4] oxazin-3-one, Enantiomer B

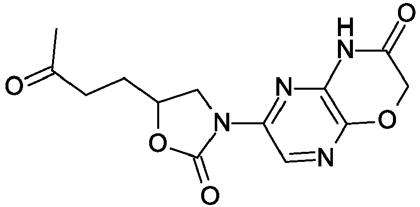

### Steps 1 and 2:

### 1-Azido-4-(2-methyl-1,3-dioxolan-2-yl)butan-2-ol

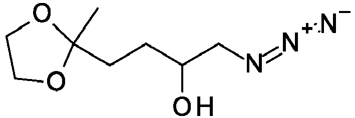

The title compound was prepared by analogy with *tert*-butyl *N*-[5-azido-2-[*tert-*butyl(diphenyl)silyl]oxy-4-hydroxypentyl]carbamate (Intermediate 156, Steps 2 and 3) using 2-but-3-enyl-2-methyl-1,3-dioxolane (CAS# 20449-21-2) in place of *tert*-butyl *N*-[2-[*tert-*butyl(diphenyl)silyl]oxypent-4-enyl]carbamate and was obtained as a light yellow solid, which was used directly without further purification. ¹H NMR (400 MHz, DMSO-*d*₆) δ 3.90 - 3.77 (m, 4H), 3.59-3.55 (m, 1H), 3.49 - 3.38 (m, 1H), 3.22 - 3.09 (m, 2H), 1.76 - 1.66 (m, 1H), 1.57 - 1.47 (m, 1H), 1.46 - 1.34 (m, 2H), 1.22(m, 9H).

### Step 3:

### 1-Amino-4-(2-methyl-1,3-dioxolan-2-yl)butan-2-ol

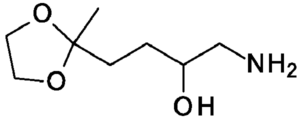

To a solution of 1-azido-4-(2-methyl-1,3-dioxolan-2-yl)butan-2-ol (3500 mg, 17.4 mmol, 1 eq) in THF (100 mL) was added Pd/C (1500 mg). The mixture was stirred at 25 °C for 2 h under hydrogen atmosphere. The mixture was filtered through celite to give a filtrate, which was concentrated to give a crude title compound (5200 mg) as a light yellow oil, which was used directly without future purification.

### Step 4:

### tert-Butyl N-[2-hydroxy-4-(2-methyl-1,3-dioxolan-2-yl)butyl]carbamate

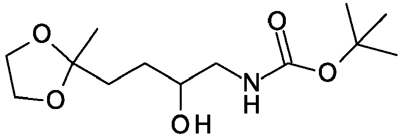

To a solution of 1-amino-4-(2-methyl-1,3-dioxolan-2-yl)butan-2-ol (5200 mg, 29.9 mmol, 1 eq) and triethylamine (12.41 mL, 89.0 mmol, 3 eq) in DCM (200 mL) was added di-tert-butyl dicarbonate (12953 mg, 59.3 mmol, 2 eq). The mixture was stirred at 25 °C for 16 h. The mixture was directly purified by flash chromatography (50-60% EtOAc in PE/EtOAc) to give the title compound (3300 mg, 12.0 mmol, 40.4% yield) as a light yellow oil. ¹H NMR (400 MHz, CDCl₃) δ 4.03 - 3.92 (m, 4H), 3.76 - 3.65 (m, 1H), 3.37 - 3.25 (m, 1H), 3.07 - 2.95 (m, 1H), 1.92 - 1.73 (m, 2H), 1.66 - 1.51 (m, 2H), 1.45 (s, 9H), 1.34 (s, 3H).

### Step 5:

### 5-[2-(2-Methyl-1,3-dioxolan-2-yl)ethyl]oxazolidin-2-one

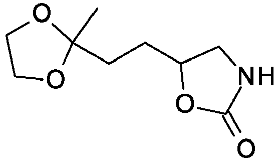

To a solution of tert-butyl N-[2-hydroxy-4-(2-methyl-1,3-dioxolan-2-yl)butyl]carbamate (3300 mg, 12.0 mmol, 1 eq) in tetrahydrofuran (100 mL) was added sodium hydride, 60% in oil (2397 mg, 59.9 mmol, 5 eq) under ice-water bath cooling. The mixture was stirred at 20 °C for 16 h. The mixture was quenched with water (5 mL) and extracted with EtOAc (2 × mL). The combined organic layer was washed with brine (5 mL), dried over Na₂SO₄ and concentrated to give a residue, which was purified by flash chromatography(EtOAc) to give title compound (1400 mg, 6.96 mmol, 58.0% yield) as a light yellow solid. ¹H NMR (400 MHz, CDCl₃) δ 4.67 - 4.52 (m, 1H), 3.93 - 3.82 (m, 4H), 3.61 (t, *J* = 8.3 Hz, 1H), 3.18 (t, *J* = 7.6 Hz, 1H), 1.90 - 1.75 (m, 2H), 1.71 - 1.58 (m, 2H), 1.28 (m, 3H).

### Step 6:

### 6-[5-[2-(2-Methyl-1,3-dioxolan-2-yl)ethyl]-2-oxo-oxazolidin-3-yl]-4-(2-trimethylsilylethoxymethyl)pyrazino[2,3-b][1,4]oxazin-3-one

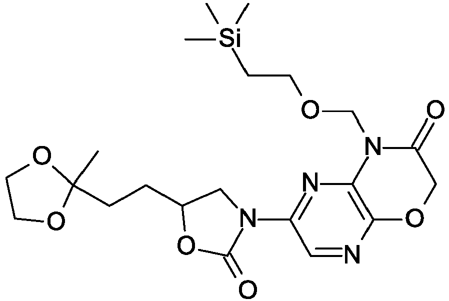

The title compound was prepared by analogy with 6-(5-allyl-2-oxo-oxazolidin-3-yl)-4-(2-trimethylsilylethoxymethyl)pyrazino[2,3-b][1,4]oxazin-3-one (Intermediate 218, Step 1) using 5-[2-(2-methyl-1,3-dioxolan-2-yl)ethyl]oxazolidin-2-one in place of 5-allyloxazolidin-2-one and was obtained as a light yellow solid. MS (ESP): m/z = 481.3 [M+H]⁺.

### Step 7:

### 6-[2-Oxo-5-(3-oxobutyl)oxazolidin-3-yl]-4H-pyrazino[2,3-b][1,4] oxazin-3-one, Enantiomer A

### 6-[2-Oxo-5-(3-oxobutyl)oxazolidin-3-yl]-4H-pyrazino[2,3-b][1,4] oxazin-3-one, Enantiomer B

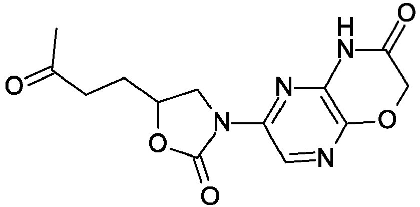

A solution of 6-[5-[2-(2-methyl-1,3-dioxolan-2-yl)ethyl]-2-oxo-oxazolidin-3-yl]-4-(2-trimethyl silylethoxymethyl)pyrazino[2,3-b][1,4]oxazin-3-one (800.0 mg, 1.66 mmol, 1 eq) in potassium carbonate (460.12 mg, 3.33 mmol, 2 eq) was stirred at 25 °C for 1 h. The solvent was removed by evaporation to give a residue, which was dissolved in EtOH (40 mL). Formic acid (0.31 mL, 8.32 mmol, 5 eq) was added under ice-water bath cooling and the mixture stirred for 0.5 h at the same temperature. After 0.5 h, the mixture concentrated and the residue purified by Prep-HPLC (formic acid system) and concentrated to give a racemic mixture of the two title compounds (400 mg, 1.31 mmol, 77.7% yield) as a white solid. MS (ESP): m/z = 307.2 [M+H]⁺. The product was separated by chiral SFC and freeze dried to give 6-[2-oxo-5-(3-oxobutyl)oxazolidin-3-yl]-4H-pyrazino[2,3-b][1,4] oxazin-3-one, Enantiomer A (160 mg, 0.520 mmol, 39.6% yield) and 6-[2-oxo-5-(3-oxobutyl)oxazolidin-3-yl]-4H-pyrazino[2,3-b][1,4] oxazin-3-one, Enantiomer B (160 mg, 0.520 mmol, 40% yield) as white solids. MS (ESP): m/z = 307.2 [M+H]⁺.

### Intermediate 240

### 6-[(5S)-5-(2-Aminoethyl)-2-oxo-oxazolidin-3-yl]-4H-pyrazino[2,3-b][1,4]oxazin-3-one; formic acid

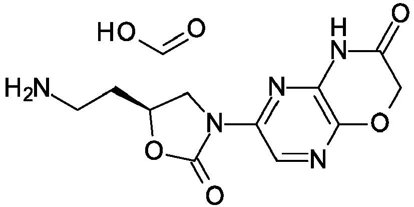

### Step 1:

### tert-Butyl N-[2-[(5S)-2-oxo-3-(3-oxo-4H-pyrazino[2,3-b][1,4]oxazin-6-yl)oxazolidin-5-yl]ethyl]carbamate

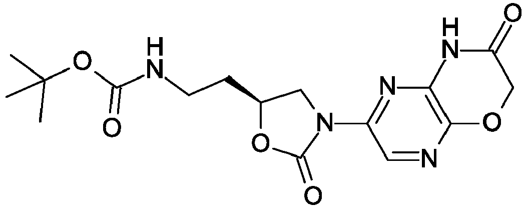

and tert-butyl N-[2-[(5*R*)-2-oxo-3-(3-oxo-4*H*-pyrazino[2,3-b][1,4]oxazin-6-yl)oxazolidin-5-yl] ethyl] carbamate

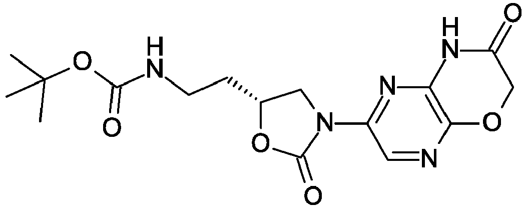

To a solution of 6-[5-(2-aminoethyl)-2-oxo-oxazolidin-3-yl]-4*H*-pyrazino[2,3-b][1,4]oxazin-3-one; 2,2,2-trifluoroacetic acid (Intermediate 24, 2200 mg, 5.59 mmol, 1 eq) in THF (80 mL) and methanol (40 mL) under ice-water bath cooling was added di-tert-butyldicarbonate (1831 mg, 8.39 mmol, 1.5 eq), then the mixture was stirred at 25 °C for 2 h. The mixture was concentrated under vacuum to give a residue. The residue was triturated with water (40 mL) and then dried under vacuum to give the crude product (2.5 g). The crude product was further triturated with EtOAc (40 mL), filtered and dried under vacuum to give the product (2.0 g). The product (1.0 g) was separated by chiral SFC to give *tert*-butyl *N*-[2-[(5*S*)-2-oxo-3-(3-oxo-4*H-*pyrazino[2,3-b][1,4]oxazin-6-yl)oxazolidin-5-yl]ethyl]carbamate (450 mg, 1.19 mmol, 21.2% yield) as a light yellow solid and tert-butyl *N*-[2-[(5*R*)-2-oxo-3-(3-oxo-4*H*-pyrazino[2,3-b][1,4]oxazin-6-yl)oxazolidin-5-yl]ethyl]carbamate (450 mg, 1.19 mmol, 21.2% yield) as a light yellow solid.

### Step 2:

### 6-[(5S)-5-(2-Aminoethyl)-2-oxo-oxazolidin-3-yl]-4H-pyrazino[2,3-b][1,4]oxazin-3-one; formic acid

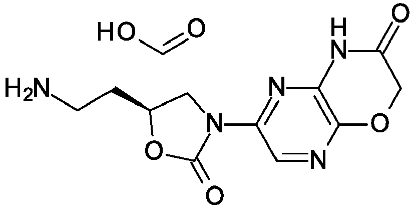

A solution of *tert*-butyl *N*-[2-[(5*S*)-2-oxo-3-(3-oxo-4*H*-pyrazino[2,3-b][1,4]oxazin-6-yl)oxazolidin-5-yl]ethyl]carbamate (450.0 mg, 1.19 mmol, 1 eq) in HCOOH (5.0 mL, 1.19 mmol, 1 eq) was stirred at 25 °C for 2 h. The mixture was concentrated under vacuum to give the title compound (370 mg, 1.14 mmol, 95.9% yield) as a light yellow solid. MS (ESP): m/z = 280.0 [M+H]⁺.

### Intermediate 241

### 6-[(5R)-5-(2-Aminoethyl)-2-oxo-oxazolidin-3-yl]-4H-pyrazino[2,3-b][1,4]oxazin-3-one; formic acid

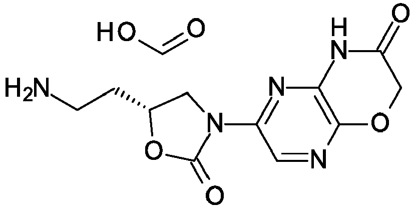

The title compound was prepared from tert-butyl *N*-[2-[(5*R*)-2-oxo-3-(3-oxo-4*H*-pyrazino[2,3-b][1,4]oxazin-6-yl)oxazolidin-5-yl]ethyl]carbamate (Intermediate 240, Step 1) by analogy with 6-[(5*S*)-5-(2-aminoethyl)-2-oxo-oxazolidin-3-yl]-4*H*-pyrazino[2,3-b][1,4]oxazin-3-one; formic acid (Intermediate 240) and was obtained as a light yellow solid.

### Intermediate 242

### 6-[5-(2-Aminoethyl)-2-oxo-1,3,4-oxadiazol-3-yl]-4-(2-trimethylsilyl ethoxymethyl)pyrazino[2,3-b][1,4]oxazin-3-one; 2,2,2-trifluoroacetic acid

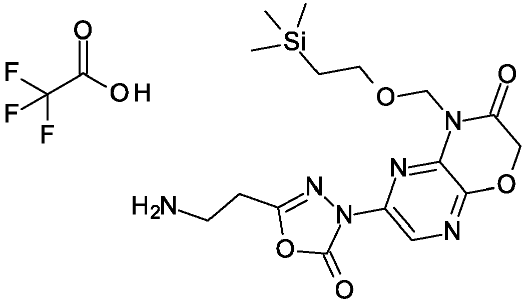

### Step 1:

### Methyl 3-(dibenzylamino)propanoate

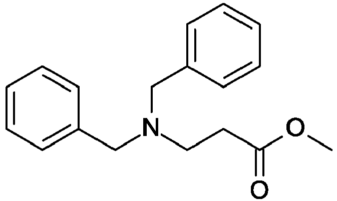

To a solution of dibenzylamine (CAS# 103-49-1, 5.04 g, 25.6 mmol, 1.1 eq) in acetonitrile (75 mL) was added 1,8-diazabicyclo[5.4.0]undec-7-ene (6.95 mL, 46.46 mmol, 2 eq) and methyl acrylate (CAS# 96-33-3, 2.0 g, 23.2 mmol, 1 eq). The reaction was stirred at 30 °C for 12 h. The reaction mixture was concentrated. The residue was purified by silica column chromatography (PE: EtOAc=30:1, TLC PE: EtOAc=5:1) and concentrated to give the title compound (3.5 g, 12.35 mmol, 26.6% yield) as a colorless oil.

### Step 2:

### 3-(Dibenzylamino)propanehydrazide

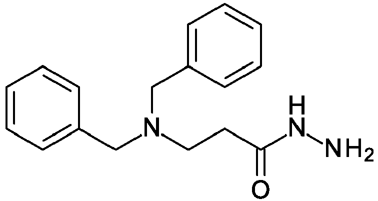

To a solution of methyl 3-(dibenzylamino)propanoate (3.5 g, 12.4 mmol, 1 eq) in ethanol (70 mL) was added hydrazine (4.04 g, 123 mmol, 10 eq) and the mixture stirred at 80 °C for 12 h. The reaction mixture was concentrated and poured into water (60 mL) and extracted with DCM (60 mL). The organic layer was dried over anhydrous Na₂SO₄ and concentrated to give the title compound (3 g, 10.59 mmol, 85.7% yield) as a light yellow gum.

### Step 3:

### 5-[2-(Dibenzylamino)ethyl]-3H-1,3,4-oxadiazol-2-one

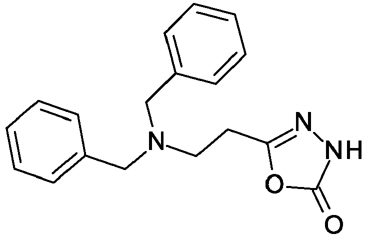

To a solution of 3-(dibenzylamino)propanehydrazide (3.0 g, 10.6 mmol, 1 eq) in dichloroethane (90 mL) was added bis(trichloromethyl)carbonate (1.26 g, 4.23 mmol, 0.40 eq) and the mixture stirred at 80 °C for 2 h. The reaction mixture was filtered and filter cake was concentrated to give the title compound (2.8 g, 9.05 mmol, 85.5% yield) as a white solid. MS (ESP): m/z = 310.0 [M+H]⁺.

### Step 4:

### 6-[5-[2-(Dibenzylamino)ethyl]-2-oxo-1,3,4-oxadiazol-3-yl]-4-(2trimethylsilylethoxymethyl) pyrazino[2,3-b][1,4]oxazin-3-one

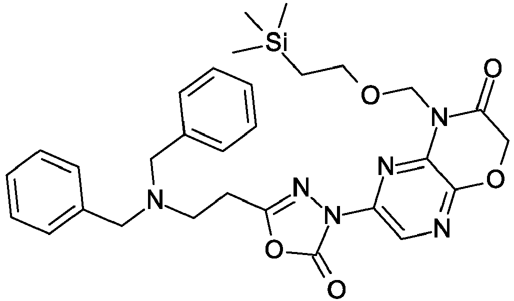

The title compound was prepared by analogy with 6-(5-allyl-2-oxo-oxazolidin-3-yl)-4-(2-trimethylsilylethoxymethyl)pyrazino[2,3-b][1,4]oxazin-3-one (Intermediate 218, Step 1) using 5-[2-(dibenzylamino)ethyl]-3*H*-1,3,4-oxadiazol-2-one in place of 5-allyloxazolidin-2-one and was obtained as a light yellow oil. MS (ESP): m/z = 589.3 [M+H]⁺.

### Step 5:

### 6-[5-(2-Aminoethyl)-2-oxo-1,3,4-oxadiazol-3-yl]-4-(2-trimethylsilyl ethoxymethyl)pyrazino[2,3-b][1,4]oxazin-3-one; 2,2,2-trifluoroacetic acid

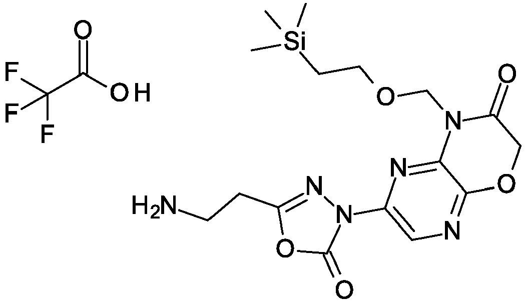

To a solution of 6-[5-[2-(dibenzylamino)ethyl]-2-oxo-1,3,4-oxadiazol-3-yl]-4-(2-trimethyl silylethoxymethyl)pyrazino[2,3-b][1,4]oxazin-3-one (1.0 g, 1.7 mmol, 1 eq) in methanol (25 mL) was added trifluoroacetic acid (0.65 mL, 8.49 mmol, 5 eq) and Pd/C(10%) (0.5 g) under H₂ (1000 mmHg) atmosphere. The reaction was stirred at 30 °C for 16 h. The mixture was filtered and evaporated to give the title compound (880 mg, 1.68 mmol, 99.1% yield) as a light yellow gum. MS (ESP): m/z = 431.1 [M+H]⁺.

### Intermediate 243

### 2-(4-Fluoro-2-formyl-indan-5-yl)oxy-N-methyl-acetamide

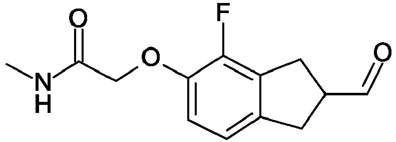

The title compound was prepared by analogy with 2-(7-fluoro-2-formyl-indan-5-yl)oxyacetamide (Intermediate 212) using 2-[[*tert*-butyl (dimethyl)silyl]oxymethyl]-4-fluoro-indan-5-ol (Intermediate 204-A) in place of 2-[[*tert*-butyl(dimethyl)silyl]oxymethyl]-7-fluoro-indan-5-ol and was obtained as a as a light yellow oil. ¹H NMR (400 MHz, CDCl₃) δ 9.78 (d, *J* = 1.2 Hz, 1H), 6.93 (d, *J* = 8.2 Hz, 1H), 6.84 - 6.74 (m, 1H), 4.51 (s, 2H), 3.41 - 3.19 (m, 4H), 2.93 (d, *J* = 5.0 Hz, 3H), 2.10 (s, 1H).

### Intermediate 244

### 6-[5-(2-Aminoethyl)-2-oxo-1,3,4-oxadiazol-3-yl]-4H-pyrazino[2,3-b][1,4] oxazin-3-one; 2,2,2-trifluoroacetic acid

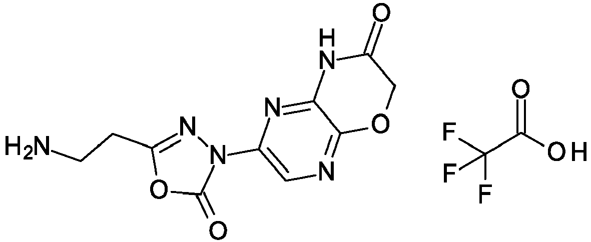

A solution of 6-[5-(2-aminoethyl)-2-oxo-1,3,4-oxadiazol-3-yl]-4-(2-trimethylsilyl ethoxy methyl)pyrazino[2,3-b][1,4]oxazin-3-one; 2,2,2-trifluoroacetic acid (Intermediate 242, 250.0 mg, 0.480 mmol, 1 eq) in CF₃COOH (4.0 mL, 0.480 mmol, 1 eq) was stirred at 70 °C for 16 h. The mixture was evaporated and extracted with EtOAc/H₂O, the aqueous phase was evaporated and MeCN (10 mL) was added, then a solid was formed. The solid was collected by filtration to give the title compound (180 mg, 0.460 mmol, 57.6% yield) as a dark brown solid. MS (ESP): m/z = 279.2 [M+H]⁺.

### Intermediate 245

### 4-Fluoro-3-methyl-6,7-dihydro-5H-cyclopenta[c]pyridine-6-carbaldehyde

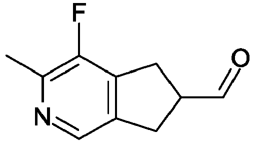

### Step 1:

### Ethyl 4-fluoro-3-methyl-6,7-dihydro-5H-cyclopenta[c]pyridine-6-carboxylate

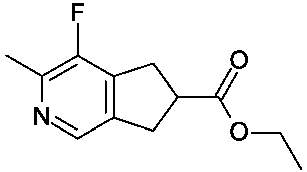

To a mixture of ethyl 1-chloro-4-fluoro-3-methyl-6,7-dihydro-5H-cyclopenta[c]pyridine-6-carboxylate (Intermediate 185, Step 7, 90.0 mg, 0.350 mmol, 1 eq) in methanol (6 mL) was added Pd/C (60.0 mg) then the reaction was stirred under H₂ balloon for 3 h. The mixture was filtered and the filtrate was concentrated under vacuum to give the title compound (60 mg, 0.270 mmol, 70.8% yield) as a light yellow oil, which was used directly without further purification. MS (ESP): m/z = 224.7 [M+H]⁺.

### Steps 2 to 3:

### 4-Fluoro-3-methyl-6,7-dihydro-5H-cyclopenta[c]pyridine-6-carbaldehyde

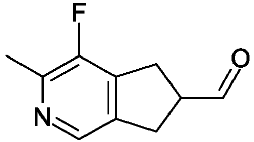

The title compound was prepared by analogy with 2-(4-chloro-2,3-dihydro-1*H*-inden-2-yl)acetaldehyde (Intermediate 11, Steps 4 and 5) and was obtained as a dark brown semi-solid. ¹H-NMR (400 MHz, CDCl₃) δ 9.71 (s, 1H), 8.10 (s, 1H), 3.31 (m, 3H), 3.08-3.20 (m, 2H), 2.41 (s, 3H).

### Intermediate 246

### 6-[5-(2-Aminoethyl)-5-methyl-2-oxo-oxazolidin-3-yl]-4H-pyrazino[2,3-b][1,4] oxazin-3-one; 2,2,2-trifluoroacetic acid

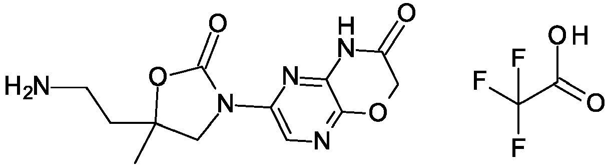

### Step 1:

### tert-Butyl N-(3-oxobutyl)carbamate

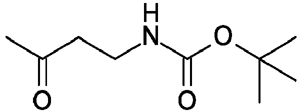

To a solution of levulinic acid (CAS# 123-76-2, 5.0 g, 43.1 mmol, 1 eq) in tert-butanol (110 mL) was added triethylamine (6.53 mL, 46.9 mmol, 1.09 eq) and was stirred at 85 °C for 2 h. The mixture was cooled to 25° C, diphenylphosphoryl azide (11.85 g, 43.1 mmol, 1 eq) was added to the mixture. The mixture was stirred at 85 °C for 18 h. The mixture was evaporated and purified by column chromatography (SiO₂, PE: EtOAc=1:1) to give the title compound (1.7 g, 9.08 mmol, 21.1% yield) as a light yellow oil. ¹H NMR (400 MHz, CDCl₃) δ 3.41 - 3.29 (m, 2H), 2.67 (t, *J* = 5.6 Hz, 2H), 2.16 (s, 3H), 1.43 (s, 9H).

### Step 2:

### Ethyl 5-(tert-butoxycarbonylamino)-3-hydroxy-3-methyl-pentanoate

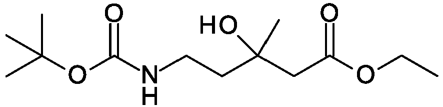

To a mixture of lithium diisopropylamide (16.0 mL, 32.0 mmol, 4 eq) in THF (75 mL) under nitrogen at -78 °C was dropwise ethyl acetate (1.6 mL, 24.0 mmol, 3 eq) in THF (20 mL). After addition, the mixture was stirred at -78 °C for 30 min. Then tert-butyl N-(3-oxobutyl) carbamate (1500 mg, 8.01 mmol, 1 eq) in THF (20 mL) was added slowly and the reaction was stirred at -78 °C for another 1.5 h. The reaction was quenched with saturated NH₄Cl solution (100 mL) then extracted with EtOAc (100 mL × 2). The organics were washed with brine (100 mL) and then dried over Na₂SO₄, concentrated to give the crude title compound (2.9 g) as a light brown oil, which was used in the next step without purification.

### Step 3:

### 5-(tert-Butoxycarbonylamino)-3-hydroxy-3-methyl-pentanoic acid

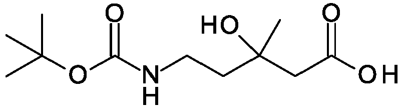

To a mixture of ethyl 5-(tert-butoxycarbonylamino)-3-hydroxy-3-methyl-pentanoate (2.9 g, 10.53 mmol, 1 eq) in THF (110 mL) and methanol (10 mL) under ice-water bath cooling was added LiOH.H₂O (2.21 g, 52.7 mmol, 5 eq) in water (55 mL). After addition, the mixture was stirred at 30 °C for another 2 h. The mixture was diluted with water (100 mL), then extracted with EtOAc (150 mL), then the aqueous phase was neutralized with HCl (1 N) to pH 3-4, extracted with EtOAc (100 mL × 2). The combined organics were dried over Na₂SO₄, then concentrated to give the title compound (1900 mg, 7.68 mmol, 72.3% yield) as a light yellow oil.

### Step 4:

### tert-Butyl N-[2-(5-methyl-2-oxo-oxazolidin-5-yl)ethyl]carbamate

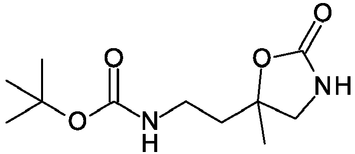

To a mixture of 5-(tert-butoxycarbonylamino)-3-hydroxy-3-methyl-pentanoic acid (1900 mg, 7.68 mmol, 1 eq) in toluene (95 mL) was added diphenylphosphonic azide (2.48 mL, 11.5 mmol, 1.5 eq) and the mixture stirred at 25 °C for 1 h. After 1 h, triethylamine (2.14 mL, 15.4 mmol, 2 eq) was added and then the mixture was stirred at 100 °C for another 16 h. The reaction was quenched with saturated NH₄Cl solution (30 mL) then extracted with EtOAc (30 mL), the organics were washed with brine (30 mL × 2) and dried over Na₂SO₄, then concentrated to give a residue. The residue was purified by flash chromatography column on silica gel (PE/EtOAc=1:1) to give the title compound (1.1 g, 4.5 mmol, 58.6% yield) as a light yellow gum. ¹H NMR (400 MHz, CDCl₃) δ 5.03 (br s, 1H), 4.83 - 4.60 (s, 1H), 3.49 (d, *J* = 8.2 Hz, 1H), 3.38 - 3.20 (m, 3H), 1.96 (t, *J* = 7.2 Hz, 2H), 1.49 (s, 3H), 1.45 (s, 9H).

### Step 5:

### tert-Butyl N-[2-[5-methyl-2-oxo-3-[3-oxo-4-(2-trimethylsilylethoxymethyl)pyrazino[2,3-b] [1,4]oxazin-6-yl]oxazolidin-5-yl]ethyl]carbamate

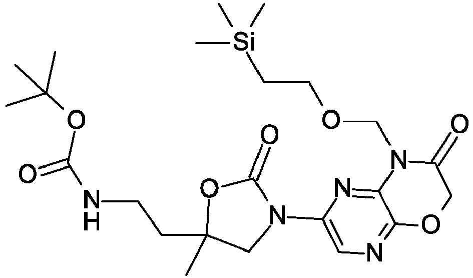

To a solution of tert-butyl N-[2-(5-methyl-2-oxo-oxazolidin-5-yl)ethyl]carbamate (504.47 mg, 2.07 mmol, 1.2 eq) and 6-bromo-4-(2-trimethylsilylethoxymethyl)pyrazino[2,3-b][1,4]oxazin-3-one (Intermediate 25, 620.0 mg, 1.72 mmol, 1 eq) in 1,4-dioxane (20 mL) was added *trans-N,N'-*dimethylcyclohexane-1,2-diamine (48.9 mg, 0.340 mmol, 0.200 eq), potassium carbonate (713.5 mg, 5.16 mmol, 3 eq) and copper (I) iodide (0.02 mL, 0.520 mmol, 0.300 eq). The mixture was stirred at 120 °C for 3 h. The mixture was filtered and the filtrate was evaporated to give the crude. The crude was purified by column chromatography(SiO₂, PE:EtOAc=3:1) to give the title compound (600 mg, 1.15 mmol, 66.6% yield) as a white solid. ¹H NMR (400 MHz, CDCl₃) δ 8.80 (s, 1H), 5.57 - 5.43 (s, 2H), 4.91 (s, 2H), 4.74 (br s, 1H), 4.06 - 3.98 (m, 1H), 3.95 - 3.88 (m, 1H), 3.75 - 3.64 (m, 2H), 3.44 - 3.22 (m, 2H), 2.08 - 1.97 (m, 2H), 1.62 - 1.53 (m, 3H), 1.44 (s, 9H), 1.00 - 0.87 (m, 2H), 0.10 -0.12 (m, 9H).

### Step 6:

### 6-[5-(2-Aminoethyl)-5-methyl-2-oxo-oxazolidin-3-yl]-4H-pyrazino[2,3-b][1,4] oxazin-3-one; 2,2,2-trifluoroacetic acid

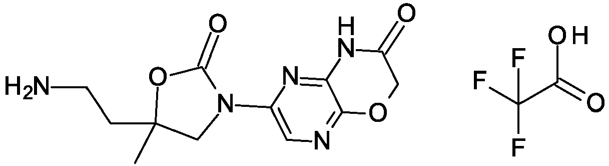

A solution of tert-butyl N-[2-[5-methyl-2-oxo-3-[3-oxo-4-(2-trimethylsilylethoxymethyl) pyrazino[2,3-b][1,4]oxazin-6-yl]oxazolidin-5-yl]ethyl]carbamate (600.0 mg, 1.15 mmol, 1 eq) in DCM (60 mL) and TFA (12.0 mL, 1.15 mmol, 1 eq) was stirred at 25 °C for 1 h, the mixture was evaporated. TFA (10.0 mL, 1.15 mmol, 1 eq) was added to the flask, the mixture was stirred at 70 °C for 24 h. The mixture was evaporated and washed with EtOAc (20 mL × 2) and extracted with H₂O (20 mL), the aqueous phase was evaporated MeCN (20 mL) was added, then a solid was formed which was collected by filtration and dried to give the title compound (200 mg, 0.490 mmol, 36.4% yield) as a dark brown solid. ¹H NMR (400 MHz, DMSO-*d*₆) δ 11.65 (s, 1H), 8.38 (s, 1H), 7.82 (br s, 3H), 4.87 (s, 2H), 3.94 - 3.82 (m, 2H), 3.02 - 2.90 (m, 2H), 2.07 (s, 2H), 1.50 (s, 3H).

### Intermediate 247

### tert-Butyl N-[2-(7-fluoro-2-formyl-indan-5-yl)oxy-1,1-dimethyl-ethyl]carbamate

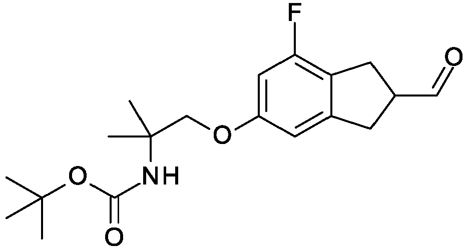

### Step 1:

### tert-Butyl N-[2-[2-[[tert-butyl(dimethyl)silyl]oxymethyl]-7-fluoro-indan-5-yl]oxy-1,1-dimethyl-ethyl] carbamate

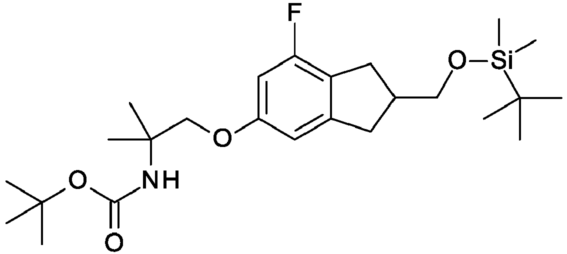

The title compound was prepared by analogy with ethyl 4-cyano-6-[2-methyl-2-[(2-methylpropan-2-yl)oxycarbonylamino]propoxy]-2,3-dihydro-1*H*-indene-2-carboxylate (Intermediate 140, Step 1) using 2-[[*tert*-butyl(dimethyl)silyl]oxymethyl]-7-fluoro-indan-5-ol (Intermediate 204) in place of ethyl 4-cyano-6-hydroxy-2,3-dihydro-1*H*-indene-2-carboxylate and was obtained as a light yellow oil. ¹H NMR (400 MHz, CDCl₃) δ 6.58 (d, *J* = 1.6 Hz, 1H), 6.49 - 6.37 (m, 1H), 4.72 (s, 1H), 3.95 - 3.84 (m, 2H), 3.65 - 3.52 (m, 2H), 3.05 - 2.88 (m, 2H), 2.78 - 2.61 (m, 3H), 1.43 (s, 9H), 1.39 (s, 6H), 0.85 (s, 9H), 0.02 (s, 6H).

### Steps 2 and 3:

### tert-Butyl N-[2-(7-fluoro-2-formyl-indan-5-yl)oxy-1,1-dimethyl-ethyl]carbamate

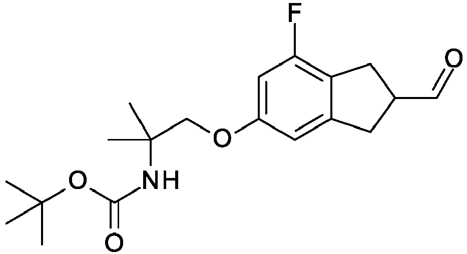

The title compound was prepared by analogy with 1-chloro-4-methoxy-6,7-dihydro-5*H-*cyclopenta[c]pyridine-6-carbaldehyde (Intermediate 43, Steps 3 and 4) and was obtained as a colorless oil. ¹H NMR (400 MHz, CDCl₃) δ 9.76 (d, *J* = 1.3 Hz, 1H), 6.61 (s, 1H), 6.53 - 6.43 (m, 1H), 4.69 (br s, 1H), 3.92 (s, 2H), 3.34 - 3.22 (m, 3H), 3.20 - 3.08 (m, 2H), 1.42 (s, 9H), 1.39 (s, 6H).

### Intermediate 248

### 4-Fluoro-6-[2-(triazol-2-yl)ethoxy]indane-2-carbaldehyde

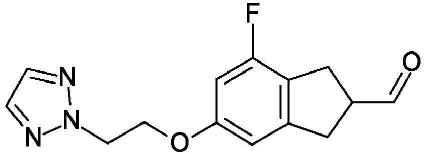

### Step 1:

### tert-Butyl-[[4-fluoro-6-[2-(triazol-1-yl)ethoxy]indan-2-yl]methoxy]-dimethyl-silane

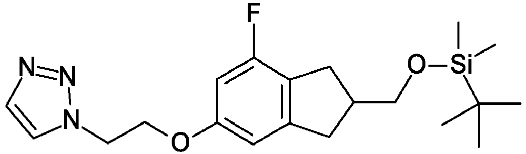

and *tert*-butyl-[[4-fluoro-6-[2-(triazol-2-yl)ethoxy]indan-2-yl]methoxy]-dimethyl-silane

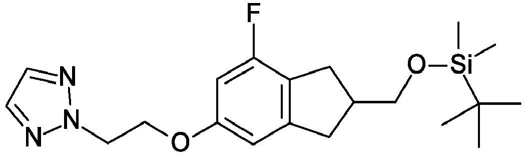

To a mixture of [6-(2-bromoethoxy)-4-fluoro-indan-2-yl]methoxy-tert-butyl-dimethyl-silane (Intermediate 217, 460.0 mg, 1.14 mmol, 1 eq) and 1/7-1,2,3-triazole (0.2 mL, 3.42 mmol, 3 eq) in MeCN (40 mL) was added K₂CO₃ (787 mg, 5.7 mmol, 5 eq) and potassium iodide (189 mg, 1.14 mmol, 1 eq), then the mixture was heated to 80 °C for 16 h. The mixture was filtered and the filtrate was concentrated under vacuum to give a residue. The residue was purified by flash chromatography on silica gel to give tert-*butyl*-[[4-fluoro-6-[2-(triazol-1-yl)ethoxy]indan-2-yl]methoxy]-dimethyl-silane (160 mg, 0.410 mmol, 35.8% yield) as a colorless oil. MS (ESP): m/z = 392.2 [M+H]⁺ and *tert*-butyl-[[4-fluoro-6-[2-(triazol-2-yl)ethoxy]indan-2-yl]methoxy]-dimethyl-silane (200 mg, 0.510 mmol, 44.8% yield) as a colorless oil. MS (ESP): m/z = 392.4 [M+H]⁺.

### Steps 2 and 3:

### 4-Fluoro-6-[2-(triazol-2-yl)ethoxy]indane-2-carbaldehyde

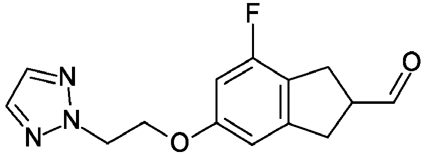

The title compound was prepared from *tert*-butyl-[[4-fluoro-6-[2-(triazol-1-yl)ethoxy]indan-2-yl]methoxy]-dimethyl-silane by analogy with 1-chloro-4-methoxy-6,7-dihydro-5*H-*cyclopenta[c]pyridine-6-carbaldehyde (Intermediate 43, Steps 3 and 4) and was obtained as a light yellow waxy solid.

### Intermediate 249

### 4-Fluoro-6-[2-(triazol-1-yl)ethoxy]indane-2-carbaldehyde

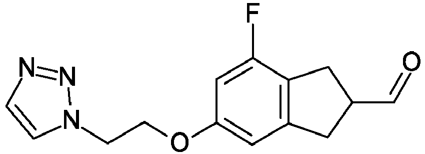

The title compound was prepared by analogy with 4-fluoro-6-[2-(triazol-2-yl)ethoxy]indane-2-carbaldehyde (Intermediate 248) using *tert*-butyl-[[4-fluoro-6-[2-(triazol-1-yl)ethoxy]indan-2-yl]methoxy]-dimethyl-silane in place of *tert*-butyl-[[4-fluoro-6-[2-(triazol-2-yl)ethoxy]indan-2-yl]methoxy]-dimethyl-silane and was obtained as a colorless oil. ¹H NMR (400 MHz, CDCl₃) δ 9.79 - 9.69 (m, 1H), 7.75 - 7.71 (m, 2H), 6.54 (s, 1H), 6.46 - 6.36 (m, 1H), 4.84 - 4.75 (m, 2H), 4.36 - 4.29 (m, 2H), 3.36 - 3.21 (m, 3H), 3.20 - 3.07 (m, 2H).

### Intermediate 250

### 6-[2-(2,5-Dioxopyrrolidin-1-yl)ethoxy]-4-fluoro-indane-2-carbaldehyde

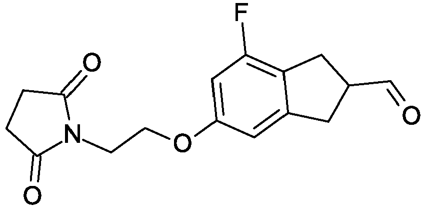

The title compound was prepared by analogy with 4-fluoro-6-[2-(2-oxooxazolidin-3-yl)ethoxy] indane-2-carbaldehyde (Intermediate 211) using succinimide (CAS# 123-56-8) in place of 2-oxazolidone and MeCN as solvent in place of DMF in Step 1, and was obtained as a colorless oil.

### Intermediate 251

### N-Cyclopropyl-2-(7-fluoro-2-formyl-indan-5-yl)oxy-acetamide

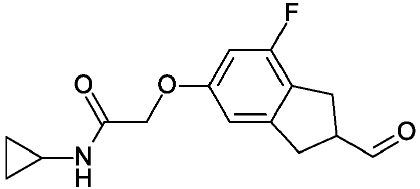

The title compound was prepared by analogy with 2-(7-fluoro-2-formyl-indan-5-yl)oxyacetamide (Intermediate 212) using 2-chloro-N-cyclopropylacetamide (CAS# 19047-31-5) in place of 2-chloroacetamide in Step 1 and was obtained as a light brown oil.

### Intermediate 252

### 4-Fluoro-6-[2-(tetrazol-1-yl)ethoxy]indane-2-carbaldehyde

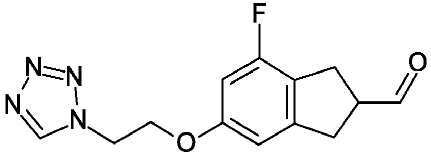

The title compound was prepared by analogy with 6-[2-(5-aminotetrazol-1-yl)ethoxy]-4-fluoro-indane-2-carbaldehyde (Intermediate 205) using 2*H*-tetrazole in place of 2*H*-tetrazol-5-amine in Step 1 and was obtained as a light yellow oil. ¹H NMR (400 MHz, CDCl₃) δ 9.68 (s, 1H), 8.69 (s, 1H), 6.48 (s, 1H), 6.34 (d, 1H), 4.77 (t, 2H), 4.27 (t, 2H), 3.12-3.23 (m, 3H), 3.04-3.08 (m, 2H).

### Intermediate 253

### 6-[2-Oxo-5-(4-piperidyl)oxazolidin-3-yl]-4H-pyrazino[2,3-b][1,4]oxazin-3-one; formic acid

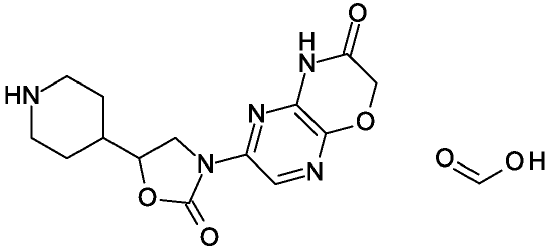

### Steps 1 to 3:

### tert-Butyl 4-(2-oxooxazolidin-5-yl)piperidine-1-carboxylate

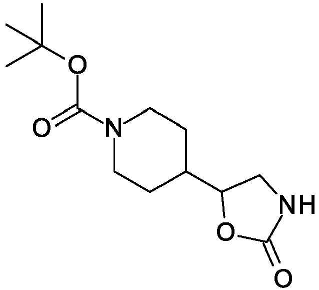

The title compound was prepared by analogy with *tert*-butyl *N*-[*trans*-3-(2-oxo-1,3-oxazolidin-5-yl)cyclobutyl]carbamate (Intermediate 7, Steps 1 to 3) using 1-BOC-4-piperidinecarboxaldehyde (CAS# 137076-22-3) in place of tert-butyl N-(trans-3-formylcyclobutyl)carbamate and was obtained as a dark brown solid. ¹H NMR (400 MHz, CDCl₃) δ 5.19 (br s, 1H), 4.41 (q, *J* = 7.7 Hz, 1H), 4.29 - 4.09 (m, 2H), 3.63 (t, *J* = 8.5 Hz, 1H), 3.36 (t, *J* = 8.0 Hz, 1H), 2.70-2.60 (m, 2H), 1.95-1.90 (m, 1H), 1.84 - 1.73 (m, 1H), 1.59 - 1.53 (m, 1H), 1.47 (s, 9H), 1.31 - 1.21 (m, 2H).

### Steps 4 to 5:

### 6-[2-Oxo-5-(4-piperidyl)oxazolidin-3-yl]-4H-pyrazino[2,3-b][1,4]oxazin-3-one; formic acid

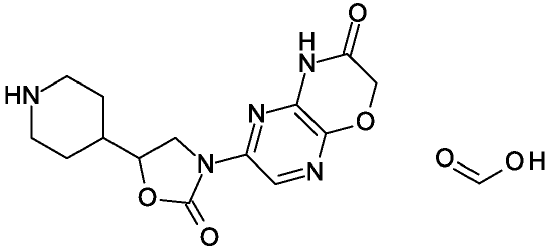

The title compound was prepared by analogy with 6-(6-oxo-5-oxa-2,7-diazaspiro[3.4]octan-7-yl)-4*H*-pyrazino[2,3-b][1,4]oxazin-3-one;formic acid (Intermediate 226, Steps 2 to 3) and was obtained as a dark brown oil. MS (ESP): m/z = 320.0 [M+H]⁺.

### Intermediate 254

### 4-Fluoro-6-[2-(tetrazol-2-yl)ethoxy]indane-2-carbaldehyde

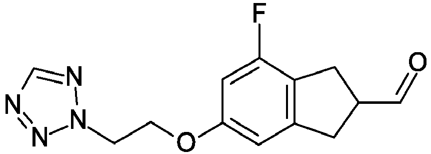

The title compound was prepared by analogy with 6-[2-(5-aminotetrazol-2-yl)ethoxy]-4-fluoro-indane-2-carbaldehyde (Intermediate 206) using 2*H*-tetrazole in place of 2*H*-tetrazol-5-amine in Step 1 and was obtained as a light yellow oil. ¹H NMR (400 MHz, CDCl₃) δ 9.66 (s, 1H), 8.47 (s, 1H), 6.47 (s, 1H), 6.35 (d, 1H), 4.95 (t, 2H), 4.43 (t, 2H), 3.16-3.24 (m, 3H), 3.03-3.09 (m, 2H).

### Intermediate 255

### 6-(2-Benzyloxypropoxy)-4-fluoro-indane-2-carbaldehyde

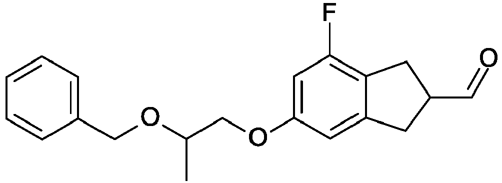

### Step 1:

### 2-Benzyloxypropyl 4-methylbenzenesulfonate

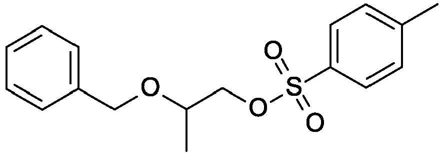

To a solution of 2-benzyloxypropan-1-ol (CAS# 33106-64-8, 550 mg, 3.31 mmol, 1 eq) and triethylamine (0.46 mL, 3.31 mmol, 1 eq) in DCM (25 mL) was added p-toluenesulfonylchloride (946 mg, 4.96 mmol, 1.5 eq) at 0 °C, and then the solution was stirred at 25 °C for 16 h. The solution was poured into water (30 mL) and extracted with DCM (50 mL × 2). The combined organic phases were dried by Na₂SO₄. The solvent was removed to give a crude, which was purified by flash chromatography (10% of EtOAc/PE) to give the title compound (325 mg, 1.01 mmol, 30.3% yield) as a colorless oil. MS (ESP): m/z = 321.2 [M+H]⁺.

### Step 2:

### [6-(2-Benzyloxypropoxy)-4-fluoro-indan-2-yl] methoxy-tert-butyl-dimethyl-silane

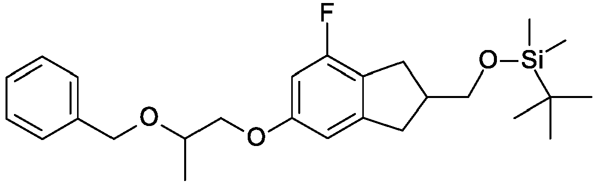

The title compound was prepared by analogy with tert-butyl 2-[(4-bromo-2-ethoxycarbonyl-indan-5-yl)oxymethyl]pyrrolidine-1-carboxylate (Intermediate 38, Step 1) using 2-[[*tert-*butyl(dimethyl)silyl]oxymethyl]-7-fluoro-indan-5-ol (Intermediate 204) in place of ethyl 4-bromo-5-hydroxy-indane-2-carboxylate and was obtained as a colorless oil. MS (ESP): m/z = 445.3 [M+H]⁺.

### Steps 3 and 4:

### 6-(2-Benzyloxypropoxy)-4-fluoro-indane-2-carbaldehyde

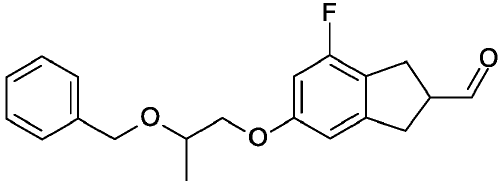

The title compound was prepared from [6-(2-benzyloxypropoxy)-4-fluoro-indan-2-yl] methoxy-tert-butyl-dimethyl-silane by analogy with 1-chloro-4-methoxy-6,7-dihydro-5*H-*cyclopenta[c]pyridine-6-carbaldehyde (Intermediate 43, Steps 3 and 4) and was obtained as a light yellow solid which was used directly without further purification.

### Intermediate 256

### 4-Fluoro-1,3-dimethyl-6,7-dihydro-5H-cyclopenta[c]pyridine-6-carbaldehyde

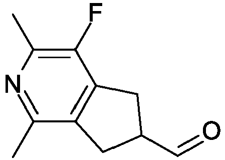

### Step 1:

### (4-Fluoro-1,3-dimethyl-6,7-dihydro-SH-cyclopenta[c]pyridin-6-yl)methanol

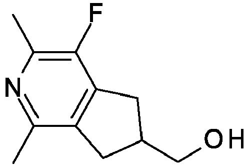

To a solution of potassium carbonate (160 mg, 1.16 mmol, 2.5 eq) in 1,4-dioxane (4 mL) was added [1,1'-bis(diphenylphosphino)ferrocene]dichloropalladium(II) (33.9 mg, 0.050 mmol, 0.100 eq), 1-chloro-4-fluoro-3-methyl-6,7-dihydro-5*H*-cyclopenta[c]pyridin-6-yl)methanol (Intermediate 185, Step 8, 100.0 mg, 0.460 mmol, 1 eq) and trimethylboroxine (0.39 mL, 1.39 mmol, 3 eq). The mixture was heated to 110 °C and stirred for 1 h. The mixture was diluted with EtOAc (70 mL) and filtered through celite. The filtrate was concentrated to give a residue, which was purified by flash chromatography (65% PE in PE/EA) to give the title compound (70 mg, 0.360 mmol, 67.3% yield) as a light green oil. MS (ESP): m/z = 196.0 [M+H]⁺.

### Step 2:

### 4-Fluoro-1,3-dimethyl-6,7-dihydro-5H-cyclopenta[c]pyridine-6-carbaldehyde

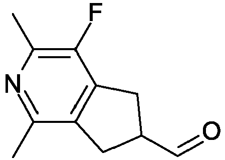

The title compound was prepared from (4-fluoro-1,3-dimethyl-6,7-dihydro-5*H-*cyclopenta[c]pyridin-6-yl)methanol by analogy with 1-chloro-4-methoxy-6,7-dihydro-5*H-*cyclopenta[c]pyridine-6-carbaldehyde (Intermediate 43, Step 4) and was obtained as a light yellow semi-solid which was used directly without further purification. ¹H NMR (400 MHz, CDCl₃) δ 9.71 (s, 1H), 3.23-3.34 (m, 2H), 3.10-3.12 (m, 3H), 2.38 (s, 3H), 2.35 (s, 3H).

### Intermediate 257

### 4-Fluoro-6-(2-methylsulfonylethoxy)indane-2-carbaldehyde

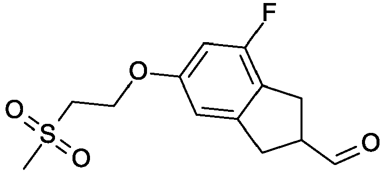

### Step 1:

### tert-Butyl-[[4-fluoro-6-(2-methylsulfonylethoxy)indan-2-yl]methoxy]-dimethyl-silane

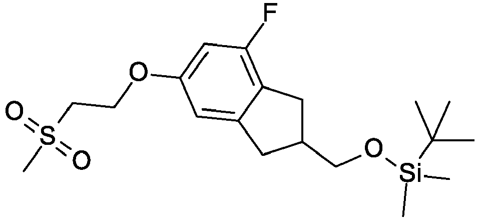

To a solution of 2-[[tert-butyl(dimethyl)silyl]oxymethyl]-7-fluoro-indan-5-ol (Intermediate 204, 200.0 mg, 0.670 mmol, 1 eq) and 2-chloroethyl methyl sulfide (CAS# 22599-69-5, 149 mg, 1.35 mmol, 2 eq) in DMF (10 mL) was added potassium carbonate (186 mg, 1.35 mmol, 2 eq) at 0 °C, then the solution was stirred at 90 °C for 16 h. The solution was poured into water (10 mL), and then extracted with EtOAc (10 mL × 2), and the combined organics dried by Na₂SO₄. The solvent was removed to give a crude, which was purified by chromatography on silica gel (PE: EtOAc=5:1) to give *tert*-butyl-[[4-fluoro-6-(2-methylsulfanylethoxy) indan-2-yl]methoxy]-dimethyl-silane (200 mg, 0.540 mmol, 80% yield) as a colorless oil. To a solution of *tert*-butyl-[[4-fluoro-6-(2-methylsulfanylethoxy)indan-2-yl]methoxy]-dimethyl-silane (200 mg, 0.540 mmol, 1 eq) in DCM (20 mL) was added 3-chloroperbenzoic acid (329 mg, 1.62 mmol, 3 eq) at 0°C, and then the solution was stirred for 2 h. The solution was poured into aq. NaHCO₃ (10 mL), and extracted with EtOAc (10 mL ×2) , the combined organics were dried by Na₂SO₄. The solvent was removed to give the crude, which was purified by chromatography on silica gel (PE: EtOAc=3:1) to give the title compound (200 mg, 0.500 mmol, 92.1% yield) as a colorless oil. MS (ESP): m/z = 425.3 [M+Na]⁺.

### Step 2:

### [4-Fluoro-6-(2-methylsulfonylethoxy)indan-2-yl]methanol

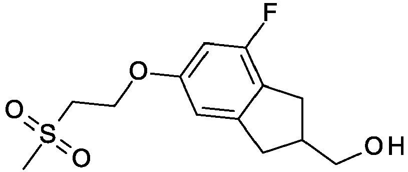

To a solution of tert-butyl-[[4-fluoro-6-(2-methylsulfonylethoxy)indan-2-yl]methoxy]-dimethyl-silane (150.0 mg, 0.370 mmol, 1 eq) in methanol (6 mL) was added HCl (gas) in MeOH (1.26 mL, 5.03 mmol, 13.5 eq) at 25 °C, and then the solution was stirred for 1 h. The solvent was removed to give a crude, which was purified by chromatography on silica gel (PE: EtOAc =1:2) to give the title compound (90 mg, 0.310 mmol, 83.8% yield) as a white solid. MS (ESP): m/z = 311.2 [M+Na]⁺.

### Step 3:

### 4-Fluoro-6-(2-methylsulfonylethoxy)indane-2-carbaldehyde

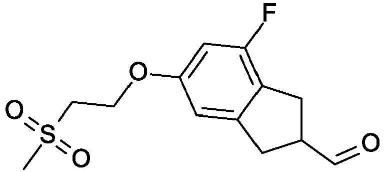

The title compound was prepared from [4-fluoro-6-(2-methylsulfonylethoxy)indan-2-yl]methanol by analogy with 1-chloro-4-methoxy-6,7-dihydro-SH-cyclopenta[c]pyridine-6-carbaldehyde (Intermediate 43, Step 4) and was obtained as a colourless oil which was used directly without further purification.

### Intermediate 258

### 6-(1-Acetylazetidin-3-yl)oxy-4-fluoro-indane-2-carbaldehyde

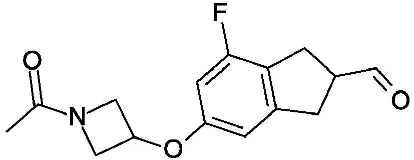

### Step 1:

### tert-Butyl 3-[2-[[tert-butyl(dimethyl)silyl]oxymethyl]-7-fluoro-indan-5-yl]oxyazetidine-1-carboxylate

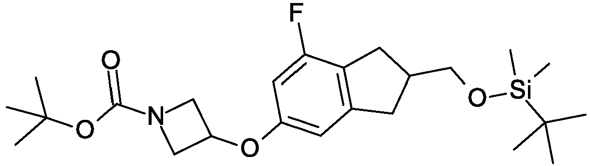

To a solution of 2-[[tert-butyl(dimethyl)silyl]oxymethyl]-7-fluoro-indan-5-ol (Intermediate 204, 400 mg, 1.35 mmol, 1 eq) and potassium bicarbonate (373 mg, 2.70 mmol, 2 eq) in NMP (10 mL) was added tert-butyl 3-bromoazetidine-1-carboxylate (CAS# 1064194-10-0, 382 mg, 1.62 mmol, 1.2 eq), and then the solution was stirred at 90 °C for 16 h. The solution was poured into water (20 mL) and then extracted with EtOAc (10 mL × 2), the organics were combined and dried by Na₂SO₄. The solvent was removed to give the crude, which was purified by chromatography on silica gel (PE: EtOAc=10:1) to give the title compound (400 mg, 0.890 mmol, 65.6% yield) as a colorless oil. MS (ESP): m/z = 474.4 [M+Na]⁺.

### Step 2:

### tert-Butyl 3-[7-fluoro-2-(hydroxymethyl)indan-5-yl]oxyazetidine-1-carboxylate

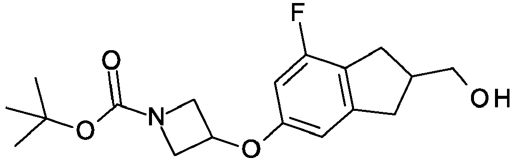

The title compound was prepared from tert-butyl 3-[2-[[tert-butyl(dimethyl)silyl]oxymethyl]-7-fluoro-indan-5-yl]oxyazetidine-1-carboxylate by analogy with (1-chloro-4-methoxy-6,7-dihydro-5*H*-cyclopenta[c]pyridin-6-yl)methanol (Intermediate 43, Step 3) and was obtained as a yellow solid. MS (ESP): m/z = 360.2 [M+Na]⁺.

### Step 3:

### [6-(Azetidin-3-yloxy)-4-fluoro-indan-2-yl]methanol hydrochloride

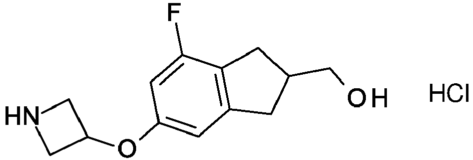

The title compound was prepared from tert-butyl 3-[7-fluoro-2-(hydroxymethyl)indan-5-yl]oxyazetidine-1-carboxylate by analogy with [4-fluoro-6-(2-methylsulfonylethoxy)indan-2-yl]metanol (Intermediate 257, Step 2) and was obtained as a a colorless oil, which was used in the next step without any further purification. MS (ESP): m/z = 238.1 [M+H]⁺.

### Step 4:

### 1-[3-[7-fluoro-2-(hydroxymethyl)indan-5-yl]oxyazetidin-1-yl]ethanone

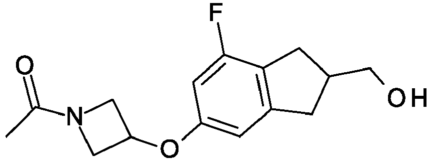

To a solution of [6-(azetidin-3-yloxy)-4-fluoro-indan-2-yl]methanol hydrochloride (80.0 mg, 0.290 mmol, 1 eq) and potassium carbonate (80.78 mg, 0.580 mmol, 2 eq) in water (5 mL) and EtOAc (5 mL) was added acetyl chloride (0.02 mL, 0.350 mmol, 1.2 eq) at 0 °C, and the mixture was stirred at 0 °C for 3 h, then stirred at 25 °C for a further 16 h. The solution was poured into water (10 mL) and extracted with EtOAc (10 mL × 2), the organics were combined and dried by Na₂SO₄. The solvent was removed to give the crude title compound (70 mg, 0.250 mmol, 85.8% yield) as a colorless oil. MS (ESP): m/z = 280.3 [M+H]⁺.

### Step 5:

### 6-(1-Acetylazetidin-3-yl)oxy-4-fluoro-indane-2-carbaldehyde

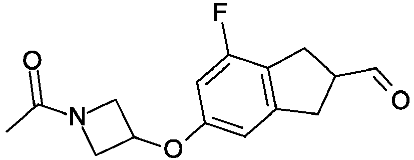

The title compound was prepared from 1-[3-[7-fluoro-2-(hydroxymethyl)indan-5-yl]oxyazetidin-1-yl]ethanone by analogy with 2-(4-chloro-2,3-dihydro-1*H*-inden-2-yl)acetaldehyde (Intermediate 11, Step 5) and was obtained as a light yellow solid which was used directly without further purification.

### Intermediate 259

### 4-Fluoro-6-(1-methylsulfonylazetidin-3-yl)oxy-indane-2-carbaldehyde

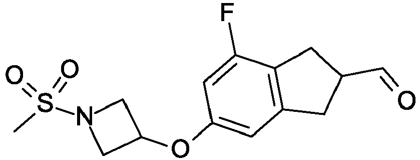

### Step 1:

### [4-Fluoro-6-(1-methylsulfonylazetidin-3-yl)oxy-indan-2-yl]methanol

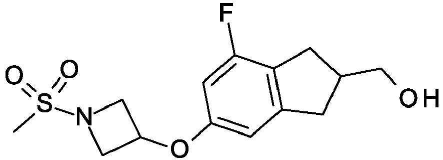

To a solution of [6-(azetidin-3-yloxy)-4-fluoro-indan-2-yl]methanol hydrochloride (Intermediate 258, Step 3, 80.0 mg, 0.290 mmol, 1 eq) and potassium carbonate (80.8 mg, 0.580 mmol, 2 eq) in water (5 mL) and EtOAc (5 mL) was added methanesulfonyl chloride (0.02 mL, 0.290 mmol, 1 eq) at 0°C, then the reaction was stirred at 0 °C for 30 min and stirred at 25 °C for further 16 h. The solution was poured into water (20 mL) and extracted with EtOAc (10 mL ×2), the organics were combined and dried by Na₂SO₄. The solvent was removed to give crude title compound (90 mg, 0.290 mmol, 97.7% yield) as a white solid. MS (ESP): m/z = 316.2 [M+H]⁺.

### Step 2:

### 4-Fluoro-6-(1-methylsulfonylazetidin-3-yl)oxy-indane-2-carbaldehyde

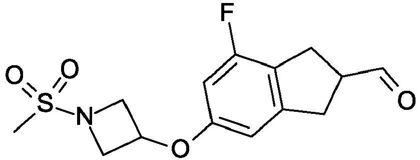

The title compound was prepared from [4-fluoro-6-(1-methylsulfonylazetidin-3-yl)oxy-indan-2-yl]methanol by analogy with 2-(4-chloro-2,3-dihydro-1*H*-inden-2-yl)acetaldehyde (Intermediate 11, Step 5) and was obtained as a white solid which was used directly without further purification.

### Intermediate 260

### tert-Butyl N-[(1S)-2-(7-fluoro-2-formyl-indan-5-yl)oxy-1-methyl-ethyl]carbamate

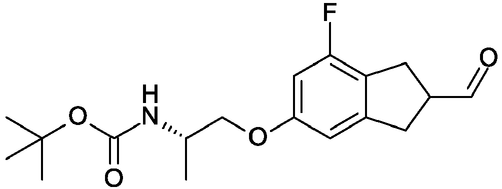

### Step 1:

### tert-Butyl N-[(1S)-2-chloro-1-methyl-ethyl]carbamate

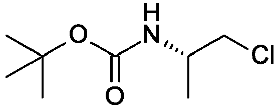

To a solution of N-Boc-L-alaninol (CAS# 106391-87-1, 2.0 g, 11.41 mmol, 1 eq) in DCM (30 mL) was added triphenylphosphine (3592 mg, 13.7 mmol, 1.2 eq) and N-chlorosuccinimide (1829 mg, 13.7 mmol, 1.2 eq) and then the solution was stirred at 25°C for 16 h. The solid was filtered off and the filtrate was concentrated to give the crude, which was purified by chromatography on silica gel (PE: EtOAc=5:1) to give the title compound (600 mg, 3.1 mmol, 27.1% yield) as a white solid. ¹H NMR (400MHz, CDCl₃) δ 4.62 (s, 1H), 4.95 (s, 1H), 3.69 - 3.52 (m, 1H), 3.52 - 3.46 (m, 1H), 1.17 (d, J=6.8 Hz, 3H).

### Step 2:

### tert-Butyl N-[(1S)-2-[2-[[tert-butyl(dimethyl)silyl]oxymethyl]-7-fluoro-indan-5-yl]oxy-1-methyl-ethyl] carbamate

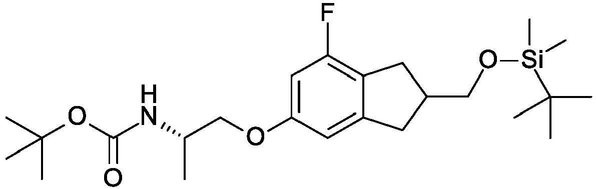

The title compound was prepared by analogy with tert-butyl-[[4-fluoro-6-(oxetan-3-yloxy)indan-2-yl]methoxy]-dimethyl-silane (Intermediate 229, Step 1) using tert-butyl N-[(1S)-2-chloro-1-methyl-ethyl]carbamate in place of oxetan-3-yl 4-methylbenzenesulfonate and was obtained as a colorless oil. MS (ESP): m/z = 476.4 [M+Na]⁺.

### Steps 3 and 4:

### tert-Butyl N-[(1S)-2-(7-fluoro-2-formyl-indan-5-yl)oxy-1-methyl-ethyl]carbamate

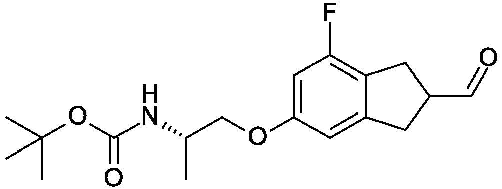

The title compound was prepared by analogy with 1-chloro-4-methoxy-6,7-dihydro-5*H-*cyclopenta[c]pyridine-6-carbaldehyde (Intermediate 43, Steps 3 and 4) and was obtained as a white solid which was used directly without any further purification.

### Intermediate 261

### tert-Butyl N-[(1R)-2-(7-fluoro-2-formyl-indan-5-yl)oxy-1-methyl-ethyl]carbamate

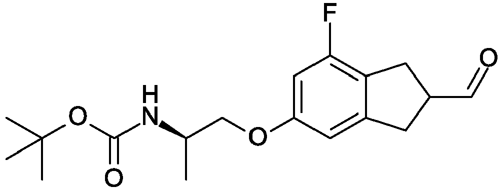

The title compound was prepared by analogy with tert-butyl *N*-[(1*S*)-2-(7-fluoro-2-formyl-indan-5-yl)oxy-1-methyl-ethyl]carbamate (Intermediate 260) using *N*-Boc-D-alaninol in place of *N-*Boc-L-alaninol and was obtained as a colourless oil.

### Intermediate 262

### tert-Butyl N-[(4-fluoro-1-methyl-6,7-dihydro-5H-cyclopenta[c]pyridin-6-yl)methyl]-N-[(2R)-2-hydroxy-2-[(5R)-2-oxooxazolidin-5-yl]ethyl]carbamate

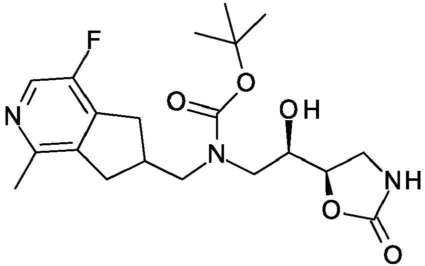

### Step 1:

### tert-Butyl N-tert-butoxycarbonyl-N-[(E)-4-chlorobut-2-enyl]carbamate

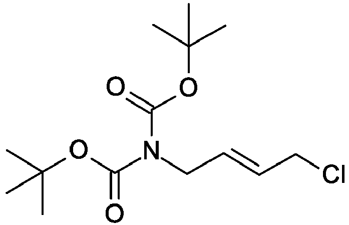

To a solution of di-tert-butyl iminodicarboxylate (5.0 g, 23.0 mmol, 1.0 eq) in DMF (100 mL) was added sodium hydride (1105 mg, 27.6 mmol, 1.2 eq) at 0°C, and then the solution was stirred at 0 °C for a further 30 min. Then a solution of trans-1,4-dichloro-2-butene (CAS# 110-57-6, 3452 mg, 27.6 mmol, 1.2 eq) in DMF (20 mL) was added and the solution was stirred at 0 °C for 10 min. The ice-bath was removed and then the solution was heated to 60 °C for 3 h. The solution was cooled to 0 °C and then was poured into aq. NH₄Cl (50 mL) and the mixture was extracted with EtOAc (20 mL × 3). The combined organics were dried by Na₂SO₄. The solvent was removed to give the crude which was purified by chromatography on silica gel (PE: EtOAc =5:1) to give the title compound (4500 mg, 14.2 mmol, 63.9% yield) as a corlorless oil. ¹H NMR (400 MHz, CDCl₃) δ 5.64-5.78 (m, 2H), 4.11 (d, J=4.4 Hz, 2H), 3.97 (d, J=5.6 Hz, 2H), 1.44 (s, 18H).

### Step 2:

### tert-Butyl (5R)-5-[(1S)-2-chloro-1-hydroxy-ethyl]-2-oxo-oxazolidine-3-carboxylate

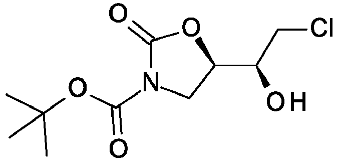

To a solution of AD-mix-β (18312 mg, 23.51 mmol, 1.8 eq) and methanesulfonamide (1244 mg, 13.08 mmol, 1 eq) in tert-butanol (60 mL) and water (60 mL) cooled to 0 °C was added potassium osmate dihydrate (482 mg, 1.31 mmol, 0.100 eq) and *tert*-butyl *N*-*tert-*butoxycarbonyl-N-[(E)-4-chlorobut-2-enyl]carbamate (4.0 g, 13.1 mmol, 1 eq), and the resulting mixture was stirred at 0 °C for 6 h. The mixture was poured into brine (20 mL) and extracted with EtOAc (20 mL × 2). The combined organics were dried by Na₂SO₄. The solvent was removed to give 4.5 g crude tert-butyl N-tert-butoxycarbonyl-N-[(2R, 3S)-4-chloro-2,3-dihydroxy-butyl] carbamate as a yellow oil. To a solution of this material in THF (100 mL) was added sodium hydride (53 mg, 1.32 mmol, 0.100 eq) at 0 °C, then the solution was stirred at 0 °C for 0.5 h. The solution was poured into ice-water (10 mL), and then extracted with EtOAc (20 mL × 2). The combined organics were dried by Na₂SO₄, then the solvent was removed to give the crude product which was purified by chromatography on silica gel (PE:EA=10:1) to give the title compound (3 g, 11.29 mmol, 85.3% yield) as a white solid. ¹H NMR (400MHz, CDCl₃) δ 4.94 (br s, 1H), 4.72 (br s, 1H), 4.60 (q, J=4.1 Hz, 1H), 3.78 - 3.63 (m, 2H), 3.55 - 3.35 (m, 2H), 1.38 (s, 9H).

### Step 3:

### tert-Butyl N-[(2R,3R)-2,3-dihydroxy-4-(4-methoxyanilino)butyl]carbamate

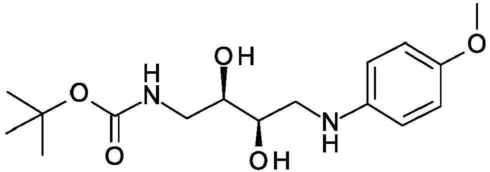

To a solution of tert-butyl (5*R*)-5-[(1*S*)-2-chloro-1-hydroxy-ethyl]-2-oxo-oxazolidine-3-carboxylate (2000 mg, 7.53 mmol, 1 eq) in methanol (80 mL) was added potassium carbonate (2079 mg, 15.1 mmol, 2 eq), and then the mixture was stirred at 25°C for 3 h. Then the solid was filtered off and the filtrate was concentrated to give crude tert-butyl *N*-[(2*R*)-2-hydroxy-2-[(2*R*)-oxiran-2-yl] ethyl] carbamate (1500 mg, 7.38 mmol, 98.1% yield) as a colorless oil. A solution of this material and *p*-anisidine (909 mg, 7.38 mmol, 1 eq) in ethanol (50 mL) and water (5 mL) was stirred at 60 °C for 16 h under N₂ atmosphere. The solid was filtered off and the filtrate was concentrated to give the crude, which was purified by chromatography on silica gel (PE: EtOAc=1:2) to give the title compound (1600 mg, 4.9 mmol, 66.4% yield) as a brown oil. MS (ESP): m/z = 327.2 [M+H]⁺.

### Step 4:

### tert-Butyl N-[[(4R,SR)-5-[(4-methoxyanilino)methyl]-2-oxo-1,3-dioxolan-4-yl]methyl]carbamate

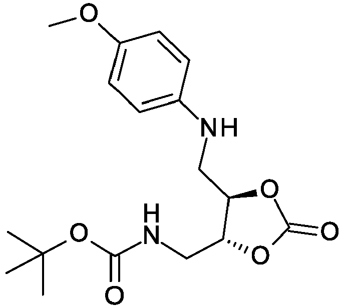

To a solution of *tert*-butyl *N*-[(2*R*,3*R*)-2,3-dihydroxy-4-(4-methoxyanilino)butyl]carbamate (1600 mg, 4.9 mmol, 1 eq) in THF (100 mL) was added N,N'-carbonyldiimidazole (953.9 mg, 5.88 mmol, 1.2 eq), and then the solution was stirred at 25 °C for 16 h. The solvent was removed and the crude was purified by chromatography on silica gel (PE: EtOAc=1: 1) to give the title compound (1100 mg, 3.12 mmol, 63.7% yield) as a white solid. MS (ESP): m/z = 353.2 [M+H]⁺.

### Step 5:

### tert-Butyl N-[(2R)-2-hydroxy-2-[(SR)-3-(4-methoxyphenyl)-2-oxo-oxazolidin-5-yl] ethyl] carbamate

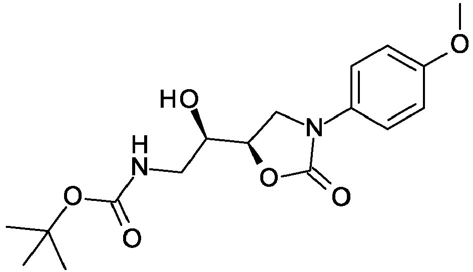

To a solution of tert-butyl *N*-[[(4*R*,5*R*)-5-[(4-methoxyanilino)methyl]-2-oxo-1,3-dioxolan-4-yl]methyl]carbamate (1100 mg, 3.12 mmol, 1 eq) and *N,N*-diisopropylethylamine (2.72 mL, 15.6 mmol, 5 eq) in DCM (80 mL) was added methyl chloroformate (700 mg, 7.41 mmol, 2.37 eq) and then the solution was stirred at 25°C for 16 h. The solution was washed with brine (10 mL) and dried with Na₂SO₄. The solvent was removed to give 1200 mg crude methyl *N*-[[(4*R*,5*R*)-5-[(*tert*-butoxycarbonylamino)methyl]-2-oxo-1,3-dioxolan-4-yl]methyl]-*N*-(4-methoxyphenyl)carbamate as a yellow oil. To a solution of this material in methanol (60 mL) was added potassium carbonate (202 mg, 1.46 mmol, 0.500 eq), and then the solution was stirred at 25 °C for 16 h. The solvent was removed to give the crude product, which was purified by chromatography on silica gel (PE: EtOAc=1:2) to give the title compound (700 mg, 1.99 mmol, 67.9% yield) as a white solid. MS (ESP): m/z = 297.1 [M+H-Bu]⁺.

### Step 6:

### (5R)-5-[(1R)-2-Amino-1-hydroxy-ethyl]-3-(4-methoxyphenyl)oxazolidin-2-one; formic acid

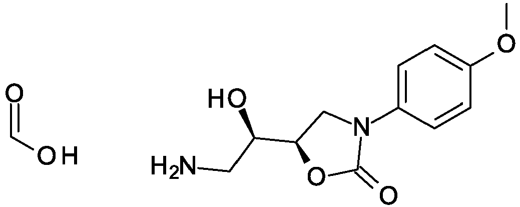

A solution of tert-butyl *N*-[(2*R*)-2-hydroxy-2-[(5*R*)-3-(4-methoxyphenyl)-2-oxo-oxazolidin-5-yl]ethyl]carbamate (400.0 mg, 1.14 mmol, 1 eq) in formic acid (1.0 mL) was stirred at 25 °C for 16 h. The solvent was removed to give the crude title compound (330 mg, 1.11 mmol, 97.5% yield) as a white solid. MS (ESP): m/z = 252.9 [M+H]⁺.

### Step 7:

### (5R)-5-[(1R)-2-[(4-Fluoro-1-methyl-6,7-dihydro-5H-cyclopenta[c]pyridin-6-yl)methylamino]-1-hydroxy-ethyl]-3-(4-methoxyphenyl)oxazolidin-2-one

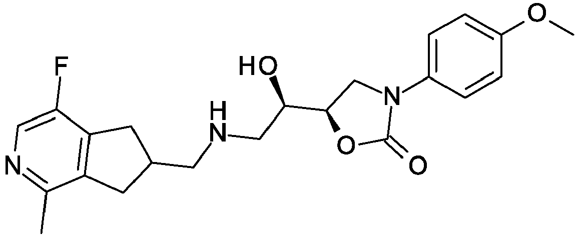

To a solution of (5*R*)-5-[(1*R*)-2-amino-1-hydroxy-ethyl]-3-(4-methoxyphenyl)oxazolidin-2-one; formic acid (332.9 mg, 1.12 mmol, 1 eq) and 4-fluoro-1-methyl-6,7-dihydro-5*H*-cyclopenta[c] pyridine-6-carbaldehyde (Intermediate 27, 200.0 mg, 1.12 mmol, 1 eq) in methanol (50 mL) was added sodium cyanoborohydride (280.6 mg, 4.46 mmol, 4 eq) at 0 °C. The mixture was stirred at 25 °C for 16 h. The solvent was removed to give a crude, which was purified by chromatography on silica gel (DCM: MeOH=10:1) to give the title compound (260 mg, 0.630 mmol, 56.1% yield) as a white solid. MS (ESP): m/z = 416.2 [M+H]⁺.

### Step 8:

### tert-Butyl N-[(4-fluoro-1-methyl-6,7-dihydro-5H-cyclopenta[c]pyridin-6-yl)methyl]-N-[(2R)-2-hydroxy-2-[(5R)-2-oxooxazolidin-5-yl]ethyl]carbamate

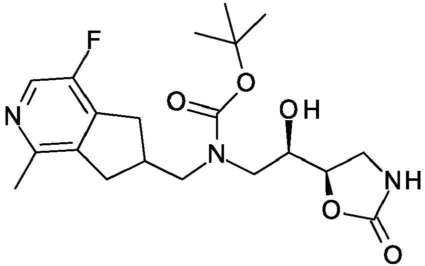

To a solution of (SR)-5-[(1R)-2-[(4-fluoro-1-methyl-6,7-dihydro-SH-cyclopenta[c]pyridin-6-yl)methylamino]-1-hydroxy-ethyl]-3-(4-methoxyphenyl)oxazolidin-2-one (260.0 mg, 0.630 mmol, 1 eq) in MeCN (20 mL) and water (5 mL) was added ammonium cerium (IV) nitrate (1374 mg, 3.13 mmol, 5 eq), and then the solution was stirred at 0 °C for 2 h. The solvent was removed by concentration and the residue was taken up in water (20 mL) and the pH of the solution was adjusted to around 10 by progressively adding aq. NaHCO₃, then the solvent was removed to give crude (5*R*)-5-[(1*R*)-2-[(4-fluoro-1-methyl-6,7-dihydro-5*H*-cyclopenta[c]pyridin-6-yl)methylamino]-1-hydroxy-ethyl]oxazolidin-2-one (200 mg) as a brown solid. To a solution of this material and sodium hydrogen carbonate (108.6 mg, 1.29 mmol, 2 eq) dissolved in water (1 mL) in MeCN (20 mL) was added di-tert-butyldicarbonate (282.2 mg, 1.29 mmol, 2 eq), then the solution was stirred at 25 °C for 16 h. The solution was poured into water (10 mL) and extracted with DCM (10 mL × 2). The organics were combined and the solvent was removed to give the crude, which was purified by chromatography on silica gel (DCM:MeOH = 20:1) to give the title compound (160 mg, 0.390 mmol, 60.4% yield) as a brown oil. MS (ESP): m/z = 410.2 [M+H]⁺.

### Intermediate 263

### 6-[5-(trans-3-Aminocyclobutyl)-2-oxo-oxazolidin-3-yl]-4H-pyrazino[2,3-b] [1,4]oxazin-3-one; formic acid

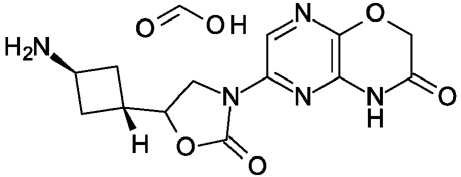

The title compound was prepared by analogy with 6-[5-(trans-3-aminocyclobutyl)-2-oxo-1,3-oxazolidin-3-yl]-4*H*-pyrido[3,2-b][1,4]oxazin-3-one; 2,2,2-trifluoroacetic acid (Intermediate 7) using 6-bromo-4*H*-pyrazino[2,3-b][1,4]oxazin-3-one in place of 6-bromo-4-(2-trimethylsilylethoxymethyl)pyrido[3,2-b][1,4]oxazin-3-one in Step 4 and formic acid in place of 2,2,2-trifluoroacetic acid in Step 5 and was obtained as a light yellow solid. MS (ESP): m/z = 306.0 [M+H]⁺.

### Intermediate 264

### 6-[5-(cis-3-Aminocyclobutyl)-2-oxo-oxazolidin-3-yl]-4H-pyrazino[2,3-b] [1,4]oxazin-3-one; formic acid

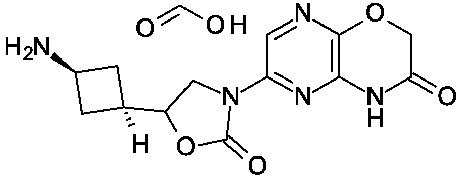

The title compound was prepared by analogy with 6-[5-(cis-3-aminocyclobutyl)-2-oxo-1,3-oxazolidin-3-yl]-4*H*-pyrido[3,2-b][1,4]oxazin-3-one; formic acid (Intermediate 10) using 6-bromo-4*H*-pyrazino[2,3-b][1,4]oxazin-3-one in place of 6-bromo-4-(2-trimethylsilylethoxymethyl)pyrido[3,2-b][1,4]oxazin-3-one in Step 4 and formic acid in place of 2,2,2-trifluoroacetic acid in Step 5 and was obtained as a yellow solid. MS (ESP): m/z = 306.6 [M+H]⁺.

### Intermediate 265

### 1-Chloro-4-fluoro-3-methyl-6,7-dihydro-5H-cyclopenta[c]pyridine-6-carbaldehyde

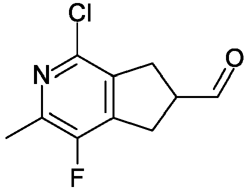

The title compound was prepared from ethyl 1-chloro-4-fluoro-3-methyl-6,7-dihydro-5*H-*cyclopenta[c]pyridine-6-carboxylate (Intermediate 185, Step 7) by analogy with 2-(4-chloro-2,3-dihydro-1*H*-inden-2-yl)acetaldehyde (Intermediate 11, Steps 4 and 5) and was obtained as a light yellow semi-solid, which was used directly without further purification. ¹H NMR (400 MHz, CDCl₃) δ 9.80 (s, 1H), 3.21-3.51 (m, 5H), 2.48 (s, 3H).

### Intermediate 266

### 4-Fluoro-6-formyl-3-methyl-6,7-dihydro-5H-cyclopenta[c]pyridine- 1-carbonitrile

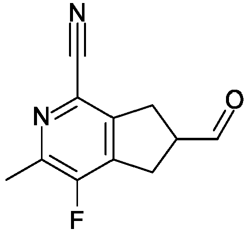

### Step 1:

### 4-Fluoro-6-(hydroxymethyl)-3-methyl-6,7-dihydro-5H-cyclopenta[c]pyridine-1-carbonitrile

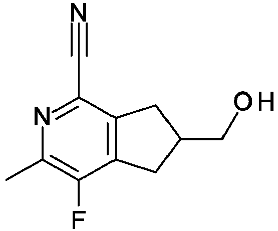

A mixture of zinc cyanide (218 mg, 1.85 mmol, 2 eq), tris(dibenzylideneacetone)dipalladium (0) (340 mg, 0.370 mmol, 0.400 eq), 2-di-tert-butylphosphino-2',4',6'-triisopropylbiphenyl (236 mg, 0.560 mmol, 0.600 eq), (1-chloro-4-fluoro-3-methyl-6,7-dihydro-5*H*-cyclopenta[c]pyridin-6-yl)methanol (Intermediate 185, Step 8, 200 mg, 0.930 mmol, 1 eq) in N,N-dimethylacetamide (8 mL) was degassed with N₂ three times. The mixture was stirred at 110 °C for 2 h. The mixture was diluted with EtOAc (10 mL) and filtered through celite. The filtrate was washed with water (2 × 10 mL), brine (10 mL), the organic layer was collected, dried and concentrated to give a residue, which was purified by flash chromatography (50% PE in PE/EA) to give the title comopund (170 mg, 0.820 mmol, 65.8% yield) as a light yellow oil. MS (ESP): m/z = 207.7 [M+H]⁺.

### Step 2:

### 4-Fluoro-6-formyl-3-methyl-6,7-dihydro-5H-cyclopenta[c]pyridine-1-carbonitrile

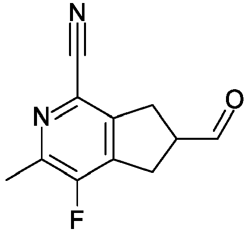

The title compound was prepared by analogy with 2-(4-chloro-2,3-dihydro-1H-inden-2-yl)acetaldehyde (Intermediate 11, Step 5) and was obtained as a light yellow oil, which was used directly without further purification. ¹H NMR (400 MHz, CDCl₃) δ 9.72 (s, 1H), 3.21-3.50 (m, 5H), 2.46 (s, 3H). 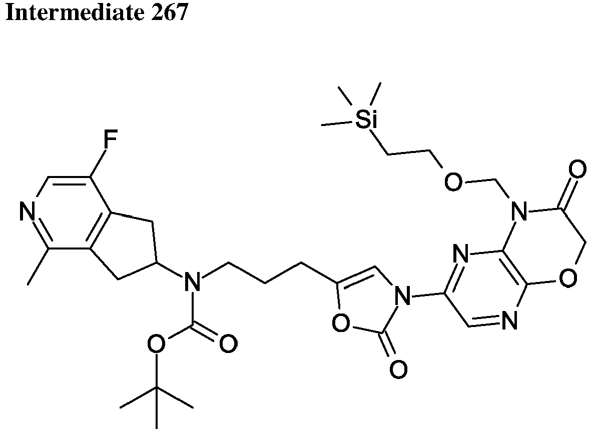 *tert*-Butyl *N*-[(4-fluoro-1-methyl-6,7-dihydro-5*H*-cyclopenta[c]pyridin-6-yl]-*N*-[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer A
and

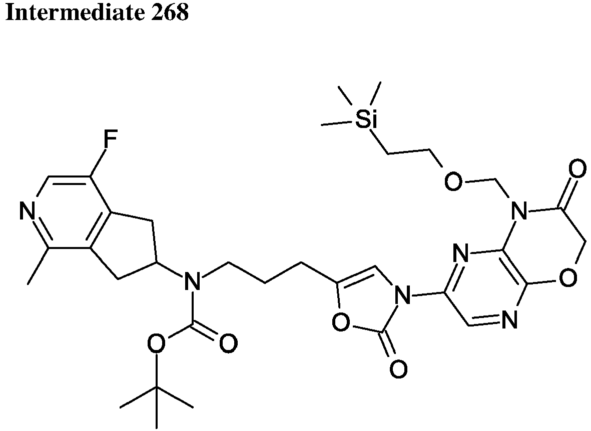

### tert-Butyl N-[(4-fluoro-1-methyl-6,7-dihydro-5H-cyclopenta[c]pyridin-6-yl]-N-[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer B

### Step 1:

### 6-[5-[3-[(4-Fluoro-1-methyl-6,7-dihydro-5H-cyclopenta[c]pyridin-6-yl)amino]propyl]-2-oxo-oxazol-3-yl]-4-(2-trimethylsilylethoxymethyl)pyrazino[2,3-b][1,4]oxazin-3-one

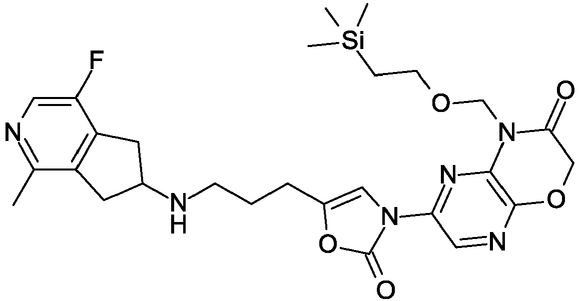

A mixture of 4-fluoro-1-methyl-6,7-dihydro-5*H*-cyclopenta[c]pyridin-6-amine (Intermediate 153, 217 mg, 1.31 mmol, 1.1 eq) and 3-[2-oxo-3-[3-oxo-4-(2-trimethylsilylethoxymethyl) pyrazino[2,3-b][1,4]oxazin-6-yl]oxazol-5-yl]propanal (Intermediate 210, 500 mg, 1.19 mmol, 1 eq) in THF (50 mL) and 2-propanol (50 mL) was stirred at 25 °C for 1 h. After 1 h, sodium cyanoborohydride (299 mg, 4.76 mmol, 4 eq) was added at 0 °C. Then the solution was stirred at 25 °C for 4 h. The mixture mixture was concentrated under vacuum to give a residue. The residue was re-dissolved in EtOAc (50 mL) and washed with brine (30 mL × 2). The combined organic phase was dried over Na₂SO₄, and concentrated under vacuum to give the crude title compound (800 mg, quant. yield) as a light brown oil. The crude product was used for the next step directly. MS (ESP): m/z = 571.5 [M+H]⁺.

### Step 2:

### tert-Butyl N-[(4-fluoro-1-methyl-6,7-dihydro-5H-cyclopenta[c]pyridin-6-yl]-N-[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer A and

### tert-Butyl N-[(4-fluoro-1-methyl-6,7-dihydro-5H-cyclopenta[c]pyridin-6-yl]-N-[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer B

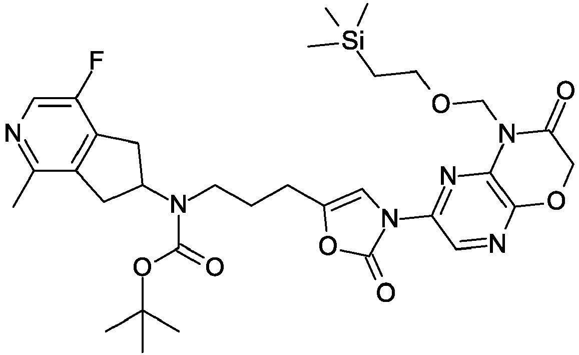

To a mixture of 6-[5-[3-[(4-fluoro-1-methyl-6,7-dihydro-5*H*-cyclopenta[c]pyridin-6-yl)amino]propyl]-2-oxo-oxazol-3-yl]-4-(2-trimethylsilylethoxymethyl)pyrazino[2,3-b][1,4]oxazin-3-one (400 mg, 0.700 mmol, 1 eq), triethylamine (0.29 mL, 2.1 mmol, 3 eq) and DMAP (11.6 mg, 0.100 mmol, 0.140 eq) in THF (20 mL) was added Boc₂O (306 mg, 1.4 mmol, 2 eq) at 0 °C. The mixture was stirred at 25 °C for 8 h. To the mixture was added EtOAc (50 mL) then washed with brine (20 mL × 3). The combined organics were dried over Na₂SO₄, and concentrated under vacuum to give a residue. The residue was purified by flash chromatography (PE/EtOAc=30%-50%) to give the racemic mixture of title compounds (290 mg, 0.430 mmol, 61.7% yield) as a white solid. MS (ESP): m/z =671.5 [M+H]⁺. The mixture was separated by chiral SFC to give *tert*-butyl *N*-[(4-fluoro-1-methyl-6,7-dihydro-*H*-cyclopenta[c]pyridin-6-yl]-*N*-[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer A (130 mg, 0.190 mmol, 44.8% yield) as a colorless oil; MS (ESP): m/z = 671.5 [M+H]⁺ and *tert*-butyl *N*-[(4-fluoro-1-methyl-6,7-dihydro-5*H-*cyclopenta[c]pyridin-6-yl]-*N*-[3-[2-oxo-3-[3-oxo-4-(2-trimethylsilylethoxymethyl)pyrazino[2,3-b][1,4]oxazin-6-yl] oxazol-5-yl]propyl]carbamate, Enantiomer B (120 mg, 0.180 mmol, 41.4% yield) as a colorless oil; MS (ESP): m/z = 671.5 [M+H]⁺.

### Intermediate 269

### 6-[5-[3-[(1-Methyl-6,7-dihydro-5H-cyclopenta[c]pyridin-6-yl)amino]propyl]-2-oxo-oxazol-3-yl]-4-(2-trimethylsilylethoxymethyl)pyrazino[2,3-b][1,4]oxazin-3-one, Enantiomer A

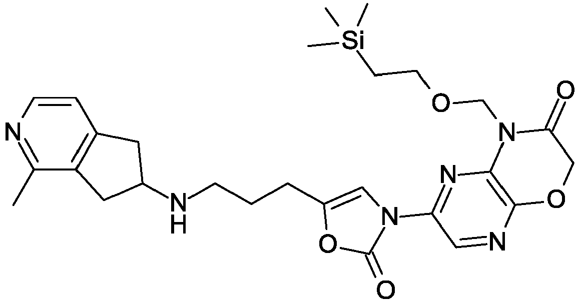

and

### Intermediate 270

### 6-[5-[3-[(1-Methyl-6,7-dihydro-5H-cyclopenta[c]pyridin-6-yl)amino]propyl]-2-oxo-oxazol-3-yl]-4-(2-trimethylsilylethoxymethyl)pyrazino[2,3-b][1,4]oxazin-3-one, Enantiomer B

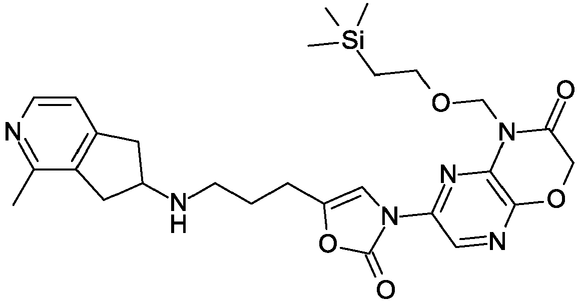

The title compounds were prepared by analogy with Intermediates 267 and 268 using 1-methyl-6,7-dihydro-5*H*-cyclopenta[c]pyridin-6-amine (Intermediate 60) in place of 4-fluoro-1-methyl-6,7-dihydro-5*H*-cyclopenta[c]pyridin-6-amine and were each obtained as a colorless oil. MS (ESP): m/z = 553.5 [M+H]⁺.

### Intermediate 271

### 2-[(7-Cyano-2-formyl-4-methyl-2,3-dihydro-1H-inden-5-yl)oxy]-N-methylacetamide

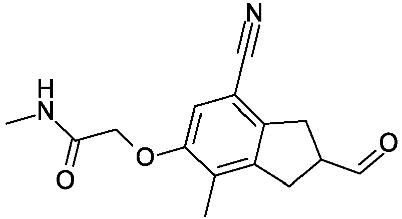

### Step 1:

### Methyl 2-prop-2-ynylhex-4-ynoate

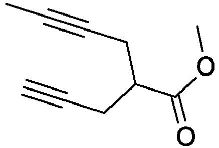

The title compound was prepared by analogy with methyl 1-chloro-3-methoxy-6,7-dihydro-5*H-*cyclopenta[c]pyridine-6-carboxylate (Intermediate 37, Step 6) using dimethyl 2-but-2-ynyl-2-prop-2-ynylpropanedioate (CAS# 188025-73-2) in place of dimethyl 1-chloro-3-methoxy-5,7-dihydrocyclopenta[c]pyridine-6,6-dicarboxylate and was obtained as a colorless liquid. MS (ESP): m/z = 165.1 [M+H]⁺.

### Step 2:

### 2-Prop-2-ynylhex-4-yn-1-ol

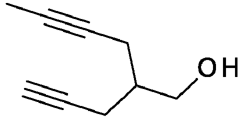

Methyl 2-prop-2-ynylhex-4-ynoate (2.43 g, 14.8 mmol, 1 eq) was combined with THF (40 mL) under inert atmosphere. The solution was cooled to -78°C and LiAlH₄ (674 mg, 17.8 mmol, 1.2 eq) was added. The mixture was then stirrd at -40°C for 2.5 h. The reaction mixture was quenched with sat. Na₂SO₄, and the mixture was filtered. The filtrate was washed with Et₂O/THF 1:1 (100 mL). The solvent was concentrated in vacuo and coevaporated with toluene to remove any water. The crude product was purified by flash chromatography (silica gel, 120 g, EtOAc in Heptane, 0-50%) to give the title compound (1.74 g, 77.7% yield) as a colorless liquid. MS (ESP): m/z = 137.1 [M+H]⁺.

### Step 3:

### tert-Butyl-dimethyl-(2-prop-2-ynylhex-4-ynoxy)silane

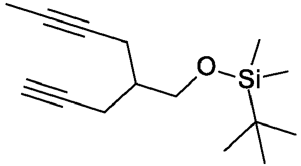

*tert*-Butylchlorodimethylsilane (1.96 g, 13 mmol, 1.2 eq) was added to an ice cold solution of 2-prop-2-ynylhex-4-yn-1-ol (1.64 g, 10.8 mmol, 1 eq) and imidazole (3.69 g, 54.2 mmol, 5 eq) in DMF (25 mL). After stirring at 0 °C for 20 minutes, the solution was allowed to warm to RT and stirred for 1 h. Water was added and the mixture was extracted with diethylether (3 x). The organic layers were washed with water (2 x) and brine, dried over Na₂SO₄ and evaporated. The residue was adsorbed on adsorbent and purified by flash chromatography (120 g silica gel; heptane/EtOAc 100:0 - 95:5) to give the title compound (1.88 g) as a colorless liquid. MS (ESP): m/z = 251.3 [M+H]⁺.

### Step 4:

### 2-(3-Bromoprop-2-ynyl)hex-4-ynoxy-tert-butyl-dimethylsilane

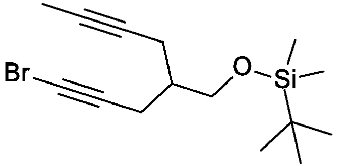

The title compound was prepared by analogy with dimethyl 2-(3-bromoprop-2-ynyl)-2-but-2-ynylpropanedioate (Intermediate 72, Step 1) using *tert*-butyl-dimethyl-(2-prop-2-ynylhex-4-ynoxy)silane in place of dimethyl 2-but-2-ynyl-2-prop-2-ynylpropanedioate and was obtained as a colorless liquid. MS (ESP): m/z = 331.1 [M+H]⁺.

### Step 5:

### [7-Bromo-4-methyl-5-(4,4,5,5-tetramethyl-1,3,2-dioxaborolan-2-yl)-2,3-dihydro-1H-inden-2-yl] methoxy-tert-butyl-dimethylsilane

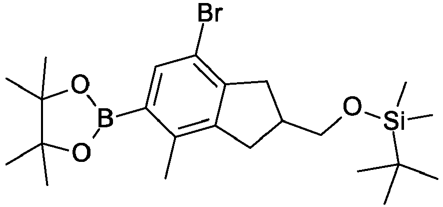

A solution of 2-(3-bromoprop-2-ynyl)hex-4-ynoxy-*tert-*butyl-dimethylsilane (222 mg, 0.674 mmol, 1 eq) in dry, degassed 1,2-dichloroethane (3.5 mL) was added over 1 h via syringe pump at RT to a solution of chloro(1,5-cyclooctadiene)(pentamethylcyclopentadienyl)ruthenium(II) (51.2 mg, 0.135 mmol, 0.2 eq) and 2-ethynyl-4,4,5,5-tetramethyl-1,3,2-dioxaborolane (205 mg, 1.35 mmol, 2 eq) in dry, degassed 1,2-dichloroethane (3 mL). The solution was stirred at RT under Ar atmosphere for 1 h. The volatiles were removed to give a residue which was adsorbed on adsorbent and purified by flash chromatography (50 g silica gel; heptane/DCM 95:5 - 50:50) to give [7-bromo-4-methyl-5-(4,4,5,5-tetramethyl-1,3,2-dioxaborolan-2-yl)-2,3-dihydro-1*H*-inden-2-yl]methoxy-*tert-*butyl-dimethylsilane (66 mg) as a light yellow gum; MS (ESP): m/z = 425.2 [M+H-tBu]⁺, and [4-bromo-7-methyl-5-(4,4,5,5-tetramethyl-1,3,2-dioxaborolan-2-yl)-2,3-dihydro-1H-inden-2-yl]methoxy-*tert*-butyl-dimethylsilane (69 mg) as a light yellow gum. MS (ESP): m/z = 425.1 [M+H-tBu]⁺.

### Step 6:

### 7-Bromo-2-[[tert-butyl(dimethyl)silyl]oxymethyl]-4-methyl-2,3-dihydro-1H-inden-5-ol

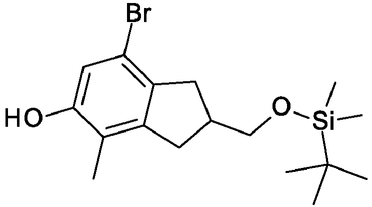

Sodium perborate monohydrate (37.9 mg, 0.380 mmol, 3 eq) was added to a mixture of [7-bromo-4-methyl-5-(4,4,5,5-tetramethyl-1,3,2-dioxaborolan-2-yl)-2,3-dihydro-1*H*-inden-2-yl]methoxy-*tert*-butyl-dimethylsilane (61 mg, 0.127 mmol, 1 eq) in THF (0.5 mL) and water (0.5 mL) and the mixture stirred for 1 h. EtOAc and NH₄Cl sat. sol. were added. The pH was adjusted to 5 with 0.1 N HCl. The mixture was extracted with EtOAc (3 x). The organic layers were washed with water (pH 5) and brine (pH 5), dried over Na₂SO₄ and evaporated to give a residue which was adsorbed on adsorbent and purified by flash chromatography (5 g silica gel; heptane/DCM 90:10 - 45:55) to give the title compound (28 mg) as a white solid. MS (ESN): m/z = 371.2 [M-H]⁻.

### Step 7:

### 2-[[7-Bromo-2-[[tert-butyl(dimethyl)silyl]oxymethyl]-4-methyl-2,3-dihydro-1H-inden-5-yl]oxy]-N-methylacetamide

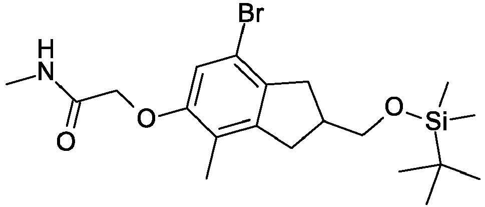

The title compound was prepared by analogy with 2-[7-fluoro-2-(hydroxymethyl)indan-5-yl]oxy-N-methyl-acetamide (Intermediate 50, Step 7) using 7-bromo-2-[[*tert-*butyl(dimethyl)silyl]oxymethyl]-4-methyl-2,3-dihydro-1*H*-inden-5-ol in place of 7-fluoro-2-(hydroxymethyl)indan-5-ol and was obtained as a white solid. MS (ESP): m/z = 444.2 [M+H]⁺.

### Step 8:

### 2-[[2-[[tert-Butyl(dimethyl)silyl]oxymethyl]-7-cyano-4-methyl-2,3-dihydro-1H-inden-5-yl]oxy]-N-methylacetamide

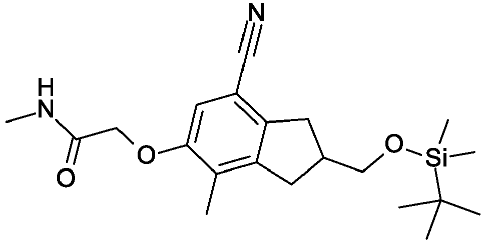

The title compound was prepared by analogy with 4-fluoro-6-(hydroxymethyl)-3-methyl-6,7-dihydro-5*H*-cyclopenta[c]pyridine-1-carbonitrile (Intermediate 265, Step 1) using 2-[[7-bromo-2-[[*tert*-butyl(dimethyl)silyl]oxymethyl]-4-methyl-2,3-dihydro-1*H*-inden-5-yl]oxy]-*N-*methylacetamide in place of (1-chloro-4-fluoro-3-methyl-6,7-dihydro-5*H*-cyclopenta[c]pyridin-6-yl)methanol and was obtained as a yellow solid. MS (ESP): m/z = 389.3 [M+H]⁺.

### Steps 9 to 10:

### 2-[(7-Cyano-2-formyl-4-methyl-2,3-dihydro-1H-inden-5-yl)oxy]-N-methylacetamide

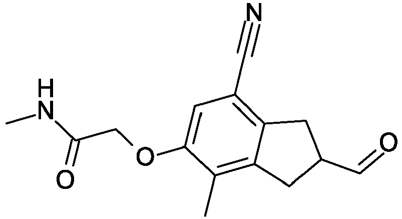

The title compound was prepared by analogy with 1-chloro-4-methoxy-6,7-dihydro-5*H-*cyclopenta[c]pyridine-6-carbaldehyde (Intermediate 43, Steps 3 and 4) and was obtained as a light brown solid. MS (ESP): m/z = 273.2 [M+H]⁺.

### Intermediate 272

### 2-[(4-Cyano-2-formyl-7-methyl-2,3-dihydro-1H-inden-5-yl)oxy]-N-methylacetamide

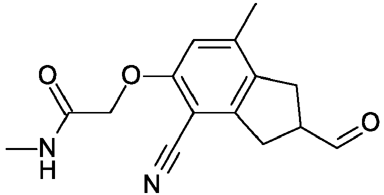

The title compound was prepared by analogy with 2-[(7-cyano-2-formyl-4-methyl-2,3-dihydro-1*H*-inden-5-yl)oxy]-*N*-methylacetamide (Intermediate 271) using [4-bromo-7-methyl-5-(4,4,5,5-tetramethyl-1,3,2-dioxaborolan-2-yl)-2,3-dihydro-1H-inden-2-yl] methoxy-*tert*-butyl-dimethylsilane in place of [7-bromo-4-methyl-5-(4,4,5,5-tetramethyl-1,3,2-dioxaborolan-2-yl)-2,3-dihydro-1H-inden-2-yl]methoxy-*tert*-butyl-dimethylsilane in Step 6 and was obtained as a light brown solid. MS (ESP): m/z = 273.2 [M+H]⁺.

### Intermediate 273

### 2-Chloro-4-methyl-6,7-dihydro-5H-cyclopenta[d]pyrimidine-6-carbaldehyde

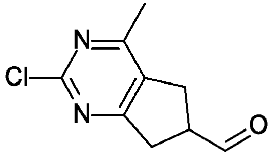

### Step 1:

### Diethyl 4-aminocyclopent-3-ene-1,3-dicarboxylate

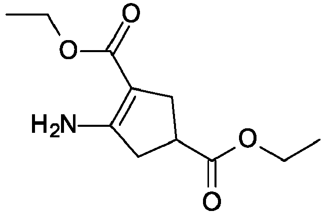

To a solution of of diethyl 4-oxocyclopentane-1,3-dicarboxylate (CAS# 54697-23-3, 2.726 g, 11.9 mmol, 1 eq) and diethyl 3-oxocyclopentane-1,2-dicarboxylate (2.726 g, 11.9 mmol, 1 eq) in EtOH (90 mL) was added ammonium acetate (4.42 g, 57.3 mmol, 4.8 eq). The reaction mixture was stirred at RT for 16 h. The reaction mixture was treated with water (50 mL) and extracted with EtOAc (2 × 100 mL). The organic layers were dried (Na₂SO₄) and evaporated. The crude material was purified by flash chromatography (silica gel, 70 g, 0% to 25% EtOAc in heptane) to give the title compound (517 mg, 26% yield) as a light brown oil. MS (ESP): m/z = 228.1 [M+H]⁺.

### Step 2:

### Ethyl 2,4-dihydroxy-6,7-dihydro-5H-cyclopenta[d]pyrimidine-6-carboxylate

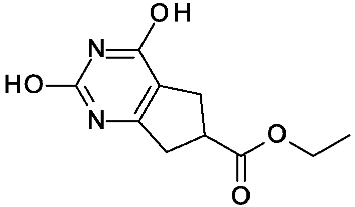

To a solution of diethyl 4-aminocyclopent-3-ene-1,3-dicarboxylate (530 mg, 2.33 mmol, 1 eq) in MeCN (11 mL) was added trichloroacetylisocyanate (823 mg, 521 µL, 4.66 mmol, 2 eq) and the reaction mixture was stirred at RT for 45 min. The reaction mixture was evaporated and dissolved in EtOH (8 mL). To the reaction mixture sodium ethoxide 21% in ethanol (1.74 g, 2 mL, 5.36 mmol, 2.3 eq) was added. The reaction was stirred at RT 2 days. The reaction mixture was neutralized with 6N HCl (894 µL) to pH 5-6 and evaporated to give the crude title compound as a light brown solid. MS (ESP): m/z = 225.1 [M+H]⁺.

### Step 3:

### Ethyl 2,4-dichloro-6,7-dihydro-5H-cyclopenta[d]pyrimidine-6-carboxylate

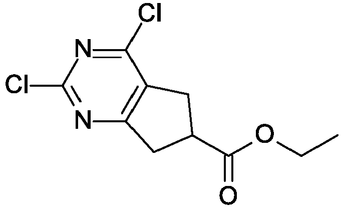

Ethyl 2,4-dihydroxy-6,7-dihydro-5*H*-cyclopenta[d]pyrimidine-6-carboxylate (820 mg, 1.46 mmol, 1 eq) was combined with phosphoryltrichloride (3 mL) to give a yellow solution. *N,N-*Diisopropylethylamine (378 mg, 501 µl, 2.93 mmol, 2 eq) was added. The reaction mixture was heated to 110 °C and stirred for 5 h. The reaction mixture was treated with 1 M NaOH (20 mL) to neutralize the reaction then the mixture was extracted with EtOAc (3 × 200 mL). The organic layers were dried (Na₂SO₄) and evaporated. The crude material was purified by flash chromatography (silica gel, 20 g, 0% to 70% EtOAc in heptane then 2-15% MeOH in DCM) to give the title compound (173 mg, 43.9% yield) as a light brown oil. MS (ESP): m/z = 261.0 [M+H]⁺.

### Step 4:

### (2,4-Dichloro-6,7-dihydro-577-cyclopenta[d]pyrimidin-6-yl)methanol

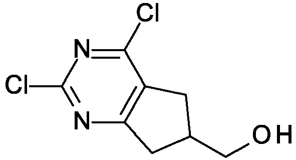

To a solution of ethyl 2,4-dichloro-6,7-dihydro-5*H*-cyclopenta[d]pyrimidine-6-carboxylate (173 mg, 663 µmol, 1 eq) in THF (3 mL) was added DIBAL-H (1M solution, 663 µL, 663 µmol, 1 eq) At -78°C. The mixture was warmed slowly up to -30°C over 1h. The reaction mixture was treated with sat. NH₄Cl (10 mL) and extracted with EtOAc (2 × 20 mL). The organic layers were washed with water (5 mL). The organic layers were dried (Na₂SO₄) and evaporated. The crude material was purified by flash chromatography (silica gel, 5g, 0% to 100% EtOAc in heptane) to give the title compound (91.5 mg) as a light brown oil. MS (ESP): m/z = 219.1 [M+H]⁺.

### Step 5:

### (2-Chloro-4-methyl-6,7-dihydro-5H-cyclopenta[d]pyrimidin-6-yl)methanol

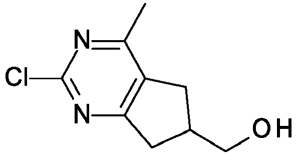

Ar was bubbled for 10 min. through a suspension of (2,4-dichloro-6,7-dihydro-5*H-*cyclopenta[d]pyrimidin-6-yl)methanol (91.5 mg, 418 µmol, 1 eq), trimethylboroxine (57.7 mg, 64.2 µl, 459 µmol, 1.1) eq and potassium carbonate (173 mg, 1.25 mmol, 3 eq) in dioxane (4.5 mL). [1,1'-bis(Diphenylphosphino)ferrocene]palladium(II) chloride 1:1 complex with DCM (42.3 mg, 0.0518 mmol, 0.124 eq) was added and the resulting brown suspension was stirred at 100 °C for 3 h. The mixture was cooled to RT, diluted with water (10 mL) and extracted with EtOAc (3 × 25 mL). The organic layers were washed with water (10 mL), dried over Na₂SO₄ and evaporated. The crude material was purified by flash chromatography (silica gel, 5g, 10% to 100% EtOAc in heptane) to give the title compound (28 mg) as a colorless waxy solid. MS (ESP): m/z = 199.1 [M+H]⁺.

### Step 6:

### 2-Chloro-4-methyl-6,7-dihydro-5H-cyclopenta[d]pyrimidine-6-carbaldehyde

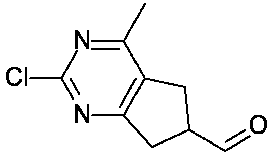

The title compound was prepared by analogy with 2-(4-chloro-2,3-dihydro-1*H*-inden-2-yl)acetaldehyde (Intermediate 11, Step 5) and was obtained as a colorless waxy solid. MS (ESP): m/z = 197.1 [M+H]⁺.

### Intermediate 274

### 2-Formyl-6-(1,3-oxazol-4-ylmethoxy)-2,3-dihydro-1H-indene-4-carbonitrile

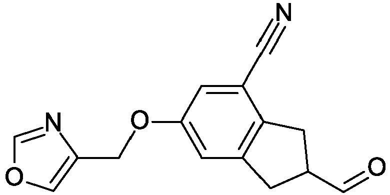

### Step 1:

### 2-[[tert-Butyl(dimethyl)silyl]oxymethyl]-2,3-dihydro-1H-indene-4-carbonitrile

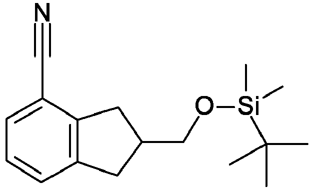

The title compound was prepared by analogy with *tert-butyl N*-[*rac*-1,2-*trans*-2-[[*tert-*butyl(dimethyl)silyl]oxymethyl]-4-chloro-2,3-dihydro-1*H*-inden-1-yl]carbamate (Intermediate 193, Step 1) using 2-(hydroxymethyl)-2,3-dihydro-1*H*-indene-4-carbonitrile (Intermediate 5, Step 2) in place of *tert*-butyl *N*-[*rac*-1,2-*trans*-4-chloro-2-(hydroxymethyl)-2,3-dihydro-1*H-*inden-1-yl]carbamate and was obtained as a colorless viscous oil. MS (ESP): m/z = 288.2 [M+H]⁺.

### Step 2:

### 2-[[tert-Butyl(dimethyl) silyl] oxymethyl] -6-hydroxy-2,3-dihydro-1H-indene-4-carbonitrile

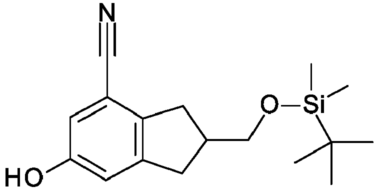

The title compound was prepared by analogy with ethyl 4-fluoro-6-hydroxy-indane-2-carboxylate (Intermediate 204, Step 1) using 2-[[tert-*b*utyl(dimethyl)silyl]oxymethyl]-2,3-dihydro-1*H*-indene-4-carbonitrile in place of ethyl 4-fluoroindane-2-carboxylate and using degassed THF as solvent, and was obtained as a white solid. MS (ESP): m/z = 304.2 [M+H]⁺.

### Step 3:

### 2-[[tert-Butyl(dimethyl)silyl]oxymethyl]-6-(1,3-oxazol-4-ylmethoxy)-2,3-dihydro-1H-indene-4-carbonitrile

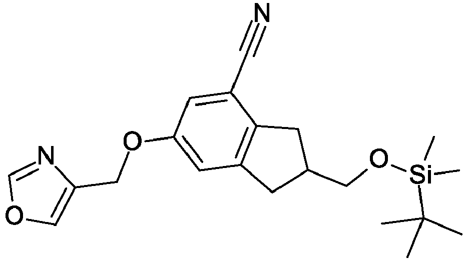

A solution of 2-[[*tert*-butyl(dimethyl)silyl]oxymethyl]-6-hydroxy-2,3-dihydro-1*H*-indene-4-carbonitrile (70 mg, 0.231 mmol, 1 eq), oxazol-4-ylmethanol (CAS# 155742-48-6, 22.9 mg, 0.231 mmol, 1 eq), triphenylphosphine (75.6 mg, 0.288 mmol, 1.25 eq) and di-tert-butyl azodicarboxylate (63.7 mg, 0.277 mmol, 1.2 eq) in THF (3 mL) was stirred at RT for 18 h. Additional oxazol-4-ylmethanol (22.9 mg, 0.231 mmol, 1 eq) (as solution in THF (0.5 mL)), triphenylphosphine (75.6 mg, 0.288 mmol, 1.25 eq), di-*tert*-butyl azodicarboxylate (63.7 mg, 0.277 mmol, 1.2 eq) were added and the solution was stirred at RT for 3 h. Water was added and the mixture was extracted with EtOAc (3 x). The organic layers were washed with water (2 x) and brine, dried over Na₂SO₄ and evaporated. The residue was adsorbed on adsorbent and purified by flash chromatography (20 g silica gel; heptane/EtOAc 95:5 - 80:20) to give the title compound (195 mg) as a white solid. MS (ESP): m/z = 385.4 [M+H]⁺.

### Steps 4 to 5:

### 2-Formyl-6-(1,3-oxazol-4-ylmethoxy)-2,3-dihydro-1H-indene-4-carbonitrile

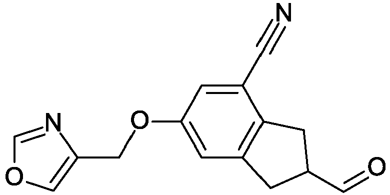

The title compound was prepared by analogy with 1-chloro-4-methoxy-6,7-dihydro-5*H-*cyclopenta[c]pyridine-6-carbaldehyde (Intermediate 43, Steps 3 to 4) and was obtained as an off-white gum. MS (ESP): m/z = 269.2 [M+H]⁺.

### Intermediate 275

### 2-Formyl-6-[(3-methyl-1,2,4-oxadiazol-5-yl)methoxy]-2,3-dihydro-1H-indene-4-carbonitrile

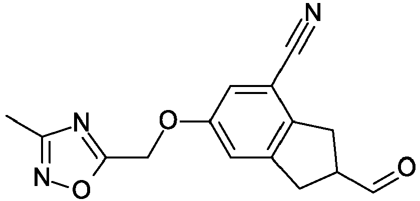

### Step 1:

### 2-[[tert-Butyl(dimethyl)silyl]oxymethyl]-6-[(3-methyl-1,2,4-oxadiazol-5-yl)methoxy]-2,3-dihydro-1H-indene-4-carbonitrile

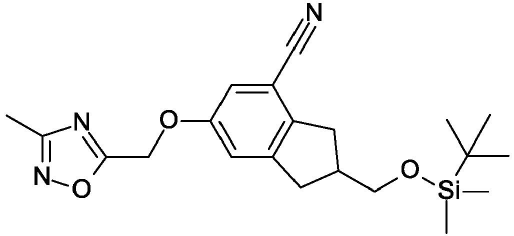

A suspension of 2-[[*tert*-butyl(dimethyl)silyl]oxymethyl]-6-hydroxy-2,3-dihydro-1*H*-indene-4-carbonitrile (Intermediate 274, Step 2, 75 mg, 0.247 mmol, 1 eq), 5-(chloromethyl)-3-methyl-1,2,4-oxadiazole (CAS# 1192-81-0, 36 mg, 0.272 mmol, 1.1 eq) and caesium carbonate (121 mg, 0.371 mmol, 1.5 eq) in MeCN (4.5 mL) was stirred at 85 °C for 6 h. The mixture was cooled to RT overnight. Water was added and the mixture was extracted with EtOAc (3 x). The organic layers were washed with water (2 x) and brine, dried over Na₂SO₄ and evaporated. The residue was purified by flash chromatography (10 g silica gel; heptane/EtOAc 95:5 - 80:20) to give the title compound (78 mg) as a colorless oil.

### Steps 2 to 3:

### 2-Formyl-6-[(3-methyl-1,2,4-oxadiazol-5-yl)methoxy]-2,3-dihydro-1H-indene-4-carbonitrile

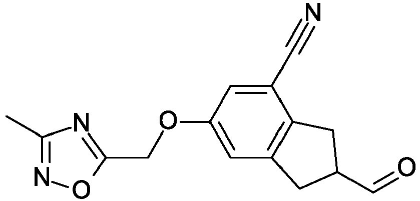

The title compound was prepared by analogy with 1-chloro-4-methoxy-6,7-dihydro-5*H-*cyclopenta[c]pyridine-6-carbaldehyde (Intermediate 43, Steps 3 to 4) and was obtained as a light brown gum. MS (ESP): m/z = 284.2 [M+H]⁺.

### Intermediate 276

### 2-Formyl-6-[(1-methylpyrazol-3-yl)methoxy]-2,3-dihydro-1H-indene-4-carbonitrile

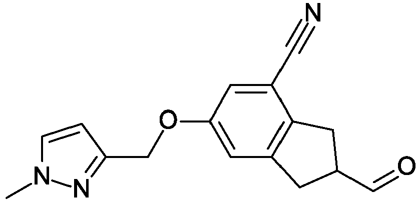

The title compound was prepared by analogy with 2-formyl-6-(1,3-oxazol-4-ylmethoxy)-2,3-dihydro-1*H*-indene-4-carbonitrile (Intermediate 274) using (1-methyl-1*H*-pyrazol-3-yl)methanol (CAS# 84547-62-6) in place of oxazol-4-ylmethanol and was obtained as a colorless gum. MS (ESP): m/z = 282.2 [M+H]⁺.

### Intermediate 277

### 2-Formyl-6-[(2-methyltetrazol-5-yl)methoxy]-2,3-dihydro-1H-indene-4-carbonitrile

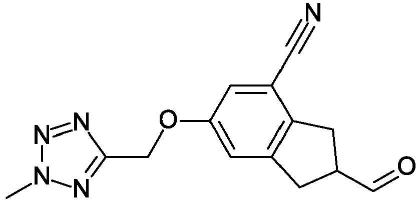

The title compound was prepared by analogy with 2-formyl-6-[(3-methyl-1,2,4-oxadiazol-5-yl)methoxy]-2,3-dihydro-1*H*-indene-4-carbonitrile (Intermediate 275) using 5-(chloromethyl)-2-methyl-2*H*-tetrazole (CAS# 55408-14-5) in place of 5-(chloromethyl)-3-methyl-1,2,4-oxadiazole and was obtained as a light yellow gum. MS (ESP): m/z = 284.2 [M+H]⁺.

### Intermediate 278

### 2-Formyl-6-[(1-methylpyrazol-4-yl)methoxy]-2,3-dihydro-1H-indene-4-carbonitrile

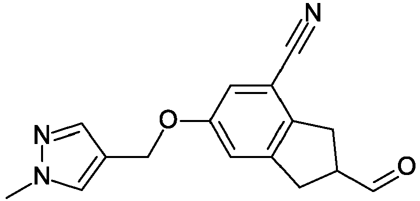

The title compound was prepared by analogy with 2-formyl-6-(1,3-oxazol-4-ylmethoxy)-2,3-dihydro-1*H*-indene-4-carbonitrile (Intermediate 274) using (1-methyl-1*H*-pyrazol-4-yl)methanol (CAS# 112029-98-8) in place of oxazol-4-ylmethanol and was obtained as a light yellow gum. MS (ESP): m/z = 282.2 [M+H]⁺.

### Intermediate 279

### 2-Formyl-6-[(1-methyltetrazol-5-yl)methoxy]-2,3-dihydro-1H-indene-4-carbonitrile

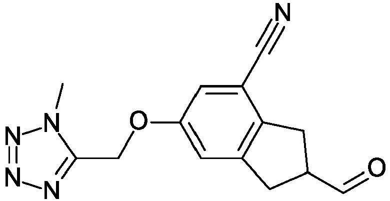

The title compound was prepared by analogy with 2-formyl-6-[(3-methyl-1,2,4-oxadiazol-5-yl)methoxy]-2,3-dihydro-1*H*-indene-4-carbonitrile (Intermediate 275) using 5-(chloromethyl)-1-methyl-1*H*-tetrazole (CAS# 57235-84-4) in place of 5-(chloromethyl)-3-methyl-1,2,4-oxadiazole and was obtained as a yellow-brown gum. MS (ESP): m/z = 284.2 [M+H]⁺.

### Intermediate 280

### 2-Formyl-6-[(4-methyl-1,2,5-oxadiazol-3-yl)methoxy]-2,3-dihydro-1H-indene-4-carbonitrile

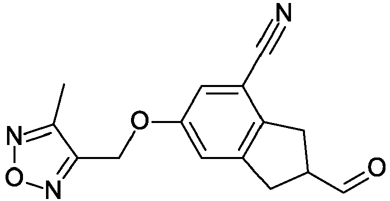

The title compound was prepared by analogy with 2-formyl-6-[(3-methyl-1,2,4-oxadiazol-5-yl)methoxy]-2,3-dihydro-1*H*-indene-4-carbonitrile (Intermediate 275) using 3-(chloromethyl)-4-methyl-1,2,5-oxadiazole (CAS# 62642-47-1) in place of 5-(chloromethyl)-3-methyl-1,2,4-oxadiazole and was obtained as a colorless oil.

### Intermediate 281

### 2-[(4,7-Difluoro-2-formyl-2,3-dihydro-1H-inden-5-yl)oxy]-N-methylacetamide

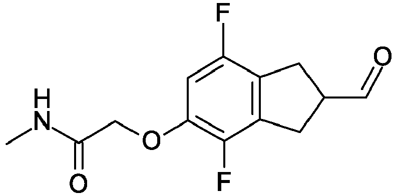

### Step 1:

### Ethyl 4,7-difluoro-5-[2-(methylamino)-2-oxoethoxy]-2,3-dihydro-1H-indene-2-carboxylate

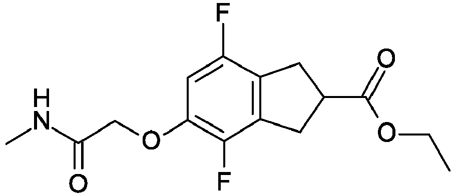

The title compound was prepared by analogy with 2-[[*tert*-butyl(dimethyl)silyl]oxymethyl]-6-[(3-methyl-1,2,4-oxadiazol-5-yl)methoxy]-2,3-dihydro-1*H*-indene-4-carbonitrile (Intermediate 275, Step 1) using ethyl 4,7-difluoro-5-hydroxy-indane-2-carboxylate (Intermediate 216, Step 6) in place of 2-[[*tert*-butyl(dimethyl)silyl]oxymethyl]-6-hydroxy-2,3-dihydro-1*H*-indene-4-carbonitrile and 2-chloro-N-methylacetamide in place of 5-(chloromethyl)-3-methyl-1,2,4-oxadiazole and was obtained as a white solid. MS (ESP): m/z = 314.2 [M+H]⁺.

### Steps 2 to 3:

### 2-[(4,7-Difluoro-2-formyl-2,3-dihydro-1H-inden-5-yl)oxy]-N-methylacetamide

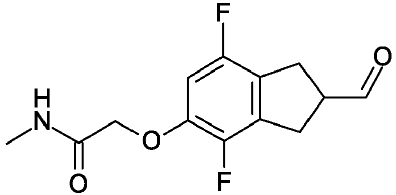

The title compound was prepared by analogy with 2-(4-chloro-2,3-dihydro-1*H*-inden-2-yl)acetaldehyde (Intermediate 11, Steps 4 to 5) and was obtained as a light brown oil. MS (ESP): m/z = 270.2 [M+H]⁺.

### Intermediate 282

### 4,7-Difluoro-5-(1H-pyrazol-3-ylmethoxy)-2,3-dihydro-1H-indene-2-carbaldehyde

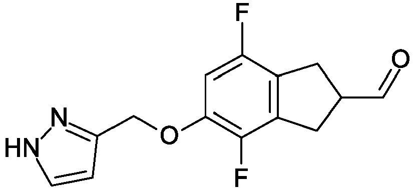

### Step 1:

### tert-Butyl 3-[(2-ethoxycarbonyl-4,7-difluoro-2,3-dihydro-1H-inden-5-yl)oxymethyl]pyrazole-1-carboxylate

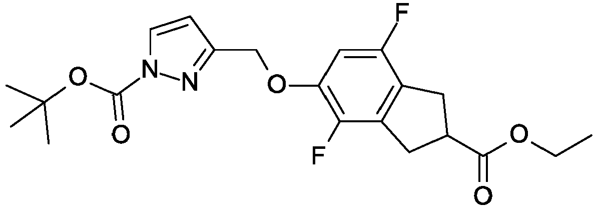

The title compound was prepared by analogy with 2-[[*tert*-butyl(dimethyl)silyl]oxymethyl]-6-(1,3-oxazol-4-ylmethoxy)-2,3-dihydro-1*H*-indene-4-carbonitrile (Intermediate 274, Step 3) using ethyl 4,7-difluoro-5-hydroxy-indane-2-carboxylate (Intermediate 216, Step 6) in place of 2-[[*tert*-butyl(dimethyl)silyl]oxymethyl]-6-hydroxy-2,3-dihydro-1*H*-indene-4-carbonitrile and *tert-*butyl 3-(hydroxymethyl)-1*H*-pyrazole-1-carboxylate (CAS# 1823967-74-3) in place of oxazol-4-ylmethanol and was obtained as a colorless oil. MS (ESN): m/z = 421.4 [M-H]⁻.

### Steps 2 to 3:

### 4,7-Difluoro-5-(1H-pyrazol-3-ylmethoxy)-2,3-dihydro-1H-indene-2-carbaldehyde

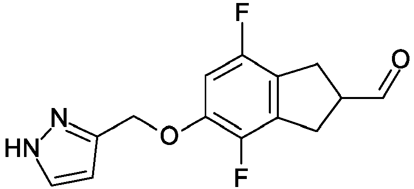

The title compound was prepared by analogy with 2-(4-chloro-2,3-dihydro-1*H*-inden-2-yl)acetaldehyde (Intermediate 11, Steps 4 to 5) and was obtained as a colorless oil. MS (ESP): m/z = 279.2 [M+H]⁺.

### Intermediate 283

### 4-Fluoro-3-methoxy-1-methyl-6,7-dihydro-5H-cyclopenta[c]pyridine-6-carbaldehyde

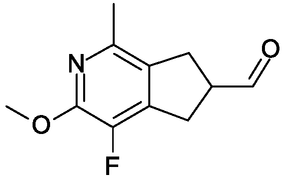

### Step 1:

### 2-Chloro-5-fluoro-6-methoxypyridine-3,4-dicarboxylic acid

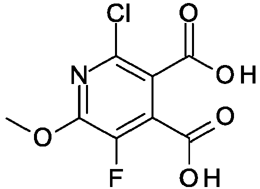

The title compound was prepared from 2-chloro-5-fluoro-6-methoxynicotinic acid (CAS# 943025-86-3) by analogy with 2-chloro-5-fluoro-6-methylpyridine-3,4-dicarboxylic acid (Intermediate 185, Step 3) and was obtained as a light yellow solid. MS (ESP): m/z = 250.0 [M+H]⁺.

### Step 2:

### Dimethyl 2-chloro-5-fluoro-6-methoxypyridine-3,4-dicarboxylate

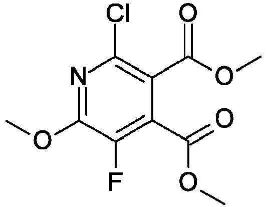

2-Chloro-5-fluoro-6-methoxypyridine-3,4-dicarboxylic acid (11.2 g, 43.5 mmol, 1 eq) was dissolved in DMF (200 mL). To the solution methyl iodide (24.7 g, 10.9 ml, 174 mmol, 4 eq) and cesium carbonate (29.8 g, 91.4 mmol, 2.1 eq) were added and and the mixture stirred overnight at room temperature. Acetic acid (5.23 g, 4.98 ml, 87.1 mmol, 2 eq) was added and the mixture stirred for two hours to quench the methyl iodide. The reaction mixture was partitioned between TBME and water. The organic layer were washed with sodium bicarbonate twice, dried over magnesium sulphate and concentrated in vacuo to give the crude title compound (11.4 g, 37 mmol, 84.9 % yield). MS (ESP): m/z = 278.1 [M+H]⁺.

### Step 3:

### (2-Chloro-5-fluoro-6-methoxypyridine-3,4-diyl)dimethanol

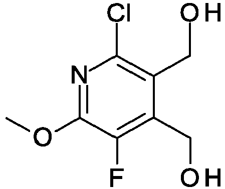

The title compound was prepared by analogy with [2-(hydroxymethyl)-4-methyl-3-pyridyl]methanol (Intermediate 15, Step 2) and was obtained as a white solid. MS (ESP): m/z = 266.1 [M+HCOO]⁺.

### Steps 4 and 5:

### Diethyl 1-chloro-4-fluoro-3-methoxy-5,7-dihydro-6H-cyclopenta[c]pyridine-6,6-dicarboxylate

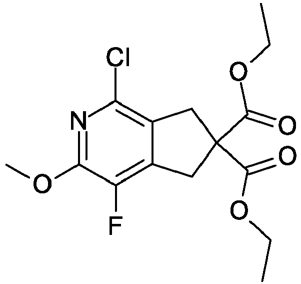

The title compound was prepared by analogy with diethyl 3-fluoro-4-methyl-5,7-dihydrocyclopenta[b]pyridine-6,6-dicarboxylate (Intermediate 209, Steps 12 and 13) using 2-butanone in place of THF and potassium carbonate in place of potassium *tert*-butoxide in Step 5 and was obtained as a colourless oil. MS (ESP): m/z = 346.1 [M+H]⁺.

### Step 6:

### Diethyl 4-fluoro-3-methoxy-1-methyl-5,7-dihydro-6H-cyclopenta[c]pyridine-6,6-dicarboxylate

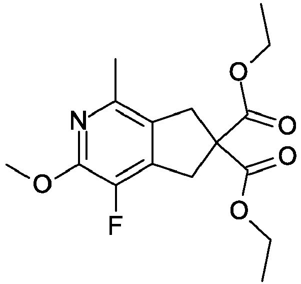

The title compound was prepared by analogy with 6-(hydroxymethyl)-1-methyl-6,7-dihydro-5*H-*cyclopenta[c]pyridine-4-carbonitrile (Intermediate 35, Step 10) and was obtained as a light yellow solid. MS (ESP): m/z = 362.1 [M+H]⁺.

### Step 7:

### Ethyl 4-fluoro-3-methoxy-1-methyl-6,7-dihydro-5H-cyclopenta[c]pyridine-6-carboxylate

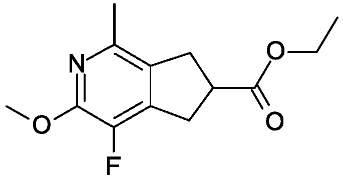

The title compound was prepared by analogy with methyl 1-chloro-3-methoxy-6,7-dihydro-5*H-*cyclopenta[c]pyridine-6-carboxylate (Intermediate 37, Step 6) and was obtained as a white solid. MS (ESP): m/z = 254.2 [M+H]⁺.

### Step 8:

### (4-Fluoro-3-methoxy-1-methyl-6,7-dihydro-5H-cyclopenta[c]pyridin-6-yl)methanol

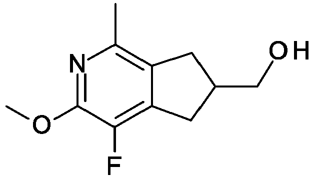

Ethyl 4-fluoro-3-methoxy-1-methyl-6,7-dihydro-5*H*-cyclopenta[c]pyridine-6-carboxylate (181 mg, 715 µmol, 1 eq) was dissolved in MeOH (3.08 mL) and to the solution sodium borohydride (81.1 mg, 2.14 mmol, 3 eq) was added. The reaction mixture was stirred at room temperature for 4 hours, heated for 30 minutes, the reaction mixture was cooled to RT and to it sodium borohydride (54.1 mg, 1.43 mmol, 2 eq) was added and the mixture stirred for 30 minutes. 1N HCl was added to neutralize the reaction mixture, then the mixture was partitioned between water and TBME (3 x), the combined organic phase was dried over magnesium sulphate, filtered and concentrated in vacuo. The crude was purified by silica gel chromatography (0 to 20% EtOAc in heptane, ISCO 20 g) to give the title compound (100 mg, 473 µmol, 66.2 % yield). MS (ESP): m/z = 212.1 [M+H]⁺.

### Step 9:

### 4-Fluoro-3-methoxy-1-methyl-6,7-dihydro-5H-cyclopenta[c]pyridine-6-carbaldehyde

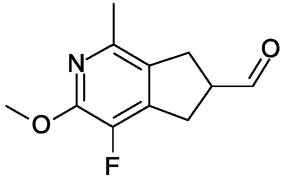

The title compound was prepared by analogy with 2-(4-chloro-2,3-dihydro-1*H*-inden-2-yl)acetaldehyde (Intermediate 11, Step 5) and was obtained as a colourless oil. ¹H NMR (400 MHz, CDCl₃) δ 9.76 (s, 1H), 3.97 (s, 3H), 3.32-3.45 (m, 2H), 3.03-3.24 (m, 3H), 2.32 (s, 3H).

### Intermediate 284

### 3-Chloro-4-fluoro-1-methyl-6,7-dihydro-5H-cyclopenta[c]pyridine-6-carbaldehyde

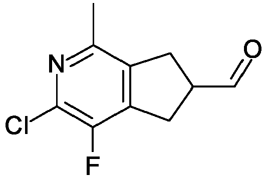

### Step 1:

### 4-Fluoro-1-methyl-3-oxo-3,5,6,7-tetrahydro-2H-cyclopenta[c]pyridine-6-carboxylic acid

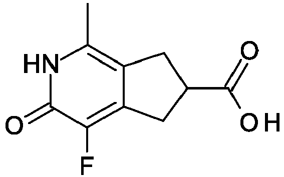

Diethyl 4-fluoro-3-methoxy-1-methyl-5,7-dihydro-6*H*-cyclopenta[c]pyridine-6,6-dicarboxylate (Intermediate 283, Step 6, 5.08 g, 15 mmol, 1 eq) was dissolved in concentrated hydrochloric acid (21.7 mL) and the mixture refluxed at 100 °C for 8 h, then at room temperature overnight and then at 100 °C for 5 h. The reaction mixture was concentrated to give the title compound (4.44 g, 15 mmol, 100 % yield) as a yellow solid. MS (ESP): m/z = 212.1 [M+H]⁺.

### Step 2:

### Methyl 4-fluoro-1-methyl-3-oxo-3,5,6,7-tetrahydro-2H-cyclopenta[c]pyridine-6-carboxylate

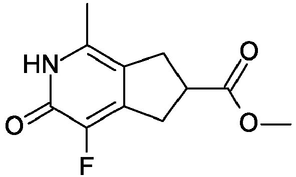

4-Fluoro-1-methyl-3-oxo-3,5,6,7-tetrahydro-2*H*-cyclopenta[c]pyridine-6-carboxylic acid (4.44 g, 15 mmol, 1 eq) was dissoved in MeOH (114 mL), and to the solution sulfuric acid (9.91 g, 5.38 ml, 101 mmol, 6.72 eq) was added at 0 °C. The reaction mixture was stirred at RT for 5 minutes and stirred at 80 °C for 3 h. Upon cooling, methanol was removed in vacuo, the reaction mixture was dissolved in water and extracted three times with DCM. The combined organic phase was concentrated to give a crude. The crude was purified by silica gel chromatograhy (0 to 10% MeOH in DCM, ISCO 70 g) to give the title compound (2.02 g, 8.7 mmol, 57.9 % yield) as off-white solid. MS (ESP): m/z = 226.1 [M+H]⁺.

### Step 3:

### Methyl 3-chloro-4-fluoro-1-methyl-6,7-dihydro-5H-cyclopenta[c]pyridine-6-carboxylate

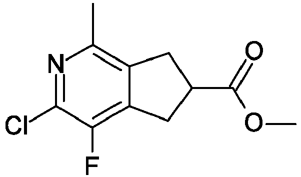

Methyl 4-fluoro-1-methyl-3-oxo-3,5,6,7-tetrahydro-2*H*-cyclopenta[c]pyridine-6-carboxylate (997 mg, 4.29 mmol, 1 eq) was dissolved in DMF (31.4 mg, 33.2 µL, 429 µmol, 0.1 eq) and phosphorus oxychloride (19.8 g, 12 mL, 129 mmol, 30 eq) at room temperature. The reaction mixture was stirred at 110 °C for 24 h, after which a drop of DMF was added and the solution stirred for 3 h at 110 °C then at RT overnight. The mixture was concentrated in vacuo and the residue was taken up in DCM, poured onto water (100 mL) and extracted twice with DCM. The organic layers were washed with brine, dried over Na₂SO₄ and concentrated in vacuo. The crude was purified by column chromatography (0 to 10% MeOH in DCM, ISCO 50 g) to give the title compound (0.955 g, 3.8 mmol, 88.5 % yield) as a yellow oil. MS (ESP): m/z = 244.1 [M+H]⁺.

### Steps 4 and 5:

### 3-Chloro-4-fluoro-1-methyl-6,7-dihydro-5H-cyclopenta[c]pyridine-6-carbaldehyde

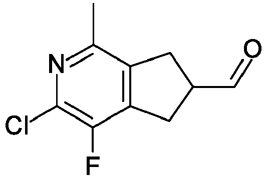

The title compound was prepared by analogy with 4-fluoro-3-methoxy-1-methyl-6,7-dihydro-5*H*-cyclopenta[c]pyridine-6-carbaldehyde (Intermediate 283, Steps 8 and 9) and was obtained as a colourless oil. ¹H NMR (400 MHz, CDCl₃) δ 9.78 (s, 1H), 3.40-3.49 (m, 3H), 3.16-3.27 (m, 2H), 2.43 (s, 3H).

### Intermediate 285

### tert-Butyl N-[rac-1,2-trans-4-chloro-2-formyl-6-[2-(methylamino)-2-oxoethoxy]-2,3-dihydro-1H-inden-1-yl]carbamate

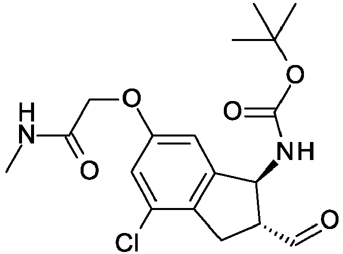

### Step 1:

### tert-Butyl N-[rac-1,2-trans-2-[[tert-butyl(dimethyl)silyl]oxymethyl]-4-chloro-6-[2-(methylamino)-2-oxoethoxy]-2,3-dihydro-1H-inden-1-yl]carbamate

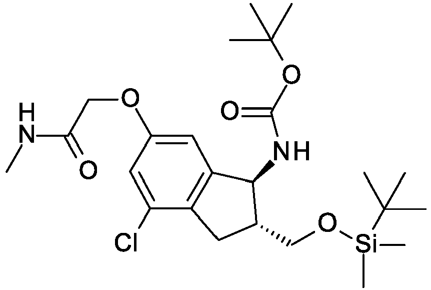

The title compound was prepared by analogy with ethyl 4-bromo-5-[2-(methylamino)-2-oxoethoxy]-2,3-dihydro-1*H*-indene-2-carboxylate (Intermediate 36, Step 4) using *tert*-butyl *N-*[*rac*-1,2-*trans*-2-[[*tert*-butyl(dimethyl)silyl]oxymethyl]-4-chloro-6-hydroxy-2,3-dihydro-1*H-*inden-1-yl]carbamate (Intermediate 193) in place of ethyl 4-bromo-5-hydroxy-2,3-dihydro-1*H-*indene-2-carboxylate and was obtained as a white solid. MS (ESP): m/z = 499.3 [M+H]⁺.

### Steps 2 and 3:

### tert-Butyl N-[rac-1,2-trans-4-chloro-2-formyl-6-[2-(methylamino)-2-oxoethoxy]-2,3-dihydro-1H-inden-1-yl]carbamate

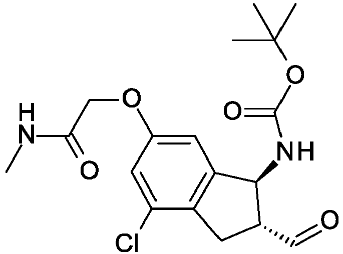

The title compound was prepared by analogy with 1-chloro-4-methoxy-6,7-dihydro-5*H-*cyclopenta[c]pyridine-6-carbaldehyde (Intermediate 43, Steps 3 and 4) and was obtained as a light yellow solid. MS (ESN): m/z = 381.1 [M-H]⁻.

### Intermediate 286

### tert-Butyl N-[(1R,2S)-4-chloro-2-formyl-2,3-dihydro-1H-inden-1-yl]carbamate

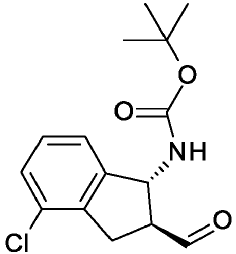

### Step 1:

### tert-Butyl N-[(1R,2S)-4-chloro-2-(hydroxymethyl)-2,3-dihydro-1H-inden-1-yl]carbamate

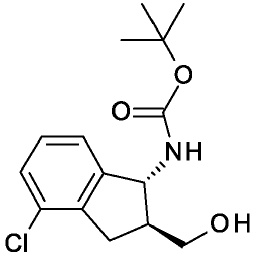

Racemic *tert*-butyl *N*-[*rac*-1,2-*trans*-4-chloro-2-(hydroxymethyl)-2,3-dihydro-1*H*-inden-1-yl]carbamate (Intermediate 88, Step 5, 5 g) was separated by chiral SFC to give *tert-*butyl *N-*[(1*S*,2*R*)-4-chloro-2-(hydroxymethyl)-2,3-dihydro-1H-inden-1-yl]carbamate (2.093 g) as an off-white solid; MS (ESP): m/z = 242.1 [M+H-tBu]⁺ and the title compound (1.462 g) as an off-white solid; MS (ESP): m/z = 242.1 [M+H-tBu]⁺. The two compounds were crystallized and the absolute configurations were determined via X-ray analysis.

### Step 2:

### tert-Butyl N-[(1R,2S)-4-chloro-2-formyl-2,3-dihydro-1H-inden-1-yl]carbamate

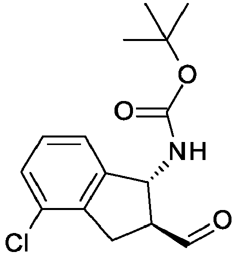

*tert*-Butyl *N*-[(1*R*,2*S*)-4-chloro-2-(hydroxymethyl)-2,3-dihydro-1*H*-inden-1-yl]carbamate (220 mg, 0.739 mmol, 1 eq) was suspended in DCM (9 mL). DMF (2.5 mL) was added until most of the solid was dissolved. The mixture was cooled to 0 °C and Dess-Martin periodinane (439 mg, 1.03 mmol, 1.4 eq) was added. After the addition the white suspension was stirred at RT for 1.5 h. The solvent was evaporated. The residue was diluted with water (basic with NaHCOs) and the mixture was extracted with EtOAc (3 x). The organic layers were washed with water (basic), water and brine, dried over Na₂SO₄ and evaporated to give the title compound (220 mg) as a light brown solid which was used crude without purification for the next step. MS (ESP): m/z = 240.1 [M+H-tBu]⁺.

### Intermediate 287

### tert-Butyl N-[(1S,2R)-4-chloro-2-formyl-2,3-dihydro-1H-inden-1-yl]carbamate

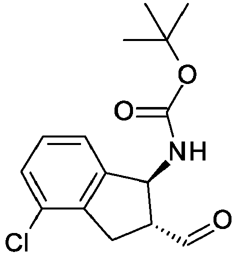

The title compound was prepared from *tert*-butyl *N*-[(1*S*,2*R*)-4-chloro-2-(hydroxymethyl)-2,3-dihydro-1*H*-inden-1-yl]carbamate (Intermediate 286, Step 1) by analogy with *tert*-butyl *N-*[(1*R*,2*S*)-4-chloro-2-formyl-2,3-dihydro-1*H*-inden-1-yl]carbamate (Intermediate 286, Step 2) and was obtained as an off-white solid. MS (ESP): m/z = 240.0 [M+H-tBu]⁺.

### Intermediate 288

### tert-Butyl 3-[(7-cyano-2-formyl-2,3-dihydro-1H-inden-5-yl)oxymethyl]pyrazole-1-carboxylate

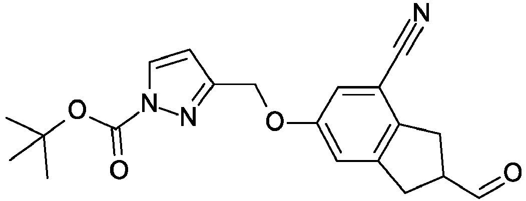

The title compound was prepared by analogy with 2-formyl-6-(1,3-oxazol-4-ylmethoxy)-2,3-dihydro-1*H*-indene-4-carbonitrile (Intermediate 274) using *tert*-butyl 3-(hydroxymethyl)pyrazole-1-carboxylate (CAS# 1823967-74-3) in place of oxazol-4-ylmethanol and was obtained as an off-white solid which was used crude without purification for the next step. MS (ESP): m/z = 268.2 [M+H-BOC]⁺.

### Intermediate 289

### tert-Butyl 4-[(7-cyano-2-formyl-2,3-dihydro-1H-inden-5-yl)oxymethyl]pyrazole-1-carboxylate

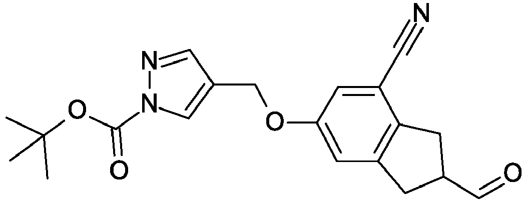

The title compound was prepared by analogy with 2-formyl-6-(1,3-oxazol-4-ylmethoxy)-2,3-dihydro-1*H*-indene-4-carbonitrile (Intermediate 274) using tert-butyl 4-(hydroxymethyl)pyrazole-1-carboxylate (CAS# 199003-22-0) in place of oxazol-4-ylmethanol and was obtained as an off-white solid which was used crude without purification for the next step. MS (ESP): m/z = 268.2 [M+H-BOC]⁺.

### Intermediate 290

### tert-Butyl 4-[(7-cyano-2-formyl-2,3-dihydro-1H-inden-5-yl)oxymethyl]imidazole-1-carboxylate

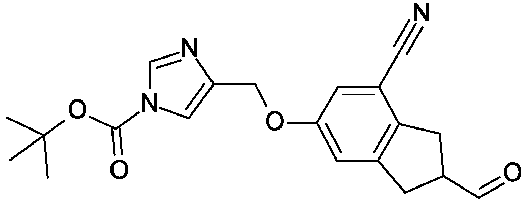

The title compound was prepared by analogy with 2-formyl-6-(1,3-oxazol-4-ylmethoxy)-2,3-dihydro-1*H*-indene-4-carbonitrile (Intermediate 274) using *tert*-butyl 4-(hydroxymethyl)imidazole-1-carboxylate (CAS# 120277-50-1) in place of oxazol-4-ylmethanol and was obtained as an off-white solid which was used crude without purification for the next step. MS (ESP): m/z = 268.2 [M+H-BOC]⁺.

### Intermediate 291

### tert-Butyl N-[1-[(4,7-difluoro-2-formyl-2,3-dihydro-1H-inden-5-yl)oxymethyl]cyclopropyl]carbamate

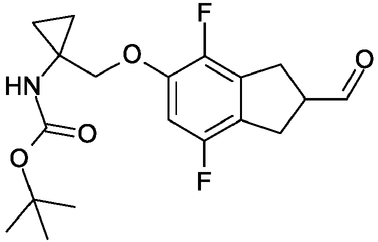

### Step 1:

### Ethyl 4,7-difluoro-5-[[1-[(2-methylpropan-2-yl)oxycarbonylamino]cyclopropyl]methoxy]-2,3-dihydro-1H-indene-2-carboxylate

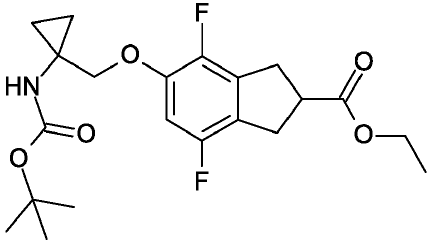

The title compound was prepared by analogy with 2-[[*tert*-butyl(dimethyl)silyl]oxymethyl]-6-(1,3-oxazol-4-ylmethoxy)-2,3-dihydro-1*H*-indene-4-carbonitrile (Intermediate 274, Step 3) using ethyl 4,7-difluoro-5-hydroxy-2,3-dihydro-1*H*-indene-2-carboxylate (Intermediate 216, Step 6) in place of 2-[[*tert*-butyl(dimethyl)silyl]oxymethyl]-6-hydroxy-2,3-dihydro-1*H*-indene-4-carbonitrile and *tert*-butyl *N*-[1-(hydroxymethyl)cyclopropyl]carbamate (CAS# 107017-73-2) in place of oxazol-4-ylmethanol and was obtained as an off-white foam. MS (ESP): m/z = 356.2 [M+H-tBu]⁺.

### Steps 2 and 3:

### tert-Butyl N-[1-[(4,7-difluoro-2-formyl-2,3-dihydro-1H-inden-5-yl)oxymethyl] cyclopropyl] carbamate

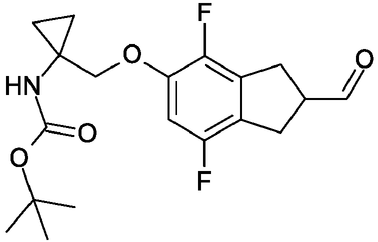

The title compound was prepared by analogy with 2-(4-chloro-2,3-dihydro-1*H*-inden-2-yl)acetaldehyde (Intermediate 11, Steps 4 and 5) and was obtained as a light yellow semisolid. MS (ESP): m/z = 312.2 [M+H-tBu]⁺.

### Intermediate 292

### tert-Butyl N-[1-[(4,7-difluoro-2-formyl-2,3-dihydro-1H-inden-5-yl)oxy]-2-methylpropan-2-yl]carbamate

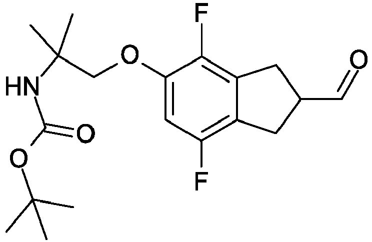

### Step 1:

### Ethyl 5-[2-methyl-2-[(2-methylpropan-2-yl)oxycarbonylamino]propoxy]-2,3-dihydro-1H-indene-2-carboxylate

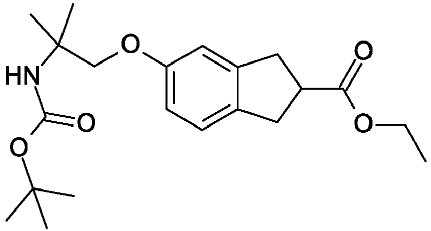

The title compound was prepared by analogy with ethyl 4-cyano-6-[2-methyl-2-[(2-methylpropan-2-yl)oxycarbonylamino]propoxy]-2,3-dihydro-1*H*-indene-2-carboxylate (Intermediate 140, Step 1) using ethyl 4,7-difluoro-5-hydroxy-2,3-dihydro-1*H*-indene-2-carboxylate (Intermediate 216, Step 6) in place of ethyl 4-cyano-6-hydroxy-2,3-dihydro-1*H-*indene-2-carboxylate and was obtained as a colorless oil. MS (ESP): m/z = 314.3 [M+H-BOC]⁺.

### Steps 2 and 3:

### tert-Butyl N-[1-[(4,7-difluoro-2-formyl-2,3-dihydro-1H-inden-5-yl)oxy]-2-methylpropan-2-yl] carbamate

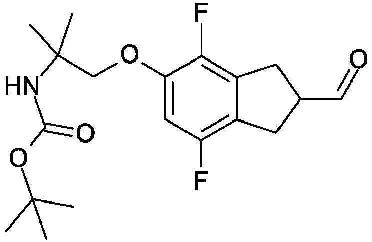

The title compound was prepared by analogy with 2-(4-chloro-2,3-dihydro-1*H*-inden-2-yl)acetaldehyde (Intermediate 11, Steps 4 and 5) and was obtained as a colorless waxy solid. MS (ESP): m/z = 270.3 [M+H-BOC]⁺.

### Intermediate 293

### 2-(4-Fluoro-2-formyl-indan-5-yl)oxyacetamide

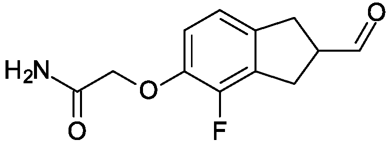

The title compound was prepared by analogy with 2-[(7-fluoro-2-formyl-2,3-dihydro-1*H*-inden-5-yl)oxy]acetonitrile (Intermediate 52, Steps 3 to 5) using 2-bromoacetamide in place of bromoacetonitrile and 2-[[*tert*-butyl(dimethyl)silyl]oxymethyl]-4-fluoro-indan-5-ol (Intermediate 204-A) in place of 2-[[*tert*-butyl(dimethyl)silyl]oxymethyl]-7-fluoro-2,3-dihydro-1*H*-inden-5-ol and was obtained as a white solid. MS (ESP): m/z = 238.0 [M+H]⁺.

### Intermediate 294

### 2-(7-Cyano-2-formyl-indan-5-yl)oxyacetamide

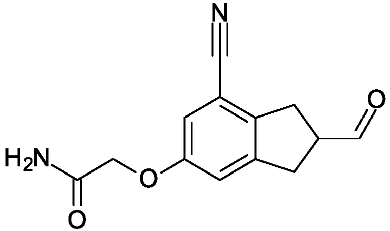

### Step 1:

### Ethyl 6-(2-amino-2-oxo-ethoxy)-4-cyano-indane-2-carboxylate

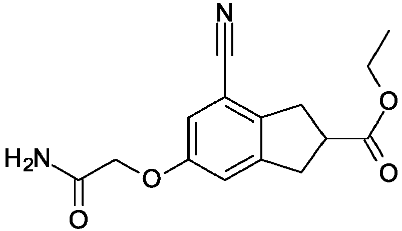

The title compound was prepared by analogy with ethyl 6-(2-methylamino-2-oxo-ethoxy)-4-cyano-indane-2-carboxylate (Intermediate 70, Step 5) using 2-bromoacetamide in place of 2-chloro-*N*-methylacetamide and was obtained as a pale yellow solid. MS (ESP): m/z = 289.1 [M+H]⁺.

### Step 2:

### 2-[7-Cyano-2-(hydroxymethyl)indan-5-yl]oxyacetamide

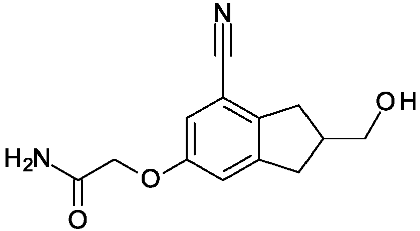

Sodium borohydride (137.8 mg, 3.64 mmol, 3 eq) and calcium chloride (269.5 mg, 2.43 mmol, 2 eq) were added to a suspension of ethyl 6-(2-amino-2-oxo-ethoxy)-4-cyano-indane-2-carboxylate (350.0 mg, 1.21 mmol, 1 eq) in tetrahydrofuran (9.8 mL) and ethanol (4.9 mL). The mixture was stirred at 25 °C for 1.5 h, then cooled to 0 °C, treated with sat aq. NH₄Cl solution and extracted with EtOAc (3 x). The combined organic phases were dried over Na₂SO₄, filtered and the solvent evaporated to afford the title compound (205 mg, 0.830 mmol, 68.6% yield) as a light yellow solid. It was used in the next step without further purification. MS (ESP): m/z = 247.1 [M+H]⁺.

### Step 3:

### 2-(7-Cyano-2-formyl-indan-5-yl)oxyacetamide

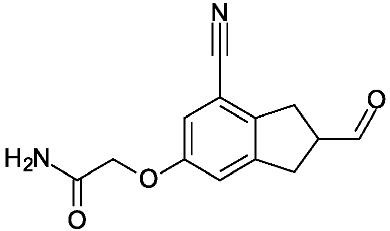

The title compound was prepared by analogy with 2-(4-chloro-2,3-dihydro-1*H*-inden-2-yl)acetaldehyde (Intermediate 11, Step 5) and was obtained as an off-white solid. MS (ESP): m/z = 245.0 [M+H]⁺.

### Intermediate 295

### 4-Fluoro-1-methyl-3-((1-((2-(trimethylsilyl)ethoxy)methyl)-1H-pyrazol-3-yl)methoxy)-6,7-dihydro-5H-cyclopenta[c]pyridine-6-carbaldehyde

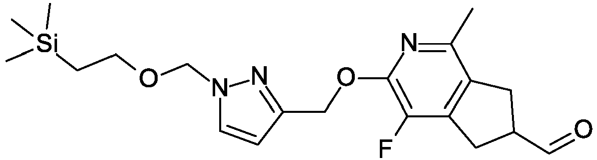

### Step 1:

### Methyl 4-fluoro-1-methyl-3-[[1-(2-trimethylsilylethoxymethyl)pyrazol-3-yl]methoxy]-6,7-dihydrocyclopenta[c]pyridine-6-carboxylate

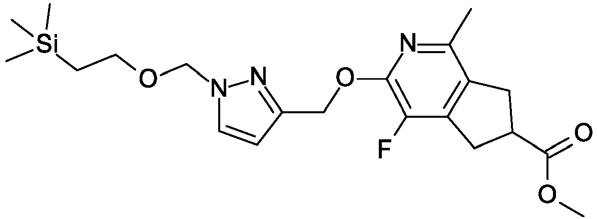

Methyl 3-chloro-4-fluoro-1-methyl-6,7-dihydro-5*H*-cyclopenta[c]pyridine-6-carboxylate (Intermediate 284, Step 3, 250 mg, 995 µmol, 1 eq) and (1-((2-(trimethylsilyl)ethoxy)methyl)-1*H*-pyrazol-3-yl)methanol (227 mg, 995 µmol, 1 eq) were suspended in toluene (16.5 mL). The mixture was degassed with argon for 45 minutes. 2-(Di-tert-butylphosphino)biphenyl (59.4 mg, 200 µmol, 0.2 eq), palladium (II) acetate (44.6 mg, 200 µmol, 0.2 eq) and cesium carbonate (1300 mg, 4.0 mmol, 4 eq) were added to the mixture, and the reaction mixtture stirred at 100 °C for 5 h. Upon cooling to room temperature, the reaction mixture was concentrated and taken up in ethyl acetate and water. The organic phase was extracted, washed with brine, dried with magnesium sulfate, filtered and concentrated in vacuo. The crude was purified by silica gel chromatography (30 % EtOAc in heptane, ISCO 50 g) to give an impure product which was purified by silica gel chromatography (0 to 2% MeOH in DCM, ISCO 20 g) to give the title compound (136.2 mg, 231 µmol, 23.3 % yield). MS (ESP): m/z = 436.3 [M+H]⁺.

### Steps 2 and 3:

### 4-Fluoro-1-methyl-3-((1-((2-(trimethylsilyl)ethoxy)methyl)-1H-pyrazol-3-yl)methoxy)-6,7-dihydro-5H-cyclopenta[c]pyridine-6-carbaldehyde

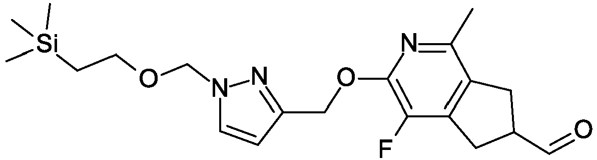

The title compound was prepared by analogy with 4-fluoro-3-methoxy-1-methyl-6,7-dihydro-5*H*-cyclopenta[c]pyridine-6-carbaldehyde (Intermediate 283, Steps 8 and 9) and was obtained as a colourless oil. MS (ESP): m/z = 406.2 [M+H]⁺.

### Intermediate 296

### (3,5-Dimethyl-1,2,3,6,7,8-hexahydrocyclopenta[d]pyrrolo[2,3-b]pyridin-7-yl)methyl 4-methylbenzenesulfonate

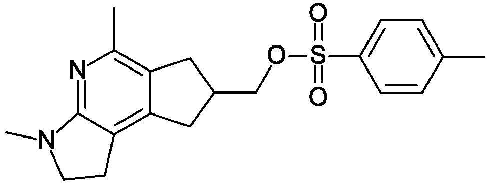

### Step 1:

### tert-Butyldiphenyl((2-(prop-2-yn-1-yl)hex-4-yn-1-yl)oxy)silane

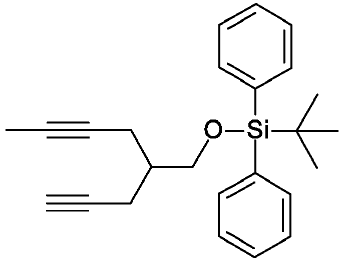

The title compound was prepared by analogy with *tert*-butyl-dimethyl-(2-prop-2-ynylhex-4-ynoxy)silane (Intermediate 271, Step 3) using *tert*-butylchlorodiphenylsilane in place of *tert-*butylchlorodimethylsilane and was obtained as a colorless oil. MS (ESP): m/z = 375.3 [M+H]⁺.

### Step 2:

### 6-(((tert-Butyldiphenylsilyl)oxy)methyl)deca-3,8-diyn-1-ol

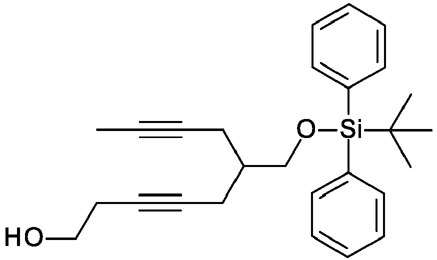

To a solution of *tert*-butyldiphenyl((2-(prop-2-yn-1-yl)hex-4-yn-1-yl)oxy)silane (1.455 g, 3.88 mmol, 1 eq) in THF (19 mL) cooled to -78 °C was added dropwise n-butyllithium (4.86 ml, 7.77 mmol, 2 eq). The solution was then stirred for 2 h at -50 °C. A solution of 1,3,2-dioxathiolane 2,2-dioxide (1.21 g, 9.71 mmol, 2.5 eq) in THF (5 mL) was added dropwise and the mixture was allowed to warm up overnight from -50 °C to 10 °C. At 0°C to the reaction mixture was added a solution of conc. H₂SO₄ (1 mL) and H₂O (1 mL) dropwise and the mixture stirred for 2 h at 0 °C. The reaction mixture was treated with Na₂CO₃ sol sat. (15 mL) and extracted with EtOAc (2 × 35 mL). The organic layers were dried (Na₂SO₄) and evaporated. The crude material was purified by flash chromatography (silica gel, 70 g, 0% to 40% EtOAc in heptane) to give the title compound (1.092 g, 65% yield) as a colorless oil. MS (ESP): m/z = 419.3 [M+H]⁺.

### Step 3:

### 6-(((tert-Butyldiphenylsilyl)oxy)methyl)deca-3,8-diyn-1-yl 4-methylbenzenesulfonate

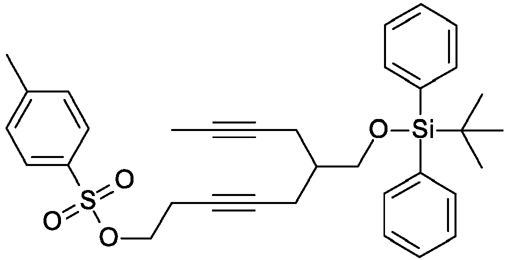

The title compound was prepared by analogy with dimethyl 1,3,4-trimethyl-5,7-dihydrocyclopenta[c]pyridine-6,6-dicarboxylate (Intermediate 59, Step 2) using toluene as solvent and was obtained as a colourless oil. MS (ESP): m/z = 573.4 [M+H]⁺.

### Step 4:

### 6-(((tert-Butyldiphenylsilyl)oxy)methyl)-N-methyldeca-3,8-diyn-1-amine

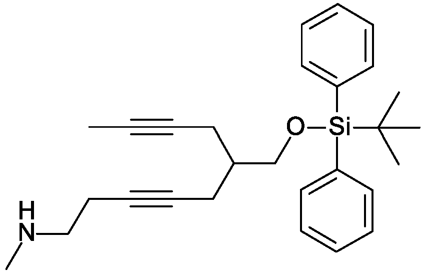

To a solution of 6-(((*tert*-butyldiphenylsilyl)oxy)methyl)deca-3,8-diyn-1-yl 4-methylbenzenesulfonate (1.036 g, 1.81 mmol, 1 eq) in DMA (10 mL) was added methanamine 2M in THF (4.52 mL, 9.04 mmol, 5 eq). The reaction mixture was capped and heated to 90 °C and stirred for 6 h. The reaction mixture was treated with sat. NaHCO₃ (5 mL) and extracted with EtOAc (2 × 15 mL). The organic layers were dried (Na₂SO₄) and evaporated. The crude material was purified by flash chromatography (silica gel, 20 g, 0% to 10% MeOH in DCM) to give the title compound (441 mg, 53.7% yield) as a light yellow oil. MS (ESP): m/z = 432.3 [M+H]⁺.

### Step 5:

### N-(6-(((tert-Butyldiphenylsilyl)oxy)methyl)deca-3,8-diyn-1-yl)-N-methylcyanamide

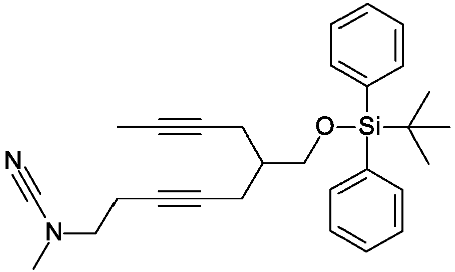

To a solution of 6-(((*tert*-butyldiphenylsilyl)oxy)methyl)-*N*-methyldeca-3,8-diyn-1-amine (593 mg, 1.37 mmol, 1 eq) in DCM (5 mL) was added a solution of NaHCO₃ (231 mg, 2.75 mmol, 2 eq) in H₂O (0.5 mL) at 0°C. Then a solution of cyanic bromide (175 mg, 1.65 mmol, 1.2 eq) in DCM (1.5 mL) was added. The reaction mixture was stirred at RT for 2 h. The reaction mixture was treated with sat. NaHCO₃ (10 mL) and stirred again for 15 min., then extracted with DCM (2 × 30 mL). The organic layers were washed with brine (10 mL). The organic layers were dried (Na₂SO₄) and evaporated. The crude material was purified by flash chromatography (silica gel, 10 g, 5% to 35% EtOAc in heptane) to give the title compound (551 mg, 85% yield) as a light brown oil. MS (ESP): m/z = 457.3 [M+H]⁺.

### Step 6:

### 7-(((tert-Butyldiphenylsilyl)oxy)methyl)-3,5-dimethyl-1,2,3,6,7,8-hexahydrocyclopenta[d]pyrrolo[2,3-b]pyridine

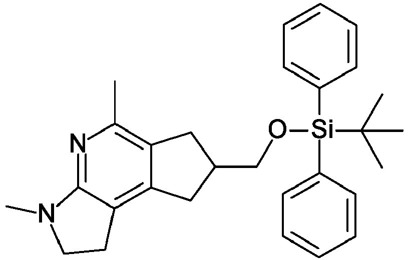

The title compound was prepared by analogy with dimethyl 1,3,4-trimethyl-5,7-dihydrocyclopenta[c]pyridine-6,6-dicarboxylate (Intermediate 59, Step 2) using toluene as solvent and was obtained as a light brown oil. MS (ESP): m/z = 457.4 [M+H]⁺.

### Step 7:

### (5,8-Dimethyl-5,7-diazatricyclo[7.3.0.0^{2,6}]dodeca-1,6,8-trien-11-yl)methanol

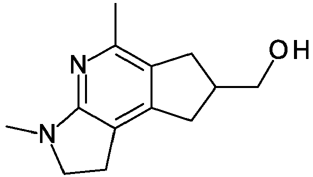

The title compound was prepared by analogy with [*rac*-(5,6-*trans*)-1-chloro-6-(hydroxymethyl)-6,7-dihydro-5*H*-cyclopenta[c]pyridin-5-yl] acetate (Intermediate 30, Step 4) and was obtained as a colorless oil. MS (ESP): m/z = 219.3 [M+H]⁺.

### Step 8:

### (3,5-Dimethyl-1,2,3,6,7,8-hexahydrocyclopenta[d]pyrrolo[2,3-b]pyridin-7-yl)methyl 4-methylbenzenesulfonate

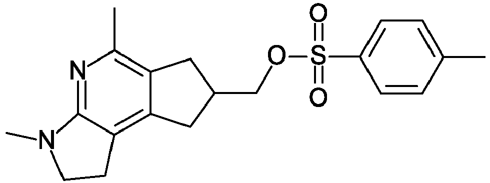

The title compound was prepared by analogy with ethyl 1-ethyl-6-fluoro-8-[(4-methylphenyl)sulfonyloxymethyl]-4-oxo-8,9-dihydro-7*H*-cyclopenta[h]quinoline-3-carboxylate (Intermediate 172, Step 9) and was obtained as a brown oil. MS (ESP): m/z = 373.2 [M+H]⁺.

### Intermediate 297

### 6-(4-(2-Aminoethyl)-2-oxopyrrolidin-1-yl)-4-(4-methoxybenzyl)-2H-pyrido[3,2-b][1,4]oxazin-3(4H)-one

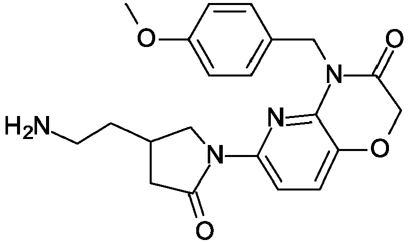

### Step 1:

### Ethyl 2-(1-(4-(4-methoxybenzyl)-3-oxo-3,4-dihydro-2H-pyrido[3,2-b][1,4]oxazin-6-yl)-5-oxopyrrolidin-3-yl)acetate

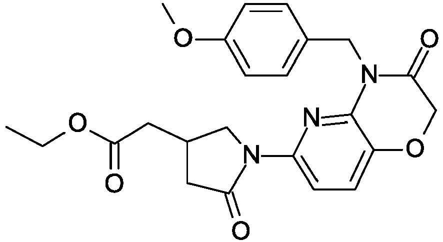

The title compound was prepared by analogy with *tert*-butyl *N*-[2-[2-oxo-3-(3-oxo-4*H-*pyrido[3,2-b][1,4]thiazin-6-yl)oxazolidin-5-yl]ethyl]carbamate (Intermediate 48, Step 4) using 6-bromo-4-(4-methoxybenzyl)-2H-pyrido[3,2-b][1,4]oxazin-3(4H)-one (CAS# 1184303-37-4) in place of 6-chloro-4*H*-pyrido[3,2-b][1,4]thiazin-3-one and ethyl 2-(5-oxopyrrolidin-3-yl)acetate (CAS# 99709-47-4) in place of *tert*-butyl *N*-[2-(2-oxooxazolidin-5-yl)ethyl]carbamate and was obtained as a red solid. MS (ESP): m/z = 440 [M+H]⁺.

### Step 2:

### 2-(1-(4-(4-Methoxybenzyl)-3-oxo-3,4-dihydro-2H-pyrido[3,2-b][1,4]oxazin-6-yl)-5-oxopyrrolidin-3-yl)acetic acid

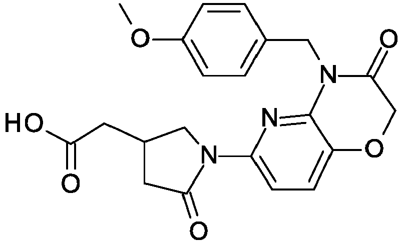

To a solution of ethyl 2-(1-(4-(4-methoxybenzyl)-3-oxo-3,4-dihydro-2*H*-pyrido[3,2-b][1,4]oxazin-6-yl)-5-oxopyrrolidin-3-yl)acetate (4.8 g, 10.9 mmol, 1 eq) in 1,2-dichloroethane (200 mL) was added trimethyltin hydroxide (13.8 g, 76.5 mmol, 7 eq) at room temperature. The reaction mixture was heated at 80°C for 30 h. The reaction mixture was cooled to room temperature and filtred. The filtrate was concentrated in vacuo to give a residue. The crude product was partitioned between ethyl acetate (250 mL) and 2 M aqueous hydrogen chloride solution (150 mL). The layers were separated. The organic layer was washed with 1 M aqueous hydrogen chloride solution (150 mL), dried over anhydrous sodium sulfate, filtered and concentrated in vacuo to give the crude title compound (7.08 g) as a yellow oil. MS (ESP): m/z = 412 [M+H]⁺.

### Step 3:

### 6-(4-(2-Hydroxyethyl)-2-oxopyrrolidin-1-yl)-4-(4-methoxybenzyl)-2H-pyrido[3,2-b][1,4]oxazin-3(4H)-one

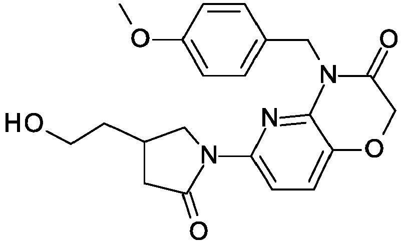

To a solution of 2-(1-(4-(4-methoxybenzyl)-3-oxo-3,4-dihydro-2*H*-pyrido[3,2-b][1,4]oxazin-6-yl)-5-oxopyrrolidin-3-yl)acetic acid (200 mg, 486 µmol, 1 eq) in THF (3 mL) was added borane dimethyl sulfide complex (243 µL, 486 µmol, 1 eq) at 0-5°C. The reaction mixture was stirred 3 h at 0-5°C. The reaction mixture was quenched with methanol (3 mL) at 0-5°C and then allowed to warmed to room temperature and stirred for 20 min. The reaction mixture was concentrated in vacuo. The residue was partitioned between ethyl acetate (40 mL) and 1 M sodium carbonate (20 mL). The layers were separated. The aqueous layer was extracted with ethyl acetate (20 mL). The combined organic layers were washed with water, dried over anhydrous sodium sulfate, filtered and concentrated in vacuo. The crude product was purified by flash chromatography to give the title compound (118 mg) as a light yellow oil. MS (ESP): m/z = 398 [M+H]⁺.

### Steps 4 to 6:

### 6-(4-(2-Aminoethyl)-2-oxopyrrolidin-1-yl)-4-(4-methoxybenzyl)-2H-pyrido[3,2-b][1,4]oxazin-3(4H)-one

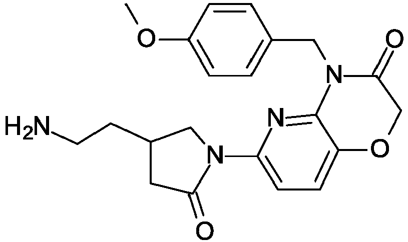

The title compound was prepared by analogy with 6-[5-(2-aminoethyl)-2-oxo-1,3-oxazol-3-yl]-4*H*-pyrido[3,2-b][1,4]oxazin-3-one (Intermediate 183, Steps 5 to 7) and was obtained as a colourless oil. MS (ESP): m/z = 397 [M+H]⁺.

### Intermediate 298

### tert-Butyl ((3-(dimethylamino)-1-methyl-6,7-dihydro-5H-cyclopenta[c]pyridin-6-yl)methyl)(2-(3-(4-(4-methoxybenzyl)-3-oxo-3,4-dihydro-2H-pyrido[3,2-b] [1,4]oxazin-6-yl)-2-oxooxazolidin-5-yl)ethyl)carbamate

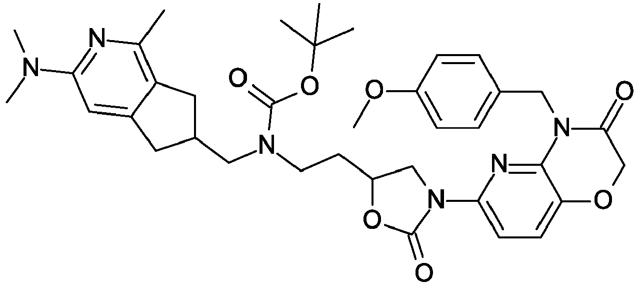

### Step 1:

### Methyl 2-benzyl-1-methyl-3-oxo-3,5,6,7-tetrahydro-2H-cyclopenta[c]pyridine-6-carboxylate

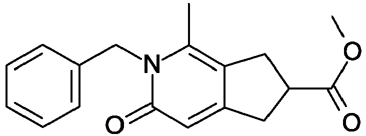

The title compound was prepared from dimethyl 1-methyl-3-phenylmethoxy-5,7-dihydrocyclopenta[c]pyridine-6,6-dicarboxylate (Intermediate 117, Step 1) by analogy with methyl 1-chloro-3-methoxy-6,7-dihydro-5*H*-cyclopenta[c]pyridine-6-carboxylate (Intermediate 37, Step 6) and was obtained as a light yellow solid. MS (ESP): m/z = 298 [M+H]⁺.

### Step 2:

### Methyl 1-methyl-3-oxo-3,5,6,7-tetrahydro-2H-cyclopenta[c]pyridine-6-carboxylate

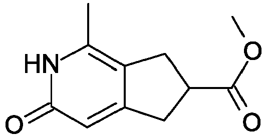

The title compound was prepared by analogy with dimethyl 3-oxo-5,7-dihydro-2*H-*cyclopenta[c]pyridine-6,6-dicarboxylate (Intermediate 37, Step 2) and was obtained as a white solid. MS (ESP): m/z = 208 [M+H]⁺.

### Step 3:

### Methyl 1-methyl-3-(((perfluorobutyl)sulfonyl)oxy)-6,7-dihydro-5H-cyclopenta[c]pyridine-6-carboxylate

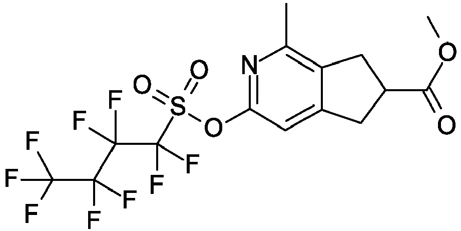

To a white suspension of methyl 1-methyl-3-oxo-3,5,6,7-tetrahydro-2*H*-cyclopenta[c]pyridine-6-carboxylate (2 g, 9.65 mmol, 1 eq) in DMF (59.7 mL) was added sodium hydride (1.74 g, 43.4 mmol, 4.5 eq) in four portions at 30 °C. The reaction mixture was warmed to 45 °C and stirred for 30 min. At 45°C 1,1,2,2,3,3,4,4,4-nonafluorobutane-1-sulfonyl fluoride (8.75 g, 5.09 mL, 29 mmol, 3 eq) was slowly added. The reaction mixture was heated at 80 °C until reaction was completed. The reaction mixture was cooled to room temperature, diluted with dichloromethane (300 mL) and saturated ammonium chloride solution (150 mL) was carefully added. The layers were separated. The aqueous layer was extracted with DCM (100 mL). The combined organic layers were dried over anhydrous sodium sulfate, filtered and concentrated in vacuo to give a brown oil. The crude product was purified by flash chromatography (80 g cartridge , n-heptane/ethyl acetate: 100:0 to 80:20) to give the title compound (3.84 g) as a colorless oil. MS (ESP): m/z = 490 [M+H]⁺.

### Step 4:

### Methyl 3-iodo-1-methyl-6,7-dihydro-5H-cyclopenta [c]pyridine-6-carboxylate

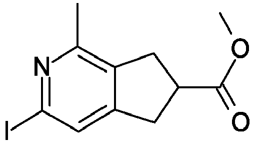

To a colorless solution of methyl 1-methyl-3-(((perfluorobutyl)sulfonyl)oxy)-6,7-dihydro-5*H-*cyclopenta[c]pyridine-6-carboxylate (3.8 g, 7.77 mmol, 1 eq) and acetyl chloride (914 mg, 828 µl, 11.6 mmol, 1.5 eq) in MeCN (76.1 mL) was added sodium iodide (11.6 g, 77.7 mmol, 10 eq) at room temperature. The reaction mixture was heated at 80 °C for 8 h. The reaction mixture was allowed to cooled to room temperature. The reaction mixture was partitioned between ethyl acetate (250 mL) and a mixture of 10 % aqueous potassium carbonate solution and 10% sodium thiosulfate solution (1:1, 240 mL). The aqueous layer was extracted with ethyl acetate (250 mL). The combined organic layers were dried over anhydrous sodium sulfate, filtered and concentrated in vacuo. The residue was triturated with toluene (30 mL) and filtered. The filtrate was concentrated in vacuo. The crude material was purified by flash chromatography (120 g cartridge, dichloromethane 100 : 0 to 90 : 10) to give the title compound (728 mg) as an off-white solid. MS (ESP): m/z = 318 [M+H]⁺.

### Step 5:

### 3-Iodo-1-methyl-6,7-dihydro-5H-cyclopenta[c]pyridine-6-carboxylic acid

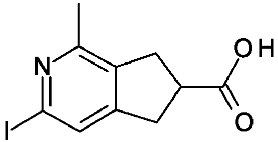

To a solution of methyl 3-iodo-1-methyl-6,7-dihydro-5*H*-cyclopenta[c]pyridine-6-carboxylate (926 mg, 2.92 mmol, 1 eq) in 1,4-dioxane (29.2 mL) was added 2M aqueous sodium hydroxide (7.3 mL, 14.6 mmol, 5 eq) at room temperature. The reaction mixture was stirred 1 h at room temperature. The pH was set to approx. pH 3 by addition of 2 M aqueous hydrogen chloride (7.3 mL, 14.6 mmol, 5 eq). The mixture was partitioned between ethyl acetate (250 mL) and water (50 mL). The layers were separated. The aqueous layer was extracted with ethyl acetate (250 mL). The combined organic layers were dried over anhydrous sodium sulfate, filtered and concentrated in vacuo to give the title compound (867 mg, 98% yield) as a white solid. MS (ESP): m/z = 304 [M+H]⁺.

### Step 6:

### (3-Iodo-1-methyl-6,7-dihydro-5H-cyclopenta[c]pyridin-6-yl)methanol

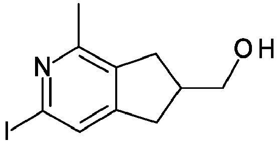

The title compound was prepared by analogy with (4-Bromo-2,3-dihydro-1*H*-inden-2-yl)methanol (Intermediate 5, Step 1) and was obtained as a colourless oil. MS (ESP): m/z = 290 [M+H]⁺.

### Step 7:

### 3-Iodo-1-methyl-6,7-dihydro-5H-cyclopenta[c]pyridine-6-carbaldehyde

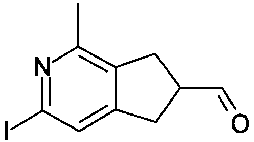

The title compound was prepared by analogy with 2-(4-chloro-2,3-dihydro-1*H*-inden-2-yl)acetaldehyde (Intermediate 11, Step 5) and was obtained as a colourless oil. MS (ESP): m/z = 288 [M+H]⁺.

### Step 8:

### 6-(5-(2-(((3-Iodo-1-methyl-6,7-dihydro-5H-cyclopenta[c]pyridin-6-yl)methyl)amino)ethyl)-2-oxooxazolidin-3-yl)-4-(4-methoxybenzyl)-2H-pyrido[3,2-b] [1,4]oxazin-3(4H)-one

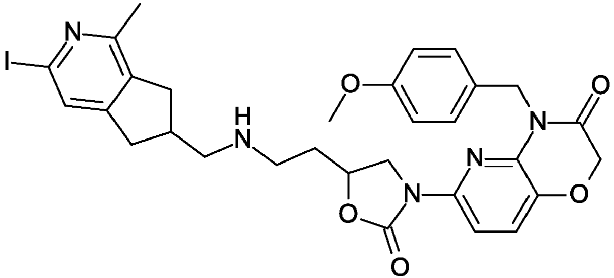

The title compound was prepared by analogy with 6-(4-(2-(((4-fluoro-1-methyl-6,7-dihydro-5*H-*cyclopenta[c]pyridin-6-yl)methyl)amino)ethyl)-2-oxopyrrolidin-1-yl)-4-(4-methoxybenzyl)-2*H-*pyrido[3,2-b][1,4]oxazin-3(4*H*)-one (Example 417, Step 1) using 3-iodo-1-methyl-6,7-dihydro-5H-cyclopenta[c]pyridine-6-carbaldehyde in place of 4-fluoro-1-methyl-6,7-dihydro-5*H-*cyclopenta[c]pyridine-6-carbaldehyde and was obtained as an off-white foam. MS (ESP): m/z = 670 [M+H]⁺.

### Step 9:

### tert-Butyl ((3-iodo-1-methyl-6,7-dihydro-5H-cyclopenta[c]pyridin-6-yl)methyl)(2-(3-(4-(4-methoxybenzyl)-3-oxo-3,4-dihydro-2H-pyrido[3,2-b][1,4]oxazin-6-yl)-2-oxooxazolidin-5-yl)ethyl)carbamate

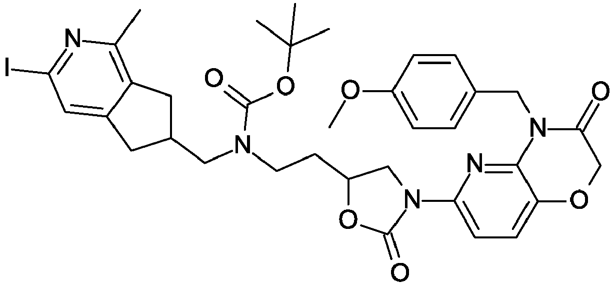

To a solution of 6-(5-(2-(((3-iodo-1-methyl-6,7-dihydro-5*H*-cyclopenta[c]pyridin-6-yl)methyl)amino)ethyl)-2-oxooxazolidin-3-yl)-4-(4-methoxybenzyl)-2*H*-pyrido[3,2-b][1,4]oxazin-3(4H)-one (310 mg, 463 µmol, 1 eq) and triethylamine (141 mg, 194 µL, 1.39 mmol, 3 eq) in dichloromethane (2.32 mL) was added di-tert-butyl dicarbonate (101 mg, 463 µmol, 1.0 eq) at room temperature. The reaction mixture was stirred for 1 h. The reaction mixture was partitioned between dichloromethane (40 mL) and 1 M sodium carbonate (10 mL). The aqueous layer was extracted with dichloromethane (3 x 40 mL). The combined organic layers were dried over anhydrous sodium sulfate, filtered and concentrated in vacuo. The crude material was purified by flash chromatography on silica gel (24 g, 0% to 100% EtOAc in heptane) to give the title compound (301 mg, 372 µmol, 80.2 % yield) as a colorless oil. MS (ESP): m/z = 770 [M+H]⁺.

### Step 10:

### tert-Butyl ((3-(dimethylamino)-1-methyl-6,7-dihydro-5H-cyclopenta[c]pyridin-6-yl)methyl)(2-(3-(4-(4-methoxybenzyl)-3-oxo-3,4-dihydro-2H-pyrido[3,2-b] [1,4]oxazin-6-yl)-2-oxooxazolidin-5-yl)ethyl)carbamate

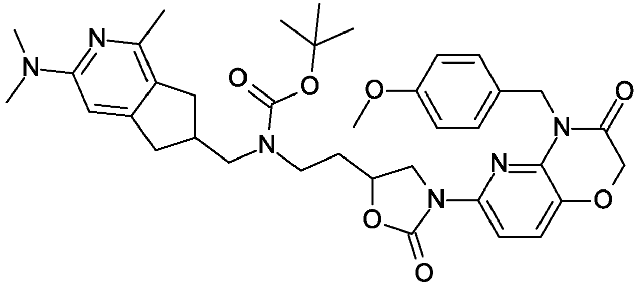

In a pressure tube *tert-butyl* ((3-iodo-1-methyl-6,7-dihydro-5*H*-cyclopenta[c]pyridin-6-yl)methyl)(2-(3-(4-(4-methoxybenzyl)-3-oxo-3,4-dihydro-2*H*-pyrido[3,2-b] [1,4]oxazin-6-yl)-2-oxooxazolidin-5-yl)ethyl)carbamate (50 mg, 65 µmol, 1 eq) was dissolved in DMSO (0.331 mL) and combined with L-proline (13.5 mg, 117 µmol, 1.8 eq), copper (I) iodide (12.4 mg, 65 µmol, 1 eq), potassium carbonate (20.7 mg, 149 µmol, 2.3 eq) and dimethylamine 2M in THF (162 µL, 325 µmol, 5 eq) to give a dark green suspension. The flask was closed and stirred at 70 °C for 2 h. The crude was extracted with water and dichloromethane twice. The organic layers were combined, dried over sodium sulfate, filtered and evaporated to dryness. The crude material was purified by flash chromatography on silica gel (4 g, 0% to 50% EtOAc in heptane) to give the title compound (21 mg) as a white solid. MS (ESP): m/z = 687.6 [M+H]⁺.

### Intermediate 299

### tert-Butyl (2-(3-(4-(4-methoxybenzyl)-3-oxo-3,4-dihydro-2H-pyrido[3,2-b][1,4]oxazin-6-yl)-2-oxooxazolidin-5-yl)ethyl)((1-methyl-3-(methylamino)-6,7-dihydro-5H-cyclopenta[c]pyridin-6-yl)methyl)carbamate

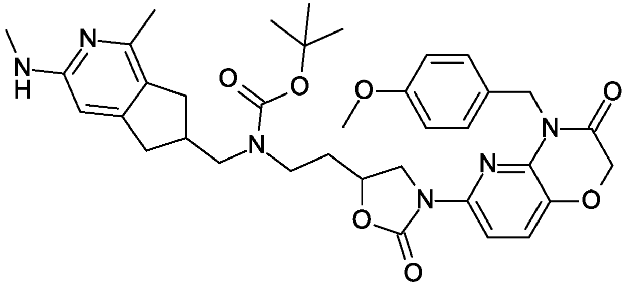

The title compound was prepared by analogy with *tert-*butyl ((3-(dimethylamino)-1-methyl-6,7-dihydro-5*H*-cyclopenta[c]pyridin-6-yl)methyl)(2-(3-(4-(4-methoxybenzyl)-3-oxo-3,4-dihydro-2*H*-pyrido[3,2-b][1,4]oxazin-6-yl)-2-oxooxazolidin-5-yl)ethyl)carbamate (Intermediate 298, Step 10) using methylamine in place of dimethylamine and was obtained as a white solid. MS (ESP): m/z = 673 [M+H]⁺.

### Intermediate 300

### (4-Methyl-6,7-dihydro-5H-cyclopenta[d]pyrimidin-6-yl)methyl 4-methylbenzenesulfonate

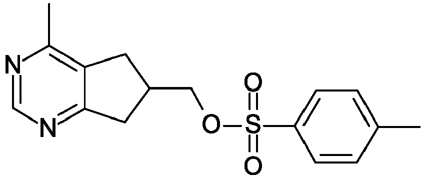

### Step 1:

### (4-Methyl-6,7-dihydro-5H-cyclopenta[d]pyrimidin-6-yl)methanol

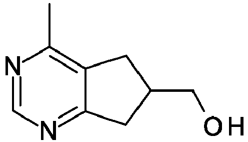

(2-Chloro-4-methyl-6,7-dihydro-5*H*-cyclopenta[d]pyrimidin-6-yl)methanol (Intermediate 273, Step 4, 20 mg, 101 µmol, 1 eq) was dissolved at RT in MeOH (1 mL) and EtOAc (1 mL). Ammonium formate (63.5 mg, 1.01 mmol, 10 eq) was added. The reaction mixture was stirred under Ar. To the reaction was added 20% palladium hydroxide on carbon (56.6 mg, 80.8 µmol, 0.8 eq) and the mixture was heated to reflux (70 °C) for 1 h. The reaction mixture was filtered through glass filter pads and washed thoroughly with MeOH. The filtrate was concentrated to give a residue. The residue was purified by flash chromatography (silica gel, 2 g, 2% to 20% MeOH in DCM) to give the title compound (13.9 mg) as a colorless waxy solid. MS (ESP): m/z = 165.2 [M+H]⁺.

### Step 2:

### (4-Methyl-6,7-dihydro-5H-cyclopenta[d]pyrimidin-6-yl)methyl 4-methylbenzenesulfonate

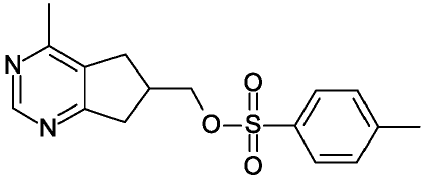

The title compound was prepared by analogy with (1,4-dimethyl-6,7-dihydro-5*H-*cyclopenta[c]pyridin-6-yl)methyl 4-methylbenzenesulfonate (Intermediate 191) and was obtained as a light yellow oil. MS (ESP): m/z = 319.2 [M+H]⁺.

### Intermediate 301

### 6-(5-(2-(((3-Benzyl-5-methyl-1,2,3,6,7,8-hexahydrocyclopenta[d]pyrrolo[2,3-b]pyridin-7-yl)methyl)amino)ethyl)-2-oxooxazolidin-3-yl)-2H-pyrido[3,2-b][1,4]oxazin-3(4H)-one

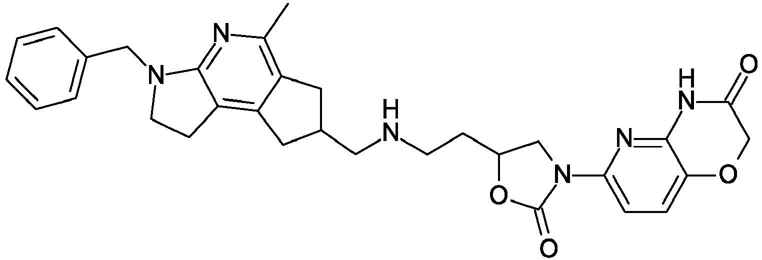

The title compound was prepared from 6-(((*tert-*butyldiphenylsilyl)oxy)methyl)deca-3,8-diyn-1-yl 4-methylbenzenesulfonate (Intermediate 296, Step 3) by analogy with 6-[5-[2-[(5,8-dimethyl-5,7-diazatricyclo[7.3.0.0^{2,6}]dodeca-1,6,8-trien-11-yl)methylamino]ethyl]-2-oxo-1,3-oxazolidin-3-yl]-4*H*-pyrido[3,2-b][1,4]oxazin-3-one (Example 416) using benzylamine in place of methanamine in the first step and was obtained as a light brown waxy solid. MS (ESP): m/z = 555.5 [M+H]⁺.

### Intermediate 302

### 6-[5-(2-Aminoethyl)-2-oxo-oxazolidin-3-yl]-4-(2-trimethylsilylethoxymethyl)pyrazino[2,3-b][1,4]oxazin-3-one

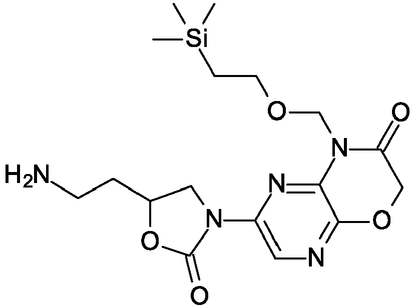

### Step 1:

### tert-Butyl N-[2-[2-oxo-3-[3-oxo-4-(2-trimethylsilylethoxymethyl)pyrazino[2,3-b] [1,4]oxazin-6-yl] oxazolidin-5-yl] ethyl] carbamate

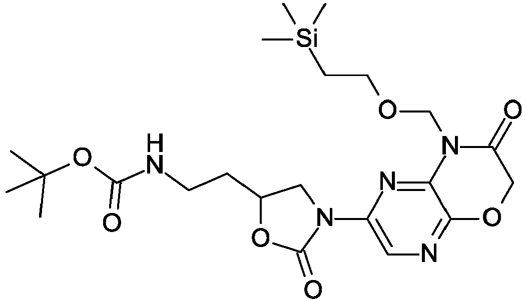

The title compound was prepared from *tert*-butyl *N*-[2-(2-oxooxazolidin-5-yl)ethyl]carbamate (Intermediate 26) and 6-bromo-4-(2-trimethylsilylethoxymethyl)pyrazino[2,3-b][1,4]oxazin-3-one (Intermediate 25) by analogy to *tert-*butyl *N*-[*trans-*3-[2-oxo-3-[3-oxo-4-(2-trimethylsilylethoxymethyl)pyrido[3,2-b] [1,4]oxazin-6-yl]-1,3-oxazolidin-5-yl]cyclobutyl]carbamate (Intermediate 7, Step 4) and was obtained as a yellow solid.

### Step 2:

### 6-[5-(2-Aminoethyl)-2-oxo-oxazolidin-3-yl]-4-(2-trimethylsilylethoxymethyl)pyrazino[2,3-b][1,4]oxazin-3-one

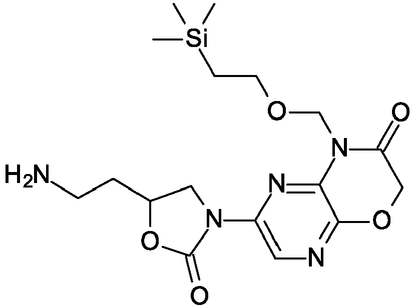

To a mixture of *tert*-butyl *N*-[2-[2-oxo-3-[3-oxo-4-(2-trimethylsilylethoxymethyl)pyrazino[2,3-b][1,4]oxazin-6-yl]oxazolidin-5-yl]ethyl]carbamate (1000.0 mg, 1.96 mmol, 1 eq) in methanol (10 mL) was added *p*-toluenesulfonic acid monohydrate (1.0 mL, 2.94 mmol, 1.5 eq) and the solution was stirred at 55°C for 3 h. The solvent was removed by concentration and the residue was taken up in EtOAc (20 mL) then washed with aq. NaHCOs (10 mL). The organics were dried with Na₂SO₄ and the solvent was removed to give the crude title compound (800 mg, 1.95 mmol, 99.6% yield) as a white solid.

### Intermediate 303

### 2-Formyl-6-((1-trityl-1H-1,2,3-triazol-4-yl)methoxy)-2,3-dihydro-1H-indene-4-carbonitrile

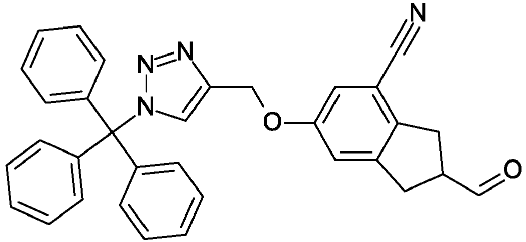

The title compound was prepared by analogy with 2-formyl-6-[(3-methyl-1,2,4-oxadiazol-5-yl)methoxy]-2,3-dihydro-1*H*-indene-4-carbonitrile (Intermediate 275) using 4-(bromomethyl)-1-trityl-1*H*-1,2,3-triazole (CAS# 960235-50-1) in place of 5-(chloromethyl)-3-methyl-1,2,4-oxadiazole, using acetone in place of acetonitrile and with heating to 60 °C instead of to 85 °C in the first step, and was obtained as a colorless oil.

### Intermediate 304

### 4-Fluoro-1-(2-tetrahydropyran-2-ylpyrazol-3-yl)-6,7-dihydro-5H-cyclopenta[c]pyridine-6-carbaldehyde

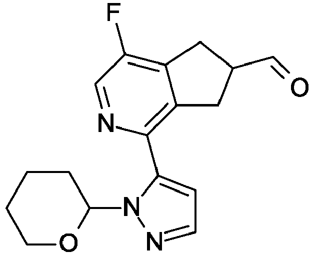

### Step 1:

### [4-Fluoro-1-(2-tetrahydropyran-2-ylpyrazol-3-yl)-6,7-dihydro-5H-cyclopenta[c]pyridin-6-yl]methanol

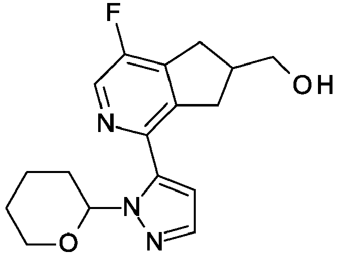

To a solution of (2-tetrahydropyran-2-ylpyrazol-3-yl)boronic acid (CAS# 1105511-68-9, 146.0 mg, 0.740 mmol, 1.3 eq), (1-bromo-4-fluoro-6,7-dihydro-5*H*-cyclopenta[c]pyridin-6-yl)methanol (Intermediate 28 starting material) (141.0 mg, 0.570 mmol, 1 eq) and phosphoric acid, potassium salt (364.9 mg, 1.72 mmol, 3 eq) in degassed 1,4-dioxane (6 mL) and water (0.600 mL), [1,1'-bis(diphenylphosphino)ferrocene]dichloropalladium(II) (41.9 mg, 0.060 mmol, 0.100 eq) was added under nitrogen atmosphere and the mixture was stirred at 80 °C for 6 h. The mixture was partioned between water and EtOAc. The organic phase was dried over Na₂SO₄, filtered and concentrated under vacuum. The resulting crude material was purified by flash chromatography (SNAP KP-Sil, 25g, cyclohexane: EtOAc, from 8:2 to 100% EtOAc) to give the title compound (106 mg, 0.330 mmol, 58.3% yield) as a light brown oil. MS (ESP): m/z = 318.1 [M+H]⁺.

### Step 2:

### 4-Fluoro-1-(2-tetrahydropyran-2-ylpyrazol-3-yl)-6,7-dihydro-5H-cyclopenta[c]pyridine-6-carbaldehyde

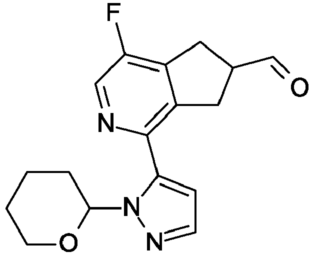

The title compound was prepared by analogy with 2-(4-chloro-2,3-dihydro-1*H*-inden-2-yl)acetaldehyde (Intermediate 11, Step 5) and was obtained as an off-white solid. MS (ESP): m/z = 316.0 [M+H]⁺.

### Intermediate 305

### (R)-4-fluoro-6-(iodomethyl)-1-methyl-6,7-dihydro-5H-cyclopenta[c]pyridine

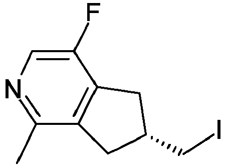

### Step 1:

### (R)-(4-Fluoro-1-methyl-6,7-dihydro-5H-cyclopenta[c]pyridin-6-yl)methanol

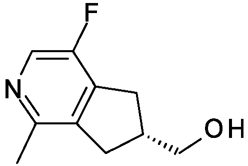

and (*S*)-(4-fluoro-1-methyl-6,7-dihydro-5*H*-cyclopenta[c]pyridin-6-yl)methanol

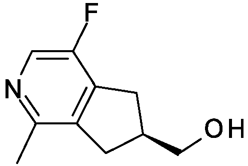

(4-Fluoro-1-methyl-6,7-dihydro-5*H*-cyclopenta[c]pyridin-6-yl)methanol (Intermediate 27, Step 9, 3200 mg, 17.66 mmol) was separated by SFC to give (*R*)-(4-fluoro-1-methyl-6,7-dihydro-5*H-*cyclopenta[c]pyridin-6-yl)methanol (1060 mg, 5.85 mmol, 31.8% yield) (Peak 1, 100% ee) as a white solid and (*S*)-(4-fluoro-1-methyl-6,7-dihydro-5*H*-cyclopenta[c]pyridin-6-yl)methanol (Peak 2, 95% ee). Peak 2 was separated by SFC to give (*S*)-(4-Fluoro-1-methyl-6,7-dihydro-5*H-*cyclopenta[c]pyridin-6-yl)methanol (1300 mg, 7.17 mmol, 40.6% yield)(Peak 2, 98% ee) as a white solid. The absolute configurations were determined by X-ray crystallography.

### Step 2:

### (R)-(4-Fluoro-1-methyl-6,7-dihydro-5H-cyclopenta[c]pyridin-6-yl)methyl methanesulfonate

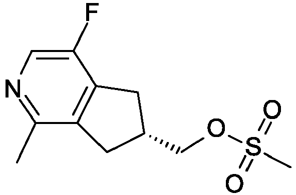

To a solution of (*R*)-(4-fluoro-1-methyl-6,7-dihydro-5*H*-cyclopenta[c]pyridin-6-yl)methanol (1.3 g, 7.17 mmol, 1 eq) in DCM (45 mL) was added triethylamine (1.09 g, 1.5 mL, 10.8 mmol, 1.5 eq). The resulting solution was cooled to 0 °C and methanesulfonyl chloride (863 mg, 583 µL, 7.53 mmol, 1.05 eq) was added. The mixture was stirred for 2 h at RT. Methanesulfonyl chloride (123 mg, 83.8 µL, 1.08 mmol, 0.15 eq) was added and the mixture stirred for 30 mins. The mixture was evaporated and the residue purified by silica cartridge chromatography (20 g; DCM to 3% MeOH/DCM) to give the title compound (1.4 g, 5.4 mmol, 75.3 % yield) as a light yellow solid. MS (ESP): m/z = 260.1 [M+H]⁺.

### Step 3:

### (R)-4-fluoro-6-(iodomethyl)-1-methyl-6,7-dihydro-5H-cyclopenta[c]pyridine

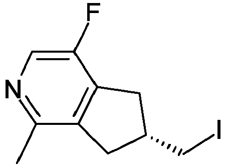

To a solution of (*R*)-(4-fluoro-1-methyl-6,7-dihydro-5*H*-cyclopenta[c]pyridin-6-yl)methyl methanesulfonate (1.4 g, 5.4 mmol, 1 eq) in MeCN (10 mL) was added sodium iodide (8.09 g, 54 mmol, 10 eq) and the mixture was stirred at 60 °C for 4 h. SiO₂ (15 g) was added and the mixture evaporated. The residue was purified by silica cartridge chromatography (50 g; heptane to EtOAc) to give the title compound (1.5 g, 5.15 mmol, 95.4 % yield) as a light yellow oil. MS (ESP): m/z = 292.0 [M+H]⁺.

### Intermediate 306

### (S)-4-fluoro-6-(iodomethyl)-1-methyl-6,7-dihydro-5H-cyclopenta[c]pyridine

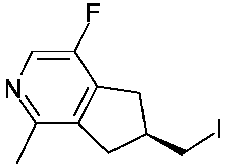

The title compound was prepared from (*S*)-(4-fluoro-1-methyl-6,7-dihydro-5*H-*cyclopenta[c]pyridin-6-yl)methanol (Intermediate 304, Step 1) by analogy with (*R*-4-fluoro-6-(iodomethyl)-1-methyl-6,7-dihydro-5*H*-cyclopenta[c]pyridine (Intermediate 304) and was obtained as a light yellow oil. MS (ESP): m/z = 292.0 [M+H]⁺.

### Intermediate 307

### 4-Fluoro-1-(1-tetrahydropyran-2-ylpyrazol-4-yl)-6,7-dihydro-5H-cyclopenta[c]pyridine-6-carbaldehyde

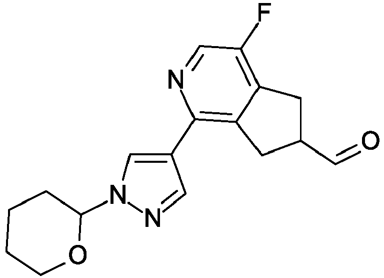

The title compound was prepared by analogy with 4-fluoro-1-(2-tetrahydropyran-2-ylpyrazol-3-yl)-6,7-dihydro-5*H*-cyclopenta[c]pyridine-6-carbaldehyde (Intermediate 304) using 1-(tetrahydro-2*H*-pyran-2-yl)-4-(4,4,5,5-tetramethyl-1,3,2-dioxaborolan-2-yl)-1*H*-pyrazole (CAS# 1003846-21-6) in place of (2-tetrahydropyran-2-ylpyrazol-3-yl)boronic acid and was obtained as an off-white solid. MS (ESP): m/z = 316.1 [M+H]⁺.

### Intermediate 308

### 4-Fluoro-1-(1-tritylimidazol-4-yl)-6,7-dihydro-5H-cyclopenta[c]pyridine-6-carbaldehyde

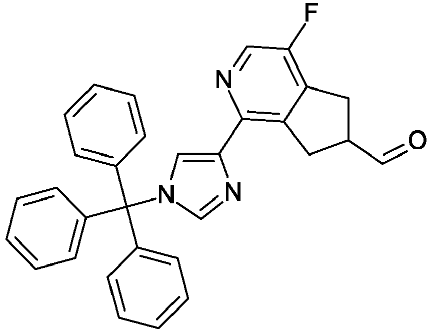

The title compound was prepared by analogy with 4-fluoro-1-(2-tetrahydropyran-2-ylpyrazol-3-yl)-6,7-dihydro-5*H*-cyclopenta[c]pyridine-6-carbaldehyde (Intermediate 304) using (1-tritylimidazol-4-yl)boronicacid (CAS# 1900755-49-8) in place of (2-tetrahydropyran-2-ylpyrazol-3-yl)boronic acid and was obtained as as an off-white solid. MS (ESP): m/z = 474.1 [M+H]⁺.

## Claims

1. A Compound represented by the following general formula (I) 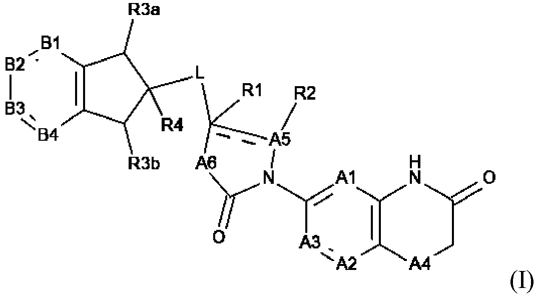 wherein
A¹ is selected from
i) -N-, and
ii) -CH-;
A² is selected from
i) -N-, and
ii) -CH-;
A³ is selected from
i) -N-, and
ii) -CH-;
A⁴ is selected from
i) -O-, and
ii) -S-;
A⁵ is selected from
i) -N-, and
ii) -CH-;
A⁶ is selected from
i) -O-, and
ii) -CH₂-;
-̅ -̅ represents a single or double bond;
B¹ is selected from
i) -N-, and
ii) -CR⁵-;
B² is selected from
i) -N-, and
ii) -CR⁶-;
wherein when B¹ is CR⁵ and B² is CR⁶, R⁵ and R⁶ may be bonded to each other to form a 4-to 6- membered ring optionally substituted with R^{a} and/or R^{b};
R^{a} and R^{b} are independently selected from
i) COOR^{a1}, and
ii) C₁₋₆-alkyl;
R^{a1} is selected from
i) H, and
ii) C₁₋₆-alkyl;
B³ is selected from
i) -N-, and
ii) -CR⁷-;
B⁴ is selected from
i) -N-, and
ii) -CR⁸-;
R¹ is selected from
i) H,
ii) NH₂,
iii) OH, and
iv) C₁₋₆ alkyl optionally substituted with R^{c},
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d};
R^{c} is selected from
i) OH, and
ii) NH₂;
R^{d} is selected from.
i) (CH2)nNH₂, and
ii) (CH2)nOH;
n represents an integer of 0 to 6;
R² is selected from
i) H, and
ii) C₁₋₆ alkyl optionally substituted with R^{c},
or R², L₂ and R^{w} may be bonded to each other to form a 4- to 6- membered ring optionally substituted with R^{d};
wherein
when -̅ -̅ represents a double bond, R₁ and R₂ are none;
R^{3a} is selected from
i) H,
ii) NH₂, and
iii) OH;
R^{3b} is selected from
i) H,
ii) NH₂, and
iii) OH;
wherein either one of R^{3a} and R^{3b} is H;
R⁴ is selected from
i) H,
ii) OH, and
iii) NH₂;
R⁵ is selected from
i) H,
ii) halogen,
iii) cyano,
iv) C₁₋₆-alkyl
v) 4- to 6 -membered heteroaryl optionally substituted with C₁₋₆-alkyl,
vi) OR^{e},
vii) SO₂R^{f},
viii) SO₂NR^{e}R^{f}, and
ix) NR^{e}R^{f};
R^{e} and R^{f} are independently selected from
i) H, and
ii) C₁₋₆-alkyl;
R⁶ is selected from
i) H,
ii) halogen,
iii) C₁₋₆-alkyl,
iv) OR^{g},
v) NR^{h}Rⁱ, and
vi) cyano;
R^{g} is selected from
i) H,
ii) -(CH₂)ₘ-R^{g1},
iii) 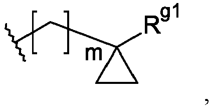
iv) 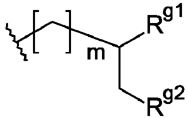
v) 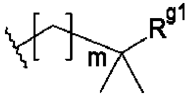 and
vi) 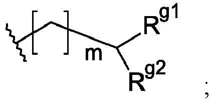
m represents an integer of 0 to 6;
R^{g1} and R^{g2} are independently selected from
i) H,
ii) OR^{j},
iii) NR^{k}R^{l},
iv) SO₂R^{m},
v) CONRⁿR^{o},
vi) NR^{p}COR^{q},
vii) NR^{r}SO₂R^{s},
viii) COOR¹,
ix) C₁₋₆-alkyl optionally substituted with C₁₋₆ alkoxy
x) heterocycloalkyl optionally substituted with R^{u}
xi) cyano, and
xii) heteroaryl;
R^{j}, R^{k}, R^{l}, R^{m}, Rⁿ, R^{o}, R^{p}, R^{q}, R^{r}, R^{s}, R^{t}, and R^{u} are independently selected from
i) H,
ii) C₁₋₆-alkyl optionally substituted with C₁₋₆ alkoxy
iii) C₃₋₈ cycloalkyl,
iv) NH₂,
v) heterocycloalkyl,
vi) -S(O)₂-C₁₋₆-alkyl,
vii) -C(O)-C₁₋₆-alkyl, and
viii) -O-C₁₋₆-alkyl;
R^{h} and Rⁱ are independently selected from
i) H, and
ii) C₁₋₆-alkyl;
R⁷ is selected from
i) H,
ii) halogen,
iii) C₁₋₆-alkyl, and
vi) cyano;
R⁸ is selected from
i) H,
ii) halogen,
iii) cyano
iv) C₁₋₆-alkyl, and
v) heteroaryl optionally substituted with C₁₋₆-alkyl;
L is selected from
i) -L₁-NR⁹-L₂-, and
ii) -L₁-heterocycloalkylene-L₂-;
L₁ is selected from
i) (CH₂)x optionally substituted with R^{v},
ii) heterocycloalkylene optionally substituted with R^{v}, and
iii) C₃₋₈ cycloalkylene optionally substituted with R^{v};
x represents an integer of 0 to 6;
R⁹ and R¹⁰ are independently selected from
i) H, and
ii) C₁₋₆-alkyl optionally substituted with R^{v},
or R¹, R⁹ and L₂ may be bonded to each other to form a 4- to 6- membered ring optionally substituted with R^{d};
R^{v} is selected from
i) OH,
ii) NH₂, and
iii) C₁₋₆-alkyl optionally substituted with hydroxy;
L₂ is selected from
i) (CH₂)y optionally substituted with R^{w} and/or R^{x},
ii) C₃₋₈-cycloalkylene optionally substituted with R^{w} and/or R^{x},
iii) heterocycloalkylene optionally substituted with R^{w} and/orR^{x},
iv) (CH₂)yNR¹⁰- optionally substituted with R^{w} and/or R^{x},
v) C₃₋₈-cycloalkylene-NR¹⁰- optionally substituted with R^{w} and/or R^{x}, and
vi) heterocycloalkylene- NR¹⁰- optionally substituted with R^{w} and/or R^{x}
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
y represents an integer of 0 to 6;
R^{w} and R^{x} are independently selected from
i) OH,
ii) NH₂, and
iii) C₁₋₆-alkyl optionally substituted with hydroxy;
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
or pharmaceutically acceptable salts thereof.

2. The Compound or a pharmaceutically acceptable salt thereof according to claim 1, wherein
A¹ is selected from
i) -N-, and
ii) -CH-;
A² is selected from
i) -N-, and
ii) -CH-;
A³ is CH;
A⁴ is selected from
i) -O-, and
ii) -S-;
A⁵ is selected from
i) -N-, and
ii) -CH-;
A⁶ is selected from
i) -O-, and
ii) -CH₂-;
-̅ -̅ represents a single or double bond;
B¹ is selected from
i) -N-, and
ii) -CR⁵-;
B² is selected from
i) -N-, and
ii) -CR⁶-;
wherein when B¹ is CR⁵ and B² is CR⁶, R⁵ and R⁶ may be bonded to each other to form a 4-to 6- membered ring as below, 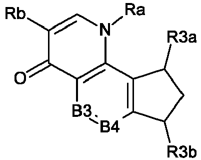
R^{a} and R^{b} are independently selected from
i) COOR^{a1}, and
ii) C₁₋₆-alkyl;
R^{a1} is selected from
i) H, and
ii) C₁₋₆-alkyl;
B³ is selected from
i) -N-, and
ii) -CR⁷-;
B⁴ is selected from
i) -N-, and
ii) -CR⁸-;
R¹ is selected from
i) H, and
ii) C₁₋₆ alkyl optionally substituted with R^{c},
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d};
R^{c} is NH₂;
R^{d} is (CH2)nNH₂;
n represents an integer of 0 to 3;
R² is H;
or R², L₂ and R^{w} may be bonded to each other to form a 4- to 6- membered ring optionally substituted with R^{d}
wherein
when -̅ -̅ represents a double bond, R₁ and R₂ are none;
R^{3a} is selected from
i) H,
ii) NH₂, and
iii) OH;
R^{3b} is selected from
i) H,
ii) NH₂, and
iii) OH;
wherein either one of R^{3a} and R^{3b} is H,
R⁴ is selected from
i) H, and
ii) NH₂;
R⁵ is selected from
i) H,
ii) halogen,
iii) cyano,
iv) C₁₋₆-alkyl, and,
v) OR^{e};
R^{e} is C₁₋₆-alkyl;
R⁶ is selected from
i) H,
ii) C₁₋₆-alkyl, and
iii) OR^{g};
R^{g} is selected from
i) H,
ii) -(CH₂)ₘ-R^{g1},
iii) 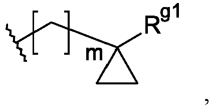
iv) 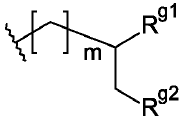
v) 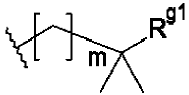 and
vi) 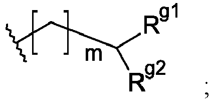
m represents an integer of 0 to 3;
R^{g1} and R^{g2} are independently selected from
i) H,
ii) OR^{j},
iii) NR^{k}R^{l},
iv) SO₂R^{m},
v) CONRⁿR^{o},
vi) NR^{p}COR^{q},
vii) NR^{r}SO₂R^{s},
viii) COOR^{t},
ix) C₁₋₆-alkyl optionally substituted with C₁₋₆ alkoxy
x) heterocycloalkyl optionally substituted with R^{u}, and
xi) cyano
R^{j}, R^{k}, R^{l}, R^{m}, Rⁿ, R^{o}, R^{p}, R^{q}, R^{r}, R^{s}, R^{t}, and R^{u} are independently selected from
i) H,
ii) C₁₋₆-alkyl optionally substituted with C₁₋₆ alkoxy, and,
iii) NH₂;
R⁷ is selected from
i) H, and
ii) C₁₋₆-alkyl;
R⁸ is selected from
i) H,
ii) halogen,
iii) cyano
iv) C₁₋₆-alkyl, and
v) heteroaryl optionally substituted with C₁₋₆-alkyl;
L is selected from
i) -L₁-NR⁹-L₂-, and
ii) -L₁- azetidinylene -L₂-
L₁ is selected from
i) (CH₂)x optionally substituted with R^{v}, and,
ii) heterocycloalkylene optionally substituted with R^{v};
x represents an integer of 0 to 3;
R⁹ and R¹⁰ are H;
or R¹, R⁹ and L₂ may be bonded to each other to form a 4- to 6- membered ring optionally substituted with R^{d};
R^{v} is NH₂;
L₂ is selected from
i) (CH₂)y optionally substituted with R^{w} and/or R^{x},
ii) C₃₋₈-cycloalkylene optionally substituted with R^{w} and/or R^{x},
iii) heterocycloalkylene optionally substituted with R^{w} and/or R^{x},
iv) (CH₂)yNR¹⁰- optionally substituted with R^{w} and/or R^{x},
v) C₃₋₈-cycloalkylene-NR¹⁰- optionally substituted with R^{w} and/or R^{x}, and
vi) heterocycloalkylene- NR¹⁰- optionally substituted with R^{w} and/or R^{x};
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
y represents an integer of 0 to 3;
R^{w} and R^{x} are OH;
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d}:

3. The Compound or a pharmaceutically acceptable salt thereof according to claim 1, wherein
A¹ is selected from
i) -N-, and
ii) -CH-;
A² is selected from
i) -N-, and
ii) -CH-;
A³ is CH;
A⁴ is selected from
i) -O-, and
ii) -S-;
A⁵ is CH;
A⁶ is O;
-̅ -̅ is a single bond,
B¹ is selected from
i) -N-, and
ii) -CR⁵-;
B² is selected from
i) -N-, and
ii) -CR⁶-;
B³ is selected from
i) -N-, and
ii) -CR⁷-;
B⁴ is selected from
i) -N-, and
ii) -CR⁸-;
R¹ is H;
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d};
R^{d} is (CH2)nNH₂, and
n represents an integer of 0 to 3;
R² is H;
or R², L₂ and R^{w} may be bonded to each other to form a 4- to 6- membered ring optionally substituted with R^{d}
R^{3a} is selected from
i) H,
ii) NH₂, and
iii) OH;
R^{3b} is selected from
i) H,
ii) NH₂, and
iii) OH;
wherein either one of R^{3a} and R^{3b} is H,
R⁴ is H;
R⁵ is selected from
i) H,
ii) halogen,
iii) cyano,
iv) C₁₋₆-alkyl,
v) OR^{e}, and
R^{e} is C₁₋₆-alkyl;
R⁶ is selected from
i) H,
ii) C₁₋₆-alkyl, and
iii) OR^{g};
R^{g} is selected from
i) H,
ii) -(CH₂)ₘ-R^{g1},
iii) 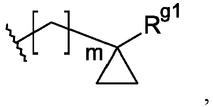
iv) 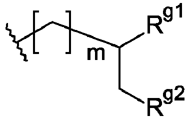
v) 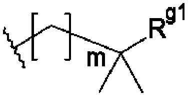 and
vi) 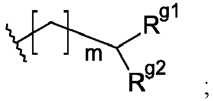
m represents an integer of 0 to 3;
R^{g1} and R^{g2} are independently selected from
i) H,
ii) OR^{j},
iii) NR^{k}R^{l},
iv) C₁₋₆-alkyl optionally substituted with C₁₋₆ alkoxy, and
v) heterocycloalkyl optionally substituted with R^{u};
R^{j}, R^{k}, R^{l}, and R^{u} are independently selected from
i) H,
ii) C₁₋₆-alkyl optionally substituted with C₁₋₆ alkoxy, and,
iii) NH₂;
R⁷ is selected from
i) H, and
ii) C₁₋₆-alkyl;
R⁸ is selected from
i) H,
ii) halogen,
iii) cyano,
iv) C₁₋₆-alkyl, and
v) heteroaryl optionally substituted with C₁₋₆-alkyl;
L is -L₁-NR⁹-L₂-;
L₁ is (CH₂)x optionally substituted with R^{v};
x represents an integer of 0 to 3;
R⁹ and R¹⁰ are H
or R¹, R⁹ and L₂ may be bonded to each other to form a 4- to 6- membered ring optionally substituted with R^{d};
R^{u} is NH₂
L₂ is selected from
i) (CH₂)y optionally substituted with R^{w} and/orR^{x},
ii) C₃₋₈-cycloalkylene optionally substituted with R^{w} and/or R^{x},
iii) heterocycloalkylene optionally substituted with R^{w} and/orR^{x},
iv) (CH₂)yNR¹⁰- optionally substituted with R^{w} and/or R^{x},
v) C₃₋₈-cycloalkylene-NR¹⁰- optionally substituted with R^{w} and/or R^{x}, and
vi) heterocycloalkylene- NR¹⁰- optionally substituted with R^{w} and/or R^{x};
or R¹, R⁹ and L₂ may be bonded to each other to form a 4- to 6- membered ring optionally substituted with R^{d},
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d};
y represents an integer of 0 to 3;
R^{w} and R^{x} are OH,
or R¹, L₂ and R^{w} may be bonded to each other to form a 4- to 6- membered ring optionally substituted with R^{d},
or R², L₂ and R^{w} may be bonded to each other to form a 4- to 6- membered ring optionally substituted with R^{d}:

4. The Compound or a pharmaceutically acceptable salt thereof according to claim 1, wherein
A¹ is selected from
i) -N-, and
ii) -CH-;
A² is selected from
i) -N-, and
ii) -CH-;
A³ is CH;
A⁴ is selected from
i) -O-, and
ii) -S-;
A⁵ is CH;
A⁶ is O;
-̅ -̅ is a single bond;
B¹ is selected from
i) -N-, and
ii) -CR⁵-;
B² is selected from
i) -N-, and
ii) -CR⁶-;
B³ is selected from
i) -N-, and
ii) -CR⁷-;
B⁴ is selected from
i) -N-, and
ii) -CR⁸-;
R¹ is H;
or R¹, R⁹ and L₂ may be bonded to each other to form a 4- to 6- membered ring as below, 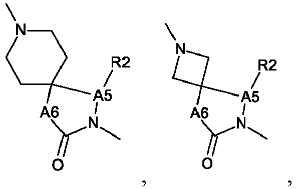
wherein the ring is optionally substituted with R^{d},
or R¹, L₂ and R^{w} may be bonded to each other to form a 4- to 6- membered ring as below, 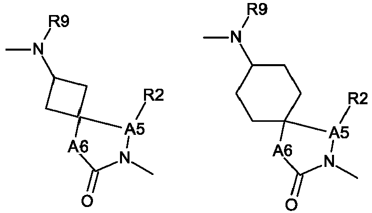
wherein the ring is optionally substituted with R^{d};
R^{d} is (CH2)nNH₂;
n represents an integer of 0 to 1;
R² is H;
or R², L₂ and R^{w} may be bonded to each other to form a 4- to 6- membered ring as below, 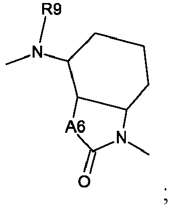
R^{3a} is selected from
i) H,
ii) NH₂, and
iii) OH;
R^{3b} is selected from
i) H,
ii) NH₂, and
iii) OH;
wherein either one of R^{3a} and R^{3b} is H,
R⁴ is H
R⁵ is selected from
i) H,
ii) halogen,
iii) cyano,
iv) C₁₋₆-alkyl, and,
v) OR^{e}, and
R^{e} is C₁₋₆-alkyl;
R⁶ is selected from
i) H,
ii) C₁₋₆-alkyl, and
iii) OR^{g};
R^{g} is selected from
i) H,
ii) -(CH₂)ₘ-R^{g1},
iii) 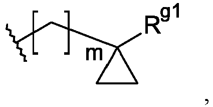
iv) 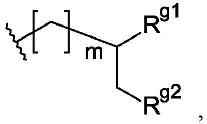
v) 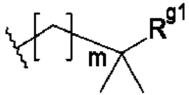 and
vi) 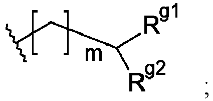
m represents an integer of 0 to 3;
R^{g1} and R^{g2} are independently selected from
i) H,
ii) OR^{j},
iii) NR^{k}R^{l},
iv) C₁₋₆-alkyl optionally substituted with C₁₋₆ alkoxy
v) pyrrolidinyl optionally substituted with R^{u}
vi) morpholinyl optionally substituted with R^{u}
vii) oxetanyl optionally substituted with R^{u}
viii) 3-oxo-morpholinyl optionally substituted with R^{u},
ix) 2-oxo-pyrrolidinyl optionally substituted with R^{u}
x) azetidinyl optionally substituted with R^{u}
xi) piperazinyl optionally substituted with R^{u}, and
xiI) 2-oxo-piperazinyl optionally substituted with R^{u};
R^{j}, R^{k}, R^{l}, and R^{u} are independently selected from
i) H,
ii) C₁₋₆-alkyl optionally substituted with C₁₋₆ alkoxy, and
iii) NH₂
R⁷ is selected from
i) H, and
ii) C₁₋₆-alkyl;
R⁸ is selected from
i) H,
ii) halogen,
iii) cyano
iv) C₁₋₆-alkyl, and
v) thiazole optionally substituted with C₁₋₆-alkyl;
L is -L₁-NR⁹-L₂-;
L₁ is (CH₂)x optionally substituted with R^{v};
x represents an integer of 0 to 3;
R⁹ and R¹⁰ are H;
or R¹, R⁹ and L₂ may be bonded to each other to form a 4- to 6- membered ring as below, 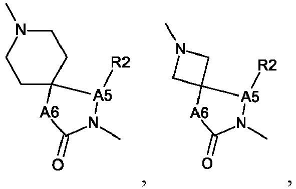
wherein the ring is optionally substituted with R^{d};
R^{u} is NH₂;
L₂ is selected from
i) (CH₂)y optionally substituted with R^{w} and/or R^{x},
ii) C₃₋₈-cycloalkylene optionally substituted with R^{w} and/or R^{x},
iii) (CH₂)yNR¹⁰- optionally substituted with R^{w} and/or R^{x}, and
iv) C₃₋₈-cycloalkylene-NR¹⁰- optionally substituted with R^{w} and/or R^{x}
or R¹, R⁹ and L₂ may be bonded to each other to form a 4- to 6- membered ring as below, 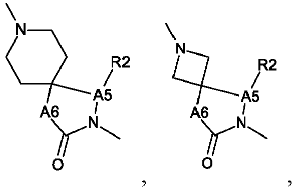
wherein the ring is optionally substituted with R^{d},
or R¹, L₂ and R^{w} may be bonded to each other to form a 4- to 6- membered ring as below, 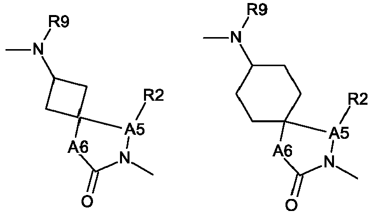
wherein the ring is optionally substituted with R^{d},
or R², L₂ and R^{w} may be bonded to each other to form a 4- to 6- membered ring as below, 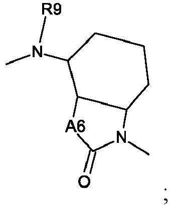
y represents an integer of 0 to 3;
R^{w} and R^{x} are OH
or R¹, L₂ and R^{w} may be bonded to each other to form a 4- to 6- membered ring as below, 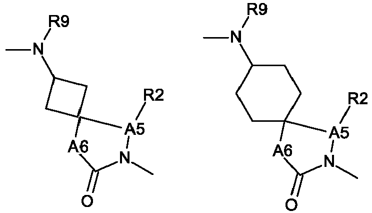
wherein the ring is optionally substituted with R^{d},
or R², L₂ and R^{w} may be bonded to each other to form a 4- to 6- membered ring as below,

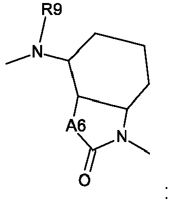

5. The Compound or a pharmaceutically acceptable salt thereof according to anyone of claim 1 to 4, wherein A¹ is -N-.

6. The Compound or a pharmaceutically acceptable salt thereof according to anyone of claim 1 to 5, wherein A² is -CH-.

7. The Compound or a pharmaceutically acceptable salt thereof according to anyone of claim 1 to 6, wherein A⁴ is -O-.

8. The Compound or a pharmaceutically acceptable salt thereof according to anyone of claim 1 to 7, wherein R^{3a} and R^{3b} are H.

9. A compound according to claim 1, selected from
6-[5-[2-[(4-chloro-2,3-dihydro-1H-inden-2-yl)methylamino]ethyl]-2-oxo-1,3-oxazolidin-3-yl]-4H-pyrido[3,2-b][1,4]oxazin-3-one;
6-[5-[3-[(4-chloro-2,3-dihydro-1H-inden-2-yl)methylamino]propyl]-2-oxo-1,3-oxazolidin-3-yl]-4H-pyrido[3,2-b][1,4]oxazin-3-one;
6-[5-[2-[(4-chloro-2,3-dihydro-1H-inden-2-yl)amino]ethyl]-2-oxo-1,3-oxazolidin-3-yl]-4H-pyrido[3,2-b] [1,4]oxazin-3-one;
6-[5-[3-[(4-chloro-2,3-dihydro-1H-inden-2-yl)amino]propyl]-2-oxo-1,3-oxazolidin-3-yl]-4H-pyrido[3,2-b] [1,4]oxazin-3-one;
2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[5-[2-[(4-Chloro-2,3-dihydro-1H-inden-2-yl)methylamino]ethyl]-2-oxo-1,3-oxazolidin-3-yl]-4H-pyrido[3,2-b][1,4]oxazin-3-one;
6-[5-[3-[(4-fluoro-2,3-dihydro-1H-inden-2-yl)amino]propyl]-2-oxo-1,3-oxazolidin-3-yl]-4H-pyrido[3,2-b] [1,4]oxazin-3-one;
6-[5-[3-(2,3-dihydro-1H-inden-2-ylamino)propyl]-2-oxo-1,3-oxazolidin-3-yl]-4H-pyrido[3,2-b][1,4]oxazin-3-one;
6-[5-[3-[(5-chloro-2,3-dihydro-1H-inden-2-yl)amino]propyl]-2-oxo-1,3-oxazolidin-3-yl]-4H-pyrido[3,2-b] [1,4]oxazin-3-one;
6-[5-[2-[(5-chloro-2,3-dihydro-1H-inden-2-yl)amino]ethyl]-2-oxo-1,3-oxazolidin-3-yl]-4H-pyrido[3,2-b] [1,4]oxazin-3-one;
6-[5-[3-[(5-fluoro-2,3-dihydro-1H-inden-2-yl)amino]propyl]-2-oxo-1,3-oxazolidin-3-yl]-4H-pyrido[3,2-b][1,4]oxazin-3-one;
2-[3-[2-oxo-3-(3-oxo-4H-pyrido[3,2-b][1,4]oxazin-6-yl)-1,3-oxazolidin-5-yl]propylamino]-2,3-dihydro-1H-indene-4-carbonitrile;
2-[[trans-3-[2-oxo-3-(3-oxo-4H-pyrido[3,2-b][1,4]oxazin-6-yl)-1,3-oxazolidin-5-yl]cyclobutyl]amino]-2,3-dihydro-1H-indene-4-carbonitrile;
6-[5-[2-[(4-chloro-2,3-dihydro-1H-inden-2-yl)methylamino]ethyl]-2-oxo-1,3-oxazolidin-3-yl]-4H-1,4-benzoxazin-3-one;
6-[5-[cis-3-[(4-chloro-2,3-dihydro-1H-inden-2-yl)amino]cyclobutyl]-2-oxo-1,3-oxazolidin-3-yl]-4H-pyrido[3,2-b][1,4]oxazin-3-one;
6-[5-[trans-3-[(4-chloro-2,3-dihydro-1H-inden-2-yl)amino]cyclobutyl]-2-oxo-1,3-oxazolidin-3-yl]-4H-pyrido[3,2-b][1,4]oxazin-3-one;
2-[[2-[2-oxo-3-(3-oxo-4H-1,4-benzoxazin-6-yl)-1,3-oxazolidin-5-yl]ethylamino]methyl]-2,3-dihydro-1H-indene-4-carbonitrile;
2-[[cis-3-[2-oxo-3-(3-oxo-4H-pyrido[3,2-b][1,4]oxazin-6-yl)-1,3-oxazolidin-5-yl]cyclobutyl]amino]-2,3-dihydro-1H-indene-4-carbonitrile;
6-[5-[2-[2-(4-chloro-2,3-dihydro-1H-inden-2-yl)ethylamino]ethyl]-2-oxo-1,3-oxazolidin-3-yl]-4H-pyrido[3,2-b][1,4]oxazin-3-one;
6-[5-[[(4-chloro-2,3-dihydro-1H-inden-2-yl)methylamino]methyl]-2-oxo-1,3-oxazolidin-3-yl]-4H-pyrido[3,2-b][1,4]oxazin-3-one;
6-[5-[[(4-chloro-2,3-dihydro-1H-inden-2-yl)amino]methyl]-2-oxo-1,3-oxazolidin-3-yl]-4H-pyrido[3,2-b][1,4]oxazin-3-one;
6-[(4R)-4-[[3-[(4-fluoro-2,3-dihydro-1H-inden-2-yl)amino]cyclobutyl]amino]-2-oxopyrrolidin-1 -yl]-4H-pyrido[3,2-b][1,4]oxazin-3 -one;
6-[(4S)-4-[[3-[(4-fluoro-2,3-dihydro-1H-inden-2-yl)amino]cyclobutyl]amino-2-oxopyrrolidin-1-yl]-4H-pyrido[3,2-b][1,4]oxazin-3-one;
6-[5-[[(5-chloro-2,3-dihydro-1H-inden-2-yl)amino]methyl]-2-oxo-1,3-oxazolidin-3-yl]-4H-pyrido[3,2-b][1,4]oxazin-3-one;
6-[5-[[2-(4-chloro-2,3-dihydro-1H-inden-2-yl)ethylamino]methyl]-2-oxo-1,3-oxazolidin-3-yl]-4H-pyrido[3,2-b][1,4]oxazin-3-one;
6-[5-[3-[(4-methyl-6,7-dihydro-SH-cyclopenta[b]pyridin-6-yl)amino]propyl]-2-oxo-1,3-oxazolidin-3-yl]-4H-pyrido[3,2-b][1,4]oxazin-3-one;
2-[[2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-indene-4-carbonitrile;
2-[[[2-methyl-1-[2-oxo-3-(3-oxo-4H-pyrido[3,2-b][1,4]oxazin-6-yl)-1,3-oxazolidin-5-yl]propan-2-yl]amino]methyl]-2,3-dihydro-1H-indene-4-carbonitrile;
6-[5-[2-[(1-chloro-6,7-dihydro-SH-cyclopenta[c]pyridin-6-yl)methylamino]-2-methylpropyl]-2-oxo-1,3-oxazolidin-3-yl]-4H-pyrido[3,2-b][1,4]oxazin-3-one;
2-[[[rac-3a,7a-trans-6,7a-cis-2-oxo-3-(3-oxo-4H-pyrido[3,2-b][1,4]oxazin-6-yl)-3a,4,5,6,7,7a-hexahydro-1,3-benzoxazol-6-yl]amino]methyl]-2,3-dihydro-1H-indene-4-carbonitrile;
6-[rac-3a,7a-trans-6,7a-cis-6-[2-(4-chloro-2,3-dihydro-1H-inden-2-yl)ethylamino]-2-oxo-3a,4,5,6,7,7a-hexahydro-1,3-benzoxazol-3-yl]-4H-pyrido[3,2-b][1,4]oxazin-3-one;
2-[2-[6-oxo-7-(3-oxo-4H-pyrido[3,2-b][1,4]oxazin-6-yl)-5-oxa-2,7-diazaspiro[3.4]octan-2-yl]ethyl]-2,3-dihydro-1H-indene-4-carbonitrile;
2-[[trans-4-[2-oxo-3-(3-oxo-4H-pyrido[3,2-b][1,4]oxazin-6-yl)-1,3-oxazolidin-5-yl]cyclohexyl]amino]-2,3-dihydro-1H-indene-4-carbonitrile;
2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
6-[5-[2-[(4-fluoro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;
4-fluoro-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile;
6-[5-[2-[(1-chloro-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-one;
2-[[2-oxo-3-(3-oxo-4H-pyrido[3,2-b][1,4]oxazin-6-yl)-1-oxa-3-azaspiro[4.5]decan-8-yl]amino]-2,3 -dihydro-1H-indene-4-carbonitrile;
6-[2-oxo-5-[2-[[rac-(5,6-trans)-1-chloro-5-hydroxy-6,7-dihydro-SH-cyclopenta[c]pyridin-6-yl]methylamino]ethyl]-1,3-oxazolidin-3-yl]-4H-pyrido[3,2-b][1,4]oxazin-3-one;
2-[[2-[5-oxo-4-(3-oxo-4H-1,4-benzoxazin-6-yl)-1,3,4-oxadiazol-2-yl]ethylamino]methyl]-2,3-dihydro-1H-indene-4-carbonitrile;
6-[2-oxo-5-[2-[[rac-(5,6-trans)-5-hydroxy-1-methyl-6,7-dihydro-SH-cyclopenta[c]pyridin-6-yl]methylamino]ethyl]-1,3-oxazolidin-3-yl]-4H-pyrido[3,2-b][1,4]oxazin-3-one;
2-[[2-[5-oxo-4-(3-oxo-4H-pyrido[3,2-b][1,4]oxazin-6-yl)-1,3,4-oxadiazol-2-yl]ethylamino]methyl]-2,3-dihydro-1H-indene-4-carbonitrile;
7-[5-[2-[(1-chloro-6,7-dihydro-SH-cyclopenta[c]pyridin-6-yl)methylamino]ethyl]-2-oxo-1,3-oxazolidin-3-yl]-1H-pyrido[2,3-b][1,4]oxazin-2-one;hydrochloride;
2-[[2-[2-oxo-3-(2-oxo-1H-pyrido[2,3-b][1,4]oxazin-7-yl)-1,3-oxazolidin-5-yl]ethylamino]methyl]-2,3-dihydro-1H-indene-4-carbonitrile;
1-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-SH-cyclopenta[c]pyridine-4-carbonitrile;
2-[[4-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide;
6-[5-[2-[(1-chloro-3-methoxy-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;
6-[5-[2-[(1-chloro-3-methoxy-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;
6-[S-[2-[(4-fluoro-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;
5-[(1-methylpyrrolidin-2-yl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
3-methoxy-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile;
S-(2-morpholin-4-ylethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[5-[2-[(4-chloro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one; formic acid;
N-[2-[[4-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]acetamide;
6-[S-[2-[(1-chloro-4-methoxy-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;
6-[5-[2-[(1-chloro-4-methoxy-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;
N-[2-[[4-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]methanesulfonamide;
6-[5-[2-[(4-chloro-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;
2-[[[(2R)-2-hydroxy-2-[(5R)-2-oxo-3-(3-oxo-4H-1,4-benzoxazin-6-yl)-1,3-oxazolidin-5-yl]ethyl]amino]methyl]-2,3-dihydro-1H-indene-4-carbonitrile;
2-[[[(2S)-2-hydroxy-2-[(5S)-2-oxo-3-(3-oxo-4H-1,4-benzoxazin-6-yl)-1,3-oxazolidin-5-yl]ethyl]amino]methyl]-2,3-dihydro-1H-indene-4-carbonitrile;
S-(oxetan-3-yloxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-indene-4-carbonitrile;
3-methoxy-6-[[2-[2-oxo-3-(3-oxo-4H-pyrazino[2,3-b][1,4]oxazin-6-yl)oxazolidin-5-yl]ethylamino]methyl]-6,7-dihydro-5H-cyclopenta[c]pyridine-1-carbonitrile;
2-[[1-chloro-6-[[2-[2-oxo-3-(3-oxo-4H-pyrido[3,2-b][1,4]oxazin-6-yl)oxazolidin-5-yl]ethylamino]methyl]-6,7-dihydro-5H-cyclopenta[c]pyridin-3-yl]oxy]-N-methyl-acetamide;
6-[5-[2-[(4-fluoro-1-methyl-6,7-dihydro-5H-cyclopenta[c]pyridin-6-yl)methylamino]ethyl]-2-oxo-1,3,4-oxadiazol-3-yl]-4H-1,4-benzoxazin-3-one;
6-[5-[2-[(4-fluoro-1-methyl-6,7-dihydro-5H-cyclopenta[c]pyridin-6-yl)methylamino]ethyl]-2-oxo-1,3,4-oxadiazol-3-yl]-4H-pyrido[3,2-b][1,4]oxazin-3-one;
2-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide;
6-[5-[2-[(4-fluoro-1-methoxy-6,7-dihydro-5H-cyclopenta[c]pyridin-6-yl)methylamino]ethyl]-2-oxo-oxazolidin-3-yl]-4H-pyrido[3,2-b][1,4]oxazin-3-one;
6-[5-[2-[(4-fluoro-1-methoxy-6,7-dihydro-5H-cyclopenta[c]pyridin-6-yl)methylamino]ethyl]-2-oxo-oxazolidin-3-yl]-4H-pyrazino[2,3-b][1,4]oxazin-3-one;
6-[8-[(4-fluoro-1-methyl-6,7-dihydro-SH-cyclopenta[c]pyridin-6-yl)methyl]-2-oxo-1-oxa-3, 8-diazaspiro[4.5]decan-3-yl]-4H-pyrido[3,2-b][1,4]oxazin-3-one;
2-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]acetonitrile;
2-[[7-fluoro-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-5-yl]oxy]-N-methylacetamide;
6-[5-[1-[(4-fluoro-1-methyl-6,7-dihydro-5H-cyclopenta[c]pyridin-6-yl)methyl]azetidin-3-yl]-2-oxo-oxazolidin-3-yl]-4H-pyrazino[2,3-b][1,4]oxazin-3-one;
2-[[3-[2-oxo-3-(3-oxo-4H-pyrazino[2,3-b][1,4]oxazin-6-yl)-1,3-oxazolidin-5-yl]azetidin-1-yl]methyl]-2,3-dihydro-1H-indene-4-carbonitrile;
2-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N,N-dimethylacetamide;
6-[5-[3-[(4-fluoro-1-methyl-6,7-dihydro-5H-cyclopenta[c]pyridin-6-yl)amino]propyl]-2-oxo-1,3-oxazolidin-3-yl]-4H-pyrido[3,2-b][1,4]oxazin-3-one;
6-[5-[3-[(4-fluoro-1-methyl-6,7-dihydro-SH-cyclopenta[c]pyridin-6-yl)amino]propyl]-2-oxo-1,3-oxazolidin-3-yl]-4H-pyrazino[2,3-b][1,4]oxazin-3-one;
2-[3-[2-oxo-3-(3-oxo-4H-pyrazino[2,3-b][1,4]oxazin-6-yl)-1,3-oxazolidin-5-yl]propylamino]-2,3 -dihydro-1H-indene-4-carbonitrile;
2-[[2-hydroxy-3-[2-oxo-3-(3-oxo-4H-pyrazino[2,3-b][1,4]oxazin-6-yl)-1,3-oxazolidin-5-yl]propyl]amino]-2,3-dihydro-1H-indene-4-carbonitrile;
6-[5-[3-[(4-fluoro-2,3-dihydro-1H-inden-2-yl)amino]-2-hydroxypropyl]-2-oxo-1,3-oxazolidin-3-yl]-4H-pyrazino[2,3-b][1,4]oxazin-3-one;
6-[5-[2-[(4-fluoro-1-methyl-6,7-dihydro-5H-cyclopenta[c]pyridin-6-yl)methylamino]ethyl]-2-oxo-1,3-oxazol-3-yl]-4H-pyrido[3,2-b][1,4]oxazin-3-one;
6-[5-[2-[(1-chloro-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-one;
6-[5-[2-(2,3-dihydro-1H-inden-2-ylmethylamino)ethyl]-2-oxo-1,3-oxazolidin-3-yl]-4H-pyrido[3,2-b][1,4]oxazin-3-one;
6-[5-[2-[(1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;
6-[5-[2-[(4-methyl-6,7-dihydro-SH-cyclopenta[b]pyridin-6-yl)methylamino]ethyl]-2-oxo-1,3-oxazolidin-3-yl]-4H-pyrido[3,2-b][1,4]oxazin-3-one;
6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile;
6-[5-[2-[(4-fluoro-2,3-dihydro-1H-inden-2-yl)methylamino]ethyl]-2-oxo-1,3-oxazolidin-3-yl]-4H-pyrido[3,2-b][1,4]oxazin-3-one;
6-[5-[3-[(1-chloro-6,7-dihydro-SH-cyclopenta[c]pyridin-6-yl)amino]propyl]-2-oxo-1,3-oxazolidin-3-yl]-4H-pyrido[3,2-b][1,4]oxazin-3-one;
6-[2-oxo-5-[2-[(1,3,4-trimethyl-6,7-dihydro-5H-cyclopenta[c]pyridin-6-yl)methylamino]ethyl]-1,3-oxazolidin-3-yl]-4H-pyrido[3,2-b][1,4]oxazin-3-one;
6-[5-[3-[(1-methyl-6,7-dihydro-SH-cyclopenta[c]pyridin-6-yl)amino]propyl]-2-oxo-1,3-oxazolidin-3-yl]-4H-pyrido[3,2-b][1,4]oxazin-3-one;
6-[3-[2-oxo-3-(3-oxo-4H-pyrido[3,2-b][1,4]oxazin-6-yl)-1,3-oxazolidin--yl]propylamino]-6,7-dihydro-5H-cyclopenta[c]pyridine-1-carbonitrile;
2-[[[cis-3-[2-oxo-3-(3-oxo-4H-pyrido[3,2-b][1,4]oxazin-6-yl)-1,3-oxazolidin-5-yl]cyclobutyl]amino]methyl]-2,3-dihydro-1H-indene-4-carbonitrile; formic acid;
2-[[[trans-3-[2-oxo-3-(3-oxo-4H-pyrido[3,2-b][1,4]oxazin-6-yl)-1,3-oxazolidin-5-yl]cyclobutyl]amino]methyl]-2,3-dihydro-1H-indene-4-carbonitrile;
6-[S-[2-[(1,4-dimethyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;
2-[[[rac-3a,7a-cis-6,7a-trans-2-oxo-3-(3-oxo-4H-pyrido[3,2-b][1,4]oxazin-6-yl)-3a,4,5,6,7,7a-hexahydro-1,3-benzoxazol-6-yl]amino]methyl]-2,3-dihydro-1H-indene-4-carbonitrile;
2-[[[trans-4-[2-oxo-3-(3-oxo-4H-pyrido[3,2-b][1,4]oxazin-6-yl)-1,3-oxazolidin-5-yl]cyclohexyl]amino]methyl]-2,3-dihydro-1H-indene-4-carbonitrile;
6-[5-[2-[[1,4-dimethyl-3-[(5-oxomorpholin-2-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[1,4-dimethyl-3-[(5-oxopyrrolidin-3-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[1,4-dimethyl-3-[(1-methyl-5-oxopyrrolidin-3-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[[[2,4-trans-6-oxo-7-(3-oxo-4H-pyrido[3,2-b][1,4]oxazin-6-yl)-5-oxa-7-azaspiro[3.4]octan-2-yl]amino]methyl]-6,7-dihydro-5H-cyclopenta[c]pyridine-1-carbonitrile;
6-[5-[2-[[1,4-dimethyl-3-(3-methylsulfonylpropoxy)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[(3-methoxy-1,4-dimethyl-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-one;
2-[[7-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide;
2-[[4-chloro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide;
2-[[4-chloro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;
2-[[1,4-dimethyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-3-yl]oxy]-N-methylacetamide;
2-[[1,4-dimethyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridin-3-yl]oxy]-N-methylacetamide;
2-[[4,7-dimethyl-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide;
2-[[4,7-dimethyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;
2-[[4,7-dimethyl-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-5-yl]oxy]-N-methylacetamide;
N-[2-[[7-cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]methanesulfonamide;
6-[5-[2-[[5-(2-aminoethoxy)-4-chloro-2,3-dihydro-1H-inden-2-yl]methylamino]ethyl]-2-oxo-1,3-oxazolidin-3-yl]-4H-pyrido[3,2-b][1,4]oxazin-3-one;
6-[5-[2-[[5-[(1-aminocyclopropyl)methoxy]-4-chloro-2,3-dihydro-1H-inden-2-yl]methylamino]ethyl]-2-oxo-1,3-oxazolidin-3-yl]-4H-pyrido[3,2-b][1,4]oxazin-3-one; formic acid;
6-[5-[3-[[5-(2-aminoethoxy)-4-chloro-2,3-dihydro-1H-inden-2-yl]amino]propyl]-2-oxo-1,3-oxazolidin-3-yl]-4H-pyrido[3,2-b][1,4]oxazin-3-one;
6-[5-[3-[[5-[(1-aminocyclopropyl)methoxy]-4-chloro-2,3-dihydro-1H-inden-2-yl]amino]propyl]-2-oxo-1,3-oxazolidin-3-yl]-4H-pyrido[3,2-b][1,4]oxazin-3-one;
6-[4-[2-[(4-fluoro-2,3-dihydro-1H-inden-2-yl)amino]ethylamino]-2-oxopyrrolidin-1-yl]-4H-pyrido[3,2-b][1,4]oxazin-3-one;
2-[2-[[5-oxo-1-(3-oxo-4H-pyrido[3,2-b][1,4]oxazin-6-yl)pyrrolidin-3-yl]amino]ethylamino]-2,3-dihydro-1H-indene-4-carbonitrile;
6-[5-[2-[[3-[(1-aminocyclopropyl)methoxy]-1-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[2-oxo-5-[2-[[5,6-trans-5-amino-1-chloro-6,7-dihydro-SH-cyclopenta[c]pyridin-6-yl]methylamino]ethyl]-1,3-oxazolidin-3-yl]-4H-pyrido[3,2-b][1,4]oxazin-3-one;
5-[(1-aminocyclopropyl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile hydrochloride;
3-[(1-aminocyclopropyl)methoxy]-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile;
6-[5-[2-[[3-(2-aminoethoxy)-1-chloro-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
3-(2-aminoethoxy)-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile;
5-(2-aminoethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
5-(azetidin-3-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[2-oxo-5-[2-[(rac-5,6-trans-5-amino-1-methyl-6,7-dihydro-SH-cyclopenta[c]pyridin-6-yl)methylamino]ethyl]-1,3-oxazolidin-3-yl]-4H-pyrido[3,2-b][1,4]oxazin-3-one;
rac-1,2-trans-1-amino-2-[[2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-indene-4-carbonitrile;
5-(azetidin-3-yloxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
rac-5,6-trans-5-amino-6-[[2-[2-oxo-3-(3-oxo-4H-pyrido[3,2-b][1,4]oxazin-6-yl)-1,3-oxazolidin-5-yl]ethylamino]methyl]-6,7-dihydro-SH-cyclopenta[c]pyridine-1-carbonitrile;
5-(2-aminopropoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
7-[2-oxo-5-[2-[[rac-1,2-trans-1-amino-4-chloro-2,3-dihydro-1H-inden-2-yl]methylamino]ethyl]-1,3-oxazolidin-3-yl]-1H-pyrido[2,3-b][1,4]oxazin-2-one;
6-[2-oxo-5-[2-[[rac-1,2-trans-1-amino-4-chloro-2,3-dihydro-1H-inden-2-yl]methylamino]ethyl]-1,3-oxazolidin-3-yl]-4H-pyrazino[2,3-b][1,4]oxazin-3-one;
S-(azetidin-2-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
2-[[2-[3-amino-5-oxo-1-(3-oxo-4H-pyrido[3,2-b][1,4]oxazin-6-yl)pyrrolidin-3-yl]ethylamino]methyl]-2,3-dihydro-1H-indene-4-carbonitrile;
rac-1,2-trans-1-hydroxy-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[4-amino-4-[2-[(4-chloro-2,3-dihydro-1H-inden-2-yl)methylamino]ethyl]-2-oxopyrrolidin-1-yl]-4H-pyrido[3,2-b][1,4]oxazin-3-one;
2-[[2-[2-oxo-3-(3-oxo-4H-pyrido[3,2-b][1,4]oxazin-6-yl)-1,3-oxazolidin-5-yl]ethylamino]methyl]-5-(pyrrolidin-2-ylmethoxy)-2,3-dihydro-1H-indene-4-carbonitrile;
6-[5-[[2-(2-amino-4-chloro-1,3-dihydroinden-2-yl)ethylamino]methyl]-2-oxo-1,3-oxazolidin-3-yl]-4H-pyrido[3,2-b][1,4]oxazin-3-one; formic acid;
2-[[2-[2-oxo-3-(3-oxo-4H-pyrido[3,2-b][1,4]oxazin-6-yl)-1,3-oxazolidin-5-yl]ethylamino]methyl]-5-(2-piperazin-1-ylethoxy)-2,3-dihydro-1H-indene-4-carbonitrile;
6-[5-[2-[[3-(2-aminopropoxy)-1-chloro-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-(2-aminopropoxy)-1-chloro-6,7-dihydro-SH-cyclopenta[c]pyridin-6-yl]methylamino]ethyl]-2-oxo-oxazolidin-3-yl]-4H-pyrazino[2,3-b][1,4]oxazin-3-one;
6-[2-oxo-5-[2-[[rac-5,6-cis-5-amino-1-chloro-6,7-dihydro-SH-cyclopenta[c]pyridin-6-yl]methylamino]ethyl]-1,3-oxazolidin-3-yl]-4H-pyrido[3,2-b][1,4]oxazin-3-one;
6-[2-oxo-5-[2-[[rac-1,2-trans-1-amino-4-chloro-2,3-dihydro-1H-inden-2-yl]methylamino]ethyl]-1,3,4-oxadiazol-3-yl]-4H-1,4-benzoxazin-3-one;
2-[[2-[rac-4,5-cis-4-(aminomethyl)-2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
2-[[2-[rac-4,5-trans-4-(aminomethyl)-2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[5-[2-[[6-(azetidin-3-yloxy)-4-fluoro-2,3-dihydro-1H-inden-2-yl]methylamino]ethyl]-2-oxo-1,3-oxazolidin-3-yl]-4H-pyrido[3,2-b][1,4]oxazin-3-one;
rac-2,3-trans-3-hydroxy-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
rac-2,3-trans-3-hydroxy-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-indene-4-carbonitrile;
6-[5-[2-[(rac-1,2-trans-1-amino-4-chloro-2,3-dihydro-1H-inden-2-yl)methylamino]ethyl]-2-oxo-1,3,4-oxadiazol-3-yl]-4H-pyrido[3,2-b][1,4]oxazin-3-one;
6-[5-[2-[[6-(2-aminopropoxy)-4-fluoro-2,3-dihydro-1H-inden-2-yl]methylamino]ethyl]-2-oxo-1,3-oxazolidin-3-yl]-4H-pyrido[3,2-b][1,4]oxazin-3-one;
6-[5-[2-[[6-(1-aminopropan-2-yloxy)-4-fluoro-2,3-dihydro-1H-inden-2-yl]methylamino]ethyl]-2-oxo-1,3-oxazolidin-3-yl]-4H-pyrido[3,2-b][1,4]oxazin-3-one;
rac-2,3-trans-3-amino-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
rac-2,3-trans-3-amino-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-indene-4-carbonitrile;
6-[5-[[[2-amino-2-(4-chloro-2,3-dihydro-1H-inden-2-yl)ethyl]amino]methyl]-2-oxo-1,3-oxazolidin-3-yl]-4H-pyrido[3,2-b][1,4]oxazin-3-one;
6-[2-[2-amino-2-(4-chloro-2,3-dihydro-1H-inden-2-yl)ethyl]-6-oxo-5-oxa-2,7-diazaspiro[3.4]octan-7-yl]-4H-pyrido[3,2-b][1,4]oxazin-3-one;
6-[5-[2-[[6-[(1-aminocyclopropyl)methoxy]-4-fluoro-2,3-dihydro-1H-inden-2-yl]methylamino]ethyl]-2-oxo-1,3-oxazolidin-3-yl]-4H-pyrido[3,2-b][1,4]oxazin-3-one;
6-[5-[2-[[6-[(1-aminocyclopropyl)methoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one
6-[5-[2-[[6-[(3-aminooxetan-3-yl)methoxy]-4-fluoro-2,3-dihydro-1H-inden-2-yl]methylamino]ethyl]-2-oxo-1,3-oxazolidin-3-yl]-4H-pyrido[3,2-b][1,4]oxazin-3-one;
6-[5-[2-[(rac-1,2-trans-1-amino-4-fluoro-2,3-dihydro-1H-inden-2-yl)methylamino]ethyl]-2-oxo-1,3-oxazolidin-3-yl]-4H-pyrido[3,2-b][1,4]oxazin-3-one;
6-[5-[2-[(rac-1,2-trans-1-amino-4-fluoro-2,3-dihydro-1H-inden-2-yl)methylamino]ethyl]-2-oxo-1,3-oxazolidin-3-yl]-4H-pyrazino[2,3-b][1,4]oxazin-3-one;
5-[2-[(rac-1,2-trans-1-amino-4-fluoro-2,3-dihydro-1H-inden-2-yl)methylamino]ethyl]-3-(3-oxo-4H-pyrido[3,2-b][1,4]thiazin-6-yl)-1,3-oxazolidin-2-one;
2-[[rac-5,6-cis-5,8-trans-6-amino-2-oxo-3-(3-oxo-4H-pyrido[3,2-b][1,4]oxazin-6-yl)-1-oxa-3-azaspiro[4.5]decan-8-yl]amino]-2,3-dihydro-1H-indene-4-carbonitrile;
6-[rac-5,6-cis-5,8-trans-6-amino-8-[(4-fluoro-2,3-dihydro-1H-inden-2-yl)amino]-2-oxo-1-oxa-3-azaspiro[4.5]decan-3-yl]-4H-pyrido[3,2-b][1,4]oxazin-3-one;
6-[2-oxo-5-[2-[[rac-1,2-trans-1-amino-7-chloro-2,3-dihydro-1H-inden-2-yl]methylamino]ethyl]-1,3-oxazolidin-3-yl]-4H-pyrido[3,2-b][1,4]oxazin-3-one;
6-[2-oxo-5-[2-[[rac-1,2-trans-1-amino-7-chloro-2,3-dihydro-1H-inden-2-yl]methylamino]ethyl]-1,3-oxazolidin-3-yl]-4H-pyrazino[2,3-b][1,4]oxazin-3-one;
6-[2-oxo-5-[2-[[rac-1,2-trans-1-amino-4-chloro-2,3-dihydro-1H-inden-2-yl]methylamino]ethyl]-1,3-oxazol-3-yl]-4H-pyrido[3,2-b][1,4]oxazin-3-one;
6-[5-[2-[[3-(2-aminoethoxy)-1,4-dimethyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-[(1-aminocyclopropyl)methoxy]-1,4-dimethyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[1,4-dimethyl-3-[2-(methylamino)ethoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-(2-aminoethoxy)-4-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-[(1-aminocyclopropyl)methoxy]-1-methyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-(3-aminopropoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[6-(2-aminoethoxy)-4-chloro-2,3-dihydro-1H-inden-2-yl]methylamino]ethyl]-2-oxo-1,3-oxazolidin-3-yl]-4H-pyrido[3,2-b][1,4]oxazin-3-one;
6-[5-[2-[[6-[(1-aminocyclopropyl)methoxy]-4-chloro-2,3-dihydro-1H-inden-2-yl]methylamino]ethyl]-2-oxo-1,3-oxazolidin-3-yl]-4H-pyrido[3,2-b][1,4]oxazin-3-one;
6-[5-[3-[[6-(2-aminoethoxy)-4-chloro-2,3-dihydro-1H-inden-2-yl]amino]propyl]-2-oxo-1,3-oxazolidin-3-yl]-4H-pyrido[3,2-b][1,4]oxazin-3-one;
6-[5-[3-[[6-[(1-aminocyclopropyl)methoxy]-4-chloro-2,3-dihydro-1H-inden-2-yl]amino]propyl]-2-oxo-1,3-oxazolidin-3-yl]-4H-pyrido[3,2-b][1,4]oxazin-3-one;
6-[5-[2-[[3-[(1-aminocyclopropyl)methoxy]-4-chloro-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[[[2,4-cis-6-oxo-7-(3-oxo-4H-pyrido[3,2-b][1,4]oxazin-6-yl)-5-oxa-7-azaspiro[3.4]octan-2-yl]amino]methyl]-6,7-dihydro-5H-cyclopenta[c]pyridine-1-carbonitrile;
2-[[[2,4-cis-6-oxo-7-(3-oxo-4H-pyrido[3,2-b][1,4]oxazin-6-yl)-5-oxa-7-azaspiro[3.4]octan-2-yl]amino]methyl]-2,3-dihydro-1H-indene-4-carbonitrile;
6-[5-[2-[[3-(azetidin-3-ylmethoxy)-1,4-dimethyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[(1-aminocyclopropyl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-((1-aminocyclopropyl)methoxy)-2-(((2-(2-oxo-3-(3-oxo-3,4-dihydro-2H-pyrazino[2,3-b][1,4]oxazin-6-yl)oxazolidin-5-yl)ethyl)amino)methyl)-2,3-dihydro-1H-indene-4-carbonitrile;
6-(azetidin-2-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-(2-aminoethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[5-[2-[[3-[2-(cyclopropylamino)ethoxy]-1,4-dimethyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-(azetidin-3-yloxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-(azetidin-3-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-(2-aminopropoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-(2-aminopropoxy)-2-(((2-(2-oxo-3-(3-oxo-3,4-dihydro-2H-pyrazino[2,3-b][1,4]oxazin-6-yl)oxazolidin-5-yl)ethyl)amino)methyl)-2,3-dihydro-1H-indene-4-carbonitrile;
6-[2-oxo-5-[[2-[rac-1,2-trans-1-amino-4-chloro-2,3-dihydro-1H-inden-2-yl]ethylamino]methyl]-1,3-oxazolidin-3-yl]-4H-pyrido[3,2-b][1,4]oxazin-3-one;
6-[6-oxo-2-[2-[rac-1,2-trans-1-amino-4-chloro-2,3-dihydro-1H-inden-2-yl]ethyl]-5-oxa-2,7-diazaspiro[3.4]octan-7-yl]-4H-pyrido[3,2-b][1,4]oxazin-3-one;
6-((1-aminocyclopropyl)methoxy)-2-(2-(((2-oxo-3-(3-oxo-3,4-dihydro-2H-pyrido[3,2-b][1,4]oxazin-6-yl)oxazolidin-5-yl)methyl)amino)ethyl)-2,3-dihydro-1H-indene-4-carbonitrile;
6-((1-aminocyclopropyl)methoxy)-2-(2-(6-oxo-7-(3-oxo-3,4-dihydro-2H-pyrido[3,2-b][1,4]oxazin-6-yl)-5-oxa-2,7-diazaspiro[3.4]octan-2-yl)ethyl)-2,3-dihydro-1H-indene-4-carbonitrile;
3-[(1-aminocyclopropyl)methoxy]-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-4-carbonitrile;
6-[5-[2-[[1,4-dimethyl-3-(morpholin-3-ylmethoxy)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-[[(2S)-azetidin-2-yl]methoxy]-1,4-dimethyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[1,4-dimethyl-3-[2-(propan-2-ylamino)ethoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-[2-(2-methoxyethylamino)ethoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-(2-amino-2-methylpropoxy)-2-(((2-(2-oxo-3-(3-oxo-3,4-dihydro-2H-pyrido[3,2-b][1,4]oxazin-6-yl)oxazolidin-5-yl)ethyl)amino)methyl)-2,3-dihydro-1H-indene-4-carbonitrile;
6-(2-amino-2-methylpropoxy)-2-(((2-(2-oxo-3-(3-oxo-3,4-dihydro-2H-pyrazino[2,3-b][1,4]oxazin-6-yl)oxazolidin-5-yl)ethyl)amino)methyl)-2,3-dihydro-1H-indene-4-carbonitrile;
6-[5-[1-[(rac-1,2-trans-1-amino-4-chloro-2,3-dihydro-1H-inden-2-yl)methyl]azetidin-3-yl]-2-oxo-1,3-oxazolidin-3-yl]-4H-pyrazino[2,3-b][1,4]oxazin-3-one;
6-[rac-5,6-cis-5,8-trans-6-amino-8-[(1-chloro-6,7-dihydro-SH-cyclopenta[c]pyridin-6-yl)methylamino]-2-oxo-1-oxa-3-azaspiro[4.5]decan-3-yl]-4H-pyrido[3,2-b][1,4]oxazin-3-one;
6-[2-Oxo-5-[2-[[1,2-trans-1-amino-4-chloro-2,3-dihydro-1H-inden-2-yl]methylamino]ethyl]-1,3-oxazolidin-3-yl]-4H-pyrido[3,2-b][1,4]oxazin-3-one;
6-[5-[2-[[3-(2-aminoethoxy)-1-methyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
2,4-cis-6-[6-oxo-2-[[rac-1,2-trans-1-amino-4-chloro-2,3-dihydro-1H-inden-2-yl]methylamino]-5-oxa-7-azaspiro[3.4]octan-7-yl]-4H-pyrido[3,2-b][1,4]oxazin-3-one;
6-[6-oxo-2-[[rac-(1R,2R)-1-amino-4-chloro-2,3-dihydro-1H-inden-2-yl]methylamino]-5-oxa-7-azaspiro[3.4]octan-7-yl]-4H-pyrido[3,2-b][1,4]oxazin-3-one;
6-[2-oxo-8-[[rac-1,2-trans-1-amino-4-chloro-2,3-dihydro-1H-inden-2-yl]methyl]-1-oxa-3,8-diazaspiro[4.5]decan-3-yl]-4H-pyrido[3,2-b][1,4]oxazin-3-on;e
3-[(1-aminocyclopropyl)methoxy]-4-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile;
3-[(1-aminocyclopropyl)methoxy]-1-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-4-carbonitrile;
3-[(1-aminocyclopropyl)methoxy]-1-methyl-6-[[2-oxo-3-(3-oxo-4H-pyrido[3,2-b][1,4]oxazin-6-yl)-1-oxa-3,8-diazaspiro[4.5]decan-8-yl]methyl]-6,7-dihydro-5H-cyclopenta[c]pyridine-4-carbonitril;e
6-[5-[2-[[3-[(1-aminocyclopropyl)methoxy]-4-methyl-1-(5-methyl-1,3-thiazol-2-yl)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-[(1-aminocyclopropyl)methoxy]-4-methyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[3-[(1-aminocyclopropyl)methoxy]-4-methyl-1-(1,3-thiazol-2-yl)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[4-methyl-3-[[(2R)-azetidin-2-yl]methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
4-methyl-6-((2-oxo-3-(3-oxo-3,4-dihydro-2H-pyrido[3,2-b][1,4]oxazin-6-yl)-1-oxa-3,8-diazaspiro[4.5]decan-8-yl)methyl)-3-((5-oxopiperazin-2-yl)methoxy)-6,7-dihydro-5H-cyclopenta[c]pyridine-1-carbonitrile;
2-((1-cyano-4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)-N-methylacetamide;
2-((1-cyano-4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)-N-isopropylacetamide;
N-methyl-2-((4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)acetamide;
N-isopropyl-2-((4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)acetamide;
ethyl 1-ethyl-6-fluoro-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylate;
6-[2-oxo-5-[2-[[rac-1,2-trans-1-amino-4-chloro-2,3-dihydro-1H-inden-2-yl]methylamino]ethyl]-1,3-oxazolidin-3-yl]-4H-pyrido[3,2-b][1,4]oxazin-3-one;
6-[2-oxo-5-[3-[[rac-1,2-trans-1-amino-4-chloro-2,3-dihydro-1H-inden-2-yl]methylamino]propyl]-1,3-oxazolidin-3-yl]-4H-pyrido[3,2-b][1,4]oxazin-3-one;
rac-1,2-trans-1-amino-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
rac-1,2-trans-1-amino-2-((((2-oxo-3-(3-oxo-3,4-dihydro-2H-pyrido[3,2-b][1,4]oxazin-6-yl)oxazolidin-5-yl)methyl)amino)methyl)-2,3-dihydro-1H-indene-4-carbonitrile;
6-[2-oxo-5-[2-[[rac-1,2-trans-4-chloro-1-hydroxy-2,3-dihydro-1H-inden-2-yl]methylamino]ethyl]-1,3-oxazolidin-3-yl]-4H-pyrido[3,2-b][1,4]oxazin-3-one;
6-[2-oxo-5-[2-[[rac-1,2-cis-4-chloro-1-hydroxy-2,3-dihydro-1H-inden-2-yl]methylamino]ethyl]-1,3-oxazolidin-3-yl]-4H-pyrido[3,2-b][1,4]oxazin-3-one;
6-[5-[2-[(4-chloro-2-hydroxy-1,3-dihydroinden-2-yl)methylamino]ethyl]-2-oxo-1,3-oxazolidin-3-yl]-4H-pyrido[3,2-b][1,4]oxazin-3-one;
2-[[2-aminoethyl-[2-[2-oxo-3-(3-oxo-4H-pyrido[3,2-b][1,4]oxazin-6-yl)-1,3-oxazolidin-S-yl]ethyl]amino]methyl]-2,3-dihydro-1H-indene-4-carbonitrile;
6-[5-[2-[[1,4-dimethyl-3-[(2R)-2-amino-3-hydroxypropoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
3-[(2R)-2-amino-3-hydroxypropoxy]-4-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridine-1-carbonitrile;
6-[(2R)-2-amino-3-hydroxypropoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[5-[2-[[4-chloro-3-[rac-(2R)-2-amino-3-hydroxypropoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
2-[[2-Hydroxy-3-[2-oxo-3-(3-oxo-4H-pyrido[3,2-b][1,4]oxazin-6-yl)-1,3-oxazolidin-S-yl]propyl]amino]-2,3-dihydro-1H-indene-4-carbonitrile;
1-ethyl-6-fluoro-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylic acid;
6-[5-[2-[(2-amino-4-chloro-1,3-dihydroinden-2-yl)methylamino]ethyl]-2-oxo-1,3-oxazolidin-3-yl]-4H-pyrido[3,2-b][1,4]oxazin-3-one;
2-[[rac-5,6-trans-5,8-cis-6-Amino-2-oxo-3-(3-oxo-4H-pyrido[3,2-b][1,4]oxazin-6-yl)-1-oxa-3-azaspiro[4.5]decan-8-yl]amino]-2,3-dihydro-1H-indene-4-carbonitrile;
6-[5-(aminomethyl)-5-[2-[(4-fluoro-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;
2-[[2-[5-(aminomethyl)-2-oxo-3-(3-oxo-4H-pyrido[3,2-b][1,4]oxazin-6-yl)-1,3-oxazolidin-5-yl]ethylamino]methyl]-2,3-dihydro-1H-indene-4-carbonitrile;
6-[2-oxo-5-[3-[[rac-1,2-trans-1-amino-4-chloro-2,3-dihydro-1H-inden-2-yl]amino]propyl]-1,3-oxazolidin-3-yl]-4H-pyrido[3,2-b][1,4]oxazin-3-one;
6-[2-oxo-5-[3-[[rac-1,2-trans-1-amino-4-chloro-2,3-dihydro-1H-inden-2-yl]amino]propyl]-1,3-oxazolidin-3-yl]-4H-pyrazino[2,3-b][1,4]oxazin-3-one;
5-[2-[(rac-1,2-trans-1-amino-4-chloro-2,3-dihydro-1H-inden-2-yl)methylamino]ethyl]-3-(3-oxo-4H-pyrido[3,2-b][1,4]thiazin-6-yl)-1,3-oxazolidin-2-one;
rac-1,2-trans-1-amino-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-indene-4-carbonitrile;
6-[5-[2-[[6-(2-amino-3-methoxypropoxy)-4-fluoro-2,3-dihydro-1H-inden-2-yl]methylamino]ethyl]-2-oxo-1,3-oxazolidin-3-yl]-4H-pyrido[3,2-b][1,4]oxazin-3-one;
2-[[rac-5,6-trans-6-(Aminomethyl)-2-oxo-3-(3-oxo-4H-pyrido[3,2-b][1,4]oxazin-6-yl)-I-oxa-3,8-diazaspiro[4.5]decan-8-yl]methyl]-2,3-dihydro-1H-indene-4-carbonitrile;
6-[rac-5,6-trans-6-(aminomethyl)-8-[(4-fluoro-1-methyl-6,7-dihydro-SH-cyclopenta[c]pyridin-6-yl)methyl]-2-oxo-1-oxa-3,8-diazaspiro[4.5]decan-3-yl]-4H-pyrido[3,2-b][1,4]oxazin-3-one;
6-[rac-5,6-cis-6-(aminomethyl)-8-[(4-fluoro-1-methyl-6,7-dihydro-SH-cyclopenta[c]pyridin-6-yl)methyl]-2-oxo-1-oxa-3,8-diazaspiro[4.5]decan-3-yl]-4H-pyrido[3,2-b][1,4]oxazin-3-one;
2-[[rac-5,6-cis-6-(Aminomethyl)-2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-indene-4-carbonitrile;
6-[5-[2-[[5-[(1-aminocyclopropyl)methoxy]-4,7-dimethyl-2,3-dihydro-1H-inden-2-yl]methylamino]ethyl]-2-oxo-1,3-oxazolidin-3-yl]-4H-pyrazino[2,3-b][1,4]oxazin-3-one;
2-[[2-[2-oxo-3-(3-oxo-4H-pyrido[3,2-b][1,4]oxazin-6-yl)-1,3-oxazolidin-5-yl]ethylamino]methyl]-6-pyrrolidin-3-yloxy-2,3-dihydro-1H-indene-4-carbonitrile; formic acid
2-amino-3-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylpropanamide;
6-[5-[2-[(1-chloro-4-fluoro-3-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-one;
6-[8-[[6-[(1-aminocyclopropyl)methoxy]-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;
6-[8-[(4-fluoro-1-methyl-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-one;
6-[2-oxo-8-[[rac-1,2-trans-1-amino-4-chloro-2,3-dihydro-1H-inden-2-yl]methyl]-1-oxa-3,8-diazaspiro[4.5]decan-3-yl]-4H-pyrazino[2,3-b][1,4]oxazin-3-one;
6-[8-[[6-(2-aminopropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;
2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-indene-4-carbonitrile;
6-[5-[2-[(rac-5,6-trans-5-amino-4-chloro-1-methyl-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-one;
6-[2-oxo-5-[2-[[rac-5,6-trans-5-amino-4-chloro-1-methyl-6,7-dihydro-5H-cyclopenta[c]pyridin-6-yl]methylamino]ethyl]-1,3-oxazolidin-3-yl]-4H-pyrazino[2,3-b][1,4]oxazin-3-one;
3-(3-oxo-4H-pyrido[3,2-b][1,4]thiazin-6-yl)-5-[2-[[rac-5,6-trans-5-amino-4-chloro-1-methyl-6,7-dihydro-5H-cyc1openta[c]pyridin-6-yl]methylamino]ethyl]-1,3-oxazolidin-2-one;
6-[5-[[2-(4-fluoro-1-methyl-6,7-dihydro-5H-cyc1openta[c]pyridin-6-yl)ethylamino]methyl]-2-oxo-1,3-oxazolidin-3-yl]-4H-pyrazino[2,3-b][1,4]oxazin-3-one;
6-[2-oxo-5-[[2-[rac-1,2-trans-1-amino-4-chloro-2,3-dihydro-1H-inden-2-yl]ethylamino]methyl]-1,3-oxazolidin-3-yl]-4H-pyrazino[2,3-b][1,4]oxazin-3-one;
2-amino-3-[[7-fluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]propanoic acid;
6-[5-[2-[(1,4-dimethyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;
6-[2-oxo-5-[2-[(1,3,4-trimethyl-6,7-dihydro-5H-cyclopenta[c]pyridin-6-yl)methylamino]ethyl]-1,3-oxazolidin-3-yl]-4H-pyrazino[2,3-b][1,4]oxazin-3-one;
6-[5-[2-[(4-methyl-6,7-dihydro-SH-cyclopenta[b]pyridin-6-yl)methylamino]ethyl]-2-oxo-1,3-oxazolidin-3-yl]-4H-pyrazino[2,3-b][1,4]oxazin-3-one;
6-[5-[2-[[6-[2-(dimethylamino)ethoxy]-4-fluoro-2,3-dihydro-1H-inden-2-yl]methylamino]ethyl]-2-oxo-1,3-oxazolidin-3-yl]-4H-pyrido[3,2-b][1,4]oxazin-3-one;
6-[5-[2-[[6-[2-(dimethylamino)ethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;
6-[2-oxo-5-[2-[[rac-1,2-trans-1-amino-6-[(1-aminocyclopropyl)methoxy]-4-chloro-2,3-dihydro-1H-inden-2-yl]methylamino]ethyl]-1,3-oxazolidin-3-yl]-4H-pyrido[3,2-b][1,4]oxazin-3-one;
6-[2-oxo-8-[[rac-1,2-trans-1-amino-6-[(1-aminocyclopropyl)methoxy]-4-chloro-2,3-dihydro-1H-inden-2-yl]methyl]-1-oxa-3,8-diazaspiro[4.5]decan-3-yl]-4H-pyrido[3,2-b][1,4]oxazin-3-one;
1-ethyl-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]quinoline-3-carboxylic acid;
1-ethyl-4-oxo-8-[[2-[2-oxo-3-(3-oxo-4H-pyrazino[2,3-b][1,4]oxazin-6-yl)-1,3-oxazolidin-5-yl]ethylamino]methyl]-8,9-dihydro-7H-cyclopenta[h]quinoline-3-carboxylic acid;
6-[8-[(4-fluoro-1-methyl-6,7-dihydro-5H-cyclopenta[c]pyridin-6-yl)methyl]-6-(hydroxymethyl)-2-oxo-1-oxa-3,8-diazaspiro[4.5]decan-3-yl]-4H-pyrido[3,2-b][1,4]oxazin-3-one;
6-[5-[[2-(4-fluoro-1-methyl-6,7-dihydro-5H-cyc1openta[c]pyridin-6-yl)ethylamino]methyl]-5-(2-hydroxyethyl)-2-oxo-1,3-oxazolidin-3-yl]-4H-pyrido[3,2-b][1,4]oxazin-3-one;
6-[5-[2-[(1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;formic acid;
6-[5-[[2-(6-amino-4-fluoro-1-methyl-5,7-dihydrocyclopenta[c]pyridin-6-yl)ethylamino]methyl]-2-oxo-1,3-oxazolidin-3-yl]-4H-pyrido[3,2-b][1,4]oxazin-3-one;
6-[5-[2-[(4-chloro-2,3-dihydro-1H-inden-2-yl)methylamino]ethyl]-2-oxo-1,3-oxazolidin-3-yl]-4H-pyrazino[2,3-b][1,4]oxazin-3-one;
6-[2-Oxo-5-[2-[[rac-1,2-trans-1-amino-6-[(1-aminocyclopropyl)methoxy]-4-chloro-2,3-dihydro-1H-inden-2-yl]methylamino]ethyl]-1,3-oxazolidin-3-yl]-4H-pyrazino[2,3-b][1,4]oxazin-3-one;formic acid;
6-[5-[2-[(1,4-dimethyl-6,7-dihydro-5H-cyclopenta[d]pyridazin-6-yl)methylamino]ethyl]-2-oxo-1,3-oxazolidin-3-yl]-4H-pyrazino[2,3-b][1,4]oxazin-3-one;
6-[5-[2-[(1,4-dimethyl-6,7-dihydro-5H-cyclopenta[d]pyridazin-6-yl)methylamino]ethyl]-2-oxo-1,3-oxazolidin-3-yl]-4H-pyrido[3,2-b][1,4]oxazin-3-one;
5-[2-[(1,4-dimethyl-6,7-dihydro-SH-cyclopenta[d]pyridazin-6-yl)methylamino]ethyl]-3-(3-oxo-4H-pyrido[3,2-b][1,4]thiazin-6-yl)-1,3-oxazolidin-2-one;
2-[[4-cyano-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;
6-[5-[2-[(4-fluoro-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-one;
6-[5-[2-[(4-fluoro-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-one;
2-[[2-[2-oxo-3-(7-oxo-8H-pyrimido[5,4-b][1,4]oxazin-2-yl)-1,3-oxazolidin-5-yl]ethylamino]methyl]-2,3-dihydro-1H-indene-4-carbonitrile;
2-[5-[2-[(rac-1,2-trans-1-amino-4-chloro-2,3-dihydro-1H-inden-2-yl)methylamino]ethyl]-2-oxo-1,3-oxazolidin-3-yl]-8H-pyrimido[5,4-b][1,4]oxazin-7-one;
1-methyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridine-4-carbonitrile;2,2,2-trifluoroacetic acid;
6-[5-[2-[[4-fluoro-6-(2-hydroxy-2-methylpropoxy)-2,3-dihydro-1H-inden-2-yl]methylamino]ethyl]-2-oxo-1,3-oxazolidin-3-yl]-4H-pyrazino[2,3-b][1,4]oxazin-3-one;
6-[5-[2-[(4-fluoro-1-methyl-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-one;
6-[5-[2-[[6-[2-(5-aminotetrazol-1-yl)ethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid;
6-[5-[2-[[6-[2-(5-aminotetrazol-2-yl)ethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid;
6-[5-[2-[[4-fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
trans-6-[5-[2-[[4-Fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
cis-6-[5-[2-[[4-Fluoro-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
6-[5-[2-[(3-fluoro-4-methyl-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-one;
6-[5-[2-[(3-fluoro-4-methyl-6,7-dihydro-5H-cyclopenta[b]pyridin-6-yl)methylamino]ethyl]-2-oxo-1,3-oxazolidin-3-yl]-4H-pyrido[3,2-b][1,4]oxazin-3-one;
6-[8-[(3-fluoro-4-methyl-6,7-dihydro-5H-cyclopenta[b]pyridin-6-yl)methyl]-2-oxo-l-oxa-3,8-diazaspiro[4.5]decan-3-yl]-4H-pyrazino[2,3-b][1,4]oxazin-3-one;
6-[5-[3-[(4-fluoro-1-methyl-6,7-dihydro-SH-cyclopenta[c]pyridin-6-yl)amino]propyl]-2-oxo-1,3-oxazol-3-yl] -4H-pyrazino[2,3 -b] [1,4]oxazin-3-one;formic acid;
6-[5-[3-[(4-fluoro-l-methyl-6,7-dihydro-5H-cyclopenta[c]pyridin-6-yl)-methylamino]propyl]-2-oxo-1,3-oxazol-3-yl]-4H-pyrazino[2,3-b][1,4]oxazin-3-one;formic acid;
6-[5-[2-[[4-fluoro-6-[2-(2-oxo-1,3-oxazolidin-3-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide;
2-[[7-fluoro-2-[[2-[2-oxo-3-(3-oxo-4H-pyrazino[2,3-b][1,4]oxazin-6-yl)-1,3-oxazol-5-yl]ethylamino]methyl]-2,3-dihydro-1H-inden-5-yl]oxy]acetamide;formic acid;
6-[5-[2-[[6-[2-(azetidin-1-yl)-2-oxoethoxy]-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid;
N-ethyl-2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide;formic acid;
2-[[7-fluoro-2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-inden-5-yl]oxy]-N-methylacetamide;
2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;formic acid;
2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methoxyacetamide;formic acid;
2-[[4,7-difluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide;
6-[2-cxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer A;
6-[2-oxo-5-[3-[(4-fluoro-l-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer B;
6-[2-oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer C;
6-[2-oxo-5-[3-[(4-fluoro-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one; formic acid, Isomer D;
6-[5-[2-[(4-fluoro-6-hydroxy-2,3-dihydro-1H-inden-2-yl)methylamino]ethyl]-2-oxo-1,3-oxazolidin-3-yl]-4H-pyrazino[2,3-b][1,4]oxazin-3-one;
6-[5-[2-[(4-fluoro-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-one;
6-[5-[2-[(4-chloro-3-fluoro-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-one;formic acid;
6-[5-[2-[[4-fluoro-6-(1H-pyrazol-3-ylmethoxy)-2,3-dihydro-1H-inden-2-yl]methylamino]ethyl]-2-oxo-1,3-oxazolidin-3-yl]-4H-pyrazino[2,3-b][1,4]oxazin-3-one;formic acid;
1-methyl-6-[3-[2-oxo-3-(3-oxo-4H-pyrazino[2,3-b][1,4]oxazin-6-yl)-1,3-oxazol-5-yl]propylamino]-6,7-dihydro-5H-cyclopenta[c]pyridine-4-carbonitrile, Enantiomer A;
1-methyl-6-[3-[2-oxo-3-(3-oxo-4H-pyrazino[2,3-b][1,4]oxazin-6-yl)-1,3-oxazol-5-yl]propylamino]-6,7-dihydro-5H-cyclopenta[c]pyridine-4-carbonitrile, Enantiomer B;
6-[5-[2-[[4-fluoro-6-(oxetan-3-yloxy)-2,3-dihydro-1H-inden-2-yl]methylamino]ethyl]-2-oxo-1,3-oxazolidin-3-yl]-4H-pyrazino[2,3-b][1,4]oxazin-3-one;
N-[2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]ethyl]methanesulfonamide;
N-[2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]ethyl]-N-methylmethanesulfonamide;
6-[2-[2-(4-fluoro-1-methyl-6,7-dihydro-5H-cyclopenta[c]pyridin-6-yl)ethyl]-6-oxo-5-oxa-2,7-diazaspiro[3.4]octan-7-yl]-4H-pyrazino[2,3-b][1,4]oxazin-3-one;
6-[2-[2-[6-(2-aminopropoxy)-4-fluoro-2,3-dihydro-1H-inden-2-yl]ethyl]-6-oxo-5-oxa-2,7-diazaspiro[3.4]octan-7-yl]-4H-pyrazino[2,3-b][1,4]oxazin-3-one;
6-[5-[3-[(4-fluoro-1-methyl-6,7-dihydro-5H-cyclopenta[c]pyridin-6-yl)amino]cyclobutyl]-2-oxo-1,3-oxazolidin-3-yl]-4H-pyrazino[2,3-b][1,4]oxazin-3-one,IsomerA;
6-[5-[3-[(4-fluoro-1-methyl-6,7-dihydro-5H-cyclopenta[c]pyridin-6-yl)amino]cyclobutyl]-2-oxo-1,3-oxazolidin-3-yl]-4H-pyrazino[2,3-b][1,4]oxazin-3-one, Isomer B;
6-[(5S)-5-[2-[1-(4-fluoro-1-methyl-6,7-dihydro-5H-cyc1openta[c]pyridin-6-yl)ethylamino]ethyl]-2-oxo-1,3-oxazolidin-3-yl]-4H-pyrazino[2,3-b][1,4]oxazin-3-one; formic acid;
6-[(5R)-5-[2-[1-(4-fluoro-1-methyl-6,7-dihydro-5H-cyclopenta[c]pyridin-6-yl)ethylamino]ethyl]-2-oxo-1,3-oxazolidin-3-yl]-4H-pyrazino[2,3-b][1,4]oxazin-3-one; formic acid;
6-[5-[2-[[4-fluoro-6-(2-imidazol-1-ylethoxy)-2,3-dihydro-1H-inden-2-yl]methylamino]ethyl]-2-oxo-1,3-oxazolidin-3-yl]-4H-pyrazino[2,3-b][1,4]oxazin-3-one;
6-[5-[3-[(4-fluoro-1-methyl-6,7-dihydro-SH-cyclopenta[c]pyridin-6-yl)amino]butyl]-2-oxo-1,3-oxazolidin-3-yl]-4H-pyrazino[2,3-b][1,4]oxazin-3-one, Isomer A;
6-[5-[3-[(4-fluoro-1-methyl-6,7-dihydro-SH-cyclopenta[c]pyridin-6-yl)amino]butyl]-2-oxo-1,3-oxazolidin-3-yl]-4H-pyrazino[2,3-b][1,4]oxazin-3-one, Isomer B;
6-[5-[2-[(4-fluoro-1-methyl-6,7-dihydro-5H-cyc1openta[c]pyridin-6-yl)methylamino]ethyl]-2-oxo-1,3,4-oxadiazol-3-yl]-4H-pyrazino[2,3-b][1,4]oxazin-3-one;
2-[[4-fluoro-2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-inden-5-yl]oxy]-N-methylacetamide;
6-[2-oxo-5-[2-[[rac-1,2-trans-1-amino-4-chloro-2,3-dihydro-1H-inden-2-yl]methylamino]ethyl]-1,3,4-oxadiazol-3-yl]-4H-pyrazino[2,3-b][1,4]oxazin-3-one; formic acid;
6-[(5S)-5-[2-[(4-fluoro-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;
6-[(5R)-5-[2-[(4-fluoro-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;
6-[5-[2-[(4-fluoro-1-methyl-6,7-dihydro-5H-cyc1openta[c]pyridin-6-yl)methylamino]ethyl]-5-methyl-2-oxo-1,3-oxazolidin-3-yl]-4H-pyrazino[2,3-b][1,4]oxazin-3-one;
6-[(5S)-5-[2-[[6-(2-amino-2-methylpropoxy)-4-fluoro-2,3-dihydro-1H-inden-2-yl]methylamino]ethyl]-2-oxo-1,3-oxazolidin-3-yl]-4H-pyrazino[2,3-b][1,4]oxazin-3-one; formic acid;
6-[(5R)-5-[2-[[6-(2-amino-2-methylpropoxy)-4-fluoro-2,3-dihydro-1H-inden-2-yl]methylamino]ethyl]-2-oxo-1,3-oxazolidin-3-yl]-4H-pyrazino[2,3-b][1,4]oxazin-3-one; formic acid;
6-[5-[2-[[4-fluoro-6-[2-(triazol-2-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
6-[5-[2-[[4-fluoro-6-[2-(triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
1-[2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]ethyl]pyrrolidine-2,5-dione;formic acid;
N-cyclopropyl-2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide;formic acid 6-[5-[2-[[4-fluoro-6-[2-(tetrazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
6-[5-[2-[[4-fluoro-6-[2-(tetrazol-2-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-one;formic acid;
2-[4-[2-oxo-3-(3-oxo-4H-pyrazino[2,3-b][1,4]oxazin-6-yl)-1,3-oxazolidin-5-yl]piperidin-1-yl]-2,3-dihydro-1H-indene-4-carbonitrile;
6-[5-[1-(4-fluoro-2,3-dihydro-1H-inden-2-yl)piperidin-4-yl]-2-oxo-1,3-oxazolidin-3-yl]-4H-pyrazino[2,3-b][1,4]oxazin-3-one;
6-[5-[2-[[4-Fluoro-6-(2-hydroxypropoxy)-2,3-dihydro-1H-inden-2-yl]methylamino]ethyl]-2-oxo-1,3-oxazolidin-3-yl]-4H-pyrazino[2,3-b][1,4]oxazin-3-one;hydrochloride;
6-[5-[2-[(4-fluoro-l,3-dimethyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;formic acid;
6-[5-[2-[[4-fluoro-6-(2-methylsulfonylethoxy)-2,3-dihydro-1H-inden-2-yl]methylamino]ethyl]-2-oxo-1,3-oxazolidin-3-yl]-4H-pyrazino[2,3-b][1,4]oxazin-3-one;formic acid;
6-[5-[2-[[6-(1-acetylazetidin-3-yl)oxy-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;formic acid;
6-[5-[2-[[4-fluoro-6-(1-methylsulfonylazetidin-3-yl)oxy-2,3-dihydro-1H-inden-2-yl]methylamino]ethyl]-2-oxo-1,3-oxazolidin-3-yl]-4H-pyrazino[2,3-b][1,4]oxazin-3-one;hydrochloride;
6-[8-[[6-[(2S)-2-aminopropoxy]-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid;
6-[8-[[6-[(2R)-2-aminopropoxy]-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid;
6-[5-[2-[[6-(azetidin-3-yloxy)-4-fluoro-2,3-dihydro-1H-inden-2-yl]methylamino]ethyl]-2-oxo-1,3-oxazolidin-3-yl]-4H-pyrazino[2,3-b][1,4]oxazin-3-one;hydrochloride;
6-[5-[2-[(rac-1,2-trans-1-amino-4-chloro-2,3-dihydro-1H-inden-2-yl)methylamino]ethyl]-2-oxo-1,3-oxazol-3-yl]-4H-pyrazino[2,3-b][1,4]oxazin-3-one;formic acid;
6-[(SR)-5-[(1R)-2-[(4-fluoro-1-methyl-6,7-dihydro-SH-cyclopenta[c]pyridin-6-yl)methylamino]-1-hydroxyethyl]-2-oxo-1,3-oxazolidin-3-yl]-4H-pyrazino[2,3-b][1,4]oxazin-3-one;formic acid;
6-[5-[trans-3-[(4-fluoro-1-methyl-6,7-dihydro-SH-cyclopenta[c]pyridin-6-yl)methylamino]cyclobutyl]-2-oxo-1,3-oxazolidin-3-yl]-4H-pyrazino[2,3-b][1,4]oxazin-3-one;
6-[5-[cis-3-[(4-fluoro-1-methyl-6,7-dihydro-5H-cyclopenta[c]pyridin-6-yl)methylamino]cyclobutyl]-2-oxo-1,3-oxazolidin-3-yl]-4H-pyrazino[2,3-b][1,4]oxazin-3-one;
4-fluoro-3-methyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridine-1-carbonitrile;formicacid;
1-methyl-6-[[2-[(5S)-2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridine-4-carbonitrile;
1-methyl-6-[[2-[(5R)-2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridine-4-carbonitrile;
2-[[7-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetonitrile;formic acid;
6-[5-[3-[(4-fluoro-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one, Enantiomer A;
6-[5-[3-[(4-fluoro-1-methyl-6,7-dihydro-SH-cyclopenta[c]pyridin-6-yl)amino]propyl]-2-oxo-1,3-oxazol-3-yl]-4H-pyrazino[2,3-b][1,4]oxazin-3-one, Enantiomer B;
6-[5-[3-[(1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid, Enantiomer A;
6-[5-[3-[(1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one;formic acid, Enantiomer B;
6-[5-[2-[(1-methyl-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-one;
6-[5-[2-[(4-fluoro-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-one;
6-[8-[(1-chloro-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-one;
6-[8-[(1-methyl-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-one;
6-[5-methyl-2-oxo-5-[2-[(rac-1,2-trans-1-amino-4-chloro-2,3-dihydro-1H-inden-2-yl)methylamino]ethyl]-1,3-oxazolidin-3-yl]-4H-pyrazino[2,3-b][1,4]oxazin-3-one;
6-[5-[2-[(4-Fluoro-1-methyl-6,7-dihydro-5H-cyclopenta[c]pyridin-6-yl)methyl-methylamino]ethyl]-2-oxo-1,3-oxazolidin-3-yl]-4H-pyrazino[2,3-b][1,4]oxazin-3-one;
6-[8-[[6-(2-amino-2-methylpropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid, Enantiomer A;
6-[8-[[6-(2-amino-2-methylpropoxy)-4-fluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid, Enantiomer B;
2-[[7-fluoro-2-[[2-[(5S)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer A;
2-[[7-fluoro-2-[[2-[(5S)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer B;
2-[[7-fluoro-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer A;
2-[[7-fluoro-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide, Isomer B;
2-[[7-cyano-4-methyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;
2-[[4-cyano-7-methyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;
6-[5-[2-[(2-chloro-4-methyl-6,7-dihydro-5H-cyclopenta[d]pyrimidin-6-yl)methylamino]ethyl]-2-oxo-1,3-oxazolidin-3-yl]-4H-pyrido[3,2-b][1,4]oxazin-3-one;
6-(1,3-oxazol-4-ylmethoxy)-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
6-[(3-methyl-1,2,4-oxadiazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
6-[(1-methylpyrazol-3-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
6-[(2-methyltetrazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
6-[(1-methylpyrazol-4-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
6-[(1-methyltetrazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
6-[(4-methyl-1,2,5-oxadiazol-3-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile;
2-[[4,7-difluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamide;
2-[[4,7-difluoro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamide;
6-[5-[2-[[4,7-difluoro-5-(1H-pyrazol-3-ylmethoxy)-2,3-dihydro-1H-inden-2-yl]methylamino]ethyl]-2-oxo-1,3-oxazolidin-3-yl]-4H-pyrazino[2,3-b][1,4]oxazin-3-one;
6-[8-[[4,7-difluoro-5-(1H-pyrazol-3-ylmethoxy)-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;
6-[5-[2-[(4-fluoro-3-methoxy-1-methyl-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-one;
6-[5-[2-[(3-chloro-4-fluoro-1-methyl-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-one;
2-[[rac-2,3-trans-3-Amino-7-chloro-2-[[2-[2-oxo-3-(3-oxo-4H-pyrazino[2,3-b][1,4]oxazin-6-yl)-1,3-oxazolidin-5-yl] ethylamino]methyl]-2,3-dihydro-1H-inden-5-yl] oxy]-N-methylacetamide;formic acid ;
N-methyl-2-[[rac-2,3-trans-3-amino-7-chloro-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]acetamide; formic acid;
6-[(5S)-5-[2-[[(1R,2R)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one;
6-[(5S)-5-[2-[[(1S,2S)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one;
6-[(5R)-5-[2-[[(1R,2R)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one;
2-[[2-[2-oxo-3-(3-oxo-4H-pyrazino[2,3-b][1,4]oxazin-6-yl)-1,3-oxazolidin-5-yl]ethylamino]methyl]-6-(1H-pyrazol-3-ylmethoxy)-2,3-dihydro-1H-indene-4-carbonitrile; formic acid;
2-[[2-[2-oxo-3-(3-oxo-4H-pyrazino[2,3-b][1,4]oxazin-6-yl)-1,3-oxazolidin-5-yl]ethylamino]methyl]-6-(1H-pyrazol-4-ylmethoxy)-2,3-dihydro-1H-indene-4-carbonitrile; formic acid;
6-(1H-imidazol-5-ylmethoxy)-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-indene-4-carbonitrile; formic acid;
6-(2-amino-2-methylpropoxy)-2-[[2-oxo-3-(3-oxo-4H-pyrazino[2,3-b][1,4]oxazin-6-yl)-l-oxa-3,8-diazaspiro[4.5]decan-8-yl]methyl]-2,3-dihydro-1H-indene-4-carbonitrile;
6-[5-[2-[[5-[(1-aminocyclopropyl)methoxy]-4,7-difluoro-2,3-dihydro-1H-inden-2-yl]methylamino]ethyl]-2-oxo-1,3-oxazolidin-3-yl]-4H-pyrazino[2,3-b1,4]oxazin-3-one;formic acid;
6-[5-[2-[[5-[(1-aminocyclopropyl)methoxy]-4,7-difluoro-2,3-dihydro-1H-inden-2-yl]methylamino]ethyl]-2-oxo-1,3-oxazolidin-3-yl]-4H-pyrido[3,2-b][1,4]oxazin-3-one;formic acid;
6-[8-[[5-[(1-aminocyclopropyl)methoxy]-4,7-difluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid;
6-[5-[2-[[5-(2-amino-2-methylpropoxy)-4,7-difluoro-2,3-dihydro-1H-inden-2-yl]methylamino]ethyl]-2-oxo-1,3-oxazolidin-3-yl]-4H-pyrazino[2,3-b1,4]oxazin-3-one;formic acid;
6-[8-[[5-(2-amino-2-methylpropoxy)-4,7-difluoro-2,3-dihydro-1H-inden-2-yl]methyl]-2-oxo-1-oxa-3,8-diazaspiro[4.5]decan-3-yl]-4H-pyrazino[2,3-b][1,4]oxazin-3-one;formic acid;
2-[[4-fluoro-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide;
2-[[7-cyano-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamide ;
6-[5-[2-[[1-methyl-3-(1H-pyrazol-3-ylmethoxy)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one;
6-[5-[2-[(5,8-dimethyl-5,7-diazatricyclo[7.3.0.02,6]dodeca-1,6,8-trien-11-yl)methylamino]ethyl]-2-oxo-1,3-oxazolidin-3-yl]-4H-pyrido[3,2-b][1,4]oxazin-3-one;
6-[4-[2-[(4-fluoro-1-methyl-6,7-dihydro-5H-cyclopenta[c]pyridin-6-yl)methylamino]ethyl]-2-oxopyrrolidin-1-yl]-4H-pyrido[3,2-b][1,4]oxazin-3-one;
6-[5-[2-(6,7-dihydro-5H-cyclopenta[c]pyridin-6-ylmethylamino)ethyl]-2-oxo-1,3-oxazolidin-3-yl]-4H-pyrido[3,2-b][1,4]oxazin-3-one;
6-[5-[2-[[3-(dimethylamino)-1-methyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[[1-methyl-3-(methylamino)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-one;
6-[5-[2-[(4-methyl-6,7-dihydro-5H-cyclopenta[d]pyrimidin-6-yl)methylamino]ethyl]-2-oxo-1,3-oxazolidin-3-yl]-4H-pyrido[3,2-b][1,4]oxazin-3-one;
6-[(5R)-5-[2-[[(1S,2S)-1-amino-4-chloro-2,3-dihydro-1H-inden-2-yl]methylamino]ethyl]-2-oxo-1,3-oxazolidin-3-yl]-4H-pyrazino[2,3-b][1,4]oxazin-3-one;
6-[5-[2-[(8-methyl-5,7-diazatricyclo[7.3.0.02,6]dodeca-1,6,8-trien-11-yl)methylamino]ethyl]-2-oxo-1,3-oxazolidin-3-yl]-4H-pyrido[3,2-b][1,4]oxazin-3-one;
6-[5-[2-[(5,8-dimethyl-5,7-diazatricyclo[7.3.0.02,6]dodeca-1,6,8-trien-11-yl)methylamino]ethyl]-2-oxo-1,3-oxazolidin-3-yl]-4H-pyrazino[2,3-b][1,4]oxazin-3-one;
2-[[2-[2-oxo-3-(3-oxo-4H-pyrazino[2,3-b][1,4]oxazin-6-yl)-1,3-oxazolidin-5-yl]ethylamino]methyl]-6-(1H-triazol-5-ylmethoxy)-2,3-dihydro-1H-indene-4-carbonitrile;
6-[5-[2-[[4-fluoro-1-(1H-pyrazol-5-yl)-6,7-dihydro-SH-cyclopenta[c]pyridin-6-yl]methylamino]ethyl]-2-oxo-1,3-oxazolidin-3-yl]-4H-pyrazino[2,3-b][1,4]oxazin-3-one;
6-[(SR)-5-[2-[[(6R)-4-fluoro-1-methyl-6,7-dihydro-SH-cyclopenta[c]pyridin-6-yl]methylamino]ethyl]-2-oxo-1,3-oxazolidin-3-yl]-4H-pyrazino[2,3-b][1,4]oxazin-3-one
6-[(5S)-5-[2-[[(6R)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one;
6-[(5S)-5-[2-[[(6S)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one;
6-[(5R)-5-[2-[[(6S)-4-fluoro-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-one;
6-[5-[2-[(1-chloro-4-fluoro-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-one;formic acid;
6-[5-[2-[[4-fluoro-1-(1H-pyrazol-4-yl)-6,7-dihydro-SH-cyclopenta[c]pyridin-6-yl]methylamino]ethyl]-2-oxo-1,3-oxazolidin-3-yl]-4H-pyrazino[2,3-b][1,4]oxazin-3-one;
6-[5-[2-[[4-fluoro-1-(1H-imidazol-5-yl)-6,7-dihydro-SH-cyclopenta[c]pyridin-6-yl]methylamino]ethyl]-2-oxo-1,3-oxazolidin-3-yl]-4H-pyrazino[2,3-b][1,4]oxazin-3-one
and pharmaceutically acceptable salts thereof.

10. A compound according to claim 1, selected from
6-[5-[2-[(1-chloro-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-one;
6-[5-[2-[(4-fluoro-1-methyl-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-one;
2-[[7-fluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide; formic acid;
6-[5-[2-[(1-methyl-6,7-dihydro-5*H*-cyclopenta[c]pyridin-6-yl)methylamino]ethyl]-2-oxo-1,3-oxazolidin-3-yl]-4*H*-pyrido[3,2-b][1,4]oxazin-3-one;
6-[2-oxo-5-[2-[[*rac*-1,2-*trans*-1-amino-4-chloro-2,3-dihydro-1*H*-inden-2-yl]methylamino]ethyl]-1,3-oxazolidin-3-yl]-4*H*-pyrazino[2,3-b][1,4]oxazin-3-one; formic acid;
6-[5-[2-[[6-[(1-aminocyclopropyl)methoxy]-4-fluoro-2,3-dihydro-1*H*-inden-2-yl]methylamino]ethyl]-2-oxo-1,3-oxazolidin-3-yl]-4*H*-pyrazino[2,3-b][1,4]oxazin-3-one;
6-[(1-aminocyclopropyl)methoxy]-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile;
6-(2-aminopropoxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-indene-4-carbonitrile; 2,2,2-trifluoroacetic acid ;
6-[2-oxo-5-[2-[[*rac*-1,2-*trans*-1-amino-4-chloro-2,3-dihydro-1*H*-inden-2-yl]methylamino]ethyl]-1,3-oxazolidin-3-yl]-4*H*-pyrido[3,2-b][1,4]oxazin-3-one;
6-[5-[2-[(1-methyl-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-one;formic acid;
6-[5-[2-[(4-fluoro-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-one;
1-methyl-6-[[2-[2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridine-4-carbonitrile;2,2,2-trifluoroacetic acid;
6-[5-[2-[(4-fluoro-1-methyl-6,7-dihydro-5*H*-cyclopenta[c]pyridin-6-yl)methylamino]ethyl]-2-oxo-1,3-oxazol-3-yl]-4*H*-pyrazino[2,3-b][1,4]oxazin-3-one;
6-[5-[3-[(4-fluoro-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one;formic acid;
2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide;
2-[[7-fluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide;formic acid;
2-[[4,7-difluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide;
6-[8-[[6-[(2*S*)-2-aminopropoxy]-4-fluoro-2,3-dihydro-1*H*-inden-2-yl]methyl]-2-oxo-1-oxa-3,8-diazaspiro[4.5]decan-3-yl]-4*H*-pyrazino[2,3-b][1,4]oxazin-3-one;formicacid;
1-methyl-6-[[2-[(5*S*)-2-oxo-3-(3-oxo-4H-pyrazino[2,3-b][1,4]oxazin-6-yl)-1,3-oxazolidin-5-yl]ethylamino]methyl]-6,7-dihydro-5*H*-cyclopenta[c]pyridine-4-carbonitrile;
1-methyl-6-[[2-[(5*R*)-2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridine-4-carbonitrile;
6-[5-[3-[(4-fluoro-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one, Enantiomer A;
6-[5-[3-[(4-fluoro-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one, Enantiomer B;
6-[5-[2-[(1-methyl-6,7-dihydro-5*H*-cyclopenta[c]pyridin-6-yl)methylamino]ethyl]-2-oxo-1,3-oxazol-3-yl]-4*H*-pyrazino[2,3-b][1,4]oxazin-3-one;
2-[[7-fluoro-2-[[2-[(5*S*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide, Isomer A;
2-[[7-fluoro-2-[[2-[(5*S*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide, Isomer B;
2-[[7-fluoro-2-[[2-[(5*R*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide, Isomer A;
2-[[7-fluoro-2-[[2-[(5*R*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamide, Isomer B;
2-[[4,7-difluoro-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N*-methylacetamide;
2-[[4,7-difluoro-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamide;
6-[(5*R*)-5-[2-[[(1*R*,2*R*)-1-amino-4-chloro-2,3-dihydro-1*H*-inden-2-yl]methylamino]ethyl]-2-oxo-1,3-oxazolidin-3-yl]-4*H*-pyrazino[2,3-b][1,4]oxazin-3-one;
6-[(5*R*)-5-[2-[[(1*S*,2*S*)-1-amino-4-chloro-2,3-dihydro-1*H*-inden-2-yl]methylamino]ethyl]-2-oxo-1,3-oxazolidin-3-yl]-4*H*-pyrazino[2,3-b][1,4]oxazin-3-one;
6-[(5*R*)-5-[2-[[(6*R*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[(5*S*)-5-[2-[[(6*R*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[(5*S*)-5-[2-[[(6*S*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[(5*R*)-5-[2-[[(6*S*)-4-fluoro-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[5-[2-[[4-fluoro-1-(1*H*-pyrazol-4-yl)-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
6-[5-[2-[[4-fluoro-1-(1*H*-imidazol-5-yl)-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-one;
or pharmaceutically acceptable salts thereof.

11. A Compound according to any one of claims 1 to 9 for use as therapeutically active substance.

12. A pharmaceutical composition comprising a compound according to any one of claim 1 to 10 and a therapeutically inert carrier.

13. A compound according to any of claims 1 to 10 or a pharmaceutically acceptable salt thereof for use as antibiotic.

14. A compound according to any one of claims 1 to 10 for use in the treatment or prophylaxis of prophylaxis of bacterial infection.

15. A compound according to any one of claims 1 to 10 for use in the treatment or prophylaxis of prophylaxis of infections and resulting diseases caused by *Escherichia coli.*

## Patentansprüche

1. Verbindung, dargestellt durch die folgende allgemeine Formel (I) 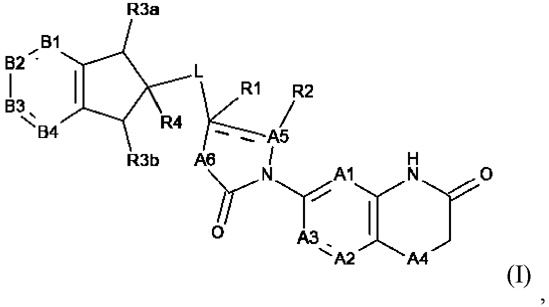 wobei
A¹ aus
i) -N- und
ii) -CHausgewählt ist;
A² aus
i) -N- und
ii) -CHausgewählt ist;
A³ aus
i) -N- und
ii) -CHausgewählt ist;
A⁴ aus
i) -O- und
ii) -S-
ausgewählt ist;
A⁵ aus
i) -N- und
ii) -CH-
ausgewählt ist;
A⁶ aus
i) -O- und
ii) -CH₂-
ausgewählt ist;
-̅ -̅ eine Einzel- oder Doppelbindung darstellt;
B¹ aus
i) -N- und
ii) -CR⁵-
ausgewählt ist;
B² aus
i) -N- und
ii) -CR⁶-
ausgewählt ist;
wobei, wenn B¹ CR⁵ ist und B² CR⁶ ist, R⁵ und R⁶ unter Bildung eines 4- bis 6-gliedrigen Ringes, der gegebenenfalls mit R^{a} und/oder R^{b} substituiert ist, aneinandergebunden sein können;
R^{a} und R^{b} unabhängig voneinander aus
i) COOR^{a1} und
ii) C₁₋₆-Alkyl
ausgewählt sind;
R^{a1} aus
i) H und
ii) C₁₋₆-Alkyl
ausgewählt ist;
B³ aus
i) -N- und
ii) -CR⁷-
ausgewählt ist;
B⁴ aus
i) -N- und
ii) -CR⁸-
ausgewählt ist;
R¹ aus
i) H,
ii) NH₂,
iii) OH und
iv) C₁₋₆-Alkyl, das gegebenenfalls mit R^{c} substituiert ist, ausgewählt ist;
oder R¹, R⁹ und L₂ unter Bildung eines 4- bis 6-gliedrigen Ringes, der gegebenenfalls mit R^{d} substituiert ist, aneinandergebunden sein können;
oder R¹, L₂ und R^{w} unter Bildung eines 4- bis 6-gliedrigen Ringes, der gegebenenfalls mit R^{d} substituiert ist, aneinandergebunden sein können;
R^{c} aus
i) OH und
ii) NH₂
ausgewählt ist;
R^{d} aus
i) (CH2)nNH₂ und
ii) (CH2)nOH
ausgewählt ist;
n eine ganze Zahl von 0 bis 6 darstellt;
R² aus
i) Hund
ii) C₁₋₆-Alkyl, das gegebenenfalls mit R^{c} substituiert ist,
ausgewählt ist,
oder R², L₂ und R^{w} unter Bildung eines 4- bis 6-gliedrigen Ringes, der gegebenenfalls mit R^{d} substituiert ist, aneinandergebunden sein können;
wobei,
wenn - - eine Doppelbindung darstellt, R¹ und R² nicht vorhanden sind;
R^{3a} aus
i) H,
ii) NH₂ und
iii) OH
ausgewählt ist;
R^{3b} aus
i) H,
ii) NH₂ und
iii) OH
ausgewählt ist;
wobei einer von R^{3a} und R^{3b} H ist;
R⁴ aus
i) H,
ii) OH und
iii) NH₂
ausgewählt ist;
R⁵ aus
i) H,
ii) Halogen,
iii) Cyano,
iv) C₁₋₆-Alkyl
v) 4- bis 6-gliedrigem Heteroaryl, das gegebenenfalls mit C₁₋₆-Alkyl substituiert ist,
vi) OR^{e},
vii) SO₂R^{f},
viii) SO₂NR^{e}R^{f} und
ix) NR^{e}R^{f}
ausgewählt ist;
R^{e} und R^{f} unabhängig voneinander aus
i) H und
ii) C₁₋₆-Alkyl
ausgewählt sind;
R⁶ aus
i) H,
ii) Halogen,
iii) C₁₋₆-Alkyl,
iv) OR^{g},
v) NR^{h}Rⁱ und
vi) Cyano
ausgewählt ist;
R^{g} aus
i) H,
ii) -(CH₂)ₘ-R^{g1},
iii) 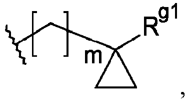
iv) 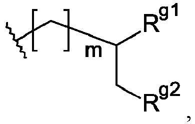
v) 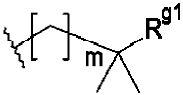 und
vi) 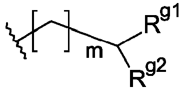
ausgewählt ist;
m eine ganze Zahl von 0 bis 6 darstellt;
R^{g1} und R^{g2} unabhängig voneinander aus
i) H,
ii) OR^{j},
iii) NR^{k}R^{l},
iv) SO₂R^{m},
v) CONRⁿR^{o},
vi) NR^{p}COR^{q},
vii) NR^{r}SO₂R^{s},
viii) COOR^{t},
ix) C₁₋₆-Alkyl, das gegebenenfalls mit C₁₋₆-Alkoxy substituiert ist
x) Heterocycloalkyl, das gegebenenfalls mit R^{u} substituiert ist
xi) Cyano und
xii) Heteroaryl
ausgewählt sind;
R^{j}, R^{k}, R^{l}, R^{m}, Rⁿ, R^{o}, R^{p}, R^{q}, R^{r}, R^{s}, R^{t} und R^{u} unabhängig voneinander aus
i) H,
ii) C₁₋₆-Alkyl, das gegebenenfalls mit C₁₋₆-Alkoxy substituiert ist
iii) C₃₋₈-Cycloalkyl,
iv) NH₂,
v) Heterocycloalkyl,
vi) -S(O)₂-C₁₋₆-Alkyl,
vii) -C(O)-C₁₋₆-Alkyl und
viii) -O-C₁₋₆-Alkyl
ausgewählt sind;
R^{h} und Rⁱ unabhängig voneinander aus
i) H und
ii) C₁₋₆-Alkyl
ausgewählt sind;
R⁷ aus
i) H,
ii) Halogen,
iii) C₁₋₆-Alkyl und
vi) Cyano
ausgewählt ist;
R⁸ aus
i) H,
ii) Halogen,
iii) Cyano
iv) C₁₋₆-Alkyl und
v) Heteroaryl, das gegebenenfalls mit C₁₋₆-Alkyl substituiert ist,
ausgewählt ist;
L aus
i) -L₁-NR⁹-L₂- und
ii) -L₁-Heterocycloalkylen-L₂-
ausgewählt ist;
L₁ aus
i) (CH₂)x, das gegebenenfalls mit R^{v} substituiert ist,
ii) Heterocycloalkylen, das gegebenenfalls mit R^{v} substituiert ist, und
iii) C₃₋₈-Cycloalkylen, das gegebenenfalls mit R^{v} substituiert ist,
ausgewählt ist;
x eine ganze Zahl von 0 bis 6 darstellt;
R⁹ und R¹⁰ unabhängig voneinander aus
i) H und
ii) C₁₋₆-Alkyl, das gegebenenfalls mit R^{v} substituiert ist,
ausgewählt sind,
oder R¹, R⁹ und L₂ unter Bildung eines 4- bis 6-gliedrigen Ringes, der gegebenenfalls mit R^{d} substituiert ist, aneinandergebunden sein können;
R^{v} aus
i) OH,
ii) NH₂ und
iii) C₁₋₆-Alkyl, das gegebenenfalls mit Hydroxy substituiert ist,
ausgewählt ist;
L₂ aus
i) (CH₂)y, das gegebenenfalls mit R^{w} und/oder R^{x} substituiert ist,
ii) C₃₋₈-Cycloalkylen, das gegebenenfalls mit R^{w} und/oder R^{x} substituiert ist,
iii) Heterocycloalkylen, das gegebenenfalls mit R^{w} und/oder R^{x} substituiert ist,
iv) (CH₂)yNR¹⁰-, das gegebenenfalls mit R^{w} und/oder R^{x} substituiert ist,
v) C₃₋₈-Cycloalkylen-NR¹⁰-, das gegebenenfalls mit R^{w} und/oder R^{x} substituiert ist, und
vi) Heterocycloalkylen-NR¹⁰-, das gegebenenfalls mit R^{w} und/oder R^{x} substituiert ist,
ausgewählt ist;
oder R¹, R⁹ und L₂ unter Bildung eines 4- bis 6-gliedrigen Ringes, der gegebenenfalls mit R^{d} substituiert ist, aneinandergebunden sein können;
oder R¹, L₂ und R^{w} unter Bildung eines 4- bis 6-gliedrigen Ringes, der gegebenenfalls mit R^{d} substituiert ist, aneinandergebunden sein können;
oder R², L₂ und R^{w} unter Bildung eines 4- bis 6-gliedrigen Ringes, der gegebenenfalls mit R^{d} substituiert ist, aneinandergebunden sein können;
y eine ganze Zahl von 0 bis 6 darstellt;
R^{w} und R^{x} unabhängig voneinander aus
i) OH,
ii) NH₂ und
iii) C₁₋₆-Alkyl, das gegebenenfalls mit Hydroxy substituiert ist,
ausgewählt sind;
oder R¹, L₂ und R^{w} unter Bildung eines 4- bis 6-gliedrigen Ringes, der gegebenenfalls mit R^{d} substituiert ist, aneinandergebunden sein können;
oder R², L₂ und R^{w} unter Bildung eines 4- bis 6-gliedrigen Ringes, der gegebenenfalls mit R^{d} substituiert ist, aneinandergebunden sein können; oder pharmazeutisch unbedenkliche Salze davon.

2. Verbindung oder ein pharmazeutisch unbedenkliches Salz davon nach Anspruch 1, wobei
A¹ aus
i) -N- und
ii) -CH-
ausgewählt ist;
A² aus
i) -N- und
ii) -CH-
ausgewählt ist;
A³ CH ist;
A⁴ aus
i) -O- und
ii) -S-
ausgewählt ist;
A⁵ aus
i) -N- und
ii) -CH-
ausgewählt ist;
A⁶ aus
i) -O- und
ii) -CH₂-
ausgewählt ist;
-̅ -̅ eine Einzel- oder Doppelbindung darstellt;
B¹ aus
i) -N- und
ii) -CR⁵-
ausgewählt ist;
B² aus
i) -N- und
ii) -CR⁶-
ausgewählt ist;
wobei, wenn B¹ CR⁵ ist und B² CR⁶ ist, R⁵ und R⁶ unter Bildung eines 4- bis 6-gliedrigen Ringes, wie nachstehend, aneinandergebunden sein können, 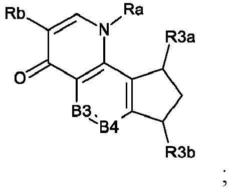
R^{a} und R^{b} unabhängig voneinander aus
i) COOR^{a1} und
ii) C₁₋₆-Alkyl
ausgewählt sind;
R^{a1} aus
i) H und
ii) C₁₋₆-Alkyl
ausgewählt ist;
B³ aus
i) -N- und
ii) -CR⁷-
ausgewählt ist;
B⁴ aus
i) -N- und
ii) -CR⁸-
ausgewählt ist;
R¹ aus
i) Hund
ii) C₁₋₆-Alkyl, das gegebenenfalls mit R^{c} substituiert ist,
ausgewählt ist;
oder R¹, R⁹ und L₂ unter Bildung eines 4- bis 6-gliedrigen Ringes, der gegebenenfalls mit R^{d} substituiert ist, aneinandergebunden sein können;
oder R¹, L₂ und R^{w} unter Bildung eines 4- bis 6-gliedrigen Ringes, der gegebenenfalls mit R^{d} substituiert ist, aneinandergebunden sein können;
R^{c} NH₂ ist;
R^{d} (CH₂)ₙNH₂ ist;
n eine ganze Zahl von 0 bis 3 darstellt;
R² H ist;
oder R², L₂ und R^{w} unter Bildung eines 4- bis 6-gliedrigen Ringes, der gegebenenfalls mit R^{d} substituiert ist, aneinandergebunden sein können;
wobei,
wenn -̅ -̅ eine Doppelbindung darstellt, R¹ und R² nicht vorhanden sind;
R^{3a} aus
i) H,
ii) NH₂ und
iii) OH
ausgewählt ist;
R^{3b} aus
i) H,
ii) NH₂ und
iii) OH
ausgewählt ist;
wobei einer von R^{3a} und R^{3b} H ist;
R⁴ aus
i) Hund
ii) NH₂
ausgewählt ist;
R⁵ aus
i) H,
ii) Halogen,
iii) Cyano,
iv) C₁₋₆-Alkyl und
v) OR^{e}
ausgewählt ist;
R^{e} C₁₋₆-Alkyl ist;
R⁶ aus
i) H,
ii) C₁₋₆-Alkyl und
iii) OR⁹
ausgewählt ist;
R^{g} aus
i) H,
ii) -(CH₂)ₘ-R^{g1},
iii) 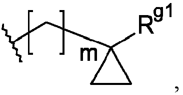
iv) 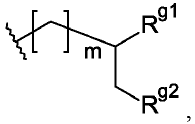
v) 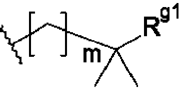 und
iv) 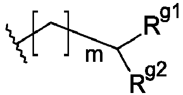
ausgewählt ist;
m eine ganze Zahl von 0 bis 3 darstellt;
R^{g1} und R^{g2} unabhängig voneinander aus
i) H,
ii) OR^{j},
iii) NR^{k}R^{l},
iv) SO₂R^{m},
v) CONRⁿR^{o},
vi) NR^{p}COR^{q},
vii) NR^{r}SO₂R^{s},
viii) COOR^{t},
ix) C₁₋₆-Alkyl, das gegebenenfalls mit C₁₋₆-Alkoxy substituiert ist,
x) Heterocycloalkyl, das gegebenenfalls mit R^{u} substituiert ist, und
xi) Cyano
ausgewählt sind;
R^{j}, R^{k}, R^{l}, R^{m}, Rⁿ, R^{o}, R^{p}, R^{q}, R^{r}, R^{s}, R^{t} und R^{u} unabhängig voneinander aus
i) H,
ii) C₁₋₆-Alkyl, das gegebenenfalls mit C₁₋₆-Alkoxy substituiert ist, und
iii) NH₂
ausgewählt sind;
R⁷ aus
i) Hund
ii) C₁₋₆-Alkyl
ausgewählt ist;
R⁸ aus
i) H,
ii) Halogen,
iii) Cyano,
iv) C₁₋₆-Alkyl und
v) Heteroaryl, das gegebenenfalls mit C₁₋₆-Alkyl substituiert ist,
ausgewählt ist;
L aus
i) -L₁-NR⁹-L₂- und
ii) -L₁-Azetidinylen-L₂-
ausgewählt ist;
L₁ aus
i) (CH₂)ₓ, das gegebenenfalls mit R^{v} substituiert ist, und
ii) Heterocycloalkylen, das gegebenenfalls mit R^{v} substituiert ist,
ausgewählt ist;
x eine ganze Zahl von 0 bis 3 darstellt;
R⁹ und R¹⁰ H sind;
oder R¹, R⁹ und L₂ unter Bildung eines 4- bis 6-gliedrigen Ringes, der gegebenenfalls mit R^{d} substituiert ist, aneinandergebunden sein können;
R^{v} NH₂ ist;
Lz aus
i) (CH₂)_{y}, das gegebenenfalls mit R^{w} und/oder R^{x} substituiert ist,
ii) C₃₋₈-Cycloalkylen, das gegebenenfalls mit R^{w} und/oder R^{x} substituiert ist,
iii) Heterocycloalkylen, das gegebenenfalls mit R^{w} und/oder R^{x} substituiert ist,
iv) (CH₂)yNR¹⁰-, das gegebenenfalls mit R^{w} und/oder R^{x} substituiert ist,
v) C₃₋₈-Cycloalkylen-NR¹⁰-, das gegebenenfalls mit R^{w} und/oder R^{x} substituiert ist, und
vi) Heterocycloalkylen-NR¹⁰-, das gegebenenfalls mit R^{w} und/oder R^{x} substituiert ist,
ausgewählt ist;
oder R¹, R⁹ und L₂ unter Bildung eines 4- bis 6-gliedrigen Ringes, der gegebenenfalls mit R^{d} substituiert ist, aneinandergebunden sein können;
oder R¹, L₂ und R^{w} unter Bildung eines 4- bis 6-gliedrigen Ringes, der gegebenenfalls mit R^{d} substituiert ist, aneinandergebunden sein können;
oder R², L₂ und R^{w} unter Bildung eines 4- bis 6-gliedrigen Ringes, der gegebenenfalls mit R^{d} substituiert ist, aneinandergebunden sein können;
y eine ganze Zahl von 0 bis 3 darstellt;
R^{w} und R^{x} OH sind;
oder R¹, L₂ und R^{w} unter Bildung eines 4- bis 6-gliedrigen Ringes, der gegebenenfalls mit R^{d} substituiert ist, aneinandergebunden sein können;
oder R², L₂ und R^{w} unter Bildung eines 4- bis 6-gliedrigen Ringes, der gegebenenfalls mit R^{d} substituiert ist, aneinandergebunden sein können:

3. Verbindung oder ein pharmazeutisch unbedenkliches Salz davon nach Anspruch 1, wobei
A¹ aus
i) -N- und
ii) -CH-
ausgewählt ist;
A² aus
i) -N- und
ii) -CH-
ausgewählt ist;
A³ CH ist;
A⁴ aus
i) -O- und
ii) -S-
ausgewählt ist;
A⁵ CH ist;
A⁶ O ist;
-̅ -̅ eine Einzelbindung ist;
B¹ aus
i) -N- und
ii) -CR⁵-
ausgewählt ist;
B² aus
i) -N- und
ii) -CR⁶-
ausgewählt ist;
B³ aus
i) -N- und
ii) -CR⁷-
ausgewählt ist;
B⁴ aus
i) -N- und
ii) -CR⁸-
ausgewählt ist;
R¹ H ist;
oder R¹, R⁹ und L₂ unter Bildung eines 4- bis 6-gliedrigen Ringes, der gegebenenfalls mit R^{d} substituiert ist, aneinandergebunden sein können;
oder R¹, L₂ und R^{w} unter Bildung eines 4- bis 6-gliedrigen Ringes, der gegebenenfalls mit R^{d} substituiert ist, aneinandergebunden sein können;
R^{d} (CH₂)ₙNH₂ ist und
n eine ganze Zahl von 0 bis 3 darstellt;
R² H ist;
oder R², L₂ und R^{w} unter Bildung eines 4- bis 6-gliedrigen Ringes, der gegebenenfalls mit R^{d} substituiert ist, aneinandergebunden sein können;
R^{3a} aus
i) H,
ii) NH₂ und
iii) OH
ausgewählt ist;
R^{3b} aus
i) H,
ii) NH₂ und
iii) OH
ausgewählt ist;
wobei einer von R^{3a} und R^{3b} H ist;
R⁴ H ist;
R⁵ aus
i) H,
ii) Halogen,
iii) Cyano,
iv) C₁₋₆-Alkyl und
v) OR^{e}
ausgewählt ist;
R^{e} C₁₋₆-Alkyl ist;
R⁶ aus
i) H,
ii) C₁₋₆-Alkyl und
iii) OR⁹
ausgewählt ist;
R^{g} aus
i) H,
ii) -(CH₂)ₘ-R^{g1},
iii) 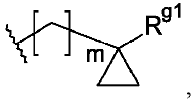
iv) 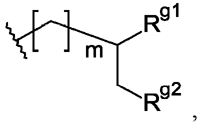
v) 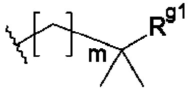 und
vi) 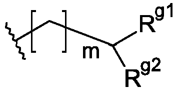
ausgewählt ist;
m eine ganze Zahl von 0 bis 3 darstellt;
R^{g1} und R^{g2} unabhängig voneinander aus
i) H,
ii) OR^{j},
iii) NR^{k}R^{l},
iv) C₁₋₆-Alkyl, das gegebenenfalls mit C₁₋₆-Alkoxy substituiert ist, und
v) Heterocycloalkyl, das gegebenenfalls mit R^{u} substituiert ist,
ausgewählt sind;
R^{j}, R^{k}, R^{l} und R^{u} unabhängig voneinander aus
i) H,
ii) C₁₋₆-Alkyl, das gegebenenfalls mit C₁₋₆-Alkoxy substituiert ist, und
iii) NH₂
ausgewählt sind;
R⁷ aus
i) Hund
ii) C₁₋₆-Alkyl
ausgewählt ist;
R⁸ aus
i) H,
ii) Halogen,
iii) Cyano,
iv) C₁₋₆-Alkyl und
v) Heteroaryl, das gegebenenfalls mit C₁₋₆-Alkyl substituiert ist,
ausgewählt ist;
L -L₁-NR⁹-L₂- ist;
L₁ (CH₂)ₓ, das gegebenenfalls mit R^{v} substituiert ist, ist;
x eine ganze Zahl von 0 bis 3 darstellt;
R⁹ und R¹⁰ H sind;
oder R¹, R⁹ und L₂ unter Bildung eines 4- bis 6-gliedrigen Ringes, der gegebenenfalls mit R^{d} substituiert ist, aneinandergebunden sein können;
R^{v} NH₂ ist;
L₂ aus
i) (CH₂)_{y}, das gegebenenfalls mit R^{w} und/oder R^{x} substituiert ist,
ii) C₃₋₈-Cycloalkylen, das gegebenenfalls mit R^{w} und/oder R^{x} substituiert ist,
iii) Heterocycloalkylen, das gegebenenfalls mit R^{w} und/oder R^{x} substituiert ist,
iv) (CH₂)yNR¹⁰-, das gegebenenfalls mit R^{w} und/oder R^{x} substituiert ist,
v) C₃₋₈-Cycloalkylen-NR¹⁰-, das gegebenenfalls mit R^{w} und/oder R^{x} substituiert ist, und
vi) Heterocycloalkylen-NR¹⁰-, das gegebenenfalls mit R^{w} und/oder R^{x} substituiert ist,
ausgewählt ist;
oder R¹, R⁹ und L₂ unter Bildung eines 4- bis 6-gliedrigen Ringes, der gegebenenfalls mit R^{d} substituiert ist, aneinandergebunden sein können;
oder R¹, L₂ und R^{w} unter Bildung eines 4- bis 6-gliedrigen Ringes, der gegebenenfalls mit R^{d} substituiert ist, aneinandergebunden sein können;
oder R², L₂ und R^{w} unter Bildung eines 4- bis 6-gliedrigen Ringes, der gegebenenfalls mit R^{d} substituiert ist, aneinandergebunden sein können;
y eine ganze Zahl von 0 bis 3 darstellt;
R^{w} und R^{x} OH sind,
oder R¹, L₂ und R^{w} unter Bildung eines 4- bis 6-gliedrigen Ringes, der gegebenenfalls mit R^{d} substituiert ist, aneinandergebunden sein können;
oder R², L₂ und R^{w} unter Bildung eines 4- bis 6-gliedrigen Ringes, der gegebenenfalls mit R^{d} substituiert ist, aneinandergebunden sein können:

4. Verbindung oder ein pharmazeutisch unbedenkliches Salz davon nach Anspruch 1,
wobei
A¹ aus
i) -N- und
ii) -CH-
ausgewählt ist;
A² aus
i) -N- und
ii) -CH-
ausgewählt ist;
A³ CH ist;
A⁴ aus
i) -O- und
ii) -S-
ausgewählt ist;
A⁵ CH ist;
A⁶ O ist;
-̅ -̅ eine Einzelbindung ist;
B¹ aus
i) -N- und
ii) -CR⁵-
ausgewählt ist;
B² aus
i) -N- und
ii) -CR⁶-
ausgewählt ist;
B³ aus
i) -N- und
ii) -CR⁷-
ausgewählt ist;
B⁴ aus
i) -N- und
ii) -CR⁸-
ausgewählt ist;
R¹ H ist;
oder R¹, R⁹ und L₂ unter Bildung eines 4- bis 6-gliedrigen Ringes, wie nachstehend, aneinandergebunden sein können, 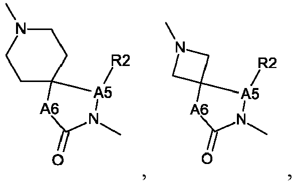
wobei der Ring gegebenenfalls mit R^{d} substituiert ist,
oder R¹, L₂ und R^{w} unter Bildung eines 4- bis 6-gliedrigen Ringes, wie nachstehend, aneinandergebunden sein können, 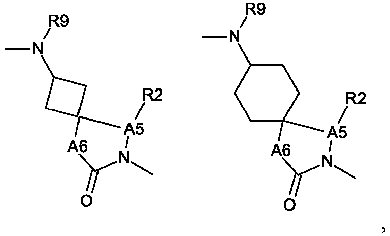
wobei der Ring gegebenenfalls mit R^{d} substituiert ist,
R^{d} (CH₂)ₙNH₂ ist;
n eine ganze Zahl von 0 bis 1 darstellt;
R² H ist;
oder R², L₂ und R^{w} unter Bildung eines 4- bis 6-gliedrigen Ringes, wie nachstehend, aneinandergebunden sein können, 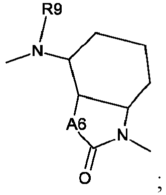
R^{3a} aus
i) H,
ii) NH₂ und
iii) OH
ausgewählt ist;
R^{3b} aus
i) H,
ii) NH₂ und
iii) OH
ausgewählt ist;
wobei einer von R^{3a} und R^{3b} H ist;
R⁴ H ist;
R⁵ aus
i) H,
ii) Halogen,
iii) Cyano,
iv) C₁₋₆-Alkyl und
v) OR^{e}
ausgewählt ist; und
R^{e} C₁₋₆-Alkyl ist;
R⁶ aus
i) H,
ii) C₁₋₆-Alkyl und
iii) OR^{g}
ausgewählt ist;
R^{g} aus
i) H,
ii) -(CH₂)ₘ-R^{g1},
iii) 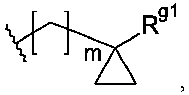
iv) 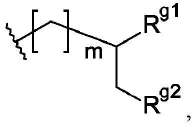
v) 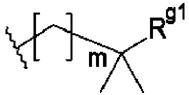 und
vi) 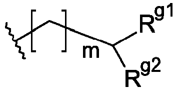
ausgewählt ist;
m eine ganze Zahl von 0 bis 3 darstellt;
R^{g1} und R^{g2} unabhängig voneinander aus
i) H,
ii) OR^{j},
iii) NR^{k}R^{l},
iv) C₁₋₆-Alkyl, das gegebenenfalls mit C₁₋₆-Alkoxy substituiert ist,
v) Pyrrolidinyl, das gegebenenfalls mit R^{u} substituiert ist,
vi) Morpholinyl, das gegebenenfalls mit R^{u} substituiert ist,
vii) Oxetanyl, das gegebenenfalls mit R^{u} substituiert ist,
viii) 3-Oxo-morpholinyl, das gegebenenfalls mit R^{u} substituiert ist,
ix) 2-Oxo-pyrrolidinyl, das gegebenenfalls mit R^{u} substituiert ist,
x) Azetidinyl, das gegebenenfalls mit R^{u} substituiert ist,
xi) Piperazinyl, das gegebenenfalls mit R^{u} substituiert ist, und
xii) 2-Oxo-piperazinyl, das gegebenenfalls mit R^{u} substituiert ist,
ausgewählt sind;
R^{j}, R^{k}, R^{l} und R^{u} unabhängig voneinander aus
i) H,
ii) C₁₋₆-Alkyl, das gegebenenfalls mit C₁₋₆-Alkoxy substituiert ist, und
iii) NH₂
ausgewählt sind;
R⁷ aus
i) Hund
ii) C₁₋₆-Alkyl
ausgewählt ist;
R⁸ aus
i) H,
ii) Halogen,
iii) Cyano
iv) C₁₋₆-Alkyl und
v) Thiazol, das gegebenenfalls mit C₁₋₆-Alkyl substituiert ist,
ausgewählt ist;
L -L₁-NR⁹-L₂- ist;
L₁ (CH₂)ₓ, das gegebenenfalls mit R^{v} substituiert ist, ist;
x eine ganze Zahl von 0 bis 3 darstellt;
R⁹ und R¹⁰ H sind;
oder R¹, R⁹ und L₂ unter Bildung eines 4- bis 6-gliedrigen Ringes, wie nachstehend, aneinandergebunden sein können, 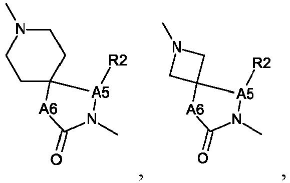
wobei der Ring gegebenenfalls mit R^{d} substituiert ist;
R^{v} NH₂ ist;
Lz aus
i) (CH₂)_{y}, das gegebenenfalls mit R^{w} und/oder R^{x} substituiert ist,
ii) C₃₋₈-Cycloalkylen, das gegebenenfalls mit R^{w} und/oder R^{x} substituiert ist,
iii) (CH₂)yNR¹⁰-, das gegebenenfalls mit R^{w} und/oder R^{x} substituiert ist, und
iv) C₃₋₈-Cycloalkylen-NR¹⁰-, das gegebenenfalls mit R^{w} und/oder R^{x} substituiert ist,
ausgewählt ist;
oder R¹, R⁹ und L₂ unter Bildung eines 4- bis 6-gliedrigen Ringes, wie nachstehend, aneinandergebunden sein können, 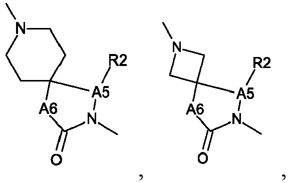
wobei der Ring gegebenenfalls mit R^{d} substituiert ist,
oder R¹, L₂ und R^{w} unter Bildung eines 4- bis 6-gliedrigen Ringes, wie nachstehend, aneinandergebunden sein können, 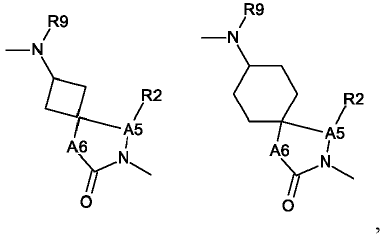
wobei der Ring gegebenenfalls mit R^{d} substituiert ist;
oder R², L₂ und R^{w} unter Bildung eines 4- bis 6-gliedrigen Ringes, wie nachstehend, aneinandergebunden sein können, 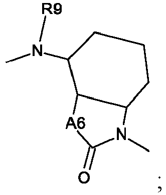
y eine ganze Zahl von 0 bis 3 darstellt;
R^{w} und R^{x} OH sind;
oder R¹, L₂ und R^{w} unter Bildung eines 4- bis 6-gliedrigen Ringes, wie nachstehend, aneinandergebunden sein können, 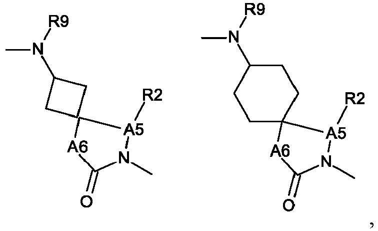
wobei der Ring gegebenenfalls mit R^{d} substituiert ist,
oder R², L₂ und R^{w} unter Bildung eines 4- bis 6-gliedrigen Ringes, wie nachstehend, aneinandergebunden sein können,

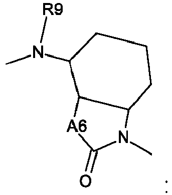

5. Verbindung oder ein pharmazeutisch unbedenkliches Salz davon nach einem von Anspruch 1 bis 4, wobei A¹ -N- ist.

6. Verbindung oder ein pharmazeutisch unbedenkliches Salz davon nach einem von Anspruch 1 bis 5, wobei A² -CH- ist.

7. Verbindung oder ein pharmazeutisch unbedenkliches Salz davon nach einem von Anspruch 1 bis 6, wobei A⁴ -O- ist.

8. Verbindung oder ein pharmazeutisch unbedenkliches Salz davon nach einem von Anspruch 1 bis 7, wobei R^{3a} und R^{3b} H sind.

9. Verbindung nach Anspruch 1, ausgewählt aus
6-[5-[2-[(4-Chlor-2,3-dihydro-1H-inden-2-yl)methylamino]ethyl]-2-oxo-1,3-oxazolidin-3-yl]-4H-pyrido[3,2-b][1,4]oxazin-3-on,
6-[5-[3-[(4-Chlor-2,3-dihydro-1H-inden-2-yl)methylamino]propyl]-2-oxo-1,3-oxazolidin-3-yl]-4H-pyrido[3,2-b][1,4]oxazin-3-on,
6-[5-[2-[(4-Chlor-2,3-dihydro-1H-inden-2-yl)amino]ethyl]-2-oxo-1,3-oxazolidin-3-yl]-4H-pyrido[3,2-b][1,4]oxazin-3-on;
6-[5-[3-[(4-Chlor-2,3-dihydro-1H-inden-2-yl)amino]propyl]-2-oxo-1,3-oxazolidin-3-yl]-4H-pyrido[3,2-b][1,4]oxazin-3-on;
2-[[2-[2-Oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[5-[2-[(4-Chlor-2,3-dihydro-1H-inden-2-yl)methylamino]ethyl]-2-oxo-1,3-oxazolidin-3-yl]-4H-pyrido[3,2-b][1,4]oxazin-3-on,
6-[5-[3-[(4-Fluor-2,3-dihydro-1H-inden-2-yl)amino]propyl]-2-oxo-1,3-oxazolidin-3-yl]-4H-pyrido[3,2-b][1,4]oxazin-3-on;
6-[5-[3-(2,3-Dihydro-1H-inden-2-ylamino)propyl]-2-oxo-1,3-oxazolidin-3-yl]-4H-pyrido[3,2-b][1,4]oxazin-3-on;
6-[5-[3-[(5-Chlor-2,3-dihydro-1H-inden-2-yl)amino]propyl]-2-oxo-1,3-oxazolidin-3-yl]-4H-pyrido[3,2-b][1,4]oxazin-3-on;
6-[5-[2-[(5-Chlor-2,3-dihydro-1H-inden-2-yl)amino]ethyl]-2-oxo-1,3-oxazolidin-3-yl]-4H-pyrido[3,2-b][1,4]oxazin-3-on;
6-[5-[3-[(5-Fluor-2,3-dihydro-1H-inden-2-yl)amino]propyl]-2-oxo-1,3-oxazolidin-3-yl]-4H-pyrido[3,2-b][1,4]oxazin-3-on;
2-[3-[2-Oxo-3-(3-oxo-4H-pyrido[3,2-b][1,4]oxazin-6-yl)-1,3-oxazolidin-5-yl]propylamino]-2,3-dihydro-1H-inden-4-carbonitril;
2-[[trans-3-[2-Oxo-3-(3-oxo-4H-pyrido[3,2-b][1,4]oxazin-6-yl)-1,3-oxazolidin-5-yl]cyclobutyl]amino]-2,3-dihydro-1H-inden-4-carbonitril;
6-[5-[2-[(4-Chlor-2,3-dihydro-1H-inden-2-yl)methylamino]ethyl]-2-oxo-1,3-oxazolidin-3-yl]-4H-1,4-benzoxazin-3-on;
6-[5-[cis-3-[(4-Chlor-2,3-dihydro-1H-inden-2-yl)amino]cyclobutyl]-2-oxo-1,3-oxazolidin-3-yl]-4H-pyrido[3,2-b][1,4]oxazin-3-on;
6-[5-[trans-3-[(4-Chlor-2,3-dihydro-1H-inden-2-yl)amino]cyclobutyl]-2-oxo-1,3-oxazolidin-3-yl]-4H-pyrido[3,2-b][1,4]oxazin-3-on;
2-[[2-[2-Oxo-3-(3-oxo-4H-1,4-benzoxazin-6-yl)-1,3-oxazolidin-5-yl]ethylamino]methyl]-2,3-dihydro-1H-inden-4-carbonitril;
2-[[cis-3-[2-Oxo-3-(3-oxo-4H-pyrido[3,2-b][1,4]oxazin-6-yl)-1,3-oxazolidin-5-yl]cyclobutyl]amino]-2,3-dihydro-1H-inden-4-carbonitril;
6-[5-[2-[2-(4-Chlor-2,3-dihydro-1H-inden-2-yl)ethylamino]ethyl]-2-oxo-1,3-oxazolidin-3-yl]-4H-pyrido[3,2-b][1,4]oxazin-3-on;
6-[5-[[(4-Chlor-2,3-dihydro-1H-inden-2-yl)methylamino]methyl]-2-oxo-1,3-oxazolidin-3-yl]-4H-pyrido[3,2-b][1,4]oxazin-3-on;
6-[5-[[(4-Chlor-2,3-dihydro-1H-inden-2-yl)amino]methyl]-2-oxo-1,3-oxazolidin-3-yl]-4H-pyrido[3,2-b][1,4]oxazin-3-on;
6-[(4R)-4-[[3-[(4-Fluor-2,3-dihydro-1H-inden-2-yl)amino]cyclobutyl]amino]-2-oxopyrrolidin-1-yl]-4H-pyrido[3,2-b][1,4]oxazin-3-on;
6-[(4S)-4-[[3-[(4-Fluor-2,3-dihydro-1H-inden-2-yl)amino]cyclobutyl]amino]-2-oxopyrrolidin-1-yl]-4H-pyrido[3,2-b][1,4]oxazin-3-on;
6-[5-[[(5-Chlor-2,3-dihydro-1H-inden-2-yl)amino]methyl]-2-oxo-1,3-oxazolidin-3-yl]-4H-pyrido[3,2-b][1,4]oxazin-3-on;
6-[5-[[2-(4-Chlor-2,3-dihydro-1H-inden-2-yl)ethylamino]methyl]-2-oxo-1,3-oxazolidin-3-yl]-4H-pyrido[3,2-b][1,4]oxazin-3-on;
6-[5-[3-[(4-Methyl-6,7-dihydro-5H-cyclopenta[b]pyridin-6-yl)amino]propyl]-2-oxo-1,3-oxazolidin-3-yl]-4H-pyrido[3,2-b][1,4]oxazin-3-on;
2-[[2-Oxo-3-(3-oxo-4H-pyrido[3,2-b][1,4]oxazin-6-yl)-1-oxa-3,8-diazaspiro[4.5]decan-8-yl]methyl]-2,3-dihydro-1H-inden-4-carbonitril;
2-[[[2-Methyl-1-[2-oxo-3-(3-oxo-4H-pyrido[3,2-b][1,4]oxazin-6-yl)-1,3-oxazolidin-5-yl]propan-2-yl]amino]methyl]-2,3-dihydro-1H-inden-4-carbonitril;
6-[5-[2-[(1-Chlor-6,7-dihydro-5H-cyclopenta[c]pyridin-6-yl)methylamino]-2-methylpropyl]-2-oxo-1,3-oxazolidin-3-yl]-4H-pyrido[3,2-b][1,4]oxazin-3-on;
2-[[[rac-3a,7a-trans-6,7a-cis-2-Oxo-3-(3-oxo-4H-pyrido[3,2-b][1,4]oxazin-6-yl)-3a,4,5,6,7,7a-hexahydro-1,3-benzoxazol-6-yl]amino]methyl]-2,3-dihydro-1H-inden-4-carbonitril;
6-[rac-3a,7a-trans-6,7a-cis-6-[2-(4-Chlor-2,3-dihydro-1H-inden-2-yl)ethylamino]-2-oxo-3a,4,5,6,7,7a-hexahydro-1,3-benzoxazol-3-yl]-4H-pyrido[3,2-b][1,4]oxazin-3-on;
2-[2-[6-Oxo-7-(3-oxo-4H-pyrido[3,2-b][1,4]oxazin-6-yl)-5-oxa-2,7-diazaspiro[3.4]octan-2-yl]ethyl]-2,3-dihydro-1H-inden-4-carbonitril;
2-[[trans-4-[2-Oxo-3-(3-oxo-4H-pyrido[3,2-b][1,4]oxazin-6-yl)-1,3-oxazolidin-5-yl]cyclohexyl]amino]-2,3-dihydro-1H-inden-4-carbonitril;
2-[[2-[2-Oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
6-[5-[2-[(4-Fluor-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
4-Fluor-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-1-carbonitril;
6-[5-[2-[(1-Chlor-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-on;
2-[[2-Oxo-3-(3-oxo-4H-pyrido[3,2-b][1,4]oxazin-6-yl)-1-oxa-3-azaspiro[4.5]decan-8-yl]amino]-2,3-dihydro-1H-inden-4-carbonitril;
6-[2-Oxo-5-[2-[[rac-(5,6-trans)-1-chlor-5-hydroxy-6,7-dihydro-5H-cyclopenta[c]pyridin-6-yl]methylamino]ethyl]-1,3-oxazolidin-3-yl]-4H-pyrido[3,2-b][1,4]oxazin-3-on;
2-[[2-[5-Oxo-4-(3-oxo-4H-1,4-benzoxazin-6-yl)-1,3,4-oxadiazol-2-yl]ethylamino]methyl]-2,3-dihydro-1H-inden-4-carbonitril;
6-[2-Oxo-5-[2-[[rac-(5,6-trans)-5-hydroxy-1-methyl-6,7-dihydro-SH-cyclopenta[c]pyridin-6-yl]methylamino]ethyl]-1,3-oxazolidin-3-yl]-4H-pyrido[3,2-b][1,4]oxazin-3-on;
2-[[2-[5-Oxo-4-(3-oxo-4H-pyrido[3,2-b][1,4]oxazin-6-yl)-1,3,4-oxadiazol-2-yl]ethylamino]methyl]-2,3-dihydro-1H-inden-4-carbonitril;
7-[5-[2-[(1-Chlor-6,7-dihydro-SH-cyclopenta[c]pyridin-6-yl)methylamino]ethyl]-2-oxo-1,3-oxazolidin-3-yl]-1H-pyrido[2,3-b][1,4]oxazin-2-on; Hydrochlorid;
2-[[2-[2-Oxo-3-(2-oxo-1H-pyrido[2,3-b][1,4]oxazin-7-yl)-1,3-oxazolidin-5-yl]ethylamino]methyl]-2,3-dihydro-1H-inden-4-carbonitril;
1-Methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-4-carbonitril;
2-[[4-Cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamid;
6-[5-[2-[(1-Chlor-3-methoxy-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-on;
6-[5-[2-[(1-Chlor-3-methoxy-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-on;
6-[5-[2-[(4-Fluor-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
5-[(1-Methylpyrrolidin-2-yl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
3-Methoxy-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-1-carbonitril;
5-(2-Morpholin-4-ylethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[5-[2-[(4-Chlor-1-methyl-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-on; Ameisensäure;
N-[2-[[4-Cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]acetamid;
6-[5-[2-[(1-Chlor-4-methoxy-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
6-[5-[2-[(1-Chlor-4-methoxy-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
N-[2-[[4-Cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]methansulfonamid;
6-[5-[2-[(4-Chlor-1-methyl-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-on;
2-[[[(2R)-2-Hydroxy-2-[(5R)-2-oxo-3-(3-oxo-4H-1,4-benzoxazin-6-yl)-1,3-oxazolidin-5-yl]ethyl]amino]methyl]-2,3-dihydro-1H-inden-4-carbonitril;
2-[[[(2S)-2-Hydroxy-2-[(5S)-2-oxo-3-(3-oxo-4H-1,4-benzoxazin-6-yl)-1,3-oxazolidin-5-yl]ethyl]amino]methyl]-2,3-dihydro-1H-inden-4-carbonitril;
5-(Oxetan-3-yloxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
2-[[2-[2-Oxo-3-(3-oxo-4H-pyrido[3,2-b][1,4]thiazin-6-yl)-1,3-oxazolidin-5-yl]ethylamino]methyl]-2,3-dihydro-1H-inden-4-carbonitril;
3-Methoxy-6-[[2-[2-oxo-3-(3-oxo-4H-pyrazino[2,3-b][1,4]oxazin-6-yl)oxazolidin-5-yl]ethylamino]methyl]-6,7-dihydro-5H-cyclopenta[c]pyridin-1-carbonitril;
2-[[1-Chlor-6-[[2-[2-oxo-3-(3-oxo-4H-pyrido[3,2-b][1,4]oxazin-6-yl)oxazolidin-5-yl]ethylamino]methyl]-6,7-dihydro-5H-cyclopenta[c]pyridin-3-yl]oxy]-N-methylacetamid;
6-[5-[2-[(4-Fluor-1-methyl-6,7-dihydro-SH-cyclopenta[c]pyridin-6-yl)methylamino]ethyl]-2-oxo-1,3,4-oxadiazol-3-yl]-4H-1,4-benzoxazin-3-on;
6-[5-[2-[(4-Fluor-1-methyl-6,7-dihydro-5H-cyclopenta[c]pyridin-6-yl)methylamino]ethyl]-2-oxo-1,3,4-oxadiazol-3-yl]-4H-pyrido[3,2-b][1,4]oxazin-3-on;
2-[[7-Fluor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamid;
6-[5-[2-[(4-Fluor-1-methoxy-6,7-dihydro-5H-cyclopenta[c]pyridin-6-yl)methylamino]ethyl]-2-oxo-oxazolidin-3-yl]-4H-pyrido[3,2-b][1,4]oxazin-3-on;
6-[5-[2-[(4-Fluor-1-methoxy-6,7-dihydro-5H-cyclopenta[c]pyridin-6-yl)methylamino]ethyl]-2-oxo-oxazolidin-3-yl]-4H-pyrazino[2,3-b][1,4]oxazin-3-on;
6-[8-[(4-Fluor-1-methyl-6,7-dihydro-5H-cyclopenta[c]pyridin-6-yl)methyl]-2-oxo-1-oxa-3,8-diazaspiro[4.5]decan-3-yl]-4H-pyrido[3,2-b][1,4]oxazin-3-on;
2-[[7-Fluor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]acetonitril;
2-[[7-Fluor-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-5-yl]oxy]-N-methylacetamid;
6-[5-[1-[(4-Fluor-1-methyl-6,7-dihydro-5H-cyclopenta[c]pyridin-6-yl)methyl]azetidin-3-yl]-2-oxo-oxazolidin-3-yl]-4H-pyrazino[2,3-b][1,4]oxazin-3-on;
2-[[3-[2-Oxo-3-(3-oxo-4H-pyrazino[2,3-b][1,4]oxazin-6-yl)-1,3-oxazolidin-5-yl]azetidin-1-yl]methyl]-2,3-dihydro-1H-inden-4-carbonitril;
2-[[7-Fluor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N,N-dimethylacetamid;
6-[5-[3-[(4-Fluor-1-methyl-6,7-dihydro-5H-cyclopenta[c]pyridin-6-yl)amino]propyl]-2-oxo-1,3-oxazolidin-3-yl]-4H-pyrido[3,2-b][1,4]oxazin-3-on;
6-[5-[3-[(4-Fluor-1-methyl-6,7-dihydro-5H-cyclopenta[c]pyridin-6-yl)amino]propyl]-2-oxo-1,3-oxazolidin-3-yl]-4H-pyrazino[2,3-b][1,4]oxazin-3-on;
2-[3-[2-Oxo-3-(3-oxo-4H-pyrazino[2,3-b][1,4]oxazin-6-yl)-1,3-oxazolidin-5-yl]propylamino]-2,3-dihydro-1H-inden-4-carbonitril;
2-[[2-Hydroxy-3-[2-oxo-3-(3-oxo-4H-pyrazino[2,3-b][1,4]oxazin-6-yl)-1,3-oxazolidin-5-yl]propyl]amino]-2,3-dihydro-1H-inden-4-carbonitril;
6-[5-[3-[(4-Fluor-2,3-dihydro-1H-inden-2-yl)amino]-2-hydroxypropyl]-2-oxo-1,3-oxazolidin-3-yl]-4H-pyrazino[2,3-b][1,4]oxazin-3-on;
6-[5-[2-[(4-Fluor-1-methyl-6,7-dihydro-5H-cyclopenta[c]pyridin-6-yl)methylamino]ethyl]-2-oxo-1,3-oxazol-3-yl]-4H-pyrido[3,2-b][1,4]oxazin-3-on;
6-[5-[2-[(1-Chlor-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-on;
6-[5-[2-(2,3-Dihydro-1H-inden-2-ylmethylamino)ethyl]-2-oxo-1,3-oxazolidin-3-yl]-4H-pyrido[3,2-b][1,4]oxazin-3-on;
6-[5-[2-[(1-Methyl-6,7-dihydro-SH-cyclopenta[c]pyridin-6-yl)methylamino]ethyl]-2-oxo-1,3-oxazolidin-3-yl]-4H-pyrido[3,2-b][1,4]oxazin-3-on;
6-[5-[2-[(4-Methyl-6,7-dihydro-5H-cyclopenta[b]pyridin-6-yl)methylamino]ethyl]-2-oxo-1,3-oxazolidin-3-yl]-4H-pyrido[3,2-b][1,4]oxazin-3-on;
6-[[2-[2-Oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-1-carbonitril;
6-[5-[2-[(4-Fluor-2,3-dihydro-1H-inden-2-yl)methylamino]ethyl]-2-oxo-1,3-oxazolidin-3-yl]-4H-pyrido[3,2-b][1,4]oxazin-3-on;
6-[5-[3-[(1-Chlor-6,7-dihydro-SH-cyclopenta[c]pyridin-6-yl)amino]propyl]-2-oxo-1,3-oxazolidin-3-yl]-4H-pyrido[3,2-b][1,4]oxazin-3-on;
6-[2-Oxo-5-[2-[(1,3,4-trimethyl-6,7-dihydro-5H-cyclopenta[c]pyridin-6-yl)methylamino]ethyl]-1,3-oxazolidin-3-yl]-4H-pyrido[3,2-b][1,4]oxazin-3-on;
6-[5-[3-[(1-Methyl-6,7-dihydro-SH-cyclopenta[c]pyridin-6-yl)amino]propyl]-2-oxo-1,3-oxazolidin-3-yl]-4H-pyrido[3,2-b][1,4]oxazin-3-on;
6-[3-[2-Oxo-3-(3-oxo-4H-pyrido[3,2-b][1,4]oxazin-6-yl)-1,3-oxazolidin-5-yl]propylamino]-6,7-dihydro-5H-cyclopenta[c]pyridin-1-carbonitril;
2-[[[cis-3-[2-Oxo-3-(3-oxo-4H-pyrido[3,2-b][1,4]oxazin-6-yl)-1,3-oxazolidin-5-yl]cyclobutyl]amino]methyl]-2,3-dihydro-1H-inden-4-carbonitril; Ameisensäure;
2-[[[trans-3-[2-Oxo-3-(3-oxo-4H-pyrido[3,2-b][1,4]oxazin-6-yl)-1,3-oxazolidin-5-yl]cyclobutyl]amino]methyl]-2,3-dihydro-1H-inden-4-carbonitril;
6-[5-[2-[(1,4-Dimethyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
2-[[[rac-3a,7a-cis-6,7a-trans-2-Oxo-3-(3-oxo-4H-pyrido[3,2-b][1,4]oxazin-6-yl)-3a,4,5,6,7,7a-hexahydro-1,3-benzoxazol-6-yl]amino]methyl]-2,3-dihydro-1H-inden-4-carbonitril;
2-[[[trans-4-[2-Oxo-3-(3-oxo-4H-pyrido[3,2-b][1,4]oxazin-6-yl)-1,3-oxazolidin-5-yl]cyclohexyl]amino]methyl]-2,3-dihydro-1H-inden-4-carbonitril;
6-[5-[2-[[1,4-Dimethyl-3-[(5-oxomorpholin-2-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[1,4-Dimethyl-3-[(5-oxopyrrolidin-3-yl)methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on,
6-[5-[2-[[1,4-Dimethyl-3-[(1-methyl-5-oxopyrrolidin-3-yl)methoxy]-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-on;
6-[[[2,4-trans-6-Oxo-7-(3-oxo-4H-pyrido[3,2-b][1,4]oxazin-6-yl)-5-oxa-7-azaspiro[3.4]octan-2-yl]amino]methyl]-6,7-dihydro-5H-cyclopenta[c]pyridin-1-carbonitril;
6-[5-[2-[[1,4-Dimethyl-3-(3-methylsulfonylpropoxy)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[(3-Methoxy-1,4-dimethyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
2-[[7-Cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamid;
2-[[4-Chlor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamid;
2-[[4-Chlor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid;
2-[[1,4-Dimethyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-3-yl]oxy]-N-methylacetamid;
2-[[1,4-Dimethyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridin-3-yl]oxy]-N-methylacetamid;
2-[[4,7-Dimethyl-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamid;
2-[[4,7-Dimethyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid;
2-[[4,7-Dimethyl-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-5-yl]oxy]-N-methylacetamid;
N-[2-[[7-Cyano-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]ethyl]methansulfonamid;
6-[5-[2-[[5-(2-Aminoethoxy)-4-chlor-2,3-dihydro-1H-inden-2-yl]methylamino]ethyl]-2-oxo-1,3-oxazolidin-3-yl]-4H-pyrido[3,2-b][1,4]oxazin-3-on;
6-[5-[2-[[5-[(1-Aminocyclopropyl)methoxy]-4-chlor-2,3-dihydro-1H-inden-2-yl]methylamino]ethyl]-2-oxo-1,3-oxazolidin-3-yl]-4H-pyrido[3,2-b][1,4]oxazin-3-on; Ameisensäure;
6-[5-[3-[[5-(2-Aminoethoxy)-4-chlor-2,3-dihydro-1H-inden-2-yl]amino]propyl]-2-oxo-1,3-oxazolidin-3-yl]-4H-pyrido[3,2-b][1,4]oxazin-3-on;
6-[5-[3-[[5-[(1-Aminocyclopropyl)methoxy]-4-chlor-2,3-dihydro-1H-inden-2-yl]amino]propyl]-2-oxo-1,3-oxazolidin-3-yl]-4H-pyrido[3,2-b][1,4]oxazin-3-on;
6-[4-[2-[(4-Fluor-2,3-dihydro-1H-inden-2-yl)amino]ethylamino]-2-oxopyrrolidin-1-yl]-4H-pyrido[3,2-b][1,4]oxazin-3-on;
2-[2-[[5-Oxo-1-(3-oxo-4H-pyrido[3,2-b][1,4]oxazin-6-yl)pyrrolidin-3-yl]amino]ethylamino]-2,3-dihydro-1H-inden-4-carbonitril;
6-[5-[2-[[3-[(1-Aminocyclopropyl)methoxy]-1-chlor-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-on;
6-[2-Oxo-5-[2-[[5,6-trans-5-amino-1-chlor-6,7-dihydro-5H-cyclopenta[c]pyridin-6-yl]methylamino]ethyl]-1,3-oxazolidin-3-yl]-4H-pyrido[3,2-b][1,4]oxazin-3-on;
5-[(1-Aminocyclopropyl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril; Hydrochlorid;
3-[(1-Aminocyclopropyl)methoxy]-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-1-carbonitril;
6-[5-[2-[[3-(2-Aminoethoxy)-1-chlor-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
3-(2-Aminoethoxy)-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-1-carbonitril;
5-(2-Aminoethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
5-(Azetidin-3-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[2-Oxo-5-[2-[(rac-5,6-trans-5-amino-1-methyl-6,7-dihydro-SH-cyclopenta[c]pyridin-6-yl)methylamino]ethyl]-1,3-oxazolidin-3-yl]-4H-pyrido[3,2-b][1,4]oxazin-3-on;
rac-1,2-trans-1-Amino-2-[[2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-inden-4-carbonitril;
5-(Azetidin-3-yloxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
rac-5,6-trans-5-Amino-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-1-carbonitril;
5-(2-Aminopropoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
7-[2-Oxo-5-[2-[[rac-1,2-trans-1-amino-4-chlor-2,3-dihydro-1H-inden-2-yl]methylamino]ethyl]-1,3-oxazolidin-3-yl]-1H-pyrido[2,3-b][1,4]oxazin-2-on;
6-[2-Oxo-5-[2-[[rac-1,2-trans-1-amino-4-chlor-2,3-dihydro-1H-inden-2-yl]methylamino]ethyl]-1,3-oxazolidin-3-yl]-4H-pyrazino[2,3-b][1,4]oxazin-3-on;
5-(Azetidin-2-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
2-[[2-[3-Amino-5-oxo-1-(3-oxo-4H-pyrido[3,2-b][1,4]oxazin-6-yl)pyrrolidin-3-yl]ethylamino]methyl]-2,3-dihydro-1H-inden-4-carbonitril;
rac-1,2-trans-1-Hydroxy-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[4-Amino-4-[2-[(4-chlor-2,3-dihydro-1H-inden-2-yl)methylamino]ethyl]-2-oxopyrrolidin-1-yl]-4H-pyrido[3,2-b][1,4]oxazin-3-on;
2-[[2-[2-Oxo-3-(3-oxo-4H-pyrido[3,2-b][1,4]oxazin-6-yl)-1,3-oxazolidin-5-yl]ethylamino]methyl]-5-(pyrrolidin-2-ylmethoxy)-2,3-dihydro-1H-inden-4-carbonitril;
6-[5-[[2-(2-Amino-4-chlor-1,3-dihydroinden-2-yl)ethylamino]methyl]-2-oxo-1,3-oxazolidin-3-yl]-4H-pyrido[3,2-b][1,4]oxazin-3-on; Ameisensäure;
2-[[2-[2-Oxo-3-(3-oxo-4H-pyrido[3,2-b][1,4]oxazin-6-yl)-1,3-oxazolidin-5-yl]ethylamino]methyl]-5-(2-piperazin-1-ylethoxy)-2,3-dihydro-1H-inden-4-carbonitril;
6-[5-[2-[[3-(2-Aminopropoxy)-1-chlor-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-(2-Aminopropoxy)-1-chlor-6,7-dihydro-5H-cyclopenta[c]pyridin-6-yl]methylamino]ethyl]-2-oxo-oxazolidin-3-yl]-4H-pyrazino[2,3-b][1,4]oxazin-3-on;
6-[2-Oxo-5-[2-[[rac-5,6-cis-5-amino-1-chlor-6,7-dihydro-5H-cyclopenta[c]pyridin-6-yl]methylamino]ethyl]-1,3-oxazolidin-3-yl]-4H-pyrido[3,2-b][1,4]oxazin-3-on;
6-[2-Oxo-5-[2-[[rac-1,2-trans-1-amino-4-chlor-2,3-dihydro-1H-inden-2-yl]methylamino]ethyl]-1,3,4-oxadiazol-3-yl]-4H-1,4-benzoxazin-3-on;
2-[[2-[rac-4,5-cis-4-(Aminomethyl)-2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
2-[[2-[rac-4,5-trans-4-(Aminomethyl)-2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[5-[2-[[6-(Azetidin-3-yloxy)-4-fluor-2,3-dihydro-1H-inden-2-yl]methylamino]ethyl]-2-oxo-1,3-oxazolidin-3-yl]-4H-pyrido[3,2-b][1,4]oxazin-3-on;
rac-2,3-trans-3-Hydroxy-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
rac-2,3-trans-3-Hydroxy-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-4-carbonitril;
6-[5-[2-[(rac-1,2-trans-1-Amino-4-chlor-2,3-dihydro-1H-inden-2-yl)methylamino]ethyl]-2-oxo-1,3,4-oxadiazol-3-yl]-4H-pyrido[3,2-b][1,4]oxazin-3-on;
6-[5-[2-[[6-(2-Aminopropoxy)-4-fluor-2,3-dihydro-1H-inden-2-yl]methylamino]ethyl]-2-oxo-1,3-oxazolidin-3-yl]-4H-pyrido[3,2-b][1,4]oxazin-3-on;
6-[5-[2-[[6-(1-Aminopropan-2-yloxy)-4-fluor-2,3-dihydro-1H-inden-2-yl]methylamino]ethyl]-2-oxo-1,3-oxazolidin-3-yl]-4H-pyrido[3,2-b][1,4]oxazin-3-on;
rac-2,3-trans-3-Amino-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
rac-2,3-trans-3-Amino-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-4-carbonitril;
6-[5-[[[2-Amino-2-(4-chlor-2,3-dihydro-1H-inden-2-yl)ethyl]amino]methyl]-2-oxo-1,3-oxazolidin-3-yl]-4H-pyrido[3,2-b][1,4]oxazin-3-on;
6-[2-[2-Amino-2-(4-chlor-2,3-dihydro-1H-inden-2-yl)ethyl]-6-oxo-5-oxa-2,7-diazaspiro[3.4]octan-7-yl]-4H-pyrido[3,2-b][1,4]oxazin-3-on;
6-[5-[2-[[6-[(1-Aminocyclopropyl)methoxy]-4-fluor-2,3-dihydro-1H-inden-2-yl]methylamino]ethyl]-2-oxo-1,3-oxazolidin-3-yl]-4H-pyrido[3,2-b][1,4]oxazin-3-on;
6-[5-[2-[[6-[(1-Aminocyclopropyl)methoxy]-4-fluor-2,3-dihydro-1H-inden-2-yl]methylamino]ethyl]-2-oxo-1,3-oxazolidin-3-yl]-4H-pyrazino[2,3-b][1,4]oxazin-3-on
6-[5-[2-[[6-[(3-Aminooxetan-3-yl)methoxy]-4-fluor-2,3-dihydro-1H-inden-2-yl]methylamino]ethyl]-2-oxo-1,3-oxazolidin-3-yl]-4H-pyrido[3,2-b][1,4]oxazin-3-on;
6-[5-[2-[(rac-1,2-trans-1-Amino-4-fluor-2,3-dihydro-1H-inden-2-yl)methylamino]ethyl]-2-oxo-1,3-oxazolidin-3-yl]-4H-pyrido[3,2-b][1,4]oxazin-3-on;
6-[5-[2-[(rac-1,2-trans-1-Amino-4-fluor-2,3-dihydro-1H-inden-2-yl)methylamino]ethyl]-2-oxo-1,3-oxazolidin-3-yl]-4H-pyrazino[2,3-b][1,4]oxazin-3-on;
5-[2-[(rac-1,2-trans-1-Amino-4-fluor-2,3-dihydro-1H-inden-2-yl)methylamino]ethyl]-3-(3-oxo-4H-pyrido[3,2-b][1,4]thiazin-6-yl)-1,3-oxazolidin-2-on;
2-[[rac-5,6-cis-5,8-trans-6-Amino-2-oxo-3-(3-oxo-4H-pyrido[3,2-b][1,4]oxazin-6-yl)-1-oxa-3-azaspiro[4.5]decan-8-yl]amino]-2,3-dihydro-1H-inden-4-carbonitril;
6-[rac-5, 6-cis-5, 8-trans-6-Amino-8-[(4-fluor-2,3-dihydro-1H-inden-2-yl)amino]-2-oxo-1-oxa-3-azaspiro[4.5]decan-3-yl]-4H-pyrido[3,2-b][1,4]oxazin-3-on;
6-[2-Oxo-5-[2-[[rac-1,2-trans-1-amino-7-chlor-2,3-dihydro-1H-inden-2-yl]methylamino]ethyl]-1,3-oxazolidin-3-yl]-4H-pyrido[3,2-b][1,4]oxazin-3-on;
6-[2-Oxo-5-[2-[[rac-1,2-trans-1-amino-7-chlor-2,3-dihydro-1H-inden-2-yl]methylamino]ethyl]-1,3-oxazolidin-3-yl]-4H-pyrazino[2,3-b][1,4]oxazin-3-on;
6-[2-Oxo-5-[2-[[rac-1,2-trans-1-amino-4-chlor-2,3-dihydro-1H-inden-2-yl]methylamino]ethyl]-1,3-oxazol-3-yl]-4H-pyrido[3,2-b][1,4]oxazin-3-on;
6-[5-[2-[[3-(2-Aminoethoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-[(1-Aminocyclopropyl)methoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[1,4-Dimethyl-3-[2-(methylamino)ethoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-(2-Aminoethoxy)-4-chlor-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-[(1-Aminocyclopropyl)methoxy]-1-methyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-(3-Aminopropoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[6-(2-Aminoethoxy)-4-chlor-2,3-dihydro-1H-inden-2-yl]methylamino]ethyl]-2-oxo-1,3-oxazolidin-3-yl]-4H-pyrido[3,2-b][1,4]oxazin-3-on;
6-[5-[2-[[6-[(1-Aminocyclopropyl)methoxy]-4-chlor-2,3-dihydro-1H-inden-2-yl]methylamino]ethyl]-2-oxo-1,3-oxazolidin-3-yl]-4H-pyrido[3,2-b][1,4]oxazin-3-on;
6-[5-[3-[[6-(2-Aminoethoxy)-4-chlor-2,3-dihydro-1H-inden-2-yl]amino]propyl]-2-oxo-1,3-oxazolidin-3-yl]-4H-pyrido[3,2-b][1,4]oxazin-3-on;
6-[5-[3-[[6-[(1-Aminocyclopropyl)methoxy]-4-chlor-2,3-dihydro-1H-inden-2-yl]amino]propyl]-2-oxo-1,3-oxazolidin-3-yl]-4H-pyrido[3,2-b][1,4]oxazin-3-on;
6-[5-[2-[[3-[(1-Aminocyclopropyl)methoxy]-4-chlor-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-on;
6-[[[2,4-cis-6-Oxo-7-(3-oxo-4H-pyrido[3,2-b][1,4]oxazin-6-yl)-5-oxa-7-azaspiro[3.4]octan-2-yl]amino]methyl]-6,7-dihydro-5H-cyclopenta[c]pyridin-1-carbonitril;
2-[[[2,4-cis-6-Oxo-7-(3-oxo-4H-pyrido[3,2-b][1,4]oxazin-6-yl)-5-oxa-7-azaspiro[3.4]octan-2-yl]amino]methyl]-2,3-dihydro-1H-inden-4-carbonitril;
6-[5-[2-[[3-(Azetidin-3-ylmethoxy)-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[(1-Aminocyclopropyl)methoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-((1-Aminocyclopropyl)methoxy)-2-(((2-(2-oxo-3-(3-oxo-3,4-dihydro-2H-pyrazino[2,3-b][1,4]oxazin-6-yl)oxazolidin-5-yl)ethyl)amino)methyl)-2,3-dihydro-1H-inden-4-carbonitril;
6-(Azetidin-2-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-(2-Aminoethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[5-[2-[[3-[2-(Cyclopropylamino)ethoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on,
6-(Azetidin-3-yloxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-(Azetidin-3-ylmethoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-(2-Aminopropoxy)-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-(2-Aminopropoxy)-2-(((2-(2-oxo-3-(3-oxo-3,4-dihydro-2H-pyrazino[2,3-b][1,4]oxazin-6-yl)oxazolidin-5-yl)ethyl)amino)methyl)-2,3-dihydro-1H-inden-4-carbonitril;
6-[2-Oxo-5-[[2-[rac-1,2-trans-1-amino-4-chlor-2,3-dihydro-1H-inden-2-yl]ethylamino]methyl]-1,3-oxazolidin-3-yl]-4H-pyrido[3,2-b][1,4]oxazin-3-on;
6-[6-Oxo-2-[2-[rac-1,2-trans-1-amino-4-chlor-2,3-dihydro-1H-inden-2-yl]ethyl]-5-oxa-2,7-diazaspiro[3.4]octan-7-yl]-4H-pyrido[3,2-b][1,4]oxazin-3-on;
6-((1-Aminocyclopropyl)methoxy)-2-(2-(((2-oxo-3-(3-oxo-3,4-dihydro-2H-pyrido[3,2-b][1,4]oxazin-6-yl)oxazolidin-5-yl)methyl)amino)ethyl)-2,3-dihydro-1H-inden-4-carbonitril;
6-((1-Aminocyclopropyl)methoxy)-2-(2-(6-oxo-7-(3-oxo-3,4-dihydro-2H-pyrido[3,2-b][1,4]oxazin-6-yl)-5-oxa-2,7-diazaspiro[3.4]octan-2-yl)ethyl)-2,3-dihydro-1H-inden-4-carbonitril;
3-[(1-Aminocyclopropyl)methoxy]-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-4-carbonitril;
6-[5-[2-[[1,4-Dimethyl-3-(morpholin-3-ylmethoxy)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-[[(2S)-Azetidin-2-yl]methoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[1,4-Dimethyl-3-[2-(propan-2-ylamino)ethoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-[2-(2-Methoxyethylamino)ethoxy]-1,4-dimethyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-(2-Amino-2-methylpropoxy)-2-(((2-(2-oxo-3-(3-oxo-3,4-dihydro-2H-pyrido[3,2-b][1,4]oxazin-6-yl)oxazolidin-5-yl)ethyl)amino)methyl)-2,3-dihydro-1H-inden-4-carbonitril;
6-(2-Amino-2-methylpropoxy)-2-(((2-(2-oxo-3-(3-oxo-3,4-dihydro-2H-pyrazino[2,3-b][1,4]oxazin-6-yl)oxazolidin-5-yl)ethyl)amino)methyl)-2,3-dihydro-1H-inden-4-carbonitril;
6-[5-[1-[(rac-1,2-trans-1-Amino-4-chlor-2,3-dihydro-1H-inden-2-yl)methyl]azetidin-3-yl]-2-oxo-1,3-oxazolidin-3-yl]-4H-pyrazino[2,3-b][1,4]oxazin-3-on;
6-[rac-5,6-cis-5,8-trans-6-Amino-8-[(1-chlor-6,7-dihydro-5H-cyclopenta[c]pyridin-6-yl)methylamino]-2-oxo-1-oxa-3-azaspiro[4.5]decan-3-yl]-4H-pyrido[3,2-b][1,4]oxazin-3-on;
6-[2-Oxo-5-[2-[[1,2-trans-1-amino-4-chlor-2,3-dihydro-1H-inden-2-yl]methylamino]ethyl]-1,3-oxazolidin-3-yl]-4H-pyrido[3,2-b][1,4]oxazin-3-on;
6-[5-[2-[[3-(2-Aminoethoxy)-1-methyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
2,4-cis-6-[6-Oxo-2-[[rac-1,2-trans-1-amino-4-chlor-2,3-dihydro-1H-inden-2-yl]methylamino]-5-oxa-7-azaspiro[3.4]octan-7-yl]-4H-pyrido[3,2-b][1,4]oxazin-3-on;
6-[6-Oxo-2-[[rac-(1R,2R)-1-amino-4-chlor-2,3-dihydro-1H-inden-2-yl]methylamino]-5-oxa-7-azaspiro[3.4]octan-7-yl]-4H-pyrido[3,2-b][1,4]oxazin-3-on;
6-[2-Oxo-8-[[rac-1,2-trans-1-amino-4-chlor-2,3-dihydro-1H-inden-2-yl]methyl]-1-oxa-3,8-diazaspiro[4.5]decan-3-yl]-4H-pyrido[3,2-b][1,4]oxazin-3-on;
3-[(1-Aminocyclopropyl)methoxy]-4-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-SH-cyclopenta[c]pyridin-1-carbonitril;
3-[(1-Aminocyclopropyl)methoxy]-1-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-4-carbonitril;
3-[(1-Aminocyclopropyl)methoxy]-1-methyl-6-[[2-oxo-3-(3-oxo-4H-pyrido[3,2-b][1,4]oxazin-6-yl)-1-oxa-3,8-diazaspiro[4.5]decan-8-yl]methyl]-6,7-dihydro-5H-cyclopenta[c]pyridin-4-carbonitril;
6-[5-[2-[[3-[(1-Aminocyclopropyl)methoxy]-4-methyl-1-(5-methyl-1,3-thiazol-2-yl)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-[(1-Aminocyclopropyl)methoxy]-4-methyl-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[3-[(1-Aminocyclopropyl)methoxy]-4-methyl-1-(1,3-thiazol-2-yl)-6,7-dihydro-SH-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[4-Methyl-3-[[(2R)-azetidin-2-yl]methoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
4-Methyl-6-((2-oxo-3-(3-oxo-3,4-dihydro-2H-pyrido[3,2-b][1,4]oxazin-6-yl)-1-oxa-3,8-diazaspiro[4.5]decan-8-yl)methyl)-3-((5-oxopiperazin-2-yl)methoxy)-6,7-dihydro-SH-cyclopenta[c]pyridin-1-carbonitril;
2-((1-Cyano-4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)-N-methylacetamid;
2-((1-Cyano-4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)-N-isopropylacetamid;
N-Methyl-2-((4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)acetamid;
N-Isopropyl-2-((4-methyl-6-(((2-(2-oxo-3-(3-oxo-3,4-dihydro-2H-pyrido[3,2-b][1,4]oxazin-6-yl)oxazolidin-5-yl)ethyl)amino)methyl)-6,7-dihydro-5H-cyclopenta[c]pyridin-3-yl)oxy)acetamid;
Ethyl-1-ethyl-6-fluor-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]chinolin-3-carboxylat;
6-[2-Oxo-5-[2-[[rac-1,2-trans-1-amino-4-chlor-2,3-dihydro-1H-inden-2-yl]methylamino]ethyl]-1,3-oxazolidin-3-yl]-4H-pyrido[3,2-b][1,4]oxazin-3-on;
6-[2-Oxo-5-[3-[[rac-1,2-trans-1-amino-4-chlor-2,3-dihydro-1H-inden-2-yl]methylamino]propyl]-1,3-oxazolidin-3-yl]-4H-pyrido[3,2-b][1,4]oxazin-3-on;
rac-1,2-trans-1-Amino-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
rac-1,2-trans-1-Amino-2-((((2-oxo-3-(3-oxo-3,4-dihydro-2H-pyrido[3,2-b][1,4]oxazin-6-yl)oxazolidin-5-yl)methyl)amino)methyl)-2,3-dihydro-1H-inden-4-carbonitril;
6-[2-Oxo-5-[2-[[rac-1,2-trans-4-chlor-1-hydroxy-2,3-dihydro-1H-inden-2-yl]methylamino]ethyl]-1,3-oxazolidin-3-yl]-4H-pyrido[3,2-b][1,4]oxazin-3-on;
6-[2-Oxo-5-[2-[[rac-1,2-cis-4-chlor-1-hydroxy-2,3-dihydro-1H-inden-2-yl]methylamino]ethyl]-1,3-oxazolidin-3-yl]-4H-pyrido[3,2-b][1,4]oxazin-3-on;
6-[5-[2-[(4-Chlor-2-hydroxy-1,3-dihydroinden-2-yl)methylamino]ethyl]-2-oxo-1,3-oxazolidin-3-yl]-4H-pyrido[3,2-b][1,4]oxazin-3-on;
2-[[2-Aminoethyl-[2-[2-oxo-3-(3-oxo-4H-pyrido[3,2-b][1,4]oxazin-6-yl)-1,3-oxazolidin-5-yl]ethyl]amino]methyl]-2,3-dihydro-1H-inden-4-carbonitril;
6-[5-[2-[[1,4-Dimethyl-3-[(2R)-2-amino-3-hydroxypropoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
3-[(2R)-2-Amino-3-hydroxypropoxy]-4-methyl-6-[[2-[2-oxo-3-(3-oxo-4H-pyrido[3,2-b][1,4]oxazin-6-yl)-1,3-oxazolidin-5-yl]ethylamino]methyl]-6,7-dihydro-5H-cyclopenta[c]pyridin-1-carbonitril;
6-[(2R)-2-Amino-3-hydroxypropoxy]-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[5-[2-[[4-Chlor-3-[rac-(2R)-2-amino-3-hydroxypropoxy]-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
2-[[2-Hydroxy-3-[2-oxo-3-(3-oxo-4H-pyrido[3,2-b][1,4]oxazin-6-yl)-1,3-oxazolidin-5-yl]propyl]amino]-2,3-dihydro-1H-inden-4-carbonitril;
1-Ethyl-6-fluor-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]chinolin-3-carbonsäure;
6-[5-[2-[(2-Amino-4-chlor-1,3-dihydroinden-2-yl)methylamino]ethyl]-2-oxo-1,3-oxazolidin-3-yl]-4H-pyrido[3,2-b][1,4]oxazin-3-on;
2-[[rac-5,6-trans-5,8-cis-6-Amino-2-oxo-3-(3-oxo-4H-pyrido[3,2-b][1,4]oxazin-6-yl)-1-oxa-3-azaspiro[4.5]decan-8-yl]amino]-2,3-dihydro-1H-inden-4-carbonitril;
6-[5-(Aminomethyl)-5-[2-[(4-fluor-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
2-[[2-[5-(Aminomethyl)-2-oxo-3-(3-oxo-4H-pyrido[3,2-b][1,4]oxazin-6-yl)-1,3-oxazolidin-5-yl]ethylamino]methyl]-2,3-dihydro-1H-inden-4-carbonitril;
6-[2-Oxo-5-[3-[[rac-1,2-trans-1-amino-4-chlor-2,3-dihydro-1H-inden-2-yl]amino]propyl]-1,3-oxazolidin-3-yl]-4H-pyrido[3,2-b][1,4]oxazin-3-on;
6-[2-Oxo-5-[3-[[rac-1,2-trans-1-amino-4-chlor-2,3-dihydro-1H-inden-2-yl]amino]propyl]-1,3-oxazolidin-3-yl]-4H-pyrazino[2,3-b][1,4]oxazin-3-on;
5-[2-[(rac-1,2-trans-1-Amino-4-Chlor-2,3-dihydro-1H-inden-2-yl)methylamino]ethyl]-3-(3-oxo-4H-pyrido[3,2-b][1,4]thiazin-6-yl)-1,3-oxazolidin-2-on;
rac-1,2-trans-1-Amino-2-[[2-[2-oxo-3-(3-oxo-4H-pyrido[3,2-b][1,4]thiazin-6-yl)-1,3-oxazolidin-5-yl]ethylamino]methyl]-2,3-dihydro-1H-inden-4-carbonitril;
6-[5-[2-[[6-(2-Amino-3-methoxypropoxy)-4-fluor-2,3-dihydro-1H-inden-2-yl]methylamino]ethyl]-2-oxo-1,3-oxazolidin-3-yl]-4H-pyrido[3,2-b][1,4]oxazin-3-on;
2-[[rac-5,6-trans-6-(Aminomethyl)-2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-inden-4-carbonitril;
6-[rac-5,6-trans-6-(Aminomethyl)-8-[(4-fluor-1-methyl-6,7-dihydro-5H-cyclopenta[c]pyridin-6-yl)methyl]-2-oxo-1-oxa-3,8-diazaspiro[4.5]decan-3-yl]-4H-pyrido[3,2-b][1,4]oxazin-3-on;
6-[rac-5,6-cis-6-(Aminomethyl)-8-[(4-fluor-1-methyl-6,7-dihydro-5H-cyclopenta[c]pyridin-6-yl)methyl]-2-oxo-1-oxa-3,8-diazaspiro[4.5]decan-3-yl]-4H-pyrido[3,2-b][1,4]oxazin-3-on;
2-[[rac-5,6-cis-6-(Aminomethyl)-2-oxo-3-(3-oxo-4H-pyrido[3,2-b][1,4]oxazin-6-yl)-1-oxa-3,8-diazaspiro[4.5]decan-8-yl]methyl]-2,3-dihydro-1H-inden-4-carbonitril;
6-[5-[2-[[5-[(1-Aminocyclopropyl)methoxy]-4,7-dimethyl-2,3-dihydro-1H-inden-2-yl]methylamino]ethyl]-2-oxo-1,3-oxazolidin-3-yl]-4H-pyrazino[2,3-b][1,4]oxazin-3-on;
2-[[2-[2-Oxo-3-(3-oxo-4H-pyrido[3,2-b][1,4]oxazin-6-yl)-1,3-oxazolidin-5-yl]ethylamino]methyl]-6-pyrrolidin-3-yloxy-2,3-dihydro-1H-inden-4-carbonitril; Ameisensäure
2-Amino-3-[[7-fluor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylpropanamid;
6-[5-[2-[(1-Chlor-4-fluor-3-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
6-[8-[[6-[(1-Aminocyclopropyl)methoxy]-4-fluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on;
6-[8-[(4-Fluor-1-methyl-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-on;
6-[2-Oxo-8-[[rac-1,2-trans-1-amino-4-chlor-2,3-dihydro-1H-inden-2-yl]methyl]-1-oxa-3,8-diazaspiro[4.5]decan-3-yl]-4H-pyrazino[2,3-b][1,4]oxazin-3-on;
6-[8-[[6-(2-Aminopropoxy)-4-fluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on;
2-[[2-Oxo-3-(3-oxo-4H-pyrazino[2,3-b][1,4]oxazin-6-yl)-1-oxa-3,8-diazaspiro[4.5]decan-8-yl]methyl]-2,3-dihydro-1H-inden-4-carbonitril;
6-[5-[2-[(rac-5,6-trans-5-Amino-4-chlor-1-methyl-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
6-[2-Oxo-5-[2-[[rac-5,6-trans-5-amino-4-chlor-1-methyl-6,7-dihydro-5H-cyclopenta[c]pyridin-6-yl]methylamino]ethyl]-1,3-oxazolidin-3-yl]-4H-pyrazino[2,3-b][1,4]oxazin-3-on;
3-(3-Oxo-4H-pyrido[3,2-b][1,4]thiazin-6-yl)-5-[2-[[rac-5,6-trans-5-amino-4-chlor-1-methyl-6,7-dihydro-5H-cyclopenta[c]pyridin-6-yl]methylamino]ethyl]-1,3-oxazolidin-2-on;
6-[5-[[2-(4-Fluor-1-methyl-6,7-dihydro-5H-cyclopenta[c]pyridin-6-yl)ethylamino]methyl]-2-oxo-1,3-oxazolidin-3-yl]-4H-pyrazino[2,3-b][1,4]oxazin-3-on;
6-[2-Oxo-5-[[2-[rac-1,2-trans-1-amino-4-chlor-2,3-dihydro-1H-inden-2-yl]ethylamino]methyl]-1,3-oxazolidin-3-yl]-4H-pyrazino[2,3-b][1,4]oxazin-3-on;
2-Amino-3-[[7-fluor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]propansäure;
6-[5-[2-[(1,4-Dimethyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
6-[2-Oxo-5-[2-[(1,3,4-trimethyl-6,7-dihydro-5H-cyclopenta[c]pyridin-6-yl)methylamino]ethyl]-1,3-oxazolidin-3-yl]-4H-pyrazino[2,3-b][1,4]oxazin-3-on;
6-[5-[2-[(4-Methyl-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-on;
6-[5-[2-[[6-[2-(Dimethylamino)ethoxy]-4-fluor-2,3-dihydro-1H-inden-2-yl]methylamino]ethyl]-2-oxo-1,3-oxazolidin-3-yl]-4H-pyrido[3,2-b][1,4]oxazin-3-on;
6-[5-[2-[[6-[2-(Dimethylamino)ethoxy]-4-fluor-2,3-dihydro-1H-inden-2-yl]methylamino]ethyl]-2-oxo-1,3-oxazolidin-3-yl]-4H-pyrazino[2,3-b][1,4]oxazin-3-on;
6-[2-Oxo-5-[2-[[rac-1,2-trans-1-amino-6-[(1-aminocyclopropyl)methoxy]-4-chlor-2,3-dihydro-1H-inden-2-yl]methylamino]ethyl]-1,3-oxazolidin-3-yl]-4H-pyrido[3,2-b][1,4]oxazin-3-on,
6-[2-Oxo-8-[[rac-1,2-trans-1-amino-6-[(1-aminocyclopropyl)methoxy]-4-chlor-2,3-dihydro-1H-inden-2-yl]methyl]-1-oxa-3,8-diazaspiro[4.5]decan-3-yl]-4H-pyrido[3,2-b][1,4]oxazin-3-on;
1-Ethyl-4-oxo-8-[[2-[2-oxo-3-(3-oxo-4H-pyrido[3,2-b][1,4]oxazin-6-yl)-1,3-oxazolidin-5-yl]ethylamino]methyl]-8,9-dihydro-7H-cyclopenta[h]chinolin-3-carbonsäure;
1-Ethyl-4-oxo-8-[[2-[2-oxo-3-(3-oxo-4H-pyrazino[2,3-b][1,4]oxazin-6-yl)-1,3-oxazolidin-5-yl]ethylamino]methyl]-8,9-dihydro-7H-cyclopenta[h]chinolin-3-carbonsäure;
6-[8-[(4-Fluor-1-methyl-6,7-dihydro-5H-cyclopenta[c]pyridin-6-yl)methyl]-6-(hydroxymethyl)-2-oxo-1-oxa-3,8-diazaspiro[4.5]decan-3-yl]-4H-pyrido[3,2-b][1,4]oxazin-3-on;
6-[5-[[2-(4-Fluor-1-methyl-6,7-dihydro-5H-cyclopenta[c]pyridin-6-yl)ethylamino]methyl]-5-(2-hydroxyethyl)-2-oxo-1,3-oxazolidin-3-yl]-4H-pyrido[3,2-b][1,4]oxazin-3-on;
6-[5-[2-[(1-Methyl-6,7-dihydro-SH-cyclopenta[c]pyridin-6-yl)methylamino]ethyl]-2-oxo-1,3-oxazolidin-3-yl]-4H-pyrazino[2,3-b][1,4]oxazin-3-on; Ameisensäure;
6-[5-[[2-(6-Amino-4-fluor-1-methyl-5,7-dihydrocyclopenta[c]pyridin-6-yl)ethylamino]methyl]-2-oxo-1,3-oxazolidin-3-yl]-4H-pyrido[3,2-b][1,4]oxazin-3-on;
6-[5-[2-[(4-Chlor-2,3-dihydro-1H-inden-2-yl)methylamino]ethyl]-2-oxo-1,3-oxazolidin-3-yl]-4H-pyrazino[2,3-b][1,4]oxazin-3-on;
6-[2-Oxo-5-[2-[[rac-1,2-trans-1-amino-6-[(1-aminocyclopropyl)methoxy]-4-chlor-2,3-dihydro-1H-inden-2-yl]methylamino]ethyl]-1,3-oxazolidin-3-yl]-4H-pyrazino[2,3-b][1,4]oxazin-3-on, Ameisensäure;
6-[5-[2-[(1,4-Dimethyl-6,7-dihydro-5H-cyclopenta[d]pyridazin-6-yl)methylamino]ethyl]-2-oxo-1,3-oxazolidin-3-yl]-4H-pyrazino[2,3-b][1,4]oxazin-3-on;
6-[5-[2-[(1,4-Dimethyl-6,7-dihydro-5H-cyclopenta[d]pyridazin-6-yl)methylamino]ethyl]-2-oxo-1,3-oxazolidin-3-yl]-4H-pyrido[3,2-b][1,4]oxazin-3-on;
5-[2-[(1,4-Dimethyl-6,7-dihydro-5H-cyclopenta[d]pyridazin-6-yl)methylamino]ethyl]-3-(3-oxo-4H-pyrido[3,2-b][1,4]thiazin-6-yl)-1,3-oxazolidin-2-on;
2-[[4-Cyano-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid;
6-[5-[2-[(4-Fluor-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
6-[5-[2-[(4-Fluor-6,7-dihydro-5H-cyclopenta[c]pyridin-6-yl)methylamino]ethyl]-2-oxo-1,3-oxazolidin-3-yl]-4H-pyrido[3,2-b][1,4]oxazin-3-on;
2-[[2-[2-Oxo-3-(7-oxo-8H-pyrimido[5,4-b][1,4]oxazin-2-yl)-1,3-oxazolidin-5-yl]ethylamino]methyl]-2,3-dihydro-1H-inden-4-carbonitril;
2-[5-[2-[(rac-1,2-trans-1-Amino-4-chlor-2,3-dihydro-1H-inden-2-yl)methylamino]ethyl]-2-oxo-1,3-oxazolidin-3-yl]-8H-pyrimido[5,4-b][1,4]oxazin-7-on;
1-Methyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridin-4-carbonitril; 2,2,2-Trifluoressigsäure;
6-[5-[2-[[4-Fluor-6-(2-hydroxy-2-methylpropoxy)-2,3-dihydro-1H-inden-2-yl]methylamino]ethyl]-2-oxo-1,3-oxazolidin-3-yl]-4H-pyrazino[2,3-b][1,4]oxazin-3-on;
6-[5-[2-[(4-Fluor-1-methyl-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-on;
6-[5-[2-[[6-[2-(5-Aminotetrazol-1-yl)ethoxy]-4-fluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[6-[2-(5-Aminotetrazol-2-yl)ethoxy]-4-fluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[4-Fluor-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
trans-6-[5-[2-[[4-Fluor-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
cis-6-[5-[2-[[4-Fluor-6-[2-(1,2,4-triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[(3-Fluor-4-methyl-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-on;
6-[5-[2-[(3-Fluor-4-methyl-6,7-dihydro-5H-cyclopenta[b]pyridin-6-yl)methylamino]ethyl]-2-oxo-1,3-oxazolidin-3-yl]-4H-pyrido[3,2-b][1,4]oxazin-3-on;
6-[8-[(3-Fluor-4-methyl-6,7-dihydro-5H-cyclopenta[b]pyridin-6-yl)methyl]-2-oxo-1-oxa-3,8-diazaspiro[4.5]decan-3-yl]-4H-pyrazino[2,3-b][1,4]oxazin-3-on;
6-[5-[3-[(4-Fluor-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-on; Ameisensäure;
6-[5-[3-[(4-Fluor-1-methyl-6,7-dihydro-5H-cyclopenta[c]pyridin-6-yl)methylamino]propyl]-2-oxo-1,3-oxazol-3-yl]-4H-pyrazino[2,3-b][1,4]oxazin-3-on; Ameisensäure;
6-[5-[2-[[4-Fluor-6-[2-(2-oxo-1,3-oxazolidin-3-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
2-[[7-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid;
2-[[7-Fluor-2-[[2-[2-oxo-3-(3-oxo-4H-pyrazino[2,3-b][1,4]oxazin-6-yl)-1,3-oxazol-5-yl]ethylamino]methyl]-2,3-dihydro-1H-inden-5-yl]oxy]acetamid; Ameisensäure;
6-[5-[2-[[6-[2-(Azetidin-1-yl)-2-oxoethoxy]-4-fluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
N-Ethyl-2-[[7-fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid; Ameisensäure;
2-[[7-Fluor-2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-inden-5-yl]oxy]-N-methylacetamid;
2-[[7-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid; Ameisensäure;
2-[[7-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methoxyacetamid; Ameisensäure;
2-[[4,7-Difluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid;
6-[2-Oxo-5-[3-[(4-fluor-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-on; Ameisensäure, Isomer A;
6-[2-Oxo-5-[3-[(4-fluor-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-on; Ameisensäure, Isomer B;
6-[2-Oxo-5-[3-[(4-fluor-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-on; Ameisensäure, Isomer C;
6-[2-Oxo-5-[3-[(4-Fluor-1-methyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-on; Ameisensäure, Isomer D;
6-[5-[2-[(4-Fluor-6-hydroxy-2,3-dihydro-1H-inden-2-yl)methylamino]ethyl]-2-oxo-1,3-oxazolidin-3-yl]-4H-pyrazino[2,3-b][1,4]oxazin-3-on;
6-[5-[2-[(4-Fluor-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-on;
6-[5-[2-[(4-Chlor-3-fluor-6,7-dihydro-5H-cyclopenta[b]pyridin-6-yl)methylamino]ethyl]-2-oxo-1,3-oxazolidin-3-yl]-4H-pyrazino[2,3-b][1,4]oxazin-3-on; Ameisensäure;
6-[5-[2-[[4-Fluor-6-(1H-pyrazol-3-ylmethoxy)-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
1-Methyl-6-[3-[2-oxo-3-(3-oxo-4H-pyrazino[2,3-b][1,4]oxazin-6-yl)-1,3-oxazol-5-yl]propylamino]-6,7-dihydro-5H-cyclopenta[c]pyridin-4-carbonitril, Enantiomer A;
1-Methyl-6-[3-[2-oxo-3-(3-oxo-4H-pyrazino[2,3-b][1,4]oxazin-6-yl)-1,3-oxazol-5-yl]propylamino]-6,7-dihydro-5H-cyclopenta[c]pyridin-4-carbonitril, Enantiomer B;
6-[5-[2-[[4-Fluor-6-(oxetan-3-yloxy)-2,3-dihydro-1H-inden-2-yl]methylamino]ethyl]-2-oxo-1,3-oxazolidin-3-yl]-4H-pyrazino[2,3-b][1,4]oxazin-3-on;
N-[2-[[7-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]ethyl]methansulfonamid;
N-[2-[[7-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]ethyl]-N-methylmethansulfonamid;
6-[2-[2-(4-Fluor-1-methyl-6,7-dihydro-5H-cyclopenta[c]pyridin-6-yl)ethyl]-6-oxo-5-oxa-2,7-diazaspiro[3.4]octan-7-yl]-4H-pyrazino[2,3-b][1,4]oxazin-3-on;
6-[2-[2-[6-(2-Aminopropoxy)-4-fluor-2,3-dihydro-1H-inden-2-yl]ethyl]-6-oxo-5-oxa-2,7-diazaspiro[3.4]octan-7-yl]-4H-pyrazino[2,3-b][1,4]oxazin-3-on;
6-[5-[3-[(4-Fluor-1-methyl-6,7-dihydro-5H-cyclopenta[c]pyridin-6-yl)amino]cyclobutyl]-2-oxo-1,3-oxazolidin-3-yl]-4H-pyrazino[2,3-b][1,4]oxazin-3-on, Isomer A;
6-[5-[3-[(4-Fluor-1-methyl-6,7-dihydro-5H-cyclopenta[c]pyridin-6-yl)amino]cyclobutyl]-2-oxo-1,3-oxazolidin-3-yl]-4H-pyrazino[2,3-b][1,4]oxazin-3-on, Isomer B;
6-[(5S)-5-[2-[1-(4-Fluor-1-methyl-6,7-dihydro-5H-cyclopenta[c]pyridin-6-yl)ethylamino]ethyl]-2-oxo-1,3-oxazolidin-3-yl]-4H-pyrazino[2,3-b][1,4]oxazin-3-on; Ameisensäure;
6-[(5R)-5-[2-[1-(4-Fluor-1-methyl-6,7-dihydro-5H-cyclopenta[c]pyridin-6-yl)ethylamino]ethyl]-2-oxo-1,3-oxazolidin-3-yl]-4H-pyrazino[2,3-b][1,4]oxazin-3-on; Ameisensäure;
6-[5-[2-[[4-Fluor-6-(2-imidazol-1-ylethoxy)-2,3-dihydro-1H-inden-2-yl]methylamino]ethyl]-2-oxo-1,3-oxazolidin-3-yl]-4H-pyrazino[2,3-b][1,4]oxazin-3-on;
6-[5-[3-[(4-Fluor-1-methyl-6,7-dihydro-5H-cyclopenta[c]pyridin-6-yl)amino]butyl]-2-oxo-1,3-oxazolidin-3-yl]-4H-pyrazino[2,3-b][1,4]oxazin-3-on, Isomer A;
6-[5-[3-[(4-Fluor-1-methyl-6,7-dihydro-5H-cyclopenta[c]pyridin-6-yl)amino]butyl]-2-oxo-1,3-oxazolidin-3-yl]-4H-pyrazino[2,3-b][1,4]oxazin-3-on, Isomer B;
6-[5-[2-[(4-Fluor-1-methyl-6,7-dihydro-5H-cyclopenta[c]pyridin-6-yl)methylamino]ethyl]-2-oxo-1,3,4-oxadiazol-3-yl]-4H-pyrazino[2,3-b][1,4]oxazin-3-on;
2-[[4-Fluor-2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-inden-5-yl]oxy]-N-methylacetamid;
6-[2-Oxo-5-[2-[[rac-1,2-trans-1-amino-4-chlor-2,3-dihydro-1H-inden-2-yl]methylamino]ethyl]-1,3,4-oxadiazol-3-yl]-4H-pyrazino[2,3-b][1,4]oxazin-3-on; Ameisensäure;
6-[(5S)-5-[2-[(4-Fluor-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
6-[(5R)-5-[2-[(4-Fluor-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
6-[5-[2-[(4-Fluor-1-methyl-6,7-dihydro-5H-cyclopenta[c]pyridin-6-yl)methylamino]ethyl]-5-methyl-2-oxo-1,3-oxazolidin-3-yl]-4H-pyrazino[2,3-b][1,4]oxazin-3-on;
6-[(5S)-5-[2-[[6-(2-Amino-2-methylpropoxy)-4-fluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[(5R)-5-[2-[[6-(2-Amino-2-methylpropoxy)-4-fluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[4-Fluor-6-[2-(triazol-2-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[4-Fluor-6-[2-(triazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
1-[2-[[7-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]ethyl]pyrrolidin-2,5-dion; Ameisensäure;
N-Cyclopropyl-2-[[7-fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid; Ameisensäure
6-[5-[2-[[4-Fluor-6-[2-(tetrazol-1-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[4-Fluor-6-[2-(tetrazol-2-yl)ethoxy]-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
2-[4-[2-Oxo-3-(3-oxo-4H-pyrazino[2,3-b][1,4]oxazin-6-yl)-1,3-oxazolidin-5-yl]piperidin-1-yl]-2,3-dihydro-1H-inden-4-carbonitril;
6-[5-[1-(4-Fluor-2,3-dihydro-1H-inden-2-yl)piperidin-4-yl]-2-oxo-1,3-oxazolidin-3-yl]-4H-pyrazino[2,3-b][1,4]oxazin-3-on;
6-[5-[2-[[4-Fluor-6-(2-hydroxypropoxy)-2,3-dihydro-1H-inden-2-yl]methylamino]ethyl]-2-oxo-1,3-oxazolidin-3-yl]-4H-pyrazino[2,3-b][1,4]oxazin-3-on; Hydrochlorid;
6-[5-[2-[(4-Fluor-1,3-dimethyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on; Ameisensäure;
6-[5-[2-[[4-Fluor-6-(2-methylsulfonylethoxy)-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[6-(1-Acetylazetidin-3-yl)oxy-4-fluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[4-Fluor-6-(1-methylsulfonylazetidin-3-yl)oxy-2,3-dihydro-1H-inden-2-yl]methylamino]ethyl]-2-oxo-1,3-oxazolidin-3-yl]-4H-pyrazino[2,3-b][1,4]oxazin-3-on; Hydrochlorid;
6-[8-[[6-[(2S)-2-Aminopropoxy]-4-fluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on; Ameisensäure;
6-[8-[[6-[(2R)-2-Aminopropoxy]-4-fluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on; Ameisensäure;
6-[5-[2-[[6-(Azetidin-3-yloxy)-4-fluor-2,3-dihydro-1H-inden-2-yl]methylamino]ethyl]-2-oxo-1,3-oxazolidin-3-yl]-4H-pyrazino[2,3-b][1,4]oxazin-3-on; Hydrochlorid;
6-[5-[2-[(rac-1,2-trans-1-Amino-4-chlor-2,3-dihydro-1H-inden-2-yl)methylamino]ethyl]-2-oxo-1,3-oxazol-3-yl]-4H-pyrazino[2,3-b][1,4]oxazin-3-on; Ameisensäure;
6-[(5R)-5-[(1R)-2-[(4-Fluor-1-methyl-6,7-dihydro-5H-cyclopenta[c]pyridin-6-yl)methylamino]-1-hydroxyethyl]-2-oxo-1,3-oxazolidin-3-yl]-4H-pyrazino[2,3-b][1,4]oxazin-3-on; Ameisensäure;
6-[5-[trans-3-[(4-Fluor-1-methyl-6,7-dihydro-5H-cyclopenta[c]pyridin-6-yl)methylamino]cyclobutyl]-2-oxo-1,3-oxazolidin-3-yl]-4H-pyrazino[2,3-b][1,4]oxazin-3-on;
6-[5-[cis-3-[(4-Fluor-1-methyl-6,7-dihydro-5H-cyclopenta[c]pyridin-6-yl)methylamino]cyclobutyl]-2-oxo-1,3-oxazolidin-3-yl]-4H-pyrazino[2,3-b][1,4]oxazin-3-on;
4-Fluor-3-methyl-6-[[2-[2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridin-1-carbonitril; Ameisensäure;
1-Methyl-6-[[2-[(5S)-2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridin-4-carbonitril;
1-Methyl-6-[[2-[(5R)-2-oxo-3-(3-oxo-4H-pyrazino[2,3-b][1,4]oxazin-6-yl)-1,3-oxazolidin-5-yl]ethylamino]methyl]-6,7-dihydro-5H-cyclopenta[c]pyridin-4-carbonitril;
2-[[7-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetonitril; Ameisensäure;
6-[5-[3-[(4-Fluor-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-on, Enantiomer A;
6-[5-[3-[(4-Fluor-1-methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-on, Enantiomer B;
6-[5-[3-[(1-Methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-on; Ameisensäure, Enantiomer A;
6-[5-[3-[(1-Methyl-6,7-dihydro-5H-cyclopenta[c]pyridin-6-yl)amino]propyl]-2-oxo-1,3-oxazol-3-yl]-4H-pyrazino[2,3-b][1,4]oxazin-3-on; Ameisensäure, Enantiomer B;
6-[5-[2-[(1-Methyl-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-on;
6-[5-[2-[(4-Fluor-6,7-dihydro-5H-cyclopenta[c]pyridin-6-yl)methylamino]ethyl]-2-oxo-1,3-oxazol-3-yl]-4H-pyrazino[2,3-b][1,4]oxazin-3-on;
6-[8-[(1-Chlor-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-on;
6-[8-[(1-Methyl-6,7-dihydro-5H-cyclopenta[c]pyridin-6-yl)methyl]-2-oxo-1-oxa-3,8-diazaspiro[4.5]decan-3-yl]-4H-pyrazino[2,3-b][1,4]oxazin-3-on;
6-[5-Methyl-2-oxo-5-[2-[(rac-1,2-trans-1-amino-4-chlor-2,3-dihydro-1H-inden-2-yl)methylamino]ethyl]-1,3-oxazolidin-3-yl]-4H-pyrazino[2,3-b][1,4]oxazin-3-on;
6-[5-[2-[(4-Fluor-1-methyl-6,7-dihydro-5H-cyclopenta[c]pyridin-6-yl)methylmethylamino]ethyl]-2-oxo-1,3-oxazolidin-3-yl]-4H-pyrazino[2,3-b][1,4]oxazin-3-on;
6-[8-[[6-(2-Amino-2-methylpropoxy)-4-fluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on;Ameisensäure, Enantiomer A;
6-[8-[[6-(2-Amino-2-methylpropoxy)-4-fluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on; Ameisensäure, Enantiomer B;
2-[[7-Fluor-2-[[2-[(5S)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid, Isomer A;
2-[[7-Fluor-2-[[2-[(5S)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid, Isomer B;
2-[[7-Fluor-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid, Isomer A;
2-[[7-Fluor-2-[[2-[(5R)-2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid, Isomer B;
2-[[7-Cyano-4-methyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid;
2-[[4-Cyano-7-methyl-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid;
6-[5-[2-[(2-Chlor-4-methyl-6,7-dihydro-5H-cyclopenta[d]pyrimidin-6-yl)methylamino]ethyl]-2-oxo-1,3-oxazolidin-3-yl]-4H-pyrido[3,2-b][1,4]oxazin-3-on,
6-(1,3-Oxazol-4-ylmethoxy)-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
6-[(3-Methyl-1,2,4-oxadiazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
6-[(1-Methylpyrazol-3-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
6-[(2-Methyltetrazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
6-[(1-Methylpyrazol-4-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
6-[(1-Methyltetrazol-5-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
6-[(4-Methyl-1,2,5-oxadiazol-3-yl)methoxy]-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril;
2-[[4,7-Difluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid;
2-[[4,7-Difluor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]-N-methylacetamid;
6-[5-[2-[[4,7-Difluor-5-(1H-pyrazol-3-ylmethoxy)-2,3-dihydro-1H-inden-2-yl]methylamino]ethyl]-2-oxo-1,3-oxazolidin-3-yl]-4H-pyrazino[2,3-b][1,4]oxazin-3-on;
6-[8-[[4,7-Difluor-5-(1H-pyrazol-3-ylmethoxy)-2,3-dihydro-1H-inden-2-yl]methyl]-2-oxo-1-oxa-3,8-diazaspiro[4.5]decan-3-yl]-4H-pyrazino[2,3-b][1,4]oxazin-3-on;
6-[5-[2-[(4-Fluor-3-methoxy-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
6-[5-[2-[(3-Chlor-4-fluor-1-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on;
2-[[rac-2,3-trans-3-Amino-7-chlor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]-N-methylacetamid; Ameisensäure ;
N-Methyl-2-[[rac-2,3-trans-3-amino-7-chlor-2-[[2-[2-oxo-3-(3-oxo-4H-pyrido[3,2-b][1,4]oxazin-6-yl)-1,3-oxazolidin-5-yl]ethylamino]methyl]-2,3-dihydro-1H-inden-5-yl]oxy]acetamid; Ameisensäure;
6-[(5S)-5-[2-[[(1R,2R)-1-Amino-4-chlor-2,3-dihydro-1H-inden-2-yl]methylamino]ethyl]-2-oxo-1,3-oxazolidin-3-yl]-4H-pyrazino[2,3-b][1,4]oxazin-3-on;
6-[(5S)-5-[2-[[(1S,2S)-1-Amino-4-chlor-2,3-dihydro-1H-inden-2-yl]methylamino]ethyl]-2-oxo-1,3-oxazolidin-3-yl]-4H-pyrazino[2,3-b][1,4]oxazin-3-on;
6-[(5R)-5-[2-[[(1R,2R)-1-Amino-4-chlor-2,3-dihydro-1H-inden-2-yl]methylamino]ethyl]-2-oxo-1,3-oxazolidin-3-yl]-4H-pyrazino[2,3-b][1,4]oxazin-3-on;
2-[[2-[2-Oxo-3-(3-oxo-4H-pyrazino[2,3-b][1,4]oxazin-6-yl)-1,3-oxazolidin-5-yl]ethylamino]methyl]-6-(1H-pyrazol-3-ylmethoxy)-2,3-dihydro-1H-inden-4-carbonitril; Ameisensäure;
2-[[2-[2-Oxo-3-(3-oxo-4H-pyrazino[2,3-b][1,4]oxazin-6-yl)-1,3-oxazolidin-5-yl]ethylamino]methyl]-6-(1H-pyrazol-4-ylmethoxy)-2,3-dihydro-1H-inden-4-carbonitril; Ameisensäure;
6-(1H-Imidazol-5-ylmethoxy)-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-4-carbonitril; Ameisensäure;
6-(2-Amino-2-methylpropoxy)-2-[[2-oxo-3-(3-oxo-4H-pyrazino[2,3-b][1,4]oxazin-6-yl)-1-oxa-3,8-diazaspiro[4.5]decan-8-yl]methyl]-2,3-dihydro-1H-inden-4-carbonitril;
6-[5-[2-[[5-[(1-Aminocyclopropyl)methoxy]-4,7-difluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[5-[2-[[5-[(1-Aminocyclopropyl)methoxy]-4,7-difluor-2,3-dihydro-1H-inden-2-yl]methylamino]ethyl]-2-oxo-1,3-oxazolidin-3-yl]-4H-pyrido[3,2-b][1,4]oxazin-3-on; Ameisensäure;
6-[8-[[5-[(1-Aminocyclopropyl)methoxy]-4,7-difluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on; Ameisensäure;
6-[5-[2-[[5-(2-Amino-2-methylpropoxy)-4,7-diffluor-2,3-dihydro-1H-inden-2-yl]methylamino]ethyl]-2-oxo-1,3-oxazolidin-3-yl]-4H-pyrazino[2,3-b][1,4]oxazin-3-on; Ameisensäure;
6-[8-[[5-(2-Amino-2-methylpropoxy)-4,7-difluor-2,3-dihydro-1H-inden-2-yl]methyl]-2-oxo-1-oxa-3,8-diazaspiro[4.5]decan-3-yl]-4H-pyrazino[2,3-b][1,4]oxazin-3-on; Ameisensäure;
2-[[4-Fluor-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid;
2-[[7-Cyano-2-[[2-[2-oxo-3-(3-oxo-4H-pyrazino[2,3-b][1,4]oxazin-6-yl)-1,3-oxazolidin-5-yl]ethylamino]methyl]-2,3-dihydro-1H-inden-5-yl]oxy]acetamid;
6-[5-[2-[[1-Methyl-3-(1H-pyrazol-3-ylmethoxy)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-on;
6-[5-[2-[(5,8-Dimethyl-5,7-diazatricyclo[7.3.0.02,6]dodeca-1,6,8-trien-11-yl)methylamino]ethyl]-2-oxo-1,3-oxazolidin-3-yl]-4H-pyrido[3,2-b][1,4]oxazin-3-on;
6-[4-[2-[(4-Fluor-1-methyl-6,7-dihydro-5H-cyclopenta[c]pyridin-6-yl)methylamino]ethyl]-2-oxopyrrolidin-1-yl]-4H-pyrido[3,2-b][1,4]oxazin-3-on;
6-[5-[2-(6,7-Dihydro-5H-cyclopenta[c]pyridin-6-ylmethylamino)ethyl]-2-oxo-1,3-oxazolidin-3-yl]-4H-pyrido[3,2-b][1,4]oxazin-3-on;
6-[5-[2-[[3-(Dimethylamino)-1-methyl-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[[1-Methyl-3-(methylamino)-6,7-dihydro-5H-cyclopenta[c]pyridin-6-yl]methylamino]ethyl]-2-oxo-1,3-oxazolidin-3-yl]-4H-pyrido[3,2-b][1,4]oxazin-3-on;
6-[5-[2-[(4-Methyl-6,7-dihydro-5H-cyclopenta[d]pyrimidin-6-yl)methylamino]ethyl]-2-oxo-1,3-oxazolidin-3-yl]-4H-pyrido[3,2-b][1,4]oxazin-3-on;
6-[(5R)-5-[2-[[(1S,2S)-1-Amino-4-chlor-2,3-dihydro-1H-inden-2-yl]methylamino]ethyl]-2-oxo-1,3-oxazolidin-3-yl]-4H-pyrazino[2,3-b][1,4]oxazin-3-on;
6-[5-[2-[(8-Methyl-5,7-diazatricyclo[7.3.0.02,6]dodeca-1,6,8-trien-11-yl)methylamino]ethyl]-2-oxo-1,3-oxazolidin-3-yl]-4H-pyrido[3,2-b][1,4]oxazin-3-on;
6-[5-[2-[(5,8-Dimethyl-5,7-diazatricyclo[7.3.0.02,6]dodeca-1,6,8-trien-11-yl)methylamino]ethyl]-2-oxo-1,3-oxazolidin-3-yl]-4H-pyrazino[2,3-b][1,4]oxazin-3-on;
2-[[2-[2-Oxo-3-(3-oxo-4H-pyrazino[2,3-b][1,4]oxazin-6-yl)-1,3-oxazolidin-5-yl]ethylamino]methyl]-6-(1H-triazol-5-ylmethoxy)-2,3-dihydro-1H-inden-4-carbonitril;
6-[5-[2-[[4-Fluor-1-(1H-pyrazol-5-yl)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-on;
6-[(5R)-5-[2-[[(6R)-4-Fluor-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-on
6-[(5S)-5-[2-[[(6R)-4-Fluor-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-on;
6-[(5S)-5-[2-[[(6S)-4-Fluor-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-on;
6-[(5R)-5-[2-[[(6S)-4-Fluor-1-methyl-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-on;
6-[5-[2-[(1-Chlor-4-fluor-3-methyl-6,7-dihydro-5H-cyclopenta[c]pyridin-6-yl)methylamino]ethyl]-2-oxo-1,3-oxazolidin-3-yl]-4H-pyrazino[2,3-b][1,4]oxazin-3-on; Ameisensäure;
6-[5-[2-[[4-Fluor-1-(1H-pyrazol-4-yl)-6,7-dihydro-5H-cyclopenta[c]pyridin-6-yl]methylamino]ethyl]-2-oxo-1,3-oxazolidin-3-yl]-4H-pyrazino[2,3-b][1,4]oxazin-3-on;
6-[5-[2-[[4-Fluor-1-(1H-imidazol-5-yl)-6,7-dihydro-SH-cyclopenta[c]pyridin-6-yl]methylamino]ethyl]-2-oxo-1,3-oxazolidin-3-yl]-4H-pyrazino[2,3-b][1,4]oxazin-3-on
und pharmazeutisch unbedenklichen Salzen davon.

10. Verbindung nach Anspruch 1, ausgewählt aus
6-[5-[2-[(1-Chlor-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-on;
6-[5-[2-[(4-Fluor-1-methyl-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-on;
2-[[7-Fluor-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N*-methylacetamid; Ameisensäure;
6-[5-[2-[(1-Methyl-6,7-dihydro-5*H*-cyclopenta[c]pyridin-6-yl)methylamino]ethyl]-2-oxo-1,3-oxazolidin-3-yl]-4*H*-pyrido[3,2-b][1,4]oxazin-3-on;
6-[2-Oxo-5-[2-[[*rac*-1,2-*trans*-1-amino-4-chlor-2,3-dihydro-1*H*-inden-2-yl]methylamino]ethyl]-1,3-oxazolidin-3-yl]-4*H*-pyrazino[2,3-b][1,4]oxazin-3-on; Ameisensäure;
6-[5-[2-[[6-[(1-Aminocyclopropyl)methoxy]-4-fluor-2,3-dihydro-1*H*-inden-2-yl]methylamino]ethyl]-2-oxo-1,3-oxazolidin-3-yl]-4*H*-pyrazino[2,3-b][1,4]oxazin-3-on;
6-[(1-Aminocyclopropyl)methoxy]-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-4-carbonitril;
6-(2-Aminopropoxy)-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-4-carbonitril; 2,2,2-Trifluoressigsäure;
6-[2-Oxo-5-[2-[[*rac*-1,2-*trans*-1-amino-4-chlor-2,3-dihydro-1*H*-inden-2-yl]methylamino]ethyl]-1,3-oxazolidin-3-yl]-4*H*-pyrido[3,2-b][1,4]oxazin-3-on;
6-[5-[2-[(1-Methyl-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-on; Ameisensäure;
6-[5-[2-[(4-Fluor-6,7-dihydro-5*H*-cyclopenta[c]pyridin-6-yl)methylamino]ethyl]-2-oxo-1,3-oxazolidin-3-yl]-4*H*-pyrazino[2,3-b][1,4]oxazin-3-on;
1-Methyl-6-[[2-[2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridin-4-carbonitril; 2,2,2-Trifluoressigsäure;
6-[5-[2-[(4-Fluor-1-methyl-6,7-dihydro-5*H*-cyclopenta[c]pyridin-6-yl)methylamino]ethyl]-2-oxo-1,3-oxazol-3-yl]-4*H*-pyrazino[2,3-b][1,4]oxazin-3-on;
6-[5-[3-[(4-Fluor-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-on; Ameisensäure;
2-[[7-Fluor-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamid;
2-[[7-Fluor-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N-*methylacetamid; Ameisensäure;
2-[[4,7-Difluor-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamid;
6-[8-[[6-[(2*S*)-2-Aminopropoxy]-4-fluor-2,3-dihydro-1*H*-inden-2-yl]methyl]-2-oxo-1-oxa-3,8-diazaspiro[4.5]decan-3-yl]-4*H*-pyrazino[2,3-b][1,4]oxazin-3-on; Ameisensäure;
1-Methyl-6-[[2-[(5*S*)-2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridin-4-carbonitril;
1-Methyl-6-[[2-[(5*R*)-2-oxo-3-(3-oxo-4*H*-pyrazino[2,3-b][1,4]oxazin-6-yl)-1,3-oxazolidin-5-yl]ethylamino]methyl]-6,7-dihydro-5*H*-cyclopenta[c]pyridin-4-carbonitril;
6-[5-[3-[(4-Fluor-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-on, Enantiomer A;
6-[5-[3-[(4-Fluor-1-methyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-on, Enantiomer B;
6-[5-[2-[(1-Methyl-6,7-dihydro-5*H*-cyclopenta[c]pyridin-6-yl)methylamino]ethyl]-2-oxo-1,3-oxazol-3-yl]-4*H*-pyrazino[2,3-b][1,4]oxazin-3-on;
2-[[7-Fluor-2-[[2-[(5*S*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamid, Isomer A;
2-[[7-Fluor-2-[[2-[(5*S*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamid, IsomerB;
2-[[7-Fluor-2-[[2-[(5*R*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamid, Isomer A;
2-[[7-Fluor-2-[[2-[(5*R*)-2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]acetamid, Isomer B;
2-[[4,7-Difluor-2-[[2-[2-oxo-3-(3-oxo-4*H*-pyrazino[2,3-b][1,4]oxazin-6-yl)-1,3-oxazolidin-5-yl]ethylamino]methyl]-2,3-dihydro-1*H*-inden-5-yl]oxy]-*N-*methylacetamid;
2-[[4,7-Difluor-2-[[2-[2-oxo-3-(3-oxo-4*H*-pyrido[3,2-b][1,4]oxazin-6-yl)-1,3-oxazolidin-5-yl]ethylamino]methyl]-2,3-dihydro-1*H*-inden-5-yl]oxy]-*N-*methylacetamid;
6-[(5*R*)-5-[2-[[(1*R*,2*R*)-1-Amino-4-chlor-2,3-dihydro-1*H*-inden-2-yl]methylamino]ethyl]-2-oxo-1,3-oxazolidin-3-yl]-4*H*-pyrazino[2,3-b][1,4]oxazin-3-on;
6-[(5*R*)-5-[2-[[(1*S*,2*S*)-1-Amino-4-chlor-2,3-dihydro-1*H*-inden-2-yl]methylamino]ethyl]-2-oxo-1,3-oxazolidin-3-yl]-4*H*-pyrazino[2,3-b][1,4]oxazin-3-on;
6-[(5*R*)-5-[2-[[(6*R*)-4-Fluor-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-on;
6-[(5*S*)-5-[2-[[(6*R*)-4-Fluor-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-on;
6-[(5S)-5-[2-[[(6S)-4-Fluor-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-on;
6-[(5R)-5-[2-[[(6S)-4-Fluor-1-methyl-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-on;
6-[5-[2-[[4-Fluor-1-(1*H*-pyrazol-4-yl)-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-on;
6-[5-[2-[[4-Fluor-1-(1*H*-imidazol-5-yl)-6,7-dihydro-5*H*-cyclopenta[c]pyridin-6-yl]methylamino]ethyl]-2-oxo-1,3-oxazolidin-3-yl]-4*H*-pyrazino[2,3-b][1,4]oxazin-3-on;
oder pharmazeutisch unbedenklichen Salzen davon.

11. Verbindung nach einem der Ansprüche 1 bis 9 zur Verwendung als therapeutisch wirksame Substanz.

12. Pharmazeutische Zusammensetzung, umfassend eine Verbindung nach einem von Anspruch 1 bis 10 und einen therapeutisch inerten Träger.

13. Verbindung nach einem der Ansprüche 1 bis 10 oder ein pharmazeutisch unbedenkliches Salz davon zur Verwendung als Antibiotikum.

14. Verbindung nach einem der Ansprüche 1 bis 10 zur Verwendung bei der Behandlung oder Prophylaxe einer Prophylaxe einer bakteriellen Infektion.

15. Verbindung nach einem der Ansprüche 1 bis 10 zur Verwendung bei der Behandlung oder Prophylaxe von Prophylaxe von Infektionen und daraus resultierenden Krankheiten, die durch *Escherichia coli* verursacht werden.

## Revendications

1. Composé représenté par la formule générale (I) suivante 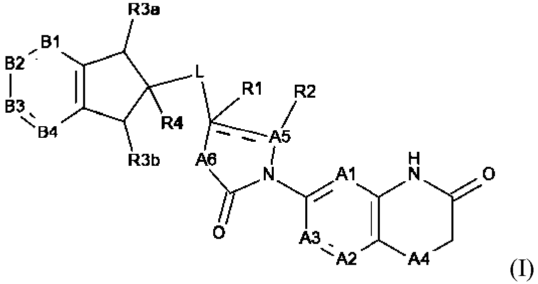 dans lequel
A¹ est choisi parmi
i) -N-, et
ii) -CH- ;
A² est choisi parmi
i) -N-, et
ii) -CH- ;
A³ est choisi parmi
i) -N-, et
ii) -CH- ;
A⁴ est choisi parmi
i) -O-, et
ii) -S- ;
A⁵ est choisi parmi
i) -N-, et
ii) -CH- ;
A⁶ est choisi parmi
i) -O-, et
ii) -CH₂- ;
-̅ -̅ représente une liaison simple ou double ;
B¹ est choisi parmi
i) -N-, et
ii) -CR⁵- ;
B² est choisi parmi
i) -N-, et
ii) -CR⁶- ;
dans lequel lorsque B¹ représente CR⁵ et B² représente CR⁶, R⁵ et R⁶ peuvent être liés l'un à l'autre pour former un cycle à 4 à 6 chaînons éventuellement substitué par R^{a} et/ou R^{b} ;
R^{a} et R^{b} sont indépendamment choisis parmi
i) COOR^{a1}, et
ii) un alkyle en C₁₋₆ ;
R^{a1} est choisi parmi
i) H, et
ii) un alkyle en C₁₋₆ ;
B³ est choisi parmi
i) -N-, et
ii) -CR⁷- ;
B⁴ est choisi parmi
i) -N-, et
ii) -CR⁸- ;
R¹ est choisi parmi
i) H,
ii) NH₂,
iii) OH, et
iv) un alkyle en C₁₋₆ éventuellement substitué par R^{c},
ou R¹, R⁹ et L₂ peuvent être liés les uns aux autres pour former un cycle à 4 à 6 chaînons éventuellement substitué par R^{d},
ou R¹, L₂ et R^{w} peuvent être liés les uns aux autres pour former un cycle à 4 à 6 chaînons éventuellement substitué par R^{d} ;
R^{c} est choisi parmi
i) OH, et
ii) NH₂ ;
R^{d} est choisi parmi
i) (CH2)nNH₂, et
ii) (CH2)nOH ;
n représente un nombre entier de 0 à 6 ;
R² est choisi parmi
i) H, et
ii) un alkyle en C₁₋₆ éventuellement substitué par R^{c},
ou R², L₂ et R^{w} peuvent être liés les uns aux autres pour former un cycle à 4 à 6 chaînons éventuellement substitué par R^{d} ;
dans lequel
lorsque -̅ -̅ représente une liaison double, R₁ et R₂ ne représentent rien ;
R^{3a} est choisi parmi
i) H,
ii) NH₂, et
iii) OH ;
R^{3b} est choisi parmi
i) H,
ii) NH₂, et
iii) OH ;
dans lequel l'un de R^{3a} et R^{3b} représente H ;
R⁴ est choisi parmi
i) H,
ii) OH, et
iii) NH₂ ;
R⁵ est choisi parmi
i) H,
ii) un halogène,
iii) un cyano,
iv) un alkyle en C₁₋₆
v) un hétéroaryle à 4 à 6 chaînons éventuellement substitué par un alkyle en C₁₋₆,
vi) OR^{e},
vii) SO₂R^{f},
viii) SO₂NR^{e}R^{f}, et
ix) NR^{e}R^{f} ;
R^{e} et R^{f} sont indépendamment choisis parmi
i) H, et
ii) un alkyle en C₁₋₆ ;
R⁶ est choisi parmi
i) H,
ii) un halogène,
iii) un alkyle en C₁₋₆,
iv) OR^{g},
v) NR^{h}Rⁱ, et
vi) un cyano ;
R^{g} est choisi parmi
i) H,
ii) -(CH₂)ₘ-R^{g1},
iii) 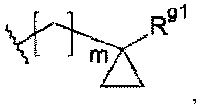
iv) 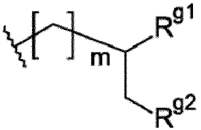
v) 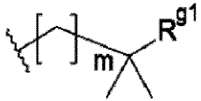 et
vi) 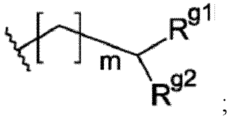
m représente un nombre entier de 0 à 6 ;
R^{g1} et R^{g2} sont indépendamment choisis parmi
i) H,
ii) OR^{j},
iii) NR^{k}R^{l},
iv) SO₂R^{m},
v) CONRⁿR^{o},
vi) NR^{p}COR^{q},
vii) NR^{r}SO₂R^{s},
viii) COOR^{t},
ix) un alkyle en C₁₋₆ éventuellement substitué par un alcoxy en C₁₋₆
x) un hétérocycloalkyle éventuellement substitué par R^{u}
xi) un cyano, et
xii) un hétéroaryle ;
R^{j}, R^{k}, R^{l}, R^{m}, Rⁿ, R^{o}, R^{p}, R^{q}, R^{r}, R^{s}, R^{t} et R^{u} sont indépendamment choisis parmi
i) H,
ii) un alkyle en C₁₋₆ éventuellement substitué par un alcoxy en C₁₋₆
iii) un cycloalkyle en C₃₋₈,
iv) NH₂,
v) un hétérocycloalkyle,
vi) -S(O)₂-alkyle en C₁₋₆,
vii) -C(O)-alkyle en C₁₋₆, et
viii) -O-alkyle en C₁₋₆ ;
R^{h} et Rⁱ sont indépendamment choisis parmi
i) H, et
ii) un alkyle en C₁₋₆ ;
R⁷ est choisi parmi
i) H,
ii) un halogène,
iii) un alkyle en C₁₋₆, et
iv) un cyano ;
R⁸ est choisi parmi
i) H,
ii) un halogène,
iii) un cyano
iv) un alkyle en C₁₋₆, et
v) un hétéroaryle éventuellement substitué par un alkyle en C₁₋₆ ;
L est choisi parmi
i) -L₁-NR⁹-L₂-, et
ii) -L₁-hétérocycloalkylène-L₂- ;
L₁ est choisi parmi
i) (CH₂)x éventuellement substitué par R^{v},
ii) un hétérocycloalkylène éventuellement substitué par R^{v}, et
iii) un cycloalkylène en C₃₋₈ éventuellement substitué par R^{v} ;
x représente un nombre entier de 0 à 6 ;
R⁹ et R¹⁰ sont indépendamment choisis parmi
i) H, et
ii) un alkyle en C₁₋₆ éventuellement substitué par R^{v},
ou R¹, R⁹ et L₂ peuvent être liés les uns aux autres pour former un cycle à 4 à 6 chaînons éventuellement substitué par R^{d} ;
R^{v} est choisi parmi
i) OH,
ii) NH₂, et
iii) un alkyle en C₁₋₆ éventuellement substitué par un hydroxy ;
L₂ est choisi parmi
i) (CH₂)y éventuellement substitué par R^{w} et/ou R^{x},
ii) un cycloalkylène en C₃₋₈ éventuellement substitué par R^{w} et/ou R^{x},
iii) un hétérocycloalkylène éventuellement substitué par R^{w} et/ou R^{x},
iv) (CH₂)yNR¹⁰- éventuellement substitué par R^{w} et/ou R^{x},
v) un cycloalkylène en C₃₋₈-NR¹⁰- éventuellement substitué par R^{w} et/ou R^{x}, et
vi) un hétérocycloalkylène-NR¹⁰- éventuellement substitué par R^{w} et/ou R^{x}
ou R¹, R⁹ et L₂ peuvent être liés les uns aux autres pour former un cycle à 4 à 6 chaînons éventuellement substitué par R^{d},
ou R¹, L₂ et R^{w} peuvent être liés les uns aux autres pour former un cycle à 4 à 6 chaînons éventuellement substitué par R^{d},
ou R², L₂ et R^{w} peuvent être liés les uns aux autres pour former un cycle à 4 à 6 chaînons éventuellement substitué par R^{d} ;
y représente un nombre entier de 0 à 6 ;
R^{w} et R^{x} sont indépendamment choisis parmi
i) OH,
ii) NH₂, et
iii) un alkyle en C₁₋₆ éventuellement substitué par un hydroxy ;
ou R¹, L₂ et R^{w} peuvent être liés les uns aux autres pour former un cycle à 4 à 6 chaînons éventuellement substitué par R^{d},
ou R², L₂ et R^{w} peuvent être liés les uns aux autres pour former un cycle à 4 à 6 chaînons éventuellement substitué par R^{d} ;
ou des sels pharmaceutiquement acceptables de celui-ci.

2. Composé ou un sel pharmaceutiquement acceptable de celui-ci selon la revendication 1,
dans lequel
A¹ est choisi parmi
i) -N-, et
ii) -CH- ;
A² est choisi parmi
i) -N-, et
ii) -CH- ;
A³ représente CH ;
A⁴ est choisi parmi
i) -O-, et
ii) -S- ;
A⁵ est choisi parmi
i) -N-, et
ii) -CH- ;
A⁶ est choisi parmi
i) -O-, et
ii) -CH₂- ;
-̅ -̅ représente une liaison simple ou double ;
B¹ est choisi parmi
i) -N-, et
ii) -CR⁵- ;
B² est choisi parmi
i) -N-, et
ii) -CR⁶- ;
dans lequel lorsque B¹ représente CR⁵ et B² représente CR⁶, R⁵ et R⁶ peuvent être liés l'un à l'autre pour former un cycle à 4 à 6 chaînons tel que ci-dessous, 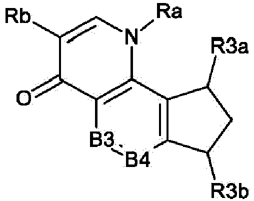
R^{a} et R^{b} sont indépendamment choisis parmi
i) COOR^{a1}, et
ii) un alkyle en C₁₋₆ ;
R^{a1} est choisi parmi
i) H, et
ii) un alkyle en C₁₋₆ ;
B³ est choisi parmi
i) -N-, et
ii) -CR⁷- ;
B⁴ est choisi parmi
i) -N-, et
ii) -CR⁸- ;
R¹ est choisi parmi
i) H, et
ii) un alkyle en C₁₋₆ éventuellement substitué par R^{c},
ou R¹, R⁹ et L₂ peuvent être liés les uns aux autres pour former un cycle à 4 à 6 chaînons éventuellement substitué par R^{d},
ou R¹, L₂ et R^{w} peuvent être liés les uns aux autres pour former un cycle à 4 à 6 chaînons éventuellement substitué par R^{d} ;
R^{c} représente NH₂ ;
R^{d} représente (CH2)nNH₂ ;
n représente un nombre entier de 0 à 3 ;
R² représente H ;
ou R², L₂ et R^{w} peuvent être liés les uns aux autres pour former un cycle à 4 à 6 chaînons éventuellement substitué par R^{d}
dans lequel
lorsque -̅ -̅^{.} représente une liaison double, R₁ et R₂ ne représentent rien ;
R^{3a} est choisi parmi
i) H,
ii) NH₂, et
iii) OH ;
R^{3b} est choisi parmi
i) H,
ii) NH₂, et
iii) OH ;
dans lequel l'un de R^{3a} et R^{3b} représente H,
R⁴ est choisi parmi
i) H, et
ii) NH₂ ;
R⁵ est choisi parmi
i) H,
ii) un halogène,
iii) un cyano,
iv) un alkyle en C₁₋₆, et
v) OR^{e} ;
R^{e} représente un alkyle en C₁₋₆ ;
R⁶ est choisi parmi
i) H,
ii) un alkyle en C₁₋₆, et
iii) OR^{g} ;
R^{g} est choisi parmi
i) H,
ii) -(CH₂)ₘ-R^{g1},
iii) 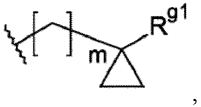
iv) 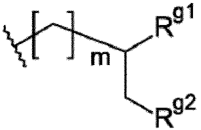
v) 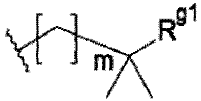 et
vi) 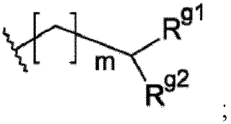
m représente un nombre entier de 0 à 3 ;
R^{g1} et R^{g2} sont indépendamment choisis parmi
i) H,
ii) OR^{j},
iii) NR^{k}R^{l},
iv) SO₂R^{m},
v) CONRⁿR^{o},
vi) NR^{p}COR^{q},
vii) NR^{r}SO₂R^{s},
viii) COOR^{t},
ix) un alkyle en C₁₋₆ éventuellement substitué par un alcoxy en C₁₋₆
x) un hétérocycloalkyle éventuellement substitué par R^{u}, et
xi) un cyano
R^{j}, R^{k}, R^{l}, R^{m}, Rⁿ, R^{o}, R^{p}, R^{q}, R^{r}, R^{s}, R^{t} et R^{u} sont indépendamment choisis parmi
i) H,
ii) un alkyle en C₁₋₆ éventuellement substitué par un alcoxy en C₁₋₆, et
iii) NH₂ ;
R⁷ est choisi parmi
i) H, et
ii) un alkyle en C₁₋₆ ;
R⁸ est choisi parmi
i) H,
ii) un halogène,
iii) un cyano
iv) un alkyle en C₁₋₆, et
v) un hétéroaryle éventuellement substitué par un alkyle en C₁₋₆ ;
L est choisi parmi
i) -L₁-NR⁹-L₂-, et
ii) -L₁-azétidinylène-L₂-
L₁ est choisi parmi
i) (CH₂)x éventuellement substitué par R^{v}, et
ii) un hétérocycloalkylène éventuellement substitué par R^{v} ;
x représente un nombre entier de 0 à 3 ;
R⁹ et R¹⁰ représentent H ;
ou R¹, R⁹ et L₂ peuvent être liés les uns aux autres pour former un cycle à 4 à 6 chaînons éventuellement substitué par R^{d} ;
R^{v} représente NH₂ ;
L₂ est choisi parmi
i) (CH₂)y éventuellement substitué par R^{w} et/ou R^{x},
ii) un cycloalkylène en C₃₋₈ éventuellement substitué par R^{w} et/ou R^{x},
iii) un hétérocycloalkylène éventuellement substitué par R^{w} et/ou R^{x},
iv) (CH₂)yNR¹⁰- éventuellement substitué par R^{w} et/ou R^{x},
v) un cycloalkylène en C₃₋₈-NR¹⁰- éventuellement substitué par R^{w} et/ou R^{x}, et
vi) un hétérocycloalkylène-NR¹⁰- éventuellement substitué par R^{w} et/ou R^{x} ;
ou R¹, R⁹ et L₂ peuvent être liés les uns aux autres pour former un cycle à 4 à 6 chaînons éventuellement substitué par R^{d},
ou R¹, L₂ et R^{w} peuvent être liés les uns aux autres pour former un cycle à 4 à 6 chaînons éventuellement substitué par R^{d},
ou R², L₂ et R^{w} peuvent être liés les uns aux autres pour former un cycle à 4 à 6 chaînons éventuellement substitué par R^{d} ;
y représente un nombre entier de 0 à 3 ;
R^{w} et R^{x} représentent OH ;
ou R¹, L₂ et R^{w} peuvent être liés les uns aux autres pour former un cycle à 4 à 6 chaînons éventuellement substitué par R^{d},
ou R², L₂ et R^{w} peuvent être liés les uns aux autres pour former un cycle à 4 à 6 chaînons éventuellement substitué par R^{d} :

3. Composé ou un sel pharmaceutiquement acceptable de celui-ci selon la revendication 1,
dans lequel
A¹ est choisi parmi
i) -N-, et
ii) -CH- ;
A² est choisi parmi
i) -N-, et
ii) -CH- ;
A³ représente CH ;
A⁴ est choisi parmi
i) -O-, et
ii) -S- ;
A⁵ représente CH ;
A⁶ représente O ;
-̅ -̅ représente une liaison simple,
B¹ est choisi parmi
i) -N-, et
ii) -CR⁵- ;
B² est choisi parmi
i) -N-, et
ii) -CR⁶- ;
B³ est choisi parmi
i) -N-, et
ii) -CR⁷- ;
B⁴ est choisi parmi
i) -N-, et
ii) -CR⁸- ;
R¹ représente H ;
ou R¹, R⁹ et L₂ peuvent être liés les uns aux autres pour former un cycle à 4 à 6 chaînons éventuellement substitué par R^{d},
ou R¹, L₂ et R^{w} peuvent être liés les uns aux autres pour former un cycle à 4 à 6 chaînons éventuellement substitué par R^{d} ;
R^{d} représente (CH2)nNH₂, et
n représente un nombre entier de 0 à 3 ;
R² représente H ;
ou R², L₂ et R^{w} peuvent être liés les uns aux autres pour former un cycle à 4 à 6 chaînons éventuellement substitué par R^{d}
R^{3a} est choisi parmi
i) H,
ii) NH₂, et
iii) OH ;
R^{3b} est choisi parmi
i) H,
ii) NH₂, et
iii) OH ;
dans lequel l'un de R^{3a} et R^{3b} représente H,
R⁴ représente H ;
R⁵ est choisi parmi
i) H,
ii) un halogène,
iii) un cyano,
iv) un alkyle en C₁₋₆,
v) OR^{e}, et
R^{e} représente un alkyle en C₁₋₆ ;
R⁶ est choisi parmi
i) H,
ii) un alkyle en C₁₋₆, et
iii) OR^{g} ;
R^{g} est choisi parmi
i) H,
ii) -(CH₂)ₘ-R^{g1},
iii) 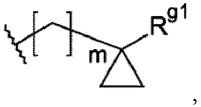
iv) 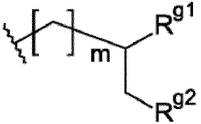
v) 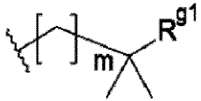 et
vi) 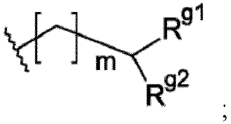
m représente un nombre entier de 0 à 3 ;
R^{g1} et R^{g2} sont indépendamment choisis parmi
i) H,
ii) OR^{j},
iii) NR^{k}R^{l},
iv) un alkyle en C₁₋₆ éventuellement substitué par un alcoxy en C₁₋₆, et
v) un hétérocycloalkyle éventuellement substitué par R^{u} ;
R^{j}, R^{k}, R^{l} et R^{u} sont indépendamment choisis parmi
i) H,
ii) un alkyle en C₁₋₆ éventuellement substitué par un alcoxy en C₁₋₆, et
iii) NH₂ ;
R⁷ est choisi parmi
i) H, et
ii) un alkyle en C₁₋₆ ;
R⁸ est choisi parmi
i) H,
ii) un halogène,
iii) un cyano,
iv) un alkyle en C₁₋₆, et
v) un hétéroaryle éventuellement substitué par un alkyle en C₁₋₆ ;
L représente -L₁-NR⁹-L₂- ;
L₁ représente (CH₂)x éventuellement substitué par R^{v} ;
x représente un nombre entier de 0 à 3 ;
R⁹ et R¹⁰ représentent H
ou R¹, R⁹ et L₂ peuvent être liés les uns aux autres pour former un cycle à 4 à 6 chaînons éventuellement substitué par R^{d} ;
R^{v} représente NH₂
L₂ est choisi parmi
i) (CH₂)y éventuellement substitué par R^{w} et/ou R^{x},
ii) un cycloalkylène en C₃₋₈ éventuellement substitué par R^{w} et/ou R^{x},
iii) un hétérocycloalkylène éventuellement substitué par R^{w} et/ou R^{x},
iv) (CH₂)yNR¹⁰- éventuellement substitué par R^{w} et/ou R^{x},
v) un cycloalkylène en C₃₋₈-NR¹⁰- éventuellement substitué par R^{w} et/ou R^{x}, et
vi) un hétérocycloalkylène-NR¹⁰- éventuellement substitué par R^{w} et/ou R^{x} ;
ou R¹, R⁹ et L₂ peuvent être liés les uns aux autres pour former un cycle à 4 à 6 chaînons éventuellement substitué par R^{d},
ou R¹, L₂ et R^{w} peuvent être liés les uns aux autres pour former un cycle à 4 à 6 chaînons éventuellement substitué par R^{d},
ou R², L₂ et R^{w} peuvent être liés les uns aux autres pour former un cycle à 4 à 6 chaînons éventuellement substitué par R^{d} ;
y représente un nombre entier de 0 à 3 ;
R^{w} et R^{x} représentent OH,
ou R¹, L₂ et R^{w} peuvent être liés les uns aux autres pour former un cycle à 4 à 6 chaînons éventuellement substitué par R^{d},
ou R², L₂ et R^{w} peuvent être liés les uns aux autres pour former un cycle à 4 à 6 chaînons éventuellement substitué par R^{d} :

4. Composé ou un sel pharmaceutiquement acceptable de celui-ci selon la revendication 1,
dans lequel
A¹ est choisi parmi
i) -N-, et
ii) -CH- ;
A² est choisi parmi
i) -N-, et
ii) -CH- ;
A³ représente CH ;
A⁴ est choisi parmi
i) -O-, et
ii) -S- ;
A⁵ représente CH ;
A⁶ représente O ;
-̅ -̅ représente une liaison simple ;
B¹ est choisi parmi
i) -N-, et
ii) -CR⁵- ;
B² est choisi parmi
i) -N-, et
ii) -CR⁶- ;
B³ est choisi parmi
i) -N-, et
ii) -CR⁷- ;
B⁴ est choisi parmi
i) -N-, et
ii) -CR⁸- ;
R¹ représente H ;
ou R¹, R⁹ et L₂ peuvent être liés les uns aux autres pour former un cycle à 4 à 6 chaînons tel que ci-dessous, 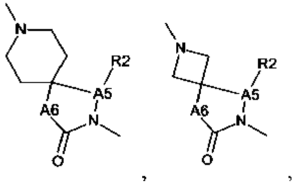
dans lequel le cycle est éventuellement substitué par R^{d},
ou R¹, L₂ et R^{w} peuvent être liés les uns aux autres pour former un cycle à 4 à 6 chaînons tel que ci-dessous, 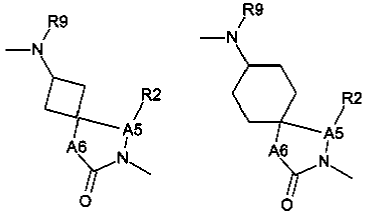
dans lequel le cycle est éventuellement substitué par R^{d} ;
R^{d} représente (CH2)nNH₂ ;
n représente un nombre entier de 0 à 1 ;
R² représente H ;
ou R², L₂ et R^{w} peuvent être liés les uns aux autres pour former un cycle à 4 à 6 chaînons tel que ci-dessous, 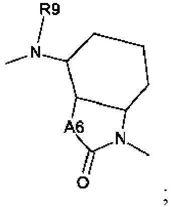
R^{3a} est choisi parmi
i) H,
ii) NH₂, et
iii) OH ;
R^{3b} est choisi parmi
i) H,
ii) NH₂, et
iii) OH ;
dans lequel l'un de R^{3a} et R^{3b} représente H,
R⁴ représente H
R⁵ est choisi parmi
i) H,
ii) un halogène,
iii) un cyano,
iv) un alkyle en C₁₋₆, et
v) OR^{e}, et
R^{e} représente un alkyle en C₁₋₆ ;
R⁶ est choisi parmi
i) H,
ii) un alkyle en C₁₋₆, et
iii) OR⁹ ;
R^{g} est choisi parmi
i) H,
ii) -(CH₂)ₘ-R^{g1},
iii) 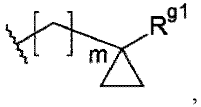
iv) 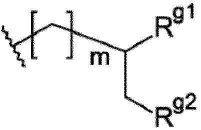
v) 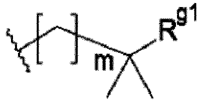 et
vi) 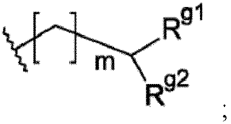
m représente un nombre entier de 0 à 3 ;
R^{g1} et R^{g2} sont indépendamment choisis parmi
i) H,
ii) OR^{j},
iii) NR^{k}R^{l},
iv) un alkyle en C₁₋₆ éventuellement substitué par un alcoxy en C₁₋₆
v) un pyrrolidinyle éventuellement substitué par R^{u}
vi) un morpholinyle éventuellement substitué par R^{u}
vii) un oxétanyle éventuellement substitué par R^{u}
viii) un 3-oxo-morpholinyle éventuellement substitué par R^{u},
ix) un 2-oxo-pyrrolidinyle éventuellement substitué par R^{u}
x) un azétidinyle éventuellement substitué par R^{u}
xi) un pipérazinyle éventuellement substitué par R^{u}, et
xii) un 2-oxo-pipérazinyle éventuellement substitué par R^{u} ;
R^{j}, R^{k}, R^{l} et R^{u} sont indépendamment choisis parmi
i) H,
ii) un alkyle en C₁₋₆ éventuellement substitué par un alcoxy en C₁₋₆, et
iii) NH₂
R⁷ est choisi parmi
i) H, et
ii) un alkyle en C₁₋₆ ;
R⁸ est choisi parmi
i) H,
ii) un halogène,
iii) un cyano
iv) un alkyle en C₁₋₆, et
v) un thiazole éventuellement substitué par un alkyle en C₁₋₆ ;
L représente -L₁-NR⁹-L₂- ;
L₁ représente (CH₂)x éventuellement substitué par R^{v} ;
x représente un nombre entier de 0 à 3 ;
R⁹ et R¹⁰ représentent H ;
ou R¹, R⁹ et L₂ peuvent être liés les uns aux autres pour former un cycle à 4 à 6 chaînons tel que ci-dessous, 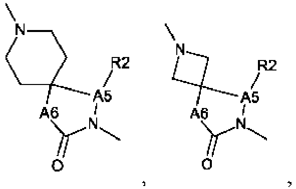
dans lequel le cycle est éventuellement substitué par R^{d} ;
R^{v} représente NH₂ ;
L₂ est choisi parmi
i) (CH₂)y éventuellement substitué par R^{w} et/ou R^{x},
ii) un cycloalkylène en C₃₋₈ éventuellement substitué par R^{w} et/ou R^{x},
iii) (CH₂)yNR¹⁰- éventuellement substitué par R^{w} et/ou R^{x}, et
iv) un cycloalkylène en C₃₋₈-NR¹⁰- éventuellement substitué par R^{w} et/ou R^{x}
ou R¹, R⁹ et L₂ peuvent être liés les uns aux autres pour former un cycle à 4 à 6 chaînons tel que ci-dessous, 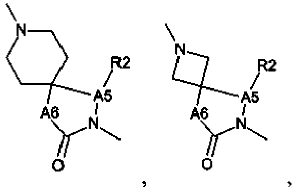
dans lequel le cycle est éventuellement substitué par R^{d},
ou R¹, L₂ et R^{w} peuvent être liés les uns aux autres pour former un cycle à 4 à 6 chaînons tel que ci-dessous, 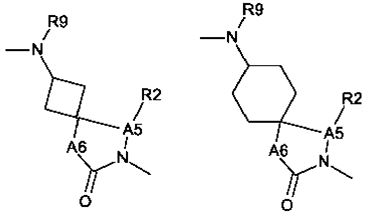
dans lequel le cycle est éventuellement substitué par R^{d},
ou R², L₂ et R^{w} peuvent être liés les uns aux autres pour former un cycle à 4 à 6 chaînons tel que ci-dessous, 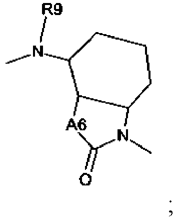
y représente un nombre entier de 0 à 3 ;
R^{w} et R^{x} représentent OH
ou R¹, L₂ et R^{w} peuvent être liés les uns aux autres pour former un cycle à 4 à 6 chaînons tel que ci-dessous, 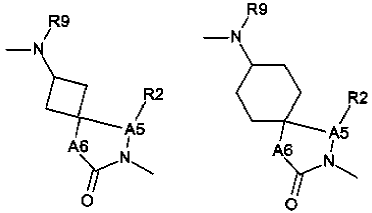
dans lequel le cycle est éventuellement substitué par R^{d},
ou R², L₂ et R^{w} peuvent être liés les uns aux autres pour former un cycle à 4 à 6 chaînons tel que ci-dessous,

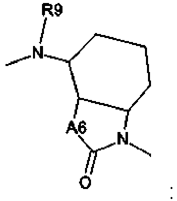

5. Composé ou un sel pharmaceutiquement acceptable de celui-ci selon l'une quelconque des revendications 1 à 4, dans lequel A₁ représente -N-.

6. Composé ou un sel pharmaceutiquement acceptable de celui-ci selon l'une quelconque des revendications 1 à 5, dans lequel A² représente -CH-.

7. Composé ou un sel pharmaceutiquement acceptable de celui-ci selon l'une quelconque des revendications 1 à 6, dans lequel A⁴ représente -O-.

8. Composé ou un sel pharmaceutiquement acceptable de celui-ci selon l'une quelconque des revendications 1 à 7, dans lequel R^{3a} et R^{3b} représentent H.

9. Composé selon la revendication 1, choisi parmi
la 6-[5-[2-[(4-chloro-2,3-dihydro-1H-indén-2-yl)méthylamino]éthyl]-2-oxo-1,3-oxazolidin-3-yl]-4H-pyrido[3,2-b][1,4]oxazin-3-one ;
la 6-[5-[3-[(4-chloro-2,3-dihydro-1H-indén-2-yl)méthylamino]propyl]-2-oxo-1,3-oxazolidin-3-yl]-4H-pyrido[3,2-b][1,4]oxazin-3-one ;
la 6-[5-[2-[(4-chloro-2,3-dihydro-1H-indén-2-yl)amino]éthyl]-2-oxo-1,3-oxazolidin-3-yl]-4H-pyrido[3,2-b][1,4]oxazin-3-one ;
la 6-[5-[3-[(4-chloro-2,3-dihydro-1H-indén-2-yl)amino]propyl]-2-oxo-1,3-oxazolidin-3-yl]-4H-pyrido[3,2-b][1,4]oxazin-3-one ;
le 2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[5-[2-[(4-chloro-2,3-dihydro-1H-indén-2-yl)méthylamino]éthyl]-2-oxo-1,3-oxazolidin-3-yl]-4H-pyrido[3,2-b][1,4]oxazin-3-one ;
la 6-[5-[3-[(4-fluoro-2,3-dihydro-1H-indén-2-yl)amino]propyl]-2-oxo-1,3-oxazolidin-3-yl]-4H-pyrido[3,2-b][1,4]oxazin-3-one ;
la 6-[5-[3-(2,3-dihydro-1H-indén-2-ylamino)propyl]-2-oxo-1,3-oxazolidin-3-yl]-4H-pyrido[3,2-b][1,4]oxazin-3-one ;
la 6-[5-[3-[(5-chloro-2,3-dihydro-1H-indén-2-yl)amino]propyl]-2-oxo-1,3-oxazolidin-3-yl]-4H-pyrido[3,2-b][1,4]oxazin-3-one ;
la 6-[5-[2-[(5-chloro-2,3-dihydro-1H-indén-2-yl)amino]éthyl]-2-oxo-1,3-oxazolidin-3-yl]-4H-pyrido[3,2-b][1,4]oxazin-3-one ;
la 6-[5-[3-[(5-fluoro-2,3-dihydro-1H-indén-2-yl)amino]propyl]-2-oxo-1,3-oxazolidin-3-yl]-4H-pyrido[3,2-b][1,4]oxazin-3-one ;
le 2-[3-[2-oxo-3-(3-oxo-4H-pyrido[3,2-b][1,4]oxazin-6-yl)-1,3-oxazolidin-5-yl]propylamino]-2,3-dihydro-1H-indène-4-carbonitrile ;
le 2-[[trans-3-[2-oxo-3-(3-oxo-4H-pyrido[3,2-b][1,4]oxazin-6-yl)-1,3-oxazolidin-5-yl]cyclobutyl]amino]-2,3-dihydro-1H-indène-4-carbonitrile ;
la 6-[5-[2-[(4-chloro-2,3-dihydro-1H-indén-2-yl)méthylamino]éthyl]-2-oxo-1,3-oxazolidin-3-yl]-4H-1,4-benzoxazin-3-one ;
la 6-[5-[cis-3-[(4-chloro-2,3-dihydro-1H-indén-2-yl)amino]cyclobutyl]-2-oxo-1,3-oxazolidin-3-yl]-4H-pyrido[3,2-b][1,4]oxazin-3-one ;
la 6-[5-[trans-3-[(4-chloro-2,3-dihydro-1H-indén-2-yl)amino]cyclobutyl]-2-oxo-1,3-oxazolidin-3-yl]-4H-pyrido[3,2-b][1,4]oxazin-3-one ;
le 2-[[2-[2-oxo-3-(3-oxo-4H-1,4-benzoxazin-6-yl)-1,3-oxazolidin-5-yl]éthylamino]méthyl]-2,3-dihydro-1H-indène-4-carbonitrile ;
le 2-[[cis-3-[2-oxo-3-(3-oxo-4H-pyrido[3,2-b][1,4]oxazin-6-yl)-1,3-oxazolidin-5-yl]cyclobutyl]amino]-2,3-dihydro-1H-indène-4-carbonitrile ;
la 6-[5-[2-[2-(4-chloro-2,3-dihydro-1H-indén-2-yl)éthylamino]éthyl]-2-oxo-1,3-oxazolidin-3-yl]-4H-pyrido[3,2-b][1,4]oxazin-3-one ;
la 6-[5-[[(4-chloro-2,3-dihydro-1H-indén-2-yl)méthylamino]méthyl]-2-oxo-1,3-oxazolidin-3-yl]-4H-pyrido[3,2-b][1,4]oxazin-3-one ;
la 6-[5-[[(4-chloro-2,3-dihydro-1H-indén-2-yl)amino]méthyl]-2-oxo-1,3-oxazolidin-3-yl]-4H-pyrido[3,2-b][1,4]oxazin-3-one ;
la 6-[(4R)-4-[[3-[(4-fluoro-2,3-dihydro-1H-indén-2-yl)amino]cyclobutyl]amino]-2-oxopyrrolidin-1-yl]-4H-pyrido[3,2-b][1,4]oxazin-3-one ;
la 6-[(4S)-4-[[3-[(4-fluoro-2,3-dihydro-1H-indén-2-yl)amino]cyclobutyl]amino]-2-oxopyrrolidin-1-yl]-4H-pyrido[3,2-b][1,4]oxazin-3-one ;
la 6-[5-[[(5-chloro-2,3-dihydro-1H-indén-2-yl)amino]méthyl]-2-oxo-1,3-oxazolidin-3-yl]-4H-pyrido[3,2-b][1,4]oxazin-3-one ;
la 6-[5-[[2-(4-chloro-2,3-dihydro-1H-indén-2-yl)éthylamino]méthyl]-2-oxo-1,3-oxazolidin-3-yl]-4H-pyrido[3,2-b][1,4]oxazin-3-one ;
la 6-[5-[3-[(4-méthyl-6,7-dihydro-5H-cyclopenta[b]pyridin-6-yl)amino]propyl]-2-oxo-1,3-oxazolidin-3-yl]-4H-pyrido[3,2-b][1,4]oxazin-3-one ;
le 2-[[2-oxo-3-(3-oxo-4H-pyrido[3,2-b][1,4]oxazin-6-yl)-1-oxa-3,8-diazaspiro[4.5]décan-8-yl]méthyl]-2,3-dihydro-1H-indène-4-carbonitrile ;
le 2-[[[2-méthyl-1-[2-oxo-3-(3-oxo-4H-pyrido[3,2-b][1,4]oxazin-6-yl)-1,3-oxazolidin-5-yl]propan-2-yl]amino]méthyl]-2,3-dihydro-1H-indène-4-carbonitrile ;
la 6-[5-[2-[(1-chloro-6,7-dihydro-SH-cyclopenta[c]pyridin-6-yl)méthylamino]-2-méthylpropyl]-2-oxo-1,3-oxazolidin-3-yl]-4H-pyrido[3,2-b][1,4]oxazin-3-one ;
le 2-[[[rac-3a,7a-trans-6,7a-cis-2-oxo-3-(3-oxo-4H-pyrido[3,2-b][1,4]oxazin-6-yl)-3 a,4, 5,6,7,7a-hexahydro-1,3-benzoxazol-6-yl] amino]méthyl]-2,3-dihydro-1H-indène-4-carbonitrile ;
la 6-[rac-3a,7a-trans-6,7a-cis-6-[2-(4-chloro-2,3-dihydro-1H-indén-2-yl)éthylamino]-2-oxo-3a,4,5,6,7,7a-hexahydro-1,3-benzoxazol-3-yl]-4H-pyrido[3,2-b][1,4]oxazin-3-one ;
le 2-[2-[6-oxo-7-(3-oxo-4H-pyrido[3,2-b][1,4]oxazin-6-yl)-5-oxa-2,7-diazaspiro[3.4]octan-2-yl]éthyl]-2,3-dihydro-1H-indène-4-carbonitrile ;
le 2-[[trans-4-[2-oxo-3-(3-oxo-4H-pyrido[3,2-b][1,4]oxazin-6-yl)-1,3-oxazolidin-5-yl]cyclohexyl]amino]-2,3-dihydro-1H-indène-4-carbonitrile ;
le 2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
la 6-[5-[2-[(4-fluoro-1-méthyl-6,7-dihydro-5H-cyclopenta[c]pyridin-6-yl)méthylamino]éthyl]-2-oxo-1,3-oxazolidin-3-yl]-4H-pyrido[3,2-b][1,4]oxazin-3-one ;
le 4-fluoro-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-1-carbonitrile ;
la 6-[5-[2-[(1-chloro-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
le 2-[[2-oxo-3-(3-oxo-4H-pyrido[3,2-b][1,4]oxazin-6-yl)-1-oxa-3-azaspiro[4.5]décan-8-yl]amino]-2,3-dihydro-1H-indène-4-carbonitrile ;
la 6-[2-oxo-5-[2-[[rac-(5,6-trans)-1-chloro-5-hydroxy-6,7-dihydro-SH-cyclopenta[c]pyridin-6-yl]méthylamino]éthyl]-1,3-oxazolidin-3-yl]-4H-pyrido[3,2-b][1,4]oxazin-3-one ;
le 2-[[2-[5-oxo-4-(3-oxo-4H-1,4-benzoxazin-6-yl)-1,3,4-oxadiazol-2-yl]éthylamino]méthyl]-2,3-dihydro-1H-indène-4-carbonitrile ;
la 6-[2-oxo-5-[2-[[rac-(5,6-trans)-5-hydroxy-1-méthyl-6,7-dihydro-SH-cyclopenta[c]pyridin-6-yl]méthylamino]éthyl]-1,3-oxazolidin-3-yl]-4H-pyrido[3,2-b][1,4]oxazin-3-one ;
le 2-[[2-[5-oxo-4-(3-oxo-4H-pyrido[3,2-b][1,4]oxazin-6-yl)-1,3,4-oxadiazol-2-yl]éthylamino]méthyl]-2,3-dihydro-1H-indène-4-carbonitrile ;
la 7-[5-[2-[(1-chloro-6,7-dihydro-SH-cyclopenta[c]pyridin-6-yl)méthylamino]éthyl]-2-oxo-1,3-oxazolidin-3-yl]-1H-pyrido[2,3-b][1,4]oxazin-2-one ; chlorhydrate ;
le 2-[[2-[2-oxo-3-(2-oxo-1H-pyrido[2,3-b][1,4]oxazin-7-yl)-1,3-oxazolidin-5-yl]éthylamino]méthyl]-2,3-dihydro-1H-indène-4-carbonitrile ;
le 1-méthyl-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-4-carbonitrile ;
le 2-[[4-cyano-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N-méthylacétamide ;
la 6-[5-[2-[(1-chloro-3-méthoxy-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
la 6-[5-[2-[(1-chloro-3-méthoxy-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
la 6-[5-[2-[(4-fluoro-1-méthyl-6,7-dihydro-5H-cyclopenta[c]pyridin-6-yl)méthylamino]éthyl]-2-oxo-1,3-oxazolidin-3-yl]-4H-pyrazino[2,3-b][1,4]oxazin-3-one ;
le 5-[(1-méthylpyrrolidin-2-yl)méthoxy]-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 3-méthoxy-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-1-carbonitrile ;
le 5-(2-morpholin-4-yléthoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[5-[2-[(4-chloro-1-méthyl-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ; acide formique ;
le N-[2-[[4-cyano-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]éthyl]acétamide ;
la 6-[5-[2-[(1-chloro-4-méthoxy-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
la 6-[5-[2-[(1-chloro-4-méthoxy-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
le N-[2-[[4-cyano-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]éthyl]méthanesulfonamide ;
la 6-[5-[2-[(4-chloro-1-méthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
le 2-[[[(2R)-2-hydroxy-2-[(5R)-2-oxo-3-(3-oxo-4H-1,4-benzoxazin-6-yl)-1,3-oxazolidin-5-yl]éthyl]amino]méthyl]-2,3-dihydro-1H-indène-4-carbonitrile ;
le 2-[[[(2S)-2-hydroxy-2-[(5S)-2-oxo-3-(3-oxo-4H-1,4-benzoxazin-6-yl)-1,3-oxazolidin-5-yl]éthyl]amino]méthyl]-2,3-dihydro-1H-indène-4-carbonitrile ;
le 5-(oxétan-3-yloxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 2-[[2-[2-oxo-3-(3-oxo-4H-pyrido[3,2-b][1,4]thiazin-6-yl)-1,3-oxazolidin-5-yl]éthylamino]méthyl]-2,3-dihydro-1H-indène-4-carbonitrile ;
le 3-méthoxy-6-[[2-[2-oxo-3-(3-oxo-4H-pyrazino[2,3-b][1,4]oxazin-6-yl)oxazolidin-5-yl]éthylamino]méthyl]-6,7-dihydro-5H-cyclopenta[c]pyridine-1-carbonitrile ;
le 2-[[1-chloro-6-[[2-[2-oxo-3-(3-oxo-4H-pyrido[3,2-b][1,4]oxazin-6-yl)oxazolidin-5-yl]éthylamino]méthyl]-6,7-dihydro-5H-cyclopenta[c]pyridin-3-yl]oxy]-N-méthyl-acétamide ;
la 6-[5-[2-[(4-fluoro-1-méthyl-6,7-dihydro-5H-cyclopenta[c]pyridin-6-yl)méthylamino]éthyl]-2-oxo-1,3,4-oxadiazol-3-yl]-4H-1,4-benzoxazin-3-one ;
la 6-[5-[2-[(4-fluoro-1-méthyl-6,7-dihydro-5H-cyclopenta[c]pyridin-6-yl)méthylamino]éthyl]-2-oxo-1,3,4-oxadiazol-3-yl]-4H-pyrido[3,2-b][1,4]oxazin-3-one ;
le 2-[[7-fluoro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N-méthylacétamide ;
la 6-[5-[2-[(4-fluoro-1-méthoxy-6,7-dihydro-SH-cyclopenta[c]pyridin-6-yl)méthylamino]éthyl]-2-oxo-oxazolidin-3-yl]-4H-pyrido[3,2-b][1,4]oxazin-3-one ;
la 6-[5-[2-[(4-fluoro-1-méthoxy-6,7-dihydro-SH-cyclopenta[c]pyridin-6-yl)méthylamino]éthyl]-2-oxo-oxazolidin-3-yl]-4H-pyrazino[2,3-b][1,4]oxazin-3-one ;
la 6-[8-[(4-fluoro-1-méthyl-6,7-dihydro-SH-cyclopenta[c]pyridin-6-yl)méthyl]-2-oxo-1-oxa-3,8-diazaspiro[4.5]décan-3-yl]-4H-pyrido[3,2-b][1,4]oxazin-3-one ;
le 2-[[7-fluoro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]acétonitrile ;
le 2-[[7-fluoro-2-[[2-[2-oxo-3-(3-oxo-4H-pyrido[3,2-b][1,4]thiazin-6-yl)-1,3-oxazolidin-5-yl]éthylamino]méthyl]-2,3-dihydro-1H-indén-5-yl]oxy]-N-méthylacétamide ;
la 6-[5-[1-[(4-fluoro-1-méthyl-6,7-dihydro-SH-cyclopenta[c]pyridin-6-yl)méthyl]azétidin-3-yl]-2-oxo-oxazolidin-3-yl]-4H-pyrazino[2,3-b][1,4]oxazin-3-one ;
le 2-[[3-[2-oxo-3-(3-oxo-4H-pyrazino[2,3-b][1,4]oxazin-6-yl)-1,3-oxazolidin-5-yl]azétidin-1-yl]méthyl]-2,3-dihydro-1H-indène-4-carbonitrile ;
le 2-[[7-fluoro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N,N-diméthylacétamide ;
la 6-[5-[3-[(4-fluoro-1-méthyl-6,7-dihydro-SH-cyclopenta[c]pyridin-6-yl)amino]propyl]-2-oxo-1,3-oxazolidin-3-yl]-4H-pyrido[3,2-b][1,4]oxazin-3-one ;
la 6-[5-[3-[(4-fluoro-1-méthyl-6,7-dihydro-5H-cyclopenta[c]pyridin-6-yl)amino]propyl]-2-oxo-1,3-oxazolidin-3-yl]-4H-pyrazino[2,3-b][1,4]oxazin-3-one ;
le 2-[3-[2-oxo-3-(3-oxo-4H-pyrazino[2,3-b][1,4]oxazin-6-yl)-1,3-oxazolidin-5-yl]propylamino]-2,3-dihydro-1H-indène-4-carbonitrile ;
le 2-[[2-hydroxy-3-[2-oxo-3-(3-oxo-4H-pyrazino[2,3-b][1,4]oxazin-6-yl)-1,3-oxazolidin-5-yl]propyl]amino]-2,3-dihydro-1H-indène-4-carbonitrile ;
la 6-[5-[3-[(4-fluoro-2,3-dihydro-1H-indén-2-yl)amino]-2-hydroxypropyl]-2-oxo-1,3-oxazolidin-3-yl]-4H-pyrazino[2,3-b][1,4]oxazin-3-one ;
la 6-[5-[2-[(4-fluoro-1-méthyl-6,7-dihydro-5H-cyclopenta[c]pyridin-6-yl)méthylamino]éthyl]-2-oxo-1,3-oxazol-3-yl]-4H-pyrido[3,2-b][1,4]oxazin-3-one ;
la 6-[5-[2-[(1-chloro-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
la 6-[5-[2-(2,3-dihydro-1H-indén-2-ylméthylamino)éthyl]-2-oxo-1,3-oxazolidin-3-yl]-4H-pyrido[3,2-b][1,4]oxazin-3-one ;
la 6-[5-[2-[(1-méthyl-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
la 6-[5-[2-[(4-méthyl-6,7-dihydro-5H-cyclopenta[b]pyridin-6-yl)méthylamino]éthyl]-2-oxo-1,3-oxazolidin-3-yl]-4H-pyrido[3,2-b][1,4]oxazin-3-one ;
le 6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-1-carbonitrile ;
la 6-[5-[2-[(4-fluoro-2,3-dihydro-1H-indén-2-yl)méthylamino]éthyl]-2-oxo-1,3-oxazolidin-3-yl]-4H-pyrido[3,2-b][1,4]oxazin-3-one ;
la 6-[5-[3-[(1-chloro-6,7-dihydro-SH-cyclopenta[c]pyridin-6-yl)amino]propyl]-2-oxo-1,3-oxazolidin-3-yl]-4H-pyrido[3,2-b][1,4]oxazin-3-one ;
la 6-[2-oxo-5-[2-[(1,3,4-triméthyl-6,7-dihydro-SH-cyclopenta[c]pyridin-6-yl)méthylamino]éthyl]-1,3-oxazolidin-3-yl]-4H-pyrido[3,2-b][1,4]oxazin-3-one ;
la 6-[5-[3-[(1-méthyl-6,7-dihydro-SH-cyclopenta[c]pyridin-6-yl)amino]propyl]-2-oxo-1,3-oxazolidin-3-yl]-4H-pyrido[3,2-b][1,4]oxazin-3-one ;
le 6-[3-[2-oxo-3-(3-oxo-4H-pyrido[3,2-b][1,4]oxazin-6-yl)-1,3-oxazolidin-5-yl]propylamino]-6,7-dihydro-SH-cyclopenta[c]pyridine-1-carbonitrile ;
le 2-[[[cis-3-[2-oxo-3-(3-oxo-4H-pyrido[3,2-b][1,4]oxazin-6-yl)-1,3-oxazolidin-5-yl]cyclobutyl]amino]méthyl]-2,3-dihydro-1H-indène-4-carbonitrile ; acide formique ;
le 2-[[[trans-3-[2-oxo-3-(3-oxo-4H-pyrido[3,2-b][1,4]oxazin-6-yl)-1,3-oxazolidin-5-yl]cyclobutyl]amino]méthyl]-2,3-dihydro-1H-indène-4-carbonitrile ;
la 6-[5-[2-[(1,4-diméthyl-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
le 2-[[[rac-3a,7a-cis-6,7a-trans-2-oxo-3-(3-oxo-4H-pyrido[3,2-b][1,4]oxazin-6-yl)-3 a,4, 5,6,7,7a-hexahydro-1,3-benzoxazol-6-yl] amino]méthyl]-2,3-dihydro-1H-indène-4-carbonitrile ;
le 2-[[[trans-4-[2-oxo-3-(3-oxo-4H-pyrido[3,2-b][1,4]oxazin-6-yl)-1,3-oxazolidin-5-yl]cyclohexyl]amino]méthyl]-2,3-dihydro-1H-indène-4-carbonitrile ;
la 6-[5-[2-[[1,4-diméthyl-3-[(5-oxomorpholin-2-yl)méthoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[1,4-diméthyl-3-[(5-oxopyrrolidin-3-yl)méthoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[1,4-diméthyl-3-[(1-méthyl-5-oxopyrrolidin-3-yl)méthoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
le 6-[[[2,4-trans-6-oxo-7-(3-oxo-4H-pyrido[3,2-b][1,4]oxazin-6-yl)-5-oxa-7-azaspiro[3.4]octan-2-yl]amino]méthyl]-6,7-dihydro-SH-cyclopenta[c]pyridine-1-carbonitrile ;
la 6-[5-[2-[[1,4-diméthyl-3-(3-méthylsulfonylpropoxy)-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[(3-méthoxy-1,4-diméthyl-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
le 2-[[7-cyano-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N-méthylacétamide ;
le 2-[[4-chloro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N-méthylacétamide ;
le 2-[[4-chloro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ;
le 2-[[1,4-diméthyl-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridin-3-yl]oxy]-N-méthylacétamide ;
le 2-[[1,4-diméthyl-6-[[2-[2-oxo-3-(3-oxo-4H-pyrazino[2,3-b][1,4]oxazin-6-yl)-1,3-oxazolidin-5-yl]éthylamino]méthyl]-6,7-dihydro-5H-cyclopenta[c]pyridin-3-yl]oxy]-N-méthylacétamide ;
le 2-[[4,7-diméthyl-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N-méthylacétamide ;
le 2-[[4,7-diméthyl-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ;
le 2-[[4,7-diméthyl-2-[[2-[2-oxo-3-(3-oxo-4H-pyrido[3,2-b][1,4]thiazin-6-yl)-1,3-oxazolidin-5-yl]éthylamino]méthyl]-2,3-dihydro-1H-indén-5-yl]oxy]-N-méthylacétamide ;
le N-[2-[[7-cyano-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]éthyl]méthanesulfonamide ;
la 6-[5-[2-[[5-(2-aminoéthoxy)-4-chloro-2,3-dihydro-1H-indén-2-yl]méthylamino]éthyl]-2-oxo-1,3-oxazolidin-3-yl]-4H-pyrido[3,2-b][1,4]oxazin-3-one ;
la 6-[5-[2-[[5-[(1-aminocyclopropyl)méthoxy]-4-chloro-2,3-dihydro-1H-indén-2-yl]méthylamino]éthyl]-2-oxo-1,3-oxazolidin-3-yl]-4H-pyrido[3,2-b][1,4]oxazin-3-one ; acide formique ;
la 6-[5-[3-[[5-(2-aminoéthoxy)-4-chloro-2,3-dihydro-1H-indén-2-yl]amino]propyl]-2-oxo-1,3-oxazolidin-3-yl]-4H-pyrido[3,2-b][1,4]oxazin-3-one ;
la 6-[5-[3-[[5-[(1-aminocyclopropyl)méthoxy]-4-chloro-2,3-dihydro-1H-indén-2-yl]amino]propyl]-2-oxo-1,3-oxazolidin-3-yl]-4H-pyrido[3,2-b][1,4]oxazin-3-one ;
la 6-[4-[2-[(4-fluoro-2,3-dihydro-1H-indén-2-yl)amino]éthylamino]-2-oxopyrrolidin-1-yl]-4H-pyrido[3,2-b][1,4]oxazin-3-one ;
le 2-[2-[[5-oxo-1-(3-oxo-4H-pyrido[3,2-b][1,4]oxazin-6-yl)pyrrolidin-3-yl]amino]éthylamino]-2,3-dihydro-1H-indène-4-carbonitrile ;
la 6-[5-[2-[[3-[(1-aminocyclopropyl)méthoxy]-1-chloro-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[2-oxo-5-[2-[[5,6-trans-5-amino-1-chloro-6,7-dihydro-5H-cyclopenta[c]pyridin-6-yl]méthylamino]éthyl]-1,3-oxazolidin-3-yl]-4H-pyrido[3,2-b][1,4]oxazin-3-one ;
le chlorhydrate de 5-[(1-aminocyclopropyl)méthoxy]-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 3-[(1-aminocyclopropyl)méthoxy]-6-[[2-[2-oxo-3-(3-oxo-4H-pyrido[3,2-b][1,4]oxazin-6-yl)-1,3-oxazolidin-5-yl]éthylamino]méthyl]-6,7-dihydro-SH-cyclopenta[c]pyridine-1-carbonitrile ;
la 6-[5-[2-[[3-(2-aminoéthoxy)-1-chloro-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
le 3-(2-aminoéthoxy)-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-1-carbonitrile ;
le 5-(2-aminoéthoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 5-(azétidin-3-ylméthoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[2-oxo-5-[2-[(rac-5,6-trans-5-amino-1-méthyl-6,7-dihydro-SH-cyclopenta[c]pyridin-6-yl)méthylamino]éthyl]-1,3-oxazolidin-3-yl]-4H-pyrido[3,2-b][1,4]oxazin-3-one ;
le rac-1,2-trans-1-amino-2-[[2-oxo-3-(3-oxo-4H-pyrido[3,2-b][1,4]oxazin-6-yl)-1-oxa-3,8-diazaspiro[4.5]décan-8-yl]méthyl]-2,3-dihydro-1H-indène-4-carbonitrile ;
le 5-(azétidin-3-yloxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le rac-5,6-trans-5-amino-6-[[2-[2-oxo-3-(3-oxo-4H-pyrido[3,2-b][1,4]oxazin-6-yl)-1,3-oxazolidin-5-yl]éthylamino]méthyl]-6,7-dihydro-SH-cyclopenta[c]pyridine-1-carbonitrile ;
le 5-(2-aminopropoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 7-[2-oxo-5-[2-[[rac-1,2-trans-1-amino-4-chloro-2,3-dihydro-1H-indén-2-yl]méthylamino]éthyl]-1,3-oxazolidin-3-yl]-1H-pyrido[2,3-b][1,4]oxazin-2-one ;
la 6-[2-oxo-5-[2-[[rac-1,2-trans-1-amino-4-chloro-2,3-dihydro-1H-indén-2-yl]méthylamino]éthyl]-1,3-oxazolidin-3-yl]-4H-pyrazino[2,3-b][1,4]oxazin-3-one ;
le 5-(azétidin-2-ylméthoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 2-[[2-[3-amino-5-oxo-1-(3-oxo-4H-pyrido[3,2-b][1,4]oxazin-6-yl)pyrrolidin-3-yl]éthylamino]méthyl]-2,3-dihydro-1H-indène-4-carbonitrile ;
le rac-1,2-trans-1-hydroxy-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[4-amino-4-[2-[(4-chloro-2,3-dihydro-1H-indén-2-yl)méthylamino]éthyl]-2-oxopyrrolidin-1-yl]-4H-pyrido[3,2-b][1,4]oxazin-3-one ;
le 2-[[2-[2-oxo-3-(3-oxo-4H-pyrido[3,2-b][1,4]oxazin-6-yl)-1,3-oxazolidin-5-yl]éthylamino]méthyl]-5-(pyrrolidin-2-ylméthoxy)-2,3-dihydro-1H-indène-4-carbonitrile ;
la 6-[5-[[2-(2-amino-4-chloro-1,3-dihydroindén-2-yl)éthylamino]méthyl]-2-oxo-1,3-oxazolidin-3-yl]-4H-pyrido[3,2-b][1,4]oxazin-3-one ; acide formique ;
le 2-[[2-[2-oxo-3-(3-oxo-4H-pyrido[3,2-b][1,4]oxazin-6-yl)-1,3-oxazolidin-5-yl]éthylamino]méthyl]-5-(2-pipérazin-1-yléthoxy)-2,3-dihydro-1H-indène-4-carbonitrile ;
la 6-[5-[2-[[3-(2-aminopropoxy)-1-chloro-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-(2-aminopropoxy)-1-chloro-6,7-dihydro-SH-cyclopenta[c]pyridin-6-yl]méthylamino]éthyl]-2-oxo-oxazolidin-3-yl]-4H-pyrazino[2,3-b][1,4]oxazin-3-one ;
la 6-[2-oxo-5-[2-[[rac-5,6-cis-5-amino-1-chloro-6,7-dihydro-5H-cyclopenta[c]pyridin-6-yl]méthylamino]éthyl]-1,3-oxazolidin-3-yl]-4H-pyrido[3,2-b][1,4]oxazin-3-one ;
la 6-[2-oxo-5-[2-[[rac-1,2-trans-1-amino-4-chloro-2,3-dihydro-1H-indén-2-yl]méthylamino]éthyl]-1,3,4-oxadiazol-3-yl]-4H-1,4-benzoxazin-3-one ;
le 2-[[2-[rac-4,5-cis-4-(aminométhyl)-2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 2-[[2-[rac-4,5-trans-4-(aminométhyl)-2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[5-[2-[[6-(azétidin-3-yloxy)-4-fluoro-2,3-dihydro-1H-indén-2-yl]méthylamino]éthyl]-2-oxo-1,3-oxazolidin-3-yl]-4H-pyrido[3,2-b][1,4]oxazin-3-one ;
le rac-2,3-trans-3-hydroxy-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le rac-2,3-trans-3-hydroxy-2-[[2-[2-oxo-3-(3-oxo-4H-pyrido[3,2-b][1,4]thiazin-6-yl)-1,3-oxazolidin-5-yl]éthylamino]méthyl]-2,3-dihydro-1H-indène-4-carbonitrile ;
la 6-[5-[2-[(rac-1,2-trans-1-amino-4-chloro-2,3-dihydro-1H-indén-2-yl)méthylamino]éthyl]-2-oxo-1,3,4-oxadiazol-3-yl]-4H-pyrido[3,2-b][1,4]oxazin-3-one ;
la 6-[5-[2-[[6-(2-aminopropoxy)-4-fluoro-2,3-dihydro-1H-indén-2-yl]méthylamino]éthyl]-2-oxo-1,3-oxazolidin-3-yl]-4H-pyrido[3,2-b][1,4]oxazin-3-one ;
la 6-[5-[2-[[6-(1-aminopropan-2-yloxy)-4-fluoro-2,3-dihydro-1H-indén-2-yl]méthylamino]éthyl]-2-oxo-1,3-oxazolidin-3-yl]-4H-pyrido[3,2-b][1,4]oxazin-3-one ;
le rac-2,3-trans-3-amino-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le rac-2,3-trans-3-amino-2-[[2-[2-oxo-3-(3-oxo-4H-pyrido[3,2-b][1,4]thiazin-6-yl)-1,3-oxazolidin-5-yl]éthylamino]méthyl]-2,3-dihydro-1H-indène-4-carbonitrile ;
la 6-[5-[[[2-amino-2-(4-chloro-2,3-dihydro-1H-indén-2-yl)éthyl]amino]méthyl]-2-oxo-1,3-oxazolidin-3-yl]-4H-pyrido[3,2-b][1,4]oxazin-3-one ;
la 6-[2-[2-amino-2-(4-chloro-2,3-dihydro-1H-indén-2-yl)éthyl]-6-oxo-5-oxa-2,7-diazaspiro[3.4]octan-7-yl]-4H-pyrido[3,2-b][1,4]oxazin-3-one ;
la 6-[5-[2-[[6-[(1-aminocyclopropyl)méthoxy]-4-fluoro-2,3-dihydro-1H-indén-2-yl]méthylamino]éthyl]-2-oxo-1,3-oxazolidin-3-yl]-4H-pyrido[3,2-b][1,4]oxazin-3-one ;
la 6-[5-[2-[[6-[(1-aminocyclopropyl)méthoxy]-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one
la 6-[5-[2-[[6-[(3-aminooxétan-3-yl)méthoxy]-4-fluoro-2,3-dihydro-1H-indén-2-yl]méthylamino]éthyl]-2-oxo-1,3-oxazolidin-3-yl]-4H-pyrido[3,2-b][1,4]oxazin-3-one ;
la 6-[5-[2-[(rac-1,2-trans-1-amino-4-fluoro-2,3-dihydro-1H-indén-2-yl)méthylamino]éthyl]-2-oxo-1,3-oxazolidin-3-yl]-4H-pyrido[3,2-b][1,4]oxazin-3-one ;
la 6-[5-[2-[(rac-1,2-trans-1-amino-4-fluoro-2,3-dihydro-1H-indén-2-yl)méthylamino]éthyl]-2-oxo-1,3-oxazolidin-3-yl]-4H-pyrazino[2,3-b][1,4]oxazin-3-one ;
la 5-[2-[(rac-1,2-trans-1-amino-4-fluoro-2,3-dihydro-1H-indén-2-yl)méthylamino]éthyl]-3-(3-oxo-4H-pyrido[3,2-b][1,4]thiazin-6-yl)-1,3-oxazolidin-2-one ;
le 2-[[rac-5,6-cis-5,8-trans-6-amino-2-oxo-3-(3-oxo-4H-pyrido[3,2-b][1,4]oxazin-6-yl)-1-oxa-3-azaspiro[4.5]décan-8-yl]amino]-2,3-dihydro-1H-indène-4-carbonitrile ;
la 6-[rac-5,6-cis-5,8-trans-6-amino-8-[(4-fluoro-2,3-dihydro-1H-indén-2-yl)amino]-2-oxo-1-oxa-3-azaspiro[4.5]décan-3-yl]-4H-pyrido[3,2-b][1,4]oxazin-3-one ;
la 6-[2-oxo-5-[2-[[rac-1,2-trans-1-amino-7-chloro-2,3-dihydro-1H-indén-2-yl]méthylamino]éthyl]-1,3-oxazolidin-3-yl]-4H-pyrido[3,2-b][1,4]oxazin-3-one ;
la 6-[2-oxo-5-[2-[[rac-1,2-trans-1-amino-7-chloro-2,3-dihydro-1H-indén-2-yl]méthylamino]éthyl]-1,3-oxazolidin-3-yl]-4H-pyrazino[2,3-b][1,4]oxazin-3-one ;
la 6-[2-oxo-5-[2-[[rac-1,2-trans-1-amino-4-chloro-2,3-dihydro-1H-indén-2-yl]méthylamino]éthyl]-1,3-oxazol-3-yl]-4H-pyrido[3,2-b][1,4]oxazin-3-one ;
la 6-[5-[2-[[3-(2-aminoéthoxy)-1,4-diméthyl-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-[(1-aminocyclopropyl)méthoxy]-1,4-diméthyl-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[1,4-diméthyl-3-[2-(méthylamino)éthoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-(2-aminoéthoxy)-4-chloro-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-[(1-aminocyclopropyl)méthoxy]-1-méthyl-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-(3-aminopropoxy)-1,4-diméthyl-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[6-(2-aminoéthoxy)-4-chloro-2,3-dihydro-1H-indén-2-yl]méthylamino]éthyl]-2-oxo-1,3-oxazolidin-3-yl]-4H-pyrido[3,2-b][1,4]oxazin-3-one ;
la 6-[5-[2-[[6-[(1-aminocyclopropyl)méthoxy]-4-chloro-2,3-dihydro-1H-indén-2-yl]méthylamino]éthyl]-2-oxo-1,3-oxazolidin-3-yl]-4H-pyrido[3,2-b][1,4]oxazin-3-one ;
la 6-[5-[3-[[6-(2-aminoéthoxy)-4-chloro-2,3-dihydro-1H-indén-2-yl]amino]propyl]-2-oxo-1,3-oxazolidin-3-yl]-4H-pyrido[3,2-b][1,4]oxazin-3-one ;
la 6-[5-[3-[[6-[(1-aminocyclopropyl)méthoxy]-4-chloro-2,3-dihydro-1H-indén-2-yl]amino]propyl]-2-oxo-1,3-oxazolidin-3-yl]-4H-pyrido[3,2-b][1,4]oxazin-3-one ;
la 6-[5-[2-[[3-[(1-aminocyclopropyl)méthoxy]-4-chloro-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
le 6-[[[2,4-cis-6-oxo-7-(3-oxo-4H-pyrido[3,2-b][1,4]oxazin-6-yl)-5-oxa-7-azaspiro[3.4]octan-2-yl]amino]méthyl]-6,7-dihydro-SH-cyclopenta[c]pyridine-1-carbonitrile ;
le 2-[[[2,4-cis-6-oxo-7-(3-oxo-4H-pyrido[3,2-b][1,4]oxazin-6-yl)-5-oxa-7-azaspiro[3.4]octan-2-yl]amino]méthyl]-2,3-dihydro-1H-indène-4-carbonitrile ;
la 6-[5-[2-[[3-(azétidin-3-ylméthoxy)-1,4-diméthyl-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
le 6-[(1-aminocyclopropyl)méthoxy]-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 6-((1-aminocyclopropyl)méthoxy)-2-(((2-(2-oxo-3-(3-oxo-3,4-dihydro-2H-pyrazino[2,3-b][1,4]oxazin-6-yl)oxazolidin-5-yl)éthyl)amino)méthyl)-2,3-dihydro-1H-indène-4-carbonitrile ;
le 6-(azétidin-2-ylméthoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 6-(2-aminoéthoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[5-[2-[[3-[2-(cyclopropylamino)éthoxy]-1,4-diméthyl-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
le 6-(azétidin-3-yloxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 6-(azétidin-3-ylméthoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 6-(2-aminopropoxy)-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le 6-(2-aminopropoxy)-2-(((2-(2-oxo-3-(3-oxo-3,4-dihydro-2H-pyrazino[2,3-b][1,4]oxazin-6-yl)oxazolidin-5-yl)éthyl)amino)méthyl)-2,3-dihydro-1H-indène-4-carbonitrile ;
la 6-[2-oxo-5-[[2-[rac-1,2-trans-1-amino-4-chloro-2,3-dihydro-1H-indén-2-yl]éthylamino]méthyl]-1,3-oxazolidin-3-yl]-4H-pyrido[3,2-b][1,4]oxazin-3-one ;
la 6-[6-oxo-2-[2-[rac-1,2-trans-1-amino-4-chloro-2,3-dihydro-1H-indén-2-yl]éthyl]-5-oxa-2,7-diazaspiro[3.4]octan-7-yl]-4H-pyrido[3,2-b][1,4]oxazin-3-one ;
le 6-((1-aminocyclopropyl)méthoxy)-2-(2-(((2-oxo-3-(3-oxo-3,4-dihydro-2H-pyrido[3,2-b][1,4]oxazin-6-yl)oxazolidin-5-yl)méthyl)amino)éthyl)-2,3-dihydro-1H-indène-4-carbonitrile ;
le 6-((1-aminocyclopropyl)méthoxy)-2-(2-(6-oxo-7-(3-oxo-3,4-dihydro-2H-pyrido[3,2-b][1,4]oxazin-6-yl)-5-oxa-2,7-diazaspiro[3.4]octan-2-yl)éthyl)-2,3-dihydro-1H-indène-4-carbonitrile ;
le 3-[(1-aminocyclopropyl)méthoxy]-6-[[2-[2-oxo-3-(3-oxo-4H-pyrido[3,2-b][1,4]oxazin-6-yl)-1,3-oxazolidin-5-yl]éthylamino]méthyl]-6,7-dihydro-SH-cyclopenta[c]pyridine-4-carbonitrile ;
la 6-[5-[2-[[1,4-diméthyl-3-(morpholin-3-ylméthoxy)-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-[[(2S)-azétidin-2-yl]méthoxy]-1,4-diméthyl-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[1,4-diméthyl-3-[2-(propan-2-ylamino)éthoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-[2-(2-méthoxyéthylamino)éthoxy]-1,4-diméthyl-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
le 6-(2-amino-2-méthylpropoxy)-2-(((2-(2-oxo-3-(3-oxo-3,4-dihydro-2H-pyrido[3,2-b][1,4]oxazin-6-yl)oxazolidin-5-yl)éthyl)amino)méthyl)-2,3-dihydro-1H-indène-4-carbonitrile ;
le 6-(2-amino-2-méthylpropoxy)-2-(((2-(2-oxo-3-(3-oxo-3,4-dihydro-2H-pyrazino[2,3-b][1,4]oxazin-6-yl)oxazolidin-5-yl)éthyl)amino)méthyl)-2,3-dihydro-1H-indène-4-carbonitrile ;
la 6-[5-[1-[(rac-1,2-trans-1-amino-4-chloro-2,3-dihydro-1H-indén-2-yl)méthyl]azétidin-3-yl]-2-oxo-1,3-oxazolidin-3-yl]-4H-pyrazino[2,3-b][1,4]oxazin-3-one ;
la 6-[rac-5,6-cis-5,8-trans-6-amino-8-[(1-chloro-6,7-dihydro-SH-cyclopenta[c]pyridin-6-yl)méthylamino]-2-oxo-1-oxa-3-azaspiro[4.5]décan-3-yl]-4H-pyrido[3,2-b][1,4]oxazin-3-one ;
la 6-[2-oxo-5-[2-[[1,2-trans-1-amino-4-chloro-2,3-dihydro-1H-indén-2-yl]méthylamino]éthyl]-1,3-oxazolidin-3-yl]-4H-pyrido[3,2-b][1,4]oxazin-3-one ;
la 6-[5-[2-[[3-(2-aminoéthoxy)-1-méthyl-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 2,4-cis-6-[6-oxo-2-[[rac-1,2-trans-1-amino-4-chloro-2,3-dihydro-1H-indén-2-yl]méthylamino]-5-oxa-7-azaspiro[3.4]octan-7-yl]-4H-pyrido[3,2-b][1,4]oxazin-3-one ;
la 6-[6-oxo-2-[[rac-(1R,2R)-1-amino-4-chloro-2,3-dihydro-1H-indén-2-yl]méthylamino]-5-oxa-7-azaspiro[3.4]octan-7-yl]-4H-pyrido[3,2-b][1,4]oxazin-3-one ;
la 6-[2-oxo-8-[[rac-1,2-trans-1-amino-4-chloro-2,3-dihydro-1H-indén-2-yl]méthyl]-1-oxa-3,8-diazaspiro[4.5]décan-3-yl]-4H-pyrido[3,2-b][1,4]oxazin-3-one ;
le 3-[(1-aminocyclopropyl)méthoxy]-4-méthyl-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-1-carbonitrile ;
le 3-[(1-aminocyclopropyl)méthoxy]-1-méthyl-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-4-carbonitrile ;
le 3-[(1-aminocyclopropyl)méthoxy]-1-méthyl-6-[[2-oxo-3-(3-oxo-4H-pyrido[3,2-b][1,4]oxazin-6-yl)-1-oxa-3,8-diazaspiro[4.5]décan-8-yl]méthyl]-6,7-dihydro-5H-cyclopenta[c]pyridine-4-carbonitrile ;
la 6-[5-[2-[[3-[(1-aminocyclopropyl)méthoxy]-4-méthyl-1-(5-méthyl-1,3-thiazol-2-yl)-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-[(1-aminocyclopropyl)méthoxy]-4-méthyl-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[3-[(1-aminocyclopropyl)méthoxy]-4-méthyl-1-(1,3-thiazol-2-yl)-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[4-méthyl-3-[[(2R)-azétidin-2-yl]méthoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
le 4-méthyl-6-((2-oxo-3-(3-oxo-3,4-dihydro-2H-pyrido[3,2-b][1,4]oxazin-6-yl)-1-oxa-3,8-diazaspiro[4.5]décan-8-yl)méthyl)-3-((5-oxopipérazin-2-yl)méthoxy)-6,7-dihydro-5H-cyclopenta[c]pyridine-1-carbonitrile ;
le 2-((1-cyano-4-méthyl-6-(((2-(2-oxo-3-(3-oxo-3,4-dihydro-2H-pyrido[3,2-b][1,4]oxazin-6-yl)oxazolidin-5-yl)éthyl)amino)méthyl)-6,7-dihydro-5H-cyclopenta[c]pyridin-3-yl)oxy)-N-méthylacétamide ;
le 2-((1-cyano-4-méthyl-6-(((2-(2-oxo-3-(3-oxo-3,4-dihydro-2H-pyrido[3,2-b][1,4]oxazin-6-yl)oxazolidin-5-yl)éthyl)amino)méthyl)-6,7-dihydro-5H-cyclopenta[c]pyridin-3-yl)oxy)-N-isopropylacétamide ;
le N-méthyl-2-((4-méthyl-6-(((2-(2-oxo-3-(3-oxo-3,4-dihydro-2H-pyrido[3,2-b][1,4]oxazin-6-yl)oxazolidin-5-yl)éthyl)amino)méthyl)-6,7-dihydro-5H-cyclopenta[c]pyridin-3-yl)oxy)acétamide ;
le N-isopropyl-2-((4-méthyl-6-(((2-(2-oxo-3-(3-oxo-3,4-dihydro-2H-pyrido[3,2-b][1,4]oxazin-6-yl)oxazolidin-5-yl)éthyl)amino)méthyl)-6,7-dihydro-5H-cyclopenta[c]pyridin-3-yl)oxy)acétamide ;
le 1-éthyl-6-fluoro-4-oxo-8-[[2-[2-oxo-3-(3-oxo-4H-pyrido[3,2-b][1,4]oxazin-6-yl)-1,3-oxazolidin-5-yl]éthylamino]méthyl]-8,9-dihydro-7H-cyclopenta[h]quinoléine-3-carboxylate d'éthyle ;
la 6-[2-oxo-5-[2-[[rac-1,2-trans-1-amino-4-chloro-2,3-dihydro-1H-indén-2-yl]méthylamino]éthyl]-1,3-oxazolidin-3-yl]-4H-pyrido[3,2-b][1,4]oxazin-3-one ;
la 6-[2-oxo-5-[3-[[rac-1,2-trans-1-amino-4-chloro-2,3-dihydro-1H-indén-2-yl]méthylamino]propyl]-1,3-oxazolidin-3-yl]-4H-pyrido[3,2-b][1,4]oxazin-3-one ;
le rac-1,2-trans-1-amino-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
le rac-1,2-trans-1-amino-2-((((2-oxo-3-(3-oxo-3,4-dihydro-2H-pyrido[3,2-b][1,4]oxazin-6-yl)oxazolidin-5-yl)méthyl)amino)méthyl)-2,3-dihydro-1H-indène-4-carbonitrile ;
la 6-[2-oxo-5-[2-[[rac-1,2-trans-4-chloro-1-hydroxy-2,3-dihydro-1H-indén-2-yl]méthylamino]éthyl]-1,3-oxazolidin-3-yl]-4H-pyrido[3,2-b][1,4]oxazin-3-one ;
la 6-[2-oxo-5-[2-[[rac-1,2-cis-4-chloro-1-hydroxy-2,3-dihydro-1H-indén-2-yl]méthylamino]éthyl]-1,3-oxazolidin-3-yl]-4H-pyrido[3,2-b][1,4]oxazin-3-one ;
la 6-[5-[2-[(4-chloro-2-hydroxy-1,3-dihydroindén-2-yl)méthylamino]éthyl]-2-oxo-1,3-oxazolidin-3-yl]-4H-pyrido[3,2-b][1,4]oxazin-3-one ;
le 2-[[2-aminoéthyl-[2-[2-oxo-3-(3-oxo-4H-pyrido[3,2-b][1,4]oxazin-6-yl)-1,3-oxazolidin-5-yl]éthyl]amino]méthyl]-2,3-dihydro-1H-indène-4-carbonitrile ;
la 6-[5-[2-[[1,4-diméthyl-3-[(2R)-2-amino-3-hydroxypropoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
le 3-[(2R)-2-amino-3-hydroxypropoxy]-4-méthyl-6-[[2-[2-oxo-3-(3-oxo-4H-pyrido[3,2-b][1,4]oxazin-6-yl)-1,3-oxazolidin-5-yl]éthylamino]méthyl]-6,7-dihydro-5H-cyclopenta[c]pyridine-1-carbonitrile ;
le 6-[(2R)-2-amino-3-hydroxypropoxy]-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[5-[2-[[4-chloro-3-[rac-(2R)-2-amino-3-hydroxypropoxy]-6,7-dihydro-5H-cyclopenta[c]pyridin-6-yl]méthylamino]éthyl]-2-oxo-1,3-oxazolidin-3-yl]-4H-pyrido[3,2-b][1,4]oxazin-3-one ;
le 2-[[2-hydroxy-3-[2-oxo-3-(3-oxo-4H-pyrido[3,2-b][1,4]oxazin-6-yl)-1,3-oxazolidin-5-yl]propyl]amino]-2,3-dihydro-1H-indène-4-carbonitrile ;
l'acide 1-éthyl-6-fluoro-4-oxo-8-[[2-[2-oxo-3-(3-oxo-4H-pyrido[3,2-b][1,4]oxazin-6-yl)-1,3-oxazolidin-5-yl]éthylamino]méthyl]-8,9-dihydro-7H-cyclopenta[h]quinoléine-3-carboxylique ;
la 6-[5-[2-[(2-amino-4-chloro-1,3-dihydroindén-2-yl)méthylamino]éthyl]-2-oxo-1,3-oxazolidin-3-yl]-4H-pyrido[3,2-b][1,4]oxazin-3-one ;
le 2-[[rac-5,6-trans-5,8-cis-6-amino-2-oxo-3-(3-oxo-4H-pyrido[3,2-b][1,4]oxazin-6-yl)-1-oxa-3-azaspiro[4.5]décan-8-yl]amino]-2,3-dihydro-1H-indène-4-carbonitrile ;
la 6-[5-(aminométhyl)-5-[2-[(4-fluoro-1-méthyl-6,7-dihydro-5H-cyclopenta[c]pyridin-6-yl)méthylamino]éthyl]-2-oxo-1,3-oxazolidin-3-yl]-4H-pyrido[3,2-b][1,4]oxazin-3-one ;
le 2-[[2-[5-(aminométhyl)-2-oxo-3-(3-oxo-4H-pyrido[3,2-b][1,4]oxazin-6-yl)-1,3-oxazolidin-5-yl]éthylamino]méthyl]-2,3-dihydro-1H-indène-4-carbonitrile ;
la 6-[2-oxo-5-[3-[[rac-1,2-trans-1-amino-4-chloro-2,3-dihydro-1H-indén-2-yl]amino]propyl]-1,3-oxazolidin-3-yl]-4H-pyrido[3,2-b][1,4]oxazin-3-one ;
la 6-[2-oxo-5-[3-[[rac-1,2-trans-1-amino-4-chloro-2,3-dihydro-1H-indén-2-yl]amino]propyl]-1,3-oxazolidin-3-yl]-4H-pyrazino[2,3-b][1,4]oxazin-3-one ;
la 5-[2-[(rac-1,2-trans-1-amino-4-chloro-2,3-dihydro-1H-indén-2-yl)méthylamino]éthyl]-3-(3-oxo-4H-pyrido[3,2-b][1,4]thiazin-6-yl)-1,3-oxazolidin-2-one ;
le rac-1,2-trans-1-amino-2-[[2-[2-oxo-3-(3-oxo-4H-pyrido[3,2-b][1,4]thiazin-6-yl)-1,3-oxazolidin-5-yl]éthylamino]méthyl]-2,3-dihydro-1H-indène-4-carbonitrile ;
la 6-[5-[2-[[6-(2-amino-3-méthoxypropoxy)-4-fluoro-2,3-dihydro-1H-indén-2-yl]méthylamino]éthyl]-2-oxo-1,3-oxazolidin-3-yl]-4H-pyrido[3,2-b][1,4]oxazin-3-one ;
le 2-[[rac-5,6-trans-6-(aminométhyl)-2-oxo-3-(3-oxo-4H-pyrido[3,2-b][1,4]oxazin-6-yl)-1-oxa-3,8-diazaspiro[4.5]décan-8-yl]méthyl]-2,3-dihydro-1H-indène-4-carbonitrile ;
la 6-[rac-5,6-trans-6-(aminométhyl)-8-[(4-fluoro-1-méthyl-6,7-dihydro-5H-cyclopenta[c]pyridin-6-yl)méthyl]-2-oxo-1-oxa-3,8-diazaspiro[4.5]décan-3-yl]-4H-pyrido[3,2-b][1,4]oxazin-3-one ;
la 6-[rac-5,6-cis-6-(aminométhyl)-8-[(4-fluoro-1-méthyl-6,7-dihydro-5H-cyclopenta[c]pyridin-6-yl)méthyl]-2-oxo-1-oxa-3,8-diazaspiro[4.5]décan-3-yl]-4H-pyrido[3,2-b][1,4]oxazin-3-one ;
le 2-[[rac-5,6-cis-6-(aminométhyl)-2-oxo-3-(3-oxo-4H-pyrido[3,2-b][1,4]oxazin-6-yl)-1-oxa-3,8-diazaspiro[4.5]décan-8-yl]méthyl]-2,3-dihydro-1H-indène-4-carbonitrile ;
la 6-[5-[2-[[5-[(1-aminocyclopropyl)méthoxy]-4,7-diméthyl-2,3-dihydro-1H-indén-2-yl]méthylamino]éthyl]-2-oxo-1,3-oxazolidin-3-yl]-4H-pyrazino[2,3-b][1,4]oxazin-3-one ;
le 2-[[2-[2-oxo-3-(3-oxo-4H-pyrido[3,2-b][1,4]oxazin-6-yl)-1,3-oxazolidin-5-yl]éthylamino]méthyl]-6-pyrrolidin-3-yloxy-2,3-dihydro-1H-indène-4-carbonitrile ; acide formique
le 2-amino-3-[[7-fluoro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N-méthylpropanamide ;
la 6-[5-[2-[(1-chloro-4-fluoro-3-méthyl-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
la 6-[8-[[6-[(1-aminocyclopropyl)méthoxy]-4-fluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ;
la 6-[8-[(4-fluoro-1-méthyl-6,7-dihydro-SH-cyclopenta[c]pyridin-6-yl)méthyl]-2-oxo-1-oxa-3,8-diazaspiro[4.5]décan-3-yl]-4H-pyrazino[2,3-b][1,4]oxazin-3-one ;
la 6-[2-oxo-8-[[rac-1,2-trans-1-amino-4-chloro-2,3-dihydro-1H-indén-2-yl]méthyl]-1-oxa-3,8-diazaspiro[4.5]décan-3-yl]-4H-pyrazino[2,3-b][1,4]oxazin-3-one ;
la 6-[8-[[6-(2-aminopropoxy)-4-fluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ;
le 2-[[2-oxo-3-(3-oxo-4H-pyrazino[2,3-b][1,4]oxazin-6-yl)-1-oxa-3,8-diazaspiro[4.5]décan-8-yl]méthyl]-2,3-dihydro-1H-indène-4-carbonitrile ;
la 6-[5-[2-[(rac-5,6-trans-5-amino-4-chloro-1-méthyl-6,7-dihydro-SH-cyclopenta[c]pyridin-6-yl)méthylamino]éthyl]-2-oxo-1,3-oxazolidin-3-yl]-4H-pyrido[3,2-b][1,4]oxazin-3-one ;
la 6-[2-oxo-5-[2-[[rac-5,6-trans-5-amino-4-chloro-1-méthyl-6,7-dihydro-5H-cyclopenta[c]pyridin-6-yl]méthylamino]éthyl]-1,3-oxazolidin-3-yl]-4H-pyrazino[2,3-b][1,4]oxazin-3-one ;
la 3-(3-oxo-4H-pyrido[3,2-b][1,4]thiazin-6-yl)-5-[2-[[rac-5,6-trans-5-amino-4-chloro-1-méthyl-6,7-dihydro-SH-cyclopenta[c]pyridin-6-yl]méthylamino]éthyl]-1,3-oxazolidin-2-one ;
la 6-[5-[[2-(4-fluoro-1-méthyl-6,7-dihydro-5H-cyclopenta[c]pyridin-6-yl)éthylamino]méthyl]-2-oxo-1,3-oxazolidin-3-yl]-4H-pyrazino[2,3-b][1,4]oxazin-3-one ;
la 6-[2-oxo-5-[[2-[rac-1,2-trans-1-amino-4-chloro-2,3-dihydro-1H-indén-2-yl]éthylamino]méthyl]-1,3-oxazolidin-3-yl]-4H-pyrazino[2,3-b][1,4]oxazin-3-one ;
l'acide 2-amino-3-[[7-fluoro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]propanoïque ;
la 6-[5-[2-[(1,4-diméthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
la 6-[2-oxo-5-[2-[(1,3,4-triméthyl-6,7-dihydro-SH-cyclopenta[c]pyridin-6-yl)méthylamino]éthyl]-1,3-oxazolidin-3-yl]-4H-pyrazino[2,3-b][1,4]oxazin-3-one ;
la 6-[5-[2-[(4-méthyl-6,7-dihydro-5H-cyclopenta[b]pyridin-6-yl)méthylamino]éthyl]-2-oxo-1,3-oxazolidin-3-yl]-4H-pyrazino[2,3-b][1,4]oxazin-3-one ;
la 6-[5-[2-[[6-[2-(diméthylamino)éthoxy]-4-fluoro-2,3-dihydro-1H-indén-2-yl]méthylamino]éthyl]-2-oxo-1,3-oxazolidin-3-yl]-4H-pyrido[3,2-b][1,4]oxazin-3-one ;
la 6-[5-[2-[[6-[2-(diméthylamino)éthoxy]-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ;
la 6-[2-oxo-5-[2-[[rac-1,2-trans-1-amino-6-[(1-aminocyclopropyl)méthoxy]-4-chloro-2,3-dihydro-1H-indén-2-yl]méthylamino]éthyl]-1,3-oxazolidin-3-yl]-4H-pyrido[3,2-b][1,4]oxazin-3-one ;
la 6-[2-oxo-8-[[rac-1,2-trans-1-amino-6-[(1-aminocyclopropyl)méthoxy]-4-chloro-2,3-dihydro-1H-indén-2-yl]méthyl]-1-oxa-3,8-diazaspiro[4.5]décan-3-yl]-4H-pyrido[3,2-b][1,4]oxazin-3-one ;
l'acide 1-éthyl-4-oxo-8-[[2-[2-oxo-3-(3-oxo-4H-pyrido[3,2-b][1,4]oxazin-6-yl)-1,3-oxazolidin-5-yl]éthylamino]méthyl]-8,9-dihydro-7H-cyclopenta[h]quinoléine-3-carboxylique ;
l'acide 1-éthyl-4-oxo-8-[[2-[2-oxo-3-(3-oxo-4H-pyrazino[2,3-b][1,4]oxazin-6-yl)-1,3-oxazolidin-5-yl]éthylamino]méthyl]-8,9-dihydro-7H-cyclopenta[h]quinoléine-3-carboxylique ;
la 6-[8-[(4-fluoro-1-méthyl-6,7-dihydro-SH-cyclopenta[c]pyridin-6-yl)méthyl]-6-(hydroxyméthyl)-2-oxo-1-oxa-3,8-diazaspiro[4.5]décan-3-yl]-4H-pyrido[3,2-b][1,4]oxazin-3-one ;
la 6-[5-[[2-(4-fluoro-1-méthyl-6,7-dihydro-5H-cyclopenta[c]pyridin-6-yl)éthylamino]méthyl]-5-(2-hydroxyéthyl)-2-oxo-1,3-oxazolidin-3-yl]-4H-pyrido[3,2-b][1,4]oxazin-3-one ;
la 6-[5-[2-[(1-méthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ; acide formique ;
la 6-[5-[[2-(6-amino-4-fluoro-1-méthyl-5,7-dihydrocyclopenta[c]pyridin-6-yl)éthylamino]méthyl]-2-oxo-1,3-oxazolidin-3-yl]-4H-pyrido[3,2-b][1,4]oxazin-3-one ;
la 6-[5-[2-[(4-chloro-2,3-dihydro-1H-indén-2-yl)méthylamino]éthyl]-2-oxo-1,3-oxazolidin-3-yl]-4H-pyrazino[2,3-b][1,4]oxazin-3-one ;
la 6-[2-oxo-5-[2-[[rac-1,2-trans-1-amino-6-[(1-aminocyclopropyl)méthoxy]-4-chloro-2,3-dihydro-1H-indén-2-yl]méthylamino]éthyl]-1,3-oxazolidin-3-yl]-4H-pyrazino[2,3-b][1,4]oxazin-3-one ; acide formique ;
la 6-[5-[2-[(1,4-diméthyl-6,7-dihydro-SH-cyclopenta[d]pyridazin-6-yl)méthylamino]éthyl]-2-oxo-1,3-oxazolidin-3-yl]-4H-pyrazino[2,3-b][1,4]oxazin-3-one ;
la 6-[5-[2-[(1,4-diméthyl-6,7-dihydro-5H-cyclopenta[d]pyridazin-6-yl)méthylamino]éthyl]-2-oxo-1,3-oxazolidin-3-yl]-4H-pyrido[3,2-b][1,4]oxazin-3-one ;
la 5-[2-[(1,4-diméthyl-6,7-dihydro-5H-cyclopenta[d]pyridazin-6-yl)méthylamino]éthyl]-3-(3-oxo-4H-pyrido[3,2-b][1,4]thiazin-6-yl)-1,3-oxazolidin-2-one ;
le 2-[[4-cyano-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ;
la 6-[5-[2-[(4-fluoro-6,7-dihydro-5H-cyclopenta[c]pyridin-6-yl)méthylamino]éthyl]-2-oxo-1,3-oxazolidin-3-yl]-4H-pyrazino[2,3-b][1,4]oxazin-3-one ;
la 6-[5-[2-[(4-fluoro-6,7-dihydro-5H-cyclopenta[c]pyridin-6-yl)méthylamino]éthyl]-2-oxo-1,3-oxazolidin-3-yl]-4H-pyrido[3,2-b][1,4]oxazin-3-one ;
le 2-[[2-[2-oxo-3-(7-oxo-8H-pyrimido[5,4-b][1,4]oxazin-2-yl)-1,3-oxazolidin-5-yl]éthylamino]méthyl]-2,3-dihydro-1H-indène-4-carbonitrile ;
la 2-[5-[2-[(rac-1,2-trans-1-amino-4-chloro-2,3-dihydro-1H-indén-2-yl)méthylamino]éthyl]-2-oxo-1,3-oxazolidin-3-yl]-8H-pyrimido[5,4-b][1,4]oxazin-7-one ;
le 1-méthyl-6-[[2-[2-oxo-3-(3-oxo-4H-pyrazino[2,3-b][1,4]oxazin-6-yl)-1,3-oxazolidin-5-yl]éthylamino]méthyl]-6,7-dihydro-5H-cyclopenta[c]pyridine-4-carbonitrile ; acide 2,2,2-trifluoroacétique ;
la 6-[5-[2-[[4-fluoro-6-(2-hydroxy-2-méthylpropoxy)-2,3-dihydro-1H-indén-2-yl]méthylamino]éthyl]-2-oxo-1,3-oxazolidin-3-yl]-4H-pyrazino[2,3-b][1,4]oxazin-3-one ;
la 6-[5-[2-[(4-fluoro-1-méthyl-6,7-dihydro-5H-cyclopenta[c]pyridin-6-yl)méthylamino]éthyl]-2-oxo-1,3-oxazol-3-yl]-4H-pyrazino[2,3-b][1,4]oxazin-3-one ;
la 6-[5-[2-[[6-[2-(5-aminotétrazol-1-yl)éthoxy]-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[6-[2-(5-aminotétrazol-2-yl)éthoxy]-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-[2-(1,2,4-triazol-1-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la trans-6-[5-[2-[[4-fluoro-6-[2-(1,2,4-triazol-1-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la cis-6-[5-[2-[[4-fluoro-6-[2-(1,2,4-triazol-1-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[(3-fluoro-4-méthyl-6,7-dihydro-5H-cyclopenta[b]pyridin-6-yl)méthylamino]éthyl]-2-oxo-1,3-oxazolidin-3-yl]-4H-pyrazino[2,3-b][1,4]oxazin-3-one ;
la 6-[5-[2-[(3-fluoro-4-méthyl-6,7-dihydro-5H-cyclopenta[b]pyridin-6-yl)méthylamino]éthyl]-2-oxo-1,3-oxazolidin-3-yl]-4H-pyrido[3,2-b][1,4]oxazin-3-one ;
la 6-[8-[(3-fluoro-4-méthyl-6,7-dihydro-5H-cyclopenta[b]pyridin-6-yl)méthyl]-2-oxo-1-oxa-3,8-diazaspiro[4.5]décan-3-yl]-4H-pyrazino[2,3-b][1,4]oxazin-3-one ;
la 6-[5-[3-[(4-fluoro-1-méthyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one ; acide formique ;
la 6-[5-[3-[(4-fluoro-1-méthyl-6,7-dihydro-SH-cyclopenta[c]pyridin-6-yl)-méthylamino]propyl]-2-oxo-1,3-oxazol-3-yl]-4H-pyrazino[2,3-b][1,4]oxazin-3-one ; acide formique ;
la 6-[5-[2-[[4-fluoro-6-[2-(2-oxo-1,3-oxazolidin-3-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
le 2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide ;
le 2-[[7-fluoro-2-[[2-[2-oxo-3-(3-oxo-4H-pyrazino[2,3-b][1,4]oxazin-6-yl)-1,3-oxazol-5-yl]éthylamino]méthyl]-2,3-dihydro-1H-indén-5-yl]oxy]acétamide ; acide formique ;
la 6-[5-[2-[[6-[2-(azétidin-1-yl)-2-oxoéthoxy]-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
le N-éthyl-2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide ; acide formique ;
le 2-[[7-fluoro-2-[[2-oxo-3-(3-oxo-4H-pyrazino[2,3-b][1,4]oxazin-6-yl)-1-oxa-3,8-diazaspiro[4.5]décan-8-yl]méthyl]-2,3-dihydro-1H-indén-5-yl]oxy]-N-méthylacétamide ;
le 2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ; acide formique ;
le 2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthoxyacétamide ; acide formique ;
le 2-[[4,7-difluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide ;
la 6-[2-oxo-5-[3-[(4-fluoro-1-méthyl-6,7-dihydro-SH-cyclopenta[c]pyridin-6-yl)amino]-2-hydroxypropyl]-1,3-oxazolidin-3-yl]-4H-pyrazino[2,3-b][1,4]oxazin-3-one ; acide formique, isomère A ;
la 6-[2-oxo-5-[3-[(4-fluoro-1-méthyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one ; acide formique, isomère B ;
la 6-[2-oxo-5-[3-[(4-fluoro-1-méthyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one ; acide formique, isomère C ;
la 6-[2-oxo-5-[3-[(4-fluoro-1-méthyl-6,7-dihydro-5H-cyclopenta[c]pyridin-6-yl)amino]-2-hydroxypropyl]-1,3-oxazolidin-3-yl]-4H-pyrazino[2,3-b][1,4]oxazin-3-one ; acide formique, isomère D ;
la 6-[5-[2-[(4-fluoro-6-hydroxy-2,3-dihydro-1H-indén-2-yl)méthylamino]éthyl]-2-oxo-1,3-oxazolidin-3-yl]-4H-pyrazino[2,3-b][1,4]oxazin-3-one ;
la 6-[5-[2-[(4-fluoro-6,7-dihydro-5H-cyclopenta[b]pyridin-6-yl)méthylamino]éthyl]-2-oxo-1,3-oxazolidin-3-yl]-4H-pyrazino[2,3-b][1,4]oxazin-3-one ;
la 6-[5-[2-[(4-chloro-3-fluoro-6,7-dihydro-5H-cyclopenta[b]pyridin-6-yl)méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-(1H-pyrazol-3-ylméthoxy)-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
le 1-méthyl-6-[3-[2-oxo-3-(3-oxo-4H-pyrazino[2,3-b][1,4]oxazin-6-yl)-1,3-oxazol-5-yl]propylamino]-6,7-dihydro-5H-cyclopenta[c]pyridine-4-carbonitrile, énantiomère A ;
le 1-méthyl-6-[3-[2-oxo-3-(3-oxo-4H-pyrazino[2,3-b][1,4]oxazin-6-yl)-1,3-oxazol-5-yl]propylamino]-6,7-dihydro-5H-cyclopenta[c]pyridine-4-carbonitrile, énantiomère B ;
la 6-[5-[2-[[4-fluoro-6-(oxétan-3-yloxy)-2,3-dihydro-1H-indén-2-yl]méthylamino]éthyl]-2-oxo-1,3-oxazolidin-3-yl]-4H-pyrazino[2,3-b][1,4]oxazin-3-one ;
le N-[2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]éthyl]méthanesulfonamide ;
le N-[2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]éthyl]-N-méthylméthanesulfonamide ;
la 6-[2-[2-(4-fluoro-1-méthyl-6,7-dihydro-SH-cyclopenta[c]pyridin-6-yl)éthyl]-6-oxo-5-oxa-2,7-diazaspiro[3.4]octan-7-yl]-4H-pyrazino[2,3-b][1,4]oxazin-3-one ;
la 6-[2-[2-[6-(2-aminopropoxy)-4-fluoro-2,3-dihydro-1H-indén-2-yl]éthyl]-6-oxo-5-oxa-2,7-diazaspiro[3.4]octan-7-yl]-4H-pyrazino[2,3-b][1,4]oxazin-3-one ;
la 6-[5-[3-[(4-fluoro-1-méthyl-6,7-dihydro-SH-cyclopenta[c]pyridin-6-yl)amino]cyclobutyl]-2-oxo-1,3-oxazolidin-3-yl]-4H-pyrazino[2,3-b][1,4]oxazin-3-one, isomère A ;
la 6-[5-[3-[(4-fluoro-1-méthyl-6,7-dihydro-SH-cyclopenta[c]pyridin-6-yl)amino]cyclobutyl]-2-oxo-1,3-oxazolidin-3-yl]-4H-pyrazino[2,3-b][1,4]oxazin-3-one, isomère B ;
la 6-[(5S)-5-[2-[1-(4-fluoro-1-méthyl-6,7-dihydro-5H-cyclopenta[c]pyridin-6-yl)éthylamino]éthyl]-2-oxo-1,3-oxazolidin-3-yl]-4H-pyrazino[2,3-b][1,4]oxazin-3-one ; acide formique ;
la 6-[(5R)-5-[2-[1-(4-fluoro-1-méthyl-6,7-dihydro-5H-cyclopenta[c]pyridin-6-yl)éthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-(2-imidazol-1-yléthoxy)-2,3-dihydro-1H-indén-2-yl]méthylamino]éthyl]-2-oxo-1,3-oxazolidin-3-yl]-4H-pyrazino[2,3-b][1,4]oxazin-3-one ;
la 6-[5-[3-[(4-fluoro-1-méthyl-6,7-dihydro-SH-cyclopenta[c]pyridin-6-yl)amino]butyl]-2-oxo-1,3-oxazolidin-3-yl]-4H-pyrazino[2,3-b][1,4]oxazin-3-one, isomère A ;
la 6-[5-[3-[(4-fluoro-1-méthyl-6,7-dihydro-SH-cyclopenta[c]pyridin-6-yl)amino]butyl]-2-oxo-1,3-oxazolidin-3-yl]-4H-pyrazino[2,3-b][1,4]oxazin-3-one, isomère B ;
la 6-[5-[2-[(4-fluoro-1-méthyl-6,7-dihydro-5H-cyclopenta[c]pyridin-6-yl)méthylamino]éthyl]-2-oxo-1,3,4-oxadiazol-3-yl]-4H-pyrazino[2,3-b][1,4]oxazin-3-one ;
le 2-[[4-fluoro-2-[[2-oxo-3-(3-oxo-4H-pyrazino[2,3-b][1,4]oxazin-6-yl)-1-oxa-3,8-diazaspiro[4.5]décan-8-yl]méthyl]-2,3-dihydro-1H-indén-5-yl]oxy]-N-méthylacétamide ;
la 6-[2-oxo-5-[2-[[rac-1,2-trans-1-amino-4-chloro-2,3-dihydro-1H-indén-2-yl]méthylamino]éthyl]-1,3,4-oxadiazol-3-yl]-4H-pyrazino[2,3-b][1,4]oxazin-3-one ; acide formique ;
la 6-[(5S)-5-[2-[(4-fluoro-3-méthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
la 6-[(5R)-5-[2-[(4-fluoro-3-méthyl-6,7-dihydro-5H-cyclopenta[c]pyridin-6-yl)méthylamino]éthyl]-2-oxo-1,3-oxazolidin-3-yl]-4H-pyrazino[2,3-b][1,4]oxazin-3-one ;
la 6-[5-[2-[(4-fluoro-1-méthyl-6,7-dihydro-5H-cyclopenta[c]pyridin-6-yl)méthylamino]éthyl]-5-méthyl-2-oxo-1,3-oxazolidin-3-yl]-4H-pyrazino[2,3-b][1,4]oxazin-3-one ;
la 6-[(5S)-5-[2-[[6-(2-amino-2-méthylpropoxy)-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[(5R)-5-[2-[[6-(2-amino-2-méthylpropoxy)-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-[2-(triazol-2-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-[2-(triazol-1-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 1-[2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]éthyl]pyrrolidine-2,5-dione ; acide formique ;
le N-cyclopropyl-2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide ; acide formique
la 6-[5-[2-[[4-fluoro-6-[2-(tétrazol-1-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-[2-(tétrazol-2-yl)éthoxy]-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
le 2-[4-[2-oxo-3-(3-oxo-4H-pyrazino[2,3-b][1,4]oxazin-6-yl)-1,3-oxazolidin-5-yl]pipéridin-1-yl]-2,3-dihydro-1H-indène-4-carbonitrile ;
la 6-[5-[1-(4-fluoro-2,3-dihydro-1H-indén-2-yl)pipéridin-4-yl]-2-oxo-1,3-oxazolidin-3-yl]-4H-pyrazino[2,3-b][1,4]oxazin-3-one ;
la 6-[5-[2-[[4-fluoro-6-(2-hydroxypropoxy)-2,3-dihydro-1H-indén-2-yl]méthylamino]éthyl]-2-oxo-1,3-oxazolidin-3-yl]-4H-pyrazino[2,3-b][1,4]oxazin-3-one ; chlorhydrate ;
la 6-[5-[2-[(4-fluoro-1,3-diméthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-(2-méthylsulfonyléthoxy)-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[6-(1-acétylazétidin-3-yl)oxy-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-6-(1-méthylsulfonylazétidin-3-yl)oxy-2,3-dihydro-1H-indén-2-yl]méthylamino]éthyl]-2-oxo-1,3-oxazolidin-3-yl]-4H-pyrazino[2,3-b][1,4]oxazin-3-one ; chlorhydrate ;
la 6-[8-[[6-[(2S)-2-aminopropoxy]-4-fluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ; acide formique ;
la 6-[8-[[6-[(2R)-2-aminopropoxy]-4-fluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ; acide formique ;
la 6-[5-[2-[[6-(azétidin-3-yloxy)-4-fluoro-2,3-dihydro-1H-indén-2-yl]méthylamino]éthyl]-2-oxo-1,3-oxazolidin-3-yl]-4H-pyrazino[2,3-b][1,4]oxazin-3-one ; chlorhydrate ;
la 6-[5-[2-[(rac-1,2-trans-1-amino-4-chloro-2,3-dihydro-1H-indén-2-yl)méthylamino]éthyl]-2-oxo-1,3-oxazol-3-yl]-4H-pyrazino[2,3-b][1,4]oxazin-3-one ; acide formique ;
la 6-[(SR)-5-[(1R)-2-[(4-fluoro-1-méthyl-6,7-dihydro-SH-cyclopenta[c]pyridin-6-yl)méthylamino]-1-hydroxyéthyl]-2-oxo-1,3-oxazolidin-3-yl]-4H-pyrazino[2,3-b][1,4]oxazin-3-one ; acide formique ;
la 6-[5-[trans-3-[(4-fluoro-1-méthyl-6,7-dihydro-SH-cyclopenta[c]pyridin-6-yl)méthylamino]cyclobutyl]-2-oxo-1,3-oxazolidin-3-yl]-4H-pyrazino[2,3-b][1,4]oxazin-3-one ;
la 6-[5-[cis-3-[(4-fluoro-1-méthyl-6,7-dihydro-SH-cyclopenta[c]pyridin-6-yl)méthylamino]cyclobutyl]-2-oxo-1,3-oxazolidin-3-yl]-4H-pyrazino[2,3-b][1,4]oxazin-3-one ;
le 4-fluoro-3-méthyl-6-[[2-[2-oxo-3-(3-oxo-4H-pyrazino[2,3-b][1,4]oxazin-6-yl)-1,3-oxazolidin-5-yl]éthylamino]méthyl]-6,7-dihydro-SH-cyclopenta[c]pyridine-1-carbonitrile ; acide formique ;
le 1-méthyl-6-[[2-[(5S)-2-oxo-3-(3-oxo-4H-pyrazino[2,3-b][1,4]oxazin-6-yl)-1,3-oxazolidin-5-yl]éthylamino]méthyl]-6,7-dihydro-5H-cyclopenta[c]pyridine-4-carbonitrile ;
le 1-méthyl-6-[[2-[(SR)-2-oxo-3-(3-oxo-4H-pyrazino[2,3-b][1,4]oxazin-6-yl)-1,3-oxazolidin-5-yl]éthylamino]méthyl]-6,7-dihydro-5H-cyclopenta[c]pyridine-4-carbonitrile ;
le 2-[[7-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétonitrile ; acide formique ;
la 6-[5-[3-[(4-fluoro-1-méthyl-6,7-dihydro-5H-cyclopenta[c]pyridin-6-yl)amino]propyl]-2-oxo-1,3-oxazol-3-yl]-4H-pyrazino[2,3-b][1,4]oxazin-3-one, énantiomère A ;
la 6-[5-[3-[(4-fluoro-1-méthyl-6,7-dihydro-SH-cyclopenta[c]pyridin-6-yl)amino]propyl]-2-oxo-1,3-oxazol-3-yl]-4H-pyrazino[2,3-b][1,4]oxazin-3-one, énantiomère B ;
la 6-[5-[3-[(1-méthyl-6,7-dihydro-SH-cyclopenta[c]pyridin-6-yl)amino]propyl]-2-oxo-1,3-oxazol-3-yl]-4H-pyrazino[2,3-b][1,4]oxazin-3-one ; acide formique, énantiomère A ;
la 6-[5-[3-[(1-méthyl-6,7-dihydro-SH-cyclopenta[c]pyridin-6-yl)amino]propyl]-2-oxo-1,3-oxazol-3-yl]-4H-pyrazino[2,3-b][1,4]oxazin-3-one ; acide formique, énantiomère B ;
la 6-[5-[2-[(1-méthyl-6,7-dihydro-SH-cyclopenta[c]pyridin-6-yl)méthylamino]éthyl]-2-oxo-1,3-oxazol-3-yl]-4H-pyrazino[2,3-b][1,4]oxazin-3-one ;
la 6-[5-[2-[(4-fluoro-6,7-dihydro-5H-cyclopenta[c]pyridin-6-yl)méthylamino]éthyl]-2-oxo-1,3-oxazol-3-yl]-4H-pyrazino[2,3-b][1,4]oxazin-3-one ;
la 6-[8-[(1-chloro-6,7-dihydro-SH-cyclopenta[c]pyridin-6-yl)méthyl]-2-oxo-1-oxa-3,8-diazaspiro[4.5]décan-3-yl]-4H-pyrazino[2,3-b][1,4]oxazin-3-one ;
la 6-[8-[(1-méthyl-6,7-dihydro-SH-cyclopenta[c]pyridin-6-yl)méthyl]-2-oxo-1-oxa-3,8-diazaspiro[4.5]décan-3-yl]-4H-pyrazino[2,3-b][1,4]oxazin-3-one ;
la 6-[5-méthyl-2-oxo-5-[2-[(rac-1,2-trans-1-amino-4-chloro-2,3-dihydro-1H-indén-2-yl)méthylamino]éthyl]-1,3-oxazolidin-3-yl]-4H-pyrazino[2,3-b][1,4]oxazin-3-one ;
la 6-[5-[2-[(4-fluoro-1-méthyl-6,7-dihydro-5H-cyclopenta[c]pyridin-6-yl)méthyl-méthylamino]éthyl]-2-oxo-1,3-oxazolidin-3-yl]-4H-pyrazino[2,3-b][1,4]oxazin-3-one ;
la 6-[8-[[6-(2-amino-2-méthylpropoxy)-4-fluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ; acide formique, énantiomère A ;
la 6-[8-[[6-(2-amino-2-méthylpropoxy)-4-fluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ; acide formique, énantiomère B ;
le 2-[[7-fluoro-2-[[2-[(5S)-2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide, isomère A ;
le 2-[[7-fluoro-2-[[2-[(5S)-2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide, isomère B ;
le 2-[[7-fluoro-2-[[2-[(5R)-2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide, isomère A ;
le 2-[[7-fluoro-2-[[2-[(5R)-2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide, isomère B ;
le 2-[[7-cyano-4-méthyl-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ;
le 2-[[4-cyano-7-méthyl-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ;
la 6-[5-[2-[(2-chloro-4-méthyl-6,7-dihydro-5H-cyclopenta[d]pyrimidin-6-yl)méthylamino]éthyl]-2-oxo-1,3-oxazolidin-3-yl]-4H-pyrido[3,2-b][1,4]oxazin-3-one ;
le 6-(1,3-oxazol-4-ylméthoxy)-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
le 6-[(3-méthyl-1,2,4-oxadiazol-5-yl)méthoxy]-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
le 6-[(1-méthylpyrazol-3-yl)méthoxy]-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
le 6-[(2-méthyltétrazol-5-yl)méthoxy]-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
le 6-[(1-méthylpyrazol-4-yl)méthoxy]-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
le 6-[(1-méthyltétrazol-5-yl)méthoxy]-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
le 6-[(4-méthyl-1,2,5-oxadiazol-3-yl)méthoxy]-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ;
le 2-[[4,7-difluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ;
le 2-[[4,7-difluoro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]-N-méthylacétamide ;
la 6-[5-[2-[[4,7-difluoro-5-(1H-pyrazol-3-ylméthoxy)-2,3-dihydro-1H-indén-2-yl]méthylamino]éthyl]-2-oxo-1,3-oxazolidin-3-yl]-4H-pyrazino[2,3-b][1,4]oxazin-3-one ;
la 6-[8-[[4,7-difluoro-5-(1H-pyrazol-3-ylméthoxy)-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ;
la 6-[5-[2-[(4-fluoro-3-méthoxy-1-méthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
la 6-[5-[2-[(3-chloro-4-fluoro-1-méthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ;
le 2-[[rac-2,3-trans-3-amino-7-chloro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]-N-méthylacétamide ; acide formique ;
le N-méthyl-2-[[rac-2,3-trans-3-amino-7-chloro-2-[[2-[2-oxo-3-(3-oxo-4H-pyrido[3,2-b][1,4]oxazin-6-yl)-1,3-oxazolidin-5-yl]éthylamino]méthyl]-2,3-dihydro-1H-indén-5-yl]oxy]acétamide ; acide formique ;
la 6-[(SS)-5-[2-[[(1R,2R)-1-amino-4-chloro-2,3-dihydro-1H-indén-2-yl]méthylamino]éthyl]-2-oxo-1,3-oxazolidin-3-yl]-4H-pyrazino[2,3-b][1,4]oxazin-3-one ;
la 6-[(SS)-5-[2-[[(1S,2S)-1-amino-4-chloro-2,3-dihydro-1H-indén-2-yl]méthylamino]éthyl]-2-oxo-1,3-oxazolidin-3-yl]-4H-pyrazino[2,3-b][1,4]oxazin-3-one ;
la 6-[(SR)-5-[2-[[(1R,2R)-1-amino-4-chloro-2,3-dihydro-1H-indén-2-yl]méthylamino]éthyl]-2-oxo-1,3-oxazolidin-3-yl]-4H-pyrazino[2,3-b][1,4]oxazin-3-one ;
le 2-[[2-[2-oxo-3-(3-oxo-4H-pyrazino[2,3-b][1,4]oxazin-6-yl)-1,3-oxazolidin-5-yl]éthylamino]méthyl]-6-(1H-pyrazol-3-ylméthoxy)-2,3-dihydro-1H-indène-4-carbonitrile ; acide formique ;
le 2-[[2-[2-oxo-3-(3-oxo-4H-pyrazino[2,3-b][1,4]oxazin-6-yl)-1,3-oxazolidin-5-yl]éthylamino]méthyl]-6-(1H-pyrazol-4-ylméthoxy)-2,3-dihydro-1H-indène-4-carbonitrile ; acide formique ;
le 6-(1H-imidazol-5-ylméthoxy)-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indène-4-carbonitrile ; acide formique ;
le 6-(2-amino-2-méthylpropoxy)-2-[[2-oxo-3-(3-oxo-4H-pyrazino[2,3-b][1,4]oxazin-6-yl)-1-oxa-3,8-diazaspiro[4.5]décan-8-yl]méthyl]-2,3-dihydro-1H-indène-4-carbonitrile ;
la 6-[5-[2-[[5-[(1-aminocyclopropyl)méthoxy]-4,7-difluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[5-[2-[[5-[(1-aminocyclopropyl)méthoxy]-4,7-difluoro-2,3-dihydro-1H-indén-2-yl]méthylamino]éthyl]-2-oxo-1,3-oxazolidin-3-yl]-4H-pyrido[3,2-b][1,4]oxazin-3-one ; acide formique ;
la 6-[8-[[5-[(1-aminocyclopropyl)méthoxy]-4,7-difluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ; acide formique ;
la 6-[5-[2-[[5-(2-amino-2-méthylpropoxy)-4,7-difluoro-2,3-dihydro-1H-indén-2-yl]méthylamino]éthyl]-2-oxo-1,3-oxazolidin-3-yl]-4H-pyrazino[2,3-b][1,4]oxazin-3-one ; acide formique ;
la 6-[8-[[5-(2-amino-2-méthylpropoxy)-4,7-difluoro-2,3-dihydro-1H-indén-2-yl]méthyl]-2-oxo-1-oxa-3,8-diazaspiro[4.5]décan-3-yl]-4H-pyrazino[2,3-b][1,4]oxazin-3-one ; acide formique ;
le 2-[[4-fluoro-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide ;
le 2-[[7-cyano-2-[[2-[2-oxo-3-(3-oxo-4H-pyrazino[2,3-b][1,4]oxazin-6-yl)-1,3-oxazolidin-5-yl]éthylamino]méthyl]-2,3-dihydro-1H-indén-5-yl]oxy]acétamide ;
la 6-[5-[2-[[1-méthyl-3-(1H-pyrazol-3-ylméthoxy)-6,7-dihydro-SH-cyclopenta[c]pyridin-6-yl]méthylamino]éthyl]-2-oxo-1,3-oxazolidin-3-yl]-4H-pyrazino[2,3-b][1,4]oxazin-3-one ;
la 6-[5-[2-[(5,8-diméthyl-5,7-diazatricyclo[7.3.0.02,6]dodéca-1,6,8-trién-11-yl)méthylamino]éthyl]-2-oxo-1,3-oxazolidin-3-yl]-4H-pyrido[3,2-b][1,4]oxazin-3-one ;
la 6-[4-[2-[(4-fluoro-1-méthyl-6,7-dihydro-5H-cyclopenta[c]pyridin-6-yl)méthylamino]éthyl]-2-oxopyrrolidin-1-yl]-4H-pyrido[3,2-b][1,4]oxazin-3-one ;
la 6-[5-[2-(6,7-dihydro-SH-cyclopenta[c]pyridin-6-ylméthylamino)éthyl]-2-oxo-1,3-oxazolidin-3-yl]-4H-pyrido[3,2-b][1,4]oxazin-3-one ;
la 6-[5-[2-[[3-(diméthylamino)-1-méthyl-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[[1-méthyl-3-(méthylamino)-6,7-dihydro-SH-cyclopenta[c]pyridin-6-yl]méthylamino]éthyl]-2-oxo-1,3-oxazolidin-3-yl]-4H-pyrido[3,2-b][1,4]oxazin-3-one ;
la 6-[5-[2-[(4-méthyl-6,7-dihydro-5H-cyclopenta[d]pyrimidin-6-yl)méthylamino]éthyl]-2-oxo-1,3-oxazolidin-3-yl]-4H-pyrido[3,2-b][1,4]oxazin-3-one ;
la 6-[(SR)-5-[2-[[(1S,2S)-1-amino-4-chloro-2,3-dihydro-1H-indén-2-yl]méthylamino]éthyl]-2-oxo-1,3-oxazolidin-3-yl]-4H-pyrazino[2,3-b][1,4]oxazin-3-one ;
la 6-[5-[2-[(8-méthyl-5,7-diazatricyclo[7.3.0.02,6]dodéca-1,6,8-trién-11-yl)méthylamino]éthyl]-2-oxo-1,3-oxazolidin-3-yl]-4H-pyrido[3,2-b][1,4]oxazin-3-one ;
la 6-[5-[2-[(5,8-diméthyl-5,7-diazatricyclo[7.3.0.02,6]dodéca-1,6,8-trién-11-yl)méthylamino]éthyl]-2-oxo-1,3-oxazolidin-3-yl]-4H-pyrazino[2,3-b][1,4]oxazin-3-one ;
le 2-[[2-[2-oxo-3-(3-oxo-4H-pyrazino[2,3-b][1,4]oxazin-6-yl)-1,3-oxazolidin-5-yl]éthylamino]méthyl]-6-(1H-triazol-5-ylméthoxy)-2,3-dihydro-1H-indène-4-carbonitrile ;
la 6-[5-[2-[[4-fluoro-1-(1H-pyrazol-5-yl)-6,7-dihydro-SH-cyclopenta[c]pyridin-6-yl]méthylamino]éthyl]-2-oxo-1,3-oxazolidin-3-yl]-4H-pyrazino[2,3-b][1,4]oxazin-3-one ;
la 6-[(SR)-5-[2-[[(6R)-4-fluoro-1-méthyl-6,7-dihydro-SH-cyclopenta[c]pyridin-6-yl]méthylamino]éthyl]-2-oxo-1,3-oxazolidin-3-yl]-4H-pyrazino[2,3-b][1,4]oxazin-3-one
la 6-[(5 S)-5-[2-[[(6R)-4-fluoro-1-méthyl-6,7-dihydro-SH-cyclopenta[c]pyridin-6-yl]méthylamino]éthyl]-2-oxo-1,3-oxazolidin-3-yl]-4H-pyrazino[2,3-b][1,4]oxazin-3-one ;
la 6-[(5S)-5-[2-[[(6S)-4-fluoro-1-méthyl-6,7-dihydro-5H-cyclopenta[c]pyridin-6-yl]méthylamino]éthyl]-2-oxo-1,3-oxazolidin-3-yl]-4H-pyrazino[2,3-b][1,4]oxazin-3-one ;
la 6-[(5R)-5-[2-[[(6S)-4-fluoro-1-méthyl-6,7-dihydro-5H-cyclopenta[c]pyridin-6-yl]méthylamino]éthyl]-2-oxo-1,3-oxazolidin-3-yl]-4H-pyrazino[2,3-b][1,4]oxazin-3-one ;
la 6-[5-[2-[(1-chloro-4-fluoro-3-méthyl-6,7-dihydro-SH-cyclopenta[c]pyridin-6-yl)méthylamino]éthyl]-2-oxo-1,3-oxazolidin-3-yl]-4H-pyrazino[2,3-b][1,4]oxazin-3-one ; acide formique ;
la 6-[5-[2-[[4-fluoro-1-(1H-pyrazol-4-yl)-6,7-dihydro-SH-cyclopenta[c]pyridin-6-yl]méthylamino]éthyl]-2-oxo-1,3-oxazolidin-3-yl]-4H-pyrazino[2,3-b][1,4]oxazin-3-one ;
la 6-[5-[2-[[4-fluoro-1-(1H-imidazol-5-yl)-6,7-dihydro-SH-cyclopenta[c]pyridin-6-yl]méthylamino]éthyl]-2-oxo-1,3-oxazolidin-3-yl]-4H-pyrazino[2,3-b][1,4]oxazin-3-one
et des sels pharmaceutiquement acceptables de ceux-ci.

10. Composé selon la revendication 1, choisi parmi
la 6-[5-[2-[(1-chloro-6,7-dihydro-5*H*-cyclopenta[c]pyridin-6-yl)méthylamino]éthyl]-2-oxo-1,3-oxazolidin-3-yl]-4*H*-pyrazino[2,3-b][1,4]oxazin-3-one ;
la 6-[5-[2-[(4-fluoro-1-méthyl-6,7-dihydro-5*H-*cyclopenta[c]pyridin-6-yl)méthylamino]éthyl]-2-oxo-1,3-oxazolidin-3-yl]-4*H*-pyrazino[2,3-b][1,4]oxazin-3-one ;
le 2-[[7-fluoro-2-[[2-[2-oxo-3-(3-oxo-4*H*-pyrido[3,2-b][1,4]oxazin-6-yl)-1,3-oxazolidin-5-yl]éthylamino]méthyl]-2,3-dihydro-1*H*-indén-5-yl]oxy]-*N*-méthylacétamide ; acide formique ;
la 6-[5-[2-[(1-méthyl-6,7-dihydro-5*H*-cyclopenta[c]pyridin-6-yl)méthylamino]éthyl]-2-oxo-1,3-oxazolidin-3-yl]-4*H*-pyrido[3,2-b][1,4]oxazin-3-one ;
la 6-[2-oxo-5-[2-[[*rac*-1,2-*trans*-1-amino-4-chloro-2,3-dihydro-1*H*-indén-2-yl]méthylamino]éthyl]-1,3-oxazolidin-3-yl]-4*H*-pyrazino[2,3-b][1,4]oxazin-3-one ; acide formique ;
la 6-[5-[2-[[6-[(1-aminocyclopropyl)méthoxy]-4-fluoro-2,3-dihydro-1*H*-indén-2-yl]méthylamino]éthyl]-2-oxo-1,3-oxazolidin-3-yl]-4*H*-pyrazino[2,3-b][1,4]oxazin-3-one ;
le 6-[(1-aminocyclopropyl)méthoxy]-2-[[2-[2-oxo-3-(3-oxo-4*H*-pyrido[3,2-b][1,4]oxazin-6-yl)-1,3-oxazolidin-5-yl]éthylamino]méthyl]-2,3-dihydro-1*H*-indène-4-carbonitrile ;
le 6-(2-aminopropoxy)-2-[[2-[2-oxo-3-(3-oxo-4*H*-pyrido[3,2-b][1,4]oxazin-6-yl)-1,3-oxazolidin-5-yl]éthylamino]méthyl]-2,3-dihydro-1*H*-indène-4-carbonitrile ; acide 2,2,2-trifluoroacétique ;
la 6-[2-oxo-5-[2-[[*rac-*1,2-*trans*-1-amino-4-chloro-2,3-dihydro-1*H*-indén-2-yl]méthylamino]éthyl]-1,3-oxazolidin-3-yl]-4*H*-pyrido[3,2-b][1,4]oxazin-3-one ;
la 6-[5-[2-[(1-méthyl-6,7-dihydro-5*H*-cyclopenta[c]pyridin-6-yl)méthylamino]éthyl]-2-oxo-1,3-oxazolidin-3-yl]-4*H*-pyrazino[2,3-b][1,4]oxazin-3-one ; acide formique ;
la 6-[5-[2-[(4-fluoro-6,7-dihydro-5*H*-cyclopenta[c]pyridin-6-yl)méthylamino]éthyl]-2-oxo-1,3-oxazolidin-3-yl]-4*H*-pyrazino[2,3-b][1,4]oxazin-3-one ;
le 1-méthyl-6-[[2-[2-oxo-3-(3-oxo-4*H*-pyrazino[2,3-b][1,4]oxazin-6-yl)-1,3-oxazolidin-5-yl]éthylamino]méthyl]-6,7-dihydro-5*H*-cyclopenta[c]pyridine-4-carbonitrile ; acide 2,2,2-trifluoroacétique ;
la 6-[5-[2-[(4-fluoro-1-méthyl-6,7-dihydro-5*H*-cyclopenta[c]pyridin-6-yl)méthylamino]éthyl]-2-oxo-1,3-oxazol-3-yl]-4*H*-pyrazino[2,3-b][1,4]oxazin-3-one ;
la 6-[5-[3-[(4-fluoro-1-méthyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one ; acide formique ;
le 2-[[7-fluoro-2-[[2-[2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]acétamide ;
le 2-[[7-fluoro-2-[[2-[2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]-*N*-méthylacétamide ; acide formique ;
le 2-[[4,7-difluoro-2-[[2-[2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]acétamide ;
la 6-[8-[[6-[(2*S*)-2-aminopropoxy]-4-fluoro-2,3-dihydro-1*H*-indén-2-yl]méthyl]-2-oxo-1-oxa-3,8-diazaspiro[4.5]décan-3-yl]-4*H*-pyrazino[2,3-b][1,4]oxazin-3-one ; acide formique ;
le 1-méthyl-6-[[2-[(5S)-2-oxo-3-(3-oxo-4*H*-pyrazino[2,3-b][1,4]oxazin-6-yl)-1,3-oxazolidin-5-yl]éthylamino]méthyl]-6,7-dihydro-5*H*-cyclopenta[c]pyridine-4-carbonitrile ;
le 1-méthyl-6-[[2-[(5*R*)-2-oxo-3-(3-oxo-4*H*-pyrazino[2,3-b][1,4]oxazin-6-yl)-1,3-oxazolidin-5-yl]éthylamino]méthyl]-6,7-dihydro-5*H*-cyclopenta[c]pyridine-4-carbonitrile ;
la 6-[5-[3-[(4-fluoro-1-méthyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one, énantiomère A ;
la 6-[5-[3-[(4-fluoro-1-méthyl-6,7-dihydro-5*H*-cyclopenta[c]pyridin-6-yl)amino]propyl]-2-oxo-1,3-oxazol-3-yl]-4*H*-pyrazino[2,3-b][1,4]oxazin-3-one, énantiomère B ;
la 6-[5-[2-[(1-méthyl-6,7-dihydro-5*H*-cyclopenta[c]pyridin-6-yl)méthylamino]éthyl]-2-oxo-1,3-oxazol-3-yl]-4*H*-pyrazino[2,3-b][1,4]oxazin-3-one ;
le 2-[[7-fluoro-2-[[2-[(5*S*)-2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]acétamide, isomère A ;
le 2-[[7-fluoro-2-[[2-[(5*S*)-2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]acétamide, isomère B ;
le 2-[[7-fluoro-2-[[2-[(5*R*)-2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]acétamide, isomère A ;
le 2-[[7-fluoro-2-[[2-[(5*R*)-2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]acétamide, isomère B ;
le 2-[[4,7-difluoro-2-[[2-[2-oxo-3-(3-oxo-4*H*-pyrazino[2,3-b][1,4]oxazin-6-yl)-1,3-oxazolidin-5-yl]éthylamino]méthyl]-2,3-dihydro-1*H*-indén-5-yl]oxy]-*N*-méthylacétamide ;
le 2-[[4,7-difluoro-2-[[2-[2-oxo-3-(3-oxo-4*H*-pyrido[3,2-b][1,4]oxazin-6-yl)-1,3-oxazolidin-5-yl]éthylamino]méthyl]-2,3-dihydro-1*H*-indén-5-yl]oxy]-*N*-méthylacétamide ;
la 6-[(5*R*)-5-[2-[[(1*R*,2*R*)-1-amino-4-chloro-2,3-dihydro-1*H*-indén-2-yl]méthylamino]éthyl]-2-oxo-1,3-oxazolidin-3-yl]-4*H*-pyrazino[2,3-b][1,4]oxazin-3-one ;
la 6-[(5*R*)-5-[2-[[(1*S*,2*S*)-1-amino-4-chloro-2,3-dihydro-1*H*-indén-2-yl]méthylamino]éthyl]-2-oxo-1,3-oxazolidin-3-yl]-4*H*-pyrazino[2,3-b][1,4]oxazin-3-one ;
la 6-[(5*R*)-5-[2-[[(6*R*)-4-fluoro-1-méthyl-6,7-dihydro-5*H*-cyclopenta[c]pyridin-6-yl]méthylamino]éthyl]-2-oxo-1,3-oxazolidin-3-yl]-4*H*-pyrazino[2,3-b][1,4]oxazin-3-one ;
la 6-[(5*S*)-5-[2-[[(6*R*)-4-fluoro-1-méthyl-6,7-dihydro-5*H*-cyclopenta[c]pyridin-6-yl]méthylamino]éthyl]-2-oxo-1,3-oxazolidin-3-yl]-4*H*-pyrazino[2,3-b][1,4]oxazin-3-one ;
la 6-[(5*S*)-5-[2-[[(6*S*)-4-fluoro-1-méthyl-6,7-dihydro-5*H*-cyclopenta[c]pyridin-6-yl]méthylamino]éthyl]-2-oxo-1,3-oxazolidin-3-yl]-4*H*-pyrazino[2,3-b][1,4]oxazin-3-one ;
la 6-[(5*R*)-5-[2-[[(6*S*)-4-fluoro-1-méthyl-6,7-dihydro-5*H*-cyclopenta[c]pyridin-6-yl]méthylamino]éthyl]-2-oxo-1,3-oxazolidin-3-yl]-4*H*-pyrazino[2,3-b][1,4]oxazin-3-one ;
la 6-[5-[2-[[4-fluoro-1-(1*H*-pyrazol-4-yl)-6,7-dihydro-5*H*-cyclopenta[c]pyridin-6-yl]méthylamino]éthyl]-2-oxo-1,3-oxazolidin-3-yl]-4*H*-pyrazino[2,3-b][1,4]oxazin-3-one ;
la 6-[5-[2-[[4-fluoro-1-(1*H*-imidazol-5-yl)-6,7-dihydro-5*H*-cyclopenta[c]pyridin-6-yl]méthylamino]éthyl]-2-oxo-1,3-oxazolidin-3-yl]-4*H*-pyrazino[2,3-b][1,4]oxazin-3-one ;
ou des sels pharmaceutiquement acceptables de ceux-ci.

11. Composé selon l'une quelconque des revendications 1 à 9 pour une utilisation comme substance thérapeutiquement active.

12. Composition pharmaceutique comprenant un composé selon l'une quelconque des revendications 1 à 10 et un véhicule thérapeutiquement inerte.

13. Composé selon l'une quelconque des revendications 1 à 10 ou un sel pharmaceutiquement acceptable de celui-ci pour une utilisation comme antibiotique.

14. Composé selon l'une quelconque des revendications 1 à 10 pour une utilisation dans le traitement ou la prophylaxie d'une infection bactérienne.

15. Composé selon l'une quelconque des revendications 1 à 10 pour une utilisation dans le traitement ou la prophylaxie la prophylaxie d'infections et de maladies qui en résultent provoquées par *Escherichia coli.*
